# EUROPÄISCHE PATENTSCHRIFT

(11) **EP 2 027 290 B1**
(45) Veröffentlichungstag und Bekanntmachung des Hinweises auf die Patenterteilung: **05.10.2011**
(21) Anmeldenummer: 07729452.8
(22) Anmeldetag: 23.05.2007
(51) Int. Cl.: C12Q 1/68

(54) **PRAEDIKTIVES GENEXPRESSIONSMUSTER FUER KOLOREKTALE KARZINOME**
PREDICTIVE GENE EXPRESSION PATTERN FOR COLORECTAL CARCINOMAS
MODÈLE D'EXPRESSION GÉNIQUE PRÉDICTIF POUR LE CANCER COLORECTAL

(30) Priorität: 24.05.2006 DE 102006024416
(43) Veröffentlichungstag der Anmeldung: 25.02.2009
(73) Patentinhaber: Friedrich-Alexander-Universität Erlangen-Nürnberg, 91054 Erlangen (DE)
(72) Erfinder: CRONER, Roland, 91341 Roettenbach (DE); HOHENBERGER, Werner, 91074 Herzogenaurach (DE); REINGRUBER, Bertram, 93049 Regensburg (DE); LAUSEN, Berthold, 91084 Erlangen (DE)
(74) Vertreter: Sandmann, Wolfgang
(86) Internationale Anmeldenummer: PCT/EP2007/055017
(87) Internationale Veröffentlichungsnummer: WO 2007/135174

(56) Entgegenhaltungen:
- GREENBAUM D ET AL: "COMPARING PROTEIN ABUNDANCE AND MRNA EXPRESSION LEVELS ON A GENOMIC SCALE" GENOME BIOLOGY (ONLINE), GB, Bd. 40, Nr. 9, 2003, Seiten 11701-11708, XP008036618 ISSN: 1465-6914
- GREENBAUM D ET AL: "Interrelating different types of genomic data, from proteome to secretome: 'oming in on function." GENOME RESEARCH SEP 2001, Bd. 11, Nr. 9, September 2001 (2001-09), Seiten 1463-1468, XP009088703 ISSN: 1088-9051
- OERNTOFT T F ET AL: "GENOME-WIDE STUDY OF GENE COPY NUMBERS, TRANSCRIPTS, AND PROTEIN LEVELS IN PAIRS OF NON-INVASIVE AND INVASIVE HUMAN TRANSITIONAL CELL CARCINOMAS" MOLECULAR AND CELLULAR PROTEOMICS, AMERICAN SOCIETY FOR BIOCHEMISTRY AND MOLECULAR BIOLOGY, US, Bd. 1, Nr. 1, 2002, Seiten 37-45, XP008015037 ISSN: 1535-9476
- CELIS A J E ET AL: "Gene expression profiling: monitoring transcription and translation products using DNA microarrays and proteomics" FEBS LETTERS, ELSEVIER, AMSTERDAM, NL, Bd. 480, Nr. 1, 25. August 2000 (2000-08-25), Seiten 2-16, XP004337487 ISSN: 0014-5793
- CRONER ROLAND S ET AL: "Microarray versus conventional prediction of lymph node metastasis in colorectal carcinoma." CANCER 15 JUL 2005, Bd. 104, Nr. 2, 15. Juli 2005 (2005-07-15), Seiten 395-404, XP009088697 ISSN: 0008-543X
- LI M ET AL: "GENES ASSOCIATED WITH LIVER METASTASIS OF COLON CANCER, IDENTIFIED BY GENOME-WIDE CDNA MICROARRAY" INTERNATIONAL JOURNAL OF ONCOLOGY, EDITORIAL ACADEMY OF THE INTERNATIONAL JOURNAL OF ONCOLOGY,, GR, Bd. 24, Nr. 2, Februar 2004 (2004-02), Seiten 305-312, XP009027443 ISSN: 1019-6439
- FREDERIKSEN CASPER MOLLER ET AL: "Classification of Dukes' B and C colorectal cancers using expression arrays" JOURNAL OF CANCER RESEARCH AND CLINICAL ONCOLOGY, SPRINGER INTERNATIONAL, BERLIN, DE, Bd. 129, Nr. 5, 15. Mai 2003 (2003-05-15), Seiten 263-271, XP009088733 ISSN: 0171-5216
- KWON HYUK-CHAN ET AL: "Gene expression profiling in lymph node-positive and lymph node-negative colorectal cancer" DISEASES OF THE COLON AND RECTUM, J.B. LIPPINCOT CO., PHILADELPHIA, US, Bd. 47, Nr. 2, Februar 2004 (2004-02), Seiten 141-152, XP009088734 ISSN: 0012-3706
- KOEHLER ASTRID ET AL: "Gene expression profiling of colorectal cancer and metastases divides tumours according to their clinicopathological stage" JOURNAL OF PATHOLOGY, CHICHESTER, SUSSEX, GB, Bd. 204, Nr. 1, September 2004 (2004-09), Seiten 65-74, XP009088735 ISSN: 0022-3417
- KOMURO K ET AL: "Right- and left-sided colorectal cancers display distinct expression profiles and the anatomical stratification allows a high accuracy prediction of lymph node metastasis", JOURNAL OF SURGICAL RESEARCH, ACADEMIC PRESS INC., SAN DIEGO, CA, US LNKD- DOI:10.1016/J.JSS.2004.10.009, vol. 124, no. 2, 1 April 2005 (2005-04-01) , pages 216-224, XP004835905, ISSN: 0022-4804
- LI M ET AL: "GENES ASSOCIATED WITH LIVER METASTASIS OF COLON CANCER, IDENTIFIED BY GENOME-WIDE CDNA MICROARRAY", INTERNATIONAL JOURNAL OF ONCOLOGY, SPANDIDOS PUBLICATIONS, GR, vol. 24, no. 2, 1 February 2004 (2004-02-01), pages 305-312, XP009027443, ISSN: 1019-6439

## Beschreibung

Die vorliegende Offenbarnung betrifft einen Microarray zur Prädiktion einer Lymphknotenmetastasierung bei kolorektalem Karzinom. Die Erfindung betrifft darüber ex vivo Verfahren zur Prädiktion einer Lymphknotenmetastasierung bei kolorektalem Karzinom Offenbart ist ferner ein. Inhibitor oder Modulator, der zur Behandlung des metastasierenden kolorektalen Karzinoms geeignet ist.

Beim kolorektalem Karzinom (KRK) ist die Lymphknotenmetastasierung (LKM) ein wesentlicher prognostischer Faktor. Aus diesem Grund werden bei allen Tumoren während eines chirurgischen Eingriffs die drainierenden Lymphknoten entfernt. Eine adjuvante Chemotherapie wird für Patienten mit LKM empfohlen. Eine valide präoperative, bildgebende Detektion von LKM, welche zu einer Individualisierung der Therapie führen könnte, ist derzeit nicht ausreichend möglich.

Das kolorektale Karzinom ist in Abhängigkeit vom Stadium heilbar. Die chirurgische Resektion stellt hierbei die entscheidende therapeutische Maßnahme dar. Die chirurgischen Radikalitätsprinzipien beinhalten neben der weiten Entfernung des Primärtumors das Konzept der elektiven en bloc Lymphknotendissektion bei nahezu allen kolorektalen Karzinomen (1, 2). Dieses Vorgehen basiert auf der Beobachtung, dass Patientenkollektive von der Entfernung des jeweiligen tumorassoziierten Lymphabstromgebiets und damit aller potentiell befallenen Lymphknoten profitieren. Nach kurativer Operation (R0) im Stadium III (UICC) konnten unter konsequenter Anwendung dieser Radikalitätsprinzipien die lokoregionären Rezidivraten beim Kolonkarzinom unter 5% und beim Rektumkarzinom unter 10% gesenkt, sowie das 5-Jahresüberleben auf 60,7% gesteigert werden (3).

Die konventionellen prä- und intraoperativen staging-Untersuchungen erlauben es nicht im Einzelfall synchrone oder metachrone Lymphknotenmetastasen nachzuweisen bzw. zu prognostizieren. Daher ist eine Unterscheidung der Stadien I / II (UICC) vom Stadium III nicht möglich, bevor die radikale Lymphknotendissektion mit histopathologischer Untersuchung des Resektates durchgeführt ist. Folglich gelten für nahezu alle Tumoren der klinischen Stadien I-III derzeit identische chirurgische Radikalitätsprinzipien.

Lediglich in einer Subgruppe des Stadiums I (UICC) mit kleinen (≤3 cm) polypösen pT1 Karzinomen ohne Lymphbahninvasion (L0) und geringem oder mäßigem Differenzierungsgrad (G1/2) kann anhand konventioneller Kriterien der Bildgebung und Histopathologie mit hinreichender Sicherheit eine lymphogene Metastasierung (≤3%) ausgeschlossen und eine alleinige lokale Resektion (z.B. endoskopische Polypektomie, transrektale Exzision) durchgeführt werden (7).

Ein sicherer präoperativer Ausschluß einer lymphogenen Metastasierung würde auch bei weiteren Tumoren im Stadium I und II (UICC) den Einsatz schonenderer Verfahren (z.B. endoskopische Verfahren, laparoskopische tubuläre Resektionen, kombinierte endoskopische und laparoskopische Verfahren, limiterte offene Resektionen) zulassen. Diese wenig traumatisierenden Eingriffe würden eine frühere Mobilisierung des Patienten ermöglichen und mit reduziertem Krankenhausaufenthalt, geringerem Schmerzmittelbedarf und weniger Komplikationen bei der Wundheilung einhergehen. Besonders für ältere und Hochrisikopatienten, bei denen die Folgen einer radikalen Resektion weniger kalkulierbar sind, hätte dies sehr große Vorteile.

Die derzeit präoperativ verfügbaren Prognosekriterien des Differenzierungsgrades, der Infiltrationstiefe und der Lymphbahninvasion reichen nicht aus, um eine zuverlässige präoperative Angabe über das Metastasierungspotential kolorektaler Karzinome zu treffen. Die Überexpression von MUC-1, ki67 oder MMP-7 in immunhistochemischen Analysen von Tumorgewebe lässt einen Zusammenhang mit einer Lymphknotenmetastasierung vermuten (8, 9, 10). Als unabhängige Prognosefaktoren konnten diese molekularen Marker allerdings bisher beim kolorektalen Karzinom nicht etabliert werden.

Mittels Microarray-Analyse konnten in mehreren Studien Gene identifiziert werden, die spezifisch in kolorektalen Karzinomen oder gesunder Dickdarmschleimhaut exprimiert werden und somit eine Differenzierung beider Gewebe erlauben (4, 5, 6).

Bisher liegen nur mangelhafte Studien vor, in denen versucht wurde ein prädiktives Genmuster für eine Metastasierung bei kolorektalen Karzinomen zu bestimmen. In der ersten Studie konnte über die Genexpression die Stadien UICC II und III nicht jedoch die Stadien UICC I und III unterschieden werden. Überdies wurden nur 20 Fälle, je 5 Patienten pro Stadium UICC I-IV eingeschlossen. Ein Patient litt an einer familiären Tumorerkrankung was für eine derartige Studie nicht akzeptabel ist, da hierbei die Genexpression beeinflusst werden kann. Es wurde eine frühe GeneChip Version der Fa. Affymetrix mit nur 6.800 ProbeSets verwendet (11). In einer zweiten Microarraystudie wurden lediglich 12 Patienten eigeschlossen. Hierbei wurde für die Analysen ein hierfür angefertigter cDNA Microarray mit 4.608 ProbeSets verwendet (12). In zwei weiteren Studien zur Tumorprogression wurden je 25 Fälle eingeschlossen. Hierbei wurden auch Fälle mit Lebermetastasen (Stadium UICC IV) und sogar Lebermetastasen selbst in die Analysen mit eingeschlossen. Auf eine Lymphknotenmetastasierung wurde nicht ausschließlich eingegangen (13-14). Aufgrund der Heterogenität des Probenmaterials sind die hierzu in diesen Arbeiten gelisteten Genmuster fragwürdig und zeigen keine Übereinstimmung mit dem eigenen Genmuster der vorliegenden Arbeit.

Es liegt nahe, dass Gene, die spezifisch in einem bestimmten Stadium des kolorektalen Karzinoms exprimiert werden, an der pathologischen Ausprägung des entsprechenden Stadiums beteiligt sein könnten. Bisher konnten jedoch die Pathofunktionen von Stadien assoziierten Genen, die mittels Mikroarray-Technologie isoliert wurden, nicht systematisch analysiert werden. Dies liegt daran, dass bei vergleichenden Untersuchungen der Genexpression mit der Mikroarray-Technologie im allgemeinen Gengruppen (Cluster) von Genen identifiziert werden, deren Expression mit einem bestimmten Tumorstadium assoziiert ist. Zudem müssen Kombinationseffekte dieser Gene bei pathogenetischen Untersuchungen berücksichtigt werden. Dies erklärt auch, weswegen einzelne Gene bisher nicht für prognostische Zwecke etabliert werden konnten. Es handelt sich um komplexe Genmuster für biologische Funktionen, wobei die Interaktionen der einzelnen Gene eine Lymphknotenmetastasierung verursachen.

Es ist deswegen eine Aufgabe der vorliegenden Offenbarung, einen Microarray zur Prädiktion einer Lymphknotenmetastasierung bei kolorektalem Karzinom und ein entsprechendes ex-vivo Verfahren bereitszustellen, die einen verbesserten prädiktiven Wert gegenüber den bisher bekannten Untersuchungsverfahren aufweisen. Es ist eine weitere Aufgabe der vorliegenden Offenbarung Substanzen bereitszustellen, die zur Behandlung des metastasierenden kolorektalen Karzinoms geeignet sind.

Diese Aufgaben werden durch den Gegenstand des unabhängigen Anspruchs gelöst. Bevorzugte Aspekte sind in den abhängigen Ansprüchen angegeben.

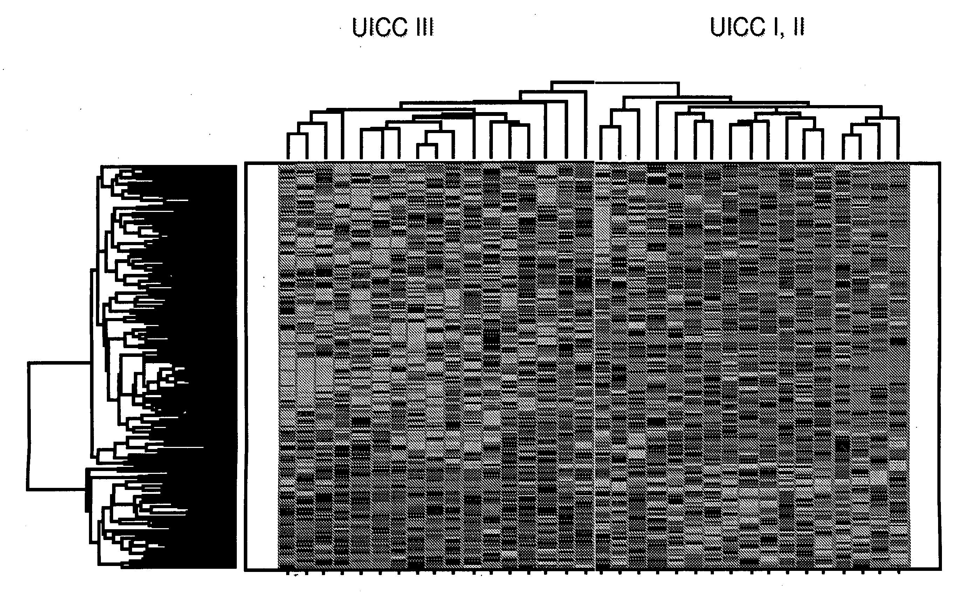

In der vorliegenden Erfindung konnte durch eine Microarrayanalyse von 40 kolorektalen Karzinomen ohne (Stadium-UICC I, II) und 40 Karzinomen mit Lymphknotenmetastasen (Stadium UICC III) ein prädiktives Genmuster von 242 Genen für eine Lymphknotenmetasasierung detektiert werden (Tabelle 1). Die sorgfältige Auswahl der Fälle und die exakte histologische Kontrolle der aufgearbeiteten Gewebe und RNA-Kontrolle gewähren eine entsprechende Datenqualität (15). Auf der Basis des vorliegenden Genmusters war die Prädiktion einer Lymphknotenmetastasierung von 67% möglich (Abbildung 1), wobei entsprechend der German Cancer Society Consensus Conference, Bochum, Germany, Februar 2004 die präoperative radiologische Diagnostik hier nur 60% ist (16). Bei dem untersuchten Kollektiv handelt es sich um die größte bisher verwendete bekannte Fallzahl mit einem Microarray, der ca. 22.280 ProbeSets (Affymetrix, HG-U133A) enthält. Das detektierte Genset kann z.B. nach Hybridisierung auf einen sogenannten Custom made Microarray für eine präoperative Diagnostik an der Tumorbiopsie verwendet werden.

Von je 40 Patienten mit KRK Stadium UICC I, II und UICC III wurde Tumorgewebe sofort postoperativ asserviert. Nach makroskopischer Dissektion zur Anreicherung von Karzinomgewebe wurde RNA isoliert und diese auf Microarrays (HG-U133A, Affymetrix) hybridisiert. PCR und immunhistochemische Untersuchungen wurden von ausgewählten signifikant differentiell exprimierten Genen durchgeführt.

Die Prädiktion für eine LKM beim KRK ist also an der Biopsie des Primärtumors anhand differentiell exprimierter Gene möglich. Dies ist die Basis für eine individuelle präoperative Diagnostik und Therapieplanung.

Bis auf DCC und die Mikrosatelliteninstabilität sind molekulare prognostische Marker für eine klinische Anwendung nicht etabliert (siehe Abschnitt Beispiele). Einzelne Marker sind nicht in der Lage, eine Lymphknotenmetastasierung am Primärtumor zu prädiktieren. Durch GenChip-Analysen konnte erstmals in einer hinreichend großen Stichprobe von 80 Fällen ein Muster von 242 prädiktiven Genen für eine Lymphknotenmetasasierung evaluiert werden (Tabelle 1). Auf dieser Basis kann in Zukunft eine Vorhersage für eine Lymphknotenmetastasierung anhand einer präoperativen Biopsie des Primärtumors erfolgen. Entscheidend ist hierbei die Zusammensetzung der Gene als spezifisches Muster welches die Lymphknotenmetastasierung prädiktiert (Figur 1).

Die vorliegende Offenbarung umfasst insbesondere folgende Aspekte und Ausführungsformen:

Gemäß eines ersten Aspektes betrifft die Offenbarung einen Microarray zur Prädiktion einer Lymphknotenmetastasierung bei kolorektalem Karzinom, wobei der Microarray Nukleinsäuren umfasst, die zur Hybridisierung an zumindest einen Teil der nachfolgenden Nukleinsäuren oder ihre komplementären oder reversen oder revers-komplementären Sequenzen sowie der hiervon abgeleiteten RNA-Sequenzen oder Derivaten dieser Sequenzen in der Lage sind, wobei die Nukleinsäuren zumindest die Nukleotidsequenzen nach Tabelle 2 umfassen.

Gemäß einer Ausführungsform der Offenbarung werden zusätzlich zu den oben genannten Nukleinsäuren nach Tabelle 2 eine oder mehrere der Nukleotidsequenzen nach Tabelle 1 im Microarray verwendet. Mit anderen Worten basiert der Microarray zumindest auf den Sequenzen nach Tabelle 2, kann jedoch zusätzlich auch auf den Sequenzen beruhen, die in Tabelle 1 angegeben sind. In einer bevorzugten Ausführungsform basiert der Microarray auf allen Sequenzen nach Tabelle 1 (d.h. auf allen 242 Sequenzen). Es sei darauf hingewiesen, daß Tabelle 1 alle Sequenzen nach Tabelle 2 enthält. Sollte der Microarray also auf mehr als den Sequenzen nach Tabelle 2 basieren, werden diese zusätzlichen Sequenzen selbstverständlich aus den Sequenzen der Tabelle 1 ausgewählt sind, die nicht bereits in Tabelle 2 enthalten sind.

Die einzelnen Sequenzen sowie ihre Zugangsdaten in Datenbanken sind in Tabelle 1 im Einzelnen dargelegt. Wie bereits oben angesprochen ist hierbei die Zusammensetzung der Gene als spezifisches Muster, welches die Lymphknotenmetastasierung prädiktiert, entscheidend. Aus Figur 1 läßt sich das differentielle Genmuster zwischen kolorektalen Karzinomen ohne (Stadium-UICC I, II) und mit (Stadium UICC III) Lymphknotenmetastasen erkennen. Diese Unterschiede in der Genexpression macht sich die vorliegende Erfindung zu Nutze, um ein diagnostisches Verfahren mit einem hohen prädiktiven Wert in Hinblick auf eine Prädiktion einer Lymphknotenmetastasierung bei kolorektalem Karzinom bereitzustellen.

Offenbarungemäß werden - wie oben angesprochen - insbesondere alle 242 Sequenzen aus Tabelle 1 herangezogen, es ist jedoch auch möglich den Microarray auf Untergruppen zu beschränken, die einen selbstständigen prädiktiven Wert haben.

Die Nukleinsäuren der vorliegenden Offenbarung umfassen auch Nukleinsäuren, die Sequenzen aufweisen, die im Wesentlichen zu den Nukleinsäuren der SEQ ID NO: 1-242 (Tabelle 1) äquivalent sind. Erfindungsgemäße Nukleinsäuren können z.B. zumindest ungefähr 80%, typischerweise zumindest ungefähr 90% oder 95% Sequenzidentität zu den Nukleinsäuren der SEQ ID NO: 1-242 aufweisen.

Der Begriff "Microarray" wie hierin verwendet ist eine Sammelbezeichnung für molekularbiologische Untersuchungssysteme, die die parallele Analyse von bis zu mehreren hundert Einzelnachweisen in einer geringen Menge biologischen Probenmaterials erlauben. Spezielle Microarrays werden manchmal auch als "Genchips" oder "Biochips" bezeichnet, da sie zahlreiche Informationen auf kleinstem Raum enthalten.

Die Microarrays werden in erster Linie in DNA-Microarrays und Protein-Microarrays unterschieden.

Microarrays dienen dazu, die RNA-Menge bestimmter Gene nachzuweisen. Es gibt hauptsächlich zwei verschiedene Arten von Microarrays, einerseits solche die auf gebundener cDNA beruhen und solche die auf synthetisch hergestellten Oligonukleotiden beruhen. Diese dienen als Sonden, die an definierte Positionen eines Rasters z.B. auf Glasträger aufgebracht werden.

Zur Analyse wird dabei in der Regel folgendes Vorgehen gewählt: RNA wird zunächst aus der Patientenprobe extrahiert und diese nach eventuellen Aufreinigungs- und/oder Vermehrungsschritten der mRNA in cDNA oder cRNA umgeschrieben und beispielsweise mit Markern versehen. Diese werden dann mit den Microarrays hybridisiert. Hierbei binden markierte cDNA/cRNA Stücke an ihren komplementären Gegenpart auf dem Array. Nach der Abwaschung der nicht gebundenen cDNA/cRNA Stücke wird das Signal des Markers in jeder Position des Microarrays mittels einer geeigneten Vorrichtung ausgelesen.

Im Kontext des Microarrays der vorliegenden Erfindung sind also die "Nukleinsäuren des Microarray" diejenigen Sequenzen, die Bestandteil des Microarray sind. Sie sind zu den Sequenzen der Nukleinsäuren der SEQ ID NO: 1-242 (die aus Patientenproben stammen) grundsätzlich komplementär und können beispielsweise nur aus einem kleineren Abschnitt dieser Sequenzen bestehen (beispielsweise im Falle von Oligonukleotidsonden). Insofern sind unter dem Begriff "Nukleinsäuren, die spezifisch zur Hybridisierung geeignet sind" solche an den Microarray gebundene Sequenzen zu verstehen, die spezifisch zumindest an einen Abschnitt einer entsprechenden Nukleinsäuresequenz nach SEQ ID NO: 1-242 binden.

Gemäß einer bevorzugten Ausführungsform sind die Nukleinsäuren von SEQ ID NO: 1 - 242 chemisch modifiziert, um die Ablesung/Auswertung der Bindung der entsprechenden Nukleinsäuren an die komplementären Nukleinsäuren des Microarray zu ermöglichen/zu erleichtern. Vorzugsweise kommt hier eine Markierung zum Einsatz, die besonders aus einer radioaktiven Markierung, Fluoreszenzmarkierung, Biotinmarkierung, Digoxigenin-markierung, Peroxidase-markierung oder eine durch alkalische Phosphatase nachweisbaren Markierung ausgewählt ist.

Die Nukleinsäuren des Microarray sind vorzugsweise an eine Festphasenmatrix, z.B. Nylonmembran, Glas oder Kunststoff, gebunden.

Der Microarray ist vorzugsweise ein DNA-, RNA- oder PNA-Array.

Die vorliegende Erfindung betrifft ein ex-vivo Verfahren zur Prädiktion einer Lymphknotenmetastasierung bei kolorektalem Karzinom, das die nachfolgenden Schritte umfasst:
- Gewinnen einer Probe des Tumorgewebes des kolorektalen Karzinoms;
- Extrahieren der RNA aus der Probe;
- Gegebenenfalls Umschreiben der RNA in cDNA oder cRNA;
- Nachweis, ob die Probe cDNA oder cRNA enthält, die zumindest den Nukleinsäuresequenzen nach Tabelle 1 oder 2 entspricht, wobei die differentielle Expression dieser cDNA oder cRNA für die Prädiktion einer Lymphknotenmetastasierung bei kolorektalem karzinom verwendet wird.

Der Nachweis der Nukleinsäuresequenzen erfolgt vorzugsweise durch PCR, insbesondere durch RT-PCR. Das PCR-Verfahren hat den Vorteil, daß sehr kleine DNA-Mengen nachweisbar sind. In Abhängigkeit von dem nachzuweisenden Material sind die Temperaturbedingungen und die Zykluszahlen der PCR abzuwandeln. Die optimalen Reaktionsbedingungen können durch Handversuche in an sich bekannter Weise ermittelt werden.

Die DNA oder RNA, insbesondere mRNA, der zu untersuchenden Probe kann sowohl bei der PCR-Reaktion bzw. RT-PCR als auch bei der Hybridisierungsreaktion entweder in extrahierter Form vorliegen oder in Form von komplexen Gemischen, bei denen die zu untersuchende DNA oder RNA nur einen sehr kleinen Anteil an der Fraktion der speziellen biologischen Probe bildet. Die zu untersuchenden Zellen können somit entweder in gereinigter Form vorliegen oder beispielsweise "verunreinigt" im Gewebeverband.

Bei der RT-PCR werden die Oligonukleotide der Erfindung zur PCR-Amplifikation von Fragmenten an cDNA Matrizen eingesetzt, die nach einer reversen Transkription von Proben-mRNA erzeugt werden. Die Expression kann dann qualitativ, in Verbindung mit geeigneten Standards und Techniken, wie einem internen Standard und einer quantitativen PCR, auch quantitativ ausgewertet werden.

Alternativ kann der Nachweis der Nukleinsäuren mittels komplementärer Nukleinsäuresonden, vorzugsweise cDNA- oder Oligonukleotid-Sonden, durchgeführt werden.

Diese Nukleinsäuresonden sind vorzugsweise zur Hybridisierung an zumindest einen Abschnitt jeder der Nukleinsäuren oder ihrer komplementären oder reversen oder revers-komplementären Sequenzen sowie der hiervon abgeleiteten RNA-Sequenzen oder Derivate dieser Sequenzen in der Lage.

Erfindungsgemäß erfolgt das Hybridisieren vorzugsweise unter moderat stringenten oder stringenten Bedingungen.

Unter stringenten Hybridisierungs- und Waschbedingungen versteht man im allgemeinen die Reaktionsbedingungen, unter denen nur noch Duplexmoleküle zwischen Oligonukleotiden und gewünschten Zielmolekülen (perfekte Hybride) entstehen bzw. nur noch der gewünschte Zielorganismus nachgewiesen wird. Unter stringenten Hybridisierungsbedingungen werden dabei insbesondere 0,2 x SSC (0,03 M NaCl, 0,003 M Natriumcitrat, pH 7) bei 65°C verstanden. Bei kürzeren Fragmenten, beispielsweise Oligonukleotiden aus bis zu 20 Nukleotiden, liegt die Hybridisierungstemperatur unter 65°C, beispielsweise bei über 55°C, bevorzugt über 60°C, jeweils aber unter 65°C. Stringente Hybridisierungstemperaturen sind abhängig von der Größe bzw. Länge der Nukleinsäure und ihrer Nukleotidzusanunensetzungen und sind vom Fachmann durch Handversuche zu ermitteln. Moderat stringente Bedingungen werden beispielsweise bei 42°C und Waschen in 0,2 x SSC/0,1 % SDS bei 42°C erreicht.

Wie bereits oben beschrieben existieren neben DNA-Arrays auch Proteinmicroarrays. Diese werden ebenfalls zur Prädiktion einer Lymphknotenmetastasierung bei kolorektalem Karzinom eingesetzt, wobei der Microarray dazu in der Lage ist, die den Nukleotidsequenzen von Tabelle 1 oder 2 entsprechenden Aminosäuresequenzen nachzuweisen.

Gemäß einer bevorzugten Ausführungsfrom handelt es sich um einen Antikörper-Microarray oder um einen Western-Blot-Microarray. Bei Antikörper-Microarrays werden beispielsweise die Antikörper fixiert (gespottet) und dann die Probe auf das Array aufgebracht. Der Begriff "Antikörper", wie er hierin verwendet wird betrifft intakte Antikörper ebenso wie Antikörperfragmente, die eine gewisse Fähigkeit beibehalten, selektiv an ein Epitop zu binden. Derartige Fragmente schließen ohne Einschränkung Fab, F(ab')₂, rekombinante "single chain" Antikörper und Fv Antikörperfragmente ein. Der Begriff "Epitop" betrifft jede Antigendeterminante auf einem Antigen, an die das Paratop eines Antikörpers bindet. Epitop-Determinanten bestehen üblicherweise aus chemisch aktiven Oberflächengruppen von Molekülen (beispielsweise Aminosäure- oder Zuckerreste) und weisen üblicherweise dreidimensionale strukturelle Eigenschaften ebenso wie spezielle Ladungseigenschaften auf.

Die Antikörper können unter Verwendung irgend eines bekannten Verfahrens hergestellt werden. Beispielsweise kann eine durch eine erfindungsgemäße Nukleinsäure exprimierte Aminosäure oder ein Bruchstück hiervon bereit gestellt und als Immunogen verwendet werden, um in einem Tier eine solche Immunreaktion hervorzurufen, dass spezifische Antikörper erzeugt werden.

Die Herstellung polyklonaler Antikörper ist dem Fachmann gut bekannt. Siehe beispielsweise Green et al, Production of Polyclonal Antisera, in Immunochemical Protocols (Manson, Herausgeber), Seiten 1 - 5 (Humana Press 1992) und Coligan et al, Production of Polyclonal Antisera in Rabbits, Rats, Mice and Hamsters, in Current Protocols In Immunology, Abschnitt 2.4.1 (1992). Zusätzlich sind dem Fachmann verschiedene Techniken der Immunologie zur Aufreinigung und Konzentration von polyklonalen Antikörpern bekannt, ebenso wie von monoklonalen Antikörpern (Coligan et al, Unit 9, Current Protocols in Immunology, Wiley Interscience, 1994).

Die Herstellung von monoklonalen Antikörpern ist dem Fachmann ebenfalls vertraut. Siehe beispielsweise Köhler & Milstein, Nature 256: 495 (1975); Coligan et al., Abschnitte 2.5.1 - 2.6.7; und Harlow et al., Antibodies: A Laboratory Manual, Seite 726 (Cold Spring Harbor Pub. 1988). Kurz gesagt können monoklonale Antikörper gewonnen werden, indem Mäusen eine Zusammensetzung die die erfindungsgemäßen Aminosäuren einschließt injiziert wird, danach das Vorliegen einer Antikörperproduktion durch Untersuchung einer Serumprobe verifiziert wird, die Milz zur Gewinnung von B-Lymphozyten entfernt und die B-Lymphozyten mit Myelomazellen zur Erzeugung von Hybridomas fusioniert werden, die Hybridomas geklont werden, die positiven Klone, die einen monoklonalen Antikörper gegen das Protein erzeugen, selektiert werden und die Antikörper aus den Hybridoma-Kulturen isoliert werden. Monoklonale Antikörper können aus Hybridoma-Kulturen durch eine Vielzahl von wohl etablierten Techniken isoliert und aufgereinigt werden. Derartige Isolierungstechniken schließen eine Affinitätschromatographie mit Protein-A oder G-Sepharose, Größenausschlusschromatographie und Ionenaustauschchromatographie ein. Siehe beispielsweise Coligan et al., Abschnitte 2.7.1 - 2.7.12 und Abschnitt "Immunglobulin G (IgG)", in Methods In Molecular Biology, Band 10, Seiten 79 - 104 (Humana Press 1992).

Die Microarray Western Methode dient zum Nachweis von Antigenen in Zelllysaten verschiedener Gewebe oder in durch isoelektrische Fokussierung gewonnenen Proteinfraktionen. Das Zelllysat oder die Proteinfraktion wird auf dem Trägermaterial des Microarrays gespottet, danach wird der Antikörper aufgebracht. In jedem Testfeld mit Antikörper-Antigen Interaktion bleibt der Antikörper haften. Felder mit Antikörper werden dann wie beim Western Blot detektiert.

Gemäß eines weiteren Aspektes betrifft die Offenbarung einen Inhibitor oder Modulator einer oder mehrerer Sequenzen nach Tabelle 1 oder 2 oder der hieraus exprimierten Proteine. Solche Substanzen werden zur Behandlung von kolorektalem Karzinom eingesetzt.

Die Offenbarung umfasst auch eine pharmazeutische Zusammensetzung, die einen oder mehrere der oben genannten Inhibitoren oder Modulatoren und einen pharmazeutisch verträglichen Träger umfasst. Eine derartige Zusammensetzung kann (zusätzlich zu den Wirkstoffen und dem Träger) Verdünnungsmittel, Füllmaterialien, Salze, Puffer, Stabilisatoren, Solubilisierungsmittel und andere Materialien enthalten, die in der Technik wohlbekannt sind. Der Begriff "pharmazeutisch verträglich" soll ein nichttoxisches Material definieren, das die Wirksamkeit der biologischen Aktivität des aktiven Inhaltsstoffes bzw. Wirkstoffes nicht stört. Die Auswahl des Trägers hängt vom Verabreichungsweg ab.

Die pharmazeutische Zusammensetzung kann zusätzlich weitere Mittel enthalten, die die Aktivität des Wirkstoffes steigern oder dessen Aktivität oder Verwendung bei der Behandlung ergänzen (beispielsweise Chemotherapeutika). Derartige zusätzliche Faktoren und/oder Mittel können in der pharmazeutischen Zusammensetzung enthalten sein, um eine synergistische Wirkung zu erzielen oder um Nebenwirkungen bzw. unerwünschte Wirkungen zu minimieren. Techniken zur Formulierung bzw. Zubereitung und Verabreichung der Inhibitoren/Modulatoren der vorliegenden Anmeldung sind in "Remington's Pharmaceutical Sciences", Mack Publishing Co., Easton, PA, letzte Ausgabe, zu finden. Eine therapeutisch wirksame Dosis bezieht sich ferner auf eine Menge der Verbindung, die ausreicht, um eine Verbesserung der Symptome zu erreichen, beispielsweise eine Behandlung, Heilung, Prävention oder Verbesserung derartiger Zustände. Geeignete Verabreichungswege können beispielsweise orale, rektale, transmucosale oder intestinale Verabreichung und parenterale Verabreichung einschließen, einschließlich intramuskulärer, subkutaner, intramedulärer Injektionen ebenso wie intrathekaler, direkt intraventrikulärer, intravenöser, intraperitonealer oder intranasaler Injektionen. Die intravenöse Verabreichung an einen Patienten wird bevorzugt. Die vorliegende Offenbarung wird nunmehr durch die folgenden Beispiele sowie die Figur veranschaulicht.

Figur 1 zeigt ein differentielles Genmuster zwischen kolorektalen Karzinomen ohne (Stadium-UICC I, II) und mit (Stadium UICC III) Lymphknotenmetastasen. Es handelt sich um eine Headmap zur Veranschaulichung differentiell exprimierter Gene Stadium UICC I, II vs. III. In den Zeilen finden sich differentiell exprimierte Gene, rot: überexprimiert, grün: unterexprimiert. In den Spalten finden sich die Fälle der entsprechenden Gruppen UICC I, II und III. Deutlich kommt ein Muster differentiell exprimierter Gene UICC I, II vs. III zur Darstellung.

### Beispiele: Die folgende Übersicht zeigt Prognosefaktoren beim kolorektale Karzinom:

**Prognosefaktoren beim kolorektalen Karzinom (College of American Pathologists Consenus Statement 1999)**

| Kategorie I statistisch nachgewiesene, eindeutige prognostische Relevanz | Kategorie IIA (nachweislich prognostische Relevanz, klinische Validierung steht aus) | Kategorie IIB (aussichtreiche Faktoren, Daten noch nicht ausreichend) | Kategorie III (Datenlage unklar/ Studien noch unzureichend) | Kategorie IV (nachweislich keine prognostische Relevanz) |
|---|---|---|---|---|
| pT-Kategorie | Grading | histologischer Karzinomtyp | morphologische Marker: | Tumorgröße |
| pN-Kategorie | | | - perineurale Invasion | |
| (AJCC/UICC) | | | - Kapillardichte | |
| | | | - peritumorale Fibrose (Desmoplasie) | |
| | | | - peritumorale, inflammatorische Reaktion | |
| | | | - fokale neuroendokrine Differenzierung | |
| V-Kategorie (Veneninvasion) | Resektatränder | MSI-assoziierte Faktoren, MSI-H | alle molekularen Marker* außer DCC und MSI-H, makroskopische Tumorkonfiguration | |
| L-Kategorie (Lymphgefäßinvasion) | | | | |
| R-Klassifikation (Residualtumor) | ypTNM | LOH 18q (DCC-Allelverlust) | alle Tumormarker* (Zellproteine u. Kohlenhydrate) außer CEA | |
| CEA präoperativ | | Tumorrandkonfiguration | nukleäre Organisationsregionen Proliferationsmarker/ index: Ki-67, DNA-Gehalt | |

| | | | | |
|---|---|---|---|---|
| MSI: Mikrosatelliteninstäbilität CEA: karzinoembryonales Antigen ypTNM: y: Klassifikation nach Radio-/Chemotherapie; p: pathologisches Staging * Auflistung der Marker siehe Literatur | | | | |

Daneben zeigt Figur 1, wie oben angegeben, ein differentielles Genmuster zwischen kolorektalen Karzinomen ohne (Stadium-UICC I, II) und mit (Stadium UICC III) Lymphknotenmetastasen, rot: überexprimierte Gene, grün: unterexprimierte Gene.

In einer Studie wurde mittels der Affymetrix GenChip Technologie die Genexpression 40 kolorektaler Karzinome ohne (Stadium UICC I und II) und von 40 kolorektalen Karzinomen mit Lymphknotenmetastasen (Stadium UICC III) verglichen. Hierbei konnten 242 signifikant unterschiedliche expremierte Gene UICC I, II vs. UICCC III detektiert werden (siehe Tabelle 1). Auf der Basis dieser differentiell expremierten Gene konnte durch verschiedene statistische Modelle (Croner et.al, Cancer 2005) eine Prädiktion für eine Lymphknotenmetastasierung von ca. 67% erstellt werden. Bisher ist eine präoperative Aussage für eine Lymphknotenmetastasierung nur durch bildgebende Diagnostik möglich. Hierbei kann eine realistische Prädiktion von ca. 60% getroffen werden (German Cancer Society Consensus Conference, Bochum, Germany, Februar 2004). Einzelne molekulare Marker konnten bisher nicht als prognostisch relevante Marker etabliert werden (Figur 1).

Mit dem vorliegenden Genmuster können sogenannte "Custom made Microarrays" (Lymphchip) hergestellt und für eine präoperative Diagnostik eingesetzt werden. Kolorektale Karzinome können dann präoperativ biopsiert werden. Die isolierte RNA aus den Tumorbiopsien wird auf den Lymphchip hybridisiert. Je nach Genexpressionsmuster kann eine Aussage über eine Lymphknotenmetastasierung getroffen werden. Auf der Basis dieser Information kann schließlich eine individuelle Therapie bezüglich Operationsplanung oder Chemotherapie erfolgen.

**Tabelle 1: 242 differentiell exprimierte Gene zwischen kolorektalen Karzinomen ohne (Stadium-UICC I. II) und mit (Stadium UICC III) Lymphknotenmetastasen. Die Sequenzen entsprechen SEQ ID NO: 1-242. * basierend auf multiplem Testen über alle 22.286 ProbeSets GenChips (Affymetrix HG-U133A).**

| Zugangs nummer | Bezeichnung | P Wert |
|---|---|---|
| Extrazelluläre Matrix | | |
| NM_021721 | a disintegrin and metalloproteinase domain 22 (ADAM22) | <0.001 |
| NM_024333 | fibronectin type III and SPRY domain containing 1 (FSD1) | <0.001 |
| NM_003613 | cartilage intermediate layer protein, nucleotide pyrophosphohydrolase (CILP) | <0.05 |
| NM_003635 | N-deacetylase/N-sulfotransferase (heparan glucosaminyl) 2 (NDST2) | <0.05 |
| Zellzyklus/Mitose | | |
| NM_000465 | BRCA1 associated RING domain 1 (BARD1) | <0.01 |
| X59065 | fibroblast growth factor 1 (acidic) (FGF1) | <0.01 |
| NM_018055 | nodal homolog (mouse) (NODAL) | <0.01 |
| NM_007370 | replication factor C (activator 1) 5, 36.5kDa (RFC5) | <0.01 |
| U79302 | tumor protein p53 inducible protein 11 (TP53I11) | <0.05 |
| NM_022094 | cell death-inducing DFFA-like effector c (CIDEC) | <0.05 |
| BF967271 | anaphase promoting complex subunit 5 (ANAPC5) | <0.05 |
| NM_002634 | prohibitin (PHB) | <0.05 |
| AI367319 | SRY (sex determining region Y)-box 10 (SOX10) | <0.05 |
| NM_018165 | polybromo 1 (PB 1) | <0.05 |
| AF074717 | RAD1 homolog (S. pombe) (RAD1) | <0.05 |
| NM_016567 | BRCA2 and CDKN1A interacting protein (BCCIP) | <0.05 |
| NM_004708 | programmed cell death 5 (PDCD5) | <0.05 |
| AF277724 | cell division cycle 25C (CDC25C) | <0.05 |
| AI042030 | SMC1 structural maintenance of chromosomes 1-like 1 (yeast) (SMCIL1) | <0.05 |
| AL161955 | triple functional domain (PTPRF interacting) (TRIO) | <0.05 |
| NM_006999 | polymerase (DNA directed) sigma (POLS) | <0.05 |
| NM_001786 | cell division cycle 2, G1 to S and G2 to M (CDC2) | <0.05 |
| NM_002898 | RNA binding motif, single stranded interacting protein 2 (RBMS2) | <0.05 |
| | | |
| Zelladhäsion/Zytoskelett | | |
| NM_006262 | peripherin (PRPH) | <0.001 |
| AF312679 | Rhesus blood group, D antigen (RHD) | <0.001 |
| M_001794 | cadherin 4, type 1, R-cadherin (retinal) (CDH4) | <0.01 |
| NM_030968 | C1q and tumor necrosis factor related protein 1 (C1QTNF1) | <0.01 |
| NM_012216 | midline 2 (MID2) | <0.01 |
| M_020129 | lectin, galactoside-binding, soluble, 14 (LGALS 14) | <0.01 |
| NM_001939 | dystrophin related protein 2 (DRP2) | <0.01 |
| AK001619 | phosphodiesterase 4D interacting protein (myomegalin) (PDE4DIP) | <0.01 |
| AW296788 | microtubule-associated protein 1A (MAP1A) | <0.01 |
| AF152503 | protocadherin gamma subfamily A, 10 (PCDH-gamma-A10) | <0.05 |
| NM_001232 | calsequestrin 2 (cardiac muscle) (CASQ2) | <0.05 |
| NM_000337 | sarcoglycan, delta (35kDa dystrophin-associated glycoprotein) (SGCD) | <0.05 |
| M_001944 | desmoglein 3 (pemphigus vulgaris antigen) (DSG3) | <0.05 |
| L20815 | corneodesmosin (CDSN) | <0.05 |
| NM_024883 | R-cadherin (retinal) (CDH4) | <0.015* |
| NM_006580 | claudin 16 (CLDN16) | <0.015* |
| | | |
| Kanäle/Transporter | | |
| NM_020243 | translocase of outer mitochondrial membrane 22 homolog (yeast) (TOMM22) | <0.01 |
| N80922 | solute carrier family 35, member D1 (SLC35D1) | <0.05 |
| AF087138 | ATP-binding cassette, sub-family C (CFTR/MRP), member 8 (ABCC8) | <0.05 |
| NM_005895 | golgi autoantigen, golgin subfamily a, 3 (GOLGA3) | <0.05 |
| AW086154 | golgi associated, gamma adaptin ear containing, ARF binding protein 2 (GGA2) | <0.05 |
| U72069. | transportin 1 (TNPO1) | <0.05 |
| NM_024844 | pericentrin 1 (PCNT1) | <0.05 |
| AF047338 | solute carrier family 12, member 4 (SLC12A4) | <0.015* |
| AB046836 | transient receptor potential cation channel (TRPM3) | <0.015* |
| NM_007357 | component of oligomeric golgi complex 2 (COG2) | <0.015* |
| | | |
| Signal-transduction | | |
| AA393484 | phospholipase C, beta 1 (phosphoinositide-specific) (PLCB1) | <0.001 |
| AY024365 | integrin-linked kinase-associated serine/threonine phosphatase 2C (ILKAP) | <0.001 |
| BE466525 | ecotropic viral integration site 1 (EVI1) | <0.001 |
| M 15887 | diazepam binding inhibitor (DBI) | <0.001 |
| NM_030959 | olfactory receptor, family 12, subfamily D, member 3 (OR12D3) | <0.001 |
| NM_000541 | S-antigen, retina and pineal gland (arrestin) (SAG) | <0.01 |
| NM_014286 | frequenin homolog (Drosophila) (FREQ) | <0.01 |
| NM_016151 | TAO kinase 2 (TAOK2) | <0.01 |
| AF095723 | G protein-coupled receptor 51 (GPR51) | <0.01 |
| AF015731 | glutamate receptor, ionotropic, N-methyl D-aspartate 1 (GRIN1) | <0.01 |
| AC004794 | olfactory receptor, family 1, subfamily I, member 1 (OR1I1) | <0.01 |
| AW138374 | Ras homolog enriched in brain (RHEB) | <0.01 |
| NM_002082 | G protein-coupled receptor kinase 6 (GRK6) | <0.01 |
| NM_016087 | wingless-type MMTV integration site family, member 16 (WNT16) | <0.05 |
| AI218134 | wingless-type MMTV integration site family, member 6 (WNT6) | <0.05 |
| AL050219 | immunoglobulin superfamily, member 4B (IGSF4B) | <0.05 |
| NM_003027 | SH3-domain GRB2-like 3 (SH3GL3) | <0.05 |
| M37981 | cholinergic receptor, nicotinic, alpha polypeptide 3 (CHRNA3) | <0.05 |
| AF229166 | docking protein 1, 62kDa (downstream of tyrosine kinase 1) (DOK1) | <0.05 |
| NM_020548 | diazepam binding inhibitor (DBI) | <0.05 |
| AA459867 | olfactory receptor, family 7, subfamily E, member 156 pseudogene (OR7E156P) | <0.05 |
| AB000277 | discs, large (Drosophila) homolog-associated protein 1 (DLGAP1) | <0.05 |
| NM_021783 | ectodysplasin A2 receptor (EDA2R) | <0.05 |
| NM_012348 | olfactory receptor, family 2, subfamily N, member 1 pseudogene (OR2N1P) | <0.05 |
| NM_022644 | chorionic somatomammotropin hormone 2 (CSH2) | <0.05 |
| M_016340 | Rap guanine nucleotide exchange factor (GEF) 6 (RAPGEF6) | <0.05 |
| NM_005754 | Ras-GTPase-activating protein SH3-domain-binding protein (G3BP) | <0.05 |
| AW192876 | casein kinase 1, epsilon (CSNK1E) | <0.05 |
| NM_000529 | melanocortin 2 receptor (adrenocorticotropic hormone) (MC2R) | <0.05 |
| AK022494 | RAB3 GTPase-activating protein (RAB3GAP) | <0.05 |
| NM_003696 | olfactory receptor, family 6, subfamily A, member 2 (OR6A2) | <0.05 |
| NM_005374 | membrane protein, palmitoylated 2 (MPP2) | <0.015* |
| NM_001883 | corticotropin releasing hormone receptor 2 (CRHR2) | <0.015* |
| NM_022559 | growth hormone 1 (GH1) | <0.015* |
| M95489 | follicle stimulating hormone receptor (FSHR) | <0.015* |
| | | |
| Metabolismus | | |
| NM_014222 | NADH dehydrogenase (ubiquinone) 1 alpha subcomplex, 8, 19kDa (NDUFA8) | <0.001 |
| AI252582 | ATPase, H+ transporting, lysosomal 9kDa, V0 subunit (ATP6V0E) | <0.001 |
| M29873 | cytochrome P450, family 2, subfamily B, polypeptide 7 pseudogene 1 (CYP2B7P1) | <0.001 |
| NM_000284 | pyruvate dehydrogenase (lipoamide) alpha 1 (PDHA1) | <0.01 |
| AF000381 | folate receptor 1 (adult) (FOLR1) | <0.01 |
| AW024233 | glycine-N-acyltransferase (GLYAT) | <0.01 |
| M33318 | cytochrome P450, family 2, subfamily A, polypeptide 6 (CYP2A6) | <0.01 |
| Z97630 | glycine C-acetyltransferase (2-amino-3-ketobutyrate coenzyme A ligase) (GCAT) | <0.01 |
| NM_001740 | calbindin 2, 29kDa (calretinin) (CALB2) | <0.01 |
| D13119 | ATP synthase, H+ transporting, mitochondrial F0 complex, subunit c (ATP5G2) | <0.05 |
| AA669797 | fumarate hydratase (FH), bone marrow | <0.05 |
| NM_000143 | fumarate hydratase (FH), mitochondrial | <0.05 |
| BC005270 | NADH dehydrogenase (ubiquinone) Fe-S protein 4, 18kDa (NDUFS4) | <0.05 |
| AB018272 | ubiquitin specific protease 34 (USP34) | <0.05 |
| J04178 | hexosaminidase A (alpha polypeptide) (HEXA) | <0.05 |
| NM_005917 | malate dehydrogenase 1, NAD (soluble) (MDH1) | <0.05 |
| NM_001190 | branched chain aminotransferase 2, mitochondrial (BCAT2) | <0.05 |
| AY028632 | catalase (CAT) | <0.05 |
| NM_003000 | succinate dehydrogenase complex, subunit B, iron sulfur (Ip) (SDHB) | <0.05 |
| NM_001869 | Carboxypeptidase A2 (pancreatic) (CPA2) | <0.05 |
| NM_004547 | NADH dehydrogenase (ubiquinone) 1 beta subcomplex, 4, 15kDa (NDUFB4) | <0.05 |
| NM_000281 | 6-pyruvoyl-tetrahydropterin synthase (PCBD) | <0.05 |
| N71116 | phospholipase A2, group IVB (cytosolic) (PLA2G4B) | <0.05 |
| NM_000818 | glutamate decarboxylase 2 (pancreatic islets and brain, 65kDa) (GAD2) | <0.05 |
| AF2801 10 | cytochrome P450, family 3, subfamily A, polypeptide 43 (CYP3A43) | <0.05 |
| BC006229 | cytochrome c oxidase subunit Vb (COX5B) | <0.05 |
| M64992 | proteasome (prosome, macropain) subunit, alpha type, 1 (PSMA1) | <0.05 |
| NM_000055 | butyrylcholinesterase (BCHE) | <0.015* |
| | | |
| Verschiedenes | | |
| NM_014814 | proteasome regulatory particle subunit p44S10 (PSMD6) | <0.001 |
| NM_001189 | bagpipe homeobox homolog 1 (Drosophila) (BAPX1) | <0.001 |
| NM_006839 | inner membrane protein, mitochondrial (mitofilin) (IMMT) | <0.001 |
| AI278616 | SET translocation (myeloid leukemia-associated) (SET) | <0.01 |
| NM_003171 | suppressor of varl, 3-like 1 (S. cerevisiae) (SUPV3L1) | <0.01 |
| NM_006604 | ret finger protein-like 3 (RFPL3) | <0.01 |
| NM_004960 | fusion (involved in t(12, 16) in malignant liposarcoma) (FUS) | <0.01 |
| AK026273 | FK506 binding protein 1B (FKBP1B) | <0.01 |
| NM_007011 | abhydrolase domain containing 2 (ABHD2) | <0.01 |
| BC006466 | diazepam binding inhibitor (DBI) | <0.01 |
| M_019009 | toll interacting protein (TOLLIP) | <0.05 |
| NM_021922 | Fanconi anemia, complementation group E (FANCE) ' | <0.05 |
| AL581473 | exosome component 7 (EXOSC7) | <0.05 |
| NM_012123 | mitochondrial translation optimization 1 homolog (S. cerevisiae) (MTO1) | <0.05 |
| BC003170 | ubiquitin associated protein 2-like (UBAP2L) | <0.05 |
| AF073483 | basophilic leukemia expressed protein BLES03 (Bles03) | <0.05 |
| U13395 | WW domain containing oxidoreductase (WWOX) | <0.05 |
| X55503 | metallothionein IV (MT4) | <0.05 |
| AK024944 | mediator of RNA polymerase II transcription, subunit 28 homolog (MED28) | <0.05 |
| AF263541 | dual-specificity tyrosine-(Y)-phosphorylation regulated kinase 4 (DYRK4) | <0.05 |
| U67122 | SMT3 suppressor of mif two 3 homolog 1 (yeast) (SUMO1) | <0.05 |
| M_018234 | dudulin 2 (TSAP6) | <0.05 |
| NM_003905 | amyloid beta precursor protein binding protein 1, 59kDa (APPBP1) | <0.05 |
| AK024527 | FLJ20874 fis, clone ADKA02818 | <0.05 |
| AF168617 | sperm associated antigen 11 (SPAG11) | <0.015* |
| BC006333 | tripartite motif-containing 14 (TRIM14) | <0.015* |
| NM_007009 | zona pellucida binding protein (ZPBP) | <0.015* |
| NM_014420 | dickkopf (Xenopus laevis) homolog 4 (DKK4) | <0.01 |
| X98568 | Type X collagen gene | <0.01 |
| BC000340 | melanoma antigen, family A, 3, clone MGC:8564 | <0.01 |
| NM_005940 | matrix metalloproteinase 11 (stromelysin 3) (MMP11) | <0.01 |
| NM_013307 | non-functional folate binding protein (HSAF000381) | <0.01 |
| NM_018678 | lipopolysaccharide specific response-68 protein (LSR68) | <0.01 |
| AF036973 | HLA complex group 4 pseudogene 6 (HCG4P6) | <0.015* |
| | | |
| Funktion unbekannt | | |
| H61826 | Tularik gene 1, NIK and IKK binding protein (NIBP) | <0.001 |
| BF223370 | hypothetical protein MGC11332 (MGC11332) | <0.01 |
| N64803 | trinucleotide repeat containing 6B (TNRC6B) | <0.01 |
| NM_016458 | chromosome 8 open reading frame 30A (LOC51236) | <0.01 |
| BF055496 | DKFZP586J0619 protein (DKFZP586J0619) | <0.01 |
| NM_004939 | DEAD (Asp-Glu-Ala-Asp) box polypeptide 1 (DDX1) | <0.01 |
| AK001854 | KIAA0931 protein (PHLPPL) | <0.01 |
| AL137303 | KIAA1055 protein (KIAA1055) | <0.01 |
| AW014299 | hypothetical protein MGC13098 (MGC13098) | <0.01 |
| AW025284 | hypothetical protein LOC222070 (LOC222070) | <0.01 |
| AK025343 | hepatocellular carcinoma-related HCRP1 (HCRP1) | <0.01 |
| AB023170 | KIAA0953 (KIAA0953) | <0.05 |
| AK027146 | hypothetical LOC388650 (LOC388650) | <0.05 |
| AK025833 | CD33 antigen-like 3 (CD33L3) | <0.05 |
| NM_018253 | YY1 associated protein (YAP) | <0.05 |
| AI431902 | hypothetical protein FLJ13491 (FLJ13491) | <0.05 |
| AK022688 | KIAA0863 protein (KIAA0863) | <0.05 |
| AK026465 | FLJ22812 fis (FLJ22812) | <0.05 |
| NM_017700 | hypothetical protein FLJ20184 | <0.05 |
| NM_024519 | hypothetical protein FLJ 13725 | <0.05 |
| NM_024035 | hypothetical protein MGC3113 | <0.05 |
| U72513 | RPL13-2 pseudogene (LOC283345) | <0.05 |
| BF436315 | Nedd4 binding protein 1 (N4BP1) | <0.05 |
| AL031228 | DNA segment on chromosome 6 (D6S2723E) | <0.05 |
| NM_018053 | hypothetical protein FLJ10307 (FLJ10307) | <0.05 |
| AF334946 | FKSG44 gene (FKSG44) | <0.05 |
| NM_022371 | torsin family 3, member A (TOR3A) | <0.05 |
| NM_004209 | synaptogyrin 3 (SYNGR3) | <0.05 |
| AF052091 | BTB (POZ) domain containing 9 (BTBD9) | <0.05 |
| AK000834 | FLJ20827 fis (FLJ20827) | <0.05 |
| AL022101 | hypothetical protein LOC343070 (LOC343070) | <0.05 |
| U79458 | WW domain binding protein 2 (WBP2) | <0.05 |
| BC005078 | hypothetical protein FLJ10996 (FLJ10996) | <0.05 |
| NM_025125 | chromosome 10 open reading frame 57 (C10orf57) | <0.05 |
| AF225421 | HT017 protein (HT017) | <0.05 |
| AL050370 | hypothetical protein FLJ33790 (FLJ33790) | <0.05 |
| AB051441 | KIAA1654 protein (KIAA1654) | <0.05 |
| AB051447 | KIAA1660 protein (KIAA1660) | <0.05 |
| NM_024037 | hypothetical protein MGC2603 (MGC2603) | <0.05 |
| AL024509 | 60S Ribosomal Protein L21 pseudogene (HNRPA3) | <0.05 |
| X72882 | KIAA0409 protein (KIAA0409) | <0.05 |
| AL137435 | hypothetical gene supported by AL137435 (LOC142779) | <0.05 |
| AW003733 | Ras homolog gene family, member D (RHOD) | <0.05 |
| AL137475 | DKFZp434F0723 protein (DKFZp434F0723) | <0.05 |
| NM_025094 | hypothetical protein FLJ22184 (FLJ22184) | <0.05 |
| NM_025052 | Yeast Sps1/Ste20-related kinase 4 (YSK4) | <0.05 |
| NM_006010 | arginine-rich, mutated in early stage tumors (ARMET) | <0.05 |
| AK025127 | SATB family member 2 (SATB2) | <0.05 |
| AK024527 | FLJ20874 fis, clone ADKA02818 (AK024527) | <0.05 |
| NM_014118 | Glycosyltransferase-like domain containing 1 (GTDC1) | <0.05 |
| BC000978 | FLJ20259 (FLJ20259) | <0.015* |
| N55205 | hemoglobin, beta pseudogene 1 (HBBP1) | <0.015* |
| NM_024748 | FLJ11539 (FLJ11539) | <0.015* |
| AF288389 | glycosyltransferase 25 domain containing 2 (GLT25D2) | <0.015* |
| AL121891 | U-box domain containing 5 (UBOX5) | <0.015* |
| AF070610 | clone 24505 (BEAN) | <0.015* |
| D85939 | craniofacial development protein 1 (CFDP1) | <0.015* |
| D38081 | thromboxane A2 receptor (TBXA2R) | <0.015* |
| AI263044 | qz29e03.x1 NCI_CGAP_Kid11 cDNA clone | <0.015* |
| NM_014772 | KIAA0427 (KIAA0427) | <0.015* |
| U85995 | parathyroid hormone-responsive B1 (PTHB1) | <0.015* |
| AW975818 | YTH domain containing 2 (YTHDC2) | <0.015* |
| NM_024593 | EF-hand calcium binding domain 1 (EFCAB1) | <0.015* |
| BF968960 | TM2 domain containing 1 (TM2D1) | <0,015* |
| AF151022 | HSPC 188 | <0.015* |
| | | |
| Protein-synthese | | |
| AF279891 | DEAH (Asp-Glu-Ala-His) box polypeptide 15 (DHX15) | <0.001 |
| D21851 | leucyl-tRNA synthetase 2, mitochondrial (LARS2) | <0.001 |
| NM_002372 | mannosidase, alpha, class 2A, member 1 (MAN2A1) | <0.01 |
| NM_007362 | nuclear cap binding protein subunit 2, 20kDa (NCBP2) | <0.01 |
| NM_022163 | mitochondrial ribosomal protein L46 (MRPL46) | <0.05 |
| AW118072 | signal recognition particle receptor, B subunit (SRPRB) | <0.05 |
| NM_005826 | Heterogeneous nuclear ribonucleoprotein R (HNRPR) | <0.05 |
| BF195526 | Heterogeneous nuclear ribonucleoprotein A3 (HNRPA3P1) | <0.05 |
| BF033354 | splicing factor, arginine/serine-rich 7, 35kDa (SFRS7) | <0.05 |
| NM_001029 | ribosomal protein S26 (RPS26) | <0.05 |
| NM_001357 | DEAH (Asp-Glu-Ala-His) box polypeptide 9 (DHX9) | <0.05 |
| NM_020191 | mitochondrial ribosomal protein S22 (MRPS22) | <0.05 |
| NM_013417 | isoleucine-tRNA synthetase (LARS) | <0.05 |
| U37689 | polymerase (RNA) II (DNA directed) polypeptide H (POLR2H) | <0.05 |
| | | |
| Genkontrolle | | |
| NM_012317 | leucine zipper, down-regulated in cancer 1 (LDOC1) | <0.001 |
| NM_002967 | scaffold attachment factor B (SAFB) | <0.01 |
| Nu_004821 | heart and neural crest derivatives expressed 1 (BARD1) | <0.01 |
| NM_006365 | Transcriptional activator of the c-fos promoter (CROC4) | <0.01 |
| BC002818 | synovial sarcoma, X breakpoint (SSX2) | <0.01 |
| M_014910 | zinc finger protein 507 (ZNF507) | <0.01 |
| AL080144 | ELYS transcription factor-like protein TMBS62 (ELYS) | <0.05 |
| M92439 | leucine-rich PPR-motif containing (LRPPRC) | <0.05 |
| NM_006963 | zinc finger protein 22 (KOX 15) (ZNF22) | <0.05 |
| NM_003927 | methyl-CpG binding domain protein 2 (MBD2) | <0.05 |
| BF530257 | Transcriptional activator of the c-fos promoter (CROC4) | <0.05 |
| M_001189 | bagpipe homeobox homolog 1 (Drosophila) (BAPX1) | <0.015* |
| BC001205 | SIN3 homolog B, transcription regulator (yeast) (SIN3B) | <0.015* |
| | | |
| Immunsystem | | |
| AF047245 | immunoglobulin lambda locus (IGL) | <0.01 |
| NM_020393 | peptidoglycan recognition protein 4 (PGLYRP4) | <0.01 |
| X95660 | immunoglobulin heavy constant mu (IGHM) | <0.05 |
| AI659611 | inducible T-cell co-stiniulator ligand (ICOSL) | <0.05 |

**Tabelle 2: eine Untergruppe aus 50 Nukleinsäuren, die einen hohen prädiktiven Wert bei der Prädiktion einer Lymphknotenmetastasierung bei kolorektalem Karzinom erzielten. p<0.015 basierend auf multiplem Testen über alle 22.286 ProbeSets GenChips (Affymetrix HG-U133A).**

| *Zugangsnummer* | *Bezeichnung* |
|---|---|
| Zelladhäsion/Zytoskelett | |
| NM_021721 | ADAM metallopeptidase domain 22 (ADAM22) |
| NM_006262 | peripherin (PRPH) |
| NM_024883 | R-cadherin (retinal) (CDH4) |
| NM_006580 | claudin 16 (CLDN16) |
| | |
| Transporter/Kanäle | |
| N80922 | Solute carrier family 35, member D1 (SLC35D1) |
| AI252582 | ATPase, H+ transporting, lysosomal 9kDa, V0 subunit (ATP6V0E) |
| AF047338 | Solute carrier family 12, member 4 (SLC12A4) |
| AB046836 | transient receptor potential cation channel, subfamily M, member 3 (TRPM3) |
| NM_007357 | component of oligomeric golgi complex 2 (COG2) |
| | |
| Signaltransduktion | |
| H61826 | NIK and IKK{beta} binding protein (NIBP) |
| NM_030959 | olfactory receptor, family 12, subfamily D, member 3 (OR12D3) |
| BE466525 | ecotropic viral integration site 1 (EVI1) |
| NM_016087 | wingless-type MMTV integration site family, member 16 (WNT16) |
| M95489 | follicle stimulating hormone receptor (FSHR) |
| NM_005374 | membrane protein, palmitoylated 2 (MAGUK p55 subfamily member 2) (MPP2) |
| M_001883 | corticotropin releasing hormone receptor 2 (CRHR2) |
| NM_022559 | growth hormone 1 (GH1) |
| D38081 | thromboxane A2 receptor (TBXA2R) |
| | |
| Metabolismus | |
| M_014222 | NADH dehydrogenase (ubiquinone) 1 alpha subcomplex, 8, 19kDa (NDUFA8) |
| AW024233 | glycine-N-acyltransferase (GLYAT) |
| NM_014814 | proteasome (prosome, macropain) 26S subunit, non-ATPase, 6 (PSMD6) |
| NM_000055 | butyrylcholinesterase (BCHE) |
| | |
| Proteinsynthese | |
| AF279891 | DEAH (Asp-Glu-Ala-His) box polypeptide 15 (DHX15) |
| D21851 | leucyl-tRNA synthetase 2, mitochondrial (LARS2) |
| | |
| Genkontrolle | |
| NM_001189 | bagpipe homeobox homolog 1 (Drosophila) (BAPX1) |
| BC001205 | SIN3 homolog B, transcription regulator (yeast) (SIN3B) |
| | |
| Immunsystem | |
| NM_020393 | peptidoglycan recognition protein 4 (PGLYRP4) |
| | |
| Verschiedenes | |
| AF168617 | Sperm associated antigen 11 (SPAG11) |
| BC006333 | tripartite motif-containing 14 (TRIM14) |
| NM_007009 | zona pellucida binding protein (ZPBP) |
| M29873 | cytochrome P450, family 2, subfamily B, polypeptide 7 pseudogene 1 (CYP2B7P1) |
| | |
| N55205 | hemoglobin, beta pseudogene 1 (HBBP1) |
| AF036973 | HLA complex group 4 pseudogene 6 (HCG4P6) |
| | |
| Funktion unbekannt | |
| N64803 | trinucleotide repeat containing 6B (TNRC6B) |
| NM_016458 | chromosome 8 open reading frame 30A (LOC51236) |
| AF151022 | HSPC 188 |
| NM_006604 | ret finger protein-like 3 (RFPL3) |
| NM_024333 | fibronectin type III and SPRY domain containing 1 (FSD1) |
| BC000978 | FLJ20259 (FLJ20259) |
| NM_024748 | FLJ11539 (FLJ115359) |
| AF288389 | glycosyltransferase 25 domain containing 2 (GLT25D2) |
| AL121891 | U-box domain containing 5 (UBOX5) |
| AF070610 | Clone 24505 (BEAN) |
| D85939 | craniofacial development protein 1 (CFDP1) |
| AI263044 | qz29e03.x1 NCI_CGAP_Kid11 cDNA clone |
| BF968960 | TM2 domain containing 1 (TM2D1) |
| NM_014772 | KIAA0427 (KIAA0427) |
| U85995 | parathyroid hormone-responsive B 1 (PTHB 1) |
| AW975818 | YTH domain containing 2 (YTHDC2) |
| NM_024593 | EF-hand calcium binding domain 1 (EFCAB1) |

Im Folgenden ist eine Auflistung der Sequenzen der in Tabelle 1 und 2 aufgelisteten Sequenzen zu finden. Alle Sequenzen sind humanen Ursprungs (Homo sapiens).
- LOCUS: NM_021721 2636 bp mRNA linear PRI 17-NOV-2006
- DEFINITION: Homo sapiens ADAM metallopeptidase domain 22 (ADAM22), transcript variant 5, mRNA.
- ACCESSION: NM_021721
- VERSION: NM_021721.2 GI:21536384

1 gcggagcgag ggaaacggac tcggcggcgc cggcatgagg agctgagcgt ctcgggcgag
61 gcgggctgac ggcagcacca tgcaggcggc agtggctgtg tccgtgccct tcttgctgct
121 ctgtgtcctg gggacctgcc ctccggcgcg ctgcggccag gcaggagacg cctcattgat
181 ggagctagag aagaggaagg aaaaccgctt cgtggagcgc cagagcatcg tgccactgcg
241 cctcatctac cgctcgggcg gcgaagacga aagtcggcac gacgcgctcg acacgcgggt
301 gcggggcgac ctcggtggcc cgcagttgac tcatgttgac caagcaagct tccaggttga
361 tgcctttgga acgtcattca ttctcgatgt cgtgctaaat catgatttgc tgtcctctga
421 atacatagag agacacattg aacatggagg caagactgtg gaagttaaag gaggagagca
481 ctgttactac cagggccata tccgaggaaa ccctgactca tttgttgcat tgtcaacatg
541 ccacggactt catgggatgt tctatgacgg gaaccacaca tatctcattg agccagaaga
601 aaatgacact actcaagagg atttccattt tcattcagtt tacaaatcca gactgtttga
661 attttccttg gatgatcttc catctgaatt tcagcaagta aacattactc catcaaatt
721 tattttgaag ccaagaccaa aaaggagtaa acggcagctt cgtcgatatc ctcgtaatgt
781 agaagaagaa accaaataca ttgaactgat gattgtgaat gatcacctta tgtttaaaaa
841 acatcggctt tccgttgtac ataccaatac ctatgcgaaa tctgtggtga acatggcaga
901 tttaatatat aaagaccaac ttaagaccag gatagtattg gttgctatgg aaacctgggc
961 gactgacaac aagtttgcca tatctgaaaa tccattgatc accctacgtg agtttatgaa
1021 atacaggagg gattttatca aagagaaaag tgatgcagtt cacctttttt cgggaagtca
1081 atttgagagt agccggagcg gggcagctta tattggtggg atttgctcgt tgctgaaagg
1141 aggaggcgtg aatgaatttg ggaaaactga tttaatggct gttacacttg cccagtcatt
1201 agcccataat attggtatta tctcagacaa aagaaagtta gcaagtggtg aatgtaaatg
1261 cgaggacacg tggtccgggt gcataatggg agacactggc tattatcttc ctaaaaagtt
1321 cacccagtgt aatattgaag agtatcatga cttcctgaat agtggaggtg gtgcctgcct
1381 tttcaacaaa ccttctaagc ttcttgatcc tcctgagtgt ggcaatggct tcattgaaac
1441 tggagaggag tgtgattgtg gaaccccggc cgaatgtgtc cttgaaggag cagagtgttg
1501 taagaaatgc accttgactc aagactctca atgcagtgac ggtctttgct gtaaaaagtg
1561 caagtttcag cctatgggca ctgtgtgccg agaagcagta aatgattgtg atattcgtga
1621 aacgtgctca ggaaattcaa gccagtgtgc ccctaatatt cataaaatgg atggatattc
1681 atgtgatggt gttcagggaa tttgctttgg aggaagatgc aaaaccagag atagacaatg
1741 caaatacatt tgggggcaaa aggtgacagc atcagacaaa tattgctatg agaaactgaa
1801 tattgaaggg acggagaagg gtaactgtgg gaaagacaaa gacacatgga tacagtgcaa
1861 caaacggatg tgctttgtgg gttacctttt gtgtaccaat attggcaata tcccaaggct
1921 tggagaactc gatggtgaaa tcacatctac tttagttgtg cagcaaggaa gaacattaaa
1981 ctgcagtggt gggcatgtta agcttgaaga agatgtagat cttggctatg tggaagatgg
2041 gacaccttgt ggtccccaaa tgatgtgctt agaacacagg tgtcttcctg tggcttcttt
2101 caactttagt acttgcttga gcagtaaaga aggcactatt tgctcaggaa atggagtttg
2161 cagtaatgag ctgaagtgtg tgtgtaacag acactggata ggttctgatt gcaacactta
2221 cttccctcac aatgatgatg caaagactgg tatcactctg tctggcaatg gtgttgctgg
2281 caccaatatc ataataggca taattgctgg caccatttta gtgctggccc tcatattagg
2341 aataactgcg tggggttata aaaactatcg agaacagagg tcaaatgggc tctctcattc

2401 ttggagtgaa aggattccag acacaaaaca tatttcagac atctgtgaaa atgggcgacc
2461 tcgaagtaac tcttggcaag gtaacctggg aggcaacaaa aagaaaatca gaggcaaaag
2521 atttagacct cggtctaatt caactgagta agtctgaacc tctaatggaa aaaggcactc
2581 caaggtcctg tgcaaatagt gtcaaaagta aattaagcag atagtcaaag aactgt//

- LOCUS: NM_024333 1760 bp mRNA linear PRI 17-NOV-2006
- DEFINITION: Homo sapiens fibronectin type III and SPRY domain containing 1 (FSD1), mRNA.
- ACCESSION: NM_024333 XM_940073
- VERSION: NM_024333.1 GI:13236584

1 acccagcgtg cgcggggccc gcgggccggg ccggggtgac ctgggctgca gccatggaag
61 aacagaggga ggccctgagg aagatcatca aaacactggc tgtgaagaat gaagaaattc
121 agagctttat ctactccctg aaacagatgc tgctgaacgt ggaggcgaac tcggcgaagg
181 tgcaggagga cctcgaagca gagttccagt ccctcttctc cctcctggag gagctgaaag
241 aaggcatgct tatgaagata aaacaggacc gtgccagccg cacctacgag ctgcagaacc
301 agctggctgc ctgcacgcgg gccctggaga gctccgagga gcttctggag acagccaacc
361 agactctgca ggccatggac agcgaggact ttcctcaggc tgccaagcaa atcaaagatg
421 gagtgaccat ggcccctgcc ttccggctat cattgaaagc gaaggtcagt gacaacatga
481 gtcacctcat ggtggacttc gcgcaagagc ggcagatgct acaggcactc aagttcctgc
541 ctgtgcccag cgcacccgtg atcgacctgg ctgagtccct ggtggcagat aactgtgtga
601 ccctggtgtg gcgcatgccg gatgaggaca gcaagattga ccactacgtg ctggagtacc
661 ggcggaccaa cttcgagggc ccgccccgcc tcaaggagga ccagccctgg atggtcatcg
721 agggcatccg gcagacagag tacaccctga caggtctcaa gtttgacatg aaatacatga
781 acttccgtgt gaaggcctgt aacaaggcag ttgcaggaga gttctctgag ccggtgactc
841 tggagacacc agcgttcatg ttccgcctgg atgcgtccac atcccaccag aacctgcggg
901 tggatgatct ctccgtggag tgggacgcta tgggcgggaa ggtgcaggat atcaaggctc
961 gcgagaaaga tggcaagggg cggacggcgt ctcccatcaa ctccccagcc agaggtactc
1021 catctcccaa gaggatgccc tcaggtcgtg ggggacggga ccgcttcacc gctgagtcct
1081 acacagttct gggggacacg ctgatcgacg gcggggagca ttactgggag gtgcgctacg
1141 agccggacag caaggcgttc ggcgtgggcg tggcctaccg cagcctgggc cgcttcgagc
1201 aactgggcaa gacggccgcc tcctggtgcc tgcacgtcaa caattggctg caggtcagct
1261 tcacggccaa gcacgccaac aaggtcaagg tgctggacgc ccccgtgccc gactgcctgg
1321 gtgtgcactg tgacttccac caaggcctcc tgtccttcta caatgcccgc accaaacaag
1381 tgctgcacac tttcaagacc aggttcacac agccgctgct gcctgctttc acggtatggt
1441 gtggcagctt ccaggtgacg acaggcctgc aggtccccag tgctgtgcgc tgcctgcaaa
1501 agcgaggcag tgctaccagc agctccaaca ccagcctcac ctaggccccc aggcacccac
1561 ccagctgggg tgtttttggg ggagtcgccg ccaagcccag gctgctggag ccaggcaccc
1621 tcctctgtca cttgctgctt ggagccttaa ctccagatgg gggggtcacc aagagggagt
1681 gggcaccctg gcgggccctc tccccacctc acctcttaat aaaggtcaga cactggccag
1741 gcaaaaaaaa aaaaaaaaaa//

- LOCUS: NM_003613 4436 bp mRNA linear PRI 18-NOV-2006
- DEFINITION: Homo sapiens cartilage intermediate layer protein, nucleotide pyrophosphohydrolase (CILP), mRNA.
- ACCESSION: NM_003613
- VERSION: NM_003613.2 GI:51944961

1 aggcgaaggc ttggaccccc agctgaggag tcctgctcaa gacacggtca ctggatctga
61 gaaacttccc aggggaccgc attccagagt cagtgactct gtgaagcacc cacatctacc
121 tcttgccacg ttcccacggg cttgggggaa agatggtggg gaccaaggcc tgggtgttct
181 ccttcctggt cctggaagtc acatctgtgt tggggagaca gacgatgctc acccagtcag
241 taagaagagt ccagcctggg aagaagaacc ccagcatctt tgccaagcct gccgacaccc
301 tggagagccc tggtgagtgg acaacatggt tcaacatcga ctacccaggc gggaagggcg
361 actatgagcg gctggacgcc attcgcttct actatgggga ccgtgtatgt gcccgtcccc
421 tgcggctaga ggctcggacc actgactgga cacctgcggg cagcactggc caggtggtcc
481 atggtagtcc ccgtgagggt ttctggtgcc tcaacaggga gcagcggcct ggccagaact
541 gctctaatta caccgtacgc ttcctctgcc caccaggatc cctgcgccga gacacagagc
601 gcatctggag cccatggtct ccctggagca agtgctcagc tgcctgtggt cagactgggg
661 tccagactcg cacacgcatt tgcttggcag agatggtgtc gctgtgcagt gaggccagcg
721 aagagggtca gcactgcatg ggccaggact gtacagcctg tgacctgacc tgcccaatgg
781 gccaggtgaa tgctgactgt gatgcctgca tgtgccagga cttcatgctt catggggctg
841 tctcccttcc cggaggtgcc ccagcctcag gggctgctat ctacctcctg accaagacgc
901 cgaagctgct gacccagaca gacagtgatg ggagattccg aatccctggc ttgtgccctg

961 atggcaaaag catcctgaag atcacaaagg tcaagtttgc ccccattgta ctcacaatgc
1021 ccaagactag cctgaaggca gccaccatca aggcagagtt tgtgagggca gagactccat
1081 acatggtgat gaaccctgag acaaaagcac ggagagctgg gcagagcgtg tctctgtgct
1141 gtaaggccac agggaagccc aggccagaca agtatttttg gtatcataat gacacattgc
1201 tggatccttc cctctacaag catgagagca agctggtgct gaggaaactg cagcagcacc
1261 aggctgggga gtacttttgc aaggcccaga gtgatgctgg ggctgtgaag tccaaggttg
1321 cccagctgat tgtcatagca tctgatgaga ctccttgcaa cccagttcct gagagctatc
1381 ttatccggct gccccatgat tgctttcaga atgccaccaa ctccttctac tatgacgtgg
1441 gacgctgccc tgttaagact tgtgcagggc agcaggataa tgggatcagg tgccgtgatg
1501 ctgtgcagaa ctgctgtggc atctccaaga cagaggaaag ggagatccag tgcagtggct
1561 acacgctacc caccaaggtg gccaaggagt gcagctgcca gcggtgtacg gaaactcgga
1621 gcatcgtgcg gggccgtgtc agtgctgctg acaatgggga gcccatgcgc tttggccatg
1681 tgtacatggg gaacagccgt gtaagcatga ctggctacaa gggcactttc accctccatg
1741 tcccccagga cactgagagg ctggtgctca catttgtgga caggctgcag aagtttgtca
1801 acaccaccaa agtgctacct ttcaacaaga aggggagtgc cgtgttccat gaaatcaaga
1861 tgcttcgtcg gaaagagccc atcactttgg aagccatgga gaccaacatc atccccctgg
1921 gggaagtggt tggtgaagac cccatggctg aactggagat tccatccagg agtttctaca
1981 ggcagaatgg ggagccctac ataggaaaag tgaaggccag tgtgaccttc ctggatcccc
2041 ggaatatttc cacagccaca gctgcccaga ctgacctgaa cttcatcaat gacgaaggag
2101 acactttccc ccttcggacg tatggcatgt tctctgtgga cttcagagat gaggtcacct
2161 cagagccact taatgctggc aaagtgaagg tccaccttga ctcgacccag gtcaagatgc
2221 cagagcacat atccacagtg aaactctggt cactcaatcc agacacaggg ctgtgggagg
2281 aggaaggtga tttcaaattt gaaaatcaaa ggaggaacaa aagagaagac agaaccttcc
2341 tggtgggcaa cctggagatt cgtgagagga ggctctttaa cctggatgtt cctgaaagca
2401 ggcggtgctt tgttaaggtg agggcctacc ggagtgagag gttcttgcct agtgagcaga
2461 tccagggggt tgtgatctcc gtgattaacc tggagcctag aactggcttc ttgtccaacc
2521 ctagggcctg gggccgcttt gacagtgtca tcacaggccc caacggggcc tgtgtgcctg
2581 ccttctgtga tgaccagtcc cctgatgcct actctgccta tgtcttggca agcctggctg
2641 gggaggaact gcaagcagtg gagtcttctc ctaaattcaa cccaaatgca attggcgtcc
2701 ctcagcccta tctcaacaag ctcaactacc gtcggacgga ccatgaggat ccacgggtta
2761 aaaagacagc tttccagatt agcatggcca agccaaggcc caactcagct gaggagagca
2821 atgggcccat ctatgccttt gagaacctcc gggcatgtga agaggcacca cccagtgcag
2881 cccacttccg gttctaccag attgaggggg atcgatatga ctacaacaca gtccccttca
2941 acgaagatga ccctatgagc tggactgaag actatctggc atggtggcca aagccgatgg
3001 aattcagggc ctgctatatc aaggtgaaga ttgtggggcc actggaagtg aatgtgcgat
3061 cccgcaacat ggggggcact catcggcgga cagtggggaa gctgtatgga atccgagatg
3121 tgaggagcac tcgggacagg gaccagccca atgtctcagc tgcctgtctg gagttcaagt
3181 gcagtgggat gctctatgat caggaccgtg tggaccgcac cctggtgaag gtcatccccc
3241 agggcagctg ccgtcgagcc agtgtgaacc ccatgctgca tgagtacctg gtcaaccact
3301 tgccacttgc agtcaacaac gacaccagtg agtacaccat gctggcaccc ttggacccac
3361 tgggccacaa ctatggcatc tacactgtca ctgaccagga ccctcgcacg gccaaggaga
3421 tcgcgctcgg ccggtgcttt gatggcacat ccgatggctc ctccagaatc atgaagagca
3481 atgtgggagt agccctcacc ttcaactgtg tagagaggca agtaggccgc cagagtgcct
3541 tccagtacct ccaaagcacc ccagcccagt cccctgctgc aggcactgtc caaggaagag
3601 tgccctcgag gaggcagcag cgagcgagca ggggtggcca gcgccagggt ggagtggtgg
3661 cctctctgag atttcctaga gttgctcaac agcccctgat caactaagtt ttgtggtact
3721 tcaccctctt ctgccctcat ttcatgtgac agccattgtg agactgatgc acaaactgtc
3781 acttggttaa tttaagcact tctgttttcg tgaatttgct tgtttgtttc ttcatgcctt
3841 tacttacttt gtcccatgct actgattggc acgtggcccc cacaatggca caataaagcc
3901 cctttgtgaa actgttcttt aaatgaaaca caagaaattg gccactggta aaactctgca
3961 gcttcaactg tacttcattt aatgccatta atgcaaatat acttcctctt ctttttgcat
4021 ggttttgccc acctctgcaa tagtgataat ctgatgctga agatcaaata accaatataa
4081 agcatatttc ttggccttgc tccacaggac ataggcaagc cttgatcata gttcatacat
4141 ataaatggtg gtgaaataaa gaaataaaac acaatacttt tacttgaatg taaataactt
4201 atttatttct ttgctaaatt tggaattcta gtgcacattc aaagttaagc tattaaatat
4261 agggtgatca tagttcctct accaagtctg gaaagaacat ctcctggtat ccacaattac
4321 accaggttgc taactgtatt tgtacatttc cctttgcatt cgcttttgtt cttgctagaa
4381 acccagtgta gcccagggca gatgtcaata aatgcatact ctgtatttcg aaaaaa//

- LOCUS: NM_003635 3778 bp mRNA linear PRI 17-NOV-2006
- DEFINITION: Homo sapiens N-deacetylase/N-sulfotransferase (heparan glucosaminyl) 2 (NDST2), mRNA.
- ACCESSION: NM_003635
- VERSION: NM_003635.2 GI:31377809

1 gagatggcgg agcagccgcg acgcggcccg tggccccgaa agcagggaag ccgggcagcc
61 ccgggacgcg gcggagcccg gggacagcgg ggacgccgcc cagtcctccg ggtatgaagc
121 taaaatgcag cggctaagaa cgcggctctg gagcttgaca ggcctgacgt cgaatcccag
181 ccctcccacc aactgagctg tgtgatcctg ggcaaggtag aggggaagag attgaacttt
241 gctgaccttt gatgtgaggc gctcagccag ggccaagggg agagcctggc aagatttgca
301 gcctgaagcc atgggccagg gggccatggt gacctgagac aagtggactc tgtatagttg
361 ccccctgctt ccccttctac ctcccctacc ctatgctaag gggactcgtc tccacctcgt
421 aaaggaaact ccccaaggga atccctgtcc cctattttcc tatccttcta cccttccaag
481 acagtcctag cctatagaac tcctacctcc catcccctga ggtggtcccc attcctccct
541 cccttcctcc ccccgccatg ctccagttgt ggaaggtggt acgcccagct cggcagctgg
601 aactgcaccg cctcatactg ctgctgatcg ctttcagcct gggctccatg ggcttcctgg
661 cttattatgt gtccaccagc cctaaggcca aggaaccctt gcccctgccc ttgggagact
721 gcagcagcgg tggggcagct ggtcctggcc ctgcacggcc tccagttcca cctcggcccc
781 ccaggcctcc agagacagct cgaactgaac ccgtggtcct tgtgtttgtg gagagtgcat
841 actcacagct ggggcaggaa attgtggcca tcctggagtc tagtcgtttt cgttatagca
901 ctgagttggc acctggccga ggggacatgc ccacattgac tgataatacc catggccgct
961 atgtcttggt catttatgag aacctgctca agtatgtcaa cctggatgcc tggagtcggg
1021 aactgctaga ccggtactgc gtggagtatg gtgtgggcat cattggcttt ttccgagccc
1081 acgagcacag cctactgagc gcccagctca aggctttccc ccttttttta cactcaaact
1141 tggggctccg ggactaccaa gtgaatcctt ctgccccgct actgcatctc acacgcccca
1201 gccgcctaga accaggccca ctgcctggtg atgactggac catcttccaa tccaatcata
1261 gtacatatga accagtgctt cttgccagcc ttcggccagc tgagcccgca gtgccaggac
1321 cagttcttcg tcgggcccgg cttcccactg tggtacagga cctggggctt catgatggca
1381 tccagcgggt gctctttgga catggccttt ccttctggct ccacaaactt atcttcgttg
1441 atgctgttgc atacctcact ggcaagcgcc tctgcctgga ccttgaccgc tacatcttgg
1501 tagacatcga tgacatcttt gtgggcaagg aagggacccg catgaaggtg gctgatgttg
1561 aggctctgtt gaccacccag aacaaactca ggaccttagt tcccaacttc accttcaact
1621 tgggcttctc gggcaagttc tatcatactg ggacagagga ggaggatgca ggggacgaca
1681 tgctgctgaa gcaccgcaaa gagttctggt ggttccccca catgtggagc cacatgcagc
1741 cacacctgtt ccacaatcgc tccgtgctgg ctgaccagat gaggctcaac aaacagtttg
1801 ctctggagca tgggattccc acggacctgg ggtatgctgt ggccccccac cactcgggtg
1861 tgtaccccat ccacacgcag ctctatgagg cctggaaatc cgtgtggggc atccaggtga
1921 ccagcactga ggagtatccc catctccgcc ctgcccgcta ccgccgtggc ttcattcaca
1981 atggcattat ggtgctgccc cggcagacat gtggcctctt cactcacaca atcttctata
2041 atgagtatcc tggaggctct cgtgaactag accggagcat ccgaggtgga gagctctttc
2101 tgacagtgct gcttaatccg atcagcatct ttatgaccca tctgtccaat tatggaaatg
2161 accggctggg cctatacacc tttgagagct tggtgcgctt cctccagtgt tggacacggc
2221 tgcgcctaca gacccttcct cctgtcccac ttgcacagaa gtactttgaa cttttccctc
2281 aggagcgaag ccccctttgg cagaatccct gtgatgacaa gaggcacaaa gatatctggt
2341 ccaaggagaa aacctgtgat cgtctcccga agttcctcat tgtgggaccc cagaaaacag
2401 ggactacagc tattcacttc ttcctgagcc tgcacccagc tgtaactagc agcttcccta
2461 gccccagcac atttgaggag attcagttct tcaacagccc taattaccac aagggtattg
2521 actggtacat ggatttcttc cctgttcctt ccaatgccag cactgatttc ctatttgaaa
2581 aaagtgccac ctactttgac tctgaagttg taccacggcg gggggctgcc ctcctgccac
2641 gagccaagat catcacagtg ctcaccaacc ctgctgacag ggcctactcc tggtaccagc
2701 atcagcgagc ccatggagac ccagttgctc tgaactatac cttctatcag gtgatttcag
2761 cctcctccca gacccctctg gcactacgct ccctgcagaa ccgctgtctt gtccctggct
2821 actattctac ccatctacaa cgctggctga cttactaccc ctctggacag ttgctgattg
2881 tggatgggca agagctgcgt accaacccag cagcctcaat ggagagcatc cagaagttcc
2941 tgggtatcac accctttctg aactacacac ggaccctcag gtttgatgat gataagggat
3001 tttggtgcca gggacttgaa ggtggtaaga ctcgctgtct aggccggagc aaaggccgga
3061 ggtatccaga tatggacact gagtcccgtc ttttccttac ggattttttc cggaaccata
3121 atttggagtt gtcgaagctg ctgagccggc ttggacagcc agtgccctcg tggcttcggg
3181 aagaactgca gcattccagt ctgggctgat gtcccagcct cccataccag caaaatgccc
3241 cctgcttccc taaggggcag gtccagagca gggcccacaa gggggattag agtggcctgg
3301 cccctccccc tctacctcag tagcccccag gcctgagatg gctgagaagg gaagggtatc
3361 cttttcccac agttctggga caaataaagg ggcttccttt ggtaccccac ataatagtgc
3421 taggtacctt tgacccatca tcttgggagg tggggaggaa tgagagggtc caggcagggt
3481 gtaggggaat gtattagtcc aatgagattt ccctcttcat ccgcagcagt gtatctattc
3541 tatacctggc tatgggagag accccttgca tgggagggac cccttgctat ggcccctta
3601 gccaggcagt gggatctacc tgtggcccgg cctccctaat gtcattcaca ttgaatgggg
3661 atgaggtcgg acagtggctc atagagccga gtatgagccc tagctgtggg ctagaaatgt

3721 ccttaataaa catccttatt tttctgttta aaaaaaaaaa aaaaaaaaaa aaaaaaaa//

- LOCUS: NM_000465 2530 bp mRNA linear PRI 21-JAN-2007
- DEFINITION: Homo sapiens BRCA1 associated RING domain 1 (BARD1), mRNA.
- ACCESSION: NM_000465
- VERSION: NM_000465.1 GI:4557348

1 cagcttccct gtggtttccc gaggcttcct tgcttcccgc tctgcgagga gcctttcatc
61 cgaaggcggg acgatgccgg ataatcggca gccgaggaac cggcagccga ggatccgctc
121 cgggaacgag cctcgttccg cgcccgccat ggaaccggat ggtcgcggtg cctgggccca
181 cagtcgcgcc gcgctcgacc gcctggagaa gctgctgcgc tgctcgcgtt gtactaacat
241 tctgagagag cctgtgtgtt taggaggatg tgagcacatc ttctgtagta attgtgtaag
301 tgactgcatt ggaactggat gtccagtgtg ttacaccccg gcctggatac aagacttgaa
361 gataaataga caactggaca gcatgattca actttgtagt aagcttcgaa atttgctaca
421 tgacaatgag ctgtcagatt tgaaagaaga taaacctagg aaaagtttgt ttaatgatgc
481 aggaaacaag aagaattcaa ttaaaatgtg gtttagccct cgaagtaaga aagtcagata
541 tgttgtgagt aaagcttcag tgcaaaccca gcctgcaata aaaaaagatg caagtgctca
601 gcaagactca tatgaatttg tttccccaag tcctcctgca gatgtttctg agagggctaa
661 aaaggcttct gcaagatctg gaaaaaagca aaaaaagaaa actttagctg aaatcaacca
721 aaaatggaat ttagaggcag aaaaagaaga tggtgaattt gactccaaag aggaatctaa
781 gcaaaagctg gtatccttct gtagccaacc atctgttatc tccagtcctc agataaatgg
841 tgaaatagac ttactagcaa gtggctcctt gacagaatct gaatgttttg gaagtttaac
901 tgaagtctct ttaccattgg ctgagcaaat agagtctcca gacactaaga gcaggaatga
961 agtagtgact cctgagaagg tctgcaaaaa ttatcttaca tctaagaaat ctttgccatt
1021 agaaaataat ggaaaacgtg gccatcacaa tagactttcc agtcccattt ctaagagatg
1081 tagaaccagc attctgagca ccagtggaga ttttgttaag caaaccgtgc cctcagaaaa
1141 tataccattg cctgaatgtt cttcaccacc ttcatgcaaa cgtaaagttg gtggtacatc
1201 agggaggaaa aacagtaaca tgtccgatga attcattagt ctttcaccag gtacaccacc
1261 ttctacatta agtagttcaa gttacaggca agtgatgtct agtccctcag caatgaagct
1321 gttgcccaat atggctgtga aaagaaatca tagaggagag actttgctcc atattgcttc
1381 tattaagggc gacatacctt ctgttgaata ccttttacaa aatggaagtg atccaaatgt
1441 taaagaccat gctggatgga caccattgca tgaagcttgc aatcatgggc acctgaaggt
1501 agtggaatta ttgctccagc ataaggcatt ggtgaacacc accgggtatc aaaatgactc
1561 accacttcac gatgcagcca agaatgggca cgtggatata gtcaagctgt tactttccta
1621 tggagcctcc agaaatgctg ttaatatatt tggtctgcgg cctgtcgatt atacagatga
1681 tgaaagtatg aaatcgctat tgctgctacc agagaagaat gaatcatcct cagctagcca
1741 ctgctcagta atgaacactg ggcagcgtag ggatggacct cttgtactta taggcagtgg
1801 gctgtcttca gaacaacaga aaatgctcag tgagcttgca gtaattctta aggctaaaaa
1861 atatactgag tttgacagta cagtaactca tgttgttgtt cctggtgatg cagttcaaag
1921 taccttgaag tgtatgcttg ggattctcaa tggatgctgg attctaaaat ttgaatgggt
1981 aaaagcatgt ctacgaagaa aagtatgtga acaggaagaa aagtatgaaa ttcctgaagg
2041 tccacgcaga agcaggctca acagagaaca gctgttgcca aagctgtttg atggatgcta
2101 cttctatttg tggggaacct tcaaacacca tccaaaggac aaccttatta agctcgtcac
2161 tgcaggtggg ggccagatcc tcagtagaaa gcccaagcca gacagtgacg tgactcagac
2221 catcaataca gtcgcatacc atgcgagacc cgattctgat cagcgcttct gcacacagta
2281 tatcatctat gaagatttgt gtaattatca cccagagagg gttcggcagg gcaaagtctg
2341 gaaggctcct tcgagctggt ttatagactg tgtgatgtcc tttgagttgc ttcctcttga
2401 cagctgaata ttataccaga tgaacatttc aaattgaatt tgcacggttt gtgagagccc
2461 agtcattgta ctgtttttaa tgttcacatt tttacaaata ggtagagtca ttcatatttg
2521 tctttgaatc//

- LOCUS: X59065 3658 bp DNA linear PRI 14-NOV-2006
- DEFINITION: H.sapiens FGF gene, exon 3.
- ACCESSION: X59065
- VERSION: X59065.1 GI:31359
- KEYWORDS: fgf gene; fibroblast growth factor.

1 tgaggactct tagaagtgct cttatcagta gcatcttaat tactttacaa tggattttaa
61 atggaaagga agtttacaat aatagcaaat gcatattgac agctctttag tgcccggtgc
121 tgttctaagt ccttatgact accctgtgaa ataagttcca ccatgacccc aattttcctg
181 aaaaggagac tgaggcatgg agagctttag tattttgccc aatgtcacac agctagtaaa
241 tggggacccc catgtgaaac tactcactga ttgtcctact ctcttgtggt tttatctttt
301 tagcagacac caaatgagga atgtttgttc ctggaaaggc tggaggagaa ccattacaac
361 acctatatat ccaagaagca tgcagagaag aattggtttg ttggcctcaa gaagaatggg
421 agctgcaaac gcggtcctcg gactcactat ggccagaaag caatcttgtt tctccccctg

481 ccagtctctt ctgattaaag agatctgttc tgggtgttga ccactccaga gaagtttcga
541 ggggtcctca cctggttgac ccaaaaatgt tcccttgacc attggctgcg ctaacccca
601 gcccacagag cctgaatttg taagcaactt gcttctaaat gcccagttca cttctttgca
661 gagcctttta cccctgcaca gtttagaaca gagggaccaa attgcttcta ggagtcaact
721 ggctggccag tctgggtctg ggtttggatc tccaattgcc tcttgcaggc tgagtccctc
781 aatgcaaaag tggggctaaa tgaagtgtgt taaggggtca gctaagtggg acattagtaa
841 ctgcacacta tttccctcta ctgagtaaac cctatctgtg attcccccaa acatctggca
901 tggctccctt ttgtccttcc tgtgccctgc aaatattagc aaagaagctt catgccaggt
961 taggaaggca gcattccatg accagaaaca gggacaaaga aatcccccct tcagaacaga
1021 ggcatttaaa atggaaaaga gagattggat tttggtgggt aacttagaag gatggcatct
1081 ccatgtagaa taaatgaaga aagggaggcc cagccgcagg aaggcagaat aaatccttgg
1141 gagtcattac cacgccttga ccttcccaag gttactcagc agcagagagc cctgggtgac
1201 ttcaggtgga gagcactaga agtggtttcc tgataacaag caaggatatc agagctggga
1261 aattcatgtg catctgggga ctgagtgtgg gagtgcagag aaagaaaggg aaactggctg
1321 aggggatacc ataaaaagag gatgatttca gaaggagaag gaaaaagaaa gtaatgccac
1381 acattgtgct tggcccctgg taagcagagg ctttggggtc ctagcccagt gcttctccaa
1441 cactgaagtg cttgcagatc atctggggac ctggtttgaa tggagattct gattcagtgg
1501 gttgggggca gagtttctgc agttccatca ggtccccccc aggtgcaggt gctgacaata
1561 ctgctgcctt acccgccata cattaaggag cagggtcctg gtcctaaaga gttattcaaa
1621 tgaaggtggt tcgacgcccc gaacctcacc tgacctcaac taacccttaa aaatgcacac
1681 ctcatgagtc tacctgagca ttcaggcagc actgacaata gttatgcctg tactaaggag
1741 catgatttta agatgctttg gcccaatgcc tataaaatgc ccatttcgaa gatatacaaa
1801 aacatacttc aaaaatgtta aacccttacc aacagctttt cccaggagac cgtttgtatt
1861 accattactt gtataaatac acttcctgct taaacttgac ccaggtggct agcaaattag
1921 aaacaccatt catctctaac atatgatact gatgccatgt aaaggccttt aataagtcat
1981 tgaaatttac tgtgagactg tatcttttaa ttgcatttaa aaatatatag cttgaaagca
2041 gttaaactga ttagtattca ggcactgaga atgatagtaa taggatacaa tgtataagct
2101 actcacttat ctgatactta tttacctata aaatgagatt tttgttttcc actgtgctat
2161 tacaaatttt cttttgaaag taggaactct taagcaatgg taattgtgaa taaaaattga
2221 tgagagtgtt agctcctgtt tcatatgaaa ttgaagtaat tgttaactaa aaacaattcc
2281 ttagtaactg aactgtcata tttagaatgg aaggaaaatg acagtttgtg aaagttcaaa
2341 gcaatagtgc aattgaagaa ttgacctaag taagctgaca ttatggttaa taatagtatt
2401 ttagatttgt gcagcaaaat aatttcataa cttttttgtt tttgttactt ggataagatc
2461 aatctgtttt attttagtaa atctttgcag gcaagttaga gaaaatgcag tgtggcttaa
2521 cgtctcttta gtatgaagat ttggccagaa aaagataccc agagaggaaa tctaagataa
2581 ttataatggt ccatactttt tattgtatga atcaaactca agcataacat tggccaagga
2641 aaattaaata ccattgctaa cttgtgaaat ggaagtctgt gatttcggag atgcaaagca
2701 ttgtagtaaa aacaccaatg tgacctcgac catctcagcc cagatatcat tcatatatct
2761 gttcaatgac tattaaggtg cctactgtgt gctaggcact gtactggata ctggggacct
2821 tgtctgtctg gtttgctgct gtatcttctc ccagggcatt atatttatga tgaaagatgc
2881 tgtggattca attctttcag tcaagaataa acacagactt tgtaggttcc tgctgaataa
2941 agcaaatccc agaaacccag attttggaag aatcagcaac ccccagcata aaataaaccc
3001 ctatcaaaat gtcagaggac atggcaaggt aaacttagca ttttcaactt tagaaccggg
3061 tcagcttcag ggggactgct ttcaaatcag ccaaagagcc tgtcagatct tcttagaagg
3121 aagaggttgg tagttccctg ctctgttttg aacatgctct agtttattaa cctggggaca
3181 ttcccattgc tgtcttaagt aagtctcata gccagctcct gtcacgtgac tctcatatgg
3241 attcattttc gggccagctc tgaacaaagc atcatgaaca tatgtgcttt tggtcgtttg
3301 caatgtgatg gtggtggagg taggtattgg tttccttgga aggcatgata agaaagattc
3361 acaatggcca acagtgtgta tgaacaaaaa actgattgga gcatcagcta gtactgaagg
3421 tccttgcttt gtgtcagagg caaaggaacc caaggcgcca agtcctcagc cttgagtgta
3481 ctgctgacaa ctaaactcac aggctgcaaa gcagacctct gatgaagatg cctgttattt
3541 cacatcactg tctttttgtg tatcatagtc tgcaccttac aaatattaat aaatgttcca
3601 ataataggtg ataatgtctc tgagtttctg agcatcctgc tgcctcatct ttctatca//

- LOCUS: NM_018055 1744 bp mRNA linear PRI 18-NOV-2006
- DEFINITION: Homo sapiens nodal homolog (mouse) (NODAL), mRNA.
- ACCESSION: NM_018055
- VERSION: NM_018055.3 GI:38176152

1 atgcacgccc actgcctgcc cttccttctg cacgcctggt gggccctact ccaggcgggt
61 gctgcgacgg tggccactgc gctcctgcgt acgcgggggc agccctcgtc gccatcccct
121 ctggcgtaca tgctgagcct ctaccgcgac ccgctgccga gggcagacat catccgcagc
181 ctacaggcag aagatgtggc agtggatggg cagaactgga cgtttgcttt tgacttctcc
241 ttcctgagcc aacaagagga tctggcatgg gctgagctcc ggctgcagct gtccagccct

301 gtggacctcc ccactgaggg ctcacttgcc attgagattt tccaccagcc aaagcccgac
361 acagagcagg cttcagacag ctgcttagag cggtttcaga tggacctatt cactgtcact
421 ttgtcccagg tcaccttttc cttgggcagc atggttttgg aggtgaccag gcctctctcc
481 aagtggctga agcgccctgg ggccctggag aagcagatgt ccagggtagc tggagagtgc
541 tggccgcggc cccccacacc gcctgccacc aatgtgctcc ttatgctcta ctccaacctc
601 tcgcaggagc agaggcagct gggtgggtcc accttgctgt gggaagccga gagctcctgg
661 cgggcccagg agggacagct gtcctgggag tggggcaaga ggcaccgtcg acatcacttg
721 ccagacagaa gtcaactgtg tcggaaggtc aagttccagg tggacttcaa cctgatcgga
781 tggggctcct ggatcatcta ccccaagcag tacaacgcct atcgctgtga gggcgagtgt
841 cctaatcctg ttggggagga gtttcatccg accaaccatg catacatcca gagtctgctg
901 aaacgttacc agccccaccg agtcccttcc acttgttgtg ccccagtgaa gaccaagccg
961 ctgagcatgc tgtatgtgga taatggcaga gtgctcctag atcaccataa agacatgatc
1021 gtggaagaat gtgggtgcct ctgatgacat cctggaggga gactggattt gcctgcactc
1081 tggaaggctg ggaaactcct ggaagacatg ataaccatct aatccagtaa ggagaaacag
1141 agaggggcaa agttgctctg cccaccagaa ctgaagagga ggggctgccc actctgtaaa
1201 tgaagggctc agtggagtct ggccaagcac agaggctgct gtcaggaaga gggaggaaga
1261 agcctgtgca gggggctggc tggatgttct ctttactgaa aagacagtgg caaggaaaag
1321 cacaagtgca tgagttcttt actggatttt ttaaaaacct gtgaaccccc cgaaactgta
1381 tgtgaaagtt gagacatatg tgcatgtatt ttggaggtgg gatgaagtca cctatagctt
1441 tcatgtattc tccaaagtag tctgtgtgtg acctgtcccc ctccccaaag attaaggatc
1501 actgtataga ttaaaaagag tccgtcaatc tcattgcctc aggctgggtt gggggagccc
1561 cacagctttc tggctggcca gtggcaatct actggccttg tccagaggct cactggagtg
1621 gttctctgct aatgagctgt acaacaataa agccattgtc tagttaaaaa aaaaaaaaaa
1681 aaaaaaaaaa aaaaaaaaaa aaaaaaaaaa aaaaaaaaaa aaaaaaaaaa aaaaaaaaaa
1741 aaaa//

- LOCUS: NM_007370 2119 bp mRNA linear PRI 17-NOV-2006
- DEFINITION: Homo sapiens replication factor C (activator 1) 5, 36.5kDa (RFC5),transcript variant 1, mRNA.
- ACCESSION: NM_007370
- VERSION: NM_007370.3 GI:31795541

1 gagttgcttt tgcgcgcgaa ctgtaagtgc cagggtctca gggtcaggtc gcggctggtc
61 actgtgcagg cgcttgggtg acgcgacgat ctcagcggat ctggtcacct tcgtctcccc
121 gccatggaga cctcagcact caagcagcag gagcagcccg cggcgaccaa gatcaggaac
181 ctgccctggg ttgaaaaata ccggccacag accctgaatg atctcatttc tcatcaggac
241 attctgagta ccattcagaa gtttatcaat gaagaccgac tgccacactt gcttctctac
301 ggtcccccag ggacaggcaa gacatctacc atcctagcct gtgcgaaaca gctatataaa
361 gacaaagaat ttggctccat ggtcttggag ctgaatgctt cagatgaccg aggaatagac
421 atcattcgag gaccgatcct gagctttgct agcacaagga caatatttaa gaaaggcttt
481 aagctagtga tcttggatga agcagacgcc atgactcagg acgcccagaa tgccttgaga
541 agagtaattg agaaattcac agaaaatacc agattctgcc tcatctgtaa ctatctgtca
601 aagatcatcc ctgccttgca gtcccgctgc acgaggtttc ggttcggtcc cctgactcct
661 gaactcatgg ttccccgcct ggaacatgtc gtggaagaag agaaagttga tataagtgaa
721 gatggaatga aagcactagt cactctttcc agtggagaca tgcgtagggc tctgaacatt
781 ttgcagagca ccaatatggc ctttgggaag gtgacagagg agactgtcta cacctgcacc
841 gggcacccgc tcaagtcaga cattgccaac atcctggact ggatgttgaa tcaagatttc
901 accacagcct acagaaatat tacagagttg aaaactctga aggggttggc actgcatgat
961 atcctgacag agatacactt gtttgtgcat agagttgact ttccatcttc agttcgaata
1021 catttattga ccaaaatggc agacattgag tacaggcttt ctgttggcac caacgagaag
1081 atccagctga gctccctcat tgctgcattt caagtcacca gagacctgat tgttgcagag
1141 gcctagatgc tctgagggcc attcacaatt ctcagggctc agcagtgatg ggagaacaga
1201 ggacagttcc aggataaact gctgcctggg gctgtgggat gaatcagtca ccccgaatct
1261 tggaaaaacc cccttccagg agaggatggg caggcattta aaaagtacca tttttgtggt
1321 tgtttggagc agggatgtac aaaataattt taatgtatta actcatactg cctgtctttt
1381 ataggggaaa aaaataacct tttttatttt aaagttataa ggtttttacc ttttagttgc
1441 ttggatgaca gggaattagc ctaccccatt ttggtctgga acagaagact ttcaaattta
1501 atatggtcca agtgtcttcc tactcaaggt aaacattatc tccaaaatta catttatgat
1561 tctaatattt ggcattgtgt ctgtatctaa tttaaacaga tgttaatgga cgtctggccg
1621 aaactattat acttttataa gatgagctga atcctcttac tttaaaaact ggtcttttta
1681 tttaccctct gtggtagaag agtcaccagc aggttccaaa ttgatgtgta tgataggaaa
1741 aaaaccttaa ttttaaatat aatatagagc cttaaactat gccactgggt ggcagaggct
1801 gtataaaacg cacttgtttt catgcaggag cggggcaagt aaggttgagc ctgactgtaa

1861 acctagaact gcggaaggac tgggactttt gtacaaattt cacatttatt ttacagcaat
1921 catgctgcat ttgtgttagt gttctttata ataaaaaaca aagcagcctc ctggctggct
1981 gtgttacaag tgactcttcc ttagacagct agaaagatgc tgactttgtc aaacttgcat
2041 ttatatgaaa accttctgtc tctaatatca cagaataaac tttctttaga tgctgttctt
2101 taaaaaaaaa aaaaaaaaa //

- LOCUS: NM_022094 1305 bp mRNA linear PRI 17-NOV-2006
- DEFINITION: Homo sapiens cell death-inducing DFFA-like effector c (CIDEC),mRNA.
- ACCESSION: NM_022094
- VERSION: NM_022094.2 GI:23943903

1 agaatgttct tttggccact gtgaagcctc aggaaggggc tcggattgct caaggaccca
61 tgggagagag gaggctttga ctgggctgcc tgcctgtgag gtctctggac tagaggtcca
121 acgcagtcca gctgacaagg atggaatacg ccatgaagtc ccttagcctt ctctacccca
181 agtccctctc caggcatgtg tcagtgcgta cctctgtggt gacccagcag ctgctgtcgg
241 agcccagccc caaggccccc agggcccggc cctgccgcgt aagcacggcg gatcgaagcg
301 tgaggaaggg catcatggct tacagtcttg aggacctcct cctcaaggtc cgggacactc
361 tgatgctggc agacaagccc ttcttcctgg tgctggagga agatggcaca actgtagaga
421 cagaagagta cttccaagcc ctggcagggg atacagtgtt catggtcctc cagaaggggc
481 agaaatggca gcccccatca gaacagggga caaggcaccc actgtccctc tcccataagc
541 ctgccaagaa gattgatgtg gcccgtgtaa cgtttgatct gtacaagctg aacccacagg
601 acttcattgg ctgcctgaac gtgaaggcga ctttttatga tacatactcc ctttcctatg
661 atctgcactg ctgtggggcc aagcgcatca tgaaggaagc tttccgctgg gccctcttca
721 gcatgcaggc cacaggccac gtactgcttg gcacctcctg ttacctgcag cagctcctcg
781 atgctacgga ggaagggcag ccccccaagg gcaaggcctc atcccttatc ccgacctgtc
841 tgaagatact gcagtgaaag cccaagtcct tggaagcttt ccccagtgaa ggactgactg
901 ggggcctcac gcttaactgg tagtgcccac aagcctggca gctgtagagc cgcgaacctc
961 cccacacctc cctcaccgcg caggaccctg agtgaggagg aggagctgga aacctggggt
1021 gggttggcca aaggagaacc tcaagctcct ggcctgatcc agctccttcc tgcccaaggc
1081 agcttagccc atccagactg gtcctgaagt ctgtccctcc attggcatga agtctgcccc
1141 ttagcaatcc ggcctcgcag gctgtacttt catggtgctc tctaccttct ggcccccatc
1201 ccggaacatt cctgagtgaa ttcgcaagcg cactagcatg tgatattagg gagtttgcaa
1261 taaattattg aggctgaaaa aaaaaaaaaa aaaaaaaaaa aaaaa //

- LOCUS: NM_002634 1826 bp mRNA linear PRI 04-FEB-2007
- DEFINITION: Homo sapiens prohibitin (PHB), mRNA.
- ACCESSION: NM_002634
- VERSION: NM_002634.2 GI:6031190

1 agtatgtgtg gttggggaat tcatgtggag gtcagagtgg aagcaggtgt gagagggtcc
61 agcagaagga aacatggctg ccaaagtgtt tgagtccatt ggcaagtttg gcctggcctt
121 agctgttgca ggaggcgtgg tgaactctgc cttatataat gtggatgctg ggcacagagc
181 tgtcatcttt gaccgattcc gtggagtgca ggacattgtg gtaggggaag ggactcattt
241 tctcatcccg tgggtacaga aaccaattat ctttgactgc cgttctcgac cacgtaatgt
301 gccagtcatc actggtagca aagatttaca gaatgtcaac atcacactgc gcatcctctt
361 ccggcctgtc gccagccagc ttcctcgcat cttcaccagc atcggagagg actatgatga
421 gcgtgtgctg ccgtccatca caactgagat cctcaagtca gtggtggctc gctttgatgc
481 tggagaacta atcacccaga gagagctggt ctccaggcag gtgagcgacg accttacaga
541 gcgagccgcc acctttgggc tcatcctgga tgacgtgtcc ttgacacatc tgaccttcgg
601 gaaggagttc acagaagcgg tggaagccaa acaggtggct cagcaggaag cagagagggc
661 cagatttgtg gtggaaaagg ctgagcaaca gaaaaaggcg gccatcatct ctgctgaggg
721 cgactccaag gcagctgagc tgattgccaa ctcactggcc actgcagggg atggcctgat
781 cgagctgcgc aagctggaag ctgcagagga catcgcgtac cagctctcac gctctcggaa
841 catcacctac ctgccagcgg ggcagtccgt gctcctccag ctgccccagt gagggcccac
901 cctgcctgca cctccgcggg ctgactgggc cacagccccg atgattctta acacagcctt
961 ccttctgctc ccaccccaga aatcactgtg aaatttcatg attggcttaa agtgaaggaa
1021 ataaaggtaa aatcacttca gatctctaat tagtctatca aatgaaactc tttcattctt
1081 ctcacatcca tctacttttt tatccacctc cctaccaaaa attgccaagt gcctatgcaa
1141 accagcttta ggtcccaatt cggggcctgc tggagttccg gcctgggcac cagcatttgg
1201 cagcacgcag gcggggcagt atgtgatgga ctggggagca caggtgtctg cctagatcca
1261 cgtgtggcct ccgtcctgtc actgatggaa ggtttgcgga tgagggcatg tgcggctgaa
1321 ctgagaaggc aggcctccgt cttcccagcg gttcctgtgc agatgctgct gaagagaggt
1381 gccggggagg ggcagagagg aagtggtctg tctgttacca taagtctgat tctctttaac
1441 tgtgtgacca gcggaaacag gtgtgtgtga actgggcaca gattgaagaa tctgcccctg

1501 ttgaggtggg tgggcctgac tgttgccccc cagggtccta aaacttggat ggacttgtat
1561 agtgagagag gaggcctgga ccgagatgtg agtcctgttg aagacttcct ctctaccccc
1621 caccttggtc cctctcagat acccagtgga attccaactt gaaggattgc atcctgctgg
1681 ggctgaacat gcctgccaaa gacgtgtccg acctacgttc ctggccccct cgttcagaga
1741 ctgcccttct cacgggctct atgcctgcac tgggaaggaa acaaatgtgt ataaactgct
1801 gtcaataaat gacacccaga ccttcc

- LOCUS: NM_018165 7508 bp mRNA linear PRI 17-JAN-2007
- DEFINITION: Homo sapiens polybromo 1 (PB1), transcript variant 1, mRNA.
- ACCESSION: NM_018165
- VERSION: NM_018165.3 GI:94400912

1 tccatgggtt ccaagagaag aagagctacc tccccttcca gcagtgtcag cggggacttt
61 gatgatgggc accattctgt gtcaacacca ggcccaagca ggaaaaggag gagactttcc
121 aatcttccaa ctgtagatcc tattgccgtg tgccatgaac tctataatac catccgagac
181 tataaggatg aacagggcag acttctctgt gagctcttca ttagggcacc aaagcgaaga
241 aatcaaccag actattatga agtggtttct cagcccattg acttgatgaa aatccaacag
301 aaactaaaaa tggaagagta tgatgatgtt aatttgctga ctgctgactt ccagcttctt
361 tttaacaatg caaagtccta ttataagcca gattctcctg aatataaagc cgcttgcaaa
421 ctctgggatt tgtaccttcg aacaagaaat gagtttgttc agaaaggaga agcagatgac
481 gaagatgatg atgaagatgg gcaagacaat cagggcacag tgactgaagg atcttctcca
541 gcttacttga aggagatcct ggagcagctt cttgaagcca tagttgtagc tacaaatcca
601 tcaggacgtc tcattagcga actttttcag aaactgcctt ctaaagtgca atatccagat
661 tattatgcaa taattaagga gcctatagat ctcaagacca ttgcccagag gatacagaat
721 ggaagctaca aaagtattca tgcaatggcc aaagatatag atctcctcgc aaaaaatgcc
781 aaaacttata atgagcctgg ctctcaagta ttcaaggatg caaattcaat taaaaaaata
841 ttttatatga aaaaggctga aattgaacat catgaaatgg ctaagtcaag tcttcgaatg
901 agtaataaaa gagcagtaca aggaggtcgt ttatcagcaa ttacaatggc acttcaatat
961 ggctcagaaa gtgaagaaga tgctgcttta gctgctgcac gctatgaaga gggagagtca
1021 gaagcagaaa gcatcacttc ctttatggat gtttcaaatc cttttatcca gctttatgac
1081 acagttagga gttgtcggaa taaccaaggg cagctaatag ctgaaccttt ttaccatttg
1141 ccttcaaaga aaaaataccc tgattattac cagcaaatta aaatgcccat atcactacaa
1201 cagatccgaa caaaactgaa gaatcaagaa tatgaaactt tagatcattt ggagtgtgat
1261 ctgaatttaa tgtttgaaaa tgccaaacgc tataatgtgc ccaattcagc catctacaag
1321 cgagttctaa aattgcagca agttatgcag gcaaagaaga aagagcttgc caggagagac
1381 gatatcgagg acggagacag catgatctct tcagccacct ctgatactgg tagtgccaaa
1441 agaaaaagta aaaagaacat aagaaagcag cgaatgaaaa tcttattcaa tgttgttctt
1501 gaagctcgag agccaggttc aggcagaaga ctttgtgacc tatttatggt taaaccatcc
1561 aaaaaggact atcctgatta ttataaaatc atcttggagc caatggactt gaaaataatt
1621 gagcataaca tccgcaatga caaatatgct ggtgaagagg gaatgataga agacatgaag
1681 ctgatgttcc ggaatgccag gcactataat gaggagggct cccaggttta taatgatgca
1741 catatcctgg agaagttact caaggagaaa aggaaagagc tgggcccact gcctgatgat
1801 gatgacatgg cttctcccaa actcaagctg agtaggaaga gtggcatttc tcctaaaaaa
1861 tcaaaataca tgactccaat gcagcagaaa ctaaatgagg tctatgaagc tgtaaagaac
1921 tatactgata agaggggtcg ccgcctcagt gccatatttc tgaggcttcc ctctagatct
1981 gagttgcctg actactatct gactattaaa aagcccatgg acatggaaaa aattcgaagt
2041 cacatgatgg ccaacaagta ccaagatatt gactctatgg ttgaggactt tgtcatgatg
2101 tttaataatg cctgtacata caatgagccg gagtctttga tctacaaaga tgctcttgtt
2161 ctacacaaag tcctgcttga aacacgcaga gacctggagg gagatgagga ctctcatgtc
2221 ccaaatgtga ctttgctgat tcaagagctt atccacaatc tttttgtgtc agtcatgagt
2281 catcaggatg atgagggaag atgctacagc gattctttag cagaaattcc tgctgtggat
2341 cccaactttc ctaacaaacc accccttaca tttgacataa ttaggaagaa tgttgaaaat
2401 aatcgctacc gtcggcttga tttatttcaa gagcatatgt ttgaagtatt ggaacgagca
2461 agaaggatga atcggacaga ttcagaaata tatgaagatg cagtagaact tcagcagttt
2521 tttattaaaa ttcgtgatga actctgcaaa aatggagaga ttcttctttc accggcactc
2581 agctatacca caaaacattt gcataatgat gtggagaaag agagaaagga aaaattgcca
2641 aaagaaatag aggaagataa actaaaacga gaagaagaaa aaagagaagc tgaaaagagt
2701 gaagattcct ctggtgctgc aggcctctca ggcttacatc gcacatacag ccaggactgt
2761 agctttaaaa acagcatgta ccatgttgga gattacgtct atgtggaacc tgcagaggcc
2821 aacctacaac cacatatcgt ctgtattgaa agactgtggg aggattcagc tggtgaaaaa
2881 tggttgtatg gctgttggtt ttaccgacca aatgaaacat tccacctggc tacacgaaaa
2941 tttctagaaa aagaagtttt taagagtgac tattacaaca aagttccagt tagtaaaatt
3001 ctaggcaagt gtgtggtcat gtttgtcaag gaatacttta agttatgccc agaaaacttc
3061 cgagatgagg atgtttttgt ctgtgaatca cggtattctg ccaaaaccaa atcttttaag

3121 aaaattaaac tgtggaccat gcccatcagc tcagtcaggt ttgtccctcg ggatgtgcct
3181 ctgcctgtgg ttcgcgtggc ctctgtattt gcaaatgcag ataaaggtga tgatgagaag
3241 aatacagaca actcagagga cagtcgagct gaagacaatt ttaacttgga aaggaaaaa
3301 gaagatgtcc ctgtggaaat gtccaatggt gaaccaggtt gccactactt tgagcagctc
3361 cattacaatg acatgtggct gaaggttggc gactgtgtct tcatcaagtc ccatggcctg
3421 gtgcgtcctc gtgtgggcag aattgaaaaa gtatgggttc gagatggagc tgcatatttt
3481 tatggcccca tcttcattca cccagaagaa acagagcatg agcccacaaa aatgttctac
3541 aaaaaagaag tatttctgag taatctggaa gaaacctgcc ccatgacatg tattctcgga
3601 aagtgtgctg tgttgtcatt caaggacttc ctctcctgca ggccaactga aataccagaa
3661 aatgacattc tgctttgtga gagccgctac aatgagagcg acaagcagat gaagaaattc
3721 aaaggattga agaggttttc actctctgct aaagtggtag atgatgaaat ttactacttc
3781 agaaaaccaa ttgttcctca gaaggagcca tcacctttgc tggaaaagaa gatccagttg
3841 ctagaagcta aatttgccga gttagaaggt ggagatgatg atattgaaga gatgggagaa
3901 gaagatagtg aggtcattga acctccttct ctacctcagc ttcagacccc cctggccagt
3961 gagctggacc tcatgcccta cacaccccca cagtctaccc caaagtctgc caaaggcagt
4021 gcaaagaagg aaggctccaa acggaaaatc aacatgagtg gctacatcct gttcagcagt
4081 gagatgaggg ctgtgattaa ggcccaacac ccagactact ctttcgggga gctcagccgc
4141 ctggtgggga cagaatggag aaatcttgag acagccaaga aagcagaata tgaaggcatg
4201 atgggtggct atccgccagg ccttccacct ttgcagggcc cagttgatgg ccttgttagc
4261 atgggcagca tgcagccact tcaccctggg ggccctccac cccaccatct tccgccaggt
4321 gtgcctggcc tcccgggcat cccaccaccg ggtgtgatga accaaggagt ggcccctatg
4381 gtagggactc cagcaccagg tggaagtcca tatggacaac aggtgggagt tttggggcct
4441 ccagggcagc aggcaccacc tccatatccc ggcccacatc cagctggacc ccctgtcata
4501 cagcagccaa caacacccat gtttgtagct cccccaccaa agacccagcg gcttcttcac
4561 tcagaggcct acctgaaata cattgaagga ctcagtgcgg agtccaacag cattagcaag
4621 tgggatcaga cactggcagc tcgaagacgc gacgtccatt tgtcgaaaga acaggagagc
4681 cgcctaccct ctcactggct gaaaagcaaa ggggcccaca ccaccatggc agatgccctc
4741 tggcgccttc gagatttgat gctccgggac accctcaaca ttcgccaagc atacaaccta
4801 gaaaatgttt aatcacatca ttacgtttct tttatataga agcataaaga gttgtggatc
4861 agtagccatt ttagttactg ggggtggggg gaaggaacaa aggaggataa tttttattgc
4921 attttactgt acatcacaag gccattttta tatacggaca cttttaataa gctatttcaa
4981 tttgtttgtt atattaagtt gactttatca aatacacaaa gatttttttg catatgtttc
5041 cttcgtttaa aaccagtttc ataattggtt gtatatgtag acttggagtt ttatcttttt
5101 acttgttgcc atggaactga aaccattaga ggtttttgtc ttggcttggg gtttttgttt
5161 tcttggtttt gggttttttt atatatatat ataaaagaac aaaatgaaaa aaaacacaca
5221 cacacaagag tttacagatt agtttaaatt gataatgaaa tgtgaagttt gtcctagttt
5281 acatcttaga gaggggagta tacttgtgtt tgtttcatgt gcctgaatat cttaagccac
5341 tttctgcaaa agctgtttct tacagatgaa gtgctttctt tgaaaggtgg ttatttaggt
5401 tttagatgtt taatagacac agcacatttg ctctattaac tcagaggctc actacagaaa
5461 tatgtaatca gtgctgtgca tctgtctgca gctaatgtac ctcctggaca ccaggagggg
5521 aaaaagcact ttttcaattg tgctgagtta gacatctgtg agttagacta tggtgtcagt
5581 gatttttgca gaacacgtgc acaaccctga ggtatgttta atctaggcag gtacgtttaa
5641 ggatattttg atctatttat aatgaattca caatttatgc ctataaattt cagatgattt
5701 aaaattttaa acctgttaca ttgaaaaaca ttgaagttcg tcttgaagaa agcattaagg
5761 tatgcatgga ggtgatttat ttttaaacat aacacctaac ctaacatggg taagagagta
5821 tggaactaga tatgagctgt ataagaagca taattgtgaa caagtagatt gattgccttc
5881 atatacaagt atgttttagt attccttatt tccttattat cagatgtatt ttttctttta
5941 agtttcaatg ttgttataat tctcaaccag aaatttaata ctttctaaaa tattttttaa
6001 atttagcttg tgcttttgaa ttacaggaga agggaatcat aatttaataa aacgcttact
6061 agaaagacca ttacagatcc caaacacttg ggtttggtga ccctgtcttt cttatatgac
6121 cctacaataa acatttgaag gcagcatagg atggcagaca gtaggaacat tgtttcactt
6181 ggcggcatgt ttttgaaacc tgctttatag taactgggtg attgccattg tggtagagct
6241 tccactgctg tttataatct gagagagtta atctcagagg atgctttttt ccttttaatc
6301 tgctatgaat cagtacccag atgtttaatt actgtactta ttaaatcatg agggcaaaag
6361 agtgtagaat ggaaaaaagt ctcttgtatc tagatacttt aaatatggga ggccctttaa
6421 cttaattgcc tttagtcaac cactggattt gaatttgcat caagtatttt aaataatatt
6481 gaatttaaaa aaatgtattg cagtagtgtg tcagtacctt attgttaaag tgagtcagat
6541 aaatcttcaa ttcctggcta tttgggcaat tgaatcatca tggactgtat aatgcaatca
6601 gattattttg tttctagaca tccttgaatt acaccaaaga acatgaaatt tagttgtggt
6661 taaattattt atttatttca tgcattcatt ttatttccct taaggtctgg atgagacttc
6721 tttggggagc ctctaaaaaa atttttcact gggggccacg tgggtcatta gaagccagag
6781 ctctcctcca ggctccttcc cagtgcctag aggtgctata ggaaacatag atccagccag
6841 gggcttccct aaagcagtgc agcaccggcc cagggcatca ctagacaggc cctaattaag

6901 ttttttttaa aaagcctgtg tatttatttt agaatcatgt ttttctgtat attaacttgg
6961 gggatatcgt taatatttag gatataagat ttgaggtcag ccatcttcaa aaaagaaaaa
7021 aaaattgact caagaaagta caagtaaact atacaccttt ttttcataag ttttaggaac
7081 tgtagtaatg tggcttagaa agtataatgg cctaaatgtt ttcaaaatgt aagttcctgt
7141 ggagaagaat tgtttatatt gcaaacgggg ggactgaggg gaacctgtag gtttaaaaca
7201 gtatgtttgt cagccaactg atttaaaagg cctttaactg ttttggttgt tgtttttttt
7261 ttaagccact ctccccttcc tatgaggaag aattgagagg ggcacctatt tctgtaaaat
7321 ccccaaattg gtgttgatga ttttgagctt gaatgttttc atacctgatt aaaacttggt
7381 ttattctaat ttctgtatca tatcatctga ggtttacgtg gtaactagtc ttataacatg
7441 tatgtatctt ttttttgttg ttcatctaaa gctttttaat ccaaataaat acagagtttg
7501 caaagtga //

- LOCUS: AF074717 1051 bp mRNA linear PRI 24-DEC-1998
- DEFINITION: Homo sapiens Radl-like DNA damage checkpoint protein (RAD1) mRNA, complete cds.
- ACCESSION: AF074717
- VERSION: AF074717.1 GI:3309650

1 ggagccgcct cgctcctctt cagggacttt gctgagaagg gctctcgggc gtccagaccc
61 caccgcaaag gtgtttggcg atccgcgaga agttgttggc cccaggagca tccctcgggg
121 ccgaatgcgc agtgggacga tgccccttct gacccaacag atccaagacg aggatgatca
181 gtacagcctt gtggccagcc ttgacaacgt taggaatctc tccactatct tgaaagctat
241 tcatttccga gaacatgcca cgtgtttcgc aactaaaaat ggtatcaaag taacagtgga
301 aaatgcaaag tgtgtgcaag caaatgcttt tattcaggct ggaatatttc aggagtttaa
361 agttcaggaa gagtctgtta cttttcgaat taatttaact gtccttttag actgtttatc
421 tatttttgga tcaagtccta tgccagggac tttaactgca cttcgaatgt gttaccaagg
481 ttatggttac cctttgatgc tgttcctgga agaaggagga gtggtgacag tctgcaaaat
541 caatacacag gaacctgagg agaccctgga ctttgatttc tgcagcacca atgttattaa
601 taaaattatt ctgcagtcag aggggctccg tgaagcattt tctgaattgg atatgacgag
661 tgaagtccta caaattacca tgtctcctga caagccttat ttcaggttat ctacttttgg
721 aaatgcagga agttcccacc ttgactatcc caaagattct gatttgatgg aagcatttca
781 ttgtaatcag acccaagtca acagatacaa gatttcctta ctgaaaccct ctacaaaggc
841 attagtccta tcttgtaagg tatctattcg gacagataac agaggcttcc tttcattaca
901 gtatatgatt agaaatgaag atggacaaat atgttttgtg gaatattact gctgccctga
961 tgaagaagtt cctgaatctg agtcttgagt atgacaattc actgatattt atgtgtacat
1021 ttatgataga tgaagttctt attctgagta c//

- LOCUS: NM_016567 1290 bp mRNA linear PRI 17-NOV-2006
- DEFINITION: Homo sapiens BRCA2 and CDKN1A interacting protein (BCCIP), transcript variant A, mRNA.
- ACCESSION: NM_016567
- VERSION: NM_016567.2 GI:17402869

1 gtgagcggca acatggcgtc caggtctaag cggcgtgccg tggaaagtgg ggttccgcag
61 ccgccggatc ccccagtcca gcgcgacgag gaagaggaaa aagaagtcga aaatgaggat
121 gaagacgatg atgacagtga caaggaaaag gatgaagagg acgaggtcat tgacgaggaa
181 gtgaatattg aatttgaagc ttattcccta tcagataatg attatgacgg aattaagaaa
241 ttactgcagc agctttttct aaaggctcct gtgaacactg cagaactaac agatctctta
301 attcaacaga accatattgg gagtgtgatt aagcaaacgg atgtttcaga agacagcaat
361 gatgatatgg atgaagatga ggtttttggt ttcataagcc ttttaaattt aactgaaaga
421 aagggtaccc agtgtgttga acaaattcaa gagttggttc tacgcttctg tgagaagaac
481 tgtgaaaaga gcatggttga acagctggac aagtttttaa atgacaccac caagcctgtg
541 ggccttctcc taagtgaaag attcattaat gtccctccac agatcgctct gcccatgtac
601 cagcagcttc agaaagaact ggcgggggca cacagaacca ataagccatg tgggaagtgc
661 tacttttacc ttctgattag taagacattt gtggaagcag gaaaaaacaa ttccaaaaag
721 aaacctagca acaaaaagaa agctgcgtta atgtttgcaa atgcagagga agaatttttc
781 tatgaggagc agggaaaacc tgaggtgctt ggaggtccag acacaagaag aggattggaa
841 ccagttccga tacagcacaa tggtggttca cgggggcaag tgacagccct ggtttctctg
901 aaggctggac taattcaatc aagatcaact ctaagtgatt tccagggaac cttcatgact
961 gttggaattg ctctgtcata ataagtcagg gatatttagg aggctcatag tctcctggag
1021 ggataaaaca tctcggcacc tagtaatggt aaattagtca atatttgtta gtttcgtttg
1081 agatctcaaa tgttaggatt ttctgaagtc tcagtgtctt tcagagtttg agataccttg
1141 ttttcatttt ttctaatttt taatttaaaa gtaataaaga atattttgta atgattagga
1201 tgaaaatttt atcgtcaatt ataagccaaa atttgctaaa attaaagtca gtatacttgg

1261 aaaaaaaaaa aaaaaaaaaa aaaaaaaaaa //

- LOCUS: NM_004708 599 bp mRNA linear PRI 17-NOV-2006
- DEFINITION: Homo sapiens programmed cell death 5 (PDCD5), mRNA.
- ACCESSION: NM_004708
- VERSION: NM_004708.2 GI:21735599

1 aggccgcgct cgcgccgagg ggctgcgaga gtgaccgcgg ctgctccagc gctgacgccg
61 agccatggcg gacgaggagc ttgaggcgct gaggagacag aggctggccg agctgcaggc
121 caaacacggg gatcctggtg atgcggccca acaggaagca aagcacaggg aagcagaaat
181 gagaaacagt atcttagccc aagttctgga tcagtcggcc cgggccaggt taagtaactt
241 agcacttgta aagcctgaaa aaactaaagc agtagagaat taccttatac agatggcaag
301 atatggacaa ctaagtgaga aggtatcaga acaaggttta atagaaatcc ttaaaaaagt
361 aagccaacaa acagaaaaga caacaacagt gaaattcaac agaagaaaag taatggactc
421 tgatgaagat gacgattatt gaactacaag tgctcacaga ctagaactta acggaacaag
481 tctaggacag aagttaagat ctgattattt actttgttta ttgtctatat gccttttaaa
541 aaaataaact tgttatgcaa aataaaaaaa aaaaaaaaaa aaaaaaaaaa aaaaaaaaa
//

- LOCUS: AF277724 412 bp mRNA linear PRI 21-DEC-2000
- DEFINITION: Homo sapiens cell division cycle 25C splice variant 3 (CDC25C) mRNA, partial cds.
- ACCESSION: AF277724
- VERSION: AF277724.1 GI:11934924

1 ctcctggaga gagacacttc ctttaccgtc tgtccagatg tccctagaac tccagtgggc
61 aaatttcttg gtgattctgc aaacctaagc attttgtctg gaggaacccc aaaatgttgc
121 ctcgatcttt cgaatcttag cagtggggag ataactgcca ctcagcttac cacttctgca
181 gaccttgatg aaactggtca cctggattct tcaggacttc aggaagtgca tttagctggg
241 atgacaatgg aaacttggtg gacagtgaaa tgaaatattt gggcagtccc attactactg
301 ttccaaaatt ggataaaaat ccaaacctag gagaagacca ggcagaagag atttcagatg
361 aattaatgga gttttccctg aaagatcaag aagcaaaggt gagcagaagt gg//

- LOCUS: NM_006999 3861 bp mRNA linear PRI 18-NOV-2006
- DEFINITION: Homo sapiens polymerase (DNA directed) sigma (POLS), mRNA.
- ACCESSION: NM_006999
- VERSION: NM_006999.3 GI:62548868

1 gcggccgctc tcagcggagg gggcggcccc ggggcccagg cgccgcggcc cggcaccccg
61 tggaagagcc gcgcgtacag cccgggcatc cagggactac atgaggaaat aattgacttt
121 tataacttca tgtccccttg tcctgaagaa gcagctatga gaagagaggt ggtgaaacgg
181 atcgaaactg tggtgaaaga cctttggccg acggctgatg tacagatatt tggcagcttt
241 agtacaggtc tttatcttcc aactagcgac atagacctgg tggtcttcgg gaaatgggag
301 cgtcctcctt tacagctgct ggagcaagcc ctgcggaagc acaacgtggc tgagccgtgt
361 tccatcaaag tccttgacaa ggctacggta ccaataataa agctcacaga tcaggagact
421 gaagtgaaag ttgacatcag ctttaacatg gagacgggcg tccgggcagc ggagttcatc
481 aagaattaca tgaagaaata ttcattgctg ccttacttga ttttagtatt gaaacagttc
541 cttctgcaga gggacctgaa tgaagttttt acaggtggaa ttagctcata cagcctaatt
601 ttaatggcca ttagctttct acagttgcat ccaagaattg atgcccggag agctgatgaa
661 aaccttggaa tgcttcttgt agaatttttt gaactctatg ggagaaattt taattacttg
721 aaaaccggta ttagaatcaa agaaggaggt gcctatatcg ccaaagagga gatcatgaaa
781 gccatgacca gcgggtacag accgtcgatg ctgtgcattg aggaccccct gctgccaggg
841 aatgacgttg gccggagctc ctatggcgcc atgcaggtga agcaggtctt cgattatgcc
901 tacatagtgc tcagccatgc tgtgtcaccg ctggccaggt cctatccaaa cagagacgcc
961 gaaagtactt taggaagaat catcaaagta actcaggagg tgattgacta ccggaggtgg
1021 atcaaagaga agtggggcag caaagcccac ccgtcgccag gcatggacag caggatcaag
1081 atcaaagagc gaatagccac atgcaatggg gagcagacgc agaaccgaga gcccgagtct
1141 ccctatggcc agcgcttgac tttgtcgctg tccagccccc agctcctgtc ttcaggctcc
1201 tcggcctctt ctgtgtcttc actttctggg agtgacgttg attcagacac accgccctgc
1261 acaacgccca gtgtttacca gttcagtctg caagcgccag ctcctctcat ggccggctta
1321 cccaccgcct tgccaatgcc cagtggcaaa cctcagccca ccacttccag aacactgatc
1381 atgacaacca acaatcagac caggtttact atacctccac cgaccctagg ggttgctcct
1441 gttccttgca gacaagctgg tgtagaagga actgcgtctt tgaaagccgt ccaccacatg
1501 tcttccccgg ccattccctc agcgtccccc aacccgctct cgagccctca tctgtatcat
1561 aagcagcaca acggcatgaa actgtccatg aaggctctca cggccacacc ccaaggcggc
1621 ggctacagct ctgtgggtag cggaggtgtg cggccccctg tgggcaacag gggacaccac

1681 cagtataacc gcaccggctg gaggaggaaa aaacacacac acacacggga cagtctgccc
1741 gtgagcctca gcagataatg gctcctggct gcgtcagcct cccccacccc tctgcagact
1801 gccccgcggc ctcggccacc ggcaggggaa ccgagaccag caccccgcac gtcagccggg
1861 ctcgcggcac gcccgccgct gatcactctg catgtttctt cgtgtggtgg tcgcgtccat
1921 cttcaagaac agctcgttgt gctcatctgt gaagccttat taaacgtgga cgttgttttc
1981 tgccttccca ggattcttcc ttcagtgctg aggcaggtcg ggctcaggaa ctgcagggac
2041 gtgaacatgc gcttgcggtt tgaggtagcc gtgtctgttc cttcgcggtt tgctattttc
2101 atttcctgtt cgtcaaagca gcagaggaga tcaaaccccg ttcgtgtgtc tttcctccac
2161 ggataagctt gggaggtcat tgttttactg ccctcacatt ttgtttgaaa tttcagaact
2221 gtttttctat gtaaatattg aaaacttatg atttgtgcaa taactcagat attttttatt
2281 taatttccta ttttcacata agttatattt aagggaggag ggaatttttt ttaaacaagc
2341 ttaggtcctt tcccgagctg cattttctaa gttgggtcat cgtgtcggct ggttgtctga
2401 cgagcatcgt tacaaacacc atgatgaggg gtttggggtt ttattttgat gtcttttctt
2461 ttggtcggaa gtgagtgaag gagccaggtc gccctgaagg ttttccaaag ggcttggctc
2521 cagagccacc tggcagactg cccgtggccc tgctgtcggg ccccaggccg ttgtcctgct
2581 ctgaccacag agttttaatg ttttggtttt cacttctttt aaactggaca acaaatccag
2641 catttcaagt gccagaagta taactttcta aggagagaag ggttgtcaca ttataaaatc
2701 tttaggaaaa tgtgaactgg aaaacgcttc ggtcagtttt agtgacatag cctgtgatga
2761 tgggtctggt gactattatt gcggaccgtg gtacccagtt ttaggaatgt ggagaaagga
2821 attctgttga ttccgttgag gaatctgtag cgtatgcatt cgttctgtta agagcaaatc
2881 taggagaagt gcttcagctg cccagtgcgc cgtggggagt gttttaacgg atcgtgtcgc
2941 aggagagcac agcccagcgt tggggccggg accgctggcg cccgacgtcg gaagcataca
3001 ggtatactat gcaagtgtat tctgccacaa caaccactgt ctttgttacc tttttttgaa
3061 caagaatata tccatcctgc ctaaccctga gtttttggag caccacagtt gtcctgggag
3121 ttggttgcat cttgtaggcc atctgacttc ctgtttttaa aacgggggtc tggtcttgct
3181 aaacactaca ggtaggttgg tctttgaagt ccactagtgg agaatgtcaa gacaagatac
3241 ttattaccat gacatctgat gcatgtgcag cagtggggag ttctagattg atctctgaat
3301 gtgatcgacg cccagcaagg acaagcttta aaatgtctgc ggtctgccct tttgaagcag
3361 gactggctca ctctgtcatt gggagctgtc agctgcgact gcaggttctc taggaggcat
3421 tccagaatag agtagcacac tgtgtctgca gttctcgatg accgaaagtt atcaaaaata
3481 tttaaaatat ttaaattgtg aacctattga taaagaatat ttataaaaac tgatctgtag
3541 gcctgtacta atctctacgc attagcaata ttgactgtaa acccacatta aggaaaccac
3601 tacgggtctg gcagtgcgtg tcccgtgggg tgtgcatttt aaaactcgat tcatagacac
3661 aggtaccatg ttccatttcc gtcatggtga agcaaatgaa ttggcctggc taccactgtg
3721 gtcgcgtgct acaggtttga caaaaagata tcatgtttcg atttttttgt gtgtggacaa
3781 caatatggaa gctaaaattg acatattttt atgtaaagtt tttctattct ttgattttta
3841 ataaactttg gaaaccagtt t //

- LOCUS: NM_001786 1235 bp mRNA linear PRI 14-JAN-2007
- DEFINITION: Homo sapiens cell division cycle 2, G1 to S and G2 to M (CDC2),transcript variant 1, mRNA.
- ACCESSION: NM_001786
- VERSION: NM_001786.2 GI:16306490

1 gggggggggg ggcacttggc ttcaaagctg gctcttggaa attgagcgga gagcgacgcg
61 gttgttgtag ctgccgctgc ggccgccgcg gaataataag ccgggatcta ccatacccat
121 tgactaacta tggaagatta taccaaaata gagaaaattg gagaaggtac ctatggagtt
181 gtgtataagg gtagacacaa aactacaggt caagtggtag ccatgaaaaa aatcagacta
241 gaaagtgaag aggaaggggt tcctagtact gcaattcggg aaatttctct attaaaggaa
301 cttcgtcatc caaatatagt cagtcttcag gatgtgctta tgcaggattc caggttatat
361 ctcatctttg agtttctttc catggatctg aagaaatact tggattctat ccctcctggt
421 cagtacatgg attcttcact tgttaagagt tatttatacc aaatcctaca ggggattgtg
481 ttttgtcact ctagaagagt tcttcacaga gacttaaaac ctcaaaatct cttgattgat
541 gacaaaggaa caattaaact ggctgatttt ggccttgcca gagcttttgg aatacctatc
601 agagtatata cacatgaggt agtaacactc tggtacagat ctccagaagt attgctgggg
661 tcagctcgtt actcaactcc agttgacatt tggagtatag gcaccatatt tgctgaacta
721 gcaactaaga aaccactttt ccatggggat tcagaaattg atcaactctt caggattttc
781 agagctttgg gcactcccaa taatgaagtg tggccagaag tggaatcttt acaggactat
841 aagaatacat ttcccaaatg gaaaccagga agcctagcat cccatgtcaa aaacttggat
901 gaaaatggct tggatttgct ctcgaaaatg ttaatctatg atccagccaa acgaatttct
961 ggcaaaatgg cactgaatca tccatatttt aatgatttgg acaatcagat taagaagatg
1021 tagctttctg acaaaaagtt tccatatgtt atgtcaacag atagttgtgt tttattgtt

1081 aactcttgtc tatttttgtc ttatatatat ttctttgtta tcaaacttca gctgtacttc
1141 gtcttctaat ttcaaaaata taacttaaaa atgtaaatat tctatatgaa tttaaatata
1201 attctgtaaa tgtgaaaaaa aaaaaaaaaa aaaaa //

- LOCUS: NM_002898 1997 bp mRNA linear PRI 18-NOV-2006
- DEFINITION: Homo sapiens RNA binding motif, single stranded interacting protein 2 (RBMS2), mRNA.
- ACCESSION: NM_002898
- VERSION: NM_002898.2 GI:52486500

1 cctccctccc tccccgtctt tcttaccccc tccctttctc tctctctctc tctctcgctc
61 gttccctaac attaaagaga aaatgctgct atccgtgact tccaggcccg ggatttcgac
121 ttttggctac aatagaaaca acaagaagcc atatgtgtca ttggctcagc agatggcacc
181 acctagccca agcaacagta cacctaacag cagtagtgga agcaatggaa atgaccagct
241 gagcaaaacc aacctataca tccgaggatt gcaaccaggc actactgacc aagatcttgt
301 caagctgtgt cagccatatg gcaagattgt ttccactaag gccatactgg acaagaccac
361 aaacaaatgt aaaggctatg gctttgtaga ttttgacagc ccttcagcag cacagaaagc
421 tgtaacagca ctgaaggcca gcggtgtaca ggcacagatg gcaaagcaac aggaacagga
481 ccccacaaat ttatacatct caaacctccc actgtcaatg gatgagcagg aactggaggg
541 gatgctgaag ccctttggcc aggttatctc cacccgtatc cttcgagata ccagtgggac
601 cagcagaggt gttggctttg caaggatgga gtccacagag aagtgtgaag ccatcatcac
661 ccactttaat ggaaaatata ttaagacacc ccctggagta ccagccccat ccgatccctt
721 gctttgcaaa tttgctgatg gcgggccaaa gaaacgacag aaccaaggaa aatttgtgca
781 aaatggacgg gcttggccaa ggaatgcaga catgggcgtc atggccttga cctatgaccc
841 caccacagct cttcagaatg ggttttaccc agccccctat aacatcaccc ccaacaggat
901 gcttgctcag tctgcactct ccccatacct ttcctctcct gtgtcttcgt atcagagagt
961 gactcagaca tctcctctac aagtacctaa cccatcctgg atgcaccacc attcatacct
1021 catgcagcct tcaggttcag ttctgacacc agggatggac catcccattt ctctccagcc
1081 tgcctccatg atgggacccc ttacccagca actgggccat ctctccctca gcagcacagg
1141 cacgtatatg ccgacggctg cagctatgca aggagcttac atctcccagt acacccctgt
1201 gccttcttcc agtgtttcag tcgaggagag cagcggccaa cagaaccaag tggcagtgga
1261 cgcaccctca gagcatgggg tctattcttt ccagttcaac aagtaacagt gggattcccc
1321 tccccatctt tactgaatag aaatgaattc ttggagatac tcatgctccc agattccaga
1381 gggttaacca ggaatggaga ccatccgtcg gccctgctaa ggactaacac ttagccatcg
1441 tttttcacag gcctgggcct ggaaaaagaa atctctacgt tcctgccctt tactattgct
1501 gatggagcct gggggaacca tcactttttt tgtgtgctac attcaaggag atcaaaaaaa
1561 cttttcttct tttgcaaaga aagctttttg tttttaactg caacgtactt ttcccctacc
1621 ttgaagagac atggtggtcg cagcttctca tctatatgaa aaagttttcg atgtattgga
1681 attatttggg aatgctttta aaacaatttg taattatttc tttacaaacc aaaacagaac
1741 agaaaggtgt ggtgctggaa catcgatgaa ggagccctac ttactgagct tagttatgga
1801 cttttttgat gcatgtgtgt atgtgtttta aaaagtatgc aggctctaaa aatgttattt
1861 tgtaaagctc tcagctcatg caccccatct cctcttcacc catattatgc cttctttctc
1921 ttgtccagat tcttcttttt ctcttttcta aacagctgag cctgcctact ttgccctttt
1981 acagctttta attttat//

- LOCUS: NM_006262 1833 bp mRNA linear PRI 23-NOV-2006
- DEFINITION: Homo sapiens peripherin (PRPH), mRNA.
- ACCESSION: NM_006262
- VERSION: NM_006262.3 GI:66932907

1 cctcgcagcg gtctgcggct ccttcccagc ccccggccta gctctgcgaa cggtgactgc
61 ccatccttgg ccgcaatgag ccaccacccg tcgggcctcc gggccggctt cagctccacc
121 tcataccgcc gtaccttcgg tccaccgccc tcactatccc ccggggcctt ctcctactcg
181 tccagctccc gcttctccag cagccgcctg ctgggctccg cgtccccgag ctcctcggtg
241 cgcctgggca gcttccgtag cccccgagcg ggagcgggcg ccctcctgcg cctgccctcg
301 gagcgcctcg acttctccat ggccgaggcc ctcaaccagg agttcctggc cacgcgcagc
361 aacgagaagc aggagctgca ggagctcaac gaccgcttcg ccaacttcat cgagaaggta
421 cgctttctgg agcagcagaa cgcggccctg cgcggggagc tgagccaagc ccggggccag
481 gagccggcgc gcgccgacca gctgtgccag caggagctgc gcgagctgcg gcgagagctg
541 gagctgttgg gccgcgagcg tgaccgggtg caggtggagc gcgacgggct ggcggaggac
601 ctggcggcgc tcaagcagag gttggaggag gagacgcgca agcgggagga cgcggagcac
661 aacctcgtgc tcttccgcaa ggacgtggac gatgccactc tgtcccgcct ggaactagag
721 cgcaagattg agtctctgat ggatgagatt gagttcctca agaagctgca cgaggaggag
781 ctgcgagacc tgcaggtgag tgtggagagc cagcaggtgc agcaggtgga ggtggaagcc
841 acggtgaagc ccgagctgac ggcagcgctg agggacatcc gcgcgcagta cgagagcatc

901 gccgcgaaga acctgcagga ggcggaggag tggtacaagt ccaagtacgc ggacctgtcc
961 gacgctgcca accggaacca cgaggccctg cgccaggcca agcaggagat gaacgagtcc
1021 cgacgccaga tccagagtct aacgtgcgag gtggacgggc tgcgcggcac gaacgaggcg
1081 ctgctcaggc agttgagaga gctggaggag cagttcgccc tggaggcggg gggctaccag
1141 gcgggcgctg cgcggctcga ggaggagctg cgacagctaa aagaggagat ggcgcggcac
1201 ctgagggagt accaggagct cctcaacgtc aagatggccc tggacatcga gatcgccacc
1261 taccgcaagc tgctggaggg cgaggagagc cggatctccg tgcccgtcca ttcttttgcc
1321 tccttaaata taaagacgac tgtgcctgag gtggagcctc cccaggacag ccacagccgg
1381 aagacggttc tgatcaagac cattgagacc cggaatgggg aggtggtgac agagtcccag
1441 aaggagcagc gcagtgagct ggacaagtct tctgcccaca gttactgaac cccttggtcc
1501 ggagccttga ctctgcccta ggcctgctca aagcccaaac cctaagacca ctcctgaatt
1561 gtctcctctc cctctgcatg tgtctaaaag gtggtaccag gcatcccttt cctggcttat
1621 ggccaagccc tacccggcca gcagtcgctg ggcctctccc tgccctgaca cttgatgtga
1681 cctatgtgct tcccttttca tgtcccgata agaagccaat gatcccccct caggacaaat
1741 ctactccagc cacgatgaga agtgggtgag ccagggtctg agtttcacat ttgaaccaaa
1801 taaaatgctg tcaagagaaa actctccagt gca //

- LOCUS: AF312679 1336 bp mRNA linear PRI 18-DEC-2000
- DEFINITION: Homo sapiens rhesus D category VI type IV protein (RHD) mRNA, complete cds.
- ACCESSION: AF312679
- VERSION: AF312679.1 GI:11878260

1 ggctggtgga acccctgcac agagacggac acaggatgag ctctaagtac ccgcggtctg
61 tccggcgctg cctgcccctc tgggccctaa cactggaagc agctctcatt ctcctcttct
121 atttttttac ccactatgac gcttccttag aggatcaaaa ggggctcgtg gcatcctatc
181 aagttggcca agatctgacc gtgatggcgg ccattggctt gggcttcctc acctcgagtt
241 tccggagaca cagctggagc agtgtggcct tcaacctctt catgctggcg cttggtgtgc
301 agtgggcaat cctgctggac ggcttcctga gccagttccc ttctgggaag gtggtcatca
361 cactgttcag tattcggctg gccaccatga gtgctatgtc ggtgctgatc tcagcgggtg
421 ctgtcttggg gaaggtcaac ttggcgcagt tggtggtgat ggtgctggtg gaggtgacag
481 ctttaggcac cctgaggatg gtcatcagta atatcttcaa cacagactac cacatgaacc
541 tgaggcactt ctacgtgttc gcagcctatt ttgggctgac tgtggcctgg tgcctgccaa
601 agcctctacc caagggaacg gaggataatg atcagagagc aacgataccc agtttgtctg
661 ccatgctggg cgccctcttc ttgtggatgt tctggccaag tgtcaactct gctctgctga
721 gaagtccaat ccaaaggaag aatgccatgt tcaacaccta ctatgctcta gcagtcagtg
781 tggtgacagc catctcaggg tcatccttgg ctcaccccca aaggaagatc agcatgactt
841 atgtgcacag tgcggtgttg gcaggaggcg tggctgtggg tacctcgtgt cacctgatcc
901 cttctccgtg gcttgccatg gtgctgggtc ttgtggctgg gctgatctcc gtcgggggag
961 ccaagtacct gccggggtgt tgtaaccgag tgctggggat tccccacagc tccatcatgg
1021 gctacaactt cagcttgctg ggtctgcttg gagagatcat ctacattgtg ctgctggtgc
1081 ttgataccgt cggagccggc aatggcatga ttggcttcca ggtcctcctc agcattgggg
1141 aactcagctt ggccatcgtg atagctctca cgtctggtct cctgacaggt ttgctcctaa
1201 atcttaaaat atggaaagca cctcatgagg ctaaatattt tgatgaccaa gttttctgga
1261 agtttcctca tttggctgtt ggattttaag caaaagcatc caagaaaaac aaggcctgtt
1321 caaaaacaag acaact //

- LOCUS: NM_001794 3063 bp mRNA linear PRI 17-NOV-2006
- DEFINITION: Homo sapiens cadherin 4, type 1, R-cadherin (retinal) (CDH4), mRNA.
- ACCESSION: NM_001794 NM_024883 XM_372872
- VERSION: NM_001794.2 GI:14589892

1 cgccggcggg gaagatgacc gcgggcgccg gcgtgctcct tctgctgctc tcgctctccg
61 gcgcgctccg ggcccataat gaggatctta caactagaga gacctgcaag gctgggttct
121 ctgaagatga ttacacggca ttaatctccc aaaatattct agaaggggaa aagctacttc
181 aagtcaagtt cagcagctgt gtggggacca aggggacaca atatgagacc aacagcatgg
241 acttcaaagt tggggcagat gggacagtct tcgccacccg ggagctgcag gtcccctccg
301 agcaggtggc gttcacggtg actgcatggg acagccagac agcagagaaa tgggacgccg
361 tggtgcggtt gctggtggcc cagacctcgt ccccgcactc tggacacaag ccgcagaaag
421 gaaagaaggt cgtggctctg gacccctctc cgcctccgaa ggacaccctg ctgccgtggc
481 cccagcacca gaacgccaac gggctgaggc ggcgcaaacg ggactgggtc atcccgccca
541 tcaacgtgcc cgagaactcg cgcgggccct tcccgcagca gctcgtgagg atccggtccg
601 acaaagacaa tgacatcccc atccggtaca gcatcacggg agtgggcgcc gaccagcccc

661 ccatggaggt cttcagcatt gactccatgt ccggccggat gtacgtcaca aggcccatgg
721 accgggagga gcacgcctct taccacctcc gagcccacgc tgtggacatg aatggcaaca
781 aggtggagaa ccccatcgac ctgtacatct acgtcatcga catgaatgac aaccgccctg
841 agttcatcaa ccaggtctac aacggctccg tggacgaggg ctccaagcca ggcacctacg
901 tgatgaccgt cacggccaac gatgctgacg acagcaccac ggccaacggg atggtgcggt
961 accggatcgt gacccagacc ccacagagcc cgtcccagaa tatgttcacc atcaacagcg
1021 agactggaga tatcgtcaca gtggcggctg gcctggaccg agagaaagtt cagcagtaca
1081 cagtcatcgt tcaggccaca gatatggaag gaaatctcaa ctatggcctc tcaaacacag
1141 ccacagccat catcacggtg acagatgtga atgacaaccc gccagaattt accgccagca
1201 cgtttgcagg ggaggtcccc gaaaaccgcg tggagaccgt ggtcgcaaac ctcacggtga
1261 tggaccgaga tcagccccac tctccaaact ggaatgccgt ttaccgcatc atcagtgggg
1321 atccatccgg gcacttcagc gtccgcacag accccgtaac caacgagggc atggtcaccg
1381 tggtgaaggc agtcgactac gagctcaaca gagctttcat gctgacagtg atggtgtcca
1441 accaggcgcc cctggccagc ggaatccaga tgtccttcca gtccacggca gggtgacca
1501 tctccatcat ggacatcaac gaggctccct acttcccctc aaaccacaag ctgatccgcc
1561 tggaggaggg cgtgcccccc ggcaccgtgc tgaccacgtt ttcagctgtg gaccctgacc
1621 ggttcatgca gcaggctgtg agatactcaa agctgtcaga cccagcgagc tggctgcaca
1681 tcaatgccac caacggccag atcaccacgg cggcagtgct ggaccgtgag tccctctaca
1741 ccaaaaacaa cgtctacgag gccaccttcc tggcagctga caatgggata cccccggcca
1801 gcggcaccgg gaccctccag atctatctca ttgacatcaa cgacaacgcc cctgagctgc
1861 tgcccaagga ggcgcagatc tgcgagaagc ccaacctgaa cgccatcaac atcacggcgg
1921 ccgacgctga cgtcgacccc aacatcggcc cctacgtctt cgagctgccc tttgtcccgg
1981 cggccgtgcg gaagaactgg accatcaccc gcctgaacgg tgactatgcc caactcagct
2041 tgcgcatcct gtacctggag gccgggatgt atgacgtccc catcatcgtc acagactctg
2101 gaaaccctcc cctgtccaac acgtccatca tcaaagtcaa ggtgtgccca tgtgatgaca
2161 acggggactg caccaccatt ggcgcagtgg cagcggctgg tctgggcacc ggtgccatcg
2221 tggccatcct catctgcatc ctcatcctgc tgaccatggt cctgctgttt gtcatgtgga
2281 tgaagcggcg agagaaggag cgccacacga agcagctgct cattgacccc gaggacgacg
2341 tccgcgacaa catcctcaag tatgacgagg aaggcggtgg cgaggaggac caggactacg
2401 acctcagcca gctgcagcag ccggaagcca tggggcacgt gccaagcaaa gcccctggcg
2461 tgcgtcgcgt ggatgagcgg ccggtgggcg ctgagcccca gtacccgatc aggcccatgg
2521 tgccgcaccc aggcgacatc ggtgacttca tcaatgaggg actccgcgct gctgacaacg
2581 accccacggc acccccctat gactccctgc tggtcttcga ctacgagggg agcggctcca
2641 ccgcaggctc cgtcagctcc ctgaactcat ccagttccgg ggaccaagac tacgattacc
2701 tcaacgactg ggggcccaga ttcaagaagc tggcggacat gtatggaggt ggtgaagagg
2761 attgactgac ctcgcatctt cggaccgaag tgagagccgt gctcggacgc cggaggagca
2821 ggactgagca gaggcggccg gtcttcccga ctccctgcgg ctgtgtcctt agtgctgtta
2881 ggaggccccc caatccccac gttgagctgt ctagcatgag cacccacccc cacagcgccc
2941 tgcacccggc cgctgcccag caccgcgctg gctggcactg aaggacagca agaggcactc
3001 tgtcttcact tgaatttcct agaacagaag cactgttttt aaaaaaaaaa aaaaaaaaag
3061 aag//

- LOCUS: NM_006580 1494 bp mRNA linear PRI 25-MAR-2007
- DEFINITION: Homo sapiens claudin 16 (CLDN16), mRNA.
- ACCESSION: NM_006580
- VERSION ORIGIN: NM_006580.2 GI:21536296

1 ccccacccga aacacactca gcccttgcac tgacctgcct tctgattgga ggctggttgc
61 ttcggataat gacctccagg accccactgt tggttacagc ctgtttgtat tattcttact
121 gcaactcaag acacctgcag cagggcgtga gaaaaagtaa aagaccagta ttttcacatt
181 gccaggtacc agaaacacag aagactgaca cccgccactt aagtggggcc agggctggtg
241 tctgcccatg ttgccatcct gatgggctgc ttgccacaat gagggatctt cttcaataca
301 tcgcttgctt ctttgccttt ttctctgctg ggtttttgat tgtggccacc tggactgact
361 gttggatggt gaatgctgat gactctctgg aggtgagcac aaaatgccga ggcctctggt
421 gggaatgcgt cacaaatgct tttgatggga ttcgcacctg tgatgagtac gattccatac
481 ttgcggagca tcccttgaag ctggtggtaa ctcgagcgtt gatgattact gcagatattc
541 tagctgggtt tggatttctc accctgctcc ttggtcttga ctgcgtgaaa ttcctccctg
601 atgagccgta cattaaagtc cgcatctgct ttgttgctgg agccacgtta ctaatagcag
661 gtaccccagg aatcattggc tctgtgtggt atgctgttga tgtgtatgtg gaacgttcta
721 ctttggtttt gcacaatata tttcttggta tccaatataa atttggttgg tcctgttggc
781 tcggaatggc tgggtctctg ggttgctttt tggctggagc tgttctcacc tgctgcttat
841 atctttttaa agatgttgga cctgagagaa actatcctta ttccttgagg aaagcctatt
901 cagccgcggg tgtttccatg gccaagtcat actcagcccc tcgcacagag acggccaaaa

961 tgtatgctgt agacacaagg gtgtaaaatg cacgtttcag ggtgtgtttg catatgattt
1021 aatcaatcag tatggttaca ttgataaaat agtaagtcaa tccaggaaca gttatttaga
1081 attcatattg aattaaatta attgctagct taatcaaaat gtttgattct cctatacttt
1141 ttctttctat tactcttata ttttcccgtc attctctctg ctaaccttcc accttatgca
1201 cacactttcc ctatatttta agataagtct gctaggatgt agaaatattt gtttgtgatt
1261 tctatatagc tattagagat tatgacatag taatattaaa atgaaatgat acttaaacag
1321 aaagcaattt ccaaagaggc cagggaccct aatctttgaa gagatgaaga aacttacttt
1381 tctccctggc ttttggttca ctttttgtac ttttaacaag tgggtgaatt atttgataat
1441 tttgaggaag attattcttt taaattcaaa ctagtatgtc aatgcctacc atta

- LOCUS: NM_030968 2923 bp mRNA linear PRI 17-NOV-2006
- DEFINITION: Homo sapiens C1q and tumor necrosis factor related protein 1(C1QTNF1), transcript variant 1, mRNA.
- ACCESSION: NM_030968
- VERSION: NM_030968.2 GI:38372915

1 acatctggct gggcttcgct ccttgcgcgt ctgtcccact ttctccctct cttcctttac
61 tttcgagaaa ccgcgcttcc gcttctggtc gcagagacct cggagaccgc gccggggaga
121 cggaggtgct gtgggtgggg gggacctgtg gctgctcgta ccgcccccca ccctcctctt
181 ctgcactgcc gtcctccgga agaccttttc ccctgctctg tttccttcac cgagtctgtg
241 catcgccccg gacctggccg ggaggaggct tggccggcgg gagatgctct aggggcggcg
301 cgggaggagc ggccggcggg acggagggcc cggcaggaag atgggctccc gtggacaggg
361 actcttgctg gcgtactgcc tgctccttgc ctttgcctct ggcctggtcc tgagtcgtgt
421 gccccatgtc cagggggaac agcaggagtg ggaggggact gaggagctgc cgtcgcctcc
481 ggaccatgcc gagagggctg aagaacaaca tgaaaaatac aggcccagtc aggaccaggg
541 gctccctgct tcccggtgct tgcgctgctg tgaccccggt acctccatgt acccggcgac
601 cgccgtgccc cagatcaaca tcactatctt gaaaggggag aagggtgacc gcggagatcg
661 aggcctccaa gggaaatatg gcaaaacagg ctcagcaggg gccaggggcc acactggacc
721 caaagggcag aagggctcca tgggggcccc tggggagcgg tgcaagagcc actacgccgc
781 cttttcggtg ggccggaaga agcccatgca cagcaaccac tactaccaga cggtgatctt
841 cgacacggag ttcgtgaacc tctacgacca cttcaacatg ttcaccggca agttctactg
901 ctacgtgccc ggcctctact tcttcagcct caacgtgcac acctggaacc agaaggagac
961 ctacctgcac atcatgaaga acgaggagga ggtggtgatc ttgttcgcgc aggtgggcga
1021 ccgcagcatc atgcaaagcc agagcctgat gctggagctg cgagagcagg accaggtgtg
1081 ggtacgcctc tacaagggcg aacgtgagaa cgccatcttc agcgaggagc tggacaccta
1141 catcaccttc agtggctacc tggtcaagca cgccaccgag ccctagctgg ccggccacct
1201 cctttcctct cgccaccttc cacccctgcg ctgtgctgac cccaccgcct cttccccgat
1261 ccctggactc cgactccctg gctttggcat tcagtgagac gccctgcaca cacagaaagc
1321 caaagcgatc ggtgctccca gatcccgcag cctctggaga gagctgacgg cagatgaaat
1381 caccagggcg gggcacccgc gagaaccctc tgggaccttc cgcggccctc tctgcacaca
1441 tcctcaagtg accccgcacg gcgagacgcg ggtggcggca gggcgtccca gggtgcggca
1501 ccgcggctcc agtccttgga aataattagg caaattctaa aggtctcaaa aggagcaaag
1561 taaaccgtgg aggacaaaga aaagggttgt tatttttgtc tttccagcca gcctgctggc
1621 tcccaagaga gaggcctttt cagttgagac tctgcttaag agaagatcca aagttaaagc
1681 tctggggtca ggggaggggc cgggggcagg aaactacctc tggcttaatt cttttaagcc
1741 acgtaggaac tttcttgagg gataggtgga ccctgacatc cctgtggcct tgcccaaggg
1801 ctctgctggt ctttctgagt cacagctgcg aggtgatggg ggctggggcc ccaggcgtca
1861 gcctcccaga gggacagctg agccccctgc cttggctcca ggttggtaga agcagccgaa
1921 gggctcctga cagtggccag ggacccctgg gtcccccagg cctgcagatg tttctatgag
1981 gggcagagct cctggtacat ccatgtgtgg ctctgctcca cccctgtgcc accccagagc
2041 cctggggggt ggtctccatg cctgccaccc tggcatcggc tttctgtgcc gcctcccaca
2101 caaatcagcc ccagaaggcc ccggggcctt ggcttctgtt ttttataaaa cacctcaagc
2161 agcactgcag tctcccatct cctcgtgggc taagcatcac cgcttccacg tgtgttgtgt
2221 tggttggcag caaggctgat ccagacccct tctgccccca ctgccctcat ccaggcctct
2281 gaccagtagc ctgagagggg ctttttctag gcttcagagc aggggagagc tggaagggc
2341 tagaaagctc ccgcttgtct gtttctcagg ctcctgtgag cctcagtcct gagaccagag
2401 tcaagaggaa gtacacgtcc caatcacccg tgtcaggatt cactctcagg agctgggtgg
2461 caggagaggc aatagcccct gtggcaattg caggaccagc tggagcaggg ttgcggtgtc
2521 tccacggtgc tctcgccctg cccatggcca ccccagactc tgatctccag gaaccccata
2581 gcccctctcc acctcacccc atgttgatgc ccagggtcac tcttgctacc cgctgggccc
2641 ccaaaccccc gctgcctctc ttccttcccc ccatccccca cctggttttg actaatcctg
2701 cttccctctc tgggcctggc tgccgggatc tggggtccct aagtccctct ctttaaagaa
2761 cttctgcggg tcagactctg aagccgagtt gctgtgggcg tgcccggaag cagagcgcca
2821 cactcgctgc ttaagctccc ccagctcttt ccagaaaaca ttaaactcag aattgtgttt

2881 tcagcaaaaa aaaaaaaaaa aaaaaaaaaa aaaaaaaaaa aaa//

- LOCUS: NM_012216 2523 bp mRNA linear PRI 17-NOV-2006
- DEFINITION: Homo sapiens midline 2 (MID2), transcript variant 1, mRNA.
- ACCESSION: NM_012216
- VERSION: NM_012216.2 GI:16445407

1 atcccggctg ctgcgcggcg tcggcgacgg tagcggcagc ggcggcgaag gcgggcggcg
61 gcctacagtg gtagcggcgg cggcggcgac cggggcccgg gagctcgcgc cggagcccga
121 gccaacccgc tgcggaggca gacgagagcc cagcgccctc gagcgagcgg aggagatggc
181 tggcacctgg gaacgctatg ggtgaaagcc cagcctccgt ggttcttaat gcctcaggag
241 gactattttc actaaagatg gaaacactgg agtctgaatt gacctgtcca atctgcctag
301 agttgtttga agaccccctt ctgctccctt gtgctcacag cctctgcttc agctgtgccc
361 atcgcatttt ggtatcaagc tgcagctctg gtgaatccat tgaacccatt actgctttcc
421 agtgtcctac ctgcaggtat gttatctcgc tgaaccaccg gggcctggat ggcctcaaga
481 ggaatgtgac tctgcagaac attattgatc gcttccagaa ggcttcagtc agtgggccca
541 attcccctag tgagagccgc cgggaaagga cttacaggcc caccactgcc atgtctagcg
601 agcgaattgc ttgccaattc tgtgagcagg acccgccaag ggatgcagta aaaacatgca
661 tcacctgtga ggtctcctac tgtgaccgtt gcctgcgggc cacgcacccc aacaagaaac
721 ctttcaccag ccaccgcctg gtggaaccag tgccagacac acatcttcga gggatcacct
781 gcctggacca tgagaatgag aaagtgaaca tgtactgtgt atctgatgac caattgatct
841 gtgccttatg caaactggtg ggtcgtcacc gagaccatca ggtcgcatcc ctgaatgatc
901 gatttgagaa actcaagcaa actctggaga tgaacctcac caacctggtt aagcgcaaca
961 gcgaactaga aaatcaaatg gccaaactaa tacagatctg ccagcaggtt gaggtgaata
1021 ctgctatgca tgaggcaaaa cttatggaag aatgtgacga gttggtagag atcatccagc
1081 agaggaagca aatgatcgct gtcaaaatca aagagacaaa ggttatgaaa ctgagaaagt
1141 tggcacagca ggttgctaat tgccgccagt gtcttgaacg gtcaacagtc ctcatcaacc
1201 aagctgagca tatcctgaaa gaaaatgacc aggcacggtt tctacagtct gcaaaaaata
1261 ttgctgagag ggtcgctatg gcaactgcat cttctcaagt tctgattcca gacatcaatt
1321 ttaatgatgc ctttgaaaac tttgctttag atttttccag agaaaagaaa ctgctagagg
1381 ggttagatta tttaacagcc ccaaacccac catctatccg agaagaactc tgtactgcct
1441 cccatgacac cattacagtc cactggatct cggatgatga gttcagcatc agctcctatg
1501 agcttcagta caccatattc actggccagg ctaacttcat cagtaagtca tggtgtagtt
1561 ggggcctgtg gccagagata aggaaatgta aggaagcagt aagctgctca agattggccg
1621 gggcgccacg aggcctgtat aattcagtag acagctggat gattgttccc aacattaaac
1681 agaaccatta cacagtgcat ggactccaga gcgggactcg ctacatcttc atcgttaaag
1741 ccataaacca agccggcagc cggaacagtg aacctacccg actaaaaaca aacagccaac
1801 cctttaaatt ggatcccaaa atgactcaca agaagttgaa gatctccaat gatggattgc
1861 agatggagaa ggatgaaagc tctctaaaga agagccacac cccagagagg tttagtggca
1921 cagggtgcta tggggcagca ggaaatatat tcattgacag tggctgccac tattgggagg
1981 tggtcatggg ttcctcaaca tggtatgcaa ttggcattgc ctacaaatca gctccaaaga
2041 atgaatggat tggcaagaat gcctcctcat gggtcttctc tcgctgcaat agtaacttcg
2101 tggtgagaca caacaacaag gaaatgctgg tggatgtgcc cccacacctg aagcgtctgg
2161 gtgtcctcct ggattatgac aacaatatgc tgtctttcta tgacccagct aactctctcc
2221 atcttcatac ttttgatgtg accttcattc ttccagtttg tccaacattt acaatctgga
2281 acaaatccct aatgatcctg tctggcttgc ctgccccaga ttttattgat taccctgagc
2341 ggcaggaatg caactgcagg cctcaagaat ccccttatgt ttctgggatg aaaacctgtc
2401 attaagtttc aggagagtat ataattcact ggctctctag ttcagcagtt cttcccctcc
2461 tacacctaag ttagcgttca atatacgaga cacaaaataa agtttgtttg aagcatccaa
2521 aat//

- LOCUS: NM_020129 794 bp mRNA linear PRI 18-NOV-2006
- DEFINITION: Homo sapiens lectin, galactoside-binding, soluble, 14 (LGALS14), transcript variant 1, mRNA.
- ACCESSION: NM_020129
- VERSION: NM_020129.2 GI:45439323

1 gctgcattac agacacagac ctgcaaacat ctatggttgt gacagagttt ctttctgaca
61 cctgagtctt tctcctgctg cacggaaagc ttgctgggag gggcttggaa tctggcatga
121 agccaaaggg catctctgag ttgcagcatt taaatgatcc cactcagaga ttcacacaga
181 agactggaca caattccgaa gagctgccca gaaggagaga acaatgtcat cactacccgt
241 accatacaca ctgcctgttt ccttgcctgt tggttcgtgc gtgataatca cagggacacc
301 gatcctcact tttgtcaagg acccacagct ggaggtgaat ttctacactg ggatggatga
361 ggactcagat attgctttcc aattccgact gcactttggt catcctgcaa tcatgaacag
421 ttgtgtgttt ggcatatgga gatatgagga gaaatgctac tatttaccct ttgaagatgg

481 caaaccattt gagctgtgca tctatgtgcg tcacaaggaa tacaaggtaa tggtaaatgg
541 ccaacgcatt tacaactttg cccatcgatt cccgccagca tctgtgaaga tgctgcaagt
601 cttcagagat atctccctga ccagagtgct tatcagcgat tgagggagat gatcagactc
661 ctcattgttg aggaatccct ctttctacct gaccatggga ttcccagagc ctactaacag
721 aataatccct cctcacccct tcccctacac ttgatcatta aaacagcacc aaacttcaaa
781 aaaaaaaaaa aaaa //

- LOCUS: NM_001939 3499 bp mRNA linear PRI 17-NOV-2006
- DEFINITION: Homo sapiens dystrophin related protein 2 (DRP2), mRNA.
- ACCESSION: NM_001939
- VERSION: NM_001939.1 GI:4503392

1 gggaagtggg agagacagag ggagagtgtg catacagcag ctgtcgggca tccctgtctc
61 agggacttct tcgctgattc acagaggcag cccagccccg gcctcccagc ttacactgcc
121 gcctgcttct cagaacctga cggaatcaga agctacatga tattcgccgt gtgagtggct
181 atcgtaatag ataacatttg aatatctact aaatgccaga aactatgaat taactcctct
241 caacaaagct aagaggtgct tgatgatcaa tagttggtgc actgcctatc ctcatccccc
301 ccatgagcct tggtttttat gcaacctatg gtcatgcagg gatgccctta caccctccca
361 cgatgtcatg actggcaggc agctgaccag ttccatcata gcagcagcct ccgaagcacc
421 tgcccccacc ctcaggttag agctgctgtc accagccctg cacctcctca agatggtgct
481 ggggttccct gcctaagcct aaagctgttg aacgggtctg ttggtgcctc tggacccctg
541 gaaccaccag ccatgaatct gtgttggaat gaaataaaaa agaagtctca caacctccgc
601 gctcgcctag aggccttctc agaccacagt ggaaagcttc agctccctct tcaagagatt
661 attgactggc tcagccaaaa ggatgaggag ttgtcagctc agctgcccct acagggggat
721 gtggccctgg tgcaacagga gaaggagaca catgcggcct ttatggaaga agtcaagtct
781 cgggccccct acatctattc tgtgctggag tcagctcagg ccttcctgtc ccagcaccca
841 tttgaggagt tagaggagcc tcattctgag agcaaagata cctccccgaa acagcggatc
901 cagaatctca gccgctttgt atggaagcag gcgacggtgg ccagtgaact gtgggagaag
961 ttgacagccc gctgtgtgga ccagcaccgt cacattgagc ggactctgga gcagctcttg
1021 gagattcaag gggcaatgga ggaactaagc actactctaa gccaagctga gggagtccga
1081 gccacttggg agcccattgg ggatctcttc attgattcac tcccagagca catccaggct
1141 attaagctgt tcaaagaaga attctccccc atgaaagatg gagtaaagtt ggtgaatgat
1201 ctggcccacc aacttgccat ttctgatgtg cacttgtcaa tggagaattc ccaggccctg
1261 gaacagatca acgtccgatg gaaacaacta caggcgtcag ttgatgagag gcttaagcag
1321 ctccaggatg cccaccggga ctttgggcct gggtcacagc actttctctc ctcctctgtc
1381 caggttccct gggaaagagc aatttcaccc aataaagttc cctactacat caaccaccag
1441 gctcagacca catgctggga ccatcccaag atgacagagt tataccaaac cctagctgat
1501 ctgaacaaca ttaagttctc agcttatcgc actgccatga aactccgcag agtccagaaa
1561 gccctgcgct tggacctggt aactttaacc acagccctgg aaatcttcaa tgagcatgat
1621 ctgcaggcca gtgagcacgt gatggatgtg gtagaggtca ttcactgcct gactgcctta
1681 tatgaacgtt tggaggagga aagaggcatc ctggtcaatg tgccactctg tgtggacatg
1741 agcctcaatt ggctcctcaa tgtttttgat agtggtcgca gcggaaagat gcgggcattg
1801 tcttttaaga ctggcattgc atgcttgtgt ggcacggaag tgaaggaaaa acttcagtac
1861 ctcttcagcc aagtggccaa ctcaggcagc cagtgtgacc agcgccacct tggtgtcctg
1921 cttcatgagg ccattcaggt gccccgtcag ctgggtgaag tggcagcctt tgggggcagc
1981 aatgtggagc ccagtgtccg tagttgcttc cgttttagca ccgggaagcc agtcattgaa
2041 gcatcccagt tcctggagtg ggtcaacctg gagccccagt ccatggtgtg gctgcctgtt
2101 ctgcatcggg taaccattgc tgagcaagtg aagcatcaga ccaagtgctc tatctgtagg
2161 cagtgcccca tcaaggggtt caggtaccgg agtctgaagc aattcaacgt tgacatctgc
2221 cagacctgct tcttgacagg cagggccagc aaaggcaata agctgcacta ccccatcatg
2281 gagtattaca caccgaccac atccagtgag aacatgaggg actttgccac aaccttaaag
2341 aacaaattcc gctccaagca ttatttcagc aaacaccctc agcgaggtta tctgcctgtg
2401 caatcagtgc tggaggctga ctacagtgag acgccggctt cttccccgat gtggccacac
2461 gccgacacac actcccgaat tgagcatttt gcgagcaggc ttgctgagat ggaaagtcaa
2521 aattgctcct tctttaatga cagcttgtcc ccagatgaca gcatagacga ggaccagtac
2581 ctgctgcggc actccagccc catcacagac cgggagccag cctttggaca gcaggctcca
2641 tgcagtgtgg ctacagaaag caaaggggag ctacagaaga tcctggccca cttggaagat
2701 gagaaccgga ttctccaggg agagctgagg cgcctgaagt ggcagcatga ggaggcagct
2761 gaggcaccca gtctggctga cggctccact gaggcagcaa cagaccaccg caatgaggag
2821 cttctggccg aggcccgtat ccttcggcaa cataagagcc gcctggagac gcgcatgcag
2881 atcctcgaag atcacaacaa gcagctagag tcccagctgc agcgtctgag ggagcttctc
2941 ctgcagccac ccaccgaatc agatggcagt ggctctgcag gctcgtccct agcttcctct
3001 ccacagcagt cagaaggcag tcacccccgg gagaagggac agaccactcc agataccgag
3061 gctgcagatg atgtggggtc aaagagccag gatgtcagcc tgtgcttgga ggacatcatg

3121 gagaaactcc gtcatgcctt ccccagtgtg cgaagttctg atgtgactgc caacaccctg
3181 ctggcctctt gatggagcca gatccccatc ctatagttca tagtcctctc ctggttccgg
3241 tcaaagcctt tcctcagcct tcacccaacc tttccagttt ccactggccc cacattcctc
3301 aactagtatt atttgggctc tgggcagcag cagggatctg gtggtatgtg aggtgcatgc
3361 gggcagtgat gggagaaggg gaggcatgat tcttctgacc ctagaaatgt tccctttaat
3421 cttcaagttc gagatcagcc ctttaagtac ctttctgttg cagcccaggc aaatatcact
3481 tggccattca gatggggag //

- LOCUS: AF152503 2553 bp mRNA linear PRI 14-JUL-1999
- DEFINITION: Homo sapiens protocadherin gamma A10 short form protein (PCDH-gamma-A10) variable region sequence, complete cds.
- ACCESSION: AF152503
- VERSION: AF152503.1 GI:5457054

1 atggccgctc aaaggaatcg ctcaaaggaa tcaaaggatt gcagcgggct ggtcctgctc
61 tgccttttct tcgggattcc atgggaggct ggagcccggc agatctccta ctcaattcct
121 gaggaattag agaaaggctc tttcgtgggc aacatctcca aggacttggg tctggcgccc
181 cgggagctgg cggagcgcgg agtccgcata gtctccagag gtaggacgca gcttttctct
241 ctgaacccgc gcagcggcag cttgatcacc gcgggcagga tagaccggga ggagctctgc
301 gctcagagcg cgcggtgcgt ggtgagtttt aatatccttg tggaagacag ggtgaaactt
361 tttgggatag aaatagaagt aactgatatc aatgacaatg ctccaaaatt ccaagcagaa
421 aatctagacg taaaaattaa tgaaaatgtc gctgcgggaa tgcgttttcc tctcccggaa
481 gctattgatc cggatgtggg cgtgaactcc ctgcagagct atcagctcag ccccaataag
541 cacttctccc taagagttca gagccgtgcc aatggcgtca agtacccgga gctggtactg
601 gagcactccc tagatcgcga ggaagaggcc attcaccacc tggtcctcac cgcctccgac
661 gggggtgacc ctctccgatc tggcactgtc cttgtcagtg tgactgtctt cgatgcaaat
721 gacaacgcgc cggtcttcac cttgccagaa taccgagtga gtgttcctga gaatttgcct
781 gtgggcactc agctgctgac agtcacagcc accgacaggg acgaaggtgc caatggagaa
841 gtgacatatt cattccgaaa attacctgac acgcaattgt tgaagttcca actaaacaaa
901 tatactggag aaataaaaat atcagaaaat ctagattatg aagaaaccgg tttctatgaa
961 atagaaatac aagcagaaga tggaggagca tatcttgcaa ctgcaaaagt gttgattaca
1021 gtagaagatg taaatgacaa cagtccagag ctgaccatca cgtctctatt tagtccagtg
1081 actgaagatt cacctctggg aacagtcgta gcccttttaa atgtgcatga tttagactct
1141 gagcagaatg gacaggtaac ctgttccatt ttggcgtatc taccatttaa attagaaaag
1201 tccattgaca gttattacag attggtgata cacagagccc ttgacaggga acaggtatcc
1261 tcttacaata tcacagtgac agccacagat gggggaagtc ctcctctatc aacggaagct
1321 cactttatgc tacaagtggc agatatcaat gacaacccac ctaccttctc tcaagtctcc
1381 tactttacct atatcccaga gaacaacgcc aggggtgcct ccatcttctc agtgacagcg
1441 ctggacccgg acagcaaaga gaatgcccag attatttact ccctggctga agacaccatc
1501 cagggggtac ctctgtcctc atacatatcc atcaactcag acactggcgt cctgtatgca
1561 ctcagatcct tcgactatga gcagtttcat gagctacaga tgcaggtgac agccagcgac
1621 agcggggatc ctccactcag cagcaacgtg tcgttgagcc tgtttgtgct ggaccagaac
1681 gacaatgcgc ccgagatcct gtaccccgcc ctccccacag acggttccac aggcgtggag
1741 ctggcgcccc gctccgcaga gcccggctac ctggtgacca aggtggtggc ggtggacaga
1801 gactccggcc agaacgcctg gctgtcctac cgtctgctca aggccagcga gccgggactc
1861 ttcgcggtgg gggagcacac gggcgaggtg cgcacggcgc gagccctgct ggacagagac
1921 gcgctcaagc aaagcctcgt agtggccgtc caggaccacg gccagccccc tctctccgcc
1981 actgtcacgc tcaccgtggc cgtggccgac agcatccccc aagtcctggc ggacctcggc
2041 agcttcgagt ctccagctaa ctctgaaacc tcagacctca ctctgtacct ggtggtagcg
2101 gtggccgcgg tctcctgcgt cttcctggcc ttcgtcatcg tgctgctggc gcacaggctg
2161 cggcgctggc acaagtcacg cctgctgcag gcttcaggag gcggcttgac aggtgtgtcc
2221 ggctcgcact ttgtgggcgt ggacggggtt cgggctttcc tgcagaccta ttcccacgag
2281 gtctctctca ccgcggactc gcgaaagagt cacctgatct tcccccagcc caattatgcg
2341 gacacgctca tcagccagga gagctgtgag aaaaacgatc ctttgtcttt gttagatgat
2401 tcgaagtttc ctatagagga taccccattg gttccagtga gttttatttt cattttact
2461 tttgttaaaa aaaaaaagat tggtttttac tttgaagttt gcggcatgat ggtggaaagt
2521 gtaaatgcta aaacactgat gagtagaatt tga //

- LOCUS: NM_001232 2682 bp mRNA linear PRI 14-DEC-2006
- DEFINITION: Homo sapiens calsequestrin 2 (cardiac muscle) (CASQ2), mRNA.
- ACCESSION: NM_001232
- VERSION: NM_001232.2 GI:119395726

1 ctcatgcgtg cgtgcgtgcg tgcgtgtgtg tgtgtgtgtg cctctgctct ttgtcctgag

61 cccacgattc cagagctggc tggacccaag gaggtgaaga gtcacttttc agccccagga
121 agggcaaaga agagagaaaa tcagcctgtc tgctctctcc ttggctcaac aaggcctcta
181 acagtcttct gtcctctatt ctgcacacgg catatttggg aacgagaaac aaaagttttc
241 ccaaatgaag agaactcact tgtttattgt ggggatttat tttctgtcct cttgcagggc
301 agaagagggg cttaatttcc ccacatatga tgggaaggac cgagtggtaa gtctttccga
361 gaagaacttc aagcaggttt taaagaaata tgacttgctt tgcctctact accatgagcc
421 ggtgtcttca gataaggtca cgcaaaaaca gttccaactg aaagaaatcg tgcttgagct
481 tgtggcccag gtccttgaac ataaagctat aggctttgtg atggtggatg ccaagaaaga
541 agccaagctt gccaagaaac tgggttttga tgaagaagga agcctgtata ttcttaaggg
601 tgatcgcaca atagagtttg atggcgagtt tgcagctgat gtcttggtgg agttcctctt
661 ggatctaatt gaagacccag tggagatcat cagcagcaaa ctggaagtcc aagccttcga
721 acgcattgaa gactacatca aactcattgg ctttttcaag agtgaggact cagaatacta
781 caaggctttt gaagaagcag ctgaacactt ccagccttac atcaaattct ttgccacctt
841 tgacaaaggg gttgcaaaga aattatcttt gaagatgaat gaggttgact tctatgagcc
901 atttatggat gagcccattg ccatccccaa caaaccttac acagaagagg agctggtgga
961 gtttgtgaag gaacaccaaa gacccactct acgtcgcctg cgcccagaag aaatgtttga
1021 aacatgggaa gatgatttga atgggatcca cattgtggcc tttgcagaga agagtgatcc
1081 agatggctac gaattcctgg agatcctgaa acaggttgcc cgggacaata ctgacaaccc
1141 cgatctgagc atcctgtgga tcgacccgga cgactttcct ctgctcgttg cctactggga
1201 gaagactttc aagattgacc tattcaggcc acagattggg gtggtgaatg tcacagatgc
1261 tgacagtgtc tggatggaga ttccagatga tgacgatctt ccaactgctg aggagctgga
1321 ggactggatt gaggatgtgc tttctggaaa gataaacact gaagatgatg atgaagatga
1381 tgatgatgat gataattctg atgaagagga taatgatgac agtgatgacg atgatgatga
1441 atagcccaac tccaaacaat tctgatgaaa acaaaatcac agcacccact accatacaga
1501 cagcacaagg tggcagcaag caattctgcc ccacacccag ccagctcctt tccctttcc
1561 atcatctctt ttcccactcc ctttgcgtca ggagcagcat cattcagcaa atgccttttc
1621 aaatgcagca atcccactta gcagggacag gagaaaaatt attcccatgt tgactgtctt
1681 gactgtcacg gaacagatct tgttctttgc tggaccatca agggtcatgg cagtgcctga
1741 acatggcagt ctagggtgaa caatccccta acacaagttt acttgtcttt gattatgaca
1801 gtaacaaaat tgacagcttt ctaactcaca ggcatagagt gaccttttaa tcagagccca
1861 gggaagacac atgattaatg atttagctcc ctccatacct cgaacatcag ttgggatccc
1921 tcctccagcc aagatgatcc ttcttagaga aggctcagcc ttggaagcaa acttataaat
1981 catattctca tggctttgtt aaacttattt caagtgatgg tcattcatat cactatgaac
2041 ttggatattc aagcctttgg atggctatgg agagggcttg aaatgtgtac aggtgtcacc
2101 atcatttcta gtatattagg aaactgggat gggaggttga tttgctctct aaacttccct
2161 ctagttggca agtctcacat attcatcagc aggagtggag ggtgggggaa aactagaaag
2221 atgaaaactt ttacattttt ctgatgggtt catgtctctg attgggtcag ctggcttcct
2281 agcctaagct gggatctgaa taccccttct ctgtagctgc tagtgagcct tcccatttag
2341 attaaagatt gctttatcca gcagtcaatt aactctccag ttatcagtac tcccacaatt
2401 ggccagggca acaataattg gagttcatac tgatgccctg aggcactgaa aaaaaaaaaa
2461 atcccaaagt gccttctgag ctgtctaaaa gttacattgt gcttggtaga tttagtgtta
2521 agtgtgcagt ataatttctt aatttatttt ctcaatcttt tagcacatgt gtaagacact
2581 gtgcaaattt tttgaaaata gagcaatact ttttgtggaa tactagctaa ctaattctgt
2641 cattaaactc atattttgaa aatattcaga caatgttgaa aa//

- LOCUS: NM_000337 8052 bp mRNA linear PRI 18-NOV-2006
- DEFINITION: Homo sapiens sarcoglycan, delta (35kDa dystrophin- Associated glycoprotein) (SGCD), transcript variant 1, mRNA.
- ACCESSION: NM_000337
- VERSION: NM_000337.4 GI:46249398

1 agagaggact tatctccagc atctagcact acagagcaga ggctgtgtgg agaatggctg
61 aaaaatcaga attggttgta gaagcagttt tctttctggt tgtgagtatg agcccggcag
121 acaccatgag cgctgttgca ggggagtcgg cctgtgcttg acacatgtgt ttcccattga
181 tagctggaga cagcccagta gctgtgagtc ggtctgacaa agccatattg aagtacggag
241 tacggtttca aagcagtcag aaaaagaacg ggaatgctgt tcaggaaatt cttcaggcat
301 gggcagggac ttggctgcag ttctgcagtt ggaaaatctg actggggcag cttctgagcg
361 caggctgggc ctgcacacac tcagcgggcc gagtggccac ctccttcaga gctgctcagc
421 acgccctggg atcgcgggcg gttttcatcg gccggtttgt gaaacggaca agagagagac
481 attactgccg ggagtgttga gtgaagggac caggtggaga tgatgcctca ggagcagtac
541 actcaccacc ggagcaccat gcctggctct gtggggccac aggtatacaa ggtggggatt
601 tatggctggc ggaaacgatg cctgtatttc tttgtcctgc tcctcatgat tttaatactg
661 gtgaacttgg ccatgaccat ctggattctc aaagtcatga acttcacaat tgatggaatg

721 ggaaacctga ggatcacaga aaaaggtcta aagctagaag gagactctga attcttacaa
781 cctctctacg ccaaagaaat ccagtcccga ccaggtaatg ccctgtactt caagtctgcc
841 agaaatgtta cagtgaacat tctcaatgac cagactaaag tgctaactca gcttataaca
901 ggtccaaaag ccgtagaagc ttatggtaaa aaatttgagg taaaaactgt ttctggaaaa
961 ttgctcttct ctgcagacaa taatgaagtg gtagtaggag ctgaaagatt acgagtttta
1021 ggagcggagg gcacagtgtt ccctaaatct atagaaacac ctaatgtcag ggcagacccc
1081 ttcaaagaac taaggttgga gtccccaacc cggtctctag tgatggaggc cccaaaagga
1141 gtggaaatca atgcagaagc tggcaatatg gaagccacct gcaggacaga gctgagactg
1201 gaatccaaag atggagagat taagttagat gctgcgaaaa tcaggctacc tagactgcct
1261 catggatcct acacgcctac aggaacgagg cagaaggtct tcgagatctg cgtctgcgcc
1321 aatggagagt tattcctgtc tcaggcagga gctgggtcca cttgtcagat aaacacaagt
1381 gtctgcctct gaaagactat ccatagtgga cattgttggc agcataaagg ccttttttgg
1441 ctttagacac tggctgccag ctatttttac tagaacacag aaagcctatc aaagaccttg
1501 tgtgtatgtg tacgtgtgtg tgcgtgcttg agtgtgttcg cgtgtgtggg tggatataaa
1561 tatatataaa tatatataaa taaatatata tatcctctgt ataaaatgag gtttcagtac
1621 aaaaggaacc atgggtgacc ctgcgatatc cactgtcatt ttccatccca tccccaccac
1681 ctgagtgaca gaaatctaaa cacacatccg tcccaacatt ccccagacca ttcagaatca
1741 cacagcgtat taaacactga cagaatcttc atctagatat tcgagtagca gcatatcttc
1801 tcttttagtg tcattacgag ggagtgatgg cgggaatctc agtcgcactc aagctctgag
1861 acctttgtat caaaaatagg catttgattt cctgttttag ctttagtaag gctggctaac
1921 ttccccctct tcaagctagg aactggcaat gctgtagaag tcagccgtag gaattcaaaa
1981 tggctggcct accttggcta ccagacatat tggggttttt gtagttgaat gaatgaggag
2041 gatgaatttc agcaaatttt gaactgctca cccaacttct gctatcttgc tccctccaaa
2101 ctcacagatt ctcctacagt caaattagga gctgtaaatc agcacaaaat aagataacag
2161 ctgttcctca gtgagctgga agctacttaa tggcctgatg ggcaatgaac aaacgggtga
2221 tatgtctctg tttaagggaa aaatggctta aaagctgttc tgttctcact tctgacttta
2281 accaaaagat ttcaacccac aatgatcagg tcaatcaaaa tccctaagag cagaactcct
2341 accccaaaag aagcctggaa gtctaattaa gagtagcata aggaacctaa ttatgtttac
2401 catgtttctt gggattggtg ggaaatgtca aaacatgccc tttattttta aaggcattca
2461 caaactctct gactttgttc ttcttatata ttttttcagt gccgggatat tcatattcct
2521 aaagccacta ttgtgttttc tctaagaagc actacatgcc accagaattg tgcactgaaa
2581 gataatacaa actgagtgtc tttatgagaa tcactgtgtc ccctgaggcc cagcagtacc
2641 tgcttccctg tatgtggaag cagcacctca ttcccgccat cagctacctc tcatcacccc
2701 accttcatca tcatgctcca gggtcaccct ggccagctct tgtgctggga caggggatta
2761 caccatctct gttcaaagag ggggaaatgt gcctatgcct taagtcaatg cactcagcaa
2821 ggagaagcac atcttattta tcttgttacc tatagtttac tttgggtgat tggaggggaa
2881 tgacttagtt atgactggac atcttaaaag ctgatagaca agccaaatgg ctggcagatg
2941 atgtggattt caaagagccc agaatgaact catcactggc ttagacagtc ctggatgcca
3001 tttggaaagt agtggccctg caagcctaaa tgaagtagta tttgtaggcc tgggtggcat
3061 ttggattttt cttttccctc aacaaggttt tactttttct tactttacaa gcaagggaag
3121 ttttgtgata ggagagaaat aaaagatttg atattttttg agatgacact caagcatcag
3181 gctgagattt gcacacatgg gatgtaaaag caagctgtgt gttgcttagt cacttactta
3241 gaagtagatg gtgggggaca gcggcgtggg tcctagcctg gccagtgatg ctgctggcgt
3301 ccagacccca gactcactcc aagcactctt gttcaatatc tcatgcagaa gagttgggct
3361 ggtcactctt aggggtgaga ccccgtgatt ggttggtttg tagcactaag gtctaaaaag
3421 gaaaaccata aaagacatca gattacgctg gatcagtata attaatattc catagggcca
3481 tgttgccaga atctgtatgt atcaatacag ggtttttcca agccaggaaa cgccctcctt
3541 ggctactagg agcacctatc ccatatcatt cagataaaca atgatagcta caaagtcatt
3601 tgtggctaga taggttaaga caggtgattt tttaaagtag actgtctttg cattttgcca
3661 tctgaagttc attaatcttt agatgacaaa aaagcaaaaa gttcccagaa cgtttttgct
3721 tagattttgt tctaatcacc acgtgaagga atgagattag caccacaagt ttcatgccaa
3781 taaaagagac tggtgtgatc ccacatgcaa aatttaatcc taagggtagt gagatcacaa
3841 acagaataaa aataagagca atcaaccata taaatcaagt acctattggg aacagacata
3901 acattcaatt tttcatttat gctaagtgac cacagtatac aaagtaataa gcaggaaatt
3961 tgatatgggt taaattatgc attttgttca gattttggaa attggtatgc attataagtt
4021 tctcaatgta catcttttta tcccaacacc ctcaaactag aatatttgtc agtggtcaag
4081 agaaaaaatt ttacctgaat tcttgggggc cggcggggag cttttacatt aaaaatctac
4141 taacgcctac tttttaaaaa atgagattct ttctaatctt tatatatgac attttctaga
4201 caatcgcacc tttgggtata ttaaacagct ggtatcataa caaagaatcc aaatgaacct
4261 tcaatatact agaagttcta gtaggttaat attgttcaga agatttttac aaattaaaaa
4321 ctgatttcca aatatgttca acatttactt ctatttgata tctgctcaag aagtcataga
4381 agtcttggga aactattcga gtatcacaga ggttttcaaa agcccttatg gtgacatcta
4441 cctaggtaaa agcctgacat gtggctttat aattgttatg ttacccaagg gataaacttg

4501 aactggcttt gaacatcctt taggtcattt tctctttgga taattttcat cgcatatccg
4561 gcaactatag accaaagttt gcttaaggtt tgaccctaga gcagaggtgg gcaaactatg
4621 acctggaaac caaatctgac ttaccacctt ttcctgtagt taaagtttta ttgtcacata
4681 gccacataca ttcatttaca tgttttctga ttttacacta cagcagcagg atggagtagt
4741 tgtgtcagag actatgtatc ccacaaagca tagattactt actatttagt cctttacagg
4801 caaagtttcc tcacccctga cctagaggtt tttgtggtat gcattggata tagcaggaaa
4861 gaaagcacat ttccaaacag cagggagtaa gcttacattt ctgtgtaggt ttgggaatat
4921 agttacttgg caaagtcttt caggaaggaa gcccttcctt atgttacatg tggaaagcct
4981 gccttccaag acatgtggaa gtaattgatc cacctgccag agaaacacag gctcagagga
5041 tgcctgggaa cagggaggat gggattagtg gaagcttaat ggaaaaggaa agttatgatc
5101 ctccaagacc cttaattgat agaccatacc aggttctgca ggtcagcatc attgtgtaat
5161 gagagtgaag taggggaccc tgtggttcaa ccttagaatc tgtttcctgt aggctctttc
5221 tgctgtctat attcattaaa gttttccact tcaccctccc atagtctaga gggatgccca
5281 ttccatggtc ctccagagaa tagttttgac ttaacatgtc tgtttagccc acatcacgtc
5341 agttatcaac accgccactg tgcttactgt tcctacagcc acaccaggct tgaagagtta
5401 gtgagaccaa caaataattg gaagtattgg aaaaagcaaa atacatgggg acaaaaaaaa
5461 tacagtgaaa ttctttttat caaactgatg ctgtgagaaa ccagatgaat gccagtttgg
5521 ctttatttct aagaatctgg gtcttcattc tctggtgtag aaggaatgca aaaaactata
5581 acaacaacaa caaaaacata ttttgaaaag acatattctg acatctctgc ttgtgtgtgg
5641 taaggcaggt tcctatcaga catttatccc tttggtcaag atcccttttg ctcatccagg
5701 gtttcatact caatatcgct taaaaaaaaa aaagtatcag ctagggatga ctctggaagt
5761 atgagtatca tggtggggag gaaggaattt tttttaaatg taaatgaccc ccatttacca
5821 gaccctaatc aaagtcactt aagggaatcc ctcagccttt tatttggaaa cagttgaaat
5881 aaactggcag cagctagatc agagtatctt gctttatttt ataaaggcca aaggtagtat
5941 gaagtttgga ccaaaaaggt aaatagatcc attccagcac ctgatactga tttttcaagg
6001 ctctatgaaa ggtcaaaaat ttcattaaac aagaccagtt ctccctcttc ccctgtccc
6061 aagaaatctt aggcatgaaa aggataagga aacagctcct ggaatgatac atttgcatag
6121 tgccctagta gcaggttggg aaaaagttat aatataagaa acaaccttcg aaaacaggcc
6181 ttttatctca aagataaaat gtctttcttg tggtctttca tcactatctc cgtggtggaa
6241 ggttccccta gttccaacat atttccatta aaatagtcaa agccacggca ttgggatgtc
6301 agatgcctct ctttctttgt ggtaatcgga atttaaaatt atacagttgc ctctgaattt
6361 ctcatgcaca aagccaaacc actgataaga gataaagcag tctgaagcct gctgcttcag
6421 ccagcacagc acaccacaca cgctcgcact ttcaaaagca atgtgatttc tatggttctt
6481 aaaagctttc ttcataaggg agtccctgaa atttctcaag gcaggtttga atggcaaagg
6541 gaaaataatt acttgtggga ggtcctcctt ttgagtattg ttagagcata catgtaaaag
6601 aaaataacct ttttggggca actcatgctc acacatgctg ttttctttgg ttctccccct
6661 accttccttt tgtagatatt gacagaatag gaggaaatga gcatccttat ttgagaaaga
6721 gcaagaatgt catgagccct tgatgcaata gtaagtgtga tgtcatcata cagtgttaat
6781 gatgctatca aatccatcaa taaacagtct caaaccttcc aacaacagtg ctcactgctg
6841 ctcctcaact tcagcccagc aagcagtaat atcatcaccc attttgagat atgcaggtgg
6901 aatagaacaa agaaacagcc actgtaatcg agaagcatgt ttactgtcta aatccacctg
6961 ttgcagtagg aagccagagt ggggttccaa atgcctcatt aagtatgtgg acagcctcac
7021 tagtaagtga gtgaatttgg cttcatcact gaacattagc taaggtcagc ttaataacac
7081 aaatatgagg ccgacttctt tgcgagaaga gaaaagaaaa catctgcttg atttaaaatc
7141 caccccacat gcctagagtt gtctaatagt ccctcacttt ccaatggctt cacatcctac
7201 ttctacattt ggggtttttt ggtgaaatca gagatagctc aggatttcat aaaacgagaa
7261 actccaaact ggtctattag gttccatggg aacacttgta gccaaaggat tgtctgaggg
7321 caggaagacg acacttgtca acaaggaaga cagtgtttct ttagttccca tattcatcta
7381 attcatgggg ttctaacatt ttggggggcc atagattctt ttgacaatct gagccaatct
7441 atgaaacttc tccccaaaaa gaaccacccc acaaaatctt gcaaaacgta agagattttc
7501 ctggatctga agctacccga agacatggga agagttgtat tctattattc aattttaaga
7561 atatttatta atattcactg agctattgct agccactgtg ctaaacattt tacatacatt
7621 ctcctatttc atcctcaaaa caatcctttg agttgggttt taatatagtt ccaatattcg
7681 gatgtgaaaa ctgaggctta tagtggctaa gaaacttgcc caaagccact acctagaaaa
7741 tggcagagct ggaatgtagg tttaactcct gaccactatg ctataaagtc accacatgtc
7801 aaactaattt tcaagttgtt gggacatgtc ccctactagg ttttaaacta gatcttcctt
7861 ggtagatgaa gctatagaac ttctattttc cctgctttct gtagtctctc acagtgacag
7921 catctatact aaagtataga tacctaaggg gaaaatatag aaagcctgcc tgaataatag
7981 aatctagaac aacaacaaaa atattaattt tttctgtgta tgcttaggtc aagcttaaaa
8041 aaaaaaaaaa aa //

- LOCUS: NM_001944 5561 bp mRNA linear PRI 14-JAN-2007
- DEFINITION: Homo sapiens desmoglein 3 (pemphigus vulgaris antigen) (DSG3), mRNA.
- ACCESSION: NM_001944
- VERSION: NM_001944.2 GI:119964717

1 aaagcagcag agacgctgca gagggctttt cttagacatc aactgcagac ggctggcagg
61 atagaagcag cggctcactt ggactttttc accagggaaa tcagagacaa tgatggggct
121 cttccccaga actacagggg ctctggccat cttcgtggtg gtcatattgg ttcatggaga
181 attgcgaata gagactaaag gtcaatatga tgaagaagag atgactatgc aacaagctaa
241 aagaaggcaa aaacgtgaat gggtgaaatt tgccaaaccc tgcagagaag gagaagataa
301 ctcaaaaaga aacccaattg ccaagattac ttcagattac caagcaaccc agaaaatcac
361 ctaccgaatc tctggagtgg gaatcgatca gccgcctttt ggaatctttg ttgttgacaa
421 aaacactgga gatattaaca taacagctat agtcgaccgg gaggaaactc caagcttcct
481 gatcacatgt cgggctctaa atgcccaagg actagatgta gagaaaccac ttatactaac
541 ggttaaaatt ttggatatta atgataatcc tccagtattt tcacaacaaa ttttcatggg
601 tgaaattgaa gaaaatagtg cctcaaactc actggtgatg atactaaatg ccacagatgc
661 agatgaacca aaccacttga attctaaaat tgccttcaaa attgtctctc aggaaccagc
721 aggcacaccc atgttcctcc taagcagaaa cactggggaa gtccgtactt tgaccaattc
781 tcttgaccga gagcaagcta gcagctatcg tctggttgtg agtggtgcag acaaagatgg
841 agaaggacta tcaactcaat gtgaatgtaa tattaaagtg aaagatgtca acgataactt
901 cccaatgttt agagactctc agtattcagc acgtattgaa gaaaatattt taagttctga
961 attacttcga tttcaagtaa cagatttgga tgaagagtac acagataatt ggcttgcagt
1021 atatttcttt acctctggga atgaaggaaa ttggtttgaa atacaaactg atcctagaac
1081 taatgaaggc atcctgaaag tggtgaaggc tctagattat gaacaactac aaagcgtgaa
1141 acttagtatt gctgtcaaaa acaaagctga atttcaccaa tcagttatct ctcgataccg
1201 agttcagtca accccagtca caattcaggt aataaatgta agagaaggaa ttgcattccg
1261 tcctgcttcc aagacattta ctgtgcaaaa aggcataagt agcaaaaaat tggtggatta
1321 tatcctggga acatatcaag ccatcgatga ggacactaac aaagctgcct caaatgtcaa
1381 atatgtcatg ggacgtaacg atggtggata cctaatgatt gattcaaaaa ctgctgaaat
1441 caaatttgtc aaaaatatga accgagattc tactttcata gttaacaaaa caatcacagc
1501 tgaggttctg gccatagatg aatacacggg taaaacttct acaggcacgg tatatgttag
1561 agtacccgat ttcaatgaca attgtccaac agctgtcctc gaaaaagatg cagtttgcag
1621 ttcttcacct tccgtggttg tctccgctag aacactgaat aatagataca ctggccccta
1681 tacatttgca ctggaagatc aacctgtaaa gttgcctgcc gtatggagta tcacaaccct
1741 caatgctacc tcggccctcc tcagagccca ggaacagata cctcctggag tataccacat
1801 ctccctggta cttacagaca gtcagaacaa tcggtgtgag atgccacgca gcttgacact
1861 ggaagtctgt cagtgtgaca acaggggcat ctgtggaact tcttacccaa ccacaagccc
1921 tgggaccagg tatggcaggc cgcactcagg gaggctgggg cctgccgcca tcggcctgct
1981 gctccttggt ctcctgctgc tgctgttggc cccccttctg ctgttgacct gtgactgtgg
2041 ggcaggttct actgggggag tgacaggtgg ttttatccca gttcctgatg gctcagaagg
2101 aacaattcat cagtggggaa ttgaaggagc ccatcctgaa gacaaggaaa tcacaaatat
2161 ttgtgtgcct cctgtaacag ccaatggagc cgatttcatg gaaagttctg aagtttgtac
2221 aaatacgtat gccagaggca cagcggtgga aggcacttca ggaatggaaa tgaccactaa
2281 gcttggagca gccactgaat ctggaggtgc tgcaggcttt gcaacaggga cagtgtcagg
2341 agctgcttca ggattcggag cagccactgg agttggcatc tgttcctcag ggcagtctgg
2401 aaccatgaga acaaggcatt ccactggagg aaccaataag gactacgctg atggggcgat
2461 aagcatgaat tttctggact cctacttttc tcagaaagca tttgcctgtg cggaggaaga
2521 cgatggccag gaagcaaatg actgcttgtt gatctatgat aatgaaggcg cagatgccac
2581 tggttctcct gtgggctccg tgggttgttg cagttttatt gctgatgacc tggatgacag
2641 cttcttggac tcacttggac ccaaatttaa aaaacttgca gagataagcc ttggtgttga
2701 tggtgaaggc aaagaagttc agccaccctc taaagacagc ggttatggga ttgaatcctg
2761 tggccatccc atagaagtcc agcagacagg atttgttaag tgccagactt tgtcaggaag
2821 tcaaggagct tctgctttgt ccacctctgg gtctgtccag ccagctgttt ccatccctga
2881 ccctctgcag catggtaact atttagtaac ggagacttac tcggcttctg gttccctcgt
2941 gcaaccttcc actgcaggct ttgatccact tctcacacaa aatgtgatag tgacagaaag
3001 ggtgatctgt cccatttcca gtgttcctgg caacctagct ggcccaacgc agctacgagg
3061 gtcacatact atgctctgta cagaggatcc ttgctcccgt ctaatatgac cagaatgagc
3121 tggaatacca cactgaccaa atctggatct ttggactaaa gtattcaaaa tagcatagca
3181 aagctcactg tattgggcta ataatttggc acttattagc ttctctcata aactgatcac
3241 gattataaat taaatgtttg ggttcatacc ccaaaagcaa tatgttgtca ctcctaattc
3301 tcaagtacta ttcaaattgt agtaaatctt aaagtttttc aaaaccctaa aatcatattc
3361 gccaggaaat tttcctaaac attcttaagc ttctattttt cccctgccaa aggaaggtgt
3421 ttatcatttt aaaatgcaat gtgatttagt ggattaagca ggagcgctgg ttcttgtctc
3481 cattgccttt tcttatatca ttgataatga tgtaagaatc acaagggccc gggcgcggtg
3541 gctcacgcct gtaatcccag cactttggga ggccgaggca ggtggatcat gaggtcagga

3601 gatcgagacc atcctggcta acaaggtgaa accccgtctc tactaaaaat acaaaaaatt
3661 agccgggcgc agtggcgggc gcctgtagtc ccagctactc gggaggctga ggcaggagaa
3721 tggcatgaac ccgggaagcg gagcttgcag tgagccgaga ttgcgccact gcagtccgca
3781 gtccggcctg ggcgacagag cgagactccg tctcaaaaaa aaaaaaaaaa aaagaatcac
3841 aaggtatttg ctaaagcatt ttgagctgct tggaaaaagg gaagtagttg cagtagagtt
3901 tcttccatct tcttggtgct gggaagccat atatgtgtct tttactcaag ctaaggggta
3961 taagcttatg tgttgaattt gctacatcta tatttcacat attctcacaa taagagaatt
4021 ttgaaataga aatatcatag aacatttaag aaagtttagt ataaataata ttttgtgtgt
4081 tttaatccct ttgaagggat ctatccaaag aaaatatttt acactgagct ccttcctaca
4141 cgtctcagta acagatcctg tgttagtctt tgaaaatagc tcatttttta aatgtcagtg
4201 agtagatgta gcatacatat gatgtataat gacgtgtatt atgttaacaa tgtctgcaga
4261 ttttgtagga atacaaaaca tggccttttt tataagcaaa acgggccaat gactagaata
4321 acacataggg caatctgtga atatgtatta taagcagcat tccagaaaag tagttggtga
4381 aataattttc aagtcaaaaa gggatatgga aagggaatta tgagtaacct ctatttttta
4441 agccttgctt ttaaattaaa cagctacagc catttaagcc ttgaggataa taaagcttga
4501 gagtaataat gttaggttag caaaggttta gatgtatcac ttcatgcatg ctaccatgat
4561 agtaatgcag ctcttcgagt catttctggt cattcaagat attcaccctt ttgcccatag
4621 aaagcaccct acctcacctg cttactgaca ttgtcttagc tgatcacaag atcattatca
4681 gcctccatta ttccttactg tatataaaat acagagtttt atattttcct ttcttcgttt
4741 ttcaccatat tcaaaaccta aatttgtttt tgcagatgga atgcaaagta atcaagtgtt
4801 tgtgctttca cctagaaggg tgtggtcctg aaggaaagag gtcccctaaa tatcccccac
4861 cctggtgctc ctccctctcc ctggtaccct gactaccagg aagtcaggtg ctagagcagc
4921 tggagaagtg caggcagcct gtgcttccac agatgggggt gctgctgcaa caaggctttc
4981 aatgtgccca tcttaggtgg gagaagctag atcctgtgca gcagcctggt aagtcctgag
5041 gaggttccat tgctcttcct gctgctgtcc tttgcttctc aacggtggct cgctctacag
5101 tctagagcac atgcagctaa cttgtgcctc tgcttatgca tgagggttaa attaacaacc
5161 ataaccttca tttgaagttc aaaggtgtat tcaggatcct caaagcattt taaccttgcc
5221 gcttaaaacc caatttaccg tgaaatggga attttgctgc attgttaaac tgtagtggaa
5281 accatgctat agtaataaag gttatataag agagaaattg aaattaaatg tgtttttaaa
5341 tttcaaaaaa aaatcaatct ttaggatgac ttaaaaattg atttgccatg taaaatgtat
5401 ctgcattttt tacacaaaac ttgttttaag cataaaattt taaaactgta ctacttgatg
5461 tattatacat tttgaaccat atgtattaaa ccataaacag tataatgttg ttataataaa
5521 acaggcaata aatttataaa taaaagctga aaaaaaaaaa a //

- LOCUS: HUMSPROT 2530 bp mRNA linear PRI 02-SEP-1994
- DEFINITION: Human S protein mRNA, complete cds.
- ACCESSION: L20815
- VERSION: L20815.1 GI:414809
- KEYWORDS: S gene; S protein; cell differentiation; keratin; Keratinocyte differentiation; loricrin.

1 ccgtgcagtc cgagatgggc tcgtctcggg caccctggat ggggcgtgtg ggtgggcacg
61 ggatgttggc actgctgctg gctggtctcc tcctgccagg gaccttggct aagagcattg
121 gcaccttctc agacccctgt aaggacccca cgcgtatcac ctcccctaac gacccctgcc
181 tcactgggaa gggtgactcc agcggcttca gtagctacag tggctccagc agttctggca
241 gctccatttc cagtgccaga agctctggtg gtggctccag tggtagctcc agcggatcca
301 gcattgccca gggtggttct gcaggatctt ttaagccagg aacggggtat tcccaggtca
361 gctactcctc cggatctggc tctagtctac aaggtgcatc cggttcctcc cagctgggga
421 gcagcagctc tcactcggga agcagcggct ctcactcggg aagcagcagc tctcattcga
481 gcagcagcag cagctttcag ttcagcagca gcagcttcca agtagggaat ggctctgctc
541 tgccaaccaa tgacaactct taccgcggaa tactaaaccc ttcccagcct ggacaaagct
601 cttcctcttc ccaaacctct ggggtatcca gcagtggcca aagcgtcagc tccaaccagc
661 gtccctgtag ttcggacatc cccgactctc cctgcagtgg agggcccatc gtctcgcact
721 ctggccccta catccccagc tcccactctg tgtcaggggg tcagaggcct gtggtggtgg
781 tggtggacca gcacggttct ggtgcccctg gagtggttca aggtcccccc tgtagcaatg
841 gtggccttcc aggcaagccc tgtcccccaa tcacctctgt agacaaatcc tatggtggct
901 acgaggtggt gggtggctcc tctgacagtt atctggttcc aggcatgacc tacagtaagg
961 gtaaaatcta tcctgtgggc tacttcacca aagagaaccc tgtgaaaggc tctccagggg
1021 tcccttcctt tgcagctggg ccccccatct ctgagggcaa atacttctcc agcaacccca
1081 tcatccccag ccagtcggca gcttcctcgg ccattgcatt ccagccagtg ggactggtg
1141 gggtccagct ctgtggaggc ggctccacgg gctccaaggg accctgctct ccctccagtt
1201 ctcgagtccc cagcagttct agcatttcca gcagctccgg ttcaccctac catccctgcg
1261 gcagtgcttc ccagagcccc tgctccccac caggcaccgg ctccttcagc agcagctcca

1321 gttcccaatc gagtggcaaa atcatccttc agccttgtgg cagcaagtcc agctcttctg
1381 gtcacccttg catgtctgtc tcctccttga cactgactgg gggccccgat ggctctcccc
1441 atcctgatcc ctccgctggt gccaagccct gtggctccag cagtgctgga aagatcccct
1501 gccgctccat ccggatatcc tagcccaagt gaagcctctg gggccccagc tagctgaccc
1561 tgaagttttc ctaccccaag gagagttact cgacagtcca taagtcaact gttgtgtgtg
1621 tgcatgcctt gggcacaaac aagcacatac actatatccc atatgggaga aggccagtgc
1681 ccaggcatag ggttagctca gtttccctcc ttcccaaaag agtggttctg ctttctctac
1741 taccctaagg ttgcagactc tctcttatca ccccttcctc cttcctcttc tcaaaatggt
1801 agattcaaag ctcctctctt gattctctcc tactgtttaa attcccattc caccacagtg
1861 cccctcagcc agatcaccac cccttacaat tccctctact gtgttgaaat ggtccattga
1921 gtaacacccc catcaccttc tcaactggga aacccctgaa atgctctcag agcacctctg
1981 acgcctgaag aagttatacc ttcctcttcc cctttaccaa ataaagcaaa gtcaaaccat
2041 catctggaaa cagtggccac ttttcactga cctctcttcg acatctagtc aacccaccca
2101 atatgccact gggtttcgct cccaattcca ccccaccctc cattacagag ctcaccacgc
2161 cctcctagat caccgtcccc aacacaccca ttgcctctca aggcccttat ctcagcccct
2221 tcctgtggcc atttccctca gtgcccagat gattccctgg gtgagggaga cactggggca
2281 ccctcagagg ttggagcagg ctccctgctg tccctggatc ctggacagat ggctcagtaa
2341 actgtgggac taggtgcaga cttttgcctt cttggagtcc tgggtctcct ctgagaggtc
2401 tgggtggtgc tcctcctacg cctctagagg tctctgtgtt cctcattttc cttcaaaagc
2461 gggctgtatt tctcttctac cttccagctc ctcccacaga ggaggaagac aataaatatt
2521 tgttgaactg //

- LOCUS: NM_020243 1396 bp mRNA linear PRI 18-NOV-2006
- DEFINITION: Homo sapiens translocase of outer mitochondrial membrane 22 homolog(yeast) (TOMM22), nuclear gene encoding mitochondrial protein,mRNA.
- ACCESSION: NM_020243
- VERSION: NM_020243.4 GI:56788357

1 tcctttccgc ttccggtgtc ccctacagtc atggctgccg ccgtcgctgc tgccggtgca
61 ggggaacccc agtccccgga cgaattgctc ccgaaaggcg acgcggagaa gcctgaggag
121 gagctggagg aggacgacga tgaggagcta gatgagaccc tgtcggagag actatggggc
181 ctgacggaga tgtttccgga gagggtccgg tccgcggccg gagccacttt tgatctttcc
241 ctctttgtgg ctcagaaaat gtacaggttt tccagggcag ccttgtggat tgggaccact
301 tcctttatga tcctggttct tcccgttgtc tttgagacgg agaagttgca aatggagcaa
361 cagcagcaac tgcagcagcg gcagatactt ctaggaccta acacagggct ctcaggagga
421 atgccagggg ctctaccctc acttcctgga aagatctaga ttgttattgc tgtttgagct
481 gtctcagtgg gataagtttg aaattcaagt gtttgaactg ctgataattt ggattttttt
541 ttttttttaa ctttggcaca ttgatctatc taaacctggt ggggagaatt atccccacat
601 tgtctcatgg aaagactcaa cttgcaactg tgccctccac actatcctta cttctgtctc
661 cactctgata ccagagtgca gccatgcaga tggttattcc agctctggtc acccgactcc
721 tttcaccaaa ttgctcctaa ctggaagatc tcactttccc cttgtggggt aggaaccgat
781 gccagtggga gggatgtgcc cctgaccatt aacgactgtt tttttttttt tttttaaag
841 aatggagttg ttggggcagg acatgcacac aatgtgaaac agacaaaatg cattacacct
901 gtagtgtaaa gtggccacta tgaatcccta tgtatgagag gagggaggca ggctgcagct
961 tcagccacag aatggggact atggaagaca gcaggagctc atttcctctg cacattttgg
1021 ctgttagacc tgtgtgtgtg tttaaaaaaa gagaagtcag tgctcacttt ttgtatttaa
1081 atattaaaaa tgattccaac tgaaagtgtc atcctaagta ccttgaaatg agaccacgtc
1141 agagacatgt actgcccctc acattttctc acctaaacca gcagcacctc catcttaaca
1201 gccataggcc caaattgttt ccaagtgaaa attcattttt agccaagtac ttcatagcaa
1261 tctttgccct gaatttaggc agtcactttg agatccatca gcctaaaaca aaggattggg
1321 tctatgtact tctttagtct ataatgacac tgtgtaatta taaagtattt gtagggaaaa
1381 aaaaaaaaaa aaaaaa //

- LOCUS: NM_005895 9252 bp mRNA linear PRI 18-NOV-2006
- DEFINITION: Homo sapiens golgi autoantigen, golgin subfamily a, 3 (GOLGA3), mRNA.
- ACCESSION: NM_005895 NM_178137
- VERSION: NM_005895.3 GI:87196329

1 ggcctgggcg cgtccctgca gcgtggcggg acggccccgt tccagtcacc cccgcctcgc
61 tgcggtggcc tcgggcctgg gcgcccgcct tcagctgcgg cggagctggc tctgtaaatg
121 ccggtgcccg cgagccctcc tgaatgcttg tctgcgcccg acgagcgcgg cctgtcccga
181 agctgtccac tgccaccact cgggcagtgc ttgctttagc ctggcccttt gcagctgaaa

241 ggcgtgacat ggtgtcgggt ggttcgtggg aagtcggggt ttcaggagtc cgtgtacttc
301 cttgtttgtc tttgtcgctg gccgacttgt cttcattcca ggtggccaga gcgagtgggg
361 ccgggcgttg tcacgggtat catgatatta gctggtttga catcaagtca tttgtgagtc
421 atcagatctt ctcctgaaaa tgggagacac agtagggccc ctcccaggag ctcttggctg
481 ttgctgatgg cagaagccaa gcttgtccaa ggttcacttg tagcccctca gcgtcagctc
541 agctggtgtc gtcctgacca tggacggcgc gtcggccgag caagatggcc tccaggagga
601 cagatcccac agtggcccct cgtctctccc cgaggcccca ctgaagcccc cggcccactt
661 ggtgccacct gaccagcagg acaaagtcca gtgtgccgag gtaaacagag catccacgga
721 aggggaaagc ccggatggac ctggccaggg aggcctctgt cagaacgggc caacgccacc
781 cttcccagac cctccgtcgt ctctcgatcc caccacaagc ccagtgggcc ctgatgcctc
841 tccaggtgtg gctggtttcc atgacaacct aaggaagtct caggaactag gtgctgaggg
901 cagtgttaga aaagaagctt tgcagtctct cagactcagt cttcctatgc aagaaacgca
961 actgtgctct acagattctc ccctgcccct ggagaaggag gagcaggtcc gacttcaggc
1021 tcggaagtgg ctggaagagc agctcaaaca gtacagggtg aagcgccagc aggagaggtc
1081 cagtcaacct gcaaccaaaa cgagactttt tagcacgctt gatcctgagc tcatgttaaa
1141 cccagaaaac ttaccaaggg ccagtaccct ggctatgaca aaagaatatt ccttcctgcg
1201 caccagtgtc cctcgggggc ctaaggtggg cagcctgggg cttccggcac atcctaggga
1261 gaaaaaaact tccaaatcaa gcaaaatccg gtctctggcc gattacagaa ctgaagattc
1321 aaatgcgggg aattctgggg gaaatgtccc ggctcccgat tctaccaagg gttccctgaa
1381 gcagaacaga agcagtgcgg cgtccgttgt gtctgagatc agcctgtccc ccgacactga
1441 cgaccgtctg gagaacacct ccctggctgg agacagcgtg tctgaggtgg atggaaatga
1501 cagcgacagc tcatcgtaca gcagcgcctc cacccgaggg acctatggca ttctgtcgaa
1561 gacagtgggc acgcaggaca ccccctatat ggtcaacggc caggagattc ctgcggatac
1621 cctgggccag ttcccctcca ttaaggacgt cctccaggcc gcagccgctg agcaccaaga
1681 ccaggggcag gaggtcaacg gggaggtgcg gagtcggaga gacagcatct gcagcagcgt
1741 gtccttggag agctctgcag cagaaacaca ggaggagatg ctgcaggtgc tcaaagagaa
1801 aatgcgactc gaaggacagc tggaagcctt gtcactggag gcgagtcagg cacttaaaga
1861 gaaggctgag ctgcaggccc agctggccgc cctcagcacg aagctgcagg cgcaggtgga
1921 gtgcagccac agcagccagc agcggcagga ttcgctgagc tcggaggtgg acaccctgaa
1981 gcagtcgtgc tgggacctgg agcgagccat gactgacctg cagaacatgc tggaggcaaa
2041 aaatgccagc ctggcgtcgt ccaacaacga cttgcaggtg gccgaggagc agtaccagag
2101 gcttatggcc aaggtagagg acatgcagag gagcatgctc agcaaggaca acacagtgca
2161 cgacctgcga cagcagatga cagccttgca gagccagctt cagcaggtgc agctggagcg
2221 gacgacgctg accagcaagc tgaaggcgtc gcaggcggag atctcgtccc tgcagagtgt
2281 ccggcagtgg taccagcagc agctcgccct ggcacaggag gcccgcgtca ggctgcaggg
2341 tgagatggcc cacatccagg ttggacagat gacccaggca ggtctcctgg agcacctgaa
2401 actcgagaat gtgtccctgt cccagcagct gacggaaact cagcacaggt ccatgaagga
2461 gaaggggcgc atcgcggcac agctgcaggg cattgaggct gacatgttgg atcaggaagc
2521 agccttcatg cagattcagg aggcaaagac gatggtggag gaggaccttc agaggaggct
2581 ggaagagttt gaaggtgaga gggagcggct gcagaggatg gcggactcgg cggcatccct
2641 ggagcagcag ctggagcagg tgaagttgac tttactccag cgagaccagc agcttgaggc
2701 tttgcagcag gagcacctgg acctgatgaa acagctcacc ttgactcagg aggctctgca
2761 gagcagggag cagtccctcg atgccctgca gacacactac gatgagctgc aggccaggct
2821 gggggagctg cagggcgagg ccgcctccag ggaggacacg atctgcctcc tgcagaacga
2881 gaagatcatc ttggaggcgg ctttgcaggc ggccaagagt ggcaaggagg agcttgacag
2941 aggagcaaga cgcttggaag aaggtaccga ggaaacgtcg gaaactttag agaagttaag
3001 agaagaatta gctatcaaat ccggccaggt ggaacacctg cagcaggaga ctgctgctct
3061 gaaaaagcaa atgcaaaaaa taaaggaaca gtttctccaa caaaaggtga tggtggaggc
3121 ctaccggcgc gacgccacct ccaaagacca gctcatcagt gagctgaaag ccaccaggaa
3181 gaggctggac tcggagctga aggagctgcg gcaggagctg atgcaagtgc acggggagaa
3241 gcggactgcc gaggcggagc tctcgcgcct gcacagagag gtggcccagg tccgtcagca
3301 catggcggac cttgaagggc atctccagtc ggcgcagaag gagcgagacg agatggaaac
3361 acacttgcag tcgttgcagt tcgataagga gcagatggtc gcggtcacag aggccaatga
3421 ggcgctgaag aaacaaatcg aagagttgca gcaagaggcc cggaaggcca tcacggaaca
3481 gaagcagaag atgaggcggc tgggctcaga cttgaccagc gcccagaagg agatgaagac
3541 caaacataag gcctacgaga acgccgtggg catcctcagc cgccgcctgc aggaggccct
3601 cgcggccaag gaggctgcgg acgcggagct gggccagctc cgagcccagg gtggcagcag
3661 tgacagcagc ctggctctac atgaaaggat ccaggccctg gaggcggagc tgcaggctgt
3721 cagtcatagc aagacgctgc tggaaaagga actgcaggag gtcatagcgc tgaccagcca
3781 ggagctggag gagtcccggg agaaggtgct ggagctggag gacgagcttc aagaatccag
3841 aggctttagg aagaagataa aacgccttga ggagtcaaac aagaagttgg ctcttgaatt
3901 agagcacgag aaagggaagc ttacgggcct cggtcagtcc aacgcagctc tgcgggaaca
3961 caacagcatc ctagaaacag ctttggccaa gagggaggca gacctagtcc agttgaacct

4021 tcaggtgcag gcagttttgc agcgcaaaga agaggaggat cgccagatga agcatcttgt
4081 ccaggccctg caggcctcac tagagaagga gaaggagaag gtgaacagcc tcaaggagca
4141 ggtggctgct gccaaggtgg aagccgggca taaccgccgc cacttcaagg cggcctcctt
4201 ggagctgagt gaggtgaaga aggagctgca ggccaaggaa cacctggtgc agaagctgca
4261 ggccgaggcc gacgaccttc agattcggga ggggaaacat tcccaggaga tagcacagtt
4321 ccaagcagag ctggccgagg cccgggcaca gctccagctc ctgcagaagc agctggacga
4381 gcagctcagc aaacagcccg tgggaaacca agagatggaa aatctcaaat gggaggtgga
4441 tcagaaagaa agagaaatcc agtccttgaa gcagcagctg gacttgacgg agcagcaggg
4501 caggaaggaa ctggaagggc tacagcagct gctgcagaac gtcaagtctg agttggagat
4561 ggcccaggaa gacctgtcca tgacccagaa ggataaattt atgctccagg caaaagtgtc
4621 ggagctgaag aacaacatga agaccctgct ccagcagaac cagcagctca agctggacct
4681 acgccgcggc gcggccaaga cgagaaagga gccgaaaggc gaggccagct cttccaaccc
4741 tgccacgccc atcaagatcc cggactgccc agttcccgcc tcgctgctgg aggagctgct
4801 gagaccaccg cccgccgtga gcaaggagcc cctcaagaac ctgaacagct gcctccagca
4861 gctcaagcag gagatggaca gcctgcagcg ccagatggag gagcacgccc tgacggtgca
4921 cgagtctctg tcctcgtgga cgccgctgga gccagccact gccagccctg tgcccccggg
4981 gggtcacgcc ggcccacgcg gcgacccaca gagacacagt cagagcaggg cttccaaaga
5041 agggccggga gagtgactgc tgtggactcg cctccgtgcg ccgctgcccc agaaggctct
5101 tatcaatgtt atttatttga ttgtgtggtc gatgtttttc taagacatga aatttaagtt
5161 ttgttttgcc tttaacaaga agtaaaatat atagcagaat gagagccaag gactagaaaa
5221 acattcgaag atcacaatta gcttttcaca tggaatgacc aactcttaaa agcctgatag
5281 gctctcggcg aggagctttg aacgtgtctg aagggttact tgtaggtcgt ggcttctgag
5341 cggccaccga tgctgctctc tgcgggtgac agggagaggc tgcgtaactg ggagcagctg
5401 tgtgacaggg tctgcggcac cgcgcctggc caggccggct gcagtttctc acttccctgt
5461 tccattcagt aagagcttta cttttccgca gaaatgaaat tttatctgta cctttggctt
5521 tttacttgtt tttttggata gccatcccac cataggatgt gtacatagat actgaatatc
5581 ataatccaat ctttgttttt tttttttttt tttttttgag acagagtctc gctttgttgc
5641 ccaggctgga gtgcagtggc acactctccg ctcactgcaa gctccgcctc ccaggttcat
5701 gcgattctcc tgcctcagcc tctcgagtag ctgggattac aggcgtgcgc cactatgcca
5761 ggctaatgtt tgtattttta gtagcaatgg ggtttcacca tgttggccag gatggtctcg
5821 atctcctgac ctcaagtgat ctgcccatct cagcctccca aagtgctggg attacaggcg
5881 tgagcccctg cgcccggcct gtcacccagt ctttaagaag catatgctca tgttattgaa
5941 gaagaaccta cttattattg attgcctttt gaaaatttgt tgggaataat ttacctgcag
6001 gatttaggga tagtcagaaa attctaagaa atataattat tttatttacc ttctaaagcc
6061 aaatattctt acacagaaag gtcctctgtt gttctggttt tactttgttg ctgaggatct
6121 ttccttcctg ctggtctctt cctctcaggc cactggccct gtgtgattcc accgtggctg
6181 gccactggga aggggcagct tggacccttg gtcaggcctg acggccatca ggaggcacaa
6241 ggacactgag gccccatatc tgatctgacc tttggggggg cacagggaga ggccggtgga
6301 ggaggaggag gagagcagac caggggctcc ctgcagcgac tcccgcggtt tcccctggag
6361 tcagccaggt gtaggtcgca ggcggtaaca aacctcacac tcctgttccc caagtgaaaa
6421 tctttaccat tgtctgtggg agcgcctgta ctcgtgtgta ggagcacctg tacttctgca
6481 gtcatcgaga agtcctggat cttttgtggt tacaccagca tcatgtggca agcagaggcg
6541 acttccggaa gagacaggca ggcaccgtga ggaaggtggc tgtgctctcc caggtgtctc
6601 agagacagat gccttattta aaatcagcac gacatgtgtg agatcttctg tttcctaccc
6661 caaatcctga aaccctgcag acactggctg actgggagag gtggggtctg taagttgtcc
6721 cctagtttgc taagaaaatc taaaataata tttattatat gagttaggag agagagaatg
6781 ggtccgcgtg gcctcctctg cagatgtact ggtctgaaat gaggttctga gtcactggcc
6841 aggccagatg tgctcatgtc ggtgtctggt gtctgttttg tggagaaaac agtatggtgt
6901 gttttaagct atttgtgttc tgttgtaata tacttttaga aggttaattg gtaaggttaa
6961 ggtagcatta accacaaaga tgtttggtat ttaaaaaata ttctctagca aatattggaa
7021 tttccaaaat atatcatttg tacagggtta attttgaaat aatacttgaa aatttcatta
7081 taaatatatc ctacttttta tcttaagttg aagatgttat ttactaaatt gttcttgtac
7141 cattagaaaa aaaaatacgg caatttacgt tcttatttat tttggctgta ctaccccttt
7201 gttttaattt taaaatcaag aaatcgggcc gggcgcggtg gctcatgcct gtaatcccag
7261 cactttggga ggccgaggcg ggtggatcac ctgaggtcaa gaggtccaga ccatcctggc
7321 caacatggca aaaccccgtc tttactaaac atacaaaaat tagccgggtg tcgtagtgcg
7381 cacctataat cccagctact tgggaggctg aggcaggaga atcacttgaa cccaggaggc
7441 ggagcttgca gtgagccgag atcgcgccac tgccctccag cctgggcaac agagcgagac
7501 tccgtctcaa aaataaataa atgattttaa aaaatctaaa atcgagaaat cacacattca
7561 gtggggagcg acttctcctt gcttatggga agtcctcaag tgagtgatgt tcaccatgta
7621 tttttttttc tcttaggaca gactaattct gaaaataccg aaggaaaagt agctctatgt
7681 tctcaccccg gttttcctgc gtgtgtgccc ttgggtgcga tgcctccccc agcgctctgt
7741 ggtcgccggt gccagggccc cctctggttt ggcagggcct ggctgccttt gctccctgca

7801 gtgagtcttt tggtgttttc atgcacggct tgtgcttctg gatctgaggc ctctcgtgtt
7861 cacgcggaca cttccttcct taagaagacg cctaaaagag gaagttggaa tttttttttt
7921 ttttttttga gacagagtct cgctctgtcg cccaggctgg agtgcagtgg cgtgatctct
7981 gctcactgca agctccgcca tctgggttca agcgattctc ctgcctcatt ctccccagta
8041 gctgggatta caggtgcccg ccaccacacc agcctaattt ttgtattttt agaggggtgg
8101 agttccacca tgttggccag gctggtcttg aactcttgac ctcaggtgat cctgagcctc
8161 agcctcccaa agtgctggaa ttataggcgt gaaccaccgc ccccggctgc agttggattt
8221 ttaaattgct tttttttatt gttgaggttt ttttatctcc aagggactct cccggcactt
8281 ctaccttcca gagttacttc agtgcataaa gtttgaatta ttttgttctt gtgggcagaa
8341 gtgggaatga tggaatatcc tcacggaaaa ggcagtgaag ttgggagtac tgcttacaaa
8401 acagggtcac cagtgcatta tgtggcgtgt tcatccccac gccgtgtgtc acgggctagg
8461 gcggcgtgtt catccccaca ccgtgtgtca caacaggcta gggcacttca cgatgtcact
8521 acttgttttt ctgatgttcc aaaaacaacg taacttggtt ttcatgtgtt tttccgtggt
8581 atatgtgaga ttgatgctac gggtcttacg gactcacacc cgttcccact ctctgcaata
8641 tggatcaggc agtgtttctg ataggatgtg aaatggactc tcctcgggtg ggtccagcag
8701 gggccctgcc caccagaaca cagtccgtgc tgtgctgcgc taaggagctg gccctcaact
8761 ctccttggtg cagggttccc acaaccgagt tctagttccc tgaggtcttt aaaaacaaaa
8821 acagaatgtt gtacgtgaag attctaggag gggagggacc agcaaatctg agagaaccgt
8881 cctggggcct cccttcgagg agccctctga tgtgaggagg gacttgagtt gagtgacgct
8941 gtggtgtgag gtgttctgag ctcactgacc ggaaggtcca ggtgaatctc gtcataagtg
9001 atctcaggct ctcacaggat ccggagggaa atgtgttaga gggtctggaa aattcagtgc
9061 ttttgagtta cttgttttta ttaaaaattt cctcacaaaa gagagtcctc aagttgtggc
9121 tgttcttggg aaaggggtca ccgtgtctga caaagtgtaa ctttaaaaag cacgttgatt
9181 ttttacaaat gtaagtgtgc ttgggaattc cttaaatttt gtgcaataaa ctattttttg
9241 gtaaagattt tc//

- LOCUS: AY024365 1422 bp mRNA linear PRI 22-MAR-2001
- DEFINITION: Homo sapiens integrin-linked kinase-associated serine/threonine phosphatase 2C mRNA, complete cds.
- ACCESSION: AY024365
- VERSION: AY024365.1 GI:13432041

1 ggcaccaggc ccgctgctgc cgcccgcccg gggtgtggag cccggccgct gctcgcgggc
61 tgagtgtctg tcgctgctgc cgcctccacc cagcctccgc catggacctc ttcggggacc
121 tgccggagcc cgagcgctcg ccgcgcccgg ctgccgggaa agaagctcag aaaggacccc
181 tgctctttga tgacctccct ccggccagca gtactgactc aggatcaggg ggacctttgc
241 tttttgatga tctcccaccc gctagcagtg gcgattcagg ttctcttgcc acatcaatat
301 cccagatggt aaagactgaa gggaaaggag caaagagaaa aacctccgag gaagagaaga
361 atggcagtga agagcttgtg gaaaagaaag tttgtaaagc ctcttcggtg atctttggtc
421 tgaagggcta tgtggctgag cggaagggtg agagggagga gatgcaggat gcccacgtca
481 tcctgaacga catcaccgag gagtgtaggc ccccatcgtc cctcattact cgggtttcat
541 attttgctgt ttttgatgga catggaggaa ttcgagcctc aaaatttgct gcacagaatt
601 tgcatcaaaa cttaatcaga aaatttccta aaggagatgt aatcagtgta gagaaaaccg
661 tgaagagatg ccttttggac actttcaagc atactgatga agagttcctt aaacaagctt
721 ccagccagaa gcctgcctgg aaagatgggt ccactgccac gtgtgttctg gctgtagaca
781 acattcttta tattgccaac ctcggagata gtcgggcaat cttgtgtcgt tataatgagg
841 agagtcaaaa acatgcagcc ttaagcctca gcaaagagca taatccaact cagtatgaag
901 agcggatgag gatacagaag gctggaggaa acgtcaggga tgggcgtgtt ttgggcgtgc
961 tagaggtgtc acgctccatt ggggacgggc agtacaagcg ctgcggtgtc acctctgtgc
1021 ccgacatcag acgctgccag ctgaccccca atgacaggtt cattttgttg gcctgtgatg
1081 ggctcttcaa ggtctttacc ccagaagaag ccgtgaactt catcttgtcc tgtctcgagg
1141 atgaaaagat ccagacccgg gaagggaagt ccgcagccga cgcccgctac gaagcagcct
1201 gcaacaggct ggccaacaag gcggtgcagc ggggctcggc cgacaacgtc actgtgatgg
1261 tggtgcggat agggcactga ggggtggcgc gcggccagga gcacgcatgg tattgactta
1321 aaaggttcat tttgtgtgtg tgcacattgt gtgttttgtg tactcctgtg ggactcccat
1381 ggttgtaaat aaaggtttct cttttttttc ctaaaaaaaa aa//

- LOCUS: HUMEDZ 405 bp mRNA linear PRI 27-APR-1993
- DEFINITION: Human endozepine (putative ligand of benzodiazepine receptor) mRNA, complete cds.
- ACCESSION: M15887
- VERSION: M15887.1 GI:181960
- KEYWORDS: endozepine.
- bp: upstream of HindIII site.

1 ggttcctggt cctcgcctcc tccgctgtct ccctggagtt cttgcaagtc ggccaggatg
61 tctcaggctg agtttgagaa agctgcagag gaggttaggc accttaagac caagccatcg
121 gatgaggaga tgctgttcat ctatggccac tacaaacaag caactgtggg cgacataaat
181 acagaacggc ccgggatgtt ggacttcacg ggcaaggcca agtgggatgc ctggaatgag
241 ctgaaaggga cttccaagga agatgccatg aaagcttaca tcaacaaagt agaagagcta
301 aagaaaaaat acgggatatg agagactgga tttggttact gtgccatgtg tttatcctaa
361 actgagacaa tgccttgttt ttttctaata ccgtggatgg tggaa //

- LOCUS: NM_030959 1869 bp mRNA linear PRI 17-NOV-2006
- DEFINITION: Homo sapiens olfactory receptor, family 12, subfamily D, member 3 (OR12D3), mRNA.
- ACCESSION: NM_030959
- VERSION: NM_030959.2 GI:31377669

1 agcaatggag aatgtcacta caatgaatga gtttcttcta cttggcctga ctggtgttca
61 ggagctgcag cctttcttct ttgggatttt cttaatcatt tacctgataa acttgattgg
121 aaatggatct atattggtga tggttgtttt ggaaccacaa ctccactccc ctatgtattt
181 ttttctggga aacctttctt gtctggatat ttcttattct tcagtgacac tgcccaagct
241 gctcgtaaac ctcgtgtgca gtcgcagggc tatatctttt ctaggctgta tcacccagct
301 acacttcttc cactttttgg gaagcacaga ggccatttta ctggctatca tggcctttga
361 ccgttttgtt gccatctgca atcctcttcg ctacactgtc atcatgaacc cccaggtgtg
421 tattctgttg gcagctgcgg cctggctcat cagcttcttt tacgctctga tgcattctgt
481 catgactgca cacctgagtt tttgtggctc tcagaaactc aatcacttct tctacgatgt
541 caagccgctc ttagaattgg cctgtagtga cacattactc aatcaatggc ttctttccat
601 tgtcacaggc agcatatcca tgggagcttt ctttctgact cttctctcct gcttctatgt
661 aattggcttc cttctgttta agaacaggtc ctgcagaata ctccacaagg ctctgtccac
721 ttgtgcctcc cattttatgg tggtatgtct tttctatgga cctgtgggct tcacatatat
781 tcgtcctgct tcagccacct ccatgattca ggaccggata atggccatca tgtatagcgc
841 cgtcacccct gtactgaatc cactaatcta cacccttagg aacaaagaag tgatgatggc
901 tctgaagaaa atctttggta ggaagttgtt taaagactgg cagcaacacc actaggacta
961 atgagggata tctgatttct attaagactg atttccatca cttcactgta agaaatgatt
1021 tgtttacctg gtttatattt ttctttcaaa aattttgagc acatattata ttaggcagtg
1081 tagtaggttc aagaaacaca ggagcaagca aaacaaattc cataatgtca tcaaacataa
1141 agtttaatgt gatggaggcc ccaaataacc aaacgcgtaa gtacatatgt caggataatt
1201 cggtggcaag gagaactaag gaggtaggaa gtatggggag ggttagagta ggaatctgct
1261 gtgtaaaaaa gaatgtggca tttctcctag gacaaaaacc tttggaattt tccaagtaat
1321 agaagtatct tactgaaatt ccatatgaga taatctttat ttttgcctat tttttttcct
1381 tttgtgtttc cttcagcagg aatgtactga tgataatttt tctcacaaca gtttttatct
1441 agaatatcat ttttgttaat cttaattctt gaagagaatt tttaatgggt atttaatttt
1501 agggagagat ttttctagcc attgaaaata tcttgttttt ccccagctta ttgaagtata
1561 attaacaaat aaaaattgca tatatttagg cttcacaaca tgatgcttta gtatatgtat
1621 acattgtgaa gagactacca caatcaagct aaacctacat tactgtccct gcattagttt
1681 actgttgaat actagaagtt cttcattcta tctaactgca ttttcatacc cattaagcca
1741 atgtgacaag ctctttgttt cagttggagt gtttgcacaa tttaaatttg ataaattttt
1801 gatagaatta aatttaaatt tatcctccaa ctaaattttt gatagaatta aatttaaatt
1861 tgtcctcca//

- LOCUS: NM_000541 1608 bp mRNA linear PRI 18-NOV-2006
- DEFINITION: Homo sapiens S-antigen; retina and pineal gland (arrestin) (SAG), mRNA.
- ACCESSION: NM_000541
- VERSION: NM_000541.3 GI:116008177

1 tggatgctgg gcatcctcgc tagatcccct acaaattccc cacatacgta ggccaggagc
61 ctcagcggtg ccccttcagg ctcatctggc aagacggtac cagcttgctc agaacagggg
121 ctggctattc atcatctcag agcatagaga ccctctcctt gccacccggc ccttcccacc
181 tggttggtga caaatcacaa ggtggtagaa gttgccaggg acagataaca tggcagccag
241 cgggaagacc agcaagtccg aaccgaacca tgttatcttc aagaagatct cccgggacaa
301 atcggtgacc atctacctgg ggaacagaga ctacatagac catgtcagcc aagtccagcc
361 tgtggatggt gtcgtgttgg ttgatcctga tcttgtgaag ggaaagaaag tgtatgtcac
421 tctgacctgc gccttccgct atggccaaga ggacattgac gtgatcggct tgaccttccg
481 cagggacctg tacttctccc gggtccaggt gtatcctcct gtgggggccg cgagcacccc
541 cacaaaactg caagagagcc tgcttaaaaa gctggggagc aacacgtacc cctttctcct
601 gacgtttcct gactacttgc cctgttcagt gatgttgcag ccagctccac aagattcagg

661 gaagtcctgt ggggttgact ttgaggtcaa agcattcgcc acagacagca ccgatgccga
721 agaggacaaa atccccaaga agagctccgt gcgattactg atccgcaaag tacagcatgc
781 cccacttgag atgggtcccc agccccgagc tgaggcggcc tggcagttct tcatgtctga
841 caagcccctg caccttgcgg tctctctcaa caaagagatc tatttccatg gggagcccat
901 ccctgtgacc gtgactgtca ccaataacac agagaagacc gtgaagaaga ttaaagcatt
961 cgtggaacag gtggccaatg tggttctcta ctcgagtgat tattacgtca agcccgtggc
1021 tatggaggaa gcgcaagaaa aagtgccacc aaacagcact ttgaccaaga cgctgacgct
1081 gctgcccttg ctggctaaca atcgagaaag gagaggcatt gccctggatg ggaaaatcaa
1141 gcacgaggac acaaaccttg cctccagcac catcattaag gagggcatag accggaccgt
1201 cctgggaatc ctggtgtctt accagatcaa ggtgaagctc acagtgtcag gctttctggg
1261 agagctcacc tccagtgaag tcgccactga ggtcccattc cgcctcatgc accctcagcc
1321 tgaggaccca gctaaggaaa gttatcagga tgcaaattta gtttttgagg agtttgctcg
1381 ccataatctg aaagatgcag gagaagctga ggaggggaag agagacaaga atgacgttga
1441 tgagtgaaga tgtcggctca ggatgccgga aaatgacctg tagttaccag tgcaacgagc
1501 aaagccccac agtttagtcc tttggagtta tgctgcgtat gaaaggatga gtcttcttcc
1561 gagaaataaa gcttgtttgt tctcccctgg aaaaaaaaaa aaaaaaaa //

- LOCUS: NM_014286 4341 bp mRNA linear PRI 17-NOV-2006
- DEFINITION: Homo sapiens frequenin homolog (Drosophila) (FREQ), mRNA.
- ACCESSION: NM_014286
- VERSION: NM_014286.2 GI:17738307

1 ccggcccggc ccgcccggcc cagccgctcc tgctgggcgc cccaaccggg tccggcccgg
61 gggggcgggg gccgcggccg ccgaggatgg ggaaatccaa cagcaagttg aagcccgaag
121 ttgtggagga gctgaccagg aagacctact ttaccgagaa ggaggtccag cagtggtaca
181 aaggcttcat caaggactgc cccagtgggc agctggatgc ggcaggcttc cagaagatct
241 acaagcaatt cttcccgttc ggagacccca ccaagtttgc cacatttgtt ttcaacgtct
301 ttgatgaaaa caaggacggg cgaattgagt tctccgagtt catccaggcg ctgtcggtga
361 cctcacgggg aaccctggat gagaagctac ggtgggcctt caagctctac gacttggaca
421 atgatggcta catcaccagg aatgagatgc tggacattgt ggatgccatt taccagatgg
481 tggggaatac cgtggagctc ccagaggagg agaacactcc tgagaagagg gtggaccgga
541 tctttgccat gatggataag aatgccgacg ggaagctgac cctgcaggag ttccaggagg
601 ggtccaaggc agacccgtcc attgtgcagg cgctgtccct ctacgacggg ctggtatagt
661 cccaggctgg agctggatgc ctgggaacca ctcacctcct tctgtgccat gaggccacct
721 cagccctgac accaaccccg tgcgtccacc cagccttctt ccgcatccac acacagccgg
781 ctgcccttga cccgggaggc cccggctctc ctctcccctg tcctgcaccc atcccccgcc
841 tgaagccacc ggctccaatt gccagcaacc tctgcttgtc cggaaaacga caacacgaaa
901 tggaaaaggc tacagccctc tgcataaacc aaggacttgg ctgcctcgca ggcagcctcc
961 gttcctcccg ctctcttgcg cgtgtgcttt tgttttttat tttgaacaga cgttttaaaa
1021 gaaaaaaaaa caactacctt ctgtcctaga agacacagac tgacagatgg ggtgaaggcc
1081 tggggacctc agagaactct gccttgccct cgtccctcgt ccttcggcag ccggagaggc
1141 tgtgggtggg ccgagggtgt ctaggggttc tgcctagtca acgttatttg tcgtcccatc
1201 ttttggcagc aaaaccacct gcgtggctag gatgattaat tatgaggatg atgatttttt
1261 ttgtgataac agtattgtgc tttttgtggg gaaagtgagg tttttttttt atatacatat
1321 ataattgata tctttaattt attggttgtt aactgttgct gctgcctggt gtgtcctcag
1381 ctcccagggc tgcgggccca ccgtttacat gtgcacgccc tgacccacct gcccacgccg
1441 acttgggagg atggtggcct gcagcggcca agaagccaaa aaaatttttt tttttttcag
1501 atactgtgct tgatttttgg agaggggaga ggtggaaatt cctaaatggc taatgcactg
1561 ttccctccag cccgaatgcc tcctgccaaa ccccttttcc ctgctgcctc tgtccccgca
1621 tccttgttct cccctgggtc cgtaacattt tttccgagga tgaacagggg acatctttag
1681 gtttctcaac tcttgctttg gtgtttgccg cagcatggaa aacagggcgc ctaaggctgg
1741 gagctggaag aaggggcatt gggtacccag gcagagtcag gagaggtggt ctttgaagta
1801 agttagcaga aatcaagggg acccccgcct ccttgggctg ggagggggat ttcaagatag
1861 ttcataactc tctcccgctc tgccttccct ccttcctatc tgctttttcc agtaaactgc
1921 atggtgtcct tccctggcct tctcttggct caaaggctgg gagggaggga aggagagaag
1981 agttccaggc aatcccatca atatagtccc tacacctggg gctgcggccc acatgtcttc
2041 acggaggctt ccagcggtgc ctgccactga ggcaggtgcg gccccaggac catcaccagg
2101 aatgcgaggc caccctggac cagaggtagg agcccaaggt ccggcccttg ctctttgatt
2161 gtgggcagcc tcctgccctc tctgggtctc agttgcccca tctgcagagc gaggaggccc
2221 gggctggttg gtcttgaagg cccttttcca tgccgacatc atgtcactct aggcctgggg
2281 ttcagtttcc tgtggctggt gatgctgtgg ttaagtttgc ttgaccccag cagcccgagg
2341 gactgtctga gtcacagcac agcccctatt gcgtggctgc tggtgtgtgg ggtcagttcc
2401 agcagatgaa tgtgtcatgt ggcacacctt gtcccttccc gcagcatttc ctggttcccc
2461 ccagaccctt gagcgctctt tgggacccag aaggagtcct tgcacaggga aggcttgagg

2521 tgagaagccg cttcccagac tgtcagggcc aggcctgggt ctagaattct tgctgctgct
2581 ttgcagagtc aacagcccat cagcccatgt tttagagggg acactttggt cctcggttcc
2641 caccctcagc aagcaggcct ccagcccgag gaaggcctct gccgtagtga cgttgccgtg
2701 tggggctgcg tggctgttcc ccttggctgg agcattcagc caaccccagc gtcccccctg
2761 aggcgttcat tggcagcccc ctaggactgc acgctggccc cacggtaacc ccccctcccc
2821 caccaacatc ctgcagggat ggggtcagtg gttccacctt cacaggccac tttgaagggt
2881 ggattctttg aggcccctgc cagtcggctc cctgctcagc tgctggcccg ggcgacctgg
2941 gactcagcac caacggctga agtttctcag ctgggctctg acctggggtc tggggcaggg
3001 aacgaacatg gtggctttgg gctgagagga tgagggaggt ctttcccagg tcaaattact
3061 ttcctttggc ctctgcctga ggctcgattt gcctctctgg tccaatggga ctgacactgt
3121 tgtacaacct gacctgtggc tgagggtgtc tgggcttaag catgtggacc ccttcggtgt
3181 gtctggcctt cctccatcgt cctgcccttt ggccttttgg tttgaagcca caggtgtggc
3241 ttctggcctt agcagatggt atgcttgcgg accgcagccc agcatgccgg tgggcccaca
3301 gcccgagcca gcccagagct gccggaaggg ccgcccttcc cggccctggc ggggtgctgg
3361 acactggcca ttttcactag agtttgcctg gcagggaccg atctctgccc cctcctctcc
3421 ccaggcctct ggctgcagtg atgccgcaga atcctgagcc aggtgcctcc tgagcagccc
3481 gtgcgcctct ccacagcggc gtttgccacc caatgcggct cgcttcagat gctctgatgc
3541 agagggcacg cccatagtcc ctctgcagag cctcgcactg gggccagggc aggcaccagc
3601 cccaggcggc cagtcggcca cggcctgtcc tcttcctcgt agcgtctgct cctcactttg
3661 tgttgatggt gacttaggag aatgttccga ttttccatga tctaagcagg ccacgtttaa
3721 aataacatca aggcaagcgt acgtgtcacc ctctgtactg acatctcctc ccctgaaatg
3781 cttttcagtt tgacagcccg tttcctagac aagtgcacct ggggtttcag gaactttgtg
3841 ttttttcgga gggggttggt ggggaggtcg ggatgcctgg gatcccttcc tggagaggca
3901 ggctgtctct ggaaaaagcc tccattgccc acccgccagg cggaaagtca ccctgttccc
3961 agcgcggttt cagcatttaa ttttaaggga gctaaggaag cgcggcgcgc cccctggtgg
4021 tggtaagccg ccaacgcacc tgggggctgc aaccccaccg gacgggtggt ccggagggag
4081 gctggagcgg ggaggcgagg aggggctgt gagtcctcag aggccctggg ccaccacatt
4141 tctggcagcg tttcccagac acccctctgc taggccatcc ctggatagca agtgaattaa
4201 cttaagggca ctgtgatggg aagccttgcc cccctctttt tttttttttt tttaatatct
4261 gcggaataaa cccaatggtt aatttttgaa tgaataaaag gcttttgttg aataaaaaaa
4321 aaaaaaaaaa aaaaaaaaaa a //

- LOCUS: NM_016151 4920 bp mRNA linear PRI 03-DEC-2006
- DEFINITION: Homo sapiens TAO kinase 2 (TAOK2), transcript variant 2, mRNA.
- ACCESSION: NM_016151
- VERSION: NM_016151.2 GI:45505129

1 gagaccggga cgagaccggg gctgtggtgc ggagagaggc tgagacggag aagaggagag
61 gcagagaggg cgcggggacc gtcagcagca ccttagctac aatcgttcag ctattctcgg
121 aagagagaag ggagagggag gaggccgggg cgggagtggg ggctgtcacc ctcggacccc
181 ggcgtgagag gggccgtgcg gccggacgtc ctcggggtgg gcccccagtc ggtggccgaa
241 gacctacagc tcaggcccct gggtcccaaa tttccaggct ttgcccctcc tcctttctca
301 gatacccggg taacagtcct catagtccag atatccggga ctcgggtccc aacctctcta
361 aacctgggtc tctgtttcat agaatttcaa atatcaggtt caggcccctg cgtgcaccag
421 tatccggggt tcattccccg ggcgttcaaa tatcggattc agtctccatc ccgttcagat
481 attcggggtt cagaccccac aatcagaaat ccggaattcg gcagctgtcg ccctcgacga
541 gggggaggac tggaccgcga ggtcagatta ggttgtcacc ccctcccctc caggggaggc
601 ttcccggccc cgcccctcag gaagggcgaa agccgaggaa gaggtggcaa ggggaaaggt
661 ctccttgccc ctctccctgc ttggcagagc cgctggagga ccccaggcgg aagcggaggc
721 gctggggcac catagtgacc cctaccaggc caggccccac tctcagggcc cccaggggcc
781 accatgccag ctgggggccg ggccgggagc ctgaaggacc cagatgtggc tgagctcttc
841 ttcaaggatg acccagaaaa gctcttctct gacctccggg aaattggcca tggcagcttt
901 ggagccgtat actttgcccg ggatgtccgg aatagtgagg tggtggccat caagaagatg
961 tcctacagtg ggaagcagtc caatgagaaa tggcaagaca tcatcaagga ggtgcggttc
1021 ttacagaagc tccggcatcc caacaccatt cagtaccggg gctgttacct gagggagcac
1081 acggcttggc tggtaatgga gtattgcctg ggctcagctt ctgaccttct agaagtgcac
1141 aagaaacccc ttcaggaggt agagatcgca gctgtgaccc acggggcgct tcagggcctg
1201 gcatatctgc actcccacaa catgatccat agggatgtga aggctggaaa catcctgctg
1261 tcagagccag ggttagtgaa gctaggggac tttggttctg cgtccatcat ggcacctgcc
1321 aactccttcg tgggcacccc atactggatg gcacccgagg tgatcctggc catggatgag
1381 gggcagtacg atggcaaagt ggacgtctgg tccttgggga taacctgcat cgagctggct
1441 gaacggaaac caccgctctt taacatgaat gcgatgagtg ccttatacca cattgcacag
1501 aacgaatccc ccgtgctcca gtcaggacac tggtctgagt acttccggaa ttttgtcgac

1561 tcctgtcttc agaaaatccc tcaagacaga ccaacctcag aggttctcct gaagcaccgc
1621 tttgtgctcc gggagcggcc acccacagtc atcatggacc tgatccagag gaccaaggat
1681 gccgtgcggg agctggacaa cctgcagtac cgcaagatga agaagatcct gttccaagag
1741 gcacccaacg gccctggtgc cgaggcccca gaggaggaag aggaggccga gccctacatg
1801 caccgggccg ggactctgac cagcctcgag agtagccact cagtgcccag catgtccatc
1861 agcgcctcca gccagagcag ctccgtcaac agcctagcag atgcctcaga caacgaggaa
1921 gaggaggagg aggaggagga agaggaggag gaggaagaag gccctgaagc ccgggagatg
1981 gccatgatgc aggaggggga gcacacagtc acctctcaca gctccattat ccaccggctg
2041 ccgggctctg acaacctata tgatgacccc taccagccag agataacccc cagccctctc
2101 cagccgcctg cagccccagc tcccacttcc accacctctt ccgcccgccg ccgggcctac
2161 tgccgtaacc gagaccactt tgccaccatc cgaaccgcct ccctggtcag ccgtcagatc
2221 caggagcatg agcaggactc tgcgctgcgg gagcagctga gcggctataa gcggatgcga
2281 cgacagcacc agaagcagct gctggccctg gagtcacggc tgaggggtga acgggaggag
2341 cacagtgcac ggctgcagcg ggagcttgag gcgcagcggg ctggctttgg ggcagaggca
2401 gaaaagctgg cccggcggca ccaggccata ggtgagaagg aggcacgagc tgcccaggcc
2461 gaggagcgga agttccagca gcacatcctt gggcagcaga agaaggagct ggctgccctg
2521 ctggaggcac agaagcggac ctacaaactt cgcaaggaac agctgaagga ggagctccag
2581 gagaacccca gcactcccaa gcgggagaag gccgagtggc tgctgcggca gaaggagcag
2641 ctccagcagt gccaggcgga ggaggaagca gggctgctgc ggcggcagcg ccagtacttt
2701 gagctgcagt gtcgccagta caagcgcaag atgttgctgg ctcggcacag cctggaccag
2761 gacctgctgc gggaggacct gaacaagaag cagacccaga aggacttgga gtgtgcactg
2821 ctgcttcggc agcacgaggc cacgcgggag ctggagctgc ggcagctcca ggccgtgcag
2881 cgcacgcggg ctgagctcac ccgcctgcag caccagacgg agctgggcaa ccagctggag
2941 tacaacaagc ggcgtgagca agagttgcgg cagaagcatg cggcccaggt tcgccagcag
3001 cccaagagcc tcaaagtacg tgcaggccag cgcccccggg gccttccact ccccattcct
3061 ggggctctgg gcccacccaa cacaggcacc cctatagaac agcagccctg ctcacctggc
3121 caggaggcag tcctggacca aagaatgctt ggcgaggagg aggaagcagt tggagagaga
3181 aggattctgg gaaaggaagg ggccactttg gagcccaagc agcagaggat tctgggggaa
3241 gaatcaggag cccctagtcc cagtccacaa aaacatggga gcctggttga tgaggaagtt
3301 tggggtctgc ctgaggagat agaggagctt agggtgccct cccttgtacc ccaggagagg
3361 agcattgttg gccaggagga ggctgggaca tggagcttgt gggggaagga ggatgagagt
3421 cttctggatg aggagtttga gcttggctgg gtccagggcc cagcactgac tcccgtccct
3481 gaggaggagg aagaagagga agagggggct ccgattggga cccctaggga tcctggagat
3541 ggttgtcctt cccccgacat ccctcctgaa ccccctccaa cacacctgag gccctgccct
3601 gccagccagc tccctggact cctgtcccat ggcctcctgg ccggcctctc ctttgcagtg
3661 gggtcctcct ctggcctcct gcccctcctg ctgctgctgc tgcttccatt gctggcagcc
3721 cagggtgggg gtggcctgca ggcagcgctg ctggcccttg aggtggggct ggtgggtctg
3781 ggggcctcct acctgctcct ttgtacagcc ctgcacctgc cctccagtct tttcctactc
3841 ctggcccagg gtaccgcact gggggccgtc ctgggcctga gctggcgccg aggcctcatg
3901 ggtgttcccc tgggccttgg agctgcctgg ctcttagctt ggccaggcct agctctacct
3961 ctggtggcta tggcagcggg gggcagatgg gtgcggcagc agggcccccg ggtgcgccgg
4021 ggcatatctc gactctggtt gcgggttctg ctgcgcctgt cacccatggc cttccgggcc
4081 ctgcagggct gtggggctgt gggggaccgg ggtctgtttg cactgtaccc caaaaccaac
4141 aaggatggct tccgcagccg cctgcccgtc cctggccccc ggcggcgtaa tccccgcacc
4201 acccaacacc cattagctct gttggcaagg gtctgggtcc tgtgcaaggg ctggaactgg
4261 cgtctggcac gggccagcca gggtttagca tcccacttgc ccccgtgggc catccacaca
4321 ctggccagct ggggcctgct tcggggtgaa cggcccaccc gaatcccccg gctactacca
4381 cgcagccagc gccagctagg gccccctgcc tcccgccagc cactgccagg gactctagcc
4441 gggcggaggt cacgcacccg ccagtcccgg gccctgcccc cctggaggta gctgactcca
4501 gcccttccag cccaaatcta gagcattgag cactttatct cccacgactc agtgaagttt
4561 ctccagtccc tagtcctctc ttttcaccca ccttcctcag tttgctcact taccccaggc
4621 ccagcccttc ggacctctag acaggcagcc tcctcagctg tggagtccag cagtcactct
4681 gtgttctcct ggcgctcctc ccctaagtta ttgctgttcg cccgctgtgt gtgctcatcc
4741 tcaccctcat tgactcaggc ctggggccag gggtggtgga gggtgggaag agtcatgttt
4801 tttttctcct ctttgatttt gtttttctgt ctcccttcca acctgtcccc ttccccccac
4861 caaaaaaaga aaaagacaaa cacaaataaa atatctgagc ggaactgtga aaaaaaaaaa

- LOCUS: AF015731 3121 bp mRNA linear PRI 27-AUG-1997
- DEFINITION: Homo sapiens NMDAR1 subunit isoform 4b (hNMDAR1-4b) mRNA, complete cds.
- ACCESSION: AF015731
- VERSION: AF015731.1 GI:2343288

1 gcttatcgat ccgtcgacct cgaggggggg cccgcgttcg ccgcgcagag ccaggcccgc

61 cgggcgagcc catgagcacc atgcgcctgc tgacgctcgc cctgctgttc tcctgctccg
121 tcgcccgtgc cgcgtgcgac cccaagatcg tcaacattgg cgcggtgctg agcacgcgga
181 agcacgagca gatgttccgc gaggccgtga accaggccaa caagcggcac ggctcctgga
241 agattcagct caatgccacc tccgtcacgc acaagcccaa cgccatccag atggctctgt
301 cggtgtgcga ggacctcatc tccagccagg tctacgccat cctagttagc catccaccta
361 cccccaacga ccacttcact cccacccctg tctcctacac agccggcttc taccgcatac
421 ccgtgctggg gctgaccacc cgcatgtcca tctactcgga caagagcatc cacctgagct
481 tcctgcgcac cgtgccgccc tactcccacc agtccagcgt gtggtttgag atgatgcgtg
541 tgtacagctg gaaccacatc atcctgctgg tcagcgacga ccacgagggc cgggccgctc
601 agaaacgcct ggagacgctg ctggaggagc gtgagtccaa gagtaaaaaa aggaactatg
661 aaaacctcga ccaactgtcc tatgacaaca agcgcggacc caaggcagag aaggtgctgc
721 agtttgaccc agggaccaag aacgtgacgg ccctgctgat ggaggcgaaa gagctggagg
781 cccgggtcat catcctttct gccagcgagg acgatgctgc cactgtatac cgagcagccg
841 cgatgctgaa catgacgggc tccgggtacg tgtggctggt cggcgagcgc gagatctcgg
901 ggaacgccct gcgttacgcc ccggacggca tcctcgggct gcagctcatc aacggcaaga
961 acgagtcggc ccacatcagc gacgccgtag gcgtggtggc ccaggccgtg cacgagctcc
1021 tcgagaagga gaacatcacc gacccgccgc ggggctgcgt gggcaacacc aacatctgga
1081 agaccgggcc gctcttcaag agagtgctga tgtcttccaa gtatgcggat ggggtgactg
1141 gtcgcgtgga gttcaatgag gatggggacc ggaagttcgc caactacagc atcatgaacc
1201 tgcagaaccg caagctggtg caagtgggca tctacaatgg cacccacgtc atccctaatg
1261 acaggaagat catctggcca ggcggagaga cagagaagcc tcgagggtac cagatgtcca
1321 ccagactgaa gattgtgacg atccaccagg agcccttcgt gtacgtcaag cccacgctga
1381 gtgatgggac atgcaaggag gagttcacag tcaacggcga cccagtcaag aaggtgatct
1441 gcaccgggcc caacgacacg tcgccgggca gcccccgcca cacggtgcct cagtgttgct
1501 acggcttttg catcgacctg ctcatcaagc tggcacggac catgaacttc acctacgagg
1561 tgcacctggt ggcagatggc aagttcggca cacaggagcg ggtgaacaac agcaacaaga
1621 aggagtggaa tgggatgatg ggcgagctgc tcagcgggca ggcagacatg atcgtggcgc
1681 cgctaaccat aaacaacgag cgcgcgcagt acatcgagtt ttccaagccc ttcaagtacc
1741 agggcctgac tatgctggtc aagaaggaga ttccccggag cacgctggac tcgttcatgc
1801 agccgttcca gagcacactg tggctgctgg tggggctgtc ggtgcacgtg gtggccgtga
1861 tgctgtacct gctggaccgc ttcagcccct tcggccggtt caaggtgaac agcgaggagg
1921 aggaggagga cgcactgacc ctgtcctcgg ccatgtggtt ctcctggggc gtcctgctca
1981 actccggcat cggggaaggc gcccccagaa gcttctcagc gcgcatcctg ggcatggtgt
2041 gggccggctt tgccatgatc atcgtggcct cctacaccgc caacctggcc gctttcctgg
2101 tgctggaccg gccggaggag cgcatcacgg gcatcaacga ccctcggctg aggaaccctt
2161 ctgacaagtt tatctactcc acggtgaagc agagctccgt ggatatctac ttccggcgcc
2221 aggtggagct gagcaccatg taccggcata tggagaagca caactacgag agtgcggcgg
2281 aagccatcca ggccgtgaga gacaacaagc tgcatgcctt catctgggac tcggcggtgc
2341 tggagttcga ggcctcgcag aagtgcgacc tggtgacgac tggagagctg tttttccgct
2401 cgggcttcgg cataggcatg cgcaaagaca gcccctggaa gcagaacgtc tccctgtcca
2461 tcctcaagtc ccacgagaat ggcttcatgg aagacctgga caagacgtgg gttcggtatc
2521 aggaatgtga ctcgcgcagc aacgcccctg cgacccttac ttttgagaac atggccgggg
2581 tcttcatgct ggtagctggg ggcatcgtgg ccgggatctt cctgatttc atcgagattg
2641 cctacaagcg gcacaaggat gctcgccgga agcagatgca gctggccttt gccgccgtta
2701 acgtgtggcg gaagaacctg caggatagaa agagtggtag agcagagcct gaccctaaaa
2761 agaaagccac atttagggct atcacctcca ccctggcttc cagcttcaag aggcgtaggt
2821 cctccaaaga cacgcagtac catcccactg atatcacggg cccgctcaac ctctcagatc
2881 cctcggtcag caccgtggtg tgaggccccc ggaggcgccc acctgcccag ttagcccggc
2941 caaggacact gatgggtcct gctgctcggg aaggcctgag ggaagcccac ccgccccaga
3001 gactgcccac cctgggcctc ccgtccgtcc gcccgcccac cccgctgcct ggcgccaccc
3061 tgctggacca aggtgcggac cggagcggct gaggacgggg cagagctgag tcggctgggc
3121 a //

- LOCUS: NM_002082 2932 bp mRNA linear PRI 18-NOV-2006
- DEFINITION: Homo sapiens G protein-coupled receptor kinase 6 (GRK6), Transcript variant 2, mRNA.
- ACCESSION: NM_002082
- VERSION: NM_002082.2 GI:51896034

1 ggctggctgc ggcggccggg gaggccgggg aggccgcggc gcggtcactg cgagccgagc
61 cgagccgcgc cgagccgcgc cgatcgccat ccggcctcgg cactcgcgcg cgatcccggc
121 cggcggcgcg gcccggcggg ccaggcggcg ccacagccca tggagctcga gaacatcgta
181 gcgaacacgg tgctactcaa ggcccgggaa ggtggcggtg gaaatcgcaa aggcaaaagc
241 aagaaatggc ggcagatgct ccagttccct cacatcagcc agtgcgaaga gctgcggctc

301 agcctcgagc gtgactatca cagcctgtgc gagcggcagc ccattgggcg cctgctgttc
361 cgagagttct gtgccacgag gccggagctg agccgctgcg tcgccttcct ggatggggtg
421 gccgagtatg aagtgacccc ggatgacaag cggaaggcat gtgggcggca gctaacgcag
481 aattttctga gccacacggg tcctgacctc atccctgagg tcccccggca gctggtgacg
541 aactgcaccc agcggctgga gcagggtccc tgcaaagacc ttttccagga actcacccgg
601 ctgacccacg agtacctgag cgtggcccct tttgccgact acctcgacag catctacttc
661 aaccgtttcc tgcagtggaa gtggctggaa aggcagccag tgaccaaaaa caccttcagg
721 caataccgag tcctgggcaa aggtggcttt ggggaggtgt gcgcctgcca ggtgcgggcc
781 acaggtaaga tgtatgcctg caagaagcta gagaaaaagc ggatcaagaa gcggaaaggg
841 gaggccatgg cgctgaacga gaagcagatc ctggagaaag tgaacagtag gtttgtagtg
901 agcttggcct acgcctatga gaccaaggac gcgctgtgcc tggtgctgac actgatgaac
961 gggggcgacc tcaagttcca catctaccac atgggccagg ctggcttccc cgaagcgcgg
1021 gccgtcttct acgccgccga gatctgctgt ggcctggagg acctgcaccg ggagcgcatc
1081 gtgtacaggg acctgaagcc cgagaacatc ttgctggatg accacggcca catccgcatc
1141 tctgacctgg gactagctgt gcatgtgccc gagggccaga ccatcaaagg gcgtgtgggc
1201 accgtgggtt acatggctcc ggaggtggtg aagaatgaac ggtacacgtt cagccctgac
1261 tggtgggcgc tcggctgcct cctgtacgag atgatcgcag gccagtcgcc cttccagcag
1321 aggaagaaga agatcaagcg ggaggaggtg gagcggctgg tgaaggaggt ccccgaggag
1381 tattccgagc gcttttcccc gcaggcccgc tcactttgct cacagctcct ctgcaaggac
1441 cctgccgaac gcctggggtg tcgtgggggc agtgcccgcg aggtgaagga gcacccctc
1501 tttaagaagc tgaacttcaa gcggctggga gctggcatgc tggagccgcc cttcaagcct
1561 gacccccagg ccatttactg caaggatgtt ctggacattg aacagttctc tacggtcaag
1621 ggcgtggagc tggagcctac cgaccaggac ttctaccaga agtttgccac aggcagtgtg
1681 cccatcccct ggcagaacga gatggtggag accgagtgct tccaagagct gaatgtcttt
1741 gggctggatg gctcagttcc cccagacctg gactggaagg gccagccacc tgcacctcct
1801 aaaaagggac tgctgcagag actcttcagt cgccaaagga ttgctgtgga aactgcagcg
1861 acagcgagga agagctcccc acccgcctct agcccccagc ccgaggcccc caccagcagt
1921 tggcggtagc agctactccg agcgccgttt acagttttgc acagtgatct tccccattgt
1981 ccactcaagt cgtggcctgg ggaacacaga cggagctgtc cccagtgtcc tccgtccctc
2041 agcccctggc ctggctgagt ttggcagggc ctgggccatc cctgggacaa aggtgcgtcc
2101 cttcagctct tctccgtgga gctcggggct ttctgtattt atgtatttgt acgaatgtat
2161 atagcgacca gagcattctt aattcccgcc gcagacctgg cgcccccgcc ttggctcctg
2221 ggggcagcca gccctggctg ggagagcggg agctggcaga ggagccactg ccaaactcaa
2281 ggctcctctg gcccagcttg gatggctgag ggtggtcaca cccctgagcc ttcagcactg
2341 tgctggccac cccggcctct gagtaagact cgtgcctccc cctgctgccc tgggctcagg
2401 ctgctaccct ctggggccca aagctgtccc ttctcagtgc ttgtcagcgc tgggtctggg
2461 gcctctgtat gccctaggcc tgtgccaaag tggccagaga ttgggctgcc tgtgataccc
2521 atcagcccac tgccccggcc ggcccagata ggtctgcctc tgccttccag ctcccacagc
2581 ctggtccctg atactgggct ctgtcctgca gacacctctt tcagaaacgc ccaagcccag
2641 cccctaggag ggggtggggc atccctggtc aaccctcaaa cattccggac tcccctcata
2701 acaatagaca catgtgccca gcaataatcc gccccttcct gtgtgcgcct gtggggtgcg
2761 tgcgcgcgcg tgtgtacctg tgtgggtgaa ggggataggg cgaggctgtg cctgtgcccc
2821 aggtcccagc cctggccctt cccagactgt gatggccatc ctggtcccag tgttagggta
2881 gcatgggatt acagggccct gttttttcca tatttaaagc caatttttat ta //

- LOCUS: NM_016087 2894 bp mRNA linear PRI 17-NOV-2006
- DEFINITION: Homo sapiens wingless-type MMTV integration site family, member 16 (WNT16), transcript variant 2, mRNA.
- ACCESSION: NM_016087
- VERSION: NM_Ol6087.2 GI:17402913

1 agcctgcaaa aaccacagag ggcaaagcca gaaagatgga aaggcaccca tgcagctcac
61 cacttgcctc agggagaccc tcttcacagg ggcttctcaa aagacctccc tatggtggtt
121 gggcattgcc tccttcgggg ttccagagaa gctgggctgc gccaatttgc cgctgaacag
181 ccgccagaag gagctgtgca agaggaaacc gtacctgctg ccgagcatcc gagagggcgc
241 ccggctgggc attcaggagt gcgggagcca gttcagacac gagagatgga actgcatgat
301 caccgccgcc gccactaccg ccccgatggg cgccagcccc ctctttggct acgagctgag
361 cagcggcacc aaagagacag catttattta tgctgtgatg gctgcaggcc tggtgcattc
421 tgtgaccagg tcatgcagtg caggcaacat gacagagtgt tcctgtgaca ccaccttgca
481 gaacggcggc tcagcaagtg aaggctggca ctgggggggc tgctccgatg atgtccagta
541 tggcatgtgg ttcagcagaa agttcctaga tttccccatc ggaaacacca cgggcaaaga
601 aaacaaagta ctattagcaa tgaacctaca taacaatgaa gctggaaggc aggctgtcgc
661 caagttgatg tcagtagact gccgctgcca cggagtttcc ggctcctgtg ctgtgaaaac
721 atgctggaaa accatgtctt cttttgaaaa gattggccat ttgttgaagg ataaatatga

781 aaacagtatc cagatatcag acaaaacaaa gaggaaaatg cgcaggagag aaaaagatca
841 gaggaaaata ccaatccata aggatgatct gctctatgtt aataagtctc ccaactactg
901 tgtagaagat aagaaactgg gaatcccagg gacacaaggc agagaatgca accgtacatc
961 agagggtgca gatggctgca acctcctctg ctgtggccga ggttacaaca cccatgtggt
1021 caggcacgtg gagaggtgtg agtgtaagtt catctggtgc tgctatgtcc gttgcaggag
1081 gtgtgaaagc atgactgatg tccacacttg caagtaacca ctccatccag ccttgggcaa
1141 gatgcctcag caatatacaa tggcattgca accagagagg tgcccatccc tgtgcagcgc
1201 tagtaaagtt gactcttgca gtggaatccc tagaaccttg gacctgagag tttcccttac
1261 ctgatcgaca tattttcctt tatctgatca acccatcaat catgtggatt tcttgggatt
1321 ctaatgttga aaaggtttat attcaccttt tgatgatttg gggaatatat attgacatac
1381 aaggaagata atctgtttcc taagcaagaa ataacaggaa agatccctta tgccaggagg
1441 cctgccatac tcaggataag atccttgaat atggaactta gttacaggac tcaataatgg
1501 tgggtgaaca ttagtcattt ttaaaagaca cctcttatag caataaggag acattaacat
1561 gaatctcatt tattctctca gtattttaac tgaagaaatt atactgtttg tgtgtggata
1621 gaagatgttg aaaagttaac ataagcattg ggtgctgact taccctttca tgtacttcca
1681 aagaaaggta atcaaaaaga atcttcttaa gtgatataat atccctaaaa aaatgatcat
1741 tacagatgtt tagtgacaaa gaatcaatat gtaaaaagta taatgaatga tttagatttt
1801 aagtgccttt tcactgggag aatctggaaa aacctccata aggtatatag caatctttga
1861 tctttagatt catactttta tcacagatca gtttcaactg ttaaaaaccc acctctgaga
1921 tactgggggg aggatcctga aacatgcggg aaaaggagag gtaaacagtg gaggtaaaaa
1981 tataatttca tacattgtaa agaaaagcac cctttaaatg tgtaaagaca gtgttttgta
2041 aagaattttg tttaaaaagt ttctattttg taaatacagt acttaagtta tatgatttat
2101 attaaaacat ttattgacaa agcctaagag ctaaggcagt aaaattatct cataaataat
2161 attagcttat tttttttcat actattaatg ctattttttt ggacatcgaa gagaatttaa
2221 cttagcagtt agttatatgg atgtgtattt cttgctaaaa tgacagtttt atatgttata
2281 gattaaaata tgttgcaaaa tatcaaaaat ttgtgttatt tcagcagtaa gattaattga
2341 attctctttt cacattagtt atgcttaact cataaggtta ttataataaa ttatattagt
2401 aaaagtctta actggaaaaa agaatctaaa tcagaatagt gatcaatttg tggatttgat
2461 atcctggata tttattatat tttatgtaat gctgcatttc tatttgaatg ttaagtggtc
2521 tttcttgttt ttaatattca tgcatgtata ttcatcatat tttacaaggt tcctggtaaa
2581 aattacaggg ctctatttaa ggatgtattt taatgtaaat gcttatgttt tttatgaatt
2641 gttaaatatt tcagtattat atagaaaaaa atagattttt aaaattcaga atggacaaag
2701 agaatattca ttttcttatt aataagataa agaaatgttt ccctgcccca cagtcttcat
2761 tctatttctc tttaatttta ttcactgagg cagagaaaca attttgaaaa aagagcaaac
2821 ccatggaaaa tgtctcagat ctaatattaa aatcaagact aagcatttaa ctgtgaaaaa
2881 aaaaaaaaaa aaaa //

- LOCUS: NM_003027 2015 bp mRNA linear PRI 17-NOV-2006
- DEFINITION: Homo sapiens SH3-domain GRB2-like 3 (SH3GL3), mRNA.
- ACCESSION: NM_003027
- VERSION: NM_003027.2 GI:20070145

1 gcgatgtcgg tggccgggct gaagaagcag ttccacaaag ccagccagta gagatgggtt
61 tcaccatgtt ggccaatatg gtctcgatct cctgacctcg tgatccgcct gcctcggcct
121 cccagagtgc tgggattaca ggtgtgagcc actgtgccca gcctcttctc tcactatccg
181 tttctgacaa atgaaaaaaa tggatgtggg tcctgcagaa cctgaggcca gctctaacca
241 gtctttcatg gatgcataca cctgcctgca ggcgctgctc ccaggttatg aggctggttc
301 ttgggtttga ctctgggaat catgctcatt gatctgcttt tcctaatcat gtcagtgaat
361 acctacacgt gaagaaaact caagctgcac cataaagcta aaggaagaag gagcttttaa
421 gtgaataaga ccattttccc aatgagcagt gtcataaaca ttgaggactc tcttaaatca
481 gaagatgaat gaatggatgg aatctttgct gggataatat gcaatcaagc taataggtgc
541 cttacctgga cctcccaaca gctatttagt gaaaaaataa gtggtgctga aggaactaaa
601 ctagacgatg aatttcttga catggaaagg aaaatagatg ttaccaataa agttgttgca
661 gaaattcttt caaaaaccac tgaatatctt cagccaaatc cagcatacag agctaagcta
721 ggaatgctga acactgtgtc gaagatccga gggcaggtga agaccacagg atacccgcag
781 acggaaggct tgctggggga ctgtatgctg aaatacggga aggagctcgg ggaagactcc
841 acctttggca atgcattgat agaagttggt gaatccatga agctaatggc tgaggtgaaa
901 gactctcttg atattaatgt aaagcaaact tttattgatc cacttcagtt actacaagat
961 aaagatttaa aagagatcgg gcatcacctg aaaaagctgg aaggccgccg cctggattac
1021 gattataaaa agaaacgagt aggtaagata ccagacgaag aagtcagaca agcggtagaa
1081 aaatttgaag agtcaaagga gttggctgaa agaagcatgt ttaacttttt agaaaatgat
1141 gtagaacaag tcagccagtt ggctgtgttc atagaggcag cattagacta tcacagacag
1201 tccacagaga ttctgcagga gctgcagagc aagctacaga tgcgaatatc agctgcatcc
1261 agtgtcccca gacgagaata caagccaagg cctgtgaaaa ggagttctag tgagctcaat

1321 ggagtttcca ccacctctgt agtgaagacg acaggttcta acattcccat ggaccagccc
1381 tgctgtcgtg gtctctatga ctttgagcca gaaaaccaag gagaattagg atttaaagaa
1441 ggggacatca ttacattaac caatcaaata gatgaaaact ggtatgaagg aatgatacac
1501 ggagaatcgg gattcttccc cattaattac gtggaagtga tcgtgccttt acctcagtaa
1561 atgtgtaaca caaactctgg acatactttc gtaactgaaa tgaattcaca ccagtgtgct
1621 ctcagtgcgg tgttctgtga catcctttgc tctctgacca acttaatgac ttttgtatgt
1681 gtgctctctt tataatgtat tttatatcac tttaatttgt ataaatgatt ttcttgtcct
1741 tgctacatga aaatattgtt ttcttttttg cttcctgtcc taaaagtcat tggttaaatg
1801 tatttgcttc ctgtggctaa aaataagtct cacccattgc agttatgtca acgaatggcc
1861 tatattcctc agctgcaatg aaatggtaac atttgaaact aagaaatgct aaatattttg
1921 tttctcgaca ttcctgatga cgtctggtct tttcttttca ttgtatttta agcttacctg
1981 tgaatagccc aataaacatg acacactgtg ttggc//

- LOCUS: HUMNNAR 1910 bp mRNA linear PRI 07-JAN-1995
- DEFINITION: Human alpha-3 neuronal nicotinic acetylcholine receptor Subunit mRNA, complete cds.
- ACCESSION: M37981
- VERSION: M37981.1 GI:189252
- KEYWORDS: neuronal nicotinic acetylcholine receptor.

1 ggggccgtct ctgcgaccgc cgcgcccgct cccgaccgtc cgggtccgcg gccagcccgg
61 ccaccagcca tggctctggc cgctctcgct gcccctggcg ctgtcgcgcc gcggctgctg
121 ctgctgctgc tgtctctgct gccagtggcc agggcctcag aggctgagca ccgtctattt
181 gagcggctgt ttgaagatta caatgagatc atccggcctg tagccaacgt gtctgaccca
241 gtcatcatcc atttcgaggt gtccatgtct cagctggtga aggtggatga agtaaaccag
301 atcatggaga ccaacctgtg gctcaagcaa atctggaatg actacaagct gaagtggaac
361 ccctctgact atggtggggc agagttcatg cgtgtccctg cacagaagat ctggaagcca
421 gacattgtgc tgtataacaa tgctgttggg gatttccagg tggacgacaa gaccaaagcc
481 ttactcaagt acactgggga ggtgacttgg atacctccgg ccatctttaa gagctcctgt
541 aaaatcgacg tgacctactt cccgtttgat taccaaaact gtaccatgaa gttcggttcc
601 tggtcctacg ataaggcgaa aatcgatctg gtcctgatcg gctcttccat gaacctcaag
661 gactattggg agagcggcga gtgggccatc atcaaagccc caggctacaa acacgacatc
721 aagtacaact gctgcgagga gatctacccc gacatcacat actcgctgta catccggcgc
781 ctgcccttgt tctacaccat caacctcatc atcccctgcc tgctcatctc cttcctcact
841 gtgctcgtct tctacctgcc ctccgactgc ggtgagaagg tgaccctgtg catttctgtc
901 ctcctctccc tgacggtgtt tctcctggtg atcactgaga ccatcccttc cacctcgctg
961 gtcatccccc tgattggaga gtacctcctg ttcaccatga tttttgtaac cttgtccatc
1021 gtcatcaccg tcttcgtgct caacgtgcac tacagaaccc cgacgacaca cacaatgccc
1081 tcatgggtga agactgtatt cttgaacctg ctccccaggg tcatgttcat gaccaggcca
1141 acaagcaacg agggcaacgc tcagaagccg aggcccctct acggtgccga gctctcaaat
1201 ctgaattgct tcagccgcgc agagtccaaa ggctgcaagg agggttaccc ctgccaggac
1261 gggatgtgtg gttactgcca ccaccgcagg ataaaaatct ccaatttcag tgctaacctc
1321 acgagaagct ctagttctga atctgttgat gctgtgctgt ccctctctgc tttgtcacca
1381 gaaatcaaag aagccatcca aagtgtcaag tatattgctg aaaatatgaa agcacaaaat
1441 gaagccaaag agattcaaga tgattggaag tatgttgcca tggtgattga tcgtattttt
1501 ctgtgggttt tcaccctggt gtgcattcta gggacagcag gattgtttct gcaacccctg
1561 atggccaggg aagatgcata agcactaagc tgtgtgcctg cctgggagac ttccttgtgt
1621 cagggcagga ggaggctgct tcctagctgg agtgcaatgg cgtgatctca gctcaccgca
1681 acctccgcca cctgggttca agcgattctc ctgccgcagc ctcccaggta gctgggatta
1741 caggaacaaa aagcccaaga gatccaacaa ttgaaacgaa aagaaaagtc cacagaaaca
1801 tccgatcaag aacctgggct atgaatttcc aatcttcaac aacctgttag gaaaaaaata
1861 aaaatgcatt aaataattgt aaaaaaaaaa aaaaaaaaat ctagagtcga //

- LOCUS: AF229166 342 bp mRNA linear PRI 27-APR-2000
- DEFINITION: Homo sapiens docking protein 1-like protein mRNA, partial cds.
- ACCESSION: AF229166
- VERSION: AF229166.1 GI:7650361

1 agatgctgga gaactccttg tgcagcccca cctgggaagg atcccagttc tgggtaacgg
61 tgcagaggac tgaggcggtc gagcactgtg gcctgcatgc ctcctacgtg ctgagggtgg
121 aggctgaaag gctgactctc ctgaccatgg gagcccagag tcaggtaccg gagctgctcc
181 tgtcctggcc ctacactctg ttgcattgct atggccggga caaggtcatg ttctcttttg
241 aggccagcgc cgctgcctct tgggccctgg aaccttcctt tccagacggc acagggaaat
301 gacatcttcc aggcagatga gactgccatc caccggcaga ag//

- LOCUS: NM_020548 754 bp mRNA linear PRI 29-DEC-2006
- DEFINITION: Homo sapiens diazepam binding inhibitor (GABA receptor modulator, acyl-Coenzyme A binding protein) (DBI), transcript variant 1, mRNA.
- ACCESSION: NM_020548
- VERSION: NM_020548.5 GI:120433591

1 gagttcttgc aagtcggcca ggatgtctca ggtacagcgc gtgcacagcc aggctgcgaa
61 ggtgcagcgg gcgggaggcc cgttgggggc tcagccggct gccagaagct ctcgggctct
121 ttccttccgt gcccctcact tgctcatggg cccatgccta gccctgattc gttggacaga
181 gcctttgtag acccttgtct gagaccgagc tatgtggggc gacctctggc tcctcccgcc
241 tgcctctgcc aatccgggca ctgggacaga ggctgagttt gagaaagctg cagaggaggt
301 taggcacctt aagaccaagc catcggatga ggagatgctg ttcatctatg gccactacaa
361 acaagcaact gtgggcgaca taaatacaga acggcccggg atgttggact tcacgggcaa
421 ggccaagtgg gatgcctgga atgagctgaa agggacttcc aaggaagatg ccatgaaagc
481 ttacatcaac aaagtagaag agctaaagaa aaaatacggg atatgagaga ctggatttgg
541 ttactgtgcc atgtgtttat cctaaactga gacaatgcct tgtttttttc taataccgtg
601 gatggtggga attcgggaaa ataaccagtt aaaccagcta ctcaaggctg ctcaccatac
661 ggctctaaca gattaggggc taaaacgatt actgactttc cttgagtagt ttttatctga
721 aatcaattaa aagtgtattt gttactttaa ataa//

- LOCUS: AA459867 436 bp mRNA linear EST 09-JUN-1997
- DEFINITION: zx51h08.s1 Soares_testis_NHT Homo sapiens cDNA clone IMAGE:795807 3' similar to TR:G902330 G902330 PUTATIVE OLFACTORY RECEPTOR ;,mRNA sequence.
- ACCESSION: AA459867
- VERSION: AA459867.1 GI:2184774
- KEYWORDS: EST.

1 tttttgaaat acatgattat gttattggtg aaggtgtcac aacatgcaag atgggggagt
61 tgagaagggt cacagaagaa attaggaatt tccgcatcct ggaagcaggt catttgtaag
121 gcaatcaagt tgtgcagctg ggcgtctaaa acactggagg actgtgggta taggtgtcca
181 agaagaaagc tcaaatctgc cgacggtngg taccttactt gggatttact atccgacagg
241 tgtcagaacg cagcccggga acagaaagta agcaggtcat ttgcaatcta ctggagcctg
301 gaggactgtg ggtattaggt gtccaaggag aaagctcaaa tctgccgacg gaggtacctg
361 gaggactgtg ggtattaggt gtcaagaaga aagccaaatc tgctgacggc aggtacctta
421 cttgggattt actatc//

- LOCUS: AB000277 3347 bp mRNA linear PRI 05-FEB-1999
- DEFINITION: Homo sapiens mRNA for DAP-1 alpha, complete cds.
- ACCESSION: AB000277
- VERSION: AB000277.1 GI:2588977
- KEYWORDS: DAP-1 alpha.

1 tgcccaagac gggtctggag cctgacttcc gggtgtccat gaccctggcg gctgcttccg
61 gtccgccatg aaagggctat caggcagccg cagccatcac cacggggtca cctgcgactc
121 ggcctgtgac tcgctgtcgc accactccga ccgcaagccc tacctgctga gcccagtgga
181 gcaccacccc gcagaccacc catactacac ccagcggaac tccttccagg ctgagtgcgt
241 gggccccttc agcgacccgc tggccagcag caccttcccc cgcaggcact acacctcgca
301 gcaagagctg aaggacgagt gtgccctggt gccccgcacc ctggccacca aggcgaaccg
361 catccccgcc aacctgctgg accagttcga gcggcagctg ccactcagcc gcgatggcta
421 tcacaccctg cagtacaagc gcacggccgt ggagcaccgc agcgacagcc ccggccgcat
481 ccgccacctg gtgcactcgg tccagaagct cttcaccaag tcgcactccc tggagggcc
541 gtccaagggc agcgtcaacg ggggcaaggc cagccctgac gaggcgcagg cggcgcgcta
601 tggcaaacgc agcaagagca aggagcggcg cgcggagccc aaggcccggc ccagcacctc
661 cccgggctgg tggagctcgg acgacaacct ggatggtgac atgtgcatct accacgcccc
721 ctcgggcgtg atgaccatgg gcaggtgccc cgaccgctcg gcctcacagt acttcctgga
781 ggcctacaac accatcagcg agcaggcggt gaaggcctcc cggagcaaca acgacgtcaa
841 gtgctccacc tgcgccaacc tgccggtcag cctggacacc ccgctgctga agaagagcgc
901 ctggtcctcc acgctcaccg tgagccgggc ccgggaggtt taccagaagg cctcggtgaa
961 catggaccag gccatggtga agtccgagtc gtgtcagcaa gaacgctcct gccagtacct
1021 gcaggttcca caagatgaat ggacagggta caccccacga ggtaaagatg atgaaattcc
1081 atgccgaaga atgcggagtg gcagttatat caaggccatg ggggatgaag acagtggaga
1141 ctcagacacg agtcctaagc cttctccaaa agttgctgcg cggagagaaa gctatctcaa
1201 ggctactcag ccatccctta cagaactcac cacactcaaa atctccaatg aacactcacc
1261 caaactccag atccggagtc atagttacct gagggcagtg agtgaagtct ccatcaaccg

1321 gagcctggac agcctggacc ctgcaggctt gctcacatca ccaaagttcc gctccaggaa
1381 tgagagctac atgcgagcca tgagcaccat cagccaggtg agcgagatgg aagtgaacgg
1441 gcagttcgag tccgtgtgcg agtccgtgtt cagcgagctg gagtcgcagg ccgtggaagc
1501 gctggacctg cccatgcccg gctgcttccg catgcggagc cacagctatg tgcgggccat
1561 tgagaaaggc tgctcccagg acgacgagtg cgtgtccctg aggtcgtcct cgccgccgcg
1621 caccaccacc accgttagga ccatccagag cagcacggtg tcatcttgca ttacaacata
1681 taagaagaca ccacctccag tcccacccag aactaccacg aaacctttca tttctatcac
1741 agcccagagt agcacagagt cagcccagga tgcctacatg gacggacagg gccagcgagg
1801 agatattatc agccagtctg gactcagcaa ctccaccgag agcctggaca gtatgaaggc
1861 tctgacagcc gccatcgaag ctgcaaacgc ccagatccat ggccctgcca gtcaacacat
1921 gggcaataac actgccactg tcaccaccac gactaccata gccaccgtca ccacggagga
1981 caggaagaag gaccacttta agaaaaatcg atgcctgtct atcgggatac aggtggatga
2041 tgctgaagaa cctgacaaaa caggggagaa taaagcaccc agtaagttcc agtccgtggg
2101 agtgcaagta gaagaagaga agtgcttccg caggttcact cgatccaaca gtgtgacgac
2161 agcagtacag gccgacctgg acttccatga taatctggaa aattctctgg aatctataga
2221 ggacaattcg tgtcctggcc ccatggccag acagttctcc cgcgatgcca gcacctccac
2281 agtcagcatt cagggctcag gaaaccatta ccatgcctgt gccgccgatg atgactttga
2341 cacggatttt gacccctcta ttctgcctcc tccggacccc tggattgact ctatcactga
2401 agaccctctg gaggccgtgc aaaggtcagt gtgccaccgg gatggccact ggttcctgaa
2461 gcttctccag gcagagcgag accgcatgga ggggtggtgt caacagatgg agcgggaaga
2521 acgggaaaac aacctgcccg aagacattct aggaaaaatc cgaaccgcag tgggcagtgc
2581 ccaacttctc atggcccaga aattctacca gttcagagaa ctgtgtgaag aaaacctgaa
2641 tcctaatgct catccaagac ccacctccca ggatttggcg gggttttggg acatgctgca
2701 gttgtccata gaaaatatta gtatgaaatt tgatgaactt catcagttaa aggccaataa
2761 ttggaaacag atggatcctc ttgacaagaa ggagagaagg gcccctcctc cagtgccaaa
2821 gaagccggcg aagggccccg cgccgctgat ccgggagcgc tcgctggaga gctcgcagcg
2881 ccaggaggcc cgcaagcgcc tgatggccgc caagcgcgcc gcgtccgtcc gccagaactc
2941 ggccaccgag agcgccgaga gcatcgagat ctacatcccc gaggcgcaga cccggctctg
3001 agcgccccgc agcccggccg ccgccgccaa gcatctgtcc cctcctcccc cggccgctcc
3061 tctgccggct gcctctcccc ctccgagccc gtccgctccc gagctcggtg acttccactg
3121 tcgcggtgta gttgtccacc tcgcaggagc cgcccccggg gcccccctca gccccccact
3181 tcccgtaccc gtttgcccat ctccttcttc accgagcttc gccccctgtc ctgatgccgt
3241 cgccctgcct catactgaga tccaaccctt tattttctgg gcaaagccaa acccacctgt
3301 gtagaagtga tgcctttagg tcacccgccg tcctcagttc tctcgag//

- LOCUS: NM_021783 3385 bp mRNA linear PRI 17-NOV-2006
- DEFINITION: Homo sapiens ectodysplasin A2 receptor (EDA2R), mRNA.
- ACCESSION: NM_021783
- VERSION: NM_021783.2 GI:40549449

1 gccttcccac catggattgc caagaaaatg agtactggga ccaatgggga cggtgtgtca
61 cctgccaacg gtgtggtcct ggacaggagc tatccaagga ttgtggttat ggagagggtg
121 gagatgccta ctgcacagcc tgccctcctc gcaggtacaa aagcagctgg ggccaccaca
181 gatgtcagag ttgcatcacc tgtgctgtca tcaatcgtgt tcagaaggtc aactgcacag
241 ctacctctaa tgctgtctgt ggggactgtt tgcccaggtt ctaccgaaag acacgcattg
301 gaggcctgca ggaccaagag tgcatcccgt gcacgaagca gacccccacc tctgaggttc
361 aatgtgcctt ccagttgagc ttagtggagg cagatgcacc cacagtgccc cctcaggagg
421 ccacacttgt tgcactggtg agcagcctgc tagtggtgtt taccctggcc ttcctggggc
481 tcttcttcct ctactgcaag cagttcttca acagacattg ccagcgtgga ggtttgctgc
541 agtttgaggc tgataaaaca gcaaaggagg aatctctctt ccccgtgcca cccagcaagg
601 agaccagtgc tgagtcccaa gtgagtgaga acatctttca gacccagcca cttaacccta
661 tcctcgagga cgactgcagc tcgactagtg gcttccccac acaggagtcc tttaccatgg
721 cctcctgcac ctcagagagc cactcccact gggtccacag ccccatcgaa tgcacagagc
781 tggacctgca aaagttttcc agctctgcct cctatactgg agctgagacc ttggggggaa
841 acacagtcga aagcactgga gacaggctgg agctcaatgt gccctttgaa gttcccagcc
901 cttaactcta atgaggtctc ttgggcccct ggcagccttg cccagttgtt ctctctggac
961 tctgttccta taccacaaca gcagcagggg cctgaaatgt gatgtccaca agagctaata
1021 ccctacagat ggggcatatc ctatcccatc ccaccagagg attgattctc catttcacaa
1081 ggactgatct ggagcatttc ttgcttccct tttgtagtct ggggagccag attccacatt
1141 catgggacta ccagacatgt tcctagctca acttgattat agagaagagg agagaggaca
1201 gtgaatgggg tagggttttc atgtctgcat ttttggtcag gtaagcctct caaaattgtg
1261 ttggcacatc tacctagcac tttagggaca aaatcaaacc cttctcccct tttagctcct
1321 ccacactgtc tccctcctca acacacacac acacacatac acacacatat acatagacac
1381 acaaacacac acacacacat taatatctat cttgggggag gcctcgtgcc ataattccca

1441 agttcatgtc tcagactgct gcattgcagc atgaggcagg gcaaacactt tccctctaga
1501 tccctggggc ctcaccctgt atttgaggtt ctcaccaccc tcagcaggga gaagggctga
1561 agttcgccat tttggaacct tacagaacat ttctgagcca aagtaatctt ccttctgggg
1621 cctgagttcc ccaaactacc ccacagcagt ccctcaaaga cagccctcaa tccatgtagg
1681 gacatctgag tatgcctctt tctattgaaa tgtcaattca atcccagctt tctcaccacc
1741 gttccccttt gattctttct caattgtctt tttgccttta gctcccacct atacatctca
1801 tgctcagaga aaaacaagtt ccttagaggt tgtattcttt attctccaag aatctgtctg
1861 aaacttgtac agctagttcc tgtcccacaa ctattaagtg gtttattaag tacattaggc
1921 agaatgtgca cttcatcacc aggttctagc tctggcaaag gagtgctgtc tacagcaaga
1981 tttttgcttt tagaatttta ttaactacat cttttgggtt catccatcta caaacactga
2041 ttaagggccc ctggggcaac caattgatca gattactaaa aggacttggg aaaaagcaaa
2101 aaggtcccat tgtactggac tgaggattag aagcaattga aatacaagcc tgtaccaagc
2161 aagcagcctg gccccacaca ggtattagca aatatgtggt aaccaaggtt ttaggccttg
2221 gcctttaggt ttcctgtttt ttttttgttt ttgtttttgt ttttgttttt ctgcaacagg
2281 ttattcttat ctcactggct ttcactgatc atgtttagac cttctggtag aagaaataat
2341 atccagacag gggatgattt ggcttcagca ggctgcaggt gttcaaaggt tgccatgtgg
2401 ctggcagtgg ttcaagccca catttgacac tgctgctcta gaggaaagat aatgatggta
2461 acacagtaat aataataata ataacaaaaa tatgataaag tgaaagagta agacttcttc
2521 agtgtgcttg ctcatggcat gaatgctatg tggacagccc aagccatacc cagaatcacc
2581 ttaattccaa ctttttgagg ttcagcaatt ggaggtggca attggctttg cattttaaag
2641 tatttcgggt aaaggtgaag tgaaggattt tcgtctttat aatttctgtt tggccatggc
2701 aaataccata gttgagtatt tgcttcagga gagttctttt tacagtttta cttttcaatg
2761 ctgaggcata tttctttgag cactgtgctt ttatgtgtct ttctacaaag gggttattgg
2821 tcagtggaag aacaaagtac acttgataaa aacattttca acatacattg agcctaaaca
2881 gcagttaagt tgtctctaaa tgaactagca aaaaaaaaaa atgtagtttt tgtttgtaag
2941 gaaggggagg tatttcctga gaatgaattt tttttttttt ggattactgt tttctctcc
3001 atataccttg acttggattt tgacaggagg gagcctggga aaataatttt ttcctccaag
3061 attctcagat ccaggttagg aaaggattca gcactacagc atacccctct acaacataca
3121 gccctgtcac attgagatca taatccctcc tgtcccactc ctctctacca accccaccct
3181 actagctagg tcttcagtgt tttacattga atattggtac attttaatta ttttttctca
3241 taaatgggtt atttatagaa attttgttaa ctcttgagcc atatgcatgt gtagatactg
3301 gcagggctat gtttgtttat gatgctctgc aaacatttca tattggccaa taaacagaaa
3361 tatatccaaa aaaaaaaaaa aaaaa//

- LOCUS: NM_012348 945 bp mRNA linear PRI 07-FEB-2000
- DEFINITION: Homo sapiens olfactory receptor 89 (OLFR89), mRNA.
- ACCESSION: NM_012348
- VERSION: NM_012348.1 GI:6912547

1 atgccactaa ctaatgaaag ccaccctgaa gaatttattc tgctaggctt tgcagaccgc
61 ccttggctag agcttcctct gttcactagt cttcttataa tgtaccctat agccgtgatg
121 ggaaacatca caatcattct catgtccagg ttagactctc gtcttcatag ccccatgtac
181 tttttcctca ccaacctctc ctttttggac atgtgttata ccacaagcat tgtccctcag
241 atgctgttta acctgggaag ctctaagaag accatcagct atatggggtg tgcggttcag
301 ctttatttct ttcacataat ggggggaaca gaatgtttgc ttttggctat tatgtccttt
361 gatcgctatg tggccatctg cagacctctt cactacaccc tcatcatgaa tcagcgcgtc
421 tgtatcctta gtttccaccg tgtggctaat tggaataatc tatgctgtct cagaggccac
481 tgccacatta caattgccac tgtgtgctct aataaactgg accacttggt gtgtgagatt
541 cctgttctga taaagattgc ctgtggtgaa aagggttcta acgagctcac actctctgtg
601 gtatgcattt ttatgttagc tgtcccacta tgcttaattc ttgcttccta tgctagtatt
661 ggaagtgctg tatttaagat caaatcttcc aagggaagga aaaggccttt tggacatgc
721 tcctcccatc ttattgtagt tttcttattt tatggcccag ccatcagcat gtaccttcag
781 cccccctcct ccatctcaag ggatcaaccc aagttcatgg ccctcttcta tggagtggtg
841 actccctcac tcaacccttt tatctacacc ctgcggaata agaatgtaaa gggggcatta
901 cgcaacttgg tgaggagcat ttcagcttta agtgatagtg ggtag //

- LOCUS: NM_022644 1134 bp mRNA linear PRI 17-NOV-2006
- DEFINITION: Homo sapiens chorionic somatomammotropin hormone 2 (CSH2), transcript variant 2, mRNA.
- ACCESSION: NM_022644
- VERSION: NM_022644.2 GI:20819984

1 cagggagaga gaactggcca gggtataaaa agggcccaca agagaccggc tctaggatcc
61 caaggcccaa ctccccgaac cactcagggt cctgtggaca gctcacctag cggcaatggc
121 tgcaggctcc cggacgtccc tgctcctggc ttttgccctg ctctgcctgc cctggcttca

181 agaggctggt gccgtccaaa ccgttccgtt atccaggctt tttgaccacg ctatgctcca
241 agcccatcgc gcgcaccagc tggccattga cacctaccag gagtttgaag aaacctatat
301 cccaaaggac cagaagtatt cattcctgca tgactcccag acctccttct gcttctcaga
361 ctctattccg acaccctcca acatggagga aacgcaacag aaatccaatc tagagctgct
421 ccgcatctcc ctgctgctca tcgagtcgtg gctggagccc gtgcggttcc tcaggagtat
481 gttcgccaac aacctggtgt atgacacctc ggacagcgat gactatcacc tcctaaagga
541 cctagaggaa ggcatccaaa cgctgatggg ggtgagggtg gcgccagggg tcgccaatcc
601 tggaacccca ctggcttaga gggctggggg agagaaacat gctgccctct ttgtagcagt
661 caggcgctga cccaagagaa ctcaccttat tcttcatttc gcctggtaat cctccaggcc
721 cttctctaca ccctgaaggg gagggaggaa aatggatgaa tgagagaggg agggaacagt
781 gcccaagcgc ttggcctctc cttctcttcc ttcactttgc agaggctgga agacggcagc
841 cgccggactg ggcagatcct caagcagacc tacagcaagt ttgacacaaa ctcacacaac
901 catgacgcac tgctcaagaa ctacgggctg ctctactgct tcaggaagga catggacaag
961 gtcgagacat tcctgcgcat ggtgcagtgc cgctctgtag agggtagctg tggcttctag
1021 gtgcccgcgt ggcatcctgt gaccgacccc tccccagtgc ctctcctggc cctggaaggt
1081 gccactccag tgcccatcag ccttgtccta ataaaattaa gttgtatcat ttca//

- LOCUS: NM_016340 6390 bp mRNA linear PRI 31-DEC-2006
- DEFINITION: Homo sapiens Rap guanine nucleotide exchange factor (GEF) 6 (RAPGEF6), mRNA.
- ACCESSION: NM_016340

1 cccgcgcaga cttgtctccg gccagtcgcg cgtgggtaga ggccttaggc gggcgttgcg
61 aagggttgtt tgctagagtt cggaccttgt tacggcgcga agttccctcc ttggggcggg
121 ggaaaggtac cgcacccgct acctagtgtg aatgaactgt ggcgtgggct gctaagggca
181 ggctgcggag tacccgggcc gtggccatga actcacccgt ggaccctggc gctaggcagg
241 cgttgaggaa gaagccaccc gagcggactc ccgaggactt aaatactatt tattcttatc
301 ttcatggaat ggaaatatta tcaaatctca gggaacatca gcttagatta atgtctgcaa
361 gagcacgcta tgagagatac agtggcaatc aggttctctt ttgttcagaa acgattgcca
421 gatgttggta tatcctactt tctggatctg tgcttgtgaa aggctccatg gtcttgcctc
481 cttgcagttt tggtaagcag tttggaggaa aaagaggatg tgattgtctt gtattagagc
541 cttcagaaat gattgtggta gagaatgcca aagataatga agatagtatt ctacaaagag
601 aaattcctgc cagacaatcc cgaagaagat ttcggaaaat taactataaa ggagagcgcc
661 aaaccattac tgatgatgtg gaggttaaca gctatctttc tcttccagct gatcttacca
721 agatgcatct cacagaaaac cctcatccac aggtgactca tgtgtcttct agtcagtctg
781 gttgtagcat tgccagtgac tctggaagca gcagtttatc tgatatctat caggctacgg
841 agagtgaggt aggagatgta gatttgacac gtcttccaga aggacctgtt gattctgagg
901 atgacgaaga ggaagatgaa gagattgatc gaacagatcc attgcagggg cgagatcttg
961 ttcgagaatg tcttgaaaaa gaacctgcag acaaaactga tgatgacatt gaacaattgc
1021 tggagtttat gcaccagctc cctgcatttg caaacatgac catgtctgta aggagagaac
1081 tctgctcagt gatgattttt gaagtggtag agcaggctgg agctattatt cttgaagatg
1141 ggcaagagct tgactcatgg tatgttattt taaacggcac tgtggaaatc agtcatccag
1201 atggaaaagt tgaaaatttg tttatgggaa atagttttgg aattactccc actctggata
1261 agcagtacat gcatggaatt gtcaggacta aagtagatga ttgtcagttt gtctgcatag
1321 cccagcaaga ttattggaga attttaaacc atgtggaaaa aaatacccat aaagttgagg
1381 aagagggaga aattgttatg gtacatgagc atcgggaact agaccggagt ggaaccagga
1441 aaggacacat tgtgatcaag gcaacacctg agcgtctcat aatgcattta atagaagaac
1501 attccatcgt ggatccaact tatatagaag attttctatt aacttacagg acatttcttg
1561 aaagtccttt ggatgttggg atcaaactat tggaatggtt taagatcgac agcttaagag
1621 ataaggtgac acggattgta ttattatggg taaataatca ttttaatgat tttgaaggtg
1681 accctgctat gactcgattt ctagaggaat ttgaaaaaaa tctggaagat acaaagatga
1741 atggtcatct ccggttattg aatattgcct gtgctgcaaa ggctaagtgg agacaggttg
1801 tgctgcaaaa ggcttcccgc gagtcccctc tacaattcag ccttaatgga gggagtgaga
1861 agggatttgg tatttttgtt gaaggagtag aacctggtag caaagctgct gattcaggac
1921 tgaaacgtgg tgatcagatt atggaagtaa atggacaaaa ctttgagaat attacattta
1981 tgaaagccgt tgaaattttg aggaataata ctcatcttgc acttactgtg aagaccaaca
2041 tttttgtgtt caaagagtta ctttttagga ctgaacaaga gaaatctggt gttcctcata
2101 ttcccaaaat tgctgaaaaa aaaagtaatc gccattctat ccagcatgtg ccaggagata
2161 ttgaacagac atcacaggag aaaggaagta agaaagttaa agcaaatact gtttcaggtg
2221 gaagaaacaa aatcaggaag atttggataa aaacacgatt tagtatcttg cctccaaagc
2281 tatttagtga tggaggccta agccaatcac aagatgacag cattgtggga acaaggcact
2341 gtaggcatag tctggctata atgcccatcc ctggaacact ctcatccagc agccctgatc
2401 tcctgcagcc taccaccagt atgttggatt tttccaatcc ttcagatatc cctgatcaag
2461 ttataagagt tttcaaagtg gatcagcaaa gttgctacat tatcatcagt aaagacacca

2521 cagctaaaga agtagttttt catgctgttc atgaatttgg tttgaccggt gcatccgaca
2581 catattctct ctgtgaagtt tctgttactc ctgagggtgt cataaaacag agaagacttc
2641 cagatcagtt ctccaaatta gctgatagaa ttcaactcaa tggaaggtat tacttaaaaa
2701 ataacatgga aacagaaacc ttatgttcag atgaagatgc tcaagaacta gttaaggaaa
2761 gccagctatc catgctgcag ctcagtacca ttgaggtggc cacccagctg tcaatgaggg
2821 actttgattt gtttcgtaat attgaaccga ctgagtacat cgatgacctt tttaagttaa
2881 attccaaaac aggaaatact catttgaaga ggtttgagga cattgtaaac caagagacat
2941 tctgggttgc ctcagaaatt ttaactgaag caaatcagct caaacgaatg aagattatta
3001 agcattttat taaaattgca cttcattgtc gagaatgtaa gaacttcaat tccatgtttg
3061 caataataag tggcttgaac ctggcatctg tagcaagact cagaggaact tgggaaaagt
3121 taccaagcaa atacgagaaa catcttcaag atctacaaga catttttgat ccatctagaa
3181 acatggcaaa gtatagaaat attcttagta gtcaaagtat gcagcctcca attattccac
3241 tcttccctgt tgtcaagaaa gatatgacat ttctacatga aggaaatgac tccaaagtag
3301 atggtttagt aaactttgag aagttaagaa tgatttccaa ggaaatccgc caagttgttc
3361 gaatgacttc tgctaacatg gacccagcta tgatgtttcg acagaggtca ctgagtcaag
3421 gaagcacaaa ttcaaacatg ctggatgttc agggaggtgc tcacaaaaaa agggcacgcc
3481 gcagctctct gcttaatgcc aagaagctat atgaggatgc ccaaatggca aggaaggtga
3541 agcagtatct ttccagtctc gatgtagaga cagatgagga gaagttccag atgatgtcat
3601 tacagtggga gcctgcatat ggtaccttga ccaagaattt aagtgagaaa agatcagcca
3661 aatcatctga aatgtctcca gtgcctatga ggtcagctgg ccaaacaact aaagcccact
3721 tgcatcaacc ccacagagta agccaggtgc ttcaggtgcc agctgttaat ttgcacccca
3781 tcaggaagaa gggacaaaca aaagaccctg cactgaatac aagtttacct cagaaagttt
3841 taggaacaac tgaagaaata agtggtaaga agcatacaga agacactatt tctgtggcgt
3901 catctttaca ttctagtcct cctgcatctc ctcaaggctc ccctcacaaa ggttacacac
3961 ttattccatc agctaaatct gacaacttgt ctgactccag ccatagtgag atttcttcac
4021 ggtccagcat cgtgagcaat tgttctgttg actccatgtc tgcagctcta caggatgaac
4081 ggtgttcctc tcaggccctg gcagtccctg aatccactgg ggcattggaa aagacagagc
4141 acgcttcagg gataggagat catagtcaac atggccctgg gtggacactc ttgaagccat
4201 ctctaatcaa gtgtttagct gtctcatcgt ctgtgagcaa tgaagagatt tctcaagagc
4261 atatcattat agaagcagct gacagtggtc gtggaagttg gacttcgtgt tcaagcagct
4321 cccatgacaa cttccaaagc cttccaaacc caaaaagctg ggattttttg aactcttaca
4381 gacataccca tttggatgac cccattgctg aagttgaacc cactgactct gagccctatt
4441 cctgttctaa aagctgctct agaacttgtg ggcagtgtaa aggaagccta gagagaaaga
4501 gttggacctc ctccagttct ctgtctgaca cgtatgaacc aaactatggg acagttaaac
4561 ggagagtatt ggagagcacc ccagctgagt catctgaagg cttggacccc aaggatgcca
4621 ctgacccagt ttataaaact gtcacttcaa gtacagaaaa gggcttgatt gtgtactgtg
4681 tcacctcacc caagaaggac gataggtata gggagccacc tcccactcct ccaggatatt
4741 tggggatttc tttagcggac ctaaaggaag gaccccacac acacctaaaa cctccagatt
4801 atagtgtggc agtgcagagg tcaaagatga tgcataacag cctctctaga ctgccaccag
4861 cttctctcag tagcaacctc gtggcctgtg ttccatcgaa gattgtaact cagcctcaga
4921 ggcataattt gcagccattc catcctaaac taggagatgt gactgatgca gatagcgaag
4981 cagatgaaaa tgaacaagtt tcagcagtct agcctttgga tgacctattt gaaaaccact
5041 gaaagtcgtg gaggaatggg caagaaccac ctcatgattc tgcaggccat tgctaacgaa
5101 cagctcattg ctacaaccag tccagaggtt ttattccctc tactccgagc aatgaaatag
5161 acctgagtta tgcttccttt catttaattt ctgcagataa atagtttcct gagcaatgga
5221 tgctatgcct ggataccagt ctccactttg cacgccggaa ctgccttggg accacagtta
5281 cagaaaaaat gtaaactcag agtgatcctt gtgtatattg ctatagattt ttctttaaca
5341 agctatttta aagataatgg cattattatt tccaagccat agcttgggct gaaggacaaa
5401 ttgaaattgt ctgccaatac caaggatatt cttgtatatt tgaaaaataa cttattattt
5461 gaattgttgt ggttttgttt gtatttgaga gctcttgtta gctgatattc atgtttgagg
5521 tcataaaatt gtctctggtc tgaccaaaca gaagtcatct ttacagaggt gatatgcttg
5581 atctacacag agatgtgact tgatctgtag caccaataca atgtaggtct cagtttgaga
5641 gaaataggaa gccctttgca gttgaggtgt taggaacctg ctggtcatgg tgtggaaggc
5701 caaatgaagc tgccacaggg tttcttgtca gtcctttggg aaatgggagg gagtagtttg
5761 ggaggagggg tgggaaccct aatttccaca gaatgaaatt ttgatgttaa atgacatgta
5821 tacaaattct tccttaagtg aaagttatgc tgcatcgaat tgtaactgaa agtatagatc
5881 caacaaatag agactgggtt ctagagagtt ctggtctata gaaacccaaa actaaaatct
5941 ctcataactc aagtatggaa tacttttttt aaagaaattc ttatcatggg tgttgtaata
6001 atgaagacga atttgacttt atgcagtgtt ctgcagcatg cctcccccac atctcatagc
6061 accaggttgt gtctgacctg acataccctg cagctctcag ctggctgcag taacatttg
6121 tgggagaaag aggagctgga gttacagaaa tgattgtctc ttggttctca gttttagcc
6181 cttgagagga catacttttc cagcctcatg ggtatggcac tcttaattaa aatttcagtg
6241 gctgtttact ggatgaggca gatttttcac atttttgcca attaaatata ttttatatat

6301 agtaaggtta attttttcag tttttttaat gtaaaagcaa gtgaaatttt aataaacttc
6361 tgtaatttcc caaaaaaaa aaaaaaaaa//

- LOCUS: NM_005754 2855 bp mRNA linear PRI 20-DEC-2006
- DEFINITION: Homo sapiens GTPase activating protein (SH3 domain) binding protein 1 (G3BP1), transcript variant 1, mRNA.
- ACCESSION: NM_005754
- VERSION: NM_005754.2 GI:38327550

1 gagtggcagt tatatagacc ggcggcggag cacgcgtgtg tgcggacgca gttgcgtgag
61 gggtttgtac tatcctcggt gctgtggtgc agagctagtt cctctccagc tcagccgcgt
121 aggtttggac atatttgact cttttccccc caggttgaat tgaccaaagc aatggtgatg
181 gagaagccta gtcccctgct ggtcgggcgg gaatttgtga gacagtatta cacactgctg
241 aaccaggccc cagacatgct gcatagattt tatggaaaga actcttctta tgtccatggg
301 ggattggatt caaatggaaa gccagcagat gcagtctacg gacagaaaga aatccacagg
361 aaagtgatgt cacaaaactt caccaactgc cacaccaaga ttcgccatgt tgatgctcat
421 gccacgctaa atgatggtgt ggtagtccag gtgatggggc ttctctctaa caacaaccag
481 gctttgagga gattcatgca aacgtttgtc cttgctcctg aggggtctgt tgcaaataaa
541 ttctatgttc acaatgatat cttcagatac caagatgagg tctttggtgg gtttgtcact
601 gagcctcagg aggagtctga agaagaagta gaggaacctg aagaaagaca gcaaacacct
661 gaggtggtac ctgatgattc tggaactttc tatgatcagg cagttgtcag taatgacatg
721 gaagaacatt tagaggagcc tgttgctgaa ccagagcctg atcctgaacc agaaccagaa
781 caagaacctg tatctgaaat ccaagaggaa aagcctgagc cagtattaga agaaactgcc
841 cctgaggatg ctcagaagag ttcttctcca gcacctgcag acatagctca gacagtacag
901 gaagacttga ggacatttct ttgggcatct gtgaccagta agaatcttcc acccagtgga
961 gctgttccag ttactgggat accacctcat gttgttaaag taccagcttc acagccccgt
1021 ccagagtcta agcctgaatc tcagattcca ccacaaagac ctcagcggga tcaaagagtg
1081 cgagaacaac gaataaatat tcctccccaa aggggaccca gaccaatccg tgaggctggt
1141 gagcaaggtg acattgaacc ccgaagaatg gtgagacacc ctgacagtca ccaactcttc
1201 attggcaacc tgcctcatga agtggacaaa tcagagctta aagatttctt tcaaagttat
1261 ggaaacgtgg tggagttgcg cattaacagt ggtgggaaat tacccaattt tggttttgtt
1321 gtgtttgatg attctgagcc tgttcagaaa gtccttagca acaggcccat catgttcaga
1381 ggtgaggtcc gtctgaatgt cgaagagaag aagactcgag ctgccaggga aggcgaccga
1441 cgagataatc gccttcgggg acctggaggc cctcgaggtg ggctgggtgg tggaatgaga
1501 ggccctcccc gtggaggcat ggtgcagaaa ccaggatttg gagtgggaag ggggcttgcg
1561 ccacggcagt gaatcttcat ggatcttcat gcagccatac aaaccctggt tccaacagaa
1621 tggtgaattt tcgacagcct ttggtatctt ggagtatgac cccagtctgt tataaactgc
1681 ttaagtttgt ataattttac tttttttgtg tgttaatggt gtgtgctccc tctccctctc
1741 ttccctttcc tgacctttag tctttcactt ccaattttgt ggaatgatat tttaggaata
1801 acggactttt aaagaagcaa aaaaaaagac tgaatttcct tgcttacttt gcatatacag
1861 actggatttt tttttttttt ttacagccat ttccccaaag gaatgtcttg catattactg
1921 acatttggta tgtttcattc attggaatat ttcttatttt ctacgtgttt gaaaagcctg
1981 taagaaatac aggatttgat aatattttga aggcaggaaa aacccaaatt gtttcttctt
2041 tgagagtcat gactaccttc tggtgtggag aaattgccat tggaaaattt gacaattttg
2101 attctcactg gtatgtttaa aaactgaata aaaggaatag aatttttttt tgataaagga
2161 tcacaaaaca attctaaaac ctaactgttt ttaccattga aatttaaatt gtgataatag
2221 gttttaaatg tctagaatgc aactgatagg cttttcttga actgttagtt tttttgaagt
2281 agttttttca tgtttaattt gtatttgtaa aaaaacaaaa agcaaaaaaa ttcccaaaac
2341 ccagataaca accagagcaa aactgttgtg ccttctattt atctttgatt tcagtcttgg
2401 caattgttta aaaaaaaaat ctagatttgt tttattaggt tcagagtatg tggggaatta
2461 tagaatccct ctttcatcac tttgtgtatg tcttttgtta acatatttgt tatgccttat
2521 tctaaaattg agtctcaaac tggaatgcct ttgaagacag atgcttctat agaggttctt
2581 tgacctaaat agttcagcat ttgtattttt attctggtat ctaatcagat tcctaatcat
2641 agcccgtaag aaggaatgtt actttaatat tggactttgc tcatgtgctc gtgtccgcat
2701 tttttttttt cttaaaatca tagccatatg gtaaattttc tattttgtta tggttctctt
2761 ttattgatgg gcatgcagtg ggtgttactt ggaaatggcc aatttttatt aaaatatttc
2821 tggaagaaaa tttaaaaaaa aaaaaaaaaa aaaaa//

- LOCUS: AW192876 689 bp mRNA linear EST 29-NOV-1999
- DEFINITION: x154h02.x1 NCI_CGAP_Pan1 Homo sapiens cDNA clone IMAGE:2678547 3'similar to SW:KC1D_HUMAN P48730 CASEIN KINASE I, DELTA ISOFORM ;,mRNA sequence.
- ACCESSION: AW192876
- VERSION: AW192876.1 GI:6471575
- KEYWORDS: EST.

1 gaagcaagag gaggtgttta atgagttctg gttcaacaca gaggccgcca acggggtcta
61 gctatgagtc tgtcctggga cccgttccca ccaggcccct tgtgggcagt ggccaccatc
121 cctccctcag gctgtcctgg ccctcaagga ccccatggca gggccttttc caccatggcc
181 tccactgtgc tgccatctct tgggaacagg gggtcatccc atcctcccaa gaaacacttc
241 ctcaggcctg gccccgatgg gctcaggcac acctggcgac atcagaaatg gggccctgga
301 gtccagacca caggccagta gttttggggg gacagctcct ccacctcctc caccggccta
361 tctggggggc tctgggtgcc caggggagcc gggcaccagt acgtgggtga gtacgtgggc
421 ccagcacagg gacaggggcg gggacagaaa ggtcccttcg ctgtcatggg gctgtcacgg
481 ngctgagcct ggctcgagga agccccgaat ttcagcatgt tccagtcaaa gacgtagtca
541 taggagaagc cctgccggtg gaagaggttg cgtaagagct gacgtacggt agagtagtcc
601 ggcttgtcgt caaaccgcag ggagcggcag aagttgaggt atgttgagaa tttcgagtga
661 tagcctatgc agaggaccct gatgggcgt //

- LOCUS: NM_000529 3652 bp mRNA linear PRI 18-NOV-2006
- DEFINITION: Homo sapiens melanocortin 2 receptor (adrenocorticotropic hormone) (MC2R), mRNA.
- ACCESSION: NM_000529
- VERSION: NM_000529.2 GI:66346710

1 attccttctc attcattttg cccagaaagt tcctgcttca gagctgaagg tgattgggag
61 attttaactt agatctccag caagtgctac aaggaagaaa agatcctgaa gaatcaatca
121 agttttccgt gaagtcaagt ccaagtaaca tccccgcctt aaccacaagc aggagaaatg
181 aagcacatta tcaactcgta tgaaaacatc aacaacacag caagaaataa ttccgactgt
241 cctcgtgtgg ttttgccgga ggagatattt ttcacaattt ccattgttgg agttttggag
301 aatctgatcg tcctgctggc tgtgttcaag aataagaatc tccaggcacc catgtacttt
361 ttcatctgta gcttggccat atctgatatg ctgggcagcc tatataagat cttggaaaat
421 atcctgatca tattgagaaa catgggctat ctcaagccac gtggcagttt tgaaaccaca
481 gccgatgaca tcatcgactc cctgtttgtc ctctccctgc ttggctccat cttcagcctg
541 tctgtgattg ctgcggaccg ctacatcacc atcttccacg cactgcggta ccacagcatc
601 gtgaccatgc gccgcactgt ggtggtgctt acggtcatct ggacgttctg cacggggact
661 ggcatcacca tggtgatctt ctcccatcat gtgcccacag tgatcacctt cacgtcgctg
721 ttcccgctga tgctggtctt catcctgtgc ctctatgtgc acatgttcct gctggctcga
781 tcccacacca ggaagatctc caccctcccc agagccaaca tgaaaggggc catcacactg
841 accatcctgc tcggggtctt catcttctgc tgggccccct ttgtgcttca tgtcctcttg
901 atgacattct gcccaagtaa cccctactgc gcctgctaca tgtctctctt ccaggtgaac
961 ggcatgttga tcatgtgcaa tgccgtcatt gaccccttca tatatgcctt ccggagccca
1021 gagctcaggg acgcattcaa aaagatgatc ttctgcagca ggtactggta gaatggctga
1081 tccctggttt tagaatccat gggaataacg ttgccaagtg ccagaatagt gtaacattcc
1141 aacaaatgcc agtgctcctc actggccttc cttccctaat ggatgcaagg atgatcccac
1201 cagctagtgt ttctaatagc taggttctat gtgaacagtc ttattgtagg ggcaacctct
1261 taactttgtg actggacaga taaaacgatg tagtaaaaga aggatagaat acaaagtatt
1321 aggtaggtac aaaagtaatt aaggtttttg ccattacttt caatgaccaa aaattgcaat
1381 tacttttgca ccaatctagt aaaacagcaa taaaaattca agggctttgg gctaaggcaa
1441 agacttgctt tcctgtggac atctaacaag ccagttctga ggtggccttt ccaggtggag
1501 gccattgcag ccaatttcca gaagttaagt acctggacat gcgactccag gcagaagatg
1561 tagggtctct gtaagccaat aataaattgg aaggaatgca ttgctgcagc tgaatttgtc
1621 tgtctcccac agccatgtgg aatctccacc ctcctctttc tccctgttag tctgatgtat
1681 tgatgccacc tcagtttcag aaagtaggct gagtataaac tataaatgtc aaataacgag
1741 cttcgagttt ccaatgataa atggaccttc tctgttagtc ttctttgctc actcagtatc
1801 ccactggcct taaaaccctt tcctgttaca tttcctcatg ctttatgagc atacatttca
1861 aaggaagaaa tgaaaattta atccatttag ttcccatgtg ggaatacata aaggccagat
1921 gaaaattgtc actatttgaa gaagctgtaa ccaaactatg tgtgttacaa tgtagaagta
1981 caagaaaaga gccccaacat gtattttaag aaataaagag agagagacag agacagacag
2041 agagagagag agagagagag agagagagtg tgtgtgtgtg tgtgtgtgtg tgtgtgtgtg
2101 tgtgtgtgtg tgtgtgtgta ttttccccat gcttttggac tatggggaaa accaaaacca
2161 aagcaagaca tcaagcaatg gtgctgttat tatagcccca agtcaaagac ctgagggagg
2221 caaacaccac ctcattctgc agatgaatgt gaaagcagac ccagtcactg ggaaatgtca
2281 tcctcccatc agccaagatg ccagcaatgg aagagtggca accccagtag gaataaaaga
2341 aacataattt gcaagttcat tcatttttaa tagctaaaaa tcagcttaaa ggagaagcac
2401 atcctgattg taagtcccca ctaagttgga gggtgacttg aatggggtga aaggtgaaag
2461 ggacagagga gagcagtggg gcttcagagg ccaccaggct caggatctgc aggatggatg
2521 gtatcttcca gaacaggcaa tgctttgccc tcaggagaat ttcccagagc tgctgagggg
2581 agaagacagc cacacacagg acagaccatt tggtgatggg tttgatatta gaagtggcag

2641 ggacaggaac ttcagaagca aaggaggcaa ggcagctgga gtcaagtgag gacagtggca
2701 ggcgtgcttt cacatggcct gtcccacaga tggaggtgaa aggtgcacct tcttgtcctc
2761 tgttctgtag aaatccttcc ctgtttgatc cttcccctgc caaatgaact atgttactct
2821 aatactaacc tgtattaatt aatatatgag atatatataa gttaattttt catgaaatct
2881 aaagcacaac cctagaacta atttttaaaa gtgttatttc taccattgaa aaagtaatgt
2941 ataacatatt ttatgtgatt aaagtgcgta ttctcaataa gaggtaacct ttttttgatg
3001 ctgcaatgct ctgtgatacc acagaggtaa gcaatgccac ttaacctgta tcataaatag
3061 tcccaaactg ctcttcctat aaaattctgc ctttgtcaac agctttgctg tctcctaatc
3121 actctcaagc tctctgctgt gcatgtgact gttgtcagaa ggaaaatcac caagaaactt
3181 cacctctcac tgcctttgat ttgttgcagt taatctaaga aacaaaatga agatggctag
3241 tctaatggtg gatgaaacaa aaatgaagtc tgagtgctaa ttcagagaac ttgcaattcc
3301 agacattttc aattctaggt cttctgctat attccaatca gaacagaagc ttcaggctc
3361 atagttactg agaaaactca cgtttttcta cctctaactt catatagaat tccaaatgaa
3421 agcaccacca aactgcacat atttgtgtga ggaagatcaa caagcttcag acttttccca
3481 tgaggactta attcttttat caaattaccc aatttttaaa ctgctgtgtg gatactgtga
3541 gtgttcagct ttatcgatga cctagccttg gaccagatag ctgaaaatgt tcaggatgtg
3601 tactcaagct gatagtaatt ctgagccctg tctaataaaa aaggaaggat gt//

- LOCUS: NM_003696 1384 bp mRNA linear PRI 17-NOV-2006
- DEFINITION: Homo sapiens olfactory receptor, family 6, subfamily A, member 2 (OR6A2), mRNA.
- ACCESSION: NM_003696
- VERSION: NM_003696.2 GI:34330167

1 tttatttttg tatgtttgcc ttagctcatt tccagattaa agaagagagc agtggcctca
61 gctcagtgat taattacatg aactccttac gtggtattcg accaatataa gcccattaag
121 agaaaggctg ggttcttgtt gtggcctact gaagactctc tactctcatc tcctgaagag
181 cagtaagaga gaccaatgac atggagtggc ggaaccatag tgggagagtg agtgagtttg
241 tgttgctggg cttccctgct cctgcgccac tacaggtact attgtttgcc cttttgctgc
301 tggcctatgt gttggtgctg actgagaaca cactcatcat tatggcaatt aggaaccatt
361 ctaccctcca caaacccatg tacttttttc tagctaatat gtcctttctg gagatctggt
421 atgtcactgt cactattccc aagatgcttg ctggctttgt tggatccaaa caggatcatg
481 gacagctaat ctcctttgag ggatgcatga cacagctcta ctttttcctt ggcttgggct
541 gcactgagtg tgtccttctc gctgttatgg cctatgatcg ctatatggcc atctgctatc
601 ctctccacta cccagtcatt gtcagtggcc ggctgtgtgt gcagatggct gctggctctt
661 gggctggagg ttttggcatc tccatggtca aagtttttct tatttctggc ctctcttact
721 gtggccccaa catcatcaac cactttttct gtgatgtctc tccattgctc aacctctcat
781 gcactgatat gtccacagca gagcttacag atttcatcct ggccattttt attcttctag
841 ggccactctc tgtcactggg gcctcctatg tggccattac tggtgctgtg atgcacattc
901 cttcggctgc tggacgctat aaggcctttt ccacctgtgc ctctcatctc actgttgtga
961 taatcttcta tgcagccagt atcttcatct atgctcggcc aaaggcactc tcagcttttg
1021 acaccaacaa gttggtctct gtactgtatg ctgtcattgt accattgctc aatcccatca
1081 tttactgcct gcgcaatcaa gaggtcaaga gagccctatg ctgtactctg cacctgtacc
1141 agcaccagga tcctgacccc aagaaagcta gcagaaatgt atagaaggga tgtgtgaaat
1201 ctgataaaat gctattaaac ttgggatcaa tgtgttctat gaagtccagt ggagttctta
1261 gggcctaaat tgggggccag agtttcaaag gcatcactat ggaactagga atactttctt
1321 taactagtcc aagaggagag agaggaatag gttgcaagct agaaaatgga tttctgcagg
1381 acag //

- LOCUS: NM_014222 859 bp mRNA linear PRI 17-NOV-2006
- DEFINITION: Homo sapiens NADH dehydrogenase (ubiquinone) 1 alpha subcomplex, 8,19kDa (NDUFA8), nuclear gene encoding mitochondrial protein, mRNA.
- ACCESSION: NM_014222
- VERSION: NM_014222.2 GI:33519464

1 ggttgggcag ggcaatacgc ctgcgtgttg ccggatgcgc atgcgcaggc gccgtgtggc
61 actcggcggt cgaaagggga gttcaaggag acgggggcga cgcggctgag ggcttctcgt
121 cggggtcggg gctgcagccg tcatgccggg gatagtggag ctgcccactc tagaggagct
181 gaaagtagat gaggtgaaaa ttagttctgc tgtgcttaaa gctgcggccc atcactatgg
241 agctcaatgt gataagccca acaaggagtt tatgctctgc cgctgggaag agaaagatcc
301 gaggcggtgt ttagaggaag gcaaactggt caacaagtgt gctttggact tctttaggca
361 gataaaacgt cactgtgcag agccttttac agaatattgg acttgcattg attatactgg
421 ccagcagtta tttcgtcact gtcgcaaaca gcaggcaaag tttgacgagt gtgtgctgga
481 caaactgggc tgggtgcggc ctgacctggg agaactgtca aaggtcacca aagtgaaaac

541 agatcgacct ttaccggaga atccctatca ctcaagacca agaccggatc ccagccctga
601 gatcgaggga gatctgcagc ctgccacaca tggcagccgc ttttatttct ggaccaagta
661 aagatgggtc cgtggcccac actcggtcat gtgctcagac aacgactgat gaaaacgccc
721 atgcggtttg catcgactga tagtgtgttc tttccgggat cacaaacatt aacaaaaaag
781 ttaacttatg tgacttggca gttattctat accatttcct gtccattaaa atttttaaag
841 gaaaaaaaaa aaaaaaaaa //

- LOCUS: HUMCYP2BA 2907 bp mRNA linear PRI 02-NOV-1994
- DEFINITION: Human cytochrome P450-IIB (hIIB3) mRNA, complete cds.
- ACCESSION: M29873 J02864
- VERSION: M29873.1 GI:181293
- KEYWORDS: cytochrome P450; cytochrome P450 IIB.

1 ggaaccatgg agctcagcgt cctcctcttc cttgcactcc tcacaggcct cttgctactc
61 ctggttcagc gtcaccctaa ctcccatggc accctcccac cagggccccg ccctctgccc
121 cttttgggga accttctgca gatggacaga agaggcctac tcaaatcctt tctgaggttc
181 cgagagaaat atggggacgt cttcacggta cacctgggac cgaggcccgt ggtcatgctg
241 tgtggagtag aggccatacg ggaggccctg gtggacaacg ctgaggcctt ctctggccgg
301 ggaaaaatcg tcatcatgga cccagtctac cagggatatg gcatgctctt tgccaatgga
361 aaccgctgga aggtgcttcg gcgattctct gtgaccacca tgagggactt cgggatggga
421 aagcggagtg tggaggagcg gattcaggac gaggctcagt gtctgataga ggaacttcgg
481 aaatccaagg gagccctcgt ggaccccacc ttcctcttcc attccattac cgccaacatc
541 atctgctcca tcatctttgg aaaacgcttc cactaccaag atcaagagtt cctgaagacg
601 ctgaacttgt tctgccagag tttcttactc atcagctcta tatccagcca gctgtttgag
661 ctcttctctg gcttcttgaa atactttcct ggggcacaca ggcaagttta caaaaaccta
721 caggaaatca atgcttacat tggccacagt gtggagaagc accgtgaaac cctggacccc
781 agcgccccca gggacctcat cgacacctac ctgctccaca tggaaaaaga gaaatccaac
841 ccacacagtg aattcagcca ccagaacctc atcatcaaca cgctctcgct cttctttgct
901 ggcactgaga ccaccagcac cactctccgc tacggcttcc tgctcatgct caaataccct
961 catgtcgcag agagagtcta caaggagatt gaacaggtgg ttggcccaca tcgccctcca
1021 gcgcttgatg accgagccaa aatgccatac acagaggcag tcatccgtga gattcagaga
1081 tttgctgacc ttctccccat gggtgtgccc cacattgtca cccaacacac cagcttctga
1141 gggtacacca tccccaagga cacggaagta tttctcatcc tgagcactgc tctccgtgac
1201 ccacactact ttgaaaaacc agacgccttc aatcctgacc actttctgga tgccaatggg
1261 gcactgaaaa agaatgaagc ttttatcccc ttctccttag ggaagcggat ttgtcttggt
1321 gaaggcattg cccgtgcgga attgttcctc ttcttcacca ccatcctcca gaacttctcc
1381 gtggccagcc ccgtggctcc tgaagacatc gatctgacac cccaggagtg tggtgtgggc
1441 aaaatacccc caacatacca gatctgcttc ctgccccgct gaaggggctg agggaagggg
1501 gtcaaaggat tccagggtca ttcagtgtcc ccacctctgt agataatggc tctgactccc
1561 tgcaacttcc tgcctctgag agacctgctg caagccagct tccttccctt ccatggcacc
1621 agttgtctga ggtcgcagtg caaatgagtg gaggagtgag attattgaaa attataatat
1681 acaaaattat atatatatat tttgagacag agtctcactc agttgcccag gctggagtgc
1741 agtggcgtga tctcggctca ctgcaacctc cacccccggg gttcaagaaa ttctcctgcc
1801 tcagcctccc tagtagctgg gattacaggt gtgtgctacc atgcctggct aatttttgta
1861 tttttagtag agatggggtt tcaccgtgtt ggccaggctg atctcaaact cctgaactca
1921 agtgattcac ccaccttagc ctcccaaagt gctgggatta caggtgtgag tcaccatgcc
1981 cggccatgta tatatataat tttaaaaatt aagatgaaat tcacataaaa taaaattagc
2041 cattttaaag tgtacaattt agtggtgtgt ggttcattca caaagctgta caaccaccac
2101 catctagttc caaacatttt ctttttttct gagacggagt ctcactctgt cacccaggtt
2161 cgagttcagt ggtcttgaac tcctgatgtc aggtgattct cctagttcca aatgttttca
2221 ttatctctcc cccaacaaaa cccataccta tcaagctgtc actccccata ccccattctc
2281 tttttcatct cagcccctgt caatctggtt tttgtcctta tggacttacc aattctgaat
2341 atttcctata aacagaatca cacaatattt gatttttttt ttaaaactaa gccttgctct
2401 gtctcccagg ctggagtgct gtggcgtgat tttggttcac tgcaacctcc gccttccaag
2461 ttcaagagat tctcctgcct cagcttccaa gtagctggga ttacaggcat gtggtaccac
2521 gcctggctaa ttttcttgta tttttagtag ggacatgttg gccaggctgg ttgtgagctc
2581 ctggcctcag gtgatccaca cgcctcagtg tcccagagtg ctgatattac aggcgtaata
2641 tgtgatcttt tgtgtctggt tcctttcacg ttgaacgcta tttttgaggt tcgtgcctgt
2701 tgtagaccac agtcacacac tgctgtagtc ttcccccatc ctcattccca gctgcctcct
2761 cctactgttt ccctctatca aaaagcctcc ttggcgcagg ttccctgagc tgtgggattc
2821 tgcactggtg ctttggattc cctgatatgt tccttcaaat ccactgagaa ttaaataaac
2881 atcgctaaag cctgacctcc ccacgtc//

- LOCUS: NM_000284 1472 bp mRNA linear PRI 14-JAN-2007
- DEFINITION: Homo sapiens pyruvate dehydrogenase (lipoamide) alpha 1 (PDHA1),mRNA.
- ACCESSION: NM_000284
- VERSION: NM_000284.1 GI:4505684

1 actgaggcgt ggcgtctgct ggggcacctg aaggagactt gggggcaccc gcgtcgtgcc
61 tcctgggttg tgaggagtcg ccgctgccgc cactgcctgt gcttcatgag gaagatgctc
121 gccgccgtct cccgcgtgct gtctggcgct tctcagaagc cggcaagcag agtgctggta
181 gcatcccgta attttgcaaa tgatgctaca tttgaaatta agaaatgtga ccttcaccgg
241 ctggaagaag gccctcctgt cacaacagtg ctcaccaggg aggatgggct caaatactac
301 aggatgatgc agactgtacg ccgaatggag ttgaaagcag atcagctgta taaacagaaa
361 attattcgtg gtttctgtca cttgtgtgat ggtcaggaag cttgctgtgt gggcctggag
421 gccggcatca accccacaga ccatctcatc acagcctacc gggctcacgg ctttactttc
481 acccggggcc tttccgtccg agaaattctc gcagagctta caggacgaaa aggaggttgt
541 gctaaaggga aaggaggatc gatgcacatg tatgccaaga acttctacgg gggcaatggc
601 atcgtgggag cgcaggtgcc cctgggcgct gggattgctc tagcctgtaa gtataatgga
661 aaagatgagg tctgcctgac tttatatggc gatggtgctg ctaaccaggg ccagatattc
721 gaagcttaca acatggcagc tttgtggaaa ttaccttgta ttttcatctg tgagaataat
781 cgctatggaa tgggaacgtc tgttgagaga gcggcagcca gcactgatta ctacaagaga
841 ggcgatttca ttcctgggct gagagtggat ggaatggata tcctgtgcgt ccgagaggca
901 acaaggtttg ctgctgccta ttgtagatct gggaaggggc ccatcctgat ggagctgcag
961 acttaccgtt accacggaca cagtatgagt gaccctggag tcagttaccg tacacgagaa
1021 gaaattcagg aagtaagaag taagagtgac cctattatgc ttctcaagga caggatggtg
1081 aacagcaatc ttgccagtgt ggaagaacta aaggaaattg atgtggaagt gaggaaggag
1141 attgaggatg ctgcccagtt tgccacggcc gatcctgagc cacctttgga agagctgggc
1201 taccacatct actccagcga cccacctttt gaagttcgtg gtgccaatca gtggatcaag
1261 tttaagtcag tcagttaagg ggaggagaag gagaggttat accttcaggg ggctaccaga
1321 cagtgttctc aacttggtta aggaggaaga aaacccagtc aatgaaattc aatgaaattc
1381 ttggaaactt ccattaagtg tgtagattga gcaggtagta attgcatgca gtttgtacat
1441 tagtgcatta aaagatgaat tattgagtgc tt //

- LOCUS: AF000381 256 bp mRNA linear PRI 17-FEB-2004
- DEFINITION: Homo sapiens folate binding protein mRNA, partial cds.
- ACCESSION: AF000381
- VERSION: AF000381.2 GI:16041647

1 acacctgcct ctacgagtgc tcccccaact tggggccctg gatccagcag gtggatcaga
61 gctggcgcaa agagcgggta ctgaacgtgc ccctgtgcaa agaggactgt gagcaatggt
121 gggaagattg tcgcacctcc tacacctgca agagcaactg gcacaagggc tgcaactgga
181 cttcagggtt taacaagtgc gcagtgggag ctgcctgcca acctttccat ttctacttcc
241 ccacacccat tgcccg//

- LOCUS: AW024233 445 bp mRNA linear EST 09-MAR-2000
- DEFINITION: wv02c01.x1 NCI_CGAP_Kid3 Homo sapiens cDNA clone IMAGE:2528352 3'similar to TR:046686 046686 ARALKYL ACYL- COA:AMINO ACID N-ACYLTRANSFERASE ;, mRNA sequence.
- ACCESSION: AW024233
- VERSION: AW024233.1 GI:5877763
- KEYWORDS: EST.

1 actgctgatt acaatttatt atttcttttc atccaccatc cactcctcaa attatacgcc
61 cagacctgcc caacactgtc ttatgttcag gattggcatc acagaggtac acagttccac
121 tggttccagc ttctgggaat gggaacatgt tgcagtgtgt aactcatttt ttgcatagct
181 tcattgctgt agtctacatg agaatagaca ggaaacccaa gtttgcccaa tttctgggcg
241 tgggaataga tgacatacgt cacaaggcca tggagccggt attccggcaa ggtgcctgcc
301 attctcatct ctccagtctg gtccattaga tcccagcaca caggggtccc ctcaggcccc
361 aggagacagc aggtgggaaa ggtctgaatg cagcgctcaa tgaatctctg gctcctctca
421 ttaccaccaa aatgccagaa tttat //

- LOCUS: HUMCPIIA3A 1748 bp mRNA linear PRI 01-NOV-1994
- DEFINITION: Human cytochrome P450IIA3 (CYP2A3) mRNA, complete cds.
- ACCESSION: M33318 M33316
- VERSION: M33318.1 GI:180986
- 3wKEYWORDS: coumarin 7-hydroxylase; cytochrome P450; cytochrome P450 IIA3.

1 gctaccacca tgctggcctc agggatgctt ctggtggcct tgctggtctg cctgactgtg
61 atggtcttga tgtctgtttg gcagcagagg aagagcaagg ggaagctgcc tccgggaccc

121 accccattgc ccttcattgg aaactacctg cagctgaaca cagagcagat gtacaactcc
181 ctcatgaaga tcagtgagcg ctatggcccc gtgttcacca ttcacttggg gccccggcgg
241 gtcgtggtgc tgtgtggaca tgatgccgtc agggaggctc tggtggacca ggctgaggag
301 ttcagcgggc gaggcgagca agccaccttc gactgggtct tcaaaggcta tggcgtggta
361 ttcagcaacg gggagcgcgc caagcagctc cggcgcttct ccatcgccac cctgcgggac
421 ttcggggtgg gcaagcgagg catcgaggag cgcatccagg aggaggcggg cttcctcatc
481 gacgccctcc ggggcactgg cggcgccaat atcgatccca ccttcttcct gagccgcaca
541 gtctccaatg tcatcagctc cattgtcttt ggggaccgct ttgactataa ggacaaagag
601 ttcctgtcac tgttgcgcat gatgctagga atcttccagt tcacgtcaac ctccacgggg
661 cagctctatg agatgttctc ttcggtgatg aaacacctgc caggaccaca gcaacaggcc
721 tttcagttgc tgcaagggct ggaggacttc atagccaaga aggtggagca caaccagcgc
781 acgctggatc ccaattcccc acgggacttc attgactcct ttctcatccg catgcaggag
841 gaggagaaga accccaacac ggagttctac ttgaaaaacc tggtgatgac cacgttgaac
901 ctcttcattg ggggcaccga gaccgtcagc accaccctgc gctatggctt cttgctgctc
961 atgaagcacc cagaggtgga ggccaaggtc catgaggaga ttgacagagt gatcggcaag
1021 aaccggcagc ccaagtttga ggaccgggcc aagatgccct acatggaggc agtgatccac
1081 gagatccaaa gatttggaga cgtgatcccc atgagtttgg cccgcagagt caaaaaggac
1141 accaagtttc gggatttctt cctccctaag ggcaccgaag tgtaccctat gctgggctct
1201 gtgctgagag accccagttt cttctccaac ccccaggact tcaatcccca gcacttcctg
1261 aatgagaagg ggcagtttaa gaagagtgat gcttttgtgc ccttttccat cggaaagcgg
1321 aactgtttcg gagaaggcct ggccagaatg gagctctttc tcttcttcac caccgtcatg
1381 cagaacttcc gcctcaagtc ctcccagtca cctaaggaca ttgacgtgtc ccccaaacac
1441 gtgggctttg ccacgatccc acgaaactac accatgagct tcctgccccg ctgagcgagg
1501 gctgtgccgg tgcaggtctg gtgggcgggg ccagggaaag ggcagggcca agaccgggct
1561 tgggagaggg gcgcagctaa gactgggggc aggatggcgg aaaggaaggg gcgtggtggc
1621 tagagggaag agaagaaaca gaaggggctc agttcacctt gataaggtgc ttccgagctg
1681 ggatgagagg aaggaaaccc ttacattatg ctatgaagag tagtaataat agcagctctt
1741 atttcctg//

- LOCUS: NM_001740 1453 bp mRNA linear PRI 17-NOV-2006
- DEFINITION: Homo sapiens calbindin 2, 29kDa (calretinin) (CALB2), Transcript variant CALB2, mRNA.
- ACCESSION: NM_001740
- VERSION: NM_001740.2 GI:6031158

1 agcgcgagtg ccagagccca gccggcgcgg agcgggagcg gtgcaggctg aggtctccga
61 gcggctcgcc atggctggcc cgcagcagca gcccccttac ctgcacctgg ccgagctgac
121 ggcgtcccag ttcctggaaa tatggaagca ctttgacgca gacggaaatg ggtatattga
181 aggtaaagag ctagaaaact ttttccaaga gctggagaag gcaaggaaag gctctggcat
241 gatgtcaaag agtgacaact ttggagaaaa gatgaaggag ttcatgcaga agtatgataa
301 aaactcagat gggaaaatcg agatggcaga gctggcgcag atcctgccaa ccgaagagaa
361 cttccttctg tgcttcaggc agcacgtggg ctccagcgcc gagtttatgg aggcttggcg
421 gaagtacgac acagacagga gtggctacat cgaagccaat gagctcaagg gattcctgtc
481 agacctgctg aagaaggcga accggccgta cgatgagccc aagctccagg aatacaccca
541 aaccatacta cggatgtttg acttgaacgg ggatggcaaa ttgggcctct cagagatgtc
601 ccgactcctg cctgtccagg aaaacttcct gcttaaattt cagggcatga agctgacctc
661 agaggagttt aacgcgatct tcacatttta cgacaaggat agaagcggct acattgacga
721 gcatgagctg gatgcccttt tgaaggatct gtacgagaaa aacaaaaagg aaatcaatat
781 tcaacagctc accaactaca gaaagagcgt catgtccttg gcagaggcag ggaagctcta
841 ccgcaaggac ctggagattg tgctctgcag cgagcccccc atgtaaagtg gggacggggg
901 ctgcttctcc acctccccca aaccctgctt ctgctgccct gatgcgtcta cccagactca
961 gagaccgtga gcgccccgcc cccaccccta cagcctgcac acacctgcct gcagagcagg
1021 aaacgagaga tagaggatgg gcagctgggg ggctgtcctg agccccctgc acccacccct
1081 gcccaggcag tctttgctca gtggatcaca cacatggaag gtgatggggg catgggtgga
1141 gggtccctaa ttctcttcgc tgtgatgcat gagctccctc gctgtatgat ttaggcttct
1201 atgtccaaca gagtggactc ttccctctcg ctcccctctg ccggtccccc atgccaccac
1261 ccaccccaaa cttccaggtt ccatccacca ccttgccaat ggtgtagctg tcctctcaga
1321 actcctgtgt gtggaaggca cccgcccttt ccttgccttc tttactcggc gtgctccttt
1381 tctctttggg tttcttgttt accaaagaag agtttacaga caataaaatg gaaaggtcct
1441 gctgtggaaa ctt//

- LOCUS: HUMATPSCP2 599 bp mRNA linear PRI 20-FEB-1999
- DEFINITION: Homo sapiens P2 mRNA for ATP synthase subunit c, complete cds.
- ACCESSION: D13119
- VERSION: D13119.1 GI:285909
- KEYWORDS: ATP synthase; subunit c; P2.

1 tctcctgcca cagctcctca ccccctgaaa atgttcgcct gctccaagtt tgtctccact
61 ccctccttgg tcaagagcac ctcacagctg ctgagccgtc cgctatctgc agtggtgctg
121 aaacgaccgg agatactgac agatgagagc ctcagcagct tggcagtctc atgtcccctt
181 acctcacttg tctctagccg cagcttccaa accagcgcca tttcaaggga catcgacaca
241 gcagccaagt tcattggagc tggggctgcc acagttgggg tggctggttc tggggctggg
301 attggaactg tgtttgggag cctcatcatt ggttatgcca ggaacccttc tctgaagcaa
361 cagctcttct cctacgccat tctgggcttt gccctctcgg aggccatggg gctcttttgt
421 ctgatggtag cctttctcat cctctttgcc atgtgaagga gccgtctcca cctcccatag
481 ttctcccgcg tctggttggc cccgtgtgtt ccttttccta tacctcccca ggcagcctgg
541 ggaacgtggt tggctcaggg tttgacagag aaaagacaaa taaatactgt attaataag

- LOCUS: AA669797 623 bp mRNA linear EST 20-JUN-2002
- DEFINITION: ag36c01.s1 Human bone marrow stromal cells Homo sapiens cDNA clone IMAGE:1118880 3' similar to gb:M15502 FUMARATE HYDRATASE, MITOCHONDRIAL (HUMAN);, mRNA sequence.
- ACCESSION: AA669797
- VERSION: AA669797.1 GI:2631296
- KEYWORDS: EST.

1 ttttttaagt ctgttttcct ttttatttta taaatgtatt ttattttata cttgtttaat
61 ccatcttaga cctagcacat cctagggttt tatatactga tcaaattgct ctgctagaga
121 tgcttaagtt caatagcagt ttcctttcaa acttatccgt ttttaagaaa tgggagtctg
181 tttttttaaa ttttatacat gtttattttc attataaatt tatgtaaatc actttggacc
241 cagcatgtcc ttaggtttta cccattcgtc aaactgctct gctgtgagat agccaagttc
301 gatagcagtt tccgttaagg ttgatccatt tttgtgtgct gtcttagcaa tctttgctgc
361 cttgtcatac cctatatggg gattgagagc tgtcaccaac attagagact cattcatcag
421 cttgttgatc ctttctgtat tggcctggat tcccaccacg cagttttctg taaaggaaac
481 tgaagcatcc cccagcagcc tggctgagtg taacacattt ttagtcatca ttggcttgag
541 aacattcatc tccaaatgtc cattgctgcc ctcgacagtg acagcaacat ggttcccatg
601 actgtgctgc accatgtcat tgc//

- LOCUS: NM_000143 1791 bp mRNA linear PRI 14-JAN-2007
- DEFINITION: Homo sapiens fumarate hydratase (FH), nuclear gene encoding mitochondrial protein, mRNA.
- ACCESSION: NM_000143
- VERSION: NM_000143.2 GI:19743874

1 cccagaaatt ctacccaagc tccctcagca ccatgtaccg agcacttcgg ctcctcgcgc
61 gctcgcgtcc cctcgtgcgg gctccagccg cagccttagc ttcggctccc ggcttgggtg
121 gcgcggccgt gccctcgttt tggcctccga acgcggctcg aatggcaagc caaaattcct
181 tccggataga atatgatacc tttggtgaac taaaggtgcc aaatgataag tattatggcg
241 cccagaccgt gagatctacg atgaacttta agattggagg tgtgacagaa cgcatgccaa
301 ccccagttat taaagctttt ggcatcttga agcgagcggc cgctgaagta aaccaggatt
361 atggtcttga tccaaagatt gctaatgcaa taatgaaggc agcagatgag gtagctgaag
421 gtaaattaaa tgatcatttt cctctcgtgg tatggcagac tggatcagga actcagacaa
481 atatgaatgt aaatgaagtc attagcaata gagcaattga aatgttagga ggtgaacttg
541 gcagcaagat acctgtgcat cccaacgatc atgttaataa aagccagagc tcaaatgata
601 cttttcccac agcaatgcac attgctgctg caatagaagt tcatgaagta ctgttaccag
661 gactacagaa gttacatgat gctcttgatg caaaatccaa agagtttgca cagatcatca
721 agattggacg tactcatact caggatgctg ttccacttac tcttgggcag gaatttagtg
781 gttatgttca acaagtaaaa tatgcaatga caagaataaa agctgccatg ccaagaatct
841 atgagctcgc agctggaggc actgctgttg gtacaggttt aaatactaga attggctttg
901 cagaaaaggt tgctgcaaaa gtggctgcac ttacaggctt gccttttgtc actgctccga
961 ataaatttga agctctggct gctcatgacg ctctggttga gctcagtgga gccatgaaca
1021 ctactgcctg cagtctgatg aagatagcaa atgatattcg atttttgggt tctggtcctc
1081 ggtcaggtct gggagaattg atcttgcctg aaaatgaacc aggaagcagt atcatgccag
1141 gcaaggtgaa ccctactcag tgtgaagcaa tgaccatggt tgcagcccaa gtcatgggga
1201 accatgttgc tgtcactgtc ggaggcagca atggacattt tgagttgaat gttttcaagc
1261 caatgatgat taaaaatgtg ttacactcag ccaggctgct gggggatgct tcagtttcct
1321 ttacagaaaa ctgcgtggtg ggaatccagg ccaatacaga aaggatcaac aagctgatga
1381 atgagtctct aatgttggtg acagctctca atcctcatat agggtatgac aaggcagcaa
1441 agattgctaa gacagcacac aaaaatggat caaccttaaa ggaaactgct atcgaacttg

1501 gctatctcac agcagagcag tttgacgaat gggtaaaacc taaggacatg ctgggtccaa
1561 agtgatttac ataaatttat aatgaaaata aacatgtata aaatttaaaa aaacagactc
1621 ccatttctta aaaacggata agtttgaaag gaaactgcta ttgaacttaa gcatctctag
1681 cagagcaatt tgatcagtat ataaaaccct aggatgtgct aggtctaaga tggattaaac
1741 aagtataaaa taaaatacat ttataaaata aaaaggaaaa cagacttaaa a //

- LOCUS: BC005270 631 bp mRNA linear PRI 15-JUL-2006
- DEFINITION: Homo sapiens NADH dehydrogenase (ubiquinone) Fe-S protein 4, 18kDa NADH-coenzyme Q reductase), mRNA (cDNA clone MGC:12313 IMAGE:3681312), complete cds.
- ACCESSION: BC005270
- VERSION: BC005270.1 GI:13528959
- KEYWORDS: MGC.

1 atcctggcgt ttgcctgcag caagatggcg gcggtctcaa tgtcagtggt actgaggcag
61 acgttgtggc ggagaagggc agtggctgta gctgcccttt ccgtttccag ggttccgacc
121 aggtcgttga ggacttcctc atggagattg gcacaggacc agactcaaga cacacaactc
181 ataacagttg atgaaaaatt ggatatcact actttaactg gcgttccaga agagcatata
241 aaaactagaa aagtcaggat ctttgttcct gctcgcaata acatgcagtc tggagtaaac
301 aacacaaaga aatggaagat ggagtttgat accagggagc gatgggaaaa tcctttgatg
361 ggttgggcat caacggctga tcccttatcc aacatggttc taaccttcag tactaaagaa
421 gatgcagttt cctttgcaga aaaaaatgga tggagctatg acattgaaga gaggaaggtt
481 ccaaaaccca agtccaagtc ttatggtgca aacttttctt ggaacaaaag aacaagagta
541 tccacaaaat aggttggcac tgactatatc tctgcttgac tgtgaataaa gtcagctatg
601 caaaaaaaaa aaaaaaaaaa aaaaaaaaaa a//

- LOCUS: HUMHEXA 1092 bp mRNA linear PRI 08-NOV-1994
- DEFINITION: Human abnormal beta-hexosaminidase alpha chain (HEXA) mRNA, partial cds.
- ACCESSION: J04178
- VERSION: J04178.1 GI:184001
- KEYWORDS: beta-hexosaminidase; hydrolase.
Chromosome 15q23-q24. 
1 ccctgagcag aaggctctgg tgattggtgg agaggcttgt atgtggggag aatatgtgga
61 caacacaaac ctggtcccca ggctctgcta agggttttcg ggggggaggt ggagggttgg
121 gcctgagagc aggagttctc ccttagagac ccaatcccat ctagccacct ctggactagt
181 acccttcttt caggcctgag agagagcagg ccgtgcaacg agccttctga agatttgtgt
241 gcaatattca ctctccacat gacttccgtc tatatccctc cagctgccct ttggtatgtg
301 ggatagggat tgttaacctt acttcccaga gagataatga ggcctggaga acataggtga
361 gttgctcaag acccagcaca agcatccatg attcctgtaa taaggcttcc ctctgctgtt
421 ttcactgcag ccttaccaag tatggttggg tgtgcaaagt ttacatttta aggacctctg
481 ctgctgccac tgtcattgtg gttaatgatg gctttaaggt ttattcttat tcccgtatct
541 ttgagagagg aagagagtgg gattgctacc cacattttaa tgaaggtgga gctgagccgt
601 agaactctct gggagccatc caacctggct gtggctcata acaaggtatt gatcacttcc
661 tttggcctga gtgagtccag ggtgcctaga caagaggtag cagcctgtgg atgtccagca
721 cctttgcagg gaatacaggg cccaatctgg cacatgcccc ttttcctcca ggcccagagc
781 aggggctgtt gccgaaaggc tgtggagcaa caagttgaca tctgacctga catttgccta
841 tgaacgtttg tcacacttcc gctgtgagtt gctgaggtaa gcaagctgtg gggccttcgc
901 aaggcggagc aggccagatc cagggctggg gaacccctta gagagaggaa gacaataatt
961 aacaatagct aacacttaca gaggcttata gtcagccctc atccacggat tcaaccaact
1021 gcgataaaaa gtattcagga aaaaaaaaat acaacaaaaa atggtacaaa taaaaaatac
1081 tgtataacaa ct//
- LOCUS: NM_005917 1268 bp mRNA linear PRI 17-NOV-2006
- DEFINITION: Homo sapiens malate dehydrogenase 1, NAD (soluble) (MDH1), mRNA.
- ACCESSION: NM_005917
- VERSION: NM_005917.2 GI:21735619

1 ctgactctct gaggctcatt ttgcagttgt tgaaattgtc cccgcagttt tcaatcatgt
61 ctgaaccaat cagagtcctt gtgactggag cagctggtca aattgcatat tcactgctgt
121 acagtattgg aaatggatct gtctttggta aagatcagcc tataattctt gtgctgttgg
181 atatcacccc catgatgggt gtcctggacg gtgtcctaat ggaactgcaa gactgtgccc
241 ttcccctcct gaaagatgtc atcgcaacag ataaagaaga cgttgccttc aaagacctgg
301 atgtggccat tcttgtgggc tccatgccaa gaagggaagg catggagaga aaagatttac

361 tgaaagcaaa tgtgaaaatc ttcaaatccc agggtgcagc cttagataaa tacgccaaga
421 agtcagttaa ggttattgtt gtgggtaatc cagccaatac caactgcctg actgcttcca
481 agtcagctcc atccatcccc aaggagaact tcagttgctt gactcgtttg gatcacaacc
541 gagctaaagc tcaaattgct cttaaacttg gtgtgactgc taatgatgta aagaatgtca
601 ttatctgggg aaaccattcc tcgactcagt atccagatgt caaccatgcc aaggtgaaat
661 tgcaaggaaa ggaagttggt gtttatgaag ctctgaaaga tgacagctgg ctcaagggag
721 aatttgtcac gactgtgcag cagcgtggcg ctgctgtcat caaggctcga aaactatcca
781 gtgccatgtc tgctgcaaaa gccatctgtg accacgtcag ggacatctgg tttggaaccc
841 cagagggaga gtttgtgtcc atgggtgtta tctctgatgg caactcctat ggtgttcctg
901 atgatctgct ctactcattc cctgttgtaa tcaagaataa gacctggaag tttgttgaag
961 gtctccctat taatgatttc tcacgtgaga agatggatct tactgcaaag gaactgacag
1021 aagaaaaaga aagtgctttt gaatttcttt cctctgcctg actagacaat gatgttacta
1081 aatgcttcaa agctgaagaa tctaaatgtc gtctttgact caagtaccaa ataataataa
1141 tgctatactt aaattacttg tgaaaaacaa cacattttaa agattacgtg cttcttggta
1201 caggtttgtg aatgacagtt tatcgtcatg ctgttagtgt gcattctaaa taaatatata
1261 ttcaaatg //

- LOCUS: NM_001190 1595 bp mRNA linear PRI 18-NOV-2006
- DEFINITION: Homo sapiens branched chain aminotransferase 2, mitochondrial(BCAT2), mRNA.
- ACCESSION: NM_001190
- VERSION: NM_001190.2 GI:50658083

1 gcgccccggg cgggcacagt tacgcgccgc acggatcatg gccgcagccg ctctggggca
61 gatctgggca cgaaagcttc tctctgtccc ttggcttctg tgtggtccca gaagatatgc
121 ctcctccagt ttcaaggctg cagacctgca gctggaaatg acacagaagc ctcataagaa
181 gcctggcccc ggcgagcccc tggtgtttgg gaagacattt accgaccaca tgctgatggt
241 ggaatggaat gacaagggct ggggccagcc ccgaatccag cccttccaga acctcacgct
301 gcacccagcc tcctccagcc tccactactc cctgcagctg tttgagggca tgaaggcgtt
361 caaaggcaaa gaccagcagg tgcgcctctt ccgcccctgg ctcaacatgg accggatgct
421 gcgctcagcc atgcgcctgt gcctgccgag tttcgacaag ctggagttgc tggagtgcat
481 ccgccggctc atcgaagtgg acaaggactg ggtccccgat gccgccggca ccagcctcta
541 tgtgcggcct gtgctcattg ggaacgagcc ctcgctgggt gtcagccagc ccacgcgcgc
601 gctcctgttc gtcattctct gcccagtggg tgcctacttc cctggaggct ccgtgacccc
661 ggtctccctc ctggccgacc cagccttcat ccgggcctgg gtgggcgggg tcggcaacta
721 caagttaggt gggaattatg ggcccaccgt gttagtgcaa caggaggcac tcaagcgggg
781 ctgtgaacag gtcctctggc tgtatgggcc cgaccaccag ctcaccgagg tgggaaccat
841 gaacatcttt gtctactgga cccacgaaga tggggtgctg gagctggtga cgcccccgct
901 gaatggtgtt atcctgcctg gagtggtcag acagagtcta ctggacatgg ctcagacctg
961 gggtgagttc cgggtggtgg agcgcacgat caccatgaag cagttgctgc gggccctgga
1021 ggagggccgc gtgcgggaag tctttggctc gggcaccgct tgccaggtct gcccagtgca
1081 ccgaatcctg tacaaagaca ggaacctcca cattcccacc atggaaaatg ggcctgagct
1141 gatcctccgc ttccagaagg agctgaagga gatccagtac ggaatcagag cccacgagtg
1201 gatgttcccg gtgtgaagct gcaggctgtg ctccagatcc accgacccgt agcatctcgt
1261 cacgccagca ctcgcctccc taccaatgac tcacctgaag tgcaatacga aataaaaggc
1321 cagcgggcgg cgtctgggtc tctggcgccc ccatgtggtt gcgacactcc caaagccgta
1381 agggccgacc caggcatctt ggcccccagc ccctcgtcgc gggttcaggt ccgcccatta
1441 ctccttgtcg tgcggtcaag gatacacctt ggccccgatt ccggatctct ccgttctcag
1501 gccagacccc tggtgctgcc gttgattttt ttttctctgt ctttgctgca attttgaaat
1561 aaaatgccaa agaacacaca aaaaaaaaaa aaaaa//

- LOCUS: AY028632 1584 bp mRNA linear PRI 27-APR-2004
- DEFINITION: Homo sapiens catalase (CAT) mRNA, complete cds.
- ACCESSION: AY028632
- VERSION: AY028632.1 GI:13562131

1 atggctgaca gccgggatcc cgccagcgac cagatgcagc actggaagga gcagcgggcc
61 gcgcagaaag ctgatgtcct gaccactgga gctggtaacc cagtaggaga caaacttaat
121 gttattacag tagggccccg tgggcccctt cttgttcaga atgtggtttt cactgatgaa
181 atggctcatt ttgaccgaga gagaattcct gagagagttg tgcatgctaa aggagcaggg
241 gcctttggct actttgaggt cacacatgac attaccaaat actccaaggc aaaggtattt
301 gagcatattg gaaagaagac tcccatcgca gttcggttct ccactgttgc tggagaatcg
361 ggttcagctg acacagttcg ggaccctcgt gggtttgcag tgaaatttta cacagaagat
421 ggtaactggg atctcgttgg aaataacacc cccattttct tcatcaggga tcccatattg
481 tttccatctt ttatccacag ccaaaagaga aatcctcaga cacatctgaa ggatccggac

541 atggtctggg acttctggag cctacgtcct gagtctctgc atcaggtttc tttcttgttc
601 agtgatcggg ggattccaga tggacatcgc cacatgaatg gatatggatc acatactttc
661 aagctggtta atgcaaatgg ggaggcagtt tattgcaaat tccattataa gactggccag
721 ggcatcaaaa acctttctgt tgaagatgcg gcgagacttt cccaggaaga tcctgactat
781 ggcatccggg atctttttaa cgccattgcc acaggaaagg acccctcctg gactttttac
841 atccaggtca tgacatttaa tcaggcagaa acttttccat ttaatccatt cgatctcacc
901 agggtttggc ctcacaagga ctaccctctc atcccagttg gtaaactggt cttaaaccgg
961 aatccagtta attactttgc tgaggttgaa cagatagcct tcgacccaag caacatgcca
1021 cctggcattg aggccagtcc tgacaaaatg cttcagggcc gcctttttgc ctatcctgac
1081 actcaccgcc atcgcctggg acccaattat cttcatatac ctgtgaactg tccctaccgt
1141 gctcgagtgg ccaactacca gcgtgacggc ccgatgtgca tgcaggacaa tcagggtggt
1201 gctccaaatt actaccccaa cagctttggt gctccggaac aacagccttc tgccctggag
1261 cacagcatcc aatattctgg agaagtgcgg agattcaaca ctgccaatga tgataacgtt
1321 actcaggtgc gggcattcta tgtgaacgtg ctgaatgagg aacagaggaa acgtctgtgt
1381 gagaacattg ccggccacct gaaggatgca caaattttca tccagaagaa agcggtcaag
1441 aacttcactg aggtccaccc tgactacggg agccacatcc aggctcttct ggacaagtac
1501 aatgctgaga agcctaagaa tgcgattcac acctttgtgc ggtccggatc tcacttggtg
1561 gcaagggaga aggcaaatct gtga//

- LOCUS: NM_003000 1161 bp mRNA linear PRI 04-FEB-2007
- DEFINITION: Homo sapiens succinate dehydrogenase complex, subunit B, iron sulfur (Ip) (SDHB), mRNA.
- ACCESSION: NM_003000
- VERSION: NM_003000.2 GI:115387093

1 acccgggaaa ccggaagccg cctcccactt ggttgctcgt acgcggctag tgggtcctca
61 gtggatgtag gctgggcgcc gcgatgttcg acgggacacc ggcggagagc gacctcgggg
121 ttaaggggtg gggctgacgt caggagccaa gatggcggcg gtggtcgccc tctccttgag
181 gcgccggttg ccggccacaa cccttggcgg agcctgcctg caggcctccc gaggagccca
241 gacagctgca gccacagctc cccgtatcaa gaaatttgcc atctatcgat gggacccaga
301 caaggctgga gacaaacctc atatgcagac ttatgaagtt gaccttaata aatgtggccc
361 catggtattg gatgctttaa tcaagattaa gaatgaagtt gactctactt tgaccttccg
421 aagatcatgc agagaaggca tctgtggctc ttgtgcaatg aacatcaatg gaggcaacac
481 tctagcttgc acccgaagga ttgacaccaa cctcaataag gtctcaaaaa tctaccctct
541 tccacacatg tatgtgataa aggatcttgt tcccgatttg agcaacttct atgcacagta
601 caaatccatt gagccttatt tgaagaagaa ggatgaatct caggaaggca agcagcagta
661 tctgcagtcc atagaagagc gtgagaaact ggacgggctc tacgagtgca ttctctgtgc
721 ctgctgtagc accagctgcc ccagctactg gtggaacgga gacaaatatc tggggcctgc
781 agttcttatg caggcctatc gctggatgat tgactccaga gatgacttca cagaggagcg
841 cctggccaag ctgcaggacc cattctctct ataccgctgc cacaccatca tgaactgcac
901 aaggacctgt cctaagggtc tgaatccagg gaaagctatt gcagagatca agaaaatgat
961 ggcaacctat aaggagaaga aagcttcagt ttaactgttt ccatgctaaa catgatttat
1021 aaccagctca gagctgaaca taatttatat ctaatttgag ttcctttaaa gatcttggtt
1081 ttccatgaat acagcatgta taataaaaat tttaagaaat aaatgttatt ctactttatt
1141 aacaaaaaaa aaaaaaaaaa a//

- LOCUS: NM_001869 1306 bp mRNA linear PRI 17-NOV-2006
- DEFINITION: Homo sapiens carboxypeptidase A2 (pancreatic) (CPA2), mRNA.
- ACCESSION: NM_001869
- VERSION: NM_001869.1 GI:4502998

1 ggccatgagg ttgatcctgt tttttggtgc cctttttggg catatctact gtctagaaac
61 atttgtggga gaccaagttc ttgagattgt accaagcaat gaagaacaaa ttaaaaatct
121 gctacaattg gaggctcaag aacatctcca gcttgatttt tggaaatcac ccaccacccc
181 aggggagaca gcccacgtcc gagttccctt cgtcaacgtc caggcagtca aagtgttctt
241 ggagtcccag ggaattgcct attccatcat gattgaagac gtgcaggtcc tgttggacaa
301 agagaatgaa gaaatgcttt ttaataggag aagagaacgg agtggtaact tcaattttgg
361 ggcctaccat accctggaag agatttccca agaaatggat aacctcgtgg ctgagcaccc
421 tggtctagtg agcaaagtga atattggctc ttcttttgag aaccggccta tgaacgtgct
481 caagttcagc accggaggag acaagccagc tatctggctg gatgctggga tccatgctcg
541 agagtgggtt acacaagcta cggcactttg gacagcaaat aagattgttt ctgattatgg
601 aaaggaccca tccatcactt ccattctgga cgccctggat atcttcctcc tgccagtcac
661 aaaccctgat ggatacgtgt tctctcaaac caaaaatcgt atgtggcgga agacccggtc
721 caaggtatct ggaagcctct gtgttggtgt ggatcctaac cggaactggg atgcaggttt
781 tggaggacct ggagccagca gcaacccttg ctctgattca taccacggac ccagtgccaa

841 ctctgaagtt gaagtgaaat ccatagtgga cttcatcaag agtcatggaa aagtcaaggc
901 cttcattatc ctccacagct attcccagct gctgatgttc ccctatgggt acaaatgtac
961 caagttagat gactttgatg agctgagtga agtggcccaa aaggctgccc aatctctgag
1021 aagcctgcat ggcaccaagt acaaagtggg accaatctgc tctgtcatct accaagccag
1081 tggaggaagc attgactggt cctatgatta tggcatcaag tactcatttg cctttgaact
1141 gagagacaca gggcgctacg gcttcctctt gccagcccgt cagatcctgc ccacagccga
1201 ggagacctgg cttggcttga aggcaatcat ggagcatgtg cgagaccacc cctattaggg
1261 ccctggggaa gaaacaagag ccattaaaat ctctttggtt tgaagc//

- LOCUS: NM_004547 541 bp mRNA linear PRI 17-NOV-2006
- DEFINITION: Homo sapiens NADH dehydrogenase (ubiquinone) 1 beta subcomplex, 4,15kDa (NDUFB4), nuclear gene encoding mitochondrial protein, mRNA.
- ACCESSION: NM_004547
- VERSION: NM_004547.4 GI:40806201.

1 gcacttcctg aagctgaagg tcagcataga caaagggcct cagaatcgcg caggcgcaat
61 tgtgccctgg ttcgccaaga tgtcgttccc aaagtataag ccgtcgagcc tgcgcactct
121 gcctgagacc ctcgacccag ccgaatacaa catatctccg gaaacccggc gggcgcaagc
181 cgagcggttg gccataagag cccagctgaa acgagagtac ctgcttcagt acaacgatcc
241 caaccgccga gggctcatcg aaaatcctgc cttgcttcgt tgggcctatg caagaacaat
301 aaatgtctat cctaatttca gacccactcc taaaaactca ctcatgggag ctctgtgtgg
361 atttgggccc ctcatcttca tttattatat tatcaaaact gagagggata ggaaagaaaa
421 acttatccag gaaggaaaat tggatcgaac atttcacctc tcatattaag tctggcaatg
481 atgactatat gtattcctgc ctaaataaat catctattaa tcattaaaaa aaaaaaaaaa
541 a //

- LOCUS: NM_000281 1025 bp mRNA linear PRI 18-NOV-2006
- DEFINITION: Homo sapiens pterin-4 alpha-carbinolamine dehydratase/dimerization cofactor of hepatocyte nuclear factor 1 alpha (TCF1) (PCBD1), mRNA.
- ACCESSION: NM_000281
- VERSION: NM_000281.2 GI:50086629

1 cggggtggga ttccaaccca gaagcgggca tttggagccc tcatgcttca ccctagctcc
61 ctgaaacaag cacggaggat ggctctgagc agcgaggaaa gtgctggaag caagcaggcc
121 gagcgcgtcc ctggctgggg acgttaatca ttaccggagg gcggcccgag cgcggccccg
181 ccccgggacg gcagcctgcg cgcccggccg ccgcctgccc tctccgctgg ccacctgctg
241 ccgcccgcgc catggctggc aaagcacaca ggctgagcgc tgaggagagg gaccagctgc
301 tgccaaacct gagggctgtg gggtggaatg agctggaagg ccgtgatgcc atcttcaagc
361 agtttcattt caaagacttc aacagggcct ttgggttcat gacaagagtg gccctgcagg
421 ctgagaaact ggaccaccat cctgaatggt ttaacgtgta caacaaggtc cacatcacgc
481 tgagcaccca tgagtgtgcc ggcctttcag aacgggacat aaacctggcc agcttcatcg
541 aacaagtagc agtgtccatg acatagaccc tgcccttcct ctttgaattc ttccgggga
601 aggggtgact gaactgggag tccagggagg gagctgagga gcccttaccc tcccaccact
661 cccctcccaa gacccagccg ccgccgttga gggctgagtc cttgctgtgg gatgtgccag
721 tgtccccacc aacaccagga atttagacct tttccctgca ccactctctt catcctgggg
781 gctctgttac actaatttga ataaactctc ccctttcttt gcaacttccc agcaacaata
841 atgattttct tgccaggccg tctcttgctc cctaattcat ttcccaggaa gctgtgatac
901 agggtgaaat aaagtcttgt cttagaaacc aggaccctaa accccacact atgtaataga
961 aacacatgtg tttttatgtc tcaaataaaa ctattatatc acttggaaaa aaaaaaaaaa
1021 aaaaa//

- LOCUS: NM_000818 2457 bp mRNA linear PRI 08-DEC-2006
- DEFINITION: Homo sapiens glutamate decarboxylase 2 (pancreatic islets and brain, 65kDa) (GAD2), mRNA.
- ACCESSION: NM_000818
- VERSION: NM_000818.1 GI:4503874

1 acccgccctc gccgctcggc cccgcgcgtc cccgcgcgtg ccctcctccc gccacacggc
61 acgcacgcgc gcgcagggcc aagccgaggc agccgcccgc agctcgcact cgctggcgac
121 ctgctccagt ctccaaagcc gatggcatct ccgggctctg gcttttggtc tttcgggtcg
181 gaagatggct ctggggattc cgagaatccc ggcacagcgc gagcctggtg ccaagtggct
241 cagaagttca cgggcggcat cggaaacaaa ctgtgcgccc tgctctacgg agacgccgag
301 aagccggcgg agagcggcgg gagccaaccc ccgcgggccg ccgcccggaa ggccgcctgc
361 gcctgcgacc agaagccctg cagctgctcc aaagtggatg tcaactacgc gtttctccat

421 gcaacagacc tgctgccggc gtgtgatgga gaaaggccca ctttggcgtt tctgcaagat
481 gttatgaaca ttttacttca gtatgtggtg aaaagtttcg atagatcaac caaagtgatt
541 gatttccatt atcctaatga gcttctccaa gaatataatt gggaattggc agaccaacca
601 caaaatttgg aggaaatttt gatgcattgc caaacaactc taaaatatgc aattaaaaca
661 gggcatccta gatacttcaa tcaactttct actggtttgg atatggttgg attagcagca
721 gactggctga catcaacagc aaatactaac atgttcacct atgaaattgc tccagtattt
781 gtgcttttgg aatatgtcac actaaagaaa atgagagaaa tcattggctg gccagggggc
841 tctggcgatg ggatattttc tcccggtggc gccatatcta acatgtatgc catgatgatc
901 gcacgcttta agatgttccc agaagtcaag gagaaaggaa tggctgctct tcccaggctc
961 attgccttca cgtctgaaca tagtcatttt tctctcaaga agggagctgc agccttaggg
1021 attggaacag acagcgtgat tctgattaaa tgtgatgaga gagggaaaat gattccatct
1081 gatcttgaaa gaaggattct tgaagccaaa cagaaagggt ttgttccttt cctcgtgagt
1141 gccacagctg gaaccaccgt gtacggagca tttgaccccc tcttagctgt cgctgacatt
1201 tgcaaaaagt ataagatctg gatgcatgtg gatgcagctt ggggtggggg attactgatg
1261 tcccgaaaac acaagtggaa actgagtggc gtggagaggg ccaactctgt gacgtggaat
1321 ccacacaaga tgatgggagt ccctttgcag tgctctgctc tcctggttag agaagaggga
1381 ttgatgcaga attgcaacca aatgcatgcc tcctacctct ttcagcaaga taaacattat
1441 gacctgtcct atgacactgg agacaaggcc ttacagtgcg gacgccacgt tgatgttttt
1501 aaactatggc tgatgtggag ggcaaagggg actaccgggt ttgaagcgca tgttgataaa
1561 tgtttggagt tggcagagta tttatacaac atcataaaaa accgagaagg atatgagatg
1621 gtgtttgatg ggaagcctca gcacacaaat gtctgcttct ggtacattcc tccaagcttg
1681 cgtactctgg aagacaatga agagagaatg agtcgcctct cgaaggtggc tccagtgatt
1741 aaagccagaa tgatggagta tggaaccaca atggtcagct accaaccctt gggagacaag
1801 gtcaatttct tccgcatggt catctcaaac ccagcggcaa ctcaccaaga cattgacttc
1861 ctgattgaag aaatagaacg ccttggacaa gatttataat aaccttgctc accaagctgt
1921 tccacttctc tagagaacat gccctcagct aagcccccta ctgagaaact tcctttgaga
1981 attgtgcgac ttcacaaaat gcaaggtgaa caccactttg tctctgagaa cagacgttac
2041 caattatgga gtgtcaccag ctgccaaaat cgtaggtgtt ggctctgctg gtcactggag
2101 tagttgctac tcttcagaat atggacaaag aaggcacagg tgtaaatata gtagcaggat
2161 gaggaacctc aaactgggta tcatttgcac gtgctcttct gttctcaaat gctaaatgca
2221 aacactgtgt atttattagt taggtgtgcc aaactaccgt tcccaaattg gtgtttctga
2281 atgacatcaa cattccccca acattactcc attactaaag acagaaaaaa ataaaaacat
2341 aaaatataca aacatgtggc aacctgttct tcctaccaaa tataaacttg tgtatgatcc
2401 aagtatttta tctgtgttgt ctctctaaac ccaaataaat gtgtaaatgt ggacaca//

- LOCUS: AF280110 1349 bp mRNA linear PRI 26-MAR-2001
- DEFINITION: Homo sapiens clone 15b cytochrome P450 subfamily IIIA polypeptide 43 (CYP3A43) mRNA, complete cds, alternatively spliced.
- ACCESSION: AF280110
- VERSION: AF280110.1 GI:11225241

1 atggatctca ttccaaactt tgccatggaa acatgggttc ttgtggctac cagcctggta
61 ctcctctata tttatgggac ccattcacat aaacttttta agaagctggg aattcctggg
121 ccaacccctc tgccttttct gggaactatt ttgttctacc ttaggggtct ttggaatttt
181 gacagagaat gtaatgaaaa atacggagaa atgtgggggc tgtatgaggg gcaacagccc
241 atgctggtca tcatggatcc cgacatgatc aaaacagtgt tagtgaaaga atgttactct
301 gtcttcacaa accagatgcc tttaggtcca atgggatttc tgaaaagtgc cttaagtttt
361 gctgaagatg aagaatggaa gagaatacga acattgctat ctccagcttt caccagtgta
421 aaattcaagg aaatggtccc catcatttcc caatgtggag atatgttggt gagaagcctg
481 aggcaggaag cagagaacag caagtccatc aacttgaaag atttctttgg ggcctacacc
541 atggatgtaa tcactggcac attatttgga gtgaacttgg attctctcaa caatccacaa
601 gatccctttc tgaagaatat gaagaagctt ttaaaattgg attttttgga tcccttttta
661 ctcttaatat cactctttcc atttcttacc ccagtttttg aagccctaaa tatcggtttg
721 tttccaaaag atgttaccca ttttttaaaa aattccattg aaaggatgaa agaaagtcgc
781 ctcaaagata aacaaaagca tcgagtagat ttctttcaac agatgatcga ctcccagaat
841 tccaaagaaa caaagtccca taaagctctg tctgatctgg agcttgtggc ccagtcaatt
901 atcatcattt ttgctgccta tgacacaact agcaccactc tccccttcat tatgtatgaa
961 ctggccactc accctgatgt ccagcagaaa ctgcaggagg agattgacgc agttttaccc
1021 aataaggcac ctgtcaccta cgatgccctg gtacagatgg agtaccttga catggtggtg
1081 aatgaaacgc tcagattatt cccagttgtt agtagagtta cgagagtctg caagaaagat
1141 attgaaatca atggagtgtt cattcccaaa gggttagcag tgatggttcc aatctatgct
1201 cttcaccatg acccaaagta ctggacagag cctgagaagt tctgccctga aagatcccac
1261 tgaaattaga caatctacca attcttcaac cagaaaaacc tattgtttta aaagtgcact

1321 taagagatgg gattacaagt ggaccctga//

- LOCUS: BC006229 523 bp mRNA linear PRI 15-JUL-2006
- DEFINITION: Homo sapiens cytochrome c oxidase subunit Vb, mRNA (cDNA clone MGC:10622 IMAGE:3952882), complete cds.
- ACCESSION: BC006229
- VERSION: BC006229.2 GI:38197026
- KEYWORDS: MGC.

1 cggaagtttt gctgctagtc gcggacgcaa tggcttcaag gttacttcgc ggagctggaa
61 cgctggccgc gcaggccctg agggctcgcg gccccagtgg cgcggccgcg atgcgctcca
121 tggcatctgg aggtggtgtt cccactgatg aagagcaggc gactgggttg gagagggaga
181 tcatgctggc tgcaaagaag ggactggacc catacaatgt actggcccca aagggagctt
241 caggcaccag ggaagaccct aatttagtcc cctccatctc caacaagaga atagtaggct
301 gcatctgtga agaggacaat accagcgtcg tctggttttg gctgcacaaa ggcgaggccc
361 agcgatgccc ccgctgtgga gcccattaca agctggtgcc ccagcagctg gcacactgag
421 cacctgcact aaattactca aaatgtgctg taaagtttct tctttccagt aaagactagc
481 cattgcattg gctccttctc ccataaaaaa aaaaaaaaaa aaa//

- LOCUS: HUMPROS30 1264 bp mRNA linear PRI 20-MAR-1996
- DEFINITION: Human prosomal protein P30-33K (pros-30) mRNA, complete cds.
- ACCESSION: M64992
- VERSION: M64992.1 GI:190446
- KEYWORDS: prosomal consensus; prosomal protein.
chromosome 11. 
1 cgcgagccgg gaccacgccg acccagcgtg cccaggccga ggaaagcgcg gcggcggcag
61 tccgaagacc caccggactg aaagagaagg acgaggtcat cttcggacgg gaggggcaag
121 ccagccatcc tgggacccca ggcgtgcagg ttctctttga gggtattcca ccctgcaaaa
181 agcatgtatt catggtcagc tctcagcaag gccagtagca gagtggtaaa ggccttggcc
241 ctccaaggct gggaaaagac aatgacaagt caaatccaga cctatgttgg atgttggtct
301 actaggtgac tgtctcctgg aaatgttatg cagctcagca aggtgaagtt tcgaaatcag
361 tatgacaatg atgtcactgt ttggaccgcc cagggcagga ttcatcaaat tgaatatgca
421 atggaagctg ttaaacaagg ttcagccaca gttggtctga aatcaaaaac tcatgcagtt
481 ttggttgcat tgaaaagggc gcaatcagag cttgcagctc atcagaaaaa aattctccat
541 gttgacaacc atattggtat ctcaattgcg gggcttactg ctgatgctag actgttatgt
601 aattttatgc gtcaggagtg tttggattcc agatttgtat tcgatagacc actgcctgtg
661 tctcgtcttg tatctctaat tggaagcaag acccagatac caacacaacg atatggccgg
721 agaccatatg gtgttggtct ccttattgct ggttatgatg atatgggccc tcacattttc
781 caaacctgtc catctgctaa ctattttgac tgcagagcca tgtccattgg agcccgttcc
841 caatcagctc gtacttactt ggagagacat atgtctgaat ttatggagtg taatttaaat
901 gaactagtta aacatggtct gcgtgcctta agagagacgc ttcctgcaga acaggacctg
961 actacaaaga atgtttccat tggaattgtt ggtaaagact tggagtttac aatctatgat
1021 gatgatgatg tgtctccatt cctggaaggt cttgaagaaa gaccacagag aaaggcacag
1081 cctgctcaac ctgctgatga acctgcagaa aaggctgatg aaccaatgga acattaagtg
1141 ataagccagt ctatatatgt attatcaaat atgtaagaat acaggcacca catactgatg
1201 acaataatct atactttaac caaaagttgc agagtggtgg aatgctattt tttaggaatc
1261 agtc//
- LOCUS: NM_014814 1308 bp mRNA linear PRI 17-NOV-2006
- DEFINITION: Homo sapiens proteasome (prosome, macropain) 26S subunit, non-ATPase, 6 (PSMD6), mRNA.
- ACCESSION: NM_014814
- VERSION: NM_014814.1 GI:7661913

1 gtcagccgct gtccccttag ccgcgatgcc gctggagaac ctggaggagg agggtctgcc
61 caagaacccc gacttgcgta tcgcgcagct gcgcttcctg ctcagcctgc ccgagcaccg
121 cggagacgct gccgtgcgcg acgagctgat ggcggccgtc cgcgataaca acatggctcc
181 ttactatgaa gccttgtgca aatccctcga ctggcagata gacgtggacc tactcaataa
241 aatgaagaag gcaaatgaag atgagttgaa gcgtttggat gaggagctgg aagatgcaga
301 gaagaatcta ggagagagcg aaattcgcga tgcaatgatg gcaaaggccg agtacctctg
361 ccggataggt gacaaagagg gagctctgac agcctttcgc aagacatatg acaaaactgt
421 ggccctgggt caccgattgg atattgtatt ctatctcctt aggattggct tattttatat
481 ggataatgat ctcatcacac gaaacacaga aaaggccaaa agcttaatag aagaaggagg
541 agactgggac aggagaaacc gcctaaaagt gtatcagggt ctttattgtg tggctattcg
601 tgatttcaaa caggcagctg aactcttcct tgacactgtt tcaacattta catcctatga

661 actcatggat tataaaacat ttgtgactta tactgtctat gtcagtatga ttgccttaga
721 aagaccagat ctcagggaaa aggtcattaa aggagcagag attcttgaag tgttgcacag
781 tcttccagca gttcggcagt atctgttttc actctatgaa tgccgttact ctgttttctt
841 ccaatcatta gcggttgtgg aacaggaaat gaaaaaggac tggctttttg cccctcatta
901 tcgatactat gtaagagaaa tgagaattca tgcatacagt cagctgctgg aatcatatag
961 gtcattaacc cttggctata tggcagaagc gtttggtgtt ggtgtggaat tcattgatca
1021 ggaactgtcc aggtttattg ctgccgggag actacactgc aaaatagata aagtgaatga
1081 aatagtagaa accaacagac ctgatagcaa gaactggcag taccaagaaa ctatcaagaa
1141 aggagatctg ctactaaaca gagttcaaaa actttccaga gtaattaata tgtaaagcca
1201 tgtaactaac aaaggatttg ctttagagat aattatttgg aatttttata gcttacttca
1261 caatgtgccc aggtcagctg tataaaataa atactgcatt gttgtttc//

- LOCUS: NM_001189 2165 bp mRNA linear PRI 21-JAN-2007
- DEFINITION: Homo sapiens bagpipe homeobox homolog 1 (Drosophila) (BAPX1), mRNA.
- ACCESSION: NM_001189
- VERSION: NM_001189.2 GI:25121985

1 atggctgtgc gcggcgccaa caccttgacg tccttctcca tccaggcgat cctcaacaag
61 aaagaggagc gcggcgggct ggccgcgcca gaggggcgcc cggcgcccgg gggcacagcg
121 gcatcggtgg ccgcggctcc cgctgtctgc tgttggcggc tctttgggga gagggacgcg
181 ggcgcgttgg ggggcgccga ggactctctg ctggcgtctc ctgccggtac cagaacagct
241 gcggggcgga ctgcggagag cccggaaggc tgggactcgg actccgcgct cagcgaggag
301 aacgagagca ggcggcgctg cgcggacgcg cggggggcca gcggggccgg ccttgcgggg
361 ggatccttga gcctcggcca gccggtctgt gagctggccg cttccaaaga cctagaggag
421 gaagccgcgg gccggagcga cagcgagatg tccgccagcg tctcaggcga ccgcagccca
481 aggaccgagg acgacggtgt tggccccaga ggtgcacacg tgtccgcgct gtgcagcggg
541 gccggcggcg ggggcggcag cgggccggca ggcgtcgcgg aggaggagga ggagccggcg
601 gcgcccaagc cacgcaagaa gcgctcgcgg gccgctttct cccacgcgca ggtcttcgag
661 ctggagcgcc gctttaacca ccagcgctac ctgtccgggc ccgagcgcgc agacctggcc
721 gcgtcgctga agctcaccga gacgcaggtg aaaatctggt tccagaaccg tcgctacaag
781 acaaagcgcc ggcagatggc agccgacctg ctggcctcgg cgcccgccgc caagaaggtg
841 gccgtaaagg tgctggtgcg cgacgaccag agacaatacc tgcccggcga agtgctgcgg
901 ccaccctcgc ttctgccact gcagccctcc tactattacc cgtactactg cctcccaggc
961 tgggcgctct ccacctgcgc agctgccgca ggcacccagt gaacccgctt gggctgaggc
1021 agcgagtgat tcccgcgctc cggctccgga ccggcgctga cagctgtagg ctgtagcctg
1081 cacggggcgc cccgccaagg aggcacctgg aggtgaaacc cagctccagc tcccgttagc
1141 caggacttgt cccctggcag ctgggctgag tctgccctga gggggcgcct ttttctaatt
1201 tgaacagagg caccctatgg cctaggggcc ctgatcgccc acctgcctgg aagcccctgg
1261 gctctattta ttatcatgac aatgttggaa ttaaattttg attcgaatat gtctgcctgg
1321 gggtggggtt ttccctgagc ggcaactcct ggagaccaca tagcctgaat cctcagaatt
1381 tcaggcctgc tgggagcttt ctgcactagg ccacactagt tcatggtatc catgctacca
1441 atctatgtgt atctacatat cttttatttt tggaaattgc atttgtaacc aaggggtgcg
1501 aaaccctggc agtcccaggc agcaccaggc caggggttga tttgaaacgt gaaggattgg
1561 gttttcaggc cctctgctcc acccctcctg tgtgtcagag ctagggtggg ggtgcccgat
1621 tcgggtgctg aatgtaagga ggggagcctc caagtgtggt gcaagccggg ggtctccaca
1681 tcttccttct ctgaagtcca ggtacctgca caagcaggaa gcgcctggga gtcccggaag
1741 gaggagagcg cacacccagg cagccctctg cggaaacttt ccttggtttc tttttatttg
1801 tgtaaaggag gttaagacgt gtcgcacttt tcagttgttt gtattcaaat gacgattatt
1861 tttctactca atgtgaatat ccctggccag cctttccacg gcgcccaccg cagtgccgct
1921 gcctggccct cagtgtctac cttctgccct ctgcgactcc agtgctctgg cccgggactc
1981 ccctatccgc ccctcactta cccttaaaca ggtgatccca cctgtcttgt caacctcgcc
2041 gcttttcgcc tccttaatgg cactgtgcac tcaactagag tattaactgt aaaaagattt
2101 gtgaagtttg gaagctctat tcgctgtatt ttttctttaa tttataaact tttagtttaa
2161 catgc//

- LOCUS: NM_006839 2697 bp mRNA linear PRI 17-NOV-2006
- DEFINITION: Homo sapiens inner membrane protein, mitochondrial (mitofilin)(IMMT), mRNA.
- ACCESSION: NM_006839
- VERSION: NM_006839.1 GI:5803114

1 acgcgggcac gcacacacgg aagcacgcct ccacttaact cgcgccgccg cggcagctcg
61 agtccaccag cagcgccgtc cgcttgaccg agatgctgcg ggcctgtcag ttatcgggtg
121 tgaccgccgc cgcccagagt tgtctctgtg ggaagtttgt cctccgtcca ttgcgaccat

181 gccgcagata ctctacttca ggcagctctg ggttgactac tggcaaaatt gctggagctg
241 gccttttgtt tgttggtgga ggtattggtg gcactatcct atatgccaaa tgggattccc
301 atttccggga aagtgtagag aaaaccatac cttactcaga caaactcttc gagatggttc
361 ttggtcctgc agcttataat gttccattgc caaagaaatc gattcagtcg ggtccactaa
421 aaatctctag tgtatcagaa gtaatgaaag aatctaaaca gtctgcctca caactccaaa
481 aacaaaaggg agatactcca gcttcagcaa cagcacctac agaagcggct caaattattt
541 ctgcagcagg tgataccctg tcggtcccag cccctgcagt tcagcctgag gaatctttaa
601 aaactgatca ccctgaaatt ggtgaaggaa aacccacacc tgcactttca gaagaagcat
661 cctcatcttc tataagggag cgaccacctg aagaagttgc agctcgcctt gcacaacagg
721 aaaaacaaga acaagttaaa attgagtctc tagccaagag cttagaagat gctctgaggc
781 aaactgcaag tgtcactctg caggctattg cagctcagaa tgctgcggtc caggctgtca
841 atgcacactc caacatattg aaagccgcca tggacaattc tgagattgca ggcgagaaga
901 aatctgctca gtggcgcaca gtggagggtg cattgaagga acgcagaaag gcagtagatg
961 aagctgccga tgcccttctc aaagccaaag aagagttaga gaagatgaaa agtgtgattg
1021 aaaatgcaaa gaaaaaagag gttgctgggg ccaagcctca tataactgct gcagagggta
1081 aacttcacaa catgatagtt gatctggata atgtggtcaa aaaggtccaa gcagctcagt
1141 ctgaggctaa ggttgtatct cagtatcatg agctggtggt ccaagctcgg gatgacttta
1201 aacgagagct ggacagtatt actccagaag tccttcctgg atggaaagga atgagtgttt
1261 cagacttagc tgacaagctc tctactgatg atctgaactc cctcattgct catgcacatc
1321 gtcgtattga tcagctgaac agagagctgg cagaacagaa ggccaccgaa aagcagcaca
1381 tcacgttagc cttggagaaa caaaagctgg aagaaaagcg ggcatttgac tctgcagtag
1441 caaaagcatt agaacatcac agaagtgaaa tacaggctga acaggacaga aagatagaag
1501 aagtcagaga tgccatggaa aatgaaatga gaacccagct tcgccgacag gcagctgccc
1561 acactgatca cttgcgagat gtccttaggg tacaagaaca ggaattgaag tctgaatttg
1621 agcagaacct gtctgagaaa ctctctgaac aagaattaca atttcgtcgt ctcagtcaag
1681 agcaagttga caactttact ctggatataa atactgccta tgccagactc agaggaatcg
1741 aacaggctgt tcagagccat gcagttgctg aagaggaagc cagaaaagcc caccaactct
1801 ggctttcagt ggaggcatta aagtacagca tgaagacctc atctgcagaa acacctacta
1861 tcccgctggg tagtgcagtt gaggccatca aagccaactg ttctgataat gaattcaccc
1921 aagctttaac cgcagctatc cctccagagt ccctgacccg tggggtgtac agtgaagaga
1981 cccttagagc ccgtttctat gctgttcaaa aactggcccg aagggtagca atgattgatg
2041 aaaccagaaa tagcttgtac cagtacttcc tctcctacct acagtccctg ctcctattcc
2101 cacctcagca actgaagccg cccccagagc tctgccctga ggatataaac acatttaaat
2161 tactgtcata tgcttcctat tgcattgagc atggtgatct ggagctagca gcaaagtttg
2221 tcaatcagct gaagggggaa tccagacgag tggcacagga ctggctgaag gaagcccgaa
2281 tgaccctaga aacgaaacag atagtggaaa tcctgacagc atatgccagc gccgtaggaa
2341 taggaaccac tcaggtgcag ccagagtgag gtttaggaag attttcataa agtcatattt
2401 catgtcaaag gaaatcagca gtgatagatg aagggttcgc agcgagagtc ccggacttgt
2461 ctagaaatga gcaggtttac aagtactgtt ctaaatgtta acacctgttg catttatatt
2521 ctttccattt gctatcatgt cagtgaacgc caggagtgct ttctttgcaa cttgtgtaac
2581 attttctgtt ttttcaggtt ttactgatga ggcttgtgag gccaatcaaa ataatgtttg
2641 tgatctctac tactgttgat tttgccctcg gagcaaactg aataaagcaa caagatg//

- LOCUS: AI278616 792 bp mRNA linear EST 23-NOV-1998
- DEFINITION: qm47a06.x1 Soares_placenta_8to9weeks_2NbHP8to9W Homo sapiens cDNA clone IMAGE:1891858 3' similar to gb:M93651 SET PROTEIN (HUMAN);,mRNA sequence.
- ACCESSION: AI278616
- VERSION: AI278616.1 GI:3916850
- KEYWORDS: EST.

1 gttttactgg aaaggaaaga tgatgctcag ttttaaacgt taaaagtgta caagttgctt
61 tgttacaata aaactaaatg tgtacacaaa ggatttgatg cttttctctc agcataggta
121 tgcttactat gaccttccaa gtttgacttg tataacatca ctgtcaaact ttgtcaccct
181 aacttcgtat tttttgatac gcactttgca ggatgacctc agggctatgt ggattgagta
241 atgggatttg aatcaatgta ttaatatctc catagctggg aaacgtgggt tcaatttgcc
301 attggtttct gaaagtattc acatcatttg ggataccaga tagctcaata ctctctgagt
361 acattgtgcc cttgattttt atctccaagt ggcagttttt aaaattggcc ttttacctgg
421 atataaatta attgtgcctg ccaccaccat ccaacagacc tggtgctcta atgccaagtt
481 atacacggga cagttgctgg catgtcttta ttggctatat aaaatgtggc caagaagata
541 tgctctcagt aagaagtctg atggtgagca gtaactgtcc ctgctttcta gtataaagct
601 ctcagatgtg accatgtgaa tcatggtggg ataatggact cagctctgta tgcttcatgc
661 cattgtgcag agtagcaccc ttatgcataa gctttttaat gctgtacaat atagtcgctg

721 aaagtaaatg ccaccttttt agcagtgaat taatggacag tctggtgaaa cttcaatgct
781 ttttgatgta ta//

- LOCUS: NM_003171 2491 bp mRNA linear PRI 17-NOV-2006
- DEFINITION: Homo sapiens suppressor of var1, 3-like 1 (S. cerevisiae) (SUPV3L1), mRNA.
- ACCESSION: NM_003171
- VERSION: NM_003171.2 GI:31543666

1 actgtccgcc gccgtgtccg cggctgcgcc agacagtgta gaacctgcgg cctcgatgtc
61 cttctcccgt gccctattgt gggctcggct cccggcgggg cgccaggctg gccaccgggc
121 agccatctgc tctgcccttc gtccccactt tgggcccttt cccggggttc tggggcaagt
181 ttctgtcctt gccaccgcct cctcctctgc ctccggtggc tccaaaatac caaacacgtc
241 cttgttcgtg cccctgactg tgaaacctca gggccccagc gccgacggcg acgtcggggc
301 cgagctaacc cggcctctgg acaagaatga agtaaagaag gtcttagaca aattttacaa
361 gaggaaagaa attcagaaac tgggtgctga ttatggactt gatgctcgtc tcttccacca
421 agctttcata agctttagaa attatattat gcagtctcat tccctggatg tggacattca
481 cattgttttg aatgatattt gcttcggtgc agctcatgcg gatgatttat tcccattttt
541 cttgagacat gccaaacaaa tatttcctgt gttggactgt aaggatgatc tacgtaaaat
601 cagtgactta agaataccac ctaactggta cccagatgct agagccatgc agcggaagat
661 aatatttcat tcaggcccca caaacagtgg aaagacttat cacgcaatcc agaaatactt
721 ctcagcaaag tctggagtgt attgtggccc tctaaaatta ctggcacatg agatcttcga
781 aaagagtaat gctgctggtg tgccatgtga cttggtgaca ggtgaagagc gtgtgacagt
841 tcagccaaat gggaaacagg cttcacatgt ttcttgtaca gttgagatgt gcagtgttac
901 aactccttat gaagtggctg taattgatga aattcaaatg attagagatc cagccagagg
961 atgggcctgg accagagcac ttctaggact gtgtgctgaa gaggttcatt tgtgtggaga
1021 acctgctgct attgacctgg tgatggagct tatgtacaca acgggggagg aagtggaggt
1081 tcgagactat aagaggctta cccccatttc tgtgctggac catgcactag aatctttaga
1141 taaccttcgg cctggggact gcattgtctg ttttagcaag aatgatattt attctgtgag
1201 tcggcagatt gaaattcggg gattagaatc agctgttata tatggcagtc tcccacctgg
1261 gaccaaactt gctcaagcaa aaaagtttaa tgatcccaat gacccatgca aaatcttggt
1321 tgctacagat gcaattggca tgggacttaa tttgagcata aggagaatta tttttactc
1381 ccttataaag cccagtatca atgaaaaggg agagagagaa ctagaaccaa tcacaacctc
1441 tcaagccctg cagattgctg gcagagctgg cagattcagc tcacggttta aagaaggaga
1501 ggttacaaca atgaatcatg aagatctcag tttattaaag gaaattttga agaggcctgt
1561 ggatcctata agggcagctg gtcttcatcc aactgctgag cagattgaaa tgtttgccta
1621 ccatctccct gatgcaacac tgtccaatct cattgatatt tttgtagact tttcacaagt
1681 tgatgggcag tattttgtct gcaatatgga tgattttaaa ttttctgcag agttgatcca
1741 gcatattcca ctaagtctgc gagtgaggta tgttttctgc acagctccta tcaacaagaa
1801 gcagcctttt gtgtgttctt cactgttaca gtttgccagg cagtatagca ggaatgagcc
1861 cctgaccttt gcatggttac gccgatacat caaatggcct ttacttccac ctaagaatat
1921 taaagacctc atggatcttg aagctgtcca cgatgtcttg gatctttact tgtggctaag
1981 ctaccgattt atggatatgt ttccagatgc cagccttatt cgagatctcc agaaagaact
2041 agatggtatt atccaagatg gtgtgcacaa tatcactaaa ttgattaaaa tgtctgagac
2101 gcataagctg ttgaatttgg agggctttcc atcagggagc cagtcacgat tgtcaggaac
2161 cttaaagagc caagctagaa ggacacgcgg caccaaagct ctagggagta aagctactga
2221 gccacccagc cccgatgcag gagagctgtc ccttgcttcc agattggtgc agcaaggact
2281 cctcactcca gacatgctga aacagctaga aaaagagtgg atgacacaac aaactgaaca
2341 caacaaagaa aaaacagagt ctgggactca tccaaaaggg acgagaagaa agaagaagga
2401 acctgattcg gactagtttt ctgttcctgt tttttttttt ttatttaatt ttgcaataa
2461 aaatttattt tgaagaaaaa aaaaaaaaaa a//

- LOCUS: NM_006604 1488 bp mRNA linear PRI 17-NOV-2006
- DEFINITION: Homo sapiens ret finger protein-like 3 (RFPL3), mRNA.
- ACCESSION: NM_006604
- VERSION: NM_006604.1 GI:5730012

1 gccacgtcac tggctcaggc tcagctgctg ggtcaccagg agaatggacc cttcctccac
61 ctcagctcag agcacagtga tgattcgtga ctttcccaat agaacttcaa atctctgagg
121 acggggggtg gggggatgtg cttgagtgtt tgtactcatg gtcttgttct cggagtgaca
181 aagctggaac acaatacctc tatgcatgaa aaggttgtca cttgtcacaa ctaacaggct
241 ttcacctcaa ggaaattttc ttcccttgtg tacttttccc ctggcagtgg acatggctgc
301 actcttccaa gaagcaagca gctgtcccgt ctgctcagac tatctggaaa aaccaatgtc
361 cctggagtgt ggatgcaccg tctgcctcaa gtgcatcaat tcgctgcaga aggagcccca

421 tggggaggat ctgctttgct gttgctgttc catggtctct cagaggaaca aaatcaggcc
481 caatcggcag ctagagaggc tggtttccca catcaaggaa ctggagccca agctgaagaa
541 gattctacag atgaacccaa ggatgcggaa gttccaagtg gatatgacct tggatgccga
601 cacagccaac aacttcctcc tcatttctga cgacctcagg agcgtccgaa gtgggctcat
661 cacacagaat cggcaagacc ttgccgagag atttgacgtg tccgtttgca tcctgggctc
721 ccctcgcttt acctgtggcc gccactactg ggaggtggac gtgggaacaa gcacagaatg
781 ggacctggga gtctgcagag aatctgttca ctgcaaaggg aagatccagc tgaccacaga
841 gcttggattc tggactgtga gtttgaggga tggaagccgc ctctctgcca gcacggtgcc
901 gctgactttc ctcttagtag accgcaagtt acagcgagtg gggatttttc tggatatggg
961 catgcagaac gtttcctttt ttgatgctga aagtggttcc catgtctata cattcaggag
1021 cgtctctgct gaggagccac tgcgcccatt tttggctcct tcaattccac ctaatggtga
1081 tcaaggtgtc ttgagcatct gtcctttgat gaactcaggc actactgatg ctccagtccg
1141 tcctggggag gccaaataag ccgccactgc aaaaaaaaac agggtcagaa aattacttgg
1201 gtgggtagac ttaggaattt tctacttggt aaaagcatta tacagtcata ggagaaagat
1261 atgggacatt tctataatct atattctaat ttgattcgat tattgagtcg taagtattaa
1321 ttattgccac catccaactc attgagtctt atggttcaca tcttgtttcc tatagaaatg
1381 ttctgtattc tcggatcaat ttccaaatgc tttacttttt catttctgta agttcaaatc
1441 aatgtttaaa ttatagaagt tatgaggtaa ataaacattt ggatatca//

- LOCUS: NM_004960 2012 bp mRNA linear PRI 18-NOV-2006
- DEFINITION: Homo sapiens fusion (involved in t(12;16) in malignant liposarcoma)(FUS), transcript variant 1, mRNA.
- ACCESSION: NM_004960
- VERSION: NM_004960.2 GI:58218971

1 cggggctgct cagtcctcca ggcgtcggta ctcagcggtg ttggaacttc gttgcttgct
61 tgcctgtgcg cgcgtgcgcg gacatggcct caaacgatta tacccaacaa gcaacccaaa
121 gctatggggc ctaccccacc cagcccgggc agggctattc ccagcagagc agtcagccct
181 acggacagca gagttacagt ggttatagcc agtccacgga cacttcaggc tatggccaga
241 gcagctattc ttcttatggc cagagccaga acacaggcta tggaactcag tcaactcccc
301 agggatatgg ctcgactggc ggctatggca gtagccagag ctcccaatcg tcttacgggc
361 agcagtcctc ctaccctggc tatggccagc agccagctcc cagcagcacc tcgggaagtt
421 acggtagcag ttctcagagc agcagctatg ggcagcccca gagtgggagc tacagccagc
481 agcctagcta tggtggacag cagcaaagct atggacagca gcaaagctat aatccccctc
541 agggctatgg acagcagaac cagtacaaca gcagcagtgg tggtggaggt ggaggtggag
601 gtggaggtaa ctatggccaa gatcaatcct ccatgagtag tggtggtggc agtggtggcg
661 gttatggcaa tcaagaccag agtggtggag gtggcagcgg tggctatgga cagcaggacc
721 gtggaggccg cggcaggggt ggcagtggtg gcggcggcgg cggcggcggt ggtggttaca
781 accgcagcag tggtggctat gaacccagag gtcgtggagg tggccgtgga ggcagaggtg
841 gcatgggcgg aagtgaccgt ggtggcttca ataaatttgg tggccctcgg gaccaaggat
901 cacgtcatga ctccgaacag gataattcag acaacaacac catctttgtg caaggcctgg
961 gtgagaatgt tacaattgag tctgtggctg attacttcaa gcagattggt attattaaga
1021 caaacaagaa aacgggacag cccatgatta atttgtacac agacagggaa actggcaagc
1081 tgaagggaga ggcaacggtc tcttttgatg acccaccttc agctaaagca gctattgact
1141 ggtttgatgg taaagaattc tccggaaatc ctatcaaggt ctcatttgct actcgccggg
1201 cagactttaa tcggggtggt ggcaatggtc gtggaggccg agggcgagga ggacccatgg
1261 gccgtggagg ctatggaggt ggtggcagtg gtggtggtgg ccgaggagga tttcccagtg
1321 gaggtggtgg cggtggagga cagcagcgag ctggtgactg gaagtgtcct aatcccacct
1381 gtgagaatat gaacttctct tggaggaatg aatgcaacca gtgtaaggcc cctaaaccag
1441 atggcccagg agggggacca ggtggctctc acatgggggg taactacggg gatgatcgtc
1501 gtggtggcag aggaggctat gatcgaggcg gctaccgggg ccgcggcggg gaccgtggag
1561 gcttccgagg gggccggggt ggtggggaca gaggtggctt tggccctggc aagatggatt
1621 ccaggggtga gcacagacag gatcgcaggg agaggccgta ttaattagcc tggctcccca
1681 ggttctggaa cagctttttg tcctgtaccc agtgttaccc tcgttatttt gtaaccttcc
1741 aattcctgat cacccaaggg tttttttgtg tcggactatg taattgtaac tatacctctg
1801 gttcccatta aaagtgacca ttttagttaa attttgttcc tcttccccct tttcactttc
1861 ctggaagatc gatgtcccga tcaggaaggt agagagtttt cctgttcaga ttaccctgcc
1921 cagcaggaac tggaatacag tgttcgggga gaaggccaaa tgatatcctt gagagcagag
1981 attaaacttt tctgtcatgg ggaaaaaaaa aa//

- LOCUS: NM_007011 9085 bp mRNA linear PRI 18-NOV-2006
- DEFINITION: Homo sapiens abhydrolase domain containing 2 (ABHD2), transcript variant 1, mRNA.
- ACCESSION: NM_007011 XM_926626 XM_938496

- VERSION: NM_007011.5 GI:93141021

1 agtagcgtct gggtccctgg actaaagccg tcaaatgtaa ccaattgctg aggagctctc
61 ccctaagaat cccgcctcca ctctgaggaa cagcttcgct gatcaatgga tcccctccta
121 gacgcaatga gcgcgcgggg caagtggaga agcggctgaa ctgcccaatg aacaagcggt
181 ttccgtggtt aggggcgtgg cagaagcggt cgtcaggggc gtggcggcag ttacttgggc
241 ggggccggta gcggcgggag ctgcactggc caggggttcc ggctgtatat ccatgagcgc
301 cgctggcagc cggggagctg caggaaccag actgggggcg agctgagcac ctgtagtcaa
361 tcacacgcag aaataataat gatacttatg ttagaagatt gttttgagaa gtgcatggag
421 ttactacatc tgaagcattt agaacagtac ctggcacagg gttctgtcct cttcatttgc
481 aagccactgc tttatccacg agggtgctcc ctacttatgc cagtgaatta attttggca
541 cagatgaatc cttcatattt atctggattt agctggtagc atttgtctcc taagacttgg
601 tcacagagga atccatgtga ccttcaggcc ttctgctcta aactggcagg agatcctgtt
661 tctactcaca gcgtccgagg gtctggtggc cgagccctga ggggcgctgt tgctcccagg
721 tggcagaatt cagcatcaga gagccacaca gctgaaagag atggatggct tttaggtttg
781 tttgaataag agatctgacc tgaccggccc aactgtacaa ctcttcaagg aaaattcgta
841 tttgcagtgg gaagaataag taacattgat caagatgaat gccatgctgg agactcccga
901 actcccagcc gtgtttgatg gagtgaagct ggctgcagtg gctgctgtgc tgtacgtgat
961 cgtccggtgt ttgaacctga agagccccac agccccacct gacctctact tccaggactc
1021 ggggctctca cgctttctgc tcaagtcctg tcctcttctg accaaagaat acattccacc
1081 gttgatctgg gggaaaagtg gacacatcca gacagccttg tatgggaaga tgggaagggt
1141 gaggtcgcca catccttatg ggcaccggaa gttcatcact atgtctgatg gagccacttc
1201 tacattcgac ctcttcgagc ccttggctga gcactgtgtt ggagatgata tcaccatggt
1261 catctgccct ggaattgcca atcacagcga gaagcaatac atccgcactt tcgttgacta
1321 cgcccagaaa aatggctatc ggtgcgccgt gctgaaccac ctgggtgccc tgcccaacat
1381 tgaattgacc tcgccacgca tgttcaccta tggctgcacg tgggaatttg gagccatggt
1441 gaactacatc aagaagacat atcccctgac ccagctggtc gtcgtgggct tcagcctggg
1501 tggtaacatt gtgtgcaaat acttggggga gactcaggca aaccaagaga aggtcctgtg
1561 ctgcgtcagc gtgtgccagg ggtacagtgc actgagggcc caggaaacct tcatgcaatg
1621 ggatcagtgc cggcggttct acaacttcct catggctgac aacatgaaga agatcatcct
1681 ctcgcacagg caagctcttt ttggagacca tgttaagaaa ccccagagcc tggaagacac
1741 ggacttgagc cggctctaca cagcaacatc cctgatgcag attgatgaca atgtgatgag
1801 gaagtttcac ggctataact ccctgaagga atactatgag gaagaaagtt gcatgcggta
1861 cctgcacagg atttatgttc ctctcatgct ggttaatgca gctgacgatc cgttggtgca
1921 tgaaagtctt ctaaccattc caaaatctct ttcagagaaa cgagagaacg tcatgtttgt
1981 gctgcctctg catgggggcc acttgggctt ctttgagggc tctgtgctgt tccccgagcc
2041 cctgacatgg atggataagc tggtggtgga gtacgccaac gccatttgcc aatgggagcg
2101 taacaagttg cagtgctctg acacggagca ggtggaggcc gacctggagt gaggcctccg
2161 gactctggca cgctccagca gccctcctct ggaagctgcg tcccctcacc ccctgtttca
2221 ggtctcccat ctccctcagt gacctggatc tgacctcaca ccatcagcag gggcacccca
2281 ccatgcacac ctgtctcgga gtaggcagct cttcctggga gctccaggct atttttgtgc
2341 ttagttactg gttttctcca ttgcattgtt aggcatggtg acaagtgaca gagttcttgc
2401 cctctgtcca gtttcagcat ctggttgctt ttaagccaag tacatctagt ttccctatta
2461 aaaatgtgtc tgaatagcga ttttgctttg ccaccaaaag gcttttccct gagaacagtg
2521 aaggatgtat gtcattttgt ggtggttgta tgtgtcctta catagacctt aaaaagagct
2581 cacccttcca ggccaatgct gaagacacag ctccgcttgg gagcctgaga acccaggctt
2641 cccaggccag agtgtggctt cttaaacggc aaaggaaatt cctttgagtc acaagccaag
2701 ttttcgccct gtctcctgag accatttccc tacgctttgc tgctgctgag agttacgtga
2761 ggcacttgtt aaaaattcag cctcccaggt ccctcccctc ggagaggctg attcactggg
2821 tctgggaagg agcctgggga ttttaatttt tcacaagtgc cccagatgat tctcatcacc
2881 aagcaaattt tggaaatgct gttcaacagc gcccttaaat tggaaacatc tttgcagctc
2941 gttttattga aattcataat caggggtgtc ctctagctcc cacggtctcc agagcagcaa
3001 ggccggctat ggagctgccg tcgtgtgacc acagtgtgat gtctcagaag ggctctgggt
3061 gggctgagca tctgggctgt gccctggctc tgcttttcac cctggacaaa gtcgctgtgg
3121 acttcaattt cttcacctct aaaatggggg acttggacca ggtagattgc tgagctcact
3181 accaggttca aagttcaatg acaaactcag tttactgagg tttgagagaa catccctcca
3241 ggggagcctg ggagctgctc tcccagtcta agcatgtaga tatcatcgtt tgccttttgt
3301 gtgtgtgtgt cccttatttg ataaaaagat gttttgagtt gttttttttt taagcactca
3361 cttgtaattt tagtttttaa acccaagtcc ctctaacttt gcctttgata ccaaacaatt
3421 caaaagttgg atctgagttt ggagaaagat atttccaacc taagtgggta ttattttgaa
3481 accagatttt taatttaata gcctatattt gtagtctgtt ggataggtgt ttccaaagtg
3541 tgtcttctca agtgaaaacg caactctagg tttcaagtac tccttttctc cgatcctgtg
3601 gtacttgaat atccaaaaac cctgcacttt gaacaatcag ctgttgctat ctggaactaa
3661 acagaactat gagtaaaatt gcctggatac tttaaaaaga tatttttcct ccttcatctc

3721 ctttgactcc aggacagact ggaatataag tagtgggtct gcatggatgt ttcagggatc
3781 aaaggagcca cctgggcgcc tgagtgccaa ccctcagggc cacaggtggg tgtggtttgg
3841 gcacgggtcc aagtgactgt gacgggaccc gtgggcatgg gtccaggtct gtaacctgaa
3901 gaagttgttt tctgacaatc accaaatcat ccgaatgaca tcaaaagcag cccttatctc
3961 agagactgag atttctgtgg tctcaacttc gctttggtat aatttctggc actcaccagc
4021 ctctatcatt atgactttcc ccccagtgta ttatttctct aataggtttc cttttcacgt
4081 tcttttagca cagactggca ctttaccctc tcagtttgga agttagcccc tctcctctgt
4141 tacttttccc ttcacccaac tacagctgtg atctagaaca ttcatagtca tatttctgct
4201 actactacct tcatttatca agacttttta tgagaatagg taaaccaagc aataacttcc
4261 taggactgaa tcaccacccc agaagagcga gaggctcctt tcatatgcct gaggccacac
4321 cccttaacct gttctgacaa aatagtggct ggcccatgta ccagctccat tcagaaattc
4381 aggaagagaa aagacagccc tgttgtcaca caaaccggtg tgggggaggg tggagcctgg
4441 tctgcacggc agtcctggtg gcccctgtgg aggacaggca gggctggcag catagccttt
4501 gttgccacac aaccggaatt tgctccccca ggactgtggg agccagtgtc ccagctgaaa
4561 tctttttagt gtgtggctct gaatggcact cacattccat tttggctcac atgaaactaa
4621 ctgaagccct ttgttcaagc ttcaggctct taggcatgga aatgagaatg tgactgtggc
4681 tgtcttacag gaaaattctt gtttgtccct gaatgagagc acagaggcat tgaattcaca
4741 gagctgcaaa cttgcctgat aaatgaggga gtggcagttt atagataggt catctttttt
4801 ccttcctcca ggtgtccttg cctttcttcc caaagtcatt catttctgat gagtatatga
4861 atccccctct tgctagtaag gttctatttg ggctaaaaca aggctgaatt tttaaagagt
4921 atttgaatat attttagaat caaattgagg ctataaattg catcaatctg gacaattcca
4981 ttgcaggaat aatatgttaa aaaccaatgg ggagaagcac ccacatctct cctgtagcac
5041 tccgtgtctc ataagcaatt tgaagacact tacaagtaac tgattccagt caaattagga
5101 ttaactgact caaaaaatgg tgtcaagttt ctttaatgtt tttatgttag aagtgagttt
5161 aacagacttg aagaaaactg ttatcttttc ctgctgtgag tttacacaaa tgattccaga
5221 gcagaatgaa agcagaaagc tgttggttac aatattcttt taacctctct gcagcatttt
5281 acacttactg ggaaccttat gattcaccgt aagagtggaa atatacctga gttcgtgtcc
5341 taatggtctc taattcacat tggatcgtgg gcaaatcacc tcacctctct gagcctgttc
5401 cctcctctta gaccatctct aagaccactt catctattta cacatcattt gcttgaacat
5461 tgctgaacat ctgcgtgaac ttggcctctc cagcccttgc aggtggaaac agctgtgtca
5521 aggctcaagg ctcacgctga ggggacttgg agggaggggg cttctgcatt aagctttcct
5581 ggtgaagacc cttgatcttg tccaaagccc tgtgtctttg actggcttct cttcagagtc
5641 cccgttgtca tcgtaagacc cttgctgttt ggagggtggt cttgtgactg tggcagctgc
5701 tggccgctgg aatgaggagc ctatctccat cctccagtgt gactcaggca gagcattgag
5761 aattcccagg gcagaaatcc ttcctgctca ggctttcatt ctaaaactac agtcttcatt
5821 aaagctgaac tttctgggta gctgagctta tatgcccggc atctgaatga gagctctctt
5881 tgtaactgtg tgacttgaga tctagtttgc cagctcctgg gaaacaatac atgtgttctt
5941 gtttgtgttt gctcagcaag cagatgtctg agatgtaaga agcttttctt ttcctgtggc
6001 attgattctg acttagagct gaagtaaaag atcactgaaa catcacgtca agttgaagtc
6061 actcataggt ctttgtcctt taggcaggac aggagagtca ttaagaagca tttcactgta
6121 gcattctatc acaatatcat ctggaattgt tttctttgcc cagaaagcct taacttgcct
6181 ctagagaatc cctggtatta caacgatatt gcggcattag aattccaact cttctgctgt
6241 ggaagtttga agcgaagctg cagcaaaacc agagaatttc ctcaagtggc ctgtaggctc
6301 cttgttatct tatgccccca cccctccctc aacaatatga gtgatccaga actggcccaa
6361 acacctcagc tctggtccct ttttgccctt cttggcctta ctctgttgtt caaagccact
6421 ttggattgct tggatgcttc gaacagccat gaaaagtagc ctgcctgtgg catttagagg
6481 ccaagcaatt gacagaaagg gtttcttcta cctctgttat ctaagcagag ggaagtaaac
6541 ctctcaccgc cccccacccc tcactgcccc cgattaccct agaattgctt tcgccaaatt
6601 gtagttgaag ctaaggaagg ggaatctggc ccctgctggg agagggaact ggaatgccac
6661 acaaggcaag gcctgcttcc ttccttcccc tctgctgctg ctgcctcgga acgctgcagc
6721 ccaggcttcc tcccacagtg gcccttggaa gcaggccgca gagtagacag ctgctccttt
6781 tggaagagtc agtcccctgt gttttctgaa ctgtttttcc tagcatgtat gtgggtagag
6841 ctttcatgca tctctagtaa taataagctg aaattagttt tttttttaat tctccaattt
6901 aaaactttta attaaaaagt aaattttaat gtcgaaaatg caaacttggg gagggcagaa
6961 agatcacaca caaggctgtc acttcatact tgcaggattg cacagcagcc gggcagaggc
7021 gctcctcact tcccagatgg ggcggcgggc agcagagacg cacctcactt cctagacagt
7081 gcggcagcca ggcacaggca cacctcactt cccagacagt tgggcggcca ggcaagcgct
7141 cctcacttcc cagatggggc ggctcccggg aagcggggct cctcacttcc cagacagggt
7201 ggccaggcag aggtgctcct cacttcccag aacaattctt tatgaatttg ataaaggact
7261 gaagtgcaac tgaaagctgc tagtgatgat ctggtaatat acaatttgtc cagtagccag
7321 tttgttttta ttgtgttttc taaccataag agatcattaa aggcaaagcc tgtatgacgc
7381 tgtacacaca caaaaaaatg gtcaccgcag gccatactac caatgaaatg gtaggtaaac
7441 aaatcttctg gtcaagagaa aaaaaaaaga aatagcactc tgcatgcttt gctctacaag

7501 acgaatttcc ctagaaagaa tccaatgaag gccgggcata gtggctcact cctgtaatcc
7561 cagcactttg gaggccgagg cgggtagatc acctgaggtt aggagttcaa gaccagcctg
7621 gccaacatgg tgaaacccct tctctactaa aaatgcaaaa attagctggg tgtggtggcg
7681 ggcgcctgta atcccagcta ctcgggaggc tgaggtggga gaattgcttg aacccaggag
7741 acggaggttg cagtgagccg agatcacgcc tctgcactcc agcctgggca acaagagtga
7801 gactccgtct ccaaaaaaag aaaaggaatc cagtgaaaat ggctgcaatt acaacaagaa
7861 gtgaaggaag aagactggtg acatctctga aggatgcagt tgaggttgat ccaggtttat
7921 ccgaatatgc tacctttctg agccttaaac cttcatctct caggtgttga tttgcttctg
7981 atagcttcat catttctccc tgaagtcttt tacactcttc tgttagtttc cttgtttcag
8041 tatcatgaag tgaagcactg tgtggttgtg gcgtgggccc gtcttgctta gatgcattca
8101 gtgggacagc tttgctgggt tccatgtcat tcaatttatc attttcattg ggatctcca
8161 tttggaatcc attaattcat gaggttttgc ctcattccac acagcttcca tatctgaagt
8221 gtttagtgga gcaaaaattg taccataaac ttgtgtttac tcttttcatt cggatcatag
8281 tcaaagggct gtagcattac tgaaacagtc acagttgacc ctgggtcaat aattccactg
8341 ttgggcctca cacagtaccg gtgaggcacg gtagtcttca ctttgaaaca cacttttcta
8401 tccgatggat ttcgcaattt aagatttgta gtgactacat ctgtgaaggg gcctttgaat
8461 ttgaggtcta tgggcgggtc gaggaccagg atctgctcgt gcttcgccgt ggccccggag
8521 gcagacgcca ttggagagac agcgcagagc aggggggcggc ttgctcgctg gggcgggggg
8581 acgatggcga gaggggaggg ggagcgagtt cgcatctctc cttttcctgg ttagactctg
8641 ttcaaccaca ttcttatgtt ggcagatctg cttccagatt gatttttaga gcaccatcac
8701 tttcacattc ctgattctga ttttgttttg ttttgtttgg gttttctgaa acttaaaatg
8761 ctgccccgaa aatactatat ttttgagttt gtgttctgaa agcctccgtg ctgctggatc
8821 tttgggggga aatacaggat ccttcagcac tgaggtgttt aagatttgca actagcaatg
8881 caattttttc taaatatggg gatatttacc tttattaaga aattatacta aacattgatg
8941 tccttgatca ttttatgttc tcatattact tttgattcta ctatgattgt gtggtggtga
9001 acaaagatca ttacaaacaa aaactgtaat tttgttatat ttgattcaat ggaatttacc
9061 taaaaaataa agactaaaaa tgtgg//

- LOCUS: BC006466 548 bp mRNA linear PRI 28-JUL-2005
- DEFINITION: Homo sapiens diazepam binding inhibitor (GABA receptor modulator, acyl-Coenzyme A binding protein), mRNA (cDNA clone IMAGE:3510767).
- ACCESSION: BC006466
- VERSION: BC006466.1 GI:13623678

1 gcctcctccg ctgtctccct ggagttcttg caagtcggcc aggatgtctc aggctgagtt
61 tgagaaagct gcagaggagg ttaggcacct taagaccaag ccatcggatg aggagatgct
121 gttcatctat ggccactaca aacaagcaac tgtgggcgac ataaatacag aacggcccgg
181 gatgttggac ttcacgggca aggccaagtg ggatgcctgg aatgagctga aagggacttc
241 caaggaagat gccatgaaag cttacatcaa caaagtagaa gagctaaaga aaaaatacgg
301 gatatgagag actggatttg gttactgtgc catgtgttta tcctaaactg agacaatgcc
361 ttgttttttt ctaataccgt ggatggtggg aattcgggaa aataaccagt taaaccagct
421 actcaaggct gctcaccata cggctctaac agattagggg ctaaaacgat tactgacttt
481 ccttgagtag tttttatctg aaatcaatta aaagtgtatt tgttacttta aaaaaaaaaa
541 aaaaaaa//

- LOCUS: NM_019009 3615 bp mRNA linear PRI 28-JAN-2007
- DEFINITION: Homo sapiens toll interacting protein (TOLLIP), mRNA.
- ACCESSION: NM_019009
- VERSION: NM_019009.2 GI:21361618

1 gcggcggcct cgaggtgaca actgtctccg tcgcaggctc cggcgggggc gcaggaggtc
61 gcccggcgcg tcactgtcgg gtcggcgagc cacgggggcc gccgcagcac catggcgacc
121 accgtcagca ctcagcgcgg gccggtgtac atcggtgagc tcccgcagga cttcctccgc
181 atcacgccca cacagcagca gcggcaggtc cagctggacg cccaggcggc ccagcagctg
241 cagtacggag gcgcagtggg caccgtgggc cgactgaaca tcacggtggt acaggcaaag
301 ttggccaaga attacggcat gacccgcatg gacccctact gccgactgcg cctgggctac
361 gcggtgtacg agacgcccac ggcacacaat ggcgccaaga atccccgctg gaataaggtc
421 atccactgca cggtgccccc aggcgtggac tctttctatc tcgagatctt cgatgagaga
481 gccttctcca tggacgaccg cattgcctgg acccacatca ccatcccgga gtccctgagg
541 cagggcaagg tggaggacaa gtggtacagc ctgagcggga ggcaggggga cgacaaggag
601 ggcatgatca acctcgtcat gtcctacgcg ctgcttccag ctgccatggt gatgccaccc
661 cagcccgtgg tcctgatgcc aacagtgtac cagcaggcgg ttggctatgt gcccatcaca
721 gggatgcccg ctgtctgtag ccccggcatg gtgcccgtgg ccctgccccc ggccgccgtg

781 aacgcccagc cccgctgtag cgaggaggac ctgaaagcca tccaggacat gttccccaac
841 atggaccagg aggtgatccg ctccgtgctg gaagcccagc gagggaacaa ggatgccgcc
901 atcaactccc tgctgcagat gggggaggag ccatagagcc tctgcctcga tgccgttttg
961 cccccgctct ttggacacgc cgacccggcg ctccccaagg aatgctgtcc caacaagatt
1021 cccgtgaaag agcacccgtg tcgccccctc ccgtggactt ctgtgccgcc ccgtccacac
1081 ctgttcttgg gtgcatgtgg gttttcggtt cctggcggtc caggacgggg cgggggctcc
1141 cctcccatct cgtgctggga ggtctcagcg cgctctcctg tccctgggac gtgcgtctct
1201 ccttctcatg ccgttctgga aaatgctctt gctgtagaga gcagctgctt ctgccagggt
1261 gttggaggtg gtggagcgcc ttccgattcc attcatggca ttttgtgatg tgatgtaatt
1321 ggaatagagc tgttgattta aggcacacac aatccctcac actgtgggtt tttttagaa
1381 cttcccagac gaaaactcac gcccttgccc taacgcgctt tgctgtgagc ctggcccctg
1441 cccagggctt gggtctggtg agctgagcag cttcctgtgg atggtgtggg gccggcctct
1501 ggcctggctc acctggccac tgtccagcca gccttgtgac agactccggc ctgaaggcag
1561 aatgaaccca cacctggagt gaggaagggg gcctggcacg gttggccagg ctctgcctga
1621 ttgccagcca gcgggcatct gaagccgggt ccttcgcccg ccggaggctg ccgtccgtct
1681 ctcctgctgc gctcgtgcca gctccgtggg tgtcctccca gggagcttct cttctcaaca
1741 ggccttgcga ggctggggtg agaggtgata gaggcagcac tgtgcatgat tccgagaggg
1801 tgtggtggca ctgccagccg actgctgaca gcttgggagc tgctgtgccc aggacgtggg
1861 ttcagcgtgg gcgaggaaag cctggcgagc gtggccctgt aaaagctttc tgaggcggga
1921 ggcgctcact tacctctgac tgcctgggcg ctgcgtgtag catcttggcc tacaggacag
1981 attttaggtg acacctggtt atgacagtca gaaatttgag aagcttctca caagtgatgc
2041 actttaaata atctgcatgc cattgagaca cctgcatgtc tggtgtttgt ggttcaagtg
2101 tcttgccgcc ggccttcgga tgtaaaccca ctgataacgg acagaaagag aatgcccaca
2161 agtgggtctt ctgtggaaga tgcagaagga ggaagttagt gcttacattt tagtcttttt
2221 ctccctcaaa aaaataggtt aagtttcagt gccagctaga aaatactgct ttctgccatc
2281 gattgggggt ggtttttgtc aaatatactg ttgataaata tttatttttg taaacttgaa
2341 gtgtgtggtg gccgtggggg agggacatgc tggcagcagg cgccttcttc agctgtgggt
2401 cctaaaggcc tttgatcctt tgaagaagaa agacatggta tttgttcagc agacgccgac
2461 cactcagacg gaggggcccc tgggattccc tgtctcagat ggcctggtct tacgcctgtg
2521 tagatttctt ctccattggg aatgaaggtg tcaggcggga ctggaacgtt ctagatggta
2581 tgttccgtga tattaacaac tctaacccag gacagaccac aagccacact cagaggcctc
2641 actgtgctgg gggcttcggt gtccaggcgc ccaggtgtgg ccaccagcac cggtttctgc
2701 cttcgcgttg ctggggtgca gtgagactgc cacacgcgtg cacatgtggc tctgtgggtg
2761 tctcctagag aggacgtggc ccctgctgcc agcccttgag cagcccgtgt gggggcccga
2821 gggacccaca cagtgggggc cagcctcgct ggagggagag caaccctttg ccgatgacca
2881 cgcttgccgc catctcttag ttttcttttt cacaagcgct ttattttttt aatagacaaa
2941 tcacattttg caaggccttt aattaaataa gattcttctt tccttcattt tatgctttat
3001 ttcctgtttg aaggcttact gtagaagtgg cttactgtag aagcagcttg ctgagcccct
3061 ccgagcggtc cccagaatta gctggttcac aacccccacc ctcccccgcc cccgcctgtg
3121 tcaggtgtgg atgaggtcgt cacactcaga aggacaggct tgtctgccag ctcacaaggg
3181 gaggctgcag tgggtttggg agctgggttt aggcccctgg tgtctgaggg cccaggcctt
3241 gccagcctct gctgctcctg ctcctgggtt tgaagatgca ggccgatcgc cagctccgtg
3301 gcagcggtca ctaaggacag cctgactgtg ccatcttgga gcctcaggcg ggctccgga
3361 gatagaagac aggtcgccgg aggctccccc tcctctcctc tcccctctgc agatgctccc
3421 tgggcgctac cctgcagggt gccaggcagg agtggtctca gaacgtgcgc ttctgattat
3481 tttactgggg tccattgtcc agatttttct ttgattgtaa aatatatttt tactttttag
3541 tcttctaatt taataaatga tccatataaa aatagagaaa taaagtcctt taagggaagg
3601 tttaaaaaaa aaaaa//

- LOCUS: NM_021922 2565 bp mRNA linear PRI 18-NOV-2006
- DEFINITION: Homo sapiens Fanconi anemia, complementation group E (FANCE), mRNA.
- ACCESSION: NM_021922
- VERSION: NM_021922.2 GI:66879667

1 aacggctgcg gcttcgggcg gccgggtttc tccggtctcc caacgccgag gagagcttgt
61 aacaggcgct ggagctggcc cgccaccgcc gcgtcaggga cggcgctgga gtcctccgtt
121 cccctcagcc tctgagctga ggccccacac cagagtaggg ggcggcgcgg cacccgtgcc
181 ccggcatggc gacaccggac gcggggctcc ctggggctga gggcgtggag ccggcgccct
241 gggcgcagct ggaggccccc gcccgcctcc tgctgcaggc gctgcaggcg gggcctgagg
301 gggcgcggcg cggcctgggg gtgctccggg cgctgggcag ccgcggctgg gagcccttcg
361 actggggtcg cttgctcgag gccctgtgcc gggaggagcc ggtcgtgcag gggcctgacg
421 gccgtctgga gctgaaacca ctgttgctgc gattgccccg gatatgccag aggaacctga

481 tgtccctgct gatggccgtt cggccatcgc tgccggaaag tgggctcctc tctgtgctgc
541 agattgccca gcaggaccta gcccctgacc cagatgcctg gctccgtgcc ctgggggaat
601 tgctgcgaag ggatttgggg gtggggacct ccatggaggg agcttctcca ctgtctgaaa
661 gatgccagag acagctccaa agtctatgta gggggctggg cctggggggc aggaggttga
721 aatcccccca ggctccagac cctgaagaag aggagaacag ggactcccag cagcctggga
781 aacgcagaaa ggactcagag gaagaggctg ccagtcctga ggggaagagg gtccccaaaa
841 gattacggtg ttgggaagag gaagaagatc atgagaagga gagacccgaa cataagtcac
901 tggaatccct ggcagatgga ggaagtgcat ctcctattaa ggaccagcct gtcatggcag
961 ttaagactgg cgaggacggt tcgaatctgg atgatgctaa aggtctggct gagagtttgg
1021 agttgcccaa agctatccag gaccagcttc ccaggctgca gcagctgctg aagaccttgg
1081 aggaggggtt agagggattg gaggatgccc ccccagttga gctacagctt cttcacgaat
1141 gtagtcccag ccagatggac ttgctgtgtg cccagctgca gctccctcag ctctcagacc
1201 tcggtctcct gcggctctgc acctggctgc tggccctttc acctgatctc agcctcagca
1261 atgctactgt gctgaccaga agcctctttc ttggacggat cctctccttg acttcctcag
1321 cctcccgcct gcttacaact gccctgacct ccttctgtgc caaatataca taccctgtct
1381 gcagcgccct ccttgaccct gtgctccagg ccccaggcac aggtcctgct caaacagagt
1441 tactgtgttg ccttgtgaag atggagtccc tggagccaga tgcacaggtt ctaatgctgg
1501 gacagatctt ggagctgccc tggaaggagg aaactttctt ggtgttgcag tcactcctag
1561 agcggcaggt ggagatgacc cctgagaagt tcagtgtctt aatggagaag ctctgtaaaa
1621 aggggctggc agccaccacc tccatggcct atgccaagct catgctgaca gtgatgacca
1681 agtatcaggc taacatcact gagacccaga ggctgggcct ggctatggcc ctagaaccta
1741 acaccacctt cctgaggaag tccctgaagg ccgccttgaa acatttgggc ccctgaccat
1801 ccaccaaggg accaccctct tggtgctcca tcaccagctt cctgaagggc atttctttct
1861 tcaccacctt gtcttgagcc ctagcctgag gataaaggct gagcctggcc atcccagatt
1921 gcaactgcct ccctgttaca ggctgcatag ggaatattgg cttcccagcc agcagggagg
1981 ctcctgggct aagggagctc agctatattt tctttttcat ttcttttgtt tttgagagaa
2041 ggtattgctc tgtcatccag gctggagtgc agtgatgcga ttgatcatgg ctcactgtaa
2101 cctctgcctc ccaggctcga tcctcccatg tcagcctccc aagtagctgg gactacatgc
2161 acatgacact atgcccagct aattttttat tttgtagata atatgctggt cagggctggt
2221 cttgaactcc tgagctcaag tgatcttcct gccttggcac ccaaagtgtt gagatttaca
2281 ggtgtgagcc accacaccag gccagagcta tatttccaaa ggctgctggc cccaggcaca
2341 ctcctcatca attctcaggc tgcagggaca aactttccga tagggctcag taggatcaag
2401 ccgacccaga gtgggcatgg gatgctccag gaggtctggg gtaccagtgg gctcagatga
2461 agctcttgtg ctcatgtata ttatgacctt ctgtgttttt gtttctgact tgaataattt
2521 atcaatggtg ttgaataaaa gcaagttcaa taatgaaaaa aaaaa//

- LOCUS: NM_012123 2537 bp mRNA linear PRI 18-NOV-2006
- DEFINITION: Homo sapiens mitochondrial translation optimization 1 homolog (S.cerevisiae) (MTO1), transcript variant 2, mRNA.
- ACCESSION: NM_012123
- VERSION: NM_012123.2 GI:74024894

1 ggccgcgccc tgcagattgt ctcttgttgc gtaagttttt ttgaccgtca ctcgtgtcag
61 cttcaaagtc agatagattt ttctcccagc atgttctact tccgaggctg tggccgttgg
121 gtcgcggttt ccttcaccaa gcagcaattt ccgttggcac ggttgagcag tgacagcgcg
181 gcgccccgga ctccgcactt cgacgtgata gtcattggtg gaggacatgc cgggactgag
241 gcagccaccg ccgccgctcg gtgcggctct cggactctgc tcctcactca ccgcgtggac
301 acgatcggtc agatgtcatg taatccttcc tttggtggca tcggaaaggg acatttaatg
361 agggaagtag atgccttgga tggcctgtgt tctcgcatct gtgaccagtc tggtgtacat
421 tataaagtat taaaccggcg taaggaccca gctgtgtggg gtctgagagc tcagattgat
481 aggaaactct acaaacagaa catgcagaaa gaaatcttga atacaccact gcttactgtt
541 caggagggag ctgtagaaga tcttattctt acagaaccag agcctgaaca cactgggaaa
601 tgccgtgtca gtggggttgt tttggtggat ggaagcacag tatatgcaga gagtgtgatt
661 ctgactactg ggacatttct gagaggcatg attgtaattg gattggagac gcatccagca
721 ggacgtttag gggatcagcc ttctatagga ttggctcaga cactggagaa gttagggttt
781 gtggtgggaa ggttgaagac tgggactcca ccccgaattg ccaaagagtc cattaatttc
841 agtattctaa acaagcatat accggacaat ccatccatac cattcagctt taccaatgag
901 acagtatgga ttaagccaga agatcagctg ccatgttact tgactcacac caaccctaga
961 gtggatgaga ttgtccttaa gaaccttcac cttaatagtc atgttaaaga aacgacaaga
1021 ggacctcgat actgtccctc cattgaatca aaagttttgc gttttccaaa ccgtctacat
1081 caggtttggt tggaacctga aggaatggat tctgacctta tctacccaca ggggttatct
1141 atgacgctac cagctgagtt acaagagaaa atgatcacat gcatcagagg cttggagaaa
1201 gctaaagtga ttcagccagg ctacggtgtt cagtatgatt acttagatcc ccgtcagatc

1261 accccttcct tggagactca tttggttcaa cgactcttct ttgctggaca gatcaatggc
1321 accactggtt atgaggaagc tgcagctcaa ggtgtgatag ccggaatcaa cgccagtctt
1381 cgggtcagtc gcaagcctcc ctttgtggtt agccgaacag aaggttacat aggagtcttg
1441 attgatgacc tcactactct gggcaccagt gaaccatacc gcatgtttac cagccgagta
1501 gagttccgtt tgtcactgcg ccctgataat gctgacagcc ggctcacact gcgagggtat
1561 aaagacgctg gctgtgtgtc ccaacaacga tatgaaagag cttgttggat gaagtcttct
1621 ttagaagaag gcatttctgt gttgaaatct attgagtttt tgagctctaa atggaaaaaa
1681 ttaatcccag aggcttctat aagtactagt agaagtctgc ctgtcagagc tctcgatgtt
1741 ctgaagtatg aggaagttga catggattca ttagccaagg ctgttccaga gcccttgaag
1801 aagtatacta aatgtagaga gctggctgaa agactgaaaa tagaagccac ttatgaatca
1861 gtgttgttcc atcaactaca agaaataaag ggagttcagc aagatgaagc tctccaactg
1921 ccaaaagacc tagattattt gactatcagg gatgtgtctt tgtcccatga agttcgagag
1981 aaactacatt ttagtcgtcc acagacgatc ggggctgcta gtcgcatacc cggagtaaca
2041 cctgccgcca tcatcaatct gctgagattt gtgaagacca ctcaacgaag acagtcggct
2101 atgaatgaat catccaagac tgatcaatac ttatgtgatg cagacagact tcaagagaga
2161 gagttatagc tttcaattca taaaagattt ttaaagagca tataaataat ttgatcaata
2221 caacagtata gataaaagaa ttatttagca catgttaaaa tagctttatt aggttactat
2281 gggtttgcca ttaatttctg agtgggacag aaattataat tgtgcttttt cgtgtatatg
2341 aaaaaactag tcgtaaacaa tttgtactct ttctttaagg agctgtaata caaataactt
2401 tgtgcagtgt tcatcaaaga gagagacagt gaacctaaaa ctgaacctgg aataaaactc
2461 aacatgcaga tttgcctact catagggact ttgcctatta agtctaccaa attaaaagtc
2521 ttatcattca aaaaaaa//

- LOCUS: BC003170 3889 bp mRNA linear PRI 15-JUL-2006
- DEFINITION: Homo sapiens ubiquitin associated protein 2-like, mRNA (cDNA clone MGC:4404 IMAGE:2906083), complete cds.
- ACCESSION: BC003170
- VERSION: BC003170.1 GI:13111994
- KEYWORDS.: MGC.

1 gagactgagt attctacctt gtaaatactg ttatttgtat atactgtaaa tgatgacatc
61 ggtgggcact aaccgagccc ggggaaactg ggaacaacct caaaaccaaa accagacaca
121 gcacaagcag cggccacagg ccactgcaga acaaattaga cttgcacaga tgatttcgga
181 ccataatgat gctgactttg aggagaaggt gaaacaattg attgatatta caggcaagaa
241 ccaggatgaa tgtgtgattg ctttgcatga ctgcaatgga gatgtcaaca gagctatcaa
301 tgttcttctg gaaggaaacc cagacacgca ttcctgggag atggtcggga agaagaaggg
361 agtctcaggc cagaaggatg gtggccagac ggaatccaat gaggaaggca aagaaaatcg
421 agaccgggac agagactata gtcggcgacg tggtgggcca ccaagacggg ggagaggtgc
481 cagccgtgga cgagagtttc gaggtcagga aaatggattg gatggcacca agagtggagg
541 gccttctgga agaggaacag aaagaggcag aaggggccgt ggccgaggca gaggtggctc
601 tggtaggcga ggaggaaggt tttctgctca aggaatggga acctttaacc cagctgatta
661 tgcagagcca gccaatactg atgataacta tggcaatagc agcggcaata cgtggaacaa
721 cactggccac tttgaaccag atgatgggac gagtgcatgg aggactgcaa cagaggagtg
781 ggggactgaa gattggaatg aagatctttc tgagaccaag atcttcactg cctctaatgt
841 gtcttcagtg cctctgcctg cggagaatgt gacaatcact gctggtcaga gaattgacct
901 tgctgttctg ctggggaaga caccatctac aatggagaat gattcatcta atctggatcc
961 gtctcaggct ccttctctgg cccagcctct ggtgttcagt aattcgaagc agactgccat
1021 atcacagcct gcttcaggga acacattttc tcatcacagt atggtgagca tgttagggaa
1081 aggatttggt gatgtcggtg aagctaaagg cggcagtact acaggctccc agttcttgga
1141 gcaattcaag actgcccaag ccctggctca gttggcagct cagcattctc agtctggaag
1201 caccaccacc tcctcttggg acatgggctc gacgacacaa tccccatcac tggtgcagta
1261 tgatttgaag aacccaagtg attcagcagt gcacagcccc tttacaaagc gccaggcttt
1321 taccccatct tcaaccatga tggaggtgtt ccttcaggag aagtcacctg cagtggctac
1381 ctccacagct gcacctccac ctccgtcttc tcctctgcca agcaaatcca catcggctcc
1441 acagatgtcg cctggatctt cagacaacca gtcctctagc cctcagccgg ctcaccagaa
1501 actgaaacag cagaagaaaa aagcctcctt gacttctaag attcctgctc tggctgtgga
1561 gatgcctggc tcagcagata tctcagggct aaacctgcag tttggggcat tgcagtttgg
1621 gtcagagcct gtcctttctg attatgagtc cacccccacc acgagcgcct cttcaagcca
1681 ggctccaagt agcctgtata ccagcacggc cagtgaatca tcctctacaa tttcatctaa
1741 ccagagtcag gagtctggtt atcagagcgg cccaattcag tcgacaacct atacctccca
1801 aaataatgct cagggccctc tttatgaaca gagatccaca cagactcggc ggtaccccag
1861 ctccatctct tcatcacccc aaaaggacct gactcaggca aagaatggct tcagttctgt
1921 gcaggccacg cagttacaga ccacacaatc tgttgaaggt gctacaggct ctgcagtgaa

1981 atctgattca ccttccactt ctagcatccc ccctctcaat gaaacggtat ctgcagcttc
2041 cttactgacg acaaccaatc agcattcatc ctccttgggt ggcttgagcc acagtgagga
2101 gattccaaat actaccacca cacaacacag cagcacgtta tctacgcagc agaataccct
2161 ttcatcatca acatcttctg ggcgcacttc gacatccact cttttgcaca caagtgtgga
2221 gagtgaggcg aatctccatt cttcctccag cactttttcc accacatcca gcacagtctc
2281 tgcacctccc ccagtggtca gtgtctcctc cagtctcaat agtggcagta gcctgggcct
2341 cagcctaggc agcaactcca ctgtcacagc ctcgactcga agctcagttg ctacgacttc
2401 aggaaaagct cctcccaacc tccctcctgg ggtcccgccg ttgttgccta atccgtatat
2461 tatggctcca gggctgttac atgcctaccc gccacaagta tatggttatg atgacttgca
2521 gatgcttcag acaagatttc cattggatta ctacagcatc ccatttccca cacccactac
2581 tccgctgact gggagggatg gtagcctggc cagcaaccct tattctggtg acctcacaaa
2641 gttcggccgt ggggatgcct cctccccagc cccggccaca accttggccc aaccccaaca
2701 gaaccagacg cagactcacc ataccacgca gcagacattc ctgaacccgg cgctgcctcc
2761 tggctacagt tacaccagcc tgccatacta tacaggggtc ccgggcctcc ccagcacctt
2821 ccagtatggg cctgctgtgt tccctgtggc tcctacctct tccaagcagc atggtgtgaa
2881 tgtcagtgtg aatgcatcgg ccaccccttt ccaacagccg agtggatatg ggtctcatgg
2941 atacaacact ggtgtttcag tcacctccag taacacgggc gtgccagata tctcgggttc
3001 tgtgtactcc aaaacccagc agtcctttga gaaacaaggt tttcattccg gtactcctgc
3061 tgcttccttc aacttgcctt cagccctagg aagtgggggc cccatcaatc cggccacagc
3121 tgctgcctac ccacctgccc cctttatgca cattctgacc ccccatcagc agccgcattc
3181 tcagatcctt caccatcacc tgcagcagga tggccagacg ggcagcgggc aacgtagcca
3241 gaccagctcc atcccgcaga agccccagac caacaagtct gcctacaaca gctacagctg
3301 gggggccaac tgaggccctg accctcttct cccggtccca tcttctgaga gggcttctca
3361 gcctggaaac tatggaaaca gcatcaaaga gaaaggaatg tgggggtttt ccgctgcccc
3421 ccacccccag cggcccaccc catgcctcag cttcatgtct gtcccattcc tataccatcc
3481 ccaccctgtt gtatgtatta taggatttgt attttctcct tttttttccc ccttccattc
3541 cttctcccct cttgcattca agattatgaa actttgctat gggccctgca cttcctttgc
3601 ttcctcctgt tcaccctggt ggtgtacgga tgaggcgggg aggtgggacc cccaaacata
3661 tatcagccca acagccctaa gtctccttct ttattattag gaaaacaaca acaacaacaa
3721 acaaaaaaat ggcgtcatga atatgaacag cattgtcaga tgaattagtt gaagtggttt
3781 tttttttgtt tttttttttt tttttgtact gtgtcctcaa atttaatgga ttaatgtgtc
3841 ttgtatatat aaaaagaaaa cctctacctt caaaaaaaaa aaaaaaaaa//

- LOCUS: HSU13395 1475 bp mRNA linear PRI 15-SEP-1994
- DEFINITION: Human oxidoreductase (HHCMA56) mRNA, complete cds.
- ACCESSION: U13395
- VERSION: U13395.1 GI:538131

1 atcttggcct gcaggaacat ggcaagggcg agtgaagcag tgtcacgcat tttagaagaa
61 tggcataaag ccaaggtaga agcaatgacc ctggacctcg ctctgctccg tagcgtgcag
121 cattttgctg aagcattcaa ggccaagaat gtgcctcttc atgtgcttgt gtgcaacgca
181 gcaacttttg ctctaccctg gagtctcacc aaagatggcc tggagaccac ctttcaagtg
241 aatcatctgg ggcacttcta ccttgtccag ctcctcccag ggatgttttg tgccgctcag
301 ctcctgcccg tgtcattgtg gtctcctcag agtcccatcg atttacagat attaacgact
361 ccttgggaaa actggacttc agtcgcctct ctccaacaaa aaacgactat tgggcgatgc
421 tggcttataa caggtccaag ctctgcaaca tcctcttctc caacgagctg caccgtcgcc
481 tctcccacgc ggggtcacgt cgaacgcagt gatcgatcct ggaaatatga tgtactccaa
541 cattcatcgc agctggtggg tgtacacact gctgtttacc ttggcgaggc ctttcaccaa
601 gtccatgcaa cagggagctg ccaccaccgt gtactgtgct gctgtcccag aactggaggg
661 tctaggaggg atgtacttca acaactgctg ccgctgcatg ccctcaccag aagctcagag
721 cgaagagacg gcccggaccc tgtgggcctc agcgagaggc tgatccaaga acgcttggca
781 gccagtccgg ctaagtggag ctcagagcgg atgggcacac acacccgccc tgtgtgtgtc
841 ccctcacgca agtgccaggc tgggcccctt ccaaatgtcc ctccaacaca gatccgcaag
901 agtaaaggaa ataagagcat tcacaacaga gtgaaaaatc ttaagtacca atgggaagca
961 gggaattcct ggggtaaagt atcacttttc tggggctggg ctaggcatag gtctctttgc
1021 tttctggtgg tggcctgttt gaaagtaaaa acctggttgg cgtgtaggtt ccgtatctcc
1081 ctggagaagc accagcaatt ctcttccttt tactgttata gaatagcctg aggtcccctc
1141 gtccatccag ctaccaccac caccaccact gcagccaggg gctggccttc tcctacttag
1201 ggaagaaaaa gcaagtgttc actgctcctt gctgcattga tccaggagat aattgtttca
1261 ttcatcctga ccaagactga gccagcttag caactgctgg ggagacaaat ctcagaacct
1321 tgtcccagcc agtgaggatg acagtgacac ccagagggag tagaatacgc agaactacca
1381 ggtggcaaag tacttgtcat agactccttt gctaatgcta tacaaaaaat tctttagaga
1441 ttataacaaa tttttcaaat cattccttag atacc//

- LOCUS: X55503 2294 bp DNA linear PRI 14-NOV-2006
- DEFINITION: H.sapiens pseudogene for metallothionein and AG/CT repetitive element.
- ACCESSION: X55503
- VERSION: X55503.1 GI:38328
- KEYWORDS: metallothionein; pseudogene.

1 ttccttnncc ttcttaaacc attcacagtt tcatcttgac ttgtgaaggt gagcacgggg
61 tgtgaaaggg gtttttgttt gtttgtttgt tttaaggcag agtctcactc catcacccag
121 gctaggagtg cagaggtgtg atctcggctc actgcaacct ccatctccca agttcaagag
181 attctcgtgt ctcagcctcc ctagtagcta accgcttttt tgcacaacat ggggatcat
241 gtaactcgcc ttgatcgttg ggaaccggag ctaaactcca ggcctcatgt gattcacctg
301 tcttggcctc ccaaagtgct gggattacag acgtgagcca ctgtgcctag ccaagaggct
361 gtgaaaggct ttgatagttc ttagaggaac cttgggcttg gaacgctggc ctcgtctgtt
421 ggttctcatt gtacataaga attatttggg ttgcttttca aaaaatatgg actccatttc
481 cccaccaaga cttatgaatc agaacatctc tcaagggttg gtcccaggaa tctgcatttt
541 cacagttatc ccaggcatga ttatgcagcc atgccagttc ccatccatgg cctgtgactg
601 tggactattg cagagtcgtt gacaactggg tcgatgctca gcagccttgt caccctcttg
661 tcaagccagt ccccaaggat atagcccaga gcttgcaccg tagtgcatta tgcctttagt
721 tccccacttc atctccatcc ttacgtttgt ggtggctctg tcctgtcttc taggaggaat
781 ctgcatgtgt ggagacaact gcaaatgcac aacctgcaac tgtaaaacat gtcggaagag
841 tgagtatggt gactgggggc accatggcgg tgggagttag aaaagtccaa tccaggccag
901 gtgcagtggc tcacgtctgt aatcccagca ctttgggagg ccgaggcggg tggattacct
961 gaggtcagga gttcaagacc agcctggcca acatggtgaa accccatctc tactaaaaat
1021 acaataatta gctgggcatg gtggtgggca cctgtaatcc cagctactca ggaggctgag
1081 gcaggagaat tgcttgaacc caggaggcag aggttgcagt gagcagagat cacaccattg
1141 cactccagcc ttggtgacaa gagtgaaact cagtcaaaaa aagaaaagga aggaagaaga
1201 agaaaggaag gaaggaagga agggagagag agagagagag aaagaaagag agagagagag
1261 agagagagag agaagaagaa agaaagaaag aaagaaagaa agaaagaaag aaagaaagaa
1321 agaaagaaag aaagaaagaa agatccctat ccccaccctc aatcctccag ccatggtgga
1381 ttggggcttg tggggagggg gtctagatgc ctttacccat ctggagagat ctgaaatgag
1441 aactatgtag agacctatgg caggtatcta cccagggata agggcatcca cagtgtttgg
1501 agaaggaaga ggactgaggg gaagttctgt cctgcccagt ngtaacaggg atagagcact
1561 ggcctggagt tatgagccct aggttctagt cttcttttgc cactgtctca tgaccttggc
1621 aaaccccctc ccttctgtgg gcttctgatg gactctagat ggtccctggg gatcccttcc
1681 aagttctaac atgcctcaaa cctcctatct caagcatttt tgaaatgtgg attaaacctt
1741 agtcacacac cccttctttt tcctaatttc ctcttaagta gttgggaggg gtcagtggag
1801 tgagaggaag tgtggttaac ccctcagcgg atctgcgcat ctcctactct ttnncaggct
1861 gctgttcctg ctgccccccg ggctgtgcca aatgtgcccg gggctgcatc tgcaaaggag
1921 gctcagacaa gtgcagctgc tgcccatgaa agccatccat cgtgcccacc ccccccaagg
1981 agagaaacct gggaagtgtc tgtacagtgc atgaatagag aaggtggaat aattgtcaca
2041 ataggttgtg ctttttatat atttgcccaa atgtggtgtt ggtcacattc atgtaaagta
2101 tttggggcaa taaagttttc actcttggtt gcctggtggc tcagagcatt gtttttccct
2161 aacccagcag gaccaaaagg tgctgcttct tttgcagatc ctgtaaccac cacccacatg
2221 tacctgtgac aggtggctgc cttctgcaag gtcctatgac tctgcctggg ggctttctcc
2281 tcctgcacaa gctt//

- LOCUS: AF263541 1746 bp mRNA linear PRI 02-AUG-2000
- DEFINITION: Homo sapiens protein kinase DYRK4 (DYRK4) mRNA, partial cds.
- ACCESSION: AF263541
- VERSION: AF263541.1 GI:9652079

1 cacattagca agaaagtcct gctgaagtca tccctgctgt atcaggagaa tcaagctcac
61 aatcagatgc cggcctcaga gctcaaggct tcagaaatac ctttccaccc tagcattaaa
121 acccaggatc ccaaggcaga ggagaagtca ccaaagaagc aaaaggtgac tctgacagcg
181 gcagaggccc taaagctttt taagaaccag ctgtctccat atgaacaaag tgaaatcctg
241 ggctacgcgg agctgtggtt cctgggtctt gaagccaaga agctcgacac ggctcctgag
301 aaatttagca agacgagttt tgatgatgag catggcttct atctgaaggt cctgcatgat
361 cacattgcct accgctatga agttctggag acaatcggga aggggtcctt tggacaggtg
421 gccaagtgct tggatcacaa aaacaatgag ctggtggccc tgaaaatcat caggaacaag
481 aagaggtttc accagcaggc cctgatggag ctgaagatcc tggaagctct cagaaagaag
541 gacaaagaca acacctacaa tgtggtgcat atgaaggact ttttctactt tcgcaatcac

601 ttctgcatca cctttgagct cctgggaatc aacttgtatg agttgatgaa gaataacaac
661 tttcaaggct tcagtctgtc catagttcgg cgcttcactc tctctgtttt gaagtgcttg
721 cagatgcttt cggtagagaa aatcattcac tgtgatctca agcccgaaaa tatagtgcta
781 taccaaaagg gccaagcctc tgttaaagtc attgactttg gatcaagctg ttatgaacac
841 cagaaagtat acacgtacat ccaaagccgg ttctaccgat ccccagaagt gatcctgggc
901 cacccctacg acgtggccat tgacatgtgg agcctgggct gcatcacggc ggagttgtac
961 acgggctacc ccctgttccc cggggagaat gaggtggagc agctggcctg catcatggag
1021 gtgctgggtc tgccgccagc cggcttcatt cagacagcct ccaggagaca gacattcttt
1081 gattccaaag gttttcctaa aaatataacc aacaacaggg ggaaaaaaag atacccagat
1141 tccaaggacc tcacgatggt gctgaaaacc tatgacacca gcttcctgga ctttctcaga
1201 aggtgtttgg tatgggaacc ttctcttcgc atgaccccgg accaggccct caagcatgct
1261 tggattcatc agtctcggaa cctcaagcca cagcccaggc cccagaccct gaggaaatcc
1321 aattcctttt tcccctctga gacaaggaag gacaaggttc aaggctgtca tcactcgagc
1381 agaaaagatg agatcaccaa agagactaca gagaaaacaa aagatagccc cacgaagcat
1441 gttcagcatt caggtgatca gcaggactgt ctccagcacg gagctgacac tgttcagctg
1501 cctcaactgg tagacgctcc caagaagtca gaggcagctg tcggggcgga ggtgtccatg
1561 acctccccag gacagagcaa aaacttctcc ctcaagaaca caaacgtttt accccctatt
1621 gtatgacctt tgctgagggt atgtcctgct cctttccacc agtgatttgt attaagacag
1681 cacttatatt gtacaatact tcagactgtt ttttttaaat acataaaact ttatgttaaa
1741 aactct//

- LOCUS: HSU67122 816 bp mRNA linear PRI 15-FEB-1997
- DEFINITION: Human ubiquitin-related protein SUMO-1 mRNA, complete cds.
- ACCESSION: U67122
- VERSION: U67122.1 GI:1762972

1 gatttgtaaa ccccggagcg aggttctgct tacccgaggc cgctgctgtg cggagacccc
61 cgggtgaagc caccgtcatc atgtctgacc aggaggcaaa accttcaact gaggacttgg
121 gggataagaa ggaaggtgaa tatattaaac tcaaagtcat tggacaggat agcagtgaga
181 ttcacttcaa agtgaaaatg acaacacatc tcaagaaact caaagaatca tactgtcaaa
241 gacagggtgt tccaatgaat tcactcaggt ttctctttga gggtcagaga attgctgata
301 atcatactcc aaaagaactg ggaatggagg aagaagatgt gattgaagtt tatcaggaac
361 aaacgggggg tcattcaaca gtttagatat tctttttatt ttttttcttt tccctcaatc
421 cttttttatt tttaaaaata gttcttttgt aatgtggtgt tcaaaacgga attgaaaact
481 ggcaccccat ctctttgaaa catctggtaa tttgaattct agtgctcatt attcattatt
541 gtttgttttc attgtgctga tttttggtga tcaagcctca gtccccttca tattaccctc
601 tcctttttaa aaattacgtg tgcacagaga ggtcaccttt ttcaggacat tgcattttca
661 ggcttgtggt gataaataag atcgaccaat gcaagtgttc ataatgactt tccaattggc
721 cctgatgttc tagcatgtga ttacttcact ccctggactg tgactttcag tgggagatgg
781 aattttttcc agaaaaactg aactgtggaa aaatga//

- LOCUS: NM_003905 1820 bp mRNA linear PRI 18-NOV-2006
- DEFINITION: Homo sapiens amyloid beta precursor protein binding protein 1(APPBP1), transcript variant 1, mRNA.
- ACCESSION: NM_003905
- VERSION: NM_003905.3 GI:66363682

1 agtgcgcctg cgcgcttgtg gagctggtgg cggcgctccg caggggctcg gctgttttcc
61 gcgcggcagg cgcggccatg gcgcagctgg gaaagctgct caaggagcag aagtacgacc
121 ggcagctgag gttgtggggt gatcatgggc aagaggcttt agaatctgct catgtttgcc
181 taataaatgc aacagccaca ggaactgaaa ttcttaaaaa cttggtacta ccaggtattg
241 gttcgtttac aattattgat ggaaatcagg tcagcggaga agatgctgga aacaatttct
301 tccttcaaag aagcagtatc ggcaagaacc gagctgaagc tgccatggaa ttcttacaag
361 aattaaatag cgatgtctct ggaagttttg tggaagagag tccagaaaac cttctagaca
421 atgatccctc atttttctgt aggtttactg ttgtagttgc aactcagctt cctgaaagca
481 cttcactacg cttagcagat gtcctctgga attcccagat tcctcttttg atctgtagga
541 catatggact agttggttat atgaggatca ttataaaaga acatccagta atagaatctc
601 atccagataa tgcattagag gatctacgac tagataagcc atttcctgaa ctgagagaac
661 attttcagtc ctatgatttg gatcatatgg aaaaaaagga ccacagtcat actccatgga
721 ttgtgatcat agctaaatat ttagcacagt ggtatagtga aacaaatgga cgaataccta
781 aaacgtataa agaaaaagag gacttcagag atttgattag acaaggaatt ctaaaaaatg
841 aaaatggggc tccagaagat gaagagaatt ttgaagaagc tattaaaaat gtgaacacag
901 cactaaatac aactcagatc ccaagcagta ttgaagatat atttaatgat gatcgctgca
961 taaatatcac caaacagact ccatcatttt ggattttagc tcgtgcctta aaggaatttg
1021 tggccaaaga gggtcaagga aatttacctg ttcgaggcac aattcctgat atgattgcag

1081 attcaggcaa atatataaaa ctgcaaaacg tttaccgtga aaaagcaaag aaagatgctg
1141 ccgctgtggg taatcatgtt gccaaattgc tgcagtccat tggccaggca ccagagtcca
1201 tttcagagaa agaattaaaa ttactctgca gcaattctgc atttcttcga gtggtaagat
1261 gtcgatcctt agctgaagaa tatggtttgg atacaattaa caaggatgaa attatttcta
1321 gcatggacaa tccagataat gaaatagtgt tgtacttaat gttacgggct gttgatagat
1381 ttcataaaca acagggtaga tatccaggag tatctaacta tcaagttgaa gaagatatag
1441 gaaagttgaa gtcttgtctc actggcttcc ttcaggaata tggtttatct gtaatggtga
1501 aagatgatta tgtccacgaa ttttgccgat atggagctgc tgagccacat accattgctg
1561 cattcttggg gggagctgct gctcaagagg tcatcaaaat aatcaccaaa caatttgtaa
1621 tttttaataa tacttacatt tacagtggca tgtcacaaac ttcagcaact ttccagttgt
1681 agagtaagca agcaccttaa gtagtgtgtt aatgattgaa actgtaattg ccttcgggtt
1741 gtgctttagt ctgtaaaatt ctaaaggaga gctgctaaat tgttttctta ataaacattt
1801 ttctcatttg taaaaaaaaa//

- LOCUS: AK024527 1636 bp mRNA linear PRI 12-SEP-2006
- DEFINITION: Homo sapiens cDNA: FLJ20874 fis, clone ADKA02818.
- ACCESSION: AK024527
- VERSION: AK024527.1 GI:10436829
- KEYWORDS: FLI_CDNA; oligo capping; fis (full insert sequence).

1 cagatatggt gatatcctta cttctggaat tcaaatcacc agttaggggt accttctcaa
61 ataaataaac ataatagcac tttacgtgtg aataaactca gagaggccct gacaaaccag
121 ggttgggctt tgcttggggg taccaacagg catctacttt ccatttctat tactgtcttg
181 atgtttctta gaattccatc ctggaatgct gaccctcaat gagaaggata aattaaaact
241 ttattaggct catttaaatg tcaattaaca aaggatgttc ttttttagat catttaaatt
301 tatttcataa gctagttgcc caaaagctgc tctgctgggc actcatacaa aattattttt
361 taaaaatata caaattaaat agcagaagag caagctcccc agaacagaaa gaaaacaagc
421 agtgctgatc accatctggg tctccttttg ggaggcagaa cccaagggga aaatagacca
481 catcccctcc tttggacgca atgccaatgc ctagggtttt gtcagccctg gagagagaga
541 gagagggaga gcgcaggtgc aaattcctcc tgtcaatgta agaattctgt gcttttgata
601 cctccaagag cctctctctg cagaaagtat ggaaaatatc gcactgttaa aataacaaat
661 gcagtcagaa caataagaaa tacaaaggtt catacctaag gtgtgaaata agaatttact
721 tgcaaactat aaggaaggag cacaacaaga cagcagatgc taaaagcacc aaagaagaga
781 ccaggtcatt tgagctgtaa tagtttaaag tttggagtga aaggcctatg gaaagaggta
841 ggactacact tgacctttgt agtgcgtgtt tgttgtttca gcctgcccag gatcccaccc
901 cttattctga agacagtacc taggttttct attgggacct accctactcc tacttttaat
961 ccaaatggct tagataaggt ggaccattcc catccttgga tctagagagt ggcatgtcat
1021 ccagccttgg ccagtcagag tcactgtggc ttgtgcaggg atgggcacat gagctaatca
1081 gagcagatga gagtcatccc cagacttttg tcagaactgc tgggaaagcc tatgcccttc
1141 cagtgggcat tgctaagctg gtacaatgca agcctgatgc tgtagtggac atccttccac
1201 catataaaga cagcctccct gggaaagcag agccatgaaa cggagaaaga cttcttacta
1261 aatccttaga gcactagatc cagcattttc catctgctat cgctgcatga caaaccacct
1321 taaaatgctt tggcttggcc aggtgcagtg gctcacgcct gtaatcccag cactttgtga
1381 ggcagaggtg agtggatcac ctgaggtcag gagttcgaga ccagcctgac caacatggtg
1441 aaaccccatc tctactaaaa atacaaaaat tagctgggag tggtaatggg cacctgtaat
1501 cccagctact caggaggcta aggcaggaga attgcttgaa cccgggaggc ggaggttgca
1561 gtgagccaag atcacgccac tgtactctag cctggatgac agagcgagac tccatctcaa
1621 aaaaaaaaaa aaaaaa//

- LOCUS: BF223370 544 bp mRNA linear EST 30-MAR-2001
- DEFINITION: 7q87e03.x1 NCI_CGAP_Lu24 Homo sapiens cDNA clone IMAGE:3705316 3',mRNA sequence.
- ACCESSION: BF223370
- VERSION: BF223370.1 GI:11130547
- KEYWORDS: EST.

1 tttacaatta taaagtttta tttttgaaga aaattatcat gtgactggca aattcaagaa
61 aaaaacatta gagctgaaaa aaagttattt taatccatca acctgttcca agaaaagccg
121 ctacacatca tcctggctaa atctgtggta accctgtttt agagacttct tgaaggctac
181 tagggggcaa agaggcatgg agattcagtc tcatggcttt tattatgcat tccaaataaa
241 tagctgtttt ctgaatgcag ttgcccaaat gcctaaaatt ttctgactgg actgattctc
301 actcctgata gatttctaaa taccttatag aagtgtcttc aggaaaagac gccaagcaac
361 cctttgaaat tcctcaaagc tgcacactca tcacctggga attattcaca acctctcctg
421 cgattgccct gtgggccaca acctctgcct gctgtccctg tcctcgcgac cagctctccc
481 tcagggcccc cctaaagtta ctgccccaaa gtccagacta ctccttgggc ctccagttaa

541 tcct//

- LOCUS: NM_004939 2706 bp mRNA linear PRI 17-NOV-2006
- DEFINITION: Homo sapiens DEAD (Asp-Glu-Ala-Asp) box polypeptide 1 (DDX1), mRNA.
- ACCESSION: NM_004939
- VERSION: NM_004939.1 GI:4826685

1 ctctgtagct gtgaccctga taccgcgtgg tgtgctccga acacatggtg cccagaacga
61 aggcggcgtc cagaagccct aggtcccaga ggtccgctca gcggcaggcg cataaggcgg
121 ggccggcgcg ggcctttcct tccatcggaa ccgttctccc ggggctgagt ccctgcccgg
181 actccgaacg ccgaagacca ggggccggaa gcgcgcgccg ccactgccac gccgtgtcag
241 tcgggaggga gggagcgagc aggcgaagcc gcggaggacg gggtgaagat ggcggccttc
301 tccgagatgg gtgtaatgcc tgagattgca caagctgtgg aagagatgga ttggctcctc
361 ccaactgata tccaggctga atctatccca ttgatcttag gaggaggtga tgtacttatg
421 gctgcagaaa caggaagtgg caaaactggt gcttttagta ttccagttat ccagatagtt
481 tatgaaactc tgaaagacca acaggaaggc aaaaaaggaa aaacaacaat taaaactggt
541 gcttcagtgc tgaacaaatg gcagatgaac ccatatgaca gaggatctgc ttttgcaatt
601 gggtcagatg gtctttgttg tcaaagcaga gaagtaaagg aatggcatgg gtgtagagct
661 actaaaggat taatgaaagg gaaacactac tatgaagtat cctgtcatga ccaaggttta
721 tgcagggtcg ggtggtctac catgcaggcc tctttggacc taggtactga caagtttgga
781 tttggctttg gtggaacagg aaagaaatcc cataacaaac aatttgataa ttatggagag
841 gaattcacta tgcatgatac cattggatgt tacctggata tagataaggg acatgtcaag
901 ttctccaaaa atggaaaaga tcttggtctg gcatttgaaa taccaccaca tatgaaaaac
961 caagccctct ttcctgcctg tgttttgaag aatgctgaac tgaaatttaa cttcggtgaa
1021 gaggaattta agtttccacc aaaagatggc tttgttgctc tttccaaggc accggatggt
1081 tacattgtca aatcacagca ctcaggtaat gcacaggtga cacaaacaaa gtttctcccc
1141 aatgctccga aagctctcat tgttgaacct tcccgggagt tagctgaaca aactttgaac
1201 aacatcaagc agtttaagaa atacattgat aatcctaaat taaggagctt tctgataatt
1261 ggaggtgttg cagcccggga tcagctctct gttttggaaa atggagtaga tatagttgta
1321 ggtactccgg gaagactaga tgacttggtg tcaactggaa agctgaactt atctcaagtt
1381 agattcctgg tcctggatga agctgatggg cttctttctc aaggttattc tgattttata
1441 aataggatgc acaatcagat tcctcaggtt acctctgatg gaaaaagact tcaggtgatt
1501 gtttgctctg ccactttgca ttctttcgat gtaaagaaac tgtccgagaa gataatgcat
1561 tttcctacat gggttgactt aaaaggagaa gactctgttc cagatactgt acaccatgtt
1621 gttgtcccag taaatcccaa aactgacaga ctctgggaaa ggcttggaaa gagccacatt
1681 agaactgatg atgtacatgc aaaagataac acaagacctg gtgctaatag tccagagatg
1741 tggtctgaag ctattaaaat cctgaaaggg gagtatgctg tccgggcaat caaggaacat
1801 aagatggatc aagcaattat cttctgtaga accaaaattg actgtgataa cttggagcag
1861 tactttatac aacaaggagg aggacctgat aaaaaaggac accagttctc atgtgtttgt
1921 cttcatggtg acagaaagcc tcatgagaga aagcaaaact tggaaagatt taagaaagga
1981 gatgtaagat tcttgatttg cacagatgta gctgctagag gaattgatat ccacggtgtt
2041 ccttatgtta taaatgtcac tctgcccgat gaaaagcaaa actacgtaca tcgaattggc
2101 agagtaggaa gagctgaaag gatgggtctg gcaatttccc tggtggcaac agaaaaagaa
2161 aaggtttggt accatgtatg tagcagccgt ggaaaagggt gttataacac aagactcaag
2221 gaagatggag gctgtaccat atggtacaac gagatgcagt tactatctga gatagaagaa
2281 cacctgaact gtaccatttc tcaggttgag ccggatataa aggtaccagt ggatgaattt
2341 gatgggaaag ttacctacgg tcagaaaagg gctgctggtg gtggaagcta taaaggccat
2401 gtggatattt tggcacctac tgttcaagag ttggctgccc ttgaaaagga ggcgcagaca
2461 tctttcctgc atcttggcta ccttcctaac cagctgttca gaaccttctg atttttacat
2521 ttactgaata agatttgagt aatgaaagtc tgtagtctta aaactctaaa acagttgtac
2581 tgcttccaag cagcagtatt tatagtaacg taagctatta atgctaactc ttgcatgtca
2641 agaaacatta gtcttaggaa ttcttcaaaa aatggcatcc caatgaaaat aaatttgatg
2701 actata//

- LOCUS: AW014299 391 bp mRNA linear EST 10-SEP-1999
- DEFINITION: UI-H-BI0p-aax-a-07-0-UI.s1 NCI_CGAP_Sub2 Homo sapiens cDNA clone IMAGE:2710573 3', mRNA sequence.
- ACCESSION: AW014299
- VERSION: AW014299.1 GI:5863056
- KEYWORDS: EST.

1 tttttttttt tttttttagg gtgttttgca gattttttat agcttttgtt tttgtttttt
61 tttagaaagt ttctctgtca tcaggcaaag accagggtgg gaacaggaaa actagaaaac
121 tggtcaaggg agaacaagtc ccttttcctc tcaccagtga acagcaggtg aggaaacaga

181 ctctgggagg aacgctgttc aggatggctt gctgcctgca tggcatgagg ctgctgtctt
241 gtgggtgctg gcagccacaa tctccatttt tctgaagacc accatttatg gctagatcca
301 gtagaggtcc agaactttcc caacactgct ggagaggaan tgtttcacag tcagaggaga
361 gagccatggg gaatggtaac acctcgtgcc g//

- LOCUS: AW025284 454 bp mRNA linear EST 09-MAR-2000
- DEFINITION: wu95h10.x1 NCI_CGAP_Kid3 Homo sapiens cDNA clone IMAGE:2527843 3', mRNA sequence.
- ACCESSION: AW025284
- VERSION: AW025284.1 GI:5878814
- KEYWORDS: EST.

1 gttttgctga ttttttatag cttttgtttt tgtttttttt tagaaagttt ctctgtcatc
61 aggcaaagac cagggtggga acaggaaaac tagaaaactg gtcaagggag aacaagtccc
121 ttttcctctc accagtgaac agcaggtgag gaaacagact ctgggaggaa cgctgttcag
181 gatggcttgc tgcctgcatg gcatgaggct gctgtcttgt gggtgctggc agccacaatc
241 tccatttttc tgaagaccac catttatggc tagatccagt agaggtccag aactttccca
301 acactgctgg agaggaagtg tttcacagtc agaggagaga gccatgggga atggaagcag
361 gggaagaagg ggtggggagg gganganana tnantggctc tatgctaatc ttagggttcc
421 ttctctgccc ttttgggtac cagagacaga ccct//

- LOCUS: AB023170 5161 bp mRNA linear PRI 16-JUN-1999
- DEFINITION: Homo sapiens mRNA for KIAA0953 protein, partial cds.
- ACCESSION: AB023170
- VERSION: AB023170.1 GI:4589549

1 gctggcggcg cccggctccg tcctgcccgc ggccggcccc cgcgtctgct ccctccccgc
61 ccgggcccct gtcggccgcc gccagtcccc gccccgactg tgaatgaaag gcgggcgccg
121 ccgagggctg gctgggaacg ccgcagcgac gccggcctct cgagaggcgc gcgccccgcc
181 gagatgtacg gtgtgtgtgg ctgctgtggt gccctacgcc ccaggtacaa aaggctggtt
241 gacaacatct tccctgagga tcccgaggat ggtctggtga agaccaacat ggagaagctg
301 accttctatg ccctctcagc tccagaaaaa cttgatcgta ttggcgccta cctctctgag
361 aggctcatcc gtgacgtggg tcgccatcga tatgggtacg tgtgcattgc tatggaggct
421 ttggaccagc tgctcatggc ctgccactgc cagagcatca acctcttcgt ggagagcttc
481 ctcaagatgg tggccaagct gctggagtca gagaaaccca acctgcagat cctcggcacc
541 aactcgtttg tgaagtttgc caacatcgag gaggacaccc cgtcctatca ccggagctat
601 gacttctttg tgtcccgatt cagtgaaatg tgccactcga gccatgatga cttagaaatc
661 aagaccaaaa ttcgaatgtc aggcatcaaa ggcctgcaag gggtggtgag gaagacggtg
721 aatgatgaac tgcaggccaa tatctgggac ccacagcaca tggataagat cgttccatca
781 ctgcttttca atctacagca tgtagaggag gcagagagcc ggtctccctc acccctccaa
841 gcacctgaga aggagaaaga gagccccgcg gagctggctg agaggtgtct tcgggagctg
901 ctgggccggg ctgcctttgg caacatcaaa aacgccatca agcctgttct catccatctg
961 gataaccatt ctctttggga acccaaggtg tttgccatcc gttgctttaa aatcatcatg
1021 tactcaattc agccgcagca ctcacacctg gtcatccagc agctcctggg ccacctggac
1081 gccaacagcc gcagcgctgc gacggtgcgc gcgggcatcg tggaagtctt gtcggaagcc
1141 gcggtcatcg ctgccaccgg ctctgtgggg cccacagtac tggagatgtt caacacgctg
1201 ctgaggcagc tgcggctcag catcgactac gcgctgaccg ggagctacga cggggcggtc
1261 agcctcggca ccaagatcat caaggagcac gaggagcgca tgttccagga ggccgtcatc
1321 aagaccgtgg gctcctttgc cagcacgctg cccacctacc agcgctccga ggtgatcctc
1381 ttcatcatga gcaaggtccc gcggccatcc ctgcaccagg cggtggacac aggcaggacg
1441 ggggagaata ggaaccgtct gacccagatt atgctgctaa aatccctcct gcaggtatcc
1501 acaggtttcc agtgcaacaa catgatgtca gccctgccta gcaacttcct ggaccgcctt
1561 ctctccaccg ccctcatgga ggatgcagaa attcgactct ttgttctaga gattctcatc
1621 agtttcattg atcgtcatgg caaccgccac aagttctcta ccatcagtac cctcagtgac
1681 atctctgtcc tgaagctgaa agtggacaag tgctctcgac aggacaccgt cttcatgaag
1741 aagcactccc agcagctcta cagacacatc tacctgagct gcaaggagga aacaaacgtg
1801 cagaaacact acgaggcgct ctatggcttg ctggccctca tcagcatcga gctggctaac
1861 gaggaggtgg tggtggacct catccgtctg gtgctggctg ttcaggacgt ggcccaagtc
1921 aatgaggaga acttgcctgt ctacaaccgc tgtgccctct atgctctggg cgcagcctac
1981 ctgaacctca tcagtcagct cacaacagtg cctgccttct gccagcacat ccatgaggtg
2041 atagagacca ggaagaaaga ggctccatac atgctccccg aggatgtgtt tgtggagagg
2101 cccaggctgt ctcagaatct tgatggggtg gtcattgagc tcctcttccg ccagagcaag
2161 atcagtgaag tcctgggagg cagtggctac aactcggacc ggctctgcct gccctacatt
2221 cctcagctga cagatgagga tcgtttatcc aagaggagga gcattggaga gaccatctcc
2281 ctgcaggtgg aggtagaatc gaggaacagt ccggagaagg aggaggtgag tgtccgtgcc

2341 accgtcctgg ggcagcccca cctcctgtag attggacgct ggactccagg ctcccctgcc
2401 tgcatagtga gcttaaggct gcatggaaag caagcttctc gagtaattcc acacttgtac
2461 tgactctccc cacgtatgcg tcatagatac ttcccttcgg aaaaattgtt agggtcctcc
2521 ctgctcccat gaggatatca taaaactcac tcttgaaagg cctggctggg gatggtggct
2581 cacacctgtg atcccaacat tttggaggct gaggcaggag gactgcttga gcccaggagt
2641 ttgagatgat ctggggcaat ataaggagac cccatctcta caaaaaataa ttgaaaaatc
2701 agccaggcat ggtgacgtgc acctgtggtc ccaggtactc aggatgctga ggtgggagaa
2761 tcacctgagc ctgggaggtc aaggctgctg taagccatga tggcagcacg gcacttcagc
2821 ctaggtgaca gagtgagacc tcgtctcaaa aaaattaaaa agaaaggcat gctggtcatc
2881 attccccact cacaggggga ccgatatgct ctgggttgga aactagaaga cttaactgga
2941 gagataaggg tgagaacagc tttcatagtg ttgcttccgt atgtatcaat aaagaatctt
3001 tttttaaatt tacactattt ttaaagcaat tttagttaca gcaaaattga gaggaaggta
3061 cagagatcgc cctttgcccc ctcccctccc ctgacacatg cacagcctcc cctcctatcc
3121 atatccccca gcagagtggt gcatttctta cagttcaacc tgccttaaca caacagtatc
3181 atctgaagtc tgtcgtttac actggggttc gccctcagcg ttgtacattc tgtgggtttg
3241 gacaaatgcg taatgacatg catcccccac cagagtacca tacagagtca tttcctgccc
3301 taaaaccccc tgtgctccac ctcttcatct ctcccctaac cactgacagc caagatgttt
3361 tttttttttt tttttaatgc tgactgttac agttcacaag aagtatggag gacacacatc
3421 tgggtgggac cgggacagaa agatttacat ggtcctcagg catggacagg ctggcattcc
3481 ccagcctctc tgcccattag ggctgatata gacatggagg acagctttga catcaagcct
3541 ttccccagat gcaatcagag gtaggagaag tctctagggg tcaggatcaa gcctccccct
3601 aactgtgccc tcgaccactt catttattgg ggcagatctc taccccaggg ccctcaaagc
3661 ttatccagaa ccttcccacg gaaggaagct ctgacttggg ctaattggac aagtaaaatg
3721 aggttgaacg aggcatcctc catggccctt ccagctccaa ggactgggaa cctccaatgc
3781 gttaggcatc ggggtgagtt ggctgctctc tccccatagt ggcgattctc ccttcccaag
3841 aggtaagaga ccctcgggaa ttgaggagtg aagtgactca gaagcgcaag gcgaaatgct
3901 tacacagcta cagagccacc tcctcactag tggcctcctg ggccaccagt atcagttcct
3961 tcctcccttc aagagtcagc ttagcccaga ggtagcttgg tggcaaagca ggggtgcagg
4021 ggccttcatc ctgtgccctt ctgccatgcc acaactctct ccactctcag ccatcagtgc
4081 cagtccagag tcccacggtg gttcctaggg ccgtactgcc cagccttgcc gcaggggtga
4141 ataaatgctt gtttggtcaa atctgaatcc tggtgcctgg cattcccagc catgcatagt
4201 ctgggctctg ccatttcatt tttccccacc tgctctggcc cgtatccact gtcccactct
4261 ggtggggctg ctctcgatct ctcccacgct gcgctccgcc atctccaggc ctttgctagt
4321 gcagaatgcc acatctgaac cacccttccc tccacactgg tgcagctgag cgcctgtgaa
4381 ctgtctgttc tccagcgccc ggcccctgtt cagtttcctc ataaacttct ctctccctgc
4441 tcttgcccag ggagcatccc ttctctcttg cccagagagc ggcattttct ttgactgttg
4501 ccaccacaca gttccctagc accctgcagg tgaatatcca atgttgctcg gtgctgacag
4561 tggatgaatg gatggacgta cagaggataa gtcctcagag cgagttaaag ccgagcatag
4621 ctctctcttg catgtaattc ctatgctagc aaattgtgca gtttctatag actcacctac
4681 agtcctgcat gtagttggtg ctcacttaat gtttgttaaa tgactttatc tcatacatat
4741 acttctgtcc tctcctactc aaacaaaaat gccaaacaaa tcaaaaaatg cacgactcag
4801 tgaattttca taaagtgtgt atgcccacgc aaccacgcct ggatgagaga gtggaacact
4861 gtcatcatcc aggggccctg cctctgtgcc ccctccccta agggagctcc cagcctgagt
4921 tgcgttactt ctggaacttt atatcaatga aatcattctg ttctgtgtct gctgcttttg
4981 cttaagatta tgtttaaatg atagagcatg aggctgggcg tggtggctca tgcctgtaat
5041 ctcagcactt tgggaggccg aggcaggtgg atcacctgag gttaggagtt caagaccagc
5101 ctgatcaaca tggtgaaacc ccgtctttac taaaactaca aaaaattagc cgggcatggt
5161 g//

- LOCUS: AK027146 2042 bp mRNA linear PRI 12-SEP-2006
- DEFINITION: Homo sapiens cDNA: FLJ23493 fis, clone LNG01831, highly similar to HSU66589 Human ribosomal protein L5 pseudogene mRNA.
- ACCESSION: AK027146
- VERSION: AK027146.1 GI:10440199
- KEYWORDS: FLI_CDNA; oligo capping; fis (full insert sequence)

1 tggaacaagc gcttcatctt gattttggaa ctgaattgga accaagaaaa gaaatagtgc
61 tatttgataa gccaactaga ggaactactg tacaaaaatt taaagaaatg gtctatagtc
121 tctttaaggc aaaattgggt gaccaaggaa acctctctga actggttaat ctcatcttga
181 cggtggctga tggagacaaa gatggccagg tttccttggg agaagcaaag tcggcatggg
241 cacttcttca actgaatgaa tttcttctca tggtgatact tcaagataaa gaacataccc
301 ccaaattaat gggattctgt ggtgacctct atgtgatgga aagtgttgaa tatacctctc
361 tttatggaat aagccttcct tgggtcattg aactttttat tccatctggg ttcagaagaa

421 gcatggatca gctgttcaca ccatcatggc caagaaaggc caaaatagcc ataggacttc
481 tagaatttgt ggaagatgtt ttccatggcc cctacggaaa tttcctcatg tgcgatacta
541 gtgccaaaaa cctaggatat aatgataagt atgatttgaa aatggtggat atgagaaaaa
601 ttgtgccaga gacaaacctg aaagaactta ttaaggatcg tcactgtgag tctgatttgg
661 actgtgtcta tggcacagat tgtagaacta gctgtgatca gagtacaatg aagtgtactt
721 cagaagtgat acaaccaaac ttggcaaaag cttgtcagtt actcaaagac tacctactgc
781 gtggtgctcc aagtgaaatt cgtgaagaat tagaaaagca gctttattct tgtattgctc
841 tcaaagtcac agcaaatcaa atggaaatgg aacattcttt gatactaaat aacctaaaaa
901 cattattgtg gaagaaaatt tcctacacta atgactctta gttcatttgg acataattac
961 cattttaaga aacctgccac ttttaaagaa caattttgag cattaaaaaa aaatggcttc
1021 aaattcctgc cagttacaca aaactccttc cccccaggcc tgagaagcca tcagtatgtg
1081 attactgaag taatggcagg tgtaggatca acaggtcccc aagatgtcat tcctgccctt
1141 ttagaagccc tgttacatct ccgaagtaca ttcattgtgt aactattttg actgacttta
1201 aaaaccaatg ctgtgaaaag cttcattcca taaacatcaa cagtgagtga tttgtagatt
1261 taccttagcc aaaataccaa tgctggaagc attgtgtttg cattgaagct gctgttcaac
1321 aagaaaattt ataaatttac taatgtctta gcatggtaaa gtttgcacat taacagaaat
1381 taagactgca aagcaggtta aacttgcttc tttataaaac agatgttggg ttaatagcat
1441 ggtttactgt attaaagact tatacaccca tttttaacct catttagaca tcaagttatg
1501 tgtagcttca caatggttca agtggcttac ttcaagaaat cttatacttg acagtacacc
1561 aattttattg actaaaaatg gatgaacttt cctaaagatt caaagggccc atcttagtat
1621 cacgcagctg actgagccct tcaaaactga catcttaagg cccaatcaag atccacatat
1681 cctgattttg aactatgtga aagtgggact gttaagtgca agactaaaat aaattatagc
1741 agacttttta gtaataactt tccattttca aacagtatat cctgtgggcc aaagggctat
1801 ttcttaaaga ggcatgtaaa tgtatttatt tatctaatgt ttttttcccc atgtaaactt
1861 gatatacaag gtttagtatt tgctcctctt tcatattatt ttcacacgta tactcagatt
1921 tggcatgtac ctttcaacat ctccataaaa ttaaacacct tttggaaaaa aaaaaaaaaa
1981 aaaaaaaaaa aaaaaaaaaa aaaaaaaaaa aaaaaaaaaa aaaaaaaaaa aaaaaaaaaa
2041 aa//

- LOCUS: NM_018253 2794 bp mRNA linear PRI 13-AUG-2006
- DEFINITION: Homo sapiens YY1 associated protein 1 (YY1AP1), transcript variant 1, mRNA.
- ACCESSION: NM_018253
- VERSION: NM_018253.2 GI:20986485

1 gagagaagag gaggtggaga aggcttgggc tcgcgccgct gaagtcggct tacccgctgg
61 ccgcctcctg acaagcggga gggatccgcg gtggacccag ggaagcggag gagcctggcg
121 gccaccccct cttcctcact tccctgtact ctcatcgctc tcggcctccg acacgaaaag
181 gaagcaaatg agctgatgga agatctgttt gaaactagac agggtcttgc catgttctgg
241 aactcatagg ctcaagtaat cttcctgcct caacctccca aagtgctgga attacagttc
301 caagatgaga tgggattctc caacatggaa gatgatggcc cagaagagga ggagcgtgtg
361 gctgagcctc aagctaactt taacacccct caagctctac ggtttgagga actactggcc
421 aacctactaa atgaacaaca tcagatagcg aaggaactat ttgaacagct gaagatgaag
481 aaaccttcag ccaaacagca gaaggaggta gagaaggtta aaccccagtg taaggaagtt
541 catcagaccc tgattctgga cccagcacaa aggaagagac tccagcagca gatgcagcag
601 catgttcagc tcttgacaca aatccacctt cttgccacct gcaaccccaa tctcaatccg
661 gaggccagta gcaccaggat atgtcttaaa gagctgggaa cctttgctca aagctccatc
721 gcccttcacc atcagtacaa ccccaagttt cagaccctgt tccaaccctg taacttgatg
781 ggagctatgc agctgattga agacttcagc acacatgtca gcattgactg cagccctcat
841 aaaactgtca agaagactgc caatgaattt ccctgtttgc caaagcaagt ggcttggatc
901 ctggccacaa gcaaggtttt catgtatcca gagttacttc cagtgtgttc cctgaaggca
961 aagaatcccc aggataagat cctcttcacc aaggctgagg acaatttgtt agctttagga
1021 ctgaagcatt ttgaagggac tgagtttctt aaccctctaa tcagcaagta ccttctaacc
1081 tgcaagactg cccgccaact gacagtgaga atcaagaacc tcaacatgaa cagagctcct
1141 gacaacatca ttaaatttta taagaagacc aaacagctgc cagtcctagg aaaatgctgt
1201 gaagagatcc agccacatca gtggaagcca cctatagaga gagaagaaca ccggctccca
1261 ttctggttaa aggccagtct gccatccatc caggaagaac tgcggcacat ggctgatggt
1321 gctagagagg taggaaatat gactggaacc actgagatca actcagatca aggcctagaa
1381 aaagacaact cagagttggg gagtgaaact cggtacccac tgctattgcc taagggtgta
1441 gtcctgaaac tgaagccagt tgccgaccgt ttccccaaga aggcttggag acagaagcgt
1501 tcatcagtcc tgaaacccct ccttatccaa cccagcccct ctctccagcc cagcttcaac
1561 cctgggaaaa caccagccca atcaactcat tcagaagccc ctccgagcaa aatggtgctc
1621 cggattcctc acccaataca gccagccact gttttacaga cagttccagg tgtccctcca
1681 ctgggggtca gtggaggtga gagttttgag tctcctgcag cactgcctgc tatgccccct

1741 gaggccagga caagcttccc tctgtctgag tcccagactt tgctctcttc tgcccctgtg
1801 cccaaggtaa tgatgccctc ccctgcctct tccatgtttc gaaagccata tgtgagacgg
1861 agaccctcaa aaagaagggg agccagggcc tttcgctgta tcaaacctgc ccctgttatc
1921 caccctgcat ctgttatctt cactgttcct gctaccactg tgaagattgt gagccttggc
1981 ggtggctgta acatgatcca gcctgtcaat gcggctgtgg cccagagtcc ccagactatt
2041 cccatcgcca ccctcttggt taaccctact tccttcccct gtccattgaa ccagcccctt
2101 gtggcctcct ctgtctcacc cttaattgtt tctggcaatt ctgtgaatct tcctatacca
2161 tccacccctg aagataaggc ccacatgaat gtggacattg cttgtgctgt ggctgatggg
2221 gaaaatgcct ttcagggcct agaacccaaa ttagagcccc aggaactatc tcctctctct
2281 gctactgttt tccccaaagt ggaacatagc ccagggcctc caccagtcga taaacagtgc
2341 caagaaggat tgtcagagaa cagtgcctat cgctggaccg ttgtgaaaac agaggaggga
2401 aggcaagctc tggagccgct ccctcagggc atccaggagt ctctaaacaa ctcttcccct
2461 ggggatttag aggaagttgt caagatggaa cctgaagatg ctacagagga aatcagtgga
2521 tttctttgag ctaggagaat aagagtctgg agactgggag ccttcacttc ggcctccgat
2581 tggtggcgca tagggtgtaa ccaataggaa acccctaaag ggtacttaaa ccccagattt
2641 tgcaactggg gctcttgagc agcttgcttt agcctgctcc cactctgtgg aatatacttt
2701 tgcttcaata aatctgtgct tttattgcta aaaaaaaaaa aaaaaaaaaa aaaaaaaaaa
2761 aaaaaaaaaa aaaaaaaaaa aaaaaaaaaa aaaa//

- LOCUS: AI431902 566 bp mRNA linear EST 30-MAR-1999
- DEFINITION: ti26e07.x1 NCI_CGAP_Kid11 Homo sapiens cDNA clone IMAGE:2131620 3'similar to TR:O23127 023127 F19G10.9 PROTEIN. ;, mRNA sequence.
- ACCESSION: AI431902
- VERSION: AI431902.1 GI:4306432
- KEYWORDS: EST.

1 tttttttttt gagccaagaa gtgcagtagg ctgcggggat tttatttctg cccccagacc
61 aaggcaacgg agccctcacc tgcctgccac ccccacactg ggcccaagca agctcaggtg
121 agcgcacata catccaccgt ggcgggctcc tctcgggtga agccatcacc gaagccctca
181 tcgtccacca ggtcgggctc acggcagcac atgggacaga ggctgttgcg cacagcagag
241 gctcggagcc agacgacaag gttccaacgc tcaccagtgc ccaagggccg ggctccatgc
301 agctggccgc cacggtggag gacaccctgg cccaccacgt gctccacctc caggggctcc
361 gtcagggctg tgggtgcctg gaagaggccc ccaaaataca gggcgccccc tgtgaagacc
421 ttgcccaagg ccacattgag ggtgagctcg gcattatcat agtggcagcc cagctccagg
481 tcctggcccg gtgcgtattt gaccacaaag ggccggtggc tgtcgagccg gcccccgnca
541 cagtcagggt acagcaaggc catcag//

- LOCUS: AK026465 2622 bp mRNA linear PRI 12-SEP-2006
- DEFINITION: Homo sapiens cDNA: FLJ22812 fis, clone KAIA2955.
- ACCESSION: AK026465
- VERSION: AK026465.1- GI:10439335
- KEYWORDS: FLI_CDNA; oligo capping; fis (full insert sequence).

1 tcttcgattc ttcgagcctt ttggcctact gaggtggtcc aagtactcca agatgttggg
61 tgagagaagc tcatgccagg taaagtcact aactaggtga gacagccagg aagagtttgg
121 gattcacaac ggcagcacct acgacagaga tactgccctt catcaaacca ctggggcaag
181 atgattcatt gaaatgttac cctgtgctca tttggtttac atttgctcct cccctggctc
241 tttcaagcag agagtgagtc ccttggtgtt tccttgcctt gggtatttct ggtggtcaga
301 gcataaaagc tatcggaagt tggtgggtct ttctgtcttt tgcaataatc ttgacctaaa
361 ttttttttta aatttcttaa ttatttttag agacagggtc tcactctgcc acccaggctg
421 gagtgcaatg gtgtgatcat agctcactga agcctccaac tcctgagctc aggtgatcct
481 cccgcctaag cctccagagt agctgagact acaagtgctc accaccatgc ccggctaatt
541 tttctttttc gtagagacag agatctcgct atgttgccca ggcttgtctc gaactcctgg
601 cctcaggtga tcttcccttc tcagcctctc aaagtattag gattataggt gtgagccact
661 gcacccagcc ttcgttgact ctttatagag actctgtaag gtgtcctgga aagagaagaa
721 agaaagggtg ggcctatgat tttttgtttt ttgagataga gtcttgctcc gtcgcccagg
781 ctggagtatg gtgacatgat cagggctcac tgcaacctcc atctcctggg ctcgggtgat
841 ccttccacct cagcctctgg agtagctgga actataggtg catgccacca tgcctggcca
901 atgttttgta tttttagtag agatggggtt tcaccatgtt gcccaggctg gtctcaaact
961 cctagtgctg ggattacaga tgtgagtcac tgcaccaggc caagggtggg cctttgagag
1021 caggaagaag ggagaggaaa agatttttac cccagaaggg aaaggggcag agtgtgacta
1081 gggctggggg tagggaggaa aggtctcaaa ccttgactct aggcagtgtc caaaggtgac
1141 aaggccttag aggacatggc tgcctggtac actcagggag agtggatctg agcacagagc

1201 tttcgcccct ccctcacaat tcccacattc caagggtgac tccaaatcag cagagccacc
1261 aagcctgagg tgccacaagg ctgggatgat ggatgttctg catttgctag tgtgaccagt
1321 ggagccagcc tgacgcctca gcactgccta agaccctggg accttggcaa aatcttgaga
1381 atgggggagg gagactcaac gatatgcctg agattgaatt tcttgccagc tcagtgggat
1441 agtggcttag agtcagattt aaattgaatt agaaaaaatc aagaaacagg ccaaaccact
1501 ccagccttgc aatccctcgg gctatcccta gccacacccc ttactctcat gctcaccaac
1561 aagcagtgcc taccaccact ggatgcccca tgtgacccat agctgaggat ctttatacac
1621 cctctcccca ggagtgggca tccacttgtg tttccctgga cagggagctt ccaatcttcc
1681 atcctgaggt cctgctagaa tgggctgtta gaatattccc tcactagata gaggggtggg
1741 gaagcaggag tccagtttca gaagcaggga agggactaca gtcatagcga gtggtggaca
1801 cgtggcggag ggtgggacac cctttcacaa agtggcccat cataccacaa cacagtatgg
1861 gacagggctg gcttctcttc agctctggca tggccgggga gggtacctac ctggggtcca
1921 gctgcccccc cgagcctgtc tatcagccat tgtacaccag gccagtccat gatggggttc
1981 acccaggatg agacctggac agcagttgag ccatgcagaa gcaagtgggt ggttaagagg
2041 cagatggtcc agttggtgtt ttcaggcaaa gtgccagctc ctcatcatgg ctcatgagga
2101 tgcttagatc cagcccctga tgacccccta tttcacctct agccatttcc tagcacagcg
2161 cctcctctgt cctagctcca gcctcactga gctgctttca gtcccttgaa ggagttgcac
2221 tctccttcct ctcggctctt ccacaggctg ctccagacgc ttgaccactc ctttctttca
2281 tgtacctaat tcctactcat ccctcaggac ccaatgtggc tgtcttctct aggaagccat
2341 ctgaacttat aagttgagtt aggagtctgt tctggacctc ccacagcacc ctgattctcc
2401 tgtcttggcc cctgtcatac cttgtgaagt cttgtttaat catctatctc atacatgtca
2461 gtatgcttct gggggaagga aatgtgtcca tcttgtttaa aacagtatct ttagggcttg
2521 atgtctagtg agggaggaag ggaaggggaa ggaaacgata agggcatggg aaggaaggat
2581 ggaaggaagg agggaaggaa gaagaaaaaa aaaaaaaaaa aa//

- LOCUS: NM_017700 1453 bp mRNA linear PRI 17-NOV-2006
- DEFINITION: Homo sapiens hypothetical protein FLJ20184 (FLJ20184), mRNA.
- ACCESSION: NM_017700
- VERSION: NM_017700.1 GI:8923172

1 agtcacagcc aggtaaccct ggagtgaagc ggtttagtta gaagggagca gataaactcg
61 tcactctagt agctttaacc ctcaccctga ggcaccttag caatcagcca ttgcctgcaa
121 gcctccaaag cttgtctttg cctaatatgg agcccaaaga agccactggg aaagaaaaca
181 tggtcaccaa gaaaaagaat ctggccttct tgaggtctag actctatatg ctggagagaa
241 ggaagactga cactgtggtt gagagcagtg tttctgggga ccactctggc accttgagga
301 ggagccaatc tgacaggacc gaatacaacc agaaattaca agaaaagatg actccacagg
361 gtgagtgttc tgtagctgag accttaaccc cagaggaaga gcatcatatg aagaggatga
421 tggcaaagcg ggaaaagatc attaaggagc tgatacagac agaaaaggat tatctcaatg
481 atctagagct gtgtgttagg gaagtggttc agcccctgag aaataaaaag actgataggc
541 tggatgtgga tagcttgttt agcaacattg agtccgtgca tcagatatca gccaagctgc
601 tgtcattgtt ggaagaggcc acaacagacg tggaaccggc catgcaagta attggagaag
661 tattcttgca gattaaaggg ccactggaag atatttataa aatctactgc tatcaccatg
721 atgaagcaca tagtatactg gagtcctatg aaaaggaaga agagctgaag gaacatttga
781 gccactgtat ccagtcctta aagtaaggcc ttttcaaatg atgattccca tctcctctca
841 gttgcctagc agggaacatt ttaaatggat gtagatgaaa ggtctcacat aaatcctatg
901 ttttatgaga cttgctggga gctctgcttt gcattccctt tataaaaagc tgacatgcca
961 gaagccctga ttgacttttt ttccccctgc gagaatgact aaaaataaca tggaagaaga
1021 tttagagctc tgcagcgatt gaaaaatgca atatcaaaat ataaaatgtg gaagaaaagc
1081 ctcttcttaa agctattgta acttgcctgg ccccacgtag ttcaaggatt atgtgagata
1141 acacgtggcc ccatgaccac tggagcacat gggttaatgg agttagggga atggcctaca
1201 actctgcatg gccgtcttct ttccccaaac tcactgtggg gagatgggtg aagacaagtc
1261 aggccttgtt aaagttagtt tcagaacaat tactcatgcc ttcctttctc atccctaaaa
1321 cattggtggg ggagctacac aatgtacttt ttcttttcta gaggaagtat ctattcactg
1381 tgaaaatctg aaaaatataa caaagtatgt gtaagataaa aaccccttgc tatttcaaaa
1441 aaaaaaaaaa aaa//

- LOCUS: NM_024035 1936 bp mRNA linear PRI 24-SEP-2005
- DEFINITION: Homo sapiens chromosome 8 open reading frame 51 (C8orf51),mRNA.
- ACCESSION: NM_024035
- VERSION: NM_024035.1 GI:13128985

1 ctgagagtca gggcggacct ggactcggcg cgcctagctc ctcggctccc tttgcgggga
61 ctccaggagt ctgcccgctc gcgccgtttc gtcctgtcac cccggcaacg ggaccctcta

121 agaggcttgt tccaaaaatg ttcacaatgg ggtttttaaa aacggttttg ttagggacaa
181 gtgcttgtgt gagtcctccg acaatgcctg ctttcttctc tctgcccgcg gagcggaggc
241 tccaggcctg gccccagtcc gaggcgcctc tgtcggtgtc gtcctgcttc caaaaccggc
301 ctccagaacc cgcctccttc cagaacctcc gtccagaacc cgcctccctc cagaacctcc
361 gtacagaacc cacatccttc tagcacctgc ctctggaacc cacgtgcttt cagaactacc
421 tgtttgtaga acttgtttcc agaacgaacc tgctttccga atctctttac ttccagagct
481 tgtttcctgg actgacctat tttcagaacc tactttcaaa accagcttgc ctccaggacc
541 acctgcttgg agaactggcc tgcagcccca ggactggacg acctgagact gcacagctcc
601 ctgcggggct cacccggctg tgtgtgtcct tccgggcctt ccatacttcc ctaggtgtct
661 ggtgactccg gagcccgctc ggatttcgtg tgggtctcgc ctcagctcag aacacaccct
721 ttgaagagca ggaaggccag ggcacaggcg gtaaccacgg ctcctggtca tcaagttcct
781 ctgaagccca ggtcttgtcc acgttcactg ccccaccaca ggcccagcgg agccttggag
841 ctgagccctg ggatcattct ggtgcctgtg ctcccggctc cgcaacaagc acacaggagc
901 ctgtcatccc cagatctaaa tcctgaaggc taagctccgt ggtctgccgc agccatctgc
961 agcaggcccc tcccagcaac agccttctca taaggcccct gccagccacg tgaaacctcc
1021 tggctagggc ctgtcttcca ctcctggcct tttgagacag ggcctcactc tgttccccag
1081 gccggagtgc agtggtacga tcacagctca ctgcagcctc cacctcctgg gctcatgtga
1141 tcctcccacc tcagcctcct gaatagctgg gactacaggc ttgcaccacc acacccggct
1201 aatttttgta tattttgtag agacagggtt tcgccacatt gcccaggccc aggctggtct
1261 caaacttctg ggctcaagca attcatctgc ctcaacctcc caaaagtgct ggattacagg
1321 gatgagccac catacccggc tttttttttt ctttctcctg gcctttgggg gcccttgaag
1381 tgtctgctcc ctgggagccc ttcagcttgt cgtccacttg gtgggttccc acccgtcctt
1441 ctcacttctg cctgaatcac acaggctaca tgagaccttc acccaccccc ttcgccagca
1501 gctctgtggt tcaagtttcc tcaagggcag gggctcctgg ccatcttacc tgtgggcctg
1561 agcatgggcc gatgcttcca aagttcacac tgggtgcact tgtgtgggga cttccatgtg
1621 agtcatctcc ttgcaggctg ccaaggtcct gtacagcaga gacccaaacc ctcttagcag
1681 tcttcagagt cagctatgtg tacaggaggc attaccatat cagtgactgt cagcaagcat
1741 gtgtgactga aatggtatcc agttcttacg tatttgtttt gcaggcttct tggttcagca
1801 gacagaggcc attgggaagt taagagaggc tctcaaactt tgagggtcat aagacttccc
1861 ctccctgcag ggtgctgttt cctgaaggaa aatattcaat tactattttc tttatttaaa
1921 aaaaaaaaaa aaaaaa //

- LOCUS: HSU72513 586 bp mRNA linear PRI 30-NOV-1998
- DEFINITION: Human RPL13-2 pseudogene mRNA, complete cds.
- ACCESSION: U72513
- VERSION: U72513.1 GI:1673516

1 gcatgatcct gaagcccaac ttccacaagg actggcagtg gtgtggccat gtggctcagc
61 cagcccatgg ggcagacctg cagaagcaag gtggccagca aaagcaggct gcatggccca
121 ctgatatggg cccatccacc catagtaagg tgccccatgc tgaggcatca ctacaaagca
181 caagctggca ggggcctcag cttggaggag ttaagggtgg ctggcattta caagaaggtg
241 gcccagacca ttggcatctc tgaggatgca aggaggagga accagtccac ccaggccctg
301 caggccaagg tgcagaggct gaaggaggac cgctcctcac tcatcctctt ccccaggaag
361 cccttggccc ccaagaaggg agacagttct gctgaagaac tcgaactgga tactcagctg
421 acaggaccag aaatgcccat cggcaatgtc tacaaggaga aagccagagt catcactgag
481 taggaggaaa acttccaggc cttgttagtc tctgtatggc ccatgcaaat gcccagctct
541 tcggcatata ggcacaaaga gccaaggaag ctgtagaaga ggacgc//

- LOCUS: AL031228 175737 bp DNA linear PRI 18-JAN-2007
- DEFINITION: Human DNA sequence from clone RP5-1033B10 on chromosome 6p21.2-21.31, complete sequence.
- ACCESSION: AL031228
- VERSION: AL031228.1 GI:3646023
- KEYWORDS: HTG; 2-RIIBP; ARE-1; ARE1; B3GALT4; beta3Gal-T4; beta3GALT4;
- BING4;: C6ORF11; COL11A2; collagen; CpG island; D6S115E; D6S2244E; D6S2245E; DFNA13; FABG; FABGL; GalT4; Gal_T2; H2-KE4; H2-KE6; HKE3;
HKE4; HKE5; HKE6; HLA_DPB1; HSD17B8; HTATSF1; KE-3; KE3; KE4; KE6;
NR2B2; PARP; RCoR-1; retinoid X receptor beta; ribosomal protein;
ring finger; RING1; RING2; RING5; RNF1; RPS18; RXRB; SAC2; SACM2L; STL3.

1 gatctgcccg cctcagcctc ccaaagtgct gggattatag gcgtgagcca ctgcgcctga
61 cctttttttt tttaaatctt ttgagagaga cgtagtcttg ctctgtctcc caggttggag
121 tgcagtggcg tgatctcggc tcactgcaac ctccgcctcc cagattcaag cgattctcct
181 gcctcagtct cccaagtagc tgggattaca ggcacctgcc atcatgccca gctaattttg
241 tatttttttg tagagacggg gttttactgt gttggccagg ctggtcttga actcctgacc
301 tcagatgatc tgcccgcctc ggcctcccaa agtgttggga ttacaggcgt gagccactgc
361 gcctggccca cacattttta ggttataaaa ttaaacgtaa tatggccagg tgcggtggct
421 cacgcctgta atcccagcac tttggaggcc aaggcgggtg gatcgcctga ggtcaggagt
481 ttgagaccag cctggccaac atggcgaaac tctgtctcta ctaaaaattc acaaaattag
541 ccgggtgtcg tggcgggggc ctgtaattcc ggcaacttgg gaggctgagg caggagaatt
601 gcttgaacct gggaggcagg ggttgcagtg agccaagact gtgccattgc actccaacct
661 gggcaacaag agcaaaactc cgtctcaaaa acaaacaaac aaacaaaaca taatatgaga
721 ctggacacag tggctcatgc ctgtaatctt aacagtttgg taggctgagg tgggcagatc
781 acttgagccc agaagttcga aacaagccat gtcacccatg acatggcaaa actctgtctc
841 tacagaagat agaaaaatta gccgggtgtg gtggtgcatg cctgtagtcc cagctactca
901 ggaggctgag gtgatcctcc cacctcagcc caggaggtta aggctgcagt gagctgtgat
961 catgccactg cactctagca tgggcaacag agtgagaccc ggtctcagaa aaaaaaaata
1021 ataataatca aatatatttg tgtaatacag atctactaat gggaagaact gaatttctct
1081 ttttgaggtt aacattttgc ctaattgatg tacaaagtta gtgttccaga tggtcaaatt
1141 tgactgtaga tattcatgtt catgctgatc tgtagagatt gcaagtattt catctttgaa
1201 aacatctttt cacacaggta atgataggtg atatgtgagg tgcttgaaat gctgtgaagc
1261 acttacgact gtgtcactgt gacttgtagt gtacaaagca gcagtgcaaa tcaggatgct
1321 gtagtcgctg tcgtgaccac tcggctgtgt gttgtagagc aaaagcagcc acagtattaa
1381 gtaaatagtg tggccccgtt ccaataaaac tttatttgtt ggatattgga atttgaattt
1441 catacggttt tcacagtctc aaaatattct tttgattttt tttcaaccac ttaaaaatgt
1501 aaaaaccatt cttggcttgt gggctataca gaactagatg atgggccagg tttggcccat
1561 gggcagtagt ttaccaagcc ttggtttaaa gccctcatat aagctgttgt agacattaag
1621 atgagttaag gcatatagtt tagcacagcg cttagcaaaa tagggagcgc tgtgttcatc
1681 attgtcattc aagatgatcg ttctctccag gtctggctga tgtgaggggt ggaggtggtg
1741 tctgctttgg atttctgctg ttcctgaggg agtatctgca ttttccacag cttttctgtc
1801 tgatttgtat tttcctctga ttccttttgt catcaggtat tcactgggca cctgctgtgg
1861 gcagggctct gcgctgaggt tctggagaca aaaggatgaa tcgttgagcc tgccctggtg
1921 tggtgctccc gttatccttt aggtataaaa acttgtggct attttttttt tttttttgag
1981 acagagtctt gatctgtcac ccaggctgga gtgcagtggc acaatctcag ctcaccacaa
2041 cctctgcccc ccggggttca agcgattctc ctgcctcagc ctcctgagta gctgggatta
2101 caggcgccca cgaaccacgc ccagttaatt ttttaatgtt tagtagagat ggggtttcac
2161 catcttggcc aggctgatct tgaactcctg acatcgtgat ccacctgctt cggcctccca
2221 aagtgcaagt gttgggatta caggcgtgag ccactgcacc cggccatggc tatggttttt
2281 gagaatgatt ggccaggtga tgcatttatt tattttatta ctatttttcg agacggagtc
2341 ttgatctatc acccaggctg gagtgcagtg gcgcgatctc ggttcattac aacctccgcc
2401 tcctaggctc aagtgattgt tctgcctcag cctccaagta gctgggagta caagtgcatg
2461 ccactgcatg cgctaatttt tgtattttta gtagagatgg ggttttgcca tgttggcttg
2521 gctggtctca aactcctgac ctcaggtgat ccacccacct cggcctccca aagtgctggg
2581 attacaggca tgagccacca cgcctagctc aggtgatgcc attagtttct acacagttat
2641 cctctgtcat cccagactca atgtgtccat cactggatag gtcaccgcct cctaaagttt
2701 ctcttgacct gctctcatct cccagaattt tcctgtcacc cagaatttga ctcaggtaca
2761 caccaccaca cctggataat ttttctattt tttgtagaga tggggtttca ccatgtagcc
2821 caggctggtc tctgtcttga actcctgggc tcaagcggtc ctcctacctc aacctcccaa
2881 agtactggga ttacagagta attactgtga gccaccacat ccagcttggc caccagctta
2941 ttcaaagtct ccaggatgct gggaccaccc cccccaccac ccgctccctg tttttgcata
3001 attgtatctt ttttttttga gacggagtct cactctgtca cccaggctgg agtgcaatgg
3061 tgtggtctcg gctcactgca acctccacct cctgggttca agcgattctc ctgcctcagc
3121 ctcctgagaa gctgggacta caggtgtgtg ccaccacacc cggctaattt ttgtatttgt
3181 agtacagatg gaatttcacc atgttggcca ggctggtctt gaactcctga ccttgtgatc
3241 cacccgcctg tcctcccaaa gtgctgggat tataggcgtg agccaccgtg tctggccttt
3301 gtaattgtat ctttgtgaat gagtgatttg gttctgccct ttttactcca tatttatacc
3361 agcctggctc taggagagtc agaaggcctg cccaagggtt tctgccctct ctgggccact
3421 gggccaaagc tgtagcttgc cctccgtggg ctacctgggt cagccactcc tgtgcttagg
3481 gcttaatcac tagttcgctg aggcctgaag tttaatcaac accttgaggc taaacagctc
3541 tggtccttgt gatcttcagc ccatccctgc ttttctctgg tcctcctcag gagcttgtca

3601 ggccacgggg gctccagtga tgaggctgac catctttctt aagaggtgtt ctggtagctt
3661 gtattatgat tggattgcgt tgacttctca aagccgaact gctgcttact agtagtacag
3721 tagactccca tttggcactg ggctggtctt tacccagggg tcccatagat gggtgtgtgg
3781 aggggagaag agccagcatg catccttgag ttgttagttc accaaaggat gtgaccctgt
3841 gttccacgag cgctcggtaa ttcctggttt gcactgatgg cctttctctt tggtaaagtc
3901 agggcgctga ttatcttgtc ggaagtatcc agctggtcct ctcttgggct ccatcactgt
3961 gcaagtctct gctcataaga actcttcagt tcccttctcc tgggttcaca gccaaggcaa
4021 aaaagagact ccttggcctc ttggcaaaca tacatcccct acctggctgg tcccccttca
4081 gcttccctct ctaaggaaca acttgaacat agaaactcca acctcacctg taatcccagc
4141 actctgggag gccaaggtgg gcaggtcatt tgaggccagg agttcaagac cagcgtggtc
4201 aacatggcga aaccctggct ctactaaaaa ttagcggtgg tggcgcatgc ctgtaatccc
4261 agctacttgg gaggctgagg cacgagaatc acttgaacca gggaggcaga ggctgcagtg
4321 agccagggtc atgccactgc actccagctt gggcaacaga gagaccctgt ctcaaaaaaa
4381 gaaaaggaac tccaacttct gaaagtaaac aacttatgca gtacatgatt cctggagagg
4441 atttggtcac caaccttccc tccttccttc cagggaaagc acacagaggg gcagggcaag
4501 ggcctccagt gcattacttc accacttccc tgagtaggtc ttctcaagtc cttggagcct
4561 tcactttgtg atttgtcact gccagagagc aatgttggga tcatgagctg tgggtcctca
4621 gtctgacttt aaagaaacct cacaaattgt ctgctcctga ttttcctgac catgtttccc
4681 accaatccta acaaacctct gcttccctct cagtctcaac actctggtgt taatgcattt
4741 gaacctgctg tgtatctact tcctgtaatt ttttgagaat tatctagaat ggctcaaatc
4801 ccacctttcc catgaagcct ttctgaccac tgcacatgct ccttcccctt gctgggctgt
4861 gtgctgcatg tgtgtacagg gggacatcac atttgggtgc agcctgtcgt gtgggcgttc
4921 cttttgacgc ccgtgtcttg agagggctgt gtggtgttag caggttagag gcagatgcta
4981 aagagacagg cctgaccatt cactgaccta gctttccttt ccctgagctt cagtttcctc
5041 acctcttaaa gggaaataaa aatagtatga gttgtgtgca gcttaactga gaggatgcct
5101 gtggagtgtt cagggtgatg gcagtggtta ttactattaa tctttttctc cccagccaga
5161 cttgatgcct ggacttggcc acagcttgct tggtaaaccc atttactgac atattaattg
5221 atccctcttc tctcaccagg tacatggcca cctctggcct agaccaccag ctgaagatct
5281 ttgacttgcg agggacgtac cagcctctga gcactcggac cctgccccat ggagcagggc
5341 acctggcctt ctcccagagg ggactgctgg tggcgggaat gggtgacgtt gtcaacatct
5401 gggcagggca gggcaaggcc agcccaccct cccttgaaca gccctacctc acccaccggc
5461 tctcaggccc tgtgcatggc cttcagttct gcccctttga agatgtgctg gggtggggc
5521 acactggggg catcaccagc atgctggtcc ctggtgagtg ggccagggga tggggcttga
5581 agtgaatgaa ctctgggtgg aagtttgggc cagagacatc caggaactgg gggcttagtt
5641 gggctggagc tgttggactg aggttcttcc ttatggctcc atgcttctcc ctccctccct
5701 tcaggggccg gtgagcccaa cttcgatggc ctggagagta atccatacag aagccggaag
5761 cagcgccagg agtgggaggt gaaggccctg ctagagaagg tgaggctccc ctgctggaga
5821 gggtgagatc ccccccattg ctggagaggg tcaggttccc ccgtcctgga gagggtgagg
5881 ttcctccttc ctggagaagg tcaggttccc ccctgctgga gaaggtcagg ttccccactg
5941 ctggagaagg tcaggttccc ccctgctgga gaaggtaagg tttcctcttc ctggagaggg
6001 tgagattccc ctctcctgga cggggtcagg tttccctctg ctggagaggg tgagattccc
6061 cctgctggag agggtcaggt ttccctctgc tggagagggt gaaattccct ctcctggaga
6121 gggtcaggtt cccccctgct ggagagggtg agattgcccc tgttggaggt gaggttcccc
6181 tgctgcagag agtgaggccc ctgctgctgg ggaaggtgtg gtttggggtt ggaggtagag
6241 aaagctctcc tgatgtctca tgctgggggt cttgggtaga ggggttcagc agcctccctg
6301 gcttctcttt ttggtgctac cctcctgtgg ctccttgctg agtcctgtcc ctctgtgaca
6361 cccccaggta cctgcagagc ttatttgtct ggacccacga gccctggccg aggtggatgt
6421 catctccctg gagcagggaa agaaggagca gatagagagg ctggtatgga gcaggcccct
6481 gaatgcccag gcccgtcttc tcccccacat cctctcccca ccatggcttg tccccagaag
6541 tgcggccagc aggtgggtgc tgccagttcc ctgactccga ggctgtaaca gatatcactc
6601 ctgtcccctc cctcagggct atgacccgca ggctaaggct cccttccagc caaagccaaa
6661 gcagaagggc cgcagctcca cggcaagcct ggtgaagagg aagaggaagg tcatggatga
6721 ggaacacagg gtaaatgagc attggcatgg gccgggcctt ccacaggctg caccctcctg
6781 cttgtgcctc tgcccttgtc agcctgccac ttctcactct gtgcctgtgt cctccccgtc
6841 tccggtcccc aggacaaggt ccggcagagc cttcagcagc agcatcataa ggaggcgaag
6901 gccaagccca cgggggcccg gccatctgcc ctggacagat ttgtgcgctg agccagactc
6961 cagggttgcc tgggaacagt ctctccccaa gatcacctgt agggaaatga gtgttccctg
7021 gaacaaggag gtgggggcag tgtggcccct tccccaactg ggggtggaca gctgtctcct
7081 ggggtgggtt ggtattaaag aggaaagcga ttttttggat aatgtgtctg gatcatcttg
7141 tggacctgtt cccctccccc ttgccaagca tgaggccatt ctaggatcgg aggggtcagt
7201 gtcctgcctc tacccccacc ccataccttg tggctgctgt ggccttagct ttgcctttta
7261 ggggccttcc tcctcagaga ggctgtcctc tctcccatgt ccctggagat gtccttccca
7321 tgcagcaggg tctggcagcc tctgggctct ccaggtttgt ggtgttctgg gggtcctggt

7381 gaagaccaca ctgcactcga agaagtggag acaagtttat tgaggagctt gacacccctc
7441 ttctgcccta gcttgagaga acaactgcag catttttttc tttttctctt cccgatgacc
7501 atcttttggg ctggcgggcc aggcccctgg gtgtctccca tatcgctgtc tttagtgaga
7561 ctgaggatct ggtataagga aacagatcag ctgcagccag tgagggcccc tgcgttgggc
7621 ctgggagaga gaaggtcctg ttcctgcctt tcctgtggcc ccaggcactc tcagctctga
7681 agccaggcta ttgctcgaca ccgcaggatg cccaggactc cctggaacca ggagcaaaag
7741 ggcgcagtcc tttccccgtc agagccaccc accagcttcc aggcttcctg catcttccag
7801 gggtccagcc tgtgggacgt cagcaggaat ttcccatagc agcaccggtc tagcgggtag
7861 tgggtggcac cagccagctt gacacactgt gttggggcga ggcctcctcg tcgggcactt
7921 acccccacaa agacatcctc taatgggaga aggggtgccc ggctggccac cttgagaatg
7981 agctgcacag cagacgctga cagcacatac cccgtgcctg aggcataggg tggaaagggg
8041 ccccaggtgt gaggccactg ctcctctgat acgcggtgcc tgccccccgg tgtccgagag
8101 gggttcacgc gccagtgcac ccggcccaag tacagaagag gcacttcctc gctgtgcaaa
8161 acctggcctc cttcctgctc agcctctctc tggggttccg tgcttctctc ccattgcccc
8221 caacggcccc ctcgcaagac cagctctgat accagttcag ggacgttgac atacacatca
8281 tcgtccgtct tgaggacgta tcgggccatg ggcagtgttt ctcagcccca gttcagcccg
8341 ctgagggtct ttagggtgag gttgcggtag gagtcctgga aggcggcctg caagatatcc
8401 ccctgggctg ctgactccga ggccaggtca ctcccctggg aaccccacac ggggtgctgt
8461 gcgttcggct ctcccagcaa gaatagcgtc tgtaccctga gcccccgggc ctcgcgcagc
8521 ccgccccacg aagcccgaat ggcgtttctc tggttcaggt tctccggagc cgtgcacacc
8581 aggatgagca ggaagggagg ggccccggga ccactgcaag cttcctggtt ggggatcaag
8641 aggcggggca gggccagggg cggccccggt gaggcggggg ctgggagcag ggaggctagt
8701 gagaggctca gcagctcctc ccccaacccc gaaggcccga agagggtcca gacgatcacc
8761 agcagcaaag cggcgagaag gaggcgccgg aagagcctga gctgcatggt gcggcgtact
8821 ccgaggggat ccgtaagagc gaagggcgga gactagctcc ccgggcctgg gtcacggccg
8881 gggacttaac cgaccacgcc cgggatgcgg aggtctgagc gcgcgaccgg gaccagcgca
8941 ccctgggggg cggggagagg cggtgcagct gcggaccgcg gtcgaggcat gctgcggggg
9001 tggctacctt gtcgccgcgg cctcgccgcc agcaagctcc gctccatggc tggccgactg
9061 cggcactcac ggcccctccc ccgcctcgcc ccggcggccg gtctgccgcc cgcttccgcg
9121 ttgcgcgctc ccatccccgc cggaagcggc gtcggagcag cgcggcaggg cgcggaaacc
9181 cgggcgggtg gagtggcacc aaacggagtg agaccggtct gtccagccgc tccccatccc
9241 aacccaccga cgtactccac cagggagacc acacccttca taaccaacgt ccgcgttttt
9301 gcctttgttg cttcggtcac agcccgaagg attttccctt atgactagat gaccccttaa
9361 aagaaaacaa aaacaaaaac aaaaaaaccc agagtgacca agacctgcaa actcacctta
9421 actgcaactg gctaggctca gctaacccac tccacaatga tgctgcacac caaccccagg
9481 gtgttggtct ctaaactggt ctgggtcagc ccctcaaggc tgaactgttt taccctcaca
9541 tttctcttta cagagaggga catcactagc tacatatagg gagactgggg ggtctgcagc
9601 atcaccactg ctaaaactaa tagcactgtg tgggcaactg agtagggcag ctctaaatgg
9661 aaaaaggacc cagcacttta acattcccta ttagaatgga cagaaaggac aggaagccat
9721 aggtatataa actatttatt aacagacaag gcctacagac ttatttcttc ttggacacac
9781 ccacggtgcg gccacggcgg ccagtggtct tggtgtgctg gcctcggaca cgaaggctgc
9841 aggtaaaaag agaagagtca caggtcatgc acagcagaaa caaaaggttg ggaaaaaaga
9901 agagcgagta ggaggaatgc tgagtcgagg taggcaggga gatgagaccc cccactcacc
9961 cccagaagtg acgcagccct ctatggcccc gaatcttctt cagtcgctcc aggtcttcac
10021 ggagcttgtt gtccagacca ttggctagga cctggattca gaggaggggg caaggatgt
10081 tttatcccct gacctcctat gcccaattct gttaggaccc tccacccttc ctctaatcct
10141 gccctcattt cagtacacac ctggctgtat tttccatcct ttacatcctt ctgtctgttc
10201 aagaaccagt ctgggatctt gtactggcgt ggattctgca taatggtgat cacacgttcc
10261 acctggcagg cagaccaagg tcagatctag atggacccct ttctgaggct gaccccaccc
10321 ccagccccct tccttgtcct cacctcatcc tcagtgagtt ctcccgccct cttggtgagg
10381 tcaatgtctg ctttcctcaa caccacatga gcatatcttc ggcccacacc ctgaatgaag
10441 tgaggggttt cggaattagt gttgaatctg atttttaaat tcctttatag cctcattcaa
10501 aaagctcaca aaggtaggat ctgcaataat ctattttgca atcccctagg tcaccctttc
10561 ctcacccacc tttccagata taaaccgttc gacagaaggt taactttggg ccggtcgcag
10621 tggctcacgc ctgtaatccc agcactttgg gaggcccaag tgggcagatg acattgaggg
10681 caggagttca aaaccagcct ggccaacatg gtgaaaccct gtctctacta aaaatacaag
10741 aaaagttgct gagcatggtg gcaggtgcct gtaatcccag ctacttggga ggctgcagca
10801 gaatagcttg aacctaggag gtggaggttg cagcaagcca agatcacgca accacactct
10861 aacctgggcg acagtgagac cccgtctcaa aaaaagaaaa aagtaacttt gttcaggggt
10921 atgactgaat catgaacaca gaatttagta ttacagacaa ccctttgaaa gcaggcagta
10981 tctttttttt cccccattgc tcagcatatg tagttgtcac taagtgcctt ctgattcttg
11041 catttaggag aacaaggggc ctgccactcc aagctgatgc agtaaaccag ggtcttatga
11101 gttacaggct ttgattaagg tattgggctg tcagttcctc agaatctcta agggtcacaa

11161 ttatttaggc aaatcactgt atccccataa tcaaagtatt ccaagatcac aaactcaatt
11221 ttgtcagtgc ccagaactga ctgcccaagt tattataaaa agaaactaat gcttacattg
11281 gtagcatcac atcaacacca gcactagcac ccatgccatc tgaactcatg aaccaaagtg
11341 atttggagag tgaaggagga actgaatact gagccctgcc cctcattttg tccaactaga
11401 acatcagaat gtggccctat ttggaaataa ggtcgttacg gaaataatta ggtaaaatga
11461 cgttatattt gattagggtg ggctctagat ccaatgacaa atcatgagag agacacgaag
11521 aggaccatga gatgacagca acagattgga gtaatgcagc tacaagccaa ggaacactaa
11581 ggattactga caaccaccag aagacaggaa gaggcaaggt aggattctta agtgcctcca
11641 gcgccaagag taattttctg cttagtttat atgaacttgt tacagtaggc tccaggaaac
11701 taaaacaaat ggcagttcct atctgcaaga aagagatcaa gggttggctt caccgtggct
11761 catgcctata tgcctgtaat cccagcactt taggaggctg aggctggagg atcactgagc
11821 ccaggagttc aagaccagcc tggacaacac agcaagatcc agattagcca gatgtggtgg
11881 tgcacacctg tagccccaac tacttggaaa gttgaggtag gaggattgac ttgagccctg
11941 gaggtcgagg ctgcagtgaa gagtgattcc gccactccag cctgggcgac agtctcaaaa
12001 aataaatgtg gctgggtgca gtggctcacg cctataatcc cagcactttg ggaggctgag
12061 gtgggtggat cacctgaggt cagaagttta agaccagttt gaccaacatg gtgaaacccc
12121 atctcttcta aaaatacaaa aattagctgg gcgtgtgcct gtaatctcag ctacttggga
12181 ggctgaggtt gcagtgagat aagattgcac cattgcactc cggcctgggt gacggggtga
12241 gactctgtct cgggataaat aaataaacat gaccaagggt caaattatta ggttgattca
12301 gaagagagca gggtctttca aatttattaa ttaaatgacg aggtttcacc atgttgacca
12361 ggctggtctc gaactcctga cctcagggga tccgcccact tcagcctccg agtgagccac
12421 tgcactaagc ctcaaattta attatatgtt tacaagtgaa aagatttact tcaaaatggg
12481 tgtgggaaga gatatagatg aaataagatt tctaattttt agttagatac atccatgaag
12541 gttattctct attacactta tgaatgctgc acctctactt aagcctcaca gccggtctct
12601 gaaataagta cagtatttca cactgcctta ttagtttcac agagcaccca acagaaggaa
12661 tttccctcta caaattcctc aacagagcca acagtatccc tactactatc cattccctaa
12721 cgtcgcctaa gtacctacca tgcaacagaa gccaagaaga caggcctagc cctgcacgca
12781 tggacacata ctggtaggct atttccacaa ctaactacac attctgactt aagggaaaaa
12841 aaaagaacgt tatgtatcta gaaagccaag aattcattag gtttgtggag agaaatactc
12901 aagtgaaata atattcttgc attcattgat tcgattctta aaagccaaag aaagatggat
12961 gtactccttc acacttcgac aggaaaggag tgtcaattat ggctcatctg agtaaatgtc
13021 ccactcagac ttcttccacc ctccatctac aggcttcttc acacttttta tctgttggta
13081 tttctactta tccctcaaga tgcccaataa tcctccaatt ctcagggaat ataaatttcg
13141 caggaagggt tgctgttatg atggtgggat gggtaaagaa accgaatcta gaagaaatct
13201 cacaagctcg tggttcagga aaatcttagc gaattcgggt gagcaaccat acctcctccc
13261 cacttcccca ggtgaaatgt tttggcttga ccttagacca ctatcttggc tttaatcact
13321 tattttctgt ggtaaatgct aaaggggtca gtctctccct aaaccacttt cttttcttaa
13381 tccagcagtc aaggtgatct ggatgagact caagtctccc cagccttgct tcattttgc
13441 ttatgtcttc acaacccaat tctcatttac attccctatt ccttacccta cttcacttac
13501 cttaatggca gtgatggcaa aggctatttt ccgccgccca tcgatgttgg tgttgagtac
13561 tcgcaaaata tgctggaact tttcagggat cactagagac tagttgaaaa agtacggggg
13621 aagaaatcag acacagtaaa ctatcaaaca gtaaggccga taaggcaaac gaacttcata
13681 caaaaaggtt aagcctgtca cacaagtggc aagacacccg ccccccattc cttaggtttt
13741 cacaccacag agcaaactcc tcagtcctgc agagtaggca catgaagcgt tcaagcttgg
13801 gaaaatgcaa cctctggaga gaggaagcag gtctcctgcc cattcccaaa agtgcgtggc
13861 aaaagccaga gttccccata tacgcccaag ccttagacag ctttcgtgcg cccgagcagt
13921 gacccttcct cgggcccgct ctccggaatt ggaaatcttt ggtgtccctt tccagggacg
13981 cgatccaagg ctccatacag aaagtcgcag gccctcaggg caggacgttt ccagaaccct
14041 gacagccggc tttccttgcc cgagctgcga tgccgctgga tcacggcacg gattccagtc
14101 ttaccatggc tgcagcacaa gcggcggcgt gtaggcctcc tgtggaagag agagcggaag
14161 tgacgcgata tcattatata gcttccagga ggaagaggcg gtgcgatcta ggagaagtgc
14221 ttccggaaac tgtttcagcg cgacctggct ggatttatga gagtatggct tgggtaccac
14281 aacttccggt gcgcctttct ttacagttcg taaggttcat aggtaagaga acagcgaagg
14341 ttccggggct agtttgtgtt tcagacttcc atatttccat ttgcagttgg gtgaagtggt
14401 tagagctgaa aggggacagc tggagtcaaa caacttaact caaatatctg ggaacgattt
14461 cgcgctgagg aagacccttt gggacttgta gctccactcc ggggaacgga ctcgccggga
14521 ctgacagttg ccggaagtga ggctgcgggg aatggccgcc gctgcgacca tggcggctgc
14581 ggcccgggaa ctggtgttgc gggctgggac ctcagatatg gaggaggaag agggcccgct
14641 ggtgagagcg cggagctgtt tctcctccgt agttccggtg ctcctagctt cagaaagacc
14701 tgagctgggt ttaggcacaa gggtgggaac ttggctgcta cccgtgatat tacctgtttg
14761 gctccccgta cccttccctg tgatcagacc cgaccctgag cgggacccta gtggctgaaa
14821 ctcatgttct ggggcacaaa atttcaactt cactccacat tttggggtag agtcacgaag
14881 tagtatagtg taaaggttaa gaacatggcc tctgaagcca gactgcctga gttctatctc

14941 tgccacttac acgctgtgtg cccttgggca agttaaccag cttctctgtg ccatcatttc
15001 tcacaccttt aaggtgagga agatagaacc tattgtatac atcgtcgtta ggattagatg
15061 aatatgtgta agattgcacc tggcatgcag gtaaacacta tgtgttttga catttctaga
15121 aagcctgggg ttccccagag gtgtgataga acacccaaga tggcaggagt ctgagggtct
15181 taaatttaag tgcaagttct gctctatcct ctgcaattcc tagctttcct cattcggtca
15241 tctccactgc actttttttt tttttttttt ttttttgaga cggagtcttg ctctgtcgcc
15301 caggctggag tgcagtggcg cgatcttggc tcactgcaag ctccaccttc cgggttcacg
15361 ccattctcct gcctcaacct cccaagtagc tgggactaca ggcgcccgcc accgcgcccg
15421 gctaattttt tgtatttttt agtagagacg gggtttcact gtgttagcca ggatggtctc
15481 gatctcctga cctcgtgatc ttcccacctc ggcctcccaa agtgctggga ttacaggcgt
15541 gagccactgc gtccggcctc cactgcactg tcttgtcttt tacctttttt tgttttcttt
15601 agacgggttt ccctgtgtta cccaggttga ccttgaactc ctgatctcct caaacaatct
15661 tcccgtctca gcttctaagt agttgggact acaaggagcc caccaccatg ctgggctacc
15721 tccactgtct cactggatcg tctcactcct tgtcgtattc cttacccatc agcacaaaat
15781 aggagagtgg gtacaacgtg agactcttag aatttttcaa agcagtacct gtggctccca
15841 gtaaaagcag ctccttctca cttgaaggac tggggatatc caattatatt ttttctttct
15901 gtctttcacc gtaccctact cccattaact tatccctttc caggcgggtg gtcctgggct
15961 ccaggaacca ctgcaacttg gggagttgga tatcacttct gatgaattca tcctggatga
16021 agtggatggt aagcgatggc atggggtggg tgcagtacct gtaatctgag aggcatgggg
16081 gctggaagga ataaggtagg gattggtgtg cagatactca atgtggggag agggagctgt
16141 ggagctggac ccttatgata aacttctatt tccagttcac attcaggcaa atctggagga
16201 tgagttagta aaggaagctc ttaaaacggt gaggctttcc cctgtactaa tctgcccttc
16261 tatctattct accccaaaag gaaaaacaac agtctcttgc ttttgttgta tcagcctggt
16321 atctgaccct ttagtccttc actgattctg ttacagtgtc cctttactga gacttccttc
16381 tgacctgtca tctttatgtt ctcccagggt gtagatctcc gtcactattc aaagcaagtt
16441 gagctggagc tacagcagat tgaacagaaa tccattcggg attgtatcct ccagcagagg
16501 gaaggggtgg tgctattgac atggggattt tgtggcgtgt ggtacagatg tcccagctgg
16561 ggtcaacatc tctgaggttt ctatcacttc aaagagaaat tggagaggta ctacaaatct
16621 gaggatctcc catcttcacc atgaggcccc ttgacttttt gtggatcaga tattcaagag
16681 agtgagaata tagcatctct acacaaccag atcacagcct gtgatgctgt cctggaggtt
16741 agtaatcatg acctgtgacc cctaatagct actctgaacc agatcatgga ctctggtgcc
16801 atttttaggt catcagcctc aagatgggga caggttaggg tcattttcaa gatgacttca
16861 ggagcctttt ttgttattac tgccatattt aatgttattt cctgataatc tggggtgtct
16921 catcatctgc caccctcaag gactgataaa tactgaagac ttcacaaaga gtctttggtg
16981 gcccacttat cccctacaaa ccctatagca ctccggcaac atctaccctc cctacagcga
17041 atggagcaga tgttgggagc ttttcagagt gacctcagct ccatcagctc tgagatccgg
17101 acactgcagg aacagtcagg agccatgaac attcgacttc gaaatcgcca ggcagttcgg
17161 gggaaacttg gggagcttgt tgatggtctg gtggtgcctt ctgctctggt cacgtgagtt
17221 accagtttga agggttataa tgtccatagc aggtggatgg gctggggtga cccaccctat
17281 ttaccatatg ggtctcctgg ctccaggtta agtgcttttt agaaattagt agccaggtta
17341 accaccattt ctttaggtat gcattaggca tcaaagatta agattaagaa tgatgtaaga
17401 tcaggtgtat agtggggagg tccaggaata gaaaactttg tacatgtgtc ctgactatat
17461 ggtacaatgc aaggtggtag agatgacagg ggctggaggt aaggagtgtg gtctgggtct
17521 ggtttaggga tgtctgggtc tcccttgtaa ctcatcccac ccctccctgc taaccagggc
17581 aattctggag gctccagtga cagagcccag gttcttggag cagctacagg agctggatgc
17641 caaggcagcc gcagtcagag agcaggaagc tagaggcaca gcagcctgcg cagatgtcag
17701 aggcgtgctc gatcggctcc gggtcaaggt gggaagtagg gatggatacc ctggaggcta
17761 ctggaagcag ccttcccaag atcttcacta tggatttcct tggcagagct cactgtagcc
17821 tgtctccagg gacttcaccc tgcctttctt gggcagcccc attcttgtct ctctgggttt
17881 ccctgaaaat cttgagaaat accaaagaga tattcaccac ccttctgcct atgtttacca
17941 tttgttgctt cccccagttc ctctcataca gcaaaagggt tgcagcttca tgccctgtgt
18001 ttggttcccc acatctaggc agtgacgaag atccgagagt ttatcctcca gaagatttat
18061 tccttcagga aacccatgac caactatcag atcccccaga cggccctgct gaagtacagg
18121 tcacaacccc aaggaagtgc atgggatggc ctttgagttt ttgagcggcc tagtgtgggc
18181 atctggtttc ttgggagaca ggtagactga catgttcctg accccctagg ttcttctatc
18241 agtttctgct gggcaatgaa cgagcaacag caaaggagat cagggatgaa tatgtggaga
18301 cgctgagcaa gatttacctg tcttactacc gctcttacct ggggcggctc atgaaggtgc
18361 aggtaaggtc aggagggaga ggtattcctg ggcacatgga ctgggaggag gggacatccc
18421 tgggcaagga atattttatt gtgttgtggg aagagacggt tatcagttct cttttctctt
18481 tccttagtat gaggaagtcg ctgagaaaga tgatctaatg ggtgtggaag atacagcaaa
18541 gaaagatatc ctagtactgg ggaaccctca tgccaggcct tgagagtggg aggactttcg
18601 actccagcca ccaatgcact gctccttacc ttttcctatg ggactgaaac cttttagaat
18661 gtgatgaaag ctatagcctc cctccccaga aagatgcaca cattattttg acacatagtt

18721 ttgcatatga ttgcaaggga gggagagaca ccctgaagcc cattcacaga tgtcagcacg
18781 tggggcccta gtactgggaa agagggctgg ctaggccacg tctgcctgtc tggggtcccc
18841 acagatagtg agtctttggg cccagttatg gtctcattgt tttccctgac tctgacccat
18901 cttacgattc ttctcaaagc catcgctccg cagcaggaac accattttca ccctaggaac
18961 ccgcggctct gtcatctccc ccactgaact tgaggccccc atcctggtgc ctcacacagc
19021 gcagcgcgga gagcagaggg tatgaggagg aacaagcttc agtcacgaaa gaggctctga
19081 gcacggtggg gagggaaggc cagacctcag ggcagagctt cccaacctgc aggccctgtc
19141 ttaatcccct tagacttggg agtggccttc tccaatcact ccagcctgct gtgatgtgaa
19201 aagagctggg aaccattgac ttaagggcat gcagagggga gagactggat gaggggaatg
19261 acagtcagag gcaggtgttg aggtggatgc aggagtagca acagcaggtg ggtgtaggcc
19321 ctttttgtac ccctatgttc ccccaccttt tcagtgccca ttcagcttgc tcttagacag
19381 cccggaccta gatagatgcg aagggtctgc ctagctctta ttactcaggt ttactgattt
19441 tccagccata tccatgggaa gtcagtgttg gggtccatcc cttctgtttt tcttctttac
19501 tcctcctctc ccagtatcca tttgaggccc tcttccgcag ccagcactac gccctcctag
19561 acaattcctg ccgcgaatac cttttcatct gtgaattttt tgttgtgtct ggcccagctg
19621 cacacgacct gttccatgct gtcatgggcc gtacactcag catgaccctg gtaagactcc
19681 tgtttcctgt accattcaac acctttgtcc ccagtcttca tcagcagcat agtggggtca
19741 tgactctggt agaactgaaa tcaggatcgc ctcattctaa aatgtctaag ccttggctgg
19801 gcgtggtagt gcacacccat aatcccagca ctttgggagt ccaaggcagt aggattgcct
19861 gagctcagga gtttgagacc agcctgggca acaacctcat ctctacaaaa aatgaaaaat
19921 tagccgggtg tgtgttgatg tgtgcctgtg gtcacagcta ctaaagatgc tgaggtggaa
19981 ggattgcttg agcccaggag gtcaaggctg cagtgagcca tgcactccag agtgagacct
20041 gtctcaaaaa aaaaaaaaaa aaaaaatagc caggtgcagt ggcttacgcc tgtaatccca
20101 gcactttggg aggctgaggc gggaggatca caaggtcagg agtttgagac cagcctgacc
20161 aacatgatga aaccctgtct ctactaacag tacaaaaatt agccaggtgt gcgcggcctg
20221 taatcccagc tactcgggag gctgaggcag gaggattgct tgaacctggg aggaggagat
20281 tgcagtgagc caagattgcg ccattgcact ccagcctggg tgacagagcg agactccatc
20341 tcaaaaaaaa aacttgttac ttagacttgt cacagtgtta ctcccccatg aaatcctcag
20401 tgcgtagggt ccctgagact acccctactg ctgacccaca atcaggcaat agcacgtgaa
20461 aactgagggt cccccaaatc ttggtgctca aacccctgac ccaagctcag ccctgaactc
20521 tgctgtgata ggcttgtgat catagatcct ggtgtggcca ggtgcagtgg cttatgcctg
20581 caatcccagt actttgggag accaaggcag gaaggttgct tgaggccagg agttcgagac
20641 cagcctgggc aatgcggcaa gaccttgtct ctacaaaaaa tttaaaaata aacttaacca
20701 ggcatggtgg tacacaccca tggtcccagt tgcttggcag gctgaggtgg gaacatcact
20761 tgagcccaga tgtttgaggc tgcagtgaac tatgattgta ccattacaat ccagcctagg
20821 tgacaaaaca acctgtctct aaagccgaaa acaccgggtg cagtggctca cacctgtaat
20881 cccagcactt taggaggctg aggtgggcgg atcgcctgag gtcaggagtt cgagaccagc
20941 ttgaccaaca tgatgaaacc ccatctctac taaaatacaa aaattagtcg ggcgtggtgg
21001 caggcgccta taattccagc tactcaggag gctgaggcag gagaattgct tgaacctggg
21061 aggcaggcgg aggttgcagt gagccgagat tgtgccactg cattccagct tgggtggcaa
21121 gagtgaaact ctatctcaaa aaacaaaaaa aaagccaaaa acatagatcc tggacaggaa
21181 agcagggggc agttccattt tacgtctctc ccttctgtcc tgtccagtgc atcatcccca
21241 aagaaacaaa tgcgtttgtt catagtcact gaggacaaca aactgacatt ctcttttaat
21301 cctctgtttg ttccccaatc acacagaaac acctggattc ttatctagct gactgctacg
21361 atgccattgc tgtttttctc tgtatccaca ttgttctccg gttccgtaac attgcagcaa
21421 agagggatgt tcctgccctg gacaggtcac tgaattctgg tccttgatgc ccaacaggcc
21481 acgaactctc ttggtcttgc atgactctgc cctgacctta gactcctact gacctggtat
21541 catcaacctt tattcccatt catttgctcc ggaggcagta gtgctgcttt ttgactcacc
21601 tttctccaac cactgccccc caacccccac aactagctat tgaccattaa actgaatgat
21661 gccccactcc ctcccatccc cctccaacat tccttgctgg ctgacctctg attctgatta
21721 agcccacagg tactgggaac aggtgcttgc cttgctatgg ccacggtttg aactgatcct
21781 ggagatgaat gttcagagcg tccgaagcac tgacccccag cgcctagggg ggttggatac
21841 tcggccccac tatgtgaggg agggcaaggg taacaaaggg ttcttgaaag gcagggctct
21901 aagcatcatt gtgggggttg gccaaggtca cagttgtgtc atgggccacg ctatgagcag
21961 gtgttctggg agggtggtga aactcatggt gagtggtgca ctgtgggtag gagggactga
22021 aggtagagtc acatcacatg gaggtgggtt ggctaaggaa gttgggaggt tgtgttggtt
22081 gggccaggag gtgggtgtga ttttccttcc cactctctcc tagatcacac gccgctatgc
22141 agagttctcc tccgctcttg tcagtatcaa ccagacaatt cctaatgaac ggaccatgca
22201 attgctggga cagctgcagg tgagggtcgg acaggagaca cttcccagca gcagggctgc
22261 ttccagtggg tggagtctga ggaggagcca gatggggccc agatgacctt gctttctggt
22321 gtcacctctt cctctctgcc ttttcaggtg gaggtggaga attttgtcct ccgagtggca
22381 gctgagttct cctcaaggaa ggagcagctt gtgtttctga tcaacaacta tgacatgatg
22441 ctgggtgtgc tgatggtaat aggtgctcct tctccttgcc ttcctaccca gaaaattcgg

22501 tttcccccac tgctgttagc tctgtgaggg cagagatgat gcctgtcttg ttcagtgttt
22561 tactcttgga gtctggcaca gtgtccagct tagtacaata agtgtggaat gaaggggaca
22621 tcctgaagtg aaggactgct ttccccaaac tagacatttc cctttgtctt cccttaggag
22681 cgggctgcag atgacagcaa agaggttgag agcttccagc agctgctcaa tgctcggaca
22741 caggtagggg tgtggggaaa agggaagaga cagccttctg tgctgttttc agctctctct
22801 ctctctctct ctgtgtgtgt gtgtgtgtgt gtgtgtgtgt gtagggagtg gcataatgga
22861 tctcaggtat gcggaattac tgtaactctt gattcacaac atactgtcat tattggtcta
22921 ttttatttta ttttgtcttt ggtttttttt tttttttttt tttgaggcaa agtgtcactc
22981 tgtcacccag gctggagtgc agtggcatga acatggctca ctgcagcctc aacagcctgg
23041 gcttaagtga tccttctacc ttagccccct gggtagctgg gaccacaggt gcgagccacc
23101 atgcctagct aattttttat ttttagaatg aattttattt ttttctttat ctatcctctc
23161 aagcatttat cctttgtgtt ataaacaatc cgattacact aagttatttt aaaatgtaca
23221 attaagttat aattgactat agtcaccctg ttgtgctgtc aaatagtagg tcttatttat
23281 tcttcctatt tttgtttgta cccattaatc atccccacct tctccccagc cttccactag
23341 ccttcccagc ctctggtagc catccttcta ctctcttcgt acatgagtac atgagttcaa
23401 ttgttttgat ttttagattc cacgaatcag tgagaacaca tgacgtttgt ctttctgtgc
23461 ctggcttatt tcacttaata taataatttc caattctatg catgttgttg caaatgactg
23521 agtctcattc ttttttatgg ctgagtagta ctccattgtg tatatatccc acatttcttt
23581 tacccattca tctgttgatg gacacttagg ttgcttccaa atcttagcta ttgtgaacaa
23641 tactgcaaca aacatgggag tgcaaatatc tcttcaatgt actgatttcc tttcttttgg
23701 gtatataccc agcagtggga ttgctgaatc atatggtagc tctactttta gtttttgag
23761 caaccttcaa actgttctcc aaagtgtttg tactaatcta cattcccacc aacagtgtat
23821 aagggttccc ttttctccac atccttgcca gcattgctat tgcctgtctt ttggataaaa
23881 gccattctga ctggagtgag atgagatctc attgtagttt tgatttgtat ttctctgata
23941 atagtgatgg tgagcccctt ttcatatatg tctgtttgcc atttgtattc ttttgaaaaa
24001 tatctattca aatcttttgc ccatttttta ataggattat tagactgtca tgtagagttg
24061 tttgagcccc ttatatattc tggttattaa tcccttggca gatgggatgt gcaaatattc
24121 tttgtctctt cactttattg tttctttagc tgtgcagaag cttttttttt tttttttttt
24181 ttttttgaga tggagttttg ttcttgttgt ccaggctgga gtgcgatggt gcgatctcag
24241 ctcaccgcaa cctccgcctc ccaggttcaa gcaattttcc tgcctcagcc tcctgagtag
24301 ctgtgattac aggcatgtgc caccatgcct ggctaatttt gtatttttag tagagatggg
24361 gtttcaccat gttggtcagg ctggtctcaa actcctgacc tcaggtgatc tgcccagctc
24421 tgtctcccaa agtgctggga ttacaggcgt gagccaccgt gcccagctgt gcagaagctt
24481 tgtaacttga cgtgatccca tttggccatt tttgctttgg ttgcctgtgc ttgtggggta
24541 tggctcaaga aatttttgcc cagacccgtg tcttggagat tttccccagt gttttcttgt
24601 agtagtttca taatttggtc ttagatttaa gtctttaatc catttgattt ggtttcata
24661 tgtggcgaga gataggggta tagtttcatt cttctgtgta atggatagcc agttttccca
24721 gcaccattat tgaagagact atctctcttt tttttttttt cagacggagt cttgctctgt
24781 cacctaggct ggagtgcagt ggtgcagtat tggctcactg caacctctgc ctccttggtt
24841 caagtgattc tcctgcctca gcctcctgaa tagttgggat tactgatgcc tgccaccacg
24901 ccgggctagt tttgtgtttt ttagtagaga cgggatttca ccatgttagc caggcttgtc
24961 ttgaactcct gacttcaagt gatcctccca cctgggcctc ccaagtgctg ggattacagg
25021 cgtgagctac cgtgcctgac caagagacta tcttttcccc agtgtatgtt cttggcactt
25081 ttaccaaaaa tgagtttact gtaggtgtgt gccagctaat tttttaattt tgtaaagaca
25141 gttgtcttta caaaagacaa ctgttgcctg ggctggtctt gaattactgc cctcaagtga
25201 aaaatcttat gattcttttt tttttttaaa gacggagtct cactctgact cccacactgg
25261 agtgcagcga tacgatgtca gctcactgca acgtccgcct cccaggttaa gtgattctcc
25321 tgcctcagcc tctgagtagc tgggactgca ggcacgtgcc accatgtcca gctaattttt
25381 gtatttttag tagagatggg gtttcaccat gttggcccag ctggtcttaa aactcctgac
25441 ctcaggtgat tcacccgcct cagcctccca aagtgctggg attataggca tgagtcactg
25501 cacccggcct agattctgtt tttaatgaca ctggaaaaca acgaattttt gttttggcat
25561 atatatagct ttacttttaa aagcaagaac acacacaaaa aaagggggaa aaaagcaaga
25621 acacaataaa tgcaacattt agatcaacga gtacctgtgc acgagaagca cagggatggt
25681 ttcagtagga tttcatagtt tgatggtgac ttttgccaag ctgctgctac ttggattgtg
25741 tggtgtgttt gtgagtgttt atatcattaa aaaacaaaac agccactgca ctgggttgtt
25801 ttatttttta atgatataaa cactgatgag acaaggcaag aaaacagcaa ggcaaaaaag
25861 aaaaaaaaaa aagccaggca tggtggtatg cacctgtagt tctagctatt tgggagacaa
25921 aggcgagagg accactttga aatccaggag ttcgagatta cagcgagcca tgatcatgac
25981 attgtagtct acccttggca acacagcaag accctgtctc aaacagcaaa acaaaacaaa
26041 aaaccccaga atcacaggat ttttttcatg cataattttt ctaatattgt catgttattg
26101 catatggcta tagctcattc cttttcactg ccatatatga cttgtgtgat taaacatatt
26161 ctataaatgg attgtttcca gtttcttgct tctgtgagca ttgttttatg aataatcatg
26221 tcttttagca cacatagata ttgattatct tcatcaaaag tttctcttgg gtataaacct

26281 aggacagtag tcttgctgct aataagatat gcagtattag tctttatgag acaatgataa
26341 attgttttcc taagtagttc cttctcacca gcaatatgta agaattccta ctgagccaca
26401 ttttctccaa catgtggtat tatcaggtct ttgccatttt ttttcttttt tttttagag
26461 accaaggatg ctgctaaaga tgctcgggat cagaaatgtt tcagattttg atttttttcg
26521 gattttggat tccttgcatt atttttactg gttagcatct gtagtctgaa aatctgaaat
26581 ctgaaatgct ccaaagagca ttacctttga gcatcatgtt gatactcaaa aacttccgga
26641 ttttggagca ttttggattt cagattattg gattagggat actcaacctg tatttcagtg
26701 ggttttattt gtaattttct gattactaat aacattgagc atcatttcaa atgtttagta
26761 actatatgta ggggttcagt caggctggtg ggaaaaatat tagtgatgat agccacaaac
26821 cctcttggaa ggcctaagag tttgcataac ttcagtaata gatatggttg aaggcgacct
26881 gatctttacc tttagttaaa taaattagag taataacaaa ggaatgtggg gaagttatct
26941 aggtagcttg tttactcata tggtcttaag actaatcttt gatgtaccgc aggtgcttaa
27001 ctgctttcta ctcaggaagt ccacaatgtc agttaccccg tagtggtgtt gactcaagtc
27061 tttgtcaatt aatctttact gaataaatgc gagtctcact agctggtcag ggccgccatt
27121 gcaactgttt acagtactct tcagggagtc tgtaaacagc ctggacacac tcagctggac
27181 tggcaaagca gagtatctgt gtgtcagtgt acctcattca tccgttgccg ggtcaggggt
27241 ctgcaaggga cagactccct gaagctggtg ctctgtgtga ggagcgtcac cacaactata
27301 caattttttt ttttcctgta aaatggctgt catagcattt gaccttttaa aaatcggctt
27361 gtggctgggc agggtgtaca cctgtaatcc cagcactttg ggaggctgag gcggatggat
27421 cagttgaggc cagtaattca agaccagcct ggccagcatg gcagaaccct gtctctacta
27481 gaagtacaaa aattaaccag gcatagtggc gcatgcctgt agtcccagct cctcaggggc
27541 gctgaacctg ggaggcggag gttgcagtga gccaagatta tgccactgca ctccagcctc
27601 agttacagag cgtgactctc tcaaaaaata aagaaaaaaa taagtctgct agtatttttt
27661 atttataaga gttctttgca tcatctacat actaatcttg ttgattatac gtaggttaca
27721 gattatcttt tagtatgtat ctttttactt tttttttttt ttttggtgag atgagggtct
27781 ttctgtgttg cccaggctcc tgggctcaag aaatccttgc accttttttt tttttttttt
27841 gagatggagt ttcactcatg ttgccgaggc tggagtgcaa tggtgtgatc tcagctcact
27901 gcacctctgc ctcccgggtt caagcactta tcctttctca gcctcctgag tagctgggat
27961 tacaggcgtg tgctaccacg cccggctaat ttttgtatat ttagtagaga tgggttttgc
28021 catgttggcc aggccagtct tgaacgcctg acctcaggtg atccatccgc ctcagcctcc
28081 caaaatgctg ggattacagg catgagccac cgcgtccagc ctttgttttt ttaatgttgc
28141 tttttgatta aagaaatttg taattttaat atgattgaat ttatctgtcc ttcattttta
28201 tgtctttgtt tttagtaaca gtcttgctct gtcacccaag ctggaatgca gtagtgtgat
28261 catggctcac tgtagcctca acctcctggg ctcaggcaac cctcctccct cagcctccca
28321 agtaactagg actacaggtg tgtgccatat gcccagctaa tttttgttgt tgttgttgtt
28381 ttgtagagac agagtctcac tatgttgccc aggctggtct caaactcctg gcctcaagtg
28441 atcctcccgc atcagcctcc caaagtgctg ggcttacagc caccatctac cacgcctgac
28501 tatttttgtc ctgtttaaga aattcctggg ctgggtgtgg tggctcacgc ctgtaatccc
28561 agtactttgg gaggctgagg cgggtggatt acgaggtcac gggatcgaga ccatcctggc
28621 caacatggtg aaacctcgtc tctactaaaa atacaaaaaa attagctggg cgtggtgatg
28681 cgtacctgta gtcctagcta ctcatgaggc tgaggcagga gaatcacttg aaccagggag
28741 gtggaggatg cagtgagcca agatcgtgcc actgcactcc agactggtga tagagtgaga
28801 ctccgtttaa aaaaaaaaaa aatgaaattc cttattactc caaggccaga aaaatattat
28861 gctacatttt cttcttaata ttgtagaatt ttggccaggt gcagtggcta aagcctgtaa
28921 tcccagcact ttgggaggcc aaggcaggaa gattgcttga agccaggagt ttgagaccag
28981 cctaggcagt atagtgagac ctcatctcta caaaaaatta aaaaaatata ttagccaagc
29041 atagtggcac acacctgtag tctctgctac tcaggtagct gaggtgggag gatcacttga
29101 gcctgggagg tcgaggctgc agtgagccct gtttgtgctg ctgcactcca gcctgggcaa
29161 cagagtgaga ccctgtctca aaaaataata ataaaattaa agaataaaca aatatataga
29221 gagaattttt ttgttacctt taagtctcaa atttacttgg aattaatttt tgtgtgtgat
29281 gtgaggtagg gatctatttc ttttttttat gtggatggtt ggcccagaac catttataga
29341 aaatcttttt cttttctttt tttttctttt cttttttttt ttttttagac agagtctcac
29401 tcttgtcacc caggctggag tgcagggcca caatctcagc gcactgcaac ctctgcctcc
29461 tgggttcaag tgattctcct gcctcagcct cctgagtagc taggattaca ggcatgtgcc
29521 accacaccca gctaattttt gtatttttag tagagatggg gtttcaccgt gttggctagg
29581 ctggtctcga aaagttttca cattttaaaa agtctttaag gattttgatt agaattacat
29641 tgtctataga ctaatttgga agaattgata ctttatgata ttgaaacttc ctatccatga
29701 tcatgatcat gggctcccta tccttttttt tttttttttt tttttttttt tttttttttt
29761 tttgagacag gttttgctct gtcactcagg ttggagtgca gtggcacaat catagctcac
29821 tgcagccttg aattcctggg ttcaagtgct tctcttgtct cagcctcccg agtagctagg
29881 actagaggca cacagcacca cacttggctt ttttttttct gtagagaata caaaatttaa
29941 aaatttaatt ttttgtagtg tctcactaaa ctgggattac agatgtgagc cactccacct
30001 ggcctcctcc tccaattttt aagagagtct tttaatgtct tttgataacc tttttaaatt

30061 ttcagcacaa agacactgta agtcgtttgc catattttta ggtgggtata atacgtatgt
30121 ttagttctac taggaataaa taaatctttt ctagtttttt gtctttaaca tcttatgttt
30181 ttgatttctt attctactgg ctaacactcc caggatgatg tttagaagtt gaacagaaag
30241 gtgatagtga gaaccctttt catattcctg attttgaaag aattgttgct ggtgctgtgg
30301 ctcatgcctg taatcccagc actttgggat gctgagatgg gagaatctct tgagcccagg
30361 agtttgagag tttgagggga tctgcctggg caacatagtg agactccatt tctttttttt
30421 tttttttttt ttagacagag tctccctctg tctcccaggc tggagtggtg cagtggtgca
30481 atctcagctc actgcaagct ccgcctcccg ggttcatgcc attcttcctg cctcagcctc
30541 ccgagtagct gagactacag gtgcccagca ccatgcccgg tatttttttt gtattttgta
30601 tttttagtag agacgggggg tttcaccatg ttagccagga tggtcttgat ctgacctcgt
30661 catctgcccg ccttggcctc ccaaagtgct gggattacag gtgttagcca ccatgcccgg
30721 ctaagacccc atttctttaa aaaaaaaaaa aaaaaaaaaa aaaagccagg cgtagtggca
30781 tgcatttgta gttctagctg cttgggaggc tgaggcggga tcattgcagg agattaaggc
30841 acccacaaag atgcacttaa accagttgtt tctgctgaga gcagctggga tttgtcactg
30901 gatttgccag gtatagagac tcgccagctg ccatcaatgc ttctggtcct ttttggtcag
30961 ctgctgcttc atactacgta ggaaagattg ttaaagccca gtgcactgtt tcctctggtc
31021 actcctctgc tatcaaggtg ttctcttttg cgttatagaa agcttaaagt tcatctaagt
31081 ccagaccagt actcctatag gaatataatg catgccacaa atgtgagcca cttaaataat
31141 tttaaatttt ctagaagcta cattaaaaag taaggagaaa cagatgaaat aattttttaa
31201 accactacat ccaaaatatt attatttcaa catataatca ataaaaaata gtactgaggc
31261 cagcacggtg gctcacacct gtaatcccag cacaggccaa ggcagggagg atcgcttgag
31321 cccaggagtt tgagaccagc ctggacaaca tggtgaaacc ccatctctac aaaaaataaa
31381 aaaaaaatga gtgggacatg ttggcacatg cctgttagtc ccagctactc aggaggctga
31441 ggtgggataa tcactgagcc aggaaagttg aggttgtagt gagctgtgat tgtgccactg
31501 cactccagcg tgggtgatgg agcaagaccc tgtttccgaa aaaaaaaaag aaaaaaaaga
31561 tatacttaaa atttattcta agatgaagtc ttcaaaatca ggtgtatata tcatgcttac
31621 aacacattat ataatttatg gctcctgcct agcaggggtc atcactgtta gttcaacttt
31681 tctttggcaa gtctactccc tgtgccccac cgtacactgc tacattattg tttgtatatg
31741 tgtgtgtgag actgcattta ggcaactgta ataggttgaa tttggttatt taatctctgt
31801 ccaaaatcac atcacaaaga tgccttgatt atagaaagat ccttccttgc agcatattag
31861 ttatttgaca ccctcagtgg atataagtga atattttgag ggaagaatag aaaagataca
31921 atttttattt atttatttat ttatttattt actgagacaa gttgctgctc tgtcgcccag
31981 gctggagtgc agtgacatga tcttggctca ctacaacctc tgcctcccgg ttcaagtgat
32041 tcttctgctt cagtctccct gagtagctgg ggttacaggc atgcaccacc acacctggct
32101 aattttagta ttaataattt atttttaaat ggaataataa tttcactttc ttcttaccag
32161 atctttctgt ctgggttgtt tgcttttatg agtttttttg tttgaatctc ccttttacat
32221 taaacaaaaa ttaacatttt attatgggaa atttcactat gctattagaa aagcatagtg
32281 aacccccagg catcatcagt gatattcggt gtatagcagt tcacggacag ttttgtttca
32341 tctataccaa cacctattgc ttatcctcct tctgtgtccc ccagttactt tgaattaaat
32401 cttatacata tacagatgaa tattatcact tcatctgtat cagaatgtta tttttattaa
32461 gaaaattatt tttgagatag ggccttgctg tagtgcccag gctggagtgt agtagcatga
32521 tataactccc tgcagcctcg aactcctggg ctcaagtgat cctcccacct cagcctccta
32581 agtaactggg actacaggtg tgtgccacca cacctggtaa cagatggttt taaaaactac
32641 aatacattat cacacttaaa aaaattatct tatctaaaat aactattcag attttcccag
32701 ttatttcata agtgattttt tttttttttt ttttgagaca cagtttcact cttgttgcct
32761 ccaggctgga gtgcaatggc ccgatcttgg cacactccaa catctgcctc ccgggttcaa
32821 gcaattctcc tgcctcagtc tcctgagtag ctgggattat agacacccac caccacgccc
32881 agctaatttt tgtattttta gtagagacag gatttcacca tgttggccag gctggtctca
32941 aactcctgac ctcaggtgat ctacccgcct cagcctccca aaatgctggg attacaggca
33001 tgagccactg tgcctggcct tcataaatga tttttatggt tcaaatcagg atccatataa
33061 agtccatata tgtgtctttt tttagtctat agatttccct tccatctcct ctgcaattta
33121 tttttggaag aaactgagtt attgagtcat ttgtcatcta gaatgatgta cagtttagat
33181 tttgctgact gcattccatg gtgtggccaa acttcctctg tctggtgtat ttctcataag
33241 tgtgtaatta gatggaaagg cttggtcaga tttaaattag tttttttttt tctacaggga
33301 tatttgagag gtagtggtat gtatttccat caggaagcac ataatgtctt cttggctctc
33361 tttgtgatgt tagtagccat caatttcagt gcctagatac attaattaga tttctgtatc
33421 tcctactatc tcaaaatttt ccatcttctg tctgtgctac attctgagtc ctttcttcag
33481 ttataaattc taattcattg tataattctt gttaatctgt tgactttcta aaactggctt
33541 ttcatatcta aaaaaatttc cttttttcaa gtctgttcat tcctcatagt tcttactaat
33601 tggtcatttt tacatccttt tttttttttt tttttttgga gacggagtct tgctctgtca
33661 tccaggctgg agtgcagtgg catgatctct gctcactgca acctccgcct cccaagttca
33721 agtgattctc ttgcctcagt ctcccgagta gctgggatta taggcatcca ccatcatgcc
33781 cagctaattt ttgtattttt agtagagatg gggtttcatc atgttggcca ggctggtctt

33841 gaactcctga cctcaggtga tctgcccacc tcagcctccc aaagtgctgg gattacaggc
33901 gtgagccact gtgcccggcc tatccttaac tttagacatt gcacacagta caactgctat
33961 ccgtatcgga tagttcttaa ggtcagcagc tattgcttat tgtgtctgct gactctcttg
34021 gtagctgccc ttcttttgtg tctagtgatc tttgagttca ttgtttgatc ttaaccaggg
34081 gaactgtatg ggccaaaatt aggattgagg atatttttct ccatagagga ttttccttaa
34141 cttttgcagc agctgaaaga tgccattcag atggatctac attagtcatg atgccagaat
34201 tgggcaaatc tgctgacacc aggacacctg tgtatatgtg tttgtgtctt tccaggaatt
34261 cattgaagag ttgctgtctc ccccttttgg gggtttagtg gcatttgtga aggaggctga
34321 ggctttgatt gagcgtggac aggctgagcg acttcgaggg gaagaaggta tgaggaaaat
34381 atggtaatga tgggatcagt ggtaagggaa gtggaaaaga aaaatgaaag gatgcagatt
34441 acatggtggg ggaatagagt atgaaagact ggattgagag aataccagaa aagagggttt
34501 gatgataagg atggctatac cttggggaga acttagtgga gttgaagatc agccagatcc
34561 ctctctgaca ctgtttcctc ctgctattag cccgggtaac tcagctgatc cgtggctttg
34621 gtagttcctg gaaatcatca gtggaatctc tgagtcagga tgtaatgcgg agtttcacca
34681 acttcagaaa tggcaccagt atcattcagg tgacctgcaa gtcccaggcc ccactcagat
34741 cccccatcat taattatttt ccccatcttt ttgctgggct cagcatcatc taagtgaccc
34801 ttggtcttca gttactagct gtgcccaaag ttctgtatga gccctaacct gattcttttt
34861 tttttgtttg ttttgttttt tttttgagac agagtctagc tctgttgtcc aggctggagt
34921 gcagtggcac agtctcggct cactgcaacc tctgcctccc aggttcaagt gattttcctg
34981 cctcagcctc ccaagtagct gggattacag gtgcctgcca ccacaaccag ctaattttcg
35041 tatttgtagt agagatgggg tttcactgtg ttggccaggc tggtctcgaa ttcctgacct
35101 cgtgatctgc ccgccatggc ctcccacagt gctgggatta caagtgtgag ccactgcgcc
35161 tggccgccta acctgattct taatcatcac tattagcacc attttacggt ttgatccctc
35221 aatgactttc tttgaccagg gagcgctgac ccagctgatc cagctctatc atcgcttcca
35281 ccgggtgctg tcccagccgc agctccgagc cctccctgcc cgggctgagc tcatcaacat
35341 tcaccacctt atggtggagc tcaagaagca taagcccaac ttctgatgtg ccagaaaccg
35401 ccctgagatc tgccggtcat ctccatggac ttctgcaccc cattccatac ccttcttcac
35461 ctggggtacc ccttccagtt ttccccttgc ttcccaggcc cttgacatgg cttacctgcc
35521 ttcactccca gcaccttgcc caacaggata agctggatcc ccttggcctt ctgaatatcc
35581 cagtgtcttc aggtttccca agaccacttc cctgtgggct tccaaaatgg cctttatcat
35641 ttctccagtc tgtcaccctc ctttcctgct cccatacacc caaggcttgt ttcttcccct
35701 gtaaaaacca ctgcctcaat ctctggttca ctcaactagt caccatgtcc tgaggcatga
35761 agcctcctca gctcttggaa ttgctggcaa ggggtgactg cctctgagtc attgtgtttt
35821 tcaaagtgat ttcttttctg tagctttttg acctaagatc tcagcaattt gaacactaac
35881 ctctcccctc ctggctcaag aattactccg aagtcagtct gcagaaaata aatatttagt
35941 atgacatgac acttatccca tttcctttct ccttcctcct gaaggttttt caggtggccc
36001 catctctaga atgacctttc ctttctgact acttcctggg cttacatccc ttctggtggg
36061 tgtaacctct aacctctagc tactcccagg ccattggagg aaggtggccc ctggctaaaa
36121 tagcagatgc tgcaagggat gagaaccagg ccgcgcagcc ccagacactg tcccaagggc
36181 tcccttggga caagcccaag ctgactctgg aatccctcct ccgtagcagc tccatagctc
36241 ctctggttgt ggctgtgaca gccccttgct cgggggctgc agcacccaac ccacacccac
36301 accgttgggg cagggctaga ccaggcgaaa ttcgtaccat ccgtttgttt gacttggagc
36361 cttcctgctg tcctcgccta tacgcctcgt ttgaacttag gctctagact ggagagacac
36421 ggggacccct ttaaggccta agaagagagg cgcagttaag gaaaaatgtt acgtttcctg
36481 tgtcctccac ccctacggcc taaacattcc ctccccaggt ccctggagag tggtggaaag
36541 cggttcctcc ccgcttaggc ccttcggatg caggtctagc ccgtcggcaa cgggaggtgt
36601 cctgagtggg tctgtgactg ccgagcacac ccgccgcgga gcggaggctg ctgcttcctg
36661 aggctgagag tggatccggc tccgggctct cctaattggc ggacgctggg gggcggcgtg
36721 gtaaggcaga acggagcggc atctcagctc tcgccttttc agggttccgc cccatatccg
36781 agagccgctc tctaattggc ttgggaaacc gtatctcagc gctttggcct agcgactttg
36841 aacgtgtttg cgcctgagac cgaagtgcag aagaggcgga gcgcaggagg agaggcttgg
36901 tgaatcagcg attcctgatt ggccaggcgt gccttgaggg cggggccaga actgcgtcct
36961 taactgacct cgcccttgcc cagatcaccg cctccgcgtt gctccgggtt taccccgcct
37021 gactcgctgc gctatgcgtt ccctcacgcc tgccggatgc caggcggcga tgtgccaggc
37081 tctgaggggc cgcgagctca ccccagacgc cggccccggg aatcctctgc tcctccactt
37141 tcccttcccg ctactggttt cttgcccacc cactcccagg tgtcactctt gggacatcca
37201 gatgttctga catttgacct gactccagcc tcagcgcgag gacgggagaa gggccaaggg
37261 tatgagattt tggacaggag aggcattggc tactctgaca aagagcgtgg attcccaaag
37321 aaagggtccc cagatacccc gcaggggaga ctgtcgagac aggcgacttt agccaactaa
37381 tgctcgcacg cgaggagggc tgtgcaggca ggcaccacgg ttcttcacag ccttccttct
37441 ttccttcccc tgtccgtcgc agaacccaaa tcctcagagc tgatcgagaa gcgcgtgttg
37501 ttgcagaagt cactgagggg cagacctgga atgtgagccg tggggcgaag ggacagctct
37561 ggagactcgt gattccggaa cccggtgggc attcaagtga tactggagca cgcagttttc

37621 ggggacatta agctcgcccc aggggtccac aggaaccctc tgacccaacg gtctctctgg
37681 acaggatgat ccagaacaga cggtgaagag acaaaacagc gtgaaaggga gcggcgatac
37741 ctaaactgac ctccggaggg cagcatgatc aagggaagga gctgttctcg ctccgctcaa
37801 acccccgcgc agcctctgca gcttactcct gcttcggaag gcggcgaggt tccaccccca
37861 ccaccccggt ccccgccgcc ctcttcgcgc tgaagctgcg gagggtcttt ttcttcagcc
37921 cccaaatcct ctgctctgtg gcttaagatt cccaggctta aacccataac tgctagggtc
37981 atcactcctc aaacttctcc ctcacttgtt ttgctgatga cggacacagg ggcctcacag
38041 actcagaagc atctggagtc atttcgaagg acagaactgt ggcaatatcc tctttccccc
38101 tcctaatctc tgagaacagt gtctgtatat ggcacggggg tccctagtgt catatagaag
38161 gactcggaga tgtcctcatg ggctgtaacg gcccccggtg ctattcaggg atctctgttc
38221 tttaaagaaa tatcatttcc ccttatttct ctacctttgt gccctcacct cgccctgaat
38281 tgtttcttaa actgggcttt ctgggaaacg tttacttcgt gttcaacagc aaagtctcgg
38341 cataaggcga gggcggcagg gggtgggggg cgggcgtttg ctcgccttgg cattaatttc
38401 agagctgctg aacgtggaca aaagagaggg aacatcctcc tttcttccat tcctgtaaat
38461 atggacccac ccaccctcca cttaagatgg gtggcttttc tgattttaag atatcaagca
38521 gcctggcgag gtggctgaca cctgggatcc cagaactttg ggaagccgag gcgggaagat
38581 cccttaaggc caggagttct agaccagcct ggccaatatg gcgaaaaccc atctctacta
38641 aaaatacaaa aattagcctg gcgtggtggt gcctgcctat aatcccagct actagggtgg
38701 ctgagtcagg agagtcgctt gaacctggga ggcggaggtt gtaatgagcc gagatggcca
38761 ctgcactcca gcctgggtga cagtgagact ctgtctcaaa aataataata ataataataa
38821 taaaaagata taaattggct gggctttaaa ataaagtagt cagaggccca gggtggtgac
38881 tcatgcctgt aatccttttg gaaggcagag gccagaggat cttgagcaca ggagtttgag
38941 actagcctgg gcaacatagg gagacccatc tctaccaaaa aaaaaaaaaa aaaaaagcaa
39001 aaagccaagc atggtggccc atgcctgtgg tcccagctag ttgtgaggct gaggcaggag
39061 gattgcttga gaacaggagt tagagattgc agtgagctat gattgcacta ctgcactcca
39121 gcctgggtga cagagtgaga ccctatctct ttttaaaaaa ttaaaaaaca aagtagattt
39181 agtgtgaaga ttcaaggaaa gaaagagaaa gcaagacaaa actttaagga gaggagaatc
39241 gaaattggct gacttcaccc tggacataat ggacacagct attcatgtta cgtactagcg
39301 ttaacaaagt gttttcagaa gcagttgtca ttaaaccaac acatttattg aagctcttct
39361 ctgtgttctg tgataggcac tgcggattca gcagagacta aatcaaagtc cttgatctcg
39421 gagagcacat atttcatcta attgaattca cataccagcc ttatgaggag gtgttcttcc
39481 tgtttcaggt aatgagcttg aatttcaaat aatgagttca aaaccatata ggcactcagt
39541 ggctgaacct tctgtgccca cattcagctt ttgttctatg actccaggct gggagtcatg
39601 gagctgagga ggcagccggt tgtagagaag caacagatta ataagtaata gtaaattatt
39661 gaaaaaatat ttacagaact gaaggaaaag ctgaacaggc ggtctttgga aaggacagaa
39721 gccaggatag ttccagcatc caggcagcag acaccaatgg tcagtgcttc tactttggat
39781 caaaaagaga gaactccaat aggcttaggg tgggtcaggt gctcacactc atctgaggaa
39841 ggggagggtc ccttgaggaa tagtaccata gactacccaa tgttggagga taatggcccc
39901 aacgcaaaaa tgggatcttg ccaccagaca aatgggatat agatgccagg ctgcaaaatc
39961 caacaaatgt acacgtgccc aaatcccgac tccatcacta atagccatgc aatcctctga
40021 ctatcaatta gattaattga cactcttttt tctctcgcta ctgtggtaca tgcagtagtt
40081 acctttattc ttcactttgt tttctcataa gactaacaag cagtttttta aaataaagaa
40141 ataaaagcaa aattgcttat tgtcttagtc tattcattgg caaactagta acattaaata
40201 aactccagac agagccactg gagaagaatc aagtacaact tagtagaaat aacaccgtat
40261 gtgtgaaggt gagtgtgtgt gtgtgtgtgt gtgtgtgtgt gtgtgtgtgt gtgatggaaa
40321 atactttgtc ctgcccaaac tgtaagtcat gaccatgccc ctctcagcag aagtttatct
40381 tcatctcatt tggaagttta taagaacaca gcttaaaaaa aacctgtgat tttggagcaa
40441 ggtcaagcct ttgctattgc ttccacattt tgttttgttt tatttattta tttacttttc
40501 agttttttgg agttttcttt atttgtttgt gtttttgaga cagggtctcg ctctgttgcc
40561 caggctggag tgcagaggca ccatctcggc tcactgcaac ctctgcctcc cgggttcaag
40621 cgattcccct ggcttagcct actgagtagc tgggattact ggcacgcacc accacatcca
40681 gctaattttt gtattttcag tagacactga aatacatgtt tgccatgttg gctgggctgg
40741 tctcaaactc ctggcctcat gtgatctgcc tgcctcggcc tcccaaagtg ctgggattac
40801 aggcatgagc caccacgcct gcctagtttt tagttttttg agacagagtc ttgctcagtt
40861 acccaggctg gaatgcagtg gcatgatcac agtttactgc agcctcgacc tcatggattt
40921 aagcaatcct cccacctcag cctctgggat agctgggact acagtcatgt gccaccacac
40981 ctggctaatt tttgtatttt ttggtaaacg aggtttcacc atgttgccca ggctggtctt
41041 gaactcctga gctcaagtga tctcctacct cggcctccca aagtgttgga attacaggca
41101 tgagccactg ccccagctgt tttattaatt tagttaaaaa tatatgaaaa gaggccctgc
41161 gcggtggctc acgcctgtaa tcccagcact ttgggaggct gaggtgggcg gatcacctgc
41221 ggttttggag ttcaagacca gcctgaccaa catggagaaa ccccatctct actaaaaata
41281 taaaaaatca gctaggcatg gtggcacata cctgtaatct cagctactcg agaggctgag
41341 gcaggagaat tgccctaacc cagaggaggt tgtggtgagc caagattgcg ccattgcact

41401 ccagcctggg caacaagagc gaaactctgt ctcaaaaaaa aaagtatata tatatatata
41461 tatatatgaa aaggctgggt gcggtggctc acgcctgtaa tcctaacact ttgggaggct
41521 gaggcaggtg gatcacctga ggtcaggaat ttgagaccag cctggccaac atggcaaaac
41581 cgtgtctcta ctaaaaataa aaaaattagc caggcatggt ggcaggcgcc tgtaatccca
41641 actacttggg aggctgaggc aggtggatca cctgaggtca ggaatttgag accagcctgg
41701 ccaacatggc aaaaccctgt ccctactaaa aatacaaaaa ttagccaggc atggtggcag
41761 gtgcctgtaa tcccaactac ttgggaggct gaggcaggag aattgcttga actcaggggg
41821 cagaagttgg cagtgagcca agatcacacc atttcactcc agtctgggca aaagagcaag
41881 attctgtctc aaaaaaaaaa aaaaaaaaaa aaaaaaaaat atatatatat atatatatat
41941 atatatatat atatatatat atatatatat acatacatac acacacacat acacacatac
42001 aggcactagc caggcgtggt ggctcatgcc tgtaatccca gcactttggg aggccgaggc
42061 gggcagatca caaaacaggc agggcatggt ggtccacgcc tgtaatccta gcactttggg
42121 aggctgaggt gggcagatca cttgagcaca ggagttcgag actagcctga gcaacatggc
42181 gaaactctgt ctctaccaaa aatacaaaaa ttagctgggt gtggtggtgc atgccagtaa
42241 tcccagctac tccggaggct gagaggcagg aggattgctt ggggaggcag aggttgcagt
42301 gagccgagat ggcaccacta gactccagcc caggtgacag agggagaccc tgtctcaaaa
42361 aaaaaaaaaa aaaagaaaga aaagaagaaa agaaaaacaa atcacctgcc ttatgaagaa
42421 acagtttgta atagatataa gacacagaat gtgcagcccc tggtatggaa taagtcctta
42481 gaaaatgtta gtttcctctc tgtccctgtg tgacatgaag ggaattccag ggccagtgcc
42541 acttcctggt ggtttccctc acttaagtgc tgtgtacccc attgcaaaag catgtcttcc
42601 aggttcctga taccacatcc catgtcctct gcctttgggg ctctcatagg attaagtgtc
42661 attttcccta tgaactagaa aagaatcttc tgaacgtctg ccttgggact gatgatatag
42721 ctgagctgaa aggacaatta gttttcctcc ccagaccaac ccaaccttca aggctgggga
42781 ctcatttgat gctcccattc aaacatttgg agaaaattgt ctctgagtac caggtctttt
42841 tagcatattt tctccaaatg ttacatcctt aggggctttg aatagtagaa gagccttcaa
42901 cattggttca gatggaagca tgcaggcatg tggagagttg ccactccctg atccctgaaa
42961 caaggcttag gatggaaaag agcctcctgg ggaggttggc tctgaatgta aagctgggag
43021 aggcacctcc tgtcaaccta agggcttgtt ggtaggggca acatgttgac ctggcttcct
43081 tcccactctg agacatcact ggctctcatg cagagctatt ttccttcttt ctttctttct
43141 ttctttcttt ctttctttct tctttctttc tttctttctt tctttctttc tttctttctt
43201 tctttctttc ttttcttttc ttttctttct ttcttttttt ttttgagacg gagtttcact
43261 cttgttgccc aggctagagt gcaacggcac gatctcagct caccacaact gccacctccc
43321 tgggccaagc aattctcgtg cctcagcctc ccgagtagct gggattacag gcatgtgcca
43381 ccatgcccag ctaattttgt atttttagta gagatggggt ttctccttgt tggtcaggct
43441 cgtctcaaac tcccaacctc aggtgatcca cctgccttgg cctcccaaag tgctgggatt
43501 acaggagtaa gctgccgtgc ctggcctttc attcttttct ttccttcttt ccttctttcc
43561 ttccttcctt ctttccttct ttccttcctt ccttcctccc tccttccctc cctccctcct
43621 ttcttctttc ttctctttct ttctccttcc ttccttctct ctctcttttc ttttctttct
43681 tctttctggg tttcgctcta ttgccgaggc tggagtgcag tgagtggcac gatcacggct
43741 cactgtagcc tcaacttcct aggctccagc gatcctccta cctcagcctc cttagtagct
43801 gggattgcag gcatgagcca ctgtgcccag tgtaatgcag ggctcttcat ccaggatccc
43861 aaagggcaaa caaaaatagg agaaaattac atttcatttt cactaatctc taactgaaat
43921 ttagcatttt ctccaatgat aaatgtaggc aataaaccta caccggtatt tgtagtacat
43981 gtgactgttg ttaatagaat ttacacattt gtgtcatctt ccagtagttg cagaaatttc
44041 aaaatgtcat gtatgctcac ggttactttg tagttagtgg tagtggttag actcaccact
44101 agatcttctt cttacacatt ttgtttcatt ttgacaactg tacttcaata tgattgtttt
44161 cctttgtaat cccatttatt ttactttatg cagttgaaga ttattctgac actccacaga
44221 cttcaccaga cttccaaaac ctgtcctaga cgtaaaaagt taagaaacct taaatctcta
44281 atgatgctgt catttcctgt tttgtattat cttacaacca gggtaattag gaatgccttc
44341 ttcttcttct tttttttttt tttttttgaa gacggagtct tgccctgtca cccaggctgg
44401 agtgcagtgg cacaatcttg gctcactgca acctctgcct cccgggttca agtgattctc
44461 ctgcctcagc ctcccaagta ggtgggattg caggcatcca ccaccatgcc cagctatttt
44521 tttgtatatt tagtagagat ggggtttcac catgttggcc aggctggtct tgaactcctg
44581 acatcaggtg atccacccac ctcagcctcc caaagtgctg ggattacagg catgagccac
44641 cgtgcctggc ttgctttctt cttgtttaaa ctaaaggtct tcttgtaaaa ttcctgctta
44701 tattttcttc attattactc aaattcaatt attcatttat ttgccaaata tttagaaaac
44761 acttctcttg tacaaaacca tgctgcataa aactgttatt atgcgaaaaa taatccttac
44821 tcgccatgcc ctctaccccg accccccaac cccatttaca atctggtaga gctggactgt
44881 ttacccacat aggacacaca ggctgcagct taccatgggc agggagtggg gatggttgtt
44941 ccaggtgcag gtaataagaa ggtgtattgt ctgtggagaa tttaaaaata attctaaaac
45001 caactttttt tttttttttt tttttgagat ggagtctgac tctttggccc aggctggagt
45061 gcagtggcac catcttggct cactgcagcc tctgcctccc gggttccagt gattctcctg
45121 cctcagcctc ccaggtagct ggcattacag gcacacacca ccacgccagg ctaattttg

45181 tgtttttagt agagacgggg ttttaccacg ttggccaggc tggtcttgaa ctcctgacct
45241 caggtgatct gcccacctca ccctcccaaa gtgctaggat tacaggtgtg agccactgcg
45301 cccagcctga agccaacttt ttattgatac tgcgcaccag caattataaa aaatgttag
45361 tgacaaaata ccccttgcag tgtagcctcc tccccccact ccaccctgct tggtatgcca
45421 ctgcacacag gggagtttaa ccatttccag tttacacaca cacacacaga cacacacaca
45481 cacacacaca cacacacaca cacaaacaca cagagccttt gagacttcct tttctcctca
45541 actcccccag cccagttctc atgtcactct actcagggta ggatctgcag cactgctggg
45601 ccagtatccc caaaggtgag ccctgtaagc tccttggcct ctggatggtc tcattaatag
45661 tccaggatga gccctgcaat cttggcatct gtaggcaggt ctcagctctt cttcacaccc
45721 ctgggcttga accccttggc aactgcgttt gacagtacat gattcccatt cttcatctcc
45781 agattttcca tccttcaaga aaacactgga tttcctggtc aagccaaggc ttaatgaact
45841 ggcctggcag cttctctggt cctcaagttt ccttttcttg ccttactttc tgaagacttc
45901 tagtagtttt acttggaagt gtcactgtct ccatttttat tcctgagttt tctgcagttc
45961 ttttttctgt ggccccacgg actcatccag agcctgcagg gagctgacac ctcatctgag
46021 ctgctagaat tgaggtgaga aaggaggttc ctaagtgcag aaatgtgttt tctcctctcc
46081 agccaccact gaaggctttg gatgtgattg agtcagacct ctcctgcaca gtctcccttt
46141 ggattacctc aagataaacc aatttgtgac tttaattaca tccgcgaagt cccttcccag
46201 ctgcacctaa gctaaagtgt gattgaatga atgaaagagg gtgtgctggc gatcagctag
46261 ggaagtgatt ccaagggtcc ctagactcca cattaccaac tgcaaaatgt gacgttctta
46321 cgttctgaca agcgaactct cctcccaaat tcattgcctg agttgatgca aatgaattca
46381 gccacgcctt ttgggtttgg catctgagaa gtgtctgtga cttgggaaga gtggtcagaa
46441 tggcgataat gggaagagac cacatttcag ctggtggaga gtgaaaggac agccaacatc
46501 ttgccctttc tgaagcatac acacattgga gattgaaagt gtctgttggc ctcacaggca
46561 ctgaggaaag cctcccatcc ccttagattt tcctcctttt tcctgaggtc ccctgaacct
46621 aatagtctgt aaccttattc catgtctggg tcaacaaacc accttccaag gaggacttga
46681 atataataat ccgtttcctc tgcattcctc cagaacacag agtccacacc atccatgagt
46741 ctgtcaaaca aaacattcct cattggtctc aatgttgagc actgaaagtt ctctgagctc
46801 atttagcacc aactcatcat ccttaaaatc cacaggatgg gccgggtgca gtgacccatg
46861 cctgtgacta caacaccttg ggaggccaag gggcagatct cttgagccca ggagttcaag
46921 accagcctgg gaaacatggt gagacacctt ctctacaaaa aaaaattttt tttttgaga
46981 cggaatctca ctctgtcgcc caggctggag tgcagtggtg caatctcggc tcgctgcaac
47041 ctccgcctcc agggttcaag cgactctctg cctcagctcc cgagtagctg ggattgcagg
47101 catccaccac catgcccagc taatttttgt atttttagta gagacagggt tttaccacgt
47161 tggccaggct ggtctcgaac tcctgacctc gtgatctacc cgcctcagcc tcccaaagtg
47221 ctgggattac aggtgtgagc caccatgccc ggcctacaaa aattttttta aaaaaattag
47281 ccaggaatgg tggtgtacac ctgtggtccc agctacttgg gaagctgaga taggaggatt
47341 ccttgagccc aggaggttga ggctgcacag tgagctgtga tcacgccact gtactccagc
47401 ctgagcaata gtgagacctt atctcagaaa acaaacaaaa caaccaaaaa tatccatagg
47461 atgaaacata tgtttgatta tgacaacttg cttatggtac cttctggatg gcccagctcc
47521 cttgcagatc cccagaccaa cagcttttgt tgtagtgaca gcttcttctt gtaacctctt
47581 tttttttttt tttgagatgg aatttcactc ttgttgccca ggctggagtg caatgacatg
47641 atctcggctc actgcagcct ctgcctccag ggttcaagcg attctgctgc ctcagcctcc
47701 tgagtagctg gaactacagg tatgcaccac catgcccggc taatgtttgt atttttaata
47761 gggacggtgt tttgccatgt tggccaggct ggtctcgaac tcctggcctc aggtgatcca
47821 cctgcctcag cttcccaaag tgcttggatt acaggcgtga gccactgcac ctggcctaca
47881 gcctctaatt tcattttcat ccaaaagctt taatggaagg cccacagatt ccctcttcaa
47941 gtaacagcaa agcgcatctc ctttaagatt tctttgatca tcttcataaa gcttgacttt
48001 gaagtcttct gaggatttct cctggaggca aaccagatat atagacattt gtatttctgt
48061 cttcttcaac atgaaattat cctcattcca tttttctttt atctcccctg tttttaggat
48121 cagtgggttt ggggggtttt ctttgcagaa attctgcatt cctagcattg acattttgta
48181 catttgcagg agagctagat gccccaccat tagaaaagcc acacttggct gggtatgtag
48241 caatgaaacc ttcagtggcc ttgggaaacc acgccttctt gtccagatcc cactcattgt
48301 gggagtcagt tggctgctgc cctaaataac ccgtttgtca ctgagatctt cttgtctgtt
48361 gcagtcctta gtgtctccat ataatttatg cattcacaac tgctcatcaa gtcatcatta
48421 tcatccactt catgtttcct acctagccag gggcagggtt caggtaatca aagaggctgc
48481 tctgctgggc ctgcaaggct ccaccaagtg tgtctgaggt gacagcccaa gcactgttat
48541 aaatatttgc gcactgttgt aaacatttgc attagtccct tctcgcactg ctataaagaa
48601 atacctgaaa ctgggtaact tataaagaaa agaggttgcc gggcgcggtg gctcatgcct
48661 gtagtcccag cactttgggt ggctgaggca ggtggatcac ttgaggtcag gagtttgaga
48721 ccagcctggc caatatggtg aaaccccgtt tctactaaga atacaaaaat tagccgggca
48781 tggtggccag cacctgtaat cccagctact tgggaggctg aagcaggaga atcacttgat
48841 ccggggaggc agaggttgca gtgagccaag atcatgccac tgcactccag cctgggtgac
48901 agagcaagac tccgtctcgg aaaaaaagaa aagaaaagat gtttgctggg cacagtggct

48961 catgcttgta atcctagcag cactttggga ggctgaggcc ggagggttaa tcacttgagc
49021 tcaagagttt gagaccagcc tgggcaacat ggcaaaaacc cgtctctata aaaaattcaa
49081 aacttagctg ggtatggtgg catgtgcttg tggttctagc tacttgggga gctgaggtgg
49141 gagaattgct tcagaccaga aggtagaggc tacagtgagc tgtgattgcg ccactgcctg
49201 ggcgacagag caagacccta tctcaaaaaa aaaaagaggt ttaattggct catggttctg
49261 tgggctctac aggaagcatg atgctggcat ctgcttgcct tctggggagg tctccagaaa
49321 cttacaatta tggcagaagg caaaggagga gcgagccttc tcacatggca ggagccgaga
49381 gagagggagg aggtgcggca cacttttaaa caaccagagc ttgccagaac tctctatcat
49441 gagaacagca ccaggaggat ggtgctaaac cattcgtgag aatccactcc cataatccaa
49501 ccacctctca ccaggctcca cctccaacac tgggactaca tgagatttgg tggggacaca
49561 gatccaaacc atatcaacat ttaacacatt gcatctttga attgtcacag caaccctgtg
49621 aggtgggtac tagtgttatc cccattttac agagaggaaa ttgatgccca gagaccttgc
49681 caaggttata gacaggacag ttagtgcatg acaaagctgg aattggaatg cagccagttc
49741 tgctttagaa gccactttct tgcctgcctt tctttcttta tttctttatc ttttctttct
49801 ttctctctct ctccttcctt ccttccttcc ttttctttct tttcttttct tttttttttt
49861 tttttttgag acagggtctg gctttgttgc tcaggctgga gtgtggtggc accatcttgg
49921 ctcactgcaa cctccacctt ctgggctcaa gtcatcctcc tacctaagct tcccaagtag
49981 ctgggattac aggtgcatac caccacacct ggctaatgtt tgtatttttt ttagtagaga
50041 tggggtttca ccatgtttgc taggctggtc ttgtactcct ggcctcccaa agtgctggga
50101 ttagaggcgt gagctattgc accccgcctg aagctacatt cttttttttt tttttttttt
50161 aatgagacga gtcttgctct gtcccccagg ctggagtggt gcagtggcgt gatcttggct
50221 cactggaacc tccgcctccc gggttcaagc aattctcctg cctcagcctc tcgagtagct
50281 gggattacag gcacctgcca ccactcctgg ctgttttttc catttttaat agagacgggg
50341 cttcaccata ttggccatgc tggtctcaaa ctcctgacct caagcgatcc gcctgccttg
50401 gccttccaaa gtgctgggat acaggcataa gccatcgtgc ctggccaagg ctacattctt
50461 aaacgccact ttttacagct tccaagaaac taactgggtg aaatccagcc tgcctcctgt
50521 ccttgttttt atcatttcat agtgaaaaca taaactccca agcccaccta ctctattgca
50581 ctgtgtctcc atgacactct gtaccttctg gctctgactc agccctccac tttccccctc
50641 cctaacctgc ctgtgccctc taataaactg tagcccccct cataccctta gcttctcctt
50701 ggagcattcc ctccacttcc tggcgttaag tgaaatttaa ctctcccttg agggcactac
50761 ctcctttgca ggcttctgaa atgagagctg ttgattttct tacagctgag ggtgctctcc
50821 tggcttcaga ttgctgcctc cggactactc ctcccccagc ccccaactct agtttaaaat
50881 aacccgcggc cttccaggca ctgcaattag gctaaacttt cctcttcctt tctcactgct
50941 actgtctgcc agacccctcc acctcactca ggggaacgct ggcccagctg accgtctgtc
51001 tttgcattga gtcctccctc tgggagactt cattgtccac ctggaggatc cattccctac
51061 ttgacctctc aataacctgg ccacttaatc cccaagaatc ttctcctctc caactcggcc
51121 acacattctc attgtcatac cctggacctt atcacctgaa agagcccctc cttgagaatc
51181 agtcaagcag cctgctcttg gcccccacct cctaaccgtc tgtccatcag tctgtctgcc
51241 ctcctcgcaa ccaattttca gtctcctagg acctttggat caatgatgcc ttccgtcttc
51301 cagcttatga gcccctccct attcagctca actccacaac ccttcatttc aacacctctc
51361 tggacaatgt cgccaactcc ccttcccctc tgtattttca tcctttgtac ccaagtggca
51421 aaaccccaac cctggatgag cccagtgatc tgttttcttc aggattttac tctgatagcc
51481 aagaactgtt ggtggtggtg ggggaaatca cacaatcagg aaatggttcc cactcctcac
51541 tcatgatcat gaactctaag gaggctccca gaattgttta gtggcccaac cacatcactc
51601 tggtcaatgg ctacgttaaa cctccctgat ccttgaacct ctgaggctct tctgtctctc
51661 acctcatgct cacttgatga cctccctctt atgtcacaga gaaaatgaaa gtcatcagag
51721 gtgagctccc tcaaccatct gtcaccagat ccacacccct ccaagaggaa cacaatgcat
51781 gcagcgcatg taccttgatt tgatcctgat ttgaaatata tgcataggct ggatgcagtg
51841 gctcacgcct gtaattccag cactttgctt gagctcagga gttcaaggca ggtggattac
51901 ttaagctcag gagttcaaga cccacctggg caacagagaa aaaccctgtc tctacaaaaa
51961 aaaaaaaata caaaaattag tcaggtgtgg tgggtcacac ctaaatagta gttccagcta
52021 cttaggaggc tgaggtggga ggatcacttg agcctgggag gtggaggttg tagtgagctg
52081 tgattgcact gctgcactct agcctgggtg acagtgagac ccagtcaaaa aagaaagaag
52141 gaaaggaagg aaggaaaggg aggagagaga gaaaggaagg aaggaaggaa ggaaaaacag
52201 aagaaaggaa ggaaagaaat taaaaagaaa gatatatata tatataaata tatatataaa
52261 agacattttg aggctaactg gggaatattt gagtatattc tgctatcaga tgaagtgaag
52321 aaatgatcgt tattttctaa ttttgttttt ttttttgaga ctgagtcttg ctctgtcacc
52381 aggctggagg tcagtggcgc aatctcagct cactgcaacc tgtccctact gggttcaagc
52441 aattctcctg cctcagcctc ccaagtagct gggattatag gtatgcacca ccacgcccgg
52501 ctaatttttg tatttttagt agagacgggg tttcaccata ttggccagga tggtctcaat
52561 ctgttgacct tgtgatcctc ctgcctcagc ctcccaaaat gctgggatta caggtgtgag
52621 ccaccgtgcc tggccatgat cattattttc ttagctgtga ttacgtattg tggttatgga
52681 atctccttat ttttgggaga tgcatgctga ggtacttagg tgtgaagcac atgctttcct

52741 atattttcat atggtccaag aatgttttag tagtcactca acaatttcta tgtttaaaaa
52801 aaaaaaaaag ggctgggcac agtggctcat gcccgtaatc aatccgagca ctttgggagg
52861 ctgaggcagg cggatcacct gaggtcggaa gttcaagacc agcctgacca acatggagaa
52921 accccatcta tagtaaaaat acaaaattag cttggcgtgg tggcgcatgc ctgtaatcac
52981 agctactcgg gagtctgagg caggagaatt gcttgaaccc agtaggcgga ggttgcgtac
53041 agccgagatc gcgccattgc actccagctt gggcaacaag agcgaaattc catctcaaaa
53101 caaaaaacta aaaacaagcc cacaaagacg tatataggcc ttggcactgt ggctcatgtc
53161 tgtaatccca gaactttggg aggccaaagc aagaggatca cctgagtcca gtttgagacc
53221 agctgggcaa caaggcgata ccttgtctct acaaaaaatt taaaaattag ccgggcatgg
53281 tgccatgcac ctgtggcccc agttacttga gaggctgagg tgggaggatc gcttgagccc
53341 aaaagtttga agctgagatg agctatgatc aggtcactgc actccaccct gtgcgatgaa
53401 gtgagaccct gtcttggaaa aaaaaaaaaa gaaccatata tatatgtgtg tgtatatata
53461 tatgtttgta tatatataat atataaatat ttgtatatat gtataaataa caattgttaa
53521 atctaagtaa aatgatcgac atacaaacaa tattgtatta tattttcaag ttttctgtat
53581 gtttgaaatt tttcataata aaaaaactgg gcaaaattct ctcacttcca ctcactgctc
53641 gaccctttca ggctggtgtt tatctctacc ttgctgctac tcaaatgttt ttttgcaatg
53701 aactttatgt cattaagtcc attgtttcgt tttcttctta cttgaactgc cactagtagc
53761 tgacgttgct ggccagttct ttcttgaaac actgtcgttt tttggcctcc aggatttggc
53821 ctcttctggc ttcctcccat ccctccatcg gctgctcttc cacccctcag ccggctcact
53881 ccgcctttga ctgaaatcac ctggtcatat tggagttcat caagagaggt cggtcctgga
53941 ccctcctctt cttatgctac cctttccttc taggggatca cacctgtcct gtggcttcag
54001 ttcccatcag ctggttactc tcaaatttat ctcttgcctc aaatctccag atttgaccac
54061 cttcctgcct ggcatcccta cttggatgtc ccaaaggtat tactaactta gctttttttt
54121 tttttttttt gggagacaga gtttcgctct tgttggccag gctggggtgc agtggcaaga
54181 ccttggctca ctgcaacctc cgcctcctgg gttcaagtga ttctcctacc tcagcctccg
54241 gagtagctgg gattacaggc atccgccacg acacccggca aactttttgt attttcagta
54301 gagatggggt ttcaccatgt tggccaggct ggtctcaaac tcctgacctc aggtgatcca
54361 cctgcctcag cttcccaaag tgctgggatt acaggcatga gccacggtgc ccagcttaac
54421 ttagcatatt tgaaacagaa gtcatgatct tcccacagaa gcagtatcat cctcagatct
54481 gaaccacagg ggccctgccc tggcccacat actttagggg gattcaccta tcacaaacaa
54541 cttatcacac acatttatta catgacacct gggcacctct gtcactgatc tgctgtcagt
54601 gctggaacag cacaacctga aatcttacac atctgctctt gtaatcaacg ccgttcaagc
54661 cctggggaat ggtgacttct gtaatctctt gccctgtatc ctggaaacaa aagatgacta
54721 tagccaattc tgtgaaaatc tgaggttgtg cctctgcctg gattctgaag catgtactgc
54781 tttggagtgt agggaggatt taagcagtgg aaacatgtaa gtgaaatcac tttaaccagc
54841 ttgttaaata ttacctctca aatagcatga caaagtatca tttcccaata gtgttgagtg
54901 tggtttttct tgtttctctt tgttttagag gtatggatgt atgttgtgct gcatttacaa
54961 cagttgcaaa ggatagctga ggaacatgtt gctgtattaa acaatcctta ttactgttaa
55021 atttatatgc atgtggtggg aggggtggtg agggataaaa aactacacat ttacagtgta
55081 cactcctcct gtgatggggg caccaaaatc tcagaaatca ccactaaata acttattcat
55141 gtaactgaac accacctgtt ccccaaaagc ctattgaaaa aaaaattgaa tacatgtagg
55201 tctagatata actcttgtga agtatgatat tgattcttta tgttgtaata gatggacatt
55261 gaatacgaga ttatgaatac aatcatcctc agtattcacc agggattgat tccaaaaccc
55321 ccactgatgt tcaaacccct tgtataacat ggcatagtat ttgcatataa cctatgcaca
55381 ttcttccaca tactttaaat catccctagg ttatctatgg tgcctatcgc aatgtaaatg
55441 ctgtataaat atatgttata ctgtattttt tatttgtatt attttttctt tctttccttc
55501 cttccttccc ttcccttcct tccttccttc cttccttcct tccttccttt ctttctttct
55561 gacagtcttg ctgtgtcacc caggctggag tgctatggca ggatatcagc tcactgcaaa
55621 ctctgcctcc caggttcacg ccattctcct acctcagcct tccaagtagc tgggattaca
55681 ggtgcccgcc accatgcccg gctaattttt ttgcattttt agtagagatg ggtttcacc
55741 gcgttagcca ggatggtctt gatctcctga ctttgtgatc cacccgcctt gacctcccaa
55801 agtgctggga ttacaggcgt gagccaccgc gcccggactg gccatatata ttttaaaaat
55861 atttttattc ttcagttggt tgaatccata ggtgtggacc tacagataca aaatgccaac
55921 tgtagtttaa tttttataaa actatctgtc ttagtccatt cagactgcta taacaaaata
55981 ccttacactg agcaatttat aaataataaa cactgcttac agttctggag gctagaatgt
56041 ccaagcaggt ggtgccagca gatttggtag ccagtgaggg cacactttgc ttcatagatg
56101 gcgccttctt gctgcatctt cacgggtgga aggggcaaac aagctccttt gggcctcttt
56161 tttttaagac agggtctcgc tgtgtcacct aggctagagt gcagtggcgt gatctcggtt
56221 tactgcaagc tctgcctcca gggttcaagt gattctcttg cctcagcctc ctgaatagct
56281 gggattacaa atgcctgcca ccatgactgg ctaatttttg tgtttttagt agagatgggg
56341 tttctccatg ttgaccaggc ttgtcttgaa ctaccgacct caaatgatta gcccgcctga
56401 gcctcccaaa gtgctaggat tacaggcatg agccaccgca cccagcctgg gcttctttta
56461 taacggcatt aatcccattc atgaggacag gcccctcatg acctaatcac cttctaaagg

56521 ccccatctct taatatcacc acattgggga ttaggtttca atttatgaat tttagggggg
56581 cacaaacatt tatgccatag caatacataa taaaaattaa taaatatgta tatatttttt
56641 tgagacaggg tctcactctg tcaccaggct ggagtgcagt ggcacaatca tggctcacta
56701 cagcctcaac ctcccaggct caggcaatcc ttctacctca gcctccagag tagctgaggc
56761 cacatatgtg tgccaccatg cccggctaag ttttctattt tttgtagaga tggcatttca
56821 ccatgttgcc caggggggtc tcaaaacact gggctcaagc agtctgccca ccttgacctc
56881 ccaaaacact gggatttcag gtgtgagcta ctgggcccag cccaaaattt caaaaaaaag
56941 caggcaaaaa gtcagcttta tctaaatgtg tttgatttaa aatattgatg tgtattcatt
57001 atcatttata ttttgtccag atgccgtgtc tcatggctga gattccagca ctttgggagg
57061 ctgaggtggg aggatcactc gagctcagaa atttgagacc agcctggaca acatagtgag
57121 accatctctc tacaaaaact aaaaaaatta gctgggtgcg atggctcatg cctgtaatcc
57181 cagcactttg ggaggccaag gcgggtggat cacttgaggt caggagttcg agaccagcct
57241 ggccaacatg gttaaacccc acgtctacta aaaatacaaa aaattagcag aagaatgctt
57301 gaacccatat gtatacatgt atatatacat atgtgtatgt gtatgtttat atatatatgt
57361 atatgataca aaattagcca ggtgtggcag cacacacctg tagtcccagc tatttgggag
57421 gctgagatgg gaggatcact tggacctggg aggtcaaagc tgcagtgaac tgggatcatg
57481 gcactgtact ccagcctggg tgacaacgtg agatcctgcc aaaaaaaaaa aggaaaaata
57541 aaaagagtga ttttacagaa aaattgagat tttcaattta tcacggcagt gccagctctg
57601 aattcctatg cggcaacgct ggaccaggag ctgctgcccc tctaggaggg gagggtgccc
57661 tccagcctgt tatagccttc ccttggcact tggcacatta cttgcctggc ccttacagga
57721 atttgaggtg caaattgatc tagatcattg aaggaagagt ctgggtcttc tcagttttgg
57781 ggcactttcg ttgccaagta aggttcttat gagattttta gaagttgaga ttaaaaaaga
57841 gtttatatgc agctggaggt gatacctaac aacaaccata ataacagcat caacaacaaa
57901 aacagtagcc tagagcagtt actctaagcc aggtaagtgt tctaaagcac ttcacacaac
57961 ttatttactc tcacgacaat gctattaggt agcactttga atacacacat tttgtaggtg
58021 aggaaattgg ggcatgtagc tgttaagatt tatatcagcc gggcgtggtc actcatgcct
58081 gttcccagca ctttgggagg ccaaagcggg cggatcacga ggtcaggagt ttgagaccag
58141 cctgaccaac atggtgaaac cccgtctcta ctaaaaatac aaaaattagc tggacatggt
58201 ggcggctgcc tgtgatccca gctattcagg aggctgaggc aggagaatcg ctttaatctg
58261 ggaggcggag gttgcagtga gctgagatca tgccactgcg ctccagcctg agtgacagtg
58321 agactctgtc tcaaaaaaaa aaaaaaaaaa aagatttata ccaatttata ccgtccaata
58381 ggaggcgtga gactcaacac agggtggagg agagaatagg tcaggaaatc acatctctcc
58441 acctgcacag ccaccatcat tgtacccttc taaagagact ggcaacaact ttcattcagc
58501 actgtcccat tgattggctc atttcatgat cacagcaacc cagttaagca aatcctcacc
58561 tttgcatcgc ctgctttgta tcacttttct ttaaaattaa aaataaaaaa aatggattac
58621 aaattcggtt aacgcccatc ccatttctct ccttccttgt agtaattatc attctaaagt
58681 tgttgggaat tattcccatg cgtactttgc acttttaaaa aatgtttgct ttcctaaaca
58741 tataccactg ttttgtgtat tttaaaactc acacaaataa tatcatacaa catccatttt
58801 tgcaagtttc ttactcatta cattgctaaa tatgtctata attcatttaa ctgctgtata
58861 gtatcccatt ataagaacaa accatacttt atttatccat tttacttaac aacagttagc
58921 ttatttccac ttcttcttga caattaactg atactgcaat gaccattttt atcctcgcct
58981 ttttgtgcat gtgtagaaat gtttttctaa ggaatagatc tagaagcaag attgctgggt
59041 catagggaat attcattttc agcattaact ggtgccaaaa ttctcttcaa atttgttgta
59101 tctgtttaca cttcaacaca aaatatatgc attcctattg tcccacattt ttgtcaacag
59161 tgttagaata attttaattt tggtgatttt gtgggtgtaa aatggtaact cgcgctgtga
59221 tttgcatttc tacattactg gttaaattga gcatcttttc ttatgtttat tgaccattag
59281 gtttctcttt tgtgactcac ctattcacag cttttgctca tattctcttg cgttgtcctt
59341 ttcatatgga tctgcaactt ctttatgaat cctggatata atcctttgtt gtttatgtag
59401 attgtgaata ttctctaagt ctgtggcttg tcttttactt tacgtgattt cttttttttt
59461 tttttttgag acggaatcct gctctgtcgc ccaggctgga gtgcagtggc acaatctcgg
59521 ctcgctgcaa gctccgcctc atgggttcac gccattctcc tacctaagcc tcccgagtag
59581 ctgggactac aggcacccac taccacgtcc agctaatttt tttgtatttt tggtagagac
59641 ggggtttcac catgttggcc aggatggtct caatctcctg accttgtgat ccacccgcct
59701 tggcctccca aagtgctggg attacaggct tgagccatcg cgcccggcct atgtgatttc
59761 ttataaaagc ttaaaaatgt tttaacgtgg tcatatgtat caatatttcc ctttattgct
59821 tgtgcattta tttatagtcg acaaataaaa attgtatcta tggtgtaaca acatgatgtg
59881 tgtatatata tgtgtgtgtg tgtgtgtgta atggctagag taattactgt atctgttatc
59941 tcacatacta tcattttaaa cgtgatattt gaaatgtact ctcctagcaa ttatttttat
60001 ttttattttt tttttgagac aggatcttgc tctgtcgccc aggctagagt gcagtggcaa
60061 gattatagct cactgcagcc tggacctccc aggctcaagt gattttctct tctcagcctc
60121 ccaagtagct gggactactg gcacgtgcca ccacatcctg ctaatttttt caatttttg
60181 tagagacagg gtctcactat gttacctagg ctagtcttga attcttgggg tcaagcaatc
60241 aagcaatcgg catcccgaaa tgctaggatt acaggcatga gccactgcgc ccagctaatt

60301 tttctatttt ttttagagat ggggtctcac catgttgccc aagctggtgt cgaactggtc
60361 tcaagcgatc cacccacctc agcctcccaa agtgctgggt ttacagacat aagccactgc
60421 agctggccag caattttcaa gtatacaata cattgttatt aactctagtc atcatgttat
60481 acaatagatc tcctgaattt attctaccta actgaaattt tgtatccttt gactaaagct
60541 ctccaatccc tgcccacacc cgccccccgc cccctagccc ctgtgcagcc accattctat
60601 tctctgcttc tgcaatttta ttttatttat ttattttttt aaatttattt tgagacaggg
60661 tctcactctg ttgtctgggt tggagtccag aggtctgatc acagctcact gcagcctcaa
60721 actcttggat tcaagagatc ctcctgcctc agcctcccga gtagttggga ctacaggcac
60781 gtgccaccat gcctggctga gtttgactct ttaggttcca catgtaaatg aaatcatggt
60841 atttgtcttt ctgtgcctgg tttatttcat ttagcataat atcctctagt tttatccatg
60901 ttgtcacaga tgacagaatt tccttctttt taaagactga atagtattcc attgtgtata
60961 tgttccataa tctctctatt tattcattga tggacactta ggttgtttcc atgtcttggc
61021 tattgtgaat aatggtgcag tgaacttggg aatacagata tctcttcaac atactgagtt
61081 catttctttc gaaatatacc tgataatgga actactggat cacatggtag ttttaatttt
61141 ttgaggaagc tccattctgt tttccaaaat aggtgtacaa atttacattc ctaccaacag
61201 tttgtgaggg ttcctttttc tccacatcct tgccaccact tgtaatctct tggtttattt
61261 ttattttttt tatcagagcc atcttagcag gtatgaggtg atatctcact gtggtttaaa
61321 tgttcatttc cttgataatt agcaatattg agcatttgtt tttcatatac ctgttggcca
61381 tttatgtgtc ttcttttgag aaatgttttt tcagatcatt tgcccattgt aaaatcagat
61441 ttttggctgg atgcagtggc ttatgtttgt aattccagca ctttgggagg ccaaggcagg
61501 aggatcactt gagcctagga gttcaagata aatctgggca acataggaaa accctgtctc
61561 tacaaaaaaa ttttaaaaat tagcagggca cggtggggtg tgcctgtggt cccagctact
61621 tgggaagctg aggtgggagg attgcttgag cctgggagtt gaaggctgca gtgagttatt
61681 attgcggcac tgtattccag cctgagcaac agggtgagac cccaacttaa agaaaaaaaa
61741 tcagattttt ggttttcaac aactcttgct attaagtttc ttataaaaat attcacccct
61801 tatcagatgt atagtttaca aatatttgt ccctttccgt aggttgtctg ttgattgttt
61861 cctttgctgt tcagaagctt tcatttgatg caatctcatt tgtccaaaaa aattattgcc
61921 cagaccaatg tcaagaagcc aagatcatgc cactgcactc cagcccagca acagagcgag
61981 actccatctc aaaaaaaaga tctgaaattg taaaaatact agaagaaaat ataggagaaa
62041 agcaattctc ctgcctcagc ctcctgagtg gctgggacta caggtgtgca ccaccacacc
62101 cagctgattt ttgtattttt agtagagatg gggcttcacc atgttggcca ggatggtctc
62161 aatctcttga tcttatgatc cacctgcctt ggcctcccaa agtgctggga ttataggtgt
62221 aagccaccgc accccgccat ttcagatctt atgtttaagt ctttaattca ttttgagtag
62281 tttttttttt tttttttttt tttggaggca gtctctctct gttcttcagg ctggagtgca
62341 gtggtgtggt cttggcaacc tctgcctccc agattcaagt aattcttgtg ccttagcctc
62401 ctgagtagct gggactatag gtgtgtgcca ccacccctgg ctaatttttg tgtttttggt
62461 aggtttcacc atgttggcca ggctggtttc gaactcctgg cctcaagtaa cccattggcc
62521 ttagcctccc aaagtgctgg gattacagat gtgagccact atgcctggcc tgagttgatt
62581 tttgtatatg aggtgagata tggtcaaatt ttattcttct acatgtggat atccagtttc
62641 cccaggactg tttattgagg agactgtcct ttccccattg tatgttcttg gcatatttgt
62701 aaaggatcag ttggccataa aatgtgtgga tttatttttg gcctctattc tggtcttata
62761 tgtctgtttt tgttccagaa ccatgctgtt tttattacta tagctttgtg gcagcttctg
62821 aaattaggta gtgtgatggc tctagctttg ttccttttgt tcaagattgc tttggccatt
62881 ttggtccttg tcttttgtgg ttccatatga atcttaggat tttttttcta tttctgtgaa
62941 aatgtcattg gaattttgat agaggttgga tggaatctac agtttggggt agtgtggaca
63001 ttttaacaat attaatcttt ccaatccatt aacatggaat atctttccat ttatttgtgt
63061 ctttaatttc tttcttcaat gctttatggt tttcagtgta cagatttctc acctctttga
63121 ttaaatttat tcctaagtat atatttttga tgctattata aatgtgattg ctttcttgat
63181 ttcttttttg gttggtgcat tattagtata cagtaatgct actgactttt gaatattgat
63241 tttgtatcct gcaactttac tgaatttatt agttctaata gtttttttgg tggtatcttt
63301 agggatttct ctatataaga tcatgtcatc tacaaagtga caatttaact tctttctttc
63361 caatttggat gcctagggag catcagtggc tcaagccagc tgtcctggtg cacagggagg
63421 cgaggctatg agttcgaggc caacctggtc aacattgacc aatttggatg tcttgtattt
63481 ctttctcttg cctatctgtt ctgactagaa cttctagtac tatattgaat agaaatggtg
63541 agaatggtta tccttgtctt gttcctgatc ttagaggaaa atcttttaac ttttcatgat
63601 tatgtatgat gttagctgtg ggtttgtcat atatgggctt tattgtgttg agatacactt
63661 tttctatacc taatttgcta agaatttgta tcatgaaagg atgttaaatt ttgtcaaacg
63721 cctttccccc atctattgat ataatcatat agtgttcttc attttgttaa ctgcttgtac
63781 ttttgtactt ttaaaatttt gtataagaaa tccttctcta ttctgatatc atcaatatgt
63841 gatcttatat tttctttgaa tagttttcag ttttgctttt tactctagct acctgaaatt
63901 tacttctggg taggatgtaa tgtagaattt attttttgtg tgtttgtttg gatagccagt
63961 taactcaaaa ccattgaact ttcccactga tttatgtcac ttccattata gccaagtctc
64021 atatctttga gggtctgtat tagtccattc ttgcactgct atagataaat acctgaaatc

64081 aggtaattta taaagtttaa ttggctcata gttccacagg ctgtatagga agcatggctg
64141 gggaggcctc aggaaactta caatcatggt ggaaggtgaa agggaagcag gcatgtctta
64201 cgtggctgga gcaggaggaa gagagggaag aggtgccaca cgcttttttt tttttttttt
64261 gagacagagt cttgctctgt tacccaggct ggaatgcagt ggcacaatct cagttcactg
64321 taacctccac cctctgggct caagcgatcc tcccacctca gcctcctgag tagctgggac
64381 cacaggcatg catcaccatg ctcggctact atttttgtat ttttgtagag atgatgtctc
64441 agcatgttgt ccaggctggt cttgacctcc tgagctcaag tgatcctcct gcctcagcct
64501 cccaaagtgc tgggattata ggcaagagcc actgcacctg gcctccacac acttttaaac
64561 aaccgggtct catgataact tactatcatg agaactgcac ccagtgggaa atccatttcc
64621 atgatccaat cacctcccac cagaccccat ctccaacaaa ggggattaca attcgacatg
64681 agatttggag agggagggac acagatccaa accctattag gatctgattc aaaaaagttt
64741 tgttctgtac tgtggtctat ttatgtgtgc atataccagg actagactgt tttaatcact
64801 ctaagtttca accagtaaac ttgtgacata tggttaggca agggatctca tattcttctg
64861 cttcaagttt tgtttttctt tggccattta ctcttccata agaattttaa ggccgggtgt
64921 ggtggctcat gcctataatc ccagcacttg aggaggccga ggcgggcaga tcacgaggtc
64981 aggaattcaa gaccagcctg accaacatgg tgaaaccccg tctgtactaa aaatacaaaa
65041 aaattagccg ggcgtggtgg tgcgcacctg tagtcccagc tactcaggag gctgagccag
65101 gagaatcact tgaacctggg aggtggagct tgcagtgagc cgagatcgcg ccattgcatt
65161 ccagcctcgg taacagagtg agactccgtc tcaaaaaaaa aaagaatttt aagaacagtc
65221 tgtcaagttc cataaaatat ctgattggag ttttgcttag aattgtactg aatttttaga
65281 tttgagaaaa attatcagct ttatatcaaa cttccgtctt tgtgagtagg ggatctcttc
65341 ctattctttt atatcttctt tatgttctct aataaaactt tataattttc tccataaata
65401 tcatacacag actttgttat atttattcct aagtatctta tgatttttgt tatgacaaat
65461 ggtattttct tttctttttt tttttttttg agacggagtc tcgctctgtc gcccaggctg
65521 gagtgcagtg gcgcaatctc ggctcactgc aagctccgcc tactgagttc acgccattct
65581 ccttcctcaa cctccccagt tgctgggact acaggtgcct gccaactcgc ccagctaatt
65641 ttttgtattt ttagtagaga cggggtttca ctgtattagc caggacggtc tcgatctgct
65701 gacctcatga tccgcctgcc ttggcctccg gaagagctgg gattacaggc gtgagccacg
65761 atgcctggcc taaaatgaaa tcttttatct gtttattgct gtggtgtaag gatattgatg
65821 tttgtgtatt ctccttgccc ccggcaagga aattgttatt tctaatagtt ggtaggttct
65881 tgtgaatttt ctttgtatag taagcaatct atgtatagta attgtctatg tatagtgatg
65941 gctttatttc ttctttttct tcttgtgtca cttcagactt cttggcagaa aggtgaatag
66001 aaacagtaaa aactggtatc cttgtcttga ctttggaagt tactttctat tcctagttag
66061 ctagattaaa aagattaaaa aagaaataga caggttctca ctatacagtg gttattcaca
66121 gcctcaatca tagagcacta cagcctcaaa ctcccaggtt caacgaacct taccacctta
66181 gcctcccaag tagctgggac tacaggcaca agccaccaca cctggctaat ttttaaatat
66241 tttgtagttc actacagcac tattcacaat agcaaagaca tggcgtcaac ccaaatgcct
66301 atcaatgaaa gactagataa agaaaatgta gtatgcagcc aggcgtggtg gctcacactt
66361 gtaatcccag cacttaggaa gcctgaggca ggtggatcag gagttcgaga ccagcctgac
66421 cgacatagtg aaaccctgtc tctactaaaa atacaaaaaa attagccagg tgtggtggca
66481 aatgcctgta atcctagcta ctcaggaggc tgaggcagga gaatttcttg aacccgggag
66541 gcagaggttg cagtgagctg agattgtgcc aatgcgctcc agcctgggtg acagagtgag
66601 actctatctc aaaaaaaaaa aaaaaaaaaa aaaaatgccg ggcacgatgg ctcatacctg
66661 taatcccaga actttgggag gccgaggtgg gcagatcaca aggtcagaag ttcacgacca
66721 gcctggccaa catggtgaaa tcctgtctct actaaaaata caaaaattaa cagggtgtgg
66781 tggcactcgc ctatagtccc agctacttgg gaggctgagg cagaagaatc gcttgaaccc
66841 aggaggcaga ggttgcagtg agccgagatt gtgccactgc tctccagcct gggtgacaga
66901 gcaagacacc accttaaaaa aaagaaaaaa gaaaatgtag tatgcgtaca ccatggaata
66961 ctatgcagcc ataaaaggga atgagatcat gtcctttaca gggatgtgga tggaactgga
67021 agccactatc ttcagcaaac taacacagga acagaaaacc aaacaccaca tgttctcact
67081 tataagtggg agctgaacga tgaaaacacc tggacacatg tgggtggggg gaacaataac
67141 ccactgaagt ctgtcagaag gggaagtggc aggagggaga gcatcaggaa gaatagctaa
67201 tggataccgg gtttaatacc taggtgatgg gttgacaggt gcagcaaacc atcatagcac
67261 atgttcacct atgtaacaaa cctgcacatc ctgcacttgt actccggaac ttaaaataaa
67321 agttgaaaaa atattttgta gagacgaagt ctcactatgt tgcccaggct ggtcttgaac
67381 ttctgagctc aagcaatcct cttgccttgg cctcccaaaa ttctgggatt ttagcagtga
67441 gccactgcac ctggcctcca gttctggatc ctgggtcttg cgacattatt ccatgctggc
67501 ttctgaagtc aatgaggcaa aaccagaaag gggaactaaa aagttaaaga gggagagggt
67561 ctcacaggca agatggagga agcctgggat gaaggcaggg ctgtggacct gaatgatgcc
67621 aggaattaag gaggaagtaa cagtgttagg taatagcaca tgggattctt gggctgcagc
67681 tacaggaggg agaatcatgg ccttaattca tcacctctga aatcctgaaa tatctagcct
67741 cctgcccaga tcctgccatg aatgcatcta ttcaaccact atttataaag tatccagtgt
67801 taggtccagg gagtggcaag agtgagcatc tgcagtcata gtccctgccc tcagggagct

67861 tacattcaat ggggcagaca gacatcagtc tgtacataat catcaacaaa tgtagaactc
67921 caacagggcc aagtgcctct gaggaaaggt acaccttagt gtgagcgctc ataacaggag
67981 aatttgaccc tgaaaagaag gtcaggaagg cttccctgag gctattgaga aggtgaggcc
68041 tgaagaatga agaggagcta agaggagagg aacagaggaa ggagccgttc cacagagggt
68101 ccagcctccc tggagactct gtgcaggagg aagcacaagg tggacagcaa ctggagcaca
68161 cgagtgaggg ggtgtggtgg gagatgaggc tggagaggag gggccacccc ttgcagggcc
68221 ttagaggtca ggttaaggac ttgggtcttt aacctaagat caatggaaaa ccactgaata
68281 ggtttaagga ggctgactga aacaatcaga tttgcatttt taaaggatcc ctcttaggtg
68341 tgtgtcatac tgtagagaag gccattccca gcctcttcag ctactgcaat gactcagaga
68401 aggccacatg gtccacatgg tggctagccc agggatgtgg agggatgaaa acgagtggac
68461 agacttgaga tatattagag cagataaaat ggataggact aggtgctgga ttcaatattg
68521 agatgctcat tctaaaccgc ccctacatca aatccttcga aggcaggggc tatgccacat
68581 gactatgata cccaggtccc agaacaactc ctgacacatg gtagactcaa tacacatttc
68641 ccgaaagggt ggaaatgatt agcagtctct caagtaatcc tttgaccact aagcgtattt
68701 aatttcttct agcatgtgtc agtaatcaac ctccttggta catcataggg agaaatattg
68761 ccgagttcca aagagtatac tctggggcaa gttaatacaa gaataaggtc tcacttaccc
68821 tataacatga agagtaacag taaactcgta agaacaggac ctggtgaagc aaggagtagg
68881 cacaggaaga tggctcatca aataggggcg ccgggtgtag tgtgaacacc tgaggcagga
68941 agaacccagt agcctctggg ggttgggtgg gattagttgg gggactagga tttgatggta
69001 gggggttgtc ctggagatct ctggaaatta agttatctat tttttttctg tttttatttt
69061 tataaaagaa ctcttaaggg catttttttc tttgtttgtt ctgttttgag atagaggttc
69121 actctgtcgc ccaggctgga gtgcagtggc gtgatctcgg ccactgcaaa ctccgcctcc
69181 caggttcaag tgattctcat gccttaacct cctgagtagc tgggattaca ggcatgggcc
69241 accacgcctt gctaattttt gtgttttttg tatagccagg gttttgccag gtttggtcag
69301 gctggtcttg aactcctggc ctcaagtaat ccacctacct tggcttccca cagtgttgga
69361 attacagcca tgagccatca tgcccagcct aagctatcac tttaaaatga cccttcaaga
69421 aagtcccaag gagtctaatc catccacgac aacagcacgt ctgagacagc ctagtgaaat
69481 ttctccgatg gaggacagga gatggaaaag tgccccagga cctcttattt atataaaaaa
69541 caaaattttt taagaccctg agaagtatgg attcttctcc agagaaaagt tcctggcttg
69601 ggagctccct taggacttgg cactttggtc tagagtgaag tggacctggc cattccagct
69661 ttgagtgggt tcaggcgaga cactcgagct accccttatg gttgctctga aggtcaccct
69721 ggctgggagt gggtggatct ctgctctgtt atgcctccca caggtggagg aagtctgtct
69781 ctgaggctgc cctgccaccc tcatagagcg ctgcagattg ggggcggtga tgcagccaca
69841 ggggctctga ttccctgcct ccccactgtg ttcatctcat tcctctccac atatgaggcc
69901 tggtgtcatg gcaggtgggt gttccaagtg aaaccacagc cacagaggca agcagtagag
69961 cctgtccctg atgtggccct ggtgatgctg caccagcctg gggctgtatc agaaaccatg
70021 cctgcagcct ttgcttctgc agacttgctg gtgctgcacc agccctgagc aatgtccata
70081 gcccttgcct cactggtcac tgtgatgggg acgcccccag gtatggttct gctgctggtg
70141 ttgcaggttg gagtgggggc cagcaccctc atctaccaca agaaggctta ggtggcacag
70201 acgtaaggct gcttgcctgt gtgtagagtg gcaggctgca acgggctgga gccttgggga
70261 gaggcccttg ccacactggt ggcagtgatt gggttcaggc aggtgtgtgc cgcagtgctg
70321 ggccaggtgt gagctgtggc aagagctgtg gccacacttt ctgcaggagt agggcctatc
70381 acctgtgtga aagtgttcgc acagggtcag gtgggcccct tggctgaagt acttcctata
70441 ttagggggag gagaaggccc ccttgcccag gctgtgctga gagaggtgag agcagtggga
70501 agagactcca cattccaggc aggaaatttg agtggatttg tgggtggaga tggggtccc
70561 gggtagggga gttggggaag ggttggttgt gacagctggc aggtaaagga gtgccatagt
70621 aggtagaggg ctgggtctgt cctctcttct cctcctcctc tccctttgct cccagcctgc
70681 agggagaggg gagagaggtg agagcagcat gggggaggag gaagcagagg agacagaggt
70741 gggagaggag aggctaagcc ccacacccaa ctgaagaagt ggttggcggt ttcagcggca
70801 cctccctcct cgctgaggtg gactgtgaag gtggagaagc ccagagcaca gcctcctgtg
70861 agcagcttcc cagggtgttg agggctgcca ggcaaggaca gggccctcag agttaggcct
70921 tgcctgggca ggggtctcct gggggaacct tccctcaggg ccctgaggct ctggctccca
70981 gagctcagct tccgcgttca gatacatggt ctgctctgct ttggagactg agaagcctga
71041 gaggtaaaat cgtgctgggt ggtaggtagg gtaagggaga gggagcttcc ccccatctcc
71101 ctcacccact gcagaaaggc ttctctgttt ccttccccag cccagtccca cctcctctgt
71161 gaggccgctc ctcacctccc tactggcctg cagtgatcct cctgcttggg agtcacagag
71221 cacccctaca cgggcccttt gccattcttt atagtataag gcaaggccgt aacattttgt
71281 ggggcatctt cttcagtgtc ctcagccttg tgtgaacatc agaagcactt gggggcttgg
71341 gaaaatgcaa ttgctggccc catctccagt ttcttaaaca gcagatctgg ggtggggccc
71401 aagaatttgc atttctaaca agttcccagg cgatgctgat gctgctagtc tgggatccac
71461 actgggagag ccactgatct gttaggcgtg taactttact tgtattatct ctcaattctc
71521 agggcaacct gttactctcc cccattttgc aggtgaagaa acagagggaa gacccagtgc
71581 cttgtgcaag gtggctcagt ttgtaagtgg cagagccgat ttgaacccac gtttgtctga

71641 ctctaatact gtatcaaccc tccttcctct accctgttcc tctgcattgt caactattcc
71701 acatgtatct tgttcctcaa caaaatagta acgttttctt ttgcatactt tatatctctc
71761 accttgcctg acccagtaac ataggcttgg ttgggaggga ctgaaggact tggaaaggaa
71821 atgccccagt ggagccagga tggaaatggg ttcctctgtg tctcccacat ggtgtcacct
71881 aaataaacag ccatgtctgt gaggttcagt catggggaga ggagggcaag agagcactcg
71941 caccaagcga tggggttagg gggacacgaa aggaaatgtg ataaatagag agggggcgt
72001 gtggggcaaa ggtgaggact ggggacccgg gagggggcag gctgtttcag agtgaaggca
72061 ggacgggcag cagagacaga gaagaggctg ccgggtggtt agggctgggg aggacaagga
72121 gggtgaggac agacgatccc ggcagcccct ggagccagaa gagaagtcag gttaactcca
72181 gacttttggc ttccacctct gtatctcacc caactcctcc ccacctgctt ctctaccctg
72241 aaactccagg aacaacgccc acctgcctcc cactacaact tccccttata ccgtacacgt
72301 cctggatgct tggggtccgg gaggagggga gtcaggatca cgggttccag ggaagtgagt
72361 gtagaaaggg gctcctacga gggtagaagt gggtgttgtc tgcttctgca ggaaaggggc
72421 cagccagccc aactggtccg cagtacttgc tggggctgga ggatgcccac ctgcatctac
72481 gggattgagg gtaccggaga ggaggcgaac aaacgagaaa tcccaagggt ggagaaggga
72541 gccatgggat cggcgggcgg gccgcgcagc tgcggaactg ggagggccaa gcgggcttta
72601 gggggaaaca cggccggctc ctctgccagt gagacagaga actgagcatg gggaggaat
72661 ttggggccag aggggctggg ggtacccaga gcagaggaaa caggaagtga ggggcagaga
72721 ggttacgggt ggtggaagga acagcagtcg gttctggaga ggcgattcct cttccccgaa
72781 tacctgcact gccccggacc gggagtgcag tcgtgcgccc ctgtcccccc agcctcaggc
72841 ttggtgccca gccccgctcg cggaactcga ggccagtaag gcagccgcgg ctttccttga
72901 tgcccccact cctctgctct cagccgctgc ctggacgagc acagaactag tgttcccagg
72961 cgaccctccc tccgctcagc cccgctgccc ggcgggcaaa ggctgtgcac tcacactccg
73021 agtctgcagc ccagcctgcc ctttttgggg aggtggcccg gggactggga tgtcgaacag
73081 ggagcttctc cgcagtcctc tccacctccc tccctctttc cagctataga aaccggctgc
73141 tcgcagtgac ttccaaacat cacggtcatt cccacatcta cacacatccc tttctctttt
73201 gagccatttt cctgctttcc ctttggtgtt gaaaccctgg atttcagcct gggcgactca
73261 ggttttaggg gggaaagttt ggggtctaac ttggcacctc tgttttttat cctctcttcc
73321 agcttcatag atggttccta ttttcataca tatttgctaa tttggagttt ccgacgacat
73381 atctcgaata acccgacatt ttctccccat ttccaaggct gattttacat tatttctagg
73441 cctccctccc caatctattt tcctttcttt ttcttgtaca cccctgataa acagacacac
73501 agaactgcag gtttcaaaga ggaaaaaagc acgtttgttg ttggtaggcg ggcgtgaatg
73561 ggagggttct tcaccttctc caacacctcg tatagatggg acgttgcagg gcaatagaaa
73621 aggcagcaga tgggggcgtg ttttgcccca ctctgtacaa tatagaagaa tctcatatag
73681 atactttgta taaaagccac gtgtcctcat ttgtgtcctc ttattggctg gctgggctgg
73741 gggtactggg ggacaaagaa gctggggtga tagaccagga cacagggata ccccatggtc
73801 cccttcctct ggctgtcccc tggtggggtc actttggatc cttggtggga gcatagcaca
73861 gctccagtgg ccgggacacc ttccagaatt tctcattcac cagctccagg gtcagcgagc
73921 cattcaacgt ctgaggaggg ggtaaaaggg agagagggga aaagagagag gaaagttgga
73981 gaagatggaa aaggaaatga atgagaggca aaaggtgacc aagggaggag gaaagaaaaa
74041 aggaataata aaagcgataa ggcaaaaaag gacatggatg atgggaaagg agaggggaca
74101 tagaaagaga tagaggatgt ataggggaag agtgctgatg agttagacca ggctcatggt
74161 tctatgtggc ccagaccacc ctccctcctc tcccctcttc taccactgcc ctcagaagct
74221 ctcaccgtga acgccccgcc tccaggtgcg atgtagatgg tgtactgctt ttcactgacg
74281 ccctccaggc tgggcaaagc aggctcctca ggaccgtcac ctcctccggc acccccaccc
74341 tccccgccac acccatcagg tcctccggtg tcagaggcgc cccctccagg ccctcctggc
74401 tcccctgctt cctgctgttg cctccgctcg agggcaatgc gcagggccaa gtacttggag
74461 aggtggtcca ctgtggcatt cccagttgtc ttcacatacc tggaatggga gggaggacag
74521 gcttagagcc agagcgcctc agatgaggac tggaggtagg ggctttccaa aatggattct
74581 gggttggggt ggtattttgg gtttgggcaa agcccacttc tgatggaaat ctaggtgatc
74641 tttggtttct cagtggctgg ggaaagacag ggctcctcac ctcgtctggc agtattctcc
74701 cttctccacg agcagggggt ggggccggaa cacgagctca atttctccac ctggctctgg
74761 ggggctgggg gccccaggag ggcttggggg gcccagcgtt ccccctccca gagtccctcc
74821 ccggtcacca gagtcttccg aaccggcacc cccacccacc ccaccagtgc cgcctccccc
74881 tgtccctaca ctgctccccc ctgcgccccc tccacggggt cgcttgggag cagggcctgg
74941 ggcagagtca ggggcggagt ctgagctcac atcttctcca tccccttctc cctccccggg
75001 ctctccttcc cccccactca tcgttgtggt ctgatctgac cctggtatcg gccgcctcac
75061 acgctgggcc ctgtagaagc aagtggttaa ggttagaagg catccaggat aaaggcttta
75121 gactttaaac ttcagagaat taagagataa gaaaaatcct aatgacgata cctaatattt
75181 attaaacaca atgtgctaag cactcagtat actaagtgtt ttgcaccaat ccccttacct
75241 aatacaatga atcctatgac acaggtaatt atattacctc tattttatgg ctgaggaaac
75301 tgaggcttag agaggttaag taacttgtta gtgatcatag gctgtcacct ctcaaagtgc
75361 ggtagcagca tacacatccc ctgtgagctt gtgaaatgca gactctcggg cccaactcta

75421 gacctactga atcacagttt gcatctgaag atctacaggt aatctggata cacatgaaag
75481 actgagagat gctggcctag gaggtgattt aacttccagc agtatgaatc cggggcttat
75541 tctcaaaatc gctctactgt tctaacttct agaaagtcat ggggtaaact acatttttcc
75601 tttttttttt tttgtgacag ggccttgttc tgtcgcccag caggctggag tgcagtgtgc
75661 agtggtgtga tcatggttca ctgcaacatc cactttcttc ccaggctcaa gtgatcctat
75721 tacctcagcc tcccaagaag gtaggactac aggcatgtgc caccatgccc agctaaatgt
75781 ttaatttgtt gtagagatga gatttcccta tgttattcaa ggtggtctca aactcctggg
75841 ctcaagtgat cctcccatcc tcacctccca aagtgctggg attacagatg tgagtcactg
75901 tgcccagccc tatatttttc ttaagaagag cagggccagt tgtttactaa gaaactgcaa
75961 tttacaccca tgatttccag ccctaatgat cagtctgtgg tcagctccta ggatgctgag
76021 gcttagaagc tagcaggcaa caaggactga cccacggacc catcccatca gttctgcttc
76081 tctcctgacc ctcacacctg tgcatggcct gcatgcgtag cccctcctca atgctggagc
76141 tcaatgcctg ctggttgtgc aggcggctca ggcggataag cactcggtct tgatgggcct
76201 cgtattcctc ccggctagga tagatcttag agatcagggc atcaaagttg gggtctggcc
76261 gtagggatcg cttggacacc agcttctttc ggcaggtagg acactccttg ttcctggggt
76321 aggagaaggg aagccctgag tggtcagtca agggaactga agcctaggct gaaccaagac
76381 cagtgagagg gccctaactt tgtacccctt catctcatct caaacatgtc tctcctatta
76441 cccgctccgt agggctgtga caatgcagtc agagcagaat ctgtggaggc actccttggt
76501 ggtcatcgta ttcttcagca tgtccaggca gatagggcac atgagttctg aatgcagtga
76561 ccgaggggaa acagcaatct ctgtgccatc cattatggct tcctgaaggc atcagtgagt
76621 agaaaggtgg tgtgggttag agggaaaggg gttttagaaa cagcagccca taaaataaga
76681 attttgagaa atacagtgag aagacccatg ttggtctcta taggagacaa gaattctgag
76741 aaagagaagg actgatgact tggagcaaat ggagaaaaga cagactttag tgtccattaa
76801 ctaagagtag gagctttgga ataggaggat ctgaggtggc taagtggcct aggagtgaag
76861 atggcacaaa tctgtggttg ggaaaaacta ttcaacaaaa ttttttaaag tcctggtgct
76921 acctgtatta gaaccccata gggtgcctgt taaaaattca gattctgaga acccaattca
76981 ctactgaatc agaatgttgg ggggcatgaa gctctgcttt taataaactc ccaaagtgag
77041 tcttatgtgc actgacttat gacactgaat gaggaacagg gaaggtaatt tttgtgtttg
77101 tgatctttca aatgaagata ataatacttg ttccacctct ctggcagggc tgttttgagg
77161 attaaatgag ctaaatgcga aagtgctttg gaaagattaa ggtgattcaa aggtagggcc
77221 aggcaaggca gaatagagca aattacggaa aagaagctgt gtgatgcggt ctgtggatat
77281 aagggtttgg gggagggggt aagcctgaaa gggagaatgc ctgtcacctg cggggtccgg
77341 tgcagctcat acagactcag ttcccacgtt ttgctggcat tctgggcatt cgccggcgtc
77401 gtcatggtga ccgggcgcag accccccacc agggctccac agccgaggag aaggcgaaga
77461 aggctggggg tggggggaag gggaggaggg cgtcaggggg gccgcccctc gcgtagaccc
77521 cgcccctcca gaagcgcccc tctccgcccc cgctcccgcc cccgcgccgg cactgcccct
77581 cacccagctc cagccgttcg cgctcccgcc gccgccgcgc ctctccctag gcccgggctc
77641 ctgggccaag ttcgctcggt ccgcaaccgc tgctcagaca gcagctcccg ccaccgccgc
77701 cgccatggcc cgggcgctgg ggccctacgt cacttccgcc tgctcctccg ctacacccgc
77761 tccccccgcc ggcggagacg tcacccacca ttcgcggcgc gggtgggacg ggacgtggac
77821 gccgcggttc tcccgcccac gggacgggct cgtcccgcgt gaggatcctg cgtcccggga
77881 gttctgcgcc acttagggaa ccacggtctc cgcccccggc ccagcaccgt cgcgcggcca
77941 acaaccttag cctcggttcc cttccagagg cccccacgcg gggcctcagc tccgggggc
78001 cggggcctgg aatatctggg accccaggag gggacagagg gctcgtgaaa aggctacct
78061 cccccactct gcgtaccctg gcgactctgg ggatcaaccc ccctcacttc tccgtaaccc
78121 ccattccagg atcccggggg aaaaggctgc aaaagatctg ccgctttagc cttctagtcc
78181 tgagaacaca cctaggtccc gatgtgcccg ggattcccct taccaccctg gggactccct
78241 ccaccctctg ctttggctct ccctagaccc tccgacccgc tctcccgctc gaggtttggg
78301 ggcggcggct cgggacgtcc agactcaact ctcggccgga gccatagact cgagaattgc
78361 gtttggacaa tcaggagccg cggcccgggg cggggaaggg agggaggcgc acgggaggaa
78421 agggggaatg gagacaccac gcggaaacag ggacatacac aagatggagc tcccattggg
78481 aagggggggt gcgcgtgtgg gtggggtaca caggggcaga atgtctggag tgggaagtgg
78541 gcagaagcct cttcgaggac tctagagtaa acgaagggga tggggaggaa agagaagagg
78601 aaggggcaga gcgatcccca ttgagtgtag ggaatgagag ggatacaggg cgagggggtg
78661 gggggcagtt agagaccctg atgggcatct tgtccccgcc cgagctgagg ctcccggact
78721 tgggctgcgg ctgccagggg gtggcctgtc cctgggacaa ggcagcagtg ggtgccctcg
78781 ccccccaccc cagttccccc ctacttccct gtcctctcct tcagtccgtg gccagtcctt
78841 atgtgggcgc cagagtcgat tagcgcagac ccccctcccc ttcctcctcc tcttcccagc
78901 cccctccctc ctgctctctc cagccccttt catcggctgg ttcattcccc taatcctggc
78961 cccctcccct aatccccccc atccgacccc aggccggctg cagctattgt aaggtggaga
79021 gaaaggggag ggggggactc agagaaagga gaggggtggg ggagggccac ctgttccaag
79081 accccctttc aaggccagac tggacaccaa gatggggcca tgaacaaatc acccttgggg
79141 accataagaa cccagggagt tggggggagg ggactggtgc tgcagaacca gtggaaaggg

79201 gtgacgcacg aacccctccc ttcaaaaaga cccggagtgt cacgcataca cagtgacaca
79261 tactctttcc tctcacaccc ggcggcgggg gttgccctgg gagaccaggc agagaaaggg
79321 aacaatcctt cgggaaaggg aaaggagggg gaggtgggga agggtctgag ggcttggaca
79381 caagaagagc cggaggtggc agggaagagg acttggaatt tattagggtc acaagcaccc
79441 ctgcttccca tattcagcat tcctgaacca tacactgcca ccccttttgt atcctgggaa
79501 cttagagtcc tcatcagcag gaggccaggc agagtggtgg gggggtgagc agagtccagg
79561 gtccttgagg cagttacatg aaaagacctc ctgggagggg cagaaacatt tggacagatg
79621 catgggtgga gacacaaatg ggctaggggg tacccacttc tgtccctggc cttgggaacc
79681 tctgcccttc ccagtggctc cagactcccc ttacttgctt tctcatatag tctgggttct
79741 acttctctgc cctctccctc cccatgctgg cctcatacca gtgacttcca ctgaggtccc
79801 tgtgatgtat ccactatctt cagatgccaa gaatgcgacc acatctgcca catctatgag
79861 aatggtgggg agagggcaga attctgctca ctcccataga cccaaaacag ccataatctc
79921 ccaccaaaga catccctccc cacaaacctc tccacagaca ccaaccacca gtcccctaga
79981 aaatccccaa actttgggga tctcttcatt caggccccct tcccagggac cccactacat
80041 tcagtgctca ccctcagggt cccccaagtg tcccatcggg atcatttcag taatctgtaa
80101 ggaaatactc atgtgggtga aagcttcttt tatggatttt tttgaagact gagtctgtga
80161 cccttttttg tcttcatttc tcatacattc attgaatatt taatgtttgc caggaactgt
80221 gcccagtatt ctctctctct ctctctctct ctctctgtga agcccagaga gattgagtct
80281 ctgaatgatt ctgccctcca cccacagcct cctaccttgt ccaccacttt ctgtggcact
80341 ttctgtgtca tgggtgttgc aatgaaccct gggaggacag agttacagcg gatcccatgt
80401 ctgtgggaag gagagtggct gccgattctg gagagggctg aatgctggta cctccctcag
80461 accccccagg tgctccccag caccctcaag catctgacca accgtccaag ctcccgggct
80521 gcggtctggg tcagcccaat cactccagcc ttggatgctg catagtttgt ctgccccacg
80581 ttccccacct ataggtgagt cagagatgtg gaatgagtca ggagtctcaa ggaggggttc
80641 tcctctctat accacttggc tggctgacct cgtccaactc aacctgacct ttcctacgat
80701 gctactgatg ttgatgatgg aaccacgaca accattggac accagggctt gtgctgcagc
80761 ctgagtgact aggaaggtgc cctggcggga aagggtaaca agggagaaaa gatcagctgg
80821 ggtgacagcc ctcctccaac tccctcccca ggctaagggg gccaaacgtc gcaggttcag
80881 agatcgccac cttgaggttg acagctatga ctttgtccca gtcatcctca gacatgtgca
80941 gcagaaactc atcctgggtg atgcccgcac aggacacaac gacagatggt gggcgagaaa
81001 agcaggccta tgggaggggg aggttacgca tcaaaaaccc cccacaaaaa gccggggcag
81061 tggaggcaat atcagagctt tagaggggga aagtggccta gcgttcacct gcacttgttc
81121 cagcaggcac ctggcggccc tggcctcaga cacgtcagcc tggaaggcag catggttccc
81181 tcggggcggc ccctccttgc tccctggccc gcccagcagc cgcaccgtct cctgtgccgc
81241 tgcccggtcc aggtcgcagg cagctacggt ggccccctct ccggccaggc gtacactgac
81301 cgctcggccg atgccgctcc ccgcacctga ggtaggacag aacacgccgg accgggcacg
81361 gggaggagag ggcagggatc agaggtcaca gggcagaaag taaaatgtgg gtaaggggcc
81421 agaggtcaca gcggccgcgc actccaaggg cacgcccctg acctcactcc aataccccaa
81481 accgcccggg gaaagaaccc cctcaacctg tgaccaaggc cagtgcggag cggagtcggt
81541 tctggagctg agacgccatg gcgggctgtg ggtgggtggg aatcccagca ctaagccaat
81601 cccagcgcag aggggcgtgg caagggcaaa gccggggcca atcaaaggcc cgccagcctt
81661 aggccatctg cggccaccag cgcaggccga cctcccaccg agggggcgtg gcccgacact
81721 gctgcttagg ggccccgggt gtggagaaga caagggggccc cattcccccc aaaaagacaa
81781 agactgcatg tggggcggaa gatcggaggt ctttatttcc ggttaccacc cctccccctc
81841 gattcaaaca cagtgatata aaaggaatag aaacaaaaaa aatcaggcca tgagaatacc
81901 aactaaccct ctcccacccc atccccccaa tcactcccaa atcaggacac acgattgatg
81961 tttgatgttt atccccgact cctcctgtta ccctctctgt ccaggactct ggtcttccct
82021 gaagcctcca ccaactggtg ggagaaagga aactggacct tggagaactt cctgtgtagc
82081 aacttggttc tccgagaaca ggtggggtag gggacaccag gtaggctgcc atcaccccct
82141 gtcccttcca gccttcttca tggccccatt tagccttcca aatgcaacat ggtcagacag
82201 tgcccaacac tatggtccct ctggcctctc attctcaccg ctccagcctc tgtaagactg
82261 ccagcccttg aaggccctaa tgccaaagta accattctcc caggctacag ttccttcctt
82321 tggcagatgt ccctggccag ggcccccaac ctctacgcac ccctgacctg gagttagggg
82381 tagaggtttg gggcaggggt agtttatcca cccctcactc aaggtgggca atcagcacca
82441 tcatgataac tccccccagc agccccagca cctccagaag tgattgcaat ggtgatgcct
82501 ccctcagcag ctcgggcaac acagacactg ttgctacgta gataaagcca cctgcagtaa
82561 atggcaggac ccagccagga cctgcaccac ctgcaatttc actgcccact gctcctcctt
82621 cagtgagaag ggcacaggct gtgcctgcca gtgcccctac tgctgtcagt agttgcagac
82681 gcatcgcctg atgggcagaa aagagaaaaa ggcactcacc aatagctaaa gattatcagt
82741 ggtttagaag tggctggtgg ggtataggta taggggacat ggatttggaa actaaacatc
82801 aaaataaaaa gagaagaggg agcagtctag aatagacaat ttctgaaaaa tcttaagaga
82861 tcagaggtcc tttccacaga ctttctgatt gagtaggcgt gtgtgtgtaa gactccacta
82921 aaccattcag atggtttata gaccagttta agaaatactg agctaaagga caaagtaacc

82981 agaacttctt ccccctcatt tctagaggac ttgtcaatga cagtatgaat tgtttggaac
83041 taataagagg ctgtttgggt ttagggtgag tgaggagaga taaagggttt gaggcagctg
83101 tgtttggcag acatcaccaa cctgcttttt gctgcagcca gactggacca agatggcaaa
83161 gtctccgacc tcgtggggca cttcatgtag caggacagtc attgtggtca ggatccctag
83221 tccccggccc cctcgaaagg aagccccaat ggccagacca tcagtgaagt tgtgtgccaa
83281 gtcagcagcc agattcaggt accccgacac acgcaggtct tgtacaggac aagaagagaa
83341 aatgatcaga ggcttccatc accacttgtt ccagtcttgg tcagctattc tcacacctcc
83401 cctcagagac cactttacac ccagtccttt agtgtttctc agatacctgc cattacccac
83461 agcacatatt ccatgtgatt tgtgatgatg tcccttgccc aaatttatca ccaactctgg
83521 cccttaccta agcctctttt ttcttcttca gcgttctgag gtctcactgg cccatctttg
83581 ggtactgtgc tccctcctcg cctcttctga acccctcttg tttccttttc ttcttcctct
83641 gagctctgct tctccttggt agaacgctct gagggaagaa aagagttggt cagagtggag
83701 aacagatctt ccaacatgtc ctctccctcc tgagggccat aagatgagga gattctgagg
83761 caggcaagtc agctttcagg aaagttgttc ctgggctcac cttgtcttcc atgtccatga
83821 cttccacgtg tatgactgtg agcgtgtcca tgtccatgac tgtgaccatg tcctcctttc
83881 acatgtctca caaatttctc cacgacaaga aaggcaacaa ttccactgag aacccacagt
83941 cccacagaca gaatggggcc ctggcctggg aatggaggca ttgagacatt aagggagatg
84001 tgcattccag actcctcctc ctcaagtgcg ggaggtgagg aatagggaga gacggacctt
84061 ccccagcacc ccaactccca tccccatctg tctcttcctc accactgtgg gagtgtccat
84121 gtccgggttg ctccagagtg tggtgagaat gaggttctgg aagaaggggg gaaaaccaga
84181 tgagggaata tctgacacaa tgagtgtgta tttgggggaa tatcgaaagg tcagggatca
84241 taaaggagaa agaacccttt tcctccaaag aacgaaatag gttagatttg aggtagaagt
84301 cagaggttac ttaccaagag catgaggaat gaggtgcagg aaagcatctc ccaggagccc
84361 accggaagca aaactgagca agatctgaag tagagagcga tgccggggag agttcgactc
84421 cacggggata aggaagagga caaaaaatgg agctgctgag atcagcactg tggcccccag
84481 tgcctggtga gggagagtca aggtcaagta ctgtggagtt tcccaacaat ccagaaccag
84541 cccttctctc tcccatcccc tggagactca cataagccca gagagtgaca gcatccaggt
84601 cctgcttgat gcctggagcc ccagactccc catagcctcc atggctatgc tcatggtcat
84661 gtccatgtcc tctgtggtag aggctctcat gggagtagcc atggctatgg ccatggtgta
84721 aatcctcatg tgaatgtcca tggtcgtgat cgtgggtatg tccatgccag atgctctcgt
84781 gagtgtggcc atggccatgg gcatggctgt ggccatggtg gaaatcttca tgtgagtgcc
84841 tgtggctgtg gccatggaag tcctcttgca gatcgtcgtg caggtcgtca tgacccccga
84901 gtccagccac cagaagcccc aaggtcgccc aggtcagcag tcccacggcc acccagtggg
84961 gggcccccag gcctctggcc atcacgctga ccagagggac agaggcagtg actccacttg
85021 actcaactct atacctacac agggtccgct ttactccaat cgcgccctcc gtccacctct
85081 atgggccgct ctcccattcc cttaagttcc cacctacatt gttcccaaga ctagttctct
85141 ttatcccgaa ccacctcccg gatgtagggc cctgggacac taatctttca ccagccactt
85201 tacagactct ctgggccctc gtctctacga ctcattaggt gaggaaaagg cctggaaagg
85261 atggtagaga aaaaacagag aattctcacc ggctccggga tcctctcctt tcccggtttg
85321 cttggacgtg gcagtcccgc ctttagatcc cgcgagaaca ggaagttccg ttccaccttc
85381 gccctaatgc ctttaccaat atggcggcac tccggtagcg acgaatccat cctgcactga
85441 cacgcatgcg cgagtgtgga ggtgatttga ggggagggtg ggcgaggcgg gctaacaggc
85501 cggaggagac cgcctcctcg gtttccaact agacgagggg gcgggaactc gcgccgagac
85561 ttcccgtctg tacaaagatg gctgccacat tggcgctgtc attttggtac tgagcagagc
85621 gacgggctta attcgaccca atccaggcca gagtctttct ctcaggggct tcctcgtgct
85681 cagctaatcc tccgatcaat ccttgggaat ccctgggacc tcttcggtat ccctactctc
85741 agccagggat catgtcttgg gccgctcgcc cgcccttcct ccctcagcgg catgccgcag
85801 ggcagtgtgg gccggtgggg gtgcgaaaag aaatgcattg tggggtcgcg tcccggtggc
85861 ggcggcgacg gccctggctg gatcccgcag cggcggcggc ggcggcggtg gcaggcggag
85921 aacaacaaac cccggagccg gagccagggg aggctggacg ggacgggatg ggcgacagcg
85981 ggcggggtga gtgccccagc agaagggcag gcgagccagt gaccgcttta tctgcgaccc
86041 ctcccccaca ccctcccccg gtgctctttt ccttgcgctc ccgccccctt gtttgtaaac
86101 acgcgtcccc tccctcccga gggccttagc accctccccc ccggggcggg gcggggaagg
86161 ggagcttccg ccctcctagc tgtgaccgct tgaggccttg ggcaccggag gtcgcgggcc
86221 tgggaagggc acgattcctc ttaaacctca ggactttggg cgtttacagg cagatcctgc
86281 gtcttaggag gggtctctcc tgcctgtctc tttcccttgc acgctctggc tacctggctt
86341 tacgctggga atagaggggc tcgatggtct accccacgtg ctgccccacc cacgagacag
86401 gtgtggcttt gggatgacct ggtattcatt caaatggatt ggttggaaaa ttttcctcac
86461 ctgaactatc ctcctctcct tgtcaactcc ctcctctcac cacaatgagg agttacgctg
86521 tttttgggtt tttttagcca gtcaaatata gcagtgggag gttgtatacc aattttagtg
86581 acacaaatgt taataagttc tgataaccca ctaccatcgg accagccgga gttacactgt
86641 tgtttgacag cagggtgtct ctgaacttgg ggatgggtgg agtgggctgg gtccggcacg
86701 gtggggtatt agagaattaa agtctctagt tggatgtttg aagactcatt ttcttttttt

86761 gtctcttctg tctcttgtgt gtctgtgtgc ctgtacatac ccctccctca gactcccgaa
86821 gcccagacag ctcctcccca aatccccttc cccagggagt ccctccccct tctcctcctg
86881 ggccacccct acccccttca acagctccat cccttggagg ctctggggcc ccacccccac
86941 ccccgatgcc accaccccca ctgggctctc cctttccagt catcagttct tccatggggt
87001 cccctggtct gccccctcca gctcccccag gattctccgg gcctgtcagc agcccccagg
87061 tgagaggttg tgaccaactt actgcctggc cactttcatt ttcttgtttt ccttgtaacc
87121 ccagatcctt gtgtgaactt ccccatggcc cattgacctt ccaatcctct aaccttgtca
87181 ggcctgtggt ctttatgaga ccccttgcct ttcctgaggt gactgatctt tcagtgcatg
87241 cttcctcccc taaaagaact ctaatattct ttgtttttgt gtgtgtgtgt gtgttttttt
87301 tttttttttt ttttgagatg gagtcttgct ctgttgccca ggctggagtg cagtggcaca
87361 atctcagctc actgcaacct ctgcctcctg ggttcaagca attctcctgc ctcagcctcc
87421 tgagtagctg ggattacagg tgcctaccac cacgcccggc taattctgat attcttgatg
87481 tccttttcat ctatctgatt ttctgccctt ttgatcccat ctgctttccc aagacacctg
87541 cccttcaggg tcactgtgtt gcacaatttc agggggggcc tgtttattca ctttggggtt
87601 gtgctccagg gatggccttt cacatagact gcagtgtaaa tgacagcctc tggaatgtgc
87661 attgcagggc cttgcttagt ggtagggaat gatttccatc acttctgtga cattctgctt
87721 cccaataagt cttcctgtga cttccctatt tcccccatcc cagattaact caacagtgtc
87781 actccctggg ggtgggtctg gcccccctga agatgtgaag ccaccagtct taggggtccg
87841 gggcctgcac tgtccacccc ctccaggtgg ccctggggct ggcaaacggc tatgtgcaat
87901 ctgcggggac agaagctcag gtatgtggct cagaggatga acagagaggg agagtctggg
87961 ccatgtatca tcacctgtgg gattcccagg gcttatggag tttggtcaga gcaagtgacc
88021 tgggggaggc ctgatgggag taaagaagct gaagctgaga tgtaggacgc gattgggggg
88081 aaggtcagag ggaaaaggaa gcagcgtgta gggtttctga acagtgagga gactgggact
88141 ggatcatcac tcagctcctg gttccctatc tctgtatttg gcaaacaggg aggactttaa
88201 gttttgctag ggggagattg gaaacagact aaatggtgaa ggtgtctcca tgcaaccttc
88261 ttattgctcc tcccctttcc ctgtaggcaa acactacggg gtttacagct gtgagggttg
88321 caagggcttc ttcaaacgca ccatccgcaa agaccttaca tactcttgcc gggacaacaa
88381 agactgcaca gtggacaagc gccagcggaa ccgctgtcag tactgccgct atcagaagtg
88441 cctggccact ggcatgaaga gggagggtaa ggacccgccc tgcccaggcg tcttagacca
88501 catgcccttc tcccttatcc cactcagact gtgtacctct ctggaagggt ggactgctct
88561 ctctcctcta gagggcgata tttgatttct ctctcccttc gcagctctcc tccctgttac
88621 cctgttctgg cccttgagtt tccttcttaa tggttaggcc tctgtggcct gcacatatga
88681 cccctcccta gactgatcct aggataggat aacttttcct tagccatgat gggctgtctc
88741 cccatttcta acctgagtgg ttcctcctct gattccatgg accttctctg gtctctgtct
88801 cctgttatct gtgatctttc ctctaactcc tgggactggg cactcttaag cttacagact
88861 ttactgaaga cctcagggct tcccttcctt cttctgtgtc tccatcttat cttcttaagt
88921 cagattccta ggattctgag agacacttta gagcatatct tggtcaaaac acccacttta
88981 gagaagatga gcctgaggct tggctttccc aaggtcatgc aacaaaggag tggcaggcag
89041 gtttgggatc agaacccaca tcttcaaaga cctaggccag tgctcctttc actgcctatt
89101 tgttgagtca gaagagcaca agatttgcac tcagacacac ttagagtcag ttgtatgacc
89161 tgccatgtat tatatgaatt taagcaaact aatctctttg aaccccgagt tcttcatctt
89221 aaaaatgaga ataaatgctt ccagcatcat tatgataatt gagtgaatgg taaaatgtgc
89281 ttagcatgtt tcttggcata ttttatttaa aaaaattttt tttttcaatc agctttctca
89341 ggttgaatct tggcatgttt tttttaacca tgtagtaggc tctccattta aattcctccc
89401 taattctttt acctacctca cccttcccga ctgacttacc tgccctttta taacccttcc
89461 ttcagggctg cccccagtcc accctttcta gtgacttccc caattcctat aaatatctcc
89521 caagggccat gagatcctga tgaggccgta aggatatttg tggaacccct tcatggctgg
89581 ctgctgactt tccatttttt ctctccccct tccccctgca gcggtacagg aggagcgtca
89641 gcggggaaag gacaaggatg gggatgggga gggggctggg ggagcccccg aggagatgcc
89701 tgtggacagg atcctggagg cagagcttgc tgtggaacag aagagtgacc agggcgttga
89761 gggtcctggg ggaaccgggg gtagcggcag cagcgtgagt gttggggtca atccactctc
89821 cttcgtgatg ggggttgggg gaggcagtct aggtctgttc tacatcccct ccccctcctt
89881 tcccctcata accttcctaa cactacttgg gactggaggt gctgccaaac aaggtctttc
89941 aaacatctga ggtggatgtg atagctcctt ctgtctccac tccccaaaca acccactggc
90001 agaaccatag gcatgtccca aataaataat tgtttgcact aatgccagaa gagaagactc
90061 acttacaggg attggtttgg atggggctca caggaagact atatgtaagg agggggtgtc
90121 aaaagcctct tacaaggggg ctcccagcat atctcaaaat cttccataac tcttaccccc
90181 gtcccctgca gccaaatgac cctgtgacta acatctgtca ggcagctgac aaacagctat
90241 tcacgcttgt tgagtgggcg aagaggatcc cacacttttc ctccttgcct ctggatgatc
90301 aggtcatatt gctgcgggca ggtcagtgac cttggatccc tttgacttct tgacatttga
90361 cccctctttg acttcccgat ctttagtgac cccagtggcc ttaccttgcg tacccaggga
90421 gccaaacttg ctgacctcgc cacctctttt ctccttctct tccactgatg tgctttgaat
90481 cccttggcct gatttctggc tcctgaccct tgctgcccca cccaggctgg aatgaactcc

90541 tcattgcctc cttctcacac cgatccattg atgttcgaga tggcatcctc cttgccacag
90601 gtcttcacgt gcaccgcaac tcagcccatt cagcaggagt aggagccatc tttgatcggt
90661 cagtggccct cggctaggct ggcatgtaga tagagggggt ggggctatag gctggtccgt
90721 gtccaaggct ggctgagctg tgacctttga gtgacctgca ggtccctctc cagggtgctg
90781 acagagctag tgtccaaaat gcgtgacatg aggatggaca agacagagct tggctgcctg
90841 agggcaatca ttctgtttaa tccaggtaag aggaggatta cctttgtctc tcaaggccaa
90901 ggcaaccttg gagcctcctc ttctccgaga ctcctaagtt acccagctat ccccctcagt
90961 aagaccctca acctggaaat ctcccaactg gccgaggaaa aacccacatc cacggatcca
91021 tcccacaaac ccagtgggcc ctgcttccct tgccaggcct ctggtaaagg gcaagcaagt
91081 gcagcccaaa aggggcatcc tgtggctcac atctgcatgg ccatcctgat ttggtttgtc
91141 ttcatttctc ttcctcctct cttccctgcc atcccagatg ccaagggcct ctccaaccct
91201 agtgaggtgg aggtcctgcg ggagaaagtg tatgcatcac tggagaccta ctgcaaacag
91261 aagtaccctg agcagcaggg acggtgagat ggggctgggg ggcactgagg ctcccttggg
91321 ggtggggagt gctatgagga tgtgttcagg gcccgtgtgc ttccagctct caatcccctt
91381 ctcccacctc caccccaggt ttgccaagct gctgctacgt cttcctgccc tccggtccat
91441 tggccttaag tgtctagagc atctgttttt cttcaagctc attggtgaca cccccatcga
91501 caccttcctc atggagatgc ttgaggctcc ccatcaactg gcctgagctc agacccagac
91561 gtggtgcttc tcacactgga ggagcacaca tccaagaggg actccaagcc ctgggcaggg
91621 gtggggggcc atgttcccag aaccttgatg gggtgagaag tacagggcag aaccaagaac
91681 ataaaccctc caagggatct gcttgatatc ccaagttgga agggacccca gatacctgtg
91741 aggactggtt gtctctcttc ggtggccttg agtctctgaa tttgtcgggt tctcccatga
91801 tttggggtga tttctcaccc tctgtccttc ccccagcaca aagcactggc cttgcctcca
91861 ggaccttgct tccttctcat cttgcctcat tttgcttccc atctgaagag tggaaatggg
91921 gaactccccc agaggtggat actggggggc aggcctccca agctgatgga catgagagta
91981 gggccctgac aggccttcct cctctcaaac ctggcagatg ggggcctctc tggaagaggg
92041 aggggccctg tcactgtcca gagtctcttt ttacacttca cctccttctg cagtcagact
92101 gaaatataaa aaaggtggtg gtggtggtga aggggctggt ggagatgtag gaaccgatct
92161 gctattttta atttcctgtg aggatagaga cttgcagtta gactcaaaga agtactgtac
92221 tttcccaggt tgactaagaa atgccagtgg tggaggtggg tgtttgggaa aggcagggcc
92281 ctgaaatggc ctgtccctag ggctctccaa gcactagcct tcccagcttc ccgccgcccc
92341 ccctatctct tcctgtctaa cttggggaag gggcctgggc tgtgaggaca gggcccccac
92401 aggggatggt ttcacgagtg tagtcccgga ggccttccct ttacagctct cctccagccc
92461 tgggcacata gcataggctg gggacacagg atcctggcct gagaattgag gggaggtggc
92521 cagcccgcag aggtggggtg ctggggctgc atgatttttg ccctgcgtcc cttctctttg
92581 gggctccttt cccctctcat acataaaatc gctttcaaat taaaatcgct gtttctgga
92641 ctgaggtgac tgtatgagga tgggcagcgc cgtttcgggc ttgggggggg tatccggttg
92701 ggagctccgg acgcgatccc tggacgccgc cgccgaggtt ccgtggggcc caagaggtgc
92761 acgcagggtg ggggacacaa ggcaagcttt gtcggggagg gggggagagg gtgtgccagg
92821 ctgggggcgg ggccggccgg aggaggaagg gggggcggtg gattctcaaa ggcgccttgt
92881 tcgctcgtgc cctctccgcg ccagcgggcg gcggcgcctc ggctcgctgc ggccttccct
92941 ccgggcgcgc tgcgggctcc gggcagaccc ggcgccgtgc ccgctcgtgg gggcgcactg
93001 ggcgccgagc gctgcgtgct tctcaggcgt tgcccgcccg gaggcggccc acgtccccga
93061 gtgaccccat ttccctggac ccttccaacc agagtcgact gctcgctcat cttaccactg
93121 ctccccctct acctcggctc tgggactccc taaccgcgtc ccccatttgc atctccttgg
93181 acctgactat cctgtgtttg tcggtgggtc ccagtctctg gcctcttgct ctccgtagaa
93241 caccttcccc cacatccccc gccccagttc gtcgctatct atagccttgt ctataaatac
93301 ccccgccccg gcccggctct gtaattacac ggggcggggt gagggaagta attatggaga
93361 cctgattatg ggtggggtgg ggactgcgac ccccaggccc gcctctcccc ctcccactgt
93421 gccctaaatc ccgcccaggc ctctgctgaa agggggctct gggcccccaa gagggaggga
93481 atgggaggga gtgtgtgtga ctggacgttt gggtcctaga aaaggaaggg gctagggaag
93541 atattggggt tcccgaaaag agaatcttag ggtacaggcc gttgagacct acaaggggca
93601 ggagagagcg agcgatagag ggagggttcc cgcctccctc cccaggtgga gactgagggt
93661 ggggtttccc ttcggtggct gtgggcgggc ggctggaggc gggggccggg ccgggggcgg
93721 gggcgaggtg ggggctctgg gcgccagggt ggccggggac acacagaagc ggcagccacc
93781 gaggagggag cagtgccggg agccccgacg gcgccttgct gcatggagct gggccgctga
93841 cagctgtcgc tgcccgcagc ctctgacctc cctgggaccc cggcgtctga ggctcatagt
93901 ctgctccctg tcttctgtca gcctcagggc atccagcgtc tcaggccgac ctgggtccct
93961 gggacccggc gtttcggctt ctcagccatg gagcggtgca gccgctgcca tcgcctcctc
94021 ctcctcctac ctctggtgct ggggctgagc gcggccccag gctgggcagg taagaggagt
94081 cctgatgcct gggtcctcgg agtcaggctg gagctctgag tgtctctggg aaggggccag
94141 ctgatgcctg gggcagcagc ttctgagtct aacaaggaga tttggggctt caaggctaca
94201 ggctggaggg caggacacct gtgttccttg gcatcagggt aggaatctct ttgtccttga
94261 agtgactggg ggagggcaga tagggctgaa aggtggagga ccactttaga gaccctgaat

94321 ggggatggat gcttgtcttg agtccttggg aagagctgaa gatggccagg gccagcccgg
94381 tccagtcatt gctggtgtgg ggatggtaga cgccaatgtt gatgggtgag gcgtgtgtgt
94441 cttatggcct ttctgtcgat atctctggga taaggggtga gcacctgtga ttcagagcag
94501 agcccagtaa gcccagaaag aaaggaagcc ttgactacct gtctcattgg cctcctggac
94561 agggtccccc tccccctccc tgtgccctcg atgctgccac gcagctggct ctggggagca
94621 ctggggctgg ctgccaacag gacggccctc cggacaggcc aggatcaggt gtctgaggcc
94681 agagccaact ctttgcattc ctgcctagcc cctcctccct ctggccagcc aggctcccct
94741 gggaccctgg tgcccacaac cctgatcttt tccttctttt tcccaggacc cagaattcct
94801 ggcttctagg aaaggggatt tgatgtcaag gagggggctg tgagcaccag gtcctcagca
94861 ggctggaaaa agcccatttc tggagacact gagggctgat ttatatttaa cttgactatt
94921 caattcttct ctgctttaga gagaaaatgg tcagaatggc agcacaaaag atttagatta
94981 gattacagaa ggaagaactt ccaagatgtg aggacagtga agcccagggc caggaaattg
95041 aggcgtatgg tgcctgtaga ctctaggagt ctttcttagg gggaagaggg atcctctggc
95101 atggaggcag ggggatggct aagatgactg tttcttattt caggggctga gtgggtagag
95161 atgggacttt agggaaatga gttactgtag aattcctaag gttagagaat aaggcatttg
95221 gggggccaga ggtagagtgg tggagggtaa tggggctacc agtggagagc ctgtggggca
95281 gatgaggctg gatacagggg ggcaagtatg tggcctggtg tgggaagatg gtgggcggac
95341 ctgtgggtag gttgtccata tgatcgaatt gtactccctt gggatttggt ggagggtggg
95401 gacctccgag gtctccttgg cctggggtct ctgtgagtct ctgtgtctct gtctcactca
95461 cccttcgtct ctcaaagcgg cccttgtgtg ttggtgtttg cggagctgcc acacgcgcag
95521 gggccaggct taggtggggg ttggggggga aggccgtggt ttcccggctc ctggactcaa
95581 agggcctttt ctctgcctgc ccgccccacc tcacccaccc cacccagctc cactctcctg
95641 attgctctgg cctctgccct gccccacact tctatgaaag ttttgctgga actctctttg
95701 ggatttttcc aatctggagc tgatggtgtg aagagtatgt gtatagggga gcaagtgagg
95761 gcaaggtgat cttttgtttt ccagggagtt tgagccctag gaagctgagt tctagttaga
95821 cagggctggt gggctggatc ttgaatcttt gggtcccagg tggtggggtt ggtgggggtg
95881 gtgagacagg attgaggttc caggtgtggg gagcttgcct gggttctggg caagcactct
95941 gatgggatcc acagatgtgt ggctcagggc cctggacctg ttccggctcc tcgtggcagc
96001 ttcagcccct gccccccatt cctccacccc tccccaactc atctcctaaa ccaggaggca
96061 cagactcctg ggagagcttt cttgtatctt tttcctcagc atgctcaggg ttgttcccac
96121 cccatccccc aaacctggga tctgtagctg cttgaagccc ctctcataga gctgcttctc
96181 tgatctcttc ctcgtggccc ccctgcacct cctgtgtcca ggatgatcga ttgcccagat
96241 gttgtttgtg ctggagacgg gacagatgcc agagacccag agagtgagag atatggaaag
96301 agagtgagag cgagggagag aaaaagatgg acagaatgtg agagaaatgg aggaagacag
96361 aagagacaga gacagaaaga ccaacataaa gagacagaga gagtaagaga gtgagagaga
96421 caggagacag atgaagagtt gagagaagta ttcagagagt acagagagag acagattcat
96481 aagctaaaag acaaagagag agcgtaagag aggcagatgc taggagagac aggggcagga
96541 gtgtcatggg gttgaatgcc agctgatgct gccctgcctc caggtacctt tgcatttccc
96601 atccattcag gtactgttca gaagcaagtg agggtgaggt gacctttccc ttgcagggag
96661 tttgagccct ggaaagctga gttctagatc ccaaatgtcc cctatttatg ccctcctgag
96721 ggcatgtccc ttctcctgat aatcatggac tctcccaggt gcaccccctg tggatgtgct
96781 ccgggccctg aggttcccct ccctccctga tggtgtccgg agagcgaaag gcatctgtcc
96841 agctgatgtg gcctaccgag tggcacgacc tgcccagctc agtgcaccca ctcgccagct
96901 tttcccaggt atgggtgaca tggtggggta ggcctggggg gaggtaatgg gatggggcct
96961 aggatcagac accaggagga aaggggttgt ggcggctccc tttgcctctc actctgtgtg
97021 tatctctctc ggttactagg aggatttccc aaagatttct ctctgctgac tgttgtccgg
97081 acccgccctg gtctccaagc tcccctcctg actctctaca gtgcccaggg tgtccgacag
97141 ctgggcctgg agctgggccg acctgtccgc ttcctgtatg aagaccagac tgggcggcct
97201 caacctccct ctcagccagt cttccgaggc ctcagcctag cagatggcaa gtaagtttgt
97261 ttgctcctct ggtctgcctg gcccacactt tcaggaggaa gtgcccccaa acccctacac
97321 tctaaactgt gaaacccttg agaccctttg ggccacacca cacctaccca ctgcccaaac
97381 ttcagtcact tctagtccag agtttgggct ttagagtgta cagccttctc tgcatgttga
97441 actagcctgt accttgggca agttacttaa aatttttgag cctcagtttc tacatctgta
97501 aaatagacat taaatagaat tggcacaaat aagaaagtga cggcatggtg tctggtgcac
97561 tgtaaacact caataaatgg tagccattgt tattactgct cttatcacca ttggcttcaa
97621 ctcttatcac tgctctccaa ccatgttgac tcccctactt cagtcagcca agagttcact
97681 tgaacctctt ccaccatttc caggtggcac cgtgtggctg tggctgtgaa gggccagtct
97741 gtcaccctca ttgttgactg caagaagcga gtcacccggc ctctcccccg aagtgctcgt
97801 ccagtattgg acacccatgg agtgatcatc tttggtgccc gtattctgga tgaagaagtc
97861 tttgaggtaa ccagagcaat cagaggcagg attgacttct ggtcccctat cttgtgccca
97921 ctacccttct ggccccagca tgtcatcttc ctattcccta ggccttcatt tttttttctt
97981 atgaattttc atttctattt tctatcttca gggctactgt tctctgcagg agaactagat
98041 gcctacctat atggcaggac tgtccacagc cacttactct gtccttcagt cttcatcagt

98101 tccctcatcc atccatcctt tcaacccctc tctctcctca cccacccatc aattcactca
98161 ctcctcagcc aacctatgca cacatccacc acccactccc ccatctattt actcatctat
98221 gcatcacacg cccacctagt catgcatgaa ggcttatatc cacccacctg tcaatcctcc
98281 ccctgcttaa ccactctccc cttcaaacca cccatctatg cttccaccca tctatcctct
98341 tcaactcctc catccagcta ttcactcacc caccccacct ccccatctac ccatgcacac
98401 aaggatgcat gcatccattc agccaccatc tatccattat ccttccacca actcaaccac
98461 tccctccttc aatatactca ctcacccatc tcctgaccca cttacttccc atccgaccag
98521 tcattcaccc acataagttc acccatctac ctattcaccc atttgcttcc atctactctg
98581 taacccctgt tcatctgccc accagtccac cctcccatac gtacatttat tcattcacta
98641 tttatcaatg ctcccattca ttaatgcatg taagccccca gccacccatc cactcatcta
98701 tccattcatc catccctcca ttcacccatc cctccattca ccctccaagc cagtcaacat
98761 tttctgaata cctgctggac aaggagaaca aagaagagaa tataaagtct ctatcacccc
98821 caagagctcc caggtgggct ctggaaatga gacaacatcc ctaaattacg gtgcaatgga
98881 gagtatgcta agcaccatgt gtgggcaaaa gtaaatgcca ttggaactca ggaatgggtt
98941 ctccccactc catttctaga ggtccagaga attctcatca tcactgccat tggaaggcca
99001 aggcatctaa agctgagccc tgtctctgga agccactgtg gaagggctca tgcattgcca
99061 caaagcgcag aggggaggca aagaattctg gaaggcctgg gatctggtcc tgattctgcc
99121 actctgggtg accttggggc agtcattgcc tttttgggct tttctgaatg gaacgtatct
99181 gaaaaatggg tagaaagaaa gatccttgtt ctgccaactt tcctggttgt tatgaaggtc
99241 ctatgagatc ccttgaaaac tgaaaagggc tatgcatatg gaggactgac agtgaccttc
99301 tcccatctta gggttttacc taggattggc ctcctatact ctttctcccg gatgataccc
99361 tctgcctctc tctgaatctc tccgctccat ctctctcatg tctttgcagg gtgatgtcca
99421 ggagctggcc attgtcccag gggtccaggc agcctatgaa tcatgtgaac agaaggagct
99481 ggaatgcgag gggggccaga gggaaagacc ccaaaaccaa cagcctcaca gagcccagag
99541 atctccacag cagcaaccat caagacttca caggccacaa aatcaggaac cccagagcca
99601 ggtgagggag ctgggagaac ccccaagtgc agcacacccc agagagggaa gacacccagg
99661 catctctcct ccttagttgt cctccccacc ctcatctccc tattcccatc accccctcct
99721 cctagtcctc attcatcagc ctttttattt tcatctaaaa ataaaaagag ttgttatgaa
99781 gaatttgtgg ctgggagctg tgttccctgc tctgcgtctc ctcttccagt cttcctggaa
99841 ctgtgtccct gggttttccc ttccttcttt gaattaagag attgggagac agtgggagag
99901 gggtgagagc tggggaggca ggtaggagag gggactgaag ccaggagaaa gcagtcggga
99961 tggtgagacc aaggaggagg aatgggaagg agtagggatg gagggaatat caaaggaggg
100021 gtgggtctgc agaagtgtgg gagtggggaa cccagtctct ggcccccacc tacctggagg
100081 ggcaggaaaa ggggaggtag tagggggaag ggaagggaag ggaagggaag ggagcctggg
100141 ttggatgggg tctggagagt tagaatacag ataggctctg gggtcagtgg tctaaagatc
100201 agagggtcaa tctcttaatc atgtctgctt ctgccccaca tgtggaaccc cttccctctg
100261 ttccacctct ccccacctcc ctcctgaccc tcaacctctc ctttcatctt caactctctg
100321 tttcccaact ccacacctcc cccgtttccc aactcccaca ccatctttat atctccctct
100381 cccgcccttc accccacacc ttccaattac cccatcccct tcctgtctat gcccaccccc
100441 caatcccagc ccactgagtc tctctactat gactacgagc ccccctatta tgatgtgatg
100501 actacgggga caacccctga ttatcaggta aactcaaggg accccttcat ttcttccctt
100561 tctcccagca cccccagcaa tcatctccta ggacaccttc ctcccctccc ccatgccgtg
100621 gggagaggca cagtgtgttt agattatggg gatttactta gttcctgccc cctccctgtc
100681 tctctgctgc agcgccctgg gagcccccac cacaggcctc ccccttcccc tagggggcat
100741 cggacccctc cctgaaagcc taccccacct gccaagaggt caatgacctg gcaggcgtcc
100801 ccctcaccag gccggaggcc ctctccaact gcaggctttg acccagcatg ggagcagcct
100861 cccctttccc gatgcccagc tcggagggca gcttccccca ctgcctggcc ccgccccagg
100921 accagaggca gcagttaccg gcaggtagag ccggggcatc cagatctctg cccccaaacc
100981 tctatgacct tggaggttca ctctccccaa actatccccc tccaaagggg ctgcaagatc
101041 tgagatttcc cccatccccc agcacagaca gaccctgccc tcgggggaca ggtgctgctt
101101 tgcctccctt ctgctaacct ttctccttcc ttcccacccc gtgctactct tctccttctc
101161 tccttggggt caggacccca ccccaggtga agaggaagaa atcctggagt cgagcctctt
101221 gccacccctt gaggaggtaa ctctatgccc caccctcacc ccatctgggg gcagtgggca
101281 cctgtgactc actcccctca gcccatgacg tcttgatgga ccagcccctc ctctggccgg
101341 agggttcttc ctcatttcca tcgatggcct ctgtctttgg gtcactctag aactatgggc
101401 ctgcttgact ctctaagtcc agcccgttcc cttcagtatc caggcctcct tccatgattc
101461 tttgattcct gagtgacttc gtctccatgt tggtgtctaa tggtcttact ttctatgtcc
101521 tatttctgtt cccgggagcc actttctgtc cagtgttgtc accttttgtc ttccctatct
101581 ctttggtcat tcctggactc ctttgtctgt cattgtgtcc atggactgtc cactctgcat
101641 ccagtcctct ggccaccctt cattttatcc accacttctt cccactggac ccactttctg
101701 gacatctctc cccatttttg gcctttgatg gcccttccat ttatatacaa ccactcccat
101761 ggctattctt gggagaaaaa atccagttct gattaacctt ggaatccttt gggctagaag
101821 ttgtcacacc ctggggtcca gggtaagcct cctctcagcc ctaacatccc catctacccc

101881 tctccctccc catccctggg aaggagcaga cagatctcca ggtccccccc acagccgaca
101941 ggttccaggc agaggaatat ggggagggtg gcacagaccc ccctgaaggg ccctacgatt
102001 acacctatgg ctatggggat gattatcgtg aggagacaga gcttggccct gccctctctg
102061 cggagacagc ccactcagga gccgtaagtg aaaggagctt ctctttcatt cttgactacc
102121 agtgggagct acaagaattg cttcttgttg gtttcgatcc ttggctgttg gctgtgagct
102181 atttcccatc tgtgacttgt cattttctgt tcacatatga tgtatatgtt gcttgaaaag
102241 attactacct gccttccttt gccaaatgtc ttaccttgtg actcttgtgg atacttctga
102301 agctcagggc aattgggaat taaaaaaaaa aaatatgtag ccacttcctg tctttcttga
102361 tcacttcctg tctaactgaa gctggatttc ataaagcttt tggtcaataa actactacct
102421 tgtgtccatt ttcagtgttt ctctatcatt tcttcttggt gaaataattt tttgttcatg
102481 tttctaccaa acgatctaac ttcctatgtc accttttttt aacctacagg ccacttatta
102541 tctgtccctt cctgccttct ccagctgtgg catccatctt ggtaactgtg tcaggttgga
102601 ctgcaaagga tttattattt ctgttctggc tctagttttt catgctatct tgtgacaatt
102661 tcctttgaca ataagccact tcctgtatgt ctcaggtcat ttaaatcata aagtttccat
102721 tgtgggcctc gatatttctc atggctactt cctgtgtgtc ctagccttga tgtttgtgtt
102781 ttctggaaga attacccttc actgtcatga ttccctttgc aggagtcaca ttctacctgt
102841 tttgtgcctt tcttttctct ctgggtcacg gtagccatgt tgttgttgaa ggttcacttc
102901 ctggatcctc tttccttcac tgcttcctgt ctgttcagcc acttcctgtt tgtccagttt
102961 gttttctagc catgttggcc atgtttaatt ttcttcggcc ctggcatcat tcttgcttcc
103021 aagtgtttgt cattttgttc tgtgtccctt agttgttctc atatcttcca gtattccctg
103081 tcagtgggac tgcttcctgt ctactccgct ccgtcttctg cctgccttgc agttctggct
103141 gcctcctcta ccctgctgcc gcatcccata cctcccctct cttgcctccc ttgagcttct
103201 actgcctttc ctttatgctg ctctctgggt atttggggtt gatatgggat ttcagagaat
103261 tttgaaacgg atcattgctg tttggtcaga atgaaggtgg agaggggcat tctgaagatc
103321 tttgttgggg ggagttgggg tggtggtgaa tgtctcccat tctccatgtt cttattttgt
103381 ttctggccct agagttggga acaggtatct gagtggaggt gtgcaaggga gggaactggt
103441 gggcttggaa tgtacttgag ggccagagga agggcaatgc attcagtggg gactgctctc
103501 caattttttt tttttttgag gcagggtctc actctgtcac ccaggttgga gtgcagtggc
103561 aggatcttgg cttactgcaa cctctgcctc ctgggttcaa gcgatcctcc tgcctcagcc
103621 tcccgagtag ctggggttac aggcatgtgc cactgcgccc ggctattttt tgtattttta
103681 gtagagacgg ggtttcacat gttggccagg ctggtctcaa actactgacc tcaaataagc
103741 tgcctgtttc ggccccacaa agtgctggga ttacaggcgt gcgccaccgt gcctggcctc
103801 caaatttact tagagtatgg atgcttaacc tttggggcat atttaaactt gtgaaccctt
103861 tgaaatccta aataaaattc tgtgcataat agcatatact ttttcatttc tggagaaaga
103921 ctcacagctt tcatcagatt cttttttttc tttgagatgg agttttgctc ttgttgccta
103981 ggctggagta cgatggcata atcttggttc acttcaacct ccacctcctg ggttcaagct
104041 attctcctgc ttcagcctcc caagtagctg ggattacagg catgtgccac cacacccagc
104101 taattttgta tttttattag agacggggtt gcaccatgtt ggtcaggctg gtctcaaact
104161 cctgacctca ggtgatctgc ctgcctccgc ctcccaaagt gctgggatta tgggcgtaag
104221 ccacctcgcc cggcctagct tttatcaggt tctcatgtgg gtccatgccc tcctcttacc
104281 ccaaataaaa atctacccac aatcttagag agattctttt atgatgtatt ggggtaaatg
104341 tgtggaaggt ttcttaaggt tctagggagg gagtttggga gatgggatag ggtcctctag
104401 aagagggttt gggaaggtgg gtggtttaaa atccctgaaa ggggccaggt gtggtggctc
104461 acgcctgtaa ttccagcact ttaggaggcc aaggcaggcg gatcacctga ggtcaggact
104521 ttgagaccag cctggccaac acggtgaaac cccatctcta ataaaaatac aaaaattagc
104581 cgggcatggt ggtgcacatc tgtaatccca gtgtaatccc agctacttgg gaggctgagg
104641 caggagaatc acttgaacct ggaaggcaga ggttgtagtg agccgagatc gcgccactat
104701 acttcagcct gggtgacaga gcaagacttc gtctcaaaaa aaaagaaaaa caaacaaaca
104761 aacaaaaaca ctggaagttt ctggggacct tcctgaaaga ggctctcagg gagatggaga
104821 ggttgtgttt gctgaggtgg ggctgggaac tgggggggca gggaaccctg aggtctcctt
104881 ggcaggcttg aaaggtttgt gaagagggag ttttagggga gggctgtggg gctttcgggt
104941 agggctgaag tggtctcgag aggatctaag aatcaaggtt ggagttgaga tggagaaagg
105001 cctagtgtcc tggctgctca cgggccccac tgggggctac atgtgtgtct ccttcaggct
105061 gcccatggac cccgagggct gaagggagag aaaggagagc ctgcagtgtt ggaacctgta
105121 agttatgctg gtcacagggc tgaggcagtg gaaataggag aagcaagtgg ggttgagtgt
105181 gctggtcctg tcgcctctga tttttaacct ttgactccac agggtatgct cgtggagggg
105241 ccccctggcc cagaaggccc tgcggtaagt ctagcagtga cctggtggcc attcttttct
105301 tagaaacccc ttctatgtgc tcatctgagc cttccccaca tatgcccagg cctcctcctc
105361 agaactcggg agcatccctc caatcaacgc ttcccagatt cccagatctg ttctgcacag
105421 accactcctc agccacaggc tgagtgtcca cacctgtctg aatgcccaca cctgacccct
105481 actcttttgt tcctcaggga ttgattggtc cccctggcat ccaggggaac ccaggcccag
105541 ttggagaccc tggagagagg gtaagggggt gtcctccatg tgacggggga gcttcggggg
105601 agctagtggt atccaagcgg ggggctacca tgcccagtaa tttcctgtta ggtggcttgt

105661 taatgaatga ggggatggtt gtgcttccca gcaaccagaa aggagggcag actctggttg
105721 gggagggtgc tgatctggtt tctcaccctc aattttcccc atgggggtgg gagactcagg
105781 aaagagctag agcaatctac acttattctg ttgaagagga tatttcaaaa tcatgctaag
105841 atcttgatgt ccccttatct ttcaccccat cttttgcatt ctcttcccac tccagggccc
105901 ccctggccga gcagggctcc ctggatcaga tggggctcct ggtcctcctg gcacatctct
105961 catgctccca gtgagttgtc tcttgggttt tggaacatgc tgatggggaa gacaaggaat
106021 tgtgtcatgt taccaagaac cagatgggca ggaaagatat ggaggagtct ctaaagatca
106081 tcaaagatca tcactccaaa gggattcgtc tttggggttg gggagatggg gcctcattag
106141 gtgacctaga acaaaacacc agtcttctag gactgggcag aacagcctat atttcaggag
106201 ctctagaacc aaaagagagt ctttctggag gcagaggctg gacagtggag gagggcagga
106261 gttgagtttc tggaatcctc taagtgatta tctggaagcc atgggaaatg ctggatgggc
106321 agaacctggg gcaaggggca gaaacgctgg agaaagcctc tggctgggag ggtcccatgg
106381 ctttcttgga agagaacctg gaccttctct ccttgccctc agttccggtt tggcagtggt
106441 gggggtgaca agggccctgt ggtggcggcc caggaggctc aggcccaggc gatcctgcag
106501 caggcgaggg tgagtggggc tgcttccctg gaaaggaaag gctctggggg gcactggaag
106561 ggttggctgg agtgagtgtg cggcagggga ggcctgggtt ggtttggggt taacagggag
106621 gttggggaga atcctgggca gtggatgagg atcaggagag gaagggtgaa tttggagagt
106681 gctggggctg gggttcccca ctgcctgtcc ctcagctctg ctgtcccccct gctcccctag
106741 ctggcgctcc gtggaccccc tggccccatg ggatacacag ggcgccctgg acccttggtg
106801 agtgagcagg gtgctcgggt ggaggatgct ttaattgtgt gtggggtgtg gatagttctg
106861 gaaaggggct tcttttgggg aacactccga ggccactgtt ggctgtgtcc ttccttacgg
106921 ccatcctctc ttctctctcc cagggccaac ctgggagccc tggcctgaaa ggagagtctg
106981 gagacttagg acctcaggtg accactttcc tccactgcac ccccatatct gttcaccagc
107041 tcagtctccc tcactcccct ccctatgcct cctaacccca ccccatctct cctctcacca
107101 gggccccaga ggacctcagg gcctcacagg ccctcctggc aaggctgggc gaagggtgag
107161 tgccccaggg gtggatgggt gttgtgggga gacaggccct gcagggtagg ggactgcggc
107221 cctgcttgtt ctgacacttc ccttgttctc ccagggccgg gcaggtgctg atggagcccg
107281 agggatgcct ggagatcctg gagtgaaggt aacaggcttg ggcccctccc tgaagcctgt
107341 agccttcagc ccacgctggg ctcagttgtt tcttggggat gacctagttc tccaggcttc
107401 cccaggatca gcaccccccc agctcagggt gcagtaggga ggggtgggct tggacagggt
107461 cgtgtcctca ctctggctat ccttcctcct cctagggtga ccgaggtttt gatggactcc
107521 cagggctccc tggagagaag ggccataggg tgagtacatt agtgggtgtg tgttgtaatt
107581 ggggatagat cttctatggg agtgaggata ggtagggaag ggaggctgga ggcttggcag
107641 gagctcagtg aaagtaatgg atgggctaag tgcagaggtt cggtgtctgc ctgtgttggg
107701 ggctctgaag cccctcttat gagttccccc attttgcagg gtgatactgg tgcccagggc
107761 cttcctggtc cccctggtga ggatggagag agggtaagtg tagtggcaaa tgtaggggct
107821 gggtgcaggg gaggtctaga aggcacagca gctttaccgt gtcctcctcc tccagggaga
107881 tgacggggag attgggcctc gagggctgcc tggagagtcg gtgagtgtcc aaggctggt
107941 gctgggggag ggggctctgg ggaagccagg agggggacat gagcactaaa gggacacagt
108001 tttatccatt catgccgtct cctcccctca ccgtgaatgc agggacctcg aggtctcctt
108061 ggccccaaag gcccacctgg tattcctgga ccccctgtaa gtgactccct gacttctgac
108121 cctgaggtga ccccttgacc tctccaactc tccacctttc tagctgcctt cctgctccct
108181 ctcctctctc ggcatgcttc ccattccctg tgagctcccc ctgctctggc cccacactgc
108241 cctgcctcag cattcgctcc ccgtgcacag ggagggccat gtggcgtggt gccctgaggg
108301 ctctgggcat aggaaataaa tcttcaagaa aaacaaaacc aaagcccaca agcccacaga
108361 tccacagtcc cacacactgc aggcctgggc cagctgtgtt tgcttgggtt tgggatggat
108421 tggaggtctg accgtcacac cctcgggaat ggccgggcct ctgaaaacca caggtccctc
108481 cgggccacct tcaggctgct cacacccatc ttgtgtttca gggcgtccga ggcatggatg
108541 gtccccaggg ccccaaaggg agcttggtga gtgatggata ggagatccca cccccattct
108601 tatcccccga ggtccctgcc agcatcctgt tggccgccat tttgacccct gcctgcttcc
108661 tgctcttgcc ttcttggcta tcgctgctgg gactcagctc catgctctaa atgtgttcta
108721 tctccatctc cttctgacct gtgcctggtc acctgtttct agggacccca gggagagcca
108781 ggacctcctg gacaacaggg cacccctggg acccaggtga gcggtgcccc tactccgttc
108841 cccaattttc atgacttccc ttgggcttcc acaatgggtg agaccctgga aggatacagg
108901 aaattaaggg tttcaggccc ttgttgctca tgctttgaat ccgggaggct ggatagaaga
108961 gaccctggga ggcccctgtg acttctcttt ccctgtatgt agggtcttcc cgggccccag
109021 ggtgccatcg gccctcatgg agagaaggta agtgactaag tgatttggag gacagggggt
109081 ttagagtggg gatgtaggga gaaccctaaa tgtctgctgg gattttgtct gtgtccagac
109141 atgacccctc tagtgaccct gagcctcttt gtcttcctgc agggtcctca agggaagcca
109201 gggctccccg gcatgcctgg ctcagacgga cccccggtga gtgggacccc acttccgaat
109261 ccagttcccc ttggccttcc accccagtgc atgacctcag agctccttta gtgacccttg
109321 cactgaaagg tcactggtgg tgcttgtcca tggacagttg gcttcactag gtcacaggaa
109381 atttgggttg gaagaagtgt agggaggggt gcagagaggg tgacaggggc cactggtcac

109441 attttctatc ccttactccc agggtcaccc agggaaggaa ggtccccctg gaaccaaagg
109501 aaaccaggtg agatcttcca tgtctttatc ccctgaggga gtctccactc agactctaga
109561 gcctcaaagt ccttgggatt cccttgtctc attattcata tggctctgcc acattctcaa
109621 ttcctgtgcc ccgtaaccct acccagacag caatgttgtt acttccattt cttccacccc
109681 tggaggcccc cacagtgaca ctctgaggcc cctttataaa tgcctctctt ttatcttcca
109741 gggtccctct ggacctcagg gacctctagg atacccagga cctcgagggg tcaaggtaac
109801 tgaccaccag gctgggagac aaagggctgt ggtcagggga gctatatggg gtcttaggag
109861 cttggtcctt ccaacccacc tccatcccct gatcagtcta cttctatccc ctctctaatt
109921 ctagggtgtg gacggaattc ggggtctgaa gggtcataag ggtgagaagg tgagcaccat
109981 gcccccagct tcccagcacc tcagtcctgc catctcctct cttcactgcc ttccctccag
110041 cctgccccgc actgccgtcc agctcccgag cccctgcttc cacctatgtt ctcagaattc
110101 ccattagaac cccagccctc tgtcatctgt ggtgccctct cacctctatc tttctcaggg
110161 tgaggatggc tttcctgggt tcaaaggtga cataggcgtg aaaggtgaca gggtgagtag
110221 agaccccaca ctggccccaa ttccgtgttc tactgagcct ccactctgga gacgccaagc
110281 accggtttat tctcctgagt ctccagactc cacagttcca gtgctgtgtt cccttgggag
110341 cctgacccct acaggatgat agccccatgg tctgtgtcat gcctgccctc ccatcactac
110401 actatcccta ccctgtgggc ttcctgtcct gcagtggctc ctgggacccc tctagccctc
110461 ctcagcctca ctgttgccca tttctcctct gggcccagag cccacctcca gtccccagca
110521 ctcacctgtc cttccctctc acacagggcg aagttggagt ccctggttcc aggggagagg
110581 atggtcctga ggggccaaag ggacgcactg gaccgactgg agaccctggg cccccagggc
110641 tcatgggcga gaaggtgatg ggatgagggt tctgtgcctg ggtcctctgg gggtgtgggc
110701 actggggtca ggatgggcat gggtgcccgc cgctgaagtc agccagtcag ttgaagatgg
110761 ccatgcatta gtcatacttc tgtctgtgta aatggcatct ctcagctgct gatgggcaga
110821 ggggatatat aagaagtcag ggaaaagtcc ctgacacgaa gagttggacg gagacgaagc
110881 ttagagagta gctgttgctt caaggcagag tgaacatgcg ttcggactcc aagcagcagg
110941 agttctgaga tgagagagtc taatagcagt gaggtctgtg gtctccagag ctgactctcc
111001 ccagcacttg ccctgtgcca ggtgctgtgc tgatgcgtcc tatctatcct atgaggtagg
111061 catgtgacgt actgttttgc tgaggtttta cagaggcaca gggatgcttg ttgattacat
111121 ggccagtgaa gggcagagcc aggggttatg cccaggcagt ctggcttcag aatctctgct
111181 tctaagccct gctctgcagc tgcctgggca ggatgagccc tggagcctct ggggaagggg
111241 gcatgcacgg gagtcggggt gtggtgggca aagggcctgc agggggattg gtcttggtga
111301 agcaggagtg ggtgctgggg tagatcccac ctccctcatg gggtagatgg agctgaggtt
111361 gacatgtggg aagcagctag atcacagtga ccctgaagga cctgatacct ctgtaggcgc
111421 aggggaggtg ggatcccagc actagaattg ggaaaaatcc actcacggga aggagagaca
111481 acaaaagtca ggcagctgtg gggcaagcaa tgaggggcta gaggctgggg tgtgtggggt
111541 gtgttgctgg gtgggggctg gctgttatat gtgagggttg aggtctccag ggtggagtg
111601 gcatctgggc ctcctctcac tcctaactgc ttcctgtccc tccacagggc aagctgggtg
111661 ttcctggtct gcctggctat cctggacgtc agggacccaa ggtgatgcca tgccccatac
111721 gtgcccctcc tcctcttccc tcctcctctt cgcttccctg atccctgatt ccagaaagca
111781 cagatgcagg gcagtggggg accccagagg gaatttagct gacccccatt ttacagatga
111841 ccgaaagagg cccagagagc agcaggggtt tgtccaaggc cacttagttc ttggcagagc
111901 tcgggtctcc ctggccactg cctttgtggc ctctcctgac cccctttgcc ccttcctgaa
111961 ccctaccttt tataacttct caatttcccc ccttctccgt tctcaccagg ggtccctagg
112021 atttcctggc tttcctggtg ccagtggaga gaagggagcc cgggtaagct gggggtggg
112081 agggcgtgag aagggaaggg ctggagaggt gtagaggggg tctgtgggga gtctcaccct
112141 gggtaatgtt ctctctgctt cattcccacc agggcctgtc ggggaagtca gggcctcggg
112201 gagaacgggg ccccacggtg agtgcagggg agagaaacag gtggctcaag gtccagaggg
112261 gggcatttgg gtttctctga caactcctct tccctctgta gggtccacgg ggtcagcggg
112321 gaccccgagg tgccactggg aagtctggag ctaaggtgaa tggtctctac aaggctccaa
112381 ctgccccctg cccactcagc cccagctctc cctcaacatc ctgacctcgg ggacagctgg
112441 aaagcagctc ttcccactcc ttgactctgt ttccccccac agggaacatc tggtggtgat
112501 ggcccccatg ggccccctgg agagagggtg agtgtggatc gaaagtcccc ttcccctgag
112561 gtccccaggg agcctgggtg agccagctcc ctcctcaccc caggaaggaa gctgagttcc
112621 cccactcagc ccctgccccc attctcctga ctccccccac ctctgtctct agggcctccc
112681 tggacctcag ggtcccaacg ggtttcctgg accgaaagga cccccggtaa gttgaccccg
112741 agctctgacc cacctcgctt ctctgagtcc ttcttcccta cctgctttcc agctgagagt
112801 tcacctgaca actgaacccc ttccagggcc cccctgggaa ggatgggctg ccgggacacc
112861 caggccaaag aggagaagtg gtgagtgatg ctcctggcta cccattctca gggggctcca
112921 tcagaccctt taaccccaga gctgtctgga aagcccacag ggacgttctc ctggcagagt
112981 ggagcctgcc ctggtgctgc tcagacccag ggcccagggc ttggtgtgat cctgcctggg
113041 atccttcctc ctgggaatct gccccacagc gcccacacct gctttcactg cattctcctc
113101 tcttcctcgc ctagggtttc caagggaaga ccggcccccc tggtcctcca ggagtggtgg
113161 gacctcaggt aggtttgtcc ctaggagaga atggacccct gaccctagca ttagtctgtc

113221 tgcccctctt ctctcggtca cacgcccctg ccccggtcca cctgacccag actcctgggg
113281 ctggggtcaa tggtgggggc tgggatttca ggcaggggtc ctgaacttca gtctgttttg
113341 gggcgcattc tcatcgtctt gtcccccagg agaggcagtt ccccaacaga gacacaaggt
113401 ggtgtagtta ctccacatgg ggcaggagaa aacccagcag tggcccagga atgccgggga
113461 atttcccagg agctttgggg cagggataaa ggggttctga aggagtgttt tagggtggcc
113521 agggtggcct cttctggcag acagcgagat gaagggcctg agagcaggaa ctctctctgc
113581 ttttgtctag ggagcagcag gagaaaccgg ccctatgggg gagagaggtc acccaggccc
113641 cccgggggccc cctggagagc agggactacc tgggacagct ggaaaagaag gaacaaaggt
113701 cagtgagggg ccaggcagag gggaagtggg ggagctgggg attggggtgc tgaaaggaac
113761 aggtagttgg agatttgagg gggggctgga gagctctctg tttaatttgg gagcatggct
113821 gtggctcatc acctctcctt tccttttagg gtgaccctgg tccccctggg gccccaggga
113881 aggatggtcc tgctggtctg aggggattcc caggagagag aggcctccca ggcactgctg
113941 tgagtgtgac tcccagaccc ctgcctgtca caagcaccaa gcatcctgag tccccacccc
114001 gaccctgtcc ctgaccccct ccatgtcact ccccacttcc atcttttgga gcctgtcttc
114061 cctctccagc tctcaccctt ctcttttcgt gaagtctcat cttccccaaa tgtctggttc
114121 atagggtgga cctggtttga aggggaatga aggtccgtct ggcccccctg gccctgcagt
114181 gagtctgggg ttccggaagt ggtaggaagg acccgggaaa ggggtgggtg aggaagggtg
114241 ggagtggcat tctgatacca ccaggggctg tggggctggg cagaggctgt ccagggacag
114301 ccagaatgca tctgattttg ggagcttttg ggaattgtgg tctgaggttc tctggtatgg
114361 gcgggctggt gtctccttcc tgggggtctg accatctgct ttctcagggc tcccctgggg
114421 aacgaggtgc agcaggatca gggggaccca ttggtccgcc agggcgccca ggcccgcagg
114481 gtccccctgg agcagcagga gagaaaggtg tcccagtgag tgtgggggta ttggggaggg
114541 gtactgggga ggggtctgtg agcctagggt tgatgggcta tgacaagttt cctgtggggt
114601 gtttgagggt gagggtcacc tccaggccat gactccttcc tcctgtccca cagggtgaga
114661 agggccccat tggcccgact ggccgagatg gagtgcaggg tcctgtgggg cttcctggtc
114721 ctgctgggcc tccaggtgtg gctggagagg atggagacaa ggtgagggga ccccacagac
114781 cccgaccaat cttctttcca gatgagatgg ctgacctggg catgacccct ggcccctgtc
114841 atgtcatcct tcccactcta cccatcacct ttctctaata tcttgtctgc cttctcctcc
114901 cagggtgagg tgggggaccc cggacagaag ggcaccaaag ggaacaaggg tgaacatgta
114961 agtgtccatg accttgactt ctgacctcct ggcctttaac ctcagtccca cctgatctct
115021 ctccctgttt tggtgtctct gactctcttc tcttacccag ggccctcctg gaccccctgg
115081 acccattggt cctgtggggc agcctggagc agcggtgagt gacccctcca cccccaccga
115141 aggcccagca gcctctggtc ctccctcagc tcccctctgc ccttgtggct gggagtggga
115201 gcaggggctc tgggcctggc acccagctgt gtgtggtatt tgtggataga gaacacctga
115261 cccatgaccc atccctgtgt ccttgtctct gctttccctc aaccctgcag ggagcagatg
115321 gggagcccgg agctcgggga ccccagggac actttggagc caaaggtgat gaaggaacaa
115381 gaggattcaa tgggccccca ggacccattg gcctacaggt gtgttggggg ataggacagg
115441 ggctgagagg tggggtcagg atggggtgat gttttgcccc agactctgcc agcagcctgc
115501 cgatgcagac tggagggcct gcggagtctg aggggagggg actgtggggc ctatctgcct
115561 agggctgtgc tttgggtggg gtcgtcacct gggcccatgt tttgtatact tgcacagggt
115621 ttgccaggcc cctctgggga gaagggagaa acaggagatg tgggtcctat ggtgagtgtg
115681 acccctactg accctagcca ccatactagt cacccccctc cctggccagc ccccacccca
115741 caccccactg ccaactccct ggcctggctg ctcctcctcc tctctccacc attcatgctc
115801 agccatctca cttcccttcc tcatctgtgg tgtatctgtt tcccccaggg accacctggc
115861 cccccaggac ctcgaggtcc agctggaccc aatggcgctg atgtgagtca tctcagcccc
115921 tgtcccttga gaccaatgtg tctggtcttc tgcctccctt tcccagacca taaactctag
115981 tatcgtctca ggggccaaga gaagccctta ctcccgggag gcctccactg cctcactgct
116041 gtaaccttgg gctgctcatc tgggactttg cccctgaaat ggcacctcca tcctaagcaa
116101 tgacacctat ttctgttcct cttccagggc ccacaaggtc ccccaggagg tgttgggaac
116161 ctgggtcccc ctggagagaa ggtaactggg aagggggtga gtggaccagt catggaagtg
116221 ggggatggga gttggatttc tgccaatttt gggtggggag acaaaagaga atgagatgtg
116281 gggaatatca gaattttttg gctggggaag gagaggaggt tttgactctt aatctctttg
116341 cagggggaac caggagagtc aggatctcca gggatccagg gcgagccagg tgtcaaggtg
116401 agtgacagct ccaaggcctt actccccagt ccccgtcacc cgcctcaacc ctgcctccac
116461 ttttcctgtg acctccttga gctggaactc ctctgcagca agagtctctg ctccctgcca
116521 tcctctatga ggcctgatcc ctggtctgag tgagcccttc ttcccctttc ttgaagggaa
116581 ggggactagg gaactcccct tgtaggcttg actttctgtg tatgtcccat tgtcactgag
116641 gggagtaggg gatggctgaa ggtgtctcgg aagcagaggc tgcatccctg atcttcaaga
116701 ttccctgggc catctgtgtc ctccgcttgt tctgcagggt ccacgcgggg aacgtggaga
116761 gaaaggagag tcggggcagc caggagagcc agggccacca gggcctaaag gccccacagg
116821 cgatgatggc cccaaaggga accctgtgag tttggggcag tgggggctga gtcctctcag
116881 gccctgtgaa tgacagacct gggaatatgg gtgtgtgtga gggaggatct tggagtgtgg
116941 ggagcccggg agtttggggg atgctctggg aatggggcat ccagagggat gcctggggtc

117001 taggatccgg agagaaagtg agagatgccc tgcagttaat accttgagga attagacaac
117061 acgcagtgca cgtggtaggg aaaggggtgc aggagacctc ttcctggggg gtactggtgg
117121 gcactgcctc tagagaggca gtggcgtgtg cctgtgggcg tctgtgctgg gtgggcttag
117181 ggagctgtga ccctgactct tattctccat ctggatcagg gtcctgttgg ttttcctggt
117241 gaccctggcc cccctggaga aggtggccct cgggtgagtc ttactcagag aagggaaggg
117301 gcaaaaaggg tggggcttct atgggggggt cctctgtgtg gccgctgggc ttgggtattg
117361 ggaagccggg ggtatggcag ggtgggcaag gggatggggt attgacagtt ttggaggtga
117421 tgccagccag gttgggggcc cacctctgac cttgcttcac ttctgcaggg ccaggatggt
117481 gctaagggtg accgaggcga ggatggtgag ccaggacagc ctgtgagtgc ctggtgaccc
117541 caccaccccc ctgagcccaa gcctcatcct ctttacccct cttctgtgcc ccactcctga
117601 ggggtccctt ggctggagga taaacactca gccaccccaa ttcctctctc cctagggatc
117661 ccctggtccc accggggaga atggaccccc agggccactt ggaaagcgag taagtgaggt
117721 ggacccctga gaccttggga ggcagtccct gggctgtgtg ggtggaggct gggcaatggc
117781 aggtgggatg ggtggggagg tgcctggtgt ctgcattgcc ctgggtgtgt gtgtgtgcag
117841 gagctggtgg gtttaagggc gtgtggtatc tcattgcccg gggcagggtg tgtgtgcagg
117901 agctggtggg tttaagggta tgcggtatct cattgcactg ggcgagtgtg tgtgcaggag
117961 ctggtgggtt gatgggtgtg cggtatctcg ggcatgtttg ttcctgggtt ctggtgtgta
118021 tgttttcacc aggggtagtg gtggttactg acaaagcaga atggaaactg gaggaggggc
118081 tggccagctt ttctgtgggc caggggtgaa cctttttagt ttctggggca ggagacgggc
118141 caccaggtag ggtgtgggca agtggccctt caccaaatgt acagactacc cagtattttc
118201 acaactgtca cagctgtatc tgttctgcac atctgtgaat cggccctcgg cgcgtgtccc
118261 tgtgtatgca cgtgtgtgtg tgcatgtgta tgtgtgtgtc taggacagga agggggaaga
118321 gttgagcctg gctgcccacg gcctcatgtg ctcttccttc ccactccacc tgcaggtcc
118381 tgctggctcg cctggttccg aggggcgaca aggagggaag ggagccaagg tgagggacag
118441 gctgccctag tgctggagca tcccctccgc ctcccacccc tcagatgcct ccgtctccag
118501 atgcccaccc catctccacc tctcccatgt tgggccctta tggggacagg cgttccctgt
118561 acttgttcct gcgctagggg ctcctagagt ttggcccttc ctccctgcct cctgcctccc
118621 accttggacc ctctgccccc tgcccacacc ccactcctct gccattcagg acacattcct
118681 tgtgtgtgtt tcagggagat cctggcgcta taggtgcccc ggggaagaca ggcccggtgg
118741 gtcctgcagg cccagcaggg aaacctggcc ctgatggtct gagggggctc ccaggctcag
118801 tggtgagtca ctgcagggaa gggctgggct ggggtgggag tgaggggcca tgggaggggt
118861 gcagtgtggg gatgctcctc ctgacccctg tggccccctc aaatcttcag ggtcagcaag
118921 gccgacctgg agctacaggc caggctgggc ccccaggtcc tgtggtgagt gactgggatt
118981 gggctgagtg aggggtgagg gcagtgccct gggacagagc tgaagccctg gaggaagtgg
119041 aggctgttgg ggagaacttg gtcccacctc tcccttaaga gaatgacttc ccatctctcc
119101 acagggaccc ccagggctgc ctggtctccg gggcgatgct ggagccaagg gagagaaggt
119161 gagtgacaga cagacacgtg gccaggtgtc tctcccatca ccctcgcccc tgaaacctgt
119221 gggacccaag tgcccactgc tctgcttcag gcctccggca gccagtccca gggagtccct
119281 gctcagtgag ggccactcac ggaccctgtg cccggctccc tctccatgtc ctcttggccc
119341 ttctccccca tccacatgac tcctctttgc attctcttga ctgcctgcac ccttctctag
119401 ggccacccag gtctcattgg actgattggg cccccgggtg agcagggaga gaagggagat
119461 cggggacttc ctgggcctca gggctcccct gggcagaagg gtgagatggt gagtagaggg
119521 catggtcccg gaagttgtgg gggttgagag tggggtggag aggggtggag ttggagttgg
119581 gactcagctg tgggtaagcg agcaggtgct caggagggtg tgggaggagg acccagttga
119641 accagccact acccttccta gggtatccca ggagcatccg gccccattgg tcctggaggt
119701 ccccccggcc tccccgtgag tactgcctct gttcctccac aaaatcccct aaacccctct
119761 gctgcccacc cacagcctcc caacgacttc catggaacta ccagcctaac aagcatagtc
119821 ctcagggtcc cccatttgtc ctgtcaccac cagtacctcc tccccatact tccagggctc
119881 tgaatgtcct agtattccca caagactgcc cctcctgtag taacccgagg ctcctgtgga
119941 cttgggacct tgcctccccg ctcctctgag tcctgtcctt cccctgcagt gaccgtctct
120001 ttcttgttcc tcatttcaca gggacctgct ggccccaaag gagccaaagg agccacagtg
120061 agtgaccccc ttcagctctg acctttgctc caccccctct ggacttgatg atgtctctcc
120121 aggacaaata gactcccccc aaggagcccc ttattccagc caggaggctg atttgatctt
120181 tctgtgacct tgatccatgc ttgacctctt gacccctcca tgacctgatt tattccttgt
120241 cagggcccag gcggacccaa gggagagaag ggtgtgcagg gccctccagg acacccggtg
120301 agtgaggagt cagggctgct cccagggccg tacccctctg ctccactgct gccacacttc
120361 accctcacac caaccttgtc tcttgccctc tccatctgtc cctgcaccca gggtccccca
120421 ggcgaggtga tccagccact gcccattcag atgcccaaga agactcggcg ctcggtggat
120481 ggaagccgtc tgatgcagga agatgaggcc ataccgaccg ggggagcccc cggcagtcct
120541 ggggggctgg aggagatctt tggctcactc gactccctgc gggaggagat cgagcagatg
120601 aggcggccaa caggacccca ggacagccct gctcgcacct gccaggacct gaagctgtgc
120661 cacccagagc ttcccgatgg tcagtgctag cctcagggtg cacaaacatc tccccaacag
120721 catgggtact gaacaagcag aatggatgct gggggagtct tggtgaaggt gttgggttaa

120781 gggtgagggc tgaggggctg ggaactgcag ctatttacta accaggtcag aagtatccca
120841 atactttagc aactgatctg ggtcagtgtg taccagttgg ttatcagccc agtgtctgca
120901 ggagcagata aacatacata cgtgcacaca cctgtttgca cctgaacaga tgaatctgtg
120961 tgagcgcctg caaccacatg ttgtcccagt atgaggctgt ccatactcgg acctcctcca
121021 gtcagagggc ctggggttac tgttgggggc agaggtgtca cgtgatgaaa gtgggctgca
121081 gcagtaggat gctgcagtag aatccagcag tagggctaga ttctagggct ctgggaggga
121141 ggaggaccag gagactaaga gcatgatggg gagggctggg agggaagagg gaatgtgggg
121201 gcaaagagca tcttggagcc tggacctagg tggccctgac ctcccctctc cctcacccaa
121261 caggagagta ctgggtcgac cccaaccagg gctgtgctcg ggatgccttc cgagttttct
121321 gcaacttcac agcagggggt gagacctgtg tgacgcctag ggatgacgtc acgcaggtga
121381 gagctggccc ctcgctgccc ctcccctgcc ccatagtgca ccccctcagg gcttagtggt
121441 ttctgggttt tggtgacagt cccctggctg ttgccccccc cggagcctgc caccccttct
121501 ccacttctta gctctttctt cttttccttt tcaccggtcc tctgatgctc actttgcctt
121561 tcgtgtaccc ccagtctcct ggctcccact cagcccctca cttctttccc agcccttgcc
121621 ttccagccat tccctgctca tgactctggc tcagctgtcc tggtgccgta gatgctcctg
121681 aggcccgtct ctgtctccct ctgaggccag ccctctctct ccccctctga ggccagccct
121741 ctctctcctt ccagttctct tacgtggact cagagggctc cccagtgggt gtggtccagc
121801 tcaccttcct gcggctgctc agcgtctcag cccaccagga cgtctcctac ccctgctctg
121861 gagcagcccg tgacggtccc ctgagactcc gtggggccaa tgaggatgag ctgagcccgg
121921 agactagccc ctatgtcaaa gaattcagag atggctgcca ggtgggaaca ggaagagctg
121981 ggttgggggc tgatctcaga ctcaggctgg aggaaggagg tgggaagacc ccttgggcag
122041 ggcacccagg gggagcaggg aggagtccgt cctgctggat tgtcagggat gcctgaggga
122101 gctcaaaagc cggtgaggaa ggtggaaagg atggaagcca tcgggtgagg ttcacttggg
122161 tacaaacacc agcgtcacac aggttaatgc agacgtgtac acagactggc acatggctgc
122221 ccagcagagg cacacggtgg gggagaagca aacacacacg catgggcaca tagacactgc
122281 cagtctgtac atcctgagtc accctgatgg ggccaaggtg ctcagaggag aggtgagcct
122341 gggcctgagg ttagagggtg gggggtgcct ccatcctgct cacactttct tccttgtctc
122401 cccctgaaga cacagcaagg ccggacggtg ctggaggtgc gaacgcctgt gctggagcag
122461 ctgccagtgc tggatgcctc cttctcagac ctgggagccc caccgaggcg gggagggtg
122521 ctgctggggc ctgtctgctt catgggatag gaccgtctct gtctgatcct gtccattcgg
122581 aaccaggccc acctggaatc ccacaacatc agctctgtgc cacctcccaa gagggctcct
122641 cactatctag ggagccctgg gccagggcgt ggagagccct cagtcggggc aggccagggg
122701 aggggtgaag tggttgcctg gacaccccac gggaggagtg gcatctgggg ctcttggccc
122761 tcccacctgg agcctgttac ccgttagaga gctgagaccc ttatttaaaa ctcacctccc
122821 aatcacccca aacaaatgga agagaagaga aaggacatgg cgtattttgt atttaaaagt
122881 aattgtatta attatttaaa gtgtggaaag caaaataaca aaaaagagaa acgccaacaa
122941 aaatcagcag atgttgaaga caggggtctc gggggtgggc tccggcaccc acatcctgga
123001 gtcaggactt tcctcagtga ctgtgtgtag gggggtttca gggctgaacc ccacctccct
123061 cccaccttcc tcccacctca cctgtcgcac ccactgtgaa agttggaata tgtggtctcc
123121 ctggcctcag ggctctgact ctgccagggt ggggctctct aacccacagg tgttggctgc
123181 ctggcccatg tgcccactgt ctcttccact tggtctgggt ttggcaggca ctgcctgcta
123241 cttgagggcc aggatgctcc cccagggaag aaacggaata gtgtggggtg tgtgcagggc
123301 tgcatcccgc agatggctgg aatattaaaa ttcttctata ttggctggta aattgccatg
123361 gccctgagcc actgagtatg ttcattgcca cccctgtccc tcccctgggc acccctcact
123421 ttccctgatc ctgcaattaa agggttaatg tgtggcatat ggaagggact cccaggaccc
123481 tgtgcccagc ttccatgctg actgatggtt aaataatgtg attgtctcct cccaggtgtc
123541 tgtgtcactg cttgtgttgt tatttcagtc tcccccgaca cccatctgat gcttcctctt
123601 cccagctaag tggttaccag aattgtatgg cttaatccag ataccctgca agccctgtcc
123661 agctggggtg tcaggcccca gagtttacaa agcctgggtg actagcataa aattacaaac
123721 aatgccccag ggacaccagg taggggtttg gggtccctat ggctccagtt tctgacacgg
123781 gtgtgcgatt gcttctgggt gtgggtggga cggtgctaca tagcccagtc taatctctgt
123841 aaatggggag gctgaggccc acttccaaga catttactgc atctgtacct ctggtcacct
123901 tatccctccc catcagcctt gtgctgtgct gtcatgaaga cagggctgtg gccaagccct
123961 ccacactcac gtgtagtcac gtgcatactc gttgagtctt tgtgttgggt ggagtcgcac
124021 agtccctgcc tgtgtccccc cactcttgac tgtcccccac ttccctagcc tccctctcct
124081 gggcactcag aagctctgct ccctccgggt taggtgtcca agaagtcctc tcttccaagg
124141 ctggccgcgc agccgggacc ccctagctgg tgcccatggg gcttcacatt cccaagcagt
124201 attgcctggc tcctgcctgt cagggcgctg cattaaaacg tgttccaggg cctcatccca
124261 atttcctctt cctcaaccct cttcctcgcg ggcgccagaa tcaagagtgc gtgcctttgt
124321 gtcccggttt cgcatccgcg gcctcggggc gggcctggca gggctgccct tcaccccgag
124381 cccgagcccg ggtaggctgc gcccccgccc ctcctccccg tacttcttcc tcccagcgtg
124441 tccccgacag catcggcttg gccgtcccgg tcccaaccca ttctcgatcg caggaagccc
124501 cacgtgggcg tctagacggc cactctggcc cgggcgggtg cgcggaaaaa cttctcgagg

124561 gtggaagcct ctcctcggcg ctggcgcctc cgcctctgcc gccccctagt gcccgcgctg
124621 agaacgcggc cccgcaccgg gtcagctgaa taatcccggt ccctcagacg cctctccctt
124681 gtcacccgct gaaccccctg accccgaagg ccgaatggag gaagaagccc ctgatctcat
124741 ctgtcagggg tccggtggtg gataggtttt ctgggatcac ccatgctctc catctcaata
124801 tccaaggccc aggggcttca gacaccccct tccctccctc tcagggtcac attaagagaa
124861 gatcagatct aaataggtgc cccccatctg tttctcccgc acccactccc agcctagccg
124921 ggctggagtt gagatttctt tggcgctctt tctgcagctg gagccaaacc cgcagaggtc
124981 gccccccgaa cagcaagagg actttcccaa aggtcacgtt tggtcagatg ctctgagttc
125041 cgagggcttt tctgcatgga aacaatgctg tacagtcact cgaataacat ttatatacgg
125101 cggctaagtc cccaggccaa tccacaaaag cctatttaat tacacagtgc tgccaaccaa
125161 ccccatctaa cagtttcacg agggagtctg tgccaagttg ctgttgagga ggccaggccc
125221 acagccttct cttggcagct aacttcttcc tcccctccag aaagctagag ccaggctcta
125281 aggaagggtg ggaggggttt aagggcaggg ctgtagaggc ggggttttgg ggtggggctg
125341 ggtgtgaaca gggcaagtgc ccaggcctct gggcctgtgg gtagtggcag tgtctccatt
125401 ggctggtggg caatggaggc ttctgggtgg ggtgggagga gatagggaag agaaaggcag
125461 aggctttagg gaactacttt gggtttgatt ggggcagaac ataaactctt gttggagggg
125521 ccaaattctg aaaagtattg actgcttatg gaaggagttg aacttgaacc caaaaactaa
125581 gagtcataga tggtgtttga aaaggcaagt gatatgatgg aattgtgcgc tggcatggtg
125641 gcgaaggaga ggagggtttg ggatggggcg cacagggaga tcatgttttt ttttgttttt
125701 tgagacagag tctcgctctg tcgcccaggc tggagtacag tggcgccatc tcggctcact
125761 gcagcccctg cctcccaggt tcaagcaatt ctcctgcctc agcctcccgg atagctggga
125821 ctacaggtgt gcactgccac gcctggctaa tttttgtatt tttaatagag acggggtttc
125881 ctcacgttgg ccagtatggt cttgatctct tgacctcatg atcctcccgc ctcggtctcc
125941 caactgctgg ggttacaggc gtgagccacc gcgtgggaga tcatgtatag agggagggct
126001 gtggggtggg tcctctggcc tgtcacaccc ttggagctgc ttttcagctt tggcttata
126061 tttttagcat ctgtaactta tggttaaaaa ataacttgtg aaataaaata aaaataactt
126121 acagaccact tatatcactg tacctgggag caccacatat cctagaaggc catggctac
126181 acagagctgt tcttgccctc ccttggctgt cttgcatgct gaggtttgta gttttgaatg
126241 gaccagggca ggtgggggtg gagatgagac agaggaggga gtgagtcaaa acaaggctct
126301 cagcctgggc aacaaagcaa aactccttct ctctctctct ttctctcttt ctttgagaca
126361 gggtctctgt cactcaggct ggagtggagt ggcaagatca aggttcactg cagcctcgac
126421 ctccgtgggt tccagccatc ctcccacctc agcctcccga gtagctgggt ctccaggcat
126481 gtaccactag tttttgtatt ttttgtagag atgggatttt gccctgtttc ccaggctggt
126541 ctagaactcc tgggctcaag agatctgccc atctcggcct cccaaagtgc tgagattaca
126601 ggtatgagcc atcacgcacg gccctttttc tctacaaaaa aataatttaa aaattagctg
126661 ggcatggtgg catgtgcctg tagttgcagc tactcaggag actcaggcag gaggttcact
126721 tgagcccagg agtttgaggt tgcagtgagc tatgctcttg ccactgcact ccagcctggg
126781 tgacagagag ataccccatc acaaacagac aaacaaacaa acaaagggct ccagctgcct
126841 cagaaataag tttctgtgta gtagctggtg gccaccttgt tttgtggttg tttagcccat
126901 tttcctgcaa gtgtggtgtg gtcagcaggg agcatcaggg ccacactgga gcttctttct
126961 gtcgagctgt gcagttcaca gactgtacat gtgtaggcca tagctatgtt tctcaactca
127021 aacctagagt aagcagaagt caccttaagt gcttataact ggccttctgt tatcttttct
127081 ttcatcttca gagtagtttc atgggaactg gcactgacag cagactgcca tctcagtagg
127141 gaagacctgt cctgctagtc cttgaataaa gaccaaggat atctacatgg gcttctcctt
127201 tagggctctc cccacacggc caggatggta gaatgaacac agctctcagc ttcagcgaga
127261 gccagcatat caccctgtct ggctttccct cagttggcag gagcaggtca atccatttg
127321 tgtacttcgc tctggcctct ctttttttgt ttgttttttg tttttgagat agggtctcac
127381 tccccttgcc taggctagca tgcagtggtg caatcatggc tcactatagg cttgacttct
127441 tatgctcagg tgattcttcc ccctcagcct cctgagtagc tgggactaca ggcgagtgcc
127501 accatgccca gctaattttt tgtattttta gtagagacgt ggtttctcca tgttgccgag
127561 gctggtctca aactcctggg ctcaagagat ccccctgcct cagcctccta aagtgctggg
127621 attatctggc ctctcttttt tttttttgag atggagtctc gctgtgtcgc ccaggctgga
127681 gtgcagtggc acaatctcgg ctcactgcaa cctccgcctc ccgggttcaa gcgattctcc
127741 tgcctcagat tcctgagaag ctgggactac aggtgcccgc caccacgccc ggctattttt
127801 ttttattttt agtagagacg gggtttcacc gtgttagcca ggatggtttc gatctcctga
127861 cctcgtgatc cacccgcctc ggcctcccaa agtgctggga ttacaggcgt aagccaccgc
127921 gcctggccta tctggcctct ctttctatta aaaacgcctt actctaggtt gaaggacttt
127981 tggttcctga ggcatggatg atttcccaaa gtacaacaga taaaagccac actggctgta
128041 cagcagctca tgtaaaggtc atgttccagg tcttggccac ttcacctata acaggcttgg
128101 actccggcag agcctcaggc caaagaatgt gggtcataga gatatcttcc tcattgtgtt
128161 cacatctccc ccgggcctgg gctatattgt tgggtcaggt aatcggcctg ctctcctcat
128221 gaaataaggc atactggtat tactaatgct gggagagatt tcttataatt ccatcccttc
128281 ccagttgtgg ttatttgtat taatacctgc aggttttcct tacttttaga actgcaaatc

128341 tatagaaaca aacaaacccc aaacccaaca aacatgtttc agagacatta gggttaggga
128401 gaagatgaga aatagcaccc tgtttgattg gggccctggg gagagaggga ctcagacaaa
128461 agagtttgag tggcaggtgg tctagttttg ggtgggttga gcagacttag ggccaaaggt
128521 ggcactgatt ctagctggca gcgggcctct ctgtatttcc aactgagagt tctccttccc
128581 ttagcttgtc agggatgctt tgcttctgag ttgcacactt aacctcagca tcagtttctt
128641 cacctgcaaa gtgggataat acttgctctg taaaggaata attccccctt tactgacagc
128701 ctttgcaatg gaaagtgagg ggagggacct gacaagctgt cttggttcca tctccatgtc
128761 ttggttattg ctgacacacg ggtgtctggg gcatgctgct gtcccttttc cacttttgat
128821 tttagtgctg ttaaaactct atacttacta ttattatttt cttgttacaa atcgcaaaat
128881 ctgattcaga atagcttaag ctccaaaagg aattgattgt aaaaatattg ggtttctcag
128941 gatttgaaga cagagttgac tcaataggga agactgagag cagttctggg aacctcaggg
129001 gctcatggac tttgtccctg gagtgtggat actcaccgct cagcagagct gctcttgacc
129061 tctgtgtctc tgggccccat ggttcaaagt catggagaga caatctgatt ggcttagctt
129121 gggttatctc tgtccctgag aaccaatttt ctgtggctga gggcccagtg taccaaccaa
129181 gaggtatgta ggatgccttt tggtggaggt ggaggggtgt tgggggcact gtttccagat
129241 aatgtgggat cctgaggccc ccaggaggtg tccgcttgct ttcccttctt cgcttctgtc
129301 taaacagact cctttgtatg catttgacca gcgtgataca accagcccct tcatgatcac
129361 atttgctctc cctgccaccc aatctcctgg atgggcaacc caaagtcaca ttcagcagct
129421 gtggccatga cctctctaac ctcagaggcc gacatctctg ccagttcata ttttgccact
129481 ttgtgattca gcacataaac tttcatggcc gttatatcca gggctgcatt gtgacttctg
129541 cgggccctat gcacttttct aaacaataaa tttgaaatca tacttatgat tgtgttgata
129601 cacaggcaac tatattaata ttatatatta aaatattttc ttcaacctta atttttttc
129661 tattttaaaa gaaatgtaaa cattgccatg ggagtattgt gggccctagg cactgtggct
129721 actgtacata atggatccat gggccctcct tgtgtctttg ttaaagattt tactggttta
129781 ctcttaaaat gtaatcacaa ttctgttacc acaattttaa taatcattcc tgttgtcatc
129841 aaatatccag tcagtgttca gatttccaag attgttttgt aggttttgtg tgtgtgtgtg
129901 tgtgtgtgtg tgtttgactc atgatccaaa taaagctgat ggtcttctaa ttctttaaag
129961 atctacattt tccgtaattt atttaaagtc ctttttgttt acttttatta tggacaaatt
130021 taagcatata caaaagtggt ttattattgc tttttttttt tttgagatgg tcttgctctg
130081 tcgcccaggc tggagcacag tggcacgatc tcaactcact gcagcaacat ccgcttccca
130141 ggctcaagcg atgctccagc ctcagcctcc caagtagctg ggactacagg cgccagccac
130201 catgcccggc taatttttgt attttttgta gagacagggt tttgccatct tgtccaggct
130261 ggtctccaac tcctgagctc aagcaatctg ctcatcttgg cctcccaaag tgttggaatt
130321 acaggtgtga cccactgagc cttgcctgtt attgctatct taaaaagaat taatacatgt
130381 taaaaatctt tttcagttct aattttgaat acagtaaata tcaataacaa tattgtaagt
130441 aaagcttttc agtgttttca gtaatttaag gggtttatac tcttaaaaca ttgagagatt
130501 tttttcaaat tgagatttaa aaaaattggg aatcactaat ctagaagaac tgttaaaaat
130561 agtcatgccc acctgtgctc acccttagag atctggattc aacccatttg gggcgtgagc
130621 aaagggactt gtgtaaaatt tccccaggca attccaaagt gcaggctgtg ttgagaacct
130681 ctgctctaaa ctggagagat cccgtaagaa ataagcagcc aggtgcagtg gctcatgcct
130741 gtaatcccag cactttgggg gaccaaggca ggtggatcac ttgaggtcag gagttcgaga
130801 tcagcctgtt caacatggtg taaccttgtc tctactaaaa atacaaaaaa ttcgccaggt
130861 gtggtggcac gtgtctgtaa tcccagctac tggggaggct gaggcaggag aatcacttga
130921 accagggaag cagaggttgc agtgaaccga gatcatgcca ctgcactcca gtctggtaga
130981 cagaatgaga ccctgtctca aaaaaaaaag aaaagaaaag aaaagaaaaa aaaggaaata
131041 agcaaagatg gccgggtgtg gtgcctcaca cctgtaatac cagcactttg agaggctgag
131101 gcgggcggat cacctgaggt taggagttcg agaccagcct gaccaacatg gagaagcccc
131161 atctctacta aaaatacaaa attagtcggg catggtggca catgcctgta atcccagcta
131221 cttaggaggc tgaggcagga gaatcgcttg aacttgggag gcagaggttg cggtgagcca
131281 agatcacggc attgcactcc agcctgggca acaagagtga aactctgtca aaaaaaaaaa
131341 aaaaaaaaaa aaaaaaaaag aaataagcaa aggcacagag tgggaaaggc aaccaaatgc
131401 cctaggtctg aaagggcctc ctgggacctg ctgcatccag cagaaatgct tgcaagtgca
131461 ggcccagaca acccaaaaaa caggggctaa aataaacagg aattcatttt tctcataaga
131521 aatctgagga tgacagcttc cagcattggt ttgagcattt aatgatgcca tcagggttcc
131581 agactcttca tatgtttaca ctccaccctc ctacaatgtt acccttacat attttctttg
131641 gctaaactat aagcacgttt ttcataatag ctgttccatt tacatcactg tcaaacatga
131701 cattcaccaa taaagaaaag acgcatgaga cgtgttcggc accccttagc tgtagaaatg
131761 cctgccacca ctttgctgtc atgaaagagc cattttatgg ctattgcaaa tgctttgaag
131821 atactaagac ttgtacctgg aagctataga tgcagtgtgg gaaactggga agtataattg
131881 ccactgaatt gttattgatt tattactgca aataaatcta tgcaggtccc ttctgaaatt
131941 gagatggtcc tgaactgaac aaatgtctta ttgacattca taacagtggc tggtattcct
132001 tctttatcct gtttgtcagt tttggaattt cactgattag ataaataaaa agaatctcac
132061 ttgctcctta ctaatgagct ccacggaggg gcatgatgtt ggaaaaggca ccatgcaagg

132121 catgattact aagaagacaa agggcttgtc ggggagctgt tagggagggt cctgagccag
132181 ggcttgggaa aaacatctca tcattttact gtatctgatg cttggaagtg acatgtgctt
132241 tgcaaactcc agtttatgtt ttgatgactt tgtgcttgtt ctgcttggtt aggattagct
132301 tcagctgtga gaaacagaaa accctggaaa caacagtgtc tccaccaaga tagaagttca
132361 tttctctctc acatgcaacc ctagggctgg tgtagtgttt aagtgtcaga gactcaggtt
132421 ccttctatct atcttttctt tccaccagcc tcaacatagc tcttctctct caggtccaat
132481 atgactgctt gaattccagc tttcatgttt gtgttccagt cagctgaagg agaaagggga
132541 aataaggaca acccctcttt tttttcgttt tttctttttt tttttttttt gagagggagt
132601 cttgctctgt cacccaggct ggagtgcagt agtggatctc agctcactgc aacctccgct
132661 ccccaggttc tagcgattct cctgcctcag cctcctgagt agctgggatt acaggcatgt
132721 gccaccatgc ctggctaatt tttgtatttt tagtagagat ggagtttcac cacgttggtc
132781 aggctggtct caaactcctg acctcaagtg atccacccgc cttggccaat acctttaggc
132841 aaagcattat agcaggagtc taaaaacaaa tgggctgttt tgttttgctc ggaaatctct
132901 ccccgaggga ttgttttgtt ttttgaagta tttgaaggct aagtatctag agcttgtgct
132961 catggagtag ctgccccctt tcctcgtgag gccaccatta gttacctctg catatatgtc
133021 tgcctctgag gtgtctggct gtctgaggag gcctctggct ttgttagtac accactagga
133081 tatttgcaac gtggatcaag actttttaat ttaatttaat tttattttat tttttgaga
133141 tggagtcttt ctctgtcgcc caggttggag tgcagtggca caatctcagc tcactgcaac
133201 ctctgcctcc caggttcaag ccattcttct gcctcagcct cccaagtagc taggattaca
133261 ggtgcctgcc accatgcctg ttgcgggaag tcagggaccc caaatggagg gaccagctgg
133321 agccgaggca gaggaacata aattatgaag atttcatttt aatatggaca tttatcactt
133381 ccctaataat gctcttataa tttcttacgc ctgtctttac ttcaatctct gaacataaat
133441 tgtgaagatt tcatggacat ttatcagttc ccaagtaata ctcttataat ttcttatgcc
133501 tgtcttactt taatctctta atcctgttat cttcacaagc tgaggatgta cgtcacctca
133561 gggccactat tgtacaaatt gactgtaaaa catgtgtgtt tgaacaatat gaaatcagtg
133621 caccttgaaa atgaacagaa taacagcgat tttagggaac aaggaaagac aaccataagg
133681 tctgactgcc tgtggggttg ggctaaaaga gccatatttt tcttcttgca gagagcctat
133741 aaacagacgt gcaagtagga gagatatcgc taaattcttt tcctagcaag gaatattgat
133801 aattaatact ctgggagaat aattgcattc ctggggggga ggtctataaa cagccgctct
133861 gggagtgtct gtcttatgcg gttgagataa ggactgaaat acgccctggt ctcctgcagt
133921 accctcaggc tcactagggt ggggaaaaac cccaccctgg tgaatttgag gtcagaccgg
133981 ttctctgctc tcgaaccctg ttttctattg tttaagatgt ttatcaagac aatatgtgca
134041 cagctgaaca tagaccctca tcagtaattc taattttgcc ctttgccttg tgatctttgc
134101 tttgcctttt gccttgtgat ctttattgcc ctttgaagca tgtgatcttt gtgacctact
134161 ccctgtttgt acaccccctc cccttttgaa gtccttaata aaaagctgct ggttttgtgg
134221 ctcaggtgga catcatggac ctactgatat gtgatgtcac tcctggcggc ccagctgtaa
134281 aattcctctc tttgtactct ttctctttat ttctcagact ggccgacact tagggaaaat
134341 agaaagaacc tgcattgaaa tattaggggt gggttcccca gatacatgcc cagctaattt
134401 ttttgtgttt ttagtagaga cagggttttg ccacgttggc caggctggtc tcgaattcct
134461 gacctcaggt gatccacccg cctcagcatc ccaaaatgct gggattacag gcattagcca
134521 ctgcacctgg ccatctcaag atggttttaa tggttagaaa tatcagccaa ttgaattttc
134581 agagacatat gccacctaat tttgtattca atacttaagt cattaaactc cagtggtcta
134641 tatacctgtt tggcctctaa gacagtttgg cctctaagtc tgtgagcact ttctgtgatg
134701 atggaaatgt tctacattta tgcaatttaa tgtggtggcc actattcaca tgtggctatt
134761 gagcacttga aacatggcta gtgcactaag aaactcaatt tgaaattcaa ttttaatttt
134821 atttaattta aatggccaca ggtagctagc tagtgctacc atattagaca gcacagctct
134881 gaggctcttt taaggaccca cataattcaa tgaagagacc caagtttaat acagatgtgg
134941 gatatgcagt cttcataatt caaacacgcc aattaatttc tgttcctctt tttcaagttg
135001 ggggctttca agcacaaagg cttttacctg gtcttgggca gaatttttct ccccaaaatt
135061 ttaccaaaat ttgtgctctt ttatagtctg tttcttttaa ctcataaaat taataatgga
135121 agacagaggt ttttgatgca gttgctttaa aaaaaaccag gttcaggcca ggcacagtgg
135181 ctcacacttg taatcccagc actttgggag gctgaggtgg gcggatcttc tgaggtcagg
135241 agtttgagac cagcctggcc agcatggtga aaccccgtct ctactaaaaa tacaaaaatt
135301 agctggatat ggtggtgcgc atctataatc ccagctactt ggggctaagg caggagaatt
135361 gcttgaacct gggaggggga ggttgcagtg agctaagatt gcgccactgc actccagcct
135421 gggtaacaga gtgagactcc atctcaaaaa taaataaata aataaataaa taaacaaata
135481 aataaataaa taaaatcagg tttactgaga tataatttaa atacagtgaa accatagctg
135541 tacagttgtt tatgagtttg acaaacatat tcattgtgta actactacta caatgaagat
135601 aatatttcta ttaccccaaa aagtttcctc atggacacag cttttttcaa gtcactcctg
135661 gttaatgtgt cattaggata ataaaacaat gttaattctt gaattggatt taggctgttt
135721 aaattttgct caatggattg tacctcggag gtgggagaat ttactctggc aacttagtaa
135781 aggttggggc tgccaagtga aggcagactg ttggtgaata taatttattg ggattgaaaa
135841 ggatacatca gccagattga tgcatgaatg ctaatcaagg gttgatttag caagatcttc

135901 tactaaggaa ataatatcat gtacagtgct aatcacattt ttttcataat tgagttaaat
135961 atttaaagtg attagaatag tcactgccac ttaatactac ttcagaatta gctgcttttc
136021 ttaatatcgg tgtaattcac aattcacttc caagctccac ctcccttttg actggccaaa
136081 atgagttgtt aaagagtcct actctttatc tcagatttct ccaacaatgt tatcataatt
136141 gttattttac atattctcct agggatttgt cactatttca tattacgttg ttttgtgggg
136201 aagtggaatt ctagaggttc ttaagagctt ttccagctcc cacagttgca atcgtaagtt
136261 cagataacat gcccaggggc aggagaaatc accatagaag ctttcttggt tctctgtatc
136321 tcccattaat gtttcataac cttccacctt catactttcc cctccacata ccctggtatt
136381 caaaatagcc cttcttatta gattgggttt tcctaattaa aagttgccat tgatgttata
136441 tcccaaagtt tctggaattc ctaaacattg tcctttgtct aaactgtttt aacgtgagtg
136501 cagagtgtct ccagacacag gttggaacag atctggaggg aagaacattg gggctgtttg
136561 tcccatcgta tgagaggatc tggttttagg gcaaagctct ttaagcttgg agaatgaatc
136621 gaaggtgtgg aagtgccctg tttctgtatg ttctaagaga aatgagctct taacgtttat
136681 aagctttttg tcttggcatt ccccttcggt ttggtcccta gcactgttaa attacatgaa
136741 ttgagggttt gccaaaacta ctgctaaatt tgcagcttct gtatctgcta aaattggact
136801 atttttgata cttcctttaa cctgttttgt aaccttgtta attttctaat gagcccattg
136861 tgctgttggg attccgatat ccccaaatct aatgagccat gaccatacac gttagctcca
136921 ttcccactgg gttctcaaat tctttttcat ttttccaatt ctttagcact ttctcttata
136981 ggcggttctg ccttaatttg aaattaaatg agaacttcag ccccttcgtc tcaccagctg
137041 ctagcatttt aagtgctcaa gagccacctg tggctagtgg cggctcggtt ggatagatac
137101 agaacatttc cctaacagca gaacattgta taggacagca ctgctcaagg gattgctctc
137161 cgatgatcac acccacttta cagatactag gactgtctaa ctatgtacac tttctttctt
137221 gcaaatacga tgtcgaaggc tttccctcag tttgtaactt ttaacaaata ttttagaatc
137281 tttattgatc aatctctcat acatctgtgc aacaaaaata tatgtattca tttaattctt
137341 taattgacaa aaattgaatc tgtttatggt atacaatatg atgtttatat atatatatat
137401 atatatatat atacacacac acacacattg tttaatggct aaatcaagct aattaatatg
137461 tgcattacct tacatatttt ttgtgttggg tgagaacact taaaattatt cacttagcaa
137521 tattcaagta cacagtacat tgttattaac tagagtcacc atgttgtaca gtacatatct
137581 tgaatttatt cctcctgtct aactgaaatt ttgtattcgc tgatgttagt ttttacaatc
137641 aactcattaa aaacataatt aggtcacaga ggttaggagg ttttgaaaaa aacaaaaaac
137701 caaacaaaaa atgtaattag gtttgtttgg caatctagca atgcttagtg tgcaatgctt
137761 tttacagccg ctgctaaaag catagcaaaa ccacagaaat ctatgtgctg ccaaaaccta
137821 tgtatttggt cgtatttatt ttgacttgtg ttttttgtgt gcttaacttt ttctctaact
137881 tttttttgta aagaatgctg gagtattccc aaattgttga caaattccct tgtttcctgt
137941 tctaagccca gatttcagtg ttggccacta ggtggggata ggatcctatt tctctatagc
138001 cttaacatga ttttgaattt cgatgctttg caatggataa tctggctttt tcctcgaaag
138061 ctttaaaaca tttcttttgt gaattcaaaa caacccacag ttttaattct ctgtagttat
138121 ccaataaaaa ggaataggaa acaagtgcta aattatttac ttacagtgtt tctgtatgga
138181 aatccttacc cccaagacag ctggcttcac ggcttgactc aatggtgaga aatctgagtt
138241 gactcacagc tttctgacag tattctgatg gtaccatgtt aggataaatt cttggccaaa
138301 cagaaaatca agggagacct gcttctcctt gtcaaactct aaattaatct actagttctt
138361 ttcataaagc aaaaggtgaa atgttgagga ctctgagttg gcgatcaatc gaaatgactt
138421 ttaaactaag tcccttttaa tgatgactga agacaatatt acattaatca ctgggtatta
138481 taaacactgg tttctcagtc ctctgctgat gagatttgct tgtttgtgta tttctttctt
138541 aaaatcccaa atagtgaagt ctctcttaag caatggaggc ttattcatag tcaaattcct
138601 tgcatgtaaa attctctggg tataaagtag attgcctccc tcttgttttt ctttttcttg
138661 agacagggtc tcactctgtt gtctaggctg gagtgcagtg gcattatcac ggctcactga
138721 agcctttacc tcccaggctc aagtgttcct cctgcctcag cttcctcagt acctgggact
138781 acatttattt tttgtttttt tttatttttt tgtagagaca ggattttgcc atgttgccca
138841 tgctggtcaa gctatctgcc ctccttggcc tcccaaagtg ctgggattat aggcatgagc
138901 caccaggccc agcctgcttt cttctgtttt accaagatgc ttgacagtgg atatataaaa
138961 tgacaacttc cctttctcat atgtggcact gagagatggg cagcctctat ttcctacaag
139021 aaatatagaa tagaaaatgt gtaatttctc atggtgttct gcaaaaatta aaaatttcac
139081 agaacactca tctgaatccc tggtttgtaa ctttggcttt ccaaaacttg cctatatgct
139141 aatatgattc ataaaacatt tgtaataata atttcaaaat aaaaatttta aaatgaattt
139201 agtcctatat cttctataat aaccaatatt tccagaaaag taggatttaa gagtatcttc
139261 tgtaccaata tatgactttc tttttctttt ttttggagac agagtctcac tctgttgccc
139321 aggctgaagt gcagtggtgt gatttcagct cactgcatca ttcagtcggc cgaaggcctg
139381 gctagaacaa aaaggcagaa aaaaggtgaa ttcactctct ctctgttttg gagtagggag
139441 tcctatattc tcctcccctt ggacatcaga tctccaggtt ctctggcctt cacactcagg
139501 gacttgcacc agcagccccc caggtttttg gtcttggact cagagttact ccaccagctc
139561 ccccggttct cagcccttca gacttggact gagccatgct acaggctccc ctgtctcagc
139621 agcttgcaga aggcctaacg tggaactcct cagcctccat agttgaggga gtgaatcccc

139681 agcataaatc cccactcatc tatctaccta tatatatcca ataaattctg tttctctgaa
139741 gaagtctgac ttcttcagcc cccaaaggcc cccaaaggcc cccaaagccc ccaaaggccc
139801 tagtgctggt ctgttacgaa cttcctggta gacaatattt tacacatggc atcccagctc
139861 cttgtgggag gaattaagtg tgtcctgtgt ggctccaggg ggacaggatc cttggaagcc
139921 tgagcctggt ttctcttagg ctttgcccag tgtgcctttc cctctgctga gtttgctttg
139981 catcctttta ctgtaatgaa tcagagccct gagtaggacc atatgatgag ccctgtgagt
140041 cctcctaggg catcatctca cctggggtgg ctctgaggac ccctaacata gctcttcctc
140101 tggcattggt atctggtaat atataaaatc ccccaaaact aacttgtcat cataatttat
140161 gaccatattt catcaaaatt tcatgtagtc acatccaaga agttcagtta agaaagaaaa
140221 actctttaag cttggttaag gctctgccat ttttgaaaat tcgtattaat ctaataatat
140281 tgtagcaact ataagttttc ctgataactt tttttttttt tttgggagtg ggggatggag
140341 ttttgctctt gttgcccagg ctggagtgca atggtgtggt ctcagctcac tgcaacctcc
140401 gcctcctggg ttcaagcgat tctcctgcct cagcctccca agtagttggg attacaggcg
140461 cccaccacca cacccagcta atttttgtat ttttagtaga gacaggattt caccatattg
140521 gtcaggctgg tcttgaactc ccgacctcag gtgatctgcc tgccttggcc tcccaaagtg
140581 ctgggattac aggcgtgagc cactgtgccc agcctcctga ttactattat ataaaatttg
140641 gacttagtgg cacatcataa catgaaatga ctggagacaa tatgacttct ctctcggtat
140701 taaacatcaa ttaagatgaa ttacaaaaat catagtaaac tgtattctaa aaccaagcct
140761 atgtctgaaa aatctggtat acacacatct tttgaaacta aggatgtgtt ttcactgtac
140821 aacctgcaag aggtgatctg gtgaacatag agttatggaa taagtgtgta tctataactt
140881 agtacaaatt atatataaag agaacgataa tgtattatgc ggctgggcat ggtggctcac
140941 ttctgcaatc ccagcatttt gggaggctga ggtgggtgga ttacttgagc tcaggagtcc
141001 aagaccagcc tggccaacat ggtaaaacct catctctacc aaaaacaaaa aattagctct
141061 caggtactct agagactgag atgggaggat cgcctgaacc ctggaggcag aggttgtagt
141121 aagctgagat catgccattg catttcagcc tgggtgatag agtgagactc catctctaaa
141181 taaataaaat aaaataatat attatgctat tatacctatt atttaaaatt gggatcttat
141241 gttgatttct acatttggga agcatattcc taagaactga cctttatcag tcctgtattg
141301 tgttcagtgg caaagccagg cttggtgtct gttccatctg ggaacgccag gagtaatggt
141361 cccactcctt gctgctgagg gtacagcatg aatttgggca gcaccatgaa ataaagcaaa
141421 tgtaagaaga tcctctatca gttgcactcc tatttaataa gaagacttga tcatcatcaa
141481 gaagttaatt tctccatgcc tgggtaaaac cacactgact catcctgagg ctcttcattg
141541 gacccagtga tcaccaaatc cttcaagcat aaaatggcca tgtgattatc tccttcctct
141601 caggatgaaa ggcagacaat aacatgtgat ttatgttcct caggaaagga catgctataa
141661 actagagaac ttgggaaaga ggaagtacat gaattaacat gcaggcacat ggtggcatgt
141721 gcctgtggtg ccagctactg ggcaggctga gtggcagcat tgtttgagcc caggaggtgc
141781 aggctatggt gaactgagat cgtactactg cactccagcc tggttaacag aatgagaccc
141841 tgtctgtctt tctctctctc tctctctctc tcacacacac acacacacac acacacacac
141901 acacacacag aaaataaagg agaaaaagaa attgttctta aacataaaat ttaaagtggg
141961 gttctttgta tcaccacatt cttttttttt tttttttttt gagacggagt tttgctcttg
142021 ttgcccaggc tggactgcaa tggtgtgatc tcggctcact gcaacctcca cctcctgggt
142081 tcaagtaatt ctcctgcctc agcccctcga gtatctggga ttacaggcac gtgccaccat
142141 gcccagctaa tttttgtatt tttagtagag atggggtttc accatgttgg ccaggctggt
142201 ctctaactac tgacctcaga tgatccaccc gcctcagcct cccaaagtgc tgggattaca
142261 ggcatgagcc accatgccca gctgactctt ttaaatttca ttcagctgtg cgcccagcct
142321 caccacattt ttatatgaag aagaaataaa ataataatta ctaattaaat aattaaatat
142381 tatttccttc tccttatatg tttgaaaaca tcaaagattt tctccaaaat tatcatagct
142441 ataaaagagt caatataata tcaaatggta acaaattaat tcctttatga tgcaaagaat
142501 caatgtaaaa ttgtgaagaa atatcatcca ttcacagaag taactacaaa taaaaaatcc
142561 caacttgcgt aatgtatttc ctcattaaga agaaggcgat tatgatgtcc taattttcgt
142621 tttcttcact tacccctaca ctctgcagta gtcaggggct tcctgggcca tgctggtgcc
142681 tgacaattct cccacttcct cccagcagct ctagcatctt ccttccccag ccacagaggc
142741 gtcccttctg atgtattctg gttggttgag ccccatcagg ctgtttcatc tccagccttt
142801 ctccctggac aggggtaggg aaaggagtgc tggattcgga gctcctccgt ccctgcgccg
142861 cctcattttc tccttggggt ctggcgcaca gtggccattg caatacaccc taaagactga
142921 aggagccagc gcagagccag gatcctcctg tgagccatgg ccctggctgc cctgcttagc
142981 ctctcggaac ctgggccatc aggggtgagt gcgcaggtgg gcgagggagg gtgggggctc
143041 tgggcgcttc tggagcccac agatgctgtt ttctttcctg ttctgttttc tctgttcttt
143101 actgttttgc ctgaggctct cttttgcttt ctctcattct aacaataata accaccattt
143161 actgaacacc ccctaagtgc caaacctcat gtccttcctc acaacaccat tttcagaaag
143221 gcttttatta acacaatttt agaaatgaaa agactgagac tcaaagagat tcataaatgt
143281 ctcctttccc ccacgtcctc tcgctgaaga gagaagagcc agggcttggc aacctcctct
143341 ggagattctc tcgtagattg tagaggtcag cttcctcaaa taggagtctg caatgtgggt
143401 tttttccttt ccttccttcc ttcctttct ccctccccct cctcctcttc ttcttctttt

143461 tcttcttctt cttttctctc tctctctcct ctctctctct cgacagggtc ctgctctgtt
143521 gcccaggctg gagtgcaatc acgtgatcac agttcactgc aaccttgaac tcccaggctc
143581 aagggatcct cacacctcag cctcctgggt aactgggact acaggtgtct gtcactacac
143641 ctggataatt atatatatat atatatatat atatatatat ttttcccctt agagatggag
143701 tctcactgta tttcctaggc tggtctcaaa ctcctggcct caagtgatcc tcctgttcag
143761 cctctcacag cactaagatt acaggtgtgg ggcaccacac ccagcccgtt agtgactctc
143821 tctcatttca ttcagtcttc aacccactgc attctccctt tttcttcccc atgtctctga
143881 tgctgctctc agggtgacct ttatacaacc caaaccagtg atcacttctc tgtctctatc
143941 tcttgtgtgt catgagtgtg tttgtgatgc gtccgctcct ctttcctgga tgttttctcc
144001 tgtctgttgt cttttatgaa cccacttcct ctttgttttc ctcctacttt ctagtcttct
144061 ccttttcagc ctcttttttc atgtctgtgt ttgtctggat gcaggagcac acactcagac
144121 ccttgctatt ctgactttat gtacttttct tgagtgacct gatccaaatt tagaacttca
144181 cttataatga gcttccttcc agtctgtgtc actaacctag tgtaccctgg gacccaacca
144241 tttagctcac tatcctcaga ttaactccat ttgttcccac agaaattcaa actaagcatg
144301 ttccaaacag aacatcttac cttctctcga ttatttcatt tttcattacc tttgcagtca
144361 ctgatgcccc agttctccac gttgggtatt ctggactcct tcatatccct acttctggca
144421 catgatttag tgattcctgt ggcttttcgt ttcattattg ccattcaaac ccatccgctg
144481 tccgcctcac cccatgttcc tgcccctgcc tacaccctca tgacttccca tctgatggct
144541 gaagagtcct attggtcttg ctttcaaacc agcctcctcc aacccatcct ccctactgct
144601 gccccctcca aacacaaatc tcatcatgtt actgtcctgc ttaagatgct tcacaacttc
144661 ctatgtcctg tgggataaaa tgcaaacacc tgccatcagc acacaaggca ggttacagtg
144721 ccgcccggcg cacatctccc ctggcttcag tcacactgaa gaactgcagt tctccttgca
144781 acttcatgcc tctctgctcc tgagcctgct tctcctgctg gacatgtcat ttcccctttc
144841 tctgttttgc taacctcagg taaacactat ccctaggaac ctttcctaaa tctcattttt
144901 caaaaatgcc ctctttatgc tcctgagtgg ctccagtgta tatcttatgg tactcagtgg
144961 tgatcaaaat aacctatgga aataaaagat taaaaaacac aaaataaaat acacatgtag
145021 caatcttctt ttctttgtct ctttggattt tggatttcaa gcagcttgag gacaaggccc
145081 aggtcttctg agttacatcc ttagtgccta aagagtgaaa attcacaatg agtgtttatt
145141 taacagaatg aaaattaaca tttgaaggtg gaatttgctt taaaatgcac ggacctttag
145201 ttttaggctt tgtgaatata ataaaaacaa agtaacctaa ggtgaacaaa cgacatctct
145261 gtagcacagc ttgtatatca tgtttttcat ctgttttttc tttgggcttc gtttctgtag
145321 gtgagtaatt ctctagccca taaactcagt ggaggaccct caacatccgt gtgtgacact
145381 gaacttgccg tcacttgacg catccaagga gaggactggt tttccccgtg ttgggcttgg
145441 aggggaaatc cttgcttagg accaacaacc atgcagtgtc atttcgcctc acttcctctt
145501 ccaggctgga ctccatgctc tctcatcttt attcttttac tgtaatagta atagactttt
145561 ttgttgtcaa ccttttgctg cgtcttatac atcaggtaaa gctaagatac tatcccatag
145621 gtgtttcaaa tgcaacaact tttatttcca ggtagaacta cacttgaaaa tatacattca
145681 acaagaacat tttgtgaatg ttatgtgcat gacaacattt ttaaatttac tatgacttaa
145741 aatgaaagtg ttttgataaa attatataag gtaccatttt tattctaaaa gtaagaacat
145801 aacttgaagt ctataaatag cactaagact tactctggag gtcttttcat gtccttcatt
145861 atttttttcc ctgaaagatt tttatttcat ctccctactt ccctgccttt ctctgtggct
145921 atatcagtat tttttatgtc tggtcctgta ggggttctga gatcctatgg gaatggggta
145981 gggggactac gcaattttgt ttctaccagg agttgtgtga cttgggtgag ttactttact
146041 tctgtggtca gttccccaat atgtaaaata atttcctctg ttaaatcaag tttagcctaa
146101 agttgcctcc ttacatattt taagtttggc ctaaaggttt ctctgtacat catgaactat
146161 aacaagtgga ggtgtaaaca gaccatagcc tacacttgtg ccaatcactg agttttggcc
146221 aatcatatgt agctaactgt ttgaactgtg ttcaagttag gcaaacattg agctgtaacc
146281 aatctggctg tttctgtacc tcacttctgt tttctgtatg ttacttttct ttttctgtcc
146341 ataaactttc ttccaccacg cagctgtgct ggagactttg agcatactct ggcttgggag
146401 gctgcccgat ttgtgaattg tttattgcgc aattaacctc atttaaaatt tattcggctg
146461 aagtttttct tttatcaggt ggtgtcagaa gtggggttgg aagtagagct tctaatgacc
146521 cccagaagtg ctgagtgacc tagcgaggta ccctccaggc ccattgtgtc tctttctctc
146581 tcggagcagc tggggatcgt ggtaagttct cttgcagatt ccgaagctcc acagatttgt
146641 gtgttgagct ctctgagttt ctttgagtaa atttctgatc ctgattgggt ttgaaagtcg
146701 cgaaagaaat tgaactgagt ccaggatcgg attggctcca gtaattaact ggcttggatc
146761 cggttagaga cctcttacat ctaactggat cagaaagaaa ccagtattaa atggcaatat
146821 tgcaaaggtg taaaatttgg cttttggaaa ttcacagaga tttttatgtt ctactccttt
146881 atttttcttg tgcacttagg taggaaaaaa aatcattggc taagttgaac aagggacctg
146941 agagcaaagc cagtatttga ggtaaaaaat gtgatactga atttctgaag aactgagttc
147001 cttctggatt atacatgcat aagtattagg ccctggaagg agcaacgcct tacagaaatt
147061 gtccaggcgt ggtggctcac acctgtaatc ccagcacttt gggaggccaa ggtgggtaga
147121 ttgcttgagg ctaggagttt gagaccaggc tggccaacac tgtgaaaccc tgtctgtact
147181 aaaaattaaa aaaaaaaatt agccaggcat ggtggcatgt gcctgtaatt ccagctactc

147241 gggaggctga ggcacaagaa tcacttgaac ccaggaggtg gaggctgcca tgagctgaga
147301 tcataatact gcactccagc ctgggtgaca gagactgtgt ctcaaataaa attttttttt
147361 ttttttttac taaagataac ttgcagtaga acattccaaa agaacaacac tgcactgaag
147421 tgcatttgaa aatgagggct cccaaattag tctcatgtac agtctcatgt agggatgcct
147481 attgatatgc agaagcttct aaaaaatttc aatatttgca tttaaagact ttacaaaaa
147541 gaattaaaaa gcttaaacaa ctaattgatt taaaaaatta aatctgcctt gcgctttttg
147601 ctgatggctg tgtgtgacag gattaggcat gtacaggatc atgggacatg gggaactttt
147661 ttctccccaa agcaggaaac ttgagagctg atgagatggc tggaaaagat ccttcatgac
147721 tgacaagcag ctgcctgaac tttttttttt tttgaaacgg agtctcactc tgtctcccag
147781 gctggagtgc ggtggcacaa tctcagctca ctgcaacctc cgcctcctgg gttcaagtga
147841 ttcttctgct tcagcctccc gagtagctgg gattacgggc gcccaccacc atgcctggct
147901 aattttttgt gtttagtaga gacagggttt caccatgttg gtcaggctgg tctcgaactc
147961 ctgacttcag gtgatccgcc caccttggcc tcccaaagtg ctgtgattaa aggcatgagc
148021 caccgcacct ggccctgaac ttttaattta gtgtcactgc aataggtggg tccttctctg
148081 tcctccctga actctttgcc ttccccaccc tgctgcagac aatgcttttc tttctctctt
148141 tctcttcttt cctttttcta tctttctttt tttttttttt ttgaaacgga gtcttgctat
148201 gtcacccagc ctggagtgca gtggtgcgat ctcggctcac tgcaagctct gcctccgagg
148261 gtcatgccat tctcctgcct cagcctccgg agtagctggg accacaggcg cccgccacca
148321 cgcctggcta attttttttt tttttgtatt tttagtagag acggagtttc accgtcttag
148381 ccagaatggt ctcgatctcc tgacctcgtg atctgcccac ctcggcctcc caaagtgctg
148441 ggattacagg cgtgagccac cacgcccggc ctcctttttc cagctttcta ttatatcttg
148501 tctagagatc acatgttgaa acttctggct ggaggccatt ccaccccact ttgaatagat
148561 taaagatagc agagccccac caggggcaag tttaagcctt accagttcaa tattgggcac
148621 taagcagagt ggctaatgtc tatgttttgt cacatgtatt ttgctgtggc tggaagggaa
148681 aatgttaatt cagttccctc atgcaatctc ttgggcagca tcttacaaaa ttgagaggct
148741 tttgcctatg gttccctgaa atcagaaaag atgattttcc tttgtgttgt ggcttggccc
148801 ccaggctatg tgcagctaac agggtcgcta gggccactca gaaagaggga acccagaagc
148861 ctggcatgct ggcaaaaggg taagaatttc ttagcagtca ggcttctggc ctctctctgt
148921 gtggaaacgg ttgtaggagt agtaaaaatc actgtctcct ctgcaaagtt ttaattaatg
148981 ataaaaagga tttgtgaggc tgggtgcggt ggctcacgcc tgtaatccca gcactttggg
149041 tggccgaggc gggtggatca cgaggtcagg agatcaagac caacctggct aacacagtga
149101 aacccagtct ctactaaaaa tacaaaaaat tagccgggcg aggtggcggg cgcctgtagt
149161 cccagctact cgggaggctg aggcaggaga atggcgtgaa cccgggaggt ggaacttgca
149221 gtgagccgag atcgcgccac tgtactccag cctgggcgac agcgagactc cgtctcaaaa
149281 aaaaaaaaaa aaaaaaaaag gtatttgtga gccaatctta agctgtagca aacctggtgt
149341 actttgtgct atgaattggt ctttctgtgt catttggtca taaaaggggt taaataggat
149401 aaaatgtggg cctaggactc ccagaagtct gctgttcaag ccagcctggc aaactggtca
149461 gttacaaact gcgggtccct gaaacaaaaa aacactggat aaggtttccc tgtcatcttg
149521 ttttatgtca ttgggagctt gaccttataa tcacatgatg gtactttctt ttggtctcca
149581 ccctctggag gacaggaatt ttggagttca tgtcatagtt agctctaaaa attatcttga
149641 gcagttaaaa gcctttgcaa gctgaaaatt ggctgctctt aggctcctcc ttctgggagg
149701 aacaatggaa accaccaacg gtgtagctta gtggctaagc tttgccatct tatgatggca
149761 gccaggcagg gttcaattcc agctcaggga atgagacctg tttggtttga tatctttctg
149821 atctttgcta tttgctgatt ctcttcccct catgaacaac ttctgactct cgtcttgaat
149881 tttcctttct ctgagctacg tttggaaatt ctagagtttg taaaaattgc ttgctacctt
149941 tttgaaaata cttcgtacac ttgtggttaa gtcataacct tgttaaagct tattggtttc
150001 acctgggaga ttacctttgg taaagttcaa aagccagaca tattggctgt ttggcctggc
150061 taaagtcaag taataagaga tttaaaatga ttattttagg ctgggcgcag tggtccatgc
150121 ctgcaatccc agcgctttgg gaggccaagg aggatggatc acctgaagtc aggagtttga
150181 gaccagcctg accaacatgg agaaaccctg tctctactaa aaatacaaaa attagctgga
150241 tgtggtggct cgcactggta gtcccaccta ttcaggaggc tgaggcagga taatcgcttt
150301 aacctgggag gcggaggttg cagtgagctg agattgaacc actgaactcc agcctgggca
150361 agagagcaag actccgtctc taaaaacaat caaataattt tttttttttt ttagaaagag
150421 cgctatagtt aaaagtcagc ttaattaaaa gtggatatcc aagctatagg tatgtttaaa
150481 gggccttttg gttttttctc ttcttggatc tttgtttttt tttaattatt tttttctttt
150541 tagccaactg aattgttttt ctccattttg tcttcttgcc acttttgttg cacacatgag
150601 aggacctaag gtaacttcta acagcctggg actccttggg aaaaacagag gaggcactac
150661 gatcctgctt tgggatataa ctcggttttc ctcctgaaac accaggaatc aaaagcagat
150721 agatccctct caaaatctaa ggctaaatat ccttttgggg atggccaaca gggtgaaacc
150781 cagtctctac taaaaataca aaaattagcc tggtgcagtg ctgcacacct gcagttccag
150841 ctactggaga gtctgaggca tgagaattgc ttgagcctgg aaggcggagt tgcagtgagc
150901 cgagattgca ccactgcact ccaacatggg tgacagagtg atactctgtc tcaaaataaa
150961 ggaaagaaag aaagagagag agagagtgga aggaaggaag gaaggaagga gggaaggaag

151021 gaaggtcatt tccacagtta attgcttaat gctggtgcaa tttctgaaaa cttcacaagt
151081 aggcacaatt ctagaatatg gtgtctttta ggaggttcct gaaaggatgg aaaggacccc
151141 aaaaagtatt cttgattaca ggtttctgat aactttagaa tcatatcatt tttactgggt
151201 aagaattcct ggagcttttt tttttttttt gagacggagt ctcactctgt cacccaggct
151261 ggagtgcagt ggcacgatct tggctcactg caagctccgc ctcccaggtt cacgccattc
151321 tcctgcttcc tggagcttaa aagactgact ggtttataaa actgccatcc caagtagaac
151381 acaaattaat tgaataccaa gaaaatactt tgcaagtctt tcatgctaaa tcacccaata
151441 ctgaaattgt ttagatacac aatttgaaga aactctgtgg tctaactcaa attacctatg
151501 ataacccatc agttatcagt gctatgcacc tcaattggag aaacaaccgt gtcaagcatg
151561 gactcatgga gaaccaaggt ggctgcctta tccttcctga gcttttaaag cttttataat
151621 ttaaagttct gcattccatg actcatcatg gaaaagataa aatgatccaa gttaaatgca
151681 tattggtgtg gtgacttcta aattgctaaa atagtttatg accaatgttt ggtttgccaa
151741 acccatattc ctgggaagac aatcaaagct tcaggtacat tctgctacct catgagccat
151801 ttaaacattt atgaagagat ttcagtcaat tgtcattttc aatgcatgtt ttctggttgt
151861 gtaaaagctt tcctatacaa gtgaactgat gttataacag tagattatta tttcacagtg
151921 tattttcacc aggtaaagaa agctttttat ggttcactga ctgaggacaa tcaacccctt
151981 cacaatctag aacctgaaga ttggatcttc tgagaacatc agagaaagac tgcccttgcc
152041 atccacactg cagtaaacat cagaaccgtg aactttggct tcatgatctc acaactgaga
152101 aggatccctc cacattcttg gaaatataca cccattggaa cccgtaaggt aaagctaacc
152161 agggaagttt cttcccagaa gaagatggta tccttgatgt gaacagcttt caccaagatc
152221 acagatcaag acttctctgc tatatggagg ctcttatctc tgagtatttt ttcccttgct
152281 gatgcctcta tgaacagtag aagtgaaaag ggggtctgtt gtgtgcactt atggggtagg
152341 tatactttta ttggtgaagg gttttgcagc cagccttata catggataac ctcacacctt
152401 gatagatgaa agatgaaggc ccagtgtagg tgagaaattt taatggtaca taggttgcct
152461 cataatcagt cagaaacaga acattgattc actcccctat tccacatcat gggttaaaga
152521 gaacattgcc aggaggcctt cagtcttcta gaagggcatt tttccttagg tcctttttac
152581 catgatttgg agtaaaacag gcaatgatta gaaatgtatc cctcatgata ggctctaaac
152641 agagtctact gtaagggcga tggttacaca acagatttta cattcttttg tgaaagttat
152701 gcttaatagt aggattgctg tagattactt actggctaaa cagagaagta tctgtgcagc
152761 tgctggcact tgtggcctat ggagaaatac atcacgtcag gtgtacagag attcagttgt
152821 aggtaattaa tgaagagact gcttaaatga gtaaactctt tatttagctc attctttgat
152881 ctatttaatt ttgggtggtt tggtttatgg ggaccctgtg taaggagcat actccaaact
152941 cttggtatta tcctcctgat agtcataata gtagtctacc tggtgcactg tattctctcg
153001 aaagttttaa atgtttgcat gcagccatct ctagaatgtc aaattgtctc tcttcaacta
153061 gaatgacaag attgaaagaa atgtgtgacc atgatggcat tttaacctat gaatgatgtg
153121 ttgagactgg aaacccaaaa tgatggtaac tgagagtggc gctaaggccc taagctttgg
153181 tcacattctc acctaagtga gaacctgacc aaaaagggga catttttaaa caaaatcatg
153241 ggaggccatt gttttggact ttaatgaaag gctaaatgca ctaagcccca acagaccaga
153301 ccaaaccaaa atggagtcac ttgtgctaaa tgtgacatga ttaaactaag actttaagga
153361 aacacataga tcctaaaaca gaccaggttt tgtttttttc tcctataaac agaacgttcc
153421 aacaaaagga ggtaaccctt acaaaaataa aattaaaata aaataaaata acctgaaatc
153481 cttgttcccc cttacaaaac tcattgttct gctatttccc agggggtttc aagaccaaat
153541 aagtacattt acaatggtga tagtggcatc gatgactaaa gttttggtaa atctctaaaa
153601 attgagaaga ttaccaaaag aggaaaattg ttaaatcaag tttagcctaa agctgcctcc
153661 ttacatattt taagtttggc ctaaaggttt ctctgtacat cgtgaactat aacaagtgga
153721 ggtgtaaaca gcctacactt gtgtcaatca ccaagtttcg gccaatcata tgtagccaac
153781 tgttcaaact gtgttcaaat aaggatacac ccagctgtaa ccaattgggt gtttctgtac
153841 ctcacttctg ttttctgtat gtcactttcc tttttctgtc cataaatctt ccaccacatg
153901 cctgcaatgg agtctctgag cctactctgc ctcgggaggc tgcctgattc acgaatcgtt
153961 cattgctcaa tttgactctt ttatattgaa ttcagctgaa gtttttcttt ttttcttttt
154021 tctgtttttt tttttcgaga cagagtctca ctccgtcacc cagtctggag tgcagtggcg
154081 caatctcagc ccactgtaac ctcctcctcc cggttcaagt gagtctcctg actcagcctc
154141 ccaagtagct gggactacag acagcatgcc tggctaattt ttgtatcttt agtagagaca
154201 aagaggttta gtagagacaa aggggtttca ccatgttggc caggctgatc ttgaattcct
154261 gacctcaagt gaaccacctg cctcggcctc ccaaagtgct gggattacag gcatgagcca
154321 ccgtgcctgg ccagaagttt ttctttgaac actttttttt tttggggaag gagtctcgct
154381 ctatctccca gactggagtg cagtggcgcg atctcagctc actgcaagtt ccacctcccg
154441 ggttcacgcc attctcctgc ctcagcctcc tgagtagctg ggactacagg cacctgccac
154501 catgcccggc tgattacagg cgtgagccac cgcgcctggc cttctttgaa cacttctacg
154561 tggaagtgtg aggattcaat gaataagttg agcacatggt acatgttcag tgaatatttt
154621 ctgattgtgt tcagtgttat cattattgga atgatgtgag ggttgtgtaa gtcttcagag
154681 accaggaaaa caactgaaat cttctatgag tcatttctat cattgtttat ccccattcct
154741 tttcttttca ttcatttttg catttctcat tttctgtgtt attcccttgc attcttaaaa

154801 actttcccat tacctgcttg cttcttgtat tctgtgtact ttcctatgtg ttcctcattc
154861 tgcagtcttc ttccccttca agcagcagac catgtgtcaa cgtatgcaga gtttgtgcag
154921 acacacagac cctctgggga gtatatgttt gaatctgatg aagatgagca gttatttgtg
154981 cacctggaca agaaggagat ggtctggcat ctgtgagagt ttattcacac tgtgaggggc
155041 agaaaatgaa gggaccaggg atggaaaggg atgggggaca aaacactaag ctggcagtcc
155101 taaccccagt gtgtttattc tactccaggt gtcaaacttt ctcagtcaga agttgacata
155161 aggaagtcac cagcttcggt ctcctttaaa tctccaggcc taactttctt ttgaatctct
155221 gctcttcaac cacaatgact tatgtacccc aagacccctc tacctcctgg ctgcctatga
155281 tcctcctgca cctagcactc agcaaagggt ctgacattta gatagcactc aatcaatatg
155341 aagaatttaa agtgtagtga caaggtggtg cccacactgg atggtctgta tgctgtggtc
155401 tcatcaggga cctttctctg cactgcattt ttcctgagaa atcccagagt tcaataaata
155461 cctgtgtatg tcagagctag acacatctga tgcctttctg tgactcctta tattctcagt
155521 ttcctaggaa tgcatatcct ggacttgtaa ttgtaggata caggtgcatt atgggtgcag
155581 atacctcagt tttgcatcct gcaagagtta gtctgcagat tgttcactcc actctgggta
155641 cggctgacac ctacagcatg cgtccagggg acaaaaatgg aaggagatga ctctgattgg
155701 atagtgagag gaacacgggt agctctggta aactgaccag ggataggcgc ccaggggcac
155761 acaaggccca gaagctgctt gtgagaagtc agggagaagg aagccaacaa ctggatgtat
155821 cagagagcca ctgagtgctc attatgtgta tcaggagtta agaatccatc ctctacacga
155881 aaaggaaagc ttagaccaaa catgtcttct accttgattt acagtaactt aatatgaaat
155941 gcaaaacaca taaaaatttt ggattgtaaa aaaaagatca gtgtttgaaa gagtgccaca
156001 cctattgctt tgcactttca acaatgggga ggcgtttctc actgtatgaa ttattagctc
156061 aggaggtcta ggctggtgaa tgaatgcaga cttagaggat aaatgtcgtc tacttgatgc
156121 attcaaaagg ccaagcaaca tttggttaag gagtatttgt gatggcatgc aaagaaagtg
156181 ttgaaagtgt gttagattat atttatcgtt ttaaattctg atgcttattt aatttcttaa
156241 cagtaatgct aaaacatgct cttagtaagt tatatgttga atcagtgcta tgttaatatg
156301 ggccctataa cacagatttg cagtctgttc accactgaat agctaagaat agtggtttca
156361 gacctaaatg gatgaagaca ccaaacatga ggatggggca gctcactgaa atatagtaaa
156421 tccaggagag gtctatgtcc aacaggccac cttatacaat agtttggatg gattttcatg
156481 gtttcttctt tcttatgtct aaaccacaat gaacttcttt attaactctt actgtttgct
156541 aaaaccctca agaaaaaaaa gtgatgagac atgacttggt gtatattctt aacagaaaac
156601 caatcatgtt gggttttgat tactcatggc actaaggaga aaggaacttg gttggaggcc
156661 ccatgagaga aatcatgcat tgaatccagg aacagaatga tagaaactgg cttcagtgca
156721 actactcagc agcagcatcc tcttgggagg ctgggggtct gtcctcgaag caggtactgg
156781 gactaaagaa acagaaaatg cttgttccaa ggtacattag tcttttaggc aaggtcagca
156841 accctgtgga ggtaaaaaac agtaatccca ggttgctggc cccctccttt aagtcccag
156901 gagagaaaaa taacggtgag aggctgaatc tcaggctgat aaagaataga gttgatctga
156961 gcttccttta gcaatggagc catgcaatgc tctttcccca ctcaccccag ggctgaggtt
157021 gaaacagggg ttcaggcctg agactctctt cctagaggag cctcagtgtc acaatgtaca
157081 gaaaggactt atgtcctcct gtaactcccc caacaaggaa atcaaagcag gaatattgcc
157141 tgtttatgca gatccttgtt gaactgaaat gaaaggaaga aagagaatgt attcagctac
157201 tgaaaccacc tcagacaatt agagaaacgt ttccatcata cttccattgt ccatcactca
157261 atggacgctc tgcaggagcc tgagtaactg ggacctcatg catctggctg cattaagatg
157321 ccaggctggg attcctctct ccttagaagc tgtctttgtt tcctgactat gtgatttcag
157381 caagctctct ggcattgctt ttttgtctca gcccctcatc atggaagaag ggtgagcaag
157441 tcaggagaaa ggtgggtatc ggtcacctca caggatttct cacctttctt cctccttctg
157501 tgcgtgaaga tgtccactcc acagatgatg agccccagca tgaagcccct ggctcctgtc
157561 agcatcttgc tctgcacaga atcagactgt gcctctagga agaacaggct caggttcaga
157621 gtcacctcca gcagctgcac ccctgctcat gtcctgtctg agatcctgca gcctgatgca
157681 gaatattggc acgaggagag cgaggggaat acaccttgca tgacaggaaa ctgagaaaca
157741 gtcactctct caattctaga acagagggtg tgacctcaac aacagacaaa ttcagattca
157801 attagttgcc tctcaaaata acttagaata agctaaaagg ctgaaggaag acaacagttc
157861 ccatgtgtaa cgtcagttca ggaaggtgtc tgcatcccta gattcttgga caaggagagg
157921 aactatgaat ccttaggacc tcctaggccg ggggcaggca agtctccatt gtccccaggt
157981 taaaactaca ctcagtgtct tctatagaag aaaaatgggg tcggacatgg tcccagggta
158041 tagacacata aaaccagatg agtggacccc agagccagag agtcccctgc tcttcagagg
158101 tggagtctag agggtcatca gaccctcact ccactccacg gtgacaggac tgtccaggct
158161 ggggtgctcc acttggcagg tgtagatgtt tccctgctgg ggggtcattt ccagcatctc
158221 caggatctgg aaggtccagt ctccattacg gatcaggttg gtggacacga ccccagctgt
158281 ttcctcctgt ccattcagga agcatcggac ttgaatgctg tctgggtaga aatctgtcac
158341 gtggtagaca agcaggtcgt ggtgctgctg gggctccttc ttggagggga gatgttcacc
158401 ctaggctgga ctaggagtgg cgaagtggaa aagtggaata gcatttgaga ttattatttc
158461 ttagacatgc tcattatgga attgatttct agatgttgaa aataggaatg atggccaggc
158521 gcagtggctt acacctgtaa tcccagcact ttgggaggct gaggcaggtg gttcgagacc

158581 agcctgacta acatggagaa accctgtctc tactaaaaat acaaaattag ctgggtgtgg
158641 tggtgcatgc ctgtaatccc agctactcag gaggctgagg caggagaatc gcttgaaccc
158701 cggaggcaga ggttgcatga gctaagatcc caccattgca ctccagcctg gacaacaaga
158761 gtgaaactcc atctcaaaaa aaaaaaaaaa aaaaaaaaaa aaagtaggaa tgatgtgatt
158821 aggtcagcag ataaggggag tattgaaact atagaaatag tattgagaag aataggaggc
158881 acacaaactt accagtttgt agcacattta aaagaaatct tatctatcag ccactgatta
158941 acagcctttg gacaaagaat atttaatcag cagcgacact accttatctt ttgagtgctc
159001 tatcctgatg caaaagatga aaaatttgct caaatgattt cttgaaatta atatatatat
159061 aaacaacatt ctcatggtct attaatccaa taactatatt tcataaatat atcaagaaaa
159121 taaagtgagc ttgacgtgac ttattctgca agtaacctat ggattttttg gcctcgccta
159181 caaacactca aaatcgtcct atttacataa tatcctgagc ccctacatgc ttaaatagta
159241 gaaatcccat ccttttccct aagttcctcc tctgcactgt gttgtcacat ccattgaggt
159301 tctcagataa ccaggtgttg gctgtgcctt ggcacctctc tatttttgcc ccctcttttt
159361 ctgccgctta cagatcaatt tccacgctac cacctgcagc ttttcctttg gacctcagcc
159421 actagtaccc ttttcctcca cagaaggcag aaaagggcca actgccccta gagaacagct
159481 caattacagg aatgttccaa atcagagctt ctaggtcctt atttactctg ttcttgagaa
159541 cacaaataca ctgacatggg cctgggccag gtaaatgaac agtctttaac taacccccaa
159601 cccttactcc ctatggcatt tttgccttgt gtccaggatt attcacattc acagcctctt
159661 ccctcgatgt gattttattt cagaggcact tggctctcgt tctacttcaa ccacctccat
159721 aaactcaaca tccatcagac tggtcagccc atgtctgaat cccaactgta gattaagtgg
159781 aacttcttcc ccataggtgt cctgggaagc atgaaagtca tgggatatat ggttgtctgt
159841 tttaaggtcc cagaacattc catgagaagc actttgctcc atcttctttg acgggagctg
159901 gtcagcctgg agcacatcag agacctccag ggggcgctgc agctaaggct tgatacccag
159961 tggcctcatt tctgacccag ttcaaatata tttcaccaag gcaaggattc ctgtttgttc
160021 tagttgcctt tgctcagtta attctagatt tgggtgggca tccacactgg aatggccaga
160081 acttctatcc tcctctttcc tccgtctccc ctacccctgt gtttttcttt attctaattc
160141 atgcccttat tatgatttcc ctggacaata atagcagttt cttatctcat ctccctgcct
160201 ctggttcctg ctctctccaa ctgacttcac agtcttctga aaacagattt ccagccttgt
160261 catttttttt tttttttttt tttttttttt ttgagacgga gtctcgctct gtcgcccagg
160321 ccggactgcg gactgcagtg gcgcaatctc ggctcactgc aagctccgcc tcccgggctc
160381 acgccattct cctgcctcag cctcccgagt agctgggact acaggcgccc gccaccgcgc
160441 ccggctaatt ttttgtattt ttagtagaga cggggtttca ccttgttagc caggatggtc
160501 tcgatctcct gacctcatga tccacccgcc tcggcctccc aaagtgctgg gattacaggc
160561 gtgagccacc gcgcccggcc tccagccttg tcattttaag atttctgaag attttcaaaa
160621 atcttcagtg ttctgacact tatctggtta catgggaatc acttgggagc tgattaaaaa
160681 cacagatctc cagacctccc cacctaggga atctgaatct ctaggcagtg agtctggggg
160741 aatctttgct tcacagagga tctcaaggag agtcttctga gcagtgaggt tggggacctg
160801 ctggcctaga ggagaaatcc acactcctta gtctggcatc agggggtctc tgcaatctgg
160861 cagctgatta ccatgaaaga aaatgctgcc atgtcgctga tttgggcctc tatgaagtct
160921 tagtgacaga ttttgcaaat tgtattactt ttatttggac acctctctca accaattacg
160981 ccagcaactt tcctaaaccc atgtcactct cctcatcctt actcaatgtg tgcatcttct
161041 ccctcttatc agatgacctt cctttcactt tcccaggaac agagggatca atggtgtgac
161101 ctcctgagcc tcctcctcct cccccttcat tctttcctct ttctctagga caaaggtcct
161161 tgtttagtga gaggctcact ggtgcttttt ggtctccttc acgggatttc tctcttggcc
161221 aaattaatac acgagttatt aagagattat ttttaggcag ctaaaaaggg taaaagttct
161281 gggtggaatt ttcctttaat aaaaagcagc cccaagccat tttttctcta acagaaagca
161341 gcctgaaaac tcaggcatag atatgcaaac tagaagcttt tatgtaaatg ctggcagctg
161401 ttcctggaag tcaggtaatt caatatggct attctcaccc tctttccctt ctacgtttac
161461 aggtgtcaag gcagcctcca ggttaaagca cgtgtacagg tatcatggcc gccaccaggt
161521 ggaggccgca tttgtataat aaaatactag ggtgggaagg ccagtctttt tgcaggttat
161581 gtaaatgaca cacctggtca aaccaatccc ctgagcccta tgtaaatcaa tcactgcctc
161641 ctcaagcctc tgtacaaaac caattgcttt ccaccagaaa caggagaccc tctcttgggt
161701 gacctgcctt atcagcatta ggaagctttt cctctcactc ctcttttcta ttaaactttc
161761 cgctcctaaa cccactcctt gtgtgtgtcc gtgctgtgaa ttcttttcgg ctatggtaaa
161821 gaaccagggt atatacccta gacagtggag ctgtttcatt ttgggagctc atccgggatc
161881 caaatcagaa tggaagatag aaacatcgga gtggtgagta tagagcaaac gtcaaatctg
161941 ttctttaatc tcaagaatct cttcatacca gtttcctttc atggagaact tcaccatcgc
162001 atgaggctgg gaaagtcttg gggcaactga aaatttctgg ccagggcaca ccctggtgtt
162061 attcaaaggc ttctggactg aacgcagcct ccgacagcct gtccaggtgt tggtaatgga
162121 tctccagcta acccgttgca aaattttcct ttcctttgta tccgtggtca ctatgtctcc
162181 tgtcctctct ctctgtgtgt gcaatttgcg ggaagtttta cagttcaggg aaacactcct
162241 gttagggaag atcggcaaat gccacaggca gtaactgtta ctctctatcc tctctggcga
162301 gcacatggta gtgctaagcc aacagcacca cctagtggaa ataaaaatcc tcttcatcag

162361 gcaccttgtc ggttttttac cgtaacactg ccgcttccaa attctttcgt gccgctagaa
162421 aagcctcttc tgtgaacgag aaagcactgt cttcaacagt ttggagtaaa atgtcctctg
162481 tagtgaaatt ttagttctga tactgtctca tcagcaggaa aaacagccat taggttccta
162541 cgttcatttc cgtctccaat taggatagta cttaattagc aaggggattt taggttcgga
162601 agttaaccag agccattttg ctaagggtaa atgtcttagc atgggccgta atggcaggca
162661 atctagcaca ctgcctccgt taaaggagcc tacccaaaga tgacatagtc tctctggaga
162721 tccatttttc tgggagccag gcagatcaca caaatttagg acgtcaaagg ggatcacata
162781 aggtggataa gctaaggttg tgtgggtaaa gtatggttaa tcccatcact tagtttatcc
162841 agttccacgg cttggaggac cacgcctaca accatgggtg gtacatttaa cacgttgcca
162901 ggacccagga accaaggaga gaaaacagta gggaggacac ttccactgtc ttctcctcca
162961 ccctgggtca caatgaaaga atggggacga aaggatactt ttattctcac ttctttttct
163021 agatgggtga cagaccagct tcagcttgca cccctctgga gtgcactctg aaacactgga
163081 actcctttaa cctcaggact ttgaagagaa aagtgactca ttttcttttg cacaagggca
163141 tggctttttt actaaacctt tgcaagcact gtaagatcag cccagctttt taaataggca
163201 tatcaggtag gcctatagaa aataatcccc cagaattaga aaggcaattt ccaaggaac
163261 catctgagaa ttccccttat ttagggttgc caatatgggg gaagtaaaga gaaatcagac
163321 tgttgctgtg tctatgtaga aaaaggaaga cataagaaac tccattttga tctgtactaa
163381 ggaaaattct tctgccttga catgctgtta atctgtaacc ctagccccaa ccctgtgctc
163441 gcagaaaacc tgtgctgtat tgactcaagg tttaatggat ttagggctgt gcagggtgtg
163501 ctttgttaaa aatgtgtttg taggcagtat gcttggtgaa agtcattgcc attctccagt
163561 ctcgagtacc cagggacaca atgcactgtg gaaggccgca gggacctctg cccaagaaag
163621 cctgggtatt gtccaagggt tccccccact gagagagaca gcctgagaca tggccttgtg
163681 ggaagggacc tgacctgacc gtccccaagg ctgattccca taaagggtct gtgctgagga
163741 ggattagtga aagaggaagg cctctttgca gttgagataa gaggaaggca tctgtctcct
163801 gctcatccct gggaatggaa tgtctcggtg taaaacccga tcatacattc tatttactga
163861 gataggagaa agccgcctta tggctggagg tgagacatgc tggtggtaat actgctgttt
163921 actgcactga gatgtttgtg taaagtcaaa cataaatctg gcctatgtgc acatccaggc
163981 acagcacctt tccttaaact tatttatgac acagagtcct ttgctcacat gttttcctgc
164041 tgaccctctc cccaccatta ccctatagtc ctgccacatc cccctcactg agatgataga
164101 gatagtgatc aataaatact gagggaactc agagaccaga gccggcacag gtcctctgta
164161 tgctgagcac tggtcccttg ggcccactgt tctttctcta tactttgtct ctgtgtctta
164221 tttcttttct cagtctctcg tcccacctga tgagaaatac ccacaggtgt ggaggggctg
164281 gcccccttca acctcaagct cccttttcat tacaggacct taggcaaaca aaggaagact
164341 tatgctaatt ttctgataac cccaataggt atatagaagc tgtccagaat ttaactcagg
164401 tgtttcacct cacatggaag gatgttatgc tgctcctaaa ccaaactcta accgcagttg
164461 aaaagcaggc agctctgcag gcagcagata attttggaga tgagcaacat atctcctata
164521 atacaccaaa agggaagaaa agagataggg aaagtgaaaa aaataacaga aacaccattc
164581 ccagtaggaa gggaagcagt tcctctcgac aaccccaact gggaccccag tagctctgca
164641 aatgaataga aatggaagca tttttaaaat atgcatatta gagggtctat gaagaactaa
164701 ggcctgacct cttaattcct ctcaactgtc tatgatagac caaaagccag atgggaatcc
164761 tgcagctttt atggaaaggc tgagagaggc actaatagag cacacttcct tagcccctaa
164821 ttcagtcaag ggatggctca ttgtaaagac aagtttatta cacaggcagc tcttgatatt
164881 agaaggaaac tgtagaagca cgctatagga ccagatagca ccttggggaa cctcctgagg
164941 gtggccactt ataataggga ccaggaggag gccccccaga aagagagaaa gctcaggaga
165001 aagacagagg ctctagtagc agctttgcaa gcttgcaaag tccaagattt ctgaggtgca
165061 tccgctagtt gctatcagtg tggcaagcca gggcatttta aaaaggagtg cccaaacagc
165121 aagaggaagc cacctcaacc ctatccagcc tgtggtggag accactggaa atcaaactgc
165181 ccccggagac ggaggtcact ggagtcagaa ccagtctcac agatggtcca gcaggactga
165241 tgggtcctgg ggctcaaacc ccagctccag tggctcaaac tgccattaca gcacgggagc
165301 caccaggtga ttctggaaat tgaaggaagg aaactagacc tccttctaaa cactcgagcc
165361 agtctctctc tcttttctcc tctttaatcc aggcctctct tcttcccata gcgtgagtgt
165421 aaggggtgtc tcaggaaaaa ctctattcca atatttttct caacctccta attgcagtta
165481 ggaggaccta ttgtttacac atgcttccaa gccattgcca cggtggctct actagtcaaa
165541 aaagcctcca aattaaccct aggaaataat ttaactgttt acaccccaca taatgtagca
165601 ggattactgt cctctagggg agactttagc taacaaacag caggtaaagc aagaaataca
165661 taaggcagga caagcaatag tcactctaat gtctctcccc agacacaagc actcaattag
165721 ctgaactaat agttcttgca agtgcactta aattaagcag gggaaagata gctaacattt
165781 ccactgactc caagtatgct ttcttagttc tccatgctca tgctgctatt taaaaggaaa
165841 gacattcttt taccactaaa gcatctccta taaaatatca ccaggaaatt aacaggttat
165901 tatcctcagt tttcctttca cgaaaaatag cagtaatgta ttatagggaa catcaaagag
165961 gaacagatga agtagccaaa ggaaataggt tagctgagca gggagctaag caggcggcag
166021 ggaagcctca aggcattaac acacttcaag cccttttaat ctcggaagcc tccataaaag
166081 aaattaaacc tcagtattcc cctgcagaaa taaaataagc cacttcttaa gggtattcca

166141 gccctgagga tgacaaactc catttactgg cctccagtca atggaaagtc cttaaaatcc
166201 ttcaccaagc tttttcacat aggaaaggat aaaacttatc atcagtgtgc tcagagattg
166261 ttttcaggca gaaaacctct aagttgttta aaaatgtaac ctctctagct cacttccaac
166321 agaaattgac acaactagcc aaaggccaac cccaggaaat tggaccacca gaaaatttgg
166381 tattggtgaa aactcatctc tctctccttc cctaagccag gctgggaagg gccctacaca
166441 attcttcttt caaccccccc agcagtaaaa gttacaagta tcaacctctg aatatatcac
166501 actcaagtca aagcctgaaa agctgaggga gcaacctttg acagcccaga ggaacatcct
166561 gaatatcaat gtggagatat agaagatctt aagctgaaaa tcataaaagg taagtaaatg
166621 agtgagggct actcgcctta gcgccaatcc tacctcacca ggtactcttt atcatttcta
166681 cctttcctct caaaattcac tgctcagtat tagaactttt ttttaatgca tatttgcaga
166741 gagattttaa ttatacatgg gactgcattt gttactttgt aaatccccaa agggaaacat
166801 tatatcttgg caagtaaagt tttaaatgga aattatttac tacgtcactt ttgtgggaat
166861 tgttatcatc atgctgttat ttgcaataga actgtatact gtggcaccca caatgtggaa
166921 ttctggttgt aaaattctaa ttcctgtaat attttgccta attatcatct ttatgacaga
166981 attaataatt gcaggaagga tttggtcaag tttgttttgc ttatagcagg agtaatagtt
167041 acagacaaga agtaagcatg aaaattttac tatcactaag tttgatagga cttttttatt
167101 gaagattggt aaatggtgca ctctaagcta tggaaagaag gttacaaata aagggatttt
167161 atataagaaa ggatcttgta tagtaaattc ttgtcctaaa aggaaatgac tggttgttta
167221 agacaagtca gaaagttgag tacattgtaa gagggtctgt gaaagtcatg aaagaattta
167281 ataattaaga aatttaataa ttaaaggaaa ggaattgcca agattaacac caaagttatt
167341 ttagccaccc aataacgttt ttctcccaat catatcataa gttataaaga atggcctaaa
167401 ccaaaaatta tgccctaata gcaagtcaag ggggaaacat gttttctcaa aggaaatgat
167461 gcttttatat taacgtttct ggtaatgtac agcgacatct agtggagaca aaccagtatt
167521 acaatccatt ggtgtaacag gtatcaaact ctactgccat agttacagtc tataggtggt
167581 aatcttaata ctcatatggt aaccctatat tttaaacctt cttgtaaaat ttatctcttt
167641 ttgcctagaa gcaatcaaac ttcaaatggt gctgcaaaca aagccacaca tggacatgcc
167701 attcttccaa gaagccttag atcaacctca ggaggagccc caactgcagc cccccgacac
167761 gacgcccctt ttcagcagga agtagccaga aagaatcgtc gtccaacacc ccctaacagc
167821 agttatggtt acgtctcctg agggaggaaa taatacagga gttattaaga aattattttt
167881 aggcagctag aaagggtaaa aattctcagt ggaattttcc tttaataaaa agcagcccca
167941 aaccatttct tctctaacag gaagcagcct gaaaactcag gcatagatat gcaaactaga
168001 agcttttata tgtaaatgct ggcagctgta cctggaagtc aggtacatcc aatatggcgg
168061 ttcccactct cttttccttg tcaccacgtt tacaggtgtc atggcagcct ccaggtaaaa
168121 ccacatgtac aggtatcctg tccaccacca gttggagacc gtatttgaat aataaaagac
168181 tagggtggga gagtcagtct tttcgtgggc tatgtaaatg acacaactgg tcaaaccaat
168241 tccctgagcg ctgtgtaaat caatcaccgc ctcctcaacc tctgtacaaa accgactgca
168301 ttccaccaca aaccgcagac cctcttttgg gcaacccact ttctcagcat gaggaaggat
168361 tttttctctc ttttcttttc tattaaactt tccactccca aacccactcc tcacgtgtgt
168421 ctgtgtcgtg aattttctcg gccatgacaa agaaccaggg tatatacccc agacaatgga
168481 gccgttacaa aatcacaatg tccagttccc aggatccagt cctatgctcc tcccgatggg
168541 tcccctctcc ctgggtggtc tcatgtaggc caggcccctc ccccgctgca aactcttctc
168601 tcacctgccc atcagaccac ccatctggcc cctcaagcac ctcaaacccg gccatctccc
168661 tgcaggtttc ttctctgcgt ggcctgctgt gccatctcca ccttcatctc cacaccgcac
168721 ccctgcacga tgtcctgtgg cctcgtttcc cctcacccca catgcagtca gctgccaggc
168781 ctgatgaagt cccaggggtc tctctccctc catcctcctc actccatgct cagcccaatc
168841 tccgtgctca ccctcctgac aagctccggt tgggctcctc ccatcaatcc ccagctcaaa
168901 gtccccttcc ctcctggcac taaggtccct taggccggcc cggctgcccc tactccctgg
168961 gcccggcccc cacggctgcc ccaggagccc tggttcacag ccctcacctc ggcgctgccg
169021 gatgagtggc tccatcagct cgtacttgtg tctgcacacc ttgtccacct cggctcgctt
169081 ccgttccata aagtccttct ggctattgaa gtactcgcct atgggccgcc ccagctccat
169141 cactgctagg aactccccca ctgcgctgtc aaaatgcacg tattcctccc ggttgtagat
169201 gagcccgtcc acaacgcgct gagtcccatt gaacgcatag cattcctgcc gttcctggta
169261 gacggaattc tctgtgaaga gcagggagag atgggggtgg tgccactccc aacaacaccc
169321 ccctcctcaa tattagctat ttcctcaaat cttcccactc aggactggat ttaaaaatac
169381 gatttttcct actaccgagt tctgtggtcc taggcaggtc acagactcca ggcatcagtt
169441 ttctcaccac gcagcgagag gatttactga aaagaatgag actcactgct cagggtggct
169501 gagtgatcat tagggagggg cgcacgctga agggaaagca gccctttctg ctggcggctg
169561 gaggaggagg gggagacatt tcacgtctca gggaggagca gaatctcatc agggggaggg
169621 tccctcaggc agagaaacag tagggatagg aggagttggg gacctggagg gcagagctgc
169681 tccccctacc caactccctg cctgacattt ccatcctgat gtcagtcctg gctcaaatgc
169741 cacctcctcc aggaagccct ccattccttc tttccctttc tacagctagg ttctctctct
169801 tctctgccag tttcttgaga atacctcaag ttgctcttgc tgttctgcat gctggacatc
169861 tctgcatgct ggaaatgcag aaatgcattt ccatgcattc ctccctccct ccctcccttc

169921 cttccttcct tctttcttct ttcaatggag tatccttccc tccctccctt ccttccttcc
169981 tttcttcctt tctttctaca gagtatcact ctgtcgccca ggctggagtc cagtggtgca
170041 atctcagctc actgcaacct ccgcctccag ggttcaagca gttttcctgc ctcagcctcc
170101 tgagtagctg ggattacagg tgtgcgccac cacgcccaga taatttttgt atgtttagta
170161 gagatggggt ttcaccatgt tggccaggct ggtctcgaac tcctgacctc gagatctgcc
170221 ctcctcggcc acccaaagtg ctgagattac aggcgtgagc caccgcactt ggccagaaat
170281 gcatttctta aaacttcttg aagtttgcca taagaaagac tatatagcct gaaagttaat
170341 tttcactttc aaacatggct gtagaaagac ttgatctcaa aacacctgga aaatatctta
170401 atcaatgaaa atcaccagaa caaccagaaa gctaagtgac tggactcctt taggtttaga
170461 ggcattgatg gtgtcatttt tacataaaga aatgtggcag ccttttccac ctgcagttct
170521 tcctagggag caccatgggc agtgtccggc aggtgcatgt gtatacagaa gtagcaatga
170581 cccagcacat gtctgagcct cttcagacct gatctgttcc tggtcacagt ggggaaatta
170641 aggtgctgtg atttgcacac agcaaattaa ggtttgtttt ggacacctgt ttcatgttct
170701 acaaattagc aactcacgtt tattaacctc acccctcata gaagatgcac agctggtggc
170761 agaggacagt agagaagggg tggggctggg cacagcggca ccgtagactc ggcctgtagt
170821 ttccagtgtc atctctaagg caagatccca ggactttaga tatcttaacc cgcctcttct
170881 cctagtgcct tgggctccag ccccctaaac acccaggccc atcgccccca gcccctttag
170941 tctgcccctc cctattcaag tgccccactc tgagtgtgta tcctctcatt ttaatttgtt
171001 agtctttgtt cctcttcctc attcaccaat aagagaacat gttttagtca agagtaagaa
171061 tttcaaaact ttcttttgca acatttaact ttttaaaggg aattttacct gcaaagcaga
171121 tataagtgat gagtatatgg tggaatttga aacttcattt tttttttgta tatggaaaac
171181 tttatcctgt ttgcggaatc tttgacaatt gagttaccca gagcacaatt tgaaaaccaa
171241 tgatctgagt gaattcatct ctcactcaga ataggttata ttaaaatata attgcagaaa
171301 gatataatgg agtccatatg ggtaagaaaa ggaacatgtc acaggtaggg attccagttc
171361 ttgcactacg actgattact aagttatctt ggacaagaaa caaccttcta actctccatt
171421 tcttgaaagg caaaatagta ataatactat ttaccttgca aaactgctgt gagaaccaaa
171481 tgagctcaca tatgtcaaat acatagtatt gtatctgtta ctgctcaatc tatcttagtt
171541 cccttcctct tttcactgtg tgacctgttt gaataggagc aaaattctaa ataaatgcat
171601 atgaaagagg aactggatca gcttatgagc tactggaaaa cctcaagaag actttccatg
171661 ggcaccagat cttaatggtt gaaaagtgtt tgttggctgt ttgctgactg gatcttcccc
171721 cctctctttt gtgctaggga ttttatttga ggagttagat taacagaaga gtgagtaccc
171781 aaagactcaa atgcacaatg agcttctgca agtgcccagc tggtgtgaca ggaggactcg
171841 ggagtgtggc cctaatgctt ggggtagtgg gtggggtgag gtaagaaact cagctgtaaa
171901 aagcccattt taaagcttgt cagagacaaa agggtgttgc ctccaggagt ctggtttttt
171961 cacctgccgt gtcatctcct gctccctagg tgttgtctca gccaagcttt tctccaccct
172021 cccctctctc actttaagcc acagcctggc tccttgtgcc tttcctcttg tgctctgagg
172081 atggagatgt gtgtggagtt ggaagagcat gcagggaatt gtggaattgg ccctgcccac
172141 tctacccact cccctatggc tccagctctc cctccagcag gttttgattg gacattcatt
172201 ctacacgggg agctctggta acccaccctc ggttcctgtc acatggctcc actgcctcat
172261 ctcatttccc ctaccaacct caacccagtc tttgcccgtc cacctgttca ctgcccacca
172321 tcatcacgct ccctcctgtg ctttctgcta ccccgcacct tgagggtttc catggcgttt
172381 cccaacacca cccctcatcc tacaaacaac tctgcctatg gacactgttg ctatggacct
172441 cctgctggac actgttcagt gtcaccagcg ctgctccagc ctcctctctc cccaacctca
172501 cccctctcca gttcccaggg ctgagccatt ctgctggtta gttctcagca cccctgtgac
172561 tacaagtgca gtttgtccac cctttcccgg acaatgaacc tgaggtaata ggtgaggggc
172621 tttggggttt gaggggctgt cctcaggaga ttcgaatact gttaccctgg aaaatgagga
172681 ggtgacatga gaacagcact ttctaggggt gtcctaggtg gatgtggaag ggtctcagag
172741 ggagggtcta tgcagaaagg tggaagtcag tggaaaacta agacacctac tctgcagtcc
172801 ttcctctcag ggtgttggtg taaatttgga ccagaaaagt aagaacatcc tgagagaaaa
172861 acaatgggtc atagaagcca taatattaca ccagccacaa ggagacagca ggagacagag
172921 gtttttccct tggttactgc tttcttggct gtctgataac ctacactcaa tctctttcac
172981 gtactcacac atccttatct cattcttctg acaggtttca acccatccta atactctaac
173041 caagttctgg ggaggctggg agaaatacct tcaacaagag tgcctttagg ggctcgaacc
173101 tgtccccctc cctcccatct tgccttcatt gtccagggag cattggctcc tgctccaccc
173161 tggagaatga gaggcattct ctgtgagcac tgaatcctca gtgatacttg tagtctggac
173221 acaccagcta agggctctct gcctgagtcc cctcaaggtt ggatgcagat gtgagcacac
173281 ccaggagtct gcacttgcca acctctctct ctgaaacctt gtctgtccaa ggttatcctg
173341 aacctcttgg ccccatttcc ccatagacaa gcaacttgac ccctgagcac ctccccttat
173401 ttactgtgtc catgttcctg gagagagaat agacctggtg gatagcaacc tatcctatag
173461 gaggtgagtt tgattctcca gctgtgatag aaggacacta ggccgtggca ggagccccac
173521 atgctgtctc agagtctggt tccataaaga gaaagtcccc taggaattgt tccctgagcc
173581 agaccctcca ggaatagcag ctctgctctt acctggagtg gccctgctct ggaccacaga
173641 tatgagcagc accatcagta atgctgtcag agccactgtc caggggcccc ctgaaacctg

173701 caggatcatc atggagttgg aaaaggttgg cagaatgaag agagctgcag tcaggaaaac
173761 aagaactcat taaagggagc tcctgtctga aatattagag accatgaacc caagcagtct
173821 tctgtgaccc taggattgga cagactctga gaaaagaacc aatgggcact gagctttgta
173881 tgagtcattg ctcactgggc agaaagttag tattaaagat ctgacaatat agagccagtg
173941 atgctgttac gaggacagat ggagaacact gacactcatt ttaaccagtc agagtcatga
174001 gttttgggga gatgatgtgt tttctttgct ctgaaggtga tctcagatat tctgctggcc
174061 cacctacagg gattatcatt tccccaattc tgccacacct cacacaccca caggacatgg
174121 cctggtgtgg aagaaatgct atctcaatgt gtaaaaggtc attcagtggc atgatttaga
174181 gagattagag tatccatccc agaactgaaa atgaggcctg gagtctgttt tgcctttgtt
174241 caaggccgtg cttcagatta gtgcacattc atattttctt cctcccacat gtctgtgagt
174301 cctgagatgt gcgggggata ctggctcctt ccataggact gtcatcaggg tcagcagggc
174361 tcagtctagg ggccttacac ctgggagcat ggacacacca cctacactac catggaagta
174421 tgcagcttga aggacactgc ctgtcttgga cttcagttct ttgtcttcag tatggggatg
174481 atatgacctg cctttacagc agggctctta aggtcaaatt agatcaacgg atctgtaatt
174541 gctttggaaa aatgagacta aaaattatac agtgaatgag gaagaaatga aaaaatgtca
174601 taaaagaccc tacattttcc aaaacatctg attctgtggt gtttatactg aatagtttca
174661 taaactttca aagaatatta ctccttaatt taaagaccta taaatgtgat cctgtacgac
174721 ctcctaatct aataaagaaa atgtaaaagt ggcatcattt gtttatataa atgttaaaat
174781 gtaaatagaa gactagcatg taaaattcaa gagaagaaaa taattatgca gtagaagggg
174841 ccagtatagg attgcgggga aaaagctcac gttccctgcc atagtcacca agacagcatg
174901 gtactggtat aaaaataggc acatagacaa agggaacaga atagagaacc cagggataaa
174961 cccaaatact tacagccaac tgatcttcga caaagcgaac aaaaacatat ggtggggaaa
175021 gacacccttt tcaacaaatg gtgctgggat aattggctat ccacatgtag gagaatgaaa
175081 ccgggtcctc atctctcacc ttatacaaaa atcaactcaa gatggattaa ggacttaaac
175141 ctgagacctg agactataaa agctctagaa gatcacattg gaaaaaccct tctagacatt
175201 ggcttaggca gggatttcat ggccaagaac ccaaaagcaa attcaataaa aacaaagata
175261 aatagttgag acttaattaa actaaagagc ttttgcacgg caaaaggaac catcagcaga
175321 ataaacagac aacccacaga gtagtcacaa tctatacatc tgacaaagga ctaatatcca
175381 gaatctacaa tgaacacagg caaatcagta agaaaaaaca aacaacccca tcaaaaagtg
175441 ggctaaggac atgcatagac aattctcaaa agaagatata caaatggcca agaaacataa
175501 gaaaatgctc aacatcacta atgatcaggg aaatgcaaat caaaaccaca atgtgatacc
175561 accttactcc tgcaagaatg gccatgataa aaaaataaaa aaacagtaga tattggtgtg
175621 gatgtggtga tcagggaact cttctaccct gctggtggga atgtaaacta gtacagccac
175681 tgtggaaaac agtgaggaga ttccttaaag aactaaaagt ggaactataa tttgatc

- LOCUS: AF334946 2129 bp mRNA linear HTC 13-JUL-2001
- DEFINITION: Homo sapiens FKSG44 (FKSG44) mRNA, complete cds.
- ACCESSION: AF334946
- VERSION: AF334946.1 GI:12276205
- KEYWORDS: HTC.

1 tgagccgaga tcgcgccact gcactccagc ctgggcgaca gagcgagact ccgtgggctt
61 ggcggcgggg caggattcca ggcaggagcc ttgcctctca ggtggcgggc tctgcgaccc
121 tgcgagatgg acgggacaga aggcagtgcc gggcagcccg gccccgctga gcgatcccac
181 cgaagcagcg tgtcctccgt gggagcccga gcggctgacg tgctggtata cctagcggat
241 gacacggtgg tgcccctggc tgtggagaac ctgccctcgc tcagtgccca tgagctgcac
301 cgcgctgtcc gcgaggtcct gcagcttcca gacatcgccc tggatgtctt cgcgctctgg
361 ctggtctccc ctctgctgga ggtgcagctg aaacccaagc accagcccta caagctggga
421 cgccagtggc cggagctgct gctgcgcttc accagtgccc cagatgatga cgtggccatg
481 gatgagcctt tcctgcagtt ccgaaggaac gtgttcttcc caaagcggcg ggagctccag
541 atccatgacg aggaggtcct gcggctgctc tatgaggagg ccaagggcaa cgtgctggct
601 gcacggtacc cgtgcgacgt ggaggactgc gaggctctgg gcgccctggt gtgccgcgtg
661 cagcttgggc cctaccagcc cggccggccg gcagcctgcg acctgaggga gaagctggac
721 tccttcctcc ctgcccacct ctgtaagcgg ggccagagtc tctttgctgc cctccggggc
781 cgtgggccca gggccgggcc gggcgagcag ggcctgctga acgcctaccg ccaggtgcag
841 gaggtcagca gcgacggcgg gtgcgaggcc gccctgggca cccactaccg cgcctatctc
901 ctcaagtgcc acgagctgcc gttttatggg tgtgccttct tccacggtga ggttgacaag
961 ccggcccaag gctttttgca ccggggtggg cgcaagccag tttctgtggc catcagtctg
1021 gaaggcgtgc acgtcatcga tagcagagag aagcatgtcc tgctgggcct gcgcttccag
1081 gagctgtcgt gggaccacac ctcccccgag gaggaggagc ccatcttgtg gctggagttc
1141 gacggggaca gcgagggcac acctgtcaac aagctcctca agatctactc caagcaggcc
1201 gaactgatga gcagtctcat tgagtactgc atcgaactga gccaggcggc ggagcccgca
1261 ggcccccagg acagtgcgac tggctcgccc tcggacccca gctcctcact ggctcctgtt
1321 cagcgcccca agctgcggag gcagggcagt gtggtgtcca gccggatcca gcatctctcc

1381 accatcgact acgtggagga cggcaagggg atcaggcgag tgaagccgaa gcgcaccaca
1441 tccttcttca gccggcagct gtccttgggc caggggagct acaccgtggt gcagcccggc
1501 gacagcctgg agcagggctg aggacgctgc acccggcagg aggagggcga ctgggggccc
1561 tggcccggca ctgtcctcct gaggggcagg cgccggctgc aacagtctca tgggtcacca
1621 cgtggggagg gctgcctcag caggtttctc agaccacgga gaagtgactt ttgggcccgg
1681 ggccatgccc gggctgtgca aagctggcca gggcctcctg tagggctcct ctgcagcctg
1741 ccctgccttc ccccggatgc tgggccctgc tgctctctca ggaccatccg atcaagctgc
1801 agctgccnac ctcacctaga aacatgcctt tgtgcccacc tcaagatggg cagtgtccct
1861 gttctgaagt gccagtggca gctccagtgg tagaggacca cgcttgaggt ttgggacctg
1921 aagctccagt gtggtgcctt tggcctcttc cctggactgg gttgaccgtc tcgtcttcct
1981 cctgctagtg ctccaccccg cgggacttgg gtgaaccagt ttccaacatt cctccggatg
2041 gtgctcaatt accccaataa ccaggtgggg cacccattct gaagaagggt aggaaggggc
2101 gaccccggga ttgtaacggg ggccagaaa//

- LOCUS: NM_022371 2138 bp mRNA linear PRI 18-NOV-2006
- DEFINITION: Homo sapiens torsin family 3, member A (TOR3A), mRNA.
- ACCESSION: NM_022371
- VERSION: NM_022371.3 GI:115647956

1 agtgcgcact cagcccggcc cgccggtcgc cacagcaaca ccaatcccgg gttcaagccc
61 gcctaggaag tgcggtcccc gcctgaccgc cccgggctta agggagcctg gctaggccgg
121 cagccggatg gtcccgcagc tcggggccgg ccatgcttcg cggtccgtgg cgccagcttt
181 ggctcttttt cctgctgctg ctcccgggcg cgcctgagcc ccgcggcgcc tccaggccgt
241 gggagggaac cgacgagccg ggctcggcct gggcctggcc gggcttccag cgcctgcagg
301 agcagctcag ggcggcgggt gccctctcca agcggtactg gacgctcttc agctgccagg
361 tgtggcccga cgactgtgac gaggacgagg aggcagccac ggggcccctg ggctggcgcc
421 ttcctctgtt gggccagcgg tacctggacc tcctgaccac gtggtactgc agcttcaaag
481 actgctgccc tagaggggat tgcagaatct ccaacaactt tacaggctta gagtgggacc
541 tgaatgtgcg gctgcatggc cagcatttgg tccagcagct ggtcctaaga acagtgaggg
601 gctacttaga gacgccccag ccagaaaagg cccttgctct gtcgttccac ggctggtctg
661 gcacaggcaa gaacttcgtg gcacggatgc tggtggagaa cctgtatcgg gacgggctga
721 tgagtgactg tgtcaggatg ttcatcgcca cgttccactt tcctcacccc aaatatgtgg
781 acctgtacaa ggagcagctg atgagccaga tccgggagac gcagcagctc tgccaccaga
841 ccctgttcat cttcgatgaa gcggagaagc tgcacccagg gctgctggag gtccttgggc
901 cacacttaga acgccgggcc cctgagggcc acagggctga gtctccatgg actatctttc
961 tgtttctcag taatctcagg ggcgatataa tcaatgaggt ggtcctaaag ttgctcaagg
1021 ctggatggtc ccgggaagaa attacgatgg aacacctgga gccccacctc caggcggaga
1081 ttgtggagac catagacaat ggctttggcc acagccgtct tgtgaaggaa aacctgattg
1141 actacttcat ccccttcctg cctttggagt accgtcacgt gaggctgtgt gcacgggatg
1201 ccttcctgag ccaggagctc ctgtataaag aagagacact ggatgaaata gcccagatga
1261 tggtgtatgt ccccaaggag gaacaactct tttcttccca gggctgcaag tctatttccc
1321 agaggattaa ctacttcctg tcatgaaggc tagaggaaga cttcctggaa ctgcctttct
1381 tccactaaca ggaccctggg acctgtagga gcaccccgtt tgggactgtg aggtgtttga
1441 gggtgtggac tggcatccag cagccactaa caaacacaca actggtgtgt aaaaggcagg
1501 ccttacatta gaagccaagc caatcctttt tctttttttt ggaggtccca ccgagataga
1561 taggaacttg gattgctgaa ttcaaaaaca gagcccattc ttaagatcac ttggtgcctt
1621 aaagacacgc attccaaagt ggaatgtggt tgaagaaagt gggccaggtg gttgaagaaa
1681 gccatgtggg agctcagcaa atcccaaggg cttattatga cactccagat ggtctcctta
1741 gcatctcagc tcttctgcaa ggaagagctt gggtgttagg cctcagaggc tgtagggtcc
1801 ttgggttaca gagccgggga gaacgaagtt ctgtgaccca ggggtggaga atacactcta
1861 ggtttgcagg ctggtgggct ttcaaattgg tacttccaga ggaaagccaa gctgcttctg
1921 ttgtgagcga atcagccaag agcctgaggc tgaagggaaa agtacacaga ggaagatatt
1981 ttacaaacca ggtcagtgta ggccaagact tatggtctac agattttggc gggggagggg
2041 ggaccttttc aaagacaata gggggtcttg acatgtttgt tgtatgtaaa gatgataaga
2101 ttaaaatttt tgattttcct aaaaaaaaaa aaaaaaaa//

- LOCUS: NM_004209 2054 bp mRNA linear PRI 17-NOV-2006
- DEFINITION: Homo sapiens synaptogyrin 3 (SYNGR3), mRNA.
- ACCESSION: NM_004209
- VERSION: NM_004209.4 GI:22091456

1 cgggaggcgg cagcggctgc agcgttggta gcatcagcat cagcatcagc ggcagcggca
61 gcggcctcgg gcggggccgg ccggacggac aggcggacag aaggcgccag gggcgcgcgt
121 cccgcccggg ccggccatgg agggcgcctc cttcggcgcg ggccgcgcag gggccgccct

181 ggaccccgtg agctttgcgc ggcggcccca gaccctgctc cgggtcgcgt cctgggtgtt
241 ctccatcgcc gtcttcgggc ccatcgtcaa cgagggctac gtgaacaccg acagcggccc
301 cgagctgcgc tgcgtgttca acgggaacgc gggcgcctgc cgcttcggcg tcgcgctggg
361 cctcggagcc ttcctcgcct gcgccgcctt cctgctgctc gatgtgcgct tccagcaaat
421 cagcagcgtc cgcgaccgcc ggcgcgcggt gttgctggac ctgggcttct caggactctg
481 gtccttcctg tggttcgtgg gcttctgctt cctcaccaat cagtggcagc gcacggcgcc
541 agggccggcc acgacgcagg cgggggacgc ggcgcgggcc gccatcgcct tcagcttctt
601 ctccatcctc agctgggtgg cgctcaccgt gaaggccctg cagcggttcc gcctgggcac
661 cgacatgtca ctcttcgcca ccgaacagct gagcaccggg gcgagccagg cctaccccgg
721 ctatccggtg ggcagcggcg tggagggcac cgagacctac cagagcccgc ccttcaccga
781 gaccctggac accagcccca aagggtacca ggtgcccgcc tactagcggc tggcaggcac
841 agaccagggc tccaaggcca ccccaccaac gcaggcccca gggtctccgg gacctccctt
901 gggtccttcc agctcagtgc cgcggacaga gtaggtggcc gctttgcgcc atccggggcc
961 aagagggggt ggacccgcgt gtctgggctg cccctgccaa gttcccccag tccctcagca
1021 cctggcccca ggactgaggt cctgagaagg ggatagcact gcccaggacg tgtgtcccta
1081 gcctggaatg gactggcctg gggaaggctt tcccctcttg ggccacacct gctcactctg
1141 gggttggggg tccagctgcc ctctacgatc aggtgcaggg gctgcccagg acaaagcggg
1201 ggcaggggaa agacaccacc ctcgccccaa gactggggat cctggccact gttcccatcc
1261 catgtccctg tgggtagtga ctgtctcgtt tctgtcatgg tggtgcgtcc cgtccggagc
1321 cactctccac tttctctcac aggctgctag aacagcccag ccctgtcagt gttgtgatca
1381 tggtccagtc ttcgggtttc acctcctagt actccacaag ctgctcctct ctctgtggcc
1441 ccggcccctg cccaggtgtg ggtggttctg gccaggaagg cacaaggtag ctgtgggcca
1501 agacaccagc cctgtcctag cccttcagta agaccttgcc aggagaggag aaggatgcct
1561 gggtgccagg caagacaagc ccctcagcag gagagaggcc cagaggctcc agctggccac
1621 cgtgccccac aagatggccc ctgtgtggtt ccctttacct tggcttcctg gcccagtccc
1681 tgcctctcca cctgcaccct gcttcctggc ccagtcccag gttggagtcc ctctgcatag
1741 ctgactactc atgcattgct caaagctggc ttttcacatt aagtcaacac caaacgtggt
1801 tgccacattt catcagacag acacctccct ctggagatgc agttgagtga caaccttgtt
1861 acattgtagc ctagaccaat tctgtgtgga tatttaagtg aacatgttta caatttttgt
1921 atatatcact ctctccctct cctgaaagac cagagattgt gtattttcag tgtcccatgt
1981 tccgactgca ccttctttac aataaagact gtaactgagc tgactgtgaa aaaaaaaaaa
2041 aaaaaaaaaa aaaa //

- LOCUS: AK000834 1395 bp mRNA linear PRI 12-SEP-2006
- DEFINITION: Homo sapiens cDNA FLJ20827 fis, clone ADKA03543.
- ACCESSION: AK000834
- VERSION: AK000834.1 GI:7021152
- KEYWORDS: FLI_CDNA; oligo capping; fis (full insert sequence).

1 cctactgggg aaaaacatct gcctgaactc caggcagtac cagcaccttc cttgtgtcca
61 gcgttgcatg gggagtgcca gacagcaagg ccccacttgt gggcaggtct gtgcactgtg
121 acatgccagc ctcttcacag cccaggtcat cctcacctcc accccatgag gtgcaactag
181 atgaaactgc cacttcccag ataaggaaga tgcacaggga gctaagaggc ccggctgaag
241 gtcccacagt gagaaaagtc agtagcccag ctgtgggcct gagccccagg tgctccagtg
301 ggcaggatgg agtcctggtc ttcagcaagg ctcagcttgg tgggggggac acatccccga
361 ccccagacag actttgagtt ctggcagctg ggcactgggg gagctttctg ggaacaagcg
421 tgaaccacac atggtccctg ccctggagtc gcttacatct cccaggagag ggggatacat
481 ccacagactg tgaacacatg ggcctaagag gggccaccca gggctgcaga actgatccat
541 cccatacaaa atcactgaaa agacccccac agaccttgct taaaaaatag tcttcttggc
601 caggtgcagt ggctcacgcc tgtgattcca gcactttgag aggctgaggt gggtggatca
661 cctgagggcg ggagttcaag accagcctgg ccaacatggg aaaaccccgt ctctactaaa
721 aatacaaaaa attagccggg tgtggtggcg gatgcctgta atcccagcta ctcgggaggc
781 tgaggcagga gaatcgcttg aacctaggag gcagagattg ctttgagctg agatcacgcc
841 gctgcactcc agcctgggca ctagagcgag actccgtctc aaaagtctcc tggcggtgtg
901 tccgcatcag agttcaggtc gttcattgtg aatggatggt gcatctttta catctaagtg
961 gactcctttt taagccgtat ttgagaccta gttggctttc ccttcccaag aatgtcctgg
1021 cccatggtcc tggagacctc tgaacctggg tgtccagcta ctagacacgt aaaaaccatt
1081 tctaagtacc ccttgtgggc caggcttata tctgtaatcc caacacttag ggaggctgag
1141 gcaggaggat tacttgagcc caggagtttg agaccagctt gggcaacagg gtgagaccca
1201 gtgtctacaa agagtaagat gattagtgga acatgctggt gtgcacctgt agtcccagct
1261 acttggaggc tgaggtagga agatcacttg agcctgggag gttgaggctg cagtgagtct
1321 tgattgtgcc actgtactcc agcctggaca acacagtgag atcctgtctc aaaaaaaaaa
1381 aaaaaaaaaa aaaaa //

- LOCUS: AL022101 119198 bp DNA linear PRI 16-JAN-2007
- DEFINITION: Human DNA sequence from clone RP5-845024 on chromosome 1p36.1-36.2, complete sequence.
- ACCESSION: AL022101
- VERSION: AL022101.1 GI:317189.5
- KEYWORDS: HTG; FLJ43580; HNRPC; LOC343068; LOC343069; LOC343070; LOC343071; LOC65121; LOC65122; PRAME.

1 gatctgctaa gttcagggat ggctcccatc ctaagcagga tcacacagtc attcttctgc
61 tattttaggg cacaaagtag caagaacgtc ccccgcctcc agaaagtcct ccaggccttt
121 ctctcccatt ccatatgaaa cccaaacagc ccggagatgc cactggcttc caaaactgaa
181 gtactttgaa ggatgttctc catcatggaa tatttctgta aatgttcttt ctccacattt
241 ctgacctcac tatcaatgcc ctgctatgtg tgcaatcgag ttaaactgaa acgtgctcag
301 tgcggcttct acttcacctg ccctcacttt gtgagcctga ggctgagggt gagctcagca
361 ccaaaggtga tcctgagtgt ctctgttgat tgagcatgca caaggcacag cagggactgg
421 taccattcat ccaagatctc agctctccct cacgaggaat ctaaggcatg ttgctatatt
481 cctcattttt aaagtggtga ccctgagact tggctaggga gaggaacctg cccgtgttca
541 ggcagcaaat gatggacaga cccctctggt gaggagctca gaggatcccc taaggagttc
601 aataatctaa atgttgaaaa gaactgattg acagactttc ccttcctgcc caattcagaa
661 ggtccagcac cacccccagg accccagtga gaatcccgca cttgggggca tttctaccat
721 gtcctgtcac ctgttcttct caaggtgctc atctgctgtc aaactcagag catccttcag
781 agcccctctg agaagatgac ctgacccctt ctccactcac agaatctcca ccagaatacc
841 aaagctcccc caggcacttg ctcagggtct ctagaatatt tctgagcttc tgtttccctc
901 ctgcagactg agctgtcaag gcaggtatct ctttatgcat cctggatacc tcagcccaaa
961 tctcctgaac aacagaacat ggccacaatg ctgggagacc ttcaccccag agcagtcatt
1021 gaggaattct cccaaaattc tattgagcta ttttggtgaa catgagaagc cataaagcag
1081 ggagttcctc agctgctgct ctggccctaa agaggcccct gggacaggac tgctggaggg
1141 tgaccctgcc tggcacgcag aaggagacct gagtcctctg gtcttcctat gggtgccagg
1201 ctgagaagag gtcctgggaa atgtctcggt gcaggggcca tcccttccca ggctcccctg
1261 ggtcagcctt agagctgaca aggctgcacc tggaatgcag tcagtctgtt tctgtccaag
1321 tcaggtctct tcttagcctg agctgttcac cctctgtgca tttctaagag tcaaaatctc
1381 tgttctggcc ctgttgccct tctctggtcc tcaaccgggt ctgattccag gtcagattca
1441 tgcacatcgt gtgctaaagt aggggcttcc tctctttctt cctccaatca agtctgagga
1501 caaggtcacc ctgaaagcag acacttgatg ctgagggagg tgctcagagc ctggggtggg
1561 aggaggtctg caccctcact ccaggctgga cagagccaga gggactgaaa gtaggaatgg
1621 ggccaagaag gggagtgatc agaggagctg atggggctga aataaagggg aaggagggag
1681 gtggcagcag agcaggcagc caggttctct ggctctcata gagctgtgtc tctgaaaggc
1741 agggacttgt gtctgagacc gtggggcact ggggcttgga cttgggtctg tgagttccca
1801 agacacttta tgtgccctgg tctgcatgag gttttagtgg ctgccccatc ctgccccagg
1861 ggcacagagt caggagagtg gggagtccca ggagaggaag agcgggtggc gagtgtccat
1921 cctggatcca aagacaagca gcaggtcaga aggaagcagc tgtggcttga ggccaaggaa
1981 aggattctct ttggaaatcg tgcagctcca tcttttcact ggtcagagcc ttatgggacg
2041 gtgaggactg ggcttttcag gagggaatga aaaagaatgg ggtctggctc agagtccctg
2101 acatccaagt tattccaaac ctcctctgac aagcaaaggg aaaatcatgg aggatggaag
2161 tcaaaccaag gctcacatgg ctgcccagag ctttccagtg gatccagaga gggcacaatg
2221 atcctcagtc agaaaagtcc tccaatgtcc agcactgagg cccaacaaga gtgcctggag
2281 gggactctct gtgacatgtc tgaagagaag attctggcac atgtgactcc tcccacaccc
2341 tctgcctccc cctgactcct ctccccttga ttcattcaaa gaccttcact gagcaccagg
2401 gattgcttcc tgcaattccg ggtgctacca gggaagatga ataaaatgca aggaagctgg
2461 aaaatgaact cagcagagga caccaggctc agcactgcaa tcccggtcag aaaaataact
2521 ctgcaggctg cgcggtgcag agcgatcata ataacccatg gggataatca caggtgtccc
2581 aaatccttta atcacttttt gttggaatat gaagaggtct ctcactgctg aatataaata
2641 tacagggaag tgaaaaacat tagtaaaacg aacatttact aagtgcaatg tgactcaaag
2701 cactgagaag ccaagggttt tatttctttg aaacaacttg ccaaggcagt ttgaacagtg
2761 atcatcttcc tcctgggctc agcaccgcag aggcagctgg tgtcttctcc acctgggaga
2821 gttgtcctgc ttttttttaa gttgggatag gccagtgagt cgggaagttt gttcatcttg
2881 ggcttggggt acaggaggag ttggtggggt gcctgtggcc actccactgc cctctgggat
2941 taggaggaga cagtggggtc aggactcacc ccctggcctg tgcttcaggt gatccctctc
3001 tgtcctgtgg atgtggggtt gggagcaggt ttggggtcct cgcctatccc cattggctct
3061 tcttgggaag atgaatttat ggggcacctg caggtggcca cgtgtgggaa ggaatctcag
3121 aacttacatg gatccatgca aatgaggctt ccttcaggca gacagaggaa ctctgagcct
3181 ccctgatgct gcaggcacct gggtttgggg accctcttgg agacaaatgc atggagcgtc

3241 ccagcaagtt tccctgtctc ccagctcctc cctgggcttc tgcatccggg agtcagggcc
3301 ggatcaagag aagccctgcg gggagtggga aggacatagg attctcaggg tctcaagttc
3361 agcttttaac attatcctca aaggtggggt ttttcccaga ggcctccttt ccacagatcc
3421 catgccttct tgctggactc acgggaaact tgccctgcta gagacatggg atatttact
3481 tttctgtgcc atggagccat gctggagagc tgtgacttcc tagctgacca cacacacaca
3541 taaacatgta aacaccatga ggtcattgta gggatgccca ctgggcctgc ggttctccca
3601 tagcacccag ttcataaaag cctccctttc actcacctgg ggcctggagt cattggcctc
3661 ctcctgtctc attgatccag catttggcct tgactggcca gtgactcaga ccccagcaag
3721 aaggacaaaa tgactagttc acgtgcttta ggggaaacac agagagaatg agaagaccag
3781 tgtctgaact ggcaggttgt gaattggctc agggagatga gactggagag gtgcagccag
3841 gggcaagggt gactcagggg tcattggcag tttgggatta gggttgtgag gccacttgaa
3901 gcctatcttc cacatccccg aggtggctga gaggaccgtg ttccctgagg ggacaaccag
3961 agggcaggga cacgcttcag aagattcttg cactgtccag acaggaggtc ctggtgtcca
4021 cttgagtggc catggacagc atagggacgt cctggaggca gagtcagcag gacttgctct
4081 taattcttcc tggggtggat gtgaagcttg tgtcctggca gagggagtgg ttggcacagg
4141 gctgagttat cccctgtggc ctcaactgtt ttcctgatta tgcctgttgt ctttgaatgt
4201 caacaaaagt agccaacatt tatggagggt ttattatgcc ccatgctttg ggctcagcat
4261 ttttacctga agattgagat tatccttcta cacatttgat ggagaaagag acacattcaa
4321 agagagaggg agaaattttc aaggccagac agcctgtaag tggtgggact ggaatgacgg
4381 aatgtctgtt tgtcaacatc tttggcggtg acatgatact gtcccagtcc ctgcaatgtg
4441 ctgctcattt tcatcctttc aaataaaact ccacagtcag aggcttgtgc aagagttggg
4501 gtccagagca tcaggtctaa tgtttgctat gtttatattc actgccaaat ctgttattcc
4561 aaagaaattt tactagtgaa atagaaatac gttgtctgac agcattaccc cctgtgatat
4621 ggtttggatc gaaccattgc tctccagcct ggcaacagag tgagactcca tctccaaaaa
4681 aaaagcaatt aattataaca acacgtctct tcactctcca aagtgtctgg gactggacaa
4741 ttaattgtca ggccctcttc tgtagcacca tacactagag catatacgtg gattaaaata
4801 aatacacaca caaaatgcaa gtatatattc ttttttcatt attattatac tttaagtttt
4861 agggttcatg tgcacaacgt gcaggtttgt tacatatgta tacatgttcc atgttggtgt
4921 gctgcaccca ttaactcata atttagcatt aggtatatct cctattgcta tccctccccc
4981 ctccccccac cccacaacag tccccggtgt gtgatgttcc ccttcgtgtg tccatgtgtt
5041 cttattgttc aattctcacc tacgagtgag aacacctatg agttttgttc tagggttttt
5101 atggtttgag gtctaacatg taagtcctta atccaccttg aattaatttt tctataaggt
5161 gtaaggaagg gatccagttt cagctttcta cttatggcta gccagttttc ccagcaccat
5221 ttattaaata gggaattgtt tccccatttc ttgtttttgt caggtttgtc aaagatcaga
5281 tagttgtaga tatgcggcat tatttctgag ggctctgttc tgttccgttg gtctatatct
5341 ctgttttggt accagtacca tgctgttttg gttactgtag ccttgtagta tagtttgaag
5401 tcaggtagca tgatgcctcc agctttattc ttttggctta ggattgactt ggcaatgcgg
5461 gctctttttt ggttctatat gaactttaaa gtagtttttt ccaattctgt gaagaaagtc
5521 attggtagct tgatggggat gccattgaat ctataaatta ccttgggcag catggccatt
5581 ttcaccatat tggttcttcc tacccatgag catggaatat tcttccattt gtttgtatcc
5641 tcttttattt cattgagcag tggtttgtag ttctccttga agaggtcctt cacatccctt
5701 gtaagttgga ttcctaggta ttttattctc tttgaagcaa ttgtgaatgg gagttcactc
5761 atgatttgac tctccgtttg tctgttagtg gtgtataaga atgcttgtga tttttgcaca
5821 ttgattttgt atcctgagac tttgctgaag ttgcttatca gcttaaggag atttgggct
5881 gagatgatgg ggttttctag atatacaatc atgtcatctg caaacaggga caatttgact
5941 tcctcttttc ctaattgaat tccctttatt tccttctcct gcctgattgc tctggccaga
6001 acttccaaca ctatgttgaa taggagtggt gagagagggc atccctgttt tgtgccagtt
6061 ttcaaaggga atgcttccag tttttgtcca ttcagtatga tattggctgt gggtttgtca
6121 tagatagctc ttattatttt gagatgcgtc ccatcaatac ctaatttatt gagagttttt
6181 agcatgaagg ttgttgaatt ttgtcaaaag ccttttctgc atctattgag ataatcatgc
6241 ggtttttgtc tttggttctg tttatatgct ggattatgtt tattgatttt cgtatgttga
6301 accagccttg catcccaggg atgaagccca cttgatcatg gtggataagc tttttgatgt
6361 gttgctggat tcagtttgcc agtattttat tgaggatttt tgcatcaatg ttcatcaagg
6421 atattggtct aaaattctct ttttttgttg tgtctctgcc aggctttggt atcaggatga
6481 tgctggcctc ataaaatgag ttatggagga ttccctcttt ttctattaag tggaatagtt
6541 tcagaaggaa cggtaccagc tcctccttat acctctggta gaattcggct gtgaatccaa
6601 ctggtcctgg actttttttg gttggtaagc tattaattat ttcctcaatt tcagagcctg
6661 ttattggtct gttcagagat tcaacttctt cctggtttag tcttgggagg gtgtgtatgt
6721 cgaggaattt atccatttct tctagatttt cttgtttatt tgcgtagagg tgtttatagt
6781 attctctgat ggtagcttgt atttctgtgg gatcagtggt gatatcccct ttgtcatttt
6841 ttattgcatc tatttgattc ctctctcttt tcttctttat tagtcttact agcagtctat
6901 taattttgtt gatcttttca aaaaaccagt tcctggattc attgattttt tgaaggttt
6961 tttgtgtctc tatttccttc agttctgctc tgatcttagt tatttcttgc cttctgctag

7021 cttttgaatg tgtttgctct tgcttctcta cttcttttaa ttgtgatgtt agggtgtcaa
7081 ttttagatct ttcctgcttt ctcttgtggg catttagtgc tataaatttc cctctacaca
7141 ctgctttgaa tgtgttccag agattctggt atgttgtgtc tttgttctca ttggtttcaa
7201 agaacatctt tatttctgcc ttcatttcgt tatgtaccca gtagtcactc aggagcagat
7261 tgttcagttt ccatgtagtt gagcagtttt gagtgagttt cttaatcctg agttctagtt
7321 tgattgcact gtgttctgag agacagtttg ttataatttc tgttccttta catttgctga
7381 ggagtgcttt actcccaact atgtggtcaa ttttggaata ggtgtggcgt ggtgctgaaa
7441 agaatgtgca ttctgttgat ttggggtgga gagttctgta gatgtctatt aggtctgctt
7501 ggtgctgaac tgagttcaat tcctggatat ccttgttaac tttctgtctc attgatctgt
7561 ctaatgttga cagtggggtg ttaaagtctc ccattattat tgtgtgggag tctaagtgtc
7621 tttgtaggtc actaagtact tgctttatga atctgggtgg ggcaacccgc tcggtcccc
7681 ttccacagtg tggaggcttt gttctttcgc tctttgcaat aaatcttgct actgctcaaa
7741 aaaaaaaaaa aagtatgaat ctgggtgctc ctgtattggg tacatatata tttaggatag
7801 ttagctcttc ttgttgaatg gatcccttta ccattatgta atggccttct ttgtctcttt
7861 tgatctttgt tggtttaaag tctgttttat cagagactag gattgcatcc cctgcctttt
7921 tttgttttcc atttgcttgg tagatcttcc tccatccctt tattttgagc ctatgtgtgt
7981 ctctgcacgt gagatgggtt tcctgaatac agcacactga tgggtcttga ctctttatcc
8041 aatttgccag tctgtgtctt ttaattggag catttagccc atttatattt aaggttagta
8101 ttgttatgtg tgaatttgtt cctgtcatta tgatgttagc tggttatttt gctcattggt
8161 tgatgcagtt tcttcctagc cttgatggtc tttacaatgt ggcatgtttt tgcagtggct
8221 ggtactggtt gttcctttcc acgtttagtg cttccttcag gagctctttt agggcaggcc
8281 tggtggtgac aaaaatctca gcatttgctt gtctgtaaag tattttattt ctccttcact
8341 tatgaagctt agtttggctg gatatgaaat tctgggttga aaatattttt ctttaagaat
8401 gttgaatatt ggcccccact ctctttctgg cttgtagagt ttctgccaag agatcagctg
8461 atagtctgat gggcttccct ttgtgggtaa cccgaccttt ctctctggtt gcccttaaca
8521 tttttccttt catttcaact ttggcgaatc tgacaattat gtgtcttgga gttgctcttc
8581 tcgaggagta tctttgtggc attctctgta tttcctgaat ttgaatgttg gcctgccttg
8641 ctagattggg gaagttctcc tggataatat cctgcagagt gttttccagc ttggttccat
8701 tctccccatc actttctggt acaccagtca gacatagatt tggtctttcc acataggacc
8761 atatttcttg gaggctttgt ttttttcttt ttattctttt ttctttaaac ttctcttcac
8821 acttcatttc attcatttca tcttccatcg ctgataccct ttcttccagt tgattgcata
8881 tgttaatgag gcttgtgcat tcgtcatata attctcatgc tatggttttc agctccatca
8941 ggtcctttaa gaacttctct tcattggtta ttctagttat ccatttgtct aatttttcc
9001 caaagtttct aacttctttg ccattggttc caacttcctc ctttatctcg gagtagttcg
9061 atcttctgaa gtcttcttct ctcaaatcat caaagtcatt ctccatccag ctttgttcca
9121 ttgctggtga ggagctgcgt tcctttggag gaggagaggc actctgattt ttagagtttc
9181 cagtttttct gctctgtttt ttgcccatct ctgtggtttt atctaccttt ggtctttgat
9241 gatggtgatg tacagatggg tttttggtgt ggatgtcctt tctgtttgtt agtttccttt
9301 ctaacagtca ggactctcat ctgcagttct gttggagttt gctggaggtc cactccagac
9361 cctgtttgcc tgggtatcag cagcagaggc tgcagaacag tggatattgg tgaaccgcaa
9421 atgctgctgt ctgatctttc gtctggaagt tttgtctcag aggagtaccc agctgtgtga
9481 ggtgtcagtc tgcccctact tggtgttgcc tcccagttag gctactcggg ggtcagggac
9541 ccacttgagg agggagtctg cccgttctca gatctcaagc tgcatgctgg gagaacgact
9601 actctcttca aagctgtcag acagggacat ttaagtctgc agaggttatt gttttctttt
9661 gtttgtctgt gccctgcccc cagaggtgga gtctacagag gcaggcaggc ctccttgagc
9721 tgtgatgggc tccacccagt tccagcttct gggctgcttt gtttacctac tcaagtctcg
9781 gcaatggtgg gggcccctcc cccagccttg ctgccgcctt gcagtttgat ctcaggctgc
9841 tgtgctagca atgagcaagg ctccgtgggc acaggaccct ccaagccagg tgtgggatat
9901 aatctcctgg tgtgcagttg ttacgcccat tagaaaagcg cagtattagg atgggagtga
9961 cctgattttc caggtgccat ctgtcaccca tttctttgac taggaaaggg aattccctga
10021 ccccttgtgc ttcccaggtg aggcgatgcc tcaccctgct tcagctcacg cacggtgcac
10081 tgcacccact gtcctgcacc cactgtctgg cactccccag tgagttgaac ctggtacctc
10141 agttggaaat gtagaaatca cccatcttct gcatcactta tgctgggagc tgtagactgg
10201 agctgttcct attcagccat cttaagtata tattctaaat actttctata tacttatatt
10261 ctaagaggtc acatgcaaat tcaaggctag gtcaaagagt agagtggcta tctatggaaa
10321 ggggagtgga agtgaatcat ggtaataaaa attaagtata gatatagata ggaatagata
10381 gacatacaca catatagctg caagaaaggg gaatgtcatg gaccaatgat gtcagtgagc
10441 catgtaaaaa ggctacaatt cttgtgattg tgtgtctgtt ttcaggatgg gttgtagctt
10501 acctttttag aaaggctgat gccacagcca tagtgaataa atggttataa aatgtgtttc
10561 ctttctgggg catctctgga gaaatctcca gtggtaggag aactccgttt actgggcagg
10621 tgatcacaca gataagattt ttcagatcca atggcactac cattaacttc attatccttg
10681 gtattctaca aaggtcgagt gaagaaatgg tatcttgaaa ctaaaattag ctaaactaac
10741 aaaggagact gggttaattt tttttttttt tttttttttg agacagagtc tctgttaccc

10801 aggctggagt tcagtggtgc tatctcagct cactgcaacc tctgcctcct gggttcaagt
10861 gattgtcatg ccttagcctc ccaagtagct gggattacag gcataccacc acacccagct
10921 aatttttgta tttttagtag ataacggggt ttcaacatgt tgcccagatt gctcaactcc
10981 tggcctcaag tgatccacca gcctcggcct cccaaagtgc tgagattaca ggtgtgagcc
11041 atcatgtcca gcaagactgg attactttaa tgaaattttt accaccccct atgggaaaac
11101 acagccagat cccccataag gtattttttt ttcaccaact gcaatcagaa acactgataa
11161 ttaagtattt actggagaac ctatgccttt gataatagaa cattatgtat cccctgcact
11221 ttttagctct gatcatgtaa ccagaggatc aactccaaca gattagtcat tgcttaagtt
11281 gttacaggtg atgacgcaaa gcccaaattg ctcaggcatg tccgatggga aaaaggttta
11341 acctcttaac tattaacaca gccaggcgga ctgtttgaat tggcatcatc tgaaaccagt
11401 tggagagatg atgcaagctt gctccaccat ccccagattg ggagacaggg tttagaactt
11461 gtctcctatc tgcttgtcag ttaactcttt tttattttta tttttatttt tttctttgag
11521 acagagtctc actctgttgc ccaggctgga gtgcagtggc atgatctcag ctcactgcaa
11581 cattggcctt ccaggttcaa gtgattctcc tgcctcagcc tccccagtag ctgggattac
11641 aggcatgcac caccatgccc agctaatttt tgtatttttg tatatttatt tatttattta
11701 tatattgatc attcttgggt gtttctcaca gagggggatt tggcagggtc ataggacaat
11761 actggaggga aggtcagcag ataaacaagt gaacaaaggt ctctggtttt cctaggcaga
11821 ggaccctgcg gccttccgca gtgtttgtgt ccctgggtac ttgagattag ggagtggtga
11881 tgactcttaa cgagcatgct gccttcaagc atctgtttaa caaagcacat cttgcaccac
11941 ccttaatcca tttaaccctg agtggacaca gcacatgttt cagagagcac agggttgggg
12001 gtaaggtcat agatcaacag gatcccaagg cagaagaagt tttcttagta cagaacaaaa
12061 tgaaaagtct cccatgtcta cttctttcta cacagacatg gcaaccatcc gatttctcaa
12121 tcttttcccc acctttcccc cttttctatt ccacaaaact gccattgtca tcatggccca
12181 ttctcaatga gctgttgggt acacctccca gacggggtga tggccgggca gaggggctcc
12241 tcacttccca gtagaggcgg ccgggcagag gcgcccctca cctcccggac tgggcggctg
12301 gccgggcggg gggctgaccc cccacctccc tcccggacgg ggcagctggc ctggcggggg
12361 ctgaccccca cctccctccc ggacggggtg gctgccgggt ggaggggctc ctcacttctc
12421 agacggggcg gctgccaggt ggagggtctc ctcacctccc agaaggggcg gcggggcaga
12481 agcgctcccc acatctcaga cgatgggcgg ccgagcagag acgctcctca cttcccagac
12541 ggggtggcgg ccgggcagaa gctgcaatct cggcaccttg ggaggccaag gcaggcggct
12601 gggaggtgga ggttgtagcg agctgagatc acgccactgc actccagcct gggcaacatt
12661 gagcactgag tgaacgagac tacgtctgta atcccggcac ctcgggaggc cgaggctggc
12721 ggatcactcg tggttaggag ctggagacca gcccggccaa cacagcgaaa ccccgtctcc
12781 accaaaaaaa tacgaggacc agtcaggcgt ggtggcgcgc gcctgcagtc gcaggcactg
12841 ggcaggctga ggcaggagaa tcaggcaggg aggttgcagt gagccgagat ggcagcagta
12901 cagtccagct tcggctcggc atcagaggga gaccgtggaa agagagggag agggagaccg
12961 tggggagagg gagaccgtgg ggagagggag accgtgggga gagggagagg gagagggaga
13021 gggagagcaa tttttgtatt tttaatagag agggagtttc accatattgg tcaggctgat
13081 ctggagctcc tgaccttgtg atccgcccac cttggcctcc taaagtgctg ggattacagg
13141 tataagccac tgtgcccagc ccagttaact gttaataaat ttttttttcc caaaatggag
13201 tcttgctctg tcacccaggc tggagtgcag aggtgcaatc tcgactcact gcaacctctg
13261 cctcacagga tcaagtgatt cttctgcctc agcctcctaa gtagctggga ttacagacat
13321 gggtcatcac acccagctaa tttgtgtatt tttcatagag acagggtttc accatattgg
13381 ccaggctgga ttcgaacccc tgaccttgtg atccgcctac cttggcctcc taatgtgctg
13441 caaatatagg tgtcagccac catgcccagc cttttttgct tttctatgca cttaggagag
13501 tgagcccatc gttcagtaac aatatgactc agtactgcaa gacctttcaa agcctatttc
13561 cagttggtga aggagggttt cgatgatcac tggaccttca tgccctacca tttggagact
13621 gcatctttta aaatgacacc aagggaaatc tgcccatgag cagcattgga tgggaccata
13681 ccaggtgact taaaattaag gataaccaag gaacaaagtc tttcttagaa gcagacatca
13741 tcacatggta gacagctttt ttaagaaaat gggacaaaac tccatttgat ctccttccat
13801 tgactgagac ttggttttgt tttgtattaa cacaaagtga tcaagcctac attttatttt
13861 gttacgtact ttcaccagtc aaagcaaaca ctttctaagt tctcctattc aaaatttagc
13921 cattctcact aaccaaagca attactggct atggagtcat ttagatgaat gggaaggatc
13981 acaactaata gtagaacctg ctctcacaca cggttggtta atattgataa ttaaatgact
14041 tggcactgag cagaagctat agatgcaaat gggtggccta tgactattgt tgatttcatt
14101 acttgtaact tatctccatg cataggaaac attagtgtaa ccgggtctaa tctaggtagt
14161 gtcccagact ccccctagaa tcaaactctt tcatttgaca cacattatga agactgaaat
14221 gctataagta ttgacataga cacagaatca gaacatgacc atgttatcct ctgccatata
14281 atcagagaac ttactgaaac tagacatttg ttcattggaa actcgaggca aatagaatgc
14341 atctatagct ctactatatg aaataaacaa tagtttcatt tattggatgc atccatactc
14401 agcacatatt tggagaagac cctattcatt cttcaatgga gatgacatgc aaggattatt
14461 taataaaatt cataaaaata ttttttcatc ccaccccagt tcaaactgtc accatgcaac
14521 ctggtgtcag tggaaggtaa ggcgcttaag gcagaaataa ttaaataaat cttcattgga

14581 agctaaatgt gagaatcaat gtggaagaca cagaccaaca aagtgggtgt gttccaaagt
14641 ctgttacaag ttggaatgct tttgtgagaa aagttaaaag aagggaatga gactcctcct
14701 atcagttttt tttttttaat tttcttttgt tttactgacc tggcaaggct caaatagagt
14761 tgagtttttg ttttgttttt gtttttccca ttggaaggta caatacagag gttacaatca
14821 ttgactttag ctgacaacat aacaagttaa acattttcct tgcaagacaa ccagtgaaac
14881 ttcatgatca gaatcaaatc agtgtccttc tcactgtaag tgggtgaagc ttcatcaata
14941 attgcagagt ttgaggcact catgaactca agatcagatt ctttactcag ggacagaatg
15001 taagccaatc ataagacctt ccacaggtgg ttaatttgga ctcctgaaaa atgtgacctg
15061 taagttttca ctggcaatat gcaggtgcac atatgacaaa taataaccag gacctttata
15121 tcacccccaa ctggtgggga atgggatcct tttgaccctt tctctccata ataccaggct
15181 actcatcttg tgtggcaata aaatatatgg tctacttcac agagaaagag attcttttaa
15241 aaaaaaaaag gatttttatt atgaaatgag caaagcaatg ggaatagatg tgagattatt
15301 cagggaggta aaggaagaca aaggttttga aaggaaaaat caggagaatt acatacactg
15361 ttttggaaga ctcattcttg gtcacaagta tcaacaccaa gggggcctca gtgcaatgtt
15421 ggaaagattc ctcctccacg ccctcaataa cccccaacat gtttaccaag tcttggttca
15481 ctccccggat cccattaaaa cacacagctc aaccctgacc agcctccacc ttcacttccc
15541 tttgtaattt tgacatgact ttttttacag gaccatcagg ttcctatgcc tgcagcacag
15601 tagcatacta atattctgag acagcagggt ttgcagcaga gagtttaacg atcacagggt
15661 ggcaaaatga aaagctggga ggagaccctc aaattcatct ccccaagaag tactgagagt
15721 tttcagagga tcatggatag caagaggctg gaaagttggt gcagtttggt ggcagtaaga
15781 ggtatgaagt catcaggatg tcaaaactgc attccttggt gagttggtgc cttgcagggc
15841 ccttcagatc acctgccatc agtagcttca ctgacatgca gaacctgaaa gaatatgtca
15901 aatgaaaaag ttaatgtttt acaaggccta aattgttgtc tgcagggcag ttaagggcaa
15961 ctgtaatcta aggtctacat gattttggga cagcaggctg ccagcaacca tgaggaagca
16021 ggtcagagag caagctgacc tcatgatgaa tgctgaatgc actgcaagct tggtttatgt
16081 ttgtttctcc ccctcccttc ttcactgatt aaatttataa agtttataag tatggttgca
16141 atttcttcca gagtagcctt aacctaagcc ctgagaccac tcaggtcctc agtggcacct
16201 ctcttccacc agcaggagtg aaaaaattgc taccttaggt gatataaaac ccacaagacc
16261 attgaataca tggagatttt tattttgatt ttgtagggac gactcctctg tttttataaa
16321 gctattttaa ctataaaaca tttttgtgat tttgatgtgg ccaaagatct cccaacaata
16381 ctactttcag attttagttt tctgtctaat atctgggaaa gattagaccc ttccctgctt
16441 cagactcagg actatgcagg tcacatatta gtgaaattcc atcagtgttt gtgaagttca
16501 tgaatgaatg aatttttttt ttccgacaaa ttctccctct gtcacccaga gtggagtgca
16561 atggtgcaat cttggttcac tgcaaccatt gcctcttggg ttcaagcgat tctcccacct
16621 cagcctcctg agtagctgga ttacaggcat gtgccatcgt ccctgtctaa ttcttgtatt
16681 tttgtagaga cggggtttca ccttgttggc ctggctcgtc ttgaatgtct gaactcaggt
16741 gacccaccca acttgtcctc ccaaagtgct gggattacag gagtgagcca ccttgcccgg
16801 tcttgaatga atgcattctt gattcccacc ctatccctaa cactgtcaat ttcttgattc
16861 atgaacttaa tatggatatc tgatatgaat ggatatctga ttcaatccat taatctgggg
16921 agagccaaaa ccccaatcag gattaactgg gtggagcttc agaaatgcaa tcagatatca
16981 cttgttgatt ggaagctagc agtggatacg tggaggggtg tgggtgggag ttgtgattag
17041 aaaggtcaat aaaagcttct aaagacccac agaagagacc caaagtcttc aagcctggag
17101 ttcctgctta gttcttcctg aggtctgagc accctccaaa ctgagtccag atctggtaag
17161 tcactaatct ctctgtaagg acactcccaa ctgacctaca gtcagctggt ctgggatggt
17221 gacagtgcag cctaagatgg catagagtta tatcctgttt tgtttttttt ctcatatgaa
17281 caatttgaag ctttgcattt tttcctttaa atgcagtttt gtctttattt caaaaaattg
17341 gattgtgctt tggtttatgt catttcaaaa ttcttgaagg gagcagtgac tcatgccttt
17401 aaccccaaca ctttgggaga ccaaggcggg aggatcattt cagcccaggg gtctgagacc
17461 aacctggaca acacggcaaa aacccatctc tataaaatat tctttattga aggggggatg
17521 gagtctcgct ctgtttccaa ggctggagtg cagtggcacg atctcaaatc attgcaactt
17581 ctgcctccca ggctcaagca attctcatgc ctcagcctcc tgagtatctg gtattacatc
17641 caactgccaa cttgcctggc taatttttgt atttttagta gaggtggggt ttcaccatgc
17701 tggccaagtt ggtctcaaac tcctgacctc aagtgatcca cctgccttgg cctcccaaag
17761 tgctggaatt acagccctga gccactggtg cttggcctct acaaaatata catatttttt
17821 aattagccgg gcatggtagc atgcatctgt cttcccaact gtatgggttg ctgacatggg
17881 agaaacaatt gagcccagaa gattgaggct gcagtgagcc atgctcatac cactgctgta
17941 ctccagcctg ggcaacattg cgaggcccta ttacagaaaa aaaatcttaa ccaaagagga
18001 tctttgacct taattttaaa ccaatcacat cctcattgta actcttccac ccaaacggag
18061 acatgggtgt ggaggtgcat gcctgtaatc ccagctacat ggaaggctga agcataagta
18121 tcacttgaac ctgggaggca gaggttacag tgagctgaga tggcaccact gtactccagc
18181 ctgggtgatg aagtgagact cagctacccc gacacgaaaa aaattaaatt ataccaccca
18241 ggtgatcatt ggattcatga agatttctac tgtgttttct tagggactgt catgtctatc
18301 tttgtaaaac tgttttaact ctgaaatatt ttgataaatt tgatgtggcc aaggatctct

18361 caacaaagat actttcgagt ttttttcttt ctgtctaatg tcaggaagag attcaactct
18421 tccctatctc acactcagaa ctacaaaggt cacatattac taaaattcca tgtttgtgga
18481 gtaaatcagt gaatgagtcc tggactttca ccatatccct aaatatttca ctttcatgga
18541 tgaatatcta atttgatagt taatctggaa gaaagacaaa aatccaatca tgattaactg
18601 gatggagctt aagaagtcta atccaatgta gttctctctc tctctctctc tttttgaat
18661 ctagccaatt tcccaggctg gattgtagtg gtataatctc agctaactgc aacctatgcc
18721 tcctgggttc aagcgatcct cctgcttcag cctccctagt agcttggact ataggcgcag
18781 accactgcac ctggctaatt tttgtaattt tagtagaggt agtgttttac catgttggcc
18841 aggatggtct caaactcctg acctcagata atccaatgcc tctgcctccc aaagtgctgg
18901 gattacaggt gtgagtcact gcgcacagcc aaagtggttc attttgaaca tgcgtaagag
18961 gtgtgtattg gaaacacctg tgtcttgcga atgatgcata acactgtcac acagctttca
19021 aagcttcttg gtgaaatttt caataatgag tcccggaaga ggattacgcc tgtaatccca
19081 gtactttggg aggccaaggc gggtggaatg tttgagtcta ggagttcaag accagcctgg
19141 acaacatagt gaaacccact gtctttacaa aaagtcaaaa aataaaagat tagctgggca
19201 tgagatccga gcttcagaga tcctcggtaa catttcccag tgctatgagt ttattgcaac
19261 agtggctaat aattcatgga ctaggaagga tcttgcctgc tctttagagg ttgggacaca
19321 ctcttcttgg taccagaagg gcagaaccat gcctctgtag ccacttattg cagaacggaa
19381 ttggagtaaa ctgagggctc tttcacacgt gctagagaaa tgactttggc cctaggagaa
19441 gtggggcttg ctggggaatg gcccgagaaa cttgcctttt cacttgattg tcctctagag
19501 tttttcctcg gagatttgtc agaatgagcc tccaggcccc atccagactg ctggagctgg
19561 cagggcagag cctgctgagg aaccagttct tgaccatctt caccctggat gagctgccca
19621 gggaggtctt ccctctgatg ttcatggagg ccttcagcat gagacgtttt gaggccctga
19681 agctgatggt gcaggcctgg cccttcctca gcctccctct gggatccctg atgaagacac
19741 ttcatctgga gaccttgcaa gctgtcctga ggggacttga tacactggtg gcccagaagg
19801 ttcgccccag gtgaggtgac tcaggtggct ttcggggaag ggtccaggca tccagggaag
19861 ggacagctgg ctcaggagga gtggtggggt tggggagcta gggtggctca gaggcttctg
19921 acggtgccca tgagaggcct tggccattgc ccagatcctc tggaaaaggt ctgctcacca
19981 tacagggtcc actgaggaaa caggagcttg tttcctccca gcagaaagta aaggtactag
20041 aagtgggtac caggcagaat ccaagaggga gcaggatgga gaagagacag aaggaggagc
20101 actgaggaca ggagcagctg actgatgtcc tggatgtgga gtgaaagctc aggtcagggg
20161 tgggtccttg cctacattct gagcttttcc cctatgttac tcacaggagg tggaaacttc
20221 aagtgctgga tttgcgggat gttgatgaga atttctggac catatggtct ggagccaggg
20281 tcctctcctg ctccccagag gccatgagta agaggcagac agtggaggac tgtccaagga
20341 tgggagagca ccagcccttg aaggtgttca tagacctctg cctaaaggaa agtacactgg
20401 atgaatgcct gagctacctt tttgggtgga tccactacag aagaggccta gtgcacctgt
20461 gttgtagtaa ggtgcagaat tactcaatgc ccacttcaag tttcagaaat ctattggaaa
20521 ggatataccc agacagtatc caggagttgg aagtctggaa aaagtgctct ctcaataaaa
20581 cgggaaagtt tgccccttac ctgagccaga tgagcaatct tcgtgaactc tttttagcct
20641 tcggttatga gcgtgagttg tacgtgagcg tccagtggcc gtgcattcct gacttggact
20701 ctccattcct ctgcctgtac tacccccaga tgctttatat aaaaaagatc agtaatatca
20761 aagagcacct ggagcacctg ctcaggtaag aaatgatggt gagctttctc tgcagaccat
20821 accacagact tatgttcttt ttcacagtaa atgttagtgg gcatctactg tgtgccagcc
20881 accggtgatg tcatagggaa tgggacgcta gaatgtcaac tcattatgct cttcagtgct
20941 ctatatcctg aaatgggtat cacaagacca ctcaaataag ggcagaggga tggcctgggg
21001 tagatgccac agagagaggt gtgtagggag ccggttagtt gagggttcag atctagtgag
21061 ggtgcatttg tgaactcctt gtgaggaaca gtgtataaag ttaatatgat gaaaacacat
21121 tcttcataca gaggatggta tgaaagaagg gaaggtgtgg ccggttgtgg tgtctcatgc
21181 ctgtaatccc agcacttcgg gaggccaagg cagggagatc atgaggtcag gaatttgaga
21241 ccagtctggc caacacagtg aatccccgtc tctactaaaa atacaaaaaa aaatgtcacc
21301 gggcatgcag acaggcacct gtaatcccag ctgcttggga gactgaggca agggaagtgg
21361 aggctgcagt gagctgagtc ggtgccacta cactccagcc taggttacaa tgtgagactg
21421 tctcaaaaaa aaaaaaagag aaagtacatc aaacctgtgc attccacagt agcagctctg
21481 tcttcagcag cttagcaaac tgctctaatt ccctgtctgt aaaacgttgt tttgaactcc
21541 aggaaagata attgatatca gaagtgcatg cttctgggat ggagggtgag ggactaggtg
21601 tgagagtggt accaatcaca caggcaaggg tgaaaggact gagcctaaaa tggagctgcc
21661 cctgaatgat ctgagtcttc atcaggcagc accttgcatg cagaccatca tctgatgatg
21721 ggaacaaact tgtgtttggg tgaaacaggc ttccccattg cagttactat aacacctgtg
21781 tggtagtaag gtgcagaatt actcaatgcc cacttcaagt ttaccattga gatgatttcc
21841 cacccccctc ctctaactgg caccattgcc cataactaac ttcttgctct ccccaggtac
21901 ctcaagaacc ccttgggggc ctttatattc agtgatgctt acctaactga tcgggacatg
21961 gagtgtctgt ctcagtaccc aagcctcagt cagctaaagg agctgcgtct gattcatatc
22021 ctaatgtgga ccacccatct tgagcccctt ggagttctgc tggagaaagt tgctgctact
22081 ctcaagaccc tcgtcttaaa ggactgtcgg atccaggacc cccaactcag ggtcctcctg

22141 cctgccctga gccactgttc ccagctcacc accttcaact ttcatggaaa tgagacctcc
22201 atgaatgctc tgaaagacct gctgcgtcac acaggcgggc tgagcaagtt aggcctggag
22261 ttgtatcctg cccctctgga gagtcttgac tacaagggtc atgtcaattg ggagatcctc
22321 accccaattc gggctgagct gatgcgtaca ctcagggaag tcaggcagcc caagaggatc
22381 ttctttggtc ccgtcccttg ccctaactgt ggctcatggc catctgagaa agtggacttc
22441 catctttgct cctagggaag gcctggttcg tgggatggat aagctttctt ctggacactt
22501 gggaactaaa atattgtaca tgggtgcatt ttttaaaatt ttattttatt ttttatattt
22561 tttattttat tttattttta ttttatttta ttttattttt tgagacagag tctcactgtg
22621 tccctcagga tgaagtgcag tggcacaatc tcagctcact gcaagcacca cctcctgggt
22681 tcaagtgatt ctcctgcctc agcctcccaa gtagctggtg ttgtgggtgt atgcccccac
22741 gcccggctaa tttttgtatt tttagtagag acagggcttc acgatgttgg cgaaggctga
22801 cctcaaactc ctgacctcaa gtgatctgac caccttggcc ttccacagtg ccaggtttac
22861 aggtgtgagc agcagggccc ggtcagccgc ttcttaaagg aagcacacag ccacgtattt
22921 gaggcacgtg ctcactgtga gtggaaaaac aaaggtgact cagccagggg caggactggg
22981 taaaaatgct gacttggcat caatgaggcc ttcagggacc tgtgtcctag acttagaaat
23041 ggaacctgaa gttctagagt gatgcaggac ttacccctgc aaggatggtt atttaaaaat
23101 gtcaaaaata aatggaacct gaatggaaac tttctggtgt cttccatgat tgatcaacct
23161 gttttagaca tttatacatc agaagtctct agaaatctgc ctcctgggtt caagcaattc
23221 tcctgcctca gcctcctgag tagctggaac tacaggaccc cgccaccatg cctggctatt
23281 ttttgtattt tttgtagaaa cgggggtttc accatgttga ccaggctggt cttgaactcc
23341 tgacttcagg caaaccatcc acctcagcct cccaaagtgc tcagattgta gacattagtc
23401 actgcacctg gcctgaaatt ctgatataag cataaaatgt gtaatgttca aatcatggta
23461 acaggaatag ccaccatctc aagaatttat catttctttg tgttagcaac attccaattc
23521 cattgtttta attatgtaga aatttactat gaactattgt caacacgagt tgccctattg
23581 tgctactgaa ccctagatct tattcctgtc tgtgtttttg ttcccattaa ccatcccctt
23641 cttattctct atttcccagt acccttccta gcttctgata accgtcattt tactattttt
23701 aagtttcgtg ttttttaatt cccaaatatg agtgcaaaca tccatgtttg tctttctgta
23761 tctggcttac ttcactcaac ataacgccct ccagttccat ccatgttgtc gcagatgaca
23821 ggatttcctt catgtttaca gctcaatgat attctgttgt gtatatttac cacattttct
23881 tgatccattc atctgttgat ggacacttat gttgattcca aattttggct attgtggata
23941 gtgctacaat aaataggaga gtgcaagctg agtgcagtgg ctcatgcctg taatcccaga
24001 attttgggag gctgaggcag gtggattact tgaggtcagg agttcgagac cagcctgacc
24061 aacatggtag tgtagatatc tcctggatat atttcttttt ttttttctgg atatatatcc
24121 agcagtggga tttatggttt atgtggtaat cctattttta ttttttggag gaaactccat
24181 actgctttcc ttagtagctg tacgaattta cataactacc aatgttgtac cagggttctc
24241 atttctccat attcttcata gcatccatta ttttctgttg gttttttatg ggggagatcc
24301 ccttactatt aaaactcaaa tccagtttgg tgtaaacaca gaaaccctgc tagagttgcc
24361 tgccaccctt gaaacaggcc attggaatga aaattgtcca cctatgcacc aggtctgtat
24421 tggacagaat gcttttgtcc cagaggttgt tcacaataga ggacatttct tttttgtttt
24481 tctttacttt tctgcctttt tttttttttt agggtagagt ttcactattg ttgcccaggc
24541 tggagtgcaa tggtgggatc tcagctcact gcaacctcca cctcccgagt tcaagcgatt
24601 ctcttgcctc agcctcccaa gtagctggga ttacattcat gcaccaccac agctggctaa
24661 ttttgtgttt ttagtagaga cagggtttct ccatgttggt taggctggtc tcgaactccc
24721 aacctcaggt tattccacaa cctcagcctc ccaaagtgct gggattatag gcaggagcca
24781 ccacatccag ctagaggaca tttctgatgt ctccatattg atggaattta aaataactct
24841 ctggtaaatt gttttctata atagccttaa ataaaaatgg agaaggtgag attaagatca
24901 ttgcagactt aggtacagaa ttggtggaaa ccagggctgc catatccagt atacagccaa
24961 tatatcagca aatcccttgg agaaaggaaa atatttctga ggaggggttt cacatgaagt
25021 tcagaaaatt cctgtgtttg aagcagtcca aatgacattt ggaccatttt taggaaagta
25081 tggcttttta ttaagtgaca acatggggat gagatttgct ttctctgtta agttgatgcg
25141 taaagctttc tttggaggga gagaaaaccc tagagtttcc tgaccttcct taacctgaac
25201 tgcttggttc cctagaagca gaaattgatc atattagaac ccaaactcat accaaccttg
25261 accttcatga agtactcaag tgtttctgct cttcttcctc atgtgatgta gaaagtatta
25321 aaagtgatga gtttaggccg ggcacggtgg ttcacgcctg taatctcagc actttcagag
25381 gccgaggtgg gtgcatcacc tgtggtcagg agttccagac cagcctgggc aacatggtga
25441 aactctgtct ctactaaaaa tacaaaaact agttgtgtgt ggtggcctgt gcctgtaatt
25501 ccagctaact ggaagactga ggcaggagaa tcacttgaac cgggaggcag aggttgcagt
25561 gagccgagat cacaccattg cactccagcc tggaaaacaa gagtgaaact ccatctcaaa
25621 aaaaaattaa taaataaata cattataaat aaataaatta attaatgctt taaagaaaaa
25681 agaaataaac tttgcctaca aatttcatat gcaattgaat acctcttaaa ttttgatgtg
25741 aaccgaccag gcatggtggc tgaggcctgt aatcccagca ctttgggagg ccaaggcaga
25801 cagaccacga agtcaggaga ttgagaacat cctagttaac atggtgaaac cccgtcttta
25861 ctaaaaatac aaaaaattag ccaggtgtag tggcatgcac ctgtagtccc ggctatttag

25921 gaggctaagg caggaaaatt gcttgaaccg gggaggcaga ggtcgaagtg agctgagatc
25981 gtgccactgc attccagcct ggtgacggag cgagactcca tctcaaaaaa taaatgaata
26041 aaataaataa atcaataaaa atattgtgac aggaaccaac attgctcaac ttgtacacta
26101 atgtcttaca aaatcctttc cttgtcacct tcaaatctcc atttcaaatg ctacactctg
26161 cataactcta ccactttgtt gccattttct gatgatggag aagaccatac atgtgtgtgt
26221 gtggcatcag aactattgac tcctcctatt gatgtttaag atattccatt acacaaacct
26281 gggttcatac tttttgttga tagatcttat gccaaaaatg taggcaaaaa atgccaagca
26341 ggaaatgcta tcacttctga agatgaattc atagagatgg aaattctttc agaatttatt
26401 tttccagctt ttttctttgt ttgtttgttc gtttgtgttt gtttgttttg agacggagtc
26461 tcgctctgtc accaagttgg agtgcagtgg tgaaatcttg gctgactgca accgcctcct
26521 cctgagttca agcgactctc atgcctcagt ctctcgagta gctaggacaa tgggtgggcg
26581 ccaccatgcc cagctaattt ttgtattttt agcagagaca gggtttcacc atgttggcta
26641 ggatggtctc aatttttggc atcgtgatct acctgccttg gcctcctgaa gtgctgggat
26701 tagaggtgtg agccaccacc gtgcccggcc attttttttt ttttcctttt gagatggagt
26761 ctcactctat tgcctgggct gggaaaggaa ctcctcctat caattatttt tttaaatttt
26821 cttttgtttt attgacctga caagggtcaa atagagttga gtttttgttt ttgtttttc
26881 cattggaagg tacaaacaga ggttacaatc attggcttta gatgacaaga taaaagaata
26941 aaacatattc cttgcaagac aaccagcaga acttcatgat cagcatcaaa tcagtgcctt
27001 ctcactgtca gtgggtggaa gccttcatca atacttgtag agtttaaagc actcatgaac
27061 tcacgatcag actctttact cagggacagg atgtaagcca agcaaaagac cttccacagg
27121 tggtgaattt ggaagcctgc ccaatgtgac ctgcaagttt tcactggcaa tatgcaggtg
27181 cagatatgac aaagaataac catgaccttt acatcacccc cagctgttga ggaatgggat
27241 ccttttgacc ctttctgtcc atagaaccag gttattcatc ttgtgtggca acaacatata
27301 tggtgtactt aacagaaaag aagactctgt aaaaaaaaaa gtttattatg aagtaagcaa
27361 agaagtggca atagatgcga gattatttgg ggagataaag gaagttgaag gttttgaaag
27421 gaaaaataag gaggattata caaattgttt tgaaagactc atacttggtc ataaggatta
27481 aaaccaaaag ggcatcagtg caatgttaga tagattcctc ttacacccac tcaataaccc
27541 ccaacatgtt cagcaagtct tgcttcactc ccaggttccc attaaaaacc cagctcaacc
27601 ctgaccagct ccaccctcac ttccatttgt aattttgaca tgactttatt acaggaccat
27661 caggttccta tgcctgctgc acagtagctt agcaatattc tgagacagca gggtttgcag
27721 cagagagttt aatgatcaca aggtggctga atgagaagct aggaggagat cctcaaattc
27781 atctccccaa ggagtactga aggtttccag tggatcctgg atagcaaggg gccggaaagt
27841 tggggtagtg gtaagaggga agaagtcaac aggatgtaga aactgcattc tttggtgagt
27901 tggtgcattg catggccctt cagatcagct ggcatcagta gtttcactga catgcagaac
27961 ctgaaagaat atctcagatg aaaaagttaa tgttttacaa tgcttgaatc gttgtctgca
28021 gggaagttaa ggggaactgt aatctaaggt ctatatgatt ttggaacagt aggctgccag
28081 caaccatgag gaaccaggtc agagagcaag aagacctcct gatgaatgct gaatgtgttg
28141 caagcttggt ttatttttct ttctctccct cccttcttca ctgattaaat ttataaagtt
28201 tatcgatgtg gtttcaattt cttccaaaga agccttaacc taagcactga gaccactcac
28261 gccctcagtg gcacctctcc tccaccagaa cgagcatgta atctgctacc ttaggttata
28321 taaaatccca aagaccattc aatacattga gatttttatt ctgatatcgt agggatgact
28381 cctctgtttt tataaagctt tttaaagtag aaagcatttt tatattttga tgtggccaaa
28441 gatctcctaa caacactact ttcagatttt atttttctgt ctaatgttgg gaacagatca
28501 aatccttccc tgcctcacac tcaagactat gaagttcaca tattaataaa ataccatcag
28561 tgtttgtgga gttcatgaat gaatgatttt tttatttttt gacagaatct ccctctgtca
28621 cccagactgg agtgcagtgg cacaaatctg gctcactgca accattgcct cctgggttca
28681 agcaattctc ctgcctcagc ctcccaagta gctgggtttc aggtgcctgc catcatgccc
28741 agctaatttt tgtatttttg tagagacggg gtttcacctt tttgacctga ctggtcttga
28801 acccctgaca tcaggtgatc tactcacctt ctccttccaa agtgctggaa ttacaggtat
28861 gagccacctc gcccaccctt gaatgaatgt attcttgact tctaccctat ccctaacact
28921 gtcaatttct tgcttcacga actgaataca gatatgtgat atgaatggat atctgactca
28981 atccattaat ctggggaaag ccaaaaaccc aatcaggatt aactgggtgg agcttcacaa
29041 atgcaatcag atattgcttt ttgattggaa gctagcagtg catacgtgga ggggcgtggg
29101 tgggagttgt gattagaaag gtcagtaaaa gcttctaagg acccacagga gagacccaaa
29161 gtcttcaagc ctggagttcc tgcttggttc ttcctgagat ctgagcacct tctagactac
29221 atccagatct ggtaagtcac taatttctgt aaggacactc ccatctgacc tacagtcagt
29281 cggtctggga tggtgacagt gcagcctacg atggcacaga gctatatcct gtcctttttt
29341 tttttttttt tttatatgaa caattggaag ctttgaattt tttcctctaa atgcagttct
29401 gtctttattt caaaaaagtt gattgtgctt tggttgatgc cattttaaaa ttcttgaagg
29461 gagcagtgac tcatgccttt aaccccaaca ctttgggagg ccaaagtggg aggatcattt
29521 cagcccaggg atttgagacc aacctggaca acatgacaaa aaccctcctc tgcacaacgt
29581 tttttttttg agggtgggga tggagtctca ctgtgttgcc cagactggag tgcagtggca
29641 tgatctcaac tccctgcaac ctttacttcc cgggttcaag caattctcat gcctcagtct

29701 ccatcctcag aagctggtgt cacagacatc tgaaaccatg cctggctaac ttttgtattt
29761 ttagtagagg tggggtttca ccacgctggc caggttggtc tcgaacacct gacctcaagt
29821 gatccacctg ccttggcctc ccaaagtgct gggattacag ctgtgagtca ccatgcatct
29881 gtagtcccag ctatttgggt ggctggtgtg ggagaatcac ttgagcccag aagattgagg
29941 ctgcagtgag ccatgctcac accactactg tactccagcc tgggcaaaag agagacactc
30001 tgtccaaaaa caaaaacaaa atcaatcaaa aaggatcttt gaccttaatt ttaaaccaat
30061 cacatcctct tccacccaaa tggaggcatg gctgtggggg gtgcatgcct gtagtcccag
30121 ctatgtggaa ggctgaagca tgagaattgc ttgaatcttg gaggcggagg taacagtgag
30181 ccatgatggt gccactgcac tccagcctgg gcgacgaagt gagactcagc tccctcagca
30241 ccaaaaacaa ttatatgacc caggtgatca tcggatacat gaagatttct attgtgtttt
30301 cttagggact gtcatctctg tctttgaaaa ctgttttaac tctgaaatat tttgataaat
30361 ttgatgtggc caaggatccc tcaacaaaga tactttcaag ttttctttct ttctgtctaa
30421 tatcaggaag agattcaacc cttccctgtc tcacactcag gactttgaag gacacatatt
30481 agtaaaactc catgtttgtg aagggaatca gtgaatgagt cctggacttt caccctatcc
30541 ctaaatcttt cattttgatg gatcaatatc taattcgatc agttaatctt taagaaagcc
30601 aaaaatccaa taaggattaa ctgggtagag attaagaagt ctagtcaaat gtagctctct
30661 ctgtctctca gttcaatcta gcctatttcc caggctggag tggagtggta taatgtcagc
30721 tcactgcaac ttctgcctcc tgggttcaag tgatcctcct acctcagcct ccctagtagc
30781 ttggactaca ggcgcagacc actgcacctg gctaattttt gctgtcttag tagagtcagg
30841 gttttaccat gttggccagg ctcgtcttga attcctgatc tcagatgatc cacctgcctc
30901 ggcctcacaa aatgctcaga ttacaggtgt gagtcactgc acccagccaa agtggttcag
30961 tttgaacatg tgtaacaagt gtgcattgga aacatctatc ttgagaatga tgcataacag
31021 tgtcacatag ctttcaaagc ttctcactga aattttcaat aatgaggctg gggtggaggc
31081 tcacacctat aatcccagta tgttgggagg ccaagagggg tagattgctt gagactagga
31141 gttcaagacc agcttggaca acatagcgaa atccactgtc tttacaaaaa gtcaaaaaat
31201 aaaagatgag ctgggtgtgg tgatgcataa ctgtggtccc agctacttgg gaggctgagg
31261 aggaagaatc ctttgagctg ggaggtcaag gctgcactga gctgggatcc caccactaca
31321 ctccaggctg ggtgacacag caagactctg tcagaaagag tgagagaggg agagagagaa
31381 agagagagag aatgagagaa gggaggcagg gaaagaagac aagaaagaaa aaaggagag
31441 agagggggaa agaaagaaag aagggaggga gagagggaaa gaaggaaaga agaaagagag
31501 agaaagagaa agcaagctta aataatgaaa agaaaacaaa tagaacctgt tctagggatg
31561 ccccatgaat gttcccaaca agcttatttg taggaactga aaatgtgggc atgtaggctt
31621 gtgacattcc cattcccatt gttttagaac cttgagtaat tagtagtttc ccccaatggt
31681 aggaggggtt cactttcagg ttcctccaca ctcactagtc actggatgga gcactggata
31741 gaaaggaagg gctcgtggtg gccctgcttc ctcactgctt cggagacgct catgctgatg
31801 cagcagaggc agaatgctgg cttaatggcc actgagtaca gagtagaatt ggagtaaact
31861 gaggactctt tcaccattgc cagagcagtg agtttggcca taggagagga tgagattgca
31921 tgggcttggc ccgagagaga tgccttttct ctgggtttgt cctctggaag ttttccctgc
31981 agattcatga agatgagcat ctggactcca cccagactcc tggagcttgc agggcggagc
32041 ctgctaaggg accaagcttt ggccatgtcc accctggagg agctgcccac agaacttttc
32101 cccccactgt tcatggaggc cttcagcagg agacgctgtg aggccctgaa gctgatggtg
32161 cagtcctggc ccttccgccg cctccctctg aggcctctga taaagatgcc ttgtctggag
32221 gccttccaag ctgtgctcga tgggctggat gcactgctta acctaggggt tcgtcccagg
32281 tgaggtggcc caggtgggct ggtggggagg gcccaggtgt ccaactgaag gaacagctgc
32341 gtcgtgagaa gtgaggaggc ccaaggggga tggtggtggt gaggaagccg agaggacttg
32401 gccattcacc agctcctcag ggaaagcact gctcaccacg caaggtccat ggaggtaaca
32461 ggaacctctc ctctaatggc actgaaaggc accatgaaaa gtgagaactg ggccgggcac
32521 ggtggctcac aatgtattcc cagcacattg ggaggctgag gtcaagagtt ggaggccagc
32581 ctgtccaaca tggtaaaccc caactctact aaaaatacta aaattagctg ggcatggtgg
32641 tgggctcctg taatcccagc tacttgtgag gttgaagcag gagaatcatt tgaatctggg
32701 aagcagaggt tgcagtgagg tgacatcaca ccactgcact ctagcctggg cgacacaggg
32761 agacttggtc tcaaaaaaaa aacaaaacaa tgtggaagtg ggtaggatcc aaggggaaaa
32821 cagagtgaag aaaagtcaga gagagggaca agaagcaggg aggggaggag ctgctatcca
32881 ggatgtggag tttaagttca gaaatgagtt ctgaaattct cagtctcacc tctattttcc
32941 cacaggaggt ggaaacttca agtgctggat ttacaggatg tctgtgagaa cttctggatg
33001 gtttggtctg aagctatggc ccatgggtgc ttcctcaatg ccaagaggaa caaaaaacca
33061 gtggaggact gtccaaggat gagaggacgg cagcccttga ctgtgttcgt agaactttgg
33121 ctcaagaaca ggactctgga tgaatacctc acctgcctcc ttctatgggt caagcagagg
33181 aaagatttac tacacctgtg ctgtaagaag ctgaaaattt tgggaatgcc cttccgcaat
33241 atcagaagca tcctgaaaat ggtgaaccta gactgtatcc aggaggtgga agtgaattgc
33301 aagtgggtac tgcccatcct gacacagttt accccatacc tgggccacat gaggaatctt
33361 cagaaactca ttctctccca catggatgtc tctcgctacg tttccccaga gcagaagaag
33421 gagattgtta cccagttcac cactcagttc ctcaagctgc gctgcctcca aaagctttat

33481 atgaactctg tttctttcct cgaaggccac ctggaccagc tgctcaggtg agggagggtg
33541 gtgagctttc tctgcagacc acagcagagc ctgttacagt aaacactagt gggcatctac
33601 tgtgagccag cctatgagga tgaaacagtg aaggggacac tagaatgtcc atgcattgtc
33661 ctgtgggcgg ccctgtcctg aaatgggtat catgcaaccc tcccaataga ggcagaggga
33721 tcagctaggg gagatgctat ggagaggctg ccatgctagg aagccagcta ctgggggtt
33781 cagatctagt gagggtgcct ttctgaattc ttcctgagga cgtgtgtcta agttaagatg
33841 atgaaaaata cgccaggggc ggtggctcat gcctgtaatc ctatcacttt gggagtctga
33901 ggcaagagga tagcttgagc ctaggagttt aagaccagtc tggctaacat accaagaccc
33961 ctgtcagaaa tgaataaaca aaagtaaaaa caaacaagat aacttttttt ttctgagatg
34021 gagtttctct ttgatcgtct aggctacagt gcagttgtga catctcagct cgcagcaact
34081 tctgcctccc aggttcatgc gattctcctg cctcagcctc ctgagtgcct gggattacag
34141 gcgtgagcca ccacacctgg ctaattttta tattttaagt agagacaggg tttcaccatg
34201 ttggccaggt tattctccaa ctcctgactt caggtgatcc acccaccatg gactcccaaa
34261 gtgctgggat tataggcgag agctaccacg cccagccaac aagataattt ttaagaagat
34321 gatgtgaagt agggaagtga agtgggcact gaagagggga atgttcagca aacctgcact
34381 tgtcagaaaa tcagctttgt gccccacagt ttggtgaaca tgaatgatcc catctctaat
34441 tccctgttgt aaaagtttct tttgagctcc aggtaaatta attatctagg caatgcatga
34501 ttctgaaaca gagggtcagg gtgcaggcac aaagaatggt gaaagtgata gatggtttgc
34561 tgatgataca ggcatgtcag ggatgcctgc agcccgccca ccgtagctga tgttgcagga
34621 tcctgtctgg gtttgtcctt tatgcctgaa tctccactgg gctcctgtgg cccagggatg
34681 tggttttctg cctgacagat gaggaaaggg agctttaggg attctgtgaa cttgatccat
34741 tcctataaat gatggtgaag tgactcagcc tcaaatggaa ttattttttc tccttttttt
34801 tttttaatac agagtctctc tctgtcaccc aggctggagt gtagtggcat gatctctgct
34861 cactgcaacc tacacctcct gggttcaagc gattcttctg cctcagcttc ccaagtagct
34921 ggaattgcag gctcccccca ccacacctgg ctaatttttg gatttttagt agagaggagg
34981 ttttgccatg ttcagcaggc tggtctcaaa ctcctgatct caaggaatcc accagtctca
35041 gcctcccaaa gtgctgggat tacaggtgtg agttactggg ccgggtctaa agtggaattg
35101 acctcggtgg caaagctctt catcacacat catcctaagt gttgaccatc aggccatcag
35161 aatgaccctg gacttgggca aaaaggtctc catccattac cttgaagcca ttccccacca
35221 ccctccactc acccctatga ttccccagaa ttaacttctt gctctctctc cccagctgtc
35281 tgaagacctc gttaaagttc ctcacaataa ctaactgtgt gcttttggaa tcagacttga
35341 agcatctatc ccagtgcccg agtatcagtc aactaaagac cctggacctg agtggcatca
35401 gactgaccaa ttacagtctt gtgcctctcc aaattctcct agaaaaagtt gcagccaccc
35461 ttgagtacct ggatttagat gactgtggca tcatagactc ccaagtcaac gccatcttgc
35521 ctgccctgag ccgctgcttt gagctcaaca ccttcagctt ctgtggaaat cccatctgca
35581 tggccaccct ggagaacctg ctgagccaca caatcatact caaaaactta tgcgtggagc
35641 tgtatcctgc cccccgggag agttatggtg ctgatggtac tctctgctgg agcagatttg
35701 ctcaaattag ggctgagctg atgaacagag tgagggactt aaggcacccc aagaggatct
35761 tgttctgtac tgactactgc cctgactgtg gcaacaggtc attttatgac ctggaggcag
35821 atcaatactg ctgttgaatg cctgcctatt tggatgggta tgtcaaacgc tttcttctgg
35881 acacttggaa actaaaacct aggtcttagg tacatcctaa agggagcaca gaacccatca
35941 tttcacacat aggctctgaa agtgggaaag gaaagctgat caagcagggg ccggacttgg
36001 gggaaatgtt gccatggatt cgatgggact ttggggacct gtgtcctgta gagtcgaaaa
36061 tgggaatctg aatgtctaga gtggaattca ggcttgagaa tacatgaggg agttactctt
36121 gcatggatgg ttgtaaagaa acaatcagaa ataaaggaaa actgagcaga atctgtctgg
36181 tgccctctat tattaagtaa cctgttttcc agtttaagcc tcaggaatct tcagttattg
36241 atggaaaaaa caaaaggcac tgactgagtt gtccaatcaa taagatgcag cccaagaaaa
36301 tcaaggcatt taaatgaaat ttggttattg taatcagttt cctcccattc ttttattgga
36361 gacagagttt cactcttgtt gcccaggctg gagtttagag tgcaatggtg ccatctgagc
36421 tgactgcaac ctccacctgg ggtttaaatg attcttctgc ctcagcctcc caagtagctg
36481 ggattacaag catgcaccac catgcccagc taatttgtgt atgtttagta gagacagggt
36541 ttcctcacta tgttggtcag gctggtctca aactcctgac tttgggtgat tcatgcaagt
36601 aggcctacca aagtgctggg gttacaggtg tgagccactg tgtcaggctt ttttgtttgt
36661 ttgtttttta aaggtctcct gtcactcagg ctacagtgca gtggcacaat catacctcac
36721 tgcaacctca atttccgggg ttcaagcgat ccacccacct cagcctcctg agtagctatg
36781 actacagctg tgtgagccac cacacctgga tacttctttt tagtagagac aaggcctcgc
36841 tgtcttcccc aggctgatct ggaactcctg agcttgtgat tctcctgcct tggcctccca
36901 aaatgcaggg agtataggcg tggaccacca cgcttggctt ggcctcctcc agttcttcac
36961 ttctttagat gtctgttaac tccttgttag tttctgtggc tgttcagtgg gttaatacac
37021 actaggtgga caccaaaggc ctggaacatt actgggcaag aacagtgagc caatccacac
37081 ggaaagcacc ttcttctcag ggtctttcac cgctatccag atgctgagac cctgcccact
37141 ccctgtgagt ctccacatgc ttccagaagc cttagttggt ggatgtcagc ttcactgcac
37201 aaggagccac tctcttcccg ctgccctgga aggggatgtc catattgtgt attagctgga

37261 gactctgggc agcatcaacc cttgcttgtt ctcctgatga ccagcagccc ttcttgaatt
37321 aaactggttg tagccagtaa agacagccac attcccttta agtaaaatac taaaactata
37381 caggcatgta acacttttta aatatttcca tctgacattt taaaagttac atcttttggg
37441 ggagctaggt cagattgatg agagattttc tcataacacc tcccctctct ccctatgaag
37501 gaagagacta gtgcagcgtg ttctggaatc tgacagcatc aaaggtggta taacgatcaa
37561 gggcctgtgg gtgatgagtg accttccctg tgctgaggaa ttctgcataa tgggcaccca
37621 agtgaaggat cctgctgagt actcaggggc tggtgttgct gtcagggatg ttagcctaga
37681 gcctcagctt cctgtaaaat gaggatgatg atgtccaaca gcttatggga ccttggtagg
37741 atccaatgag atggttcatg tttagggctt ggcatggggt ctggcataca gtaagatcaa
37801 tacatcttgt tctttttctt cttctcagca gaagtcccag cacttttcat ctttcaatct
37861 cacctccttt tcctgataat agagaggcaa caagaactca gggcatgcaa tggggctcaa
37921 cttctactct ctgccacaat ttcatcatga ttgccccaaa gagcagagcc ccaggagcca
37981 gcagggggca gggtgggcat ttctggactg gattcattca taataagatc aaaatttcca
38041 atccgtatgt ctcgggtgcc atctgctgat agatccgacc agatggtata attgagtgtt
38101 gcaaggatta tattttatgg tgtttttaaa aatgtactat tatgagccag gtgcagtgag
38161 tcatacctgt aattccagca ctttgggagg ctgaggcagg tggatcacct gaggttggga
38221 gtttgagacc agcctgagca acatgaagaa accccttctc tacttaaaat acaaaaaatt
38281 agccaggcgt ggtggcgcac gtctgtaatt gcagctactc gataggctga ggcgggagaa
38341 tcatttgaac ctggaggtgg aggttgtggt gagctcagac tgagccattg cactccagcc
38401 tgggcaaccc tagcaaaact ccgtctcaaa aaaaaagata aaataagatt tattattatg
38461 gccgggcatg gtgtctcaca cctctaatcc cagcactttg ggaggccaag gcagcctcag
38521 gattttgaga ccagcctggc caacatggtg aaaccccatc tctactaaaa atacacaaaa
38581 tttgctggga gtggtggcat tcgcctgtaa tcccaggtat tcaggaggct gaggcaggac
38641 aatcacttga acccgggagg tgagggttgc aatgagacga gatggcacca cttcactcca
38701 gcctgggcga cagagcatga aaaaaaattt actataatgt gaatactatt agagtataaa
38761 tatttgtgtt gtaatttatg tatatgaaag attagaactt ttaaagaatg caacgtgata
38821 tttcaagaat ggttaatggc caggtgtggt ggttcatgcc tgtattcctg gcactttggg
38881 aggccgaggt gggcagatca cgaggtcagg agttccagac cagcctggcc aacatgatga
38941 aaccccgtct ctacgaaaaa tacaaaaaat tagcctggcg tggtgacagg tgcctgtaat
39001 cccagatagt caggaggctg aggcaagaga atcgcttgaa cctgcgaggc aaaggttgca
39061 gtgagccaag aatgcaccac tgcactccag cctgggtgaa agaggaagac tccgtctcaa
39121 ggagggtgag aaaaagaata cttaacttgg tttgaaatgt caaaacaaat gagattttaa
39181 aaactaattt taaagacact gaacaataat catttcttct ttaaaatata tttagaataa
39241 tacaatttta gctttgaaag gaaacattac agttttaaaa aatattgagt ttattttatt
39301 ttattttatt ttattttatt tggagacaaa gtctcactct gttgtccaga ttggagtgca
39361 gtggcatgat cacggcttac tgcagccttg acctcctagg ctcaggtgat ctccctgcct
39421 cagtccccct agtagctgga acaacaggca tgcaccatca tgcctgactt atttttgtat
39481 tcttagtgaa gaccaggctt caccatgttg cccagactgg tcttgaaatt ctgggctcaa
39541 gcgatccacc tgcctcggcc tcctaaattg ctgggagtga gttcctatag gcatgagcca
39601 ccgcacccag ccttgagttt atttatttat ttattttgga gatggagtct ccttctgtca
39661 tccgtgctgg agtgcagagg tacgatctct gttcactgca acttctgcct ccaggattcc
39721 agcaatgctc ctgtctcagc ctccagagta gctgggatta caggcatgca acaccacacc
39781 tgattaattt ttgtattatt attattatca gtttttagta gagacagggt tttgtcattt
39841 tagctaggct ggtctggaac ccctgatctc aggtgatcca cctgcctcgg cttcccaaaa
39901 tgctatgact atagacgtga gccaccacac ccagcctatt ttttccttta cagcagtttt
39961 agattcacag aaaaactaag cagaaactgc agagttctca tctaccttct tcccccttca
40021 atacacagca cccccacagg atcagcaccc acaccagcac agagcattca tcacaaccaa
40081 tgagccacag ggacacatca ttatcaccca atgtccatag ttcacatgag ggatcattgc
40141 tggttttgta cattctatgg attttaacaa agggataatg acatgtatcc accattagag
40201 catcatggag agtagttttc tttccctaaa attcctctgt cctcttccca ttcatcccat
40261 tgtgctccca accccttgca tccactgggc tttctactgt ctccttagaa aaatgcaaaa
40321 gctttttctg gaatgtctaa caggatgagt cttttcagat tgccttcttt cacttgtaca
40381 ataacgtgca tttaggaatt tttcatgtct ttttacagct ttataatagt tcactgactg
40441 gatggatcag tttgcttatc cagtcactga ccgaagggca acttgctagc ttccaagttt
40501 tggcgattat gagtaagttg ctgtaaacat ccaggtgtgg gtttactcac ttcattaaat
40561 atccaggagc atgattacgg aattgtaggg gtatggtatg ttttacaatg atttcttctt
40621 tcttgacaat ctcacttgtt cgatattgct gctaaaggtc aggaactttg tctccatcat
40681 cctgtgttcc cactgctgag catggaacgt ggcacttggt agcaaatgct gttgaccacg
40741 tgatgcatgg aaacgtttat catgggtata gtcactaaat tgctaccttg gggacatcaa
40801 cattagctca ctaccaataa tataaataaa ttggattatg gaaaaaaatg gcccttgtga
40861 tactgtggat actccaggtg tatcatgaac gtccagcaat tgaccaggca cagtggctca
40921 catctgtaat cccagcactt gcagagactg aggtgggtgg atcacttcag tcaggagttc
40981 aagacgactc tggccaatat gatgaaaccc tgtctctatt aaagacacaa aaattaacta

41041 gggggttgag ccaagatggc cgaataggaa cagctccagt ctacagctcc cagcctgagc
41101 ggtgcagaag acgggtgatt tctgcatttc caactgaggt accaggttct tctcactggg
41161 gagtgtcaga aagtgggtgt aagacagtgg gtgcagtgca ccgagcatga gccaaagcag
41221 cgtgaggcat tgcctctcct gggaagtgca aggagtcagg gaattccctt tcctagtcaa
41281 agaaaggggt gacagatggc acctgtaaaa tccggtcact cccaccctaa tactgcactt
41341 ttccaacagt cttagcaaat ggcacaccag gagattatat ctcgtgtctg gctcaaaagg
41401 tcctatgccc acggagcctc actcattgct agcacagcag tctgagatca aactgcaagg
41461 cggcagcaag gctgggggag gggcgcctgc cattgttgag gcttgagtag gtaaacaaag
41521 cagccaggaa gctggaactg ggtggagccc actgcagctc aaggaggcct gcctgcctct
41581 gcagactcca tttctggggg cagggcattg ccaaacaaaa ggcagcagaa tcctctgtag
41641 acttaaatgt ccctgtctga cagctttgaa gagagtagtg gttctcccag cacgcagctg
41701 gagatctgag aatggacaga ctgactcctc aagtgggtct gtgaccccta agtagcctaa
41761 ctgggaggca cccaccagca ggggcagact gacacctcac atggccaggt actcctctga
41821 gacaaaactt ccacaggaat gatcgggcag caacatttgt tgttcaccaa tatccactgt
41881 tctgcagcct ctgctgctga tacccaggca aacagcttct ggagtggacc tccagcaaac
41941 tccaacagac ctgcagctga gggtcctgac tgttagaagg aaaactaaca aacagaaagg
42001 acatccacac caaaacccag tctgtacatc agcatcatca aagaccaaag gtagataaaa
42061 ccacaaagat ggggaaaaag cagagcagaa aaatgggaaa ctctaaaaat cagagttcct
42121 ctcctcctcc aaaggaacgc agctcctcac cagcaatgga acaaagctag agggagaagg
42181 actttgatga gttgagagat gaaggcttca gatgatcaaa ctactctgag ctaaaagagg
42241 aagttcgaac ccatggcaaa gaagtcaaaa acattgaaaa aaaattagat gaatggctaa
42301 ctaaaataac caatgcagag aagtccttga aggacctgat ggagctgaaa accatggcac
42361 aagaaccacg tgacaaatgt acaagcctca gtagctgatt ccatcaactg gaagaaaggg
42421 tatcagtgaa ggaagatcaa acgaatgaaa tgaagcgaga agagaagttc agagataaaa
42481 gaataagagg aaatgaacaa agcctccaag aaatatggga ctatgtgaaa agaccaagtc
42541 tatgtctgac aggtgtacct gaaagtgatg gggagaatgg aaccaagctg gaaaacactc
42601 ttcaggatat tatccagcag aacttcccca atctagcaag gcaggcaaac attcaaattc
42661 aggaaataca cagaatgcca caaagatact ccttgagaag agcgactcca agacacataa
42721 ttgtcagatt caccaaagtt gaaatgaagg aaaaaatgtt aagggcaacc agagagaaag
42781 gtcgggttac ccacaaaggg aagcccatca gactgacagc ggagctctcg gcagaaactc
42841 tacaagctag aagagagtgg gggcctatat tcaacattct taagaaaaga atttcgaccc
42901 cagaatttca tatgcaacca aactaagctt cataagtgaa ggagaaataa attcctttac
42961 agacaagcaa atgctgagag atttgtcacc caccaggcct gccctataag agctcctgaa
43021 ggaagcacta aacatggaaa ggaacaactg gtaccagcca cttcaaaaac atgccaaatt
43081 ggaaggacca tcgatactag gaagaaactg catcaactaa agagcaaaat aaccagctaa
43141 catcataatg acaggatcaa attcacacat aacaatatta accttaaatg taaatgggct
43201 aaatgctcca attaaaaaac acagactggc aaatttcata aagagtcaag acccatcagt
43261 gtgctgtatt caggaaaccc atctcacatg cagagacaca cataggctca aaataaaggg
43321 atggaggaag atcttccaag caaatggaaa acacaaaaag gcaggagttg ccatcctagt
43381 ctcggataaa acagacttta aaccaacaaa gatcaaaaga gacaaagaag gccattacat
43441 catggtaaag ggatccattc aacaagaaga gctaactatc ctaaatatag atgcacccaa
43501 tacaggagca cccagattca taaagcaagt ccttagagac ctacaaagag acttagactc
43561 ccacacaata ataatgggag acttcaacac cccactgtca acattagaca catcaatgag
43621 acagaaagtt aacaaggata tacaggaatt gaactcagct ctgtgccaag cggacctaat
43681 agacatctac agaactcaca atccaaaatc aacagaatat acattcttct cagcaccaca
43741 ctgcacttat tccaaaaatt gaccacatag ttggaagtaa agcacacctc agcaaatgta
43801 agagaacaga aattataaca aactgtctct cagaccacag tgcaatcaaa ctagaactca
43861 ggattaagaa actcactcag tgtgtgatgt tccctttcct gtgtccatgt gttctcattg
43921 ttcaattccc acctatgagc gagaacatgc agtgtttggt tttttgtgct tgtgatagtt
43981 tgctgagaat gatggttccc agcttcatct atgtccctac aaaggacatg aactcatcat
44041 tttttatggc tgcataggat accattagga ggtataccta aggctaaatg acgagttaat
44101 gtgtgcagca caccaacatg gcacacgtat acatatgtaa caaacctgca cgttgttcac
44161 atgtacccta aaacttaaag cataataata ataataaaag aaactcactc aaaaccgctc
44221 aactacatgg aaattgaaca acctgctcct gaatgactac tgggtacata acgaaatgaa
44281 gacagaaata aagacattct ttgaaaccaa tgagaaaaaa gacacaacat accagaatct
44341 ctgggacaca ttcaaagcag tgtgtagagg gaaatttata gcactaaatg cccacaagag
44401 aaagcaggaa agatctaaaa ttgacaaccg aacatcacaa ttaaaagaac tagagaagca
44461 agagcaaaca catccaaaag ccagcagaaa gcaagaaata gctaagatca gagcagaccc
44521 aaaggaaata gagacacaaa aaccccttca aaaaatcaat gaatccggta gctggttttt
44581 tgaaaagatc aacaaaattg atagactgct agcaagacta ataaagaaga aaagatagaa
44641 gaatcaaata gatgcaataa aaaatgataa agggcatatc accacggatc ccacagaaag
44701 acaaactacc atcagagaat actataaaca actctatgca aataaactag aaaatctaga
44761 agaaatggat aaattcctca acacatacac cctccccaga ataaaccagg aagaaggtga

44821 atctctgaat agaccaataa caggctctga aattgaggca ataattaata gcttaccaac
44881 caaaaaaagt ccaggaccag atggattcac agtcgaattc tatcagaggt acaaggagga
44941 gctggtacca ttccttctga aactattcca cttaatagaa aaagagggaa tcctccctaa
45001 ctcattttat gaggccagca tcatcctgac accaaagcct cgcagagacg caacaaaaaa
45061 agagaatttg agaccaatat ccctgattaa catcaatgca aaaatcctca ataaaatact
45121 ggcaaaccga atccagcagc acatcaaaaa gtttatcctc caatatcaag tcggcttcat
45181 ccctgatccg caaggctggt tccacttacg caaatcaata aacgtaatcc atcacataaa
45241 cagaaccaat gacaaaaacc acatgattat ttcaatatgt gcagaaaagg cctttgataa
45301 aattcaacac cctttcaggc taaaaactct agataaacta ggtattgatg gaacgtatgt
45361 aaaaataata agagccattt atgacaaaac cacaaccaat atcatactga atgggcaaaa
45421 gctagaagca ttccctttga aaaccagcac aatgcatgga tgccctctct caccactcct
45481 attcaacata gtattggaag ttctggccag ggcaatcagg caagagaaag aaataaagag
45541 tattcaaata ggaagagagg aagtcaaatt gtctctgttt gcagatgata tgattgtata
45601 tttagaaaac cccatcgtct cagcccaaaa tctcctaaag ctgataagca acttcaacaa
45661 agtctcagga tacaaaatca atgtgcaaaa atcaaaatca ttcctataca tcaacaatag
45721 acaaacggag agccaatcat gagtgaactc ccattcacaa ttgctaaaag aaaataaaat
45781 acataggaat acaacttaca aggggtgtga aggacctctt caaggagaac tacaaaccac
45841 tgcttaagga aataagagag gacactaaca catggaaaaa cattccatgc tcatgggtct
45901 gaagaatcaa tatcatgaaa atggccatac tgcccaaagt gatttataga tccaatgcta
45961 tccccatcaa gctgtaatgg agtttcttca cagaattaga aaaaactact taaacttca
46021 tatggaagca aaaaagaacc tgtatacaca acacaatcct aagcaaaaag aacaaagctg
46081 gaggcatcac gctacctgac ttcaaactat acgacaaggc tacagtaacc agaacaacat
46141 ggtacagtta tcaaaacaga tatgtagacc aatggaacag aaaagaggac tcagaaataa
46201 tgccacacat ctacaaccat ctgatccttg acaaacctga caaaaacagc caatggggaa
46261 aggattccct atttaataaa cggtgttggg aaaactggct agccatatgc agaaaactga
46321 aactgaaccc cttcctttca ccttatacaa aaattaactc aagatggatt aaagacttaa
46381 atgtaaggcc taaagccatt aaaaatccta gaagaatacc taggtgatac cattcaggac
46441 ataggcatgg gcaaagactt catggctaaa acactaaaac caatggcaac aaaagccaaa
46501 attgacaaat gggatctaat taaaataaag agcttttgca cagcaaaaga aactatcatc
46561 agagtcaaca ggcaacctat agaatgggaa aattttttgc aatctatcca tcagacaaat
46621 ggctaatatc cagaatctac aaggaactta agcaaattta caagaaaaaa acaaacaacc
46681 ctgtcaaaaa gtgggtgaag gatactaaca gacaactctc caaaaaaacc atttatccag
46741 ccaacaaaca tatgaaaaaa tgttcatcac cactggtcat ttgatttgca tttctctaat
46801 gcaaatcaaa accacagtga gataccatct catgccagtt agaatggtga tcattaaaaa
46861 gtcaggaaac aacagatgct ggaaaggatg tggagaaata ggaatgcttt gacactgttg
46921 gtgggagtgt aaattagttc aaccattgtg gaagacagtg tggcaattcc tcaaggatct
46981 agaaccagaa ataccatttg atccagcaat cccattactg ggtatatatc caaaggatta
47041 taaatccttc tactataaag acacatgcac aagtatgttt attgcagcac tattcacaac
47101 agcaaagact tggaaccaac ccaaatgccc atcaatgata gactggataa agcaaatgtg
47161 gcacatatac atcatggaat actatgcagt cataaaaaat aagttcattt cctttgcagg
47221 gacatggatg aagctagaaa ccatcattct cagcaaacta acacaggaac agtaaaccaa
47281 aacaccacat aagtgggagt tgaacaatga gaactcatgg tcacaggtag gggaacatta
47341 cacatcaggg cctctcgggg tgtggagggc taggagaggg gtagcattag gggaaatacc
47401 taatgtagat gacgggttga tgggtgcagc aaaccaccat ggcatgtgta tacgtatgta
47461 acaaaactgc acgttctgca catgtatccc agaacttaaa gtggaaagaa agaaagaaag
47521 aaagaaagaa agaaagaaag aaagaaagaa agaaagaaag aaagaaagaa agaaagaaag
47581 aaagaaagag agaaagaaag aaagatggaa ggaaggaagg aaggaaggaa agaaagaaag
47641 agaaagtaag aaagaaagaa agaaagaaag aaagaaagaa agaaagaaag aagaaagaaa
47701 gagagaaaga aagaaagatg gaaggaagga aggaaggaaa gaaagaaaga gaaagtaaga
47761 aagaaagaaa gaaaaaagaa agaaagaaag aaagaaagaa agaaagaaag aaagaaagaa
47821 agaaagaaag aaagaaagaa agaaagaaag aaaagacaag acagtggagg ggaggggagg
47881 ggagaggagg tgaagggaag ggaagggaag ggaagggaga agaaaagaaa tacccataaa
47941 ataggaaagc tggctggtca caggagaagc atgaaaatat caagcagtga tttcatatag
48001 cagcaagaaa agagcctgta aaattagctg caagaataag aataagcctt gacccataag
48061 atccaaacaa gctggaaggg gctaagctgg ctaacactga attggtcaga catggcactg
48121 ggtttgaccc ttgccctacc ccaggcctaa ttatacacct attatgacag taagtcacac
48181 accagcgcca ggacggttct gagaatgccc atatttagta taaaaatagt taacacctag
48241 ccacgtgcag tggctcatgc ctgtaatccc aacattttgg gaaactgagg caggcggata
48301 acctggtgtc gggagattga gaccaccctg accaacatgg agaaatctcg tctctcctaa
48361 aaatacaaaa ttagccgagt gtggtggtgc atgtctgtaa ccccagctac tccagaggct
48421 gaggcaggag tattgcttga acacgggagg cggagattgc agtgagccaa gatcgcgcca
48481 ttgcactcca gcctgggcaa caagagcgaa actccatctc aaaacataaa taaataaata
48541 aataaataaa taaatgacac ctcagttcta agaaaacttc accatttttt cttaaaatcc

48601 taatgattat ttcaacctct ccttacagat cctataaaat tagaaaccca aactctcttg
48661 tacctgactc gctctcctga ataagccctc tcttgagtgt gttcctttgc tttgcaatag
48721 acacttcttg ccttttgctt cattctgcct acttcctaaa ctctttcttg cagcggtgac
48781 aagaatgtgg acactggttg gtgattgagt ctctgggcac ctggagacga cctaagcact
48841 atggcaatag tcagtctaaa aatcacacag gatatcacaa ttcactctct ggttttcttg
48901 ggggaaaaat ccagtaactt tggccccata tccccaaggg gcatcactca gcacaaactg
48961 agaagcagca gtcctaccgc tgggttgtaa gtatgtggct ttattccggg gttctctatt
49021 ccattccatt ggtctatgtc tcgaccttca tactggtacc acgcagtttt gcttactatt
49081 gccttactgt ataaattgaa gtcaggtaat gtgatgcctc catatttgtt cattttgctt
49141 cggattgctt tggctcttca gcctcttttt catctccata tgaatcttag gattcttttt
49201 ttaatcttgt gaaaatggtg tttgtatttt ggtgtatgaa atttttagac tgacgttttt
49261 agattgatgt ttgatgtttg caggttttag attgctttgg gcagtgtggt gattttcaca
49321 acattgtgtc tgtcaatcca tgagcatggg atatttttct acttttttgt tgtctatgat
49381 ttttttcagc agtgtcttgt agttcatctc atagagatcc tttacctaat ggttaagtgt
49441 attcctaggt tgtttttgtt attgtcactg tttttgttgt tgttttgcaa ctattgtgaa
49501 gggatggagt tcttgaattg attctcagct tgcttgttgt tgatatcaaa cagtggtact
49561 gatttgtaca tattaatttt gtacctgaga tttaagtgaa ttcacttatc acatctacga
49621 gtcttggtgg aatctttccg gttttctaag cacatatgat cacatcattg gcaaacacag
49681 gtagtttcac ttccttcttt ccaatttaat tatactttat tccttttgct taccagattg
49741 ctctgacaaa cattttcagt cctatgttga ttgcaagtgg ataaagtgag catttttgtc
49801 tgcttgtagt tcctagcagg aatactttca acgtttcttc attcaatatg atgttgcatg
49861 tggatttatt tttggcttct attattttga tgtatgttct ttctaggcat agtttgtgta
49921 agcgtagtct attattttac aagctcagat ttgtattctg ttttacctga gtgcattgtg
49981 agatttggca tctattttac ctgatataag tacagctact cttgctgttt ttggtttcca
50041 gttgcatgga atatcttatt ctaccccatc actttccatc tacatgtatg cttataggtg
50101 aattgagttt ctggaaaaca gcatatagta gggtcgtatt tttttactca ttcaaagacc
50161 ctttgccttt cacttgcaga attcagataa attatattca ttgtttttat tgataaaagc
50221 ttagtgctcc catttcattc cttgtttttt ggttgtttag agacttctct cttccatcct
50281 tttcttattg tctttctttg tgtttaagta attttctctt ctggaatact tggaatgtga
50341 ctcttctggc cagaaacctc tgtggctggg ggcacctttg ccagagtttt gatggggttc
50401 actgggttcg ttctgcccat gcagcctggt agactatgct tggctcatgt ttcaggcctg
50461 gagcacatgc ctattaaggg cgggtcaggg ctggagtggt gaggggtgtg tgagtgagca
50521 gggggtctgg ccactttgga cggtcaccgg ctgctgctgc tgcagcagtg ggatgggcag
50581 ctccagatgc cagcatgtgt gccagatttc tgcaaggctg caaatgaatc aggcacagca
50641 caaacagctt ccatggttgt cactggaata cacagtgaca ccaacactga aagcttggaa
50701 atgccaggaa ctgcagaacc cccaaaaggg agtcacagcc ctggctcagg aagctcccac
50761 atctgggctc ctggaagagt agttgctctt ctctttttct cttcacctac aatttggtga
50821 gcaaggggca tgtttcagct ttatttgtgt tattgctctt ttaccccacc attaggcggg
50881 tctcaagttt ttgtcctgtg accaggaaga gtgaaatatg cagacaagtg gagggtgagt
50941 gagataaaga agagctttat tgagcaatag aacagcttag agacccacag tgggtaactt
51001 ctttctgcag ccagggcgtc ctgatgagtg tttagctctg agcagagagg aggccctggg
51061 gtgggtgacc cctccctcct ggcaggttat tccatcatca ccactgctct cagtagagag
51121 aaggccctgg agtgggttgc tgctctctgc aggaaagtca tctcatcatc tctacagctc
51181 tcagcagaga aaaggacccg gagggggttg cttgtctcta caggaaaatc atcccaacag
51241 tgggtagttc ctctctgcca ctggtcttcc taatgttctc cctgagtctg ggtttttttt
51301 gacatcagac aggagaaagt atgcactcat tgggtcatag gtggccatga gcaggcacag
51361 aaaaggcaac acatgttccc actctggtcc ataggactgg tggcccagcc cacgggcttc
51421 agaccctcct tgatcagaag gtggagcttc accagtgacc ctcaccttcc tgtccaggat
51481 tctgtctgct tcccaccacc aaccatggag cccaggtcac ttgtaccaag gagcatccaa
51541 agaccagtgc tgatcagtcc gcaacacccc tcagcctccc tcctacactc atcaaggccc
51601 aaagtccaga gggttcaaga cagcagtggg atggtgcatc agcactgacc cgagtgtgca
51661 cagacccacc tgggctgtga cagcatctgg gcttgaccac aaccacactc caaaattaga
51721 gcaggtgcca tgagagatga ggcagtgaga gcggacaccc ccaagctgca ggagaagggg
51781 ggatctcctg gaccctcgag agtactgggg gacctcattt ggtaactgtg acctggacaa
51841 cttcagttgc gtctttggag ctactgccct gccaactcag gaggaccagg actccctctt
51901 gtcccaggct cccatcagct ctgaagtgta cgcagccttg gctgtgccct ctctctgtgt
51961 ttccctgcag aagtgacagt tgagaagcag gtacacagca gctctggcca accccacaca
52021 aacaaaccca atgctcctgg gtgtggttta accagcccca actgcactat catccaggag
52081 cttgcaggct aacggcaggc agtgagcagt gaagtagagg ctgtggtgga gactccagac
52141 ctgggacaag gttccatttt gcaatgagag ggtgtgggtg gcacagtagg ctgcctcagg
52201 gaaatggagc acaggcctgg ctcatgaccc tgtcaagggg ggtgcctcca ggagtggttc
52261 atagttccca ggcccagcaa tcgggctggt cactcctatg gggggcggat ctcggaaaca
52321 cagcatgggg tggatccgca tagaccctcc cttcaagacc tgggagcttg acactgttag

52381 caggatgggc acagtggcca gatagctggc caggtccttg aaacaggtgc catttctgct
52441 tctcaccctg gccccctgat ggatggcccc agctatgcct tctgggcctg gcatccgcac
52501 atcttgtgcg aacgtggcac caccccattc ctatcttctc cttggggccc ctctctgccc
52561 gtcccttcgc gcctgaccga gctgctcccc ttgggcaaaa aagtaagaaa aaaactgatg
52621 actgaagaga agtaaagaat gggtggaaat catctgtatg cctgttttcc cagcgctttg
52681 ggaggccaag gtcagtggat cactcaaagc caggatcttg agaccaggct gatcaacatg
52741 gaaaaacctc atctccatta aaaatacaaa aatcagccag tcttggtgaa acgtgcctgc
52801 cgtcccagct aattgagtgg ttgaggcaca agaatcactt gagccctgaa ggaaaggatt
52861 gcaatgagcc cagattgcac cactgcactc cagcctaaat gacaaactga gattttgtct
52921 ccaaaacaaa acaaagaaca agaatgggtg ggaaatactt aaaatgatca aattttattt
52981 ggttgctttg atgttctaca gctgaaactc aatcacagac aaagtagtat ttcattattt
53041 ttccatcagt aactcaataa ctagatattt ctggtggata aattgctaca acacgttaaa
53101 agttttcatt caggtgctct ttatttctga tattccttgg taaccatcct tgcagggata
53161 acattctcat cactgtagaa ctttagcttc tctttccgac tctgtaggac acgggtccct
53221 gaagttctca ttgatgtcac ctcaacattt tcctccagcc ttgccccctg ctgttatgtt
53281 ttctctctca cactgagcac ttgcctgtgc ttcctttaag ttgcatgtgg cctggacaca
53341 gtcactcatg ccagtaatcc cagcacttta ggaagctgag gcaggaggat cccagagcaa
53401 cacagagaaa ccctgtctca aattgtcttt aataaaaatt ttggaattat taaaaaatga
53461 aataaataag aaaagagaaa aatagtttgc acctacatag tagattttag tgtccaagtg
53521 cctggaagag aactttggat ttctctaccc cactgggcac gccttcccta gcagcaaaga
53581 tggagctcca gttcctcaga cggtgatgag ccacaggaag ggcagggggt ggggccaatg
53641 aagatcctct tgggctgcct gaattccctc agtgtacaca tcagctcagc ccgaagtggg
53701 gtgaagatct cccaattgac acgaaccaag gaattcaaac tctcctcagg ggcaggatac
53761 gtctccaggc ttaacttgct cagcccactg gtgtggcgca gcaggtcctt cagggcgtca
53821 atagacatgc aattgctgcc aaagtagaag gtggtgagct gggagcagca gctcaggcca
53881 ggcaggatgg cactgagttg ggagtagtgg atctgacagc cctctaacac gagggtctcg
53941 agagaggcag caattttctc tagcagagct cctaggggtt caagactgat gcggaacagc
54001 agcacgtagc tgagattcag atgctttagg taaccgaggc ttgggaactg ggagagacac
54061 ttcaagtcct cttctaatag gttgccacaa gttaattcca agttctccaa ggggttctgg
54121 aggcacctgt ggagatcaag aagttagttc tgggcaatgg taccagttag atgaaggtag
54181 tggggaataa cctcaaggaa aatacctgct tcaaccaaac acaagtttgt tcccaccatc
54241 tgatgatggt cctcatggaa gttgctgcat gatgaggacc ctgatcgttc aggggctgtc
54301 ccattttagc ctcagccctt tcaccattgc ttgtgtgatt gggtcaaggc cataaaatct
54361 ctaaagcctt tttgtttttc atcttttagc agaaaacttc atctctgggc tacaggtacc
54421 ccgtgggaga tgtgaacaaa gaactcaact cagcaaggtc tagggacatc agctagggct
54481 acatgtcggc aggggctccc tgacatgcct gcatctgcaa accaactgtc actttttacc
54541 actctcacgc ctactccctc acctccatcc cggaagcacg catttcccat gtcaattacc
54601 tttcctggag ttcaaaacaa cgttttgcag acagggaatt aaagacagga tcattcgtga
54661 tcactaagcc ggtgaggaca gacgttctat tgtgaaatgg acaggtttga tgcgctttcc
54721 ctcctttcat accctcctct attatctctt tgacatcata tcaacttgaa acacactttg
54781 taacaggaaa ttcacacgtg cacccccaat agagctgaaa cccccactga ctagcttgta
54841 catgatgtcc ctctctagct tctaccccag gtgacccctc tgcccttatt ggagcgatcc
54901 tgtgatagcc actccaggac atggaacact gaatgggaca atgtgttgac attctggtgt
54961 tcccttcact gtgacgttgc cactggctgg cacacagtac acgccttcta atgtttgctg
55021 taacagaaca agtctgtgct gtggtctgca tacaaagtgc acaatccttt ctcacctgat
55081 cagctgttcc aggtgcccac tgaagaaggt gatcaatttt attttaagca actggaggtg
55141 ttccagcctg aggaacacag aggtgaatct ggtgactaac catccctcga gttcattatc
55201 tgacgtgtaa tgatggcacc tggagaaaac gagtttgcaa agagtcttca tctccttcag
55261 gtaacaataa agctttctta tcagatgtgg ccagcacgtg ttgtgaattt ccagctcccc
55321 aatactatta atgtatatta ttttcaatga ctttctgaga tatttaattg gcgttagata
55381 attgaccagc ttactacagc acaggtgtac taaacctctc ctttggtaaa cccactggaa
55441 gaggtatctc aggcattcat cctggggtat ttccttgagg cagatgtcta tgaacacctt
55501 taagggctgg tgctctcccg tccttggaca gtcctctgct gtctgcctct tactcatggc
55561 ctctgggaag caggacaggg cccaggctcc aggccatctg gcccagaaat tctcatcaac
55621 atcccgcaaa tccagcactt gaagtttcca cctcctgtgg gtaaagtaag ggagaggctc
55681 agaatttaga gggactcatc cctgaccttt gctttcattc tcatcccata aatcagctgc
55741 tcctgtcctc agtgctccct gttctctttg tcttttctca atccctttc cctttggatt
55801 ctgagtggtc cccacttcta ttccctttac cttccactga gtaaaggccg gtttctgttc
55861 ccacagtgga ccctgtatgg tgagcagtcc tttctctgag gatctggaca atggccaaag
55921 cctccctgag cttcctcgcc aacaccatca gaagactctg ggccgcactt gggttacttc
55981 tctgcctgac cctgctgttc tttccctgga cacctgagcc ctatctacta gccctcctgg
56041 gtcacctcac ctggggcgat ccttctgtgt aagcagcata tgaagccctt ccagcaatgc
56101 tttcaatggc tccagatgaa gcgtcttcat cagcgatacc agagggaggc aggtgaaagg

56161 ccaggcctgc accatcaccg tcagagtctg gaagtgtctc ctgctgaagg cctccctgaa
56221 gagtgggaga tagagcaccc tgggcagctc ctccatggca gagatggaca aggcctggtc
56281 tctcagcagg ctctgccccg ccagctccag tagtctcggt ggggcctgga tgctcatcct
56341 gatggatctg caagaaaaat ctctagaaga caaatccagg gagaatgtat cactctcagg
56401 gcaaacacaa tcacctcatc ttctcctagg accaatatca ttgctctggt agaggtagaa
56461 aaattaccac tttaccccaa ttccactgtg cttgttggcc tcaaatctat acttctgctt
56521 ctgctggtac caggaagaat atcttccaaa caccaaggag ggaggggtca aagagaccat
56581 tgacccatta attttcatcc ttcgctccac tgaatcccag aaccactgga cagtgccact
56641 gaggatcctg aaagccaagc tctacctctt tgaggaaaaa tttcttgtca cttaccaccc
56701 taaagcattg agaatgagag tgtcccgtgg gcccagacag cctccattct cagttcacac
56761 catgaacatg ctgggggaac actaaaggga ctccctaaaa tcgatgccat tatttttat
56821 tttgaaaatt ttctaccaga aatggaccag gtgctgtggc ttatgtctgt aatcccaaca
56881 ctgctggaca ccaaggcagg cagatcactt gaggtcagga gttcgagaac agcctgggcc
56941 tacataatga aaccatgtct ctactaaata tacaaaagtt aagaatcatt tgactccaga
57001 aggcagaggt ttcagtgagc cgacatcaca ccactgcact ccagcctggg tgacagcgtt
57061 ggactctgac tcaaaaataa acaaattgat aaattaatta atttaaatat tagccaggtg
57121 tggtcatgca tgactgtaat cctagctact ctggaggcag aggaaggaga atcacttgaa
57181 gcccagagac agagtttcca gggagcccag ctcagggccc tgcactccag tctgggtgac
57241 gcactcagag tacatcgcag aaaaaaacaa aataattcac tggaactgta aaagtggtgt
57301 gatggtattc cacagcattt ggaaggtatg tatagaaatg ctaactgtag ctgggcatgc
57361 tggcttactc ctgtaatccc agcactttgg gagtctgagg ggggcagatc tcctgaggtc
57421 aggagtttga ggccagcatg gccaacatgg caaaaccctg tgtctactaa aaatacaaaa
57481 attagctggg catggtggtg agtgcctgta atccaagcta ctcaggaggc tgaagcagga
57541 gaatcacatg taactaggag gcagaaattt cagtgagcca aaccacacca tggcactcca
57601 gcctgggcaa caatagggaa actccatctc aaaaaaaaaa tcaaccagaa actgtaaaag
57661 tgctaccatg ctattctaga gcactgtaac tctaagatga aggttcctat agacatcact
57721 tccacatact cacaattacc cactttttga tggatcgatc ctagggtcaa aaataaatcc
57781 cacaatctga gcaaaactgc actcttgaga ttggtgtgtg ggataccttt aaggatttta
57841 tgaaaatgaa agcatacttg gaggatcaca ataacaccaa gtctatgaac tgtaattgaa
57901 aggcacaaaa acaaataact tcaaatgtca agaaataaaa attcatgtca ctgtaaagtt
57961 ttaatatatt tttaaaaaac ctgcttccat aagaatttta aaatgacaaa aaccaagcac
58021 aaatcacaat ctgatggatg aagacaaaac tacatttaga ggaaaaatga aagcctaaat
58081 ctgttcatct cacaaaacag acagaaaaat attgtgtgcc actttgggat gtgtgtcacc
58141 gtccctgact ggctggctgc tgatcagatg ggcatgaccc taagaaggtg gtgacttacc
58201 agcgctggac tcactttgca gagttctggg acctctcagg gaaccaacca gtagcttcag
58261 gtgtgagtgc tgtgggtctc ttctgggtac cctcaggagc ttttatagac ctttctaacc
58321 ccacccttcc cttctcaatc accagcttcc aatcagaaag tgatacctga ttagatcttg
58381 cagtcacacc cagttaatcc tgattgagtt ttcagctttc ttctgactaa ttgatcgaat
58441 tagatacaca tttatggaag taaaagaata aataataggg tgaaagtcca aaactcattc
58501 attcatttat tccccaaata ctgatgaagt ttggctaata gatgactttc atagtgatac
58561 agggaaggga ttaatctgtt cctgatatca gacaaaaaaa aaaaaaaaaa ccttaaggtg
58621 tccttattgg aggatgtttg gccacatcaa aattgtcaaa atgtttcaga gctacaacag
58681 cctgaagaag atagtgatgt cattcccaag aaaacagaat aaaaagctgt gtatatcgaa
58741 tggttacctg tgttttatgc tatctaacat agcagatcat atgcacattc aggtagaaga
58801 aaggaaccac tgagggtgtg atctatctca agactaagtc aaggcttcac tgaaggaaat
58861 caggacaaaa tgaccaagtg aggtgcggac tgagtggaat gagactaggt tttctaatgg
58921 gaacctgcaa aggaaacaag acaatgtaaa acatggtggt tatcttgtgg gcatctagat
58981 gtcaggactg aaagtccttt gtcaagattg agtttattta ttgattcttt gattttcaga
59041 ctgggccacc atctgtcacc caggctggag tgcagtggca tgatttcagc tcactgcaac
59101 ctcaactttc tgggttcaag cgattttctc accttgcctc ccgagtagct gggaattatg
59161 ggtgcgctcc aacatgcccc actaattttt gtatttttag ttgagatggg gtttcaccat
59221 gttggcccgg ctggtctcga actcttgacc tcaagtgatc tccccacctc agcctcccaa
59281 agtgctagga ttacaggcat cagccatctc acacacccta gattgagttc agaaattcaa
59341 aggagaatca tcaaaagaga taggtcagac tcttaaccat aacattggct ttgagaacac
59401 agggggcagg tatagtcttg gccctactag aaggtaaagg gtgtttaccc acaaaaatga
59461 tgggctcctc tcagaaaacc agcttgcaaa gatggaatct gagaatgtga gctggagcag
59521 aggccagaga gaagatcggg gccacacttg ggaagggaaa gcaagcaagc tcaggcctcc
59581 aatcccagcc ctttgggaag ccaagaaagg cagagtgcta gagctcagga atttgtgagg
59641 agtatgggca atgtgatgaa accctgtctc taatacaaat acaagatatt agccaggggg
59701 aggcagtgtg cacctgtagg ccaagctgcc caagaagttg aggtgggagg atcacctgag
59761 cccagtgagg cttctacttc ccacgcccca ctttgtaaac ctgaggctga gggtgagctc
59821 agcaccaata atggttgtga gaatctgtgt tcactgagca tccacgaggc acaacagatg
59881 gctggtaccg atcatcccgg acctcagctc ttcctcatgg agaatctaag gcacgttgct

59941 attttcccca tttctaacct gataaacctg agacttggcc agagaaaaat ctgcccatgt
60001 tctggcagca aatgattggc aaacaggcat gagccaccac ggtcagccag aaaaaagtac
60061 ttaataaatt accagctaac taaatgcaac actgcatcag aaagtgatag acaggccagg
60121 ctccgtggct catgcctgca atcccagcac tttgagaggc tgaggcaggt ggatcacctc
60181 aggtctggag ttcgagacca gcctgaccaa catggagaaa ccccatccct actaaaaata
60241 caaaattagc caggtgtggt ggcgcatgcc tgtaatccca gcaactcagg aggctgaggc
60301 tggagattcg cttgaaccaa gaaggtggag gttgcagtga gtcgagatcg tgccattgca
60361 cttcggcttg ggcaacaaga gtgaaactcc agctcaaaaa aaaaaaaaaa agaaagaaag
60421 aaaacaatat agtaatatat aatggccatt ccaggaatgc cagccaatca cagaaaaatc
60481 taagtgtaat tcagcatact gacaaactaa aggggaaaaa gcaaggttcc tacaaaatgc
60541 agaaaggaat tgaagaaaaa tcaaattaaa tttatcatag catattttgg aaaatgagat
60601 gttgtatgtt gaaaacttgc attaaacatc agatgtaatg gataaacatt agctcccttc
60661 ctagtgagat atgaaacaag gtaagaccct cagcaactta ggatttgaag gcacgtaggt
60721 attttggtta gtagtaaaga ctccagatcc agcagatcca gactgtttaa tttaggttca
60781 aaactggctc agtaccatcc tggctaacac ggtgaatccc catctctact aaaaatacaa
60841 aaattagcca gacatggtgg caggcacctg tagtcccagc tactcgggag gctgaggcag
60901 gagaatggag tgaacccggg aggcagagct tgcagtgagc cgagatcgcg ccactgcact
60961 ccagcctggg cgacagcacg agactccatc ccagaaaaaa aaaactggct cagtggccaa
61021 tggctgtgtg gtcttaccca acttacttaa cctctctgtg ccttagctca ttcacatata
61081 aaatgggata atagcaatat tgacttcaca gagtgttata agttaatcta tttaagtact
61141 tcgagctgga tttgacatag ggcaagcaag gatattttta ttgttattat atttgaaaaa
61201 tatattagtt acaaacttag gtgagagacc aaggtctatg gtaaaggtga ttataagcct
61261 tcatacaccc ctgttgttct gaaaatctta attataacaa ggcccggtgg agtggctcac
61321 gtctgtaatc ccagcacttt gggaggctga ggtgggcgga tcacctgagg tgaggtgggc
61381 ggatcacctg aggtcaggag ttcgagacca gcctcaccaa agtggcaaaa ctatctctct
61441 attaaaatac aaaaaatagc tgggcatggt ggtggacacc tgtaatccca ggtactcggt
61501 aggctgaggc aggagaatca cttgaatcca ggaggcagag tttgcagtga accaagatgg
61561 aactattgct ctccagcctg gcgacggagt gagactccat ctccaaaaaa aaataattaa
61621 ttataacagc atgtccattc actctccaaa gtgtctagga ctggacaatt aattgtcagg
61681 ccctcttctg tagcacccta cactatagca tatatgtgga ttaatataaa tacacataca
61741 aatttcaagt atatattcca tatactttct atatacttat tttctaagag gtcacatgca
61801 aattcaaggc tatgtcaaag agtagagtgg ctatctatgg aaaggggagt ggaagtgaat
61861 catggtaata aaaaataggt gtagatatag atatgaatat gtagacatac acacatatag
61921 cggcaagaaa agggaatgtc atggaccaat gatgacagtg agccatgtaa aaaggctaca
61981 atttttgtga ttgtgtgtcc attttcagga tgggttgtag cttacctttt tagaaaggct
62041 gatgccatag tcatagtgaa taaatgatta taaaatgtgt ttcctttctg gtgcacctct
62101 ggagaaactt gacacagagt ctctgatgcc caagctggag tacagtggtg ctatctcggc
62161 tcactgcaac ctctgcctcc tgggttcaag taattctcat gcctcagcct cccaagtagc
62221 tgggattaca ggcatgccac cacacccagc taatttttgt atttttagta gataatgggg
62281 tttcaccatg ttgcccaggc tggtcaactc ctggcttcaa gtgttccgcc agcctcagcc
62341 tcccaaagtg ctgagattac aggtgtgagc caccacgccc agtaagactg gcttacttta
62401 atataatttt taccacccct atgggaaaat acagccagac ccatcataag gtattttttt
62461 tcaccaacta caatcagaag caccgatcat aaagtattta ctggagaacc tatgcctttg
62521 ataatagaac ctcatgtatc ccctgcacat tttagctctg actgtgtaac caggggttca
62581 actccaacag attaaccatt gcttaagttg caaagcctaa attgctcagg catgtccgat
62641 gggaaaaagg tttaacctct taactgttaa cacagcctgg ctgactgttt gaattggcat
62701 cacctgaaac cagttggaaa ggccatgtga gcttgctcca ctatccccag attggggaga
62761 caggcttgga acttgtctcc tgtctccctg tcagttaact atttttattt tttattttta
62821 tttttatttt tttctttgag acagagtctt gctctgttgc ccaggctgga gtgcagttgc
62881 gtgatctcag ctcactgcaa acttggcctt ccgggttcaa gtgattcctg cctcagcctc
62941 cacagtagat gggattacag gcgtgcacca cgatgcctga ctaatttttg tatttttagt
63001 agagagggag tttcaccata ttggtcatcc tggtctggaa ctcctgacct tgtgatctgc
63061 ccgcctcggc ctcctaaagt gctgggatta caggcatgag ccattgtgcc cggcccagtt
63121 aactcctaat aattttttat tttttcccaa aatggagtct tgccctgtca cccaggctgg
63181 actgcagagg tgcaatctca gctcactgca acctctgcct cccaggatca tgccattctt
63241 ctgactcagc ctccagagta gctgggatta caggcatgga tcaccacacc cagctaattt
63301 gtgtattttt agtagagaca gggtttcccc atgttgtcca ggcggtctca aacccctgac
63361 cttgtgatct gactccctcg gcctcctaaa gtgctggtat tataggcatg agccaccgtg
63421 cccagccttt tttgcttttc tcaaaccatg gtggtgtagt attgggttct atgcacttag
63481 gagagtgagc tcatcattca gtaacaatat gactcagtac cacgagacct ttcaaagact
63541 atttccagta ggtgaaggag gcttttaatg atgattggac cttcatgccc taccatttgg
63601 agattgtgtc ttttaaaatg gccctaaggg aaatctgccc atgagcagca ttggatgaga
63661 ccataccagg tgacttaaaa ttaaggataa ccaaggaaaa aagcctttct tacaagcaga

63721 catcatcaca tggtagacag ctgttttaag aaaatggaac aaaactccat tctatctcct
63781 tccattgact gagacttggt tttgttttgt actaacacaa aatgatcaag cctacatttt
63841 attttgttac gtactttcac cagtcaaagc aaacactttc taaggtctcc tcttcaaaat
63901 ttagccacta tcactaacca aagcaattgc tgcctatgga gtcatttaga tgaataggaa
63961 ggatcacaac taatagtaga acctgctctc acacacggtt gggtaatatt tatgattaaa
64021 tgactcggca ctgagaagaa gctatagatg caaatgggtg gcctatgact attattgatt
64081 tcattactgg taacttatct ctatgcatag gaaacattag tgtaactggg tctaatctag
64141 gtggtgtccc agactcttgt ggctcaggct ggagtgcaat ggcacaatct cagcccactg
64201 caacctccac ctcccgggtt caagtgattc tactgtctca gcctcccaag tagcagggat
64261 tacaggcatg tgccactaca gccagctaat tttgtatttt tagtagagac atggtttctc
64321 tgtgttggtc aggctggtct ccaacaccca acctcaggtt atcctccggc cttggcctcc
64381 caaagtgata ggattacagg cacgagccac cgaacctgga tagagaacat ttctgatggc
64441 tccatattga tggaacttca aataactctc tggtaaatta ttttctataa tacccttaaa
64501 taaaaaatgg agaagctgag atccagatca atgtagactt tggtacagag ttggtggtac
64561 cagggctgcc atatccagtt tacaccccaa cctgatatat cagcaaatct cctggagtaa
64621 ggaaaatatt tctgaggagg ggtttcacat gaaattcaga aaattcctgt gtttgaagca
64681 gtccaaatga cattttgtgg caacaaaata tatggtctac ttaacagaga aggagactgt
64741 gtaagaaaaa aaaaaaaaag atgtttatcc tgaaatgagc aaagcattgg gaatggatgt
64801 gagattattt tgggagaaaa atagaagatg aaggttttga aaggaaaaat aaggaggatt
64861 atataaattg ttttgaaagg ctcatccttg gtcctaataa tcaaaaccaa aggggcatca
64921 gtgaaatatt ggatagattc ctcctccacc ccctcagtaa cccccaacat gtttaccaag
64981 tcttggttca ctcccaggat cccattaaaa caccaagctc aaccaagccc atcctctacc
65041 ctcacttcct tttgcaattt tgacatgatt ttattacagg accatcagat tcctatgcct
65101 gctgcacagt agcttaccaa tattttgaga cagcagagtt tgcagcagag agtttaatga
65161 tcacagggtg gctaaatgag aagctaggag gagatcctca aattcatctc cccaaggagg
65221 agatccactg agggtttcca gtggatcctg gatagcaagg ggctggaaag ttgatataga
65281 gttaagaggg atgaagtcat caggatgtca aaactgcatt ctttggtgcg ttggtgcctt
65341 gtagggccct tcagatcagc tggcatcagt actttcactg acatgcagaa cgtaagaata
65401 tctcaaatga aaaagttaat gttttacaag gcttaaattg ttgtctgcag ggcagttaag
65461 gggaactgta atctaaggtc taaatgattt ggggacagta ggctgccagc aaccatgagg
65521 aagaaggtca gagagcaagc tgacctcatg gtgaatgctg aatgcgctgc aagcttggtt
65581 tatttttgtt tctccccctc ccttcttcac tgattaaatt tataaagttt ataggtatcg
65641 tttcaatttc ttccaaagaa gccttaacct aagcactgag accactcacg ccctgagtgg
65701 cacctctctt ccaccagcac gagtgaagaa actgctacct taggtgatat aaaacccaca
65761 agaccattcc atacatggag atctttattc ttattttgta gggatgactc cgtttttata
65821 aagttgtttt aactagaaag catttttata attttgatgt ggccaaagat ctccccacaa
65881 cactactttc aggttttatt tttctgtcta atgtccggaa cagatcaacc ccttccctgc
65941 ctcacactca ggacctgaag gcgacatagc agtaaaattc catcagtgtt tgtggatttc
66001 atgaatgaat gcattttttt tttgacaaaa tctccctccg tcacccagtc aggagggcaa
66061 tggtgtaatc ttggctcact gcaaactcag cctccagggt tcaagggatt ctcccacctc
66121 agccacatga gtagctggac tacaggcagc caccatcgtg cctggctaac ttttctattt
66181 ttgtagagac agggtttcac catgtgggcc aggcgggtct tgaactcctg acatcaggtg
66241 atccacctac cttggcctcc taaagtgctg ggattacagg tgtgagccac ctcatctggc
66301 cttgagtgaa tgaattcttg acttccactc tatccctaac gctgtcaatt tcttaattca
66361 tgaaatgaat atgcatatgt gatatgaatg gatacctggt tcaatccatt agtcttcgga
66421 cagccaaaaa cccaatcagg attaactgag tggagcttca gaaatgcaat cagatatcgc
66481 tttttgattg gaagctagca gcggatatgt ggaggggcgt gggtgggagt tgtgattaga
66541 aaggtcaata aaagcttcta aagacccaca ggacagactc aaagtcttca agcctggagt
66601 tcctgcttgg ctcttcctga ggtctgagct acaactacaa ccagaggtct aaactacaac
66661 cagatctggt aagtcactaa tttctggaag gacactccca tctgacctac agtcagccgg
66721 tctagaacgg tgacagtgca gcctacgacg gcacagaact atatcctgtc tttttttctt
66781 tttttcatat gaacactttg aagctttgat tttttttcta aatgcaattt tgtctttatt
66841 tcaaaaatgt tgttgtgctt ttctttacat catttcagaa ttcttgttgg gagccatttt
66901 gtgaagagac gaagactgag ctggtttggc tgcatttctg gcctcgagcc gcagtcagtt
66961 tctccccgta gaacccggca gtaggagact tagaatcgaa tctcttctcc ctcccgcctc
67021 ctgtttttgg ctttttgaga aaccttatca tccaacacaa tggccagcaa cgttaccaac
67081 aagatggatc ctcactccat gaactcccgt gtgttcattg ggaatctcaa cactcttgtt
67141 gtcaagaaat cggatgtgga ggcgatcttt tccaagtatg gcaaaattgc gggctgctct
67201 gttcataagg gctttgcctt cgttcaatat gataaggaga aaaatgcccg ggctgctgta
67261 gcaggagagg atggcagaat gattgctagc caggttgtag atattaacct ggctgcagag
67321 ccaaaagtga accgaggaaa cgcaggtgtg aaacgatcag cagcggagat gtacggctcc
67381 tcttttgact tggactatgg ctttcaacgg gattattatg atggaatgta cagtttccca
67441 gcacgtgtac ctcctcctcc tcccattgct ctggctgtag tgccctcgaa acgtcaacgt

67501 ctatcaggaa acacctcacg aaggggcaaa agtggcttca attctaagag tggaaagcgg
67561 ggatcttcca agtctggaaa gttgaaaggt gatgaccttc aggccattaa gcaggagttg
67621 acccagataa aacagaaagt ggattctctc ctggaaaacc tggaaaaaat tgaaaaggaa
67681 cagagcaaac aagaggtaga ggtgaaaaat gctaagtcag aagaggagca gagcagtagc
67741 tccatgaaga aagatgagac tcatgtgaag atggagtctg aggggggtgc agaagactct
67801 gctgaggagg gggacccact ggatgatgat gttaatgaag atcaggggga tgaccagctg
67861 gagttgatca aggatgatga aaaagaggct gaggaaggag aggatgacag agacagcacc
67921 aatggccagg atgactctta agcacatagt ggggttgaga aatcttatcc cattgtttct
67981 ttacctagga gcttgtctaa caacaaattt ttcaccagat cctctccctt agtatcttca
68041 gcacatgctt actgttctcc ccatctttgt ccttcccatg ttcattaatt catattgccc
68101 tgtgcctagt cccattttca cttcccttga cacccctagt agttgtccta agtcttaccc
68161 tgtaattttt ccttttaatt ttgacacctc tttatgactt aacagtaaaa aggatgtatg
68221 gtttttatca actgtctcca aaataatttc ttattttgtt tatttagttt atttatttgt
68281 tagtttagtt tagttttgtt tatttgttat ttagtttact gagaatgatg ttttccaatt
68341 tcatccatgt ccctacaaag gacatgaact catcattttt tatggctgca tagtattcca
68401 tggtgtatat gtgccacatt ttcttaatcc agtctatcat tgttggacat ttgggttggt
68461 tccaagtctt tcctattgtc aataatgccg caataaacat acatgtgcat gtgtctttat
68521 agcagcatga tttatagtcc tttgggtata tacccagtaa tgggatggct gggtcaaatg
68581 gtatttccag ttctagatcc ctgaggaatc gccacactga cttccacaat ggttgaacta
68641 gtttacagtc ccaccaacag tgtaaaagtg ttcctatttc tccacatcct cttcagcacc
68701 tgttgtttcc tgacttttta atgattgcca ttctaactgg tgtgagatgg tatctcattg
68761 tggttttgat ttgcatttct ctgatggcca gtgatgatga gcattttttc atgtgttttt
68821 tggctgcata aatgtcttct tttaagaagt gtctgttcat gtcctttgcc cacttttga
68881 tggggttgtt tgtttttttc ttgtaaattt gtttgagttc attgtagatt ctggatatta
68941 gccctttgtc agatgagtag gttgtgaaaa ttttctccca ttttgtaggt tgcctgttca
69001 ctctgatggt agtttctttt gctgtgcaga agctctttag tttaattaga tcccatttgt
69061 caattttgtc ttttgttgca agttggaatg cttttataag aaaggttgaa agaaggggat
69121 gggactcctc ctatcagttg tttttttttt aatttctttt tgttttatcc tggcaaggct
69181 ctaatagagt tgagcttttt tgtttttgtt ttttccattg gaagggacaa tacagaggtt
69241 acaatcattg actttagatg acaacatgac aggcaaaaat attttccttg caagacaacc
69301 agcaaaactt cacgatcaga atcaaatcag tgtccttctc actgtcactg ggtgaagcct
69361 tcatcagtac ttgaagagtc tcaggcactc atgaagtcaa gatcagattc tttactcagg
69421 gacaggatgt aagccaatca taagaccttc cacagatgat caatttggaa gtctacctaa
69481 tgtgacctgc aggttttcac tggcaatatg caggtgcaga tatgacaaag aataatcctg
69541 accttcatat cacccctagc tgttgaggat tgggattctt ttgtcccttt ctctccataa
69601 aaccaggcta ctcatcttgt gtggcaacaa aatatatggt ctacttaaca gagaaagaga
69661 ctccacagga aaaaaaaaaa aggattttta ttatgaaatg agcaaagcca tgggaataga
69721 tgtgagatta ttcggggagg taaaggaaga caaaggtttt gaaaggaaaa atgaggagaa
69781 ttacataaat ttttttgaaa gactcattct tggtcacaag tatgaaaacc aagggaccct
69841 cagtgcaatg ttggaaagat tcctcctcca ccctctcaat aacccccaac acatttacca
69901 agtcttcgtt cactcccagg attccattaa aacacccagc tcaaccctga ccagcctcca
69961 ttttcacttc cctttataat tttgacataa atttgttaca ggaccatcag gttcctatgc
70021 ctgctgcaca gtagcttacc aatattctga gacagcaggg tttgcagcag aaagtttaat
70081 gatcacaggg tggctgaatg agaagctggg aggagatcct caaattcatc tccccaagga
70141 gtactgaggg tttccagtgg atcctggata gcaaggggct ggaaagttgg tgtagtttga
70201 tggcaataag aggtatgaag ttatcacgat gtcagaacgg cattctttgg tgagttgatg
70261 ccttgcaggg cccttcagat cagctggcat cagtagtttc actgacatgc agaacctgaa
70321 agaatatctc agatgaaaaa gtgaatgttt cacaatgctt aaattgttgt ctgcagcgta
70381 gttaagggga attgtagtct aagttctaca tgattttggg acagtaggct gccaggaacc
70441 atgaggaagc aggtcagagg gcaagctgac ctcctggtga atgctgaatg cgctgcaagc
70501 ttggtttgtt tttgttcctc cccctctctt tttcactgat taaatttata aagtttagag
70561 atacggtttc aatttcttcc agacaagcct taacctaagc cctgagacca ctcaagccct
70621 cagtggcacc tctcttccac cagcgtgagc aaataaattg ctaccttagg tgatatagaa
70681 cccacaagac cattcgatac atggagattt tttttctgat tttgtaggga tgacttctct
70741 gtttttataa agctgtttta actataaaac aattttataa ttttaatgtg gccaaagatc
70801 tcccaacact actttcaggt tttatttttc tgtctactat ccggaacaga tcaaaccctt
70861 ccctgcctca aactcaggac tatataggtc atatatcagt aaaattccat cagtgtttgt
70921 ggagtttatg aatgaatgaa ttctttttat ttttattttt ttgacaaagt ctacctctgt
70981 cacccagaac ggtgtgcaat ggtgcaatct tggctcactg caaccattgc ctccgggatt
71041 caagcgattc tcccacctca gcctcctgag tagctggatt acaggcacct gccatcatgc
71101 caggctaatt tttgtgtttt tgtagaaatg gggtttcacc atgttggcct tgcaggtctt
71161 gatcgcctga cctcaggcga tccagccaac ttggactctc aaagtgcagg gattacaggt
71221 gtgacccacc tcacccggcc tttaatgagt gaattcttga tttccaacct attcctaaca

71281 ctgtcaattt cttgattcat gaacttaata tggatagctg atatgaatga atatctgatt
71341 caatccagta atctggggac agccaaaaac ccaatcaaga ttaactgggt ggagcttcag
71401 aaatgcaatc agatagcact ttttgattgg aagcaagcac tggatacatg gaggggtgtg
71461 ggtgggagtt gtgattagaa agatcaataa aagtttctga agacccacag gagagaccca
71521 aagtcttcaa gtctggagtt cctgcttggt tcttcctgag gtctaagcat gctgcaaact
71581 cagtccagat ctagtaagtc actcatttct ggaaggacac tcccatctga cctatagtca
71641 gtcggtctgg gacagggaca gtgcagccta tgatggcacc aagctatatc tgtcttttat
71701 atatatatat gaacaatttg aagatttgaa ttttttcctc tatatgcagt tttgtcttta
71761 tttcaaaaaa ttggattgtg ctttggttta tgtcatttca aaattcttga tgagagcaat
71821 gactcatgcc cataacctca acaccttggg aggccaaagc gggaggatca tttgagctca
71881 gcggtttgag accaacctgg gcaacatggc aaaaacgcag ctctacaaaa cattcttttt
71941 ttgaagcagg gatggagcct caccctgttg ccaaggctgg agtgcagtgg caagagctca
72001 actcactgca acctcttcct cccaggctca aacaattttc atgcctcagc ttcctgagta
72061 gctggtatta catccctctg ccaccatgcc tgcttaattt ttgtattttt agtagtggtg
72121 gggtttcacc atgctggcca ggttggtctc aaactcctga catcaagtga tcctcctgcc
72181 tcagcccccc aaagtgctgg aatgacagcc ttgagccaat ggtggtcagc ctctacaaaa
72241 tatatatatc ttttaatttg ccaggcatgg tagcatgcgt ctgtattccc agctatttgg
72301 tttgctgaca tgggagaatc acttgagccc agaagattga ggctgcagtg agccatgctc
72361 acaccactgc tgtactccag cctgggcaac agagtaagac cctgctaaaa acaaaaacaa
72421 aacaaaaaca aacaaacaaa aaaccttaac caaagagaat ctttgacctt aattttaaac
72481 caatcacatc ctcactgtaa ttcttcctcc cgaatggaga catgggtgtg gggtgcatg
72541 cctgtaatcc cagctacgtg gaaggttgaa gcatgagaat tgcttgaatc tcagaggcgg
72601 aagttacagt gagctgagat ggagccgctg cactccagcc tgggcgacaa agtgagattc
72661 agcttcaccc acaccaaaaa aaatgagatt ataccaccca ggtgatcatt ggatacatga
72721 agatttctat tgtgttttct tggggactgt caactccgtc tttgaaaact gttttaactc
72781 tgaaaaattt tgataaattt gatgtggcca aggatccctc aacaaagata ctttcaagtt
72841 ttctttcttt ctatctaata tcaggaagag attcaaccct tccctatctc acactcagga
72901 ctatgaagga cacatattag taaacctcca tgtttgtgga gggaatcagt gaatgagtcc
72961 tggactttca ccctatccct aaatctttca ctctgatgga tgaatatcta attcaatcag
73021 ttaatctgga agaaagccaa aaatccaatc aggattaact gggtagagtt taagaagtcg
73081 aatcaaatgt agttctctct cttttctttt tttttttttc tttgaatcta gcctatttcc
73141 caggctggag tgcagtggag tattgtcagc tcactgcgac ctctgcctcc tgggttcaag
73201 cgagcctcct gcatcagcct ccctagtagc ttggattata ggcacagaca actgcacctt
73261 gctaattttt ctaattttgg tagaggtagg gttttaccat gttggccagg ctggtctcaa
73321 actcctgacc tcggataatc cacctgcctc tgcctcccag agtgctggga ttacaggtgt
73381 gagtcactgc acacagccaa agtggttcat tttgaatatg tgtgagaggt gtgtattgga
73441 aacatctgtg tcttgccaat gatgcataac actgtcacat agctttcaaa gcttctcgct
73501 gaaattttca ataatgagac caggcagagg ctcatgcctg caatcccagc actttgggag
73561 gccaaagtgg gtggattgtt tgagtctagg agttcaagac cagcctggac aacatagtga
73621 aacccactgt ctttacaaaa agtcaaaaaa taaaagatta gctgggtacg agatctgagc
73681 ttcagagatc ctcggtaaca tttgccagtg ctatgagttt agtggatcag tggctaataa
73741 ttcatggact acgagggatc ttgcctgctc tttagaagtt gggacacatt cttcatggta
73801 ccagaagggt agaactgtgt ctctgtggcc acttattgca gaaaggaact ggagtaaact
73861 aagggctctt tcacacacgc tagagaaatg actttggccc tgggagaatt gggggttgca
73921 ggggattggt ctgaggaact tgcctttttg ctggattgtg ctgtagagtt tttccttgta
73981 gatttgtcag aatgagcctc cagaccccat ccagactcct ggagctggca gggcagagcc
74041 tgctgaggaa ccagttcttg actatcttca ccctggatga gctgcccagg gaggtctccc
74101 ctctgttcat ggaggccttc agcatgagat gttgtgaggc cctgaagctg atggtgcagg
74161 cctggccctc ccttcacctc cctctgggat ccctgatgaa gacacctcat ctggagacct
74221 tgcaagctgt gctgaaggga cttgatacac tagtgtccca gaaggtttgc cccagttgag
74281 gtgactcagg tggcctggtg ggaagggtcc agacatccag ggaagggaca gctggctcag
74341 gaggagtggc agtgtgtggg agctagggtg gctcagaggc ttctgatggt gcccatgaga
74401 agccttggcc attgcccaga tcctctggaa aaggactgct taccatacag ggtccactga
74461 ggaaacagga acctgcttcc tcccagtgga aggtaaaggt actagaagtg ggtaccatgc
74521 agaatccaag ggggaacagg atggagaaaa gacagaagga ggagcactgg gacaggagca
74581 gctgactcat gtcctggatg cggagtgaaa gttcaggtca agggtcggtc cttgcctaca
74641 ttctgagctt ttcccttatg ttactcacag gaggtggaaa cttcaagtgc tggatttgag
74701 ggatgttgat gagaatttct ggaccatatg gtctggagtc agggtcctcc cctgctcccc
74761 agaggccatg agtaagaggc agacagtgga gaactgtcca aggatgggag agtgccagcc
74821 cttgaaggtg ttcatagacc tctgcctgaa ggaaagtaca ctgcatgaat gcctgagcta
74881 cctctgaagg tggatccact acagaagagg cctagtgcac ctgtgttgta gtaaggtgca
74941 gaattactca atgcccactt caagtttcag aaatctattg gaaaggatat acccagatag
75001 tatccaggag ttggaagtct ggaaaaagtg ctctctcaat aaaacggaaa agtttgcccc

75061 ttacctgagc cagatgagca atctttgcaa actttttagc cttcggttat gagcctgaat
75121 tatacgtgag cggccaatgg cagttcattc ctgacttgga ttggccattc ctgtgcctgt
75181 actaccccca gatgcattat ataagaaagg tcagtaatat caaagagcac ctggagcacc
75241 tgctcaggta ggaaacgatg gtgggctttc tgtgcagacc ataacacaga cttttgttct
75301 ttttcacagt aaatgttagt gggcatctac tgtgtgccat ccactgagga tgtcacaggg
75361 aatgggatgc tagaatgtca actcattaag ctgttcagtg ctctatatcc tgaagttggt
75421 atcacaagac cactcaaata agggcagagg gatggcctgg ggtagatgct acagagagag
75481 atgtataggg agctagttag ttggggattc acatctagtg aaggggcatt tgtgaattcc
75541 tccttaggaa gtgtgtataa agttaatatg atgaaaacgt attcttcata aagaagaggg
75601 tatgaaagaa gggaaagtgc agtctggtgt gatgtctcat gcctgtaatc ccaggacttt
75661 ggaaggctga ggcaggcaga tcacgaggtc aggagtttca gaccaccctg accaacacag
75721 tgaatcccta tctctattaa aaatacaaaa tttagctgag catggtgcca ggcacctgta
75781 atcccagcta cttgggaggc tgaggcaagg gaagtgaagg ctgcagtgag ctgagattgt
75841 gccactgtaa tccagcctaa gttacaatgt gagactctgt gtcaaaaaaa aaaaaagaaa
75901 agaaaagaaa aagaagaagg gaaagtgcat gaaacctgtg catttcacag tagaagctcc
75961 gtcctcagca gcttaataaa caccaatgat cctgtctgta attccttgtc tgtgaaaggt
76021 tgttttgaac tccaggaaag gtaattgata cgggaagtgc atgcttctgg gatggagggt
76081 gagggagtag gtgtgagagt ggtaccaatc acacaagcga ggctgaaagg actgagccta
76141 aaatggagct gcccctgaat gatctgagtc ttcatcaggc agcaccttgc atgcagacca
76201 tcatctggtg atgggaacaa acttgtgttt gggtgaaaca ggcttcccca ttgcagttac
76261 tataacacct gtgtggtagt aaggtgcaga attactcaat gcccacttca agtttaccat
76321 tgagatgatt tcccaccccc ctcctctaac tggcaccatt gcccataact aacttcttgc
76381 tctccccagg tacctcaaga accccttggg ggcctttaca ttcagtgatg cttacctagc
76441 tgatcgggac atggagtgtc tgtctcagta cccaagcctc agtcagctaa aggagctgcg
76501 tctgattcat atcctaatgt ggaccaccca tcttgagccc cttggagttc tgctggagaa
76561 agttgctgct actctcaaga ccctcgtctt agaggactgt cggatccagg cccccaact
76621 cagggtcccc ctgcctgccc tgagccactg ttcccagctc accaccttct actttcatgg
76681 aaatgagacc tccacgaatg ctctgaaaga cctgctgcgt cacacaggcg ggctgagcaa
76741 gttaggcctg gacatggatc ctgcccctct ggagtgtctt gacaacaggg gtcatgccaa
76801 ttgggagatc ctcaccccaa ttagggctga gctgatgcgt acactcaggg aagtcaggca
76861 gaacaggagg atcttctttt tttttttttt tttttttttt tttgagacgg agtctcgctc
76921 tgtcgcccag gctggagtgc agtggcacaa tctcggctca ctgcaagctc cgcctcccgg
76981 gttcacgcca ttctcctgcc tcagcctccc aagtagctgg gactacaggc gcccgccact
77041 acgcccggct aattttttgt atttttagta gagacggggt ttcaccgttt tagccgggat
77101 ggtctcgatc tcctgacctc gtgatccgcc cgccttggcc tcaggaggat cttcttcggt
77161 cccaccccct gcccttcctg tggctcatgg ccatctgaga aactggagtt ccatgtgctc
77221 ctagggaagg cctggttagt gggatggata agctttcttc cggacacttg ggaactaaaa
77281 tcttgtacat gggtgcattt ttaattttaa tttaatttaa ttttttatat ttttatttta
77341 ttttatttta tttttgagac agagtctgac tctgtccctc aggatgaagt gtagtggcac
77401 aatctcagct catggcaagc tttacctcct gggttcaagt cattctcctg cctcagcctc
77461 ccaagtaact ggtgttatgg gtgtgggccc caagcccggc taatttttgt atttttagta
77521 gagacagggt ttcacgatgt tggcggaggc tggcctctaa ctcctaacct caagtgatct
77581 gattgccttg gccttccaca gttccaggtt tacaagtgtg tgcagctggg cccggtcagc
77641 cgtttcttaa aggaagcaca cagctgtgtg tttcaggtac gtgctcaccg cgagtggaaa
77701 aacaaaggtg actcagccgg gggcaggact gggtaaaaat gctgacttgg aatcaatgag
77761 gccttcaggg acctgtgttc tagactcaga aatggagcct gaagttctag actgatgcag
77821 gagttgccac cgcaaggatg gttatttaaa aatgtcaaaa ataaatggaa cctgaatgga
77881 aactttctgg tgtcttccac gattgatcaa cttgttttag acatttatgt atcagaagtc
77941 tctagaaatc accctcctgg gttcaagcaa ctctcctacc tcagcctcct gagtagctgg
78001 gactacaggg gaccgccacc acacctggct aatttttgta ttttaagtag aaagggggtt
78061 tcaccatgtt ggccagtctg gtctcgaacc cctgacctca ggaaaaccac ttgcctcagc
78121 ctcccaaagt gcttggattg tagacatgag ccactgcacc tggcctgata ttctgataca
78181 agcataaaat gtgtaatatt caaatcatgg taactgggat atccaccatc tcaagaattt
78241 atcttttctt tctgctagca acattccaat tccattattt taattatgta gaaatttact
78301 acgaactatt gtcaacatga gttgccctat tgtgctacta aaccctagat cttattcctg
78361 tctgtgtttt tgttcccatt aaccatcccc ttcttattct ctatttccca gtacccttcc
78421 tagcttctga taaccatcat tttactattt ttaagtttca tgttttttaa ttcccaaata
78481 tgagtgcgaa catgccatgt ttgtctttct gtatctggct tacttcactc aacataacgc
78541 cctccagttc cttccatgtt gtcgcagata actggatttc cttcatgttt acggctcagt
78601 gatattctgt tgtgtatatt taccacattt tcttgatcca ttcatctgct gatggacact
78661 tacgttgatt ccaaattttg gctattgtgg atagtgctgc aataaatagg agagtgcagg
78721 ctgagtgcag tggctcatgc cagtaatccc agaatttcgg gaggctgagg caggtggatt
78781 acttgaggtc aggagttcaa gaccagcctg accaacatgg tagtgtagat atctcctgga

78841 tatattactt tttttttttc tggatatatg tccagcagtg ggatttatgg tttatatggt
78901 aatcctattt ttattttttg gaggaagctc cattctgctt tccatagtag ctgtactaat
78961 ttacataact accaatgttg taccagggtt ctcatttctc catattcttc atagcatcca
79021 ttattttctg ttggtttttt atgggggaac tccccttact attaaaactc aaatccagtt
79081 tggtgtaaac acagaaaccc tgctagagtt gcctgccacc tttgaaacag gccattggaa
79141 tgaaaattgt ccacctatgc accaggtctc tattggacag aatgttttgt cccacaggtt
79201 gttcacatta gaggagattt cttttttgtt tttctttact tttctgcctt tttttttttt
79261 ttttttgagg tagagtttca ctattgttgc ccaggctgga gtgcaatggt gggatctcag
79321 ctcactgcaa cctccacctc ctgagttcaa gtgattctcc tgcctcagct tcccaagtag
79381 ctgggattac attcatgcac caccacagct ggctaatttt gtatttttag tagagacagg
79441 gtttctccat gttggttagg ctggtctcga actcccaacc tcaggttatt cccccacctc
79501 agcctcccaa agtgctggga ttacaggcat gagccaccac atctggctag aggacatttc
79561 tgatgtctcc atattgatgg aatttaaaat aactctctgg taaattgttt tctataatag
79621 ccttaaataa aaatggagaa gctgagatta agatcattgc agacttaggt acagaattgg
79681 tggaaaccag ggctgccata tccagtttac agccaatata tcagcaaatc ccttggagaa
79741 aggaaaatat ttctgaggag gggtttcaca tgaagttcag aaaattcctg tgtttgaagc
79801 attccaaatg acatttggac catttttagg aaagtatggc tttttattaa gtgacaacat
79861 ggggatgaga tttgctttct ccgttaaggt gatgcgtaaa gctttctttg gagggagaga
79921 aaaccctaga gtttcctgac cttccttaac ctgagctgct tggttcccta gaagcagaaa
79981 ttgatcatat tagaacccaa actcatacca actttgacct tcatgaagta ctcaagtgtt
80041 tctgctcttc ttcctcatgt gatgtagaaa gtattaaaag tgatgagttt aggccaggca
80101 ctaactggtt cacgcctgta atctcagcac tttcagaggc cgaggtgggt gcatcacctg
80161 tggtcaggag ttccagacca gcctgggcaa catggtgaaa ctctctctct actaaaaata
80221 caaaaactag ctgtgtgtgg tggtctgtgc ctgtaattcc agctaactgg gagactgagg
80281 caggagaatc acttgaacca ggaggcagag gttgcagtga gccgagatcg caccattgta
80341 ctccagcctg gaaaacaaga atgaaactct atctcaaaaa aaaattaata aataaataca
80401 ttataaataa ataaattaat taatgcttta aagaaaaaag aaataaactt tgcctacaag
80461 tttcatatgc aattgaatac ctcttaaatt ttgatgtgaa tcgaccaggc atggtggctg
80521 aggcctgtaa tcccagcact ttgggaggcc gaggcgggca gaccacgaag tcaggagatt
80581 gagaccatcc tagttaacat ggtgaaaccc cgtctttact aaaaatacaa aaaattagcc
80641 aggtgtagtg gcatgcacct gtagtcctgg ctatttagga ggctaaggca ggaaaattgc
80701 ttgaaccggg gaggcagagg tcgaagtgag ctgagatcgt gccactgcat tccagcctgg
80761 tgacacagtg agactccatc tcaaaaaata aatgaataaa ataaataaat caataaaaat
80821 attgtgacag gaaccaacat tgctcaactt gtacactaat gtcttacaaa atcctttcct
80881 tgtcaccttc aaatctccat ttcaaatgct acactctgca taactctacc actttgttgc
80941 cattttctga tggtggagaa gaccatacat gtgtgtgtgt ggcatcagaa ctattgactc
81001 ctcctattga tgtttaagat attccattac acaaacctgg gttcatactt tttgttgata
81061 gatcttatgc caaaaatgta ggcaaaaaat gccaagcagg aaatgctatc acttctgaag
81121 atgaattcat acagatggaa attctttcag aatttatttt tccagctttt ttctttgttt
81181 gttcgtttgt gtttgtttgt tttgagacag tctcgctctg tcaccaagtt ggagtgcagt
81241 ggtgaaatct tggctgactg caacctcctc ctcctgagtt caagcgactc tcctgcctca
81301 gtctctcgag tagctagtac tatgggtggg cgccaccatg ctcagctaat tttgtatttt
81361 ttagcaaaga cagggtttca ccatgttggc taggatggtc tcaatttttt ggcatcgtga
81421 tctacctgcc ttggcctcct gaagtgctgg gattagaggt gtgagccacc accatgcccg
81481 gccttttttt tttttttcct tttgagatgg agtctcactc tattgcccag gctgggaatg
81541 ggattcctcc tatcaattct ttttttaaat tttcttttgt tttattgacc tgacaaggct
81601 caaatagagt tgagtttttg tttttgtttt ttccattgga agggacaata cagaggttac
81661 aatcattggc tttagatgac acgataaaag aataaaacat attccttgca agacaaccag
81721 caaaacttca tgatcagcat caaatcagtg ccttctcact gtcagtgggt ggaagccttc
81781 atcaatactt gtagagtttg aagcactcat gaactcacaa tcagactctt tactcaggga
81841 caggatgtaa gccaatcgaa agaccttcca caggtggtga atttgggagc ctgcccaatg
81901 tgacctgcaa gttttcactg gcaatatgca ggtgcagata tgacaaagaa taaccatgac
81961 ctttacatca cccccagctg ttgaggaatg ggatcctttt gaccctttct gtccatagaa
82021 ccatgttact catcttgagt ggcaacaaaa tatatggtct acttaacaga gaagaagact
82081 ctgtaaaaaa aaatgtttat tatgaagtaa gcaaagaagt ggcaatagat gcgagattat
82141 ttggggagat aaaggaagtt gaaggttttg aaaggaaaaa taaggaggat tatacaaatt
82201 gttttgaaag actcatactt ggtcataagg attaaaacca aaagggcatc agtgcaatgt
82261 tagatagatt cctcttacac ccactcaata acccccaaca tgttcagcaa gtcttgcttc
82321 actcccaggt tcccattaaa aacccagctc aaccctgacc agctccaccc tcacttccat
82381 ttgtaatttt gacatgactt tattacagga ccatcaggtt cctatgcctg ctgcacagta
82441 gcttagcaat attctgagac agcagggttt gcagcagaga gtttaatgat cacaaggtgg
82501 ctgaatgaga agctaggagg agatcctcaa attcatctcc ccaaggagta ctgaaggttt
82561 ccagtggatc ctggatagca aggggccgga aagttggggt agcggtaaga gggaagaagt

82621 caacaggatg tagaaactgc attctttggt gagttggtgc attgcatggc ccttcagatc
82681 agctggcatc agcagtttca ctgacatgca gaacctgaaa gaatatctca gatgaaaaaa
82741 ttaatgtttt acaatgcttg aatcgttgtc tgcagggaag ttaaggggaa ctgtaatcta
82801 aggtctatat gattttggaa cagtaggctg ccagcaacca cgaggaacca ggtcagagag
82861 caagaagacc tcctgatgaa ggctgaatgt gttgcaagct tcgtttattt ttgtttctct
82921 ccctcccttc ttcactgatt aaatttataa actttagaga tgtggtttca atttcttcca
82981 aagaagcctt aacctaagcc ctgagaccac tcacgccctc agtggcacct ctcctccacc
83041 agaacgagca tgtaatctgc taccttaggt tacacaaaat cccaaaaacc attcaataca
83101 ttgagatttt tattctgatt tcgtagggat gactcctctg tttttataaa gctttttaaa
83161 gtagaaagca tttttatatt ttgatgtggc caaagatctc ctaacaacac tactttcaga
83221 ttttattttt ctgtctaatg tcgtaacaga tcaaatccgt ccctgcctca cactcaagac
83281 tatgaagttc acatattagt aaaatgccat cagtgtttgt ggagttcatg aatgaatgat
83341 tttttatttt tttgacagaa tctccctctg tcacccagac tggagtgcag tggcacaatt
83401 ctggctcact gcaaccattg cctcctgggt tcaagcaatt ctcctgcctc agcctcccat
83461 gtagctgggt ttcaggtgcc tgccatcatg cccagctaat ttttgtattt ttgtattttt
83521 gtagagacag ggtttcacct ttttgacctg gctcgtcttg aacccctgac atcaggtgat
83581 ctactcacct tgtccttcca aagtgctgga attacagcta tgagccacct cacccaccct
83641 tgaatgaatg tattcttgac ttctacccta tccctaacac tgtcaatttc ttgcttcacg
83701 aactgaatat agatatgtga tatgaacgga tatctgactc aatccattaa tctggggaga
83761 gccaaaaacc caatcaggat taactgggtg gagcttcaca aatgcaatca gatattgctt
83821 tttgattgga agctagcagt gcaaacgtgg aagggcatgg gtgggagttg tgattagaaa
83881 ggtcaataaa atcttctaaa gacccacagg agagacccaa agtcttcaag cctggagttc
83941 ctgcttggtt cttcctgagg tctgagcacc ttctagacta catccagatc tggtaagtca
84001 ctaatttctg taaggacact cccatctgac ctacagtcag tcagtctggg atggtgacag
84061 tgcagcctac gatggcacag agctatatcc tgtccttttt ttttctcata tgaacaattt
84121 gaagctttga attttttcct ctaaatgcag ttctgtcttt atttcaaaaa agttgattgt
84181 gctttggttg atgtcatttc aaaattcttg aagggagcag tgactcatgc ctttaacccc
84241 aacacattgg gaggccaaag tgggaggatc atttcagccc aggggtttga gaccaacctg
84301 ggcaacatga caaaagccct cctctacaca acgttttttt tttttttgag ggtggggatg
84361 gagtctcact gtgttgccca gactggagtg cagtggcatg atctcaactc actgcaacct
84421 ttacctcccg ggttcaagca attctcatgc ctcagtctcc atcctcagaa gctggtgtca
84481 cagacatctg aaaccatgcc tcgctaattt ttgtattttt agtagaggtg gggtttcacc
84541 atgctggcca ggttggtctc gaacacctga cctcaagtga tccacctgcc ttggcctccc
84601 aaagtgctgg gattacagct gtgagtcact ggtgcttggc ctctactttt tttttttta
84661 attagccgag catggtgaca tgcatctgta gtcccagcta tttgggtggc tggtgtggga
84721 gaatcacttg agcccagaag attgaggctg cagtgagcca tgctcatacc actcctgtac
84781 tccagcctgg gcaaaagaga gacaccctgt ccaaaaaaca aaaacaaaat caatcaaaaa
84841 ggatctttga ccttaatttt aaaccaatca catcctcttc cacccaaatg gagacatggc
84901 tgtggggggt gcatgcctgt agtcccagct acgtggaagg ctgaagcatg agaattgctt
84961 gaatcttgga ggccgaggta acagtgagcc gatatgacac cactgcactc tagcctggcc
85021 gatgaagtga gattcagctc cctcaacacc aaaaagaatt acgccaccta ggtgatcttt
85081 ggatatatga agatttctac tgtgttttct tagggactgt catctctgtc tttgaaaact
85141 gttttaactc tgaaatattt tgataaattt gacatggcca aggatccctc aacaaagata
85201 ctttcaagtt ttctttcttt ctgtctaata tcaggaagag gttcaaccct tccctatctc
85261 acactcagga ctttgaagga cacatattag taaaactcca tgtttgtgaa gggaatcagt
85321 gaatgagtcc tggactttca ccctatccct aaatctttca ttttgatgga tgaatatcta
85381 attcgatcag ttaatattta agaaagtcaa aaatccaatc aggattaact gggtagagat
85441 taagaattct aatcaaatgt agctctctct gtctctctgt tcaatctagc ctatttccca
85501 ggctggagtg gagtggtata atgtcagctc actgcaactt ctgcctcctg ggttcaagtg
85561 atcctcctac ctcagcctcc ctagtagctt ggactacagg cggagaccac tgcatctcac
85621 tgaaattttc aataatgagg ctggggcgga ggctcacacc tataatccca ctatgttggg
85681 aggccaagag gggtagattg cttgagacta ggagttcaag accagcttgg acaacatagc
85741 gaaatccact gtctttacaa aaagtcaaaa aataaaagat gagctgggtg tggtgatgca
85801 taactgtggt cccagctact tgagaggctg aggaggaaga atcctttgag ctgggaggtc
85861 aaggctgcac tgagctgaga tcccaccact acactccagg ctgggtgaca gcaagaccct
85921 gtcagaaaga gagtgagaga gggagagaga gaaagagaga gagaatgaga gaagggaggc
85981 agggaaagaa gacaagaaag aaagaaggga gagagaggag gaaagaaaga agggagggag
86041 agagggaaag aaggaaaaaa gaaagagaga aagagaaagc aagcttaaat aatgaaaaga
86101 aaacaaatag aacctgttct agggatgccc catgaatgtt cccaacaagc ttatttttag
86161 gaactgaaat tgtgggcatg taggcttgtg acactccctt tcccattgtt ttagaacctt
86221 aagtaattaa taatttcccc caatggtagg aggggttcac tttcaggttc ctccacactc
86281 actagtcact ggatggagca ctggatagaa aggaagggct agtggtggcc ctgcttcctc
86341 actgcttcgg agacgctcat ggtgatgcag cagaggcaga atgctggctt aatggccact

86401 gagtacagag tagaattgga gtaaactgag ggctgtttca ccattgccag agcagtgact
86461 ttggccctgg gagaagatga gattgcatgg gcttggcctg agagtgatgc cttttctctg
86521 ggtttgtcct ctggaagttt tccctgcaga ttcatgaaga tgagcatccg gattccaccc
86581 agactcctgg agcttgcggg cggagcctgc tgaggaccca agccttggcc gtctccaccc
86641 tggaggagct gcccacggaa cttttccccc cactgttcat ggaggccttc agcaggagac
86701 gctgtgaggc cctgaagctg atggtgcagg cctggccctt ccgccgcctc cctctgaggc
86761 ctctgataaa gatgccttgt ctggaggcct tccaagctgt gctcgatggg cttgatgcac
86821 tgcttaccca aggggttcgt cccaggtgag gtggcccagg tgggctggtg gggagggccc
86881 aggtgtccaa ctgaaggaac agctgggtca tgtgaagtga ggaggcccaa gggggatggt
86941 ggtggtgagg aagccgagag gacttggcca ttcaccagct cctcagggaa agcactgctc
87001 accaggcaag gtccatagag gtaacaggaa cctctcctct aatggcactg aaaggcacca
87061 tgaaaagtga gaactgggcc gggcacggtg gctcacaatg taatcccagc acattgggag
87121 gctgaggtca agagttggag gccagcctgt ccaacatggt aaaccccaac tctactacaa
87181 atactaaaat tagctgggca tggtggtggg ttcctgtaat cccagctact tgtgaggttg
87241 aggcaggaga atcatttgaa cccgggaaga agaggttgca gtgaggtgac atcacaccac
87301 tgcactctag cctgggcgac agaaggagac ttggtctcaa aaaaaaaaac aaaaaaatgt
87361 ggaagtgggt aggatccaag gggaaaacag ggtgaagaaa actcagagag agggacaaca
87421 agcagggagg ggaggagctg ctatgcagga tgtggagttt aagttcagaa atgagttctt
87481 aaattctcag tctcacctct attttcccac aggagatgga aacttcaagt gctggattta
87541 caggatgtct gtgagaactt ctggatggtt tggtctgaag ctatggccca tgggtgcttc
87601 ctcaatgcca agaggaacaa aacaccagtg caggactgtc caaggatgag agaacggcag
87661 cccttgactg tgtttgtaga actttggctc aagaacagga ctctggatga atacctcacc
87721 tgcctccttc tatgggtcaa gcagaggaga gatttactac acctgtgctg taagaagctg
87781 aaaattttgg gaatgccctt ccgcaatatc agaagcatcc tgaaaatggt gaacctagac
87841 tgtatccagg aggtggaagt gaattgcaag tggatactgc ccatcctgac acagtttacc
87901 ccatacctgg gccacttgag gaatcttcag aagctcgttc tctcccacat ggatgtctct
87961 cgctacgttt ccccagagca gaagaaggag attgttaccc agttcaccac tcagttcctc
88021 aagctgcgct gcctccaaaa gctttatatg aactctgttt ctttcctcga aggccacctg
88081 gaccagctgc tcaggtgagg gagggtggtg agctttctct gcagaccaca gcagagcctg
88141 ttacagtaaa cactagtggg catctactgt gagccagcct atgaggatgt aacagtgaag
88201 gggacactag aatgtccatg cattgtcctg ttggcggccc tgtcctgaaa tgggtatcat
88261 gcaaccctcc caatagagtc agagggatca gtcaggggag atgctataga gaggctgcca
88321 tgctaggaag ctagctactg gggggttcag atctagtgag ggtgcctttc tgaattcttc
88381 ctgaggatgt gtgtctaagt taagatgatg aaaaataggc caggggcgtt ggctcatgcc
88441 tgtaatccta gcagtttggg agtctgaggc aagaggatag cttgagccta ggagtttaag
88501 agcagtctgg gtaacatacc aagacccctg tcagaaatga ataaataaaa gtaaaatcaa
88561 acaagataaa ttttttttct gagatggatt ttcactttga tcatccaggc tagagtgcag
88621 ttgtgacatc tcagctcgca gcaacttctg cctcccaggt tcaagcgatt ctcctgcctc
88681 agcctcctga gtacctggga ttacaggcgt gggccaccac acctggctaa tttttatatt
88741 ttaagtagag acaggttttc accatgttgg ccaggttatt ctccaacccc tgatttcagg
88801 tgatccaccc accttggact cccaaagtgc tgggattata ggcgagagct accacgccca
88861 gccaacaaga taatttttaa gaagatgatg tgaagtaggg aagtgaagtg ggcactgaag
88921 aggggaatgc tcagcaaacc tgcacatgtc agaaaatcag ctttgtgccc cacagtttgg
88981 tgaacatgaa tgatcccatc tctaattccc attgtaaaag tttcttttga gctccaggta
89041 aattaattac ctaggaaatg tatgattctg aaacagaggg tcagggagca ggcacaaaga
89101 atgatgaaag tgatagatgg tttgctgatg atacaggtgt gtcagggacg cctgcagcct
89161 gcccacccca gctgatgttg caggatcctg tctgggtttg tcctttatgc ctgcatctcc
89221 actgggcttc tgtggcccag ggatgtggtt ttctgcctga cagatgagga aagggagctt
89281 tagggattct gtgaacttga tccattccta taaatgatgg tgaaatgact cagcctgaaa
89341 tggaattatt ttttctcctt tttttttttt ttaatagagt atcactctgt cacccaggct
89401 ggagtgtagt ggcatgatct ctgctcactg caacctacac ctcctgggtt caagcgattc
89461 ttctgcctca gcttcccaag tagctggaat tgcaggctcc cgccaccaca cctggctaat
89521 ttttggattt ttagtagaga cgaggttttg ccatgttcag caggctggtc tcaaactcct
89581 gatctcaagg aatccaccag tctcagcctc ccaaagttct gggattacag gtgtgagtta
89641 ctgggccggg cctaaagtgg aattgacctc ggtggcaaag ctcttcatca cacatcatcc
89701 gaagtgttga ccatccggcc atgagaatga tcctggactt gggcaaaatg gtctccatcc
89761 attaccttga agccattccc caccaccctc cactcacccc tatgattccc cagaattaac
89821 ttcttgctct ctctccccag ctgtctgaag acctcgttaa aggtcctcac aataactaac
89881 tgtgtgcttt tggaatcaga cttgaagcat ctctcccagt gcccgagtat cagtcaacta
89941 aagaccctgg acctgagtgg catcagactg accaattaca gtcttgtgcc tctccaaatt
90001 ctcctagaaa aagttgcagc cacccttgag tacctggatt tagatgactg tggcatcata
90061 gactcccaag tcaacgccat cctgcctgcc ctgagccgct gctttgagct caacaccttc
90121 agcttctgtg gaaatcccat ctgcatggcc accctggaga acctgctgag ccacacaatc

90181 atactcaaaa acttatgcct ggagctgtat cctgccccgc aggaaagtta tggtgctgat
90241 ggtactctct gctggagcag atttgctcaa attagggctg agctgatgaa gaaagtgagg
90301 cacttaaggc accccaagag gatcttgttc tgtactgaca actgccctga ccatggcgac
90361 aggtcatttt atgacctgga ggcagatcaa tactgctgtt gaatgcctgc ctatttggat
90421 gggtatgtca aacgctttct tctggacact tggaaactaa aacctaggtc ttaggtacat
90481 cctaaaggga gcacagaacc catcgtttca cacatgggct ctgaaagtgg gaaaggaaag
90541 ctgatcaagc aggggcagga cttgggggaa atgttgccat ggattcgatg ggactttggg
90601 aacctgtatc ctgtagagtc gaaaatggga atctgaatgt ctagagtgga attcaggctt
90661 gagaatacat gagggagtta ctcttgcatg gatggttgta aagaaacaat cagaaataaa
90721 ggaaaactga gcagaatctg tctggtgccc tccattatta agtaacctgt tttccagttt
90781 aagcctcagg aatcttcagt tattgatgga aaaaacaaaa ggcactgact gagttgtcca
90841 atcaataaga tgcagcccaa gaaaatcaag gcatttaaat gaaatttggt tattgtaacc
90901 agtttcctcc cattctttta tttgagacag agtttcactc ttgtcgccca ggctggagtt
90961 tagagtgcaa tggtgccatc tcagctgact gcaacctcca cctggggttt aaatgattct
91021 cctgcctcag cctcccaagt agctgggatt acaagcatgc accaccatgc ccagctaatt
91081 tgtgtatgtt tagtagagac agggtttcct cactatgttg gccaggctgg tctcaaactc
91141 ctgactttgg gtgattcacg caagtaggcc taccaaagtg ctggggttac aggtgtgagc
91201 cactgtgtca ggcttttgtt tttgtttttg tttttaaag gtctcctgtc actcaggcta
91261 cagtgcagtg gcacaatcat acctcattgc agcctcaatt tcctgggttc aagcgatctt
91321 cccacctcag cctcctgagt agctaggact acagctgtgt gagccaccac acctggatac
91381 ttttttttta gtagaaacaa ggcctcgctg tcttccccag gctgatctgg aactcctgag
91441 cttgtgattc tcctgccttg gcctcccaaa atgcagggag tataggcgtg gaccaccacg
91501 cttggcttgg cctcctccag ttcttcactt ctttagatgt ctgttaactc cttgttagtt
91561 tctgtggctg ttcagtgggt taatacacac taggtggaca ccaaaggcct ggaacattgc
91621 tgggcaagaa cagtgagcca atccacacgg aaagcacctt cttctcagcg tctttcaccg
91681 ctagccagat gctgagaccc tgcccactcc ctgtgagtct ccacatgctt ccagaagcct
91741 tagttggtgg atgtcagctt cactgcacaa ggagccagtc tcttcccgct gccctggaag
91801 gggatgtcca tattgtatat tagctggaga ctctgggcag caccaaccct tgcttgttcc
91861 cctgatgacc agcagccctt cttgaattaa acttgttgta gccagtaaag acagccacat
91921 tccctttaag taaaatacta aaactataca ggcatgtaac actttttaaa tatttccatc
91981 tgacatttta aaagttacat ctttttgggg agctaggtca gattgatgag agattttctc
92041 ataacacctt ccctctctcc ctatgaagga agtgactagt gcagcatgtt ctggaatctg
92101 acatcatcaa agggtggata acgatcaagt gcctgtgggt gatgagtgac cttccctgtg
92161 gtgaggaagc ctgcatagtg ggcacccaag tgaaggatcc tgctgtgtac tcaggggctg
92221 gtgttgctgt cagggatgtt agcctagagc ctcagcttcc tgtaaaatga ggatgatgat
92281 atccaacagc ttatgggacc ttggtaggat ccaatgagat ggttcatgtt tagggcttgg
92341 catggggtct ggcatacagt aagatcaata catcttgttc ttttttctct tctcagcaga
92401 agtcccagca tttttcatct ttcaatctca cctccttttc ctgataatag agaggcaaca
92461 agaactcagg gcatgcaatg gggctcaact tctactctct gccacaattt catcatgatt
92521 cccccaaaga gcagagcccc aggagccagc aggggcaggg gtgggcattt ctggactgga
92581 ttcattcata ataagatcaa aatttccaat ccgtatgtct cgggtgccat ctgctgatag
92641 atcggaccag atggtataat tgagtgttgc aaggattata ttttatggtg tttttaaaaa
92701 tgtactatta tgagccaggt gcagtggctc atgcctgtaa ttccagcact ttgggaggct
92761 gaggcaggtg gatcacctga ggtcgggagt ttgagaccag cctgagcaac atgaagaaac
92821 cccttctcta cttaaaatac aaaaaaatag ccaggcgtgg tggcacacgt ctgtaattgc
92881 agctactcga taggctgagg cgggagaatc gtttgaacct gggaggtgga ggttgcggtg
92941 agctcagact gagccattgc actccagcct gggcaacagt agcaaaactc cgtctcaaaa
93001 aaaagataaa ataacattta ttattatggc cgggcatggt gtctcacacc tctaatccca
93061 gcactttggg aggccgaggc agcctcggga tgttgagacc agccttgcta acatggtgaa
93121 accccgtctc tactaaaaat acacaaaatt tgctgggagt ggtggcattc gcctgtaatc
93181 ctaggtattc aggaggctga ggcaggacaa tcacttgaac ccaggaggag aagattgcaa
93241 tgagacgaga tcgcgccact gctctctagc ctgggcgaca gagcatgaaa aaaaaattta
93301 ctatagtgtg aatactatta gagtataact atttgtgttg taatttatgt gtatgaaaga
93361 ttagaacttt gaaagaatgc aacgtgatat tttaagaatg gttaatggcc aggtgtggtg
93421 gttcatgcct gtattcctgg cactttggga ggccgaggtg ggtagatcac gaggtcagga
93481 gttccagacc agcctgtcca acatgatgaa acccggtctc tacgaaaaat acaaaaaatt
93541 agcctggcgt ggtgacaggt gcctgtaatc ccagatagtc aggaggctga ggcaagagaa
93601 tcgcttgaac ctgggaggca aaggttgcag tgagccgaga atgcaccact gcactccagc
93661 ctgggtgaaa gaggaagact ccgtcccaag gagggtgaga aaaagaatac ttaacttggt
93721 ttgaaatgtc aaaacaaatg agattttgaa aactaatttt aaagacactg aacaataatc
93781 atttcttctt taaaatatat ttagaacaat acaattttat ctttgaaagg aaacattaca
93841 gtttttaaaa atcttgagtt tatttcattt tattttattt tgagacaagg tctcactctg
93901 tcgtccagat tggagtgcag tggcatgatc acggctcact gcagccttga cctcctaggc

93961 tcaggtgatc tccctgcctc agtccccctg gtagctggaa cgacaggcat gcaccatcat
94021 gcctggctta tttttgccag gtttcaccat gttgcccaga ctggtcttga aattctgggc
94081 tcaagcgatc cacctgcctc ggcctcctaa attgctggga gtgagccctt ataggcatga
94141 gccaccgcac ccagccttga gtttatttat ttatttattt tggagatgga gtctcattct
94201 gtcatccagg ctggagtgca gtggtacgat ctcagttcac tgcaacctct gcctccaggg
94261 ttcgagcaat tctcctgtgt cagcctccag agtagctggg attacaggca tgcaccacca
94321 cacctggata atttttgtat tataattatt attatttttt aatagagaca gggtttgtca
94381 ttttagtcag gctgatctcg aacccctgac ctcaggtgat ccacccacct cagcttccca
94441 aaatgctacg actatagacg tgagccacca cgcccagcct atttttttct ttatagcagc
94501 tttagattca cagaaaaact aagcagaaac tgcagagttc tcatctacct tcttccccct
94561 tcaatacaca gcacccccac aggatcagca cccacaccag cacagagcat tcgtcacaat
94621 caatgagcca cagggacaca tcattatcac ccaatgtcca tagttcacat gggggatcat
94681 tgctggtttt gtacattcta tggattttaa caaaggataa atgacacata tccaccatta
94741 gagcatcatg gagactagtt ttgttcccct aaaagtcctc tgtcctcttc ccattcatcc
94801 cattgtactc ccaacccctc acaaccactg ggctttctac tatctccata gaaaaaggca
94861 aatgtctaac aggatgagtc ttttcacatt gccttctttc acttgtacaa taacgtgcat
94921 ttaggaatct ttcatgtctt tttaaggctt cataatagtt cactgactgg atggatcagt
94981 ttgcttatcc agtcactgac tgaaggccaa cttgctagct tccaggtttt agtgattatg
95041 aataagctgc tataaacatc caggtgtggg tttactcatt tcattaaata tccaggagca
95101 tgattacgga attgtagggg tatggtatgt tttacaatga tttcttcttt cttgacaatc
95161 tcacttgttc gatattgctg ctaaaggtca ggaactttgt ctcaatcatc ctatgttcct
95221 actgctgagc atggaacgtg gcacttggta gcaaatgctg ttgaccacgt gatgcatgga
95281 aacgtttatc atgggtatag tcactaaatt gctaccttgg ggacatcaac attagctcac
95341 taccaataat ataaataaat tggattatgg aaaaaatggc ccttgtgata ctgtggatac
95401 tgtatgtgta tcatgaaagt ccagcaattg acaaggcaca gtggctcaca tctgtaatcc
95461 cagcactttc agagactaag gtgggtggat cacttcagtc aggagttcga gacgactctg
95521 gccaatatga tgaaaccctt tctctattaa agacacaaaa attaactagg gggttgagcc
95581 aagatggccg aataggaaca gctccagtct acagctccca gcatgaacag tgcagaagac
95641 gggtgatttc tgcatttcca actgaggtac caggttcaac tcaatggaga gtgtcagaaa
95701 gtgggtgcag gacagtgggt gtagtgcatc gagcatgagc caaagcaggg caaggcattg
95761 cctctcctgg gaagggcaag gggtcaggga attccctttc ttagtcaaag aaaggggtga
95821 cagacggcac ctggaaattc aggtcactcc caccctaata ctgcactttt ccaatggtct
95881 tagcaaatgg cacaccagga gattttatcc catgcctggc tcagagggtc ctatgcccat
95941 ggagcctcac tcattgctag cagagcagtc tgagatcaaa ctgcaaggcg gcagcaaggc
96001 tgggggaggg gcgcctgcca ttgccgaggc ttgagtacgt agacaaagca gccaggtggc
96061 tggaactggg tggagcccac tgcagctcaa ggaggcctgc ctgcctctgt agactccact
96121 tctgggggcc gggcattgcc aaacaaaagg cagcagaatc ctctgcagac ttaaatgtcc
96181 ctgtctgtct gacagctttg aagagagtag tggttctccc agcatgcagc tggagatctg
96241 agaacggaca gattgcctcc tcaagtgggt ccctgacccc tgagtagcct aactgggagg
96301 cacaccccag tagaggcaga ctgacacctc acatggccag gtactcctct gagacaaaat
96361 ttccagagga acgatcaggc agcaatattt gctgttcacc aatattcgct gttctgcagc
96421 ctcttctgct gatacccagg caaacaggtt ctggagtgga cctccagcaa actccaacag
96481 acttgcagct gagggtcctg actgttagaa ggaaaactaa caaacagaaa ggacatccac
96541 accaaaaccc catctgtaca tcagcatcat caaagaccaa aggtagataa aaccacaaag
96601 atggggaaaa aacagagcag aaaaatggga aactctaaaa atcagagtgc ctctcctcct
96661 ccaaaggaat gcagctcctc accagcaatg gaacaaagct ggatggagaa ggactttgat
96721 gagttgagag aagaaggctt cagatgatca aactactctg agctaaagga ggaagttcga
96781 acccatggca aagaagtcaa aaacattgaa aaaaaaatta gaagaatggc taactaaaat
96841 aaccaatgca gagaattcct tgaaggacct gatggagttg aaaaccatgg catgagaact
96901 acgtgacaaa tgtacaagcc tcagtagctg attcaatcaa ctagaagaaa gggtattagt
96961 gatggaagat caaatgaatg aaatgaagtg agaagagaag ttcagagaaa aaagaataaa
97021 aggaaatgaa caaagcctcc aagaaatatg ggactatgtg aaaagaccaa atctatatct
97081 gactggtgta cctgaaagtg atggggagaa tggaaccaag ctggaaaaca ctcttcagga
97141 tattatccag cagaactttc ccaatctagc aaggcaggcc aacattcaaa ttcaggaaat
97201 acagagaatg ccacaaagat actccttgag aagagcaact ccaagaaaca taattgtcag
97261 attcaccaaa gttgaaatga aggaaaaaat gttaagggca gccagggaga aaggtcgggt
97321 tacccacaaa gggaacccca tcagactaac agcggatctc ttggcagaaa ctttacaagc
97381 cagaagagag tgggggccaa tattcaacat tcttaaagaa aagaattttc aacccagaat
97441 ttcatatcca accaaactaa gcttcataag tgaaggagaa ataaatttcc ttacagacaa
97501 gcaaatgctg agagattttg tcaccaccag gcctgcccca caagagctcc tgaaggaagt
97561 gctaaacacg gaaaggaaca actggtacaa gccacttcaa aaacatgcca aattgtaaag
97621 accatcaagg ctaggaagaa actgcatcaa ctaacgagca aaataaccag ctaacatcat
97681 aatgacagga tcaaattcac acataacaat attaccctaa aatgtaaatg ggctaaatgc

97741 tccaattaaa agacacagac tggcaaattg gataaagagt caagacccat cagtgtgctg
97801 tattcaggaa acccatctca cgtgcagaga cacacatagg ctcaaaataa agggatggag
97861 gaagatctac caagcaaatg gaaaacacaa aaaggcagga gttgcaatcc tagtctctga
97921 taaaacagac tttaaaccaa caaaggtcaa aagagacaaa gaaggccatt acatcatggt
97981 aaagggatca attcaacaag aagagctaac tatcctaaat atagatgcac ccaatacagg
98041 agcacccaga ttcataaagc aagtccttag agacctacaa agagacttag actcccacac
98101 aataataatg ggagacttca acaccccact gtcaacttta gacacatcaa tgagacagaa
98161 agttaacaag gatatacaag aattgaactc agctctgcgc caagcggacc taatagacat
98221 ctacagaact cacaatccaa aatcaacaga atatacattc ttctcagcac cacactgcac
98281 ttattccaaa aattgaccac atagttggaa gcaaagcact cctcagcaaa tgtaagagaa
98341 cagaaattat aacaaactgt ctctcagacc acagtgcaat caaactagaa ctcaggatta
98401 agaaactcac tcagtgtgtg atgttccctt tcctgtgtcc atgtgttctc attgttcaac
98461 tcccacctat gagcgagaac atgcagtgtt tggttttttg tgcttgtgat actttgctga
98521 gaatgatggt ttccagcttc atccatgtcc ctacaaagga catgaactca tcatttttta
98581 tggctgcata ggataccatt aggaggtata cctaaggcta aatgacgagt taatgtgtgc
98641 agcacaccaa catggcacac gtatacatat gtaacaaacc tgcacgttgt tcacatgtac
98701 cctaaaactt aaagcataat aataataata aaagaaactc actcaaaacc gctcaactac
98761 atggaaattg aacaacctgc tcctgaatga ctactgggta cataacgaaa tgaagacaga
98821 aataaagaca ttctttgaaa ccaatgagaa aaaagacaca acataccaga atctctggga
98881 cacattcaaa gcagtgtgta gagggaaatt tatagcacta aatgcccaca agagaaagca
98941 gaaaagatct aaaattgaca acctaacatc acaattgaaa gaactagaga agcaagagca
99001 aacatattca aaagctagca gaaagcaaga aatagctaag atcagagcag acctgaagga
99061 aatagagaca caaaaaaccc ttcaaaaaat caatgaatcc aggagttggt tttttgaaaa
99121 gatcaacaaa attgatagac tgctagcaag actaataaag aagaaaagag agaagaaaca
99181 aatagatgca ataaaaaatg ataaaggca tatcatcacg gatcccacag aaagacaaac
99241 taccatcaga gaatactata aacacctcta tgcaaataaa ctagaaaatc tagaagaaat
99301 ggattaattc ctcgacacat acaccctccc atgactaaac caggaagaag ttgaatccct
99361 gaatagatcc ataataggct ctgaaattga ggcaataatt aatagcttac caaccaaaaa
99421 aagtccagga ccagatggat tcacagtcga attctatcag aggtacaagg aggagctggt
99481 accattcctt ctgaaactat tccaatcaat agaaaaagag ggaatcctcc ctaactcatt
99541 ttatgaggcc agcatcatcc tgataccaaa gcctggcaga gacacaacaa aaaaagagaa
99601 ttttagacca atatccctga tgaacatcga tgcaaatatc ctcagtaaaa tactggcaaa
99661 gtgaatccag cagcacatca acaagcttat ccaccatgat caagtgggct tcatccctgg
99721 gatgcaaggc tggttcagca tatgaaaatc aataaacaga atccagcatg taaacagaac
99781 caacgacaaa aaccatatga ttatctcaat agatgcagaa aaggcattcg acaaaattca
99841 aaaaccttca tgctaaaaac tctcagtaaa ttaggtattg atgggacata tctcaagata
99901 ataagagcca tctatgacaa acccacagcc aatatcatac tgaatggaca aaaatgggaa
99961 gcattccctt tgaaaactgg caccagacag ggatgccctc tctcaccact cctattcaac
100021 aaagtgttga aagttctggc cagggtaatc aggcaggaga aggaaataaa ggatattcaa
100081 ttaggaaaag aagaagtcaa attgtccctc tttgcagatg acatgattgt atatctagaa
100141 aatcccatcg tctcagccca aaatctcctg ataggcaact tcagcaaagt ctcaggatac
100201 aaaatcaatg tgcaaagtca caagcattct tatacaccaa taacagacaa acagagagca
100261 aaatcatgag tgaactccca ttcacaatag cttcaaagag aataaaatac ctaggaatcc
100321 aacttacaag ggatgtgaag gacctcttca aggagaacta caaaccactg ctcaatgaaa
100381 taaaagagga tacaaacaaa tggaagaaca ttccatgctc atgggtagga agaatcaata
100441 ttgtgaaaat ggccatactg cccaaggaaa tttaaagatt caatgccatc cccatcaagc
100501 taccaatgac tttcctcaca gaattggaaa aaactacttt aaagttcata tggaaccaaa
100561 aaagagccca tatagccaag tcaatcctaa gccaaaagaa taaagctgga ggcatcacgc
100621 tacctgactt caaactatac tacaaggcta cagtaaccaa aacagcatgg tactggtacc
100681 aaaacagaga tatagaccaa tggaacagaa cagagccctc agaaataatg ccacatatct
100741 acaaccatct gatctttgac aaacctgaca aaaataagaa atggggaaac gattccctat
100801 ttaataaatg gtgctgggaa aactggctag ccatatgtag aaagctgaaa ctggatccct
100861 tccttacacc ttatacaaaa attaattcaa gatggattca agacttaaac attagaccta
100921 aaaacataaa aaccctagaa gaaatcctag gcaataccat tcaggacata ggcatgggca
100981 aggactttaa gtctaaaaca ccaaaagcca tggcaataaa agccaaaatt gacaaatggg
101041 atctaattaa actaaagagc ttctgcacag aaaaagaaac taccatcaga gtgaacaggc
101101 aacctacaga atgggagaaa atttttgcaa tctactcatc tgaaaaaggg ctaatatcaa
101161 gaatctataa tgaactccaa caaatttaca aaaaaaaaac aaacaacccc atccaaaagc
101221 gggtgaagga tatgaacaga cacttctcaa aagaagacat ttatgcagcc aaaagagaca
101281 tgaaaaaatg ctcatcatca catgaaaaaa tgctcatcat caccggccat cagagaaatt
101341 caaatcaaaa ccacaatgag ataccatctc ataccaatta gaatggtgat cattaaaaag
101401 tcaggaaaca aggggtgctg gagaggatgt ggagaaatag gaacagtttt acacagttgg
101461 tgggactgta aactagttca accattgtgg aagtaagtgt ggcaattcgt cagggatcta

101521 gaactagaaa taccatttga cccagccatc ccattactgg gtatataccc aaaggattat
101581 aaatcatgct gctataaaga cacatgcaca agtatgttta ttgcggcact attcacaata
101641 gcaaagactt agaaccaacc caaatgtcca acaatgatag actggattaa gaaaatgtgg
101701 cacatataca acatggaata ctatgcaacc ataaaaaaga tgagttcatg tcctttgtag
101761 ggacatggat gaagctgaaa accatcattc tcagcaaact atcgcaagga cataaaaccg
101821 aacactgcat gttctcactc ataggtggga attgaacaat gagaacacag ggacacagga
101881 aggggaacat cacacagagg ggcctgttgt ggggtggggg gaggggggag ggatatcatt
101941 tggagatata cctaatgtta aatggcgagt tactgggtgc agcacaccaa catggcacat
102001 gtattcatat gtaagtaacc tgcacattgt acacatgtac cctaaaactt aaagtataat
102061 aaaaaaaatt aaccaggcat ggtggcatgt acctgtagtc ccagctactg gagaggctga
102121 gggaggagaa ttgcttgaac ccaggatgtg gaggttgcag tgattgaaga tcatgccact
102181 gcactccagc ctgggcaaca gagctagatg ccatatcaga aaaaaaaaaa aagagaaaag
102241 aaaacaaaac aaaagaaagt ccagcatggt aagaggtaca tagaggtaca tggggtgag
102301 cttcatttga ttttcatcct ttttcccttc tctggacaga attctcaatg caaaacattc
102361 caaaaacaca gagcaaatgt cttctataat cttcccctta tctgtgactt cccttcacag
102421 tgtatgtgct agcgtcttcc agactttttt gtatgacgtg ctatacagaa gatcagatca
102481 aatgggcaca ttcccaatcc taataagtgg tgacttgcca gatctggact cacgttgcag
102541 ggtgctggga cctctgccag aatcaagcag tagctccagg agccagggct ttgggtctct
102601 tctgtgcatc ttcaggagct tttattgacc tttctcacca cgaccccctt ctcaattacc
102661 aacttccaat ccaaaaatga catccaacta gatcctgaac ttccatccag ttaaccctga
102721 ttgagttttc aactttcttc ttattaagtg attaaattag atatagattc ataaaagtga
102781 aagaattaat aatagggtga aggtctaaaa ctcatttatt cacttattcc ataaacactg
102841 gtaaagtttg actaatatgt gactttcata gtgatacagg gaaggattta agctgtttct
102901 gacattagaa aaaaaaatat atatatatgt ggtatcttta ttagagaacc tttggccaca
102961 tcaaaagcat caaaatgttt tagagttaaa acaactttaa gaagacagtg atgtcatccc
103021 tgaaaaacac aataaaaatc ttggtgtatc cacttgtcac ctgggttttg tgctacctaa
103081 catggtagat catatgccca ttcaggtgga aaacaggaac tactgagggt gtaatttatc
103141 tcaaggttaa ggtcaaggat tcactgaaag aaatcaggcc taaattacaa agtgatgtgg
103201 gggctgggct ggacaggact gagtgttttc atgggaccct gggagggaag ctagacaaca
103261 taaaacatgg caggtatttt gtgggcattt agacaaaagg attgaaagac ttccttctag
103321 attgagttta aaaatttaaa aaaaaacata attacaaaag agataatgca gatccttaaa
103381 gcatcacagt gtctttgagg gcagagagag cagacacagt cttggctcct acttgaaggt
103441 gaggcatcat tactcacaaa cgtgttgggc ttccctcaga ataccagctt gggaagagtg
103501 aatctgagtg tgtgagctgg ggcagagccc agagaggagc agtgtggtca gacataaaga
103561 gggagacttt tcgatctgga agcatgaatg gtggaagctg tgtatctgca gaatttgtga
103621 gagaaatgaa ccccttgggg aaatcctgca ccatcctcag gaccccagcg agaatcctgc
103681 agtttggggg gtctttctac catgttctgg ttgcctgtgc ttctgaaggt gctcctctgc
103741 tgtccaggtc agtgcagcct tttgagtcca tctgaaggga tggcttaacc tcagttccac
103801 tcactgaatt tctactggga ttccaaagct tctccaggct tttcatcggg ggttctaaag
103861 tatttctgaa cttgcatttt cctcccccag cctgagctgt cagagaagct gagtcctctg
103921 cttcctggaa atgtcagcca atctctcctg cacccaggag tatggccaca aacaatgcta
103981 agaaacactc tccctggaat cagcagtgac tcatacattc tccccaaatt ctactgagct
104041 atttcagtgc ccacagcaag gcataaagca gggagctcct cagctgctgc tctggaccta
104101 aagaggcaac cgggaccaat ggaggggaac cttgtctacc ttgcacagga agagccgact
104161 cctctggttc tcctagaagt gccaggctgt tggcagagcc tgggatggtc ccagtgtggg
104221 ggccatccct tcccaggctc tgctggccca gccttagagc tgaggaggct gcacctggaa
104281 tgcagtgagt ctgtttctga gtcagcgctt ttcctgtggt gtttgccctc tgtgcatttc
104341 taacagtcaa aaatctctgg tcaggccctg ctggcctcct ctagtcctca aggggtttt
104401 attccagatt gcatttataa ctatcttccc caagaccagg ggcttcccct cttttttcct
104461 ccaataaaga tatgaggaga aggtcactct gagagcagac agtggatgtt gagggaggtg
104521 cctgtagcag cctggggtcc gggagatctg cgaactcact gccgccggat ggagccaggg
104581 ggactccaga aagggagtgg cccagaggag ctgatggggg ccagagtaaa tgaggaggag
104641 agagatggca gcagccctgg ggaccgggtt ctctggcttt cacagagcca catccctgat
104701 aaactgggga cttggatctg agactctgaa aggactgggg cctggatgtg ggctgtgag
104761 tttccaaggc cctttgattt caccctggtc tgcatgaggt tccattggct gccccaccca
104821 atcccagcgg cagaaagtca gtctagcagg gaaacctggg gagagagggt cagtggcaag
104881 catccaccct agatgcaaag acaagggaca ggtcagaggg aggaagcctt ggcatgagtc
104941 caggggaagg gaggcacttt ggataggcgc agttctgttt ctgcacttgc cacagcctca
105001 gaggactgtg aggattgaac tttgcaggag ggaatgaggg agaatggggt ctggactgga
105061 gtccctgtca tccaagtgac cccctatcta ctcctgcaaa ccagatgaag gtcttgctag
105121 atgtaagtca aaccagggcc tatcaggcta accagcactt tctggtggat cctgaaggag
105181 cacagtgatc ctcggtcaaa aaaatctcac attgtccagg gctgaggatt gtcaggtggt
105241 gctgaagggg gagactctgg ggaaaaaaca gagaagattt tggtacattt cacactctca

105301 acgcccagtg cttccccctt cctattagtc aggcgtggtt ccaggataat cacttgaacc
105361 cgggaagccg aggttgcggc gagttgagct agagccattg caccccaggc tgggtgggca
105421 acaggagcga aactctacct caagaaacaa caacaacaag aacaacaaca agaaaacttc
105481 cactgagtgc ctgggtcgtg ccacctggta ttctgggtgc tgctgagaaa gatgctgaga
105541 aagatcctgg tattctgggt gctgctgaaa caagacacaa ggcagctgga aatgagctc
105601 agcagaatat acctggctca gaactgcagt gcagatcaga aaaagaatgc ctgtgcagga
105661 taaaacacga acagccatct tttcttttct ttttcttttt tttttttttt tttgtttctt
105721 ctttttaaag acagggcctc actctgtttc taagactggt gtgcagtggt gcaatctcag
105781 ctcactgcag gctcagcctc tcaggctgaa gcaatcctcc catgtcagcc tcccaatttg
105841 ctggaaacgc aggtgtgtgc caccatgcct ggctcatttt gtttatcttt tgtagagacg
105901 aggtctcact gttgcccagg ctggtcttga atgcctgtac tcaagtcatc ctcccaattt
105961 gagctcccaa agtgctgaga ttacaggggg gagccccatg tcctgcaaag ccatcttcat
106021 aatccatgtg gaaaatgatg gttatcccaa atcctttcat cactctttgt cagaacattc
106081 actactctca ctgccaatta gaaatgtaca ggagaatgaa agaaaaaagt taatattgtc
106141 caggtgcggt ggctcagccc ataatcccag cacttgggag gccaaggagg gcagatcacc
106201 tggtgtcagc aattcatacc agcctagcca acatagtgaa accccatctc cacaaaagta
106261 gaaaaattag gttggcatga tggcaggtgg ctgtaatccc agctactcag gaggctgagg
106321 cagaagaatc tcttgaacca gggagccaga ggtagcagtg agccgagatt ttgcctttgc
106381 actccagcct gggtgacaca gtcagactcc atcttaaaac aaaacaaaaa gaagttaata
106441 tttattaagt gcaccgtaat tcaaaatact aagaattcaa gtgctttatt tccttggaaa
106501 aactcaccaa gaggcgttaa aacggtgatg gccttcccca cagcacagca cagaggaagc
106561 agctgacatc ttccaggcct tggggcactg tcttgcttct ttctaagtgg ggatccgctc
106621 ccaacacact ctcctttgtg cccctcaggc catgaatatc tctgaggtct gtgagtgtga
106681 ggtctcttgc tggtggtgtt ccttcctgtt tcttgggact ttcttttttg gtgggtctct
106741 tggcataagg gatgcgagcc tggtgagaac aggctggact ctgcatcagg acacagaggc
106801 cctggagggg cagacagtgg agcaagcagg ggctgaattt acctgctttt tactcaaagg
106861 aagcccctca ccactgtctt ccctgccaca gcggctccaa taaaaagaaa aaaggtgtta
106921 actcagcaca ttccctggaa cagcaagaag aaaacatccc tcttggtatt cgtataagta
106981 ttaggactag tgttagctgg tttctctctc cttttgacat aataactcaa gataccctgg
107041 ggatctcgag tgcatggacc agcgagtctg aaggagtttg ttctttggat atgaacccat
107101 gagaagtgga tgtgtattct gtggccaaag tcactgacct cttttattag aggagacaga
107161 agtgcccagg aaccaccccc aggcctgtgc ttccagggac cgctctctct ctttccatgt
107221 gcatgcctga gacggttctg ggtcttcact catcaccatt tgctcttcta gagttgatgc
107281 atctactgta tacttgcagg ggacccggtg aagaaagaaa tccaagaacc cacgtgggtc
107341 cacatagagt gaggttgcct ccagacaggc acaggaaccc cgagcctctt agccactgcc
107401 agagcctgag actggggcac tcacatgcag acaaatgcat gggactttag aagaagcagg
107461 gttggaggta agagaggagg gcatggatgg aacgcagggg tcactggaag cttcagggcc
107521 agaagcactt gggagtgggg aaggcatcat caacagaaag gtcagagctc cttccatgcc
107581 ctgaggtcac agcgggtgtc cttctctccc agctcctccc tgggctcttg tgtctggagt
107641 cagggctggc tcagctgggg ttctttggtg agtgggaagg acatagggca ctcaggtct
107701 caagtgcaac ttttaaaatg atactcaaat gagaggtttc acacagtggc ctcctgtcca
107761 cagatcccat gtcttcttgc tggactcctg ggggatttgc ccagccaggg acatgaggta
107821 ttttacattt atctgccaag tgcaaaacat cttaatctat atgaggccag ctccatcctg
107881 gagggttgtg actttctaag tgctcacacc acatacacat atatgtacat accatgaagt
107941 cattgcagag atgccctctg ggcctggggt tctctcacag aacccagttg ataaaagcct
108001 cccttctact cacctgagac ctagagacat cagccacccc ctgccccatt cgcccagcat
108061 ttttcctaaa gtggaaacca actcagaacg cacataaatg aacacagcct ctaactcatg
108121 gacttagggg gaacagacag aatgagaaga ccagggttga aagtggcatg tcgtgaagtg
108181 gcacaggcag atgaggctgg agaggtgcag tcagaggcag gggtggcaca ggagtcattg
108241 gcagttttgg actggggtgg tgaggccacc tgaggcctgt tttccagttc cccattatgg
108301 cccggaggac tttgatccct gagagaggta accaggaggc aggggcaaca ataaggtggc
108361 cactgcaatg cccaggcagg aggtcttggc atcacctggt gtggctgcag acagcctggg
108421 gacatcctgg aggtagagtc aatgtgactt gctcttatgg tttcaggagt gggtgtgagg
108481 cctgaggaaa agcgatgggt tgacagcacc tcctagagtt tggtcttgac tcagcgaagt
108541 tgggaagagt aagtgaaaag ccagcatgaa gacaacgact caaggtcttt gtaggacatt
108601 ttaaattcaa ggcatttatg ttattaagag aaaaaaaccc aagaacaata taacactcat
108661 gaaataaccc tgttcaataa aattgtaaag gtgtatacaa tttattcatt cttaggactc
108721 acacgataga tgttcccaga tgcttcccat ggactatata tcctactggt gtaggtggga
108781 gtaaggtgag agccaggatt ccaatcccag cagttttcaa atcccagacc ttcaactaag
108841 gtcaacatca gatctggaag aggaaagagg tgcagtttct tttctaattc catcaggatc
108901 attctgggtc tggccacagg gcacactgca ctgttctcct atctcagagg agagggtagg
108961 atcccaaaag ccaaatgtgt ccagatatgt gttggcataa tgttttaaaa aatagagttt
109021 cagttagaaa ggaggagtga gttgaatatc tatagcatag taaggtgact atagttagta

109081 ataatatatt atataattga aaatactgag acagtaaatt ttaaatgtct ccccataaaa
109141 agagtaagaa tgtgaagtgg taggaatgcc aaatagcttg atttcactat tccacattgt
109201 agagtatgaa tatatcaaaa catcatactg taccccatga acagatacaa tttttattaa
109261 taaaattttt ataaaataga cgaaaaaaac cttatgggca tcacatagga ataccagtga
109321 ttggtctcag gaagacggaa aactgcaaag ataaaataag aactcagcaa aggaacacaa
109381 ggtaaaacaa aactcaatat aagagccaga aaatggcaaa agtcccggtc agagtgactt
109441 gtccaagatt cttccaaaga aacccaaaga aggaagaaag gggtggtaat ttcaagtctc
109501 tgaaatcctc tgctttcctg gaatatctaa tgactattct accttctaat taaagaaaca
109561 cccctgtcaa tattagacag atcaatgaga cagaaaatta acaacgatat tcaggacttg
109621 aacgcagctc tggaccaaac agacctaaca gacatctaca aaactctcca ccccaaatca
109681 acagaatata catttttctc agcaccatat cacacttatt ctaaaattga ccacataatt
109741 ggaagtaaaa cactcctcag caaatgtgaa agaatggaaa tcataacaaa cagtatctca
109801 gaccaaagtg caatcaaatt agtactcagg attaagaaac ttactcaaaa ctgcacaact
109861 acatgaaaac tgaacaagct gattctgaat gacaactggg caaataacaa atttaaggca
109921 gaaataaata agttctttga aaacaatgag aacaaagaca caatgtacca gaatctctgg
109981 gacacagcta aagctgtgtt tagagggaaa tttatagcac taaatgccca taggagaaag
110041 gaggaaagac ctaaaattga cattgcaatt aaaagaacta gagaagcaag agcaaacaaa
110101 ttcagaagct atcagaagac aagaaataac taagatcaga gcacaactga aagagataga
110161 gacatgaaaa acccttcaaa aaatcaatga atccaggagc tggtttttag gaaaagatca
110221 acaaaataga ccactagcca gataaattaa gaagaaaaga gagaagaatc aaacagacac
110281 aataaaaaat ggtaaagggg atatcaccac tgatcccaga gaaatacaaa ctaccatctg
110341 agaatctata aacacctcta tgcaaataag ttagaaaatc tagaagaaat gtgtaaattc
110401 ctggacacat agatgctccc aagactaaac caggaagaag ccaaatgcct aaagagacca
110461 ataacaaatt ctgaaattga ggcaataatt aatagcctac caacaaaaca aagtccagga
110521 ccagaagaat tcacagtcaa attctaccat tcctctgaaa ctattccaaa caatagaaaa
110581 agagggtctc ctccctaact catttttatg aggccagcat catcctgata ccaaaacctg
110641 gcagagacac aacaaaaaaa aggaaatttc aggccaatat tcctgatgaa catcgatgca
110701 aaaatcctca ataaaatact ggcaaaccga atccagcagc acatcaaaaa gcttatccac
110761 caagatcaag ttggcttcat ccctggcatg caaggctggt tccacttatg caaatcaata
110821 aacgtaatcc atcacataaa cagaaccaat gacaaaaacc acatgattat ttcaatatgt
110881 gcagaaaagg cctttgataa aattcaacac cctttcaggc taaaaactct agataaacta
110941 ggtgttgatg gaatgtatgt aaaaataata agagccattt atgacaaagc cacagccaat
111001 atcatactga atgggcaaaa gctacaagca ttcccttgga aaaccggcac actgcatgga
111061 tgccctctct caccactcct attcaacata gtattggaag ttctggccag ggcaatcagg
111121 caagagaaag aaatagagta ttcaaatagg aagagaggaa gtcaaattgt ctctgtttgc
111181 agatgacatg attgtatatt tagaaaaccc catcgtctca gcccaaaatc tcctaaagct
111241 gataagcaac ttcaacaaag tctcaggata caaaatcaat gtgcaaaaat cacgagcatt
111301 cctatacatc aacaatagac aaacagagaa ccaatcatga gtgaactccc attcacaatt
111361 gctaaaataa aataaaatac ataggaatac aacttacaag ggatgtgaag aagaactaca
111421 aaccactgct caatgaaata aaagaggata caaacaaatg gaaaaacatt ccatgctcat
111481 gggtaggaag aatcaatatt gtgaaaatgg ccataatgcc caaagtaatt tatagattca
111541 atgctatccc catcaaggta tcattgagtt tcttcacaga attagaaaaa ctacttaaaa
111601 cttcatatgg aagcaaaaaa gaacctgtat acacaacaca atcctaagca aaaagaacaa
111661 agctggaggc atcatgctac ctgacttcaa actatacgac aaggctacag taaccaaaac
111721 aacatggtac agttatcaaa acagatatgt agaccaatgg aacagaacag aggcctcaga
111781 aataatgcca cacatctaca accatctgat ccttgacaaa cctgacaaaa acaaccaatg
111841 gggaaaggat tccctattta ataaatggtg ttgggaaaac tggctagcca tatgcagaaa
111901 actgaaactg aaccccttcc tttcacctta tacaaaaatt aactcaagat ggattaaaga
111961 tttaaatgta agacctaaaa ccataaaaac catagaagaa aacgtaggca ataccattca
112021 ggacataggc atgagcaaag acttcatggc taaaacacca aaagcaatgg caacaaaagc
112081 caaaattgac aaatgggatc taattaaaat aaagagcttt tgcacagcaa aagaaactgt
112141 catcagagtc aacaggcaac ctatagaatg ggagaaaaat tttgcaatat atccatcaga
112201 gaaatgacta atatccagaa cctacaagga acttaaacaa atttacaaga aaaaaacaaa
112261 caaccccgcc aaaaagtggg tgaaggatac taacaggcaa ttctcaaaaa taaaacgttt
112321 atccggccaa cagacatatg aaaaaatgct catcataact ggtcatttga tttgcacttc
112381 tctaatgcaa atcaaaacaa cagtgagata ccatctcagg ccagttagaa tggtgatcat
112441 taaaaagtca ggaaacaaca gatgctggaa aggatgtgga gaaataggaa tgctttgaca
112501 ctgttggtgg gagtgtaaat tagttcaacc attgtggaag acagtgtggc aattcctcaa
112561 ggatctagaa ccagaaatac catttgatcc agcaatccct ttactgggta tatatccaaa
112621 ggattataaa tccttctact ataaagacac atgcacaagt atgtttactg cagcactatt
112681 cacaacagca aagacttgga accaacccaa atgcccatca atgatagact ggataaagca
112741 aatgtggcac atatacatca tggaatacta tgcaaccata aaaaataagt tcatttcctt
112801 tgcagggaca tggatgaagc tagaaaccat cattctcagc aaactaacac gggaacagta

112861 aaccaaaaca cctcatgttc tcactcataa gtgggagttg aacaatgaga actcatggac
112921 acagataggg gaacatcaca catcagggcc tctcagggtg tggagcacta ggagaggggt
112981 agcattagga gaaataccta atgtagatga cgggttgatg ggtgcagcaa accaccatgg
113041 catgtgtata cgtatgtaac aaaactgcac attctgcaca tgtatcccag aacttaaagt
113101 ggaaagaaga aagacagaaa gaaagaaaga aagagagaga gagagagaga gagagagaaa
113161 gaaagaaagg aaggaaggaa ggaaggaagg aaggaaggaa ggaaggaagg aaggaaggga
113221 aagaaagaaa gaaagaaaga aagaaagaaa gaaagaaaga aagaaagaaa gaaagaaaga
113281 aagaaagaaa gaaagaaaga aagaaagaaa agaaagacag acagacagac aggacagggg
113341 aggggagggg aggggaagca aagggaaggg aagggaaggg aaaagaaaag aaatacccat
113401 aaaataggaa agctggctgg tcacaggaga agcatgaaaa tatcaagcag tgatttcata
113461 cagcagcaag agaagagctt gtaaaattag ctacaagaat aaggataagc cttgaccaat
113521 aagacccaaa caagcaggac ggggctaagc tggctgacac tgaattggtc agacatggca
113581 ctgggtttga cccttgccct accccaggcc taattataca cccattatga cagtaaatca
113641 cacaccagcg ccaggacggt tctgagaatg cccatattta gtacaaaaac aggtgaaacc
113701 tagccaggcc cagtggctca tacctgtaat cccaacactt tgggagccca aggtgggtgg
113761 atcacctgag gtcgggagtt tgagaccatc ctgaccaaca tggagaaatc tcgtctctac
113821 taaaaataca aaattagccg agtgtggtgg cagatgtctg taatcccagc tactccagag
113881 gctgaggcag gagaattggt tgaacccagg aggcagaggt tgcagtaagc caagatcatt
113941 gcactccagc ctgggcaaca agagcgaaac tccatctcaa aacataaata aataaataaa
114001 taaatacata aataaataat aggtgacact tcagttctaa gaaaacttca ccattttttc
114061 ttaaaatcct aacgattatt tcaacctctc cttagagatc ctataaaatt agaaacccaa
114121 actcccttgt acacaactca ctctcctgaa ttggccctct cttgagtatc tacctttgct
114181 ttgcaataga cacttcttgc cttttgcttc attctgccta cttcctaaac tctttcttgc
114241 agcgatgaca agaatgtgga cactggttgg tgattgagtc tctgggtacc tggagatgac
114301 ctaatcacta tggcaatagt cagagtctaa aatcacaccc ggatattaca attcactctg
114361 gtttttcttg gggaaaaaat ccagtaactt tggccccatt tctccaaagg acatcactca
114421 gcacaaactg ataaacagca gtcctaccac tgggttgtaa gtatatggct ttattctggg
114481 ttctctattc tgttccattg gtctatgtct tgaccttcat actggcacca tgcagttttg
114541 cttactattg ccttaccata taaattgaag tcaggtaatg tgatgcctcc acatttgttc
114601 attttgctta ggattgcttt ggctctttag cctctttttt ggctccaaat gaattttagg
114661 attctttttg taatcctgtg gaaatgatgt ttgtattttg gtgtatgcaa tttttagatt
114721 gatgtttgat gttcgcaatt tttagattgc tttgggcagt gtggtcattt tcacgatatt
114781 gtgtctgtca atccatcagc atgggacgtt tttctccttt tttgtcatct atgatttttt
114841 tcagcagtgt cttgtagttc tccttgtaga gatcctgtac ctaatggtta agtgtattcc
114901 taggttgttt ttgttattgt cagtttttgt tgttgttttg caactattgt aaagggatgg
114961 agttcttcat ttgattctca gcttgcttgt tgttggtata aaagggtgtt actgatttgt
115021 acatacagat tttggacctg agacttaagt gaattcattt atctcatcta cgagtctttt
115081 ggtggaatct ttcaggtttt ctaagcacat acgatcatat cattggcaaa cacaggtagt
115141 ttcacttcct tctttccaat ttaaatgtcc tttatttctt ttgcttacca gattgctctg
115201 acaaaaactt tcagtcctat gttgattaca agtggataaa gtgagcattt ttgtcttctt
115261 ctggttctta gtgggaatac tttcaacatt tcttcattca atatgatgtt gcatatggat
115321 ttgtcatttt tggcttctat tattttgatg tatgttcttt ctaggcatag tttgtgtaag
115381 tgtagtccat tattttacaa gcttagattt gtattctgtt ttacctgagt gcgttctgag
115441 atttggcatc tattttacct gacataagta cagatgctcg tgctgttttt tggtttccag
115501 ttgcatggaa tactttattc taccccttca ctttccatct acatgtatcc ttataggtga
115561 agtgagtttc tggaaaacag catatagtag ggtcttattt ttttactcat tcaaagaccc
115621 tatgcctttc acttgcagaa gtgagataaa ttatattcat tgtttttatt gataaaggct
115681 tagtactccc atttcatttc ttgttttttt ggttgtttag agacttctct cttccatcct
115741 tttcttattg tctttctttg tgtttaagta attttctctt ctggaatcct tggaatgtga
115801 cttttctggc cagaaacctc tgtggctggg ggcacctttg ccagagtttt gatggggatc
115861 actgggttcg ttctgcccat gcagcctagt agactatgct tgactcatgt tttaggcctg
115921 gagcacatgc ctattaaggg caggtcaggg gtagagcagt gaggggtgtg tgagtgagca
115981 gggggtctgg ccactttgga cagtcactgg ctgctgctgc agcagtgggg caggcagctc
116041 caggtgccag cacgggtgcc agatttcgtc aaggctgcaa atgaaccagg cacagcacaa
116101 gcagcttcca tggttgtcac tggaatacac agtgacacca atactgaaag cttggaaatg
116161 ccagtaactg cagaaccccc agaaaggagt aacagccctg gctcaggaag ctcccatgtc
116221 tgggctcctg gaagattagt agctcttctc tttttctctt cacctacaac ttggtgagca
116281 agggctgtgt ttcagctttg tttgtgttat tgctctttta gtcccaccat taggcgggtc
116341 ccaagttctt gttctgtgac caggaagaat gaattatgca gataagtgga gggtgagtga
116401 gataaagagg agctttactg agcaatagaa cagctcagag acccacagtg ggtaacttct
116461 ttctgcagct agggtgtcct gatgagtgtt cagctctgag cagacaggag gcgctggagt
116521 gggtgacccc tctctcctgg caggttattc catcatcacc actgctctca gtagagagga
116581 ggccctggag tgggttgctg ctctctgcag gaaagtcatc tcatcatctc tacagctctc

116641 agcagagaaa aagacccaga gtgggttgct tgtctctgca ggaaaatcat cccagtagtg
116701 ggtagttcct ctctgccact ggtcttcctg atgttctccc tgagtctggg gtttttccag
116761 catcagactg gagaaaagta tgtgctcatt gggtcatagg tggccatgag caggcacaga
116821 aaaggcaaca tatgttccca ttctggtccg taggactggt ggcctagcct acgggcttca
116881 ggccctcctt ggtcagaagg tggagcctca ccagtgaccc tcaccttcct gtccaagatt
116941 ctgcctccct cccaccacca accatgttgc ccaagtcact tgtaccaagg agcatccaaa
117001 gaccagcgct gatctgtccg caatcccccc tcagcccccc ttctatactc atcagggccc
117061 aaagtccaga gggttcaagg cagccatggg atgctgtata tgcactgacc aaaatgttca
117121 cagacccacc tgggctgtga cagcatccag gctcgaccac aaccacaccc caaaattagg
117181 gcaggcgcca tgagagacca ggcagtggga acagacaccc tgaagctgaa ggagaagtgg
117241 ggatctcctg ggccctcaag agcactgggg gacctcagtt ggtaactgtg acctgggcag
117301 attcagttgc acctttggag ctgctgccct gccaactcga gaggaccagg actccctctt
117361 gtcccaggct cccatcagct ccgaagtgta cgcagccttg gctgtgccct ctctctgtgt
117421 ttccccacag agaggacagg tgagatgcag gttcacagca gctctgacca accccgcaca
117481 aacaaaccca atgctcctgg gtgtggttta accagcccca actgcactat catccaggag
117541 cttgcaggct aacagcaggc agtgggcagt gaggtagagg ctgtggtgga gactccagac
117601 ctgggtccag gttccatttt gccatgagag ggtgtgggtg gcacagctgg ctgcctcagg
117661 gacatggagc acaggcctgg ctcatgaccc tgccaagggg ggggcctcca ggagtggttc
117721 gtggttccca ggcccagcaa tcagactcca tggtctcccc tacagggggc agatcttgga
117781 aaaacagcct ggggtggatc cgcatggaac ctcccttcaa gacccaggag cttgacactg
117841 ttaacaggat gggcacagtg gccagatagc tcaacaggac cttgaagcag gtgccacttg
117901 tgcttctcac cctggccccc tgatggatgg ccccagctct gttttctggg cctggcatcc
117961 acacttcttg tgcgagcggg gcaccacccc attcctatct tctccttggg gcccctctct
118021 gcctgtccct ttgtgcctga ccgagctgct ccctgcaggc aaaaaaagga agaaaaaaac
118081 tgatgactga agagaagtaa agaatgggtg gagatcatct gcacacctgt tttcccagca
118141 ctttgggagg ccaaggttgg tggatcactc gaagacagga tcttgagacc aggctgatca
118201 acatggaaaa atcccatctt tattaaaaat acaaaaatca cccaggcttg gtggaatatg
118261 cctgcagtcc cagccatttg agtggttgag gcacgagaat cacttgagcc ctgaaggaca
118321 ggattgcagt gagcccagat tgcaccatcg cactccagcc taaatgacaa actgagattt
118381 tgtctccaaa acaaaacaaa gaacaagaat gggtgggaaa tactcaaaat gatctaattt
118441 ttatttggtt gctttgatgt tctacaactg aaactcaatc acagacaaat tagtatttcg
118501 ttatttttcc atccgtaact cgataactag agatttctga tggataaatt gcacaacagg
118561 ttaaaagttt tcattcaggt gctctttatt gctgatattc cttggtaacc atccttgcag
118621 ggataacagt ctcatcactg tagaacttta gcttctcttt ccgactctag gacacgggtc
118681 cctgaagttc tcactgatgt cacctcaaca ttttcctcca accttgcccc ctgctgttat
118741 gttttttccc tcacactgaa cacttgcctg tgcttccttt aagttgcatg tggcctggac
118801 acagtcactc atgccagtaa tcccagcact ttaggaagct gaggcaggag gatcccataa
118861 gcccagcagt ttgagaccag ctggagcaac acagcgaaac cctgtctcaa attgtcttta
118921 ataaaaattt tggaattatt aaaaaatgaa ataaataaga aaagagaaaa atagtttgca
118981 cccacatagt agattttagt gtccaagtgc ctggaagaga actttggatt tctctacccc
119041 actgggcacg ccttccctag cagcaaagat ggagctccag ttcctcagac ggtgatgagc
119101 cacaggaagg gcagggggtg gggccaatga agatcctctt gggctgcctg acttccctca
119161 gtgtacacat cagctcagcc cgaagtgggg tgaagatc
//

- LOCUS: HSU79458 1691 bp mRNA linear PRI 01-FEB-1999
- DEFINITION: Human WW domain binding protein-2 mRNA, complete cds.
- ACCESSION: U79458
- VERSION: U79458.1 GI:4205085

1 actagtggat ccgtcgacgg ccattatgga tggatgggga gagactatgg cgctcaacaa
61 gaatcactcg gagggcggcg gagtgatcgt caataacacc gagagcatcc taatgtccta
121 tgatcacgtg gaactcacat tcaatgacat gaagaacgtg ccagaagcct tcaaagggac
181 caagaaaggc actgtctacc ttacccctta ccgggtcatc tttctgtcca agggcaagga
241 tgccatgcag tccttcatga tgccatttta tctcatgaaa gactgtgaga tcaagcagcc
301 cgtatttggt gcaaactaca tcaagggaac agtgaaggcg gaagcgggag gtggctggga
361 aggctctgct tcctacaagt tgactttcac ggcagggggc gccattgagt tcggacagcg
421 gatgctccag gtggcatctc aagcctccag aggtgaagtc cccagtggag cctatggcta
481 ctcttacatg cccagcgggg cctatgtcta tcccccgcca gtcgccaatg gaatgtaccc
541 ctgccctcct ggctacccct atccaccgcc cccacctgag ttctatccag gaccccccat
601 gatggacggg gccatgggat acgtgcagcc cccaccaccg ccctaccctg ggcccatgga
661 acctccggtc agcggccccg atgtcccctc cactcctgca gccgaagcca aggccgcaga
721 agcagccgcc agcgcctatt acaacccagg caatcctcac aacgtctaca tgcccacgag
781 ccagccgccg ccacctccct actacccacc ggaagataag aagacccagt aggccctcct
841 gcctccctgc ctcccaccct catctctcta ccctacccct cccatcgggg ctgtgctggg

901 gcttggggag gggagggggc gccttgttct ccctccaggt ctgatcataa acaattacca
961 ggaactagca ttgtgggaca ttagggcccc cggcctcggg agaggtgccg cccagcttcc
1021 catgccagcc cggagcccac agtgctgccc agcgtacctc cctcaccgtc tggggctctt
1081 ctgggagcac ggagcatccc ctgttcctgt ttcacctctc agcttctccc ctcgaaggga
1141 ctctctggcc acctcctcca ccgcagtcca gctccctcag tctggcaccc actgctacac
1201 tcagcctcat gagccacttc agaccagcca ggtgtcttcc cgggccctgc cagaccctgc
1261 tcacattccc tctgctggtc tgtgctggtc tcagaaggcc accgcgcccg cattccactc
1321 agccagggtc cagctgcagc ccccgccacc cttccttccc ttccctgtcc tgggtcatgt
1381 tgttgccacc ctgtgtgact tttgaagctg taaaatgagc ttccagggct tgggtggcgt
1441 cggggcaggg ccgccgaggc tgggaggaag cccttctgcc ttttgctggt gtttctggaa
1501 tttgctttcc ctcacctctc acttccttct agaaggagct tcctgactgg aaccagagaa
1561 tgcatgtctg tccacttggt ggctgctggg tggggccggg aacaagggcc cctgaccctg
1621 tgtgctggcc gggacctgcc accagccccc cagcctgctt cttcccctta agcttatcga
1681 taccgtcgac c//

- LOCUS: BC005078 1339 bp mRNA linear PRI 06-JUN-2006
- DEFINITION: Homo sapiens coiled-coil domain containing 93, mRNA (cDNA clone IMAGE:3609481), complete cds.
- ACCESSION: BC005078
- VERSION: BC005078.1 GI:13477224

1 cttgttctgt gcatcttcag aatcccccag gccctctctt gatccttttc atgaagtcca
61 gagcattcct tctattacag gctgaagatg cattgtaaaa ggtaatgcta taatagtatt
121 ccctcttctc aataaatgtg caaaactgga gaatctggtg ctgatctgca gcatacttga
181 caacacaggg aataccgtgg atgttccacg ccagccttac atccgtcccc attgcctgcc
241 catgtccagt ggccctgcgc aacaaaagta agggcttgtc ttaccacact ctccccagca
301 tctagctcaa ggtagatgac tgactgggaa attccggttg gataaaatgg cttttccaca
361 ttgcaacttt gcatctctat taggaaaatt tagattgaac ctttcctcta cttgatgctt
421 agtttgagaa cagttttcac aactttagag ggtctctctc agagtatgtt atactgtaac
481 cttgaagttt gagatgaaac aaggtgtcag aggttttgaa tctttcaaag gaattgcctt
541 ttacagattt tttttttatt ccctaagaag ggaaaaagag agggaagaac tacactaatg
601 ttagagataa ggtatgtttt ggctcaaaat gtgtcatgaa cctaaaggta acttgatggg
661 cagaaatgtc tcagccatgt gaaccaagaa gtgggaccac agtgcaaggc tgtgggtggc
721 agcactagat atggtggtgg caggagtgtg acatgtaggc actccatggg ggacagctgg
781 acggtgtcta tcaaaagtgc aaatgcgtaa gctctttgaa ctagcaatgc tactcctaag
841 aattgtacct gtgtacaagg tacttgtgta caagagtttt tgttgaaaca ttgtaatagc
901 aaaaagttgg agagtgccac caatacaggg gaatggttaa atacattatg gccagcttaa
961 acaatggaat agtttgtaga aatttaaaac aatggatcct gtgtgtgtgt actaccatag
1021 aatgggcccc aagatgcatg attaagtgag gaaaaaagcc catggtatcc taatgtttgg
1081 aatggagaaa gtgtgtagat gtatgtgtgt gcatatgttt tcacacagac gtatacacgt
1141 gtatatcata tgtccacctg catatgccta ggatgtctct gggaggatat ctagcagccc
1201 ggcagcctga attgcctctg ggaagagggg tgggaatgac ttttcaatgt acaccttttg
1261 taccttttga attttgtacc atgtacatgt attatctata aaaaaatagt tcttaatttt
1321 taaaaaaaaa aaaaaaaaa //

- LOCUS: NM_025125 2100 bp mRNA linear PRI 17-NOV-2006
- DEFINITION: Homo sapiens chromosome 10 open reading frame 57 (C10orf57), mRNA.
- ACCESSION: NM_025125
- VERSION: NM_025125.2 GI:31542685

1 gaagcgcgct cccggggagg tgttgcagcc atggctacgg cagccggcgc gacctacttt
61 cagcgaggca gtctgttctg gttcacagtc atcaccctca gctttggcta ctacacatgg
121 gttgtcttct ggcctcagag tatcccttat cagaaccttg ggcccctggg ccccttcact
181 cagtacttgg tggaccacca tcacaccctc ctgtgcaatg ggtattggct tgcctggctg
241 attcatgtgg gagagtcctt gtatgccata gtattgtgca agcataaagg catcacaagt
301 ggtcgggctc agctactctg gttcctacag actttcttct ttgggatagc gtctctcacc
361 atcttgattg cttacaaacg gaagcgccaa aaacaaactt gaagttgtct gaaagcttgc
421 tctacacttt tacattcatc ctcacccttt tttttgtggg gtagaggagg tgcagtaatt
481 tactcagtga tctttctact ttctagaaac tgtccttcaa agctctttaa gaccccctcg
541 ttagtcagtt ttttctctta tatgctctgg ttgagcttga atagaccagt tgttacttaa
601 gaaagaaaca gagaaagatt ttagcttttc aatcctattt ggcagaggac ttcagctacc
661 ttcttacagt ctttggctgt gttggtaccc tcgtgtgctc tgagctaagc cacatactaa
721 actgactttt tggtttgtat acccttgctc ccgccttctg atgaaaacac cttaccctca
781 caaccaccat ctttcctctc ctttccaaag ctctttccac cttgctgcac taagataaag
841 tgacactttc actatatgtc aattccacac acatttatta ggtacctgtg aggtaggatc

901 ctatcctctc aaacttccat ttctcatgct acagagaaag ataaggaaga tgagcaagtg
961 cctggaatgg ggcaggctga gcagtcacac aggcatagag gcacgctgag aacctggagg
1021 ggagactgca gagtgccttc cctgatgctg cagccggaag tgatccttcc ctccacctgg
1081 cccctgggac actgtgctct gcagtgtgca gggcctgatg gcactgctag attgctcctt
1141 cagctcaggg ccacagctta aacagcttta cctttcccct cagcacctgt cccactatct
1201 tgcacacagg tgctctaacc atgtttattg aacaaaggag ggaaactgat ttcactttca
1261 cttgttcatt atcattccaa tttttatgtg aaaatggcac aacccatttg ggtaccctc
1321 accccaaaat aaaagcccaa gtctaccttt gactggtacc accttttttg tggtttcgtt
1381 ggtgagaaac ctttatcttt ttcatacctt tctattctca atcacttctc caaaagtgtg
1441 tctttccagc tctgatttat tcaaaacaca agcatttctg tttagagatt ctagcccatg
1501 ggttatctgg ctagttatta cctctcctgt tcacttagtt atactttatt attgctcaca
1561 ggctggggag gcagaatgac tctgtcacca ctaggagcca ttagggcttc ttccctggag
1621 gactgcctgc ttgctttctg gggacactag ccctcatttc ccttctgtgg tacagtgggg
1681 caaattattt gtattaagca aacatttatg ggaaacaacc cgctcccgaa aacggagccc
1741 ccaagtaaag cacaaccctg aaagattatg aactatgaat tgtctctagt agagataaat
1801 ttctgcaaac atatctcagt cttccctctg tttctctggt gattaagaag ttcctttttg
1861 gtaaggaaaa ggatttttaa ccatagagtt aggcatcatg gaaattcaaa ccagatttct
1921 taatacctgg tcttcctcaa agagaaataa taacagtaat agtggtgctg ggaacaatat
1981 ggcagattat tgaatgaaat tgattaactt gaataaaatg ctgtgaattt tctctaaaaa
2041 aaaaaaaaaa aaaaaaaaaa aaaaaaaagt aaaaaaaaaa aaaaaaaaaa aaaaaaaaaa

- LOCUS: AF225421 1309 bp mRNA linear PRI 02-SEP-2000
- DEFINITION: Homo sapiens HT017 mRNA, complete cds.
- ACCESSION: AF225421
- VERSION: AF225421.1 GI:9963844
- KEYWORDS: FLI_CDNA.

1 agaaaagcaa gggcagggtt cagaaagact gaatgtaaag ctgggctctg agtctgccag
61 gcccttcgtg tgttgctctg cacattaatt aaagggtcta cgaggtcagc aaggacgccc
121 aagaagactc agtcatgaaa ggtgaactgc tcctgttttc cagtgtgatt gtcctgctcc
181 aggtggtatg cagctgcccg gacaagtgtt actgtcagtc atctacaaat tttgtagact
241 gcagccagca gggtctggcc gaaatccctt cccatttacc tcctcagact cgaacgctgc
301 atttacaaga taatcagata caccatcttc ctgcttttgc atttaggtca gtgccatggc
361 tcatgacctt aaacttgtcc aacaattccc tttcaaatct ggcccctgga gctttccatg
421 ggcttcagca cttgcaggtt ttaaatctaa cccagaattc actcctttcc ctggaaagca
481 gacttttcca ttccctccct cagctgaggg agcttgattt gtcatcaaac aacataagcc
541 accttcccac atccttggga gagacttggg agaacctaac tatacttgcg gttcaacaaa
601 accagcttca gcagcttgat cgagcgctcc tggaatccat gcccagtgtg aggcttttac
661 ttctcaagga caacctctgg aaatgcaatt gccacttgct cggtcttaaa ctctggctgg
721 agaaatttgt ctataaaggg ggactaacag acggcatcat ctgtgaatca ccagacacct
781 ggaagggaaa ggacctcctt aggatccctc atgagctgta ccagccctgc cctcttcctg
841 ctcctgatcc agtgtcctcg caggctcagt ggcccggctc tgcccacggt gtggtcctga
901 ggcctcctga gaaccacaac gcgggggagc gagaactctt ggagtgcgag ctcaaaccca
961 agccaaggcc ggccaacctg cgtcatgcca ttgccactgt catcatcact ggcgttgtgt
1021 gtgggattgt gtgtctcatg atgttggcag ctgccatcta tggctgcacc tatgcggcaa
1081 tcacagccca gtaccatggg ggacccttgg ctcaaaccaa tgatcctggg aaggtggaag
1141 aaaaagagcg atttgacagc tcaccagcct gagagctttt gtctcaaata ggattggtca
1201 ttgcaggcca gaagatagtg tctgagtagg gctgatgtgt ttcctgttag tctgatttg
1261 cttttgccaa aagacaaaaa aaaaaaaaaa aaaaaaaaaa aaaaaaaaa//

- LOCUS: AL050370 2515 bp mRNA linear PRI 18-FEB-2000
- DEFINITION: Homo sapiens mRNA; cDNA DKFZp566C0546 (from clone DKFZp566C0546).
- ACCESSION: AL050370
- VERSION: AL050370.1 GI:4914605

1 agcattttcc acgtgattat caactttaca gccatcattt caatgtctgt actattccac
61 atggtggatg aactagagtc tactcatctt ttattgttgg gacatttagg ctttttgcaa
121 ataaaaatat ttatcatagc agttccttcc ctcccctgat ggcctcataa gcctgtggtc
181 ctaggacttg actctttccc aaattagggt gcccacgctt ccttattccc tgaccacatt
241 cttccagcaa accctcctgg agctgagtga taggcactca tggtgggagg ccccatttaa
301 caccatccat gcagcactga tttgttatag gctgctgtgg ggggaggggg tctccaagga
361 aacagttgag gaaacagaga gtgaggtgag caggcagcta aatacagaaa ctgttaaggg
421 catcagaaac acccatgggc atgtggacag ctgtagaaat agggctgcaa gacacctcag

481 ctagaatcta atgacctgag agcaccagga aaagagaagg gagttgacag tgtatgcagc
541 taggaacaca tccccgtcgg gggtctgctc tgaggaccct ctataaccct aaagcagaca
601 gtacctctcc cagctcagta tgatgtgctc tatgattaat ctaacatgag gcgactgtgt
661 tctccatcaa aggagagact gttcctgtgt gtgtgtgttg aggggtgagg ggaggggga
721 acaccaggga gacctttaag gatggggtgg catttgagca ggacctcgaa agatgaggag
781 gcttttgaca aagggagtcc agaggcagag gacagaacat agaactagat gaagagccct
841 tgagggcagg gacggtggac ctccccctcc ctcccagatg ctggctcagg cctggtggac
901 actggcaaat gtcgttttga atgagtgccc ccggggggag aggtggcagc atgtgggcgg
961 tggggcattt gttggggggg gcggtgagtc ttcaccactg ctgctcttgc ccctcaggtg
1021 cagtgctttg accccaagga ggaccggtgg agcctgcggt caccagcacc cttctcacag
1081 cggtgtctcg aggctgtctc ccttgaggac accatctatg tcatgggggg tctcatgagc
1141 aaaatcttca cctatgatcc aggcacagat gtgtgggggg aggcagctgt cctccccagc
1201 cctgtggtaa gtggaggctt caaggcagga gagcggaacc cgcttctaag gctctggatc
1261 ttggagcttg tgaatcttgt gactccatcc taggtggttt tattatacat cactctctag
1321 ctcatcctca cagtgatagt gaaccatgca tttcacagat gaggagactg gccttagaga
1381 ggttcagcac ctggcccagg ccaacaacta ggccctgtta aggcagagcc tagaaccctg
1441 cagtcccaag tgctatagct ttgagtgggg atttggtcca aatgaccctg tggtctctcc
1501 tgccccgaag gccactgcta gtaaccaatc atgaggtaag atgtggggaa gggagttgct
1561 aaggtctgct ctagtgatat cagagcaggg aggcccaagt cctggaagct ctggcccctg
1621 caattccctg cctcccctac cttcttctcc atcccctgaa actccagaga ctacacagat
1681 atggttggac tggaggtctc tgccagttct gacctttggt gaccttagag gtgagatttc
1741 tggcttgagt ctgagattct aagatggcgg cagtcgagat tattgttatt ggtgtgctta
1801 ccacattcca ggctctatgt aaaatgcttt atatgcttaa cttccattaa ctgttaaggg
1861 aaccctgtga agaaggcact ttagttcttc tccctttgcc gatgaggaaa ctgaggtcca
1921 gagaggttaa gtgacttgcc taacgccatt cactgaataa gggtcaagct agagctggaa
1981 ctcaggtcca tccagtttcc tgcctctcta cagtccagat tgcctgttca cagcctgtgg
2041 gtcaaggttc tattatctgg cctggccagg cagggacaga ggaagcccca ggctggacct
2101 gatagttgtc tttctgtttg tccacccagg aaagctgtgg agtcactgtg tgtgacggga
2161 aggtccacat ccttggcggg cgggatggtc gcggagaaag caccgataag gtcttcacct
2221 ttgaccccag cagtgggcag gtggaggtcc agccatccct gcagcgctgc accagctccc
2281 acggctgtgt caccatcatc cagagcttgg gcaggtgatt cagatttgga cagcctgagc
2341 caggaggcgg agaggcaggc ggagctcaga tgcacactct gctccctcat ggcacctcca
2401 cgcaaacagc ccttaactta atggtccctt ttcttgtata aataaaatct tgttgggtct
2461 gtgttccagc tgcagtctgc cctgcctgga gatgcaaaaa aaaaaaaaaa aaaaa//

- LOCUS: AB051441 5147 bp mRNA linear PRI 06-OCT-2001
- DEFINITION: Homo sapiens mRNA for KIAA1654 protein, partial cds.
- ACCESSION: AB051441
- VERSION: AB051441.1 GI:13359180

1 gttttctaaa atgagagccg tcagaggcag ttgaaccaga gcgactccat cttgattagg
61 ggctgggtaa caaaaggctg agacctactg ggttgcattc ccaggaggct aggaattcta
121 agtcacagga tgagatagga catcagcata agatacaggt tacaaagacc ctgctgataa
181 aacaggattc ggcaaagacg ccagccaaag cccaccaaag ccaagatggg cacaaaagtg
241 acctccagtc atcctcacta ttcattgtgc accaactata ttagcatgtt ggagtcactc
301 accagtgcca cgacagttta caaatgccac ggcaacgtca ggaagttgcc ctatattgtc
361 taaaaggagg aggaattctc ggttccggga actgcccacc ccttacccgg aaactcatga
421 ataatccacc ccttgtttag catgtaatca ggaaataact ataagtatat tcagttgagc
481 agcccatgcc gctgctctgc ctatagagta ggttttcttt ttttgagatg gagtctcgct
541 ctgttgccca ggctggagtg catttttgca accttgggtc actgcaacct ctgcctcctg
601 ggttcgggag attctcctgc ctcagcctcc cgactagctg ggactacaga catgtgccac
661 caggcccagc tactttttgt atttttagta gagacagggt tttgccatgt tggccaggct
721 ggtctcgagc tcctgacctc aagtgatccg cctgcctcgg cctccctagg tgctgggatt
781 acaggcgcga gccactgcgt gtggcccaaa ttctttcttg cttgagatct aagaaccctc
841 tcttgggatc tggatcgggt cccctttctg gcaacagagt cacctccttc aagaggccct
901 gatgtcctca gcaggtcgct aagggcctgt ctagctccct gaggcccaaa cactgtgcca
961 ttttgacagc caggggcccc aggtccatct tgaggctgtg aacatgctgg ctgtccaggg
1021 gtctgtgagc tggtatcagg ggagggtggg gcagcaaagt gaagccgctc ccagggcaga
1081 agcctccatg cccacatccg ggaaatcttc ctctcggagg ccctgcctgg gaggcatctg
1141 caggcgctcc cccgctcata gaaggtctgc tcatccagcc cacccctgaa gtcactatca
1201 ggcagggggt agggcaggcc atggggacct acctgccctc catgcattcc aaagaatcga
1261 agtcctccat agggtccagg tcctcttcag agtcagtcat gccaagaacc tccatggtcg
1321 ggggttccag gagctgctca tacaggaggg tctccatcaa gatgtctctg gagaggggtc

1381 ttacttcaga catttctcct ctggggcaaa aagacagcat atgactggac ccctaagaac
1441 tgagcagtct gggggctctc tcacaggatg acccccagat tgtatagccc tactcaccag
1501 ctcttttgat ccgctaaagc cgtcaatatc ccccaacccc agctcccaca ggcatgaatc
1561 ctgctacatg ctggctgtgt gcctctgggc aagtcgccta gcatctctgt tcttggaagg
1621 gaggaacttg aagatgattc ataagggccc tttctttctt tctttgtttt tggatactga
1681 gtcttgctgg aggctggagg gcagtggcac aatcttggct cactgcaacc tctgcctcct
1741 gggttcaagc gattctcctg cctcagcctc ccgagtagct gggactacag gcatgagcta
1801 tgcctggcta attattttgt atttttagta aagacggggg tttcatcatg ttggccaggc
1861 tggtcttgaa ctcctgacct caaagtgatc caccccacct cggcctccca aagtactggg
1921 attacagcac tttggattaa tttattaaat acccattacc ctctaccaca aagaagggac
1981 ctaatctgaa cctttaacca tctggcctct gggcccaggc cctcagattg acttgatatg
2041 aatggcaaag atcccatcac caactgggcc taggctggcc ttagggccca gatctgcatg
2101 ggaaaagtgt ggagccaggg agtggaaggt ggggagcctg cccaggaggg tccttgcagc
2161 agcgacctcc tgctctggtc tctcctggcc accccaccag cccaagggag gcccagtcct
2221 gtcagctcct acctggcacc agctctgaat gccttactgg tctccagcct tcagtggagc
2281 tctctccaac ctaatctcaa ggtagcagcc tcaaccctga ccctgtcact gctctgttcg
2341 gctccctaga cctggcctgg tgcagtgcgg ctcatgcctg taatcccagc actttgagag
2401 gtggaggcgg gtggatcacc tgagcttagg agtttgagac tagcctggct aacatggtga
2461 aacgccatct ccgctgaaaa tgcaaaaatt aaccaggtgt ggtggcgctc gcctgtaatc
2521 ccagctactt gggaggctgg ggcgggagaa tcacttgaat ccgggaggca gaatatgcag
2581 tgagccgaga ccgcgccact gcactccaac ctgggtgaca gagcgagact tgtctcaaaa
2641 acaagtgggg ggttgtgcca catccactaa caggccctcc ccctgtggcc ctgccacccc
2701 aatcttggtt cccgctctaa ccacactcaa gtatgctttc catggatggt gcctggtcct
2761 gccatgcctc caaagtcttc aaatgtgcag ccaacctcag accggcccat gcctacacct
2821 attggtcctt ttttaaaaat taaattttat tattattttt ttcacagaca gggtctcacc
2881 cgtttgcccg ggctggagtg cagtggcctg atcatgctca ctgcagcctc aaactcctgg
2941 gctcaggtgg tcatcccacc tcagcctcca gggtaactcg acaggtatgt accactgcac
3001 ttggctcatt tttatagaat tgctgtagag atagggtctt gctttgttgc ccaggcttgt
3061 ctcggaactc ctggcttcaa ctcccgcctg ggcctcccaa agtactgaga ggatgggtat
3121 gaggctggcc tattcaggca tcaccttctt ggaccctcag agtctgcttg gggcctcct
3181 gggggtcctg cagcccctgt ctctgctccc ctctggctcc agctgaccac tgcactccag
3241 gctgggctac agggcaagac tccatatcag aaaaaaaaaa aattagaatg gagatactag
3301 taacacctat tgcacaggac tgttgtgggg atgaaatgat ccggcacaga gtaaaccgct
3361 aagagagggg ctgctgctct tatcagtttc tggttgtcag cccaaggcct ggcatagagc
3421 agacatatgt ttgttgactg aagacatgaa ggcaacctat tcccagagga cccactgtga
3481 ggtcactgca gctgcagatc caggaaactg gactggtttt tattatcttt tttttccag
3541 caaacttgca aatgagtagt tgaaaccctt ctttggtgaa ggaagacaca cctagccatg
3601 tgtccccacc caggctacag cccgcactta gcctcctaca cactcaccag tggaaacacc
3661 aggcgccagt cagaagccct attttaccct aagaggttag gatgcaaatc tagtatgtgc
3721 agaaaagagt gccacaccca cggccttgag ggcacctgca gggctccgag tgggagggag
3781 ggcaggttct ggacagagca gcctacattt ggtgaacagc tctgcactcg acagctcgcc
3841 tatggctcat ctggaagatg gggaaacgcc atctacctcc caagcccctc ccaggttgcc
3901 cagaagcaca agcaagtaga gatgtgttct gctcagccct cagcaggtgt tcaagggccg
3961 aggctgcagg agtgtcagag cggggttggg ggcagaggtg gatggggctg gacctgagga
4021 tgaggtcgaa ctcctggtag ggaccctcag actctcatga cctcgcccag tcctgtcaaa
4081 gccaagcagg cgggatctga cacttcctgt ttcacaggca ggcaaaccga gaggtcagg
4141 ggccacgctg gggtcacagg gagggccagc tgagtgggcc cagctagaaa accctggtcc
4201 agtcttccac gtgccaccct cccagtcaca gcctggccct ggaccatgag ggtggggtgg
4261 cacaaagccc ttggagcgca cacaactcca gtgtaaccca gcaggaaacg ccctgagcct
4321 gcagccaggc tggacctcag ttccaccccc tgctcggccc ctgctgtgtg aggcagaaca
4381 accagctcac ctctgagcct tgcagaaaca gccgggcctc cagggctgct gagaggatga
4441 aaacatacac tactgcccat tttccaccac ctagcagggg ctccataaaa gctggttaac
4501 acaatcaaca gccttagaag agaggagacc agaggccact cacagctcag gacgccacgg
4561 tggactgaac atgttgctgc ctttctggag ggtagtgtga ccatcagcat ccaccagtca
4621 cacacgcaca tgacctttga tttctaggga tgcatcccac acacacccct gcatggacct
4681 ggcagccttg aaaaattgaa gccatagatc acaggcaccg ctgtgctgtc atcacagccc
4741 ttcgggaggc cgaggcgggc acgtcgcacg agcccagttc aagaccagcc tgggcaatac
4801 aacgagaccc cctctctaaa aataaattaa caataaaaaa ttttaaaaat atatgaaatg
4861 ggccagccgc ggtggcttac acctgtaatc ccagcacttt gggaggccaa ggtgggcaga
4921 tcatgaggtc aggagttcaa gaccagcctg actaacatgg taataaaact ccgtctctac
4981 cccgtttcaa aaaaaattag ccaggtgtgg tggtgcgcgc ctgtaatcca gctactcggg
5041 tggctgaggc aggagaatcg cttgaacctg ggaggaggag tttgcagtga gccgagatca
5101 caccattaca ctccaacctg ggcgacaaga gcgaaaaact ccatctc//

- LOCUS: AB051447 6799 bp mRNA linear PRI 06-OCT-2001
- DEFINITION: Homo sapiens mRNA for KIAA1660 protein, partial cds.
- ACCESSION: AB051447
- VERSION: AB051447.1 GI:13359192

1 ataagaataa aaattacccc aaatttccaa atcaagaagt aatcatggtt caggtttggg
61 cagatgttct ttctaggcat gaacacacgt tatctcattg tttacttaac accgggttat
121 aaacatttac ccatagcatt tgaaaggtag ctatagatag aaaagaatca gagaagttct
181 aaaacagctc ttgcgctttg tttcaaattc tctgcaggaa agatgaggtc ttcagccttt
241 tttttagctg gacggcaccg ttgcagcagt ggtgaacagg gcactggatt gagtcaggaa
301 acccagctgt gaccttgggc aagccacttg ccctctttga gcttcactcc tgctaaggca
361 aggggcgcta ttcgtaccct gtctgcccac ctcacaggct ctggtgaagt ccttgatttg
421 aacgccttta gctcccaagg ttgtggtttg gagatagggc aggtcacatg accatgaaga
481 ctgaaggaga aacgtggaag cacgtgtgcc tgttgcttct tttccaactt aaaatgcttg
541 gtgatctcct gaagactcca gcctcctctc tgggaagcca ggatccacag accctttacc
601 tgcgggtcat gggcagtccc agatggtccc cctccccaac agagggggtg cagtgagacc
661 tccggaagtt actgcctctg ttaccctcaa agggattttc agatcagaca gcccccctac
721 tccaagggac gtgtgtggag cttggtacct ttatttatct cctgctccaa cccctgtgga
781 ctgcctgcac ccagaatggg gcctgtcctg ctggcaagtg gctgagaacc tccactccac
841 tcagcagggc tgttccccat ttaccgaaaa gctccgagag aaaataacta accccatggc
901 gccgctgtag ctactggcag agcctcctgg tccccacctc tagcgcctgt ggtttttgtt
961 tcatgcagag tgagcagtga atctgggatc ccatcagcag tcagtttggg tgcctgcgag
1021 gcacaatgat agatgttggt gaagggtatg tgtgaggata ttaattaata ttaacatgct
1081 agttatatta atattctcat tggaatttgt ggggctttgc aagttatatt tcaaatatat
1141 ggttttatgt aatcctttta actgccctga aaccttgaaa ttattgcacc tattttatag
1201 atggagagac tgaggctcag aggggtgaat tgcctaagat cctgggggag gaagcaccca
1261 ggttttctgg ttttgagtcc tgggcccttc ctgctgagta gctaccccca acacagacct
1321 gcccttggag agcttgcagc cacactggga aggccagtgt attggattgc tgcttagacc
1381 tggaaagcac gtgaataaag cttcaggtta aaaccatggg ggttccagga ggcagcagtc
1441 ggctctgcct gggggtgagc tgaggagccg gtgctctctg gaacaagggt agttgggctg
1501 aggctcagtg gacagtggag gttggcaggt gaagtgcagg aggtctttgc agggagtggg
1561 accaccttga gcacacacag aggaatgaga caggcaggtt actcaaggag cagaggtctc
1621 gtgaccactt cccagagcat gtggggtcct agcctcatct ccaggaggag aaagtgcatc
1681 tatacacaga tttgtcaatg gagtttaaat aggatgtggg aaaatctaga ttttccaaaa
1741 cagtacatat ttgctttgag aagaaaggta gatgcaggat gcataggtta gataatttta
1801 atagcagtaa cctcagagca tgtaagtatg atttgattta ctggagtgcc tggccgtctc
1861 agtcagtggg agcacggctt gggctgggag atgaggttga caagggttct ttctctcaaa
1921 tgcttccttt ggtttgctaa gaggtatctc ctactcggcc gggggcagaa gacttttcct
1981 tcttttccca gtttgcagta gttgggccag atttgtggaa gtgggagaaa ggcctgccct
2041 gcttctacat agagttggct gtcctgactt gatactcggt gtgccttcca gagacccgcc
2101 tccatctcct caactccctg gcttgatgct taggtggtga tggctgttgg gcacaggagt
2161 tacataacag atctgtgatg gacccaggag cagagccagt tgagtgaatg tcatggagtg
2221 ggagtggtct tgcatggctg tggtgtcccc tgcagcttgt gcagggtatg tggcaagagg
2281 tgctcaccac tcatctggaa tggctagact ggaagcactt ggccctcttg ggctctgcac
2341 ccccaccccc tcccacctgg cctgcctgct catcttcatg ggcgcctggg gagaccaatt
2401 atggctgctt gtcatagtgg ctcaggtcac cgttcacact tcctgggacc aggacatcag
2461 agccctgaga agggtcaagg ggccaagtgg gcctagcctt ttactgacag ctgggaaatg
2521 caagcgtgtg gaccagagca ccaagtgagt tggggccggt gtgggttcag caccgtgtcc
2581 ctacccagag ctccatttgt tgaaaacagc ctttctctac cgtttcttca cttggacaac
2641 tttaaactat gtattggctg gtcgcggtgg ctcacgcctg taatcccagc actttgggag
2701 gccgaggtgg gcaggtaact tgaggtcagg aggtcgagac cagcctggcc aacatggtga
2761 aacctcatct ctactaaaaa tacaaaaatt agcccagcgt ggtgacacgc acctgtaatc
2821 ccagctactc gggaggctga ggcagaagaa tcgcttgaac ttgggaggca aagattgcag
2881 tgagctgaga ttgcatcacc gcacttcagc ctgggagaca gagcgagact gcatctcaaa
2941 aaacaaacag aaacctacat attttctata tttcccccaa cattgaggct catttcttgg
3001 atgaacaatt taaatgtact gtgcctctct ggcaatattt tccaaaatta cagatgtttc
3061 tatactttca ccggcagctc tgcctcccag aatttattct acggatgggt taacacgtgt
3121 gcaaaatgat ttatttgcaa ggttcgtcat tgttgcctta tttttaatag caaaagattg
3181 gaggcagctt aaatgttcat tcgcaggggc caatgaacaa accatggccc gtctaaacat
3241 gggataccgc gtggccataa tacataagat ggacgctcaa cgcactgtgc cggattgagc
3301 agcaaggtgg attgccgagg gaagaagcag gtctgggcgg tgtgtctcgg agctgccatc
3361 agtgtaaaag ggaagagaat caaaagtgtc ttgcttgtct atgcccaggg ggtctctggg

3421 cagacaccgc aagtcggtga ttgtgatgcc tctggagggg gtgctggtca tgggagattg
3481 cttgtttgct ggagatccca tgtacctttt gattgctgaa gcaggtgaat gtacgccttt
3541 ccaagaaatt aaaatgggcc aggtgcggtg gctcacgcct gtaatcccag cagtttggga
3601 ggctgatgtt ggaggatcac ttgaggtgag gggttcgaga ccggcctggc caacatgatg
3661 aaaccccatc tccactaaaa atacacaaat tagccagaca tggtggtaca tgcctgtaat
3721 cccagctgct ctagaggctg aggcaggaga atcatgtgaa ccctagaggc tgagtttaca
3781 gtgagccaag atcatgccat tgtactccag cctgagctac agagcgagac tctgtctcaa
3841 ataataaaat aaaataaatt aaaaacataa ggactgtaac cttgcctcct gcccagtgta
3901 ggaaggtcaa ggttctggct acttctcaag tacaggagcc tcactcaggc cccagaccac
3961 taatcaaaaa atatgtgctt ggttctcaca aaggggccga gtgtgagggc ttgggtgttg
4021 cttggtaaat acgacccccg gtcccggcct tggagagatg gagccctctc tgggcccctt
4081 ggacacactg ctgttggctg actttgtcat tttcaaccct tgctccgatt ggctcacgtc
4141 atgatttctg aaacctttgg gggcttcccc actgacagaa agatacactt taactcagca
4201 ctgggcatcc caggccctct ttactgggcc tcttcttgag ccgcacttgg cctgtcaccc
4261 cttcctctgt ctgccctctt aactccccac ctccgtgcct ttgctcatat agttcccttt
4321 gcctgccttt ccgtccagag cagtctccac gtgcccaggt cctgtctgac tttcaagggc
4381 cagcttagtt tccacttctg cactgccttc tgacctccct ggcttctgtg taaactgccc
4441 agatcaagcc acacaatggt tcctgcaccc aaggaagctc cctggggccc cctcctggcc
4501 actcgctctt cgccggtagt caccactcac accttggcac tttcgcgtgg tgcctgccgc
4561 tgcctgtttg ggcctcccac acacagagtg tacagaacgg actcctcggt gtctggctgc
4621 cttcccgcag cactgtcaga tcatccaggt tgcctgtagt ggccctttgg tttttttctc
4681 tgctgcgtag gagttcacca aatataccac tatttattca ttctcctgtg gacaggcatt
4741 gggttatgtc cagcctcttc ggtgaattca ttcttgtctt tgggggcgcg tgtgcgctct
4801 ttgctgggta tacacccagg gtgggttgat ggcttacctg actcagaatg tgtttgcatg
4861 aatgaaattc aggttggtat gagaaatcta gggtgtcctg gctggagcca ggcttcttga
4921 ttacagggac agagcaggta cagggatcct ggtttagaca gcctgctccc atggggtggt
4981 agcattgttg gggtgcagga tgctgaatct gcaggggacc tatccgctca gtgcccagtg
5041 ggattttagc tggctggaaa ggtggtcaca tgtagagggg ctcaacaatc cagctaaaga
5101 ggctgagcgt tggtccattg ttctcaattt gagagaaaac tgagatcatc aaaattagga
5161 ctggtatgta ctaaaggaaa gaacctaatt acaaggctga attgagtaag ccctcgctga
5221 gggactttgg atttctttgt tgttcccctt tatttctgca cccccaccca agtgacagat
5281 atgtacatga ttggatgatt ttgctttcct ggttgagaga ttcctgggaa cttggcccag
5341 gagaaggggg agaaatgtgg agccgctaga gtggcctccg cttgtttgtg ttgattgaag
5401 gggagacgga aggagagctg tggacccctg accccttgtg agggcatgtg atccttttca
5461 aaaggctcac caggcagaag tgcctggcca ggggccgctc tttccctcta atcccctctg
5521 gagaagggcc aggctgtggg ttgctgacct gctctgatgt ggatcagcct cccccaataa
5581 tgcagctgcc cagaagctca gagagcccag gcaaccccca aaggcaggag ggccggctgt
5641 cattcccgtt gtcattccca ggcggctgga gtgggagcag agcggtcagt tcagatgaac
5701 agtgctcgag tctgacccca accagcgagt tatggtaaga tggaaggttc tccatctata
5761 ttaaataaga gaacaaaagc cctcccaggc tgcatgaata ttccagggat atatatgtga
5821 acgggttgcc agtttagctt ggcctgtggg tggcagccgc ctgagtgagc acttcgtggc
5881 tgcagctcta aagggtttgg atctgaaact aatgaatgaa aatatgacct cagaagattt
5941 aaagagagca aatacccagc aacagaacct gggtcccaga gactgttggg agcatgaaat
6001 cccaggctgg ccgaaggagg aagtgggaga gcaatggcag ctgacatcac atggtgccag
6061 accttctcag tgctttctgt gttcactcat tattccgtcc ctctctctca gaggcaggta
6121 tggctgcttc cccattttat agatgaggaa gctaaggcaa ggagaggttg tgtaacttgc
6181 tcacagacac aaagctagcc agtggcaaag ctggaggtca ggtctaggtg gtcaggctcc
6241 agagttctgc ggatttcaca gcacggcagt ggcagtcgga agaaccattt gtcaggtgat
6301 tgtgggcaaa tgacgtcagc ccttcaaacc tctgttttgc atctgcaagc tgcttgctgc
6361 tgcaacaaat taccagaaac ttagtgactt aaaacacaaa ttaggtcggg tgcggtggct
6421 cacatctgta atcccagcac tttgggaggc tgaggtgagt ggatcacttg aggtcaggag
6481 ttcgagacca gcctggccaa catgatgaaa ccctgtctct accaaaaata taaaaaatta
6541 gccaggcatt tggccgggtg tggtggatca cgcctgtaat cccagaactt tgggaggaca
6601 aggtgggcgg aacacaaggt caggagttca agaccagcct gaccaatatg gtgaaagcct
6661 gtctctacta agaatacaaa attagcagga cgtggtggca cgcgcctgta gtcccagtta
6721 ctgggaggcg gaggttgcag tgagccaaga tcacgccact gcactccagc ctgggtgaca
6781 gagtgagact ccgtctcac//

- LOCUS: AL024509 150024 bp DNA linear PRI 18-JAN-2007
- DEFINITION: Human DNA sequence from clone RP3-522P13 on chromosome 6p21.31-22.3, complete sequence.
- ACCESSION: AL024509
- VERSION: AL024509.1 GI:3947836
- KEYWORDS: HTG; 60S Ribosomal Protein L21; CpG island; D10S102; FBRNP; Heterogenous Nuclear Riboprotein; hnRNPA3.

1 gatcataggg gtggtccttc gtgaatggct tagcacaatc ccctcagtgc tgttctcatg
61 ataggtacta agtgagttac tatgagatct gtttgtttaa agtgtataga ctgcacctcc
121 ccactgtctc ttcctcctac tctggccagg tgaagtattt gctctccctt tgccctccac
181 catgatcata agtttcctga ggcctcccca aaagctgatg tcaccatacc tgtacggaat
241 catgggccta tggaaccgtg agccagttag cctcttttct ttataaatta cccagtctca
301 ggtatttcct catagcagtg tgagaacaaa ctagtaaaca gtgtaaatgc tttgtaaata
361 gttgttagtt aaactgtact gttttgggaa taatgacaaa gaaaagagtc tgtatgtatt
421 cagtacagag gcagccatcc attttttaaa aatatcttta atccacagat gcagaaccca
481 tgagtgtaga gggctggact gtatacatga atacatattg acatctttat ttctatatct
541 atatatccaa atatattttg aaaatcatga gtttacactg atacctctag tttgaatccg
601 ataacacagg ttcattctag tttactccct tttcatattt atacctttct tctctgacag
661 tgagaaatca ggcttccttt atcctcagta tattcactta ctggctaatt tcccctcccc
721 attgcctata aactactcat ctcccattgg tgctgccacc acaatccctc aatctcatat
781 gcatatgggt gacctcctca ctccacttgg tctctgacat cctgtgtgag ctccctgtca
841 ctgccctccc ccccatatgg gtgctcttct cacccctctt tggcttaaaa acccaattct
901 gggttgctac tgatacaccc aaacccagac acagatgccc tcctcactcc actggggctg
961 caatccctca gactgggctg ctgccactcc tgcccaagac ctacctcact ctgcccttct
1021 taatgacttt gaactgaact aatcaaggag gaaggaagga aggcacgaat caactaaaat
1081 atctttgaga caatttggaa cataagtaaa tctattttat agctctttag aacagctaaa
1141 atgttcttca tgatctcacc tggtctactt atggtggagt caggtgagtg cgctggaata
1201 gtgtgctctt aatgtacaag catatgcttt caatttacaa gaggaagcct tcactgaaaa
1261 ttccactgtc aagcagcact gatacatggc cttgtaaaaa gaaagctctg tttattggag
1321 tcaacacaat tattgtatcc agggtaaaga ctttttttaa atgaaatctc ctaaaatgat
1381 ctttgagttc gagtcatagt aaggaattga tgagtcgtgc caaggaggcc tataatgtta
1441 tagaagacaa gctgggtaga aagtatttct ctctcagaat gaattgatac ctgtagcaac
1501 accacatcta atagaaaaac aatgttaaag catctcaaag cactactgaa aatgaaaaa
1561 cattactata gtgaaaaaat attgaaagaa taattagaaa tacaagcacc agcacatata
1621 actacttcta aataatgtat ataacaattt ttggtaatgg tggccagtct acttatgtgt
1681 agactgagaa agaaatataa atatatgtat ctatgtacat ccttcaaaat atatagaaac
1741 tgtgactatt ttaactattt gtgtcctaaa actaaaaggt acagcattaa aaatatagaa
1801 tttttttcta tcaaagtgac atataatcac tataataaat atgatacagg agaaagagaa
1861 aaatgcaatc atgtattgca ttttttaatt aaattaaact tctgccaccc aggtttaact
1921 ctatctaata ttttggtata tttcctttca atcatattgt ctgttttcag aaatgggatc
1981 atgctataca tactgcttat gttttgaatt ttttcccaat gacacatcat aaacatttc
2041 ccatcacctt ggatatttga taacataatt ttaatggcta tgtagtattt tgttgtatag
2101 acacaccata atttatttaa ccaattttct attgtcgggt atttaagtgg tttctaattt
2161 tggacattac aaatgatgtg gggggggggt tgggttattt tattttattt ttttctgaga
2221 cagggtcttg ctccatcact caggcctgag tgcagtggtg tgatctaggc tcactgcaac
2281 ctccacctcc caggctcaag caatctccaa gtagctggga ctacaggcac atgccaccat
2341 tcccagatac atttttcaaa atatttttag tagagatagg atttcaccac gtgcctaggc
2401 tggtctcaaa ctcctgggct caagcaatcc acctgcctca gcctcccaaa gtgctgggat
2461 tacaggcatg ggtcaccacg cttggtcaaa aatgttttaa taaacaactg tatataaatc
2521 tttgcttgca tctatgatta tttcttcaga ataaacgttt agtagcagaa attgagttgc
2581 tagagcaaat agtgttgtgc aaaattatgt taacagatca ataggatatg cagaagagta
2641 atatactttc ctaaagtatg tgacagttgt tgataaatat aattaatttg aattgtcagt
2701 gttttggttg aattgtgtgc ttaacaatct ctaggaaaac taaagaacaa actgtctgcc
2761 aaaagtctca gagaaacagg attattactt tgatgctggt tccaagggcc tcccttagct
2821 ttcctggtaa taaattattg tctcatatca cactggcaga gaaaacagta aaatatttat
2881 tttggaatat ctgctttcta tctggcaggg atttgacatt ttaaaacctt ttcaaaatat
2941 tttagaacca caaaatacag taagctttga attaagaatt aattagggtt ttctttatcc
3001 ctactgtttg atcaattttt caaaagccaa aaaaaaaaaa gtgctacaca gaatacaatt
3061 aactgttagt tttcagtaat tttaaagtgt gctgttgctt agaaggcaga ctggatcaga
3121 tgacctcttg ggatcctttc gagctcagta attctaactt ttatattcaa tgagttttat
3181 cttgcactta tcacccattg ctcaacctgg ccctttcctc tttaccacat attttcttta
3241 tgtcttaatt ttccttttat actcacagag ggtgtctaac cctttattgg tataattatg
3301 gaaatgtttt tatgtgaaca ttaatctaac aatgaaggag acaagagcag aatgccaggt
3361 tcagtaatgc ttatgcagtc atgagcatga aacactctta gaacatttta ttttatttat
3421 ttactttttt aagacagggt cttactcttt tgcccaagct ggagtgtagt agcgcaatct
3481 cggctcactg caaaaccccc tccccggctc aagggatcct cccaagtcag cttcctgagg
3541 agctgggatt agcatgtgtt accacacctg gctaattttt gtatttttag tagagctggg

3601 gtttcatcat attggccagg ctggtctcaa actcctgacc tcaagtgatc catccatctc
3661 ggactcccaa agtgctagga ttataggcat aagccaccat gcctcttaga acattttaaa
3721 aaattaagtc ttggcttgaa atctgatgta gtgtgactat acttgagaat aataggaaat
3781 ttcccaaatg ctaaagactt aaagtacaag agaaagtcag tgttcttttt tcatacataa
3841 ctttctacag gaaagataaa agaaaaacta tcacaatatt atgtattatt tatcctcgct
3901 agtttatcaa acaacagttt tgtaatttaa tgaaattcct gttttctata tgagtatatt
3961 gctctaacat atgctgtata tgattaatgt ttcatttgtt tggctagtta ctgctcattg
4021 ctctgtgtgt gtgtgtgtgt gtgtgtgtgc gagacagaga gagaccatgt taaacacagt
4081 attattcacc tgttgttcgc ctgttggtta acgctctggt atccatgtcc cacttaacct
4141 ttcccaacag tctacacttc tattggaaat ttattgttac tctaccccca actatcccat
4201 tatttcaggg ataaaatgaa tgacatgtac tgcgcccaag aaagtcttat tgccctcatt
4261 gcagtgatta gatcagtcat gggtctgtgg tctaagtcaa gtgcgaattt caggattttt
4321 gctgacaatt ctggaacaca tgtttctttc tttctgtgta gtgtgggatg tggatgtgaa
4381 gggagaactg ctatcctcac tttgccacca tatagtatgt tcacctcaga atgaatcgaa
4441 cacattttct ctgactttac aatacaaaaa aatgtttttg aagaattgcc aggcactgtg
4501 gctcatgcct ctaatctcaa cactttggga gaccaaggca gggagattac taaaggccag
4561 gagttcaaga ccagactggc caacatggca aaatcccatc tctactaaaa atacaaaaaa
4621 attagccagg cgtgttggtg tatgcctgca gtgccagtta cttaggaggc tgaggcatga
4681 gaattgcctg aacctgggag atggaggttg tagtgagcca agatcatgcc actgcactcc
4741 agcctgggtg acagagcaag accttgtctc aaaagaaaaa agaaaaagca aacaaaaaag
4801 aagaaattgg gcaggcccag tggctcacac ctgtaatccc agcactttgg gaggctgagg
4861 caggcaaatc acctgaggtc agaagtttga gacaagccta gtcaacatgg taaaaccctg
4921 tatctactaa aaatacaaaa attagtcggg cgtggtggtg cacacctgta atctcagcta
4981 cttgggaggc tgaggcagga gaatcacttg atccccggag gcagaggtta cagtgagctg
5041 cgactgcacc actgcacact agcctgggtg acaaagcaag acttgtctaa aaaaataaat
5101 aaataaataa atccaacaca caagaggcaa aattaaagag aaatttaacc ctgattgatc
5161 tgtacctaaa agaataattt ccctctattg tataaggaag tttgaattaa gcttttggtt
5221 acttgcaact gaaaataaaa ctactgcagg atctcagacg tggatatggc cttggaagca
5281 cagaagaaga caaatggttc tggccttctc cagaaacaat accttgtaag aacttcttgg
5341 tttaggaaga gactaaggaa agtataagaa gaggagatct tttactttca ttcattgtta
5401 ctgacttttt gcttagttgt tctgttcatt attgagaagt atgtttaagt ttccaacaat
5461 gattgtggat ttgtctattt ctgtctttag ttctgtcaga tgtttgcttc acacattttg
5521 aagctctgtt aattaggcac atatacgttt ataattgctc cattttactg atgtatttga
5581 cttttttatt agtatgaagt atttgtcttt gtctctagta atacttcttt attgaagtct
5641 attttgttta atcttaataa aatcactcca gttttctcca gttttccatg ctacatattt
5701 ttctatcctt ttacttttaa tctatttgtg tctttgcatt taaaatgcat ctcttgtaga
5761 caccttataa ttggttcttg attttttttt aattcagtct tacaataatc tctgcctttt
5821 ggttgtttag ttcatttaca tgtaatgcag caaaggccag gcatggtggc tcacacctgc
5881 aaacccaaca ctttgggagg ctgaggcagg cagatcgctt gagcccagga attttagacc
5941 agcatgggca acatggtgaa accccatctc tactacaaat acaaaaatta gctgggcatg
6001 gtaggatgca cctgtagtcc tagctaccca gaaggctgag atgggagaat cacctgagcc
6061 tgggcagcag aggttgcggt gagccaagat catgtcactg cactccagcc tgggccacag
6121 agtgagaccc tgtctcacaa acaaacaaac aaacaaaaac aaacaatatt taatgctaca
6181 aagaaagtta acacagcaga ttatttttat ttacttcttg cttatttctg tagctataac
6241 agtgactctt ggtctaatgc taagaactaa agtactgtaa tgtttacctc gtactaggta
6301 atgatgtcat tctatacacc caatccagta ggtcaatatg aaccaggtgg tcagcagatt
6361 taaagtaaac atgaagattc atgatgtgca cctggtacct gctttggggt atgcacaatg
6421 gctactgatg tggcagttat gtattaatgt gacacgctct cttcaagaat cttggactcc
6481 aagccccaaa tgggctttcc tgaatatgga aatcttgtat atgtctttac aatttgaagc
6541 tggagagagg attacaccag tgtgataatc ttagaaaggg aggactcagg aacctgcaca
6601 tgatctctct agcctttgcc taggtatata tttttcctgc tgttcctgct ctgttatctt
6661 gtgctatgat aaacttcagc tatccggttt aacttcatgc taagttgtgg tgagtcctcc
6721 agtgaatcac ccaactagtg ggtgattatg ggacccctga gacagatata attattgaga
6781 tggtggtatt tatttatttt tttatcttta cttttatctt tgagacagac tcttgctctg
6841 tcacccaggc tggagtgcag tggtgtgatc ttggctcact gcaacctcca cctcgtgggt
6901 tcaagcaatt ctcttgtctc agcctcctga gtagctggga ctataggtgc atgccactat
6961 gcctggctaa ttgttgtatt tttagtagag atggggtttc accatattgg tcaggctggt
7021 ctcgaactcc tgacctcagg tgatccacct gcctcaacct cccaaagtga aagttctggg
7081 attacaggta tgagccaccg tgcctggctg agatggctgt atttagatct gccattctgc
7141 tatttattga ttacttgctc agttattttt tgtttctctt ttcatgcttt ggtttgtgtt
7201 aatcaattat tattttattt tagtgtttaa tttgttcttt tctaataatt tctattattc
7261 ttttgagatt tcctatctgt ttactacctt tcatattttc ctataattct tacaatatat
7321 tttcttttaa tcttttgaca attttaataa tagctacttt gaagtctttt tatgttaaat

7381 tcaacgtctg ggtccacttt cagtcaattt ctattgattg tctttttgcc tatatatgga
7441 tcacactttc ttgttttttc agatgtctaa tacattttgg ttgaaaagtg gacattatag
7501 ataacatgta gtagctttag aatctattgt gttctgaaga ttattagtca tttgttgtat
7561 taggcagttg gaagttaact cacctggaat caaattgcaa actttgtctt ccctactatt
7621 caaatgctaa tatctttgcc tgtttttata gtctccagct gctgcttttt tttttttttt
7681 tttttttttt tttgagacgg agtctcgctc tgtctcccag gctggagtgc agtggcgcga
7741 tctcggctca ctgcaagctc cgcctcctgg gttcacgcca ttctcctgcc tcagcctcct
7801 gagtagctgg gactacaggc gccagccacc acgcccggct aatttttttg tgtttttagt
7861 agagactggg tttcaccgtg ttagccagga tggtctcgat ctcctgacct cgtgatccgc
7921 ccgcctcggc ctcccaaagt gctgcgatta caggcgtgag ccaccgcgcc cagcccagct
7981 gctgcttttt taaaaaaccc tggccatttg ggaggtttcc tcttttcctg cataatttat
8041 tagtcaacca aatatttgga cagagaatat actcagactt tatggcttct ctgtgactta
8101 cttgcttcta aggatgcctc ctcaattcta acttctttgc caaccttgga ctctgttctc
8161 ttacccatca agcctgcaag gctttagctt tctgctacct gagctgcaca ttcttggaga
8221 ataaatacac tcagctgaaa agcagaaaac tcattgatct tactctgggt cattctattt
8281 tttgaaagta aatttctctc tggtttctgc ctgcttttat actatgttct ttaggtctcc
8341 cacatgcatg ctcagtatag caggtgaatt gatccaatga gtttttaatt tcagatattg
8401 tatttttctc ttctagaatg tctagttggt tctttggtta tgaattcaaa ttttctttta
8461 acacacatct tattcttttt tatcaacttt catacatttt tgacatatca atcatggtta
8521 gtttcaaatc attgtctgct aaatccaaca tgatgtgtct acttctattt tctgttttga
8581 tttttaaatc accttttcct gtgtttttgc atatctagca attttgactg tattctgtag
8641 gatatatata aatgaatcat agaggctcca gatggcatct tactccacca tgagtttctc
8701 ttttcttctg tagatagggt gagagattga ttacctcaat ccaaccaggg gtttagctgg
8761 attgggacca cattgtaatt tcagtaagct aggtcttctc tggttcaccc ctattcctta
8821 gacatgttcc tccaaggctg tcgattgaga ccctagaagt ctttgtctct ttagtcctga
8881 aagagtacag cacattctat tctgctcttt gaagattcca agcttcaaat gtggcagatt
8941 gtcttgaggt agaatccagt tatgtgcttg aagcaggccc cttctctcta gtaggatttt
9001 gtctcctaaa gaatgcagaa ttattgattt cgttctactt cttcaaacac agcctcttag
9061 cttctttcta ataaccccag aattcaacta atgtttcaca gggacactaa ccacatttgc
9121 tgctctccaa atttccaatc tgtcatacca gccttgcaca gttttattgg tttctctttt
9181 ctccggcaga gtcccactgc atagctaagc ctgatgctcc ctcagtgctt agattcagca
9241 aagggctcaa tgaagaaaac agcaggtaat tatcaactta ctccagaagg acttcccatt
9301 ctctctggaa tttcaattca tctagtctgt gttgctctca cagctctttg atgtctaaaa
9361 aatgtgattt ttgtaattta tccagttttc tgattgttgt acaggagcac tatgcagcta
9421 tgacctactt ccacaagtgg aaagccactt atgggcaaca taatcaaatt ggtgtctatt
9481 ggctgggcac tgtggctcac gcctgtaatc ccagcacttt gggaggccaa agcgggtgga
9541 tcacttgagg acagaagttg aagacaagcc tggccaacat ggtgaatccc tgtctctact
9601 aaaaatgcaa aacttaacca agcgtggtgg cacatgcctg taatcccagc tacttgggag
9661 gctgaggcac aagaatcgct tgagcctagg aggcacaggt tgcaccactg cactccagcc
9721 tgggtgacag agtgaggctc tgtctcaaaa gaaaaaaaaa aaaaagccca aattggtgtt
9781 attgcagcca ggcatggtgg ctcatgcctg taatcccagc actttggaag gccagggaag
9841 gcagattgcc tgcctgggca atatggcaaa acccgatctc tacaaaaaaa tagaaaaatt
9901 agcccagcat ggtggtatct gcctatagtc tcagctactc aggagattta ggtgggagga
9961 ttgcttgagc ccaggaggtg gaggttacac tgagtcgaga tattgccact gctctccagc
10021 ctgggtgaca gagccagatc ctgtctccaa aaaaaaaaaa aaaaaatatt tttttaaagg
10081 aaaaaaattg gtgtttattg cagtgacaca aaaactgatc ttgatattag atcatactag
10141 ctatttgact ctagataatt cacttaatat ctcttaggga taatagtggc tacttctctg
10201 ggttcttata taggtcaggt gaaataatat ttatataaag cactttcaag atgcaaagtg
10261 ctttacataa aaagcagtcc tttcattcat tcaaagtcac ttattgaata cttaatatat
10321 gctagacact ttactaggag gtgggtggga gaaatgaata agattggaaa acatggtctc
10381 acaagaagct cgtaactcta gcagggaaga ataatacata attaaataac agcattatat
10441 ttttactatt gtagatgtag gtataaggta aaatggcacc atagagaaag gagtaactca
10501 gtatactctg atagcgaaga gaaattatag gacacttccc agagaaagtg aggatttagc
10561 caaatcttag ggacttccaa gtgaaaatta attcattgtt gtagagacta cctgttgtct
10621 acctgaatcc attctctatc tgttccatag aagacagtca tagttggtta aatagctatg
10681 cagctagaga atataatctc atcctgcctt gtacctaggt atgatatggc catatcaaag
10741 ttctcactta gatgaaaata agtaaacttt gccatgtatg gatcctggcc ttaaaactat
10801 atatatatat gtatattata gttttaagtt ttaaactata tatatatatg tatattatag
10861 ttttaagttt taaactatgt aaatacacac atacacacac acacacacac acacacacac
10921 acacacacac acattctcca tgctgcttct ctctttaaga gctagagtac aggcagtctt
10981 attcttaact tcgccatgca gacaaggata gcaacctagg agacagctga gcagcaagat
11041 gaaagaaact ccagtcccta aaaggtcata tgcgtcagag acatcctact aactctgact
11101 gctcacctct aaactgtttc aagagagaaa agtaactcct gttttattta agccactcta

11161 ttttggtaac tttttgttac agtatctcat ctgtacctaa ttgccctaaa taaatactta
11221 caaaaaacaa tcagaaaaaa gatgaacaac tcaaatggaa aatgggtgaa ggccatgaac
11281 aatttatgta gatggccaat aaacatatgg aaacaaatgt tcaaacttac gattaaagaa
11341 gtacaaatga aaataagatg tctttttacc tatcaaatga ttatatgtac ataatacaac
11401 ctagcaattc acttcaaagg gtatttattt tagaatatgg gaatatgttt agagttacta
11461 taatttatat aagtcagaat tcagaaataa cttatattct caataccagg ttactgagtt
11521 aaataaatta ttgtatagtg gaatgatatt ccatcattaa aaataatttt ggtataggcc
11581 caaataagag taatcccagc tgactggaca gtcatgtttg aatgactata tttgtagacc
11641 cagattctag atacagaaat acactttaag agattgggcc caatgcaagg atcaaaaaaa
11701 taattgtcat tcttttccaa aatcttatta gagggaaaaa acatccctag aagttatgac
11761 tgtaacctgg caactaaaaa attacatgat ttgtgcaggc ccctgcctga gaaattattc
11821 catgaactga agtttaactg aagtggttat gattcatcac agaggaccca taataaaaac
11881 atagtgagta tttttctgag gcttttcata accatgaatc aatgacacaa gttctggaat
11941 ttaactttga atgtggcttc aaattctaga ggacaagaag tgtaagtgaa ctttgtagcg
12001 cccttggtgt tgtgttggtt tgttttctca tgctcatcaa taatccacag aaagaaaaat
12061 aacaaatctc actttgctgt tgtgttgagt tgttgcctct tataaagtca ttacccaaaa
12121 gtaaatggag aatataagat aattggtcta aatttatttt aaactgttat tttttataag
12181 gccaaaccaa atgtgatgaa aatgataaga ttttaaaaca attaaatgaa atatggaaat
12241 gggaagacca tcttacttcg gtttcagaac atcctagtaa aatggctaag tatgtagagg
12301 catgtttatt gtggtttcat tgaaaggctt tgcctactaa agagtgtttg attattcaaa
12361 atatctcaaa atatatagtt tacagaaaga ttcggagaac tatgaactgt ggagggaata
12421 aaaccttttt tttaaagcca cataatgaaa ccaccaatga aacaacaatc tacttcacag
12481 caaagtggaa aaacatctga aagaacattt tcaaactcaa tggggtgaca gtgcactgtg
12541 aaaaattcct ccaatgaaat ttctaacatt gtttaagaat gctttgtgtt ggccaagcat
12601 ggtggcttat gactgtaatc ccagtagttt gggaggccaa ggcggaggaa tcatttgaga
12661 ccaggagttc aagaccagcc taggcaacat agtgaaaccc tgtctctaat agctgggtgt
12721 ggtggcatgc acctatagtc tcagctacgt gggaggctga ggtgggagga tcacttcacc
12781 ccaggaggta gaggctgcag tgagccccat gattgtgcca ctgcacacca atttgggtgg
12841 cagagtgaga ccctgtctca aaaaaaaaac ccacaaaaaa cagaatacta tgccttcata
12901 ttgcacaata acataattta tacttccaga aagacagcat ttattattaa aaacagcaaa
12961 tgacaatcat cattaatttg ttttactatt attttataag aaaatattta aatatgtata
13021 gctattttct tatgttgaaa aatatttaaa atatttttaa tagcaaataa tcaacattaa
13081 tatttgttct cagttcatac tgagatatta tgtaagaaaa atactaatgg cattgattac
13141 gatattatta ataagctaat atatgctatt agataaataa ctattaaaaa ttaaaaccat
13201 tatgcttctg acaagataaa gtacatctat gaaatattgg ggttttatat tcatatagaa
13261 tgttgctgtt gaaactagct tcacagagtt tatgaactca agaatatttt ttatttctct
13321 gatttttgcc agaatcaaca tgacatgaac gtgtacattg tatctctgtg aaagaaaaga
13381 ggtgcatatt actttgcaga tacttaagtt gaaatgttta tggaattagc taatttagat
13441 tcaccttttt tactgcatgc agatttctaa tatctctagt tctatgtctt tcaagtttta
13501 tttgagtaaa ttcattcttt ctagtttttg cttttaaaaa ttattttatg gaagttcatt
13561 tatctacatt agaggatctt cacagtatat taaggggaaa tggcaggcta tgagcttatt
13621 ttgtgcaaat atgatataag gatagaaaaa agtctatagc ggatactggt tgcacagtaa
13681 ggaccacttg aggagctttt tttttatttt ttgagatgga gtctcactct gtcacccagg
13741 ctggagtgca gtggcacgat cttagctcac tgcaacctct gcctcccggg ttcaagcgat
13801 tctcctgcct cagcctcccg agtagctgca actacaggtg cgtgccacca cgcctggcta
13861 attttttgta tttttagtag agacagggtt tcaccatgtt agccaggatg gtctcaatct
13921 cctgaccttg tgatccaccc gcctcggcta aacatccaat gaccaaaccc tacctcagcc
13981 tgattaagtc agaatctctg agtgggtgtc ctagacatct gcttcaatag ctccccaggt
14041 cagctaaggc tgagaactac tgatttagaa gcacgcacac agcctccctg aacacacaca
14101 cagagtacat ctacctgtaa tgtggatcac aggaagtttt atattcttct tttggcttat
14161 ctgctttgtc tacaataaac atgtacattt tcctaaaata cacatgtact tgaatgcacg
14221 tagatggaag ggagggagag aaggtgaaag gaaagatttt taaattaatt ttaaaaaatt
14281 ttttgtagag acaggagtct cactatgttg cccaggatgg tctgagactc ctggccttgg
14341 aaaatgcttc cacctcagca tcccaaactg ctgggattac agatatgagc ccctgcacct
14401 ggccaattaa aaaaaaaact gttttaaaca aatgaacccc acgtcaacac agcaaggatg
14461 caagacaaca gtgggggaaa cacacatggt gtctttctgc cgtcctcaca ggtggctgaa
14521 aaaagcttca ggagcagaag agtgagtcag aaggagctca catctgctga gtttctgtca
14581 tgtgtcattg cttaattttt cactgccttt aattcccaca cctacctcgc agggttgata
14641 gatgattttt ctcttataga tggggaaatg ggctctcaga cagatgaagt cacttgctga
14701 gtcgcatggc aagcactctg acatacgaac actgtctgag ccaaagtcac tacctgactc
14761 ctaaaactca ccttctgtgg tgaggaggag tggtggttgg caccagggct ctttcctttt
14821 gtgtaactaa cagaaagctg ttaatctttc ttgttttttt tttcactttc ctggaaaatt
14881 gatacttcat taaagctttc tagatgccac ttattctacc caagaccacc ttctgctgct

14941 tatttttaga tgactacaga ttcttcattc actatggaag cttcacaaat cactttgtca
15001 gacaaattgt ttgaccttcc actggagagc agttgttacc actttctaat aatggcccat
15061 aaacatattt tttaaaacca cccggattcc tttgaaaaca tggccaggac aaagtcttgt
15121 cacttgtgtc taaatgcacg gtaattttgc tgtcagtcag catgatttga cagctgacag
15181 cagttgatga taaacaacaa aagaaaagga gggtctaagt ggcaaaagct acagaagcca
15241 gacagaatca agcaagaaga agatatattt tagaaacaac agctctaata actctaataa
15301 gtaaattaac catttaaaat agtatgtcaa tcaagttttt taaataaatg aagctgtgga
15361 gtccagcctt ctttttcaca tgcttttttt tttttttttt tttttaaaga aaaccagttt
15421 tgctctgtca cccaggctgt agtgtagtgg tgtgattaca gctcacggca gcttcgacct
15481 cccaggctca agtattcctc ctgcctcagc ctcccaagca gctgggacta caggtgtgca
15541 ccactacacc tgcctaattt taaaaatttt atatagaaat ggattcttgc tctgttgccc
15601 aggctggtct tgaactcctg gcctcaagtg atcctcccac tttggcctcc ccaagtgctg
15661 ggattaccag catgagctac cacgtccagc ttcacatgtt tattaatgcc gatgctgtat
15721 atctacattt cagtaatata tatttttgat taaaggaaac attttaagtt aaaaggatca
15781 gcctgagcaa tatagcgaga ttccactaaa atatatatat atataaacta gccaggtgtg
15841 gtgtctgtgg tcccagatac tcaggaggct gagttgggag gatcacttga gccccgagtt
15901 tgaggctgca gtgagctatg actgtgtcac tgcactccag cctgggcaac agagcaaaac
15961 cctgtctcaa ataaagggag tgggggaagc attctgattt cttcctgacc ttgttcttta
16021 tcgcctccct caagttcaca ttatttattg gttggtggtc ccaaataact ttagaacagc
16081 atagtggaac cccaaataac ttcaaaggca gactggctta tttgttcaca gaatttcaaa
16141 tatctaagat ggtgcccttg accaattgct tttccctctg atcacatttg acctctgtct
16201 tctcagacac ttttattgca catgatggta taaaaaagag aacataagct ctggagttgg
16261 gctgttctgg atttgaatcc tggcgatgca agttattggt tatgggagct tgacaataat
16321 tgttttattt ctctaagcct tagtttctac attgtcaaag ttgaaagaaa actttttcca
16381 tgcactcctt agaaaaatgt cagactaaac acgccaagtg cctagcccag tgcttggtac
16441 agggtaggca cttaaaaaat gttagtttac atcccacgcc tgattaactt taatgttgct
16501 cccatcctac ttccatcttc agcatattat cattaagaca gtggttctct ctgaatgtta
16561 tgctacatcc tccaagagac tacatgctcg ttggaaatga agacaatgcc cagtctttta
16621 taaagatctt gtaagccctg gctccaatga tggacagtaa ctgatatgca tggctaaccc
16681 aaacttgtag cccagtgtaa tctgggtgta tgcctaggct agcgcttgtc atatcaacta
16741 gctattaata tactctgttc agaaaacctg gcaatggctg ttacattttt tagttctagc
16801 aagatatttt ccgatatcca gtaaaaacat agatttttta aaaactattc aaatcaatac
16861 acactaggca ggatcatttg aaagtgtgct ctctgcaaag tcagcaagtc aatattttaa
16921 ggagggtatt gattttttat ttgtaaacac atgagcccag gctactctac aggaccatgc
16981 tgtgggcctg ttcagagatg agaccacatc attaaaaaaa cgaaacaaca accaaaaagc
17041 tttgatgcgc tcttggacag gatccacggc aaatacacct tcaagtaaat tgccccgagt
17101 ctcaaagtgt tccagatgca acatgttatt ttacagtctt ggacagtttc caaactatct
17161 atcaactgaa aatgattcac tgatctatga tataaagttc tgtgtgcctt gagggaaaag
17221 atcaggactt tttgaaatct catctacctt tgtagatcag agcatatagt accatgaatt
17281 aagtaggtgt taaataactg tgggttggat tgaacttatt gatcaggggt gatatgtttg
17341 ttggcagaaa taattagtga aatactttat ttttatattt taatttaaaa agtgatgctc
17401 acaatcttac tgcagctgag atttttcaag gctggggtac tttccataag ttatagagct
17461 tgctaattat gtttggaggt ttgaatatag ttgcacatat ttagagtcaa attccttcta
17521 ttaataaata ttcttgcctg tgagtatttc tccttcaaat tctgtcttct tcagtttcaa
17581 acagtttaaa acataaacgg gcttaaatca agctacaaaa tgtgacttct aagccaacag
17641 ttgagaaaga ccatcttttc tatttctaca aaaaaataat gagatgattg aacatcattc
17701 caaatgggag tcatcacctg tcctaaagat tgctttaaat aaacatctca ttcagtgaac
17761 tgataaagtg gtaaattaga tacattgcta attactatgt tgtcctgtag ctgtaagtaa
17821 aagccaagag gctgggtgca aagtggctca cacctgtaaa ttctagcact ttgggagacc
17881 aaggcaggag gatcgcttga gcccaggagt tcaacctaga gtgagggacc aattacttcc
17941 tattgaaaag gcaaacatgt attataaaaa gctttgatat ttggccaggc acagtggttc
18001 atgcctgtaa tcctagcact ttggtaggcc aaggtgggtg gatcacctga ggtcaggagt
18061 ttgagaccag cctggccaac ataacaaaac cctatctcta ctaaaaatac aaaaaagtag
18121 ccagacatgg tggcaggcac ctgcaatcac agctactcag gaggctgagg catgagaatc
18181 gcttgaaccc aggagtcgga gattgcagtg agccgagatg gtgccactgc actccagcct
18241 gggggataga gcgagactct gaaaaaaaaa aaaaaaaaaa aagctgtgat attcaatgcc
18301 aacgaagctt cagtaagacc ggtgctctga ggcactgctg acggcaacag aaaaagatac
18361 tttcctggaa agaatttagg aagccacaaa aaagttccta gcctctgatc caacttctgg
18421 gatttcatcc taagaaattt gtccaaagag gaataaaagc catgtgcatg aacatgtcca
18481 ttgcagcaca atttatactg acaggaaatg agaagcaaca atatggcaat gtttaaggaa
18541 gttacagtgt atctactcga tggaatttta atttcaagaa caattatcgg cccctgtaat
18601 cccagcactt tgggaggcca aggggggtgg atcatttgag gtcaggagtt cgagaccacc
18661 ctggccaaca tgatggaact ctgtctctac taaaagtaca aaaattaggt gggcgtggtt

18721 gtgctcacct gtggtccaag ctactcagga ggctgaggta ggaggattgc ttgaacccag
18781 gaggcagagg ttgcaatgag ctgagatcat gccactacgc tccagcctgt gtgacagagc
18841 aagactctgt ttcaaaaaga aaaagaacaa ttatcaaatc aattgcaata taaaaacatg
18901 cttggaaaat gtcaaactaa caaatgaaaa ttgttaaata caaaggtatg tgttgtgtta
18961 tagcaacagc tatgtaatca atgaatataa ttcagaaaga ttagccaagg atacaccaga
19021 agtgatggca gttgctttgt tagaaatata gcagtatagg agtataggta atttaaaagt
19081 atttctattt cccaaatata ccctactatg attatattta ataagtttaa aatactacat
19141 aaggccttca gagaaggaaa ttaaatatct tttcctattt atttttccag tgagcaataa
19201 acttaacttt cagtgaagtc ttccttaaac acaacttgca tttctcccgc tttggcttaa
19261 gtatatttta tcttgctctg ttgtaaggat aaacatttcc atcctctcta tttgttattt
19321 tggctaagga caccacattt ccatgttaga atttcttcag tttttttcta ttgccttgag
19381 attgcatgca ggtatgttga gaactgagga ttttaattat aaatatacat agatttaacc
19441 taataaagga ctttcttttt ggaggcatgt ttgacaattg gctggttaat gtacaattgg
19501 ctggttaata atgcaatctg atggtgtact tataactcta aaattctatg gtagtttcta
19561 ggctgttctt gaaaggctta tcattttaag taagaaatca cacaaatagg ccaggtgcag
19621 tggctcacac ctgtaatctc agcacttcgg gaggccaaga tggaaagatc acttgaggcc
19681 agaagttcaa gaccagcctg ggcaacatag tgagatctca tcgctacaaa aattaagcaa
19741 agttagctgg gtgtggaggg acacacccgt ggtcccagct actcaggagg ctgaggtggg
19801 aggatcattt gagccctgga gttcaaggct gaagtgagct cagattgcac cactgcactc
19861 tagcctgggc atcagagtga gatcctgttt caaaaaaaag aagaaagaaa gaaagaagaa
19921 gaaaaaagaa gaaatcatga ccgggcacag tggctgacgc ctgtaatccc agcactttgg
19981 gaggctgagg cgggcagatc acctgaggtc aggagtttga gactagcctg gccaacatgg
20041 tgaaaccctg tctctactaa aaatacaaaa attagccagg cgtggtggtg tgcacctgta
20101 gtcccagcta cttgggaggc tgaggcagga gaatcacttg aacccaggaa gtgaaggttg
20161 cagtgggccg agatcatacc actgcagccc agcccaggca gcagagcaaa actccgtcta
20221 aaaaaaaaaa aatcacacag atatatcatt taagattaag gcagggatgt gggctttctt
20281 gtacgtgaga gctcttctca ccctcaaact aagtaaattg gtattattca gctttcctca
20341 tgtgattaaa aaaaaatcaa actttatggt tgtacactga aacttctcca ggttccctct
20401 tttcttttga gttgtggaaa cttgaactgt atttatgcaa aaataaaata tccaagatcg
20461 tattcttttg gggccaattc tgatggtggt atttgctatt gctgtctttg gatatctctt
20521 tagttctgca gaaaacttac ctactaataa tggttaataa tgtgtgcctc agcagcaaca
20581 gatgcaataa gattatagtt aagaacctgg gttcaagtac tagctctttc atttactagt
20641 tgtgttccca ttgttgaaca aataaataaa acaataaact tatatttcct attacttgtt
20701 aataaaagtg aatcagaaag cctaatgaga ataaatgttt taaaagatga tgagggagct
20761 ttaaaaaaat aaattttgct gcactctaag atgtaaaata tatgtaatta tatgtgcatg
20821 ttagtgtgcg gatatccagg acattgacac taaggtgatg gttatgcaat tgattgacac
20881 tgaaatatgt tggttcagtg ccactaagga gccagcctca gatcttaaac atctcaacaa
20941 ttactatgag tccatggggg ccatacaagt tgtaggctcc ttatcacatt tgtcttaaat
21001 agtatttttt aaattacaag aacccttagc ccagataatc catgttgtca tacagtgaat
21061 agatatcaga tgtgttgaca tgttggttct ctcagcccac agggcagcca ggcagggcct
21121 ggggatgtgc tcgatcccct gggaagacct ccttcagttg agagctacct tatctgttta
21181 tgctgatgtg ctgaacaaat ataatcattt tctatgtgtg catgtgatag actctggtct
21241 cactgcagat cacgtctaca ttcaagtaat atacttcgat ttgtcttgac ttttcacact
21301 agttctcatt ctagctcttc ctattttgtt taaattattt atgcttttct cacagccagt
21361 gctgcttttt caaatgtggg atgtccaagg actggagcat ctgttagtga gcagcaaggt
21421 tcctggcaat gtctgtatgt gcttggaggc atcattaaaa tgtccccgga actgtgactt
21481 agagctggat aagacaagag ggatatgtgg aatctaacca ggtctcagtt aggagacatg
21541 aaaagaattt cagaaaaaaa aaattaatct taaattattt taatataatc aaataataaa
21601 acaatctaga atgagttgcc gaattaatcc agccttgaca cttacgtaac atgttacttg
21661 gttacatgga gggcgtgggc ttggaaactt ccacatgccc gtcatggctg agagggaaat
21721 tgtcttagat gcctcagctt aagctcctta ccaaaatctc tggtatgata gtgggaagac
21781 tgaattgttt ttttcttgaa agtgaattag tcctaaaaag agctagaatt tagtacagga
21841 atgctttttt aaaagcaaaa aattagttct aatatttcag gtttatgttt gtggataaac
21901 tgtgactaat gtggtagctt taagtaatgt aatgtttaag aaattctgaa tatattaatt
21961 attcaataga ttagccaaat ggccctgaat ttaaaatttg aaacatcacc attgatccag
22021 acttctctta ggttgaaagc cttaagacaa ttttatgtaa tttataaggg ttcagcataa
22081 atacaagatt attaggttta ttatgcttat ttaaataacc aggaaggttt ctttgtaagt
22141 cagaaaacta aagaacttaa aaaatttgaa aataataaat gtataagtta cttaaaatat
22201 ttaacaaagt ttgcaaatcg gactaagcaa taatcaaagg tcaccttaaa aatgaatttt
22261 ataatattac cttatttata aattgaattt ttaaatatat aaaataaact taaatgtaag
22321 gggaaactgg gcttttgcat ttgttactta aatcaatcaa atgttaattt taaaaaccaa
22381 agtgagccct gaataatttg gaagagggtt tagagatgcc aaaaattcct gttgatcaaa
22441 agatatcaca tcgaaagggt gattaaaaca tttattccga aaggaaaatg tgtggtatcc

22501 attttgttaa aaatgataaa ttataatgtt ttatacaaaa taaatgaatc ttatcattgc
22561 ctccttatga cgaaattagt gtttttgtca ttaaaaatga atttgttagg tttgcagtct
22621 cattgatctc tcaggattta gaaataatta ttttttgctg aattaaagat agctctattt
22681 tttaattgtt attagcttca aaaagagtag caaattcaca ggagggaaag tttaagctca
22741 tttcggataa aaattgggat cttaagttta agcatgaagg acatccactc tcccttgctt
22801 atttcagtct ctctctcaga cacatttaga agcagaagag ttatgtatag ttaagttcag
22861 cctcccctgt tttcagtgag aaaaccaagg ctaagaaatg ttacataatt taagtaaaaa
22921 tatgtggtga gaggtacaat atggtgtggt tgatagttga caggatttgg tgtcataaac
22981 cctgttgggt tttttgtttg tttaagattt ttgttgttgt tgttgttgtt gttgttgttg
23041 ttgttgttgc tgggcgcagt ggctcacacc tgtaatccta gaacttcggg aggccaaggt
23101 gggcagatca cctgatgtca ggagttcaag accagcctga ccaacatggt gaaaccccgt
23161 ctctactaaa aatacataca ttagctgggc atggtggcgt gcaactgtag tcccagctac
23221 ttgggaggct gagccacgag aatcgcttga acccaggaga attccttgaa cccaggaggc
23281 ggaggttgtg gtgagccgag attgtgccac tgcactccag cctgggcaac agaatgagac
23341 tctggctcaa aaattaccat atgtttgttt cctaataata aacatttatt gaattcttta
23401 aatatttcaa tatgctttat atgtatttat ttcccttaaa caccagagca acctagtgaa
23461 ctaagtgata tccccatttt atagatggga aaactgagac tcaaagcgat taagataatg
23521 agaggatcac gtgagcccag gagtttgagg ctgcagggag ttgtgacagt gccactgtac
23581 tccagcctag gtgagagtga gacccttcta aaaaaaattt ttttttttaa tattcagaag
23641 atctgatttt aaaacttgcc ttcattacgt gtttgttgcc ttgtgactct gggatgtctt
23701 cctaccaaga ccttacccta ggttagctca gcaggttttt gtgacgagca aacataaaca
23761 agtataggaa aacactgtct aaaagtaata tattattcat tcacaatggg gtgaagccta
23821 gaacccattt attctgactc aatttaatgt tattttgctg actaattctt aatcaagtgt
23881 tctttacctt caaataattt tattttccat ctgtgcccgc ttttgcctat agtgatgaat
23941 atttattgag tatgcatctt gttcttggca ccacgtaagt atcacacacg tcaaggtata
24001 tgaaaacttg gcttatgtta tcaagagact tcatgggatg atgagaaatg cagaaaaaaa
24061 ctgaaatcct gatcttctct tttttccatc acttctctgc acctcatatt ttccatctat
24121 aaaataagtg ataataccac cacatgaaaa atcatagaaa gaatttagca acgtaactag
24181 taaaagtgaa tacatacaaa ccatatgtga tagaatggat gaatcctact aagaacagtt
24241 accaggtaac tgaagctaaa caactctcag aggactgagg gatgttacat ttctgaccaa
24301 gtagagtaga aacaccttgc taaatactta tgtattcact aaaggcccca gaaaagctgc
24361 acctttggag caaagctaaa atagggtact ccacaccgac cctaacaaag attaacaaca
24421 acctttaagc cggtcatggt gatgcatgtc tgtagtccca gctactcagg aggctgaagc
24481 aggaggacta cttgagccca agagttgagc ccagccttgg caatatagca gatccagttt
24541 tttataaaac aaacaaacaa acaaatcatt tgaaagaatc agtaagcctg ctagaaaaaa
24601 actcaaaata atattgttat gtttaaagga agattacaaa gtctagacac taaatgatac
24661 aaccttcacc atgtccatca tctaatccaa gattctaacg cagagaagca agaaaatgtg
24721 acccaaaaac aaaaccagcc ttttggccag aaccgtcatc ttccagtaat ttgccaaaat
24781 gatgaataca aaggcaaaaa ggaaaggcac tggatatatg ttctctaggc ctttacaaaa
24841 acatggagtt gttcctttgg ccacatatat gctaatctat aagaaaggtg atattgcaga
24901 catcaaggga aggggtactg ttcaaaaagg aatgccccac aggtgttacc atggcaaaac
24961 tggaagagtc tacaatttta cccagcatgc ttttggcatt gttttaaaca aacaagttaa
25021 gggcaagatt cttgccaaga gaattaatgt gcatattgag cacattaaac actctaagag
25081 ccgaggtagc ttcctgaaat gcgtgaagga aaatgatcag aaaaagaagg aagccaaaga
25141 gaaaagtacc tgggttcaac tgaagcacca gcctgctcca cccagagaag cacactgtgt
25201 gagaaccaac gggaaagagc ttgagctgct ggaatctatt ccctatgaat tcatgcataa
25261 taggtgttaa agaaataaaa taaaagacct ctggactatt taaaaaaaaa aaaaaaaaaa
25321 ggccaggcgc ggtggctcgc gcctgtaatc ccagcacttt gggaggccag ggcgggcaga
25381 tcacaatgtc aggagatcaa gaccatcctg gctaacacag tgaaacccca tctctactaa
25441 aaatacaaaa aattagccag gcgtggtggc aggtgcctgt agtctcagct acttgggagg
25501 ctgaggcagg agaatggcgt gaacccggga ggcggagctt gcagtgagcc aagatagcgc
25561 cactgcactc cagcttgggt gacagagcga gacaccgtct caaaacaaac aaacaaacaa
25621 caacaaaaaa aattaaacca tgtaaccaat agagatgtgt gtgtgtgtgt gtgtgtgtgt
25681 gtgtgtgtgt gtggtgttgt gtgtggttag gcaagcaaaa acaacaaaaa gcaagcaaac
25741 aataaatcta acatttagga tcaaaatatt atttgaggga gatgcaatgg gatgagacaa
25801 gaaaggcaca tgtagataaa tataggttac ttattctgat tgttgggttg gatgtttatt
25861 atattattga atggtattgt gaatggatca aggatgatgg catataaaca cccaaagttt
25921 atggttaaca caattctgtg catctgaagt gcaggagaac aaggtgggaa gtaaaaaaga
25981 agagtagaaa agaacacagc attatgaagt ctgtggcaga aaacaatata actttctaga
26041 gcataaaaaa aaggccaatg tgagcacggc tggaactgga ggacgttatg ttaagtgaaa
26101 taagccagga acagaaagtt aaacaccaca tgttctcaca catatgtgga agctaaaaaa
26161 aaaaaaaatg ttgagctcat agaagtaaaa agtaggacag aggataccag aggctgggga
26221 gggtcatggg aagggtgggt cgggggcagg atagggagag atttgttaaa ggacaaaatt

26281 acaggtagat aggaggaata agttctattg ttctatacca ctgtaggtaa tacatagttt
26341 cgaatagcta gaggaggagg aggatacaga atgttcccaa cccaagaaat gaccattgtt
26401 tcagatgatg gatatgctaa ttaccctgat ctaatcacca tacattatgt ttcaaaacaa
26461 tactatatac ccccatgaat atgtacaatt attatttgtc agtttaaagg tttttaaaag
26521 attaaaatta aaaaaaaaaa gaaaaaaacc cggccgggcg ccgtggctca cgcctgtaat
26581 cccaacactt tgggaggccg aggcgggcgg atcatgaggt cccgagatcc agaccatcct
26641 ggctactttc gtgtctacta aaaatacaaa cagggcctgt agtcccagct acttgggagg
26701 ctgaggcagg agaatggcat gaacgcagga ggtggagctt gcagtgagct gagatcgggc
26761 cactgcactc cagcctgggc gagaaaaaaa aaaaaaaaag aaaggaaaga agaaagaaag
26821 aaagaaagag agagagagag agagagagag agagagagag aaagaaagaa agaaagaaag
26881 aaagaaagaa agaaagaaag aaagaaagaa agaaagaaag aaaaaaggcc gcccggtgcg
26941 gtggctcatg cctgtaatcc gagcactttg gaagaccgag gcgggcggat cacgaggtca
27001 ggaattcaag accagcctga tcaacacagt gaaaccccat ctctactaaa aacacacaaa
27061 aaattagcca ggtgtggtgg ctggtgcctg tggtcccagc tacttgagag gctgaggcag
27121 gagaattgct tgaacccaag aggcggagat tgcagtgaac caagatagtg ccaccccact
27181 ccagcctgag caacacagtg agactctgtc tcagaaaaaa aagaaaaaaa aacatttctt
27241 gtgtgcctga cacatagaaa atcagcaaga tgtatctctc agggcagact ctcactgact
27301 catcccatag aggctgtcat tcaagcattt attaaatcat gatgtgaaat gcaacagtca
27361 taaattctgg aatgtttaac ccatttagca cgggggaggg tagaaaagtc acatagtagc
27421 actcaataaa tgttagttca catccccttt ataactgtaa agttctgcaa aggcaatact
27481 agtgtttatt ttcgtatgga gaaataaaat agaacaagca tgatatcatg aaaacaacac
27541 ttaaaatgag agttctagct ttgccaataa ctagctgtat gatattagga aaattattct
27601 cactggactc catctgtagt aactggagtt tggtatattg gaaatttcca agtgagttcc
27661 acaaaacact aatgtctttt aggatgttaa taggagctaa atgagaaaga atctgtgatc
27721 aagtcctatt tgtaagtgga ctgaatgttt gtgtcccccc cagattcata ttttgaagtc
27781 ttcacctcct gtatggctgt atttggagat ggggcctcta aggaagtatt taaggttgaa
27841 ttaggtcata agggtgcaga actgatccaa ttggattagt gtcattaata gaagagacat
27901 aagaagagca cctctcactc tctccaatat catcctccaa gaaaagaccc tgtgaagaca
27961 aaacaagaag gcaggtatgt gtaagccagg aagagagcct tcactggaaa cctaacaagt
28021 cagcaccttg atcttgaact tccagcctcc agactgtaag aaaattaatt tttcttgttt
28081 aagaccctag cccatgatat ttttttatgg cagcccaagc tgccaaatac ggttttatgt
28141 ataacccttc tcaaagattt ctaatactcg gagaagttct tttttttttt tttttttttt
28201 ttttttttga gacggagtct cactgtcgcc caggctggag tgcagtggcg caatctcggc
28261 tcactgcaag ctccgcctcc caggttcacg ccattctcct gcctcaatac tctaagaagt
28321 tctacagtga aaaaaaaaaa aaaaagaaag aaagaaaaca agggaaattt atctaatgta
28381 ctgcttctga aacttatttg tctattaaac acttttttaa aatcaccatc ttttgaaaca
28441 gggcttggga aacactagcc aggatttttc agttctgtca gttctgaaac taggattcct
28501 tctaaaacct atacgatgta actgataaag ttagatttaa acatgagtga gactccactt
28561 ataaactagg ttataacaag gaaattcaaa ggagattgta aatgcagagg cagcacagtg
28621 aatgaagtaa acacttgaaa aacagcatca tatgtcatgc gtggtggctc atgcctgtaa
28681 tcccaacatt ttgggaggct gagttgggcg gatcacctga ggtcaggagt tcgagaccag
28741 cctgcccaac atggtgaaac cccgtctcta ttaaaaatac aaaacttagc caggtgtggt
28801 ggcagacacc tgtaattcca gctactcggg aggctgagac agaagaattg cttgaaccca
28861 ggaggtggag gttgcaatga gccgagatcg caccacttca ctccagcctg ggaaacagag
28921 ctccatctca agaaaaaaag aaggaaggaa ggaaggaagg aaggaaggaa ggaaggagag
28981 agagagagag agagagagag agagagagag aaagaaagaa agaaagaaag aaagaaagaa
29041 agaaagaaag aaaagaaaga aaggaaagaa agaaagaaag aaagaaagaa agaaagaaag
29101 aaagaaagaa agaaagaaag aaagaaagaa agaccatcac tgctttgtgt ggagacagct
29161 tctgtgttct ttcaggacaa cttgagaagt tttaccaaca aaaactctcc tgggatctcc
29221 atttgttcat tccataaata ttgagtgctt acggtgtgtc agggactgtt ctaaacacta
29281 gcatgataga tggcatcatg agacaccaac gtgtgtcaga agagagctct cattgcaaac
29341 actaatttgt acagattttt aattgcagca tgagcaaaag tagctagcaa ctctgctagt
29401 taccagcaga tggcagtagc agtcacttct ttactggcat tttagattgg gaatgtgtag
29461 aagcaccaca acctatcatc agaactactc tgtggtgtca atgtgtgact tagggacagt
29521 tatctaaccc gtctggagtg aagcttccct atttaaatac tagagataat aaaagcacct
29581 acctcttaag ttgtctcgag agttacatga gataaaacac ttgaagtgct ttatcttcaa
29641 catgaaggaa gggttaataa atgctagtta ttacttagta tggttgatac tgtttttttg
29701 ccatttgaca aaatgtcaat tatccaaaag aggaagagaa gcagatggct aaaagttgta
29761 ttgcttcata gagcaataac aatatcagca ataaaggaaa agaaaatcta aatatctaaa
29821 aatataacaa agggtgcaat acaatcttaa ggtaatcgct agaaagaaag caaaaatctg
29881 agcaggtttt taaattagta cttttccaac aggtcacttt taaagtattt tgactgtctg
29941 ataactccct acctgcgctg actcaacata attgtgaatt atttgtaaat ttcctattca
30001 tcaaagatgc agcagatttg cgaacagagt ttctagctag atagccaagg gaatcatggg

30061 gctattagac agctaacttt ttttcttaag cttattgttt attggaacca aagataagtt
30121 gtttcaaggc taggacctcc ggaatgactc agttaaaatg agccagaatt cagaatttag
30181 aagcctcaga gccagcttac ctcacttctt aaaaatattt ggaggaaaac actagggaac
30241 tgagttgtaa tggggaggtg ggtaagagga gtaaaaccct cttatatacc cttgagagct
30301 gtttgaacag caaagattcc caaaacaggg acaaactatt tctagggata atctatatat
30361 gcaaatataa ctgtttattg aatgaattcg tccatatggg aacgattatg atgcttcagg
30421 acatggatga tttcactaca atctcaagag ttcagttaaa gaggaaaact ttccatctgc
30481 aatccaaaac atcccacact tttctctagt aacaaatagg gaggtctcaa aagacaagtt
30541 atccttttta ggagatctcc acagcctgaa ctaatgagca actgaacaat aataatctca
30601 tttttatgac aattcatgtg acgacattga acaatgggaa aataggagat aagcaatgct
30661 gatgaaatgt gtcagtggct tagcatcatt gatggctatt attattttta cttcttatgt
30721 ggccagattt tttaaacaga aaatttggaa agatttctca gactgctatt ttctcttttt
30781 aggttgactt atttattgcc cccacttcag aaattacttt ttgaaattgc cccagatatc
30841 aggttccaga ggacagaagc aaacaacaaa ggttaatcct gagtatacag atattaataa
30901 aaatagaaaa gaaatgtgaa aaataagtac attcactaat aaaagtagtt tagtattctt
30961 tttgtgattg atcaatatgc taaaaataca acacacacac acatacacaa caaagttagt
31021 aaaactaaac aaaaacacgt atcgcccatc attcagcatt cacaatacaa tgatttgctg
31081 aaatttctct taattttttc tatgttttat gccaacttta ttgtggtagc agattgtggt
31141 atattccctc ccctagagca tggaagggac ctgtaaatat gatgggatct ttcattatta
31201 ggttgtgtta tatgtagtac agatgacttt aagaaagagg gactagggtg aggaggaaag
31261 ggagggagag aattaaccta atgcatgcag ggcttaaagc ctagatgaca agttgatggg
31321 tacagaaaac aaccatggca cttgtatacc tatgtaacaa atctgcacgt tctgcacatg
31381 tatcccagta cttaaagtta aataaataaa taaataaata aataaataaa gagagagaga
31441 gagagagaga gagagagaga gactagccag gtgcagtgag tggctcacgc ctgcaatccc
31501 gcactctggg aagccaaggc aggcagattg cttgagtcca ggagtttgag accagcctga
31561 gcaatgtagt gaaaccctgt ctctactaaa aatacaaaaa tgttagccac gcatggtggt
31621 gtgcacctgt aatcccagct actcaggagg ctgagacagg agaatcactt gagcctggga
31681 ggttgaggct gcagtgagcc aatattgtgc caccgcactc cagcctgggc aaccacagtg
31741 agaccctgtc tcaaaaaaaa aaaaaaaaaa aaaaagagag agagagagaa agagagacta
31801 tacttggtgg tcctgacctg tccaggtgag tccttaaaaa ggacaagtct ctttctggtg
31861 aaagaggtta gaagcatgag aggagtttga tgcaagagag attctagctt tgctcacttt
31921 gaaaatggaa aggggccaca tgccaaggaa agtaagctgg ccctcggagc tgagaatggg
31981 ccctgagtga cagccagcaa ggaaacagag atttcagttc cataatcaca aggaactaaa
32041 ttctgtcaac atcctgaatg aggtacaaag cagattcttc ccaataacct acagaaataa
32101 acatttctag taatatatat atcattctga gagttatttt atcttttctg atttccaaaa
32161 atagaatccc catggaatct gaggttagac aactgtatgc ctcctgttac cacaagtaca
32221 cactcttttg tgtctagatt tctttttctt agcataatgt ttttgagatt catccatatt
32281 gccatatata atattagttc atttcgtttt attgctgagt gtttctctct ctttatttta
32341 ttttatttat ttttttgaga caagatctca ctctgtcacc caggcaggag tacagtggtg
32401 ggatcatggc tcactgtagt ctcgaccttc tggactcagg tgagtcttcc atcttagctt
32461 cccaggtagc taggactaca ggcacacacc accatgccca gcaaattttt tttttttttt
32521 ttttgagacg gagtcttgct ctgtagccca ggctggagtg cagtggtgcc atcttggctc
32581 actgcaagct ctgcctccca ggttcacacc attctcctgc ctcagcctcc cgagtagctg
32641 ggactacagg tacccaccac cacacctggc taattttttg tatttttagt agagacgggg
32701 tttcaccgtg ttagccagga tggtctccat ctcctgacct cgtgatccgc cctcctcggc
32761 ctcccaaagt tccgggatta caggcgtgag ccaccatgcc tggcctgctt aggatttctt
32821 atgtatacag tcatgccatc ttcaaataag acaggctttt cttcctttct gcccattatg
32881 tctttcattt cttattcttg ccttattagt attgctaaga cttctagcat aatattgagt
32941 ataaatagga agagtagata ctcttgtctt ggttctgatt taaggaggga agcataaggg
33001 ggactaaatg cttccaccag taaatataac attacttata ggttttccat agattcaatt
33061 tattaatttg aggaaattct cctctatcct agttaatgag agtttttatt ttgaatggat
33121 gttgaatttt gccttatggt tgtttgtgtg tgtaaagaga taattgatgt gactgttttt
33181 ccttttcttt atgggatggt ttttattttg aaattcaaat gtctttcgta gatttagggc
33241 ttttcagatt ttttctattt cttcttgagt cacttttgac atttttatct ttcaagggat
33301 gtgtaggttt catttaacct gtaaaattta ttggcataat gttcttcata atatctccaa
33361 tattatccct tttaatattt gtaatcatgt ccccctctca ttcctaatat tggtcatttg
33421 tgtcttctat ctttttgtct tgatcagtcc accaagtgtt gcataaaatt taataaattt
33481 ttttgcaaag acccagatta agtttactaa atttaaaaaa gttatgggtc caatttcaat
33541 ttcattgatt tctgctatga cctgtattat tttctttctt ctatttactt tggattttat
33601 tttctcttct tttcccagct tctcaaagtc tccaccagat gcccaaaatg agaacagatg
33661 acttcccact tggactggtc agaactcaaa tatctctctg ccctgtgtga gctctagaag
33721 tagttcacct tacaattctc cagtggtttt tgcctgccct tgtggagttt caccttattc
33781 ctgtctggcc acatacacag caaagactca aagagacccc tatgcagatt tttggagctc

33841 ttttcagcac agctccctca tctctagaac tctacctcgt aacattcagc cccttggcc
33901 tctctgatcc ctacatctgc ttgactcaat agactctgtt taggatcccc ctctttgcat
33961 ctacagtctg gttatttcca ggaagatatc aggggcaatc atagtgatca ccttgtttgt
34021 ttcaacttct tttagtgatt aaatcttgtc ctacctgatg tccaatgttt gaaaaataaa
34081 agctttttac gttctgcata tttttctggg tttttgtggg agagaatgtc caatccttgc
34141 tattccattg cagccagagg cagaatttct ctcactacag atttttgaag ttttctttta
34201 cttccatgct cttctatgtc ccttgttggg gtttgtttgt tttggtctct ggctttcatg
34261 gtagaggctt tcctcaaatg tcagtgtgcc tttgatgtct gtatttacag tgaggcataa
34321 caaagctgat ctgaagcttt acatgaatgg gcaggctttg tcaaatggtg ggtctcaatt
34381 gagggggaca ggtggggact tgacctattc actggccagt ccctgtaggt cagtatctca
34441 taggtttttc atttaaagat gattaataac ataggagagg aattctccaa ttctctgact
34501 ggaattataa gccttgctgc tagacttctg ataactaaac agagaatgaa tttttgaggg
34561 gcttatattt aggaatattt ccctttccat atggtatggc ttctcacttt caccctctgc
34621 tgttccaaga ctaggtcttt tttggtttaa cctctccaaa gagttaactt cccatctgag
34681 gcagctgtgc agggggtgca tagactggct gtgtgaggta ggggagggaa tctatggagt
34741 gtctagagct tttaaaatct gtaccaatcc tccttcattc tgtacttccg catttagaga
34801 taacttatgt atctagttcc tgagattttt cttggattct gctatagaaa ttaacttgct
34861 ttttattcac tctctccccg gagggttaga gtttatcttt cactagtctg ctaagtcagc
34921 taccatgcat ctattagctt tctcagatcc aaaatgttga attttcttgt ttgttactat
34981 ctcctatttt ctttgtcctt gtggctttat gctttttttt tttttttttt ttttgagatg
35041 gagtctccct ctgtcaccca ggctggagtg cagtggcatg atctcagctc actgaaatct
35101 ctgtctcccg ggttcaagca attctctgcc tcagcctcca gagtagctgg gattacaggt
35161 gcccaccacc atgcccagct aatttttttt ttttgtattt ttagtagaga gggggtttca
35221 ccatcttggc caggcaggtc ttgaactcct gaccttgtga tccacctgcc tctgcctccc
35281 aaagtgctgg gattacaggc ataagccact gcacccagct gctctatgca tttttatata
35341 aattattagg cttctttttt ttgaaaagtt aaaaactctt taattcttta ttcctggtac
35401 cactatcaca atttacaggg ccatacacct gatgtaacga aaagaaaaaa aaaagttaca
35461 acagataaaa gacttcagga atgtacatct aattgacact acattgcatt aatcaatagc
35521 tgtatttttt gcaaactgtg gttatgacag tcctgaacaa gaagggtttc ctatttaagc
35581 tgcagtaact tttctaacta tggataattg ttccttttat ggcagatttt tccagttcct
35641 ctaatgcatt tgagacgact gtctcaaagt aacctgctgc tttcctggta actcctcact
35701 ttaactcctg ctaggaactg aagccctttt ttgctgcttt ttagaacctt ctgttattat
35761 atccaccact tttaccacca gatccataat caccaccata gggactgccc gagcttcttc
35821 cacccagaaa gcctcctttc atgggtccat aatttgattg ctgttgtcca ctataatttc
35881 caaaatcact atacttccca ccaccatcat agttaccact gtcaaaattt cctccttcat
35941 tgcaaccatc acatcctcca ccattgccac catatccacc accttggttt tcatgtcctg
36001 gtccaccacc accatagccc cttattttac tataaccaag accaccacca tagttgccac
36061 catcacttcc aaatccatta tattcaccat cacctcctct gcaactatct ctgcttccag
36121 cacctccatc atagcctcct cttccaccaa agttttcacc acagccaaaa ttacctccac
36181 cgcctccaaa ttttgctctg tgacaattac aactttatca actgtattat gatcctaaaa
36241 agttatggca aagccaggtg cagtggctca tgcctcccaa gcactttagg ggccaaggtg
36301 ggaggatcgc ttgatccagg gagtttgaga ccagcctgag ccacataagg agaccccatc
36361 tctacaaaaa atttaaaaat cagctgggtg taatggcaca tgtctttgct cccagctact
36421 tgggaggctg aggtgggatg attgcttgag cccatgaggt tgagtgagct atgatcaggc
36481 cactgcactc cagcctgggt gataaagtga gatcctgtct cgaaataaaa aataaataaa
36541 agtaaatttt aaaaaagcaa atcccctctt ttttccactc tgcctgcctt ccataacatc
36601 tatggtttaa atagtgccat acttttcttt cctctctctc tctctttcct tcttttcttt
36661 ttttgagaca gggtctctct ctgtcaccca ggctggaatg tagtcgtgca atcttggctc
36721 actgcaccct ctacctccca ggctcaagta atcctcccat ctcagcctcc caagtagctg
36781 ggaccacagg cacgtgccac tatgcgcggc taatttttgc atttttttgt agagacgggg
36841 tttcactatg ttgcccagtc tggtctgtcc caaactcctg ggctcaagca atccacccac
36901 ttcggcctcc caaagcattt ggaattacaa acgtgagcca ctgcacctgg ccttgccatg
36961 ctcttcaaat tacattcttc tctatcttcc ttgatatcac aaacaaagat tttcttcatg
37021 gttagatggg caccaggcct tatagaatcc tctctagaaa cagctctctt tggttccagt
37081 acacacctgt cagccttgtg tgtccacctc tttaacacga gagtaagtca caaaaccaaa
37141 gcctctggaa tgtgttgttt gggagtctct cattatcaca cagcctgtga gtatgtgcca
37201 tttctcaaac tatcgtctgt attttcaaag ctgtgaacac agtaaacagt tttctcaagt
37261 gctctggttc ctttggatca tgtgcctcca ttttgagccc ggactcgcct tttccaaatt
37321 gagttcaatt tgggactgag aggaagagca gtcttctttt ttaagaatag ttttaggtct
37381 acagaaaaat cgagcagaaa gcacagagag tttccatatg cttcctaccc cagcggtgtc
37441 ccctatcatt gttttgcatc agtgtggtac atttgttgca agtaatgggc tagtaataat
37501 attgatacaa cattattaaa gtctataatc tacattagag acttgatgct gttttattct
37561 tctcctctca ttggaaagtg gcagaaataa atgtaagtat taaatcttcg agggttaaca

37621 gaaagtcttc tatgtaggcc tttggctact tctctccttt ttgtcaccct caaaattcta
37681 gagtcatatg tattcccttt ttcccttcat cttttccatc tcttcattat gcttcaaccc
37741 attgtacttt atcttctctg tcattttact aaaatatatc tttccacaga aaacaatacc
37801 tcctttatta ctaagttcaa gtatgcatta ttattcctta tcaaatttaa ctttgtagca
37861 gcacaggaca ctgttaaccc tttctccctt cttgaattat tttttactta acttcccaac
37921 ttggtatctt accttcctga ttcccagcta ctctaatcat tgatccttta ctttctctac
37981 attagcccca ttctctctct ctctctctct ctctctctct ctgctttagc cagtctctaa
38041 gatggtcctc aatgattccc aactcttggt attcacattc ttgtgtgtca ccttcctcca
38101 ttgacctggg ttgatccatg tgaccagtaa aatagaacag aagtgatggt atgtcatttc
38161 caagattaag ttataaaaga tgttgcattt tttttttgtc ttggtcttgc tctctttctc
38221 ctctagactg ctccatgagg gctgcatgaa gcagccacat aatttaccat tacaacctct
38281 ggcaaatagt aagtgctcaa ctcatatttg ctgagtcaat ttatataaga acctttccac
38341 attttattgc ttcctctctc cccctcctac gaatttctat agcacttact gaacgcattt
38401 tgacatctag cacattttgt atttattatt ttataaaagg gcttggctca gttgagcctt
38461 ctgaaggaat gttccctcac ctactatgtg gctggcactg ggctagttgc aggtcaatcg
38521 aagctcatct tgatcatcct cctagagaag gagctccttg taggtgagtg gggccaagcc
38581 ctggtcacct gtatgcttcc cagggaacct agcctaggag cctgtagagt agcataaaaa
38641 cgatattaaa atgggttggg tgtcgtggct cacacctgta atcccagcac tttgggaggc
38701 cgaggctggc ggatcacttg aggtcaggag tttgagacca gcctggccaa catggtgaaa
38761 ctccatctct actaaaaata caaaaattag ccccatgtgg tggcacacac ctatagtccc
38821 agctactctc cttgggaggc tgaggcaaga gaatcacttg aacccaggag gcggaggttg
38881 gagtgagcca agatcgcgcg ccactgcact ccagcctggg tgaccaagca agaccctgtc
38941 tcaaaacaac aacaacaaca acaactatag taaaatgaac atttacctgg aaatctcaaa
39001 ttctgagttc aatctttgac ccttaaggct gtgactctgg gcaagtcact ccacaaatgt
39061 gaacttattt cctcattgtg ataaagatat agtaattata ctttttaccc aaggagtcac
39121 tgggtagctc agatatgtca aacccttatt tcctcacttg tcataggtca cttattgtca
39181 gatgaatcat ggatttcata atatattttg catttgtgtt gggaatgggg tgtgaagagc
39241 atgattttaa ccatctatat caattataaa acattcagat ttcagtaact ttaacataag
39301 aaaatgtttc tttgaatcaa gacaacacgt tatctattga agtgttcaag tgttctgaag
39361 ttggaaaacc attgtaaaac tgggagctct aattatgtca cattcaaaca gatgtgttct
39421 gaattaatga ctgagttgat tttcctcttt tttgttcagt catgtgttaa ggattcgttc
39481 tgttttatta ttgttatgaa acattgcaaa cacatacaaa agtgaaagca atgccttccc
39541 atggatctga tatccagctg caacaaaaat caacacaagg tcactctgat ttcatctata
39601 tctctactca atactctcca ccccccacca tccctatggg ctattttcaa gcaaatctca
39661 ggtctcatat aatttcattt gtaaatactt taaaggagta ttgtttaata gccatttacc
39721 ttccaccgtc ttctagtcag agataaacta agttttctta gaggtgtttg caagcttagt
39781 tcacattgcc taccctcctg aaagatcttt attaaccaag ttgaaaaata caccttctta
39841 agaaggcccc ttccccaatg gttactgggg gtggaaggta atcttaaaat cattacttgg
39901 ggagtaaaat gctggtggtt cccacatttc tgaatacctt tccattttaa aaccagttct
39961 taatgcagta gacgagatca gaaactgacc tacgtaagtt tgtgattttt tttttgtta
40021 gagtgctgat ttcttcttaa cttttttgtt tgctttatct ccatgggttt aatcattaca
40081 tgatatggtt ctaatgatag catgttgtgt cattttaggg acactatttt ctgcaaactt
40141 ttttactcag agtgttttct gaccataatg cattacaaaa tgtactatat agatgtgaat
40201 atatgcttag aattaaaaat aaaaatccat ggataagttt tcaaaataat gttgccactc
40261 actgtatgtt tcaaaagaga tggggtttca ctatgttgct cagtctggtc tgtcccaaac
40321 tcctgggctc aagcaatcca cccacttcgg catcccaaag catttggaat tacaaatgtg
40381 agccactgca cctggccttg ccatgctttt caaattacac tcttctccat cttccttttc
40441 aaaataatgt tgccactcac tatatgttaa atgttaaaca ttttacagat tcaaatgcag
40501 tagggattct ctgaaaaagt tttatcttac taacagcttt cagattatca gtactagttg
40561 tatggcttaa acacagccac atgtaaatac ttacttagat gaagctatgg ctaatcacat
40621 atgtaacaca atcagtttca tgtagtggtt tgatcactcg tgtattcaag actatataaa
40681 cagggatgaa tttgaggaag ttggagtgat ggagtgagaa aagtttgttt ttgcagcagt
40741 agcagtagaa ggggcaggcc gattcagtaa acaagcacac atttcagaat gcaaactgca
40801 ttctccaagc tggcaccatg gttttacggt gctagggatg tttactgctt gttgggtttg
40861 cagctttata aacagtgctc cttggagttc tctgcttgct attctgaaac atatttgagt
40921 tttgcaaccg agaagtaaat gactttgatt tccaaaatca gtcagtcatt tttcagtgtt
40981 gtgataggag attcatttga attcacttct tcagagatgc aggagccaat caggatcctg
41041 ttgcagtgaa aatgattctt ttattgaaac cccagcttcc tttaaaagaa gccaaagagt
41101 actgaattac agaactgcca tacagccttc cctttattct ttccatcacc caggccacag
41161 gaaaaaaaaa aaaaagacaa acttttgttc ttattttcac aagttatcac atggtttgga
41221 aacttcaggg catttctgaa acttcattgg tatgtgccag aaaagtcctc tttaacacac
41281 caggttgaat gaaagacagt ttgagatgtt aatttccatt tactgcagaa aaaatatatt
41341 taatgcatta aggaaatgtc tagcttaaag aaattgccca cttttgaaac aaatggttgg

41401 aggcctgagg tccaaagctc taaaatcagt taacaaatgt ttattgaaca cctattttga
41461 actaggctgt tgaagaagtt caaaagtcta agataaattc ccactttcaa ctcacctaca
41521 gtctagttgg gcagaaaaaa tccaaactca ggggacaatg caagagttag atagtaaaac
41581 cagacaaata cacaagcaat gaaacacgac accatatggt tgggtttaga aaagtgtttc
41641 aggcaataca tattgtagag gcttagggat cacagtaatc tgaaaatccc tggaaaatat
41701 tcacaaagaa ggtagaccat tgggatgggt ttgaaaacat agataagaat cagataacct
41761 taagaggaag gaggaagcaa acagtgagtc tggttgtaat agagaaggga gtgggagagg
41821 ttaaggaggg tgggaagggg ccaggttggc aaatccttag tggtaaggcc tggctgtggg
41881 gctgaagatg attctaaggg gtccaaggtc aacagcagtg tttaaaatta gattgccaag
41941 tgcatggatt ccctttcctt tcctcctttt ctgtccttta aaagcagaac ctgattagac
42001 agtctgggac attcccagcc ctaggctaaa aagcagcgat tgaggcacaa agtattcttg
42061 gccctgaagt tgaacctctg gggatggagc tcagggaagt catagggagt ttgggcccag
42121 gtaggatcag acctatccgc aggcttacat aggctccagt gattaaagct cagcttcctg
42181 aatatgcccc gaggataaaa gagacttttc cgagtgaaag gcattttcca aggcgctctc
42241 aacgggaatg tctgagatag cgaactgcgg ctgcagagca ccagctgaac gcacacacac
42301 aagtgtcttc tcacggtatg attagtgttc acaggtttcg tctagctcat cagaaatgaa
42361 gaatagcgat gatgaagtga catgtcaccg ggagggttga aaaagaaaaa gtgcgtaaga
42421 gggtcccaaa aagaaccagc ttccgtgtag acgggtctgc agaatctgcc gtctcccgtc
42481 ttctgggtcc cctggcccgc ccggagtggg gacaaggaaa tggaaagccc ggctcacccg
42541 gctctcctcc ctcgcagcca gcagatggct cccgggctct ctcgcgagcc cgctcgcccc
42601 ctctccagac gcagggcttt tgcggtttgg gagggaggct gcgaggaggg gcgcggtgcc
42661 cgccgccgcc accgcagcac cgcacagcgt ctctcctggg ggtgcgacct acgccgggga
42721 cccagcgctc gggcaccgga ggccggcctc gcaccggtgc ggaggccggc tctcgcgagg
42781 tgaggaggaa acgcaggagg aagaggagaa ggagaaggag gagaaggagg agggccggag
42841 ctgcccccgc ccagccggag cccggcagga gcgcgaggaa aacctactcc ccccgccccg
42901 cgccgctctc cccgccctct cccacgcctt ccgctttcac ctagggctgt aggtgcggcg
42961 cggaggctgg gcgggagcta cgccggccca agccccgccg gggaccagcg agccgggagg
43021 aggagcaggc gccacagccg ccccgcgccc cgcgcccgct tgtaatccgg tccgctcctt
43081 attcagccgc cgggaactgc gaggaggcgt catgtagcag cagcagcaaa tccgcctcgc
43141 atttgcaact cttttttttt tttttggtgg ggcggggggc gcgcggcaaa attctgtctc
43201 cgccccccct tttcttgccc acttccattt gcaagctgca tctgcctctc taaaaaaatt
43261 gaggagttcg gggaagggca gggggccata aatcagagtt ggacctgcaa taacccccac
43321 acctacaggg caaccatgac cgaggagagc tctgacgttc ccagggagtt gataggtaag
43381 attcacgcgg ttgttggttt tccaccttcc tctgcgtact cctcacctct ctctcccttc
43441 ccacctctgc tttcccgcag gtctcgagaa gtcttggcgc cccttagggc agagtggtgt
43501 tgggcagggt cactgggggt gccgggaagt ttgttcccat ccgaccgcct gtagagccaa
43561 gtgcagagag ggaaggcttc ggggaggaat cgggagtcct cgggaggtgg gtgggaggag
43621 ggtctcgact gtgagccctg ggcacctcct cttgcccgga agcggggatc caggttggcg
43681 aaagaagggg gcgggccatc ttagacttgg gagttctgca agcgagggga gccctttaca
43741 gctcccagcc cactgcccca aactcgggaa agtccgggaa agcctattgg aaggacaggt
43801 ggcgagcgca cagtaggtcg tctccggcac gtgtcaggcg ctttcctgaa aatgccccgc
43861 gggaggtgat cttctgacac gcagacttgg gggagtgggc gctagtctgg taaaatgact
43921 gggcttggca aggtgtggct tggtgtagcc agctgggagc gaaaggttta caggctgac
43981 gcagactcag gccaggaaat agagggttaa tgataaaagc cttattttaa taatgattaa
44041 cctaccgatt ctccctatag gggatctctt agggaacccg tgtgtgttac gctgtgcaag
44101 gtgacccatt gtttgagtac cttgaggttt tttttttttt ggtggtggtg gggcggggc
44161 tggttaagtc ttggggttta acttaattat ctggagggtt aaaatggcag agctgaaata
44221 ggatgggaga gtgttctgaa aacctagcgt ttaagcatgt atatgtgtgt agcatgttat
44281 actgtaacaa ggtatgtgcg tctgtgtaca gatgtgtttt gagccctagt gacctatatt
44341 ccctcctgta ccattcttgg cacactacac agcctacagc tcacaaagat gatgtttaat
44401 cggcgctgac aacaagagga agttctgagc acccgttgat ctccatgtgt ggaatttgcc
44461 cctctgttgt ttcttttagc taataatact ttggaggaat ttgtgtatgc ccttccgttg
44521 gtgactgtta actcagcagg taaaacttga tgtagagagg ctgttgctag gtttgtatgg
44581 aagcccctac ttctgctgaa ttacagacaa acctgatctt ttaccagaat ttacagtatg
44641 aaattattca cagacgcacg cgcgtgtgcg cgcgcgcaca cacacacaca cacacacaca
44701 cacacacacg cacctctcat catgatccca gcagatcctc ttctctctcc ccaaccctgt
44761 ggagtctgca tcttttgctg gagtgagtga cacatccggt tctgggaggc tgcgaccttt
44821 ccaacacccc aagcaaacgc cagaggctct gaagacgcct ccagaggtcc tgagaatgag
44881 tgtagggcag ggcttgcctg gctggtcagg ggataaggat ggctaaagtg ggtggcagat
44941 ttttacttta tataaggagt tgtccttaag tctggaaatg gaaagtgtag gtaattactt
45001 aggcccaaga gaaagagtga agggtagagt ggaaataagg tgtctgggtt tctattcatg
45061 gttctcaaaa ggaatagttt gtatatttta aaagttgagt ttgaccttta tcttgaaatg
45121 gaatttttgt ttttattaag tattgcctat ctctttcaag agagtgacat attatgcccg

45181 atatgatttt tctgggcctt tcagtggtca agttaggctg ggatagaata aatacctgca
45241 cctgctttcc tctaaattca agtgctcaca agagttctgc tctgatcaat tcaaaatggc
45301 gaccgaccac atgaaaacct ctatatattc ccttaagatt ccctcaagat atttaaaaag
45361 tgttacagac ttggctgagc gtggtggctc acccctgtaa tcctagcact ttgggaggcc
45421 gaggcaggtg gatcacctga ggtcgggagt tggagaccag cctgaccaac atgaagaaac
45481 ccggtctcta ctaaaaatac aaaaattagc tgggcgtggg ggcacgtggc tgtaatccca
45541 gctactcggg aggctgaggc aggagaatca cttgaacccg ggaggcggag gttgccgtaa
45601 gccgagatca agccatttca ttccagcctg ggcaataaga gtgaaactcc atctcaaaaa
45661 aaaaaaaaaa aaaaaaaagt gttatagact ttatgtagaa acaatttaaa tgtagagaag
45721 aataagaaga ctatatgtca ctcctgttac cacttagagg tagttatttt aaagggtata
45781 tgattttcca gtctgtttcc atgtgtatat gttcttaagt caatttcaga tgcccatttc
45841 ccgcaattct tttttagggg agggaggagt ggcctgtgct tgtttctcga gttctgataa
45901 gataggagct ttgtgaagaa ctcatagtgc agtgagctct tctacatgtt cccttgcccc
45961 aaacattcca cctccttgct gttccttgaa caggctgtgc tttctcagat ttccatgctg
46021 ttctttatgc gtacactgtc tttttctccc cgttttcact ctttatctca gcagctctaa
46081 actttcatgg cggtatttcc aagaagaaaa ttttgattac cccttattaa acatatattt
46141 gttaaactac atgctccatt tagtgtgtat aatctaaaac acaaactgaa acacacattg
46201 taagtagaaa tgaaaattct attttcttct ttcatctcaa tggattatat tgtacactct
46261 actttggaaa ccagtgatct tatttcctat gccttcaaga ttcagctcag ggatcctctc
46321 ctccctgaaa cctttgcgtg cttccgaatc catgatctta tgcattgtgt gtgaatttct
46381 tttatgacat ctctcagtga cctccactag attgcaaact cctgtgaaat gcaaagacca
46441 tctctatcta gcacctagta gagtacctga catacaagag gtactcctat gtacatttat
46501 ttgttgaaca tgtgaataga ccaaaatgat tataatacag aatacagagg tatgggagca
46561 tagaggaggg gactgtattt tggaggataa gtagaatata accaggagga caatagggaa
46621 agggcatccc aggaaagaga ataatagata cacaatccta ggggtgtaaa agatgaagat
46681 attaggaata attggatgtt taggtatgtg ctgagaagtc acactgaaaa taaatgtgaa
46741 agaccttgaa tggcatgcca tgagttttgg gtttattcaa aaatgactgc aactcgtaga
46801 ctgggaggga cgtgatgaat ctcacagttt tggaagagtc actgtggtta tggaggtgac
46861 agtgggctga tgggttagag gccatggact tagtcttggt gagagattct gaggccctta
46921 ctgcagtcat gcctaggtag tcaggagccg tggaggaggt agattttagg agactgactt
46981 tctgtctgag agaaaggagt tggacataag cattttccag caacattagt gtccagagta
47041 ggagataatg agtttgattt cagacgtatt cattttgagg tactcatggg agccctagga
47101 agacttttcc accaccatct gaaatgtgaa gtctccagat ttctccttct attgcctttg
47161 accacttctt gggggtattg tgctgtcatg attttggaag gacagatggc tgggagtgcc
47221 tttgtttgag taaatcatct ggtatagttc tggcttattt ttattttttt gagacagagt
47281 ctcactgtgt cactcaggct ggaatgcagt ggcacgatct cagctccact gcaacctcca
47341 cctctcggtt tcaagcaatt ctcgtgcttc agcccctgga ctaactgaga ttacaggcac
47401 gcaccaccac acctggctaa tttttttttg tatttttagt agaggcaggg tttatccatg
47461 ttggccaggc tggtcttgaa ctcctggcct caagcgatcc accttcctca gcctcccaaa
47521 gtgctgggat tacagacgtg agccaacgtg cccagcccgg ttctgttctg ttttgttttg
47581 ccgtattccc caaacatcat gtcaagactt actcatagag aatgctaata tttgtatgac
47641 actatggtga acccagaggc tgctgggcac cagaggtaac cagccagggg cagagggaat
47701 ttatgaattc ctgttgtgtt ctgcatgcaa cgtagtgttt catcatactg cccaacttca
47761 ctgtttgctg ttctttgttt tttcttttat atgtatctta ttttccaaac aagattatgt
47821 catgccttgc atgggctggt attatgtcgt gcttttgtgt ttgcctcagc atatcaatat
47881 acagtacgag gtacaaagta ggtgtttatt aaatacttga tgggtgataa ccatccaact
47941 tgttaaaaca agtaaaaaaa gacaaacggt cgggcacggt ggctcatgcc tgtaatcccg
48001 gcactttggg aggccaaggc aggcggatca cttgaggtca ggagttcaag accagcctga
48061 ccaacatggt aaaaccccat ctctactaaa tacaaaaaat tagccggacc tggtggtgtg
48121 cgcctgtagt ctcagctgtt cgggaagctg aggcaggaga atcacttgaa cccaggaggc
48181 ggaggttgca gtgagccaag atctcaccac tgcactccag cctgggtgac agagtaagac
48241 tctgtctcaa aagacaacaa cagtaaaaac aaccaaatat actcctccaa atgcttactc
48301 ctcctcagtg ctttttttct tattaataac ataattcctt ttttttttca gaaagcataa
48361 aggatgttat tggcagaaag ataaaaattt cagtgaagaa gaaagtaaag ttggaagtta
48421 agggagacaa agttgaaaac aaagtgctgg taagtattgc tgtttatttt tattaaatgt
48481 ttggaaatga aagtgtgttt tcattgttta gattgcttaa ttttcatgca gcattacttc
48541 tgtgttttat atattgttca aaagttctga tgaccatttt gtcttaggtt aattggaata
48601 tttctttgtc aaaagttaaa aatagggtaa tttgaatttt aaagatcaaa ggcttttaga
48661 ttgtatttct caaagttgta attgctgcct atatttaggt aatctcttag agcctgtttt
48721 cataatattc agagtttgat gttaaatgga acttttttat ttggtgtgtg gtgggtgatc
48781 ctatgacttt attcataaag tcttatggag gagtttaaaa agttcatctc atgttggttt
48841 aattacttaa ttagattttc tcagatgtta tatatgagtc catctaaggg gtgatgagct
48901 aacaaaacat tgtcatttta cttagggtgt tggtgctttg gagggaaact cctgctgaag

48961 gcagtatctg ctagtgttgc gactggactt ggggttcctt ctagtttgtg agaggagatg
49021 ctgagggatt gggccctgct gcagaaaact tcttagagag gtgtcagtga cagagttgta
49081 cctttagaga ggtgaaaaca ttttgtgata ttttaatggt ttctaggttg agattgtggc
49141 aaatctaaat ttaagcttag gtggaaagaa tattttgatg gagagaatac tgtttaactt
49201 tttatcatgg atattttcaa acatatgcaa aaagcagata atagtatatc gaactctgtg
49261 tacctgtcct tcccagcttc aaccattttc agcattgtac taatattaaa tatcttcccc
49321 atggtgtttg tttgttgttg ctgttttttt tttcctctgg agaattttaa aacaaatccc
49381 aggtatgtta ttgaaagagt ataatttctt aattttttaa agtctttttg agacaggatt
49441 tcagtctgtt gcccaggctg gagtgcaatg gtgtgatctt ggctcattgt agcctcaacc
49501 tcccaggctc aagcgatcct cccacctcag cctcctgagt agctgggact acaggtgtga
49561 gccatcatgc ccagctaatt ttgtttattt tttgtagaga cagggtctcc ctgtattccc
49621 gaggctggtc tcaaactcct ggactcaagc gatctgcctg cctcggcctc ccgaagtgct
49681 gggattacag gcatgagcca ctgtgcgtgg cctgaaaaag aatattttta atattccatt
49741 gcttcagtta aagttgccaa agaatttgaa gtgtctttct gatcacatct gtcttgagaa
49801 tcttctaaaa tatctaactc caggatatgt gattgtcttt tcttgttagt aaggattatg
49861 cttgtgatgg atgacttctg tttctatgtt gtctatatat acaagtcggg atggtaaagg
49921 cattgaatgg gaaaacttga atggcttttg cccagcaaca aaatgtgctt atgtaaactg
49981 catgattaag ttatcagtag aagaaatgat tggtgccatt gttctgttgt atttaacttc
50041 agtaaaggtg agatttcctt aagttatgtt tttcttgatg ttgttcaaca atgattgtgc
50101 tattttttgg aagtggtagt gtagagaaga aatattgttt ctgtcatttg cttttttaca
50161 aatttaaagt aagtttttaa taatgtagta actttataag atgaatcaac ttctctacaa
50221 cttaaatata aggtagtggt tgctatgaca gtctaaaaaa ttggggcttg gtattatttg
50281 atatggaaat tattattatg ctaagaacaa acccacatca tgagtgcaat ttaccatgtt
50341 aaaatttcaa agacaaattt aaatgtccag tataaaatta gcactattta aacatgctta
50401 tgattagtac ttaattgtaa gtttttttgg cctcatctaa cagctggaat tgattaaagt
50461 tgtaaccaaa ttaaaaatgt atttgagttt aagcatttat ctccccaaat atggaatcta
50521 agcagtttct agaagcatgg ccggaactca ttgtggctgt tacttacttg catgggcagg
50581 aattattaat agaaatactc aaggtctggc tttctttgac aggcttcaag ttagaacaag
50641 aagatgtatg aagtaacctc aaagttgtgg atttctgagt ttctgagttt agagaaattt
50701 aattgcttaa tccattgtta gcctactaaa atggttcata aatgagttgg ctgaatatct
50761 acagtacgct ttttgagttg tgcttttgtt acttccccca ttgcccacca ttcactcctt
50821 ttcttcttat aaaagtagga gtgtgctgta ttttacgtgg ttctttcttc ttacacttga
50881 gactttatac tatttctagc ttatcttttg tagagggctt atcttcatct tcctataatc
50941 ataatagcta atatttctaa aaatctgttt ttgaagtcta gtcaagtgaa gcaatgggat
51001 tggaggaaga gcaaagaaat ctataatgag ttgtgatcaa ttagttgtaa acacccctac
51061 actcagacca gccatgatca taatagctaa tatttattaa acactggcca agtgccagag
51121 actgtgctga gtacttaatt acgcattgcc ttgttatatc cccactacaa cgctatgagg
51181 taggcacttt ttacatctcc attttacaga tgaggaaatt gagatttggc gaggccaaag
51241 taaattgctt cacatcattt ggctagttaa taacagctgg gattcgaacc cagctctgcc
51301 tcactcagag cctatctgct ttgcctttgt ggtcaaaaag aaggcagctg ttactttgtt
51361 ccatggttgc tgacttcaaa caatgatagg ggagatttgg actttagagt ctctctgtct
51421 ttgattgatt gatttataca ctagttttat atttattgag aacctgctgt gtcccaggca
51481 catggtgtat acaaaatagt cctagtcttt gacttcatgg gactcgaagc ttacagtcta
51541 gttggggagg taaacatgta gccaggcagt ggtttcagat gtgctatgag tggatgaatc
51601 ggggcttcag gagcacggag aggaactccc aactcaggcc tgagaatcag ggaagacttc
51661 agaaggaaga taagcttaag ctggcgttca ctggatgagt aggagttagc caagcgaaga
51721 gagtggagac atgttctgga caaagggaac agaatacgtc tggaagcctg agagagctta
51781 tgttagagaa acatactaag gtacagctgg ggtaaggcat atacagggta gagaatgggc
51841 aggtgtaaag cctgagtcct gtccttgcag ttgggatgtc aggtgaaagt taggactggt
51901 taaacaggtc tcttcttgcc acctgtgtat aatgaaatat taaggctcag tggttaaggg
51961 cccagatttt gggttcacac tgttgctcag taactttgtg acaatgttaa gaatcaggtt
52021 tttttttttt ttttttccgt atggaaaaaa tcttatgtta taacagtatc tattggtatg
52081 tggttgcttt gcatatcaag tggaatttat ttaaatactg catagcacag tggctgcatg
52141 cagcaaacac ccaaaaaatg ataaggcaag aggaacaaag gggtcctgca ttctgtttc
52201 tccgtggtgc ggaaagtgcc attccttctg aagcccaagt atctgctgcc tccattgaca
52261 ggttgaggtc agttcaccat aaggccaaga gggtttattc acagtttgtg gcaccttatt
52321 agcaggatag agggcaacaa gctggatcta aaattcctaa ggtatgctct cctcaagtga
52381 aatcagaaat gctagatgtt ctggggcagg agtgttctat tctcaaataa atttggcaga
52441 gtgttaaaga taaatcagtt tctttactgc atgagttttc agagccatta atacagccct
52501 gtgcattgta aatttctaag aattggataa agtatgctgt tttttccaaa cttatttagc
52561 cagagaccct aatgaccatg ggctaagaag gaacaggcca gagatgatgc ctcttgggac
52621 agttaagaaa agatagaaca ggcttgaata ggctctccat cattaggagt gttctcctga
52681 ggaatggtcc tttagccatg ttttttaaaa tctacattgt gagtagttcc actctgggct

52741 acgttggctt gacatgtcat atgttgttga aatacctttc tctgttgtct ttaggggatg
52801 tcattagaaa gtgattctat gtttatttga ctctaattcc cttctcccat ttgctaaatg
52861 aatcttcacc caatttttat agtacttgga gtgtaaaaaa tgtattttaa cttatgtatt
52921 ttttaaaatt aaactttttc atttttaaat aattgtagat ttgtctgcag ttatatcaga
52981 taattcagag aggttatctt atgcccttta tttagttttc cctttttgca aaattacagt
53041 ataatgtcac aaccaggata ctgacattga ttgacactgt aaaatgcaga tttccatcac
53101 cacaaggacc cctaacattg cctttttata gccataccta gcattcccca cttctctgat
53161 ccctggtagc cactaatctg ttctccattt ctataaattt gtcattttaa gaacattata
53221 taaatgtact catacagtat cccctttttg ggattggttg ttcttgctca gcataattcc
53281 ctggaccttc atccaagttg tatcaattta gctaaggagt acttcatggt atggatgtac
53341 cacgtttggt ttagccattg gctagttgat ggacatctga gttgttttca gtttttgtct
53401 attaggaata aaactggtgt aaacatttgt gtacagattt ttgagtgaac atactttcc
53461 ttgctctgtg ataaatgcca aggagtgcaa ttgctggttt gcatgggaat tgcgtgttta
53521 cttttataag aaactggcaa actgtttccc agagtggctg taccattata tggttttacc
53581 agcaatgtat gagtggtcta gtttctctaa atccttggca gcatttgtca ttgtctggac
53641 ttcttttttt tctgagacag ggtctcactt tatcacccaa gctggagcac agtgacatga
53701 acacagctcc ctgcagcctc aatctcccag gcttaagtga tcctcccacc tcagcctccc
53761 aagtagctgg gactacaggc gtgtgccacc acacccggct aatttttgta ctttttgtag
53821 agatgggttt caccacgttg tccagctgct tcttgaactc ctgggttcaa gcaagctttc
53881 tgccttggcc tcccaaagtg ctgggattct tttttttttt tttttttttt ttgagaacta
53941 agttgaagga aaaacaatgt ctggcaggag caagagccct gatgacctct tgtttcccac
54001 agttctgctg ttgcctgttt gggggagagg aatgtggcac tcctctctcc aggatggtgg
54061 ccttaaagtg tatgaaaagt cattcctagt ctccactttg gtttcttctg tttgatttgg
54121 aatttgggtt acatcatctt tgccaacccc ttcttcttcc acaccctttg taaaatgggt
54181 cagaagcagg acttcttttc ttcttaaact gagcaagtag gggaagtggc atacaaatcg
54241 aactattctg ttttgtttac ctattctctt taaaactgcc catctgttgt ccatttgttt
54301 tcttttgaaa agttaatctg aaatccaccc ccccggcccc tgaacgacag ggtctcactg
54361 tgttgcacaa gctggagtgc agtggcacta tcacaatcac gagccttgct caagtgattg
54421 cctcttgcaa tcacttgaac tgagccttgc ccagttcagc ctcttgagta gctgggacta
54481 caggcacatg ccaccatgcc tgcctaatta aattttttta ttaattaatt ttttttagag
54541 acagtctcat tattttgccc aggctggtct tgaacttctg gactcaagcg attctcccac
54601 gtcggcctcc caaagtgctg ggattatagg catgagccaa tgcaccaggc ctgaaatccc
54661 tttaacatat ttttttcccc ttggctattt atttatggct tttgaacatc ttaattagac
54721 tggattggag taactataag ttaacttaaa aaaatgtcga aaggcatagg ggtattattt
54781 tgggtaacgt atccctaaaa tattttggtg gcagtgctgt atttcattaa cataaagtat
54841 aaactctatt ccacctgctt tttttttaag aggacatgaa aaaagaaata tctctcatat
54901 agaagttttg gaagtatttg acccacttag tcacaaatta tcttcaactc agatattctg
54961 caatatggaa actattgttc ttttgcagta ggccatactc attttgcatg accagtctgt
55021 ttaatgcttt tctagtttgc cagtctctag tacctaactt gattcagcag tatgacctgt
55081 gccatagtca cttttcagac ggatattcat gctgaaattt gtgttcagct tcttaaggca
55141 acatcagcct tggccagtgg ttttcagact tagtgcctgt ttaaaacaat tttttttttc
55201 atggaaccca taatgtagta atgttttggg attgtatttt gggagttcat agttaaacct
55261 ataatagtgt aaacctaatc actgatttca gtgaggctgt tttgatgaac acaaattaga
55321 aatagtaggg gagatttggt ttttgctctg gttgcatgga atagagagac tcccgattca
55381 ccagttaagc agtacacata tcacagtttt taaaataact agtcttgcaa tctcaggatg
55441 ctttccaagt aaatagagac ggtaggggaa gctttattct gagatctgca caaaagtggg
55501 ttatcctggc agcacagtgt acctcgtagt tgttctctga tgtgtaaaac ttggtgtaac
55561 acatctgagt gtgtgtgttg gtgttttatt ccagtagtta aaatagacat aagacaaatt
55621 catataaaat gtttgtagac tgcaatgtct tctgaagaat gctgtcagaa aatcattatt
55681 ctaggtcccc aggtttacat ccctctgtga gacgctcttg taccagttgt tgggtaattt
55741 gaacagttgc ctttgtggta ccattaggag tctcaacttt taaggatcac ttacagttct
55801 gtgcataggt gattttcaaa gcacctgggt gaagttacat ggctctagaa aattgagctt
55861 tagaaaacag caataacact gaaacaatct ttgggagtga gttatcagag aacgtgcatt
55921 gttgccaagg atgctggcat aaattctggg aacctcaaaa taaaagaatc catatttaag
55981 cacttcacaa tttacctgaa ccgaacactg gagtcagttc ctacctgttt ccactaatct
56041 ctgggtaggg gctgggggca ggctctggcc ctgggtgtgc gttttcagga ggcagttctg
56101 gctgacttga acttgttacc tgagctgcat ttacattcta ctctctcctc atctgcaact
56161 gccccagttt cttctgtagt taggataggc gagtcaaatg accttaggca aagaagctgt
56221 tgaacataca ccaggctcgg tgtaactttt cattgttcta aaaagatact ctttttccag
56281 tctctgggat ttagccaccc ttggaccagg gcaattaggt atataccctg gagaggggag
56341 gaaggtaagt gatggggggg agtaggagag tagcatgaaa gggacattgt ggacattaag
56401 aagccaggaa atctaatcaa aatatggaat gaatagggag aactcttctt tttttcatag
56461 ttataaaata gttacagaga aaaataaaga ctttagtaaa aggttactgt aaaaacttta

56521 ggtttcattt cattggaaac gctgttaata tctttgttaa aatagatgaa aaatgtagct
56581 acaatctgaa ggataaaaga tttttgttcc ctctcttatc ccctctgctt tctaaaatgc
56641 tttttttttt taatagaata taagaatttg gattggtata tatggggtat atcattgtct
56701 aaatctttac gttactgtgt tttatttatt tttttccctc ttgatagacc actttcccca
56761 gctctttgag gaaagagggg caaagagggg aaagaaggat gcttggtgtg tgtgtgtgtg
56821 tgtgtgtgtg tgtgtgtgtg tgtgttttgt agcagtagta gtagtggcag taggtaaaag
56881 agtgttcact gggttatttt cctcatcttg tttcttagga gaaggttttt cttatttatt
56941 tttgagacag tgtcttgctc tgtcatccaa gctggagtgc agtggttcaa acacagctca
57001 ccgcagcctc gatatcctgg gctcaagaga tcctcctgtg tcagcctctc gggagctggg
57061 actacaatca catgccacca tgccgaattt ttttttttcc tagagatggg gtctcacttt
57121 gttgcctagg ccagtcttaa actcctgggc tcaagcagtt cttccacctt agcctcccca
57181 agtagtgggg ttacatatgc gagctggtct tcccttccct tcctctcctt tcccttcctt
57241 tcccccttcc cttccctctt cccttccccc ttcctcttcc cttttgacag ggcctcactt
57301 tgtcaggctg atgtgcagtg gcatgatctc aactcattgc aacctgtgcc acccaggctc
57361 aagtgatcct cccacctcag cctcccaagt ggctaagact acaggcatgg gctgccccac
57421 ctggctaatt tctgtatttt ttgtagagat ggggtttcac catgttgccc aggctggtct
57481 tgaactcctg agctcaagtc atccacctgc cttggcctcc caaagtgctg gaattatggg
57541 tgtgaaccac tgtgcccaac ccaaggtttt tctttttttg aaagcattat cacttggtga
57601 acattggcca gcaagtgtct tggtttcttt agatatgtgt agatgcacag gacgtcctct
57661 tagaggtagg aagcaggcat gtacacatgt cctgagctgt acttacagcc tcaccagcct
57721 ttggcaaggg tttgagacag atgtgcttag ttcctgctaa gaaggcaaat gtgtctattt
57781 ctgctgatcc ctgggccctg gagtctcagg agacatttat ggacattgac tgggattagt
57841 aggtatatcc accagacagg atctggtgta gacagattga ctcaaacaga ctctggggaa
57901 atttattgag accttgccct cattgattgc tgcctagagg atcttggggc tggaggactc
57961 ttggcagcca tctaaccatg tgtcctaacc taggagaagt ttcttctgta gcacctccga
58021 cagctaggta tctacttttt gcttcactat tcccagtgat gagaaaagca aaagccccca
58081 aaagccctgt cttcttttga actgggatct gcttctcact ctccatttct tcttgcatag
58141 tctactggaa tatcacagaa caatttttct ccctcctatc catgagtgca gttcaagtat
58201 ttgaagaagg ttatcatact tttctttggt cttatagtcc tcaggctaaa taggctcaca
58261 tcctggagtc tcagtccttg gaagatgttg ttttcagact tatcactatt ctgatcacct
58321 tgctttggaa tcaccctact ttgtccatga accttttaaa caactgttgg tgtttgggga
58381 tcacacatgc ttggcgaaca cccttgaact gaattaaaca gactttctca gtctttaatg
58441 ggctcctgtg gattgtgcct tttgaagagg gtatggagcc tgtggcatca cagcatctgt
58501 gttcctccag gcagggtggg gaacatcaag ctaagtgcaa cctgcctggt gaagaatgct
58561 acctatgcat tgcttggaca aaaggttttg ttattgttgt tttttaaatt ttggaagtat
58621 ccctgatatt tgcattacta aaacaataat aaaacatagg aaagaaaaag cgaacatcac
58681 ctaatcctat caatttagat ataatcattg gaaaaaaaat cagtattttt catatttttc
58741 tgtgctaact tacataaaat atatgatata ttttaaaaaa tggaatcatg aatactattt
58801 tgtggtatac ttttaaaatt taatttttga ggtcaaagct ttctagggca gtagaaagca
58861 tgatactcca tttacccact gtagaggagt actgtagttt atctaaccat gtcccttatt
58921 gtcagataat ttaggttatt tccaatattt tgccttataa ctgtccatgt cctcatattt
58981 atttctcttg gataaattcg tggaagtgga atcattgcca tcattgccta ataggcattt
59041 tgaaggatgc tttttttttc acctgattgt tatttaggaa gtctcaatag agaataaatc
59101 ctccaccttg tcaatttcat tgtcttaaac aagagtctac ttggcagaat ggcttgttct
59161 ctttgcttgg atccgtgtat ttcttagacc agtgctcctt ggaccctagc tcctgaggca
59221 aacctttttc atgcaattta gggttctttc agatctccct gtgtggaagt gttgtcttct
59281 actacagtat gtttgctgga atgagaaagt cctgtgactt cacttgtggt ttcagagtca
59341 cagcttccaa gtatcattgt cttggcagaa tcagccatag ctttttcctt ggctgaatcc
59401 taggtaggtg tgagcctgta ttatatgatt cagatctttg cccttgtcgt gggactttct
59461 taccttgggc ctttgaatgc tctcataaat ggtagttgag tagattcaca aatgataagg
59521 cacatggcac tttcattaga tgaaatgtaa ttaacaagaa ccataagcta atatgcatgt
59581 agaactagtc caatgtctta gaagataatt tagtatcata atgaaaggag acattgtttt
59641 tcttcccaga cagttctgaa ggcagttgat tttatctgta tagccaacaa caatttctta
59701 agtctcatgg tccttcccca ctccactaga agctgcagag tagtccttct cattctcatt
59761 tactgtccca atttcctgac cctttcacta tctccctgtc ctgggagagg ggtgtccagg
59821 tgatgaggac ctatggcctc ttttctgcca tgaggagctc cttgtggagc cctgtcctcc
59881 ttgctgaggc cctcaagagt gcagcctgct tttctgtgtc ctttcaaagc taccattgtg
59941 tactatgttg cttgtggctg tcttttcttt caagcctggc agataggatg atattcctca
60001 gggacctcct tatttgctgt ctcagaaaac aagcacagag gactttctta gttaccctgt
60061 agaacagaca aagaaaatca agacgcccag atgcggggaa gtgactgaga accttgcaag
60121 gttcttttaa ggcaatctct ctgctctttg atgcctaacc ttatgggcca cagccacact
60181 cacttgcaga atctgagcta aggtgtggcc aggagaagtc caggccagtg aatgttatca
60241 gtggtcattt cttggtttcc tgaatccatc tgtcttaaat cagctgtcac tagcaaccaa

60301 aaaaagatct tatcatctgt cataggcatg acctgaagag aagcagctgt gctttgtttt
60361 ttgctaagtc ttgggcagtt cagtttggca cttagagtat tccagctcct tttcttataa
60421 gaatatctat ctttatctat ctatctatct atctatctat ctatctatct atctttaact
60481 aactaactaa ctaactaact aactaaagca ttttaaaacg tggcatctcg ctttgttgct
60541 caggctggtc tccaactgtt ggactcaagc actcctccta tcttggcctc ctgagtgtct
60601 gagactacag gcacaggcca ccatgcccag cctcagctcc ttttctttta aatctcttgg
60661 tccaatttat attctttgcc agcatccttg ggacagcaca aatgttgaag tttggggaaa
60721 cattttgtac aactttgttc actgaatcac agtcactgct tgtgctgttg ataatcaaaa
60781 ctaggctgca cagagtgacg tagtcacata tttggcttgg gtgtacagat gtttcccttc
60841 aagcatcttg gatgtcttgc tccttatcag aattttatct cccccaaatt cattgtttgt
60901 gaattaactg taaagttctg ggagcgattt ccttttcaac agtttgcctt tctagaaaaa
60961 aacttttgga gtttaaatag cacttaaccc tctatcattt tactctctct gaagtggtct
61021 ctgtggagaa catgttgggt cttctctgtt caatatctct ctctctcttt cttggcaaag
61081 cctggatgcg agaagttggt ccaatctttc ctgtccaggg ttgcttgtta atacagcatc
61141 tggagcaaag gaaaggctat tagcaccttg tcacttggag tttcttaagc attgttccat
61201 ttatgtgttc tttctgcaga gatttaatga aagtggcatc tgtctttaca cctgacatat
61261 gcaggttgtc cgcagaactg aacaagtggt gtcagcaaca ctgtatatgc aggaaggctt
61321 catgctcacg tcgttttttc tgtgttctct gggagtagcc aacttcttta ttcatcagag
61381 tagggcttta cattttaata ttctagagag agatgcttgt ggtgatatat ttagccttaa
61441 attcaggaat ggcttctttt ttcaatgtct ttacatttta cgcctcccat tttctgtgtt
61501 aataggtaag tgtgcctaca tttaaattct gaaatggcat ttgctaacat ttaatcttgg
61561 ccaagttatt taacttctct gtgccttact ttcttcatct gtaaaatggg tgtaataaca
61621 aaaaactaca ttgtagcttt gttttgaaaa ttaagtggga tattacatat aaaatatttc
61681 ataatagtgc ctggcacata gtaagtcatg agtaagtctt agctgttatt gttaggggta
61741 gtgtcgtgat aatgctaaat gctttgatta aaagaatcta attgtatatt tgagtcacag
61801 attattcaga tatattttat ataaatattt caaaagaaat acctcaactt tgcatgtctt
61861 cctggccctg tacaaggtac atgttgttat gttttctctg ctcagagcta ttttaagcca
61921 gtacagactg tctacagaaa tgggtttaga atgttttttc actctgctgt tcttaaagag
61981 ggtacactgc ttttgagaaa tcttttttaa agaaagggtt caagttcagt gttgagaaat
62041 ttgtctctga ggacaacgct gtgcatttcc aatacgtatc tgtttttcat cttgctttca
62101 ttgcagatct gcatgtattt agatatatgc tcaagtccct ctttcacttc ccagttactt
62161 agagtgaaaa aaagaaaaca tcagtagttg gtgcctgttc attcacttag ggctttcttg
62221 gtcctttcag ggcactgctg tctgttgctg ttgcctcact cagtttgctt cctcttggat
62281 ttcagttatg attgcttttt tttttttttt tgagacggag ttttgctctt gttgcctggg
62341 ctggagtgca atggcgcgat ctcggctcac cgcaacctcc gcctcctggg ttcaagcgat
62401 tctcctgctt caaattcccg agtagctagt attacaggtg tgtgccacca tgcccagctt
62461 attttgtatt tttagtggag acagggtttc tccatgtttc agggtggtct cgaactcctg
62521 acctcaggtg atctgcccgc ctcagcctcc caaagtgctg ggattacagg tgtgagccac
62581 tgcgcttggc cgattgcttt tattttatag ccggtccttg atcgggggtg cagggtgcat
62641 tatctgagtt cattggtgtg tgtggctctg tgtctccata gtcagaagga ttcctgggca
62701 tgctggattc tttttttttt tttttttggg gaaagagtct cactctgttg tccaggctgg
62761 agtgcagtgg tgcaatctta gctcactgca acttctgcct cccgggctca agggattctc
62821 ctgcctcagc ctcctgaata gctgggatta caggtgcctg ccaccatacc cagctaattt
62881 tttgtatttt tagtagagac ggggttttac catgttggcc aggctggtct caaagtcctg
62941 acctcaggtg atctgcccgc ctcggcctcc caaagtgctg ggattacagg cctaagtcac
63001 agcacccggc ccatgctggg ttcttaatct ccaggctgtg ctggactctc accttctaac
63061 caaccttgag gtatttcagt cccagcacta ttgacatttt ggtcaaaaca attgttgtgg
63121 gggctgtcct gtgcatgata ggatgtttac cagcatctgt ggcccctccc cactagatcc
63181 tggcaccatc acccactctt cccacagttg ggataattaa aaatgtctcc agaggctggg
63241 cacggtggct catgcctgta attccagcac tttgggaggc cgaggcgagc ggatcacctg
63301 aggtcaggag tttttgagac cagcctggcc aacatggcga aaacccatct ctactaaaaa
63361 tagaaaaatt agctgggtgt ggtggcgggt gcctgtaatc ctagctactt gggaggctga
63421 ggcatgagaa ttgcttgaaa ctgggaggta gaggttgcag tgagctgaga ttgtgccact
63481 gcactccagc ctggggcgac agagtgagac tccacctcaa aaaaaaaaaa aaaaaagttt
63541 ccaaacattg tcacatgtcc tttggattgt agaattggcc cccccactga gaatcattgg
63601 tttagatcag tgggtttcaa atgtcagaga gcaggtgtgt attttatcat atttagcttc
63661 tagagttatg tctaagtctc gaaatttaca gttttatgat atagccattg ttgatttatc
63721 taaaagtcta tatgcattta aaatgcttat ttttgaaata tttagataac caaatctgta
63781 tgtaacaaaa ataacaaatc ccaccacata gcaatttgtc ttgttttaag atacctaaaa
63841 gatcagacca gtggttctca atatttggtc ctgagaaaca gcaacattaa catcacctga
63901 taacttatca aaaatgcaaa agcaggccaa gcgtggtggc tcatgcctgt aatcccagca
63961 gtttggaagg ccaaggaggg aggatcactt gaggccagga gttcgagacc agcctggcca
64021 acatggcaaa ctcctgtctc tgctaaaata tggaggcaca cacttgtaat cccagctatt

64081 tgggaggcta aggcatgaga gtcacttgaa ctcgggaggt agaggttgca gtgggctgag
64141 atcgtgctac tgcactccag cctgggtgac agagtgaaac ttttcctcaa aaccaaacaa
64201 aacaaaaaag caaaatcaaa gcataggaga cctatctaga cctactaaat cagaaactct
64261 ggcagtggag ccctgcgatc tgttttaaca agcccttcag gtcattttga tgcacactga
64321 agtttgagaa acacttggtt agttacttag gtaactaggt aactaagtat attactaagt
64381 tacttagata attaagttag ctccagttag agacccatag aataaacctt attgagaaat
64441 aagtataacc tgctatatac agaactttga aatctgctta ggaaaatgac ttttaccaga
64501 cagatcatgc taagtgtgtg ctggtcatat tggcttccct gcaagagcct gccatgtcca
64561 cagctttctt tacaggattt ccttttgtta gtatccccaa gcttggtgca tgcaacttca
64621 cagtttggtg gccatagctg cttgaactag gtatgcttat gcctgactcc caaggtacgc
64681 atcgataggc taaccagcag catgtattgg gaagagattg atcctcttgg gagtttgaga
64741 cagagacaaa gaaatgggaa gacagcaaaa ggcatgagga ataaaagccc ttagttaagc
64801 agaaaccatg aggagatgaa gaggctctta atgagccagt ggtaacaaga acaataggag
64861 gtggaattgg gtgacagagc acaggatggc tgctttggaa agggtgactt ttagaatcga
64921 agttgagtca atgtacttaa actaatatgt gcacagtatg ttttagtttt ctagaaggcc
64981 caactgggca gctgctcata ggggttcctg ttttgtactt ccctattttg cttgcttctc
65041 tagggagcct catgttaaat catcattaac tgaggcaatt tcataatatg tctattcctt
65101 gttgcctgga tgaacccatt gaatatataa ctatattaga agaccaagtg gtttcagatg
65161 ggttgcaggt gggtttcatg gcatgctgct tcgttgaagc agtgactctc agcctggctg
65221 tgcctcagaa acacctgggt agcctctgcc tctttattta aaaactttct gtatgtgaga
65281 ttctcacggg cagttgaagt ctgtattaca ggagagaatg ggagatagaa gctgttactt
65341 tcagcttgtt gatgggttga ctttcccaaa ataaagctgc ataggcgtac cttcagaaaa
65401 cttcaggcct tacttcattc atttccttag atcagaccaa ataagccagc tcaccatatt
65461 tattccagag atgtgttgct ttgcctccaa taacattttc cttaacattt cctgttagag
65521 cctctgggta ctatttcttt tgagggagat gctaagagtt tgaaaagcct taaaaaaatc
65581 attaaaaata catggaatag agtgttgaaa tcagcattgt aaatcatttt ctgaaaattc
65641 agtaagtcta aagaagtttt tctgttttga atgatcggca ctgaccctat ccagttggtc
65701 agtgcctccc ttatcctgct agtcacctct tcctcctgtc tttcctatta gaaccaagga
65761 aagtatacaa gtacctccat gcagcaaatc cactggattc tctgacacaa atcaatccag
65821 aggggaatga gtgaagatac agaatacttt acattgttgc tcagatggtt ggctgagttc
65881 cattagggta atatattctg ggaatgtggt ccagtgcttg gtttcaaggc cagcttaaaa
65941 gatttggggc ctgaccaaga gcataatcag gattctctta ttctccaacc tagtatcctt
66001 gaggagattg taaactactc gagagtccag gactgtttac ctattgtact tttatgttaa
66061 gtacctctac ccctaaaagc aatctctttg tgatatacac agaatatttg cccagtaagt
66121 cactcagcct tgagtacaaa ttttaagccc gagctgcttt cctcaggggc ccagccagtc
66181 tctttactca gcagccttat ctgacatagc ccatctttaa tttagctttg ttcacgatgc
66241 agggatcaat aacagcgatg ctgggaggga gggagaatgg ccaactgcaa acagccgccc
66301 atctcagcac ttgaactgaa ctctactttg gtcaggatga ttagtgcagt ctggagagcc
66361 attaagtgct ctctgactag ttcatctgag ttctgcttcc cactacactc aatgactaac
66421 ccaggagatt acagatgctt gaggagaaag agtgtcgtgt ctccctgtgt ggtactgagt
66481 agtgtttgta ccactgtctc aggaatgtga attacagtgc aaatgagctg cctctcttgt
66541 ttcctttctt gcacgtcatt ttagagagag tgatttattt tctgtttgcg aaggcttggt
66601 ctcatcattt agatgagaga gaaattgcat gtactttcaa cttttggctg gttataggca
66661 tctccttttt ttttttttcc attgtgagag ttgtttatgc tgattgaatg aaaagattcg
66721 ttttcttagg ctttcagtga tgacactcca tttcttccat gtgataaaat tataatgcgt
66781 gattttattt tattattttt taaaagtaaa actgtcacct aacatttcct ctacagttgg
66841 gtaaaaaact aagaaaagca tctcatttcc tattggttcc atgagcaacc ctttttagca
66901 tacagatcag aagccttgtg tggctctaag cctatttcct caattgggga agtaacagac
66961 tctgttccca agatatgaat aggtgtgctg ggtgagaagt gctctgtgct caaggacttt
67021 gacccttcat tttccaaact taaacaagtt tctccatttc tctgttctta gaacctttgg
67081 tattcatata cttaacatgt acatttcttg aagcgccaat tatcatctca caggacactg
67141 gtgttccagg gaacttggag aaaagctgac aagttatttt agtgtgttat cttttacaca
67201 aagggactct gttttagaat ttgccatatc tgctttaacg ctgtacaaat tagccaactg
67261 gagaaatata tgttaaataa cgggtgaaca acacataatc acccaaccta cttttaggaa
67321 gattactaaa acattccatt taacaatcat tcagcactat ctgtttgtaa aggacacgta
67381 agttttaaag gaagaagctg gtgacttggg aatggactgg aataaagtca gagaagtcct
67441 ttgtccaggg aacttcatca ttgggtttga cttgagaaga acaaatcaga taagattagt
67501 ctcctcatta taaatgttta aggtaatttc agatcactct gctttaaaag tcttgtgaaa
67561 tggggttggg aaagtttcct taaatcattt acacttgttg attgtgtgca gtttatatta
67621 tgagcctgct gcccccacag gtgctgagct gtgctcacac atgggagggg acatcacaga
67681 aaagaagacg tagatttatt acctatggtt ctgaactggg acaattgata aaaattacag
67741 gtgctgagga ttggcttgaa acgcagaata atcttcctaa cagagctgtc cactcataga
67801 atgtgcttat tgtgagggtg gggatagagg atggggaatt gaattccttg cagctgggta

67861 agttcaagta tagattgctt gccctcttaa ctaggatgtc ctatagaaca gccgtcccca
67921 acctttttgg taccagggag tagttttgtg gaagacaatt tttccacaga ccgagtgggt
67981 agggggatgg ttttgggata aactgttcca cctcagatca tcaggcatta gattctcata
68041 aggagtgtgc agcctgcttc cctcgcatgt gcagttcaca gtagggttca cgctcctatg
68101 agaatcaaat gccttggctg atctgacagg aggcagaact caggtggtaa tgtttggtca
68161 cctgccgctc acctcctgct gtgcggtctg gttcctaaca ggccacagac tggtactggt
68221 ccacagccca ggggttgggg acacctgcta tagaacattc aagtaggaaa atacttcctt
68281 tggtctaaga ttctgaaatt tacaatgttc cctgctcata tatcaaatga tgtattagct
68341 agagcttatt gaatgtcaca gttttactgt caagaactta aaacttctat tatgaggcag
68401 agcatttggt aaggatagtt gagtccctca gtgactgttt ggattggttc tatgtcctag
68461 gattgagtac atgatatcat tttgatgagg ttaaaatcca attaggaggt ctgtcccttt
68521 ggctgctggc tgggtttgtt actttttagt tccagtagag atatgtgaag cttttctgaa
68581 catgtttagc aaggtttgat tttggcattt ctgaatataa ctgtgagaag aattatcttc
68641 ctgctgtcct ggtggcagaa gacattggac tttgtttgct tgtctctttg ggggcagata
68701 cacgggcatt taaagagcca ttctaatttt aggaacttga gaatcaggag tcctgagggt
68761 tagtgctaat actttaaaag gttcttaggt ctgcaatttt agttaatagt gtggggctga
68821 aaaagccttt ttccaggaat attgtgagag taagtgggaa gtagagcttt tagaatttta
68881 ggagaaaagg gcatggctat ttcagttaat aatacatgaa gagttttctc ctttcagagt
68941 cctcctttct ttgggtttca aaaggtaaga caatccacgg gttttttagt ttgtctttca
69001 ggtgaattgt aaagttttcc tgtaaatact gtcattgcac tgacatggat attgctgagc
69061 agataaaaca aatagcaagt tgaggaggca gttaattcac actcttcctt ctctaatatg
69121 gtctgcggct ctaagaattg ttgtctcatc catggctttt agacccttaa attttagtga
69181 gtttcatcac acggaaacta ttgataattt cttcttctga agttttttgt aagtggtccc
69241 tgctagctaa tggtgcacaa aatgaagcaa tacaacattt ctcttatggg gactgggaga
69301 gaactgacat cctttataaa tatgtagctg atatataggt taccagccta aggctgcct
69361 ggtggggaaa gtgaggcctg tgcctgtctc taccactgaa gacatgttca tcaagccaat
69421 atatgcctca gacattaaaa atatcctttt tttcatttta aaataattgc cattaagtat
69481 acataacata aaatttacca ttttaaccac tattaataaa tatatgttca gcggcatgaa
69541 gtatattctc aatgttatgc aacgtatcca tttccagaac tttttcatcc caaacagaaa
69601 ctgtatcaaa cactgagtcc tcattccacc tccccatcgc ccctgacaat cactaatcta
69661 ctttctgttt ccatagaaac agaaaatatc cctatttggc atgttttata taaatcaatt
69721 atataatacg tggccttttg tgtctggctt gtgtcattta gcagaagggt ccccaacccc
69781 agggctgcgg ataggtactg ttccatgacc ttttaggaac ctgggtcaca cagcaggagg
69841 tgagcggcag gtaagtgagc attaccgcct gagttctacc tcctgtcaga tcagcggtgg
69901 cattagattc tcatagttac gagaaccctg ttgtgaactg tgcattcaag agatgtaggc
69961 tgtgtgctcc atatgagaat ctagttgatg ctcgatgatc tgaggtggaa ctttttcatc
70021 ctgaaaccat ctctcctccc ccccactttt attgaaaagt tgtcttccag gaaaccagtc
70081 cttggtgcca acaaggttgg ggactgctga cttagcatat gttttcaagt ttcctctatg
70141 ttgtagcatg tgctagaagt tcattccttt ttaatgctga ataatattct gttgtatgca
70201 tataccaatt tttaaaaaag tcattcatac ttcaatggac ctttgaattg tttttatttt
70261 ttggctactg tgaatactat tgtgaacatt actgtatggc tgaacattgc tgtatctgag
70321 ttccttcttt cagttctttt gagtatatac ctaggagtgg aattactgga tcatatggca
70381 gttctgtgtt taaccacctg tttcctgcag tgccttggct tttttttttt tttttttga
70441 cgagtctcat tcttgtcgcc tgggctggaa tgcactggca cgatctcggc tcactgcaac
70501 ctctgcctcc ccagttcaag tgattctcct gcctcagcct ctggagtagc ttggcttaca
70561 ggcgcctgcc accacgcctg gctaattttt ttgtattttt agtagagaca ggttttcacc
70621 atgttggcca ggctggtctc gaactcctga cctcaggtga tccgcctgcc ttggtctccc
70681 aaagtgctgg gattacagct gtgagccacc gcgcccggtc gccctggtcc ttttgaaact
70741 gggcagtaat acctgtgctt gtaagagtgt ttacaaatat ttatcagcct tttggaactt
70801 taaattagaa aagtgaacag actgtatttg ttagcacttt atatacttac attatgtcat
70861 ggaacttatt attcgtgttt cctgaatgtc tgttgtttgg atacctgtgt gtgtttccta
70921 ctctattaga aattccttga cctgatctgc agttctccat ctttgtccac cttttttgta
70981 ccttgtactc aataaatgtt tgttgataaa ttgattagat gtgcaacata ttctaaagta
71041 ttatttttaa aaaagagatc catttacaag aagcacattg gaattatttc caggttttgt
71101 tttgctaaag ttaagatctg acattatttg ttaatttctc ttggttatgg aatgaaaagt
71161 attcccactg ttaaaaataa atgttagatg taggaagtaa acagagatgt atttagacat
71221 tggggaaagg cttccactgg gatctgaaat attgtgggaa gagaatcgct attgttagag
71281 tagttctgtg ccgtgttagt ctttattttg tttagatact gcttttttgg gggaccactc
71341 tgcaggcatc taaatctgaa gtgtaagcct cttttttgat aaatgaggga tagctaaaag
71401 atgggttatt ttggttattg gttttgcttt taatagataa ccagaatttt tacctcttgg
71461 gagagaatgt tatctactta tttatagcaa gcaagtgttg ttacggcagg tggctgtgga
71521 tgttggcatt caaatggcac tgataagact ttaaacaagg tagccatcca gttatgctta
71581 ggctaaaaaa tggctgtgtg cagattaggc tacatatttc acatggatat aaagttctac

71641 ttttattaac aaacataaca gtgcattgct tttgtgtttt tagtttctct tagcccttat
71701 ttcttgtctc ttttggtggc ttgctgttgt ctatagctga gctgaaactt tctgtgcagg
71761 aattaagctt aaaatatcag tttgtagtca gctgtggagt atgataaaac tttgagcaac
71821 acagtttcca acaacacccc tttgtcactg tgcctgagaa agctcttatt aatagaattt
71881 catgtgaggt tccgtgagaa ggactattgt acaaccattt tttttttttt tttttttttg
71941 agatggagtc tccctttgtc acccagactg gagtgcagtg gcacgatctc ggcttactgc
72001 aacctctgac tcccagattc aagtgattct cctgcctcag cctcccgagt agcagggact
72061 acaggcgcgt gccaccacac ctggttaatt ttttgtattt ttcatagaga tggggtttca
72121 ccttgttggc caggctggtc tcgaactcct gaccttgtga tccacctgcc ttggcctccc
72181 aaagtgctgg gattacaggc gtgagccact gttccggcca tttttttttt taacagtgaa
72241 acagggaaac ataatatttt tcttacttta aaaaatcctt cagtttataa tcatcaggag
72301 tattggatat cttttaagtg aaggtggaag ttcacttaat aaaaaaggaa gctcattttt
72361 ccttctttga atggttaaca tgggagtacc ccaaggtgga agctttacat gactgagaaa
72421 gcccatccac tcattgtcat agaatcaact attaattcaa caaatactta tggagccttt
72481 gccatgccta tgagccttgt gctagaagcc agggataagt aaaataaatt ctgcacctgc
72541 ctcctgcaac ttagtccaat ttccaggtct cccttagatt tttctctgtt ttaattttta
72601 ttttgaaaat ggagacaggg tctcactatg ttgcccaggc tggtcatgaa ctcctgggct
72661 caagtgatcc tcccgcctag gcctcttaaa gtgctgagat tacaggcatg agccatcaca
72721 cctagcctct ctcttaaaac aactttatta aagatgtaat tcaggctggg tgtggtggct
72781 cacatctgta atcccagcac ttgaaaatta cttgaagttg ggaggtggag gttgcaatga
72841 gctcagatca tgccactgca ctccagcttg ggtaacagag caagagtccc tctcaaaaaa
72901 aaaaaaaaaa aaaaaagtaa ttcacatacc atacatttca cctgtttaaa gtgtacaatt
72961 cagtggctat tagtatattc acagagttgt gcatctatta ccagaatcaa ttttgagcct
73021 tttttttttt ttaaccccaa aaagaaacct cttcccttta acacttatcc ccagtccacc
73081 tatccttctc agcccttggc aacagctagt ctactttctg tctctatgga ccggcctagt
73141 ctagatattt tatataaatg gaattatata atatgtggcc ttcatgtctg gcttatgtca
73201 tttagcataa tattttcaaa gttcttccat gttgtagcat gggttagaac ttcattcttc
73261 ttttattatg gttgaatgat attccactgt atgaatatac cacatctttt tttttttttt
73321 ttttttggag acagaatctt gctctgttgc ccaggctaga atgcaatggc acgatctcgg
73381 ctcactgcaa gctccgtctc ctgggttcaa gggattctcc tgtctcagct tctcgagtag
73441 ttgggattac aggtgcacgc caccgcaccc ggctaatttt tgtattttta gtagaggtgg
73501 ggcttcacct ccttggccag gctggtctta aactcctgac ctcatgatcc acccgcctct
73561 gcctcccaaa gtgctgggat tacaggagtg agccaccccg ccgggccaca tcttttttta
73621 atctattcct tcactaatag attgtgggtt gtttccacct tctcactatg tgagtaatgc
73681 tcttatgagc gtgcatacaa gtttttgtgt gggcatatgt gctcaagtct ctgatcccct
73741 agatgtcttg cctatcctac agatccaggc ctaccacaaa aaagtgtaga aggatcatat
73801 accatgagaa cataaaggag ggacatgtag acttaattag ggagtcaggg aaggctgccc
73861 ctgaggccac catatccaag ctgcggcttg agagataggt tgaatagaaa agtttttcaa
73921 actgcagctg atcacccatt agtaggttgt gaattcagtt tagtgaatca tggccagcat
73981 tttttaggag gtgaactaaa atagaatagt agatgttaga ggacattcca tgtggtaagg
74041 gtaagtattg ttgcatgaaa tttattttca gtagctatat gagcgcatct agcaaattgt
74101 gacgtaagaa gtattctata ttccgggttg caatcagaaa aatatgaaag ccactgctct
74161 agggattttt taaaaggaga ttatttatta atctggagct ttaaatatgg ttccactaga
74221 agattggaga gatacaggag agagaagaac ctagaaagct gaagatcctt tgaaagctta
74281 aagttctgtg atttctacac ttttaaaatg attcatttat tcaacaaata ttaaaatatt
74341 taccataggc ctagattgca ctaaattctg ggagcatatg taagatttgt gtgattttgt
74401 tttttttgtg atctggcaga gaagatgtac aagcaattaa aatgacttct ataaaacatg
74461 gtgggttgag gccgggtgcg gtggccaagg tgggtggatc acctgaggtc aggaggttga
74521 gaccagcctg gccaacatgg tgaaattcca tctctactaa aaatacaaaa aactggccgg
74581 gtgtggtggt gggcgcctgt aataccagcc actcgggagg ctgaggccag agaattgctt
74641 gaacccagga ggcggaggtt gcagtgagcc aagatcgtgc cactgcactc cagcctgggt
74701 ggcagagcaa gactcttgtc tcaaaacaaa acaaaacaca aaaaaaacca tgacggttg
74761 aacgcataag tacctttagt ttttccaaaa tgtctctaaa atgaaataaa ataaataatg
74821 aaataaaaaa gacacaggca cctcaaaggt aggagaatag gagaggagat cacagcagtt
74881 aattgccatc aagatgtctt tggaaggagt cactgactaa gctgactggc ttagtcactg
74941 actggctaag cagaggaagc tggactctaa ggatctcaaa gaggatatca gtaagtgagc
75001 cagttagctc cataaaactc cagaaaagtt caggatttgg aagctctagg tcttgcataa
75061 ggctgtagga tatatagagc gggggaaaaa aagagcatta gatggagatc tgtaaaagct
75121 gttaagcccc agttcccacc cccacccatc agatccaggt gcttgcccct cctctcaccc
75181 tgaaggttct attgtgaaaa gctgtaaatc attagccatc catcccccca acctgcaaag
75241 gtacacacct aatagatatg atacacaccc atcagaaaca atggcacact tagtttagaa
75301 gggaaaagtg ggcataccct tgtgggtgtg tcatgatctt agagggccag gggataagta
75361 gtttgaaagt gatctccaag gtgattttga agacacaatt ctcccatccc actctcgatt

75421 actagccact gattttgtct caggaggcat tagccgcctg tcaaactgct gggacattat
75481 ttttctctta gtcaataaaa gtagtttgac atccatcaca tatagcattt gctaggtctt
75541 gtgggaggca acgattacaa caaaagggta aaatattgtc ctgtgctcat ggaacttcaa
75601 atctagttat tggtgattat atacattcta aattaaacac tttcaaaata agacatcttg
75661 tcaaggtgaa ggattaaaaa cctttttgtc ctatcctgtc tatatttcct ctcacccct
75721 cccatctggg tgtgctcaga taaactttag agcacccaag gtttatagaa ctcctgacag
75781 cgacctcacc aaaggctgga cttcctattt gtcataaact tgttgaaatt ttgtttgact
75841 gctttagtac aggagtatat tccccaagac aagagacctg agagcttttc cctggttaag
75901 ataccaagga tgatttccaa attttagaca tccttcccct tgttccacca attttttttt
75961 tcttctggga aaatagccag gatgattgca aaacataagc ttgtaaaaag gcaaaactcc
76021 atggatgtaa gaaagtaaat ttcttgaggg ccacacccat gataacgctg gaattttcat
76081 ttaattccta actcattttt tgttgttttt gtttttttaa actcaaatgt gtctctttaa
76141 ttgaggtcac ttacttggtt gggagattaa tattctggtg ggaaactttt cttttagag
76201 tttatattgt tttattcctt cagtcactca gtattactaa tggggtagct tttggaattt
76261 tccatccccc ccactttcag attacttttg tctttttttt tttccctgag aaattagtga
76321 aaacagtttg aagtcttgcc acgttgtaag gagatttgca ttatttaagg aaacagctgg
76381 cagatctcta taatgcccca cctctgttct gccagtgcct aaaatttcct ttgcatgact
76441 cagttaaaaa aaaaaaaaaa aaaaaaaaaa accagaggag gggaacgagt ggagagaaga
76501 aacccttgtt tccctttctg agttgtaaaa agcttttatc aaggtttaca tttagtcatg
76561 cttctgtcct ctagtggatg gcagcatgtg atgaactcag agaaactctt ctcccaaatt
76621 ggccagtaag aactctgcaa aaactgtgga tgagctgagc ttacctccca taattttatt
76681 ttgaaaataa aaaattttcc ccacatagta aacacagtta tcttcacgag tgtgggaatg
76741 attaagaaaa aaaaaccaag cttagatttt gcaagatggg tgacctgaag ttttgcctag
76801 agggagagaa gggtctcttc ctcctttcac tctgttcttg acagcgcctg cagcctgccc
76861 tggaagaacg ggtttcttct tcagatagaa aggaattgct ggctcttctg ccagccttgc
76921 ccataggcct ggctgtttcc ctgatgcacc ttccctgctc aagttgctat ccttatcatt
76981 ttatattttt gtgccagtat agatttggtc ctataaggct gcagatacat agtggcaga
77041 aagatggggg gatgagagca ctgagcctac aacctatttg gagggactct gggcagcatg
77101 aggatgaggg gcagggccag ggccaccgct aactagttag cagtcaggca aatagtcaag
77161 gacagagacc ttgtatacat taatagtgcc agcattttaa agatctttac ccaggaagg
77221 ctgtatatat acagttattt gggagaaacc agaaatggtg tatttaaaaa aattggatgc
77281 cttaaacttc caaataaatg cgttttagag ctcacttatc tggaggttgg cctataacat
77341 tccctctctg atgtcttgcc tttcaactgt tttttttcac ttctcagagt gggcatgaga
77401 aaccacagga tgtagaaaca tggagtcagt tttgctaaag caggccctca tgagacttgg
77461 aagggaagag gtgatgagtc caatgagagt ttaaaattag atgttcagtt attgattcta
77521 tgttttcttt gtagtcagtc tttttctaat taaagtgggt aagacatttg aatataggga
77581 gataaatgtg ctatgttaaa ggatataaaa aaatcctgga ggaagtctga actctaaact
77641 ctttggcaag tctttctgcc tgaattttga agcaaactta aagattatat atgtggctgg
77701 gcctagtggc ttatgctgtg gctcctgacc ttgtgatcca cccacccgcc tcggcctcca
77761 tcccagcact ttgggaggcc gaggcgggtg gatcacgagg tcaggagccc aagaccatcc
77821 tggccaacat ggtgaaaccc tgtctctact aaaaatacaa aaattagctg ggtgtggtgg
77881 cacgtgcctg tagtcccagc tacttgggag gctgaggcag gagaattgct tgaacccggg
77941 aggcagaggt tgcagtgagc tgagatcaca ccactgcact ccagcctggg cgacagagca
78001 agattctgtc tcaaaaaaaa aaaaaattat atatatacac acccccacac acaatacata
78061 catacacata tatacgtata catacacata catatatatg tatgtataca ttcacacatg
78121 cacttatata ttcacaatga tagttaagct agtttttgcc ctccttatca gtatgtcaca
78181 tatccatgtt gtttcctggt atatccagct gtttccttaa tgagactgcc tccatctttc
78241 agatatttac caacagttga gtatatactt ggctttattg caggacctgg ctatgataca
78301 gatggatgta ctctgtatag atgcccaaaa taacttttat ttttatgatg gttagatttt
78361 gcctgcatat ttaaggtttt gctgtatata ctaatttgct tcagagtcag aaaaatagca
78421 gcaaactctg agaggggact gaaaaagtat cactgactta ggggttatta tagtaggaat
78481 ggaactacag agatagcctt tgtcactgct ttgtgagaaa gcagtggaga aaaatacaat
78541 agctcctatt tattaaaagc ttcctatatg tcagtactat atgtattatc tgttttaatc
78601 ctcacagtaa ctgtgtgaag cgggtcctaa ttatcttcct tttacagatg aagaaagtga
78661 aggcagtctt taggtgtcct cccaaagttg cagaacatga gaggcagagt gtggatgagg
78721 ctctatgtct tagattataa ggacagtgct catttccatt gcattaagga aaatatccta
78781 gtctatcctg tttaatccta ggaaccctgt ggtgtggaga ggttttctct tacataccaa
78841 ttctaagcct ctgctttgat cctttgtctt ggtaggcatc ttctctcctc atttactgct
78901 cactgtgtga ggatgatagg cactcctcag ttataaacat cgcccttgca aacatagcag
78961 cttggtcgct ctctctgcgg tctaccctaa gggctcccta aatgcccaca catggcggca
79021 ggtgccagac tggggcccga gcagggcact ggctctccac agcctcctcc ataccgctgc
79081 ctgctttttt tctctcatct cttcccccac ccgccattgt ttttttgtgt ttccttccag
79141 gcagattagt taattccacc tcaggtaatc tcttattctt tctatgacaa aatgcattct

79201 tagtttcaca ctgttgtcat ataaatgaag gaaccctttc acacaaattg ggaattggca
79261 ggatttgatt catcaatatt ctaggctgtt agttttcctt ttgctgtgct cacacagaac
79321 cagatacagc agaattaaag agtaataggc accctctagt cccttgctac tcaaattgtg
79381 gtccatgacc agcgtcattg ccaatacttg gggcttgtta gaactgcaaa atctcagccc
79441 ctcccaggcc tacagaacca ggtcctgtag tctaacaaga tctgcaggag cttatttgca
79501 cattacactt gagaaacact gtaagcctac cctatttctg tccttttctt cctaagctaa
79561 atgttatgac ttggtcacag ctgaaagcct tcagtgccat tattagtaat gacgttggtg
79621 ctgataaggt ttgtagataa cattaaactg aaaatgtttc ttgacgttaa actgagaaat
79681 gcttctaatg accaggaaga cagaattgaa agtagcctta attaattgaa gtgattactg
79741 taaaacaggt taaaattgca tagggaacac tgcctgcctg gcgaagcatt gcatggtgtg
79801 aggagtgggc acaggcgcag cactcaggtg ttttgcgttt gtctagaaac actgtccttt
79861 cagtccttaa gcctcatgga cagctgtttc tcggggactg tcagggtctc aattttgaat
79921 tgtcttactg attttagatg agatagttta tattccagag ggcttttttt tttttttgag
79981 acggagtccc actctgtcgc ccaggctgga gtgtgatctc cactcaccgc aacctccatc
80041 tcgcgggttc aagcgattct cctgcctcag cctcccaagt agctggaatt acaggcgcat
80101 gtcactgcgc ctggctaatt tttgtatttt tagtagagac ggggtttcat catgttggtc
80161 aggctggtct tgaactcctg acctcaggtg atctgcctgc ctcggcctcc aaaagtgctg
80221 gaattagaca tgagccacca cgccgggcca ttattattat aatttttttt aaagactagt
80281 ctagtgtaat agaagaggga aagagaagaa caagaagttc tgtctgtaac tgactgtgga
80341 caatcaatgg agataattca cagcctgcag attagccaga gggccaattg ttaaatcctc
80401 attatgctgc cactcttgag tcctgttagg ttactagaaa gcagtgcacc tggagactcg
80461 gtacttctgt ctcagagctg tctttctaaa gtaagattta gcattaggcc cttagttttt
80521 aactgggaag ttgagcagag agggaatgtt tgaagactgt tactacaatt ttaaaactaa
80581 aaggtgtcac tgagggtact gagcgtgctg tttattctct aatgctcagt tccatgggag
80641 aagtttctct caaagttgag ttagggtgtt tttttcctaa atgctttctt tctttttttg
80701 ggatggggtc tcagtctgtt gcccaggctg aagtgctatg gcttactgca gccttgacct
80761 tctgggctca aacagtcctt ccatctcaac ctcctgagta gctgggacta taggtgtgca
80821 ttactgtgct tagctaatgt atttttattt ttatttttta aagatgaggt ctccctatgt
80881 tgcccagtct tgaactcctg ggctcaagca atccttctgc cttgacctcc caaagtctca
80941 aagtgttggg attacaggca tgagccactg caccacccta aatgctttta tttagtgaga
81001 gccataacat catattgttt ccatattctt ttcatcttcc aggaaattcc taagcttttt
81061 tttttttaaa ctatatgttg gaagctttcc tttacttagg actttttttt tttttttttt
81121 aatagagatg ggggatctca tcgtcaccca ggttagaatg cagtgatacc atcacagctc
81181 gctgcagcct ccacctcctg ggatcaaccc ctacctcatt ctcctgacta ggactacagg
81241 cactcaccac cacactgggc taattaaaaa aaaaaattct tttttgtaga gaagtggtct
81301 tgctatgtca cccaggttga tctagaactc ctgacctcaa gtcacccgtc cgcattatcc
81361 tcccaaagtg ctgagattac agacgtgagc cactgcactt ggcctattta gggcttctaa
81421 ttcactttcc ttttccttct tgtctaattc ttgtgttttt agaatctgca ttttatttta
81481 agtcatctca aattcctttt ggaagtagtg agggagtaaa tgctaacctt gtgtagaaac
81541 cgtaaagcgt attcaatttg tttctgtttt atgaacttga ccttgtgtga gcatcagcca
81601 tatgcaggac tattgggctt ggaatgtgcc agaccaagca gtctctaatt ttgttctcag
81661 ttcagagttg aggggctgag gaagtttggg gcaagagtga ggtctgcttg ggaggattgc
81721 cttgacagct gtgcgcacag tgggttggaa ggggagcagc agggcatccg tttggcagct
81781 gccttggtgg tctcagacta ccgcatccat gctgaaattg gaaaggagaa ggtcgggaag
81841 ggaaagactg gggacaaagc ggactgattt gggcaacggg tgaggtgcag agagtaagga
81901 acagggacga atatatgtga ccctggttat caggccttga tgttgctttg aaaacagtcc
81961 tctccagttt ttgatatttt ttcttgtata tggggagctt gataaactaa gccttggacc
82021 tttgttgttt tgatctgagc tctttgtcat ctgtcctact ccgggaaatg tgaagtggct
82081 gcagaaggca cctaacctgt tcttgttccc ttttctctgc ctcgtacctc aagttttcta
82141 ccactagagg agaggcgggc tgaacttcct tagcaagatc tctttcattt tttgggagtc
82201 ccattttcta atacaactcc agacccatca gccacccggg agaccccacg tgggagctgc
82261 gttccctttg ccagggagat gctcctggga gttgcattag gaaggcgagt caggaacaga
82321 tggctgtcct ggggggtctg gatgtcggcc cagccccatc tcctcaaagc cattgtttgc
82381 cctggtttgt gaggtgctca acagcaggga cagatgcaat cagagaagga tgaaatgctc
82441 tttttctttg gacctgaaat gggaccctat acttagtgca gccttactca gtgactacca
82501 aaatttgaaa attgtgtttt tctgaggctg ggctcattga cttccgtgag ctcttattag
82561 ctgtttactc atgaattagg caatttaatt tgggtagagg agggcaaatc aaatgagaag
82621 gagctgccgt gtttaaaatt actatgagga tagactgttc ttaaaggatc ctactcaagc
82681 ctatagaagc cttccttagc ttacatgtcg agtaaattaa ttagacgaat tgtgaattat
82741 tctgatttat tcagagaaat cctttgctct gcaggccgta ctgggcatca tgtggctgac
82801 ttcttgttct gactttctat gaatgtttgc aagtaataaa atatattttt atgattgaga
82861 cttatttagt aataatattt aatattaata tttaataatt ttaggaaaat aatttaggta
82921 aggttttgct gtgtattgta gcatgcattc ttctaagcca tgccctttga tattaaaagt

82981 tgtgctagtt tcaaagggct cgatttcctt aacaggaagg cacctataaa tctggcagta
83041 ggtaatgaag ggagaatggt ggtcttttca gtaggggagg gcagtgcaca cagagagtta
83101 tatgtttttt agtctaactg ccctaatcta atctcatgaa aggtacatac acatttaata
83161 cacatttttc tggggcccat agcttttatt acattttcaa aggagtagat gactcctaga
83221 agataggaac ctctgcagaa taacttcttg agtgagataa cattgttgcg atttgagaaa
83281 tactgaatct ctacatttgg tcactttttt tttttttttt tttttttaaa cagagtctcg
83341 ctctgtcacc caggctggag tgcagtggag agatctcagc ttactgaacc tccacctccg
83401 gggttcaagc gattctcctg catcagccta ttttcacctc tttaaagaaa tatattgtta
83461 tcgctccctg aaagtggatt agttaagtac tgtttaccct gcctatgaag aaaacttact
83521 tgcacatgtt atgttcggac attagtcaat gaagattagt caatgagaac tagtcgatga
83581 acatggtcaa tcttgcttaa agactcaaaa tatgctggag tatggtgtta attgtggtag
83641 tttatggcat ttttctgaag agaaaaaaca ctccagcaag gtaggtagga tagtatattc
83701 tctgtcttct tcctcctgcc cacccctctg tctacattag aaacaggttt tggaggagta
83761 gtgggaacat tgggtgattt ctagtggggt tagagaaagc agtacctggt tctttggcca
83821 catctaccaa agcaaagacc tagacttgta gaggagagta ttgtttgctt gtttgaggat
83881 tgtatttccc aggtttgttc atttgcatga gtggctgaat aatgtaacag agacttcaga
83941 agtaaggaaa atggttgtca ccaaaatatc ttgttttacc tttatttgga ctaaatggaa
84001 aacacatgtt taatagctaa ggctattgag tgcttatcat ttgccaggaa ctgtttctga
84061 gcctttcaca tataacacct cattttatcc accctctacc cctaacaaat ggtgtggttt
84121 gagttggcct tggtgaggta tgaaaaggaa acaaagcagt gacctcttcc tgcaggctgg
84181 tcagatgttt gggataacat tccttagcat tcttggtgga agtggtgatt tcgttgaaag
84241 ctgcttccca gttgctctgg agtgcaggtg ggaggggtgg actttggctt ttccaaagga
84301 tcccaggaat gggaaggacc ttaaagatct agaccagggg tatccaatct tttggcttcc
84361 ctgggccaca ttgtaagaag aagaattatc ttgggctaca cgtaaaatac gctaacacta
84421 atgatagcta atgatcttaa aaaattgcca aaaaaatctc ataatgttta agaaagtgta
84481 tgaatttttg ttgggctgca tttaaagcca tcctggactg catgtggccc atgggccatg
84541 aattggacaa gctttatcta gactgttctg aactactcat ctgcctcccc cagcccccac
84601 ccccacccca cacatccaga attctccacc cattggttgt ctggcctctg cctgaatagc
84661 tgagtacctg tcttttccaa aactacccct tgaggggaga cttacagcac cctgtagcat
84721 agtatatgat atttggctag taaaatgtga tcctttaata tttttcaatc tttattgagc
84781 acccacccac ggggttggag gaatttaggg ggttctgtgg caaaacaaga atagagtctt
84841 tgcatttgac ttctcagact tgtagggaca tacaggtaag ctggatgttc tggtgagcgc
84901 tgctagaggc cacacaagat gcttccaaag cctagaggag ggtctgatcc agccttgagg
84961 agacaaggtg gcatctgaat taatgagtgc attaatggtg aatgcatact gttattataa
85021 agctcaggga tgttacagcc tctgatactt tacacaaata gaaaggttaa ggtaattttc
85081 tgtataaaat aacaaaaata ccatctaaaa atgataacgc aggaaatttg aatattcagt
85141 actgttttaa acctttcatt cttttaatta attttattta tttcacttat ttattaattt
85201 aatttaattt ttttttttta gctgaagtct tgctgttgcc caggctggag tgcagtggtg
85261 cgatctcagc tcactgcaac ctccgcctcc tgggttcaca ccattctcct gcctcagcct
85321 cccaagtaac tgggactaca ggcgcccgcc accacgcccg gctaattttt tgtattttta
85381 gtagggatgg ggtttcactg tgttagccag gatggtctca atctcctgac ctcatgatcc
85441 gcctgccttg gcctcccaaa gtgctgggat tacaggtgtg agccactgag cctggcccta
85501 tttatttgtt ttttaaagag accaggtcac gctgtgttgc ctaggctgcg ttagaactcc
85561 tgggctcaag agatgctcct gcctcagcct ccaaagtagc cgcgatatag cacgtaccac
85621 catgcctggc tctctttcag ttaactttaa agaaatctgg tgtttttgtt tttgtttttt
85681 gagacagagt ctcgctctgt cacccaggca ggagtgcagt ggcactatct cagctcactg
85741 caacctctgc ctcctgggtt caaacaattc ttgtgcctca gcgttctgat tagctgggat
85801 tacaggcgct ctccaccata cccggctaat ttttgtattt ttagtagaga caggtttcg
85861 ccatgttggc caggctagtc tcaaactcct gacctcaagt gatccgcctg cctcagcctc
85921 ccaaagtgtt gggattacag gcctgagaca ctatgcccag ccttcactgc ttttatgcaa
85981 aggaataaat agagtgatta ttaaagtgct ctggatcaca cacatacaaa cacacatatg
86041 cacatacata cacacacaca aaaatacaca aactttaggt aacataaatg tgtgtggctg
86101 tcagtgaatg ctgcattttt ctgagcttgt aaccttccct ttactaactg tctccacatt
86161 atgaccacct gaaaaatgct acactgcagt ggtacattct tcagcccaac taggattgga
86221 tcagaggagt tactgggttg atagtcttgg actgaagctc tctaaagagc agaatttctg
86281 tgatgtgcag gtagcttact gttaccctgt aagctccatt ttttatattt ctttgcccct
86341 ccttctcaat attcacacag ttttcacagg actgattcaa gtgtgaagcc aggatctcat
86401 cttttaacag agtggcattc cgcacagtct gaggcatgat ctctttgtgt ctaggttgct
86461 ccatttagct tcaaaggcag catgatgatt tatggtccat ttgtattagg gaagactcag
86521 aggagggcta gaattgtgat ggttggatta attcattttt tccaggcgca ccctttcctc
86581 cctattcttg gttgttttgc agatatccca agtaattcca gattcatttg gaaggtttct
86641 ctctcagtag cctgctctct aggtagttct caaattctaa tagacgcaca gacacaaaaa
86701 tctgcttggt aggtggggtt tttacttttt agtgactttt atcttgtgtg cctgacctgg

86761 tgcagcatct ggaaaagtat tggtagaaaa gaatattctc agtgagaagg agaaggaaca
86821 agtaaaacaa tgtgaactaa aggcctggca cagtggctca cgcctgtaat cccagcactt
86881 tgggaggcca aggtaggagg gtggcttgaa accaggaatt cgagaccagc ctaggcaaca
86941 gagcgagacc ctgtctctac aaaaaaaaaa aaaaaaaaaa ggcaaaaaac cccccagaat
87001 tagccaggtg tggtggcaca tgcctatagt cccagctact cgggaggctg aggcgggagg
87061 attgcttgag cccaggagtt tgaggctgcc atgagctatg atcacaccac tgcactccag
87121 cataaaaagc ttggttattt atcttagtta aagctgtcct taaagaattt ataaatggtt
87181 gcaaatattt ctgaaaatca tagatgtatg gtttctatgg ttcctggtta ctttatccag
87241 taaagtaaca tgacagctta ttttatggat agtcactaga ttgtaaacct ttggaagttg
87301 ggactgttcc attcatcatc ttcacattcc ccactgtgca aagcacagtt tgctcatttg
87361 ttttgtttca tttttttccc tttcaactat agttttcaca tgtaaaccat caaattataa
87421 gagattaaag ataatttagg atttagggtg ttttgatttt ctagctcata aaataaggat
87481 ggaagagcag tgatcagttg tggtaattga ctgttatggc agaaggcaaa aaggcacaga
87541 gaaaagagat agaggacaag cctgttctgg ttggcatccc aagtctagat cctccatctg
87601 ggagcccatg gctcagataa tactccaggt aatcatgaat tgtgggagtt aaccctgttt
87661 aatgccccac aaaggaacag acctgtagga acaccttctc ctgtctgctg ctgccacaga
87721 gggtagcaaa tcccatgcta ggcgtggaga ggagacttgg gcatgtccct ggagggacac
87781 tgctgcagtc acttcactta cagagctggg gaggccctga atggtgtcag agttcacagt
87841 gcaaagcagt aattatgttg aagaatgtga catctcaaga ttcaacaagt tttttttttt
87901 tttttgcctt aaacatgaat gcctattcat agagggatta taggagcaaa gctctcttag
87961 atattaaaca ccctgatcag aagaaaaaga gaagcaaatt cctgatgtca ggaaatagca
88021 gcaaattttt acagaatgct taagcctggc acagcaatcc taagaatgtt gttcagtttc
88081 atggcttttc ctctcatatt gggctgaatg taacttgtcc ttcatttatg taacaaaata
88141 tttgagtgct tactgtgagc taagcacttt ctcagtgttg ggagtaaagc aacaagcaaa
88201 gcaaatccct aagctttggg gagatggaaa ataaatatct gataaagtat atggtagtca
88261 taagagctgg gaggatagga gatggagtag ggtgatgggt gctgttggag ataatatgtc
88321 agggaagggg gggtgtttag tagagggctg aagtgaggga gaaggtgttc caggtgaggg
88381 cacaggtgtg aatgtagacc ctaatgtgga agtgtgcttg ggtgggtcaa ggaagtgaac
88441 cctaagagga ccactgggca gaagtggaag agctaggata gggaggtggg gctgagggtg
88501 accaggggcc aagccatgca gagcctctta ggtcctgagc tttctcagtg gtctcccctt
88561 catctgcttt ggagcttgct ttcctatgtg gcaacattag ttctagtgtg atgaatatta
88621 gtgggaaaat agaaagttct atataaaact tacttctgga aactaaagga ttcttgctta
88681 gacctcagat gttgatgggt ggtagattaa aggccgtttt ctcatttgtt gagctgtgcc
88741 ctgagatgag ttttctatat tcgtgtggta gagaattcat tgaaacttga atacgtgcca
88801 ttatgtgagg cagaagtgta tattctatga aagggtgttg acatattgtc acataccatg
88861 agctgcttaa tctgggaata tttgtaaaag tgtcatctgg tgtgaaggct gatatttaac
88921 tatctttaaa aatactggaa aaagacaatt tgcttgcaga ttttagggtt ttgctgtggg
88981 tctgtttttg tggaaatgat ttgtgttcag tagcattttt tcccctacag ttttcatatg
89041 gtactgtttt gacttgtttg gaagataatt tgcttggctt aaagggaatt attcgaacaa
89101 tcttagaagt ttaaaaaatt atgttagcta gaaatcagaa gtttttgcct ttactgaaaa
89161 gttcattatt tattcattaa tttatccatc catttataca acaaaaagtg attcagcatg
89221 tagcgtgtag cagacagtgt gctgaagata cagcagatgt aacagatatg gcttgattag
89281 tcttgctagg cagcaagaga caaacatatg catacacttc cacactaatt atggtaaggg
89341 ctaaaactta ttgaaaacag tatagtgata gggaatttgg agtggggtgg ggagctgtgt
89401 aggatagtca gatgagactt tttttttgga gacagagtct tgctctgtcg ccaggcttta
89461 gtgcagtggc gcaatctcag ctcactgcaa tccccacctc ctgggttcaa gcaattcccc
89521 tgcctcagcc tccctagcag ttgggactac aggcgcgcac cagctcgccc ggctaatttt
89581 ttgtatttta gtagagacgg agtttcaccg tgttggccag gatggtgtcg atttcctgac
89641 ctcgtgatcc gcccacctca gcctcccaaa atgctgggat tacaggcgtg agccactgcg
89701 ctcggcctag atgagacatt tttgagaaaa taacatttaa gatgaggcct gaaggaaaat
89761 agtgccagcc atttgaagag tgagggaaag gggtttccag gcagattcaa tatattgtgc
89821 aaaggccctg aggaagtgtg tggttttagt ggagagctaa agaaccatga ggcaagagca
89881 tcgggatatc caagaagtca tgctggatta catatactgg tcagtaagga gcttggattt
89941 gattttactg gggaaataac ataatctgat atgtttaaaa tgattactga ggcttctaag
90001 tagagcatta cactgttaac agtagtggga gtgagaggtc aggcttggtc tagggcagtg
90061 cttctcaatg tgtggtctca ggatcagccc tatcagaatg acctggaaac ctgtaagaaa
90121 tgcatacttc aagtcttatt atggacttgc tgattcagaa cctggaggtg ggactcagca
90181 atctgcttta acaagtcctt tggatgatct gatatacaga aaagtttgag aaccactggt
90241 ctagggattg ctggaggaga tggtaagcag tagatggatt ctggtgtatg ttttgaaggt
90301 agggatgata ggacttttgt atatgggagc tgaggaaaaa ggagaaatca gaaatattat
90361 tattattatt aatgtttcaa gacggaatcc cactgtcacc caggctggag tgcagtggca
90421 tgatcttggc tcactgcaac ctgataagga ccttgtttat tgtgatgagg gagactggtg
90481 ggggtagcaa aaggacataa cttttgaaca cattgcgttt ggagtgttca tgaaatgtcc

90541 aagtgaagat gttggctgtt gatggcaaaa tgagcgtaca gtccagaggg aagatgtagt
90601 ttcaaataca gctcagcaga aagatacagt ttccagaaag gaatttggga cttgccagtg
90661 tacagggagt atttaaagca attgaaatag gtgtgatatc accaaggaga gagatagcaa
90721 ggagagaggg tgcaagagcg ggcaaaggac caagctggaa ggaactcttg acattcacag
90781 gagcaagagc cagcaaaaga acagaaagaa cggccaggga gagaagagaa gaaaaccagg
90841 aaactggttt catggggaaa tatttcagga acagagtgat caactgtgct taatttgtat
90901 gatgaagcgg agatttgtag catagagatc actaggcatt ctatttggac tgtcagtgaa
90961 attgaaataa aaagtcaaga atcaacttga tttccatcac aggttttaga ttagaagaaa
91021 ccagagaaac acagccccaa ttttctattt gctagttttc caaacagtaa gttttcattg
91081 atttcactgg ctcccctccg gctactattt gctgtgcaca aacaaagtct gaatgagcat
91141 tttgtagtcc tgaaaaggag tagcatatgg gcttcaacag ccggtcatat tttcttttat
91201 tactatattt aaagcaacat tcaagattaa aaaaaaattc ctggcaacta gcataacaag
91261 ctgcaataaa atcattcctg gctattgata tgggctactt tgtgcctcat gtcatgttat
91321 tgaatccaac ttcttggaat tgactgtctt aatctgtcac ttagcaaatt tatattggtg
91381 aggaagaaca aaaacagatt tataacttgc atatctcttc aagcttctgt taatatgctc
91441 aatattagaa tgttctactt ttagcgtttt gggtcataag ctatagaaca ttggggcttg
91501 gtgagaggat atgggcatgg ggagaggaat gaatatttgg cattatttat gtttgaacca
91561 ttgagctggg gtctttaggt ttattatttc atttaattcc cactctgacg taggtggtat
91621 ttccctcatt agtagataag gaaatgagct agtaaagaaa actatttaaa tccagatcta
91681 aaatggatct ttattctgtg ccaagctctt gggggaagag tatgtgtgga ggaggagttg
91741 tggggactca cagagcagtg gcaaggcaag ccagagatac agcaaaggga tatcaaggac
91801 taatgaggta ctttaaagga gatcgagtca tggtaaggca tttaaattag ttttgttagc
91861 aagtactgca tggaacagga agactctgta catgctcagt gtagggcttg gtccgtcaag
91921 actggctagg agttttctag atagagcctt gaatcttgca ttacagtcac tacagagcat
91981 gagctctgtg gacatttcat gaaaagcttc ctttctactt ggatatattt ttttacttct
92041 tgtgtgcata cttgatttat ttactgttcc ttatagagct gcaaggcctt gtttcttttc
92101 tatagaagtt atatttctct gagttcagat gtcacatctt cgttaccttc tctcaagttt
92161 tgctatacaa gcttaggaac gtcatctctt tctcttccag agttatcttc catcatgtag
92221 gctttggagc cactttcctc agagcctcca tttgcctcac ctcttcttgc ctgggacctt
92281 gtaaaaacag tgattgtggt aattaaattg agatatgagg aatatatata actattatgt
92341 agtcataata attaaaaatt taaaaaaaat gcaatgagag gggaaaaaaa tctcctggat
92401 tggtgagcta gacaaaactt catgttaatt aacatggatg ctttaatcca tctattcctg
92461 agtccaggac atcaaatctt gggttctaca atgggtgcca gtcttaggag ggtcatatgt
92521 atttccagtc acaaaagatc tttctgtcat gttggagaag acaaaagttt ataagaatca
92581 gtgggccatt taaaatgata atgagcaact taggcgtaaa gccagatgtc acaggctggt
92641 ggcttgcagc ctgctctact gtgctttaaa tacgcagtat ttaaaaaatt gatgttggtt
92701 cccaatattt ataaattggg agatatcagc cataaatcta gattccatcc tcccccacct
92761 ttaaaaaaaa gaaagttgga tctgggaaca ctaggtctat gtttgtgctt agcaggaatc
92821 ggaagagctg ggttgtgacc aggctctttc ttttagcagt ggcaagctct tttcagcttt
92881 ctgccatccc gaaagcaccc tagtgtttct cattggccgt gatgcccatg ttgactgcaa
92941 tttgtcattg tcctcaaggc tgtcgttttt tccacagagc cggctttgct ctttgacaaa
93001 gctactttgg cctccttata catttgagtt cattgatcct tagcctaaat gatcaggaca
93061 tgacacattc agaagtttgt attatcttgt aaaagcacat gagcaaagtc tgtctagtaa
93121 gtgacattgc tggtattcag accaggatat ggctgagagg aagcacatat gtaaattgaa
93181 tgcatcaggg atcttttgga gaagtcaaac aagaattctg attacctcat cagtgatacc
93241 taaatgcaga tttcttcttg cttcagacaa aatttttcaa agatactcaa agtatctcag
93301 ctcttgttgt atgtgtgttt tgaaagatag ggttgaagaa aacaatgaaa agataacagg
93361 gatatctgtc ctccagagtt gttcagggga ttcagcatat gagaagttat ttgcgaagtt
93421 ttagttccca gtctgggcat cagctcatta tcattagtct ctgatatgag ttgtttaatg
93481 atgaacttgt gcaaagcact tgatcctatt cagcgtcaga cattcattgt taaaagaaca
93541 cattgaggac actgttgtag gtcaggaaat tgaaggtagg gagttgaggg caagtagaag
93601 agagtttcat tctggagtta gttcagtaaa cattgaatgc tcatgatgtg tcaggcttta
93661 tgctgctacc agaggcagga agataaataa gacagaacat gcagacttga acagtacggt
93721 gtggtaagag gtgtgcccat ggtactgtgc gattatagac tggcgttggc ccagtgcagg
93781 gctagaggga tgctccatgg tgatgactga gttgagcctt aaggcccgga tgagctggcc
93841 aggtagtgat gacaagaggg agagggattt aaagtgtgaa gttcaattta aggaacagag
93901 atgatgcagg aagggctaag atatggaagg ctttgatgaa gattttgggg agccagggaa
93961 gaaggggaga gatgatcaga tttccatttt gattgcagct tggtaactgg attacagtgt
94021 ggcagaactg gaggctggga gaccaggaag gtttcaacca agttagaaaa tcatttttgg
94081 tttagaagac agtgaggggc tgggtgtggt ggctcatgcc tgtaatccta gcactttctc
94141 aggctgaggc aggaggatca cttgagctca ggagttcgag accagcctgg gcaacatagt
94201 gagacgcacc cccccgccca tctctattaa aaaaaaaaaa aaaagacaga tgaggatggg
94261 atctggagtg gcacaagcca tgatttaccc cctcattagg tgagatactt tttggaagtg

94321 ctgacctttt gccattttga agaaccagct tttagatgct ccattatgga cttagattga
94381 ggaagattga tcaattttat tattttgctg ctttacattt tcaagaggtt tttttttttt
94441 tttttttttg aggaggaggg aggagaaggg aacttactgt aatgtaattt tgctaaaagt
94501 ttcccagaag tgaatcatgt ttatcaagat aaattgtaga aagaagaaat ttgtttgctt
94561 ataatattgt ttctatggga aatcattctt caacaacaag cagcaaactt cctgggagga
94621 ggcactcagt atattcttct gcccctgagg aagttgagga ttgaagtatc tcccttgctt
94681 gtactctggg actagttgac tctaagcctc cttccagcct cctgctgtca aaacaacttt
94741 tcttctgttt attttcatat aggagctgca caactcaaat gttatgtgtt atgccaggac
94801 ctcagtagca ggtgggggtg aaaagccctg cacagtattt actgggaaag gattgcctgt
94861 tcctgaggca ctgcctccat gatagacgct ccactgatgc tgcagcccat gataggctaa
94921 agagattttg tgggtactaa gttctattaa aatttagttc aggctgtcat cgctgttctc
94981 caggaaacct cagtaccctt ttaattagcc ccacccaggc tgtgacctca ccctgatcca
95041 ttgtctgtac agctgccaga gtgattttct gaactgcaca tccaagtgta tcattctcct
95101 acttaaaatc cttcagcagt tccccagagc taatccatgg gagtgggaag gcaagaatct
95161 gagtctcttg caggacccgt gaggcatccg tcccccaagg gagccgaagt tcagtccttc
95221 ctattagatc tctagaaagc tacgtgactg gaccccatta ttctacactc cctgaagccc
95281 acttacttta atttttgatg cttgtctttg gcttttacaa attgatatga gaagccaact
95341 gtgaagttca ggtccatgtt gtttgttaaa ggaatcataa gtctgttttg caattcaatg
95401 ctttctttaa ggacctatga atacttgtta aatgaatagt tcatattcta aattgctttt
95461 agcatttagt gccaggaaaa aagaactttt ctcatttaaa aatcttgtgt gcttcgttta
95521 aagaatcaat taaaataata gggtgtttgg ttttttaaaa acataaattt tgttttttta
95581 gagatgggga ggtggtctca ctctgttgcc caggctggcc ttgaactcct ggactccaag
95641 tgatcctctc gcctcagcct cctgaatcac taggactatt gtgtatttgc cttttgactt
95701 ttgttgccag gagcctcaaa tctaaagata ggtcataata gtcataaaag tagtgatgaa
95761 ctggaaatct gctgtttttg tctttccagc ctaggagcct tttccccatt tactcgtttg
95821 tttacaaata ttttttgagt ggttgtcatg tgctgggcac tgagaaagct gtagtgaata
95881 agatgggtag gcaaaaatgg aagtggttcg attgtgctca tccaggtatg agataatagt
95941 ggcttgggtg agtgtttgca gtgaaggtgt agagaagtgg gtgagttctg aatgtatttt
96001 taaggtagaa gcaagtgaat ttgctgatga tcaaaagtgg ggaatgaggg aaaaagaaga
96061 atccaggacg attcccaggt tttgattagg tatgtgatac tgacttttgc tgaaactgag
96121 ggacatgttg aggaagaagg gaaggacagc tgaaaacatt tgggaaatgt taatttgtaa
96181 tacttgttac atatccaaat ggagatgtca tttagttttc cagagctcag ggtagagatt
96241 ctgtctccat tccatagcac cacccaccat gcaaacatgc atacacacac acacacacac
96301 acacacacac acgcgcacac acacacacac acttctttta gggaacttct catgtccaca
96361 gcaaccatgt gattctacta tggtttcagc ttcttcagcc acaaaagacc tgatacttcc
96421 gtgcctgctg gcctttgtgc tatagtctcc tggccacagt gattagtcca gggggtggac
96481 atatgaccca agagtccttc attggggtct agggaaatac cgccttgaag agagaggctc
96541 tctttttctg ctggagagac taaatggttt acactcagat gtgattcctg ctacgtggag
96601 gaagtccatc tgaatagtac agattgatga ccttgcccag aaaagcagag attctacaga
96661 aatgtggaaa gtcccctccc tgctgtttac tacactgtat cccttggaat gccagcgctt
96721 aaaggtgtta agtgagtaaa acttctttgg tcaaataaat ttaaagaaag aaatttcttg
96781 acagcagaac ttttcagatg agcctttgta atgaatatgc ggctgggcat agtggctcat
96841 gtctgttatc ccagcacttt gggaggctga ggtgggtgga ttgcttgacc ccaggagttc
96901 gagaccagcc tgggcaacat cacaagaccc cttctctgca aaaaatacaa aaaatacaga
96961 aaaatcagtg atgagtgcag ctgtagtccc tgctacttgg gaggctgagg caggggcatc
97021 gcttgagcct ggaaggtcga ggctcctgtg agccaagatt gtgccactgc gctccagcct
97081 gagtgacaga gcaagacact gtgtaaataa ataaaaatat gttttgtgct tctttaagag
97141 gaggattagt aaacagtgtt ccccaaatct atttgacagt ggagggtttt tttttgtttt
97201 taaatgggac atctataaat atctctcagt tggaacccag cttgaaatat tgcttgaaat
97261 gctataacag gttgtcattt ttttgtagtc atagtgggga tgggtgagtt gttctttatt
97321 tctggcaaaa ctggtaggct ccatataact gtcattactt cctgatgttt tatagcttgt
97381 taaggagaca aaagcctttt ttcttctcct agtattttaa catgttagta ccagatatta
97441 tctatgctta ggtatgaatt ttggctcaga gattttctca gcatcacacc aatgataagt
97501 ttcatgatcc tagaatgtac tttccctgta ttctttctcc tctgctggtg ttctgtgagt
97561 ccacgtgtat gtgtgtgcag gacaacactt aaagtggtta ttctttcttc aaagactcag
97621 ttcaacacac ccctggtggc tgttgtagct cagggttgag tagtgatcct ggtattaaag
97681 atgagaaatt tgttaggtag ccgctatgag ctgcaaggtt tcctgaggca gaggtatccg
97741 gaacagagtc agggaggcca tggggccaga ggccttcctg attctggaca cacagagtac
97801 cttggtttcc ccaccctacc ttaaaaatat attcggattc acttataccg attaattact
97861 tagagagagt gaatgtatta tagttatgtg aacttgtact ccctggctgt ctctcttctt
97921 atgccttggt gatttgtcta gaggataaat tgctttttgg cttttataaa ttctgttttc
97981 ttctaccaac tgctaaccat taacatctca atcaaatggg ggaatttgca gttatcagct
98041 taactaatct ccatgcccac atttcttgct tctctccctg ttactgaccc agatccttga

98101 tgataactca ttgtagattt tgttatcatt taaaaccttt tcaactaggt acttgatata
98161 atagttctta ttaattttta attaatttta tattatttag tcatttgctg agttggtaga
98221 tatttatctt gtgatttctt ttaaaaaagt cttagttctg agatgaacct ttaacagcac
98281 ccttctgtgg cagtctacta agcacagtgt taacctgagg tctttggaaa ggccagcttc
98341 tcacagatct aagggatgca atttgctggc aaatctttag ttggccagtc ttgactacat
98401 ctgatcaaag ttgggaagtg agctgctgga gacggtgaca ttttctgaag catgaacagt
98461 aacaatctga aaccataggc ccttcacacc tcttctgtca tttcgtcttt gctaccgtcc
98521 ttggaaatag cctgtcttta caaaggtttc tatataggtt aagatacttg cttgaggtca
98581 tgcaaataag agctagtctg gaggaaaaac tcagtttcct gtctcttctg ttcatttcac
98641 tgtcctgcat ggggttatgc tttttctttt caatgaagcg taggaagtct ccttgggaat
98701 catctgtgct gatctctgtg tgtgtgtttt aaattgcaat ttgtgttgca actggtaatt
98761 ccagttcttg tatattacta aggctttctc tgtagatgga tagttaatcc gtgaaaatcc
98821 cgaaaagcat gcagctctgt tgatttgatt ttgttataag ataaggaatg cataatcact
98881 ctgggttcct ggctttaaat tataaggatg tgttaaatta tgaaaatgaa atgaggtttc
98941 taaaaattct atattctgga aaatgctatt aagcaatttc cattttgatg caatgtatat
99001 ttaaaaagat atattttctg tgtatagttg tactgttttt aatctccttg tgattttcct
99061 gaagctgtgc ttaaatgtga acaattgttt aaaataaacc aataaaggag agaaatagga
99121 tgaagatatt ttacatgttt tactaattct gacatgtccc caagtgataa atggtattca
99181 gaggctgtct gtacaagaag ggttcttaca gtccatttct agactaattt cagagaacgc
99241 agcacctgag tggccaggtg agctgtgctc actgtggggc ggcagggtgg gcgctaaagc
99301 cttgagtcca tgctcaatgc gctttcccct gccccgtgct ggcatccaga tttttgttta
99361 ttgtctctag tttctacatc ctttgtaggc aatgaaactc accttatatg gaattgtacg
99421 tgtcttttat aggaagagta tgtgtgtcta agttaatcac tcctggtggg atctctgtct
99481 gctctgttac tgtgttggcc attgttactg ctctgttact gtgctggccc aatgaaatag
99541 tctctcggcc atttgactac ttcagtttag gccctagatt gttttgcaac ttgcttccta
99601 gttcatcccc cttccaccca tcttccatat ttccaggaga aaaaaaataa aaataaaaac
99661 tttaatgtct cccccaaccc ccagatttta ggtactgttt tctgccccct gtctcgcttt
99721 ctagctgttt ctcctctcct gtttgcatgc acccttctca ttctggcttt aggaactact
99781 tgctgtcttg acagtgacag gtttcatccc tccatgcttg taggttgttg caccatccta
99841 tgaccagaat accttcctct ccccatttca gccattagta ctcagcttct gtatgtgctt
99901 ccctaggaag tccttccagt gacagactgt actctactca cctcccctgt gagcagccta
99961 aggcagtatg gtagcgaaga gtgtgggctg tggtctcagg ttgcctgggt tcaaacccag
100021 ctcctagctg tgtgacgttg gacaagtcac ttaacttctt tgggcttcag ctctcatctc
100081 taattaatag tatttaaggt tgttttaggg ttaaatgact taatggatgt aaatcattca
100141 tgaaagtgca catacgaaca tcctgttgct cacaatctag tattactagc ttttaagcct
100201 ggaaaaccag tatgattcca tttttgtacc ctcaggcccc ttttatagta cataggacat
100261 ggtaggcact caataaatgt tgcttacttg aataaatgaa acaaaaaagt caggaaacac
100321 agaacactta ttttagtgtt tgttaagtta tgccctgctt tgttccagaa aatgatttat
100381 ttcaattagg gtttcagatg taccctttaa ggagcaatgt atgtatctgt aaactttata
100441 ttttcctcag agtcacaagc cgtttggaat ttcattttga aattctcttt ccactaacat
100501 ttatagtttt ggcacagttg ccctttaaaa agacagcgta tcaattgaac ataaatatat
100561 tgtgtttaaa ctatatgcct gcttgaataa gtgcagtact tatttgtcag ggcatatatt
100621 atgttggagt atgatgtcat gaggcttaag gaaactaatg cttatcattt gttagtgctt
100681 aaaacaaagt agagatagcc aaatgcattt ctctacttcc ttattgccat acagctctcc
100741 ttgtaagtta ataagtatct tattgcaaat cagccctgta tctgttccaa attcaaatgt
100801 tgatggttga gcttccagta ttttctccca tatattcaca ctgaataaca tttcctgagg
100861 attgcataag acccatttaa catttgtgaa ctatgagact tctttgtaca tttctgtttc
100921 tttagttgta ttacggtaat cgggcatatg tcttctaacc ttgatgatct cacctctata
100981 taatataagg atgttgggag aaatatgaaa tctagaccaa ctctaaaaaa ttgaatgtca
101041 aggtaattgt cttgtcacac aatggtgtaa ccagccaaca tcctcgaaca gatccgtgtc
101101 cataatggca tcattgactt ctgaactgta tggtggtgtt ctctgaaagg tgctatagag
101161 aaaacacaag tgtgaagggc gattgtgatg gttgtgggtc agaggctcct caagtggagt
101221 gaatgaggcc agccaggaat gaagacaatg cagtgaatga ttcttttgtt gcatagtaaa
101281 atgaagtact agaaaatgtt ctcttaacaa agaatcatgt ttggtcagag tggacgtgat
101341 atagtgagga gtttttgtaa attcttcatt tagcgactaa tattttggct cagttaaaga
101401 tatgaaaaac attttttaac aaaaaggtgt tagtggccgg gcatggtggg ctcactcctg
101461 taatctggct ttaggaacta actagctggt tctgactagc gtatgagggt ggggttaggt
101521 tttccctatc ttccccccttc ttccatagca ctttgtgagg ctgaggcggg tggaccacct
101581 gaggtcgggg gttcaagacc agcctggcca acatggcgaa accccgtctc taccgaaaat
101641 acaaaaatta gccaggcgtg gtggcgcatg cctataatcc cagctactcg agaggctgag
101701 tcaggagaat cgcttgaacc caggagtcag aggttacagt gagccgagat tgcaccactg
101761 cactccagcc tgggcaacag agcaagactc tgtctcaaaa aaaaaaaaat aaaataaaag
101821 gtggtagtat aaaaaaaggg gcactgtatt ttatagttac aaatactgag tcattaatgt

101881 tacaggcgta caatgctcca cttgctatga gagtgtgctg ctaagctctg tctccagaac
101941 ttttgcccct tggtagactc tgtcttgcac aggtttcttg tcagatcata gtcctcattt
102001 tctgtgcata agatggaaat agcatgcaga ttataccaca gattgtccta gtagttcttt
102061 tatatgtttt actcaaaaaa gctggtccta cttttttttt tttttttatt tcttgactag
102121 ttttgttagg agaatttaca cttttggtag tagttttgcc attatgtcat tacttatgaa
102181 caaatgtagt tattgcactt actttataga gccagctcag aaaatttgat ttcaatgaaa
102241 ttctttgtgt ttagcacttt tccaataagt gggccattga ttcggttata acaaactatt
102301 ttgatgaatg agttgttctg tttatggaag gattttacac ttaaatgttg cttcttgggt
102361 tgtgctattt ttgtactttg aaagttacat aaaatgaaaa gaaccagcac ataagtttta
102421 tatgctgaga gttgaagggt gatcccattg tttctgaatt tgagtctctg ccagcatgaa
102481 tcctcctatg agggtcccta aaggctatat tatagtttcg atgaaatgta tacattctat
102541 gaatgaaaaa aatatagaat ttccttttca tgattttttt tcttagtttg ttcccatttt
102601 tgtccccatc aatataaaat aaattaaaat gaccacaaca aactcaagga ggtgaaaatc
102661 aagaaattgt tacctgtatt atggacaggt ggtgtagcgt cacctctctc atacacaccc
102721 actatctcca ataagcctag cctgggaagg agaatatttt cctgttagct atttgagtcc
102781 tcttgcgaag tcatttgaat atgtgagttc tggacattgc agataactag ttggttctga
102841 ctagcgtatg aggttgggat tagattttcc ctattttctc tcttcttcca tagtgagatt
102901 tgtggagggt caattagagt cttccttgac acaaggttat ttctttaatg tagcacaggg
102961 gaaggacatc tttttccttt tatgaattct gcccaatcct tttctgggct gatctttgat
103021 tcctatcatg gggtctgtcc agttttcagt attcttcctt gtttgggtat tcaggtagta
103081 gatatctcag atattcttcc ttgagtacaa aagtatctcc cagcagtgtg gccttaggca
103141 gatttgtggt ctttttggct gtgctgtcca gtactggaaa ggataccaga tagaagcttc
103201 agttctcagc agggtcaggt tgcctctggc tgggcagtgg gtgtccagat gcagggatag
103261 tggtgtagat gcagggcagt gattaaaggc tttggagttt gaatcccaac cctgctcagt
103321 gtgaccttgg gcaagttact tacctgtgtt aagcctcaat aacctgtgcc attggggcct
103381 ttataaagat taatgaggtg taatataaaa tacttcacat agtcattttc tggcacatac
103441 tcagtacgtg gtggaggtta tggtgatttt attaaaatag aattggctgt agatgcccct
103501 ccccactttc caagatccca acatcatgcc tagtctgcct gcattgttgt cgagcatgaa
103561 gaaataatcg attttgtaat ttattcggct ccttattact tttccattct tgcagatcca
103621 ttgcccatat ccttaagatg ttttctttaa gccttaccct agtcagagac tattttctaa
103681 atagaactaa aagagaaatt tgattgagca tctgaatctg accttctccc caattaaccc
103741 agttgggcag accttcattt agagtgtcat ttatttttca agccacgtgg taaggaagca
103801 gagggttctc cttacttcta aagtcaattt ccattaaaac cttcagtgtt cagtgtgtag
103861 cttaatgcct tttaaaatga ttgctatttt tgaaccagtg tttttgtcac aacgttttga
103921 gtgtagaata ttccttattt gacaaccctg agcttgtagg agtttataaa tggctaagaa
103981 caatctgatt agaatgtatt gcttttggtt ggaggccaag aatattggat cattggttta
104041 gggtatgaca cttaaagaga ccaaagagca gagtacataa tgcccagata ggccaattca
104101 tgtagctttg ttccataatg taagattgaa ccccaaaccc tggccaccta tctttgagat
104161 atatgctgtt ggccacagga gcattaatta ctccgggcag atcagcagac catgaactac
104221 atgctacgag gggaaatcca aatgcctcaa tgtacaggat agtgggtcag tagaagaaac
104281 ctcacaaagg aaagctggag aaggctgtca atgaaggttt tttttttttt tttttttttt
104341 ttttaagtcg tgtaagaatt gctaagtgac agatcagagt ccagaagaca ggaaaggagt
104401 taggaaattc tgagatattt aaattctgac catgtaataa atgattccct tgtggaagat
104461 aaggggtaag taccaaaagg aaggagtgtc acaatgtggc agttttctaa tgaacttagg
104521 tcttaaaagg aagcacacac aagatagaag gctcacaatc aggagggact ggcagtgatc
104581 ttagctcatg ttaatatact gtgtgtcagg cagtgttcta agcactcaca tccttgtttg
104641 atctcatgat aaccttatga agtagatggt attattattg ccctttacaa gaggcgtaaa
104701 cgggaggctt tgggcgttta ggtatttgct gtcatttaca tagctagtta agtaactgcg
104761 ctgggattta agttgaggta gtcagattcc agagagatct gagacttgca gaaggtgcca
104821 gagacaataa aaaaaagtag ttgaaagaag tgttcagagt cataaggaaa gaatatattt
104881 gcatctgagt ggaactgatg gtggctgtgg gtagtcatag agcatagcct tttaactcct
104941 gttgtctatc tatcttttat atatagaaaa ctcagaatgc cggctgagca tggtggctca
105001 tgcctataat cccagcactt tgggaggctg aggcgggtgg atcacctgag gtcaggagtt
105061 caagaccagc caggccaaca tggtgaaaca cagtctctac taaaaataca aaaattagcg
105121 tggtgcacac gcctgtagtc ccagctactt gggagggtga agcaggagaa tcacttgaac
105181 ctgcagggca gaggttgcag tgagctgaga ttgtgccgct gctccagcct gggcaataga
105241 gcgagactct gtctcaaaac aacagcaaca acaacagaaa actcagaatg tcaaaaatat
105301 gttggagaag taataagaca gacatagttt ttctaactga atttaagcct cttagcccga
105361 cagaatttct ccacagggtt ctgagaacat tttggatcca gattgttcaa ccaagtccat
105421 gatgatctgt gaggaagcat ggagaacaga aataacactg gaaaggcgga ggagggggca
105481 caggtgcact ttggtgcgga aaagtttggg aagcattgtt tgaaagaagt tgatatcttt
105541 aaatattcag ggctagttgc tagcaactta ctatggctag ttgttgaaag ctgcagtgag
105601 tctgttcatt agctagtggg cagcccacat ttagaagaat gtaggtgtga gatgggagag

105661 actttggcat aagaatggtt cagatggaat gggaacacca gggggtgatt cgatcattaa
105721 gctttcctct gagaatgtga tttggtatct ttgaaactag gccagagatt tatttggatc
105781 tttggttttc catttgtgaa gaggtcattt agtctcattt ccagatgaag aaatttgggt
105841 tcagagagga tgggagtgac tttccctaag ccactgagct gtttggtgac caaggcaggc
105901 tggatgctca cctgctttct cgctgctctc ttcttactgc agctcacctg tggacttgtg
105961 gactgggcca tcaggagtct ctaggaggca gcattgattt tgggataggc ctacctgggt
106021 tcaaatccca gctctgacac ttaatctgta acagtgttac attattctga ggattagtga
106081 aaagggaaca cttagcatat aggaagggct aagtgtatgt tcttgcctct ttctagttag
106141 gcttgaaatc tttctttatt ttatgtactt attattacta agttttctag cacctcccaa
106201 ctcatgacca aaaagtttca ttttcatctt atttttctct ttggatgatt tttataaggg
106261 tatttcttat agtttctcta tatttaaata ctttatactg tctagggtat ttaggcaaag
106321 gattcaacta cagggctttt agaaatcctt tccagctcta aaattatagg atttaattgt
106381 ttttgaaaaa gatgccttct accttcctcc tcctccagct gcacacgtag tttactggtt
106441 actgtggtaa ctgcactcta ataatcctgt caggaaatct gatgcaacta tctgtctaat
106501 cttattttcc agacacaaaa caagcacacg gcttccttct aagaatcctt ctaaagcgct
106561 ccagcctaag cattgtttct tcttgttctg atgtttttta agattgcact tagagaagaa
106621 tgcacctcat tgggtgtttc ctcaatgtgg aatgttttat agtatccagt gaccagtgca
106681 tcactctaaa gcggtgtgtg ttttcatgca ctctcctgga gttgatgaaa ttttgggtag
106741 tttgaattat aattttaaaa aatgcagaat agggattaga gaggatttaa atttattttg
106801 cacatagagg attgaacgta attactcaga gagttagcta aaatcagtat ttttgctttt
106861 tttttttttt ttggacaagc cagttttaca aaatgtccct ctgctgatcc atgaacaagg
106921 gataagtctg taccttttca tcctccctcc cttcttcagc cattcacctt ccttagtagg
106981 ggtcaagtgg gatccctgtg atgctactcc tggcaaggaa cctctgtact cccagcctct
107041 ccaccaatct ctacttgacc ctcagaagca atctcatcac acgtcttatt cccgtttatg
107101 tcagtcacca taggaagcct ggactcccgt gtcatctctc actcctgtgt cctgatcctc
107161 atttgcctct ttacttcagc ttttccaatc ctgcttccct cccctaggga actcatgaag
107221 ccccttcact gtgcagtttg tgtgctcacc atcagtcatc agcaaacccc ccacttcctc
107281 accttgtctg ggagcatgac cttcatctcc ttgctttatt ggacacttgg gtctctcctg
107341 gcagtactgc ccctccttca gctgtctcaa gtggccaccc tttttcatct ttcccgtaca
107401 ccatgggtag agctggggta gcctcgtcta tgctattttc atagtgctgc tcctccttct
107461 cctgaaacgc cacctcctct gactttcagg tctctgcatg gcccactttg cctccttgtc
107521 actagtcctc tgctcttcca cgtggtcatt cccctctttc tttgaaaaat tttgtttctg
107581 gttcactgtc cctgtctcca acactacttc tgtgagttct ccaggatttc agtccccatg
107641 ttcactccat cccacggtca tatcttgatc ctctcatcac cggtgacttc agtgcctcca
107701 tcactcactt tgaaacatcc cactcttatc accacttcct cttctctcgc aagcctcatg
107761 tggtacctcc aacctcaata gtccattgat ccaccaggtt tttcagtcca ttgaatccat
107821 cacgtcttca cggacccaca ctcaccccat gtcctcactt ctccccttcc ccagcttgga
107881 gtccacgatc aaccactgta atctctacca tggcatagat ccttatcacc cttgacccct
107941 caggcttggt cattcttgct tgactaaatc acagttctgg ttaaatcctt ccctctcttc
108001 acctgttctg tgcttgcacc catgaggcca aatgtgcttg gagaatacca cagcaccata
108061 ttgtttgtct cactctaagc tgatcacaga tcttaagtgg gcccttgatg ctacctggca
108121 gtctcattgt gtttccactg tctcttcatt cccaccactc tctcagacaa ctgttgcata
108181 ctttctactt gctgttcaaa catccatggt ctccttacac ttggttgttt cctttttcac
108241 cagaaacccc agaaataatc agtagagact gccaccagca cacccaccac ctgcctgcgt
108301 ctgtgcccat gctcccacct ccttgggcat gatgttctat ctccatctac acgtaccttc
108361 tcaaggacat tgctgtatga aattttccct ccctttccta tgtcatctat tttcttcttt
108421 ctacctgatt cccactggca tatgagttat tatttttctt attaaataaa aacatcctct
108481 cttgactctg atttctcttc cagctactag ccagtttctt ttcttgcttt tactgcagaa
108541 cgtttgaatg agtcatctgt atgcattagg tctaacatct cccctcccat tctatcctga
108601 gcccatcaca gtcaagtttg caccttaact caccgtactt cactggcatt gtgcttttcg
108661 gggccacggg tagcatttcc ttgccgaagc ccatggtcag gacccactcc cttcttactt
108721 catttaccag cagcatttaa cagtcagtaa gtcccctcca tgaaatacat tctttacagg
108781 actgcttgga cacacatgca cctcattttt ttggttttcc tggtctttct cagtctcctt
108841 tgctaattcc ttatcgctcc aaactctaag caaagtgttt cagggtactt gttcctcatc
108901 gtgtctactc tcagtgcttt aaatatcacc tgtaagatga cttctgcatt cgtatttgta
108961 gcctggatct ctctcttgaa ctccagaccc cttcatctgg ctgcctgctg gacatctctc
109021 ctgggatgcg tctaacaggc atttcaaaac ataacatgcc caatctccaa tctgcttcct
109081 ccacagtcat tcccatctca gttaatagca tcctgctctt ccacttactt aagagaagac
109141 atttggtgtc attcttttct tttatttctt ttttgagaca gagtctccct ctgttgccca
109201 ggcttgagtg cagtggcgtg atttccgctc actgcaacct ccacctccca ggttcatgca
109261 gttattgtgc ctcagcctcc tgagtagctg ggcttacagg tgcctgccac cacgcccaac
109321 taatttttgt atttttagta gagatggggt ttcgccatgt tggctaggct ggtctcaaac
109381 tcctgacctc aagtgatctg accaccttgg tctcccaaag tgctgggatt acaggtgtga

109441 gccactgcgc ccagtctggt gtcattcttg atttctcttt ctcacatacc ccatcagcaa
109501 atcctatagg ctctatcttc aaaacgtatc cagagttgat ttcttatcac ctttattgct
109561 atcaccctgg tctaagctgc cataattttt cacctggatt aggagagtat ctttctaact
109621 ggtctttcag cttccagcct tatccccttc agtctgttat cagctgtgtg cagattacgt
109681 cactcttctt cccatctcat tcagagtaaa actgaagtgt ttaccgtgac tgacaagctg
109741 tggtgtgccc tagccctcca ttaccctctg gcttcatctc ccacttcccg cccacttgct
109801 tccttgtttc cccctctggt cttgctgttt cttgaaacag tccaggcctg agtctacctc
109861 agagcttttt gcatttgttc ttccctctgc ctggaattcc cttcagcttc ctgtccttta
109921 tccatgtcct catgatccct tatcccactc cctcacttcc ttcaggtctt tcttcaaatg
109981 tcactttttc agtgaggact tccctggcca ccaatcttaa ctttccacac ccctcctgac
110041 actttctgtc tcctttgcct aatttattat tctagtattt agcataatct aatagagtat
110101 atgttttgtt gatttatctt attccttgtc tcttttctct cctactggag tgtaagttcc
110161 acaaggccag ggacctttgt tttgctcatt gcttattcct ggggactagg gcgtagtttg
110221 tgtcatggta gggtgtcata agtatttgtt taagagaaaa ataactattt ctggctctag
110281 gcacattttg gcttaaagtc actgtaaatc tgcttgccac aatgcactaa taaagctaat
110341 tattttattt catgttccct cttaaagtat atttattaca ggtgtcaaat taatttaaaa
110401 gcataatatt aggcatgaga gaatacaaat agagaagagt tcctgcagct tttcatgctg
110461 ctttaagtga gtcccattca tggtacagtt ctctctacat gcatttagaa atatgtcagc
110521 ttatttattt aaatgtatct tgccttatta gaaaaacaaa aaagatttaa gggggctgtt
110581 tggaaatgtg atgatgtaag gatggggcaa cttaacatgc tgtttcaaat gtttagaccg
110641 tacattattg atgtcaataa tctcctctct aagaggcact gatatctgtc tattgtgcag
110701 tttgcagcat tccatgccaa agcttttgta tttattactt cagttctacc tctcttgttt
110761 gggaaataat atctccagtc ctctaaatgt aagctaattt atattatagg caaaaagcaa
110821 ttgaatggca gtttctcaaa gtcaagttaa tcacaactac ctaaatttag ccatgtgata
110881 ttttaccaac agcccattga tctcctgccc cactggctat tgcatgtaac tataaagcaa
110941 aggacagaac ccaggcttgt gcactctggt ttttctctta gtatttctgg gtttccctta
111001 gtgtcatagt taaccagagt gaagaagatt cagagcaatt catatttctg cttcctagta
111061 agttttcaaa aacatttcat ttaaagaaat actgtgaaat taacattttt gagtggggta
111121 tggttctttg aatttttgaa agatcatttt acttggaaat tattttagat atatgaaaga
111181 atggcaaagg tagtttagaa agttcccata taccctttac tcagctttcc ctaatgttaa
111241 catcttattt aactgtagta aatggattaa aactgagaaa tcaacagtaa tacaatacta
111301 ttaactaaac tgcagacttt atgtagattt ttaccagttt gtccactaat gtcctttttc
111361 catttcaggt tctcatccag gatatcatgt tttacttatt catttgtctt ttagtcttac
111421 aatcagtagt agcaccactt tcaaacttac atggatagag aaaggaggtt aagttaatgc
111481 tgtggtgaag tcatgagagt gtacagtaag tcctcactta atgtcatcga tagggtcttg
111541 gaagctgtga ctttaagtga aacatcatgt aaagaaacca gtttggccac aggctcattg
111601 atataaacaa caattaagtt cctactgcag atttctagtt acaaaagatc atcaaacttc
111661 tatacaaaca cttaaagcac ttcttttctt tttttttgag atggagtctc actctagggt
111721 ggagtgcaat ggtgcgatct tggcccactg caacctccgc ctcccgggtt caatcgattc
111781 tcctgcctca gtctcccgag tagctgggat tacaggcatg cgccaccaca gccggctaat
111841 tttgtatttt tagtagagat ggggtttcac catgttgggc aggctggtct cgaactcctg
111901 gcctctggtg atccaccctc ctcggcctcc caaagtgttg ggattacagg cgtgagccac
111961 tgtgccttgc tgttaaacgc gcttctaatc ttaaacatag aaataaatgt gagttataca
112021 cacatttaag aaagagcaat aggctgggcg tgatggctca cgcctgtaat ctgagcactt
112081 tgggaggccg agctgggcgg atcacgaggt caggagatcc agaccatcct ggccaacatg
112141 gtgaaacccc atctctacta aaaatacaaa aattagctgg gcgtggctgc gtgtgcctgt
112201 aatcccagct attcaggagg ctgaggcagg agaatcactt gaactcggga ggcagaggtt
112261 gcagtgagcc gagatcgtgc ctctgcactc cagcctggtg acagagctag actctgtctt
112321 aaaaaaataa aaaataaaaa taaaaaaatg aaaacaagat aattatccaa tttttggtga
112381 atcagtgagt gatagcagtc atcttggtga tgggttaaat caaggagtaa atgtttgcaa
112441 agtgaaaatt gtagagtcct ccctaccatc acgcgtttta aaaacaaata tagtgagctt
112501 gctgagtgct tttgtacctc atctttattg ttatgtattt gtatgattat tgtctacttg
112561 gtgaatttaa atttgacagt aatttgtatt aattccttcc ttccttcatt catttttcaa
112621 cttgcttgct ttagttcagg gttgaaggtg gctggagcgt atcctggcag gcagggcgca
112681 aggtgggaac caactctgga cagggctcca ttccactgca gggtgcacat acacatgcac
112741 acacgcattc tctcaatcag actgggacag ttaggcatgc cagtgaacct aatgtgcatg
112801 gctttgcaat gtaggaggaa acagaaacct ggaaaaactc actcagacat ggcgaaaaca
112861 tgcaaactct acaccagaca gtggtcccag tcaggaattg atttcttttt cttctcatca
112921 gtgttattaa ccaaatgaaa tgacattatt cacggacctg ctgtataggt ttaaggcaga
112981 tagactgttg ggactgggag acattctgat ccaggggcta cagacgggtg gccctgtggg
113041 gctcatatac tgttggaaat gtgatttgaa tttgccaaaa tttaatgact aaaaggtttt
113101 ccagaaaaat ttatgtttgt ggcttctctt taaaagattg gtagttccca ctgcctgagg
113161 cctgtgttta ccaccaggga tgatcacatg gccctgaggc ggcccttcac catgggcaag

113221 gatcccaccc tcccaatact gccctgatct aaaccagtcc cctcattttt tttttttttt
113281 tttttttgag atggagtctt gttctgtggc caggctggag tacagtggta caatttcggc
113341 tcactgcaac ctccaactcc ctggttcaag ctattctcct gcctcaggct cccgagtagc
113401 tgggattaca ggcacatgcc accacgccca gctaattttt gtaattttag tagagatggg
113461 gtttcaccat gttggccaag atggtctcga tctcctgacc tcgtgatccg cccgccttag
113521 cctcctaaag tgctgggatt acaggtgtga gccaccacgt ccagcctcca ccagccccct
113581 catttgataa atggaaatgc ctcgggggac cagcacagtg gcagaaagcc acctcgcctt
113641 gtctggcctt tgtctgctcc tctcggtcta agagagagct gggcaggcat ggttttggct
113701 tctttgggca gcaaagatga tagctgaaag aggatgcagt attaactccc ctccaggtct
113761 aggcctattc ttagtatttt tgcttgtgtc cctcaaatac tagagtttgc agactggtgc
113821 ccggggctgc atttgctttg tagtcccttc ctgtttacct gtatgtggtt atctaaagtc
113881 ttatagttac taatggtaaa aatcaagtga taagcctgaa aatccagatt tgtgacttat
113941 tttgataaat cagatgacct gcagcactgg ggccaccgtc tcaccaggca tgggctcctg
114001 ggctggggtc aaggctgcct cctcagatgg gcacaggctc atttggccct ttattctcgt
114061 ttttggggcc cgcctgggtt cctgtgggca tttgagtttg cagtgtttgc ctcaagttat
114121 taggttgaaa atcagagaag ccccagtagt ttcctgtttg tgtttcttga taatagttat
114181 tcttgacaag gagttattga agtaattcct ttaaagttct tgaggagaga gggaatttgg
114241 aacagggact tgtaatagaa tctgagatga ggtttgccaa aagtcagcca tctgattttt
114301 gtcttctgtg cccctactgg ctctccctgg atgtgctgtc agttcccttc atgtaaccta
114361 aggtttaatg atagacagat attagcttag agtattcccc cttttggggc atttttgttt
114421 aaacacaact tgaagcacag tatatttaaa gaattgagtg taagctacca tggtggaatt
114481 tcaggcattc attggatggt atccctgtaa tttcatccca gaagtgtgta agcatagaaa
114541 cgagagtaga gcactccctc tttggatcat tatagaaaca tggaagagat tattaaaggg
114601 gtgttgtatg tatgtgtaac tcttaagggt ctagatcttt gtggccctct atggtagctg
114661 ctagtcacat atggccaccg agcacttgaa atatgctagt ctacattgag atgtgctgtg
114721 agtataaaat acactctaga tttcaaagac ctactgtgaa acaaaggatg taaaatgtct
114781 cattaataat tttttatatt gattacatgc ttaaaggata atgttttgga tatatgaggt
114841 taaataaaat attattaaag gtactttcat ctttttaaaa tatgtggcta caagaaaatt
114901 taaacttcca cacacagcac atattctgtt tctattggac agagctgctc tagattctgc
114961 tgtatttgat ggtaagaaag ggtgagcagc ctgttgtcta gatcttggta aaccaaagta
115021 caaagaacaa atgtggagag actgaaaatc gtaaattgtt ttcacacctc ttgcctttaa
115081 taaagtttat tcttaactgc tcactttacg gtttttggtg catttagttg gatgcgaggt
115141 tgccttttca cttccttcat tgtattcatg gtgtggcagt gttacacaat ggttcagaac
115201 aagggacttg aagtcaaaca gttgctactt ggaattccag cccagctact tgaccttggg
115261 tcaattattt taacctccct gagcctcgtt gtcctcctct ataaataatg gacgatgatg
115321 atgcacccca gtgaatgacg attgtggaaa ataacatatg taaagtgccc agtgttatga
115381 ttattattcc tgacaatatg aaaaaaaaaa tcttattcag cacttctctg tgctaggagt
115441 taagtctgcc cccaaaatca ggaaacggct cctttttgct tctgtggtgt gatactgggt
115501 ctatcagaaa ccatttaaaa taaatagatt ttttttctat tcactagttt catggcatag
115561 gtttttattg tatcagtctc tttttgttcc tgatgttcaa tatttatcaa aacccagcaa
115621 ggactaaatg tttaagaaaa cgtttggcac cttgaattct cacagaagtt gaaaacatgt
115681 attggataca gtaaaaaaaa aaaaaaaagt attcatctat aggtgactac ataaatgacc
115741 ccagataaca ggaactggac aatggaggat gggagcagtc acaaaaacca tgccatgggg
115801 accatcaacc tggtcatctt gctctttctc tttgtggttt tcaggtactt gtaggatact
115861 tttttttttt ttttgaggtg aggctaaact tttattatgt gtcttccagc ttttattatg
115921 tgctgtggaa cttattaaac ttaatatatg actaatgtat ataactcagg ggaacgtctg
115981 tgtaagttaa ctactagaga aaccttgaca ggaaaaatat gaagatagtt ttgagtggac
116041 tttatcattc tcccttgatc ataaaagaat tgagacttgg aagctgggaa cttgggactc
116101 agaaggccca ctggtggcct gctaacatgt aagagtgcac agattggtaa atgtttttta
116161 aagcttattt attttctctg tgtctatgaa agacactacg ggtgtcattc tctgcttcac
116221 actcatgtct cttgtactaa atagaaggca agtcttatat ttcccacttc ctgctacttt
116281 gcttggcagt gacttttcaa ataattaaaa aaactgtctt gctggagtag ctctctctta
116341 cccatggaca gtcttgttat atccaaatca gtcagatagt atatgtaaga atgctttgtg
116401 agctaaagtc acttaataac tacagtctta ttactaacct gaccatggct gctggccatg
116461 aagctcactt gatttcatcc acaggttcca gtcaacccct cctgccacta cctgttgtag
116521 gtgtggatgg tgtttctgtg tgtcttctcc tgccgccctg cctggatctc agctgctatc
116581 ttttggccat cacaagcctg gctttaccca ccaactgtag gtcctggctg tcctgctcag
116641 gatatgacag ctgctctcta gtaagaattc tctgattcct gatagatgac ccctcttcgt
116701 aaattatgat tatcttaaat aatatgatat ctataattgt aataatagaa gctaacaact
116761 gattgcttac tgtgtgctag atgctcttgt aattgatttg ttctcttttg agtctcaaca
116821 atcctaggag agatgtgtta ttatccctat tattcagatg ataaaactga ggtgcagaca
116881 gggtaaatgc tgtcccaaag tctcagatta gaacccaggt ctatcagagt aggtcttgag
116941 gttttaacag tgcttgtttt ctgatctttg tttatgttgt tttcctgatc tcttgccatg

117001 tgtctaaata tttctagtct tcaaacacaa gttctacctc tgaagtgaca ttttcattaa
117061 ggactctagg ctgcatgtta tgtgctctta tccccattgc tctgagccag ctgaaggatg
117121 tcttgcttgt ctctatggct tagctagcct tttataatgt atttttttct gattgtttct
117181 tgtgcatcaa tcttgatttt ccaaatttta ttgttagtac ttctaagatg agggcacttt
117241 gtacgtttct cttagattct tcaacatact tagcatggta gtgagtaagc tcattaagaa
117301 aaatttatga actagacatt taagaaacag acatagagaa tttgctactg acagtgtcta
117361 ttggctgacc ctgtaccatt cactaccctt ttcattttag cttctggagg gattagtcta
117421 agagtgcttc tccgttagaa gaagttttgc cctgcagggg acatttggca gtgtctagag
117481 acagatattt tggattttca caactaggaa tggggtgcta ctggcatcta tggggtagag
117541 gccagggatt ctgataatgc acagataaaa atagataatc cacagataag aatattgtct
117601 ggttccaaat gttgatagtg ccaaggctga gaaactctgg actagaggta gcaatatgat
117661 atgttctggt caattaaata taagtggaag ggtctgctgt ggctttcttt tcttttttgg
117721 gacagtattt tgtctgtcac ctaggctagg gtgcagtgat gtgatcatag ctcactgcag
117781 cctgcagttc ctggactcaa gtgatcctcc tgcctcagct tccgagtagc tgggactata
117841 gttgcgtagc accacacttg actaattaaa tttttttttt tttttttttt tttttttttg
117901 tggagacagg gccttgctat attgcccaac atggtcttga actcctggcc tcaagtgatc
117961 ctccctcctt ggcttcccaa agtgctggga ttacaggagt gagccaccgt acttgccctg
118021 ctgtggcttt ctaggaaaat tatgcttcca tgagagagga ttgccccttc atgttttcag
118081 cttcttctgc tttcaacatg agcttgaacc tggaggtaga atatccatgc atcttgtgac
118141 caagagggaa caaacatgaa gataaaagtc agcatgctaa gggtggtgaa gtgaaaagat
118201 aaaaaatgtt tgagttttct tttacttgta accaaacaca ttcctgagct aatagaccac
118261 agaaacttga gttgggaagg aattaagccc agcctgacct catcgaagac ttttccaacg
118321 tttctggtga cataatccac tgctttggga gggagtctaa ttttgttgca gatctatatc
118381 cattccaaca gttctctctt acagtgagtg aaatttgacc cccaaaactt ctgcttatag
118441 acccagtgtg atgctgacct gagagtcacc tggacagtca ctcccacact ttcatagatg
118501 tcagtgaaaa gtcatcctga tcaagctggc gagtctactg acagagaccc tcttccaaat
118561 attctggagg gatgtacatc tagaatgtaa taccctctcc ttgggtagct ctttagagcc
118621 acagtccaaa ggtgaggcca ttaataaaat aaaaagatgt gacctctggg acccccaagc
118681 cctttggttt cctacatagg actttagagt caacataaat atactagaag cttctgatat
118741 tataaccaca tgaatgagcc aagaaggaac tctcagtggc ttgactgccc ttaatctttc
118801 ctctatgtct tgggctagac accaggaata ctactttcct cctgattacc tttattttca
118861 atccagttta caaataacta agtgcctcaa aacccctcag agtaaggaca ggtggctcat
118921 gtctgtaatc ccagcacttt gggaggctga ggctggagga tcgctttagg ccaggagttt
118981 aagactagcc tgggaaacat agggagactc tgtctttatg aaaaaagaaa aaaaattaat
119041 tgggtgtggt ggtgtgcacc tgctactgcg tagcagtcca gagtagttgc gtagtcccag
119101 ctactctgga ggttgaggcg ggaggatcac ttgaacccag caatttaagg ctgcagtgag
119161 ttatgatcgc accactgcaa cccagcctgg gtgacagagc aagaccctgt ttggaaaaag
119221 aaaaataaac aaccaaccaa ccaaagaaac ctctcagaat aaatgcctga gagctgtttc
119281 tgctaaatat ctttcaattt aaaatttttt aattctggta aaaagagaaa accacatgaa
119341 atttgtcatc ttaaccattt ttaattgtgc agttcagtaa agtatattga cattactgtg
119401 ccacagatct ctggaacttt tccgtcttgt ctgctgacta tcttttatgg cagtgtaggg
119461 tgagccatgt ctttacacag ttgttcattc actgcttgcc ctgctactta cactcctcct
119521 gcaaaatgcc cctagcttat agtacaagaa agaggtttct ttggctggac tttcctcctt
119581 ctaagacttc tttttttttt tttttttgga gacggagttt cgctctgtcg cccaggccgg
119641 actgcggact gcagtggcac aatctcggct cactgcaagc tccgcttccc gggttcacgc
119701 cattctcctg cctcagcctc ccgagtagct gggactacag gctcccgcca ctgcgcccgg
119761 ctaatttttt gtatttttag tagagacagg gtttcacctt gttagccagg atggtctcga
119821 tctcctgacc tcatgatcca cccgcctcgg cctcccaaag tgctgggatt acaggcgtga
119881 gccaccgcgc ccggtcctcc ttctaagact tctgtgtcag ccattcagtg agtttgctca
119941 agtcctctta cagtccttac catttcatta gttgaatgct taagtagaaa tgggtttaaa
120001 aaaccagaga tttggtttaa agagagggaa tatctgggag cagcaaggat tctaggtggg
120061 tagggtgtaa acctcaccaa gtaactcagc tgaaaatact ttctcaattt agtacaaggt
120121 cccagcacgg aaagatgtac aaggttattt ttcctggcgc ttctcccttc cgtatagccc
120181 atatgttagt gcccttagtt cttaatgccc tctggcagcc aagttcttat cttggggagc
120241 tacatcacag gtgttgggtg ctgtttaatt ttcagctaat tgagcttttt tgctgctaga
120301 gtcttgtatg gtgttttctt gaattcatga attagtcatt aatttgtgtt tagcaggcag
120361 gcaccctggc agtctgtggt ctgtccagca gaccttcttt tggactggac ggatgctctt
120421 tgtgcagttt ttagtgaatg atggtgggaa aaacagaatc ctcttccaga acccttaaac
120481 ggcagccttt tctttacttc cagctcttac cttgcaggtg ggtgtgggtc ttaacttcag
120541 aatggctact tttagtgggg ttaactattg ggtaggatgg gagactgtgt tctgaggctc
120601 agcagaagtc attgtgtttt tttttttttt ttggcactct gtaaggtttg aggaaattct
120661 gtgatttgaa tgaatttttt ctcaggagga tttacttcta aaataaacgt aattaattag
120721 ataactggaa tttcctacca cttagatggt tgttgtcagg ttctgtttcc ttgctacaat

120781 ttgaaagcac ttgaaatttc agattcatga atttatacca caaaaactca aaacagttga
120841 aaagtacata gatgctcatg cctgtaagcc cagcactttg ggaggccgag atgggcagat
120901 cacttgaggc cgggagttcg agaccaccct ggccaacatg gcaaaacctc atctctagta
120961 aaaatgcaaa aaaattagcc ggatgtgttg gtacatgctt gtaatctcag ctacttggaa
121021 ggttcaggca tgagaatcac ttgaacctgg gaagcagagg ttgcagtgaa ccaagattgt
121081 gccactgcac tctagcctag gggacagagt gaaattttgt gtcaaaaaga aaacaaaaat
121141 aaaaaaacac atttttttga gtgttatgga gttatgattc caattcttta attttaataa
121201 gccttgaaaa gctgcagttt actccatcat gagtagggat aagcttctca gtataaacaa
121261 cctgtagttc tgtggttaat gaaataagaa gcttgaacta ctttcttaag ttaaaagtgc
121321 cttttcagca agtagatgct caatgaagtt agcacaccta tttcaacttt aatcaataag
121381 gtggaaataa tgcagtatgg caggtacatt actttcagtt gcattttcta ggtgtgatta
121441 tctcagagtt tgaaatcgta cattttatga ttttatgtta gtgcaggatg aatcttttca
121501 gttataactg agctggctgt gagcatttag cctgacaggt tgggctgctt ttcttactgg
121561 ttttgtccgt ataaggagta ccacagaagt acctcacacg cattttaaaa taacttttcg
121621 gtaataaaaa ttaatattgg ttaagaataa acttagctta tctaatgcgt gggccaacca
121681 tttctgagca aaattgtgag cagagtttgt cttgtgtgtt ggctgtggtc ttaaaaacac
121741 tagcagacag tgttatgttg actgtaatat ttcttctgta gagtaaaaga tagatttcat
121801 ttctgggtga agcattgtaa ttggcttata cttttgtttc tggtcatatt atgtagacag
121861 tagaaaagaa gtgggaatat aaaggtagga gaggtattac ttgaatgtga cgcataaaaa
121921 gtatataagt agacagaaaa gaagtgggaa tatacaggta ggagaagcat tacttttatg
121981 tgatgcataa aaaatatata gagttttcta ttgttttggg ggtggggtta gaaaacatgc
122041 tatagataaa gaagagtcaa aattagaaaa ttaagtaaga tgtttcctgt tatacttaaa
122101 cattgcaata cttacaaagc atatcataag gtatatataa agtggtcatt atgaactttc
122161 tttacccagg tcaagcaacg cctctaaggg ggaaaaataa tttggtgaat ccccatactc
122221 tatgataggg catatagagc cattttatta taaagaagtg atatattgct gaagtgagta
122281 ctaagtagca agcagtggaa atgtgtacca tgcatttctg agaaataatg cctgggaatg
122341 cactgcaaaa caggtgtact tggtgtatat gcaatttaag taacataaca acttgagaag
122401 gattcaaaga tgggaatagc tatcatgtga atgaaggcat tgcactgtag aaatatctta
122461 ctataacaat tttattatat gatcattgtg gccaagaata ctgatcattt agcttagttt
122521 tagaagctaa atatgaagta tcatcattct atgtacaatg tattctgtgg tggttgtgca
122581 tttattattg ggatcacagc gtgtcttttt tctgccaaga tggacactgg gcaccatggt
122641 cacaggcttc ttgggtgcta ctaatagaag gctctctctt ggctttctct gttgggcaga
122701 agtctgtgaa tgccattata gctaagactc tcccattctg gccaggaaag gaagctggaa
122761 ggacttggac tcactgattt gtgctgacac ccattcagcc ctgccgttgg aactctttaa
122821 tgtgcttttc agtgagctag tgtttcagaa cctcatgaca gggcaggctg atttctcaga
122881 tgcatacttc tctgacctct ggcttcccct agctcactga ggctcgttct gcctgttgat
122941 gaggctctcc tggcagattt gacactggac ccaagagggc agaagaatca gctgttaagc
123001 tggtacaggg ctcaaagata aataagaaga cactcagcag agcgtaactt tgaggcaggc
123061 acacaaccac ttgggtcagt gtagtatttg gggcatgtgg gtttgtgttt ggaatttgag
123121 aggaagcact ctgatgatcc agcagccttt gtgctccagt gacatgctaa tttttttttc
123181 ctgatattgg gattgcgttc ccttgactat tgcagcagag aaactgaaat gggaatcatt
123241 tcctgtgggt gcactgatgt gcacagtggg cggaaccggg aaggcaagtc agaggcattt
123301 ttctcatttc tgaatccatg agtctggctt ttcccttagg gtatttgggc caattgttat
123361 agtgtgtccc actgtgtaaa ttgaaggttt agccaagtct ggctgaatgg tttagtcttg
123421 caaaaactaa agcactttgt ttttaaaaaa ggaaaaaaaa gtttaacttt ttttattttt
123481 aacatggcat tattggacta aatgatgtta ggcatgttaa cagagtaaaa gggagaactt
123541 ctttctgcag tttggcaggc ctgtcacttc acaggtgtat cactgtccaa atgagggatg
123601 ccagataaga catcattcag cctcccggag ggtgggggga gcaggtgtgg cacaatacag
123661 tcattgttca ttgtcttccc gcttacagta ttgtaaaacc tgcactgcat taaaaatcct
123721 attaatagaa cattggatta ggaagaatac ttaggcttct ccctccttcc atcagtttta
123781 aaaggaattt agaaaaatca ctatttcttg tatgtatgag tgggggaggg aaagaaggaa
123841 gaaaagagag actgactttg tctttgaaat acagaagaga acatcttgca gtgaggaatg
123901 agaatctaaa aagataacca ggcttcttgt aagaaaactt tcatagacag ttattcagca
123961 ggcagatttt taattttcat gtttccagct tgtttagtct gtggggactt tgatttctgg
124021 aagcccactg ggatcgctgc taaaccttac atactttttt tgttgtatca aattcagttc
124081 aactaccaga aaattttttt tggcaattat ttttttctct gtcatgtttt ctgtccaatg
124141 agaggagaag actgggatag ccaaggcaaa tgctgctcaa attattgtgc ccaagaggag
124201 agatttggcc ctgtgatact gcagggggac tgttgagaaa gagtaacagg gacatattgg
124261 gaagtgtgca gcctgggctt cccaactgat gtcttagcta atttgaaatg ttgtgattag
124321 accttccctt attcacgttg aggaatgaat atgctgacag ccatatgcat gtgattttag
124381 gattattaag tgtaatggaa tgtccaaacc atcactcagt tttacatagc acccttcaag
124441 aactagaatg cctgggaaac atggctgtcc cagttaagtt gaggttttat ctgaaaccca
124501 ttttgttttc aaacatgttg gaaatgttcc taaaaatgcc tgagtgtgct ttgggggtgt

124561 gcttggtgag tatgctttcc tacccttgag tgtttgagac ttgttttcat tatgttaggg
124621 gcttatgata aagtcttcat tttcagatct ttatgtagaa gatatatata attgagcaga
124681 ttttgaagtt tcgctcaaat cttgtttgta tttaagaata ctttttctta ttcattattc
124741 tgattaattt ttcttttaaa actggcaaca ttttgcaggt agtttatggt tttgcaaatt
124801 agaatatggg tcattcctct aaatagaaac agtgtttttg gtcttgatgg gtctgtgaat
124861 taggtgaaac tagttttatc tttctccttc tacttctagg ggaaagtctt tacctttccc
124921 cccctgcaca ctcctcccac cccacccttc caagcttgat ctcagcaaat tcattgactt
124981 ccaaaattca tttatatgac tttaatctta agtcatcttt atgaaaatgg cttatcttta
125041 acttactacc tgtttcagga atagttgcag gcagagcttt tttcttattt ttcttggtat
125101 gaaaggcttt gtatcctgct aaaggaattt caggatttat agttataagt agctatattt
125161 ttagtcatga gctttgctat ggtttgaaag tgtccgccaa atttcatgtg ttggaaactt
125221 aatccccaaa tccatatgtt ggttgcattt ggaggtgggg cctgtgggat gtaataaggg
125281 ttaaataagg tcatcagggg ggttcccatt gatgttacag gtggctttat aagaagagag
125341 acctgagcta gcatgctctt gctctctcac cgtatgatgc tctctactat gttatgacat
125401 agcgggaagg ccctcgccag atgcagactc ttgagtttga acttttcagc ctccagaact
125461 gtaagaaata catttctttt tcaggaccca taagacagag caaaaaataa aaagaaaaaa
125521 agaaattaaa aaaagaaaga aaaaatttta aaaaattctt ttgaaaaata aattacacag
125581 tctgtgatac tgtgttatag caacagaaaa tgaactaagg cgggttccca gaacctttat
125641 aatcacatca gcttgttaac tgctgttcca cttgtaagca gctccccctc aggccagata
125701 ttactggcat aagtggaaaa cctaggcacc tgaaagtttc tgacagcctt tcagaaagtt
125761 ccagaaagga gtgcctttgt tttcctcact aagtgagaga ttaaaaaaga aaatcagtaa
125821 aagtcacttc ccctaatgcc tacagttgtc atcctggaat cacactttta ttgaatccaa
125881 ttcagcaaac atttatcgaa gaccagctat gtatagttaa aaccactaca cgggccgggc
125941 gtggtggctc atgcctgtaa tcccagtact ttgggaggcc gaggtgggtg gatcacctga
126001 ggtccggagt ttgagaccag tctggacaac atggtaaaac cctgtctcta ctaaaattac
126061 aaaattatct gggcatggtg gcacatgcct gtaatcccag ctaattggga ggctgaggca
126121 ggggaatcac ttgaacccag gaggtggagg ttgaggtgag ctgagatcgt gctattgcac
126181 tccagcctgg gaaacagaga ctccgtctca aaaacaaaac aaaacaaaac aaaaaaacca
126241 ctacacgagc tgtagaaaag cccatttgtt tctcttctaa agtatataat ggggctgggc
126301 acagtggctc acacctgtaa tttccacact ttgggaggcc aaggcaggca catcacctga
126361 ggtcaggagt ttgaaaccag cctggccaac atggtgaaac ctcgtctcta ctaaaaatag
126421 aaaaattagc tgggcatgat ggcacatgcc tgtaatccca gctactctgg gggctgaagc
126481 atgagaattg tctgaacctg ggaggcggag gttgcagtga gccgagattg caccactgca
126541 ccccagcctg ggtgacagag tgagactctg tctccaaaac aaagaaagaa aagaaagaaa
126601 gaaagcacac agtgggtgaa gaaagtactc actactcagg tagtgagtac ctgaccaatg
126661 tagaatgact tttaaaattc cagacttggg agcatttctc aggatgccag taggtattaa
126721 aaattatttt tgaccaagta aatttggaga aagatgggtt aagtgaagtt ggatagggat
126781 tttctttttt atcggtgctt ccaaaggggg atggtatatg cagcatttcc caaatttatt
126841 tgaccaggga ccatgtattt atttatttag gagcagctca gaggactagt gttccatgga
126901 gaacactgtg acaaaggctg ttctacacca cggaagattt ttttcctctc tctcttttag
126961 agggctcttt tattttggtg tatacatgtt tattattcag agccagcaaa ctctgtaact
127021 ttctccattg gttatataaa aagtgttagc attttccttg gatatttgtg aggttttgaa
127081 gggccgggat aatgaccgtt gagtgagctc ttacttggtg ccagctacat tgctgagcat
127141 ttcctctaga gtctcattag tgggaagtac aaattagcag ttctgctttt taggtgagag
127201 gataacttga ccaatatgca atttatgcat catttccaac atgccagaca ttttaaggtg
127261 aagagatttt aaaaatcatg tatagtttta tatatagatt catatatagt tgtaagaagt
127321 tatacagagt gattctgtgt ccctttcacc cagtttctcc caatggtaac gtgttgccta
127381 acagtattac agtatcacaa tcaggcagtt aacactgatt cagtgatcaa cactttaaca
127441 ctttttagat ttttttcttc ctttctctct tctcttctct ctcgtttctt tctttttttg
127501 agacagggtc tcgctctgtt gcccaggctg gactacagta gcaatatcat ggttcagcac
127561 agccttgacc tcctgggttc aagcgatcct ccctcctcag cctcccatgt agctagctat
127621 gaccacaggc atgcactacc atacccataa tttaaaaaaa aatgtgtaga gatggggtct
127681 cactatgttg cctcggctgg tcttgaactc ctgggctcaa gtgaccctcc tgcctggcct
127741 cccagagtgt tgggattaca gacatgagcc accatgccct gccatttttt agatttcata
127801 ggttttacac acattctttt tgtgtgtgtg tacatttagt tctatgcagt ttttcacatg
127861 tgaaaatttg tgtgaccacc accgcagtta agatacagaa cagttccatc acaaggatcc
127921 caaatggtat gcctttataa ccatagccaa ctctctcctt ctttataatt ttgtcatttt
127981 aagtagtctc tataaatgga actatacaac atgtaacctt ttgagattgg ctttttaaca
128041 tcctagctgc ttttaatcca ctgttgccaa tttctatagc aatcctatat ggattctttt
128101 ttctttttct tctttttttt gagacagagt ttctgtcttg tcacccagac tggagtgcag
128161 tggcgcgatc ttggctcact gcgacctccg tctcccgggt tcaaatgatt ctcctgcctc
128221 agcttcctga gtagctggtg ttacaagtgc ccgccaccac gcccggctaa ttttttgtat
128281 ttttagtaga gatggggttt cgccatgttg gccaggcttg tcttgaactc ctgagctcag

128341 gtgatccatc cgcctcggcc tcccaaagtg ctggggtgaa ttcctttaag tagatgagga
128401 agtgaaaccc cggaagagta cagtgtcatg cccaaagcta tattattagt ggaccaggaa
128461 gcgaggaggg gcccaagcat ccctgattct cagccttgac tcctttcact cagcgttggc
128521 ctttctctat ctggattccc agtgttcata ttctgtttct accaacacag ttctcctttt
128581 ctcttcctct gtactcaaaa taaagtctct cttgtttttt ataaatctta aattttaaaa
128641 aagaagcttg gatcacatta ttattttgga agtgcagaga ttggatattt tttccgtcca
128701 gctctatttt ggtcttgtta ccattttcag tgttttctgt gctgagttgc ttcaccacct
128761 tgatgctgcc acaaatgatg aggatggttg tagtatagag aataaaggtg gagattcttg
128821 cacatcattt cttgaatcca tgtataggca tctttggctt gcagcccatc tgagtgctgt
128881 attcagccta ctagctctgg ttgctctggt gctaaacata ttcccagcgc ctttgggact
128941 taactcacta caggcaagaa gctgtcagtg cactgaggga tggtgggaaa gcaaaatgca
129001 tacatttcta tttatcgcag tagtgccaga tgctgtatat ttaagaaaaa aacttccagt
129061 attggagaag aattgccgtc cgcccccctc actttattta tttatttttt taaacagggt
129121 ctgggtctgt cacccagact ggagtgtttt gttgcaatct cagcccactg caaccttcac
129181 ttcccgggct caagtgattc tcctgcctca acctcctgag tagctgggac tataggcatg
129241 tgccactgta cctggctcat ttcttttttt agtagagatg gggttttgcc atgttgccta
129301 ggatggcctc gaactcttga gttcaagcga tccgcctgtt ttggcattcc aaaatgctag
129361 gattacaggt gtacaagtgt gagccaccat gcctgaccat aagccttttg aaagctgtac
129421 ttttgaaatg tatgtgggat gtagtattta agaagattag gaaacagcaa aatgtgtaga
129481 tttttgttga tcattgaacc gcctagacat tctcttcttt cagtgtccat ttacgtatgt
129541 atgtattatg tatttagaga cagtgtcttg ctctgttgct caggctggag tacagtggca
129601 ctatcacagc tccctggaac ctcgaactgg gttcacaacc tcccatttca gcctcccaaa
129661 tagctaggat tacaagtgtg tactaccatg cctggctaat taaaaaaaaa aaaaattgta
129721 gagatgtgct ctcactgtgt tgcccaggct gatcttaaac tcctagcctc aagcagtcct
129781 tccatcttgg cctctcaaag tgctgggatt acaggcatga gccaccatac atggcctttc
129841 agtgtctttg aactagctat tccctctgcc tggaaaactt tcctaagatt tccatgcagt
129901 ttggggcact tcctcacctt cttcaaatct ctgtacaatg ttaccttttt tctgtgtctg
129961 tgcctgacat ttaaaattgt aacacctcac cctcacccca ttctgcttta cttttctcca
130021 tcacactttg atcagctttg aacatgcccc tatgtcttcg ttatgtatct atgcattgtt
130081 ttctctttcc tagaatgtaa acctcctgaa ggcagggatt tttgcctatt ttgctcactg
130141 ctgtatatcc aaccttttag aatagtgcct ggcacatact agatctcatt atacattata
130201 caagtaaatg aaagttttca agattaaaga tttgtatgaa ttaagttaag taacagcacc
130261 ctcttacagg acacaagaga ataatcatgt ctgtacaggt tggcccctgg taagatgctc
130321 atttaaaaat ggttgtgtca gtttaatata agtgtgttct tttcttgcct acttaaaatg
130381 tatttctaac tatgactttt cttttggaaa atgattgact ttgggggagc tctttcagag
130441 gtaaaggaag ttaactagca gatctaggta actgaacctt gtgtactatt ctagaaatgt
130501 ttatacttct gccaaattga agcatgagtt cagtgtttgc ataaccacct gtgcagttac
130561 tcatagtagc attccagtat gcatttgctg aactatactc ttcaattttg caataaaagt
130621 attttcacat gttggctgct actgtgaaaa ttggtttctc tcaaatgttc ggaaagcttg
130681 tgctgcagtt ttgatgtggg gggtgtggtt tagtgctgca taacatttac ttttttggtg
130741 gaagggggat ctgtgatgta aacttttggt ttcaatttct ctatttggag ctctttttgt
130801 tagtgctaag aattctgagg gtatccctat agatgctggt taaaaaacaa acaaagcaat
130861 gttgtcattg acagtttggt caagtgggat gtaagctgct gagtggggtg aagacctggt
130921 ctccttgggg ctgatttggg gtttgggagt ggtttggagg gtggctgcca ttgaggagca
130981 ggaagtgtgg gggttgctgc tgtgtctgga aggacctcct aggggagagc tagcaaacca
131041 acatcaccag tgcccccaga acactcagct gtgtgtgatg tggctgtgtg tgcccttgct
131101 ggtactcagt cctgttaagg cttgaagagg aaggtaagga gggtaagact tggagaggga
131161 agagttaagg agggagaata ttaatgcagt aaggaagtaa ttcatatttt gagacaggtt
131221 cccatgtgtg aacacttgac ttccaaacag cccacacttg gcttcattat ctacagacca
131281 cctctcctgt gctgcattca gactctcccc caaaccccga cggagcctcg gtctgtcgcc
131341 taggctggag tgcagtggca tgatctcggc tcactgcagc ttctgcctcc caggttcaaa
131401 tgattttcct gcctcagtct cccgaatagc tgggatttac aggcacatgc caccataccc
131461 ggctgatttt tgcattttta gtagaaatga ggtttcccca tgttggccag gctggtcaca
131521 aactcctgac ctcaagtgat ctgcctgcct tgacctccca aagtgttggg attacaggcg
131581 tgagccaccg cacccggctg cattcagacc tttccacgtc agctgaacta tcagtaaaat
131641 tctgagcaca actctgagag aaattcagag ttcaaatcag ttggtatgcc agaagcagag
131701 aaccctgatc cttactggag ttgactagga caccttctct ttattagtta aaggtatggc
131761 taaatttaaa taactaatag aggtcagaca cgacttactt agtttctgag agtagttgtt
131821 aactgtcctt ttcccaaggg aaataagcta agctgaatta cttaaaccac actttttccg
131881 tttcttaaaa tgatttccat ttccctgccc taggaaaagt aggcggctgc agagaggttg
131941 tgacagaatt ataaagctat ttgagttaaa ttagcccagt acatcactct tagagcacat
132001 tgaacagaca gctaacacct cctgataagc accagctaca tacagatcaa tgttttcaaa
132061 tgaaaccaaa gacatacatt caatttaggg agacaaaaca gacattactc aggaatctca

132121 tagaacccca gatgattatg atataaccaa taacataaga cccacctgat tggcagttct
132181 gtgaatcacc ctaaaatctc tactaaaatg gttaagtcct gtactcttta ctctggagtc
132241 ctcttgtcta cctttcagaa atggttgaaa tatttagatg tggaattaat gagattttac
132301 atgtctacac tcttgcttta tatattttaa tgtgataaat ggcctcatgt atgtatattc
132361 ttccttgtcc cagactcatg ttttttgagt aaaatcttta attggtcaaa atgtgttgta
132421 ttgatcagta tgttatatag aatgatgtac ttattgattt actgtaaata attacagtgg
132481 agcaattctg ttaggagggt tcgtttttgc cattttagca aggaaaattt gccagttatt
132541 ttggctgaat ttaaaacccc ttacatgatt agatcacatg tctatttctg ctgtaataga
132601 ggagtgtaag taactaagtt tatctgaact tttaggagtc tccttttagg ctcaaattag
132661 ttagtctggt gtctatacag ggcctttgtt aaagactgga tttaaatatt gggtggatct
132721 gtcttggatc ctgttctagg gaacgcttct tggaagtccc agacggggaa ctcatcataa
132781 cctggtttta ccccgtagca tggccctgtg ccatcgacac cattagagca aggctcagtg
132841 tctcgagctt tagtttaatt ctcaacccat tgatcgctct tgcagtcagc ttaacagttg
132901 aatgcaatgc tgtattttgt tttttttttt tttttttaaa gctagtccag aaattaccac
132961 agctgcttct cagttaatca gatgcttaag actcccgttt ccacattgct tggctgctac
133021 atttttataa caccacagct gtctggatct agcttgtgcc cctcgccccc cttacccctt
133081 tccatctgcc acctctcacc acttcctcac ttgctatgta tcgaggtggc aaattcccaa
133141 gtaaactgct gactttcctc ccctgcagga aggctgggcc tttggttcac agtagcctct
133201 ttcaccagaa ctctcgtagg ctaggaacca accctggagt actgctgctc tcagaagtgc
133261 ccacatttgc ctctggagct tgctttgtgt gtttgggtgt tgaattttcc ccttgacttg
133321 gaaacttttg tgaagaaact gaaaggtgct ttcagattaa gcagctgtgg ctcccccttc
133381 cagccacgct ctttgttttt aaagctcaaa gtcttatttg aagtgaggag agaaggtagg
133441 taggttgcag tccaggtttt tctgtttgtg aatgacgttg cttaccttgg ctgattcctt
133501 ttcctaatga cggtgcttct actcttggcc ttttgccatg tgtgtaccat ccactgggag
133561 ctgttttccc ttgtccttta aagcgtatcc gactctatct ccagtgctct tctagcaagc
133621 tgttacagta agcataaaaa caagcgcatt aaactaggaa taaaatgcca agtttctgtc
133681 tccttgccaa cctcattaga aaacagtgat aaagtatgta tgcatataat tacagtaatt
133741 aaacatttga aaccagctca atagggaacc ttcttaatgc ttttatgagt gtgatatttt
133801 catttcagaa ctggactctg tgtggcttct attttattca agaccttcat ctttatgggg
133861 gtaggcgggg gaaggtgctc cttttgacca cagcgccaag catctgcaag agtagcttta
133921 ttacacatgt attagcaggg aacagagcaa cctttccatc actgtgttta tttttgaggg
133981 tgtgtagcca gttgtcaaat ataaagacat gaatggcctg gctagtaaag aagacgtgga
134041 aaataattgt aaactgtctg ccatgatgtg cttgctctta agagcaaagg acaggaatat
134101 tttgtctttt gtactgcccc gacggagtca tacagcccct gcatttgtgt gggatgatag
134161 agtagcacta tggggtttgt ctgcctcgta gctcttccac cctgagagct attctcactt
134221 aggaagtgga aagtctaaag gaccaactgt aggttgaacc aggagtttca ttcactaaac
134281 atattttttt tttagttgat atatatatat gtaaaatttc aatagctttt gaggtacaaa
134341 tggtttttgg ttacacggac aaattgtata gtggtgaagt ctgcgatttt agtgcaccca
134401 tcatctgtgt atattgtacc caatatgtag ctttttttta tccctcatct ctctcctccc
134461 ctcccctctt ctgagtcacc aaagttcgct aaatgactct gtatgctttt gtgtacccat
134521 agtttagctc ccacttataa gtgagaacat acggtatttg gttttccatt cctgagttac
134581 ttcacgtaga ataatggcct ccagctccat ccaaattgct gtgaaagata tttcattctg
134641 ttttatgtct gagtagtatt ccatagtgta tatagaccac atttctttta tccattcatc
134701 agttgatggg cacttaggtt ggttctgtat ctttgcaatt gtgaatggtg ctgtgatgtg
134761 tgcacgtgtc tttttgatag aattacctct tttcctttta gtagttgccc agtagtgaga
134821 ttgctggatg gaatggtatc tttactttca gttctttaag gaatcttcat gctgtttcc
134881 atagaggttg tactaattta cattcccacc agcagtgtat aagtaagtgt tctcttttta
134941 ccacatccat gccaacatct attgtatttt acctttttag taatggccat tctggctggg
135001 gtaaggtggt atcttgttgt ggttttaatt tgcatttccc tgatgatcag tgatgttgaa
135061 cattgttttg tatatttgtt ggccatttgt atatcttctt ttgagaaatg tctattcatg
135121 tcatttgccc actttttgat gggattattt ggtttgtttt cttgctaatt tatttgagtt
135181 acttgtagat tgtgggtatt agttctttgt aggatgcata gtttgcaaat attttctcct
135241 actctgtggg tcatctgttt actctgatgg tgatttgctg tgcagaagct tttagttta
135301 attaggttct atttatttat ttttgttttt cttgcatttg cttttggggt cttagtcata
135361 aattctttac ctagaccaat gtccagaagt ttttcctagg ttgtctactg taattggttt
135421 cagctcttag atgtaagtct ttgatccatc ttgagttgat ttttgtatat ggtgagagat
135481 agggatccag tttcattctt atacatgtgg ctatccagtt ttttcagcac catttgttga
135541 atatggtgtc ctttctccaa tttatgtttt tgtatacttt gtcaaagatc aattgtggtt
135601 gtaagtattt ggttttatta ctgtgttctc tgttctgtta cattggtcta tgtatctact
135661 tttataccta taccgtgctg ttttggtaac tataaccttg tagtataatt tgaagttagg
135721 taatgtgatg ccttcatgtt tgttcttttt gcttaggatt gttttggcta ttcaggttct
135781 ttttggttcc atatgaattt ttttttctaa ttctgtgaaa aatgatgtta gtattttgat
135841 agaagttgca ttgaatctct agattgcttt gggtagtatg gccattttca tgaaattgat

135901 tcttccaatt catgaggatg ggatattttt tccatttgtt tgtattatct ttgatttcct
135961 tcagcagtgt tttgtcgttc ttgaagagat ttttcacctc cttgttaagt atatttctag
136021 gtaatttatt ttatttttgc tgttattata aaagggattg agttcttgat ttgattctga
136081 atttgattgt tgttggtgta tggcagtact actgatttgt gtacattgat tttgtaacct
136141 gagacttcac tgagttcatt tatcaaatct aggagtcttt tggaggagtc tttagggttt
136201 tctaggtata taatcatacc actggcaaac agagatagtg taatttactc ttttccaatt
136261 tggataccct ttatttttct ctcttgcctg attgctctga ctagggctcc tggtgctatg
136321 ttgaatagaa gtagtgaaag tgggcatcct tgtcttgttc cagttctgaa gggcaatggt
136381 ttcaactttt ccccttcagt atgatgttgg ctgtgagtct gtcatacatg gcttttatta
136441 ttttgaacta tattccttct atccctagtt tgttgagaat ttttatcata aagggatgct
136501 ggattttatc gaatgctttt tctgcatcta ttaagatgat caatagattt ttgtgttttt
136561 aaaaaattct gtttatatga tgtttcacat ttattgactt gcatttgtta aatcatccct
136621 acatcctgga tgaaacccac ttgatcatgg tgaattatct ttttgatgtg ctgttggatt
136681 cagtttgcta gtattttgtt gaggattttt acatctttgt tgattcaagg gtattgttct
136741 gtagttttct ttgtcttttt tttttctgtt atatcctttc ctggttttgg tatcagggtg
136801 atattggtgt catagaataa gttatagagg atgccctctt tctcaacctt ttggaatagt
136861 ttcagcagat tggtaccaat ttttctttga atgtctggta caattcagct gtgaatctat
136921 ctggccttcg gctttttttt tttttttttg gtctgttcag gatttctatt tcttcctgat
136981 tcaagctagg aagattgtat gtttccagga atttgtccat ttcctgtaga ttttctagtt
137041 tgtttgcatg gaggtgttca tagcagtctt gaatgatctt ttgtatttct tttggttata
137101 atgtctcaat tttcatctct ctctctctct tttttttttt tttgacagag tctcgcttta
137161 tcaccaggct gaagtgcagt ggtgcgatca tggctcactg caacctccac ctccctggtt
137221 caagcgattc tcctgcctca gccttccgag tagctgggat tacaggcatg cgccactatg
137281 cctggctaat ttttttgtat ttttagtaga cgtggggttt caccatgttg gccagaatgg
137341 tctcgatctc ctgacctcgt gatccgtctg cctcagcctc ccaaatgctg ggattacagg
137401 cctgagccac tgtgcccggc ctccattttc atttctaatt gagcttactt gaatcttttc
137461 ctggttaatc tagctaatca tctatcgatt ttgtttatct tttcaaaaaa ccaagctttc
137521 gtttccttga tgtatttttt gtttgtttgt ttcagtttca tttagttctg ctctaatgaa
137581 aaggtctttg ttatttcttt cattctgcta gctttgggtt tggtttgttc ttgtttctct
137641 agctccttga ggtatgatgt tagattgtaa gtttgtgatc tttcatactt tttgatgtag
137701 gcatttagtg ctataaactt aactcatagc actgctcttg ctgtatccca gaggttttga
137761 tgatgtgtat cactattatt cattttgaaa tatttcttaa tatccatctt gatttcattg
137821 ttcacctaaa agtcatacag gagtaggtta atttctatgt atttgtatag ttttgggaat
137881 tccttttgga gatgatttct agttttattc cactgtggtc tgagaagata cttggtatga
137941 ttttgatttt ttaaaattta tcaaggcttg ttttgtggcc tatcatatga tgtgtcttgg
138001 agaatggtcc atgttctgat gagaagaacg tatattttgc agttcttggg tgggatattc
138061 tgtgactata ttaggtccat ttgttctaga gtatagttta aggttgatgt ttcttagttg
138121 actttcttcc ttgatgatct gtgtggtgct gttagaggag tattgaagtc tgcactatta
138181 ttgtgttgct atttcttttc ttaggtcgtg tagtaattgt tttatgaatc tggagttcaa
138241 gacttaggtg catgtatatc taggattgta atatcatctt gttcgattga tccttttatt
138301 attatataat ggcatttttt gtcttttttt actgttgttg ttttaaagtc tgttttatct
138361 gatgtaggca tagcttttcc tgctcacttt tggttctcat ttacatggaa tatctttttc
138421 caccacttta cctggagtct ataagaatcc tttcatgtta ggtgattctc ttgaagacag
138481 cagatattcg atttgtaact ttttatccat tctgccaaag tatatctttt aaatggagta
138541 tttaatctca agatgcaagg tactgttcag ttatgttgat tgttacatag gtacttagtt
138601 ttcttcattg tattattgtt gataagtcct ataagtttta tgctttctag aggttctatt
138661 ctggtgcata tcaacctttt gattcaagat taataactcc ttttagtatt tcttgtaggg
138721 ttggtctggt agtgacacat tccctcagca tttgcttctc tgaaaaagac tccttcattt
138781 gtaacactta gtcttgctgg ataaaaaatg attggctgac agttattctg tttgaagagg
138841 ctaaagatag gactccaatc ccttctggct tgtaaggttt ctgcggagaa gtctgctgtt
138901 agtctggtat gttttcccct gtaggttacc tgatactttt gtctcactac tcttagaatt
138961 ctttccttca tgtttacttt agatagcctg atgactgtat gccttgatgg tgtccttttt
139021 gcagtgaatc tcccaggaat tatttatgct tcttgcattt ggttacctaa atctccagca
139081 aggccaggga aatttttctc acctattttc tcaaataagt ttcccaaatt ttttgctttc
139141 tcttctcctt cagtaacacc attgattcta aggcttgact gttttacata atctcatatt
139201 tcttggagac tttgttcatt tcttttaatt cttctttatt tttgtctggg ttaatttgaa
139261 aggcttatct tcaagctctg aaattctttt ttctacttgt tctagtctag cgttaaaact
139321 ttctactata ttttgcaatt tcctaagtgt atctttaatt tccagaagtt cggattggtt
139381 tttcttcaga atatctgtct ctttagaaaa ttttttgttc atactgtgaa ttgtttttc
139441 aatttcttta tgctgatttt tacctctgtc ttgtatttcc ttgaacagct ttaatagtcc
139501 accttttgaa ttctttatct ggtatttcaa agatttcatc ttggtttgga tccattgcag
139561 aagagctagt gtgatctttt ggggttgtta tagaaccccg ttttgtcata ttgccagaat
139621 tattattatt attttttgag atggagtttt gttcttgtca cccaggctgg agtgcaatgg

139681 cacgatcttg gctcactgca acctctgcct cctgggttca agggattctg ctgcctcagc
139741 cttgcgagta gctgggctta cggtgcccac caccatgccc agctaatttt tgtattttta
139801 atagagatgg ggtttcacca tgttggccag gctgatctcg aacttcctac ctcaggtgat
139861 ctgtctgcct cagcctccca aagtgctggg attataggca tgagccaccg tatctggctg
139921 ccagaattat ttttctagtt ccctctaatt tgggtaggct attctaatta ttcttgaatt
139981 tatttttgat ttgacttaaa aatttttttt ctcccttgag gatgtgactt tatcgtttat
140041 tgtaggctaa ttcagctctt ggtgctttca gggatgaaga ctgtgtatga cttccttggt
140101 tatagagagt ctttgtatga cagctttctt agatgctggt tgtagtagca tagtgcttgg
140161 tttgtgagca ggttcattgt ctcctgtggg attggaattg cgaggtctct taaagcttat
140221 ctcattgcac tttttaatta attaattttt ttctgcagta ttttatttac tgagttgaac
140281 agcttaggct tcaggccagt aggagagatg tccatgggta aaaatcagtt gtggctaaag
140341 caggttggta aatgcaatac tcagtggtgg gcagaggtcc cagccttgat agaagcagct
140401 ggggcagctc tcagtgaaat gcactgaggt cttttcaagg gaaagggaag aagccacctc
140461 agcttccctg ctaggccagc aggaaagtga tccaccttcc aattacactc ctgacccaga
140521 gttccagcta ttctgatcag gcacctcttt tcatctgcag gaatgttgat gttcaacata
140581 gaaagggatt gtgactgtac ccctcatcca agcttgaacc taggggacat gcattctctg
140641 tggatgcagt taccctgaag tattccagaa aggctgtcta caggtgcatc catgctgagc
140701 tcccatgggg gaagcctcag ctatgtcgat agtggatgaa gaggagaaga aatcaccttc
140761 tccaagaccc ttcatgggca ccagggctga ctggctgttg gggtagagcc acagacgttt
140821 tctgctgagc caagcattgc aactgtgcct ctgctaaaag aaacttccca gaaatgggaa
140881 gttctgggac ttaaggcctg ccatcttgtg ttttttgttt catggagtgc tcccttgatg
140941 tcatgtactt cttcttccct taggagtaga aatccctgaa gtccagacta ctgtgaatgc
141001 aactcttatc tgggtctagc tgcctagtgg ggctgccaca ctccagggtg gtgctggggg
141061 atgtctccaa ggaatccagt gatgtgacct gtcctcaagt atcccagcag tgggtaccag
141121 caccagttct gatggggatg gcaggggagt gctgtgagat tccttggtca tgaaaagcct
141181 tagtgtgttg gttttctcaa gtactggttg tagtagtaga tgaactggtc acatggacag
141241 actcaggatc tcttggttag ccagggtgtt gcaggcaata gtgattgctg aggtcacaca
141301 tgacttttct ccttcctgca tgttgtcttc ttctgcctga agatgctaat ctcctatgtc
141361 agttggcctc caaccagaag gtggtgcttg caaaagagta ccagctgtgg tggtggtagg
141421 atttatgctt tgccttatgt acccagggga agtactctgg tgtctcaggc aattggtggg
141481 gctatagagc tccgaaaagt ttctgttctt tttattaagc taccaggtta ggattgaggg
141541 gcaaagccag gtgagggctg ggtcaggcaa gtctgtgctc tagctctcca tatgtgggca
141601 aaagtagagg tcccagtggg gatcagaagg cagttttctg actgctgggg tatattgaag
141661 gaaaaagcac agctgcttct gctgcaccaa agagtctgca tggagaatgg ggagtagcag
141721 gtagcagcaa gccctacgca gctttcatgc acttggcaag gcaaatctca tagccacagt
141781 gtaccactag cagcagctag ctgggtttca gacagttgac actcagaact caaaactgcc
141841 ccaggccatt agcctttgca tggccttcag gccacatttc tcttggtctc cctacacagc
141901 aggggcaccc ctgtgctctc ttatggctgc agcacacttc ccacttgccc ctcagttctg
141961 gccaagggag cttgttccca ctcatatcgt gaatctcagt tgagcttctc tcaacctgtg
142021 attgctgtct gagttagctg gctgacttcc acaaggtccc ctgtgaggta ggatcagcag
142081 tggcttccct ctgtccttgc tggagactgg gagtgcactg aaagcatgtc cagtgctact
142141 ccttctcata tactccccac ggctcactga atcagcttta gtgctgggta gggttgaggg
142201 cttcccttgt ggcctggatt gccaggttcc ccagtaggag tgcatatcct agaggtagtt
142261 tatcctcctc tcacactcta gggacttaga gttttccatc tgcctcgtag taagtttctg
142321 ggttttttcc tgaaaaaaaa attcacagtg tgaatctctc cacactattt tgtctttcca
142381 agtggtagag gcatgctcac agtgcctcca atccactctc ttggggatta aaaaaaaaaa
142441 aacaggtgtt attgggtacc tttggggtga gagttagtgt acaatttagg gacactattc
142501 ttggaaccag acagaccttg gtcagactcc tggctcagtc attttctggt tgtatggacc
142561 acagcaaatt taaaacttta aatctcggtt ttctcatctg taaaactaaa ataatggtgg
142621 tcacacatac ccataagatt attttgagac ttaaataaga taatgcatac aaagcattta
142681 gcacaaagtt tgactcagta ggtgctattt ggtgttcatg ccattattag ctttgtatta
142741 aagaggtata tattgtccta tcttctagga gcttacagtc cagtagggcc ataagataca
142801 tattgaggaa agaaaggggg atgtcagcct gctgtgatgt gttgggagag actttcttga
142861 ggagttggga ttggggccac tgtgttgtac aatgccaggg aacaccattt acatcatctt
142921 tgagaatggg tccctctgga gtagtacagt tgcactgctt ggaattttga atgtgaccat
142981 gaaggatgtg tagaatttgg atagatggaa gaaggagggg tttgggtttg gtaggaaaaa
143041 acaagattct ttcagaggat ggtgagacct ctcagctggc cagagagctt tttgcattga
143101 gcagaggtag tcaggctgtg tgtcttgagg tggctccagg gctttgcttc tttctgatca
143161 ctaaaaggtg gatggaatga accttctggt gaaatgggta caggtcaggg tgaaatgcct
143221 ttgatctctt cacattgcct tccttgagtc tcctcagggg ccgggattgc tatatttcca
143281 aaagggcact tttctgtgaa tagcctttta gcctggttaa gtaaaccaaa aggctcactt
143341 gctttttctg ccatttcttt ttctttaaca ttacttgtaa ctcattttgt taaataccac
143401 atttactcaa tatgtgaagt gatcaagatg ttgtatgtgt gaccctgtac cctggttcta

143461 ctttgtatag ccttacattt cttttgaact tctttatgca ctttttttct ttccttttct
143521 tacctccatg gaatttattc tgctctttaa ggactctcaa aggcatagta gatcatggca
143581 taaattaggt ggcaagaaag gatttctttt atcattaggc ttctattctt ctaaagctgt
143641 gtctttggga gattttaatt gtgaggatgt aaataaagcc atatggacca aaagtgaata
143701 gcatatttaa ttatcttgta aataacttta gcttagtaaa tctccgacgt tattttaaaa
143761 acacagtatt ttattgggta tttagtatta tatgatactc taaagcagaa gtcaccaaac
143821 ttttactcta aagggccaga tagcaaatat tttaggcttg caggccgatt ctctgttgta
143881 gatactcagt tctgccatgg tagtgtgaaa gcagccatag aaagtataca aactgatgga
143941 ggtggttatt ttgcattaaa actttattta aagcaataga cagtgggtca gatttggctc
144001 tctggagtgg tttgctgacc cttgccgtca aggaaataga aagagatttt gttgggttgg
144061 gaaggtaaat ggaaaattac aggaattaca aaatttccag ataatgaact ctacaaaaaa
144121 ctaaaaaaaa cttatacctc ctattgtttg aattagattt catgtttgat atgaatttgt
144181 aaataaatcc caaatatctt tattgatagt tcttcctttt taaaatctaa ggacttggtt
144241 cacaacaggc ctgtctttat taatcaacca tttattgttt tctttgcacc aggcactgtg
144301 taggtcattg gaaatttaca aataacttag acacgcattt ttcctttaag gagccacagt
144361 ctagactgtg tactcttttt tgccagatgt ggactcatgc cttggaccac atgaaatatt
144421 tgctctaaag tagcaagaaa cctgcattgg aaaagggact ttttgagggt acacctatct
144481 ttttttctct ttctactata tgtgtgttag gactgctata ttttcgtttc tctttattgt
144541 tgtattgtta gagaaagtct gctagaattt ataaatggtg agaatatttt gataacttag
144601 ttgccaaaat tcctagtctt gattccagag gttgtgtttt cctgcccttt aaaattttca
144661 cttagtcacc ttaattcagt ttttgaattt ctttgagttc tgcctatcaa attataaaca
144721 agcaccacca aattgcagaa ggttatctct taatagtatt gaaataggca gttataaaaa
144781 attgtgtcac ctaagtctaa cagctcttct agaatgtttt cctttagacc taagccattc
144841 tggcccttgc acattgggtg ggtctgtgat ctgtttctac tcacaacact tctgacacaa
144901 atggctggat tttttccaca ccaacaacca attcttcgac tctgacacta cccagagtta
144961 gcacagaccc cacagattat gggcttagac ccacaagact cttctccact ttagatgcca
145021 gttgtaagtc caggtctcct atacttctga ccaagagatg ataaatcttg agtttctatg
145081 agccccactc tttgggttca gtaattctct ataaaggctc acagaactca cgaagtcact
145141 ttacttccta ttactggttt gttatagaag ttacaaccca ggaacagcca aatggaagac
145201 atgcacagga caaggtatag aggaagggca ttgccctctc tagatgccca ccctcctagc
145261 acctttgtgt gttcaccaat ccagaagctc tttgaactct ttaacttaga ggttttatgg
145321 cgtttccatt acatagtcat ggttgactac atcactggtc attggtgatt agttcaatct
145381 ccatcccttc ttccctcccc agaaatgggg caggggctga aagttcattc ctcaaatttc
145441 gtggttggtt cccctggcaa ccaccagccc tcatcctgaa gctctctagg ggcccaccaa
145501 gagtcatcac atttgcctaa actcaagtat gattgaaaat gggctataat aatgaacaag
145561 agacactctt ctcattccta ttgcccagga gattccaaag gttttagaaa ctctgtgcta
145621 ggaactggga atgaagaact tatttttatt tttttaagac agagtctcac tctgtcaccc
145681 aggctggagt gcagtggctc aatctcagct cattgcaacc tccgcctttc tggttaaagc
145741 gattctcatg cctcagtctc ctgagtagct gggattacag gtgtacgtca ccacgtctgg
145801 ctaatttttg tatttttagt agagatgggg tttcatcatg ttggccaggc tggtcttaaa
145861 ctcctgactc cagttgtgtc cggagttggt tccttccagt gggttcttgg tctcactgac
145921 ttcaagaatg aagccgcgga cctttgcggt gagtgttaca gctcttaaag gtggcacgga
145981 gccaaagagt gagcagcagc aagatttatt gtgaagagcg aaagaacaaa gcttccacag
146041 tgtggaaggg gacccgagtg ggttgctgct gttggctggg ggtggccagc ttttattccc
146101 ttatttgtcc ccgcctatgt cctgctgatt ggtacatttt acagagtgct gattggtgca
146161 tttacaatcc tttagacaca gagtgctgat tggtgcgttt ttacagagtg ctgattggtg
146221 catttacaat cctttggcaa gacacagggc gctcattggt gcatttttac agagtgctga
146281 ttgatgcatt tacaatcctt tagctagaca cagggcgctg attggtgcgt ttacaattct
146341 ctacctagac agaaaagttc tccaagtccc cactcgaccc aggaagttca gctggcttca
146401 cctctcacaa gtgatctgcc tgcctgggcc tcccaaagtg ctgggattac aggcatgagc
146461 cacaatgcct ggccttatat ttcttattat atcataaaat cacagtggag ttggatgggg
146521 gtgagggagg gcagtctgtg aaagagagag gttctgaagt ccaaatgagg gcagaatgtg
146581 gacttctctg gccctgagga gtgggttggg ttgagtacta tggggtgatt tgattggaca
146641 ggttctgatg agtctggtag tgggtacctc aggagccatg aggacattta aatgtcacag
146701 tgtcacagaa aacatagaag attgttccct aacttgggta gtggcatgag aaagtgggtc
146761 tgttgttggg aataaggtag gctcggatgg tggtttttga gggtgccaag aaatgtcttt
146821 ataaggtttc aaatgtacat agatgtagaa ggaagaatgg tgccattcta aagttttttt
146881 gaagtgaaaa acaaaaataa tatattttaa agatacaaat aatttccata aacatataac
146941 aatccataga gaagagggta atgtccttct ggtgacatta attgaaatac tttatgccag
147001 attggacaaa atctatgttt aaattactgg tattagtatt ttggtatttt ccctttagta
147061 agataatctt gataaccttg atttgctttt tgtgtcctga ttaccttgac attaatatct
147121 tgaaatagaa aaactgtggt tcttgctttg gtagtacata tactaaaact agaacaataa
147181 agacattaac atggcccctg tcgaaggatg acatgcaaat ttgtgaaatg tttcatattt

147241 tttaaaacaa aattatggct tgtatatttt taaaactttg tattatgcaa aacttcttga
147301 gaacaaataa ttatgggaac ttttaaatac atgcaaaaat agaataggat aataaacttc
147361 ccatctacat tttatttatt tatttattta atttttgagg tggagtctcg ctctgtcgcc
147421 caggctagag tgcagtggtg tgatcttggc tcactgcaac ctccacctcc tgggttcaag
147481 caattctccc tgcctcagcc tcctgagtag ctgggattac aggcatttgc caccatcccc
147541 gggtaatttt tgtattttta gtagacatgg ggtttcacat gtttgccagg caggtctcga
147601 actcctgacc tcaagcgctc cgcccacctt ggcctcctaa agtgctggga ttacaggcgt
147661 gagccaccgc gcccggctgg tttttaagac atgaaatttc agaatgtttg ttttcaagaa
147721 agaaagccca attgcctcaa tgctttattt gaggacagag cctgaggcac aggagaatag
147781 cacggtggga cattactggc accattgcca acacagctca cacgcagcaa cagcacgttt
147841 tgttggtttt gccagcgttg atgctcagat ccctctgtgg ccaccaatag ccagcacctg
147901 tgtcaaaggt ggctgcctca attaagacac aaaccaaaaa ttctaattat atagtagttt
147961 ctgtagtatc agccacgtgt acaataggta ctcaggtgtc atttgctttt tcttctacca
148021 caagagtggc cttggactcg tgtgtccgtt gggtgatggt tgaggttgct ctgagccagt
148081 cccctcccct ggtccactca gtggaagctg tgtatgccca catggcactg tatggacacc
148141 agtttgtgtt tttttaacaa acattgctat ttttagattt gctttcagaa tgcgacctat
148201 ggggcagagg acccagaaat atatagcttc tacctcattg cctcgttctc agggatatcc
148261 aagcctcact gccttgagaa attaagagac agttccagca ggtactgtct tacttgatga
148321 ctataaattc tataatcatt gctaataata gagtgttttg ttcaagccgc ttaaatttgt
148381 ttattttttc ctctcggcaa gcctaacatc atcatcctca aaagcatggt tgcctgtgaa
148441 tttccttttg gatagatgta acactattta atttcgccca tccattcatc tatccaagca
148501 tccagcaaac atttatcgag tacctattat gtgctgagta ctcttttaga tgctggaggg
148561 acaatgatta ataagataat ctttctttac agagttcaca ttcggtgaga gaggaaagtt
148621 ggacattaaa acagatagta atgtggtgag ctaagtgcag ctataaagtt atgtacaact
148681 tatcaggaga accgttatct aaggcatcca gttcacagaa gggaggaggg aacaccatgt
148741 aggcaagggg aatttcacta ttattgccat atatccaagg ccaagagtgg tgagagaatg
148801 ggctggagat gtaacaaagg tgctctgtga cagagtttga acttcagttt gctggtgagg
148861 tgtggggaag tctatgaacg ggattcgaac agttgagtgg catgattata tttgtgttct
148921 agaagaatcc ctctggtggc tctgtgcaat gtggttttca gggagtgagg actggagaca
148981 gagtagataa gtgagaatgc tgaggggcct aatgattcgt tcatgcattt catttagtaa
149041 acatttattg agcacttact atggaccatg ccctgttata gtcatttgga atttattttc
149101 acatagataa acagaagaga taaatagtag tagctttttt gggataaata gataccaaat
149161 aaatcacagt gttcgctttg atcatgacaa acttttgaat acatgttttc tagagttata
149221 tattgatttg gtggtatttt tattttggat ttggatcagt cagcactaac ataaaaaatg
149281 gcctctttca tggtgcaaat tagtaaggct tcactttcag ctcttgaatt tttccaagac
149341 tcattattcc agtctgtaga ttattctgag ccttagactc tcattttctg ctcattagca
149401 cctcaaagaa tggagaggaa ggaagagaga gaaacttcag tctgacttgc tacttgttag
149461 aagatttggt aaagaaggtg atgaaattaa tatttagttt tcaaaatcag gttacagatt
149521 gtttttgggc ttgttcttgc ctgcagttct tgctttctct caaccatagc taacttgctg
149581 tctggataca tccgagtgta tttgcctcat gcacaatgaa gggtaggatt aaccttggaa
149641 atttgctgtg ggcaaatagc ccaagaatta atacatttaa taatacattt aattatttta
149701 ccaatcacac tggttatttg cagtgtcaaa gcagttacct tggtatgata taaggacatg
149761 gaattaattt tgttgtttat aatctttgtg tgtgtgtggg gtgggtggtg aggacggaat
149821 cttgctctgt cgcccaggct ggagtgcaga ggctcaatct tggctcactg caacctccac
149881 ctcacagatt caagtgatcc tcctgcctca gccttccagg tagctgggat tacaggtgtc
149941 tgccaccaca cctggctaac tttttgtgtt tttagtagag atggggtttc accatgttgg
150001 tgaaactgct gacctcaagt gatc
//

- LOCUS: AL137435 2458 bp DNA linear PRI 25-JAN-2005
- DEFINITION: Homo sapiens genomic DNA; cDNA DKFZp761G0924 (from clone DKFZp761G0924).
- ACCESSION: AL137435
- VERSION: AL137435.1 GI:6807994

1 cttgtcttac aatttttaat tgtttaaaag aaggtttgat atataaaaac aagaaaagga
61 gcagctttga gagttaaaaa agccgaggct gaaatgatgc attacaaagt atatagattc
121 tgtactccag ataagatggg gtttagaggg agtggtagaa acatttgctc cttaatggca
181 aaggtaataa actcctgctc tcactgcaag aattgaccat gagatggggt caggaatctt
241 caggggagca gacagtggtg tggcccttgt ctaggtctcc atttatacct gaagatggag
301 cggaatacgc actccagatg cctggaacac cattgcatgg gagtgtgggt cacggatccg
361 agggatacac actctagatg actggaagtc aatagcctgg gaagcgcagg tcacaccccg
421 agggaaaggg cttcaggatg aacatctgca gaaggcgtgg acaaggatgg gaggcaacag
481 ggtcactggg ctttccagga ctcagagaat caaaaggctt ccaggtcatt tctggtagag
541 ctgttggtgg ttacaggaag caatttggat cccagactcc tggagatgaa cagctttacg

601 gtgagctcag ctctgagtgt ggaagcactg ccatcccagg ggcttctgtc gcagaagaaa
661 cggaaatctg gcaaaaggat ggccaggctg gccagaaacc actggtctag atagaaaagg
721 aatatgctac tgttgccagc caaggctttg tttgttttgg ggaaaaaata ttgctctgag
781 tgaaatatct atttctggca tctattaaca attggttatg gttccttgtt ttaattatat
841 cattacattt ttaaaatgaa ggatttaatt tgcttttgct cctatttgtt gctgatgtcg
901 ctcagttctc caaatggctg ctgtcttcct ctagtactta gtctgccatc tagaagcaaa
961 ttaaagacac ccaagaaact tcagttattg tattggtaac tataaaataa tgattaacaa
1021 acagaaggac aggaagcggg agaaggcagt caagtaggaa gtagaaaatc taacactgag
1081 agagcacccc ccaacatagg gactgtgtgg aggttttacc caaatgatgc ttcatttaat
1141 tttcaccaaa agaaagtgag gtccttcccc atctgaggga caggcctctg acacccatct
1201 ccgtagcagt catcccacag gttggccatg gctgagtagg tcaggacaga acaccttctc
1261 gaagctgggc tatcgtcctc tcactctttg atttggaatt cagtctttat cattgaaggg
1321 ttagaactgt aatagacaaa caattgggaa ttaagaggct gccagatcct gttatatgga
1381 gtaacaaaat aggacattag atggatagat ggagagagac agaaagagag atggatagat
1441 ggatggattg agagatgata gataacagat agaggacagg aaagaggaga gaaagaaaga
1501 aagggcacga gaaaaagaaa aagagagaga gccacgtaca gagagaaatg tgcatgggaa
1561 gcagagcccc ggttccagtt tcttcctgta gttcccctgc attcctattc cttggttcca
1621 tgagacaccc caagtcctta caacaaactc tcattctaag ctaaaagttt gtttctgttg
1681 cctgcacaca aagactccta actaataaaa cagtgaacaa aatgactgat atctgtaata
1741 gggtaacctg ataacacttt taagattgat tagattgaga ccaaacagaa gggaaaacac
1801 ctaggcaccg tgaagaccta tacagtcatt aacattctcc tgacttagaa cctgtgataa
1861 ttagggagtg acctgcagca gtttccttca ctctgtgagc catgcatgca aatgaggggt
1921 gcaggagagg aatgcagacc tggggcccac ccgcattcaa gtgcaattga tggggccatt
1981 gcaggtcacc ctgatctgcc cagtgcccct agctgctgct ggtagacttg taggatccta
2041 taatttaggc ttctcattaa ttcagatgaa ccgtgacttt ggtgtgccct tctccattcc
2101 agttatgtgg aagaaatttc atctcttggt cttttatgca ttttaagatt tgatagcaac
2161 gctagtggct catgcctgta atccagcact ttgggaggcc gaggtgggca gatcacaagg
2221 tcaggagatc gagaccatcc tggccaacat ggtgaaacac tgtctctact aaaaatacaa
2281 aaaaaaaaaa aattagctgg cgtggtggca cgtacctgta atctcagcta ctcaggaggc
2341 tgaggcagga gaatcgcttg aaccagggag tcggagattg cagtgagctg agatcgcacc
2401 actgcattgc agcctggtga cagagtgaga ctccatctca aaaaaaaaaa aaaaaaa//

- LOCUS: AW003733 839 bp mRNA linear EST 08-MAR-2000
- DEFINITION: ws16b04.x1 NCI_CGAP_Kid11 Homo sapiens cDNA clone IMAGE:2497327 3'similar to SW:RHOD_HUMAN 000212 RHO-RELATED GTP-BINDING PROTEIN RHOD ;, mRNA sequence.
- ACCESSION: AW003733
- VERSION: AWO03733.1 GI:5850649
- KEYWORDS: EST.

1 aagtgatgtc gttttttttg ttacagttac cagtatgaca ggtgggaagg ggccgggtgg
61 ggttctggcc agagggacct aagaacatta ggtcctggca ggtgcagccc tgggagcagc
121 cacgaggggg catcaggctg cacaggcggc tccaggatgt cggtccggga ccgggcatag
181 gggttagcga gtgattctcg ggcaccgcag gacgagccct acacagagca ggtgggccca
241 ggaaccccct ttctcagtcc aggactccca ggcctcaggg cctggctttg gtggcgcccg
301 tctgttgcag tggagttcgg cggtcacagc ctagggaccg ggtccagccc agccagctca
361 gcagcaggtc agcgccctgc cccttcccgc gtcgctggga cgccccgagc cgctcaggtc
421 accacgcaaa agccctgggt aatccgccgc cagaagttgc gaccgcggct gctgaggcc
481 acctcggcgg cctcctggaa gacggcgtgg acgttgtcat ggagccgagc cgagcactcg
541 aggtaggcca ccgcgcccac ggaccttcgc atctnctggc ccctgtggta ggtcacaggc
601 tccaatccgt ttctttcgag cttgtttacc agtgattngt ccttgcgcag gtcagtcctg
661 cagcccacga cgatgatggg taccttcttg cagaaaatga tcactttctg gtaccaccgc
721 tctaangatg ttgcaagctg ntctggcttg tgacatcgaa gcanagcagc aggacgcctg
781 ctgtcagggt agaacagggg cccgcagcgg gcatagtcac tcttgcctgc tggtnccca

- LOCUS: AL137475 5418 bp mRNA linear PRI 18-FEB-2000
- DEFINITION: Homo sapiens mRNA; cDNA DKFZp434F0723 (from clone DKFZp434F0723).
- ACCESSION: AL137475
- VERSION: AL137475.1 GI:6808086

1 tttctttctc ttgtactcta cacccaaaac atcagaaaat ccttttgact ctgacatcaa
61 aagacatcca gaatatgacc catttttaca attcctcttg ctacatagct ggctcaagcc
121 acaatcatct tagagctaga ttaagagcct aggtattata acagattatt gttataatag

181 gtcattgtga tagatctcct tgcttctgcc ctggttccct ataggtttac actcaacata
241 gcagttaaga gcttttcctt gaatatatta ggtcatgtca ctctaaataa aagccctgaa
301 tggtttccca gcttcctcag agtgaaagtc aaagtcctta cagtgaccta taacaaactc
361 aaactataac aaaacaaagt tttcttccct caccttgctt ctctgacctt agctattact
421 attctccact tctcattctg ttgcagacat cctggtctcc ttttggccct cagagactcc
481 aaccaaactc ccacctcata gcatttgtgt cctgctgttc cctttgcctt gactgctcat
541 ccccaagcta cccagaatgt ttgtttcctt gtttttctcc cctcagttct attcttaaac
601 ataacttatt ttattttatt ttgttttatt tttgagacag tcttgctctg ttgcccaggc
661 tggagtgcag tggtgcaatc tcggctcact gcaacctcca cctcccggct tcaagtgatt
721 ctcttgcctc agcctcccga gtagctggga ttacaggtgc acaccatcaa ggctggctaa
781 tatttgtatt tttagtagag gtgaggtttc accatgttga ccaggctggt ctcgaactcc
841 tcacctcaag tgacctacct gtctaggcct ccccaaatgc tgggattaca ggactcagcc
901 accacgtcca gctacataac tttattatag aaacccctga acactttaca tcaaaaagca
961 tcactcactc taaccctgtc agagcccccc accctgctct gcttctcttc ataacactta
1021 ataccattca gcactatgca ttttctggtt ttttcctgtc cttcctcact aacacgtaag
1081 cttgatagaa ggagaagctg tatgtgtttt gctcagtgtt gattctccac cacccagaac
1141 acctcctagc ataaggtcag cactcagtaa gttgctgaag gaaagaaggg atgggtttga
1201 gcagagtgga gatggagatc agctgatgaa cttttgagat ctagtaggtg gcagaatagt
1261 cagattgtga tgaattgttg ggtattgagc aggaaaaggg agatgaagga aataagaggg
1321 ctctaggagg ctctccaagt ttctgcccta cacactgagt tgatggtata ctgttccctg
1381 aaatagagaa gatgggaaaa ggatttgatt aaggacaggg acagagaagg ataatttcaa
1441 ctttgaggaa gttgagattc aggttattag gacatattca actgaagata tcctggaggt
1501 agctggctct agaactatta actgagtttg tgctgaacac tcatctctgt taggtacaaa
1561 actgcatgga aaggagatga agagagactg tatagtattg agtcattttc cagggaaaag
1621 ctaaatcctt ttcacccctt caggtgaact taccaaacca gctgctactc agaagctcac
1681 agcctttcag aaaaaggtat tctccaagcc tccaaggagt cttattgtct caaggcaacc
1741 atttttcagt ttgactctag gggtgcgtgt ttgccattca gaacctacta tattttaccg
1801 cacctcctga cattgttaca caacatttat tgaaagcaca tttgatggta atgaagtttc
1861 tattaactat gattaaatct cataaagcac ctgaagataa tttaagatgt gcagaagcag
1921 aaaatgaaac attcggttta gtacattagg aaacgtaatt gattaaatta tctcatttac
1981 aataagtgct ttcatgagct atgcagtgca catattttcc ccagtttgtc attatgattt
2041 gccaacagat atcttaagac ttcactttct ttttacacct ccccgcaact ctaagctcgt
2101 gcctctgcct gcctgctcat ggagcactct gtgcagagct ctgagtgatg ttttctatct
2161 gccaggcctt atggaagatc atgtgtatgg ctatatatag gcacaggttg tttctcaatt
2221 atcttcataa ttgactttga ttttgcatta gtgatgattc atgtagtctc acattaagcc
2281 cctctttcac tgaacaactg cagcctgaat ttctataatg cactcctaac catgcagctt
2341 acagaacccc gattggattc ctctgggagc cttttgcaat cacattgatt ctctaaggtc
2401 ttatttttag agaaatcaca aattaaaaca gtttaagcga ttccgttgca gtatccagca
2461 attaagcatg gtgctcagga acgagaaccc caagcaaaaa ctaattctga agtccctaag
2521 gactggaaat ggcttctgcg gggtttatgc ctggaagtac agatgtaacc aaattagaat
2581 ggcacaggac tcacaaacta agtgtgaaat taattttaaa aacacataca tgcatttcta
2641 agtaactctc acattggcca ccccatggat acctatgatc ttttttgtgc tatatgacag
2701 aatcagtgga cagcatgcac catgtggccg ggagcaaggt aagagtccac ttactccacc
2761 tgtcatcctg agggtcctga cacaagcaca ggccaaatat tcagtgatct gtttatttct
2821 ctctccctca atgcccatac acatgaacac acatatacac agccaggtgc cccaaactc
2881 agtcttccca taaattcaaa atcaaacatg catgatgtca gaggaccaat acttaagtag
2941 taatgatttt ttttaatggg cagcagacag cagggaaata agtttaaatg tttatttttg
3001 ctgttttccc aaattcttag actttgcaag taatgatatc tttgtatctc cagccttcct
3061 gaagcaacat aaggaaatag gaacagaatg gctttactgg atttttccct catgtcttgt
3121 gacccctgtc ccaagttacc tatttctgtc tatcctcaca gggtcttaat tctctcaaag
3181 aagttagctc ataatagcta ttaactcttc caagctcttt gaattttaat accctaagat
3241 ttaaaagatc aaaagtcatt cagttgatac tagaatgaga gatgctaggc agggacgtct
3301 ttcacaagct ttacaaacca aaccagccag gccgttgtct aatctcctac taatgagagc
3361 tgtattaatg ttgccccagt atcttgaagt ttccctagcg tcctttacat atttctgatg
3421 tatgagaatg acttggggaa catattaaca catagattct taggagctcc tcaaccagga
3481 attcaaattt atttgttctg ttctgaggtg gtgcatattt aatttaacaa gctccagcca
3541 cactctgaga ttctgatgca tgtggattct gatccacctt ttgagaaaca tttttctaaa
3601 atcatggttc tcagccccac ctgcaccttg gagtcacctg aggagctttg aaaaatagag
3661 atacctgggt cctgaaaacc ctccacctta aattccaata gccttgatac agcctggaaa
3721 ttgggaattt taaaaggcct acaggtgata ctaaagagca taaggtcaaa tgccatactg
3781 ctagccattc cagccatctc ctctctccag ctctgcagga agcataaaat gaagacaatg
3841 caaaggcctc tcccataagc aagcctgggt acaacgagcc taagccagta ccaacacccc
3901 aatcctcagc tcgtcattat ttcaccatgg agttctggat cctaatctgc ccagctgcca

3961 tggattccac aggagattct tatcaggatg agaaaagcag tgagctttca gcctgtcact
4021 ctattatata caaccagaag cctgctcagg aacgaagatt actctgggta gcagaatgaa
4081 actccattaa aaacaggatt gaaaggctgg gtcttacaga ggagatcaga tcatgaggac
4141 tctcccctca tgaatgaatg aaagaatgga ttaatgaatt aatgggttat catagaaggg
4201 aaactggtgg cttcacaaca agatgagaga tctgacctcg aatatttgcc tgctcagcct
4261 cattgccatg tgatgccatg tcctgtgcca cctctggact ctgcagggag tccccaccag
4321 caagaaggct ctcaccagac atagctcctc gaccttggat ttctcagcct ccagaacggc
4381 tctacagatt gattttaaag ccacctacac tgcagtttct tcaatttagc tctacttatc
4441 tgattatgca tcacaaaagg aagcagaaat tcagcttaat tgtgaagcca ttgcagcaga
4501 accaagcaaa tgatagaaaa cctcatgatc acagacagaa tcacactcac tgatgctgaa
4561 tgaaatgaca gacatccgtc cctctgcaga agcctgaata ctgatcacag aattttacgt
4621 tctgacattg tgtgtctctt gtcagatagt gttgtccttt actttccccg cttcagttgt
4681 gaaagtcagg tgcagtctca tactattttt ttttaaaatt cattatgaca agtaaatggt
4741 ctctgggaaa gcgaacatcc tcatccactg tttactgaaa ctgtaacact gtcatgagtg
4801 aagaatgaaa catgtttaat gtaaagagtt tgcagaatga tgatcagagg aaaagaacat
4861 taaacagaag aaacataaat ataagaatgg ggagagagat gtaatcaaaa gacagcttgt
4921 aagggaggtt acatgctgcc atttgctttt cagctgtgtt ttccatagtg ggacagatct
4981 tgctctttgt tacttctact tttgcttttt cccatctgca aagagggaaa caatctgctt
5041 ttgaatctct ggtgaagcat cacctagaag ctgctgccaa agcttgatat ccagagtgct
5101 ctaaaacata gtgtgatgtg ccctttcatc ttaaaggatt atttttggct gggcagacct
5161 ctctaagata gattataagg agctggcttt taaaggcaat gaaaactgct tctgtattca
5221 catttacccc ctccctccat tatctgaggt atgtaaataa tgttttaaag tggtgatgtg
5281 agaatattaa ttatgttttc atttcttgtt cctgttacca tgtaatatat cttcaagtgc
5341 tgagaaaaca tcttacaaaa ccattaaaga ttccaaatgt tcatacataa aaaaaaaaaa
5401 aaaaaaaaaa aaaaaag//

- LOCUS: NM_025094 2275 bp mRNA linear PRI 23-DEC-2002
- DEFINITION: Homo sapiens hypothetical protein FLJ22184 (FLJ22184), mRNA.
- ACCESSION: NM_025094
- VERSION: NM_025094.1 GI:13376655

1 ccctaagctc acccctgacc atacaccctc tgcaggcgct atcttctctg gcctcccact
61 ctcctcaggc acctctctct tccctgatca tgccccctct ggagacccaa tcttccctag
121 ccccaccctc tctgcagaca cctcctgctt ccctgaccac accccctctg gagaacctac
181 cttctctagc cccgcctcct ctgcagacag cttctgctcc cctgaccaca ccccatctgg
241 agactccacc ttgtccagcc ccatgccctc tgcaagcacc accttcgcca ttgaccacgc
301 cccctccgga gacgccctct tccatagcca cgcctcctcc acaggcccca cctgctctgg
361 cctcaccccc tttgcagggc ctgccctctc cccctctgtc tcctttggcc acgcccctc
421 cacaggcccc acctgccctg gccttgcctc ctctgcaggc ccctccctct ccccctgcct
481 caccccctct gtctcctttg gccacaccct ctccacaggc cccaaatgct ctggcagtgc
541 accttctgca ggcccctttc tctccccctc cctcaccccc agtgcaggcc cctttctctc
601 cccctgcctc acccccagtg tctccttcgg ccacgccccc ttcacaggcc ccaccctctc
661 tagccgcacc tcctctgcag gtcccaccct ctccccctgc ctctccccca atgtctcctt
721 cagccacgcc ccctccacag gccccacccc ctctcgctgc gcctcctctg caggtcccac
781 cctctccccc tgcctctccc ccaatgtctc cttcggccac gccccctcca cgggtcccac
841 cccttcttgc cgctcctcct ctgcaggtcc cgccctctcc ccctgcctca ctcccaatgt
901 ctcctttggc taagccccct ccacaggccc cacctgctct ggccacacct cctctgcagg
961 cccttccctc tccgcctgcc tcattccctg ggcaggcccc tttctcaccc tctgcctcac
1021 tcccaatgtc tcctttggcc acgcctcctc cacaggcccc acctgttctg gcagcgcccc
1081 ttctgcaggt ccctccctct ccccctgcct cacccaccct gcaggcccca cgccgccccc
1141 cgaccccggg tccggatacc tccgtctcgg gcccacggct gaccctggcg ttggcccccg
1201 gcccgccgcc gccgccctcg cgcagcccgt ccagcacgct gagcggccca gacctggccg
1261 gccacagcag cagcgccacg agcacgccag aggagctgcg tggctacgac agcgggcccg
1321 agggcggtgc cgcagcctcc ccgccccccg acgcagagct cgccgcttgc cacccggctg
1381 cctggagtcg aggccccgct ccgccgccgg ctttccgcgg agccccaggt gcgcccctgc
1441 cgtggcctcc cgctaccgga ccgggctctg ctgacggtct gtgcaccatc tacgagactg
1501 aagggcccga gtcggcgacc cccgcccctg gcgcactgga tccggggccc agtcccggca
1561 cgagcggtgg gaaggcggcg gctggggccg gggccggggc gtcctcgcgg agcccgaagc
1621 aggcgcgcct gggcgagctg ccactggggg cgctgcaggc gagcgtcgtg cagcacctgc
1681 tgagccggac gctgctgctg gcggctgccg agggtgccgc gggcggcagc ggcgggggcc
1741 caggaggtgc ggagggtggt ggcgtcacag ggggcgcccg ggctgcactc agcgacgccg
1801 aactcggccg ctgggccgaa ctactgtctc ccctggacga gtcccgtgcc agcatcacct

1861 cggtcaccag cttctctccg gacgacgtgg cctccccgca gggtgactgg accgtggtgg
1921 aggtggagac cttccactga gccagaccac agccccgcct gctacacccc acccctgcct
1981 taggatccgc ccctccgggt acgccgtttg ttttagaccc cgcctccact gccctggagc
2041 cccgctgggt ggattagtct tagctcccta gagcctgagc ctttggcctc ggaggctcgg
2101 gacctaccca tagctttgac ctaggcccgc ccctcgagct ccgccccttt ggcctaggac
2161 acgccccgtt tccccgagtc ccgccccgtg tgcagtgtat tgcccacccc gcacagcctg
2221 agtttgcaat aaaactggga cactgggact tgcaaaaaaa aaaaaaaaaa aaaaa//

- LOCUS: NM_025052 4165 bp mRNA linear PRI 18-NOV-2006
- DEFINITION: Homo sapiens yeast Spsl/Ste20-related kinase 4 (S. cerevisiae) (YSK4), transcript variant 1, mRNA.
- ACCESSION: NM_025052 NM_001018043
- VERSION: NM_025052.3 GI:68077163

1 ttcaaagaaa cagcagcttt tggacatttt aatgagttct atgccaaaac cagaaagaca
61 tgctgagtca ttgcttgaca tttgtcatga tacaaactct tctccaactg atttgatgac
121 agttaccaaa aatcaaaaca tcatcttgca aagcatcagc agaagtgagg agttcgacca
181 agatggtgac tgcagtcatt ccacactggt taatgaagaa gaagatccca gtggtggtag
241 acaggactgg caacccagga cagaaggtgt tgagatcact gtaacttttc caagagatgt
301 cagtcctccc caagaaatga gccaagaaga cttaaaagaa aagaatctga taaactcatc
361 gcttcaagaa tgggcacaag cacatgcagt ttctcatcca aatgaaatag aaacggtgga
421 gctcaggaaa aagaagctga ccatgcggcc cttagttttg caaaaagagg aaagttccag
481 ggagctctgc aatgtgaact tgggcttttt gctaccaaga tcttgtttag aactgaacat
541 ttccaagtct gtaaccagag aagatgctcc tcattttctg aaggagcagc aaagaaaatc
601 tgaagagttt tcgacctctc atatgaagta cagtggccga agcatcaagt tccttctgcc
661 accactgtca ctcttgccca cgcgatctgg tgtccttact atcccccaaa atcacaagtt
721 tccaaaagaa aaagaaagaa acattccaag tctcacatct tttgtgccta agctctcagt
781 gtctgttcgt caatctgatg agctcagccc atcaaacgag cctccgggag ccctagttaa
841 gtcgttgatg gatccgactc tcaggtcttc tgatggcttc atttggtcaa gaaacatgtg
901 ctcttttcct aagactaacc atcacaggca atgcctggag aaggaggaaa actggaaatc
961 caaggaaata gaagaatgta acaaaattga aatcactcac tttgaaaaag ggcagtcttt
1021 ggtgtctttt gagaatttga aggaaggcaa tattcctgca gttagggaag aggatattga
1081 ctgccatggt agtaaaacgc gaaaacctga agaagagaac tctcaatatc tttcatcaag
1141 aaagaatgag agttcagtag ccaaaaacta tgaacaagat ccagaaatag tatgtaccat
1201 tccaagcaag ttccaagaaa cccagcattc agaaataact ccaagccagg atgaagagat
1261 gagaaataat aaagctgctt caaaaagagt ttcattacat aaaaatgaag caatggaacc
1321 aaacaatatt ttagaagagt gtactgtact taaaagctta tccagtgtag tctttgatga
1381 ccccattgat aaactcccag aaggttgtag cagcatggag acaaacataa aaatatcaat
1441 agcagaaaga gccaaaccag aaatgagtag gatggtgcct cttatccaca tcaccttccc
1501 tgtggatgga agccccaagg aaccagtgat agccaaacca agcctccaaa caagaaaggg
1561 aaccattcat aacaaccata gtgtcaacat acctgtacac caagaaaatg acaagcataa
1621 gatgaattcc cataggagta agttggattc aaagaccaag acaagtaaga agacacctca
1681 gaattttgtg atttctactg aaggtcccat taagcctacc atgcataaaa ccagcataaa
1741 aacacaaatt ttcccggctt tgggacttgt ggaccccagg ccttggcaat tgcccaggtt
1801 tcaaaagaaa atgccacaga tagcaaagaa gcaatcaact caccggactc agaaacctaa
1861 aaagcaatca tttccttgca tctgtaaaaa tccaggaaca cagaagtcat gtgttcctct
1921 ctctgttcaa ccgacagagc caagactaaa ttacctagat cttaagtata gtgatatgtt
1981 caaagaaatc aattcaactg ctaatggacc tggaatctat gaaatgtttg ggacccctgt
2041 ttattgtcat gtgcgagaga ctgaaaggga tgaaaacacg tattaccgtg agatatgttc
2101 ggctccatca ggcagacgta tcaccaataa atgtcgatct tcacacagtg agaggaagag
2161 caatatcaga acaagacttt ctcagaaaaa aacacatatg aaatgcccaa agacttcatt
2221 tggcattaaa caagagcaca aagtcttaat ttctaaagaa aagagttcca aggctgtaca
2281 tagcaaccta catgacattg aaaatggtga tggtatttca gaaccagact ggcagataaa
2341 gtcttcagga aatgagtttc tatcttccaa agatgaaatt catcccatga acttggctca
2401 gacacctgag cagtccatga aacagaatga attccctcct gtctcagatt tatccattgt
2461 tgaagaagtt tctatggaag agtctactgg tgatagagac atttctaaca atcaaatact
2521 caccacaagc ctcagagatc tgcaagaact tgaagagcta catcaccaga tcccatttat
2581 cccttcagaa gacagctggg cagtgcccag tgagaagaat tctaacaagt atgtacagca
2641 agaaaagcag aatacagcat ctcttagtaa agtaaatgcc agccgaattt taactaatga
2701 tctagagttt gatagtgttt cagatcactc taaaacactt acaaatttct ctttccaagc
2761 aaaacaagaa agtgcatctt cccagacata tcaatattgg gtacattatt tggatcatga
2821 tagtttagca aataagtcaa tcacatatca aatgtttgga aaaaccttaa gtggcacaaa
2881 ttcaatttcc caagaaatta tggactctgt aaataatgaa gaattgacag atgaactatt

2941 aggttgtcta gctgcagaat tattagctct tgatgagaaa gataacaact cttgccaaaa
3001 aatggcaaat gaaacagatc ctgaaaacct aaatcttgtc ctcagatgga gaggaagtac
3061 cccaaaagaa atgggcagag agacaacaaa agtcaaaata cagaggcata gtagtgggct
3121 caggatatat gacagggagg agaaatttct catctcaaat gaaaagaaga tattttctga
3181 aaatagttta aagtctgaag aacctatcct atggaccaag ggtgagattc ttggaaaggg
3241 agcctacggc acagtatact gtggtctcac tagtcaagga cagctaatag ctgtaaaaca
3301 ggtggctttg gatacctcta ataaattagc tgctgaaaag gaataccgga aactacagga
3361 agaagtagat ttgctcaaag cactgaaaca tgtcaacatt gtggcctatt tggggacatg
3421 cttgcaagag aacactgtga gcattttcat ggagtttgtt cctggtggct caatctctag
3481 tattataaac cgttttgggc cattgcctga gatggtgttc tgtaaatata cgaaacaaat
3541 acttcaaggt gttgcttatc tccatgagaa ctgtgtggta catcgcgata tcaaaggaaa
3601 taatgttatg ctcatgccaa ctggaataat aaagctgatt gactttggct gtgccaggcg
3661 tttggcctgg gcaggtttaa atggcaccca cagtgacatg cttaagtcca tgcatgggac
3721 tccatattgg atggccccag aagtcatcaa tgagtctggc tatggacgga aatcagatat
3781 ctggagcatt ggttgtactg tgtttgagat ggctacaggg aagcctccac tggcttccat
3841 ggacaggatg gccgccatgt tttacatcgg agcacaccga gggctgatgc ctcctttacc
3901 agaccacttc tcagaaaatg cagcagactt tgtgcgcatg tgcctgacca gggaccagca
3961 tgagcgacct tctgctctcc agctcctgaa gcactccttc ttggagagaa gtcactgaat
4021 atacatcaag actttcttcc cagttccact gcagatgctc ccttgcttaa ttgtggggaa
4081 tgatggctaa gggatctttg tttccccact gaaaattcag tctaacccag tttaagcaga
4141 tcctatggag tcattaactg aaagt//

- LOCUS: NM_006010 875 bp mRNA linear PRI 18-NOV-2006
- DEFINITION: Homo sapiens arginine-rich, mutated in early stage tumors (ARMET), mRNA.
- ACCESSION: NM_006010
- VERSION: NM_006010.2 GI:54873599

1 ggcgcggcgg gtgcggttca gtcggtcggc ggcggcagcg gaggaggagg aggaggagga
61 ggatgaggag gatgaggagg atgtgggcca cgcaggggct ggcggtggcg ctggctctga
121 gcgtgctgcc gggcagccgg gcgctgcggc cgggcgactg cgaagtttgt atttcttatc
181 tgggaagatt ttaccaggac ctcaaagaca gagatgtcac attctcacca gccactattg
241 aaaacgaact tataaagttc tgccgggaag caagaggcaa agagaatcgg ttgtgctact
301 atatcggggc cacagatgat gcagccacca aaatcatcaa tgaggtatca aagcctctgg
361 cccaccacat ccctgtggag aagatctgtg agaagcttaa gaagaaggac agccagatat
421 gtgagcttaa gtatgacaag cagatcgacc tgagcacagt ggacctgaag aagctccgag
481 ttaaagagct gaagaagatt ctggatgact ggggggagac atgcaaaggc tgtgcagaaa
541 agtctgacta catccggaag ataaatgaac tgatgcctaa atatgccccc aaggcagcca
601 gtgcacggac cgatttgtag tctgctcaat ctctgttgca cctgaggggg aaaaaacagt
661 tcaactgctt actcccaaaa cagccttttt gtaatttatt ttttaagtgg gctcctgaca
721 atactgtatc agatgtgaag cctggagctt tcctgatgat gctggcccta cagtaccccc
781 atgaggggat tcccttcctt ctgttgctgg tgtactctag gacttcaaag tgtgtctggg
841 atttttttat taaagaaaaa aaatttctag ctgtc//

- LOCUS: AK024527 1636 bp mRNA linear PRI 12-SEP-2006
- DEFINITION: Homo sapiens cDNA: FLJ20874 fis, clone ADKA02818.
- ACCESSION: AK024527
- VERSION: AK024527.1 GI:10436829
- KEYWORDS: FLI_CDNA; oligo capping; fis (full insert sequence).

1 cagatatggt gatatcctta cttctggaat tcaaatcacc agttaggggt accttctcaa
61 ataaataaac ataatagcac tttacgtgtg aataaactca gagaggccct gacaaaccag
121 ggttgggctt tgcttggggg taccaacagg catctacttt ccatttctat tactgtcttg
181 atgtttctta gaattccatc ctggaatgct gaccctcaat gagaaggata aattaaaact
241 ttattaggct catttaaatg tcaattaaca aaggatgttc ttttttagat catttaaatt
301 tatttcataa gctagttgcc caaaagctgc tctgctgggc actcatacaa aattattttt
361 taaaaatata caaattaaat agcagaagag caagctcccc agaacagaaa gaaaacaagc
421 agtgctgatc accatctggg tctccttttg ggaggcagaa cccaagggga aaatagacca
481 catcccctcc tttggacgca atgccaatgc ctagggtttt gtcagccctg gagagagaga
541 gagagggaga gcgcaggtgc aaattcctcc tgtcaatgta agaattctgt gcttttgata
601 cctccaagag cctctctctg cagaaagtat ggaaaatatc gcactgttaa aataacaaat
661 gcagtcagaa caataagaaa tacaaaggtt catacctaag gtgtgaaata agaatttact
721 tgcaaactat aaggaaggag cacaacaaga cagcagatgc taaaagcacc aaagaagaga
781 ccaggtcatt tgagctgtaa tagtttaaag tttggagtga aaggcctatg gaaagaggta

841 ggactacact tgacctttgt agtgcgtgtt tgttgtttca gcctgcccag gatcccaccc
901 cttattctga agacagtacc taggttttct attgggacct accctactcc tacttttaat
961 ccaaatggct tagataaggt ggaccattcc catccttgga tctagagagt ggcatgtcat
1021 ccagccttgg ccagtcagag tcactgtggc ttgtgcaggg atgggcacat gagctaatca
1081 gagcagatga gagtcatccc cagacttttg tcagaactgc tgggaaagcc tatgcccttc
1141 cagtgggcat tgctaagctg gtacaatgca agcctgatgc tgtagtggac atccttccac
1201 catataaaga cagcctccct gggaaagcag agccatgaaa cggagaaaga cttcttacta
1261 aatccttaga gcactagatc cagcattttc catctgctat cgctgcatga caaaccacct
1321 taaaatgctt tggcttggcc aggtgcagtg gctcacgcct gtaatcccag cactttgtga
1381 ggcagaggtg agtggatcac ctgaggtcag gagttcgaga ccagcctgac caacatggtg
1441 aaaccccatc tctactaaaa atacaaaaat tagctgggag tggtaatggg cacctgtaat
1501 cccagctact caggaggcta aggcaggaga attgcttgaa cccgggaggc ggaggttgca
1561 gtgagccaag atcacgccac tgtactctag cctggatgac agagcgagac tccatctcaa
1621 aaaaaaaaaa aaaaaa//

- LOCUS: NM_024659 (formally NM_014118) 2459 bp mRNA linear PRI 17-NOV-2006
- DEFINITION: Homo sapiens glycosyltransferase-like domain containing 1 (GTDC1),transcript variant 2, mRNA.
- ACCESSION: NM_024659 NM_014118
- VERSION: NM_024659.2 GI:40254964

1 tcgacccacg cgtccggaca aggaggagga ggaggaggag gtctggctag ctgtccctaa
61 cgtcatttcc tggtctcaac tctttaagac cctgacatac tctgagattg gagtgaagat
121 ttctgtgaca tgctgggtat ctctgcatta gccagattgg acatatgtaa agggacatca
181 gtatatctgg atcatcagat tgtgcttcaa gaagaccata gggaagaacg cattttcatc
241 ttgctgcaaa agttgcaata gaagatctga aggtttgaat gggccaatga gtatcctcat
301 cattgaagca ttctatggag gctcccataa acagctggtg gatcttcttc aagaagagtt
361 aggagactgt gtcgtttata cccttcctgc aaagaaatgg cattggagag cccggacatc
421 tgctttatat ttctctcaga ccattcccat cagtgagcat tacaggaccc tctttgcaag
481 ttcagtgctt aacctgaccg aactggctgc ccttcggcct gaccttggga aactgaaaaa
541 gattctgtat tttcacgaga accagttgat atatcctgtc aagaaatgtc aggagaggga
601 tttccaatat ggatacaacc aaattctttc atgcctggtg gctgatgtgg ttgtattcaa
661 ctcagttttt aatatggaat catttctcac ttccatggga aaatttatga agctgattcc
721 tgatcacaga cccaaggatc tggaaagcat catcagaccc aagtgccaag ttatttactt
781 tcccatcagg tttcctgatg tgagcagatt catgcccaag cacaaaacaa cccatttaaa
841 gaagatgctc ggccttaaag gaaatggcgg tgcggttctg tccatggccc ttccttttca
901 gccagagcag agagattcag aggatttatt gaagaatttt aattcagagt gtgatacaca
961 ctgtggcctt gatactgcac gacaagaata tttgggtaac tcattaagac aggaatcaga
1021 cttgaaaaaa tccacctcgt cagataattc aagctctcat catggtgaaa ataaacaaaa
1081 tctgactgtt gatccctgtg acattttggg tggagttgat aatcagcaaa gactgctaca
1141 cattgtctgg cctcacaggt ggttggaagc tgtgtactgt gggtgttacc cactttgtcc
1201 taaagatttg gtttatcccg aaatatttcc agctgaatat ctgtattcta cacctgaaca
1261 gctttcaaaa aggctccaga atttctgcaa gagaccagat attataagaa aacatctcta
1321 taagggtgaa atcgctccgt tttcttgggc agccctacat ggtaaattca ggtctctgct
1381 tacaacagaa cctagggaag atttgtgaca gatggggcta agtcacaaac ttgcagccta
1441 aggcagaatc tgaagaactt tccagagtgt gcccatattt acctgatcag agagaaaaga
1501 aaatctgcag aggaagctga gcctggctgc ttgtcatagc tgacacagag ccatctgcca
1561 caaacctgtg gcggcttcag atctccaatc cctgccacca ccccaactca aattaaatac
1621 agattcctag agacgttatg atggttacac atgtcctcgg catcacatgt aggagactgt
1681 tcaaaaaaaa tatgtggcct gttgtataac cgcactcatg tatcccatat gtggtgccac
1741 attgaatttc cggttgaatc cgtttttatc ctttgtactg gatgacatgg tgcctgaatt
1801 ctttcttttc gccgacacga tggcagccaa actgcagctt caaacgctca cacttggctg
1861 ggtttctacc taggttgcca ggttatcatc ggagccttct tgtgtcctca aagggccacg
1921 aggcctgaaa ggaggatcag aatgctttgg gattaattgg gcagccatcg cagaattgtt
1981 tgtgggcaaa gggctgcttt agcacttttc ttttagcaaa ttaatgactc tcaggcacag
2041 ggggttttaa gtgaaggtat taataagagg tctggcaggt attcccatga ttcacagagt
2101 tacatttgca tttaattaat cttaaagttg caagataaac agctgtaatt cggacaaaca
2161 tgacaaacac agtgaagcca actatcccat aaaatgaaca ctgacatact tgttttaatt
2221 tttttcccag cgtaaaaata gaaaaatcaa aatactccta acaaaaccag tgattttgat
2281 agaaatattt ctccaatata cttgcatcca cctacaaata taaccttttc aagataaatc
2341 gcttatgatt tcaatagtca aactgctgtg tttgttgatg taaagatgtt ttgaatggct
2401 agatggtaaa ataaattctt aataaagtac ccactgcaat ttttaaaaaa aaaaaaaaa

- LOCUS: AF279891 2441 bp mRNA linear PRI 17-JAN-2003
- DEFINITION: Homo sapiens dead box protein 15 mRNA, complete cds.
- ACCESSION: AF279891
- VERSION: AF279891.1 GI:9624452

1 atgtccaagc ggcaccggtt ggacctaggg gaggattacc cctctggcaa gaagcgtgcg
61 gggaccgatg ggaaggatcg agatcgagac cgggatcgtg aagatcggtc taaagatcga
121 gaccgagaac gtgatagagg agatagagag cgagagaggg agaaagaaaa ggagaaggag
181 ttgcgagctt caacaaatgc tatgcttatc agtgctggat taccaccttt gaaagcttcc
241 cattcagctc actcaaccca ctcagcacat tcaacgcatt ctacacattc tgctcattca
301 acgcatgccg gacatgcagg tcacacgtca cttccacagt gcattaatcc gttcaccaac
361 ttaccccata ctcctcgata ctatgatatt ctaaagaaac gtcttcagct ccctgtttgg
421 gaatacaagg ataggtttac agatattctg gttagacatc agtcctttgt actggttggt
481 gagactgggt ctggtaaaac aacacagatt ccacagtggt gtgtggagta catgcgatca
541 ttaccaggac ccaagagagg agttgcctgt acccaaccca ggagagtggc tgcaatgagt
601 gtggctcaga gagttgctga tgagatggat gtgatgttgg gccaggaggt tggttactcc
661 attcgatttg aagactgcag tagtgcaaaa accattctta agtatatgac tgatgggatg
721 ttacttcgtg aagctatgaa tgatcccctc ctggagcgtt atggtgtaat aattcttgat
781 gaggctcatg agaggacact ggctacagat attctaatgg gtgttctgaa ggaagttgta
841 agacagagat cagatttaaa ggttatagtt atgagcgcta ctctagatgc aggaaaattc
901 cagatttact ttgataactg tcctctccta actattcctg ggcgtacaca tcctgttgag
961 atcttctata ctccagaacc agagagagat tatcttgaag cagcaattcg aacagttatc
1021 cagattcata tgtgtgaaga ggaagaggga gatcttcttc ttttcttaac tggtcaagag
1081 gaaattgatg aagcctgtaa gagaataaag cgtgaagttg atgatttggg ccctgaagtt
1141 ggtgacatta aaatcattcc attgtattct acacttccac ctcagcagca gcaacgcatt
1201 tttgagcctc cacctcccaa aaaacagaat ggagcaattg gaagaaaggt agttgtgtca
1261 actaacatag cagagacgtc tttgacaata gatggtgtgg tgtttgtgat tgatcctgga
1321 tttgcgaaac agaaggtcta caatcctcga atcagagttg agtccctttt ggtgacagct
1381 attagtaaag cttcagctca gcaaagggct ggtcgagctg gacgtaccag acctggaaaa
1441 tgcttcagac tttacacaga gaaagcttat aaaacagaaa tgcaggataa cacctatcct
1501 gagattttgc gttctaattt aggatcagtt gtgttacaat tgaagaaact tggtattgat
1561 gacttggtac attttgattt tatggatcca ccagctcctg aaactctgat gagagccctg
1621 gaacttttga attacctggc tgctttaaat gatgatggag atctgactga attgggatcc
1681 atgatggcag agtttcctct agatccacag ctcgcaaaaa tggttattgc aagttgtgac
1741 tacaactgtt ctaatgaggt cctatctatt actgctatgt tgtcagtccc acagtgtttt
1801 gttcgcccca cggaggccaa gaaagccgca gatgaggcca agatgagatt tgcccacata
1861 gatggagatc atctgacact gctgaacgtc taccatgctt ttaaacaaaa tcatgaatcg
1921 gttcagtggt gttatgacaa cttcattaac tacaggtccc tgatgtccgc agacaatgta
1981 cgccagcagc tatctcgaat tatggacaga tttaatttgc ctcgtcgaag tactgacttt
2041 acaagcaggg actattatat taatataaga aaagctttgg ttactgggta ttttatgcag
2101 gtggcacatt tagaacgaac agggcattac ttaactgtga aagataacca ggtggttcag
2161 ttgcatccct ctactgttct tgaccacaaa cctgaatggg tgctttataa tgagtttgtt
2221 ctaacaacaa agaattacat ccggacatgt acagatatca agccagaatg gttggtgaaa
2281 attgcccctc aatattatga catgagcaat ttcccacagt gtgaagcaaa gagacagttg
2341 gaccgcatca ttgccaaact tcaatccaag gaatattcac agtactgaat tcagtgctta
2401 gaactgaagt tattgagagg acagctttaa aagatgaatg a//

- LOCUS: NM_002372 5128 bp mRNA linear PRI 18-NOV-2006
- DEFINITION: Homo sapiens mannosidase, alpha, class 2A, member 1 (MAN2A1), mRNA.
- ACCESSION: NM_002372
- VERSION: NM_002372.2 GI:51477713

1 gcgggcggtg ctggagcgcc aagtggcgct ggagaaccgg cgcttccttt cgccgcttcc
61 gccgccatct ccgcgtttgt ggggcgggaa agagggaggg ggctagcggc tgcagctgga
121 gcgggcttct ctccggggac ggtcctttcc tccctgctct ccttttcctt ctttccgcgt
181 tgccgccgcc cgccccctgc gcctccccgc ggagcctggg tccgggaggg ggaaggtagg
241 ggcggcgggg ggcgggagag tctggcgagc ggacgctagc tctgaggaaa ctcatcaatc
301 cgtgagcccc ggagtccggg gtgcacatcg gcccagccgc agcgtcggcg gcggcggcgg
361 cagcagcacg aaggggctca gtcggggtag gcgggggcgg tgccggtgcc gcgggggcgg
421 gcccgaccgt cccgcccaga agttgtaggg cttggctcct cgcgatcttg ttcctttccc
481 ctccgcttct ctgacctagc tgcgcggccc cggcccggga gctgccgaac ccgcgcctcc
541 cctgggtgag gaggacacgc ctgccctcgt cgagaaaact tttcctgccg actcagttgg
601 ggcggcggtg gcaggaagtg cgggcagcga cctctcctcc gcctgccccg cgcgccctgc
661 cggaggtcgg cgctgagctt gcgatcaagt ttgtgggggc cccccttccc agttgccggc

721 gagtctcgcc tcgagagggg cgcccgaccc cggggagggc ggcaggccag ggcgaaggcc
781 aagggcgtgt ggtggcgccg gagactaggt gcggagcaag gcggggactc gcacccgcat
841 ccgagagcgc ggaggtcgcg cagcccggga gaagggagcc tccggcggct gcttcctaga
901 gtccacagtg cgctgtctcc tttggctgag gagagtgtcc tggccccgag tctatcgagg
961 aaaatgaagt taagccgcca gttcaccgtg ttcggcagtg cgatcttctg tgtggtgatt
1021 ttctcgctct acctgatgct ggaccggggt cacttagact accccaggaa cccgcgccgc
1081 gagggctcct tccctcaggg ccagctctca atgttgcaag aaaaaataga ccatttggag
1141 cgtttgctag ctgagaataa tgagatcatc tcaaatatta gagactcagt catcaatttg
1201 agtgagtctg tggaggatgg tccgaaaagt tcacaaagca atttcagcca aggtgctggc
1261 tcacatcttc tgccctcaca attatccctc tcagttgaca ctgcagactg tctgtttgct
1321 tcacaaagtg gaagtcacaa ttcagatgtg cagatgttgg atgtttacag tctaatttct
1381 tttgacaatc cagatggtgg agtttggaag caaggatttg acattactta tgaatctaat
1441 gaatgggaca ctgaacccct tcaagtcttt gtggtgcctc attcccataa cgacccaggt
1501 tggttgaaga ctttcaatga ctactttaga gacaagactc agtatatttt taataacatg
1561 gtcctaaagc tgaaagaaga ctcacggagg aagtttattt ggtctgagat ctcttacctt
1621 tcaaagtggt gggatattat agatattcag aagaaggatg ctgttaaaag tttaatagaa
1681 aatggtcagc ttgaaattgt gacaggtggc tgggttatgc ctgatgaagc tactccacat
1741 tattttgcct taattgatca actaattgaa ggacatcagt ggctggaaaa taatatagga
1801 gtgaaacctc ggtccggctg ggctattgat ccctttggac actcaccaac aatggcttat
1861 cttctaaacc gtgctggact ttctcacatg cttatccaga gagttcatta tgcagttaaa
1921 aaacactttg cactgcataa aacattggag tttttttgga gacagaattg ggatctggga
1981 tctgtcacag atattttatg ccacatgatg cccttctaca gctatgacat ccctcacact
2041 tgtggacctg atcctaaaat atgctgccag tttgatttta aacgtcttcc tggaggcaga
2101 tttggttgtc cctggggagt ccccccagaa acaatacatc ctggaaatgt ccaaagcagg
2161 gctcggatgc tactagatca gtaccgaaag aagtcaaagc tttttcgtac caaagttctc
2221 ctggctccac taggagatga tttccgctac tgtgaataca cggaatggga tttacagttt
2281 aagaattatc agcagctttt tgattatatg aattctcagt ccaagtttaa agttaagata
2341 cagtttggaa ctttatcaga tttttttgat gcgctggata aagcagatga aactcagaga
2401 gacaagggcc aatcgatgtt ccctgtttta agtggagatt ttttcactta tgccgatcga
2461 gatgatcatt actggagtgg ctattttaca tccagaccct tttacaaacg aatggacaga
2521 atcatggaat ctcatttaag ggctgctgaa attctttact atttcgccct gagacaagct
2581 cacaaataca agataaataa atttctctca tcatcacttt acacggcact gacagaagcc
2641 agaaggaatt tgggactgtt tcaacatcat gatgctatca caggaactgc aaaagactgg
2701 gtggttgtgg attatggtac cagacttttt cattcgttaa tggttttgga gaagataatt
2761 ggaaattctg catttcttct tattttgaag gacaaactca catacgactc ttactctcct
2821 gataccttcc tggagatgga tttgaaacaa aaatcacaag attctctgcc acaaaaaaat
2881 ataataaggc tgagtgcgga gccaaggtac cttgtggtct ataatccttt agaacaagac
2941 cgaatctcgt tggtctcagt ctatgtgagt tccccgacag tgcaagtgtt ctctgcttca
3001 ggaaaacctg tggaagttca agtcagcgca gtttgggata cagcaaatac tatttcagaa
3061 acagcctatg agatctcttt tcgagcacat ataccgccat tgggactgaa agtgtataag
3121 attttggaat cagcaagttc aaattcacat ttagctgatt atgtcttgta taagaataaa
3181 gtagaagata gcggaatttt caccataaag aatatgataa atactgaaga aggtataaca
3241 ctagagaact cctttgtttt acttcggttt gatcaaactg gacttatgaa gcaaatgatg
3301 actaaagaag atggtaaaca ccatgaagta aatgtgcaat tttcatggta tggaaccaca
3361 attaaaagag acaaaagtgg tgcctacctc ttcttacctg atggtaatgc caagccttat
3421 gtttacacaa caccgccctt tgtcagagtg acacatggaa ggatttattc ggaagtgact
3481 tgcttttttg accatgttac tcatagagtc cgactatacc acatacaggg aatagaagga
3541 cagtctgtgg aagtttccaa tattgtggac atccgaaaag tatataaccg tgagattgca
3601 atgaaaattt cttctgatat aaaaagccaa aatagatttt atactgacct aaatgggtac
3661 cagattcaac ctagaatgac actgagcaaa ttgcctcttc aagcaaatgt ctatcccatg
3721 accacaatgg cctatatcca ggatgccaaa catcgtttga cactgctctc tgctcagtca
3781 ttaggggttt cgagtttgaa tagtggtcag attgaagtta tcatggatcg aagactcatg
3841 caagatgata atcgtggcct tgagcaaggt atccaggata acaagattac agctaatcta
3901 tttcgaatac tactagaaaa aagaagtgct gttaatacgg aagaagaaaa gaagtcggtc
3961 agttatcctt ctctccttag ccacataact tcttctctca tgaatcatcc agtcattcca
4021 atggcaaata agttctcctc acctaccctt gagctgcaag gtgaattctc tccattacag
4081 tcatctttgc cttgtgacat tcatctggtt aatttgagaa caatacagtc aaaggtgggc
4141 aatgggcact ccaatgaggc agccttgatc ctccacagaa aagggtttga ttgtcggttc
4201 tctagcaaag gcacaggctg gttttgttct actactcagg gaaagatatt ggtacagaaa
4261 cttttaaaca agtttattgt cgaaagtctc acaccttcat cactatcctt gatgcattca
4321 cctcccggca ctcagaatat aagtgagatc aacttgagtc caatggaaat cagcacattc
4381 cgaatccagt tgaggtgaac ctgactttca catttggatt gagaatcatt ggcttttata
4441 cctttcttgg tttgacgtgc aataaagaag cacattattt tagcttctgg ctactgtgag

4501 aacatgaatt ctgtgattct gtgggttttt tcttttttct tttaccagta cagtaagaaa
4561 aaaaaaaaaa aaaaaaaagc catgctatca atcaagattc ttttttttta aactttctcc
4621 catgaactac caccatcagt atgaattgat gcaacaaatg aagaaatatt taaagacagc
4681 ctctcaacag attgtatctc aggttaaatg ctaactaatt atgtctgtgt tgggggttgc
4741 gaagagattc ttaaaagtat ctgtgtgttg atcatcagtt ttacaaaaac acctatttgg
4801 ctgaaatgga ataaaatgtt tgtgggtaaa agctaatggc caaaatggtt gcaatcattc
4861 atactagtta gaaaaattat gtgttgaaat aagtggaaaa gtgcaatcca tccaccctta
4921 tgattaacgt agatgatttt tatacctttt tctgatgtac ctcttgacct tctccttccc
4981 ttcctaccct ttctaagtat ttccagaaat acctgatttt gaatcattca acagtagaaa
5041 aagaggcata ttttcattac ttgacaatgt gggatgggtg caatttattc catcttcact
5101 aaaatagaag caattccata ggtaccat//

- LOCUS: NM_007362 2175 bp mRNA linear PRI 18-NOV-2006
- DEFINITION: Homo sapiens nuclear cap binding protein subunit 2, 20kDa (NCBP2), transcript variant 1, mRNA.
- ACCESSION: NM_007362
- VERSION: NM_007362.3 GI:110349728

1 agatgctcgc ctccgcgcgt ttccgcttcc gccatcgcgg acgcagcctc gtgccgggag
61 tcgccgcatt gtggtccgct tctctgcact atgtcgggtg gcctcctgaa ggcgctgcgc
121 agcgactcct acgtggagct gagccagtac cgggaccagc acttccgggg tgacaatgaa
181 gaacaagaaa aattactgaa gaaaagctgt acgttatatg ttggaaatct ttcttttac
241 acaactgaag aacaaatcta tgaactcttc agcaaaagtg gtgacataaa gaaaatcatt
301 atgggtctgg ataaaatgaa gaaaacagca tgtggattct gttttgtgga atattactca
361 cgcgcagatg cggaaaacgc catgcggtac ataaatggga cgcgtctgga tgaccgaatc
421 attcgcacag actgggacgc aggctttaag gagggcaggc aatacggccg tgggcgatct
481 gggggccagg ttcgggatga gtatcggcag gactacgatg ctgggagagg aggctatgga
541 aaactggcac agaaccagtg agtggtgaga gctctgtcag tgacaaacac tcctttggcc
601 tgttgaattt gctgaagaac atcacctaaa gtctgcacac gagcccattt ttaccaagat
661 ttgatcagtg tctttactga gctggaagcc tctgaaagtt attaaaggac agaatccaaa
721 agaatgcctt taattcttgt ctgagaatct tggccatgtg tcagattatc agaacaattt
781 tgttaccagg tcagaaattg tgttctttga caacagattg gatctgtaat gttgattagt
841 ctttagccat aaccactaca cttttagaaa gacagaaaaa tgtaagaatt tgtttttacc
901 ataatgagtc ttaagtaggt tcatgatcta cattggggcc tgggattatt tttttaattt
961 taagtttgca tgagatagcc taataaatgg aggtggggcc aggcatggtg gctcacacgt
1021 gtaatcccaa cactttggga ggctgaggag gaaggatagc ttgaggccag gagtttgaga
1081 ctagactggg caacatagca agaccccgtc tctacaaagc acaacgaaaa acaacaaatg
1141 gagttgtgct atgttgtatt gctttgcaca aaattaggaa caggtgtttg acaattgaat
1201 ttgttttctg tgaattctaa cctctaaagg catgcttaga ggtcaaggac cttcctgtgt
1261 agttggtgca aaagcaatct ccacaggaca gcactgcttc catgcttcat acatcaggaa
1321 atgaggccag aacttgagta tttactaaca cgtttttcaa aagatgtcag tgttatacct
1381 aaagctaaaa aaaagcaagg gtttgtcata gagggaacct ctaaataatt tcaggggtag
1441 gggagatgtt gtcaatagga aatgggataa aatatcaaga gacaatgaaa acactgcctt
1501 gacatgagga ccagcaagtt tattcttttc attttcagtg atgttgggaa tggactgggt
1561 tttaaaaggg agcttgaaga gggaatgttt gacagtcaca gaaggttcct gcagcagatg
1621 cctcttttag ccatttctca tttttttcct caaattttac ctactgaggc tcaagccttc
1681 acagtgagct gatggtctct acagggaggg gagtctaggg aatttatttg gtatttgtaa
1741 ggcaagaggt gatttctctc taatatatct gagttattgc tcatttaaaa ctgttaagtc
1801 cagtataatt ttccctgata tgaaaaaatg tgcatttttt tcacttagca acaaagtacc
1861 ttctaatttc caatagtccg tgaaagttgg ggctgaagta cctaagtgtg aatgtctctc
1921 ccgttaaact gagtgtagaa atctgaattt ttaaaagagc tgtaactagt tgtaagtgct
1981 taggaagaaa ctttgcaaac atttaatgag gatacactgt tcatttttaa aattccttca
2041 cactgtaatt taatgtgttt tatattcttt tgtagtaaaa caacataact cagatttcta
2101 caggagacag tggttttatt tggattgtct tctgtaatag gtttcaataa agctggatga
2161 acttatcgtt aaaaa//

- LOCUS: NM_022163 1015 bp mRNA linear PRI 17-NOV-2006
- DEFINITION: Homo sapiens mitochondrial ribosomal protein L46 (MRPL46),
- nuclear: gene encoding mitochondrial protein, mRNA.
- ACCESSION: NM_022163
- VERSION: NM_022163.2 GI:26667176

1 gagaacgaaa gatggcggcg cccgtaaggc ggacgctgtt aggggtggcg gggggttggc

61 ggcggttcga gaggctctgg gccggcagtc taagctctcg cagcctggct cttgcagccg
121 caccctcaag caacggatcc ccatggcgct tgttgggcgc gttgtgcctg cagcggccac
181 ctgtagtctc caagccgttg accccattgc aggaagagat ggcgtctcta ctgcagcaga
241 ttgagataga gagaagcctg tattcagacc acgagcttcg tgctctggat gaaaaccagc
301 gactggcaaa gaagaaagct gaccttcatg atgaagaaga tgaacaggat atattgctgg
361 cgcaagattt ggaagatatg tgggagcaga aatttctaca gttcaaactt ggagctcgca
421 taacagaagc tgatgaaaag aatgaccgaa catccctgaa caggaagcta gacaggaacc
481 ttgtcctgtt agtcagagag aagtttggag accaggatgt ttggatactg ccccaggcag
541 agtggcagcc tggggagacc cttcgaggaa cagctgaacg aaccctggcc acactctcag
601 aaaacaacat ggaagccaag ttcctaggaa atgcaccctg tgggcactac acattcaagt
661 tcccccaggc aatgcggaca gagagtaacc tcggagccaa ggtgttcttc ttcaaagcac
721 tgctattaac tggagacttt tcccaggctg ggaataaggg ccatcatgtg tgggtcacta
781 aggatgagct gggtgactat ttgaaaccaa aatacctggc ccaagttagg aggtttgttt
841 cagacctctg atgggccgag ctgcctgtgg acggtgctca gacaagtctg ggattagagc
901 ctcaaggaca ttgtgtgatt gcctcacatt tgcaggtaat atcaagcagc aaactaaatt
961 ctgagaaata aacgagtcta ttactaaaaa aaaaaaaaaa aaaaaaaaaa aaaaa//

- LOCUS: NM_005826 2663 bp mRNA linear PRI 17-NOV-2006
- DEFINITION: Homo sapiens heterogeneous nuclear ribonucleoprotein R (HNRPR), mRNA.
- ACCESSION: NM_005826
- VERSION: NM_005826.2 GI:14141188

1 cgcgcgcgcc atttctagtc gttttcaaag cgcctcgcgc tgattctcac gggcccggct
61 gccggccccc gctctgccct gcataataaa atggctaatc aggtgaatgg taatgcggta
121 cagttaaaag aagaggaaga accaatggat acttccagtg taactcacac agaacactac
181 aagacactga tagaggcagg cctcccacag aaggtggcag aaagacttga tgaaatattt
241 cagacaggat tggtagctta tgtcgatctt gatgaaagag caattgatgc tctcagggaa
301 tttaatgaag aaggagctct gtctgtacta cagcagttca aggaaagtga cttatcacat
361 gttcagaaca aaagtgcatt tttatgtgga gttatgaaga cctacaggca gagagagaaa
421 caggggagca aggtgcaaga gtccacaaag ggacctgatg aagcgaagat caaggccttg
481 cttgagagaa ctggttatac tctggatgta accacaggac agaggaagta tggtggtcct
541 ccaccagaca gtgtgtactc tggcgtgcaa cctggaattg gaacggaggt atttgtaggc
601 aaaataccaa gggatttata tgaggatgag ttggtgcccc tttttgagaa ggccggaccc
661 atttgggatc tacgtcttat gatggatcca ctgtccggtc agaatagagg gtatgcattt
721 atcaccttct gtggaaagga agctgcacag gaagccgtga aactgtgtga cagctatgaa
781 attcgccctg gtaaacacct tggagtgtgc atttctgtgg caaacaacag actttttgtt
841 ggatccattc cgaagaataa gactaaagaa aacattttgg aagaattcag taaagtcaca
901 gagggtttgg tggacgttat tctctatcat caacccgatg acaaaaagaa gaatcggggg
961 ttctgcttcc ttgaatatga ggatcacaag tcagcagcac aagccagacg ccggctgatg
1021 agtggaaaag taaaagtgtg gggaaatgta gttacagttg aatgggctga ccctgtggaa
1081 gaaccagatc cagaagtcat ggctaaggta aaagttttgt ttgtgagaaa cttggctact
1141 acggtgacag aagaaatatt ggaaaagtca ttttctgaat ttggaaaact cgaaagagta
1201 aagaagttga aagattatgc atttgttcat tttgaagaca gaggagcagc tgttaaggct
1261 atggatgaaa tgaatggcaa agaaatagaa ggggaagaaa ttgaaatagt cttagccaag
1321 ccaccagaca agaaaaggaa agagcgccaa gctgctagac aggcctccag aagcactgcg
1381 tatgaagatt attactacca ccctcctcct cgcatgccac ctccaattag aggtcggggt
1441 cgtggtgggg ggagaggtgg atatggctac cctccagatt actacggcta tgaagattac
1501 tatgatgatt actatggtta tgattatcac gactatcgtg gaggctatga agatccctac
1561 tacggctatg atgatggcta tgcagtaaga ggaagaggag gaggaagggg agggcgaggt
1621 gctccaccac caccaagggg gaggggagca ccacctccaa gaggtagagc tggctattca
1681 cagagggggg cacctttggg accaccaaga ggctctaggg gtggcagagg gggtcctgct
1741 caacagcaga gaggccgtgg ttcccgtgga tctcggggca atcgtggggg caatgtagga
1801 ggcaagagaa aggcagatgg gtacaaccag cctgattcca agcgtcgtca gaccaacaac
1861 caacagaact ggggttccca acccatcgct cagcagccgc ttcagcaagg tggtgactat
1921 tctggtaact atggttacaa taatgacaac caggaatttt atcaggatac ttatgggcaa
1981 cagtggaagt agacaagtaa gggcttgaaa atgatactgg caagatacga ttggctctag
2041 atctacattc ttcaaaaaaa aaaattggct taactgtttc atctttaagt agcattttgc
2101 tgccatttgt attgggctga agaaatcact attgtgtata tactcaagtc tttttatttt
2161 tcctcttttc ataaatgctc ttggacatta ttgggcttgc agagttccct tattctgggg
2221 attacaatgc ttttatcgtt tcaggcttca ttttagcttc aaaacaagct gggcacactg
2281 ttaaatcatg attttgcaga acctttggtt ttggacagtt tcattttttt ggatttggga
2341 tagattacat aggagtatgg agtatgctgt aaataaaaat acaagctagt gctttgtctt

2401 agtagtttta agaaattaaa gcaaacaaat ttaagttttc ttgtattgaa aataacctat
2461 gattgtatgt tttgcattcc tagaagtagg ttaactgtgt ttttaaattg ttataacttc
2521 acaccttttt gaaatctgcc ctacaaaatt tgtttggctt aaacgtcaaa agccgtgaca
2581 atttgttctt tgatgtgatt gtatttccaa tttcttgttc atgtaagatt tcaataaaac
2641 taaaaaatct attcaaaaca tta//

- LOCUS: NM_001029 699 bp mRNA linear PRI 18-NOV-2006
- DEFINITION: Homo sapiens ribosomal protein S26 (RPS26), mRNA.
- ACCESSION: NM_001029
- VERSION: NM_001029.3 GI:71559137

1 ggagacacat aacctcgatt ttcttccgcc atccggctaa atagtcccat gtgcactttg
61 ttccatggat aaataaacac taggaacgca tttccaccct agatttcagc agaaatgctg
121 aatgtaaagg aatatttgag taaagtgagt tgccgttctt gaagcccgtc tcctaaggat
181 tctcccggtg tccgcgtagg gatctcatgc tatataggag ggccctgcca ggcaccgtct
241 cctctctccg gtccgtgcct ccaagatgac aaagaaaaga aggaacaatg gtcgtgccaa
301 aaagggccgc ggccacgtgc agcctattcg ctgcactaac tgtgcccgat gcgtgcccaa
361 ggacaaggcc attaagaaat tcgtcattcg aaacatagtg gaggccgcag cagtcaggga
421 catttctgaa gcgagcgtct tcgatgccta tgtgcttccc aagctgtatg tgaagctaca
481 ttactgtgtg agttgtgcaa ttcacagcaa agtagtcagg aatcgatctc gtgaagcccg
541 caaggaccga acacccccac cccgatttag acctgcgggt gctgccccac gtcccccacc
601 aaagcccatg taaggagctg agttcttaaa gactgaagac aggctattct ctggagaaaa
661 ataaaatgga aattgtactt aaaaaaaaaa aaaaaaaaa//

- LOCUS: NM_001357 4308 bp mRNA linear PRI 18-NOV-2006
- DEFINITION: Homo sapiens DEAH (Asp-Glu-Ala-His) box polypeptide 9 (DHX9), mRNA.
- ACCESSION: NM_001357
- VERSION: NM_001357.3 GI:100913205

1 gctggccccg ccccctagcg ccgcggtcgg agccatttcg ccgattcctc catgcgagtt
61 gctgtgcgtt tctctgttgt ctcggtagaa ggccagagtc acacacggtc ctaagagctg
121 ggcaccagga agcgaaggct gatctgaaga agacacttga atcatgggtg acgttaaaaa
181 ttttctgtat gcctggtgtg gcaaaaggaa gatgacccca tcctatgaaa ttagagcagt
241 ggggaacaaa aacaggcaga aattcatgtg tgaggttcag gtggaaggtt ataattacac
301 tggcatggga aattccacca ataaaaaaga tgcacaaagc aatgctgcca gagactttgt
361 taactatttg gttcgaataa atgaaataaa gagtgaagaa gttccagctt ttggggtagc
421 atctccgccc ccacttactg atactcctga cactacagca aatgctgaag gagatttacc
481 aacaaccatg ggaggacctc ttcctccaca tctggctctc aaagcagaaa ataattctga
541 ggtaggggcc tctggctatg gtgttcctgg gcccacctgg gaccgaggag ccaacttgaa
601 ggattactac tcaagaaagg aagaacaaga agtgcaagcg actctagaat cagaagaagt
661 ggatttaaat gctgggcttc atggaaactg gaccttggaa aatgctaaag ctcgtctaaa
721 ccaatatttt cagaaagaaa agatccaagg agaatataag tacacccaag tgggtcctga
781 tcacaacagg agctttattg cagaaatgac catttatatc aagcagctgg gcagaaggat
841 ttttgcacga gaacatggat caaataagaa attggcagca cagtcctgtg ccctgtcact
901 tgtcagacaa ctgtaccatc ttggagtggt tgaagcttac tccggactta caaagaagaa
961 ggaaggagag acagtggagc cttacaaagt aaacctctct caagatttag agcatcagct
1021 gcaaaacatc attcaagagc taaatcttga gattttgccc ccgcctgaag atccttctgt
1081 gccagttgca ctcaacattg gcaaattggc tcagttcgaa ccatctcagc gacaaaacca
1141 agtgggtgtg gttccttggt cacctccaca atccaactgg aatccttgga ctagtagcaa
1201 cattgatgag gggcctctgg cttttgctac tccagagcaa ataagcatgg acctcaagaa
1261 tgaattgatg taccagttgg aacaggatca tgatttgcaa gcaatcttgc aggagagaga
1321 gttactgcct gtgaagaaat ttgaaagtga gattctggaa gcaatcagcc aaaattcagt
1381 tgtcattatt agaggggcta ctggatgtgg gaaaaccaca caggttcccc agttcattct
1441 agatgacttt atccagaatg accgagcagc agagtgtaac atcgtagtaa ctcagcccag
1501 aagaatcagt gcggtttctg tggcagagcg agttgcattt gaaagaggag aagagcctgg
1561 aaaaagctgt ggctacagcg ttcgatttga gtctatactt cctcgtcctc atgccagtat
1621 aatgttttgt actgtaggtg tgctcctgag aaaattagaa gcaggcattc gaggaatcag
1681 tcatgtaatt gtagatgaaa tacatgaaag agatattaat actgacttcc ttttggtagt
1741 actgcgtgat gttgttcagg cttatcctga agttcgcatt gttcttatgt ctgctactat
1801 tgataccagc atgttttgtg aatatttctt caattgcccc atcattgaag tttatgggag
1861 gacttaccca gttcaagaat attttctgga agactgcatt cagatgaccc actttgttcc
1921 tccaccaaaa gacaaaaaga agaaggataa ggatgatgat ggtggtgagg atgatgatgc
1981 aaattgcaac ttgatctgtg gtgatgaata tggtccagaa acaaggttga gcatgtctca

2041 attgaacgaa aaggaaactc cttttgaact catcgaggct ctacttaagt acattgaaac
2101 ccttaatgtt cctggagctg tgttggtttt tttgcctggc tggaatctga tttatactat
2161 gcagaagcat ttggaaatga atccacattt tggaagccat cggtatcaga ttctacccct
2221 gcattctcag attcctcgag aggaacagcg caaagtgttt gatccagtac cagttggagt
2281 aaccaaggtt attttgtcca caaatattgc tgaaacaagc attaccataa acgatgttgt
2341 ttatgtcatt gactcctgca agcagaaagt gaaactcttc actgctcaca acaatatgac
2401 caactatgct accgtatggg catcaaaaac aaaccttgag caacggaaag ggcgagctgg
2461 ccgagtacgg cctggattct gctttcacct gtgcagccga gctcgttttg agagacttga
2521 aacccacatg acaccagaga tgttccgaac accattgcat gaaattgctc ttagcataaa
2581 acttctgcgt ctaggaggaa ttggccaatt tctggccaaa gcaattgaac ctcccccttt
2641 ggatgctgtg attgaagcag aacacactct tagagagctt gatgcattag atgccaatga
2701 tgagttgact cctttgggac gaatcctggc taaactcccc attgagcctc gttttggcaa
2761 aatgatgata atggggtgta ttttctacgt gggagatgct atctgtacca ttgctgctgc
2821 tacctgcttt ccagagcctt tcatcaatga aggaaagcgg ctgggctata tccatcgaaa
2881 ttttgctgga aacagatttt ctgatcacgt agccctttta tcagtattcc aagcctggga
2941 tgatgctaga atgggtggag aagaagcaga gatacgtttt tgtgagcaca aaagacttaa
3001 tatggctaca ctaagaatga cctgggaagc caaagttcag ctcaaagaga ttttgattaa
3061 ttctgggttt ccagaagatt gtttgttgac acaagtgttt actaacactg gaccagataa
3121 taatttggat gttgttatct ccctcctggc ctttggtgtg taccccaatg tatgctatca
3181 taaggaaaag aggaagattc tcaccactga agggcgtaat gcacttatcc acaaatcatc
3241 tgttaattgt ccttttagta gccaagacat gaagtaccca tctcccttct ttgtatttgg
3301 tgaaaagatt cgaactcgag ccatctctgc taaaggcatg actttagtca ccccctgca
3361 gttgcttctc tttgcctcca agaaagtcca atctgatggg cagattgtgc ttgtagatga
3421 ctggattaaa ctgcaaatat ctcatgaagc tgctgcctgt atcactggtc tccgggcagc
3481 catggaggct ttggttgttg aagtaaccaa acaacctgct atcatcagcc agttggaccc
3541 cgtaaatgaa cgtatgctga acatgatccg tcagatctct agaccctcag ctgctggtat
3601 caaccttatg attggcagta cacggtatgg agatggtcca cgtcctccca agatggcccg
3661 atacgacaat ggaagcggat atagaagggg aggttctagt tacagtggtg gaggctatgg
3721 cggtggctat agcagtggag gctatggtag cggaggctat ggtggcagcg ccaactcctt
3781 tcgggcagga tatggtgcag gtgttggtgg aggctataga ggagtttccc gaggtggctt
3841 tagaggcaac tctggaggag actacagagg gcctagtgga ggctacagag gatctggggg
3901 attccagcga ggaggtggta gggggcccta tggaactggc tactttggac agggaagagg
3961 aggtggcggc tattaaaact tggttatgtc agttcctgtg tgtagacagt aaggaaaaaa
4021 aggcatgcta tgtgttacgt gtttttccca gtatgtttat ttgccaccaa aaagtaaatg
4081 cattttcacc cattctgtgg ttcattgtag tttaaggaaa ccaagcatat agatgcatta
4141 gtgattttgt ttatattatg taaaatataa cgatctctta aaaataccac agtttgtatt
4201 ttttctttaa ggagtaaaga tttgccttta aataacttgg tattttcctg gctttcgttt
4261 aatacaatag aaaataaagt attacaccga aaaaaaaaaa aaaaaaa//

- LOCUS: NM_020191 1155 bp mRNA linear PRI 17-NOV-2006
- DEFINITION: Homo sapiens mitochondrial ribosomal protein S22 (MRPS22), nuclear gene encoding mitochondrial protein, mRNA.
- ACCESSION: NM_020191
- VERSION: NM_020191.2 GI:16554602

1 tgataatcat ggcgcccctc ggaacaactg tattgctgtg gagcctcttg aggagttctc
61 cgggcgtgga acgggtctgt ttccgggctc gaatccagcc ctggcacggt ggcctgctcc
121 aaccgctacc ttgctctttc gagatggggc tgccacgccg ccggttcagc tccgaggccg
181 cagaatctgg tagcccagag accaagaaac ctacatttat ggatgaggaa gttcaaagca
241 tactcacgaa aatgacaggc ttgaacttgc agaagacttt taagccagct atacaagaac
301 tgaagccacc aacctataag ctaatgactc aggcacagtt ggaagaggct acaagacagg
361 cagttgaggc agctaaagta cgattaaaaa tgccaccagt tctggaagag cgagtaccaa
421 taaatgatgt gttagctgaa gataagattt tggaaggaac agaaacaacc aaatatgtgt
481 ttactgatat atcatatagc ataccacacc gggagcgttt tattgtcgtc agagaaccaa
541 gtggcacact acgcaaagcc tcttgggaag aacggaccga aatgatacaa gtttatttcc
601 caaaagaagg tcgtaaaatt ttgacaccaa taattttcaa ggaagaaaat cttaggacta
661 tgtatagcca ggacaggcat gttgatgtcc tcaatctctg ctttgcccag tttgagccag
721 attccacaga gtatatcaag gttcatcaca agacctatga agatatagat aaacgtggaa
781 aatatgacct tttacgttca acaagatact ttggtggaat ggtgtggtat tttgtaaata
841 ataaaaagat tgatggtttg ctgattgacc agattcagag agatttaatc gatgatgcaa
901 ccaacttggt ccagctgtat cacgtgctcc atccagatgg ccagtcggct caaggggcca
961 aggatcaggc tgctgaggga ataaatttaa tcaaggtctt tgcaaaaaca gaagcacaga
1021 agggagccta tatagaacta acactgcaga cttatcaaga agcactcagt cgccattctg

1081 cagcttccta aaaatatttt aaaaatacat ttattttact aaaaaaaaaa aaaaaaaaaa
1141 aaaaaaaaaa aaaaa//

- LOCUS: NM_013417 4510 bp mRNA linear PRI 18-NOV-2006
- DEFINITION: Homo sapiens isoleucine-tRNA synthetase (IARS), transcript variant long, mRNA.
- ACCESSION: NM_013417
- VERSION: NM_013417.2 GI:94721238

1 cggttgcagc gtggacgccg gatgagttgc ttttaggctt gctggcccgc ggggctgtcc
61 aggcacgcga ggcccctcag gtacgccctc tcttccctgc aggatccggc cctcaaagac
121 gagggtcacg cacgcgttac aaccccgaaa cagtagcaca agatttaatt tttaaaagag
181 cgtgtttctt cggggcttgc cgttcgttcg tttccagcct caggaattta tggtcgcctt
241 tttgaatgag caacaaaatg cttcaacaag ttccagaaaa cataaatttt cctgctgaag
301 aagagaaaat cttggagttt tggactgaat ttaattgttt tcaggaatgc ttaaagcaat
361 caaaacataa accaaaattt accttctatg atggtcctcc ttttgcaact ggactgcctc
421 actatggaca tatacttgcg ggtacaatta aagatatagt tacaagatat gctcaccaga
481 gtgggtttca tgttgacaga agatttggat gggattgcca tggcttacct gtggaatatg
541 aaattgataa gacactggga atcagaggac cagaggatgt ggccaaaatg gggattacag
601 agtataacaa tcagtgccga gcaattgtga tgagatattc tgctgagtgg aagtctactg
661 ttagcagact tggccgatgg attgactttg acaatgacta taaaactctg tatccacaat
721 tcatggaatc agtctggtgg gtcttcaaac aactctatga taaaggcctt gtttatagag
781 gtgtgaaagt catgcccttc tctacggcat gtaacactcc actttccaac ttcgagtcac
841 accagaatta taaggatgtt caagatcctt cagtatttgt aactttccct ttggaagaag
901 atgaaactgt atctttagtt gcttggacaa ccactccctg gactctacct agtaaccttg
961 ctgtgtgtgt taatccagaa atgcaatatg tgaaaattaa agatgttgcc agaggacgat
1021 tactcatttt aatggaagcc agattgtcag ccctctataa attggagagt gactatgaga
1081 tccttgaaag atttcctggt gcctatctta aaggcaagaa gtacaggccc ctgtttgact
1141 atttcctgaa gtgtaaagag aatggcgctt tcactgtgct tgttgacaac tatgtgaagg
1201 aagaagaagg cacaggggtt gtccaccaag ctccttactt cggtgctgag gactatcggg
1261 tctgtatgga ctttaacatt attcggaaag actcactccc tgtttgccct gtggatgctt
1321 caggctgctt cacaacggag gtgacagatt tcgcaggaca gtatgtgaag gatgctgaca
1381 aaagtatcat caggactttg aaggaacaag gccgacttct ggttgccacc accttcactc
1441 acagctaccc tttttgctgg agatcagaca ctcctctaat ttacaaagca gtgcccagct
1501 ggtttgtgcg agtggagaac atggtggacc agctcctaag gaacaatgac ctgtgctact
1561 gggtcccaga gttggtacga gaaaaacgat ttggaaattg gctgaaagat gcacgtgact
1621 ggacaatttc cagaaacaga tactggggca cccccatccc actgtgggtc agcgatgact
1681 ttgaggaggt ggtatgcatt gggtcagtgg cggaacttga agaactgtca ggagcaaaga
1741 tctcagatct ccacagagag agtgttgacc acctgaccat tccttcacgc tgtgggaagg
1801 gatccttgca ccgcatctct gaagtgtttg actgttggtt tgagagtggc agcatgccct
1861 atgctcaggt tcattacccg tttgaaaaca agagggagtt tgaggatgct tttcctgcag
1921 atttcattgc cgagggcatc gaccaaacca gaggatggtt ttataccctg ctggtgctgg
1981 ccacggccct ctttggacaa ccgcctttca agaacgtaat tgtgaatggg cttgtcctgg
2041 caagtgatgg ccaaaaaatg agcaaacgga aaaagaatta tccagatcca gtttccatca
2101 tccagaagta tggtgctgat gccctcagat tatatctgat taactcccct gtggtgagag
2161 cagaaaacct ccgctttaaa gaagagggtg tgcgggacgt ccttaaggat gtactgctcc
2221 catggtacaa tgcctatcgc ttcttaatcc agaacgttct gaggctccag aaggaggaag
2281 aaatagaatt tctctacaat gagaacacgg ttagagaaag ccccaacatt acagaccggt
2341 ggatcctgtc cttcatgcag tctctcattg gcttctttga gactgaaatg gcagcttata
2401 ggctttatac tgtggtgcct cgcctggtca agtttgtaga tattctgacc aattggtatg
2461 ttagaatgaa ccgcagaaga ttaaagggtg aaaatgggat ggaggattgt gtcatggccc
2521 tagaaacctt gtttagtgtt ctgctttctc tttgcagact tatggctccc tacacacctt
2581 ttctcactga attgatgtac cagaatctaa aggtgctgat tgaccctgtt tctgttcagg
2641 acaaggacac actcagcatt cactacctca tgctgccccg tgttcgagaa gaattgattg
2701 acaagaaaac agagagtgca gtatctcaga tgcagtctgt gattgaactt ggaagagtga
2761 tcagagaccg aaaaactatt cccataaagt atcctttgaa agaaattgtg gttatccatc
2821 aagatccaga agctcttaaa gatatcaagt ctttggagaa gtatatcatt gaggaactca
2881 atgttcgaaa agttacactg tctacagata aaaacaagta tggcattcgg ctaagggcag
2941 aaccagatca catggtcctg gggaagcgtc tgaagggagc ctttaaggca gtgatgacgt
3001 ccatcaagca gttgagcagt gaggagctgg agcagttcca gaagactggg accattgttg
3061 tggaaggcca tgaattgcac gatgaagaca tccgcctcat gtacaccttt gatcaggcca
3121 caggtgggac tgcgcaattt gaagcacact cagatgctca ggctttggtc ctcttagatg
3181 tcactcctga ccagtcaatg gtagatgaag gaatggctcg ggaagtcatc aatcgcatac
3241 agaaacttcg caaaaagtgc aatctggttc caactgatga aatcacagtg tactataaag

3301 caaagtctga aggaacatat ctgaatagtg ttattgaaag ccacacagag ttcatattta
3361 ccaccataaa ggctcccttg aaaccatatc cagtttctcc atcggataaa gtccttattc
3421 aagaaaaaac acagttgaag ggatctgaac tggaaattac actcaccaga ggatcttccc
3481 ttcctggtcc tgcttgtgca tatgtcaatc ttaacatttg tgcaaatggc agtgaacaag
3541 gtggagtatt gctcctggaa aatccaaaag gtgacaatag gttggacctt ttaaagctga
3601 agagtgttgt cactagcatt tttggtgtga aaaatacaga gctggctgtc ttccatgatg
3661 aaacagaaat acaaaaccaa actgacttac tgagtcttag tggaaaaaca ctttgtgtga
3721 ctgcaggatc ggctccctct ctgatcaaca gttctagtac tcttctttgt cagtatatca
3781 acctacagct cctgaatgca aagccacaag agtgtttaat ggggacagtg ggcactctcc
3841 tgcttgaaaa cccacttggg cagaatggac tcacccacca aggtcttctg tatgaagcag
3901 ccaaggtgtt tggccttcgg agcaggaagc taaagctgtt tctgaatgag acccaaacgc
3961 aggaaattac agaagacatc cccgtgaaga ctttgaatat gaagactgtg tatgtttctg
4021 tgttaccaac aacagcagac ttctagcatg tacttatcaa tgttgttcgg tcagcccttc
4081 cctaattaca cctatcccct acacatacat gcacatagac acacacatga acacactgaa
4141 gatatttcct tcaggtgtgt gtaaaatatg ctgcttggat tgaaattcaa atgggattga
4201 ttagtcaagt aacttgagac ctcacagtaa tcttcacact taaccttaga cacctatgca
4261 gtcatgttgg gagcaggtta caatgttact tcagcccaca gtttatttct atacttgagt
4321 tcttaagtac agaagataga agtgatttaa atggcatagt atatatatca ttttctggcc
4381 ttttaaaatt tatttgagac ctcttgatga aatggacata ttatatattt ctgccacctg
4441 gattttcctg gataatttga tggaatattt taagtttcag taaatcagaa caataaacaa
4501 actcagatat//

- LOCUS: HSU37689 867 bp mRNA linear PRI 07-MAR-1996
- DEFINITION: Human RNA polymerase II subunit (hsRPB8) mRNA, complete cds.
- ACCESSION: U37689
- VERSION: U37689.1 GI:1017822

1 ccctcactaa agggacaaaa gctggagctc caccgcggtg cggccgctct agaactagtg
61 gatcccccgg gctgcaggtc cggtcttgtc cacgctaggg ggtgcacgta ctcccaactg
121 tggtcgcgct ctcacccctt ctgctgctct cgtggccccc tcgcgatggc gggcatcctg
181 tttgaggata ttttcgatgt gaaggatatt gacccggagg ccaagaagtt tgaccgagtg
241 tctcgactgc attgtgagag tgaatctttc aagatggatc taatcttaga tgtaaacatt
301 caaatttacc ctgtagactt gggtgacaag tttcggttgg tcatagctag taccttgtat
361 gaagatggta ccctggatga tggtgaatac aaccccactg atgataggcc ttccagggct
421 gaccagtttg agtatgtaat gtatggaaaa gtgtacagga ttgagggaga tgaaacttct
481 actgaagcag caacacgcct ctctgcgtac gtgtcctatg ggggcctgct catgaggctg
541 cagggggatg ccaacaacct gcatggattc gaggtggact ccagagttta tctcctgatg
601 aagaagctag ccttctgaac ctcgcctgaa gccagcctct ctgccaagtc actcaggtca
661 tgggcattgt tcaagcctga gtggcagccg ctcttgctca cctgttgagg aagggctggc
721 tcactgtcca ccgtggcggc atctttaact ggcctccact caatgggaaa ctgactcgcc
781 tgtgaaagac acagtgggag agctgaaaat gaatcagaag ctttatgtat atgattttta
841 aattaaactt tactttttca gactgcc//

- LOCUS: NM_012317 1388 bp mRNA linear PRI 17-NOV-2006
- DEFINITION: Homo sapiens leucine zipper, down-regulated in cancer 1 (LDOC1),mRNA.
- ACCESSION: NM_012317
- VERSION: NM_012317.2 GI:22035627

1 ctggccacca agcgcctccg tggacacgca cttcctgcga ggcctccgtg cgcaccttgg
61 ccgagccgaa ccgagccgag tcctgtcctt ccaggccgtt cgcaatggtg gatgagttgg
121 tgctgctgct gcacgcgctc ctgatgcggc accgcgccct gagcatcgag aacagccagc
181 tcatggaaca gctgcggctg ctggtgtgcg agagggccag cctgctgcgc caggtacgtc
241 cgccgagctg cccggtgccc ttccccgaaa cgtttaatgg cgagagctcc cggctccccg
301 agtttatcgt gcagacggcg tcttacatgc tcgtgaacga gaaccgattc tgcaacgacg
361 ccatgaaggt ggcattccta atcagcctcc tcaccgggga agccgaggag tgggtggtgc
421 cctacatcga gatggatagc cccatcctag gtgattaccg ggccttcctc gatgagatga
481 aacagtgctt tggctgggat gacgacgaag acgacgacga cgaagaagag gaggatgatt
541 attaggccct cgaccctcgg gcctcggggg ggagggccct gcacgccgcc accccctccc
601 cgcagccctc accccgccag gagccactgc tctccccctt gccctccggt ccccttacct
661 acccgcgccc gtctgctctc tctcttcatt tctccgtagt gcttgtcttt gttccaggaa
721 tagcgctcca gttacctgct gctggggtcg gggctggagc ctcactcact cggaagtgct
781 tggaagtgtc atctaccctg gccatccccg ggatccctcc cctgctaatc tgaactgtgt

841 cctgaacccc agctattggc caggcccctg ttacaaactg cgcagaccac ccatagccct
901 gctgctgcca cctgccctgc tgccaggctt acatcttccc agagaactat gctgccactt
961 cacatccact caccacatcc cacctacaga ctaccacctt tcgagatcag gaccaacctg
1021 gaaaatgcca gagttggcta tgccttttca gacattgatt tggacagctc accaacccct
1081 gaaggggccg ttcagcctgg ttagttttct acctactccg agtgtcctcc cctgccccac
1141 cagattgctg caggggcgcg gtgtgcctgg cagccaaatt gttgacactt cttttttcct
1201 atgcactggt tttacacagc tgtcattttt ctttcaaaat tgcagcagtc ccacagatgt
1261 gtgcatttgg acaaatagta cttaaaaaca aaacaaacaa gcactcagcc cagctcctca
1321 atactacctg gaaaaagcat tggcattatt ttcaataaat atcaagcact aaaaaaagaa
1381 aaaaaaa//

- LOCUS: NM_002967 3018 bp mRNA linear PRI 17-NOV-2006
- DEFINITION: Homo sapiens scaffold attachment factor B (SAFB), mRNA.
- ACCESSION: NM_002967
- VERSION: NM_002967.2 GI:21264342

1 ataaaaccgg cccggttctg tggaaagtgg gcggcggagc cagggtccct ggaatggcgg
61 agactctgtc aggcctaggt gattctggag cggcgggcgc ggcggctctg agctccgcct
121 cgtcagagac cgggacgcgg cgcctcagcg acctgcgagt gatcgatctg cgggcggagc
181 tgaggaaacg gaatgtggac tcgagcggca acaagagcgt tttgatggag cggctgaaga
241 aggcaattga agatgaaggt ggtaatcctg acgaaattga aattacctcc gagggaaaca
301 agaaaacatc aaagaggtct agcaaagggc gcaaaccaga agaagagggt gtggaagata
361 acgggctgga ggaaaactct ggggatggac aggaggatgt tgagaccagt ctggagaact
421 tgcaggacat cgacatcatg gatatcagtg tgttggatga agcagaaatt gataatggaa
481 gcgttgcaga ttgtgtcgaa gacgatgatg ctgataacct ccaggagtcc ctgtcggata
541 gtagagagct agtcgagggg gaaatgaaag agcttccgga gcagcttcag gaacatgcta
601 tagaggacaa agaaactata aacaatttag atacttcatc atctgacttc actatattac
661 aggaaattga agagccatcc ctggagccag aaaatgagaa aatactcgac attttggggg
721 aaacttgtaa atctgagcca gtaaaagaag aaagttccga gctggagcag ccatttgcac
781 aggacacaag tagcgtgggg ccagacagaa agcttgcgga ggaagaggac ctatttgaca
841 gcgcccatcc ggaagagggt gatttagatt tggccagcga gtcaacagca cacgctcagt
901 cgagcaaggc agacagcctg ttagcggtag tgaaaaggga gcccgcggag cagccaggcg
961 atggcgagag gacggactgt gagcctgtag ggctagagcc ggcagttgag cagagtagtg
1021 cggcctccga gctcgcggag gcctctagcg aggagctcgc agaagcaccc acggaagccc
1081 caagcccaga agccagagat agcaaagaag acgggaggaa gtttgatttt gacgcttgta
1141 atgaagtccc tccggctcct aaagagtcct caaccagtga gggcgctgat cagaaaatga
1201 gttctcccga agatgactcg gatacaaaaa ggctttccaa agaggaaaag ggtcgcagca
1261 gttgtggtag aaatttctgg gttagtggac tctcttctac aaccagagct acagatttga
1321 agaatctttt cagcaaatat gggaaggtgg tgggcgccaa ggttgtgaca aatgcccgga
1381 gtcctggagc tcgctgttac ggttttgtca cgatgtccac agcagaagag gccacaaaat
1441 gcattaacca cctgcacaag acggagctcc acggaaagat gatctccgtg gagaaagcca
1501 aaaatgaacc tgtgggaaag aaaacctctg acaaaagaga cagtgacggg aaaaaggaga
1561 agtcgagcaa cagtgacaga tctacaaacc ttaagaggga tgataaatgt gacagaaaag
1621 atgatgctaa gaagggtgac gacggaagtg gagaaaagag taaggaccaa gatgatcaga
1681 aacctggccc ctcagagcga tctcgagcca caaagtcagg aagtcgaggg accgaacgga
1741 ctgtagtaat ggataaatcc aaaggggtgc ctgtgattag tgtaaaaacg tccggtcca
1801 aagagagagc ttccaaaagc caggatcgca aatcagccag cagagagaag cggtccgtcg
1861 tgtcctttga taaggtcaag gagcctcgga agtcaagaga ctcagagtcc catagcaggg
1921 tgcgtgaacg cagtgaacgc gaacaacgca tgcaggcgca gtgggagcgc gaggagcgtg
1981 agcggctgga gattgcccga gagaggctgg ccttccagcg ccagcggctg gagcgggagc
2041 gcatggagcg ggaacggctg gagcgcgaac gcatgcacgt ggagcacgag cgcaggcgcg
2101 agcaggagcg catccaccgt gagcgcgagg agctgaggcg ccagcaggaa ctgcgctatg
2161 agcaggagcg gcggcccgcg gtgcggcggc cctacgacct ggaccggcga gatgatgcct
2221 attggccgga agccaagcgg gccgccctgg atgagcgcta ccattctgac tttaaccgcc
2281 aggaccgctt ccacgacttt gaccacaggg accgcggccg ctaccccgac cactcggtgg
2341 acaggagaga aggttcaagg tcaatgatgg gagaacgaga aggacagcat tacccagaac
2401 gccatggagg accagagcgc cacggccggg actcccgcga tggctggggg ggctatggct
2461 ctgacaagag gatgagcgag ggccgggggc tgcctcctcc ccccagacgt gactgggggg
2521 accatggccg aagagaggat gaccggtcat ggcagggcac ggccgacggg ggcatgatgg
2581 acagggatca caagaggtgg caaggtggcg agagaagcat gtccggtcac tccgggcctg
2641 gccacatgat gaaccgagga ggaatgtcag ggcgcggcag ctttgcccca ggcggggcct
2701 cccgggggca ccccatccca cacggtggca tgcagggcgg gtttggaggc cagagccggg
2761 ggagcaggcc cagcgatgcc cgcttcactc gccgctactg agtacttgga atcctgtgtc

2821 ctgtctcgtg gcaacaaggc tatgttctgt taggagttac cttaaactgt gtaaaaatat
2881 ttttttttaa tctgctgcca tattgtagct caatacaatg tgaatttgtt tttcgttttg
2941 gggttttttt tttttgtaat aaatgtgttt ccgttcacat accctttaaa aaaaaaaaaa
3001 aaaaaaaaa aaaaaaaa//

- LOCUS: NM_004821 1750 bp mRNA linear PRI 17-NOV-2006
- DEFINITION: Homo sapiens heart and neural crest derivatives expressed 1 (HAND1), mRNA.
- ACCESSION: NM_004821
- VERSION: NM_004821.1 GI:4758505

1 gcggtcttat ataagccaga tccgcagggg agtccgcaga agggttaaac aggtctttgg
61 gcttcggcga cctcgcccgc ggcagaaacc ggtaagaaga cagtgggctg cgcgtctcat
121 tttcagcctt gcccggactc tcccaaagcc ggcgcccagt agtggctcca gagcccacag
181 gtggcccccg gcagtctctg gggcgcatgg agcggcgtta atagggctgg cggcgcaggc
241 cagtagccgc tccaacatga acctcgtggg cagctacgca caccatcacc accatcacca
301 cccgcaccct gcgcacccca tgctccacga acccttcctc ttcggtccgg cctcgcgctg
361 tcatcaggaa aggccctact tccagagctg gctgctgagc ccggctgacg ctgccccgga
421 cttccctgcg ggcgggccgc cgcccgcggc cgctgcagcc gccaccgcct atggtcctga
481 cgccaggcct gggcagagcc ccgggcggct ggaggcgctt ggcggccgtc ttggccggcg
541 gaaaggctca ggacccaaga aggagcggag acgcactgag agcattaaca gcgcattcgc
601 ggagttgcgc gagtgcatcc ccaacgtgcc ggccgacacc aagctctcca agatcaagac
661 tctgcgccta gccaccagct acatcgccta cctgatggac gtgctggcca aggatgcaca
721 gtctggcgat cccgaggcct tcaaggctga actcaagaag gcggatggcg gccgtgagag
781 caagcggaaa agggagctgc agcagcacga aggttttcct cctgccctgg gcccagtcga
841 gaagaggatt aaaggacgca ccggctggcc gcagcaagtc tgggcgctgg agttaaacca
901 gtgagccgag gcccgcgccg aggacctggc caggccagcc actcctgaag ccccgggagg
961 agaggaaggc agcggcgaac gccaggctct gggctccggc gactggtgct acgcatcccg
1021 cggagcttct gctgagcgcc ggcaggtcgt cggctgcaac cacacacttg gatcgcacgt
1081 gcaatgtcct ttgatttttt ttaatacatt aagagaaaga gaaatatata tatatccacc
1141 cccagcccaa ccgagggcgg cccttggcgg caacatgcaa gaaggaggga ctgtcgaacc
1201 caagggctca aagacgcact cttccaccct tttggagcga atttagaacc tcagccctat
1261 ctccatttcc ctatctggct ctttctctct tgtccctcca tatgatccgc cccgacgccg
1321 tcttctctaa ttaaaatgca ataaggaatc aattcttttc ttgcctgaga aagagaacca
1381 gacgcaggaa gatgaaaggc tgccctttgt tcttcgaatc gtggtggttt tattttattt
1441 ttctttttgt cgctgcactt cctgtttagt tccaaggaaa acactttctc tctttctctg
1501 tctctctctt ttcttccttc ttttccttcc tttttgtttc tatctaaata aaagctttcc
1561 ctgtgttgga aagtttttat gtatttaaac tacctaccat gcctgttgtg ctcaggtgtt
1621 tgttcatcct gccatcccca acccttttct acctcaagtc tgtgtgacca ctcacagccc
1681 ccctcccttc gccaaagcag tgtctatgct cttgattaat aaaacatttt ctgaaatcaa
1741 aaaaaaaaaa //

- LOCUS: BC002818 1430 bp mRNA linear PRI 15-JUL-2006
- DEFINITION: Homo sapiens synovial sarcoma, X breakpoint 2, mRNA (cDNA clone MGC:3884 IMAGE:3636268), complete cds.
- ACCESSION: BC002818
- VERSION: BC002818.2 GI:33877218
- KEYWORDS: MGC.

1 ctcagagtac gcacggtctg attttctctt tggattcttc caaaatcaga gtcagactgc
61 tcccggtgcc atgaacggag acgacgcctt tgcaaggaga cccacggttg gtgctcaaat
121 accagagaag atccaaaagg ccttcgatga tattgccaaa tacttctcta aggaagagtg
181 ggaaaagatg aaagcctcgg agaaaatctt ctatgtgtat atgaagagaa agtatgaggc
241 tatgactaaa ctaggtttca aggccaccct cccacctttc atgtgtaata aacgggccga
301 agacttccag gggaatgatt tggataatga ccctaaccgt gggaatcagg ttgaacgtcc
361 tcagatgact ttcggcaggc tccagggaat ctccccgaag atcatgccca agaagccagc
421 agaggaagga aatgattcgg aggaagtgcc agaagcatct ggcccacaaa atgatgggaa
481 agagctgtgc cccccgggaa aaccaactac ctctgagaag attcacgaga gatctggaaa
541 tagggaggcc caagaaaagg aagagagacg cggaacagct catcggtgga gcagtcagaa
601 cacacacaac attggtcgat tcagtttgtc aacttctatg ggtgcagttc atggtacccc
661 caaaacaatt acacacaaca gggacccaaa aggggggaac atgcctggac ccacagactg
721 cgtgagagaa aacagctggt gatttatgaa gagatcagcg accctgagga agatgacgag
781 taactcccct cagggatacg acacatgccc atgatgagaa gcagaacgtg gtgacctttc
841 acgaacatgg gcatggctgc ggacccctcg tcatcaggtg catagcaagt gaaagcaagt

901 gttcacaaca gtgaaaagtt gagcgtcatt tttcttagtg tgccaagagt tcgatgttag
961 cgtttacgtt gtattttctt acactgtgtc attctgttag atactaacat tttcattgat
1021 gagcaagaca tacttaatgc atattttggt ttgtgtatcc atgcacctac cttagaaaac
1081 aagtattgtc ggttacctct gcatggaaca gcattaccct cctctctccc cagatgtgac
1141 tactgagggc agttctgagt gtttaatttc agattttttc ctctgcattt acacacacac
1201 gcacacaaac cacaccacac acacacacac acacacacac acacacacac acacacccac
1261 caagtaccag tataagcatc tgccatctgc ttttcccatt gccatgcgtc ctggtcaagc
1321 tcccctcact ctgtttcctg gtcagcatgt actcccctca tccgattccc ctgtagcagt
1381 cactgacagt taataaacct ttgcaaacgt tcaaaaaaa aaaaaaaaa//

- LOCUS: NM_014910 7605 bp mRNA linear PRI 18-NOV-2006
- DEFINITION: Homo sapiens zinc finger protein 507 (ZNF507), mRNA.
- ACCESSION: NM_014910 XM_375594
- VERSION: NM_014910.2 GI:55925467

1 gggatgagga gggggaaacc gggggaaaag agggaaaaga gccgggagag aggtgggcaa
61 ggacgagggc gagggcacgg ccgggtcctg gctggccaaa cgaggctcgc ggaagcagca
121 gccgccgcct gaccgcagat atggaagaaa gtagcagtgt tgccatgttg gtgccagata
181 ttggggaaca ggaagctata ctgactgctg aaagtatcat cagtccttca ttggaaattg
241 atgaacaaag aaaaactaaa ccagatccat taatccatgt tatccagaag ttaagcaaga
301 tagtggaaaa tgaaaagtca caaaaatgtc ttttaattgg gaagaaacgc ccacgttcaa
361 gtgctgcaac acactctctt gaaacccaag aactttgtga gattccggct aaagtaatcc
421 agtcacctgc tgctgatact agaagggctg agatgtcaca aacaaatttt acccctgaca
481 ctcttgccca gaatgaaggg aaggctatgt cttatcagtg tagcctttgt aagtttctat
541 catcatcctt ttccgtgtta aaagatcata ttaagcaaca tggtcagcaa aatgaagtga
601 tactgatgtg ctcagagtgc catattacat ctagaagcca ggaggaactt gaagcccacg
661 tggtgaatga ccatgacaat gatgccaata tccacaccca atccaaagcc caacagtgcg
721 taagcccctc cagctctttg tgtcggaaaa ccacagaaag aaatgaaacc attccagata
781 tcccagtaag tgtggacaat ctacagactc atactgtcca aactgcatct gtggcagaaa
841 tgggtaggag gaaatggtat gcatacgaac agtacggcat gtatcgatgc ttgttttgta
901 gttatacttg tggccagcag agaatgttga aaacacacgc ttggaaacat gctggggagg
961 ttgattgctc ctatccaatc tttgaaaatg aaaatgaacc cctaggcctg ctggattctt
1021 cagcagctgc tgcgcctggt ggggtcgatg cagtcgtcat tgctattgga gagagtgaac
1081 tgagtatcca caatggccca tcagtgcaag tgcagatttg cagctcagaa cagttatcat
1141 cttcatctcc tttagaacag agtgcagaaa gaggagtaca cctaagtcag tcagttaccc
1201 tggaccccaa tgaggaagaa atgctagaag tgatttctga tgcagaggag aatctgattc
1261 ctgatagcct gcttacatca gcacagaaaa tcatcagcag cagccccaat aaaaaaggc
1321 atgttaacgt gatagtggag cgattgccaa gtgctgaaga aaccctttca cagaagcgct
1381 tcctcatgaa cactgaaatg gaagaaggga aggacctgag cctgacagaa gctcagattg
1441 ggcgcgaagg aatggatgat gtttatcgtg ctgataaatg tactgttgat attgggggat
1501 tgatcatagg ctggagcagt tcagagaaaa aagacgagtt aatgaataaa ggcctggcta
1561 ctgatgagaa tgccccacca ggccggagaa ggacaaattc tgagtctctt cgattacact
1621 cattagctgc agaagccctt gtcacaatgc ctataagagc tgcagagttg acaagagcca
1681 acctggggca ctatggagat ataaaccttt tagatccaga tactagtcaa aggcaagtag
1741 atagtacatt ggcagcgtac tcaaaaatga tgtcgccact taaaaactct tcagatggat
1801 taactagtct taaccaaagc aactccacct tggtagcact cccagagggt aggcaggaat
1861 tgtcagatgg gcaggttaag acaggcatca gcatgtcctt actcaccgtc attgaaaaat
1921 tgagagaaag gacagaccaa aacgcttcag acgatgacat tttgaaagag ttgcaggaca
1981 acgcccagtg ccaacccaac agcgatacaa gtttgtccgg aaacaatgtg gtggaataca
2041 tcccgaatgc tgaacgaccc taccgttgcc gcctgtgtca ctacacaagt ggcaacaagg
2101 gctacatcaa gcagcactta cgagtccatc gacagagaca gccttatcag tgtcctatct
2161 gcgagcacat agcggacaac agcaaagatt tggagagtca catgatccac cactgtaaga
2221 caagaatata ccagtgcaag cagtgtgaag aatccttcca ttataagagt caattgagga
2281 accatgagag agaacagcac agtcttccag ataccttgtc aatagcaact tctaatgagc
2341 caagaatttc cagtgataca gctgatggaa aatgtgtcca ggaagggaat aagtcttcag
2401 tccagaaaca atatagatgt gatgtgtgtg attatacaag tacaacatat gttggtgtca
2461 gaaaccacag gcgaatccat aactctgata agccgtacag atgctctctg tgtgggtatg
2521 tgtgtagcca tcctccttct ttgaagtctc atatgtggaa acatgcaagt gaccaaaatt
2581 acaactacga acaagtaaac aaggctatta acgacgcgat ttcacaaagt ggcagagttc
2641 tggggaaatc ccctggaaag actcaattaa agagcagtga agagagtgca gatcccgtca
2701 ctggaagttc agaaaatgca gtgtcatctt cagaactgat gtcccagact cccagtgaag
2761 ttctgggtac caacgagaat gagaaactga gccctacaag taatacctca tatagtttag
2821 aaaaaatctc cagtctggcc cctcctagca tggagtactg cgttttactc ttctgctgtt
2881 gtatttgtgg ttttgaatca accagcaaag aaaacctctt ggatcatatg aaagagcacg

2941 agggtgaaat tgtaaacatc atcctgaata aggaccacaa tacagctcta aacacaaatt
3001 aggtggaata atgactcgag caggaaagca gtagaagagg attccttcac cacagtttca
3061 cctttacgct gtcagacaac ttcctgccac agaagaagtc gttgatgtga tttttgagga
3121 aatgacagat gtgactttgg aaccaaactt gtaataaaag gaattccaaa tggacaagca
3181 gtaatgatat ttaaatattt tgagtgaggg ggagtgggtc aaagaggaag tagaggtgta
3241 atcctgtatt aaccctctgt caccccttct tagtgtcgag tgtatttatt aataaaagct
3301 tattagagtg tagaaatgcc agcaagagtt aagaaagggc ttttcaggaa ctattctaaa
3361 agtcataaaa gggtcacagt cttaagcaga gcttagtttt cttctcactt gtcaaatgca
3421 gactgtgagg cctccagtga gaaatgggag gcagttctgg gagggggttt tttcagtgtc
3481 accaacagag tcaacaagga aacataatta gagggctttg aaatgatcta ctgaaatacc
3541 taaattgata gaaattaatc atactcaatc taggacatgt tcctttactc cttaaaaaag
3601 aaaggaaaga tctctaaatt caaagctaga ttgtaaatag gcattcaaga agtattgtct
3661 taaatttacg atgaatttct tgtgagcaga ggacgaaaca ggtgaattct caccagactc
3721 aaaccagatc tcaattcgta gtaatgctaa atcgtcttcc gtgttctcag aggttctttt
3781 cagtgtctgt cagaacttgg catactttct ccatagtacg tagaccttct aatactctgc
3841 taaatgaaac cacacttgtt atctgaaagt gtgtgaaaga aacaatgtat gaaaatttac
3901 atttgatcat ccggcattgg aaatagtttg aactatttcc aaaaatccct taggggatga
3961 ggggtgaaat ttaaaagctc ctgaaaatga gtactgctgt gttgagcttt tctttcctgg
4021 ggtatattaa tttagttata tttagcagaa gaggaatata accaaatgtg actaaaatat
4081 agcagaaatt caggttgttt gtaaaaattt taataacaaa tagcatttgg caagtctata
4141 ggattcttcc tatctggaaa ttctattttg ttagagtgcg ataggacata tggaaaacca
4201 acacaaagtg ctttagcatt aaaactcacc atcaaataga tgtaatctta ttaattacac
4261 atttgtattt ttaattattt caaggagtga agaaaacagg gtgtttttgt ttttgtagtt
4321 ctgattactc atttatgcat tgttttgttt aagtaaattc tgcctacaag tgaaaaggtc
4381 ggtgcgctct tctgtgcacc atcctcacgg tgctatttcc gaatatctga atattgattt
4441 ctaacacctg gatgaggctg tgaccgatca acttggaaca ctgtaaaggt tttcgaatgg
4501 gatacgctgt aggcacgttt taagaagtat tctgttccta aagtccaggt atgattggat
4561 taatatttat aaaattatta ttaggaatta tttaagtggg gccaggcatc ttgcaacatt
4621 ttctgatttt tttttctttt ctttctaatc tcattttggt gatatttaaa caaacataaa
4681 tgattgtaac tttgcagctt ttttatttag gtagctttaa tttatttatg aaagttaatc
4741 catttctgat acgtggtttt taaaaatatg aaatggattt atatatacta tattcctcaa
4801 atccactgta tgtggactta tattcatttt ctcctttttt cggaattgaa acattttaat
4861 ttcaaattca aatagaacat ttaaaatgat ttcattatta ttacccatac tgttgccact
4921 catattgtaa gtcagttttt tcattgctgg tacaatgact cagtatttct ttataaaaat
4981 ctgttgttct gaaaatcaac aaccttagaa ggattttgtc ttagaaaatt tccttggctt
5041 tagttttata tcatatttag aaaataataa tgaaaagcat ccccaaaatt tcaatgatgt
5101 gaatttttaa attaccttta tttctttaaa ctaattccat tgattgttac ttaaattttc
5161 cacctggaat catggattta aaatttcccc acctcatggg gaaaaaacta acccagaagt
5221 tcaaagcctt taaagtttca tctgattctg gccttagaat tgtccttaaa agctttctcc
5281 tggccgatag gaggaagcat atatcaagga ggttctttcc cttcccatta tcattttgtg
5341 gcaagcctta agttaacaat gtgtctacca agaacaattg agttttctaa aagtaataat
5401 gaagattatg caattctaac tgtagaagag atagctatta aaataatcag tagcctgggc
5461 atagtggctc acacctgtaa tcccaatact ttagaaggcc aaggctggag gactgcttga
5521 gtccagaagt ttaagaccag cctgggcaac acggggagac cctgtctcta caagaaatta
5581 aaaattagcc aggcatagtt ggcacgtgcc tgtagcacca gctacttggg aggttaaggt
5641 gggaggatca cttgagcctg ggagatagag gcttgcagtg agccgtgatg gcaccattgc
5701 actccaaccg ggtgacagaa tgaggtcctc tgtttacctc cccccccaaa aaaaaaattc
5761 agtaggaatt ccttcataga ataacatgtt atttattggt attgacttct tattagacag
5821 agctcatagt atttgttttc tgctccgaac acttaaaaaa tactaagacg tgatagtgaa
5881 aatagttttg aaaagaaact acatatggca aaggtgggga ggggggaaga tgatgcattc
5941 tgatcattat aaagaatact tttcagggct ctatcatttt ctccctttct ctaatcatcc
6001 agaatatggc gggtgtccca gtgttcaaca gtatcatgct aatattccat ttgatcggta
6061 gtccaagttc tttggccaga tggtgcaatg ttgtgacacc gaccgaagcc tttgttctgg
6121 atctttcttc ctattgaagg tggctgctgg tggcttcata atttctgttt tttttctca
6181 caaagtaaat ggtgggcatc catgtttaca ttgcaccttc ccgtgctgta attggcttga
6241 caaagacaag caggcttctc tgttgagact tattgtgttt ttagttttca tagacactta
6301 tttaatcttt ttaaattgta cagcaaggtg ctctaagtaa tattcgataa aatatattta
6361 atagaaattt gcgtttgata ttcatgaaca tgaatacatg tattttttta agaaataagt
6421 attgtgtaac actatggcat tgcttctata gccaaagtat aaaaatttct ggaatactga
6481 catgtaaaga ctacagttaa ttctgacact gtatcttatt aaaataggat gatttgcatt
6541 ttgtaaaatt atcctgcaca tcaagctgca tgccttaaag cggaaactct aggactgtgt
6601 tcatgggaga gcagttcatc tgttcagaac agtgaggcaa ggtctgtagt gcttctttaa
6661 ctacctttgg aaatactgta caatgttaga ataatttatt ttgctttaca ggagtttgtc

6721 atgtattgac tttaatattg tattttggta ataaattttt tgttaaaaaa aacttggctt
6781 caagttctat taaccacagg ttgtgttcat tcttttcctg gaaatgatga ctttattgct
6841 tagaaagttg taaataatta aaagcttccc aagagttaaa tgaaggttat gatgactctt
6901 gataacttgt taatctgttg ctgactagca ttagactgtt acacgaagat aaactgaata
6961 agcatttagc tttttaaatc attttaaaaa ttaagtctaa ttttgaagct tttttccttg
7021 ctacccagtt cagtttctct tccaaatgtg acacgcttat atgctttcaa ttctgatata
7081 gtcaaagtgc taaagactag ttctgctatt gacgaaggaa ttagcattcc ttagtgaagg
7141 ccaaaaacta cagtgatatg ttcacacctt gatcactctg tcgtctatac agacagctat
7201 tgacatattt gttgcattga cgaagctaac atcatctgac tgtatcgtgg gtctggttat
7261 ccatttgtgc cctagaatcc tgggtcacct cagttgaaag tgaccaaggc caaatatttc
7321 gtcccatgct tcccagggac aacataatca tatcccctgt ctttgaaaaa cccaatctga
7381 aactgtgtca ctaacctcca aattaaagaa tccgagcatg gaagctctag aacgtatggt
7441 ccagtgtggc agccactata atatctaagt gtaaaataca tagcagttgc aaagacttag
7501 tacaaaaaga atggaaatgt attaacagtt ttttatattg attacatgtt gaaatgatat
7561 tttggtttaa ataaaatatt aaaatcaaaa aaaaaaaaaa aaaca//

- LOCUS: HUM130LEU 4782 bp mRNA linear PRI 11-FEB-2002
- DEFINITION: Homo sapiens leucine-rich PPR-motif containing protein (LRPPRC)mRNA, complete cds.
- ACCESSION: M92439
- VERSION: M92439.1 GI:177109

1 aagtttttaa tgatacctgc cgctcaggtg gcctaggtgg tagtcatgcc ttgcttctac
61 ttacgtagtt gtggttctct cttgcctgaa ctaaagcttg aagagagaac agaatttgct
121 cataggatat gggacacact tcagaaatta ggtgctgtgt atgatgtgag tcactataat
181 gctttactta aagtctatct tcaaaatgaa tataaattct caccaactga tttcctggca
241 aaaatggagg aagcaaacat tcaaccaaat cgagtgacat accagagatt gattgcttct
301 tattgtaatg taggagatat tgaaggtgcc agcaagattc ttggatttat gaaaactaag
361 gatctcccag ttacagaggc agtattcagt gcccttgtga cagggcatgc cagagctggt
421 gatatggaga atgcagaaaa cattctcaca gtgatgagag atgccggaat tgagcctggt
481 ccagacacat acctcgcatt attgaatgca tatgctgaga agggcgacat tgaccatgtt
541 aagcagactc tggagaaggt ggagaagttc gagcttcacc ttatggaccg tgatttactg
601 caaattattt ttagcttcag taaagctggg tatctcagta tgtctcagaa attttggaaa
661 aagtttacat gtgaaagaag atatattcca gatgcaatga acctcatttt acttttagtc
721 actgaaaaat tggaagatgt agcgttgcaa attttactag catgccccgt atcaaaggaa
781 gatggcccaa gtgtctttgg cagtttcttt ttacaacact gtgtgactat gaatacgcct
841 gtggagaagc taacagacta ctgtaagaag ttaaaggaag tccagatgca ctcctttcct
901 ctgcagttca ccctccattg tgctttactc gccaataaaa ctgatttggc aaaagcctta
961 atgaaggctg tgaaggagga aggttttcct atcagacctc actatttctg gccattgcta
1021 gttggacgtc ggaaggaaaa aaatgttcaa ggtataattg aaatcctcaa aggaatgcaa
1081 gaattgggag tacatcctga tcaggaaaca tatacagatt atgtgattcc atgctttgat
1141 agtgtaaact cagcacgagc cattttgcag gaaaatggat gtctgtctga tagtgatatg
1201 ttttctcaag ctggattgag aagtgaagca gcaaatggga acttagactt tgtattatca
1261 tttttgaaat caaatacatt gcccatctcg ctgcagtcta taagaagtag cctactgcta
1321 ggcttcagga ggtctatgaa tataaatgtt tggagcgaga taacagaatt attgtacaag
1381 gatggacgtt attgccagga gcctcgagga ccgacggaag ctgttggcaa ttttctttat
1441 aacttgattg acagcatgag tgactcagag gtacaggcca aggaggagca tttgagacaa
1501 tacttccatc agctggagaa gatgaatgta aaaattcctg aaaatatcta cagaggcatt
1561 cgtaatctcc tggaaagcta ccatgttcct gaattgatta aggatgctca cttgttggtt
1621 gagcgtaaga atttagactt tcaaaaaact gtgcaactta catcatctga attggagtca
1681 acacttgaaa cactaaaagc tgaaaatcaa cctataagag atgtcctaaa gcaactcata
1741 ttagtgcttt gttcagaaga gaatatgcaa aaagcccttg aattgaaagc aaaatatgaa
1801 tccgacatgg ttactggtgg ctatgcagct ttaataaatt tatgctgtcg acatgataaa
1861 gtagaagatg ccttgaactt gaaagaagaa tttgaccgct tagattcatc tgctgtcctt
1921 gacaccggca actatctagg ccttgtaaga gtattggcaa agcatggcaa gctccaagat
1981 gctattaaga ttctgaagga gatgaaagag aaggatgttc ttatcaaaga tacaacagcc
2041 ttgtcctttt tccacatgct aaatggcgca gctttaagag gtgaaattga aacagtaaaa
2101 cagttgcatg aagccatcgt gactctaggg ttagcagaac catccaccaa cataagtttc
2161 ccattggtca ctgtacactt ggaaaagggc gacctatcta ctgctcttga ggtcgccatt
2221 gactgctatg aaaagtataa agtattacca aggattcatg atgtcttgtg taaactggta
2281 gagaaaggcg agactgatct aattcagaaa gcaatggact ttgtgagcca agaacaaggt
2341 gaaatggtga tgctctatga tctcttcttt gccttcctac aaacaggaaa ttacaaagag
2401 gccaagaaga tcattgagac tccagggatt agagctcgat ctgcaaggct tcagtggttt

2461 tgtgacagat gtgttgcaaa taatcaggtt gaaactctgg aaaaattagt ggagctgaca
2521 cagaagctat ttgaatgtga tagagaccag atgtactaca atctgctaaa actgtataaa
2581 ataaacggtg actggcaaag agctgatgca gtctggaata aaatccaaga agaaaatgtt
2641 attcctcgtg aaaagacatt aagattatta gcagaaatcc ttagagaggg taaccaggaa
2701 gttccgtttg acgtacctga gttgtggtat gaagatgaaa aacattccct gaattcttcg
2761 tcagcctcaa ccacagaacc tgatttccag aaagatatat tgattgcctg ccgattgaac
2821 caaaaaaaag gggcatatga tattttcctg aatgcaaaag agcaaaacat tgtgtttaat
2881 gctgaaacct acagcaatct cattaaatta ctgatgtcag aagattattt tacacaagca
2941 atggaagtga aagcattcgc ggagacccac atcaagggct tcacactgaa cgatgctgcc
3001 aacagccgcc tcatcataac gcaagttagg cgggattatt tgaaagaggc tgtgacaaca
3061 ctgaaaacag tattggatca gcagcagacc ccttctaggt tagcagtgac ccgtgtcatc
3121 caggcattgg ccatgaaggg tgatgttgaa aacatagaag tagttcagaa gatgttaaat
3181 ggactcgaag actccattgg actttcaaaa atggttttca tcaataacat tgctttggct
3241 caaataaaga ataataacat agatgccgca atagaaaaca ttgaaaatat gcttacttca
3301 gagaataaag tcattgaacc ccaatacttc ggcttggcat acttattcag aaaagtaata
3361 gaggagcagt tggaaccagc agttgaaaag ataagcatca tggcggagag attggccaat
3421 cagtttgcaa tttataaacc tgtcactgat tttttccttc aacttgtgga tgcaggcaag
3481 gtggatgatg ccagagctct cctacagaga tgtggtgcaa ttgctgaaca aaccccgatt
3541 ttgttgttgt tcctccttag gaattctagg aaacaaggaa aggcatcaac tgtgaaatct
3601 gtgttagaat tgattcctga attaaatgaa aaggaagaag catacaattc cctcatgaaa
3661 agctatgtct cagagaaaga tgtcacatct gctaaagcac tgtatgaaca tttgactgca
3721 aagaatacaa aattggatga tctgtttcta aagcgttacg catctttgct gaagtatgct
3781 ggagagcctg tccctttcat tgaaccccct gaaagctttg aattttatgc acagcagcta
3841 agaaaattga gggaaaactc ttcttgaaat aaccaggcga tactttgttt tgtatatatt
3901 tgtgattctg tgtctacatg ttattttgaa gtatatctga gggaaaaata aatgaaaatt
3961 ttctttatgt acttatgtat gtgtgatgca tgttcaaagt cttattgacc ataactctgt
4021 gcacttggtt attggacatt tttggagttt tttctctggg aaaaatcgat agtgttttct
4081 tcaatgctgc tgctgtgtga agccatactt ttcaggattc ttccctaatt ggctctttgg
4141 tttccctgct ctgtttcatt tatttcatta aaatgttatt cctttattta agattcactt
4201 attagtctgc tgtttctctg aaaaatttta gagctaggta tagtgaccgt gaacttctaa
4261 cgcataatat ctgtgataca gccattccgt acatgtgtga gtctgcataa ctttcgaact
4321 ttcgaacttt gttaaatgtt ggcactagga gtcatcagat ctaggattca tcattttcca
4381 gtgagaagca gagacccaaa ggcctgttac ttgtgcttgg tcaggggact gtctgtcatg
4441 cctggaggct cttcggcaca cttccccatc tttcccttct gccactgtgg cttcaagcac
4501 ctctgttcat agagcgtctc tgaaattgag tctcggtcat gacttatccc gaagtagagc
4561 aatgtgtttc ctctcattgt agtttcagga ctttgtcagt acaagctctg ccctaggctt
4621 cttactttat actcatatcc tgaaaagatg tgatttcatc tatgaagggg taaaatattg
4681 gtttgtattt aattgtttga aataaaagtg atccctataa aaaaaaaaaa aaaaaaaaaa
4741 aaaaaaaaaa aaaaaaaaaa aaaaaaaaaa aaaaaaaaaa aa

- LOCUS: NM_006963 2630 bp mRNA linear PRI 18-NOV-2006
- DEFINITION: Homo sapiens zinc finger protein 22 (KOX 15) (ZNF22), mRNA.
- ACCESSION: NM_006963
- VERSION: NM_006963.3 GI:55775473

1 tgaaatcaga ctttggagcc acaaagaacc attccaattc tgggtcgtcc gcttacacac
61 ggtgaacgga gcacatttct tctctgagcc ttggttcctt tcctgtaaat ggagtataac
121 cactcccatc ttgcaggagc tttttggtga taattaaggt gacttcgtga aaagtaccag
181 ggtctcaaaa atctcaaatt ttgtggaaag tatgcagttt ctaggttgga gaggctagag
241 tttggtttca aaagcctcga ccacacacag gggcggttac agctcttgag gtcccggggg
301 tcacaggacg ccttccgagt tggggggagg agaaaacaaa accacaggaa gaaacgaaca
361 acgctttcgg gtgggcccgg gaccatagag ggtcgctccg ccactttcct agagcgaccg
421 cggcggcgcc agagcgcggc gaggccctca cttccggcgg cgcgggaggc gcccagcgag
481 ccagagtggt ggctggtccc gcgcgaaaat tctgagctgt acacctctag gaaatgaaac
541 actagttcag aagaagcctg taaactctct tacaaataca tttggttatt caccatgagg
601 ttagcaaagc ctaaagcggg tatttctcgg agctcaagcc aaggaaaggc ctatgagaac
661 aagcgcaaaa caggccggca gcggcagaag tggggcatga ctattcgatt tgactcaagc
721 ttcagtagac tcagaagaag cttggatgac aaaccctata aatgtactga atgtgaaaag
781 agtttcagtc agagttcaac tctttttcaa caccagaaga tccatactgg aaagaaatcc
841 cataaatgtg ctgattgtgg gaaaagtttc tttcagagtt ctaatctcat tcagcatcga
901 cggatccata cgggggaaaa gccctacaaa tgtgatgagt gtggagaaag cttcaaacag
961 agctcaaatc tcattcagca ccagagaatt catactggag aaaaacccta tcagtgtgat
1021 gagtgtggcc ggtgtttcag ccagagctcc caccttattc aacatcagag aacccacact

1081 ggggagaaac cctaccagtg cagtgaatgt ggcaaatgtt tcagtcagag ctctcatctg
1141 aggcagcaca tgaaggtgca taaagaagag aagcctcgta aaacccgggg caaaaatatc
1201 agggtgaaga ctcacttacc ctcttggaaa gctggtacag gaaggaagtc tgtggctggt
1261 ctccgttaag tatagggctt tttgacagct ttttgagacc tcttaagaaa aaataaaaag
1321 taaaaaatga aaggaatctt ttttagaaat agagatgctt tatagtagat cacttaaata
1381 ctggatcttt tgctagtgtg aaaacattgg gaatttaatg acattattga gctgaaagaa
1441 attacatgag tccagctacc ctcatttctt ttttatgtga catggagcac aaagaactga
1501 aaatatgttt tgagggagca tgacatcctt gacctcattt gaaattactt ccattttcaa
1561 aaaccatcat gttttgcaga tacattttga gaaaaaccat catgttttga ggatacattt
1621 gtgaaagtgc aggcatgcca atgactactc agttttagga cttttgtgga agaaaaatta
1681 aaagggagaa gaaatcaatt ccactacttt tgaagttctg caacagatga gattttgttt
1741 gtaaagtttt gtagtatatt aaccattggt ttatcactct agattcaaca tattaaaatg
1801 tattcaagct cacagatttt taatcagtaa ggcctatcta tattagttgt cttttatttc
1861 ttctccttgt ggacagttgt taatgaaagg agtaaggatt tccctttttt tgttttgttt
1921 gttttgtttt ttttaaccaa attcttagag atactataga atccaaatga gaactgaatt
1981 ggacctcaag tcttctattc ctactaatag agttctttgt gatggtaact gctgtgtcgt
2041 ttgttttcca caagttggga tggattcatg tcgatacatc cccatgccct tgacctcttc
2101 tggcattctc ctgtgctctg acaaactgag ccagcctttt agatctacat gaataaacaa
2161 actattttac caagaaaaat ctcagcttgc ttactgctta attaaaaacc tacaatttac
2221 acacctccct gccttcaaga ctgtgagctt tggatagccc tgcttaaatg tttgtcaaca
2281 acaagagtcg taatgtgtca gtacattgta gctcctttga aatttaactc tttctgtgtt
2341 acatgaattg ttttaaggag tccggcaaac atgtttcttc acttccatga gaatggtgcc
2401 aagtgtcaga ctctaatgag ccctcagctc aggttttaat ttctattgaa tgctaacatt
2461 cttctgattt tttggtatct tttataatca tatcggtttc tgctgtatta atgacaatta
2521 ttcatgaaaa taaaaaatga aatattttgt atactaaaaa aaaaaaaaaa aaaaaaaaaa
2581 aaaaaaaaaa aaaaaaaaaa aaaaaaaaaa aaaaaaaaaa aaaaaaaaaa//

- LOCUS: NM_003927 2584 bp mRNA linear PRI 17-NOV-2006
- DEFINITION: Homo sapiens methyl-CpG binding domain protein 2 (MBD2), transcript variant 1, mRNA.
- ACCESSION: NM_003927
- VERSION: NM_003927.3 GI:21464121

1 gggggcgtgg ccccgagaag gcggagacaa gatggccgcc catagcgctt ggaggaccta
61 agaggcggtg gccggggcca cgccccgggc aggagggccg ctctgtgcgc gcccgctcta
121 tgatgcttgc gcgcgtcccc cgcgcgccgc gctgcgggcg gggcgggtct ccgggattcc
181 aagggctcgg ttacggaaga agcgcagcgc cggctgggga gggggctgga tgcgcgcgca
241 cccgggggga ggccgctgct gcccggagca ggaggagggg gagagtgcgg cgggcggcag
301 cggcgctggc ggcgactccg ccatagagca gggggccag ggcagcgcgc tcgccccgtc
361 cccggtgagc ggcgtgcgca gggaaggcgc tcggggcggc ggccgtggcc gggggcggtg
421 gaagcaggcg ggccggggcg gcggcgtctg tggccgtggc cggggccggg gccgtggccg
481 gggacgggga cggggccggg gccggggccg cggccgtccc ccgagtggcg gcagcggcct
541 tggcggcgac ggcggcggct gcggcggcgg cggcagcggt ggcggcggcg cccccggcg
601 ggagccggtc cctttcccgt cggggagcgc ggggccgggg cccaggggac cccgggccac
661 ggagagcggg aagaggatgg attgcccggc cctccccccc ggatggaaga aggaggaagt
721 gatccgaaaa tctgggctaa gtgctggcaa gagcgatgtc tactacttca gtccaagtgg
781 taagaagttc agaagcaagc ctcagttggc aaggtacctg ggaaatactg ttgatctcag
841 cagttttgac ttcagaactg gaaagatgat gcctagtaaa ttacagaaga acaaacagag
901 actgcgaaac gatcctctca atcaaaataa gggtaaacca gacttgaata caacattgcc
961 aattagacaa acagcatcaa ttttcaaaca accggtaacc aaagtcacaa atcatcctag
1021 taataaagtg aaatcagacc cacaacgaat gaatgaacag ccacgtcagc ttttctggga
1081 gaagaggcta caaggactta gtgcatcaga tgtaacagaa caaattataa aaaccatgga
1141 actacccaaa ggtcttcaag gagttggtcc aggtagcaat gatgagaccc ttttatctgc
1201 tgttgccagt gctttgcaca caagctctgc gccaatcaca gggcaagtct ccgctgctgt
1261 ggaaaagaac cctgctgttt ggcttaacac atctcaaccc ctctgcaaag cttttattgt
1321 cacagatgaa gacatcagga aacaggaaga gcgagtacag caagtacgca agaaattgga
1381 agaagcactg atggcagaca tcttgtcgcg agctgctgat acagaagaga tggatattga
1441 aatggacagt ggagatgaag cctaagaata tgatcaggta actttcgacc gactttcccc
1501 aagagaaaat tcctagaaat tgaacaaaaa tgtttccact ggcttttgcc tgtaagaaaa
1561 aaaatgtacc cgagcacata gagcttttta atagcactaa ccaatgcctt tttagatgta
1621 tttttgatgt atatatctat tattcaaaaa atcatgttta ttttgagtcc taggacttaa
1681 aattagtctt ttgtaatatc aagcaggacc ctaagatgaa gctgagcttt tgatgccagg
1741 tgcaatctac tggaaatgta gcacttacgt aaaacatttg tttcccccac agttttaata
1801 agaacagatc aggaattcta aataaatttc ccagttaaag attattgtga cttcactgta

1861 tataaacata tttttatact ttattgaaag gggacacctg tacattcttc catcatcact
1921 gtaaagacaa ataaatgatt atattcacag actgattgga attctttctg ttgaaaagca
1981 cacacaataa agaacccctc gttagccttc ctctgattta cattcaactc tgatccctgg
2041 gccttaggtt tgacatggag gtggaggaag atagcgcata tatttgcagt atgaactatt
2101 gcctctggac gttgtgagaa ttgtgctttc accagaattt ctaagaattt ctgctaaata
2161 tcacctagca tgtgtaattt tttttccttg cctgtgactt ggacttttga tagttctata
2221 agaataaggc tttttcttcc cttgggcatg agtcagatac acaaggaccc ttcaggtgtt
2281 actagaaggc gtccatgttt attgtttttt aaagaatgtt tggcactctc taacgtccac
2341 tagcttactg agttatcagg tgcaggtcag actcttggct acagtgagag gcagcttcta
2401 ggcagagttg cttaatgaaa gggtttgtaa tactttacaa accattacct gtacctggcc
2461 tggcctccaa aatattaaca ttctttttct gttgaaactc gcgagtgtaa ctttcatacc
2521 acttgaattt attgatattt aattatgaaa actagcatta cattattaaa cgatttctaa
2581 aatc //

- LOCUS: BF530257 852 bp mRNA linear EST 11-DEC-2000
- DEFINITION: 602071432F1 NCI_CGAP_Brn67 Homo sapiens cDNA clone IMAGE:4214622 5', mRNA sequence. *transcriptional activator of the c-fos promoter (CROC4)*
- ACCESSION: BF530257
- VERSION: BF530257.1 GI:11617620
- KEYWORDS: EST.

1 ggcagcacaa gccatgtgga atctccacca ggtgtacaga acggtgcctc tgcgtcctgc
61 cactcaggac ctctcaagtc cccgatgtga tggctcctca gcatgatcag gagtgagcgt
121 aattcagacc tatttagatc cgtcagccca cggaccaggg ggaccttgag caggacagtc
181 tttgtctcca agcagtcatt ccctgttata caagcaagag tcaggaggag gaatggtttt
241 ggctatcatc ccccacaaga cagcaatggg gcctttaggc ctggaaggaa ggaccaggct
301 gcggctggca tatggaggtt gagcccgtgg gcacggggca tcagcttcac ccggctgagg
361 atgccttctg ctgggttggt ccatctggca ccagctggct tcccagcacc gccccagaac
421 aatcagactc ctccttctgc tgtggccatg gcaaattggc tgccatattg tacctcagag
481 cactagctcc tatctggatt ttcccgcggg gttcctagag ggtgggagat gggtctccag
541 gatttttccc tttcccctga tatctatcca ataagaattc ataataacat atgatttttc
601 taatagccta agtggaggat tactatgtgt caggctctgg tgccagacac tctctacctg
661 ttattcaatg tggctgagaa cagacttatg ggcatgaaat tcagaacgtt ccccattcag
721 tttcctggta ctaagaagac atttcttttc caacagggaa tccatgaact gctaaccggg
781 ttgggagcct cccagggtaa ccagattttt gggtacccaa cttggctggg gggaggcacg
841 gggaggagat cc//

- LOCUS: AF047245 294 bp mRNA linear PRI 06-APR-1999
- DEFINITION: Homo sapiens clone bsmneg3-t7 immunoglobulin lambda light chain VJ region, (IGL) mRNA, partial cds.
- ACCESSION: AF047245
- VERSION: AF047245.1 GI:4566041

1 ctgttgtgtc tgtggccatg ggacagacaa tcaagatcac atgccaagga gacagcctca
61 aaaagtttta tgcaacctgg taccagcaga agccagggca ggcccctgtt cttgtcgtct
121 atggtaaaaa caaccggccc tcaggaatcc cagaccgatt ctctggctcc agctcaggga
181 acacagcttc cttgaccatc actggggctc aggcggaaga tgaggctgac tattactgta
241 attctcggga cagcagtggt aaccatgtat tattcggcgg agggaccagc ctga//

- LOCUS: NM_020393 1853 bp mRNA linear PRI 10-JAN-2007
- DEFINITION: Homo sapiens peptidoglycan recognition protein 4 (PGLYRP4), mRNA.
- ACCESSION: NM_020393
- VERSION: NM_020393.2 GI:56550072

1 acaggaccca cagatatctg ctgccatcca cactctccag attggtgtcc tgggaccacg
61 tggggatgct gccgtggctt cttgtcttct ctgctctggg tatccaggcc tggggtgatt
121 cctcctggaa caaaacacaa gctaaacagg tatcagaggg gctccagtac ctatttgaga
181 acatctccca gctcactgaa aaaggccttc ccacagatgt ctccaccacg gtctctcgca
241 aggcatgggg ggcagaagct gttggctgca gtattcagct gaccacgcca gtgaatgtcc
301 ttgttataca ccatgtccct ggactggagt gtcacgacca gacagtctgc agccagagac
361 tgcgggaact gcaggcccat catgtccaca acaacagtgg gtgtgatgtg gcctacaact
421 tcctggttgg ggatgatggc agggtgtatg aaggtgttgg ctggaatatc caaggagtgc
481 acacccaagg ctacaacaac atctccctgg gctttgcctt cttcggcact aagaaaggcc

541 acagtcccag ccctgctgcc ctgtcggcca tggaaaacct aatcacctat gctgtccaga
601 agggccacct gtcatccagt tatgttcagc cacttcttgg gaaaggcgag aactgcctgg
661 cccctcggca gaagacaagc ctgaagaagg cttgccccgg cgttgtccca cggtctgtgt
721 ggggagccag ggagacccac tgtcccagga tgactctccc agcgaagtat ggcatcatta
781 tccacactgc cgggaggacc tgcaacattt ctgatgagtg ccgcctgctg gtccgggaca
841 tccagtcttt ctacatagac aggctcaagt catgcgacat tggttataac ttcctggtgg
901 gccaggatgg cgccatttat gaaggggtgg gctggaatgt ccaaggctcc tccacccctg
961 gctacgatga cattgccctg ggcattacct tcatgggcac cttcacaggt ataccaccca
1021 atgctgcagc actagaggca gcccaagacc tgatccagtg tgccatggtc aaagggtacc
1081 tgactcccaa ctacctgctg gtgggccaca gtgatgtggc ccgaaccttg tctcctgggc
1141 aggctttgta caacatcatc agcacctggc ctcatttcaa acactgagag aagccccagg
1201 tccttctgag actgctttcc ctcccctgtc aggtctctcc tgtcctaacc atccagcttg
1261 gctcaacacc ttttgccctc ctcccctgcc acacagtcct gtgcctcctt tttcaggttg
1321 ggatgatcat gccctctcct gccaacatcc tccaagggcc tccaaacctc atagctggac
1381 attcacagcc ctctgagtct gagtccagat ttcttctctc cttaccttcc tctcccttgg
1441 aaacccaact cctcagccag gtgagacaat gggctggttc ttgtttcatt tctctctctc
1501 tctccattcc cctctgcctg gtgagccttc ccctggtgtc tgcctggcag cccccacctc
1561 ccacctatca cccctcaccc ataactcagg tcaacgtgac caaccttcct tgcttacaca
1621 taaacttgta tatatttgga tgtagccctt atttaatggc tgtcattatt tatagatatg
1681 tctatccttg ctacttggtt gtgagtttct ccaggggagg aactgtgttt tattcatctc
1741 tatgtcctct gtttctcagc agtgtctgaa atttaatggg ttctactgat gtttattaga
1801 gaaatggatg aataaatgaa tgaagagatc caaaaaaaaa aaaaaaaaaa aaa//

- LOCUS: X95660 451 bp mRNA linear PRI 19-JAN-2001
- DEFINITION: H.sapiens mRNA for variable region of IgA (VH4 family).
- ACCESSION: X95660
- VERSION: X95660.1 GI:1261909
- KEYWORDS: IgA; variable region; VH4 family.

1 gaaacacctg tggttcttcc tcctgctggt ggcggctccc agagggtcct gtcccagctg
61 cagttgcagg agtcgggccc aggactggtg aaggcttcgg agactctgtc cctaagttgc
121 actgtttccg gtggctccat cggcagaggc agttactact gggcctgggt ccgccagtcc
181 ccagggaagg ggctggagtg gattgggagt gtctattata ctgggagcac caactacagc
241 ccgtccctca gcagtcgagg caccatttca gtggacacgg ccaaggacca gttctccctg
301 aatttcaact ctgtgaccgc cgcggacacg gccgtttatt actgtgcgag ggccgtcggt
361 ggtaaaaacc caatctttga gtactggggc cagggaatcc tggtcaccgt ctcctcagca
421 tccccgacca gccccaaggt cttcccgctg a //

- LOCUS: AI659611 537 bp mRNA linear EST 15-DEC-1999
- DEFINITION: tu06a12.x1 NCI_CGAP_Pr28 Homo sapiens cDNA clone IMAGE:2250238 3'similar to contains Alu repetitive element;, mRNA sequence. *inducible T-cell co-stimulator ligand (ICOSL)*
- ACCESSION: AI659611
- VERSION: AI659611.1 GI:4763181
- KEYWORDS: EST.

1 cttttataaa caccacacat cttctaatgc tttcatgtta aaagcgggag tggatcccac
61 attcacctaa aggcccaggt agggactttt tttgcagaaa accaatttct tgtaacaagt
121 actggaacct cattgtagcc ttgagtcctc actcctcctt ttgttctaag tctgggtctg
181 agaagtgcag gcctcacaca gctgtcagtt ccaagtgacc agggctgtaa caggtcctgg
241 ctgaccctgg gaatgtcagg ggattctggg aagtaagaag gaggggcatt tgcccatagc
301 cccggggcag acaatgtgtc ccgagggcca ggccaggctg tctactccat ctccctgggg
361 cgagtgcacc tcgggtcacc tggcagcatc ctttccagcc cttctggaga cagtcagcag
421 ctgattcatt ccgggaacca gcatggcagg tgccggggct ggtcagggcc cagggctgta
481 cccctcagca ctgccccctt gggactgttg ggctccgagg agcctggcgc tgnggtc//

- LOCUS: HSU79302 1976 bp mRNA linear PRI 28-NOV-2000
- DEFINITION: Human clone 23855 mRNA, partial cds. *tumor protein p53 inducible protein 11* (*TP53I11*)
- ACCESSION: U79302
- VERSION: U79302.1 GI:1710287

1 caccgccagc caaagcagtc ccttccattt caggcaccgg cctgggcccg ggagacaccg
61 caaggctatt taagatggtg tggtttgccc atctacgttg atcgtcccaa gaaaggggca

121 ccgtctgcag aacttgccag gaaagtcatc ctgggcggcg cgggccacgc cagggtctcc
181 ccgcgcggct tcctcccccg gcgccttctc tcgggcgcgc gtgcgccccc tgcaggcggc
241 ccccgggaga gcctcgcggg ggagccggga gcctggtttc cccgccaacc cccatcccgc
301 ccgccccggc ggccaccctg ggggcgaggc agcccggcgc gtgggagggg acggcgcgcc
361 attggcttgt gcgcggaacc gagaggaggg accagcgcgg gccgccagac tcgcccttct
421 gcagaggccg ggctggagga gaagatggcg gccaagcagc ccccgcctct gatgaagaag
481 cacagccaga cggacctcgt gagccgcctg aagacccgca agatcctcgg cgtgggcggg
541 gaggatgacg acggggaggt gcatcgctcc aagatcagcc aggtcttagg caatgaaatc
601 aagtttacca ttcgggagcc tttggggctc agggtctggc agttcgtctc tgctgtgctc
661 ttctccggca ttgccatcat ggcgcttgcc ttccctgacc agctctatga tgcggtcttt
721 gatggagccc aggtgaccag caagaccccc atccgcctct acggcggtgc cctcctcaag
781 gtggaaactg agtcagacag aggcgaggtg acttgtctga ggtcacagct agtgagtggt
841 gaagccaaga ttggaaccca aggcaatcca gcttggaagg acaaaggatg gggtctctga
901 aacatctcca gcctcagccc tgctcctggc cttctctctc atccagacat ggctgcagcc
961 tctgggctgg gactggcctc cctacctccc cttcaatcca gtgcttccct gctgccagaa
1021 ggagctttcc agaaagcaca tgtcaccaca gccctctctt gctggaaccc aacctctaca
1081 ggagaccaag ctgcctccta cagaccacag gacctccaag actcaccatc gccacctctt
1141 gttattcctg ccctgacttt cctatcacca ccatcaaagg agagtccccc agagattaca
1201 tctcatggcc cgggcttgcg gagtgctggg ctcccagaca aatgcaatca aaggagacat
1261 gtctcctggg aagagcctcc cacaacctca aagccccgaa agccctccgt gtttatgttc
1321 ttgcttgatc cccacaaccg tccagcaaag ccagcacagc agagatgatt ctatttcccc
1381 aggagtggtg cccagagaag ttaagcagtt tgcctgagat gacacagccg tgttggagcc
1441 gtgcttgctt gggatgaact aagtggcgcg catttgattt tactgctgat ttcacagctc
1501 cttcagctac cccaagctgc catcaatgtc ccatcaattt cactttgtct gctcctctct
1561 gcaatgagct ttctcagctc tcagcctgtg ttcagtaaag agacgttgct ggtcttgtgt
1621 cacgtctcct cctgcctgaa taatgctcgt gtgcagcatg aaggagaggt ctctcagaac
1681 tcaagggcct gaagctccca gaagcttcca gatccagagg caacaggtgt cttccggtcc
1741 cagggagggc aagtttcctg aacctcgaag gcaggcagac agtggaggcc tcccttcctt
1801 ctcacagcat cctccagctc caaggcaggc ttcccccaac ccccagggcc caggaactgt
1861 catcagagca gaaatcaagc ccagtcccag gcctcacccc acagggcatc tgccccattg
1921 atatttgctt ggtaataaat actggagact gagggaaaaa aaaaaaaaaa aaaaaa//

- LOCUS: BF967271 840 bp mRNA linear EST 23-JAN-2001
- DEFINITION: 602287103F1 NIH_MGC_96 Homo sapiens cDNA clone IMAGE:4374504 5',mRNA sequence. *anaphase promoting complex subunit 5 (ANAPC5)*
- ACCESSION: BF967271
- VERSION: BF967271.1 GI:12334486
- KEYWORDS: EST.

1 agcggagtgc tgtgccttgt ggtgactact ggcggcagcg cgccgcgggc ccgagactta
61 gtctcgggcc gccatggcca gcgtccacga gagcctctac ttcaatccca tgatgaccaa
121 tggggttgtg cacgccaatg tgttcggcat caaggactgg gtgacgccgt acaagatcgc
181 ggtgctggtg ctgctgaacg agatgagccg cacaggcgag ggcgccgtca gcctcatgga
241 gcggcggagg ctcaaccagc tgctcctgcc cctgctgcag ggcccagata ttacactgtc
301 aaaactttac aagttaattg aagagtcttg tccacagctg gcaaattcag tgcagatcag
361 aatcaaactg atggctgaag gcgagttgaa ggatatggaa cagttttttg atgacctttc
421 agattctttc tctggaactg aaccagaggt tcacaaaaca agtgtagtag gttgtttctg
481 cgtcacatga tcttggccta cagtaagctt tctttcagcc aagtgtttaa actgtacact
541 gcccttcagc agtacttcca gaatggtgag aaaaagacag tggaggatgc tgatatggaa
601 ctgaccagta gagatgaggt gaaagaaaac attggaacaa gaagcacttg atgtatctgt
661 aagagaagag gaggtattct tgcagtgggg ctctgtccca aaaacaaggc gaatttttc
721 tttctcaacg ggttcttggt aagaatgtga gataagggct cactccggtt ctgggaggat
781 tcacacttgt gaatttattg gttgggaagc ttatcgagat taaaaccggg tcacatgttg
//

- LOCUS: AI367319 485 bp mRNA linear EST 13-FEB-1999
- DEFINITION: qq20h08.x1 Soares_NhHMPu_S1 Homo sapiens cDNA clone IMAGE:1933119 3', mRNA sequence. SRY *(sex determining region Y) -box 10 (SOX10)*
- ACCESSION: AI367319
- VERSION: AI367319.1 GI:4137064
- KEYWORDS: EST.

1 ttttccggaa cctttaattt tattatgtgg aatgcttaat gcagagttaa taggggctag
61 agtggctagg agaggggact actgagatat ataacaggag acagtaatga gttacatgtg
121 gatttggggg gctgcagaac aggaaaatag gggcagatat agctacatac tttattcaaa
181 taccactggg aggcaagggg taagaggcaa gggcagaatg tacaggtcag gattctagtg
241 tcttcagcct gccgacagca acctgggtgc tgggccctgt gtctcctgga gccaagggtt
301 agggcctgag cagctgtcac tctctgggaa gcccagggag tggggctgac tgagcagggg
361 ccatagagcc tagtaaggga agagggagct ggcaaggagc atctggtcat gcttttcctg
421 tccccaaggc ttctgtagag ggactgtgtc ctgactcggc tcanatctgt ggcaacggag
481 tctcc //

- LOCUS: AI042030 476 bp mRNA linear EST 30-JUN-1998
- DEFINITION: ox60a09.x1 Soares_NhHMPu_S1 Homo sapiens cDNA clone IMAGE:1660696 3', mRNA sequence. *SMC1* structural *maintenance of chromosomes 1-like 1* (*yeast*) (*SMC1L1*)
- ACCESSION: AI042030
- VERSION: AI042030.1 GI:3281224
- KEYWORDS: EST.

1 ttttagtgat ggggatttat taaagcattc acagggaaat aaggaagaca agaacagcta
61 cacaggcagc ctcatggaag aacagaaact tgaaaatggt tcatgatcca aagcagctct
121 agagcctggc ttatcttgtc accccctcag ctgcaggcat ctgttgctcc cttctctagt
181 gcttcccaaa ttggtgactc aactcctttc tggctgtatt tatactcctc ctactgctac
241 tgactaactt ctttctgccc ccctcgatgc tagcccatgg ctttttctgc ctcatgggtc
301 ctgcagcttc atggctcccg gcgcttcagt tcctattaat ccatggcttt tctgcattgt
361 agctcctgct gcctcttcca ttcctgttgc catgttgcca catggttctg gctaccttgg
421 gttcttgagt ctcatcgctt ctactgtctc atgatcttat ctattcaagc cttctg//

- LOCUS: AL161955 2827 bp mRNA linear PRI 23-MAR-2000
- DEFINITION: Homo sapiens mRNA; cDNA DKFZp434H0229 (from clone DKFZp434H0229). *triple functional domain (PTPRF interacting*) *(TRIO)*
- ACCESSION: AL161955
- VERSION: AL161955.1 GI:7328007

1 cggaagtcac gggaaggact cagcaacaag gtatctgtga aggtgtgttc ggggtcttc
61 aggagtccgt gtcatcccag catgagagaa aggatcaggg agggcagaga ctctgcagac
121 ctgaagctgg gcttgcttat gacctccctc ccacccacca gccccgtgcc ctgcgtgtcc
181 tgtagggtct tgttaggggc actatgctgg ccagcacagg gatgggtgcg tcctacaagg
241 aaccaggtag acccctgcac acttccgcag gtgcacatgt ggcttctaaa gaatatggtt
301 aggtttgcaa atgaagcaca gagactaggt tctcagaagc agcgaaggtg cagagaagct
361 atggctcagg cttccttcag tgttcctgcc ttaaagccaa ggggaagtgg ctagatgtgg
421 gggtgcatat gggctctctg gctgtgacag atccccttgg ggacaacttc agtcagcttt
481 agtcctataa tcggattgcc tgacacctgc ggtgggggtt gcctggagaa aaagaaggta
541 tctcctgaaa gggactctga gcgccagggg ccagggcgtt ggcggctctg cattaggaac
601 gcatctgagg accagctgga ctcagcctgt agcatgagaa aaacacacat ctaagcagtg
661 tttttgtgtt tgtgggtagg aagaaaaaga cccacccaaa ttagtgtgac atgaaacttt
721 tgagtgcagt gaaaatgtgg tctgttttga ttgatgccca ggcaagtcag tttctgcaaa
781 tctttcaaca ataattgtag ccctggaatg aaaggaatac accttaaagt agctcatttc
841 agttaatgct tgtttgctgt ttccatttca gcttctcaat cccaactaca tttatgacgg
901 tgagttctgt tctttttctt ctagtcctag agatgattct agacctgtgg ggcatgtttc
961 agagcacttg agtgtggcct ctggaaatga gttttccgtg gcgctctagg cgtgcatagc
1021 aggttaggtc ctatcaatct gtcggggaca tgtgggtggg tgtggaggag gagccagggc
1081 tgatgggcct ttaccgactc ctttcccatg cagttccccc agaattcgtc attccattga
1141 gtgaggtcac gtgtgagaca ggggagaccg ttgttcttag atgtcgagtc tgtggccgcc
1201 ccaaagcctc aattacctgg aagggccctg aacacaacac cttgaacaac gatggtcact
1261 acagcatctc ctacagtgac ctgggaaagg ccacgctgaa gattgtgggc gtgaccacgg
1321 aagatgacgg catctacacg tgcatcgctg tcaatgacat gggttcagcc tcatcatcgg
1381 ccagcctgag ggtcctaggt aagcaccgtg caacgaggat tctacgtgac ccagtggggc
1441 acgtctttca ggagtctcct taagtcctga gtctgccctt acactccaag tggcctgaaa
1501 gatagaacaa taagtcattt gtggggcttc aaaagacagt tgtaagtagc agaccagagt
1561 gtctgggatg tcacaggaga gtgacctctc ggcaccttat gtaaagtcgg gggagacacc
1621 ggagctcctg ttgctgggag gaggatgggg gcctgcctgg tgaaggcctc tggctgccca
1681 ccatggggca ccagggcccc ccacgaccag cttctgagtt ggagcagggt gctttccgtg

1741 ctcctagtac tcaaaacact catatccagc tacccagctg aatctggatc tggtctaggt
1801 tggtctcatg tagaatgtgc atgggaaaag caggaaagga gatgtgggtg agtcccgggc
1861 ctgcgcaggc cctgtgcctg tctgagaccc taacagcaac ccttctgggt gaatgcacgc
1921 cctcctcacc ccatccagga ggagccaggc ccagggcggc tcagtagctt gtccagagca
1981 tctggctagg aggcatctgc catggggcct gcttgctcca gtgtgaagag cttgttcttt
2041 ctcatcagct aaaccacaag ctccctgggg cagaaccaat atcttaggct ctttcaaaac
2101 gtcctgtagt tgcctgatgt agatagctgc tgagaaaggt gtggactgtt ctctgctgcc
2161 atcttgcaga aggccggcca tgcagtactg ggctgcacca caaatggagg tgcctccaga
2221 ggacaaataa aactccaaat gaaagataaa ttgagtcctg gagcaccccg tctgtagaca
2281 ataagcagaa tttgtcttag aacttttgtc ccagatatct taatttcctg tttttaacaa
2341 cattaaatca gaagacatgt tctgattttt tggatcatgg aacaagaaat ctaaaactct
2401 agaatttggc agtaaacaat aaaaggtaaa gattttctaa tttgtaaata acacagaaat
2461 tacacaggtg attttttttt tttaatcact ggcaaaagct aaaggacttt agaaaggtat
2521 actattcagc tgggcgtagt ggctcacacc tgtaatccca gcgctttggg aggccgaggt
2581 gggtggatca ccaggtcagg agatcgagac catcgtggct aacacagtga aaccctgtct
2641 ctgctaaaaa taacaaaaaa ttagccggcg tggtggcaag cgcctgtagt cccagctact
2701 cttgaggctg aggcaggaga atcacttgaa cccaggaggc agaggttgca gtgagccgag
2761 atcatgccac tgcactccag cctgggtgac agagcgagac tctgtctaaa aaaaaaaaaa
2821 aaaaaaa //

- LOCUS: AK001619 3405 bp mRNA linear PRI 12-SEP-2006
- DEFINITION: Homo sapiens cDNA FLJ10757 fis, clone NT2RP3004578, highly similar to Homo sapiens mRNA for KIAA0477 protein. *phosphodiesterase 4D interacting protein (myomegalin)* (*PDE4DIP*)
- ACCESSION: AK001619
- VERSION: AK001619.1 GI:7022984
- KEYWORDS: FLI_CDNA; oligo capping; fis (full insert sequence).

1 gtgcaatttg tagaggctgc agcacacgag agtgaacagc agaaagaggc ttcttggaaa
61 cataaccagg aattgcgaaa agccttgcag cagctacaag aagtattgca gaataagagc
121 caacagcttc gtgcctggga ggctgaaaaa tacaatgaga ttcgaaccca ggaacaaaac
181 atccagcacc taaaccatag tctgagtcac aaggagcagt tgcttcagga atttcgggag
241 ctcctacagt atcgagataa ctcagacaaa acccttgaag caaatgaaat gttgcttgag
301 aaacttcgcc agcgaatacg tgataaagct gttgctctgg agcgggctat agatgaaaaa
361 ttctctgctc tagaagagaa agaaaaagaa ctgcgccagc ttcgtcttgc tgtgagagag
421 cgagatcatg acctagagag actgcgcgat gtcctctcct ccaatgaagc tactatgcaa
481 actatggaga gtctcctgag ggccaaaggc ctggaagtgg aacagttatc tactacctgt
541 caaaacctcc agtggctgaa agaagaaatg gaaaccaaat ttagccgttg gcagaaggaa
601 caagagagta tcattcagca gttacagacg tctcttcatg ataggaacaa agaagtggag
661 gatcttagtg caacattgct ctgcaaactt ggaccaggcc agagtgagat agcagaggag
721 ctgtgccagc gtctacagtg aaaggaaagg atgctgcagg accttctaag tgatcgaaat
781 aaacaagtgc tggaacatga aatggagatt caaggcctgc ttcagtctgt gagcaccagg
841 gagcaggaaa gccaagctgc tgcagagaag ttggtgcaag ccttaatgga aagaaattca
901 gaattacagg ccctgcacca atatttagga gggagagact ccctgatgtc ccaagcacac
961 atctctaacc aacaagctga agttaccccc actggccgtc ttggaaaaca gactgatcaa
1021 ggttcaatgc agataccttc cagagatgat agcacttcat tgactgccaa agaggatgtc
1081 agcataccca gatccacatt aggagacttg gacgcagttg caggctggaa aaaagaactg
1141 agtaatgcca aagaggaact tgaactcatg gctaaaaaag aaagagaaag tcagctttac
1201 ccctcaaaca tgggagaaaa agtttcagca ttttaattca aattggtaaa aggctgagca
1261 acccttaaag ctgccataat ggaagaagac tgtgagctag cttaaaaaag agagaatgaa
1321 ggggaagaaa caaaacccct gtgtggcaga gtgtcgagat gagatgagca tttatttgtc
1381 tccgtcgaat tccttacgga gataaggtgc agctgcaggg gatgacaccg aagatacaag
1441 cactgagttc actgacagta ttgaggagga ggctgcacac catagtcacc agcaagtaag
1501 tcagtgtcag ggtctgggac ttccagggtg gacagcacac agcccttctg aggtctgata
1561 gtctcctgca gcccttgcgg acttagtgag aagatatctt tttacataaa ctacttctca
1621 gtcatctacc tgttctcctt gaacttccca gctgcctttc caacccatat cttcactcca
1681 tttttctctc caatccaccc tttggtttgt attgtatctc tttcttctgc ttccttcccc
1741 ctcctaccca cctttgcccc tatttattct ctgtagctat agcttcagaa gcatttttac
1801 ttgcaagacg atggacacat tccccttggg ctttttgtaa ctgaaatgca tcacagaaga
1861 cagggagtca tcgaagggct gctcggggag gtggcagggc ggaggaactg cttgggaaga
1921 aactccaaga agattggaat gcttccaaag caagaatctt tctcagtgaa atctcattat
1981 acaaagagaa ccttatgcaa cctgacaaac cactgaggtc atggtgactc agtgatcagc

2041 agatggtact tcaacagcaa tcccctgtca aacctcagaa cttgaggctg aaacattgct
2101 tccacccacc atcagtgaag atgtaactag catgttacaa gagtgaataa tctggacttc
2161 agagattaag tcaccaatag tgatctcaca agtactcacc ggaactccta taatgtctcc
2221 actttgtcca tgccatttag caatctcatc tcctaaatgg actgtgccta tgattcttaa
2281 ggagaaagtg aatcattggt agatatcctg cacaagcagc tggactttcc agtaatagct
2341 ttcttggggc tattaggaaa attaaacaag aaatgaggct ttctgggtct gcctgtatgt
2401 cttctgcata agaaaaagaa gagacatcga atcaaccaat aagaagagcc caaataagca
2461 tcctcaaatc ttttgggatt tggcacttgg ggacatgagt agttgtctgg gatacgtcat
2521 attctcaaca gtttctttgt agtagtagga tcaccttctt ataataggat caccttcttg
2581 ttgctatagc tgtacccgac cttcccttct cccttgagtg cttgcatgag ctccactttt
2641 ccttttgctt gaacagcttc tcctgagtcc tccttaccga tggttgtgac tttaattata
2701 tacatctctg tccctccaga cagatccctc tgtcctcact ctctgatttc attgaggatc
2761 ttgggtgaga gagagggacc tgcaggatga acaaatgtct actctaagac agctagattg
2821 ggaggttggc tggtcactga tggttataat gactgtggga caggattaac ttcagaataa
2881 atgaacagga gacacagatg tgaagaaagt ttctgattga tatggtctga agtactcctg
2941 gtattgcaag tcatttgctc taattctcaa ttgtaggcaa actgatttgt aaatttgctt
3001 cttcagcctt ctttcctgta gcctagcatg gagaatctga ccagacccca ttttgagaag
3061 gtcagcctac actggaatga actttttaca ttagggcatt tgtatttccc tcacaatact
3121 tgccacatta cttggcatag gagagatgct tagtgtaatt atgagttaac aagcctttgg
3181 atcagggctt gactcatgat agacaaagta tatgcctgct ggatggaaga atctcttggg
3241 cgagcaccat ttttctttcc atcacctttc cttgaaaata tatcttcagc tttgggtagg
3301 aggaatcttg gtgtatgaaa tcattgcaaa tttacttcat cttttctgga gtttgaagtt
3361 gtgactctcc tgctaccaat taaataaagc ttactttgcc ataac//

- LOCUS: AW296788 417 bp mRNA linear EST 16-JAN-2000
- DEFINITION: UI-H-BWO-ajb-d-08-0-UI.s1 NCI_CGAP_Sub6 Homo sapiens cDNA clone IMAGE:2731071 3', mRNA sequence. *microtubule-associated protein* 1A *(MAP1A)*
- ACCESSION: AW296788
- VERSION: AW296788.1 GI:6703424
- KEYWORDS: EST.

1 tttttttttt tttttttaca ctatctgcaa tgaacttggt ttattgttag agttggaggt
61 ggctccgtta cagcagctgg gaactatagc agggaagcgt ggggaggagc agaacacagc
121 agcacgggga gggtggagct aggaagaaca caaggtgggc tcattttggt ttcccagtcg
181 tttggcagag aaggcaagcc cacatgacta ccagcttctg ggtttctcag actggcagta
241 gtctggcagg gctgctcact ggcttgcttg gggcgaggtc ctctcccggg gtggtatttt
301 cagcccgttt tcatttcact ctgttagagt taagctgtgg ctcagcttct ccctttttcc
361 ttcctccagc tcattttccc caagtcctct gggctggctt ccacctccca gatcctc//

- LOCUS: N80922 542 bp mRNA linear EST 29-MAR-1996
- DEFINITION: zb07e07.s1 Soares_fetal_lung_NbHL19W Homo sapiens cDNA clone IMAGE:301380 3', mRNA sequence. *solute carrier family 35, member D1 (SLC35D1)*
- ACCESSION: N80922
- VERSION: N80922.1 GI:1243623
- KEYWORDS: EST.

1 aaaaaaagga gggggagtag ggggaagaaa ataattttcc tttattaaaa tgtactgtnn
61 caatataaaa gaaattttgt tctttttaaa aaataggtag aatcacctgc atcatgtaat
121 ggccgtaatt gtgtccagca gatattttta aaaaagaaaa gacagtgaac agaaactgct
181 acctaaggat tctagaggca atgagacaca gagtcctgtt tttcaggaca gaccgtattg
241 cccaagcctc taagcattta cggagcataa attaagaatc ttgttttgcc ttgagtctca
301 ccatgaaata atgacacggt agccacattt ggctggaggg cctaatatgt accaggcatt
361 gtgctaagta gtttcatatc catgtctcat ttattccgaa cttttttcct cagggagaga
421 aaaatcaatt acagtcttgt tcataggaaa aaccaaactt acaattttgg gaaagccaga
481 acatgtacag tagaggagga ggcctcaagc tacttacaga attctaagag ctgtggtgct
541 at //

- LOCUS: AF087138 4826 bp mRNA linear PRI 23-SEP-1998
- DEFINITION: Homo sapiens sulfonylurea receptor 1 (SUR1) mRNA, complete cds. *ATP-binding cassette*, *sub-family C (CFTR*/*MRP), member 8 (ABCC8)*
- ACCESSION: AF087138

- VERSION: AF087138.1 GI:3643189

1 agctgagccc gagcccagac cgcgcccgcg ccgccatgcc cctggccttc tgcggcagcg
61 agaaccactc ggccgcctac cgggtggacc agggggtcct caacaacggc tgctttgtgg
121 acgcgctcaa cgtggtgccg cacgtcttcc tactcttcat caccttcccc atcctcttca
181 ttggatgggg aagtcagagc tccaaggtgc acatccacca cagcacatgg cttcatttcc
241 ctgggcacaa cctgcggtgg atcctgacct tcatgctgct cttcgtcctg gtgtgtgaga
301 ttgcagaggg catcctgtct gatggggtga ccgaatccca ccatctgcac ctgtacatgc
361 cagccgggat ggcgttcatg gctgctgtca cctccgtggt ctactatcac aacatcgaga
421 cttccaactt ccccaagctg ctaattgccc tgctggtgta ttggaccctg gccttcatca
481 ccaagaccat caagtttgtc aagttcttgg accacgccat cggcttctcg cagctacgct
541 tctgcctcac agggctgctg gtgatcctct atgggatgct gctcctcgtg gaggtcaatg
601 tcatcagggt gaggagatac atcttcttca agacaccgag ggaggtgaag cctcccgagg
661 acctgcaaga cctgggggta cgcttcctgc agcccttcgt gaatctgctg tccaaaggca
721 cctactggtg gatgaacgcc ttcatcaaga ctgcccacaa gaagcccatc gacttgcgag
781 ccatcgggaa gctgcccatc gccatgaggg ccctcaccaa ctaccaacgg ctctgcgagg
841 cctttgacgc ccaggtgcgg aaggacattc agggcactca aggtgcccgg gccatctggc
901 aggcactcag ccatgccttc gggaggcgcc tggtcctcag cagcactttc cgcatcttgg
961 ccgacctgct gggcttcgcc gggccactgt gcatctttgg gatcgtggac caccttggga
1021 aggagaacga cgtcttccag cccaagacac aatttctcgg ggtttacttt gtctcatccc
1081 aagagttcct tgccaatgcc tacgtcttag ctgtgcttct gttccttgcc ctcctactgc
1141 aaaggacatt tctgcaagca tcctactatg tggccattga aactggaatt aacttgagag
1201 gagcaataca gaccaagatt tacaataaaa ttatgcacct gtccacctcc aacctgtcca
1261 tgggagaaat gactgctgga cagatctgta atctggttgc catcgacacc aatcagctca
1321 tgtggttttt cttcttgtgc ccaaacctct gggctatgcc agtacagatc attgtgggtg
1381 tgattctcct ctactacata ctcggagtca gtgccttaat tggagcagct gtcatcattc
1441 tactggctcc tgtccagtac ttcgtggcca ccaagctgtc tcaggcccag cggagcacac
1501 tggagtattc caatgagcgg ctgaagcaga ccaacgagat gctccgcggc atcaagctgc
1561 tgaagctgta cgcctgggag aacatcttcc gcacgcgggt ggagacgacc cgcaggaagg
1621 agatgaccag cctcagggcc tttgccatct atacctccat ctccattttc atgaacacgg
1681 ccatccccat tgcagctgtc ctcataactt tcgtgggcca tgtcagcttc ttcaaagagg
1741 ccgacttctc gccctccgtg gcctttgcct ccctctccct cttccatatc ttggtcacac
1801 cgctgttcct gctgtccagt gtggtccgat ctaccgtcaa agctctagtg agcgtgcaaa
1861 agctaagcga gttcctgtcc agtgcagaga tccgtgagga gcagtgtgcc ccccatgagc
1921 ccacacctca gggcccagcc agcaagtacc aggcggtgcc cctcagggtt gtgaaccgca
1981 agcgtccagc ccgggaggat tgtcggggcc tcaccggccc actgcagagc ctggtcccca
2041 gtgcagatgg cgatgctgac aactgctgtg tccagatcat gggaggctac ttcacgtgga
2101 ccccagatgg aatccccaca ctgtccaaca tcaccattcg tatcccccga ggccagctga
2161 ctatgatcgt ggggcaggtg ggctgcggca agtcctcgct ccttctagcc gcactggggg
2221 agatgcagaa ggtctcaggg gctgtcttct ggagcagcct tcctgacagc gagataggag
2281 aggaccccag cccagagcgg gagacagcga ccgacttgga tatcaggaag agaggccccg
2341 tggcctatgc ttcgcagaaa ccatggctgc taaatgccac tgtggaggag aacatcatct
2401 ttgagagtcc cttcaacaaa caacggtaca agatggtcat tgaagcctgc tctctgcagc
2461 cagacatcga catcctgccc catggagacc agacccagat tggggaacgg ggcatcaacc
2521 tgtctggtgg tcaacgccag cgaatcagtg tggcccgagc cctctaccag cacgccaacg
2581 ttgtcttctt ggatgacccc ttctcagctc tggatatcca tctgagtgac cacttaatgc
2641 aggccggcat ccttgagctg ctccgggacg acaagaggac agtggtctta gtgacccaca
2701 agctacagta cctgccccat gcagactgga tcattgccat gaaggatggc accatccaga
2761 gggagggtac cctcaaggac ttccagaggt ctgaatgcca gctctttgag cactggaaga
2821 ccctcatgaa ccgacaggac caagagctgg agaaggagac tgtcacagag agaaaagcca
2881 cagagccacc ccagggccta tctcgtgcca tgtcctcgag ggatggcctt ctgcaggatg
2941 aggaagagga ggaagaggag gcagctgaga gcgaggagga tgacaacctg tcgtccatgc
3001 tgcaccagcg tgctgagatc ccatggcgag cctgcgccaa gtacctgtcc tccgccggca
3061 tcctgctcct gtcgttgctg gtcttctcac agctgctcaa gcacatggtc ctggtggcca
3121 tcgactactg gctggccaag tggaccgaca gcgccctgac cctgacccct gcagccagga
3181 actgctccct cagccaggag tgcaccctcg accagactgt ctatgccatg gtgttcacgg
3241 tgctctgcag cctgggcatt gtgctgtgcc tcgtcacgtc tgtcactgtg gagtggacag
3301 ggctgaaggt ggccaagaga ctgcaccgca gcctgctaaa ccggatcatc ctagccccca
3361 tgaggttttt tgagaccacg ccccttggga gcatcctgaa cagattttca tctgactgta
3421 acaccatcga ccagcacatc ccatccacgc tggagtgcct gagccgctcc accctgctct
3481 gtgtctcagc cctggccgtc atctcctatg tcacacctgt gttcctcgtg gccctcttgc
3541 ccctggccat cgtgtgctac ttcatccaga agtacttccg ggtggcgtcc agggacctgc
3601 agcagctgga tgacaccacc cagcttccac ttctctcaca ctttgccgaa accgtagaag
3661 gactcaccac catccgggcc ttcaggtatg aggcccggtt ccagcagaag cttctcgaat

3721 acacagactc caacaacatt gcttccctct tcctcacagc tgccaacaga tggctggaag
3781 tccgaatgga gtacatcggt gcatgtgtgg tgctcatcgc agcggtgacc tccatctcca
3841 actccctgca cagggagctc tctgctggcc tggtgggcct gggccttacc tacgccctaa
3901 tggtctccaa ctacctcaac tggatggtga ggaacctggc agacatggag ctccagctgg
3961 gggctgtgaa gcgcatccat gggctcctga aaaccgaggc agagagctac gaggggctcc
4021 tggcaccatc gctgatccca aagaactggc cagaccaagg gaagatccag atccagaacc
4081 tgagcgtgcg ctacgacagc tccctgaagc cggtgctgaa gcacgtcaat gccctcatct
4141 cccctggaca gaagatcggg atctgcggcc gcaccggcag tgggaagtcc tccttctctc
4201 ttgccttctt ccgcatggtg gacacgttcg aagggcacat catcattgat ggcattgaca
4261 tcgccaaact gccgctgcac accctgcgct cacgcctctc catcatcctg caggaccccg
4321 tcctcttcag cggcaccatc cgatttaacc tggaccctga gaggaagtgc tcagatagca
4381 cactgtggga ggccctggaa atcgcccagc tgaagctggt ggtgaaggca ctgccaggag
4441 gcctcgatgc catcatcaca gaaggcgggg agaatttcag ccagggacag aggcagctgt
4501 tctgcctggc ccgggccttc gtgaggaaga ccagcatctt catcatggac gaggccacgg
4561 cttccattga catggccacg gaaaacatcc tccaaaaggt ggtgatgaca gccttcgcag
4621 accgcactgt ggtcaccatc gcgcatcgag tgcacaccat cctgagtgca gacctggtga
4681 tcgtcctgaa gcggggtgcc atccttgagt tcgataagcc agagaagctg ctcagccgga
4741 aggacagcgt cttcgcctcc ttcgtccgtg cagacaagtg acctgccaga gcccaagtgc
4801 catcccacat tcggaccctg cccata//

- LOCUS: AW086154 494 bp mRNA linear EST 14-OCT-1999
- DEFINITION: xc77c06.x1 NCI_CGAP_Ov32 Homo sapiens cDNA clone IMAGE:2590282 3'similar to TR:O14564 014564 HYPOTHETICAL 67.1 KD PROTEIN. ;, mRNA sequence. *golgi associated, gamma adaptin* ear *containing,* ARF *binding protein* 2 (GGA2)
- ACCESSION: AW086154
- VERSION: AW086154.1 GI:6041228
- KEYWORDS: EST.

1 ccgagtcggc ccagggtccc ccgggcccgg cagcgtcgct ggagctgtgg ctgaacaaag
61 ccacagaccc aagcatgtcg gaacaggatt ggtcagctat ccagaatttc tgtgagcagg
121 tgaacactga ccccaatggc cccacacatg cgccctggct actggcccac aagatccagt
181 ctccgcaaga gaaggaagct ctttatgcct taacggtgag tttggcttgc cttgtagcct
241 aacctttcct gtccttgtgc tatagagagc cgagaggcgc tttgcttcac acagatcctt
301 ttactggaga ggggaggtcc ctgagaggtt accttaagca gttgagaggg ggagatctgg
361 gccagggtcc caccctcttc tcctgtnctc gtctgttctc aggcacattg atctatgtgg
421 agacgaaggg gaagttttat ttatttgcct tcttaacagg gctggcagtc atgtctttta
481 gagaaggcag ttta//

- LOCUS: HSU72069 2909 bp mRNA linear PRI 25-MAY-1997
- DEFINITION: Human karyopherin beta2 mRNA, complete cds. *transportin* 1 *(TNP01)*
- ACCESSION: U72069
- VERSION: U72069.1 GI:1657775

1 ggggagcagt tccgccgggt ttcactgtcc gtgacttcct tcggggcacc tccaggagca
61 aaccaagatg gagtatgagt ggaaacctga cgagcaaggg cttcagcaaa tcctgcagct
121 gttgaaggag tcccagtccc cagacaccac catccagaga accgtgcaac aaaaactgga
181 acaacttaat cagtatccag actttaacaa ctacttgatt tttgttctta caaaattaaa
241 atctgaagat gaacccacaa gatcattgag tggtcttatc ttgaagaata atgtgaaagc
301 acactttcag aacttcccaa atggtgtaac agactttatt aaaagtgaat gtttaaataa
361 tattggtgac tcctctcctc tgattagagc cactgttggt attttgatca caactatagc
421 ctccaaggga gaattgcaga attggcctga cctcttacca aaactctgta gcctgttgga
481 ttctgaagat tataatacct gtgagggagc atttggtgcc cttcagaaga tttgtgaaga
541 ttctgctgag attttagaca gtgatgtttt agatcgtcct ctcaacatca tgattcccaa
601 atttttacag ttcttcaagc atagtagtcc aaaaataagg tctcacgctg ttgcatgtgt
661 caatcagttt atcatcagta ggactcaagc tctaatgttg cacattgatt cttttattga
721 gaatctcttt gcattagctg gtgatgaaga accagaggta cggaaaaatg tgtgccgagc
781 acttgtgatg ttgctcgaag ttcgaatgga tcgcctgctt cctcacatgc ataatatagt
841 tgagtacatg ctacagagga ctcaagatca agatgaaaat gtggctttag aagcctgtga
901 attttggcta actttagctg aacagccaat atgcaaagat gtactcgtaa ggcatcttcc
961 taagttgatt cctgtgttag tgaatggcat gaagtactca gacatagata ttatcctact
1021 taagggtgat gttgaagaag acgaaacgat tcctgatagt gaacaggata tacggccacg

1081 ttttcaccga tcgaggacgg tggctcagca gcatgatgaa gatggaattg aagaggaaga
1141 tgatgatgat gatgaaattg atgatgatga tacaatttct gactggaatc taagaaaatg
1201 ttctgctgct gccctggatg ttcttgcaaa tgtgtatcgt gatgaactgc tgccacatat
1261 tttgcccctt ttgaaagaat tactttttca tcatgaatgg gttgttaaag aatcaggcat
1321 tttggtttta ggagcaattg ctgaaggttg catgcagggc atgattccat acttgcctga
1381 gcttattcct caccttattc agtgcctctc tgataaaaag gctcttgtgc gttccataac
1441 atgctggact cttagccgct atgcacactg ggtggtcagc cagccgccag acacgtacct
1501 gaagccatta atgacagaat tgctaaagcg catcctggac agcaacaaga gagtacaaga
1561 agctgcctgc agtgcctttg ctaccctaga agaggaggct tgtacagaac ttgttcctta
1621 ccttgcttat atacttgata ccctggtctt tgcatttagt aaataccagc ataagaacct
1681 gctcattctt tacgatgcca taggaacatt agcagattca gtaggacatc atttaaacaa
1741 accagaatat attcagatgc taatgcctcc actgatccag aaatggaaca tgttaaagga
1801 tgaagataaa gatctcttcc ctttacttga gtgcctatct tcagttgcca cagcactgca
1861 gtctggattc cttccgtact gtgaacctgt gtatcagcgt tgtgtaaacc tagtacagaa
1921 gactcttgca caagccatgc taaacaatgc tcaaccagat caatatgaag ctccagataa
1981 agattttatg atagtggctc ttgatttact gagtggcctg gctgaaggac ttggaggcaa
2041 cattgaacag ctggtagccc gaagtaacat cctgacacta atgtatcagt gcatgcagga
2101 taaaatgcca gaagttcgac agagttcttt tgccctgtta ggtgacctca caaaagcttg
2161 ctttcagcat gttaagcctt gtatagctga tttcatgcca atattgggaa ccaacctaaa
2221 tccagaattc atttcagtct gcaacaatgc cacatggcca attggagaaa tctccattca
2281 aatgggtata gagatgcagc cttatattcc tatggtgttg caccagcttg tagaaatcat
2341 taacagaccc aacacaccaa agacgttgtt agagaataca gcaataacaa ttggtcgtct
2401 tggttacgtt tgtcctcaag aggtggcccc catgctacag cagtttataa gaccctggtg
2461 cacctctctg agaaacataa gagacaatga ggaaaaggat tcagcattcc gtggaatttg
2521 taccatgatc agtgtgaatc ccagtggcgt aatccaagat tttatatttt tttgtgatgc
2581 cgttgcatca tggattaacc caaaagatga tctcagagac atgttctgta agatccttca
2641 tggatttaaa aatcaagttg gcgatgaaaa ttggaggcgt ttctctgacc agtttcctct
2701 tcccttaaaa gagcgtcttg cagcttttta tggtgtttaa tctaatacac ttaagctgca
2761 gtcccaaaat taggggtcct tcagtcttgg agactataag ggagcctctg cacccaggga
2821 aaatgttacc ctttacaggg gggaagggta aaccagtagg gaatacagta caatcccaac
2881 cctactggag ggcggggagg ggaggtgtt//

- LOCUS: NM_024844 2311 bp mRNA linear PRI 18-NOV-2006
- DEFINITION: Homo sapiens nucleoporin 85kDa (NUP85), mRNA. *pericentrin 1 (PCNT1)*
- ACCESSION: NM_024844
- VERSION: NM_024844.3 GI:82880675

1 cggtactctg agtgcagtca gtcctcggcg ctgttttccc tgatttgctg tcgacgccag
61 gaaggaagga cgcgtgcaga ggaccgcaga ggggtggccg tggctgagag gagacagcgc
121 cgcagcactg agggtttggg cttgcaggcg ctgcaggaga cgcccaggcg gagtcttgtc
181 tcgcagccag ctctgagcgg gaggcctgag cgggaagcat tggcgtccga gcgacttcta
241 ggagcctggg gttcggcgct atggaggagc tcgatggcga gccaacagtc actttgattc
301 caggcgtgaa ttccaagaag aaccaaatgt attttgactg gggtccaggg gagatgctgg
361 tatgtgaaac ctccttcaac aaaaaagaaa aatcagagat ggtgccaagt tgccccttta
421 tctatatcat ccgtaaggat gtagatgttt actctcaaat cttgagaaaa ctcttcaatg
481 aatcccatgg aatctttctg ggcctccaga gaattgacga agagttgact ggaaaatcca
541 gaaaatctca attggttcga gtgagtaaaa actaccgatc agtcatcaga gcatgtatgg
601 aggaaatgca ccaggttgca attgctgcta aagatccagc caatggccgc cagttcagca
661 gccaggtctc cattttgtca gcaatggagc tcatctggaa cctgtgtgag attcttttta
721 ttgaagtggc cccagctggc cctctcctcc tccatctcct tgactgggtc cggctccatg
781 tgtgcgaggt ggacagtttg tcggcagatg ttctgggcag tgagaatcca agcaaacatg
841 acagcttctg gaacttggtg accatcttgg tgctgcaggg ccggctggat gaggcccgac
901 agatgctctc caaggaagcc gatgccagcc ccgcctctgc aggcatatgc cgaatcatgg
961 gggacctgat gaggacaatg cccattctta gtcctgggaa cacccagaca ctgacagagc
1021 tggagctgaa gtggcagcac tggcacgagg aatgtgagcg gtacctccag gacagcacat
1081 tcgccaccag ccctcacctg gagtctctct tgaagattat gctgggagac gaagctgcct
1141 tgttagagca gaaggaactt ctgagtaatt ggtatcattt cctagtgact cggctcttgt
1201 actccaatcc cacagtaaaa cccattgatc tgcactacta tgcccagtcc agcctggacc
1261 tgtttctggg aggtgagagc agcccagaac ccctggacaa catcttgttg gcagcctttg
1321 agtttgacat ccatcaagta atcaaagagt gcagcatcgc cctgagcaac tggtggtttg
1381 tggcccacct gacagacctg ctggaccact gcaagctcct ccagtcacac aacctctatt
1441 tcggttccaa catgagagag ttcctcctgc tggagtacgc ctcgggactg tttgctcatc
1501 ccagcctgtg gcagctgggg gtcgattact ttgattactg ccccgagctg ggccgagtct

1561 ccctggagct gcacattgag cggatacctc tgaacaccga gcagaaagcc ctgaaggtgc
1621 tgcggatctg tgagcagcgg cagatgactg aacaagttcg cagcatttgt aagatcttag
1681 ccatgaaagc cgtccgcaac aatcgcctgg gttctgccct ctcttggagc atccgtgcta
1741 aggatgccgc ctttgccacg ctcgtgtcag acaggttcct cagggattac tgtgagcgag
1801 gctgcttttc tgatttggat ctcattgaca acctggggcc agccatgatg ctcagtgacc
1861 gactgacatt cctgggaaag tatcgcgagt tccaccgtat gtacggggag aagcgttttg
1921 ccgacgcagc ttctctcctt ctgtccttga tgacgtctcg gattgcccct cggtctttct
1981 ggatgactct gctgacagac gccttgcccc ttttggaaca gaaacaggtg atttctcag
2041 cagaacagac ttatgagttg atgcggtgtc tggaggactt gacgtcaaga agacctgtgc
2101 atggagaatc tgataccgag cagctccagg atgatgacat agagaccacc aaggtggaaa
2161 tgctgagact ttctctggca cgaaatcttg ctcgggcaat tataagagaa ggctcactgg
2221 aaggttcctg agaactgctt caatgtggta tctttgtatg gcaatgtata tagattttt
2281 taaaagaata aatgttgttt tgcaaatgta g//

- LOCUS: AA393484 530 bp mRNA linear EST 12-AUG-1997
- DEFINITION: zt71f01.r1 Soares_testis_NHT Homo sapiens cDNA clone IMAGE:727801 5' similar to SW:PIP1_BOVIN P10894 1- PHOSPHATIDYLINOSITOL-4 ,5-BISPHOSPHATE PHOSPHODIESTERASE BETA 1 ;, mRNA sequence. *phospholipase C, beta 1 (phosphoinositide-specific) (PLCB1)*
- ACCESSION: AA393484
- VERSION: AA393484.1 GI:.2046452
- KEYWORDS: EST.

1 ccaacgttta ctccccccaa ccctcaagct ctcaagtggt gaccacgtcc ttccggccag
61 ctgcaggtgg agctggagca agaataccaa gacaaattca aaagactgcc cctcgagatt
121 ttggaattcg tgcaggaagc catgaaagga aagatcagtg aagacagcaa tcacggttct
181 gcccctctct ccctgtcctc agaccctgga aaagtgaacc acaagactcc ctccagtgag
241 gagctgggag gagacatccc aggaaaagaa tttgatactc ctctgtgaat gctcctgcca
301 ggccttcaga aattgcatgg ccactccagc gtcatcggac tctctcttat tacaaagatc
361 actgcccagg accatcttcc cgagaagcat cccttagcct aaaatccaca ccaaagggag
421 agttccagag gaatccatga agaattccca tgcccaggct ccatgtgtca tgtggaaacc
481 tccacaggtc tgctagtgaa gaatgcatgt atgtgagatt tttgttttct//

- LOCUS: BE466525 661 bp mRNA linear EST 27-JUL-2000
- DEFINITION: hx94b10.x1 NCI_CGAP_Kid11 Homo sapiens cDNA clone IMAGE:3195451 3'similar to gb:S69002 ECOTROPIC VIRUS INTEGRATION 1 SITE PROTEIN (HUMAN);, mRNA sequence. *ecotropic viral integration site 1 (EVI1)*
- ACCESSION: BE466525
- VERSION: BE466525.1 GI:9512223
- KEYWORDS: EST.

1 tttaaatcat tcagtttaag gtcactagac tttagatgag tgaccctgca ggtttataag
61 gcattctgct cagcagtctt gtaaatagtc ctatatgaaa gagccatgct actgttggac
121 ttggtcccac tctggtcaac cttgataacg tcatacgtgg cttatggact ggatagcact
181 ggattccgcc gcagccctgg ccatactgtg ccacacgttg gaagaactgt gggatgtaga
241 atggagggac tccttgtcag acagtgacag catcatagca tatgcctgcg atttggactt
301 tctgtgtaac ggctgcttaa gttcctctgg cacatgggaa gtactaaaag aagacagctc
361 agcttcagaa tattgattat cttccatttt cctcattttg aggctatctg tgaagtgcct
421 tatatgatct agagcagaaa gtccactttt atattcttcc tctttatacc tcactgggga
481 tgtcttgcaa ctcatctcca aggcactggt ttcttcatag tcatcctcag ggtttcctc
541 atgtaaatta cttgtcactg gttcctttcc tgttttgcag tgatatcatt ggcttcatgc
601 tgctcatcta acaacacctc atcttcaact tctttatcat ccagcaagtc tggattttga
661 c//

- LOCUS: AF095723 743 bp mRNA linear PRI 13-FEB-2002
- DEFINITION: Homo sapiens GABA-B receptor splice variant 1 mRNA, partial cds. *G protein-coupled receptor 51(GPR51)*
- ACCESSION: AF095723
- VERSION: AF095723.1 GI:3719471

1 atcaccctct gcctggtatt cgtgccgaag ctcatcaccc tgagaacaaa cccagatgca
61 gcaacgcaga acaggcgatt ccagttcact cagaatcaga agaaagaaga ttctaaaacg

121 tccacctcgg tcaccagtgt gaaccaagcc agcacatccc gcctggaggg cctacagtca
181 gaaaaccatc gcctgcgaat gaagatcaca gagctggata aagacttgga agaggtcacc
241 atgcagctgc aggacacacc agaaaagacc acctacatta aacagaacca ctaccaagag
301 ctcaatgaca tcctcaacct gggaaacttc actgagagca cagatggagg aaaggccatt
361 ttaaaaaatc acctcgatca aaatccccag ctacagtgga acacaacaga gccctctcga
421 acatgcaaag atcctataga agatataaac tctccagaac acatccagcg tcggctgtcc
481 ctccagctcc ccatcctcca ccacagacat gtgccaccct ccttccgagt catggtctcg
541 ggcctgtaag ggtgggaggc ctgggcccgg ggcctccccc gtgacagaac cacactgggc
601 agaggggtct gctgcagaaa cactgtcggc tctggctgcg gagaagctgg gcaccatggc
661 tggcctctca ggaccactcg gatggcactc aggtggacag gacggggcag ggggagactt
721 ggcacctgac ctcgagcctt att//

- LOCUS: AC004794 39854 bp DNA linear PRI 04-JUN-1998
- DEFINITION: Homo sapiens chromosome 19, cosmid F20569, complete sequence. *olfactory receptor, family* 1, *subfamily* I, *member 1 (OR1I1)*
- ACCESSION: AC004794
- VERSION: AC004794.1 GI:3184261
- KEYWORDS: HTG.

1 gatccaagag ccaacagtcc aacagccaat attattaggg aaagtaattc taatatttac
61 taaagcccct aggagaattt caacaagtat cctacacacg ttctgtgtgg ctgataggaa
121 ggattttttt tttaagagac agggtctcac tccgttgtcc aggttggaat gcagtggtgc
181 gatcacagct cactgcagcc tcaacctcca gggctcaagt gatcctccca cctcagcctc
241 ctgggtaact gagaccacgg gcatgtgcca ccaatcctgg ctaatttttt taaatttttt
301 gtagatataa ggtcttgctg ttttgcccaa gctggtctca aacttctagg ctcaagggat
361 tctccaacct tggcctccca agtagctagg attataggca tgagccaccg tgtccaactc
421 cagtgccaag caggtgatca agaagtgttt gataggccgg gtacagtggc tcaggcctgt
481 aatcccagca ctttgggaga ccgaggagag cagatcactt gaggtttgag atcagtcttt
541 ccaacatggt gaaaccctgt ctctactaaa aatacaaaga ctagctgggc atggtggtgg
601 gcaccagtaa ttccagctac tcaagaggct gaggagggag aattgctaga acccaggagg
661 tggaggttgc agtgagccga gattgctcca ctgtgctcca ggctgggtga aagagtgaca
721 ctccatctca aaaaaaagtg tttgataaat ttatttattt aataccgatt tattgattga
781 ttgatactct tcggagcttt ccttaatgag ccagtttctt ccataatgca atgaatgaaa
841 ggacctctca gattcaggca tctgagtgcc cgactggagt ctgggacccc ccagacctct
901 ctgcctggga gcttccatga agctcagcct gagctggaag gagaccgtag ggagatgtcc
961 tagtgagagt gaatccctag tcactgtttc ctcttcttac ccactcgatg atcccaaagc
1021 acacccatct ccaaaagaac aggtcacatg tggcagagga gaaacaaaat aattagcatc
1081 cgcttcctta acaaggagct atggggacga agtctcctag agtccccaga ctataaatac
1141 cctgatgttc ccctggctcc cccactcacc tgcagagtct gggggcgggg gggtacacat
1201 atgaatcctt cccatagaca tcaggctaac cccagcgatc acaggtgagt agcccccatg
1261 cccttcctag gggtttagtg ctgacctcgg gatcctctag cagttggtgg agaaggaggc
1321 ttcccgggac cctccccttg gtgatgccat cctggtgagc aggtgaggga tggggaaagg
1381 attgagctga gggtgggggt ggagggttgt gggagaggaa tctgtctcct tcctagaggc
1441 cgagagagag agaagagact gagaccaagt ctagactctc tgagtggaca aagcacgcag
1501 acggcagacc ctctgtggca ggatatgtgg aggaaaagca ctgagggtct gcagctgcag
1561 gacacctgca cagataggag aggtcttgag aaacaagttt ctccaatgca gcagctcccc
1621 aagtgtggtc cctgggctag aagcaccagc atcctctgga aactatctag aagtgctggt
1681 tcttacacca gacatttcag ggaaagggct tagctttttg tgtatgatga gtttgggagc
1741 gggggggagg gggcgggggg attgttttgt ttgtttgttt gttctttctg tttttgagac
1801 agggtctcgc tctgttgccc agtctggagt gcagtggcat tatcatagct catcgcagcc
1861 tcgacctccc aagctcaagt gatcctccct cctcagcctc ccgggtacct gggataacag
1921 acatatacca ccatgcctgg ctaattttat tttcgtagag gcagggatct tcctatgttg
1981 cccaggatgg tctcacactc ccgggctcaa gagatcctcc taccttggcc ccccaagcag
2041 ctgggactaa caggtgtgtg ccaccatgtc cagctaattt tttattttgt agagatgggg
2101 gtctcactgt gttgcccagg ttggtctcta actcctggac tcaagggatt ctcccacctc
2161 agccttctga gtagctggga ctatggaagc atgccaccat gcccagcaga ttgttttatt
2221 ttttgtagat ccagggcggg attgggggtg gggtggcctc actttgtctg ccaggctggt
2281 cttgaactcc tggcctcaag caattctccc acttcagcct cccaaagtgc tgggattaca
2341 ggtgtgagcc accatgcctg gcccgctttt tgtgttttaa caagccttcc aggtgaccct
2401 gaagcgtgct caaggttgag aaccgctgct ctatgcaacc caagtcaaca actgaggact
2461 gttacaaata tgttgccagg aagggaatcg cagtattcta tccagcctag agaaaaccaa
2521 gcattgcatt gcaaaaacct caagtccaac actaaaaagc ctcaaaaagt taacataggg

2581 ctttgctgaa ggcctcttgg ggttaccaag gagattatgc tgctaacctt cctaaaaacc
2641 cctttcatcg gctgggcgct gaaatgatga gattactggg ctcacgcctg taatctcagt
2701 actttgggag gccaaggtgg gcggatcacc gggtcaggag ttcaagacca gcctggccaa
2761 caggcttagt agaggcgaaa ccccgtctct actaaaaata caaaaattaa tcgggcgtgg
2821 tgatgcacac ctgtaatccc agctattcgg gtggctgagg cacaagaatc acttgaaccc
2881 aggagacgga tgttgcagtg agccgagatc gtgccactgt actctagcct aggtgacaga
2941 gtgagactct gtctcgaaaa gaaacacgtg taaatgcttg taattgaata tttttccctt
3001 gcacgtgttg acagcacaga atttagcttc gaaatgcctc ataaagaggt gagagtggcc
3061 attcttaggg aggcagtgga gtatgagagt gaaaataagt ctaaattcaa aggcgagatc
3121 aggcacttgc aagctgagtg cccctggcag ctcgctcagc cccagtctgc tcactgtaaa
3181 ttgtagctaa cacaatctcc ctgtgggagt tattgtaaga cctatgcatt aagaaactgg
3241 ctcctggctg gctgcagtgg ctcacgcctg taatcccagc actttgagag atcaagccgg
3301 gcagatgatg aggtcaggag ttcaagacca gtctgacaaa catggtgaaa ccccatctct
3361 actaaaaata caaaaattag ccatgcgtgg tggtgctcgc ctgtcatccc agctactcag
3421 gaggctgagg caggagaatc ttttgaaccc aggaggcggg ggttgcagtg agccaagatc
3481 gcgccactgc actccagctt gggtgacaga gtaagactcc atctcaaaaa agaaaagaaa
3541 gaaagaaacc ggctcctcat caggataaat agctaatgca tgctgggctt aataccgagg
3601 tgatggcttg ataggtgcag caaaccacta tggcacacgt ttacctatgt aacaaacctg
3661 cacgtcctgc acgtgtatcc tggaacttaa aataaaatga taaaattaaa taaaataaaa
3721 atcatgctga gcagccaata gctgggggaa aaaaaaagaa agaaactggc tcctaagaag
3781 gtttaaccca ttacagatat cattattgct gagggcaaaa taaaaacacc tgtcccagct
3841 actaggaagg ctgaggtggg aggaacacct gagctcagga ggtacaggct gcagtgagct
3901 gtgattgtgc caccgcactg cagcctgggt gacagagtga gaccctgtct caaaataaat
3961 taaattaaat taaatagatc ttgtattaga taattttgcc taactgtagg ctaacgtaag
4021 tgttctgagc acattgaagg tgggctaggc taagctgtga tgtttggtag gttaggtatg
4081 ttcaatgcat ttttgactta tatatatata tatatatata tatatatata tatatatata
4141 tatatttttt tttttgagac agagtttcgc tcttgttgcc aaggctggag tacagtggct
4201 caatctcagc ccactgcaac ttctgcctcc caggttcaag cgattctcct gcctcagcct
4261 cccaagtagc tgggattaca ggcatgcacc acaacgccta gctaattttg tattttcagt
4321 agagacgggg cttcaccttg ttggccaggc tgacctctca aactcctgac ctcaggtgat
4381 ccccctgcct cggcctccca aagtgctggg attataagaa tgagccacca cgccaggtct
4441 gacttatgac attttcaatg tatgacccca ttatcagcct cagaacatct gtacctatca
4501 tttcttggtg gtgagaacat aaaattgact ctgagtttct ctggtataca atgcattgtt
4561 attaactata gtcactatgc tgtacaatag atctcttgaa ttatgcctcc catctaattg
4621 aaattttgta ttctttgatc aatatctccc taacacccac ctcccgcccc cagcccctgg
4681 taaccaccat tctactctgc ttctatgaga ttggccattt taaattccac atataagtga
4741 gaccatgtgg tatctgtctt tctgtacctg gctattttat gtaacagaat gtccttcagt
4801 ttcatctata tggttgcaaa tgatagaatt tcctcttttt tccatggctg aataaatagt
4861 attccattgt atattcctac cacattttct ttatccattc atcggttttt aacacaaaac
4921 tgatgttaaa gattacatat tgttggtgga aggatgaagg ggaaggaggg ttgagggggg
4981 aggaaagaaa agaggagatg gccaggcgcg gtgcctcatg cctgtaatcc cagcaccttt
5041 gggagtccaa ggcgggtaga tcacttgagg tcaggagttg agaccagcct ggccaacatg
5101 gtgaaagccc gtctatacta aaaatacaaa aattagccag tcatggtggt gcgtgcctgt
5161 aatccccgct acttgggagg ctgaggcacg agaatcactt gaacccagga ggcagaggtt
5221 gtagtgagct gagatcgtgc cactgaactc cagcctgggt gacagagcaa gactctgtct
5281 caaaaaaaga aaaaggaaaa agaaaacagg gagagaacac aggaaaggaa gagaaggaaa
5341 aacagaagat ggtcaggttc cctttactgt atagagtttt tgttgaggtt tgattctgct
5401 tgattttctt tttttatttt tgttttttgt tttttgtttg agacagaatc ttgctctata
5461 gcccaggcta gagagccgtg gcacaatctc tgctcgctgc aaactttgcc tcccgggttc
5521 aagctattca cctgcctcag cctctagagt agctgggatt gcaggcgccc accaccacgc
5581 ctgattaatt tttttgtatt tttagtagag acggggattc accatgttgg ccaggctggt
5641 ctcgaactcc tgacctcaag caatctgccc gcctcagcct tccaaagtgc tgggattaca
5701 ggcgcgggcc acttggtttt gttttctatg aagctttgct ccactgaagg aagcaaaagt
5761 gtttatagag aacatccaag ttgactatga gcgactccgt tagatccttg agaccctgac
5821 tgtctcaggc atgaatgact tggcttgagg agcaacagaa gcccactgaa gatggcttga
5881 ggttaactga acacaaagga atttgatgga tttgatggaa ttttcacaga acagtatagg
5941 tttccgtggg aagctgggac cagaatggca tctctgacac ctggctctgt gtggtttttc
6001 tccctttttg ttcccactag tcacagacca tacatggaac cagaaaagca aaccgaaatc
6061 tcagaattct tcctccaggg actctcagaa aagccagagc atcagaccct cctcttcaca
6121 atgttcctct ccacatacct ggtcaccatc attggaaatg ccctcattat cctggccatc
6181 atcacggact ctcacctcca cacacccatg tacttctttc tcttcaacct ctcactcgtt
6241 gacaccctat tatcctccac caccgtcccc aagatgctag cgaacatcca ggctcagagc
6301 agagccatcc cctttgtggg ctgcctcacc cagatgtatg ccttccacct gttcgggacc

6361 atggacagct ttctcctggc agtaatggcc atcgaccgct tcgtggccat tgtccaccca
6421 cagcgttact tggttctcat gtgctcccct gtctgtgggc tgctgctggg agcatcatgg
6481 atgatcacca acctccagtc tctcatacac acctgcctca tggctcaact gaccttctgc
6541 gccggctctg aaatctccca cttcttctgt gacctcatgc ccctgctgaa gctctccggc
6601 tcagacacgc acaccaacga gctggtgatc tttgcttttg gcattgtcgt gggcaccagc
6661 ccattctcct gcatccttct ctcgtacatc cgcattttct ggacagtctt taagatccct
6721 tctactcggg gcaagtggaa agccttctcc acctgtggct tacacctcac tgtggtgtca
6781 ctgtcctatg ggaccatctt tgctgtgtac ttacagccca catcccccag ctcctcccag
6841 aaggacaagg cagccgccct aatgtgtggg gtgttcatcc ccatgctcaa cccctttatc
6901 tacagcatac ggaacaagga tatgaaggca gccctgggga agctcatcgg caaagtggcc
6961 gtcccctgtc ctaggccaga acagttattg gatgtttatc atgttccagg atcactgttg
7021 gctgctaggg acacagagat gcatcccatc ccctaccctg gaggagttca gagtctagct
7081 gggaacagag acatggaata aatccttcca gtacaacgtg ataaatgtgt cataaagaga
7141 tgaacaaagt gtatgagcac ccaaaggaaa ggtgtgcatt ttccccagga tttatcttcc
7201 ccaaaaagat cagaatagca aggatcaaac tggcctgaaa ttagccctcc tggaggatca
7261 gccttttagg attgctatgt gaataagagt agttgaaaac aagagacgta gctacactca
7321 ttttagtatt gacaaaggcc aggtgtggaa gctcaagcct gtgatcccag cattttggga
7381 ggccaaggtg gaaggatcac ttgaggccag gagtttgaga ccagcctggg caacatacag
7441 agaccctatc tccacacaca cacacaaaat cactttagca agacatggtg gtatacgcct
7501 gtagtctcag ctaatcagga ggctgaggca ggcagatcat ttgagcccag gagttcaagg
7561 ttgcagtgag ctatgactgc accactgcac ttcagcagtt tgggaaactg agcaagatct
7621 gtcactcata ggtagataga tagatagata gatagataga tagatagata gatagataga
7681 cagacagata gatagagagg ataagataag ataggtagat agggtggata gataagatat
7741 gtagatagat agatagatag atagatagat agatagatag atagatagat atacagatag
7801 atacatagat agataggata gataggtagg taggtaggta ggtcaaccga tcgatagatc
7861 tatctattga cagacagata gatgattgat tgatagatag atcatataag tagatagatg
7921 atagatagat agatagatag atagatagat gagataggta ggtaggtcag tcgatcgatt
7981 gatcaatcga tcaacagata gatgatagat agatggatgg atagatagag atagaggagg
8041 taggtagata gatagataga tagataggta gatacagaca gcccatgtat tagccagtgt
8101 ttcctaagtg ttgtggaaga actttgaaaa tatttgcaag tgagattagc tcacgtgctg
8161 ttcgcttagt cagccagcaa gcctcagcct cctggggggt cttgagacct ccacttgtaa
8221 ctatgactgt ggttggggct tactttctca attcccaaca ggcttcctgt ctccccacaa
8281 cccccagtca aggggactct gatgcagacg gtccatcaat cgtagtttgg gaacacagac
8341 cctagcctag ctccaaaatc tatgtgtttc ctgtgacatt catcgcctga ctctgaaccc
8401 agtatgattc tacacgcagg gtcagccaca gcagatcagg tggcccgggg cacttaaaaa
8461 gttagtgtta tgggttgaac tgtggctccc aaaaaaagat acgtgggagg ccaggcgcag
8521 tggctcacac ctgtaaactc tggcactttg ggaggttgag tagggagaat catttgaagc
8581 taggagttca ggaccagcct gaacaacata gtgagatctc atccctacaa aaaataaaaa
8641 tgaaaaaaga aaaaattacc tgggtgtggt ggtgtgcacc tgtcatctta gttactcggg
8701 aggctgagac aggaggacct cttgagccaa ggaggtcgag gctgcatgag ctatgttcct
8761 gccactgcac tccagcctgg gaaacagagc aaggccctgt ctgcaaaagt aataataata
8821 aaataaaaaa gatatgttga agtcctaacc ccaaggacct cagaatgtga ccatatttga
8881 agatagggtg tttacagagg tcattagatt acagtaaggt cattaggttg gtccctaatc
8941 tgatacgact ggcgtcctta tgaaaagggg agatttggac acaggcaccc acacacatgg
9001 agaccgccac atgaagatgg aggcagagat tggggtgatg ctcctacaag ccaaggaatg
9061 ccagggactg cctgcagacc accagaagcc aggagaggcg tggaacaggc tatccctctc
9121 agccctcaga aggaaccaag gaatcaaagc tgctgacaac tgatcttttt tttttttttt
9181 tttttttgag acggagtctt gctctgtcat cccggctgga tgcagtggca tgattttggc
9241 tcactgcaac ctccacctcc caggttcaag caattctcct gcctcagcct cccaagtagc
9301 tgggattaca ggcacccacc accacgccca gctaattttg tgtttttagt agagatggga
9361 tttcactatg ttggccagac tggtctcaaa ctcctggcct ccagtgatct gcccacctca
9421 gcctcccaaa gtgctgggat tacaagcgtg ggccactgca cctggcccaa caccttgatc
9481 tcggactctt aacctccaga actgtaagac aacaaatttc tattgtttaa gtcacctagt
9541 tcatggtaat ttttaaattt tttttctttt tagagacaga caggatctac tgtgtcaccc
9601 aggctggagt acagtggtgc aatcatagct aaagtgcaac ctcacctcaa actcctgggc
9661 tcaagcaatc ctcctgcctc agcctcctga gtagttggga ctacaggcct atgccactgc
9721 aatcagctat atatatttgt agagacaggg tctcactatg ttgcccaggc tggtctcaaa
9781 ctcccggcct caagagatct gccctcctcg gcttcacaaa gtgctgggat tgcaggtgta
9841 aaccaccaca ctcggcctga tttgtggtac tttgttgcaa cattgctaga aaacaaatgc
9901 agttagttta agaaatgtca gcaacctaca aaacctacaa agtgtcccag tacatcctct
9961 caagtttaca attgaccatg tgttctggcc aagggaccct gggctagaac tggagaaaag
10021 gatgaaggat ttagattgca tggattcact tactgggtat cagcatgatg acaataatga
10081 tgatgacaac cgagatttat tatctgaaca atgctaactc ctttatatgc acaatctcaa

10141 taagtgttaa gcaacacccc tacaaggtag gtgcagaagt aattgtggtt ttgccactga
10201 aagtaacagc aggctgcgca tggtggctca cacctgtatt cccagcactt tgggaggccg
10261 aggcgggcag atcacaaggt cagtagatcg agaccatcct gaccaacatg gtgaaacccc
10321 ctctctacta aaaatacgaa aattaggccg ggcacagtgg ctcatgcctg taatcccaga
10381 actttgggag gctgaggtgg gcggatcatg aggtcaggag atcaagacca tcctggctaa
10441 catggtgaaa ccccatctct actaaaaata caaaaaatta gccggacatg ttggcgggca
10501 cctgtagtcc cagctactcg ggaggctgag gcaggagaat ggcctgaacc cgggaggcgg
10561 agcttgcagt gagccaaggc cacgccactg caccccagcc tgggtgacag agcgagactc
10621 ctcctcaatt taaaaaaaaa tacaaaactt agccaggcgt ggtggcccac acctgtagtc
10681 ccagctactt gggaggctga ggcaggagaa tggcgtcaac ccgggaggcg gagtttgcgt
10741 gagccgagat cgccccactg cactccagcc tgggtgacag agcgagactc tgtctcaaaa
10801 aaaaaaaaaa aaaaaaaaaa aagtttgtaa tttaaaagtt tgagcatcac tgggctagat
10861 gggggtcctg tagataaccg ggacccaaat aggctttagg tacagagata caggagaacc
10921 agatttgggg tgccgctcta gccactgtgt ccaggaggga accttcccaa ccgccctaaa
10981 agctctgaaa actacagtta gagaagtggc cactaggtgg caataatgtc atttcacaaa
11041 ttggatttaa aacgcttttt tttttttttt tttttttggg tttttggttg tttttttttt
11101 ttccccggaa acttgtattc tagctgtgaa ctggcccagg tccatggccc ttaggtgtaa
11161 tctccccctg gaagggcaca acaggtgcaa agtttccatg atgagaagga gcctgtgttg
11221 tttaaagaaa tcacggagac tggtgtggcc tgagcccaga gtgacaggga agctgggttt
11281 gcagggggcc cgtggagggc actgggagta acacggtgtg tggaccacgg ggagtgggac
11341 catgacaagg tttaaggagg atagggggtg gggctgtgct tcagaacgaa cctgccagtt
11401 atccatgcca tagatctgga gtcagcaaac tttttctgtc aaggtccagg tcgtcaatat
11461 tgtcagcttt agggacctta ccatctccgt tgcaatgact gaactctgct gctgtctggc
11521 gaaagcagcc atagatgaga catcaacgaa tgggcttggc tgtgttccac taaaactttg
11581 tttataaaca cagaagcttc catttcatat catagtacat gtcatacctt gtagcctttg
11641 ggtttgtttt caaccacttt taaaatgtta aaactactct aggccaggtg cattcactca
11701 tgcctgtaat cccagcactt tgagaggcca agggaggagg attgcttgaa gccaggagtt
11761 cgagactgga gtgggcaaca tagtgagacc tcccccatct ctacaaaaat tttttaatta
11821 gctgggcatg gtggtgcatg cctgtagtct cagctactca ggaggctgag gaaggaggat
11881 cacttaagcc caggaggtca aggctgcagt gagctatgat tgcaccaccg cactccagcc
11941 tgggggatag agtaagacca ctgtctctaa aaaacttaaa aaataaataa ataaataaaa
12001 ataaatgcaa aactattttt atttcaaggg ctggatttag cctatggccc ataggttgcc
12061 aatcctcgta tagatggatg ggagtgacaa gagttaaggg gaccaagaaa gaccaaaaag
12121 tgagaggtga agtgggctga aaaccatcag aagataagga gctggtgcca caggaccttt
12181 gcaaatgctg ttccctcttc ctgacccctc ctgagtaaat tcctgcttgg cattgagatt
12241 tcagcacaaa ctccacttcc tcaaggaagc ctcccctaac ctccatccct tgcccagcta
12301 catcagttgc cctaatgtta cactcgctat cccctctctc atcatcactc atcattagtg
12361 atctcttcat cactgcagtg gttttgtggg gtcttttgtt ttgttttgag acagagtctt
12421 gctctgtctc ccagtctgga gtgcagtggg gcgatcccgg ctcactgtaa cccccacctc
12481 ctgggttcaa atggttctcc tgcctcagcc tcccgagtag ttgggattac aggcacgcat
12541 caccatgcct ggctaatgtt ttgtattttt agtagaaacg gggttcaccg tgtttgccag
12601 gctggtctcg aactcctggc ctcaagtgat ccacctgcct cagcccccca aagttctggg
12661 attacaggtg ttagctgctg cacccggcct cctcactgca gtggttttat attagtttgt
12721 gtaacccttt gattaatgtc tgtttcaccc atttgactga gctgggagct gggacctgaa
12781 atagtgttat cttgtttatc tttgcacctc tgtgccccta tgattagtac ggtagccact
12841 caatgtatgt tttctggcca cgcaccgtgc ctcacacctg taatcccagc actttgggag
12901 gtgaaggcgg gcaatcacct gaggtcagga gttcaagacc agcctggcca accacggcca
12961 acgtgatgaa gccccatccc tactaaaaat acaaaaatta gccaggcgtg gttgcacgtg
13021 cctgtaatcc cagctactca ggaggctgag gcaggagaat cgcttgaacc cgggaggcaa
13081 aggttgcagt gacccaaaat catgacactg cactccagcc tgggcgacag agcaagactc
13141 ttgtctcaga agaaaaaaaa aaacaaaaaa cacttttttt ctttatttta atttaatctt
13201 attttatttt atttatttta ttttttgaga tggactctca ctttgtcgcc caggctaaag
13261 tgtagtggtg tgatcttggc tcactgcaac ctccgcttcc cgtgttcaag ttattctcct
13321 gcctcagtct cccaagtagc tgggactaca ggtgcccgcc accatgccca gctaattttt
13381 ctatttttag tagaggcggg gtttgaccat gttggccagg ctggtcccga actcctgacc
13441 tcaaataatc cacccacctc agcctcccaa agtgctggga ttataggcat gagccaccgc
13501 acccagccac aaaatatatt ttctgaatga atgaatgaat gaggcagtgt gatggaaagg
13561 ttttaggaat accggatggg gcagccaaag taggctgctg agttggaaca aagatacctg
13621 cagtcaaggt taagtgagag atgcactcct cctttgatat ccccaacctt cagttatgcc
13681 tgtcccacac tcaggtgcct tgtgggactg ggactctgct gaaattaaga agccaggggc
13741 ctcatcccac agagcaaaac cagcaagatg gcgggactcc ttaattcagc aacaacaaaa
13801 gacagaaaat aatctggaaa ttctctaaaa tcttagcaag catgctggaa gaggtttgag
13861 ccaatcttat ccagtaaatt tttttttttt ttttttttga gacagggtct cgctctgtcg

13921 cccaggctgg agtgcagtgg tgtgatcatg gctgaatgca gcctcgacct cctgggctca
13981 agcaatcctc ccacctcagc ctcctgagta gctagggcta taggcacgca ccaccacaca
14041 tggctaattt tttttacttt tttatagaga cgaggactca ctatgtggcc tattccggtc
14101 ttgaactcct ggcctcaagc catcctccca ccttggctcc caaaccgctg ggattacagg
14161 tgtaagccac tgcacacggc ccaagcctat attaagagta caagaacaga ccgggcacag
14221 tggctcacgc ctgtaatccc agcactttgg gaggccaaga tgggtatatt acctgaggtc
14281 aggagttcga gaccagcctg gccaacatgg tgaagccccc atctctacta aaaatacaaa
14341 aactagccag gcgtggtggc atgcacctgt agctccagct actcaaggct aaggcaggag
14401 aatcgcttga acctgggagg cggaggttgc agtgagccaa gattgtgcca ctgcactcca
14461 gcctgggtga cacagcaaga ttctgtctca aaaaaaaaaa aaaaaaaaag tacaagaaca
14521 cttccttctc tccatgtgaa attttattgg gtgattcaaa gcaaatctgg agattcggga
14581 actgaggctc agagacgaaa agtgaccacc ccaaggccac atgccagatc tggggcagag
14641 tgggggaatc cagcccaggc tggtgtgacc tcaaaaccca gcctctccct ccctcatgat
14701 gctggtagcc tcccaggtgg gtgacttgcc caaggtcaca ctgcagcttg gagcaactca
14761 aggcctgtcc cgggaggagg gagcagcccc tgtgtgggac tctggggagt gaaaagcgga
14821 caagccagca ggagtggcca ctttctggaa agtgtaagaa agattcacta agatttgggc
14881 tgaagttcgc agagatggat tgggacaatc ctcccaactg tccatttgcc aggaacttgg
14941 caggaaatgg aggtggtttt gtttgtgtaa acctttgtgg ctggggatcc caaaggcatc
15001 cctcaccttt aatgatttgc tagaaagact cacagaactc agtaatgtta ttatactcat
15061 ggtttattgc agtaaacaga tacagattaa aatctgcaaa gggccagcca cagtggctta
15121 tgcctataat cccaacactt taagtgagag atgcactcct cctttgatac ccccaacctt
15181 caattatgcc tgtcccacac tcaggtgcct tgtgggactg ggactctgct gaggcaggag
15241 gatcacttga agccagaagt tcgagatcag cctgggcaac atggccagac cctgtctcta
15301 caaaaaattg taaaaattag ccaggcgtgg tgatgcacac ctgtagtccc agctacttgg
15361 gaagctgaag tcggaggatc actcagatct aggagttcaa ggttgcagtg agctagaatt
15421 gcacaactgc actccagatg aggcaacaga gtaacccccc cccctttttt tttttttgga
15481 gacagcatct agctctgtca cccaggctgg gatgcagtgg tgtgatcaca gctcactgca
15541 atttctgcct cctgggctca agtgatcctc cctccttggc ctcccaaagc aacgggatta
15601 caggtgtaag ccaccacacc tggcccccat taaaaaaaaa aaatctgcaa aggtaaaagg
15661 cagatagagc agcatctagg agacaccagg catgagcttc cagctgttcc ctcccagtgg
15721 agttgtacag acatagacaa tgctgaattc tcacagccac aatgtgtaac aacaggtgca
15781 gagtgttgct aaccaaggag ctgacccaag ccttggtgtt ctgggttgtt tttgtttgtt
15841 tgttttttgt ttttgttttt gtttttttga aacggggtct ctctctgtca cacaggctgg
15901 agtgcagtgg tgcgatctcg gctcactgaa acctctgcct ccagggttca agcaattctc
15961 tgcctcagcc tcccgaggag ctgggattat aggcgcccac caccatgccc cgctaatttt
16021 ttgtattttt agtagagatg gggtttcacc atcttggcca ggctggtctt gaactcctga
16081 cctcgtgatc cacccgcctc ggcctcccaa agtgctggga ggtgttctgg gttcttattg
16141 gaggttggcc actgtattac aatagttccc gcttcttcat gaggtttcag ttacctgctg
16201 tcaacagcag tccaaaaata ctaaatgaaa aatttgagaa ataaacactt tgggttgggc
16261 atggtggttc acgcctgtaa taccagcact ttgggaggcc gaggcgggcg gatcacgagg
16321 tcaggagatt gagaccatcc tggctaacat ggtgaaaccc catctctaat aaaaatacaa
16381 aaaaaatagc tgggtgtggt ggcgggcgct gtagtcccag ctactcagga ggctgagaca
16441 ggagaatggc gtgaacctgg gaggcagagc ttgcagtgag ccaaaatcac accactgcac
16501 tccagcctgg gcaacagagt gagactccat ctcaaaaaaa aaaagaaaga aaaagagaga
16561 gaaaaaaaaa taaacaatta gtaagttttt ttgtgggtac atagtaggtg tatatattta
16621 tgaattacat gtcatatttt gatacaggga tgtacacatc agggtaaaat gggtatccat
16681 cccctcaagc atttatcttt tgttttacaa acaatctgat tatgctctgt aagttatttt
16741 caaatgtaca attaaattat ttttgactac agtcaccctg ttgtgctagc aattcatacg
16801 tttttggttt tgggttttga gacagtctca ctccgtcacc caggctggag tgcagtggca
16861 caatcttggc tcactgcaac ctccacctcc tgcattcaag caattgtcct gcctcagcct
16921 cccgagcagc tgggactaca ggtgcccgcc accacaccca gctaattttt ttgcattttt
16981 agtagagatg gggtttcacc atgttggtca ggctggtctc aaactcctga cctcaggtga
17041 tttgcccacc tcagcctctc aaagtgctgg gattacaggc atgagccact gcgcctggcc
17101 acaactcata cgttttaaag tgcgtgccat tctgagtagt gtgatgaaat cttgcaccat
17161 tccactccat cccagctggg atgtcaatcc tccctttgtt tggcacatct gcactgtaaa
17221 caccccaccc atcagtccct tactagccat ggcagttatg agatcgacaa aacacagtat
17281 agctagggtt ctgtactacc cacggtttca ggaatctacc gggcatcttg gaacatgttc
17341 cctgcagata atggaggatc actagtgtcc tgggactacc ataacaaaat accacagcct
17401 gggaggctta aacaacagaa atttattttc tcatagttct ggaggctgga agttcaaggt
17461 caagttacgt tggtagggtt gttctgtggc ttctctccag gccttgtaag tggctggctg
17521 ttccctgaga cttcacattg tcttccttct atgtgtgcac acatctgtat ccaaatttcc
17581 tcttctcgta aggcactagt cctactggat taggactcac cctaaagacg tcattttaac
17641 gtaattacct ctgtctctag ctccggggta tttctccgcc aactcaccat gactgccaca

17701 gaagtgctga gctctgccca aacctgaccg gtccctccac ctgcattttg tctttcaacg
17761 aatgggtctg tatgtgcctg cgggtggtta acccaccagg taccaaaagc acagcgtatc
17821 aggcctgcag ccaacgagaa agaaaattgg aggaagaaaa atacatacat gtggggctgg
17881 gtgtggcggc tcatgcctgt aatcccagca ctttgggagg ccaaggtcgg gggatcactt
17941 gaggtcagga gttcaagacc agcctggcca atatagtgaa accctgtctc tactacaaat
18001 acaaaaatta gctgggcgtg gtggcacagg cctgtaagat cgcaccactg cactccagac
18061 tgggcgacag agagagaccc tgaaaaaaaa aatagaaaaa gaaaagaaag ggaagggaaa
18121 gggaagggaa gggaagggaa gggaagggaa agggaaaggg aaagggaaag ggaaggggga
18181 aggggaaagg gaaagggaag gaaaggaagg gaagaaagaa agaaggaaag aaagaagaaa
18241 gaaagaagga aagaaagaaa gagaaagaaa gaaaggggaa ggaaggaagt agagagagag
18301 aaagaaagaa ggaaggaagg agagaaagaa agaaagaaaa ggaaggaagg aaggagagag
18361 aaagaaagaa gaaaggaagg agagggagaa agaaagaaag aaagaaggaa ggaaggagaa
18421 agaaagaaag gaaggaagga aggaagaaag aaaatacata catggccagg agcagtggct
18481 cacacctgta atcccagcac tttgggagac caaagaggaa gagaagactc tatctccaaa
18541 tacagtcaca ttctaaggga ccgggagtta agacttcaac ataggaattt ggcaagggac
18601 tgaaggggac ataatttggc ccatgggtgt ccaatctttt catttccctg gaccacattg
18661 gaagaagaat tgtcttgggc cacacataaa atacactaac actaacagat agctgatgag
18721 ctaaaaatcg caaaaaaaaa atctcatagt gttttaagaa agtttacgaa tttgtgttgg
18781 gtcacattca aagccttcct gggccacatg cagcctgtgg gtggcgggtt aaacaagctt
18841 gatttagccc ataacagcca catagtatgg ctgaccattt gtgtgactga ccttagctac
18901 tcagcctcaa gcccctcgag aggtcaaact gacacagtga ggcccaaggc ctccaccata
18961 aaacacgttt ttggcataaa ctctctggtg tgacctccag gtatacaaag agactctcat
19021 cacacaggat actccaccag cttgaaggca actcttcagg agccagacaa gggctagact
19081 ttactttgga atgtgcagga gagcccagaa gcatgggagg aaggaggcag gagctaagcc
19141 tgatgtggga tctctgtcaa agccctgccc acccagccac ctgtcctcac ctgatcagcc
19201 acctctctgc aggctgctcc tgcttcctct ggtacaccca gcctgtagag tctagggggt
19261 ccacaccagc acaccaggtc tgggagctgg acatcagata agctttgttt aagagacagg
19321 gcctcagctg ggcacggtgg ctcacacctg taatcccagc actttgggag gccaaggagg
19381 gcagataact taaggtcagg agttctacta aaaatacaaa aatctagcca ggcatggtgg
19441 tgggcgcctg taatctcagc tactcaggag gctgaggcag gagaatcgct tgaatctggg
19501 aggcggaggt ttcagtgagc cgagattgcg ccactgcact tcagcctggg caacaagagt
19561 gaaaaccctg tctcaagaaa aaaaaaaaaa gagagagaga gacggggaga gacagggtct
19621 cgctctgttg cccaggctag aggcagtggt gcagtcatag ctcattgaag cctcaaactc
19681 ctgggctcaa gtgatcctcc tgcctcagtc tccagaatag ctgagactac aggtgtgaac
19741 caccatgtcc agcttggaga gctcttggca tggctgcagt ggccagttgc tgagtctatc
19801 cctgaggctg tgcaggggcc tagccagcag gaaagctcca cagccttgca gctgataaac
19861 agagaccact ctggaggtgg catggagcaa ctatggaccc ctcctgcacc tcagtttcag
19921 ctctatagct gtctggccag atttttccaa gggtcaatgg gaggctctgg tcaagcaatg
19981 ctgtaccact gactgtcacc caggtgagac tgtgccctgc ctcaggaagc tgctttatgc
20041 taacagaatt cataaccatt tcattatata caatcatccc ctctccctcc agctcctgaa
20101 tgctactgtg atggatatct ctgggcagcc acccctttgc tggttcctct gaggtctgac
20161 ttccagccag ggaggcctag gcaggctgag gtattcacct tcttctcctg gaagatgcca
20221 gatagacgca agcctcaggt gtggaaacaa atgggaatga ccacagcggg tggaggaagc
20281 ttgggtcctg ctccccaaaa ccttctctat ctcccatccc ccaggaactg agcaccccag
20341 ggagctggta gaatggagat gtagccccac agaaccacgc cccatgccca tagccttgaa
20401 aacagcctaa tttattattc cctttgcaca tgaaacagag gcctagacag aatgtctgtg
20461 tttggagtcc accagaagcc aaccttgaga caaggattca agtatttagg aaataaacag
20521 gaaatactgt aagtggggtg gatatgtggg agagggaagg gaaggaagtc agcaaagagt
20581 gtattgataa ccaggtttct acagtggaca gctggagctc aatcccgtgg gtgaacacta
20641 ggggtcagtg tagaacatga acctcaatca tccaacacaa gggtgaggga gctggggtat
20701 ttatccatca attccccttg actaccacag aggtgctgag ctctgcccaa aactgacagg
20761 aaatggtctt ttggtctttc aactaattgg tctatatgtg tctggggctg gttaatccac
20821 caggtaccaa aagcacagca catcagacct gcagccaatg agaacattta ggggaaagaa
20881 aacacacacg tggccaggtg cagtggctca cacctgtaat cccagcactt tgagaggcca
20941 aggaagaagg atcgcttgag gtcaggagtt tgagaccagc ctgggcaaca tagcaagatc
21001 ctgtctctac aaaaaaaatt taaaagctag ctgggtatgg cagtgcatgc ctataggcct
21061 agccactcag gaggctgagg caagaggatt gcttgaaccc agaagttcaa gtctgcagtg
21121 agctatgatc acaccaatgc acaccagctt gggtgacaga gggagattat gtctctaaaa
21181 ataataaggc caagcacggt ggctcacacc tgtaatccca gccacttggg gggccaaagt
21241 gggaagatca cttgagccca gaagtttgag aacagtctag gcaacatgat gagaccctat
21301 ctccacaaaa aaatagaaaa atcagcctgt ggcatggtgg cacatgcctg tagttccagt
21361 tgcttgggag gctgaggcag gaacatcact tgagcctagg aggttgaggg tgcagtgagt
21421 tgtggtagtg ccactgcact acagcctgag caacagagca agaaaaataa ataaaataat

21481 aataataaag taaataaaaa taacatgaaa ataattttta aaaaatttga gacagagtct
21541 tagtcaccca ggctggagtg cagtggcacg atcttggctc actccaacct ctaccctctg
21601 ggttcaagcg attcccctgc ctcagcctcc cgagtagctg ggattacagg tgcctgccac
21661 catgcctagc taatttttat atttttagta gaaacggggt ttcaccatct tggccaggct
21721 agtcttgaac tcctgacctc gtgatccacc cgcctcagcc tcccaaagtg ctgggattac
21781 aggcatgagc cacagtgccc ggccatgaaa ataaattttt ttaaaagaaa agaaaatggg
21841 ccaggcacgg tggctgaccc ctgtaatctc agctctttgg gaggccaagg tggatggatc
21901 acaaggtcag gaaatcgaga ccatcctggc caacatggtg aaaccccatc tctactaaaa
21961 atataaaaat tagctgcacg tggtggtgtg tgcctgtaat cccagctact caggaggctg
22021 aggcagcagc atcgcttaaa ccagggagtc acaggttgca gtaagccaag atcgtgccac
22081 tgcactccag cctgggcaac aaagcaagac tatgtctcta taaaagaaaa gaaaagaaaa
22141 tgtacgcata caggctctaa aatctgtaca gcaactagca aaatccacag gaataagcat
22201 acaaacatca ttatgaaact tagaattcat aaccatttca ttacatacaa tttgcagata
22261 atctgttctc acttatggaa atttctgatt gctaagaaag aatcagagcc aatggtgaat
22321 taaataggct acatgcaggc aaataaagac aacaggaaaa gtacaaaaac tcaaaaagat
22381 ttacacaaaa taataataat aaatatttat gtggtatgtg ggtgtcatat gggtggggct
22441 aagggttgct ctcactccca tgtcagaccc cagctcctct gagactctga aataccccat
22501 tgggacctta gtcctgcagc ctctaccctc aacttgtaaa caggtattgt cttgacccct
22561 gacttctttc cccattgtca atagctgcct caaagacatc tgcgcaggga tctcataggg
22621 aggagggtgc actccaccct tttacagact gatgcccccc aaacacacat atccaagaaa
22681 agtgaacagc acctaggaga actgtatatc aacagttgta taatgagtat accaatatat
22741 gcataatgag agtcccagaa ggagagaagt gaaaggagca gaaagaatat ttgatgaggc
22801 tgggtatggt ggttcatgcc tataatccca gcactttggg aggccaaggt gggcagatca
22861 cttaaggtca gaaatttgag accggcctgg ccaacatgac gaaaacccat ctctactaaa
22921 aatacaaaaa ttagctgggc atggtgacag gcacctgtaa ccccacctac ttgagaggtt
22981 gaggcaggag aatcacttga acccagaaag cagaggttgc agtgagccaa gaccatgcct
23041 ctgcactcca gcctgggcaa cagagtgaga ctccatctca aaaaataata ataataatat
23101 ttgatgaaat aatggccaaa aaacttccca gaagcccaga attgatgaaa gacatgaatc
23161 tacatatcca caaagctcaa caaactccaa gtacaataaa cacagagaac cacacaaaga
23221 cacattaaaa ccaaacacac acagcctcct cgtccaggcc tcttggacac cccctcctat
23281 acagacctcc atcacctgac acctgatctc ctacctgaat attcagcagt gaacaacata
23341 gacaagcatc acttgatcac ttggtggaga cccagtctgg acgaggaggc agataaacta
23401 gaacaaacca ctgggactgt taaattcaag ttgtggcaag tgttagaaga aaggaaactg
23461 agtaatgtga tactgactag acagaggtgg tggacagaaa gggtctccct agaaggtgcc
23521 attggaccta aaggatgaga gagaggtggc ctcagaagaa ccagggcttc ccaggcagaa
23581 ggaaccatgt gtgcaaagga cctgaggcag aaatagcttg atatgaagga gaaccagaaa
23641 ggaggccatg gtgagtgaat gggacagcca aagagatgag gctggaagtc ttgtgagacc
23701 atgctgggaa tctgaatttt attctaggtg aggtgagcag tcataggaac actattcagc
23761 aggatggtgc cacaatctga tttcagtttt aagtccactg tgggtaatat atggagaatg
23821 aaatgtaggg ctaagacagg ttatattttc ccagatggcc acaatatctc ccaccctaca
23881 tccacttgta caatgtcacc ttgacatgcc tcccatgtag agaggtataa tctgtgcccc
23941 ctttccttga atctggtggc agaagtgaca cagcatgaca ctcaaggctg ggtcagaaga
24001 gatgcaactt cttccttgtt cacttttaga gggctgggtg gctgtctaag aagtctgact
24061 accccaagac caccatgctg gaaggaaacc cgaaccacac ggagaggcct gtgtagctac
24121 cctggtccac agccccagca acagctggcg tcagttgcaa gacctctgag tgaagacacc
24181 tccagatgac tcaggaccct gccatttttt agtcaccctc tagcctaagt ctcccctgtc
24241 caaactgaac cacagcatct gtgagcatca tgtaaaccaa aaggtatctc agataagtct
24301 caataaattt agaaagctta ttttgccaag gttatggaca tgcctgtgac acagacttag
24361 aagctcctga caacataaac tagggtagtc agggccacta ccttgattca ggtctgtaat
24421 cctagctact tggtaggcca aggcgggcag atcacttgag accaggaggg tttttgttt
24481 tgttttgttt ttttgttttg tttttttgag acggagtttc actcttgttg cccaggctgg
24541 aatgcagtga cacaatattg gctccctgca acctccgcct cccgggttca agagattctc
24601 ctgcctcagc ctcccgagta gctgggatta taggcatgca ccaccacccc cagctaattt
24661 tgtgttttta gtagagaagg ggtttcccca tgttggtcag gctggtctcg aactcccaac
24721 ctcaggtgat ccgcccgcct tggcctccca cagtgctggg attagaggcg tgagccaccg
24781 cacccagccg agcccaggag tttgagacca gtctgggcaa catgatgaga ctctatctct
24841 acaaaaagta gggaaattag ccaggtatgg tgacacatgc ctatagtccc acttgctcgg
24901 gaggctgagg caggagcatc acttgagccc aagaggctga gactgcagtg agctgtggtg
24961 gtaccactgc actccacctt ggtctagttt tatatatttt agggagacat gagacagcaa
25021 tcaatatatg taagatgcac atgggtttgg tctgggaagg caggacaact caaaagtagg
25081 gagaaggctt ccaggttata ggtggataag agagaaacaa ttgcattctt tcgagtttct
25141 gactagcctt tctgaatatg caatttacag agatagtcac ttatgcctta gtctggctta
25201 gtgaaacaaa taggtaagga agcgatcaga tatgtgtttg tctcatgtga gcagagggac

25261 aattttgagt tctgcctgtc cttcatccat aaggaatttc cttgtaggca aattgtgggg
25321 gaggtatgta gctttttttt tcttttcttt tcttcttttt ttttagacgg catcttgctc
25381 tgatgaccag gctagagtgc agtggtgcaa ttttgattca ctgcaacctc cacctcccgt
25441 gttcaagcaa ttttcctgcc tcagcctccc aaatagctgg gattaccagt gcccacccac
25501 catgcctggc caattttttt tctttttcat atttttagta gagatggagt ttcaccatgt
25561 tggccagcct ggtttcgaac tcctgacctc aagtgatccg tcgcctcagc ctcccaaagt
25621 gctaggatta caggagtgag ccaccgcgcc cagctagctt ttttaacctt gtagctatct
25681 tacttaggaa tagaatggga ggcaggtttg tcctacacag ttaccagttt gacttcttcc
25741 ttttggctca gtgatttgga ggtctcaaga tttattttcc tttcacaatc acgacatggt
25801 tgttttacat cattacgttt ggggatggtt tgtcaaacag cagtagtaat tggaatttgg
25861 aggtgagaga attgagtggg gaaagagtgg catggggaga ccaaggaaga agcttggggt
25921 ggggtgggta gtggggacgg tcattgaaac gaatgacact acggcttgca ccaggttgaa
25981 gcagtacctg gcaaggagag agaggatacg tggaggctgc gatttggaga cagacccaag
26041 agaacttcct ggatgccatc acccagagtc ccatccaggg agggtgggtg aggtggaaga
26101 caagggatcg ggcctgcgcc acgatcgtca cgtcccatcg aagctggagc ggatccgggc
26161 acccggagcc cccattccaa cccctcccgg ccccaggcaa ctacatctcc cagcatcgcc
26221 cgctcgggcc gcgggaaagg ggaaaaaaaa aaaaagtttg ggccaagcat tcccggaatg
26281 tgcttcctgc tggcgcgggg cgggggcggg gcggggaggc ccgggcgggg cggggagggc
26341 gcggggcggc gcggccggag cccggggcgc gcactcggct cggcccggcc cggccgcag
26401 catggccgag ccgctactca ggaaaacctt ctcccgcctg cggggccggg agaaacttcc
26461 ccggaaaaag tcggacgcca aggagcgcgg taagcggaga tcggtgggga acagggggcg
26521 ccgagcccca cccggcctgg gagcggggtc ccagggcccc gaggacagac ggtggggggg
26581 atcaggggga gccccccgca ccccgctgca gactggggtg cctccagcca ggggagggta
26641 ggggtcctgg aaggaagggg ctgggaggga ggggggcgcg gaccgttatg tgctgctggc
26701 cggggatgcc agcgctgcgg gctccttggg aggcccggga gggggcctcg cccacgacag
26761 gaaatggggc cgcgactgag ggcctggagg gtggagggga ggaggggctg ggaggccggg
26821 acactggggc ccagtcgggc tgggcaaggg gcatcgggag ccctggaggc gcaggatggg
26881 agatgggagg cgtactcctg caaaggaaag agaggggcgg cccgtcaatc agagtagctg
26941 ccccctccgc ctcccaccta ctaacagtga cggcaactgc gcctgcctta gccccaggaa
27001 actgtccctt ttatacagat ggggaaactg aggcacggag caattgggtc atgtgcccag
27061 ggccatcgac ttaaacagct ctttggacta tgctacaaac agagacccta gagggtggac
27121 atcagaggtg gccagaggag gagttcagca gggtagcaag taaaaacctt agagaaacag
27181 ctgcgtggga tagggggacc caggaaggaa ggagttaaag gaggaaggcc agaccattcc
27241 atggacgtac cccttccccc caggccttcc caaagattcc caatgttccc agggaaggga
27301 gaagggacag agatgcccaa acacaaacac acacaggaaa cagcccagga catgagacag
27361 gagaggaggg agggaagggg aagtcccaaa gcattgagcg tggggggtct ggcctgggct
27421 gagggacaag ggcccatccc aggatggcga cagagtttgg aagacaggag ggagggtaaa
27481 aggggcaggg gactgggagt gactgtgctg cagggaatag aaaacccagc tcctcactcc
27541 tgagcacgta gtgcctgctg tgggctgtat acttttccaa aaggctcctg gaagcctccc
27601 tggacatgga aatcacatcc ccccatttca caggaggata ctgagcctca gagagggaag
27661 cgacttgccc aaggccacgc aggtgcaaag ctccatgcca cggttattga gagggtagag
27721 gaagtggaga ctggaacgca gtacctacgg gccccaggaa gcctgactta gaaccctgag
27781 gctgcaggga tggagtcccc ttttgggatg ccagggactg agtagggggt gctctaagct
27841 gataactgag atcgctagcc tgtggctgcc tattccaaac aaaacagcag gatccaagga
27901 accatgactt atcaggggcc ggccagggcc gtacacagca tcccacccac tgatcaattg
27961 cacagggctg ctctgcaggc agtgaggggc tgggtgggtc tcactcccct tgctctctgc
28021 caggccaccc agcccagcgc ccagagccca gccctccaga gccagagccc caggctcccg
28081 aagggtccca ggccggagca gaggggccct ccagccccga ggcatcaagg agccctgcac
28141 ggggagccta cctgcaaagc ctggagccca gtagccgccg atgggtgctg ggtggggcca
28201 agccagctga ggacacctct ttagggcctg gggtacctgg cactggggag cccgccggcg
28261 agatctggta caaccccatc cctgaggaag accccagacc tccagcacct gagcccccgg
28321 ggccacagcc tggctcagct gagtcagagg gcctggcccc ccaaggtaag aacaagcttc
28381 ccatcccctt ccccaaggtg agcaccagct ccacatccct tcccttcagg gtagcaccag
28441 cctcacactc cctcctccca gaggagcacc aacctcacac tccttccctt caggggagca
28501 ccagcctcac atccccaccc cgtcagatgc tcccagagga gcatcagcct cacaccctcc
28561 tccccgccag gggaacacca gcctcacact ccttcccttc aggggagcac cagcctcacc
28621 cccctcccct aagcccaggt tcctggaccc tgaagtctgc tctccacaca ggtgcagccc
28681 ccgccagccc cccaaccaaa gcctcccgca ccaagtcccc gggccccgcc aggcgcctct
28741 ccataaagat gaagaagctg ccggaactgc ggcgccgcct gagcctgcga ggcccccggg
28801 ctggcaggga gcgcgagagg gctgcccctg cgggctccgt catcagccgc taccacctgg
28861 acagcagcgt ggggggcccc gggccggcag cagggcctgg gggcacccgg agcccgaggg
28921 ccggttacct cagcgacggg gactcaccgg agcgcccagc tgggccccca tcacccacct
28981 ccttccggcc ctacgaggtg ggtcccgcag cccgggcacc cccggccgca ctctggggcc

29041 gcctcagcct gcacctgtac ggtctcgggg ggctgcggcc agcgccgggg gccaccccca
29101 gggacctctg ctgcctactg caagtggatg ggaggccagg ggcccgaaca gggccactgc
29161 gagggggccc ggacttcctg cggctggacc acaccttcca cctggagctg gaggccgcca
29221 ggctcctgcg cgccctggtg cttgcgtggg accctggcgt gagaaggcac cggccctgtg
29281 cccagggcac cgtgctgctg cccacggtct tccgaggtag gacatgcggc tgcaggggag
29341 gaggggcagg gacccccaaa ccccaccgcc accccccgca gagtgcctag ggggctgggc
29401 tccgggcgga aggtgtggcc tggagcagcg aggccagggt acatgaagct tcagagcaca
29461 cccggctcag gcccccttgt gtcctcaggg tgccaggccc aacagctggc cgtgcgcctg
29521 gagcctcagg ggctgctgta tgccaagctg accctgtcgg agcagcagga agcccctgcc
29581 acagctgagc cccgcgtctt tgggctgccc ctgccactgc tggtggagcg ggagcggccc
29641 cccggccagg tgcccctcat catccagaag tgcgttgggc agatcgagcg ccgagggctg
29701 cgggtgagca cccaccccac cccaaccctc tggcccccag acctcagacc actcttcagg
29761 acaccctatg tacagatgcc agacccctac ccccaggcct gagccactcc tagctccaat
29821 cccaacctag acaagggcag aaggcgcccc cttgactgta gcaccctggg cagaacccat
29881 gggtcatcca ttgatttaaa aacaggatcc aagccctcct ggggctcaga gtctgatggg
29941 ggaaaagata cgactctagt cattcacaaa tgtaaaacta taactgggac tggagttggg
30001 gtggggtgta cctagggtct gggagagctt aaaacaaaac ctggggtagt catggaaagc
30061 ctcctcaaag aagtgtcagt ttcctaagtc aaaggagatg gcagccaaga caagtagtcc
30121 aagcagaggg tttacaaggc caggttgtca aggtagttcc aacctcccag cccacaatgc
30181 ccattgctgc ctggcccaga attccagtgc tcaccccact gggccctgat tagtctgtgg
30241 ccccggcaag aagacattca ctctctcttc ccacccccag gtagtgggac tgtaccgtct
30301 ttgtggctca gcggcagtga agaaagagct tcgggatgcc tttgagcggg acagtgcagc
30361 ggtctgccta tctgaggacc tgtaccccga tatcaatgtc atcactggtc agcatggccc
30421 cctctcccct gcctgctcac ccccattcaa tgcctcactt caaggccttg gggctcctct
30481 gggacctcag tcctcctatc tgaccttgct ttcacccacc tcctcttccc ccttagctgt
30541 gccctcctta gccttagaag ccagtggtgc cctgaccaga ggggaccctg tgttcacagg
30601 catcctcaag gattatcttc gagagttgcc caccccactc atcacccagc ccctgtataa
30661 ggtggtactg gaggccatgg cccgggaccc cccaaacaga gttcccccca ccactgaggg
30721 cacccgaggg ctcctcagct gcctgccaga tgtggaaagg gtgagttggg cctggtggga
30781 gtgatgggac ttgagagtgg gctgatacca atagaatgtt tcacccatgc ctgggcctga
30841 gatgggcatg agctggcagc atcatcatat ccccaagaaa taggtgctat tagcatccca
30901 cttcatagat ttggaaactg aggcccaaag caggtgacag ttgcccaagg tcacacagcc
30961 aggatgtgtc aaaaatgcag gctgaacgct gatctttcaa cctacaaggg aggttcctgg
31021 ctgagggaac ctagacaagt cccatccctc tctgtgcttc aatatcttca tctgtaaaat
31081 gagattcatg gcagctctgc ttttccaatg cttagttgcc tttggcttca gtcttccctc
31141 caacaaaata ggctcataat actaacatct aatgattaat agtcttaacc cgaccacatc
31201 ccagctatga gactgtgaac ctgcttccct actctagcca ccttagtctc ttgcaggtag
31261 gtgccacagg ggcctgactt tgtctgactt acatcctctc aaccctctcc caggccacgc
31321 tgacgcttct cctggaccac ctgcgcctcg tctcctcctt ccatgcctac aaccgcatga
31381 ccccacagaa cttggccgtg tgcttcgggc ctgtgctgct gccggcacgc caggcgccca
31441 caaggcctcg tgcccgcagc tccggcccag gccttgccag tgcagtggac ttcaagcacc
31501 acatcgaggt gctgcactac ctgctgcagt cttggccagg tgagttcatg cccagggcct
31561 gcaccaccaa tctgagccag gctgctacag tgatgccacc ttaagggacc aatcagtgtc
31621 ttaggcttga agctacgccc cctcatggag taaactggta cctggttgcc tcagcaacca
31681 gttcagatcc aaccaatctg tctgttttgt ctttttttat tttcattttt ttgtaaagac
31741 agggtttctc tatattgccc aagctggtct caaacgcatg atctcaagcc atccttccgc
31801 ctcagcctcc caaagtactg ggattataaa ccaaatcacc ttgccccaca gagctgattc
31861 atctttttat ttttattttt tatttttttt gagatggagt cttgctctct agcccaggct
31921 ggggtgtggt ggcatgatct cagctcactg caacctctgc ctcccaggtt caagggattc
31981 tcccacctca gcctcccgag tagctgggat tataggcacg caccaccagt cctggctaat
32041 ttttatattt taagtagagc agggtttcac tgtgttggcc aggctggtct tgaactcctg
32101 acctcaagtg atccacccat ttcaggcctc tcaaagtgct gggattacag gcatgcacta
32161 ccaatcctag ctaatttttg tatttttagt ggagatgggg cttcacggtg ttggccaggc
32221 tggtcttgaa ctcctgacct caagtgatcc gcctgcctca ggcctcccaa agtgctggga
32281 ttacaggcat gatccaggca tgatccacag acctgcccct acccatcttt gatgcagtca
32341 gaggccctgc ctcttgacat gccaatcaga ggcctagttg cgtaagcaac cagtcctagc
32401 tctgccagtg aaaacctgaa agggtcgaag gccgctttcc acaggccaat caggagccta
32461 gttccctaag cagccaatca gaggtcggct gtcgcctctt tctatgacct accctgtctc
32521 ccttcagatc cccgcctgcc ccgacaatct ccagatgtcg cgccttactt gcgacccaaa
32581 cgacagccac ctctgcacct gccgctggca gaccccgaag tggtgactcg gccccgcggt
32641 cgaggaggcc ccgaaagccc cccgagcaac cgctacgccg gcgactggag cgtttgcggg
32701 cgggacttcc tgccctgtgg gcgggatttc ctgtccgggc cagactacga ccacgtgacg
32761 ggcagtgaca gcgaggacga ggacgaggag gtcggcgagc cgagggtcac cggtgacttc

32821 gaagacgact tcgatgcgcc cttcaacccg cacctgaatc tcaaagactt cgacgccctc
32881 atcctggatc tggagagaga gctctccaag caaatcaacg tgtgcctctg agccagatga
32941 cggggtggga ccccggttag taaggaccgg gcgcccagtg gctaaggcgg tgccctggtg
33001 accaaggaga gccagacctg ttgctcaggc cgagctcctg gttgccagcg agttaccacg
33061 ggaccagtcg cgtgtatggc tgagactcat tcccagtttc cagggcccgg tatttggaca
33121 ctagttgcca agtctggggc ctggggattt tagggaccag cggttgtgac catctttcct
33181 gagcaccaag ggcttcccct tttgttgcca aaaaggtagt tctcgcgctt gctaggctgg
33241 cctctcttgc ctccccttgg ccggggcaac accagttact gtgagcatca ccctggtgtg
33301 gtgagtcacc tctagtcggc cctcttgctg ctgccaacca aatcagtatt agctttgagc
33361 actgcactgt ttctccctcc cttggacgac acaaagacta gcatgaggca ctgtttgtgg
33421 ggggcagccc ctatcctggg ttccagcatg gacacagggg tagcctgggg cttatagaga
33481 aacagctggt ttcccctacc cttttccggg gaagtcccca cgattggcct ctagtcagca
33541 aatggagata acagagtctg gcctttccaa tccccatctc cttgcccccc ccttgccccc
33601 ccccccgaaa aaaattgagc acttaaaccc ctcccttttg gaggggcccc cctgaagcgt
33661 caggctgggg gcagtctggt acggaacata tttattgcct ccatgcatgt gtgtgtgtgt
33721 ctgtgaggac tggtgtgcgt ggacacgtct gaagcaggcg tgtggggctc tttcagggac
33781 cacagaggag ggagcagttt gcagtgccca gccaccctga aatccccaat aatggtgcct
33841 cagtgggccc cagagttcca gtgggagagt acggttccct cctgtctccc tcttcttttc
33901 cgcacctcca tctttgtgga taataaataa atatgcacag gttctgagac tgagtacttg
33961 ggcctacatg ggccagaggt ggtttattca gtctccataa gagaggggac aaacggcaca
34021 gttgggtccg tgaagaggag ggtccaagag ctcacaagag agcctctctg agatttgacc
34081 cctccatccc ctcctcaggg aggttgtgga gtcactgggc ctcatgggat aggcccaggg
34141 caagcaatga tgagactcca aaccagctgg acacagtcca ggggcaggaa ggcagccaag
34201 cgtcctgacc cacaaggccc tatacagcaa tggagccatc gcggaagtcc gtcctcccaa
34261 tgaggatgtt gaccacaacc gggtggccgt ctcggcactg ctgctgggca tcgtgcagca
34321 ccttgaccac ctgatcctcg ttctcccgtg agagcagcaa gccccggccc cccagaccca
34381 tggctgcctt gtgataatct aaaagaagag attaagtcgg atagtggcaa ggactcagcc
34441 caccgcacat gcagccagcc caggagagga acacaagccc agctgggact gaaacagcct
34501 cctgtcctcc caacctcgct gaggccccct caccagtgta ggccaggcca caggccacgt
34561 tgctgcccag agagggcacc tgctcccgag aaatctgtgt ccagccagca tcattcccta
34621 ccaaggccat cactgggatc tacaggaaga tgatgaaggc aggccgcagc cttcagcccc
34681 atggcttggc cagccagagg acagagacag ggatagggcc tctgccagag aggaggctcc
34741 ctccccacct cagcccaccc ctggtgacat ctcccagaac cccaggccct aagcttccct
34801 caggccagcc cagtcacctt gtgtctgacg aatgtatcaa attcgatgag gctgtagcca
34861 aaagctccgt ccccaaacag gcaccagacc tagtcgggag gggacccagg atggttaggg
34921 tggatcagga cctagagagc agcaagtctc ccaactccag tgcctcacct cagcatctgg
34981 ccggcacagc ttggccccaa gtgcaaatcc tgcaccaact cccagagtcc caaaggcccc
35041 taagaggacc aagagcaggt gggtgtgaag gcgtcctgat ggatggtgcc cactgcccca
35101 ggcgggggcc cttccttacc aggatcgagc cagcgcaggg ggccgcgggg ctgtaccaga
35161 tgggcagcag tgcccacgaa gtccccgcca tccaccacca gaattgagtt gtcaggtagc
35221 gtttcctcca ccagctgcag cacctgcact gggttcaggt gctgggccac aggcatcgct
35281 gccttctccc tgcgacagga cagcgaaggt caccagggtt ggcagcaggg tctgccggga
35341 acccccttcc cacatccact ccacccaggc cccaccgaaa ggtctgctcc ttctgccggt
35401 cggcttcccg cagctcctcc acccagtctg gggcccaggt ctggccctga aggccctcca
35461 ctaacttcag cacgaaggaa cccacatctc ctggggaagg aagaggacac agctgtgggg
35521 agctggcttc ctcctgttct tccagcatca gaggcctcaa aacagcagga agccagcagc
35581 agcatccgag caggcagacg ggaagcaggc caggggtgct cctagaaaaa aaacaggtga
35641 aacaccccag aacctgccag aaccagggga gggcagccca ggtgaaaagg gaagacaagg
35701 cccaagaaac aagggttaag gagcacaagg caggtgacaa acccaagctg gcctcgtccc
35761 ctccctccac ctccttctgc ctgggaaaca ctcaatgaaa agagctacat ccaccaagca
35821 catctctcaa atccaggcac gtggaagcgc acagcgaacg ttactgaagt gaactaacaa
35881 acagccccgt gatgtatctt tcaccacccc atttttcaaa tgatcaaatg gaggctctga
35941 gaaggtgagg ctctgccgct gttcatctcc agttcaggcc tcctcgcttc tgtcctggcc
36001 attttgagca gcccccctgg cctccaccca gcccccttcc ggcctgccgt ccatggctgc
36061 tgagtggacc ttcccaacac agatgcaacg gtgctacttc tctgcttaat acaagagtgt
36121 cctgtcgcct aggctgcagg ggagtcaaca tcctcagaat gatgaaccaa gttcaagtcc
36181 aaggggacta aaataagggg aggggagaga tacggcagga agacaggcta aggggccaac
36241 gagtgtgctc tctgtcctga gggcagtggg cagtgtggga gggaacgtga tcacatgcac
36301 gttttcaagc ctccctctgt gtccacccca ccatttgttc ctacagaagc atctatcagg
36361 ccgggcgcag tagctctggt ctgtaatccc agcacttttg agaggccgag gcaggaggat
36421 cacttgaggc caggagttcc acgccaacct gggcaacaca gtgagatgct gattccacaa
36481 aaaaaaatta acaaattagc ctggcatggt ggtgcatact tgtagtccca gctactcagg
36541 aggctgaggt gggaggattg cttttgagcc tgggagtttg agggtgcagt gagctgtgat

36601 tgcactactg cattccagcc tgggtgacag agcgagacct cgtctctaaa aaataaaaat
36661 aaaaaaaatt taaagaaaga agcattcatc agtttgagag ccaactattt gtctctggtt
36721 gactggctac ccctagggca aggtctgggc ctcaatcact gtaccaggct caagccagct
36781 gcagcagaaa ccaggaggag agggactggg aggagcacat atgtgtacgg ggggattaag
36841 gggctcaccc tgcacagcct cctggggctt ccagaagatg tctgagttga gcaacatctc
36901 ttcccgatta cgattgacga tgatgatctt gctgctgtgg ctgaggacac ggccatagga
36961 taggcggaag tcacacacag ttcctggtgt gggggacagg aatgcagtag gcagaggccc
37021 catgtcgtct accttacttg atccactgtg gtggactgga tgcaaatgag tccctgttcc
37081 tcaccgagtc tccctgcacc tgtgccacct acagtgacct tgcagctccc cacaccccct
37141 gaatcgggac tggcctcatg acatgctttg gccaacagga tagggcagag gcaggttata
37201 ttttcccaga tggccataat atctcccacc ctacatccat ttgtacaagg tcaccttgac
37261 atgcctccca tggagagagg tgcagcctag gtcccctttc cttgaaagtg acacaccatg
37321 acacccaaca aggaagatat gcagtttctt ccttgttcac ttttagaggc ctgggtgggg
37381 ccaaggaatg tgctctctgt ctaagaagtc tgactaccct gaggccatca tgctgggagg
37441 aaaccggagc cacacataga gacctgtgta gttgccctag tccacagccc ctccaacagc
37501 tggcgtcaat tgcaagacct ctgagtgaag acacctccag atgattcagg acccccaccc
37561 cccattcctg cagactctac ttcaagaagc cacacagcct ccactcactc tcttgaataa
37621 ccgcactctc ctagccccat agttttctca tccttcacca agtcggccag cccaggctag
37681 cctactggag gatgagaaac aagggggcca tggagacagg ggtgaagaac tctatgaccc
37741 cagctgaggc catcccagac gagctgcagg gccagccaat ccccatacag gtgagcaagc
37801 ccagcctgga tcagcacagc tgccttcctg cctaacccaa gtgcagactc atgaatgagc
37861 ccagccaaaa ccacagctac cacctacctt ataggtggat cttgagaaat cagaaatgca
37921 tttttagcca ttgagtttgg ggatggttgt tacagggcag tagctatctg atacacctat
37981 tatgcgtctg gttgggttca aagcgcttta tgtgaaaatg tgtatcaagt acaataggtg
38041 cccactagac cttcctcctc cttcgtgtga agctgggtaa cagggtgagg gggttcctgg
38101 gggaaggagg gaaaggaaga ggctcaggcc cacctgctag gacaatgaca tccgccttct
38161 tcagggccgc actgcggttc tcccggatgt ggagggggtg gttgcggcct aacagccccc
38221 gtgccatccc tccaaggaag cagggaacac ccaaggtctc cacggcagcc ctgggagtcc
38281 aggcagagca gaggtcagtg tggccggggc ctcccgcctc cccaagagca cccgaggact
38341 ggggactggg aacgtgaaaa caagggcagg gctggcgctt ctcaccgaag cttgtcggca
38401 gacgttgggg tgagcagggc ctgactcccc agcaccatca gaggcctctt ggcccggctc
38461 aggatctcca cacagcgctg aacctggagc gagaggtggg gatcaaaggg ctgtcttttt
38521 atttatttat ttttttgaga cacagtttca ctctgttgcc caggatagag tgtaatggca
38581 tgatctcagc tcactgcagc ctccacctcc caggttcaaa cgattctccc gcctcagcct
38641 ccagagtagc tgggattaca ggcgcgtgcc accatgcctg gctaactttt gtattttcag
38701 tagagacagg gtttcaccat gttggccagg ctagtcttga actcctggcc tcaagtgatc
38761 cgcctgcctc ggcctcccta agtgttggga ttgctgctgt gagccaccgc gtccagccaa
38821 agggccgccc ttgagtcgcc atgagcaccc aagattaagg ccccagtact gactcaggga
38881 gagggtcagg gggccctctc ccctcaggga gagggtaggg tcctcagaca caaaagagag
38941 gcaattcacc tgctgcgggg aagcctgggg gatgtccagg ggcagcggtc cctcgggctg
39001 aggctcccag gctcctgcaa agaggttggc caggtaattc tctaaatacc tgcaaaaggg
39061 agggggaaga aaccaagttg ccagaattaa ctaactacaa ggattccagg atctgagcaa
39121 ggaaaggggc cgggagaaaa gccaggagca aagtggcatg gggacagggt ggatgagtga
39181 cacctcaggg acagacgaac cctagtggcc aggtcttcaa ctgcctgttt gccctcggta
39241 gcctctgggg tccaccccaa ctccacgtag ccccccagaa gcaaggaacg caggctcagc
39301 atgccaggat taagcctgtg ggtccaacag atgtgagctc aagtcctggc tctgtcactt
39361 cctagctgtg caacttaggg caagtgactt cacccttctg agcttccacc tctgtcaatg
39421 taaatatgaa cctccctccc caggttcaag gatctgtcca cttaggacat gaggcgcttt
39481 gcatgggagt tctgaacact gtcctctcct gcttcaaaca gggtcttctc ggtctccttg
39541 ctcactgggc cccagggccc caactttcct gaaggacctg tgcagggccc tggagacaca
39601 aagatagatc ctgcccagcc ctcccctcag ggggcgctct atccagcgaa agacagacac
39661 atgggcattg gcaggaaaca gtcggagctc agagtcagaa gcctctgctt tcaccgttaa
39721 ggacaggcca agacagcaca gaagcagccc aggatggaac cgtgggaagc cccgcaagta
39781 aagaaaaggc aagggaggct gaggcaggag gattgctcga ggccaggagt tcaaggctgt
39841 ggtgagctat gatc//

- LOCUS: AW138374 297 bp mRNA linear EST 29-OCT-1999
- DEFINITION: UI-H-BI1-adb-e-09-0-UI.s1 NCI_CGAP_Sub3 Homo sapiens cDNA clone IMAGE:2716145 3', mRNA sequence. *Ras homolog enriched in brain (RHEB)*
- ACCESSION: AW138374
- VERSION: AW138374.1 GI:6142692
- KEYWORDS: EST.

1 cggccgcgcc gggagagagc ggcatacaga gcaggggcgg cggcgggcgc ggctgcctct
61 cgctcgctag ctcgcgcgct cccaaccgcc cggaaccgac cgcgcggcgg cgcccctccc
121 ccccacaaca cgcccacgtg accggccggc gtcagcaccg cccctcttcg cgccgtggcc
181 cgccgcctcc gccccccgaa atacgcgggg ggtgcgtcgg ggcgacgttt tactttaaag
241 gcaaaaaaaa ggggacgccg cgatgccccc agaaaagtca cgactgacct cgtgccg
//

- LOCUS: AI218134 499 bp mRNA linear EST 01-DEC-1998
- DEFINITION: qh27f10.x1 Soares_NFL_T_GBC_S1 Homo sapiens cDNA clone IMAGE:1845931 3' similar to gb:X03072_cds1 WNT-1 PROTO- ONCOGENE PROTEIN PRECURSOR (HUMAN);, mRNA sequence. *wingless-type MMTV integration site* family, *member* 6 *(WNT6*
- ACCESSION: AI218134
- VERSION: AI218134.1 GI:3797949
- KEYWORDS: EST.

1 gcggccgcag cacagcaggt cgcagccgct gaggtccggg gcgctgctat tgcaggcgcg
61 accgcgcgtg ccgggggagc cggtgcgtcg gttgggggcg cagaagtcgg gcgaatcggc
121 ggcgtagagg aggtccgctc ggcccggcgg cttgagcgtg cggacggcgg gcagcagggc
181 cttgccgtcg ttggtgccca tgacgcgtga ggcgccgtgg aagcgctcca gcagccgcgc
241 gcccacctcg cgaaatggag gcagcttctg ccagcaggtg cgcagcgcgc atgatcccga
301 cagcccgtgg catttgcact cggtgcgcgt gtggctccgc acggcctgcg ggaggcagag
361 gggcggacgc atgtggacag gtagggggtg cagggaaggg cccagcggat ggacagaaaa
421 taagattggg aatgggaggt ganaggaaga gaggaaaatg cagacaaaag gagaccgcaa
481 gttcaggggg aggaaatca//

- LOCUS: AL050219 1031 bp mRNA linear PRI 18-FEB-2000
- DEFINITION: Homo sapiens mRNA; cDNA DKFZp586J1623 (from clone DKFZp586J1623). *immunoglobulin superfamily, member 4B (IGSF4B)*
- ACCESSION: AL050219
- VERSION: AL050219.1 GI:4884460

1 agccgtaggt gccactgtca ctcttgttga ggaaagggaa gatcagggca ctctcctggg
61 tcatcttcag gggtggcaca ctgccctcct tctcccatag gtactgctgg gggctgtcga
121 ggcagacaaa tcagaagagc tcaggagagc ataggccagg gagctaaaag aaaaaaaaaa
181 ataactggga gcagggatgt caaggactgt ttgatgaggc tggagaaatt atcggggcca
241 gagacactgg agctgctata cgccaggtgg cctcttgggg ttcgttctgg ggaagctgta
301 gatctccaga cttgtatctt actttaactt ggcctttgtg ggaaactcag ggactgagtt
361 agggagtggc cccttcccat ttgtgctgct ttgcagaaga actaattgca ggcggacatt
421 attggacagt cagggaaatg agaggctcag gtcagacaat acagaggaga taggaaggag
481 ctgttatgtg aagaaggcaa tctgagaatg tggagagtaa ggaggagacc aggaaatgga
541 tcttcttaca ctggattgcc gcgaccctca cagtgtagca acagcttctg gccctcacga
601 ggatggggag ggtctggcct aatcatcgca gttggtgtgt ctgtggagaa ggaaatggta
661 tcaatgaata cctccccaca cggcttagga gattccctca cttagttgca cactacagct
721 tctacctcta aatgtttccg cagctcaaga agtgcaacaa agaccttcct tctcctactt
781 ctcatgaaca aggaccatta ttgctattga atatgtgagc aacttctctt gcaaccttgt
841 gtgataggat tctggaaggt ctgtccttgg tgggccctaa gagatgtctt aacagaaagc
901 tgagaaggga aatgagtggt ttacctgtgc ctagcaaacc tcctagcaca taggaaagac
961 ctaatacata ttttttacag aacaaataaa gttgaacttg tcacatattg agctcacaaa
1021 aaaaaaaaaa a//

- LOCUS: AK022494 3389 bp mRNA linear PRI 12-SEP-2006
- DEFINITION: Homo sapiens cDNA FLJ12432 fis, clone NT2RM1000018, highly similar to Human mRNA for KIAA0066 gene.
- ACCESSION: AK022494
- VERSION: AK022494.1 GI:10433912
- KEYWORDS: FLI_CDNA; oligo capping; fis (full insert sequence).

1 aggcccggcg ctcctcaaga tggctgccga cagtgagccc gaatccgagg tatttgagat
61 cacggacttc accactgcct cggaatggga aaggtttatt tccaaagttg aagaagtctt
121 gaatgactgg aaactgattg gaaactcttt gggaaagcca ctcgaaaagg gtatatttac
181 ttctggcaca tgggaagaga aatcagatga aatttccttt gctgacttca agttctcagt
241 cactcatcat tatcttgtac aagagtccac tgataaagaa ggaaaggatg agttattaga
301 ggatgttgtt ccacaatcta tgcaagattt gctgggtatg aataatgact ttcctccaag
361 agcacattgc ctggtaagat ggtatgggct acgtgagttc gtggtgattg cccctgctgc

421 acacagtgac gctgttctca gcgaatctaa gtgcaacctt cttctgagtt ctgtttctat
481 tgccttggga aacactggct ggtgccactc tttgtgcaaa ttcaccacaa atggcgaaga
541 atgtatgtag gagaatgtca aggtcctggt gtacgaactg atttcgaaat ggttcatctt
601 agaaaagtgc caaatcagta cactcactta tcaggtctgc tggatatctt caaatcaaag
661 attggatgtc ctttaactcc attgcctcca gttagtattg ctattcgatt tacctatgta
721 cttcaagatt ggcagcagta tttttggcct cagcaacctc cagacataga tgcccttgta
781 ggaggagaag ttggaggctt ggagtttggc aagttaccat ttggtgcctg cgaagatcct
841 attagtgaac tccatttagc tactacatgg cctcatctga ccgaagggat cattgtggat
901 aatgatgttt attctgattt ggatcctatt caagctccac attggtctgt tagagttcga
961 aaagctgaga atcctcagtg tttgctaggt gattttgtca ctgaattttt taaaatttgc
1021 cgtcgaaagg agtcaactga tgagattctt ggacgatctg catttgagga agaaggcaaa
1081 gaaactgctg atataactca tgctttgtca aaattgacag agccggcatc agttccaatt
1141 cataaattat cagtttcaaa tatggtacac actgcaaaga agaaaatccg aaaacacaga
1201 ggtgtagagg agtcaccgct aaataatgat gttcttaata ctattctcct gttcttattc
1261 cctgatgctg tttctgagaa accattagat ggaactactt caacagataa taataatcct
1321 ccatcagaga gtgaagacta taatctctac aatcagttca agtctgcacc atctgacagt
1381 ttaacataca aactggcttt gtgtctctgt atgatcaatt tttaccatgg agggttgaaa
1441 ggagtggcac acctctggca ggaatttgtt cttgaaatgc gtttccgatg ggaaaacaac
1501 tttctgattc caggattagc aagtggaccc ccagatctga ggtgttgttt actgcatcag
1561 aaactacaga tgttaaattg ttgtattgaa agaaagaagg cacgtgatga ggggaaaaag
1621 acaagtgctt cagatgtcac taatatatat ccaggggatg ctggaaaagc aggagaccag
1681 ttggtgccag ataatctaaa agaaacagat aaggaaaagg gagaggtagg aaaatcttgg
1741 gattcctgga gtgacagcga aggagaattt tttgaatgcc taagtgatac tgaagaactt
1801 aaaggaagtg gacaagagag tggcaagaaa ggaggaccta aggagatggc aaatttaagg
1861 ccggaaggac ggctctatca gcatgggaaa cttacactgc tgcataatgg agaacctctc
1921 tacattccag taacccagga accagcacct atgacagaag atctgctaga agagcagtct
1981 gaagttttag ctaaattagg tacatcggca gagggggctc accttcgagc acgcatgcag
2041 agtgcctgtc tgctctcaga tatggagtct tttaaggcag ctaatccagg ttgctccctg
2101 gaagattttg tgaggtggta ttcaccccgg gattatattg aagaggaggt gattgatgaa
2161 aagggcaatg tggtgctgaa aggagaactg agtgcccgga tgaagattcc aagcaatatg
2221 tgggtagaag cctgggaaac agctaagcca attcctgcta ggaggcaaag gagactcttc
2281 gatgatacac gggaagcaga aaaggtgctg cactatctgg caatccagaa acctgcagac
2341 cttgctcggc acctgttacc ttgtgtgatt catgcagctg tactcaaggt aaaggaagaa
2401 gaaagtctcg aaaacatttc ttcagttaag aagatcataa agcagataat atcccattcc
2461 agtaaagttt tgcacttccc caatccagaa gacaagaaat tggaagaaat cattcaccag
2521 attactaatg tggaagctct cattgccaga gctcggtcac taaaagccaa gtttggaact
2581 gagaaatgtg aacaggagga ggaaaaggaa gatcttgaaa ggtttgtgag ttgcctgctg
2641 gagcagcctg aagtgttagt caccggtgca ggaagaggac atgctggcag gatcattcac
2701 aagctgtttg tgaatgccca gagggctgca gctatgactc caccagagga ggaattgaag
2761 agaatgggct ccccagagga aagaaggcag aactccgtgt cagacttccc accccctgct
2821 ggccgggaat tcattttgcg caccactgtg ccgcgccctg ctccctactc caaagctctg
2881 cctcagcgga tgtacagtgt tctcaccaaa gaggacttta gacttgcagg tgccttttca
2941 tcagatactt ccttcttctg attcttctag cattactcgt tggtggcttc agagacagtg
3001 ctgcctcctc ctgagggagg gaaggtacca gggagaacct gggaggtcct ggagagggcc
3061 ctgtccagtt gggtgatcag gaatcaaacc agcatcggaa agacttccca gcaccaagct
3121 tgagctgtgt cgtttcgtgg agggggcagc gaggatgggc ttgagctgtt gagagatttc
3181 tgccctagag atggcctttg tatatggggg ggtggtgggg ggacacaaac acatcagaca
3241 ctccgtcctc acactggcag gacgatgttc atcgcattct cttctgtgac cagcctctag
3301 gctagcggct gcattcgtgg tctgtgcaaa cacttcgtgg ttctatatat cagcagcaag
3361 tgtgcaaaat aaaggacctg ttaactcag //

- LOCUS: AI252582 240 bp mRNA linear EST 10-NOV-1998
- DEFINITION: qv25b02.x1 NCI_CGAP_Ov31 Homo sapiens cDNA clone IMAGE:1982571 3',mRNA sequence. *ATPase, H+ transporting, lysosomal 9kDa, V0 subunit (ATP6V0E)*
- ACCESSION: AI252582
- VERSION: AI252582.1 GI:3849111
- KEYWORDS: EST.

1 gcctactact actatacggc tgcgagaaga cgacagaagg gtggtcagta ggagagcacg
61 ttcagaggga agagccatct caacagaatc gcaccaaact atactttcag gatgaatttc
121 ttctttctgc catcttttgg aataaatatt ttcctccttt ctatggaaaa aaaaaaaaaa

181 aaaaaaaaaa aaaaaaaaaa acaaaaaaaa aaaaaaaaaa aaaaaaaaaa gtcgtatcga
//

- LOCUS: Z97630 79528 bp DNA linear PRI 18-JAN-2007
- DEFINITION: Human DNA sequence from clone RP3-466N1 on chromosome 22q12-13,complete sequence. *glycine C*-*acetyltransferase* (*2*-*amino*-*3*-*ketobutyrate coenzyme A ligase) (GCAT)*
- ACCESSION: Z97630
- VERSION: Z97630.11 GI:4582128
- KEYWORDS: HTG.

1 ctccaggagc tcagctccct gaaaggaaaa tgatgccaag ccaggcgcag tggctcacac
61 ctttaatctt agcaatttgg gaggctgagg caggtggatc acttgaggtc agaagttcaa
121 gaccagcctg gccaatatgg tgaaacccca tctctactaa aaatacaaaa cttaggccgg
181 gcacagtggc tcacacctgt aatcccagca ctttgggagg ccgacgtggg cggatcacct
241 gaggtcggga gttcaagacc agcctgacca acatggtgaa accccatctc tactaaaaat
301 acaaaaatta gccaggcgtg gtggtgcatg cctgtaatcc cagctacttg ggaggctgag
361 gcaggagaat cgcttgaacc tgggaggcag aggttgcagt gagccgcgat cgagattgcg
421 ccattgcact ccagcctgga tgacagatcc agactctatc tcaaaaaaaa aaaaaaaaaa
481 aaaaaaagaa gaggaaagaa agaaaaagga aatgacgcca gagtgggcag gtccaacagg
541 actttcagcc caaaccacac atggcacctg tgtccctcac caatgcctgg cctctggcac
601 tgagcttggg tcacctgtcc acatctactt cccatggagt gagttatttc ctttgaggca
661 tgagtgtcct ctctgccagc tggacaggac tgtcctgatg ggtcagatag cagctaatcc
721 tatcaggaga gaggaactgg ggcccaatct catttctctt ggcggtcccc agactagcca
781 caaatttgcc cacctcctgc caggttaaga gaaaggcagt tttgagagtt gattaagagc
841 atggagtttc ctgtaatccc agaactttgg gaggccaagc tgggaggacc acttgaggcc
901 aggagttcaa gaccagcctg ggcaatatag tgaggcccca tctctacaga aattttaaaa
961 attagctgaa cgtggtgatg cgcacctgtg gtcccagcta cttgggaggc tgaagcagga
1021 ggatcacttg agcccaggag ttcaaggttg cagtgagcca tgatttgtgt ttcagcctta
1081 gtgacagagt gacaccctgt gtcacaaaca aataaacaac aacaacaaca aaaggccagg
1141 cttggtggtt cacacttgta atctcagcac tttgggagat agaggcgggt ggatcacctg
1201 aggtgaggag ttcggaacca gcctggccaa tatggtgaaa ccccgtctct actaaaaata
1261 caaaaattag ctgggcgtgg tggtgggtgc ctgtaatccc agctacttgg gaggctgaga
1321 caggaggatc acttgaacgt gggaagtgga ggttgcagtg ggctgagact gcaccattgt
1381 actccagcct gggcaacaag agcgaaactc cgtctccaaa aaaaaaaaaa caaaacaaaa
1441 aaaagagcat ggaattggac tgctttaggt aagttccttc cccacctgtc aaatgaagtg
1501 ataacaacag caaaagaccg aatgttaata cctacggata gatcacttag cacagtgtgg
1561 gcagatagta actgctcaat aaatgggaac tactgttaca tacctgtatg tagttctcta
1621 atatccctat tttataaaaa tgaggtcccc ccatattttt cttagcttcc ttttgtcaat
1681 taatatatca tagacatttt tccaggtctg cacatgtagc ctcctctatg tgagagccgc
1741 atggccttcc aaaggacgcc gtccatgcaa gaggctaaac tatgtctgtg ggttcaaaac
1801 cctcagtctg tgccatggtg ctttaaaact tggaaatggc tcatacctgt aatcccaaca
1861 ctttgggagg ccaaggtggg aggatcgctt gagcccaaga gtttgagaca aacctgggca
1921 acatgtgaaa cactgtctct acagaaaata ccaaaattag tatttttggt gtggtggcat
1981 gtgcctgtag tcccagctat ttgggaggct gaggtaagag gatcgattga gccctggagt
2041 tcaaagctgc aatagccata attgcactcc agcctgggct acagagaaga cctgtcttaa
2101 gaaacacaaa caggctgggc gcggtggctc acgcctgtaa tcccagcact ttgggaggcc
2161 caggcgaggg gatcacgagg tcagtagatc gagaccatct tgcactccag cctgggagac
2221 agcgagactc catctcagaa aaaaaaaaaa aaagaaaaag aaacacaaac aaaaacaaac
2281 ttggaaatta gttaaggttt agttcagtta gatcagttaa agtttaactg ttaaactgtc
2341 acctgcattt ttatttcttt gtctggaaat gtgtgtttgt ctttctgttc atttgacttc
2401 taacccacat gggatgggtt ttcctaactt gggagaagca ggtgagaggt cagcctccac
2461 agggtgaggc tgtcaaggag gaggggggaa ccaggacagc cagtggccag ggcaggacaa
2521 aagaaggccg gccatctttg ttttgttttg ttttgttttg ttttgttttg ttttgttttg
2581 ttacgaggca gagtcttgct gtgcctcacc caggctggag tgcaataatg agatcacagc
2641 tcattgcagc ctccatctcc cgggttcaag tgattctcct atctcagttt cccgagtagc
2701 tggggctaca ggcgtgcgtg tgccatcaca cctggctaat tttgtatttt taaaaattat
2761 tttattttat tttattttat tttattttga aacggagtct cgctctgtca cccagtctgg
2821 agttcagtgg cacgatctcg gctcagtgca agctccgcct cccgggttca tgccattctc
2881 ctgcctcagc ctcctgagta gctgggacta taaccgcctg ccacgacgcc cagctaattg
2941 ttttgtattt ttagtagaga cggggtttca ccttgttagc caggatggtc tcgatctact
3001 gacctcgtga tccacccgtc ttagcctccc aaagtgctgg gattacaggc gtgagccacc
3061 gcgcccggcc tattttgtat atttttagta gagatggggt ttcaccatgt ctgctgggct
3121 ggtctcgaac tcctgacctc aggtgatcgt ctaccttggt ctcccaaagt gctgggatta

3181 caggcgtgag ccactgtgcc cgcctttttt tttttcttct ttttgttgtt gttggtttta
3241 tttatttatt tggacagagt ctcactctgt tgtctgggct ggagtgcagt ggtgcaatct
3301 cagctcactg taacctctgc ctcccaggtt caaacgattc tcctgcctca gcttccaggg
3361 tagctgggat tacaggcgcc caccaccaca cccagcttat ttttgtgttt ttggtagaga
3421 cagggtttca ccatattgac caagctggtc tcaaactcct gatctcaagt gatctgccca
3481 cctcggcctc ccaaagtgct gggattacag gcgtgagcca ccgtgccggg ccatgcgcca
3541 tctttgtttc tgttccagat gcctgggagt ccttccatct cttccctcac cccccacagc
3601 caacccttta gcaaggactt ttcctcaata tttctcatat ctgcctcttc cttccacccc
3661 cacggccacc actttaattc agaacttgga tcactgagat ggcccctaat ggtttcactg
3721 ccttcttctg cccttcccta gtccattgtc catatggcaa cccggggcag gtgcagtttg
3781 gaaaggaggt ggcactacct ggtaatgctg aagctgtgta tacttctagc tccttcccct
3841 ttgcaccagg gcacttgcag taggaaagtc acagcgacat tgtctgcaaa gcaagaaact
3901 ggaaatgtcc aggcacagca gctcacgcct ataatcgcag cactctggga ggccaaggtg
3961 ggtggatcac ttgagcccag gagtttgaga ccagcctggg caacatagtg agacgcccat
4021 ctctataaaa aaagaagaaa gtggaaacaa ctcaaatctc catcaacaac agtggattct
4081 tttcctgttg ctgctctaac cagttacccc aaactcagcg gctgaaacac acacatttat
4141 tatcttacag agctggaggt caggagtcca aaatgatttt actgaactaa aatcaacaag
4201 tcagcaggta tgcatttctt ctgcaggctc taagggagaa tctggttttt gccttttttt
4261 ctggctttca gaagctgcct gctttcctgg gttcatgggt ccttccatcc taacgtcagc
4321 agtagtgggc aggcctttct caggacactg tctctgattc tgactcttct gcctccctct
4381 ctcccattta aggatccttg gccgggcacg gtggctcacg cctgtaatcc cagcagtttg
4441 ggaggctgag gcaggcagat cacctgaggt caggagttcg agaccagcct gaccaacatg
4501 gagaaacccc gtctcttcta aaaatataaa attagtcaga cgtggtggcg catgcctgta
4561 atcccagcta ctcgggaggc tgaggcaggg gaatagcttg aacccaggag atggaggttg
4621 cagtgagctg aaattgtgcc attgcactcc agcctgggca acaagagcaa aactccgtct
4681 caaaaaaaaa aaaaaaattc cttgtgattc cactgggccc accaggataa tccaggatca
4741 tctcccatct ccaggccggg tgattagcaa cctttgttct cccttgccgt taaaggaaca
4801 tttttgaaag tttcagagat taggatgtgg tcatatttac aggccatttt tctgcctccc
4861 cacacagtta cccacacagt tacccatcgc ataatagggc tggggctggg ggatgtgagc
4921 agaatccctc catacccaac atataaccac gcgaggcccc cagctgaggg cccggtgtgt
4981 tgggcagaga gctgaaggca ggggaacact gtccagtcgc tgcacagcag tgtgaggaaa
5041 ggaagaggag tcgtctgtaa aaacccattg actgtgctca tttcacactg cgtgcctcta
5101 tcaaaacatc tctagccagg cgcggtggct catgcctgta atcccagcat tttgcaaggg
5161 tgaggcaagc agaccacctg aggtcaggag ttcgagacca gtctggccaa catggcaaaa
5221 ccccgtctct actaaaaatc ctaaaattag ccagggcctg gtggcgcgtg cctgtaatcc
5281 cagctgctca ggaggctgag gcaggagaat cgcttgaccc gggaggcaga ggttgcagtg
5341 agccgagatc atgccactgc cctctagcct gggcaacaga gcaagactcc atctcaaaaa
5401 caaaacaaaa caaaacaaaa tctcatgtat cccatacata tatacatcta ttatataccc
5461 acaaaaatta aaattaaaaa aaagaaaaaa acaaaaaatc cccattgact gtactctctg
5521 ttatattcct ggggccctca aatgttgatt acttttcagt tttgagaatc taagtgacac
5581 ctagcctgga caggaggagg ggagagcccc tgtttctggc aggtttctaa gaggaatcac
5641 aggactgggc tgagaggagg ggacccagca cccaggctag agcagccacc cctccatccc
5701 tgagtgaggg gccatctgct gcccccgcc tctccctcgc cccacccccc gcctctatct
5761 caagccttgc tcctttcccc tcgggtgcct ccacctgccg tttgccccgt cctcacagag
5821 tgtgaatctt gactcaaagt gtggacgtca aagccccttg gaagtgggaa aggcccaggc
5881 gggtgcagat tgtttatttg cttgctgggt gctgggtgtg aaaggaacag acacgtgctt
5941 gcctcgtggg gctcagtctc tagtggggaa gatgggcact gctgtcattg taaagtgtga
6001 ttttgtgaaa agtgctaaga aggaaaggac aggggctatg agagggggtt gtggagtcct
6061 aattaggaag gaggagttga actggtgaga ttgagggaaa gcaaaaagcg aaagcaggta
6121 agctctaagt gtgcctttct tcatgctcca gaacacatgc cctcctgcac aaataattca
6181 caatcttcct gcaaccagcc atcaccagac gctcagctga cagaaaattg caaattagct
6241 cactgcaacc tcggcactat cagtactgcg taaagccctc tccagcatac agcacgagca
6301 ccatcctgtc ccgtccctag caagcctctg tctccttgca gacagcccct tctctgctgt
6361 gctgcctgtt gcaaatttgc aacgcatttt ctttttcttt ttttctttct tttttgttt
6421 tttgagacgg agtctcgctc tgtcgcccag gctggagtgc agtggcgcga tctcgcctca
6481 ctgcaagctc cgcctcccgg gttcacgcca ttctcttgcc tcagcctccc aagtagctgg
6541 gactacaggt acgtgccacc actcccggct aatttttttg tatttttaat agagatgggg
6601 tttcactgtg ttagccagga tggtcttgat ctcctgacct cgtgatccac ctgcctcagc
6661 ctcccaaagt gctgggatta caggcgtgag ccaccgtacc cggccttttc tatttttttt
6721 ttttttttga gacaagtttt caccgtgtca cccaggctgg agtgcaatgg cgcaatcttg
6781 actcactgca atctctgcct cccagactca agggattctc ctgcctcagc ctcccaagta
6841 aatgggacta caagcatgtg ccaccacgcc cggctaattt ctgtattttt agtagagatg
6901 gagtttgcca tgttggccag gctggtgttg aactcctgac ctcaggtgat ctgtacgcct

6961 cagcctccca aagtgctggg attacaggcg tgagccatca ctcccggctg caacatattt
7021 tcataccttc tctaataaat gtgcctttct ttacctataa ctgtcttggt aaattcttct
7081 tactcctggg ctactggcct cagatagttg ccgctcacct gagacagggg tgacaagacg
7141 gcttattcat agaaagcata atgaccaaat tagaagaact gggatatcca tcaccttaaa
7201 catttatctt ttctttatgc tgggagcatg caaattattc tcctgtagct atttttgaag
7261 tatacagtgg gttatggtta actgcagtca ccctactgat ctatggaaca ctatgtctta
7321 tttcttctag ctaactgtat attgtaccca ttgatcagct tctcttcttc ctcccctcct
7381 tgctatcctt cctggcctct ggtaaccacc aatttactct ccatctttgt gagatccact
7441 tttttttttt tttttgagtc ggggtttttt agtagagacg gggtttcacc atgtgagcca
7501 ggatggtctc catctcctga ccgcgtgatc cgcccatctc ggcctcccaa agtgttggga
7561 ttacaggcgt aagccaccac gcccagcctt tttttttttt tttttttgag acggaatttt
7621 actcgttacc caggctggag tgcaatggca cgatctcagc tcaccacaac ctccgcctcc
7681 cgggttcaag caattctcct gcctcagcct cccaagtagc tgggattaca ggcatgcgcc
7741 accacgccca gctaattttg tatttttagt agagacgggg tttctccatg ttggtcagac
7801 tggtctcgaa ctcccgacct caggtgatcc tcccgcctcg gcctcccaaa gtgttgggat
7861 tagaggtgtg agccactgcg ccaggccgag atcccccttt tttttttttt tttttttgag
7921 atgaagtctc gctctgttgc ccaggctgga gagtgcaatg gcgcaatctg ggctcaccgc
7981 aacctccacc tcctgggttc aagtgattct tctgcctcag cctcctgagt aactggaatt
8041 acaggcgcct ggcaccacgc ccggctaatt tttgtatttt taggagagat ggggtttcac
8101 cacggtgacc aggctggcaa agatccactt ttttagtgtt catgtatgag tgaaaacatg
8161 caatatttgt ctttctctgc ctggcttatt tcagttaata tgatgacctt cagttccatc
8221 tgtgttgttg caaatgacag gatttccttc ttattggtgg ctgaataata ttccctttgt
8281 ttatatacca cattttcttc atccattcat ccattgatgt gcgcttgggt tgactccata
8341 ttttggctat tgtgaatggt gctgcaatta ccatgggagt gcagatatct cttcaatatg
8401 ctgatctcct tcctttgga tatataccga gtagtgggat tcatctgaac catttttttt
8461 ttagactagt caagttcagt agtgagaagg gagcaaaaag tagaacaagg ggccaggcgt
8521 ggtggctcat gcctgtaatc ccagcacttt gggaggctga ggtgggtgga tcacttgagg
8581 tcaggagttt gagaccagcc tggccaacat agtgaaaccc cgttccacta aaaatacaaa
8641 aattagctgg gcgtggtggt gggagcctct aatcccagct actcgggagg ctgaggcatg
8701 agaattgcct gaacccagga ggcagaggtt gcagtgaacc gagatcgcgg cactgcactc
8761 cagcctgggt gacagagtga gactccatct caaaaaaaaa aaaaaaagtt agccgggcgt
8821 ggtggcgcac acctgtaacc ccagctactc aggaggctga ggcaggagaa ccgcttgaat
8881 ccaggaggcg gagattgcag caaactgaga tcacggcact gcactccagc ctgggcaaca
8941 gagtgagact ccgtctcaaa aaacaacaac gaacaacaaa aaatacaatt gcaaaccacc
9001 accatttgac actgcctata tccaccccca accctgctcc agttgtttct ttgcaattgc
9061 cactttccag catactatat aatttacttt tttttttttt tttgagatgg agttttgttc
9121 ttgtcgccca ggctggagtg caatggcgtg gtctcagctc attgcaacct ccgcctccct
9181 agttcaaacg attctcctgc ctcattctcc caagtagcta ggattacagg ggccccccac
9241 cacgcccggc taatttttgt attttcagta aagacagggt ttcaccatgt tggccaggct
9301 ggtcgcaaac tcctgacctc aggtgatcca cccacttcgg cctcccaaag tgccgggatt
9361 acaggcgtga gccaccgcac ctggcctaat ttacttcttt atgtagtcta ttgtgtatct
9421 atcttttcca gggaacctca gctccacaag ggcagggctc tcttgtcact ttggtttatg
9481 gccatatcac agagcccatc ccagtgcctg gcacatatta aggaatgcat taggattcca
9541 aaaccagcca ggagcggtgg ctcatgcctg taatcccagc actttgggag gccgaggcga
9601 gtggatcacg aggataggag ttcaagacca gtccggccaa gatggtgaaa ccccgtctct
9661 actaaaaata caaaaaatta gttgggcgtg gttgtggaca cctgtaatcc tatctattcg
9721 ggaggctgag gcagagaatt gcttgaaccc gggaggcaga ggttgcagtg agccgagatc
9781 gcgccactgc actctggcct gggcgacaga gcaagacgcc atctcaaaaa aaaaaaaaaa
9841 aaaaaaaaaa gattacaaag cccagaatgc cacaagacca caggtggtgc agtgaacagg
9901 gcattgcctt gagagtctat catccctggg caagtcatta aacatagggg tgggggtggg
9961 agcctcctgt ctgtaacgtg gaggtgtcac gtgccaaggg ctcaggcaca cgcgcgcgca
10021 cgcacaaaca cacccattag taaggacacg acccctggca ttctaatgcg atttatactt
10081 ttccatgaag gaattcattt ccccctctca ctaaccccag agacaggtag agccgacagg
10141 tggaagaact gaggcccgag agagacagcg ggggaccctc cccgcgccac ggcacagcag
10201 ctgtccgacc ccctcctcac gcccgggggg cgcgacggct gccgagggtc gcgggcaggg
10261 ggcgcggcgc cggggactac atttcccagg agcagttgcc gcggttccgc cgttcctggg
10321 gtttcaagag cctcgcgtta aagggggcgg gggaggttgc gcagtccgcc agctggaaga
10381 tggctctgcg cccggtgggg acccgagagc cgcctgcctg ggtgcccagg cggggtccgg
10441 ctgcagtgaa gaagcccccc agctctgcag tgtccctgag gggaggcgag gcgaggcgag
10501 gcgaggcgag gccaagccaa ttggaggccg tctttcctcg tcttgcccgc ctcggcttcg
10561 gcctctgcgc ccaagcccca gggaaccccc tcccttcccc tccaactccc cggaacaggg
10621 gaaggggcgg tgaacctcgc cgcctggcgg gagaaggggt gtccactagc tggaagcccc
10681 tgcaacccga ggcaacccct ttccccaatt ctggcttcag ttccgggaat ttacaggtcg

10741 ctgggatggg cggggagaag ggacaggaga gatacaaaat gaaatgaacg cccaggtgtc
10801 agggagctga gccttacagt accccgcccc caatccatcc ttctaagtcc cggcctccg
10861 cggtaaaata ggggtgatgg ggatagtgat caaaggcagg aggcgcaaag gatccagaaa
10921 ccggtgggga gcactgtgca aactgtaaag cgctgtgcaa ataaaattat tgatcgtcgt
10981 gtcattggat tcatccagtc tttaagacat gcacggatct ggggcggggg atggggtgac
11041 atggttctgg gctctcaggg tggaacatct cggatacggg gagggggggc tatttgggtg
11101 cgtattaacc caaccaggtc ttaatgggaa cccgagttgt ctttgaaaat aaccaaaaca
11161 aagccaccag aatgttgtgt tttcaaatga cgaaacttaa gagaaattga caccatcaaa
11221 agacgaacct tgaccttttg aactaacctg accccccatt gcttcccttc cccgcctgtg
11281 ccacggagat aagcccagag agaaggcatc tgacaccgag tcttgcgctc cccctccccc
11341 ttcctcccct aatcatttgg attaatccat cccttggcgt gcgggggaaa cccattaatt
11401 tgtcctggct tcagagactc ccaggtccag taggagccct cccacaccca cgcagctacg
11461 atcccgtccc caacatcaaa accctcgaac ccctcccctt tacccctccc cagtagccca
11521 ctgtgtctgc aaacagctct tggaccatac cgggatgcgt tcatcatttt gaccagcaac
11581 aaactcccac tcccagggta ttgtttaagt gaacagtgac cggcctctat gcttagggaa
11641 taaaagcggg gttgtgggag aggggcccag ggagcaaacg tcgcagaatc tcagcccagg
11701 gccgctgcca agcggacccg gcacgtgagt aggaggtcgg ccgcagacaa ggggtcctag
11761 ggtctctgag gtctctcctg ctggcctctg ggcccctaaa gcccctcgag cttcagaact
11821 ccctacagac agtggcgccc cgtgtccgcc tcgtctacac ctgccaagca gcacgggaaa
11881 atctgtgtgc attttaacaa agacatcgca tcttcccagc aaaatggcgg tggtcgcggc
11941 cacggctctc taaacaactc cacactttcc gaggacaggt cacaacccaa gggacataac
12001 cccctcctcg acctctaccc tagcgccccg ctttcccaag tccccaaagc actcagggcc
12061 tctcctcatt acagggcccc cccggaccgt ccctggttag agcttctggg gcagcttctc
12121 cattgtggac cgccctcccc cgcccccaca ttgccttcaa ggagccttgg gacggggact
12181 gaacccccag gctgggcctc acggatgggg ggcagcgagt tctgtctctg acaccccaaa
12241 acttccaggc taagagaagc acacaaatga attcccgatt agggtatatg ggagatgtag
12301 gggtctttga ggagaagagg ccacccggga ggccctcctc atctctcaga gggtccgggc
12361 agcactgggg gttcggcctc aggagggaac ctcaaggcag aggtcggggg gcgcgctggg
12421 ggagagacgg cagaggaggg ggaagggaca aagtttgaaa gtacccttcc aactctttca
12481 gggaaagttt ccttgggggc cccatggaca gagggggtag aggtgtggag ggaccggtgg
12541 cggggtgacc cccgagaggt gaccggaccc cgggggagcg acccctcccc cctgtcccgg
12601 ctcggcccgg ctgggagtcg cccagctcgg ggccgcgtgt gttagttggg gccgctttcc
12661 ggcagctcca ccgggctagg ggaccccgca gcgggccggg agcagagccc ccggtagcct
12721 caggggagag ctttgctacg gggggtttcc cgcaccgagg ctccctagcc ccgcagagcc
12781 agccccccgc aaaggggaaa tgtgccggca caccagcggt cctcggcgcc gtttggggtg
12841 cgtggcgggc gcggggggcg gcggccgggc cagcgggccg cccggcgggg aagcccaggg
12901 agccaggcag cggcccaggg cggcggcggg gagcaggaag gcccgggccg gggggaaagg
12961 tcggatttgc tcggcggaag aaacacagat ggcggcggcg cagcgccatt ccggccgggg
13021 agcaggcagc cagcagccct gtcctcaccg cggtccgccc gccgccgcta aatacccgga
13081 tgcgccgccc aagcgccaga cgcggagctg ggaaaaggga ggcagaggag gcggaggcag
13141 aggcagaggc agaggcagag cccgagcccg gtgccgagac caagcgacag accggcgggg
13201 ctgggcctcg caaagccggc tcggcgagct ctcccgacac ccgagccggg gaggaaaagc
13261 agcgactcct cgctcgcatc cccgggagcc gcactccaga ctggcccggt agtcaggggc
13321 tcaggagcag atcccgaggc aggctttgct cagcctccga cgagggctgg ccctttggaa
13381 ggcgccttca acagccggac cagacaggcc accatgaccg agaattccac gtccgcccct
13441 gcggccaagc ccaagcgggc caaggcctcc aagaagtcca cagaccaccc caagtattca
13501 gacatgatcg tggctgccat ccaggccgag aagaaccgcg ctggctcctc gcgccagtcc
13561 attcagaagt atatcaagag ccactacaag gtgggtgaga acgctgactc gcagatcaag
13621 ttgtccatca agcgcctggt caccaccggt gtcctcaagc agaccaaagg ggtgggggcc
13681 tcggggtcct tccggctagc caagagcgac gaacccaaga agtcagtggc cttcaagaag
13741 accaagaagg aaatcaagaa ggtagccacg ccaaagaagg catccaagcc caagaaggct
13801 gcctccaaag ccccaaccaa gaaacccaaa gccaccccgg tcaagaaggc caagaagaag
13861 ctggctgcca cgcccaagaa agccaaaaaa cccaagactg tcaaagccaa gccggtcaag
13921 gcatccaagc ccaaaaaggc caaaccagtg aaacccaaag caaagtccag tgccaagagg
13981 gccggcaaga agaagtgaca atgaagtctt ttcttgcgga cactccctcc tgtctcctat
14041 tttctgtaaa taattttctc cttttttctc tcttgatgct caccaccacc ttttgccccc
14101 ttctgttctg actttataag agacaggatt tggattcttc agaaattaca gaataattca
14161 tttttcctta accagttgtg caaggacagc aacaaccaat ctaatgatga gaatgtactt
14221 atattttgtt ttgctattaa cctacttacg ggttagggga tttgcggggg gggcttgtgt
14281 gttttgttgg cttgtttgcc atgaaggtag atgtgggtgg ggagaagaca caaggcagtt
14341 tgttctggct agatgagagg gaacccagga attgtgaggt tagcaggaat atctttaggg
14401 tgagtgagtt ttctttgagt tgggcacccg ttgtgagagt ttcagaacct ttggccagca
14461 ggagagaggt ggtagggagc agccagccgg caaaggaagg aggggaaaaa aaaccgccac

14521 cgggctgact tccacctccc agtggtgagc agtgggggcc caaacccagt ttccttctca
14581 tttttgttag tttgcgcttt cggcctccct attttcttag ggaaggggag tggggtccaa
14641 gtgacagctg gatgggagaa gccatagttt ctcccagtca gctaggatgt agccattggg
14701 ggatctttgt ggcttcagca aattctcttg ttaaaccgga gtgaaaactt caggggaagg
14761 gtggggagtc agccaagtgc ctcagtgtgc cctgttgaaa cttaggtttt tccacgcaat
14821 cgatggattg tgtcctagga agacttttct tttcctctgg atttttgttc ctcctgtaca
14881 agaggtgtct ttgcttggtt tggtggggct gcggccactt aaaacctccc gatctctttt
14941 tgagtccttt attataagta gttgtagctg cgggaggggg agggggagtg ggcgggcagt
15001 ggatagtaag acttactgca gtcgatttgg gatttgctaa gtagttttac agagctagat
15061 ctgtgtgcat gtgtgtgttt gtgtatatat acatatctag ggctagtact tagtttcaca
15121 cccgggagct gggagaaaaa acctgtacag ttgtctttct cttattttta ataaaataga
15181 aaaatcgcgc acttgcgcgt ccccccccca cccccttttt taaacaagtg ttacttgtgc
15241 cgggaaaatt ttgctgtctt tgtaatttta aaactttaaa ataaattgga aaagggagaa
15301 actgagcggt gtatttttcc tcactttgaa gactggagaa tgaatgcgga gcggttaggc
15361 gggcaggcag tggggacatc tggggcgttt gcacctgaga atggcggggg agggttcagg
15421 agctggacag aagccaggaa tccttagtcc cggaggctgc ggaacgtgcc ttagccggct
15481 tctaggcttc tgcctttggg gtccagaggg taccccattt ccggccgaga ggtgagtacg
15541 tgggaatgcg gtggggagtc tggtgggctt ggccctgacg cgacttcctc cgcccccttc
15601 cctcccctct gccatccgga cttcggcagt ctcaacttac aagtcaagcc tctcggtccc
15661 tggcggatcc cgccaggccc cgcccacccg tcctaggccc gcctccccgc ctttttaagc
15721 gagcccccct agctccgatt ggctgctgga gcccggcccc cggccacgcg accaggctgc
15781 gtccgcgatg cgcacggctc ccaggcaggc aggcgcgctc gggcgaggta ggagcgatgt
15841 ggcctgggaa cgcctggcgc gccgcactct tctgggtgcc ccgcggccgc cgcgcacagt
15901 cagcgctggc ccagctgcgt ggcattctgg agggggagct ggaaggcatc cgcggagctg
15961 gcacttggaa gagtgagcgg gtcatcacgt cccgtcaggg gccgcacatc cgcgtggacg
16021 gcgtctccgg aggtaacgct ccgttccggg agtcgttcca agacctttcc cgagcttgcg
16081 ctggaatgga ggtcctggag gtgggggtgg gcggctccta cactcttagc tactgttccc
16141 cttcgcatct ctcagggcga ctttatggct tcctaatatt gactcttgcc tggttaaacc
16201 cttttgacgg agggaggcga tttgcccagc aagaaaaccc gcatttcttg agcggctcct
16261 ttgtgaaggt ctctaccaat tgttgctgca cgtgaactcc tgctaacttc tagacgcagg
16321 aatgactaat tccactttac aaacgaagag gctgaaatcc acagatgtga aatggcttgc
16381 ccaaaagccc ccagctagct gcaggcagcg ctggaatatt ggagctggaa cattgcctga
16441 ctggccagag aggacagctt gctgggtaaa ctcatagcaa gcaaggggtc ccagggctgc
16501 cacagaaatc acatgatgag ttggccctgc caggtctccc tcatttcttt ctttcctttt
16561 ttttttctga gatggagtct cgtcttgctc tgtcgcccag gctggagtgc agtggcgaga
16621 tctcggttca ctgcaacctc tgcctcccgg gttcaagcga ttttcatgcc tcagcctccc
16681 aagtagctgg gactacaggc acgtgccagc acacccggct aatttttgta tttttagtgg
16741 agactgggtt tcaccatgtt ggccaggctg gtctcgaact cctgacctca agtgatcctc
16801 ccgcctcagc ctcccaaagt gttgggatta caggcgtgag ccaccactcc cagccctccc
16861 tcatttcact ctaaccagtg gttacacctg acttattcgt gtcaggcctc aggtgagtgg
16921 taatcatcaa caagctccca aagctgcctt ctgcccagga aataggcata gaacccttgg
16981 tgctgttgca ctcatttgta aaacagaggt cccaggtaat cccttctctc cctcagggga
17041 agcttagagc cggtcaaaag atgggaaatt aggagaccca agttctcatc ctgtttttgc
17101 ctttcccagg ctgtgtgaac tttcccctct ggctgtttgc tcctaaggtc agctgtgttc
17161 actctgggct ttagaattcg gggactggta tctcaggcac ctacctttgg gggtgtgatt
17221 ctggcagcaa agtaaactgt ggagttccag gactgcagca ggcgtctgat ccagccctct
17281 tctttaaccc tcaaggaaac caaggggtca gataaggagt cagtggtggt cttcaggcac
17341 aggagaagcc tgtgaggaag ggctgagttc ctccttgggt agaatgtgat tcaaagccag
17401 agtttaggct gggcacagtg gcttgtgcct gaaatcgtag cactttggga ggccaaggca
17461 ggaggattgc ttgaggctag gagttcgaga ccagcacgga caacataatg agaccccatc
17521 tctacaaaaa ataaaaaaat ttaatcgggc atggtggcac gcacctgtag tcccagctac
17581 tcaggaggtt aagtgggagg attgcttgag cctgggaggt tggggctaca gtgaaccatg
17641 attgcaccac tgtactccaa cctgggcgac agagctagac cctgtctcaa aaaaaaaaaa
17701 gtttaaactt cttgctcttc aagatgtgta gcactgacgc tccttctgta gctgttgaat
17761 ttggtgcctc ctcaatggtg actccaggtc tgtttccttt ctggaccatt tccccagggc
17821 ctggcactgt catctttcca ggcctgccct tgccccacct gagctgctgt atccatctcc
17881 tctccttcac ctcaggaatc cttaacttct gtgccaacaa ctacctgggc ctgagcagcc
17941 accctgaggt gatccaggca ggtctgcagg ctctggagga gtttggagct ggcctcagct
18001 ctgtccgctt tatctgtgga acccaggtac acagtgagtg gtggggggtt gtggggactg
18061 accccagctg cctgcttccc acagtcagca aggctggttt agccagctca gctcagccac
18121 agccctgggc ctactgttgg cctcagagct gtgcagcaga aaggctgagt gcaggcctct
18181 gttttgctga aaagctttgg ccaaggaact ttatatatat attttttctt tttgagacag
18241 agtcttgctc tgtcacccag gctggagtgc agtggcacaa tcttggctcc ctgtaacctt

18301 cgcctaccag actcaaggga ttctccatgc ctcagcctcc tgagtagctg cgattacagg
18361 catgctacca cacccagcta attttttttt tttttttttt tttgagatgg agtctcgctc
18421 tgtcgctgag gctggagtgc ggtggcatga tcttggctac tgcaagctgc gcctcccagg
18481 ttcacgccat tctcctgcct caacctcccg agtagctggg actacaggcg cctaccacca
18541 ctcccggcta attttttgta tttttagtag agacggggtt tcactgtgtt agccaggatg
18601 gtctcgatct cctgacctcg tgatccaccc accttggcct cccaaagtgc tgggattaca
18661 ggcgtgagcc accgcactcg gcctaatttt tatatgttta gtagagatgg ggtttcacca
18721 tgttggccag gctggtcttg aactcctggc ctcaagtgat ctcctccacc ttggccttcc
18781 aaagtggaaa ctgccttttt gagcattcat tttcccacct gcaaaacaga gtccctcaca
18841 cctgaccttc tataagaaaa gaggagtagg cggcaggaac tgagactaag ggctctgggg
18901 ctgttctgac gggagcacag ttgccagtgc tgccctttag tgtccctgtt cctgtgggca
18961 agtatcttaa cttttctctg tctcaattac ttcataggga aaatgaagat aatagcactt
19021 aataggatca ttacaaatta aagacttaat acatgtaatg cttagaatag gccgggcgca
19081 gtggctcatg cctgtaatcc cagcactttg ggaggctgat gcaggcggat cacctgaggt
19141 caggagttca agaccagcct ggccaacatg gcgaaaccct ttctctacaa aaatataaaa
19201 attagctggc atgatggtgg gtgcctgtaa tcgcagccac ttgggaggct gaggcaggag
19261 aatcgcttga acctaggagg tggaggttgc agtgagctga gatcacgcca ctgcactcca
19321 gcctgagcaa cagagtgaga ctctgtctca aaaaaaaaaa aaaaaaaaaa aagcatagaa
19381 cagcctggca catagtactg ctcaatgaat gttggctatt atttattttc caaatgttac
19441 aaaataaatt tatataagta tgaaacttaa atagtactta ttatgtgcca ggaattcttt
19501 taagaaaatt acaactattg gccaggcgtg gtggcttatg cctgtaatcc aggcactttg
19561 ggaggccgag gtgggtggat tactgagttc aagaccagcc tggccaacat ggtgaaaccc
19621 tgtctgtgct aaaaatacaa aaattagctg gccgggctcg cttgaaccca tgaggcaggg
19681 gttgcagtga gccacgacgg tgcgaccaca ctccagcctg ggtgacagag ggaaactctg
19741 tctcaaaaaa aaaaaaaaaa gaacattaca agtattaact cattaatcct aagtgatagt
19801 tgctattctt attcccgcta ttgagatgag gacactgaga tccagagagg ttaagtgact
19861 tgcccagagg cacacagcct gttgtgggat ttgaaccctg gctcaagagt ccatgctggc
19921 tgggcatggt agttcacacc tgtaatccca atacttttgg agcccaaagc agaaggatca
19981 cttgatgcct ggagtttgag accagcctgg gcaacatagc aagaccctct ctctaaaaaa
20041 aaaaaaatgt ttttaattag ctgggtgtgg tggtgcgcac ctgtagtccc agctactagg
20101 gaggggaggc tgagttggga ggatcacttg agcccaggag ttcaaggcta cagtaagcta
20161 tggtctcact actgcactcc agcctgagtg acagagaatc cctgtctcat ttgccaaaaa
20221 aaagaaagag tctgtgctcc tgactacttg tgattatcat taactgccct ctcagaaagg
20281 tgagagtgaa atgaatgtca gaggcagaac tcctgactcg catccagtca cttcccagca
20341 aagccctcac ccagacccaa cagttttcaa atttacaaat tagaggccca ggtgaaccca
20401 gaattacctc tgtgatcttt ttcttcacca ttaggcgacc aaaaaagaca ctcagtatgg
20461 tgttaggaag acacatcttt cttctccacg agtgcccgta attccaccat tagactgcca
20521 ctgtaaaacc aaagtctgcc cagggaggga acctaacagg tccccagttc ctcagtggga
20581 actgtctgtc agattcctgg cagctctggg atagcccatg tgtggcagaa agtaaggaga
20641 ctgaggctgc aggctttctg ggttgggtct ctgtggtcct ccaagcctct gtcccctcga
20701 acttgtccca catgacccca caggcatcaa cacgtgtctc actcattttc ttcagagcat
20761 ccacaagaat ctagaagcaa aaatagcccg cttccaccag cgggaggatg ccatcctcta
20821 tcccagctgt tatgacgcca acgccggcct ctttgaggtg tgtggaagct gtcctgcggg
20881 tgagggttgg tggggcctgt taggctgagg aaggcttggg tgtgggcagt cgggagaacc
20941 tagcaggatt cagtgccatg cctagaggct ccagagcccg cttctggagg tcaaggttgg
21001 gccaggtagg tgctccagga ggaatcctgg ggcttgcagg gagcctgagt tccagagcct
21061 ttgggggata agctacatca gctggaaggc tgggggaggc gggggcctgg tactcaccaa
21121 atatattctc acctcctctt actagagcag ggcgctggtc cagcccggca ctgagacacg
21181 ggggccccag agagaagcac agaacacctt gcctctctac ccagaggagc gccctggctg
21241 gcagtacagt tttttgattt gctggagtcc cggtcaaggg agaagcccag ggtggttaaa
21301 tgatggctct acgctcactg cctccctcca ggccctgctg accccagagg acgcagtcct
21361 gtcggacgag ctgaaccatg cctccatcat cgacggcatc cggctgtgca aggcccacaa
21421 gtaccgctat cgccacctgg acatggccga cctagaagcc aagctgcagg aggcccaggt
21481 ggggcgacgc ctgggtccac cctcagcctc cgcctgggga aggaagtgag gcttagagag
21541 gccaactcct cactcacaga gatagcctga aagtttgaaa cagcagagat ttgtccaaga
21601 tgtgaaccca gatccctcac acccagagca ttattctttt ttttttttcc ctgagacaga
21661 gtttcactct gtcgccaagg ctggagtata gtggtgtgat ctcggctcac tgcaacctcc
21721 acctcctggg ttcaagtgat tctcctgcct cagcctccca agtagctggg attacaggca
21781 tccgccaccc cacctggcta atttttgtat ttttagtaaa gacggggttt catcatgttg
21841 tccaggctgg tctcgaactc ctgacctcag ttgatttcac ccacctaggc ctcccaaagt
21901 gctgggatta tagacgtgag ccactgtgcc tggcaccaga gcattattcc attgtgccat
21961 ggtgccttga ggtttttccc tgtgtatgtg tgacagtctc actctgttgc ccaggctgta
22021 ttgcaatggt gcagtcatag ctcactgcag cctcaatctt ctggactcga gcgatcctcc

22081 cacctcagtc tcctgagtag ctgggactac cggtgcccac cacacccagt tttttattta
22141 tttgtttttt agatggaatt tcactctttt tgcccaggct ggagtgcaat ggcgtgatct
22201 cagctcactg aaacctctgc ctcccaggtt caattgattc tcctgcctca gcctctggag
22261 tagctgggat tacaggcaca caccaccgtg ctcagctaat tttctatttt tagtagagat
22321 ggggtttagc catgttggcc aggctggttt cgaactcctg acctcaggtg atctacctgt
22381 ctcagcctcc caaagtgttg ggattacagg catgagccac cgcacccagc ctgtttgttt
22441 gtttttgaga cagggtctca ctataatgct caggctagtc ttgaactcct gggctcccaa
22501 agttacaggc atgagccacc atacccagcc agtttttaaa atttttagta gagacagggt
22561 ctcactatgt tgcccaggtg ggtctcgaac ccgtaagctc ccaaagtcct gggattacag
22621 gtgtgagcca ctgtgcccgg cctgccttga gtattagcaa gcaccttctg tgtggcaggc
22681 ccttgctagg cataaggata gaatgtacat gttggtgaga cctggtccca gcctagcctg
22741 ccctctggga ccttacacag gagactgaga tgtgagccca ttctcactgt ccagagctca
22801 gggtggagat gggtgacgct gagaggcaca gatgagggag aggatgttcc agcccccaag
22861 atggtggtac ctctgtgccc acctctgtgc tgggcactgg gcaggatgag ctctcagagc
22921 tccaagcagc acaagagacg ggtggtttgg cccaacttga caccacccac catctgctca
22981 tgtctttcct gcagaagcat cggctgcgcc tggtggccac tgatggggcc ttttccatgg
23041 atggcgacat cgcacccctg caggagatct gctgcctcgc ctctagatat ggtgccctgg
23101 tcttcatgga tgaatgccat gccactggct tcctggggcc cacaggacgg tgggaccatg
23161 tggcacctga ggcctggggt ggggcttctg agggtggctg ggaaacgagg aggccagtcc
23221 ccagcatgat ccataatccc tgggctctca ggaggccagt gacagcagcg gtggcttccc
23281 ttgcaggggc acagatgagc tgctgggtgt gatggaccag gtcaccatca tcaactccac
23341 cctggggaag gccctgggtg gagcatcagg tacctgcaag gttgtgtccc tggggtcccc
23401 ttgtcctttt gaagggccct ggaggggctc agggaagtgg ggagggcagc atggactgta
23461 ctttcaggag ggggttgggt aggctctggc ccctgaccaa cccctccccc cacctcttcc
23521 cttcttctca gggggctaca cgacagggcc tgggcccctg gtgtccctgc tgcggcagcg
23581 cgcccggcca tacctcttct ccaacagtct gccacctgct gtcgttggct gcgcctccaa
23641 ggccctagat ctgctgatgg ggagtaacac cattgtccag tctatggctg ccaagaccca
23701 gaggtgcgac tcccagcagg gcaggctcgg gggcggagag acgtggtgag agccagcctc
23761 atgtgtctgc ccaggcccac agacagctct gtgcccaccg ggtctgcttc tgcctgttcc
23821 cctcccttct ctctgtccct gcctctcccc tctagcttct gtgtctcctt cttatccctc
23881 ctgtctttcc acctcccttg ttgttctccc tgccccctgc ctggctcccc tctgcctctt
23941 agagcttgta actgtctttg ttgatccttc ttgcagactt gggcatagac ctcgggcctg
24001 gtccctgcaa ggagcgggtg tgaatgctcc acggcccctt agctacctgt gacaccttgt
24061 gcccacaggt tccgtagtaa gatggaagct gctggcttca ctatctcggg agccagtcac
24121 cccatctgcc ctgtgatgct gggtgatgcc cggctggcct ctcgcatggc ggatgacatg
24181 ctgaagagag gtaagggtgc tgagacaagg gaactggtgg tgggtcctga gagaagagaa
24241 agggaaaccc ctagactgtg acccagcccc gtacccagcc acgctgcggc ctcttaggcc
24301 agctgtctgt ctaagcttga acaccccaag ccatgaggca gtcccgaatc tgagacctgg
24361 ggcagttccc ttgccctctg tgttctgccc ccaagcctgt tggttctggc acgcccttgc
24421 tttctaccct cccaggcatc tttgtcatcg ggttcagcta ccccgtggtc cccaagggca
24481 aggcccggat ccgggtacag atctcagcag tgcatagcga ggaagacatt gaccgctgcg
24541 tggaggcctt cgtggaagtg ggcgactgcc acggggcact gccctgagct ctgggtaagg
24601 acgagaagag ccaaggtccg cctactgcca cagggtcaaa ggaggttttc gatcagccca
24661 gaccagaggc tctgagccct gaaccaaagt cccagagctg ggctgggacg tgacctgtgc
24721 tgagggctgt gagaatgtga aacaacagtg tgaaaattgg ctgtgacact ctggtctgct
24781 ttattgtctc tgggcaggcc cagtcctgtg gccggttgaa gaatcaggca ggagccaggg
24841 ctctgagggg aggcgcctga ggactgcaga tctccactga cctctttccc tagattaaga
24901 tgggacccag tggccgggca cggtggctca ggcctgtaat cccagcactt tgggaggcca
24961 aggtaggcgg atcacctgag gtcgggagtt tgagaccagc ctgaccaaca tggagaaacc
25021 ccgtctctac taaaaatacg aaattagcca ggcgtggtgg cgtatgcctg taatcccagc
25081 tgctcaggag gctgaggcag gagaatcgct tgaacccggg aggtggaggt tgcggtgagc
25141 cgagatcatg ccattgcact ccagcctgag caacaagagc agaagtctgt ctcaaaaaaa
25201 aaaaaaaaaa aaagctggca cccaggcctt tgggagatag gcccagtcca ccgtttgtct
25261 cgggtagttt ctgtgctgct gtggagggct gctgagaact agccccacag gccttttacc
25321 ctgccactgc ccgctactct gcagcccctg agtctagtct tagagtctag cttcttcctt
25381 ctccccactg gaagatgtcc tgtagctgcc cagataggag gaggctgggt gtccatgttt
25441 ggaaggagag gcagaggatt caggcccaaa agaaaagaat gaggatcacc cctggggaag
25501 ctccagacag tctggaaccc tgtgttcgcg cccactctct ccctgcctta ctctggcttt
25561 cggcaagctc cagtccctgt gtgtgcctca gtcctcctgc cactatgcct tgaggtctga
25621 ggtccttcca tctcgctcat tccaaggcct acgatgatca gccaccaggg ggtgctgcga
25681 gcacgggccg cggccgccgg ctcgccccgc ccctccagac tgggggcctt gggccgcggc
25741 tgttcaggct gcccagcagt caaggccagg agaggcgatt tgctgctgcc aaggccccat
25801 cctcccttgt gccccacaac ggctcgccgc tttcctgttg gaggggcctg cgggaggagg

25861 gcggtctctc cctggcgacc ttgtggaccc cttgtggggt ggggagtggg gtccccgcag
25921 ctccaccctc agcgtggtgt gaccttggga agctcttagt ttgcttggga cagaggacga
25981 aaaggccctt tatcattaac gacatgaaac tagccctacg tctctctctg acgtcctgca
26041 cctccagccc acccatttgc ctgttatctt gggatgaaca atgtaatatg agctgggctc
26101 tggcccagcc ctgcaggatc tgaaagctgg gattcaggga atcccagcct gcgatccgga
26161 ggaccttgct gctggggtcc aggagctggg ggaggggggg tgtggggggt cggtgtgagg
26221 gtggaaggca gcctggctgg ggtagctgtg gccacattag cggcaggaac atttgcagcc
26281 caccaccacc ggctagggca cgtggcacca ggcgcgctgc tgcaggggat gtgcacaagg
26341 gctcaaccag taatgtcgag ccagcagctg gatgagcatg gtctgctcta gccagcagct
26401 catgttaagc gccacgggca gctgcggggt gacgtgtggc agtcggtgtg ctgcacccgg
26461 cggggccaga acagagggcc caggtccacc cagaccgacg tgaggcggca cgaggcgaga
26521 tccagacagc ggcgcagaaa gtgcgtcgcc gcccctgtgg ctcccaggag ccgccgctgg
26581 cgttgccctg ctgcgctggc agcgccgcga ccccctgggc tagccgatcg cggtagggct
26641 ccacgccgtt ggccaacccg cggctcagcg tgtcctgctg caccagcccg ggtccgccgc
26701 cgcgcttctt ccgcagtcat ccagaaggtg tctgtaggag gcgcagcagc cgctctgggt
26761 gcacgtgttt cggcttgggc gcctaccggt agtcgccggc cgcccacgac aagctgtagg
26821 gccacgcggg gcgggagggc cggccgtgga ccagctgcaa gtctgcgttc tgcagaatca
26881 gaggctctga ggaccgcccc aagggcgcaa tgtccttgtt ctctgcgtcg cgcacggggt
26941 tcagggccgc gggctaccag aagcggcagt aggagtggat ggaggtggca ccgggcgcac
27001 ccaaggtcta ggctcacggc cgcgctcttt atggccgcct gttccttcct ccttccctcc
27061 acctccgctc tcatatcagc ttagccttgc cgctgctggg agctcatcgc cctaggccaa
27121 gaaggacctt gtgggcaggg ggctcccaag ggccaggagt gcgaagtcat gaatctcaga
27181 tgctttccag cccgttccag ggttcaggca tgcacattcg tccaggcttc tgggagtcta
27241 agggtgcgtt actttcacgc agtggttgca ggtctgtcaa agtttctaaa atcccaccaa
27301 gctgagtctc gccattcagg gatcataaga gtctacaatt cctttgcgca attcaattct
27361 gaattcaatt cagaattaat tcaattctgg ccgggcgcgg tggctcatgc ctgtaatccc
27421 agcactttgg gaggccgagg cgggcggatc acgaggtcag gagatcgaga ccatcctggc
27481 taaaacggtg aaaccccgtc tctactaaaa atacaaaaac aaaacattag ccgggtgtgg
27541 tgggcgcctg tagtcccaac tactcgggag gctgaggtgg gagaatggcg tgaacctggg
27601 aggcggagct tgcacttgca gtgagctgag atcgcaccac tgtactccag cctgggcaac
27661 agagcgagac tgtctcaaaa atatatatat atattaattc aattctgaag cttctgaatt
27721 caggtttggc aagtctagat tagattccta atagtttcaa ttccgtgaga ctctgcttcc
27781 aaaaattgag gaatttctgc atctaaagaa ggtgacatca ggctctatag aggcacctct
27841 gagccctcgt gaaaaccccg gggcctttga ggagctggtt cttttctttt cttttttttt
27901 ttttttgagt cttgctctgt tgcccaggct ggagtgcagt ggcacagtct ccgctcactg
27961 caacctccgc ctcccgggct caagccattc tcctgcctcg gtctcccaag tagctgggat
28021 tacaggtgtg caccaccata cctggctaat ttttagtaga gatgggtttc accatgttgg
28081 ccaggctggt cttgaactcc tgacctcaag tgatccaact gccttggcct cccaaagtgc
28141 taggattaca gacatgagcc accacgcctg gccttaagga gctagttctg agcatctacc
28201 ccagctcctc cggcctggga tgcgagtcct ggctccatca gcgcccctca cactgttttc
28261 tctcccctta cgcacttccc cttgatgctc cccaacccca gccccagctt ctaaaaacac
28321 tacactcagc ccctccacct ctcccctccc ttccttaact cactgcagac tgccttttgc
28381 cctcaaaaca cctgtccagt ttaccagcag cctcctcatt gctgaatcca gtgtaccttg
28441 ctccccaggt gctagcctcc ctaaccctct ggaggcattt ggccatgtgg cccctcctcc
28501 acacagttca ggacagaact ccatgaccaa atcacacagc cgttcaccaa gacctattct
28561 cctctttggc tcttgcctag acgagaggct cctaacaggt cactcatcaa atctctccca
28621 ctctgtccat ccttttcacc ccgggcaata atagactttt tttttttttt tttttgaga
28681 cagagtcgct ctgttgccca gtctggagtg cgagcgatct cggctcactg caaccaccac
28741 ctcccaggtt caagcgattc tcgtgcctca gcctcctgag tagctgggat tataggtgtg
28801 agccaccatg cccagctaat ttttgtattt ttagtagagg cagggttttg ccatgttggc
28861 cagtctggtc tcgaactctt gacctcagat gatccgccca cctcagcctc ccaaagtgct
28921 aggattacag gtgtgagccg ctgcgcctgg cccataacaa actttttaca aaagataatg
28981 tgggggaaa aaaagaaaga aagaaagaaa atgtgcgggt catgtctctg ctgtagaatc
29041 ttagtagttc cccgttgcta tcaggtaaag ccagactttt tggacaggta cctaaaccat
29101 ctgggctcca gctggactaa ggctctggaa agagcttccg gagaaggatc tcaggatttg
29161 cagagcctga gggaggagtc tctggagtct gccgaaagga gactcaagtc ctggtttccc
29221 actccaggaa accccagtca agactaggaa tccttttttt tttttttttt tttttttttt
29281 ttgagacgga atctcgctct gtcgcccagg ctggagtgca gtggcacgat cttggctcac
29341 tgcaacctcc aacccctggc ttcaagcaat tctctgcctc agcctcccga gcagctggga
29401 ttacaggtgc ccgccaccac acccagctaa tttttgtatt tttagtagac atggggtttc
29461 atcatcttgg ccaggctggt cttgaactcc tgacctcatg atccacctgt cttggcctcc
29521 caaagtgctg ggattacagg catgagccac cgcgcccagc ctcttctttt tttttttttt
29581 tttttttttg agacaatgtc tcactccatc acctgggctg gagtgcagtg gcgtcatctc

29641 ggctcattgc aacctccacc tcccaggttc aagcgattct cctgcctcag cctccagagt
29701 agctgggatt acaggcgccc gccacgacgc tcagttaatt tttgtatttt tagtagagac
29761 aaggtttcgc cacattggcc aggctggtct cgaattcctg gcctcaagtg atctgcctgc
29821 ctcagcctcc caaagtgctg ggattacaga cgtgagccac catgcccggc ctaggaacct
29881 tttctaaaca tggattggag caagaaggtg cagggatagg ggatggagtc acatcaggct
29941 gggccacagg aggtgggagt gccagagact ggctgggccc tgggcacaga ggggttaact
30001 acaaagcaaa tcacagagag cgagatcaat gggttactgg ctgcagagag aggaaaaggc
30061 agcagcaacg cagagaggca gagaagagcc aggcagaaga gcctgccacc tgggggaaat
30121 cagggactga ggaggaagtg gccagtggct actccagagg gagtgcccaa ctccggctca
30181 gcagaggggg cagaggagag gaggggcctt aaaggcaaag ggggagaagt agaaggcaga
30241 ccagtacctg cagggaggca ccaaggggca gccgctgaat caggaggccc acccagcccg
30301 gccattccac ccaggatcaa gcccactgcc caaggcggag gtgggtgttg cccatcctgg
30361 gctttgggga cctactgcat gctgggtgct gggatggggg cagacattgc ctttcttccc
30421 tactccctgc ctgacccccc tttcccctac tttgtcccac caccccagtg gtctcaagcc
30481 tgcaaggaga tccacaggca gtaggagaca tgggctgtag agtgggatgg gggtgagcat
30541 gggtgcggtg tgcacacaca caagttgtca gaggcaccac ctgttacaag ctctcagatg
30601 agcactctag cttaagaggc aggataccag ggtggcaaca gccccagagc tgccatttcg
30661 agtgtgatat tatcctccca tgtcccctct ctgggcctca atttccctat gtgcagagag
30721 gaagcagtga cctacacggt cttcccagtt ttgccattgt gatgatctca ctatactgga
30781 ccctccaatg actcagggat gttacccgtg ttttgctaag gctacagctt gtagccagag
30841 aaagccctaa acccatgtcc cagggtctcc cctgagcttc agatccccat tgagaaggaa
30901 aacataccag tttctgggtt tggcccctcc ccccatcgcc atatcttcat cctacttcgc
30961 ttgtgccttc cccagcctcg tgggttgaaa gtgtccctca aatacctagg tagtcctcca
31021 ggcaggactc caggggtgtt ccttaggaaa ctgaggaact ctcacccctt gaacaccacg
31081 agcttgctca cacagtgaca ctcagcgatg actttggctc tgctctcccc tcctccatct
31141 cccacgagct tccagcccag aacacctggc cagacccagg tcgggggagt tagatcccgg
31201 ggtcaagcaa ccagaactgg gggctcttgc ctgaggattc cagcttctct tcccaggtgc
31261 ccgtctgatg gggagatggc tgatgcccag aacatttcac tggacagccc agggagtgtg
31321 ggggccgtgg cagtgcctgt ggtctttgcc ctaatcttcc tgctgggcac agtgggcaat
31381 gggctggtgc tggcagtgct cctgcagcct ggcccgagtg cctggcagga gcctggcagc
31441 accacggacc tgttcatcct caacctggcg gtggctgacc tctgcttcat cctgtgctgc
31501 gtgcccttcc aggccaccat ctacacgctg gatgcctggc tctttggggc cctcgtctgc
31561 aaggccgtgc acctgctcat ctacctcacc atgtacgcca gcagctttac gctggctgct
31621 gtctccgtgg acaggtgcgc tgtgcctggg gcctggctgg gcagggctgt gggggcgggg
31681 gttgggggag gagtcctgaa cagatcctca ctggccttag gaaggagaga gtgggggacc
31741 agaaagggag gtgggtggga ggaaacaaaa gctccctgac ccctcgcaag cagcctctgg
31801 gcacctgcag ggcgtgcttg aggggactgt cctgcccttc ccctcctcca ctgtgaactt
31861 ccagaggacg cctctgagtc tcaagtggca gcacagggtc tggcacatag taagtgctct
31921 gtaagcgcga aatgaatcgc aaaagaagct cacgaatgcg ttcatcagtt tttttgtttt
31981 gttttgtttt gttgtttttt ttttttggat cttggctcac tgcaacctct gcctcctggg
32041 ttccagcgat tctcctgcca cagcctcctg agtagctggg attacaggcc accacacctg
32101 gctaattttt tgtattttta gtagaaacgg ggttttgcca tgttggacag gctggtctcg
32161 aacccctgac ctcaagtgat ccgcccgcct cggcctccca agtgctggga ttacaggcgt
32221 gagccaccgc gcccagccca gctattttct aactgcccac acctggccaa gctgtgcaca
32281 catctgcttc cacagcttga aacttggggt caaatccagg ctcactccag ctgatgaccc
32341 tgggcaagtc acttctctct ggacctcatc tgacgcatcc ataaaataat cctagaaata
32401 acaagtcacc gggatcgggc ccttgctagg tgcaagggcc taagcacctt gcgcgttcac
32461 acccttaatc cccgccacgt cccccacggt tcacaggagg cgcactgggc cgcagggccc
32521 gggcgcggga cgtggcgcgg gcccctgcgg gagggcacct gcccgccccg ctgaccaggc
32581 gccctccgca ggtacctggc cgtgcggcac ccgctgcgct cgcgcgccct gcgcacgccg
32641 cgtaacgccc gcgccgcagt ggggctggtg tggctgctgg cggcgctctt ctcggcgccc
32701 tacctcagct actacggcac cgtgcgctac ggcgcgctgg agctctgcgt gcccgcctgg
32761 gaggacgcgc gccgccgcgc cctggacgtg gccaccttcg ctgccggcta cctgctgccc
32821 gtggctgtgg tgagcctggc ctacgggcgc acgctgcgct tcctgtgggc cgccgtgggt
32881 cccgcgggcg cggcggcggc cgaggcgcgg cggagggcga cgggccgcgc ggggcgcgcc
32941 atgctggcgg tggccgcgct ctacgcgctc tgctggggtc cgcaccacgc gctcatcctg
33001 tgcttctggt acggccgctt cgccttcagc ccggccacct acgcctgccg cctggcctca
33061 cactgcctgg cctacgccaa ctcctgcctc aacccgctcg tctacgcgct cgcctcgcgc
33121 cacttccgcg cgcgcttccg ccgcctgtgg ccgtgcggcc gccgacgccg ccaccgtgcc
33181 cgccgcgcct tgcgtcgcgt ccgccccgcg tcctcggccc cacccggctg ccccggagac
33241 gcccggccta gcgggaggct gctggctggt ggcggccagg gcccggagcc cagggaggga
33301 cccgtccacg gcggagaggc tgcccgagga ccggaataaa ccctgccgcc tggactccgc
33361 ctgtgtccgt ctgtctcact cccgttctcc gaaggcggga cgccaccggg ccagggatgg

33421 ggcaatgcca cgagctctct gaggggcgtt gagtggagcg acttgtcccc gctccacagc
33481 ggagcaccag cccagtggac acccctgggt gtgaatatga acagcacagc ccgcccccag
33541 cagggcagcc cccagagcct gcccagagcc agcctggcag tccctctgcc cattcttcca
33601 actgggtgac acctaggccc tgcttcctcc accacctctt aactatctcc ttcttggttc
33661 caagctcagt cctaggtaat ttaacatcaa cagctaataa atggctaata tttaagtggg
33721 accaggcgct tggctcatgc ctgtaatctc agcgttttgg gaggtcgagg cgggaggatc
33781 gcttgaggca gcagttcaag gctgcagtga gccatgatca cgctactgca ttccagccta
33841 gacagcaaag caagaccttg tctctaaaaa catttattaa gcaggtacca tatgcctcat
33901 gctacattct ttgtcaaaac agctttgcaa atagatatga ttaacctttc cttttttttt
33961 tttttttttt ttgtctttta gagacagagt tttgtctccc aggttggact acagtggcgc
34021 catctcggct cactgcaacc tccgtccccc gggttcaagc aattctcctg cctcagcctc
34081 cagagtagct gggattacag gcgcccacca ccacatccga ctaatttttt tttttaatag
34141 agatggggtt tcaccatttt ggacaggctg gtcttgaact cctgaccgca gatgatccgt
34201 ccgcctcggc ctcccaaagt gctgggataa caggcgtgaa ccactgtgcc cggctgttta
34261 gccccatttt acagatgtag aaatcaagga tcatagagat ttagtgattt gccacaccgc
34321 tggtgtttgg cagaggttgg atacaaaccc atggctgtgt aactttttct tttttttct
34381 attattatta ttattattat ttttgagaga gagagagaga ttgttatcca ggctgcagtg
34441 cagtggcaca atcttggctc aagacaacct ccgcctccca ggttcaagcg attctcctgc
34501 ctcagcctcc cgactagctg caactatagg cgtatgccac cacacccggc taatttttgt
34561 atttttttat tatttttatt ttttggtaga gatggggttt caccgtgttg gccaggctgg
34621 tcttgaactc ctgacctcaa gcgatccgcc ggcattggcc tcccaaagtg ctgggattac
34681 aggcgtgagc caccgcgccc ggcccagagg taacatttga catgtatttt cttggacaaa
34741 ggtggggagg ggcatgccag gtagagcaac agcctgtgca aggcagggga cgtgcagggc
34801 agtaagtggc tcctgcagct gaggcaggga gagattgggg caggggaggc ctggagggca
34861 ggagcttcat ccttcaggca gatggtgatg ccactgaagg ttttcaggag aggaggggc
34921 ctatcagatg tgcagttcag aaagctccca gggctgctgt gtacaagctg gaatggaggc
34981 ctagacttgg cagaggactc cgatcaggtg ggttctcctg ggcccttctt ggaagtgagg
35041 agactggtaa agagtctgct ccgtgagctc tgttgaggaa agccaacgag gcctggccta
35101 ggactgggaa gatatttttg ccgtggactt ggcaggaaga tggatggcct gctgccaggc
35161 ttgggcttgg ttcccgaggc tccgaggttt gcttcagggg gctaatgaaa cacttggaag
35221 agcagatggt cgtaataata ataactcagg agagaattgt gtgtccagta gagcccaagg
35281 caggcaagag ggtcggagcg gaggctgaat tgagtcctgg ccactgctcc ccagggcctg
35341 agagaaccct cctgatctga gccctctgtc aagtgtaggg ctgagcctgg ggcagccctg
35401 gggacctgga aaggcacttt tcttagttgg aaggaggttt ggattctaca accttcttac
35461 cttcctgttt gtgcaaacct cttaagaaga ggcctggccg gatcacgagg tcaggagatc
35521 gagaccatcc tggctaacac agtgaaacac catatctact aaaaatacaa aaaattagct
35581 gggcgtggtg gcaggtgcct gtagtcccag ctactcggga ggctgagaca ggaaaatggc
35641 gtgaacccgg gagacagagc ttccagtgag ccgagatcac gccactgcac tccagcctgg
35701 gggacagagc gagactccgt ctcaaaaaaa aaaaaaaatt actctttagg gggcctggcg
35761 cagtagctca cgcctgtaat cctagcactt tgggaggctg agacgggtgg atactaggtc
35821 aggagttcaa gaccagcttg gccaagatgc tgaaacccca tctctactaa aaatacaaaa
35881 aaaaaattgg tcggccttgg tggtacacac ctgtaatccc agctactcgg gaggctgagg
35941 caggagaatc gcttgaaccc aggcagcaga ggttgcagtg agccaagatc gcgccactgc
36001 actccagcct gggtgacaga gcaagactct gtctcaaaaa agaaaaaaaa gatttactct
36061 ttagggagct ctttctggct gcccaggcag gctttcatag ggaggaagta cagagtgtgc
36121 tgactggagc agagaggtca gtcaggaggc tcgtgtgaga atccagagag aaagacatag
36181 aggtattgga agtggtgggg agacatggac agatgggtca gccacttagg aggtacaatt
36241 gctaggactt ggtgactgtt ggagggtaag gaggagagac gtgtcccaga tgacacccca
36301 gacttctggc ttgggctatt tgggggtggc agtggggata actgaggggg acagttttgg
36361 tggtcatggt gggggtgccc gcagaatagc ggtgtactct gagtacgtgc cacgtttcac
36421 aaccactgtt tgcaggtggg caagggcagg gagcttgtcc tttcattccc tgctgtcctc
36481 ccacctagaa cactgcctag cacatagcaa gcactaagtc aataagtcaa tatttatttt
36541 tatttttatt tttatttttt gagatggagt ctcactcttt tgcccgggct ggggtgaagt
36601 ggctgaatct tggctcactg caacctccac ctcctgggtt caagtgattc tccggcctca
36661 gcctcccaag tagctgggat tacaggtggg cgccaccacg tctggctaat tttgtattt
36721 ttggtagaga tggggtttcg tcgtgttggc caggctggtc ttgaactcct gacctcaggt
36781 gatccacctg ccttggcctc ccaaagtgtt aggattacac gtgtgagcca ctgcgcctag
36841 cccaatattt ctaacatgaa agaaacacga agtataaggc aaaggatctt aacccgcaag
36901 gtgaaatcac caaatgtttc ttcttcctgc catcttctaa gccttcctcc gcacctcccc
36961 gactcttcag taattcgcag agcagccagc aaccagcaaa actcacccct gtgagtacaa
37021 cctcagctga gctctctttc ctgccctggc ccatggccag ctcagggacc actgagctct
37081 ctatcctgcc cagtccagcc agctcaggga ccactgagct ctctatcctg cccagtccag
37141 ccagctcagg gaccacttgg gtgttttgtg actcactcca gtgtctgagg ccgacttcat

37201 ctggctggct cttcagacac aattggggtt acaggaaata tcctggctgg aaatggggtg
37261 aggcagattg gctgtctaca tggctgtctg gcaagtgttt gatgactgac ctataagcca
37321 ccttcagtca agcctttggt tcactgtcta catggccagg gagggagtca acaatagaca
37381 ctccacctcc gcaacggcca gttctcctgg atccatggct ccagggatgg aggatgctgc
37441 aggagacaca ggccttgctt cccagctaag gactgtgggt catctcaggg cgatttcaca
37501 gtcccagcat gccccagctc tcagctctgc aaataccaag cagcctgcct aggggacagt
37561 gggctcagga gtgcccttgg caggtcctca cctatctgct cccacagctc tgtagcaagg
37621 gttctaacct tttttgagtg tgaagcctgc tgagaatgag aatgagagct gtggacctgt
37681 ttcccagaaa cgcatgtgtg tactctccac acaaaacctt gcaccgtttc ggggctcac
37741 acctccctaa gggctcgcta attgaacccc ttgggcctga ggttgagaaa ccttgcctca
37801 attcttcccc aggtgatcag gccagggtga gagaggacag acctggcaag caggacccat
37861 gagaaagccc ccttcctgga ctttgaggcc cgctccctcc tgcccctgcc tcccctctgc
37921 cctggactcc tctctgctct gccccattcc cagggccatg gccatggggg ggctgtgttt
37981 ggatacaatg gcaaccctgg gcccagaaag tgggcaggct cacctggagg ggatcagagt
38041 aacatggcag gaagcgagtg aaaagctacc ccagaaccgc acctctgctc tccatgtgac
38101 tggggtgcca tcctccctta cctacctcag gcagtggcct gagttccacg ggcgcgcccc
38161 acagctccct ctgccgcctc tcttcaggct tggggccact gcactttccc ttccccagcc
38221 ctgccaacct ggtggggcac tgggcttccc tctcacaggg tcctggggac aggcctaccc
38281 tacatctcat acccacagac agaccctttt ttcctccaag gcctggccta ggttctatga
38341 gttcaatgca gatctgagcc ataccaagct gggtttgggg aacacactct cctcctctga
38401 aaggtagaca gggattccaa cttggtgaag ggagagtagg ggcagagcca ggcgggacac
38461 ggactgatca cctggagaaa gtcaccatca gcagccttgg ggcaggggcg ggcccagcag
38521 tgccatcccc tgggttttcc ctgagcaggc ctccgctagg agcgcacgct tccttctgcc
38581 tgtccatggc ccacagcaaa ggactggtgc tcctgcagcg gggagcgggg ctgctgacag
38641 gactgaacct caaggtgagt ttttggggct tcactacaat actagtttct ggttgctgct
38701 gcaataaatt accacacact tagtgactta aaacaacaca aatttattcc cttagagttc
38761 taaaatcagc cagaattata aaattagcct cactgagcca gtccaggtgg gagcagggct
38821 ggggccttcc agggctctgt gggaggatgc atttcctggc tctgaggtgg cctggactct
38881 gtggcttgag ctgccctcct caactccagt ttcctgctcc atcgctacac ctccaacctc
38941 ctctcccgtc acctgctctt ctgttataca gacccgggtg aggacatcaa gccctcctga
39001 acaagctggg ataatcttcc tggcgaaaga tccttaactt cattacatct gcaaagcccc
39061 tttggccata caaggtaatt ttgacaggtt cctgggatta ggacatgggc atcttgggag
39121 gccactactg gcctaccaca actgggcagc aaaactatta caccctccgg tataatagtt
39181 ttggtgtttc aatgactggg aggaaaaggg ttggaatttt ttgctttggg gtccctctta
39241 accttgtatt tttaaggtct gggactcacc aaccctcccc ttccaaccag agaaactcac
39301 tgcagtatct ccttgaaagt ctggtgacgt gtctgtctaa gtgctggtga gaggcacagg
39361 accaactgag ggcctcacgc agtacccctg gccacagggt gcccgcctaa gcccacacct
39421 gctgactgca acaagcccat ggccaggacg gaacacagag aagggtctga ggcctggagc
39481 gcgccatgaa ggccatgggg caagtgaggt ctgctgagat agcgcaggtc tgcggagctg
39541 aagtgcagct gcagaggctg gtctggtgct gcgagaactg gaagaagctg ggagctgtgg
39601 tccctgtcca cagagatgct ggaaactgtg gctgggagtg gctctgaggc cgtggagaga
39661 gagcatctct gccccacggc atctgcgggt gagggcaagg tcctcaccag acaagtgcag
39721 ctccaagtcc cagatgttgg gctttttctt ggtactgaga cagcagtggg gcagggtggg
39781 gcagtggcag gaaggcaggg agcctctctt gctacctctt ctccatgctc tgcatctgca
39841 gactgaggga ggtgaagctg gccaggaggt cagtcacttc atccacctgc aggacggaac
39901 agaggctctg ttatggaca cggggtgtgc caggctccct gggtctcttc cacaatccca
39961 caggcacaga acacaggcac ggtctctcct cccagggcgt gtggtggctt ctgactacgc
40021 agcagttgac caagggtcaa tctttgacac caggtggcct agcgctgtcc tagagggcag
40081 ggccatgaac acacactagc cactggcaac ttcaaaggaa gtgaacggag gctttgaagc
40141 ctgcaacccc acaatcatag gctctttccc ccactttttt tgtttgagac agggccttgc
40201 tctggttttt ttttttgttg tttttataat ttttgccttt ttccacatgt tctgagagga
40261 ccttgctttg tcactcaggc tggagttcag tggcaatcac agctcactgc agcctcgaac
40321 tcctagcctc aagtgatcct cccacttcag cctcccatag cgttgggatt acaggtgtga
40381 gcccctgcgg ctggccccag cccacagcct ctttctgata cgagtgtctg gtggcatcgg
40441 agcagggtgg actggccctg aggctcctgc caggccctgg gtctgggcct gtggctgcag
40501 ctcacctgct ctttggtact ggtcatctgc aggcgggtgc agaggtcatc caggcgttct
40561 cgctgctcat gttgccctag gcgctccaga tactccctca gcatatggat gatctggaac
40621 aagagggtga gctgagctgc ctgggttcct agggaggcag acaggctgat gctgcttcca
40681 aaaagcacag tggagcaggt gccaactggg gtcagtgtga aacaacccag gagccctggg
40741 accagaggca ggtgtgtctg tggttagggc agcatctgtc cagtgctgcc ttggtgccgt
40801 ggtctccaca cagaaggggt actcacctgc ttcacctcca gacgcacagc ctctaggcac
40861 tgggcatggc cctcctgcag gatattcagc tttgacttgg ccaggtgcag ggtgtgcga
40921 ccagctcggt ccagggcatc tacacgggcc cctgcaggta ccagctgagg tgagcattct

40981 gggggtgagg gggagaaaga ggacagagag gggaagaaac cacatacctc ctcgtagcag
41041 tgtggtgatg acaggaacgt ggttggtgca ggccgctgag gaagaacaac agagacttaa
41101 gaatccagga ccaccagagc ctggctctgc gtggccactg gagggagcag ggctgtgtct
41161 cccagacgcc tgtgccaagt tatgcctact gagaaggtat cgccaaggca cagctaggta
41221 ggactcagaa gtcaagttca actccctccc tgtacagcag aggaaactga ggcctggaga
41281 taggatctgc ctcagtgtga gccacagtgc tgagtgctgc tgggctagcc taggttgcta
41341 aattggctct gccgagggca cagccaactg ctaaatgatt ggaaggccca tgggacacag
41401 gctgctgggc agtgcagccc tgggagtgca ggcctaggat cttccctgac actcacagtt
41461 tctgccagga gcatgccggg ccccaccctc ttcatctaaa gagcctttct ttgctgcaaa
41521 tgagttgtct tagtgacttc tgacatgctt acccaggtgc agtggcgtgt tccccagccc
41581 atctcgctgg ttaggatcag caccatggtc caggagcagc tgcactggaa acaggcaaac
41641 ccaagaggag ttgtcggagg cttccattgc ctgcaccctg aacccctaac ctagctagag
41701 aggctggctc agagtagggg acacggaatg caagcatgga tggctgtgtt acttcattca
41761 ttcactcact cactcactta cttaccagag ttcatgggca aagccctgag ggccaggaac
41821 tgaggatttg gggagggcct cttggcctat gcaggtgtag gaatgagatt tgaagggtta
41881 agtaaagttc ttccaatagc agctgctcct ggtgaaggct gtccagggca ttttctgctt
41941 ctcccacagc caagcaccat ggtaggaatt aagtcaataa atgatgctga gctccatgga
42001 taacctctcc ctcctggcag tcttgggatc acctcagggg tgtgctgtat tatcaccagg
42061 gtctgccaat acccagaagg atgcataggc aggtagcctg gatcccagac atcatacagg
42121 gaggtcaggc tgagcctcac accatgtaca aaaactaatt caagagggac catagacata
42181 agtgtgaatg ctaaaacagg cttccagaag aacatgaaca tcttgggcag gcaaagattt
42241 cttaaatagg tcacaaaaat actaaccata ggaaagaaaa ggccaggcat gggagctcag
42301 gcctgttaat cccagcactt tgggatgccg aggcaggtgg atcatttgag ctcaggagtt
42361 caagaccagc ctgggcaaca tggagaaacc ctttctctat actaaaaaca caaaaattag
42421 ctgggcatag tgatggcaga cttgtagtcc cagctacttg agaggctgag gtggcagggc
42481 ggcttgagcc cagaaggtcg aggttgcagt cagccgagat ggtgccagtg cactccagcc
42541 taggcaacag agacagaccc tgtctcaaaa aaaaaaaaaa aaaaaaaaaa aaaaaaagag
42601 aaaggaaaga aatattgata aattggattc attaaaatta agaacatggc tgggcacggt
42661 ggctcatgcc tgtaatccca gcactttggg aggccaaggc agccagatca tctgaggtca
42721 ggagttcaag accaggctgt tcaacatgtt gaaactgtct gtactaaaaa cacaaaaatt
42781 agctgggtgt ggtggcatac ccctgtagtc tcagctactt gggaggctga ggcaggagaa
42841 ttgctggaac ctaggaggtg gaggttccag ggagccaaga tcatgccact gaactccagc
42901 ctgggtgaca gagcgagact ctgtctccaa aaaataaaaa ataaaatagg ccagggcaca
42961 ggagctcacg cctgtaatcc cagcactttg ggaggccaag gcgggtggat catcggaggt
43021 caggagttcg agaccagcct gaccaacatg gtgaaatccc atctctacta aaaataaaaa
43081 attaggtggg tgtggtgaca cgcatctgta atcccagcta ctcaggaggc tgaggtggga
43141 gaatagcttg aacctgggga gcagagcttg cagtgagcca agatcacacc actgcactcc
43201 agcctaggca acagagtgag actctgtctc aaataaataa ataaataaat aaaaataaaa
43261 ttaggaactt gtgttcatca aagtatacca ctcagaaaat gaaacagcaa gctcagaaaa
43321 tcaagtcatt tatatttgat aaatgatttc taaccagaat atacaaagaa ttcttacaaa
43381 tcaataagaa aaaggccagg cgcagtggct catgcttgta atcccaatac tttgggaggc
43441 caaggtgaca ggatcgcctg agcccaagag ttagagaaca gctgggtaac atagcaggac
43501 cacgtctcta caagatgttt taaaattagc tgggcttggt ggtgtgcacc tgtcgtccca
43561 gctacttggg aggctgaggc aggaggattg cttgagccta ggagattgag gctgcagtgg
43621 gcagtgatca ggccactgca cgtaagcctg gatgacagag tgagactttg tctcaaaaaa
43681 agaaaaagac agaaaactag ttttttttgt ttgtttgttt gtttttgaga cagagtcttg
43741 ctttctctcc cagtctggag tgcagtcacg cgatctcggc tcactgtaag ctccgcctcc
43801 cggcttcaca ccattctcct gcctcagcga gtagctggaa ctacaggcac ccgccaccac
43861 acccggctaa gtttttgtat atttagtaga gatggggttt caccgtgtta gccaggatgg
43921 tctcgatctc ctgacctcat gatccacccg cctcggcctc ccaaagtgct gggattacag
43981 gcgtgagata ccgcgcccag gcaaaacttg tttttaaaaa aatggacaaa agaggccagg
44041 tgcggtggct cacgcctgta atcccagcgc tatgggaggc cgaggcgggt ggatcacctg
44101 aggtcaggag ttcgagacca gcctgaccaa catggagaaa cccggtctcc actaaaaata
44161 caaaattagc cgggcgtagt ggcgcatgcc tgtaatccca gctacttggg aggatgaggc
44221 aggagaatca ctttaacccg ggaggcagag gttgtggtta gccgagatca cgccattgca
44281 ctccagcctg ggcaacaaag agtgaaactc tgtctcaaaa aaaaaaaaaa aaaaaaaaaa
44341 aggacaaaag atttgaacat atagttcata aaagaggatg taaaaatggt caataattgg
44401 acacagggag gggaacatca cataccaggg cctgtcgggg ggtgggggcc aaggggaggg
44461 agagcattag gacaaatacc taatgcatga ggggcttaaa acctagatga cgggttgatg
44521 ggtacagaaa accaccacgg cacaggtgta cctatgtaac gaacctgcac gttctgcaca
44581 tgcatcccag aacttaaagt ttaaaaaaaa aaatggcccg ggcatggtgg ctcacacctg
44641 taatcccagc actttgggag gccgagacag gtggatctcc tgaggtcagg agtttgtgac
44701 tagcctggcc aacatggtga aaccctgtct ctactaaaaa tacaaaaatt aactgggtgt

44761 gatgatgcgt gcctataatc ccagctaccc gggaggctga agcaggagaa ttactcaaac
44821 ccaggaggtg gaggttgcaa tgagccgaga tcacgccatt gcactccagc ctgggcgaca
44881 ggcaagactc cgtctcaaaa aaaaaaaaaa aaggccaata aacacatgaa gaggtgctca
44941 acgttactgg ccatcagaga aatgcaaatt aaaccactat gagacaccaa aagggttaag
45001 attaaagaga gacaacagca agcactggca gggacgtagg gaaaggtgga actcttacac
45061 tgctggtggg gagtgggagt gtgtgtcagt acatcccttt tgggaaaagg tttagtagta
45121 cctactaaag ctaaatacat gtgtacccta gtgacccaac aatgccattc ctaggtttat
45181 agaggataga tgagtgttta cagccacaaa tcacatatac aagaatgttc acaacacttc
45241 attcataaag ctaaaacctg gaaacaccac aaatgtccgt caatagtaga atggataaat
45301 ggggtatatc acaatgaaat acaaaccagc ataagaaagt actaactaca gtccaccctc
45361 cctaactgtg aatactgtgt ccacagattc aatgaaccct ggactcagaa cccgaggata
45421 tggagggcca acttcaaagg acttgagcat ccatggagtt tggtatctga gtggggtcct
45481 ggaaccaatc ccaagaacac tgagggaaga ctgtactgat ataggcagta ttcatgggtg
45541 aatctcatgg agataacttt gagtaaaagt atgcaaggct ggtgcagtgg ctcacgcctg
45601 taatcccagc actttgggag gccaaggcag gtggatcacg aggtcaggag ttcaagacca
45661 gcctggccaa catggtgaaa ccccatctct actaaaaaat acaaaaaaat taaccggtgt
45721 ggccgggtgc agtctctcat gcctgtaatc ccagcacttt gggaagccga ggcgggtgga
45781 tcacgaggtc aggagatcga gaccatactg gctaatacag tgaaaccccg tctctactaa
45841 aaacatataa aaaattagcc aggcatggtg gcgggtgcct gtagtcccag ctacttggga
45901 ggctgaggca ggagaatggc gtgaacccgg gaggcagagc ttacagtgaa ccgagatggc
45961 accactgcac tccagcctgg gcgacagagc gagactccat ctcaaaaaaa aaaaagactc
46021 ctataaaaag agaaaagctg tcctgctgct gtggttggca ctgagtaaat gttacctgaa
46081 agtcttgaag gcctgggccc tgcactctcc agtggtctct tagttggaga tatcccttca
46141 tcctcagtca atcatattat ctcccccagg tttaccctgc cccagtctaa cattgccagt
46201 tccacaggct aacagaggga gaagggcagg gcactgcagt gccaagtctt ccttatatcc
46261 agaacaactg ggcatcccaa ggctgtgcag acagcagcgc ctcccccaag gcctgtgcag
46321 agacactact tgcctagccc tactccctcc tccctcccca ggactcacca atctggtcat
46381 tgccattgca tgaggcaaag tgtagagctg tgcggccctt gtcatcagct gcacagggat
46441 ccgcgccatc ttccagcagc tgctgcactg acaccaccaa gacagaggga ggaaggggga
46501 gaaagcggcg atgttagcta agctgcagac ccgagcgatg gagagggagg ctcaggggtg
46561 cctctagaca agacatcgac aatgcatagg aaagcagccc ttcctagatg ctggtcctat
46621 gggaacaagc aacttgttct tctgagggca caactgaagg ccatggattc tgagtgctca
46681 aactacacgt ctagtgatag aacatcgtag gaggttcaag gccctcaggg tcagaaattt
46741 tagggtcaca ggaatcccta gcatagacag tggtccccct cccgtgacct cggcttggat
46801 acacatccat tctggctgga aacagctgcc caagctattg gcctatgcta tgctcccagc
46861 aggcagcaat cccccagcta cttggggcct cccaagtagc ccccagctaa tttttgtatt
46921 tttagtagag acggggtttc accatattgg tcaggctggt ctcgacctcc tgaccttagg
46981 tgatccgccc acttcagcct cccaaagtgc tgggattaca ggcgtgagcc accacacccg
47041 gcccgaaaaa atgtttatga tacacaaaaa attatgcagg ctacagcatg tgtatatagc
47101 agtttgcaag ccagcccata aagaaaattc cagtgtatag gaaagaagcc ttgaaggata
47161 ttcatcaaaa tattaacaga ttagttcctt tgcggacatg gattttttct tatatatttc
47221 tctaatttcc acaacatttt taaaaaattt ttttcttatg ttatttattt atttgagaca
47281 gaatttcgct ctgtcgcgca ggctagagtg cagtggcgtc atctccgctc actgcaagct
47341 ctgtctccca ggttcacacc attctcctgc cgcagcctcc caagtagctg gcactacagg
47401 cgcccaccac cacgtccggc taattttttg tattttcagt agagatgggg tttcacagtg
47461 ttagccagga tggtctcgat ctcctaacct cgtgatctgc ccagctcggc ctcccaaagt
47521 gctgggatta caggcgtgag ccaccgcgcc cagctaacat ttactttttg tagagacgag
47581 gtctccctat gttgtccagg ctggtctcaa actcctgagc ccaagtgatc ctcttgcctt
47641 agcctcccaa agtgctggga ttacaggcat gagccacctc acctggccgt ggcagctaag
47701 ttgagtattc actcccatta accacaagtt ccaaagttag tgcctactct ttgcagaaga
47761 ctgtgctggc tgccagggat acaaaagtat acatggtctc caccatcaag gagtccacag
47821 ggtggtaaat gctatgacat caggaagttc aaagtgacag gggagcatgg ggagagtgag
47881 tgtctaagcc agacttacct tcagcaagat gactgcacag tcagacaata aatacagtga
47941 gcctgctcca agggcgtgag tttgcagact gccttcccac atattgatta cctggccttt
48001 tccttcccca accagcccat gggaccctgt agctggacct actgtgcaca ctcacctgtt
48061 tccacatcat tggcattggc cgagtccctc agtctcttca gagctgtaaa gagagtaacg
48121 gatgccagtt taaaaaaaca gccatggctg ggtgcggtgg ctcatgccta taatcccagc
48181 actctgggag gccaaggcag gcagatcaca aggtcaggag atcgagacca tcctggctaa
48241 catggtgaaa ccccatctct actaaaaata caaaaaaatt agccgggcgt ggtggcaggt
48301 gcctgtagtc ccagctactc gggaggctga ggcgggtgaa tggcgtgaac ctgggaggtg
48361 gagcttgcag tgagcagaga tcgcgccact gcactccagc ctgggtgaca gagtgagact
48421 ccgtctcaaa acaaacaaac aaacaaacac acacaaaaaa aacacagcca ttggctgggc
48481 atgatggctc acgtctgtaa tcccagcact ttgggaggcc gaggcaggtg gatcacttga

48541 ggccaggagt tcaaaaccag cctggacaac atggtgaaac cccatctcta ctaaaaatac
48601 aaaaaaatta gccgggtgtg gtgcacatgc ctgtaatccc agctactctg gaggctgagg
48661 taggagaatc acttgaaccc aggacatgga ggttgcagta agccaaaatc gcgccactgc
48721 actccagcct gggtgacaga gtgagacgct gtctcagaaa aaaacaaaaa caacaaaaac
48781 caacaaaaaa aacccagcca tctagctggg tgtggtggtg tgcacgtgta acaccagcta
48841 cttaggaggc cgaggtagga gaatgggttg aagccggaag gcggaggcta cagtgagatg
48901 agatcacgcc actgcattcc agcctgggca acaaagtgag actgtctcag gaaaaaaaaa
48961 aaaaaaaaaa gccacaatgg ctgggcgcag tggctcatgc ctataatccc ggtactttgg
49021 gaggccaagg caggtggatc acttgaggcc aggagttcaa gaccagcctg gccaacatgc
49081 tgaaacccca tctctactaa aaatacaaaa ctagccatac gtggtgactc acgcctgtaa
49141 tcccagaacg ttggaaagct gaggtgggca gattgcctgt gctctggagt ttgaaaccac
49201 cctgggcaac atggtgaaac cttgtctata ctaaaataca aaaaattagt tgggcatggg
49261 ggtgcacgcc tacaatccca gctacttggg aggctcaggc aggagaatcg cttaagccca
49321 ggaggcagag gttgcagtga gccaagactg tgccactgta ctccagctcg ggcaacagag
49381 tgagactctg tctcacacaa acacacacac acacacacac acacacaaaa aacataggcc
49441 gggcacagtg gctcacgcct gtaatcccaa cactttgaga ggccaaggtg ggtggatcac
49501 gaggtcagga gtttgagacc agcctggcca ataaggtgaa atcccttctc tactaaaaat
49561 acaaaaatta gccaggcatg gtggtgcatg cctgttatcc caactactca ggaggctaag
49621 gcaggagaat cacttgaact tgagagccgg aggttgcagt gagccaagat cgcgccactg
49681 cactccagcc tgggcaatag agcaagactc tgtctcaaaa taaataaata aataaataaa
49741 taaataaata aataaataaa taaataaaat aaaaacacaa aaattagccg ggtgtggtgg
49801 tgcatgcctg taatcccagc tacttgggag gctgagacac gagaatcact tgagcccaaa
49861 atcacaccat ggcactccag cctgggtggc agagcaacac tccatctcaa aaaaaagaa
49921 agaaaaaata tttctcaatc ttaggttaag ttctaactta aatcaatcta acatggtaaa
49981 accccatctc tactaaaaat acaaaaacta gctgggtgtg gtggtgcggg cctgtagtcc
50041 cagctacttg ggaggctgag gcagtgagcc gagattgcac cacctgcact gcagcctggg
50101 tggtaaagcg agactctcgt ctcaaaaaaa gaaaagaaag acagtgccct gggaacaagg
50161 agaaacttga aatgaggcgg agctagtaca aaggccacat cctcctgcaa atcctccaag
50221 acttttggag ccagatttcc tctctatcac ttgtagtcac agggtgacct tgagcaagct
50281 agttaaccca gctgggcctc aggtctatca tctgcaaaac aaggctgagt tgttctgcca
50341 gccccgagtt attgcaaatg atttacacag aagctctttg tgagctggtt acacagataa
50401 tcttatattc tccttcaaag caggtcgttc ccaaatgatt tgcctaaatt tggtatttcc
50461 taagcagatt ttccccctcc aatggctaat ggaaagcaat gtctttgtca tcggagatat
50521 ggtcaaaaat tgcaattcag atacatgaca attccttgct ttcatacagc actttatggt
50581 caatacaacg cttccaaatt cttatttcat cttcgtgaca tcactaaaat atggctagag
50641 tcatagccag tcctggaaga aatccccaaa gtccccattc ttaatctctc agtgcctggg
50701 tttccatgtc acccatgcat tgtagaagac aatatggtag atataccaga ggagaagttt
50761 gagtcgggct gacccggatt ctaataccag caacagccac ttgctatgtg acctggaata
50821 agacaatttc tctgagcctc agtttcctct tcagtaaagt gggaacaatt tctatgtctc
50881 aaagagttct tgtgaggaca gatgatcatg tatcagcagt gtttggggca cagcaaaggc
50941 agcataacag ctaaacatcc attaccttac ttaactcatc ccatttacaa gatgagaaaa
51001 ctagggctga ggccggccca gtggctcatg cctgttatgc cagtactttg ggaggccgag
51061 gtgggtggat cacctgagat cagaagttcg agaccagcct ggcaaacatg gtgaaacccc
51121 atctttacta aaattacaat aattagccgg gcttggtagt gggcgcctgt aaccccagct
51181 actcgggagg ctgaggcagg agaatcgctt gaacccgggg ggcggaggtt gcagtgaacc
51241 aagatggcgc cactgcactc cagcctgggc aacagagcga gactccgtct ccaaaaagat
51301 aataataata aataaataaa aataaaaaaa tttaaaaaga aaactaaggt tgaaagggtc
51361 tatcatttgc cctaaggtct aactgtggta aaggagtcca agtccgtgca tttaaacaag
51421 ctggtacgac ttggctgcag gctctggcct cccctctcca tgataataaa agtgttcccc
51481 agtacctcat tttattattc accaataagc gttccgagtc atgggaagtt tccattcaga
51541 aattacccag tgagagaaga ctggggtgac tggctcagag caggaagact ttctaaaaaa
51601 accatcatca ccatcgttac tgaaaggaga ctcaagggtc agtgtggccc tcagcgcaga
51661 cgtcggggga ccccggctga gggtcggggg cgaccaaagc gcggccactg gtcctctgag
51721 gccccgcccc tcgcccgggc tgccccgccc cgggcccccg cgccgctcgc gccgctcacc
51781 gtgcacctcc ttgcccgtgg gcccgagccg ccggtggggc ctggcagcgc gcctcagccg
51841 gccagcgggt atcttgcagc gcagctcgtc gcgcggctcc gcatcctgct gccacaggac
51901 gtgcaagtag cgcagcggcg actgggcccc gccggacgcc cggcccgaga ggcccgcgcc
51961 gccgccgccc agcgcagacc cgaagtcagc gaaggagaag aggccctcag cgtcagtcag
52021 cggctccggc gccaccgcgc actcgccctc cgagctcgag tggcctgagc gcggctcgtc
52081 gtccgcgtcc ccggcggcgg ctgccatggc aacggctccg cgcgggcctg gccgcctgag
52141 cctcgttccc gaccgaccaa cggacctact tccctccgcc ctgagtcgtg ctgtcagcga
52201 gctggcgggc gggcagggcc cgcaaccacg gcaaccgagc gggggcgggc tcaggccgag
52261 acagagcggc gaaccaatga ctacccttcc agcttaaacc ggtcccaaaa ccaatcggaa

52321 agggagggca cagtacaagc aggataattg gacagggttt ccggcaacgt gactgacaga
52381 cattgtaacc aattacaaac ttgaagtgac cttggctttc gctgtacgtc gctaccggag
52441 ccagtaaaac agcatttcag acgctttttt tttgcgacgg agtctcgctc tgccgccagg
52501 ctcgcgatct cggctcactg caacctccgc ctccttgaag caattctcct gcctcagcct
52561 cccgagtagc tgggactaca ggcgcgcgcc accacgccca gctaattttt gtatttttag
52621 tagagacggg gtttcaccat gttggccagg atggtctcga tctcttgatc tcgagatgtg
52681 ccggcctcgg cctcccaaag tgctgggatt acaggcgtga gccaccgcgc ccggcctttt
52741 tttttctttt tcctctccat ttcccagtct aatgagaaaa caagcagata ccttagcaaa
52801 tcagattaag atgatttgca ggcactcaca aaggaaaatg aagaagccac gccatccatg
52861 tagatataga tgttgtaaat caatgaacgc aaaaaaaaaa aaaaaaaaga acttcccaca
52921 aatgcataat gtacgtggga aagggaaaat gtgttgaaat ttggagatac cactacaaac
52981 tgtgagtaat agtgtctaaa tgcaatggat ttcctttagt cagagttctc taggtaaccc
53041 cgacgtagat gactgatcta caaagatttg taatacaaag tgattacatt agagcaatag
53101 tttagttcga cagccagtca ctgagccagg taactgctag gtcctggaca caggatcaga
53161 cttggtgccc ctgagatgct cacaatccaa tgggagaaga ccaggaaaca aataagtgtt
53221 cctcaagcaa catttcatgg gtgcacgttt tcataaaact tctcacaatc ccgtacccca
53281 caagcacttc tataaagtag acaagaagga ccatccctat tttataggtg aggagttgaa
53341 aagcagaggg aagagactca ttcacagttg cccagctctc accggaccag gtcttgtgtc
53401 ctggcaaaag ttctttccat atataaaccc tcccactcaa cccagttaaa atgtaatctt
53461 tccgcacggt gggtcacgcc tgtaatccca gcactttggg aggccaaaat gggcagatga
53521 caaggtcggg agttcaagac aagcctggcc aacatgatga aacctcgttt ctactaaaaa
53581 tacaaaaatt agctgggtgt ggtggcgcac gcctgtaatc ccagctattc aggacactga
53641 ggcaggagag tagcttgaac ctgggaggca gaggttgcgg tgagctgaga tcgcaccatg
53701 gcactccagc ctgggtgaca gagcgagact ccatctcaga aagaaacaaa aacaaaaaaa
53761 aggcctggcg ctgtggctca cgcctgtaat cccagcattt tgggaggctg aggcaggcag
53821 atcacgaggt caggagattg agaccattct ggctaacatg gtgaaacccc gtctctacta
53881 aaaatacaaa aaaattagcc tggtgtggtg gtgggcacct ttagtcccag ctactcagga
53941 ggctgaggca ggagaatggc gtgaacccag gaggcagagc ttgcggtgag ccgagatcgc
54001 accactgcac tccagtctgg gcgacagaga aagactccat ctcaaaaaga aaaaaaaaag
54061 taatctttct cttacaattg gaaaagcact tccagaattt tcagtgtttg cagtcgccat
54121 tgtgaggttt atcattgtac tatatggaaa aaaatgctat gtttatttgt actctcctcg
54181 tctgccccat aatggataca gtgctgagac aggatctctc actgtcactc aggctggagt
54241 gcagtggtgt gatcacagct cactgcactc aagctttcct cccacctcag cctcctgagt
54301 agctggggcc acagatgtgt accaccgtgc ctggctaatt atttttttga tttttgtag
54361 acatggtctc acttttttgt tcaggctggt cttgaactcc tcgcctcaag ggatcctcct
54421 gcctccacat cccaaaatgc tgagattaca ggagtgagcc acagtgcctg ggccccaatt
54481 tttttttaag atggggtctc actatattgc ccaggctgat cttgagctcc tgagctcaag
54541 tgatcctccc accttggcct cccaaagtgc tggcatgagc caccacatcc agccccatga
54601 aaatcttctg acttcacaac tccttctcaa atgtcatgaa cattttgaga attgttgtca
54661 aattggagca acctgtatca aattcctgtc atatatggac tataagattt caggtaattt
54721 cttttctttt tttttttttt gagacggagt ctcgctctgt cgcccaggct ggagtgcagt
54781 ggcaggatct cggctcactg caagctccgc ctcccgggtt cacgccattc tcctgcctca
54841 gcctcccaag tagctgggac tacaggcgcc cgccactacg cccggctaat tttttgtatt
54901 tttagtagag acggggtttc accattttag ccgggatggt ctcgatctcc tgacctcatg
54961 atccacccgc ctcggcctcc caaagtgctg ggattacagg cgtgagccac cgcgcccggc
55021 caatttcttt tcttttttct tttttttttt gaggcagttt tgctcttgtt gcccaggctg
55081 gagtgcagtg gtgcaatctt ggctcaccac aacctccgcc tcctaggttc aagtgattct
55141 cctgcctcag cctcccgagt agctgggact acaggcatat gccaccatgc ccggctaatt
55201 ttatattttt agtagagacg gggtttcact atgttggcca ggctggtctc gaactcctga
55261 cctcaggtga tccatctgcc ttggcctccc aaagtgctgg gattacaggt gtgaaccact
55321 gtgccaggcc tcggagaagg gttattattt aaactagaaa ctaaatgtct cccaaagtta
55381 gctcggttcg agccaggaat gattaagggc agtctgaaga ttaaatggaa gatgagggtt
55441 ggttagatca gatctctttc actgtcatta ttttctcact gttacaattt ttgcaaaggt
55501 ggtttcagca ataaaaatat ttcacaaatt ttacaaaagc aaaggaccat gaggacattg
55561 ttggggaccc tccctgaacc aaggaagaga ctgtgactct tccttggttc agggaaggtc
55621 ctgaaccaaa tcgagggtcg gtaaagctta agctccatta tttccaaatt tgtgtcacct
55681 ctggtgacag cacaaggaag aaacacaaaa caagaacgca gcatagggag ttggggctgg
55741 gcattgcaat tctcaatagg atgttgagtg gatggcatca gagcagacac ctgaaggtgc
55801 catgcagata ccgatgggga agggaatggg agtgggggtg ccactgcatt cgattctaga
55861 cttggataac agtgagtgca aaatcccaga ggtggagcgt gcctcagtgg gaaaagcaag
55921 gagggcggcg tgacaaaggg acccagagag tcctggtcac acagcaaatc agtggcggag
55981 tagggactgg aactcggatt tccactaggt gttgtgtatg ggcagatggc taaactaagg
56041 cctctccttg actcatacac cggtttttat tggtttgctt tataacccca tctctacctt

56101 gagggtttcc cctggcctcg aacccggtgt actcaagagc tgcccatgtc ccaggcttcg
56161 cgacacagct ggcctcatct ttgcctgctc caactttaat gtccaaagtt gggtggctcc
56221 tgttctccat tccctcttac tgcatgggac caaatctcaa tttttttttt tctttttttt
56281 tttagacgga gtcttgctct gtcgcccagg ctggagtgca gtggcgcgat ctcggctcac
56341 tacagcctct gcctctcagg ttccaccgat tctcctgctt cagcctcctg agtagctggg
56401 attacaggcg gacgctacca ctcccggcta atttttgtat ttttaataga gacggggttt
56461 cgccatttcg gccaggctgg tctcgaactc ctgatctcag atcatccacc cgcctcggcc
56521 tcccaaagtg tagggattac aggcgtgagc catcgcgccc ggccaaatct caatttttat
56581 ccccagtaag tttagtcttg tgaagtccga acgtttgaat aatttactcg ctgcaggcaa
56641 accgcctaca actaaatcca tcaggccccc gtatccgaat cttccttcac gcgagaagcc
56701 ggccatcttt tgtgtagcat tctttctggt tcctctaact ttgcctctcc tttacgccaa
56761 atacgtgtcc ttatatctga atctacacca ctattataga ttcaccttct ttgagaagtc
56821 cctatttgaa gagaggaaaa ttcaggctac ttgtaaccct cttattcttt ttagacggag
56881 ttttccctct tgttgcccag gctagagtgc aatggcgcga tctcggctca ccgcaacctc
56941 cgcctcccgg gttcaagcga ctctcctgcc tcagcctccc gagtagctgg aattacaggc
57001 atgcgccacc actcccggct aattttgtat ttttagtaga gacggggttt ctccatattg
57061 gtcaggctgg tctcaaaccc ccggcctcag cctcaggtga tccgcccgcc tcggcctccc
57121 aaactggtgg ggttacaggt gtgagccacg gaacgcggct cctcttcctt ctggatatgg
57181 ctgtctcaga tggccagagg cctcggaaag gcttccggtt tcccggcagg cccgagtggg
57241 gctgaacttc cggcctcagg acgcaggcgc gggccgctca tttcgctctt tccggcggtg
57301 ctcgcaagcg aggcagccat gtcttatccc gctgatgatt atgagtctga ggtaaggtgg
57361 ccgtaagggc gcggtgggct ccacgacggg gtgatctctg ggaccactgg atgtgggtcc
57421 cggcgcgagg cagcttccgg gtcgcggcca gctccggccg acctcccgcc cggccctcag
57481 gctgctccca cagttcccgg tccctagaca accctggctc tgcccgccca cttcgcgtgt
57541 tcgattggct tgtccttccc ctttctaaga ccggagacta aggcctgtta gacgtttctg
57601 tgtttctgca cctgagtttt tttggtctag gctccgtctg gtcaaatctc gctgattccc
57661 acagcctctt gtcctttgct ccacctcatt gcacccgtaa tgttgtattg tcattgtctg
57721 tttactgtcc cttttctcta aggcgtgtga gttcctcaaa ggcaggcaca gtgtcttatt
57781 cgcctctgta tccttagctc tctgcttggc atctagactc taggatgagc gttttgaatt
57841 cacgacttgg cggctgtcct tgactgtgtc tctttccttc taggcggctt atgaccccta
57901 cgcttatccc agcgactatg atatgcacac aggtgagacc acgggttagg ctggctgagt
57961 actcgtaggg atcagttcct ttggaatgtc ttagaaccag ctctgacagg catcaaaaat
58021 tgctctttga tggtccaggt ttggagtcag tcattagctg ccagtgcgag ggttttata
58081 aagcctaata aaccaggctt tttatcatag tccacctcag tcttggaaga gaaggattac
58141 ttacctggct atggttttct tgtgtaaact attagccaaa taagaaaagc ttttttgttt
58201 ttgtttttgt ttttgttttt gagatggagt ttccctctgt cccccaggct ggagtgcagt
58261 ggtgcgatct cggctcactg caacctccgc gtccctcgtt caagcagtag ttctcttgtc
58321 tcagcctcct gagtagctgg gattacaggc gcccaccacc attcccagct aatttttaaa
58381 tttttttaat agagacgagg tttcgccgtg ttgtccaggc tggtcttgaa ctcctgacct
58441 caggtgatcc acccgcccca acctcccaaa gtgctgggat tacaggcgtg agccaccgcg
58501 cctgccccaa ggaagaaaat cttgatgtga aaaatttaag taattcaaac caagcccgaa
58561 tgaaatcttt ttaggaggtg tctgaggttt cctgaaacgt tatagtgact tgacttagta
58621 gtcagtgtta tccattgttc taagtacttt gtatgaaata ttttgtttaa tccttacagc
58681 agttctgtat gttaatgtct cctaaagaat agaaaatata gcagagaggt ttattagtag
58741 taacttggca cagtcatata tttaaaaggt gacagttatt cattcaagaa atattaagta
58801 cctgttacca cttactttcc aacagtcttg cctctctccc ctccaacgca cacttttcta
58861 ggccctgagg gtatgtctga gtacaagaca gacgagattc ctgacttcct agacttggga
58921 gaagggtgca aaacagataa taagtgaaaa ataactttaa gtcatggtaa tatttttgaa
58981 gaaacaggga caacgagatt gggagtgact tgagatgttt gtatctctga gtattcaaaa
59041 gggaatttta cgcagacctg agtttagttt ctggggtggt cttgccattt cgttctatca
59101 tatatgtggg tttgagcata ctctgtattt gttgtatcca acctccagga gatccaaagc
59161 aggaccttgc ttatgaacgt cagtatgaac agcaaaccta tcaggtgatc cctgaggtga
59221 tcaaaaactt catccagtat ttccacaaaa ctgtctcaga tttgattgac cagaaagtgt
59281 atgagctaca ggccagtcgt gtctccagtg atgtcattga ccagaaggtg tatgagatcc
59341 aggacatcta tgagaacagg tatgggctac tggctgaaag ggcctctttc tgggtgggac
59401 tggcctggta atatagttcc tacaaatgag gtgagcactc tgtaaacagt actttcgggg
59461 gggttggggg gtgggggtct ttgtgagtcc ttgaggctag ggacatttta ttttgttaga
59521 ctttatttta tttatgcatt tgtttatttt ttgaaacgga gtgtcactct gtcgcccagg
59581 ctggagtgca gtggcaagat ctcggctcac tgcaacctcc gcctcctggg ttcaagtgat
59641 tctccagcct cagcttcccg agtagctggg actacaggca tgtgccacca tgcctagtta
59701 atttttttgt atttttagta gagacagaat ttcaccatgt tggccaggcg tgtgttgaac
59761 ccctgacctc aagtgatcca cccgcctcag cctcccaaag cactgggatt acaggtggga
59821 gccaccatgc ctggcctaat tttgtatttt ttagtagaga cggggtttca tcatgttggc

59881 caggctggtc ttgaacttct gacctcaagt gatgtacccc cgttggcctc ccagagtgct
59941 aggattacaa gcgtgagcca ccatgcccgg cctgttggac tttatatcct cagaaattta
60001 tacaaagcct aaaataatat cactattaac aattataata ataatgatta gcatttgttt
60061 ttattaccag gatagatcca tcaatgttca gtgtgttgtt tgtgttttta ctcatttacc
60121 cttcacagta agcccatgaa gtagcagttt ttctcatttt acagatgagg aaacagcaca
60181 gaaataagtc atgtagctag tttaatggat gtgctgagat ttgaacctgc agtgtctggc
60241 accaaagcct aatttctaaa ctgtgctatg cagcccttca gtacatatca gagcaagctg
60301 aactccgcag gttttgaggg gctgccgttg tgtaatgagc gttagagata agataggtca
60361 gtcaaagctg ggatagtcag gagtgacttc accaaggaaa tagaatttaa gcaagtcaga
60421 aggatgaggg agagaaaaca aatagccaaa ggcctattga tcaatgaata ggatctctga
60481 catgtgtttt ctttttttgt tgttttgaaa gagctgattg tagttgaaaa gcagaggaaa
60541 cctcagacat tgcatggttt taggcctcac ccctgccact ctcgtgagcc taagttctac
60601 ttggtgtgga ctccaaggtc cttcccgccc ctgcaagcca cccattaggt tgagctgaga
60661 gatcagccaa tgggaacaga gtcacttatg accaagaaga attgatctgg gggttagtta
60721 gtgaggagag agaaagggag aaaaccttgt tcggaggttg aacacctcca gccgaagaag
60781 gtgcagcatc gaggtctttc accactagca aatggatcca agtcatgcgg caccaaaaga
60841 ggttagcagc tgaagggtgt tgaagtgaca caacaccaaa acatgctacc aatgatgaat
60901 ccgtatctgt acaggtctcc agcagcctca gttcttgcct cctcaaaaga aagaattcga
60961 ctgaggggta aaatgctacc gatgatgagt ccgtatctgt acaggtctac agcagcctca
61021 gttcttgcct cctcacaaga aagaattaca ctgagggcta aaatgctact gatgatgagt
61081 ctgtatctgt acgggtcgac agcatcctca gttcttgact cctaacaaga aagaattcga
61141 ctgaggggca caaggcagaa gaagagaggg aggcaagttt gagagcagga gtgaaagttt
61201 attaaaaagc tttagaacag gaatgaaaga aaggaaagga cagttggaag agggccaggt
61261 gggtgacctg aaaggcaagt gtgctgaact gtgttttctt tattccccgg gtcttttctg
61321 attagaagta tataaatcag cgcctacaaa aattagctgg ggcatggtgg cagcatgcct
61381 atagtcccag ctagttggga ggctgaggtg ggaggatcac ttgagcctgg gaggttgagg
61441 ctgcagtgag ctgcacaatg gagcgagacc ctctctcaaa attaaaaaat ggacctgatg
61501 ttcaatagaa atgtagctag acattaattt taatttttca gccaagcgaa gtggagattt
61561 atttaaaata ttatttctat atgtaatcag tatttaaaaa ttaaggggat atttgccctt
61621 tttttctttt atacttaaag tttcctaaat ccagtgtgta tcagaaatac ttgctttgtg
61681 tttagatttt atagagttta tgactggaaa agtaattcaa atttgaaatt tgaatcttca
61741 aatagtagag taattccaaa tagacctaaa agttttccaa tactgagtta agtgttggtt
61801 tttaaactta aatggcaact aactgaaatt taaaatttaa ctctttgctt atatagtagc
61861 cacatgtagc tagtggctat tgcattgggc agtgcccgca gatacagatg acagttgaag
61921 ccaagcatct tgtaagtctt tctctaggag aatgtgagaa gaaatgctgg tgccttaaga
61981 agtatgtttc tttagggatg ggtgggcagg caaagaaagg atctagtaaa ggaggcagaa
62041 agaaaagtga aaagtagata gtaaattggc aaagaaatgt cacaggaacc aaaaagtctg
62101 cagaagaaac tgaggaagag agtatcacca aatgttttag tgaggtccag atgagaaaaa
62161 aagccagttg aatttggtaa cttggagggc cttgataacc ttagcaaggt caatgtcagt
62221 agagtgatgg aagcaacagc cagagagcag tggggtgaga aaggggaatt ggtaagtatc
62281 ggggacataa agggtcactg agtggctttg tgttttgttt ctgtgttttt tctgtttttg
62341 ttttttgttt tttgagacag gatttcgctc ttgttgccca ggctagagtg caatggcgca
62401 atctcagctc atcgcaacct ccgcctcctg gctccaagcg attctcctgc ctcagcttcc
62461 caagtagttg ggattatagg tgtgcaccac catgcctggc taattttgta tttttagtgg
62521 agacggggtt tcaccgtgtt ggtcaggctg gtctggaact cctaacctca agtgatccac
62581 ccgcctcagc ctcccaaagt gttgagatta cgggtgtgag ccagcgcgcc tggcctgttt
62641 cagtgtttct tttctgttct gtttctgtgt ttctccccaa cccccatccc gtagagacag
62701 ggccttccta tattgtccag gcttgtcttg aattcctggg ttcaagcgat acccctatct
62761 tgtcctccca aagtgttgag attacaggcg tgagccatca tgcctggcct gttcagtgtt
62821 ttttagggga acttgagcat gtttatatgt tggactaaaa gagccagaga agcaggaggg
62881 gtggggcttg aggagattgg aggaaagtat taatagctgc tgagggcatc aggagaagaa
62941 aattaaacaa cctgttgcag gtttgtcaag tagcagtgtt tggattggat aggcagcagt
63001 gtttaaattg gatgctgtag gtctgttgtg gccttggact gcagcagact gatttgcttc
63061 tgtgtttaga gacgtttgga tgtgctccag gagatggaaa cgctgtgtta aagaatggtg
63121 gccaaaaaag tagttcttat agcagaaaac agcaagcagt taaatatggc tatagttaat
63181 gatacagagg caggagatta tcctgaaaag cttgatgggt ccctaactga tacaaaaaca
63241 atattttctt gtgttgagaa agattgaagt gtggagttgg gttgaggaca gttgtgggct
63301 tgaagggctt tggcctcagc tctgtttatt tgacatcttc ccctttctaa gcctgggtcc
63361 tcatctgtaa aatgttagga ttggcattct ccagtccttt tggaatttta gagatttttt
63421 tcttgatttt tagctggacc aagctgactg aaagattctt caagaataca ccttggcccg
63481 aggctgaagc cattgctcca caggttggca atggtaggtg tgagctcttt atatcttgtg
63541 cactgagtgg aatccagtgg ctgagtcatt agacaggaag ctcaagaggg tctgtgtctg
63601 ttttgctcac cattttgttt ccttggtgct tggtatgtag taggtccttg gcaaaataac

63661 ttgtcgaagg ctggatgatt taagagagga cagagatctc tcatatatgg tgaaatttag
63721 atctttgtac tgacttgttt ctatttcctt ctataatttg ggggtcatta cagtcactct
63781 ggaataagct ctctagcttc ccacctttga ctttcttaga tgatgtcctt tatatatggg
63841 tgaggtaggc aaatccaact tcattatttt gcttgtggat attcagctac cctggcacca
63901 atttatttat ttatttattt tattttatta ttattatttt ttttgagatg gagtctcgtt
63961 ctgtctccca ggctgaagta cagtggcaca atcttggctc actgcaacct ccgcctccca
64021 ggttcaagcg attctcctag gttcaagcaa ttctcctacc tcagcctccc gcgtagctgg
64081 gattacaggc gcccgccacc acgcccagct aatttttgta tttttagtag agacggggtt
64141 tcaccgtgtt ggccaggctg gtctcgaact gctgacgtca ggtgattcgc ccacctcagc
64201 ctcccaaagt gctaggatta taggcatgag ccaccgcacc cagcctgccc agctaatttt
64261 taaaaatttt ttgtagatac cgggtcacac tttgttgacc tggctgatct tgaactcctg
64321 acctcaagtg atcttcccag agtgctgaga ttacaggcat gcaccactgt gcccagccac
64381 attgtcttga ttactatatt tttgtagtaa gttttgaaat ggggaattgt aggccgggcg
64441 cagtggctca cgcctgtaat ccaagcactt tgggaggccg aggcgggtgg atcacctgag
64501 gtcgggagtt cgagaccagc ctgattaacg tggagaaacc ccatctctac taaaaataca
64561 aaatcagccg ggcatggtga cacatgcctg taatcccagc tacttgggag gtggaggcag
64621 gagaatcact tgaacccgga aggcagaggt tgcggtgagc cgagatcgcg ccattgcact
64681 ccagcctggg caacaagagc gaaactccgc ctcaaaaaaa aaaaaagaaa gaaagaaatt
64741 gggaattggt aagtcctcca actttgttct tttttttttt ttccccaaga ttgttttgac
64801 tattctgggt ctcttggatt tgcttattga ttttaggatg gacttgtcaa tttctgcaaa
64861 gtagccagct gggattttga gaggaattgc ctagaatctg aagatcactt tggggagtat
64921 tgccgtctta acagtattaa gtcctccagg ccaggcgcgg tggctcatgc ctgtaatccc
64981 agcactttgg gaggtgaagg tggacggatc acgaggtcag gagatcgaga ccatcctggc
65041 taacacggtg aaaccccgtc tctactaaaa atgcaaaaaa gttatctggg caggccggca
65101 tggtggcggg cacctgtagt cccagctact cgggaggctg agacaggaga atggcgtgaa
65161 cccgggaggc agaacttgca gtgagctgag attgcgccac tggactccag cctgggcgac
65221 agagcaagac tgcgtcccaa aaaaaaaaaa aaaaaaaaaa accaatatta agtcctccaa
65281 tctatggaca tgggatgtct ttgatttatt taggtctttg gttgcgttca ccagtgtttt
65341 gtgttttcag tgtacaagtc tctcatttgt tttgttatag ctaacgtttt tttgaacact
65401 taactccttt tttttttccc cctaagatgg agtcacggag tcttgctgtc ttgcccaggc
65461 tggagtgcag tggcgcgatc tctgctcgct gcaacctctg cctcctgggc tcaagcaatt
65521 ctcctgcctc agcctcccga gtagctggga ttacaggcac ctggcggatt ttttgtattt
65581 ttagtagaga tggtgtttca ccatcttggc caggctgatt ttgaactcct gacctcaaat
65641 gatccacccg cctcagcctc ccaaagtgct gggattacag gcatgagcca ctgcacctgg
65701 cctgaacatt tactctcaat tgactaaaca cagaatgagt tcaaaactca ttctgtggct
65761 gggtgtcgta gctcacggct gtaattctgg tactttggga agctgaggtg ggaggatttc
65821 ttgaggctgg gagatttaga ctagcctgag caacataggg agaccccctt ctctacaaaa
65881 aattttttaa aaaaattatc caggcatggt ggcacatgcc tgtagtccca cctactcggg
65941 aggctgaggt ggaaggatca cttgagccct ggaagtcgag gctgcagtga gccatgatca
66001 tatcactgca ctccaacctt ggcaacagag cgagaccctg tcttcaaaca aacaaacaac
66061 attctgaaat taatattttc tttcttcctt tttttttttt tttttttttt tttgacatgg
66121 gggtctcact gtgttgccca tgctggagtg tagtgtgata atggctgact gcaacctgtg
66181 cctgctgggc tcaagcgatc ctcccacctt agcctgccga gtagctggga ccatgggtgc
66241 gcaccaccac gcttgtctaa ttactcttgt gtgctggttt ttttatgcac ttggatggca
66301 gaagtctcta aatggtgaaa ggaaatagat ctgaatgaga tcttaatctc atcgacagag
66361 attgaggttg atggagtcaa atgagaagtg gattttctca ttgcatgtat tcttggggca
66421 tatgcatagc aatttagatg ttgtttagtc ttcagaatta cctttattcc tccccaccaa
66481 agagctaaag ctgccaggat accccagttt tatggttagt gaagcaccct tctgccatct
66541 ctttcagatg ctgtcttcct gattttatac aaagaattat actacaggca catatatgcc
66601 aaagtcagtg taagtattta agtgtcgcag tttttttttt gtttttgttt ttttaactct
66661 gtgctgtttt tgtccctaga acacagtcat ttgtttatat tggttcagtg tctgggcatg
66721 attggcatgt ttttgagttt taagaagcgg tggaaggaac agtttaaccc agaaagctgt
66781 tcagacacac taacgctata taacctatgc attcttttaa atttctctgt ccccacaatt
66841 ctcctggact gggcaatcac ccatgcactg gttataggct gccagcctca taggatattt
66901 ccaggtacct gctgctgctt ctgcttgggg ttctttttcc cttcagagca ttgagcctct
66961 gtttctctga gatgtacata acactgtcac tgtatgccaa gcacaatgct aggcacttag
67021 cacacatctt cacatatcta attctactat caatcgtgca aagttggtac ccgaggtgcc
67081 aagaggttac cttttctcaa atttatccag ctagtaagtg acaaaactcc ttgaatctag
67141 accagcttgt tataacacca gtattctaca cattatccca actttgagca cttgctagat
67201 tctgttcttc ccgacttcta aattatagag tacgtgaagt ttcttttttt tttttttttt
67261 ttttgagaga gagtgagtct cgctctgtcg cccaggctgg agtacagtgg cgcgatctca
67321 gctcactgca ggctccacct cccaggttca cgccattctc ctgcctcagc ctcctgagta
67381 gctgggattg caggtgcctg ccaccacacc tggctaattt ttttgtattt ttagtagaga

67441 cggggtttca ctgtgttagc caggatggtc tccatctcct gacctcgtgt tccgcccgcc
67501 ttggcctccc aaagtgctgg gatgacaggc gtgagccacc acgcccggcc tagagtgcct
67561 aaagtttctt aaaatgccat ctgtactatt taaggctggg cgcggtggct cacgcctata
67621 atcccagcac tttgggaggc taagggcagg tcacctgagg tcaggaattt gagaccagcc
67681 tggccaacat ggagaaacct cgtctctatt aaaaatatga aactacaaaa ataagctggg
67741 cgtggtggcg ggtgcctgta atcccagcta cttggaaggc tgaggcagga gaatcgcttg
67801 aacccgggaa gcggaggttg cagtgagccg agatcgtacc actgcactcc agcctgggtg
67861 acagagtgag actcttgtct caaaaagaaa agaaaaatgc catctgtatt atttatatac
67921 accaatgact taatctctat ctttagcctc agttgcccca ctgaagtcca gacttgtata
67981 ttcagctgtc agcatctgca cttggctgtc tgagtcatct gaagcttaac acatctaaaa
68041 ctgaattctt attttccctc taaactagtt tttctcatgg taatcatctc aataaatgct
68101 accgtttatt accaagttaa ttggactaaa agccttggca ttattcttga cttttctctg
68161 acatgatatc cagcaaatcc tgttaagctc tgtcttcaac atacttcttt ttgttgtttt
68221 gttttatttt gttttgtttt tgagacggag tctcgctctg tcatccaggc tggaatgcag
68281 tggcgccgtc tcggctcact ccaacctctg tctcctgggc tcaagcaatt ctccaggctc
68341 aaacgattct ccttcctcag cctcctgagt agctgggatt acaggcatgc accaccacgc
68401 ctggctaatt tttgtatttt tagtagagac agggtttcgc tatgttgccc aggctggtct
68461 cgaactcctg acctcaggta atccgcccgc ctcggcttcc caaagtgcta agattacagg
68521 cgtgagccac tgtgcctggc cttcagcata cttcttgaat cagccacttt tcactgccac
68581 actgctgctc acctagttga agtcattatc atctcttccc tgaattactg caggagctgc
68641 ttacctggtg tcagtttcca ctcttggctg tttattctca acataataac cagcatgatc
68701 cttttgctac atagtttcat caggtcactt ccaatcctac catgacttcc catcacactt
68761 actaattcca ggttttagcc ctgtttccct tctcactcat ttcctgaaac tttccccctc
68821 cctccctggg ctcctggcaa tattcgtggc acttcagagc acttatctct tgtacgttgt
68881 atttgtttat ttaggggagg gagtttctta atctactgga aggttaactc caccggggta
68941 tgtgcttgtc ttgttcacca atataatcca gtgcctagaa taatgtctgg tcataatggt
69001 gttaattgag catgtgtgtg cttgcatgca tgtgtaagtg aaggaaattg gcaccaagat
69061 ctccacctaa ggaggtccta gtttctcaca gttactcacc cctcttctcc ttgaggaccg
69121 cttgatatgg ctagaactcc tgccttggcc acaggagcca ctgtcagtag tggtaggtgg
69181 aaaagttctt gctgccagag taaggggcca tgtttttccg gcatcccagc gggatctctg
69241 actactttat ctgatagaaa gtaaagattc ctggccaggc gctgtggctt acgcctgtaa
69301 tcccagaact ttgagaggct aaggcgggtg gatcacgagg tcaggagttc gagaccagcc
69361 tgaccaatat ggtgaaaccc cgtctctact aaaaatacaa aaattagcca ggcgtggtgg
69421 cgtgtgcctg taatcccagc tactcgggag gctgagacgg aagaatcact tgaccccagg
69481 aggcagaggt tgcagtgagc tgagattgca ccgctgcact ccagcctagg caccagagcg
69541 agactccgtc tcaaaaaaaa aaaaaagtaa agattcccta ctaccattga tgctaaagtt
69601 caggtacttt gtggatatgc taagaatcta gccattatgt gtacactgct tttttgggaa
69661 caccatgaca ccatagccca tagtatagct ggttttacca cctctaatct ctctgtttgg
69721 tctgtagatg aagattcagc agaagcttat tgatgctgtt atctttttat tagctaccag
69781 gactcctaca taaatgacca gttttaagcc cttgggaagc agtttttcat tgaatgtgct
69841 tagattgtgc ttaatgtatt aagcacctta cataactttt attaaacaga gacggggtct
69901 tgccatgttg cccagcctgt tctcaaactc ctggcctcag gtgatcagcc cacttcagcc
69961 tcccagagtg ttgagattac aggcgtgagc cactgcgccc agctatataa tcttacttca
70021 ttgtgactag tcaacaatct tttgcccaca ttttacagag attaaatacc ttgtgagaac
70081 cccacagtta ataagaggtg ggactgactc caaagttgtg tttcttctcc atcacaaata
70141 cattgctcat ggtatggttc cccagctcag aaactatcag tggttctcag atgcaagcaa
70201 cataaagacc agaccagggg tcagccacct tctctgtgga gggtcagata ataagtattt
70261 tagtctcgag ggccatgatg gtttctgtca gatctgttca gctatgtctt tgtggcagtt
70321 atcaacaata tgtaatgagt gggcatggct gtgttccagt aaaacttctt tatttacaaa
70381 acaggtggcg gaatagattt gcaggccccc tgacctagat gcttgtttgg cgttggaaga
70441 ctgcaacatt cctggcccgt agtctagtga tgcccggtgc agcattatct tctcttaact
70501 ccctagcctt caagtgccac gatttaactt cttcgccttt gctagtgtgg tttttttctg
70561 aaacactttt ccttctctct gctttgccag atcatcatcc ctgctctgag ggtcatttaa
70621 ggtctgttgc tgtagaacct aaccacgtgt tgcagtattc agtgcgtcag ctgctatagg
70681 actttctatc ttccctctca cttgcatatt tacatagtaa gaaagagagg acagatacca
70741 attctcttgt atttgttcac agctgtcaca gtataccatg ggtaaaacac attaaaatta
70801 aatatctgta tcttgatatc tcttgttttc tttacagggg ggaccttcct tggagcagag
70861 gtttgaatcc tattacaact actgcaatct cttcaactac attcttagtg agtctgagat
70921 ttggccaaga gggtgtgtgt ggttatgatg aggttcagca ttggcctcca gctttttttt
70981 tttttttttt aaattagcag gatcttaatg tgtaggtggg aagaggggag gtcttgtgat
71041 tcatggtcct tttaagagag aaattacaaa gcattctgca gcctacagaa tgggcagagt
71101 cattgctttg aatctgactt ttctgtgatc tcctgcacag atgccgatgg tcctgctccc
71161 cttgaactac ccaaccagtg gctctgggat attatcgatg agttcatcta ccaggtatct

71221 ggtcagcttc agcctaattt gaaataactg gtccatgcca ggaatagctg ttactattgt
71281 ttgtgtaaaa aagctgcagg gtgtaaaaat atccccattg tagtgtaaaa taccccatgg
71341 cagtagactc cagtttcagc tggctttcgg aagaggcagg gaagcctaag cactccagat
71401 aggaagctgg aagtcggata agttcagctc ctgaccttca tcaagctctt actagacctt
71461 gttaggtggt ctggtggtaa gaacacaggc ttcacaggct ttggagcgag gtggaagtgg
71521 attctaactt gggtgctgcc acctgctggt agctctgtaa cagtgatgtg ctatttaaac
71581 tcagagcctc actttcttta cccacacaat ggaaatgttc tattatcttt tttttttaa
71641 gagtcagggt caggccgggc gcggtgactc acgcctgtaa tcctagcact ttgggaggca
71701 gaggcgggca gatcacgagg tcaggagatc aagaccatcc tggctaacac ggtgaaaccc
71761 cgtctctact gaaagtacaa aaaattagcc gggcgtggtg gcaggcgcct gtagtcccag
71821 ctactcagta ggctgaggca ggagaatggc gtgaacctag gaggcggagc ttgcagtgag
71881 ccaagatcgt gccactgcac tccagcctgg taacagtgca agactccatc tcaaaaaaaa
71941 aaaagaaaga gtcagggtct cactctgttg ctcaggctag agtgcagtga taatagctta
72001 ctgcagccta gaactcctgg gttcaagtga ttttcccacc tcagccacct gagtagctgc
72061 cactacaagg cccacaccaa cttacgtgga tatttatatt actgtagaga cgagggtctt
72121 gctgtgttgc ccatgctggt gttgaactcc tggcctcaag tgagcctctt gacttggcct
72181 cccaaagtga gccaccatgc ctagccatct atatcttgat tgtggtggtg gttacttaag
72241 tgtatgctcg atcagaatac aatagaacat tttcatcaca acaagaatat cacctacctc
72301 aggagttgct gttcaaggtc gaatgagaac tgtgccaaat gcaaataaag tacagcagct
72361 gtgtagcctg ggggtaaaag tccaggtttt gctgttgctg tttactggct tttttttttt
72421 ttttggagat ggagtctgac tctgttaccc aggctggagt gcagtggcat tatcccggct
72481 cactgcaacc tctgcctccc gggttcaagc gattctcttg tctcagcctc cccagtagct
72541 gggattacag gtgcccacca ccacgcccag ctaatttttg tatttttagt agagacgggg
72601 tttctccatg ttgaccaggc tggtctcgaa ctcccaactt caggtgatcc gccctccttg
72661 gcctcccaaa gtgctgggat tacaggtgtg agccaccgcg cctggcctac tggcccattt
72721 taaaatggta acatgttctg ttgtgggcac attacttaac cacactgtgc attggtttcc
72781 ccatctctaa aacggattgc tgcgaatgtt gctgagatac tacgtataga gtgtttagca
72841 tagtgcctgt cacagaataa ttaatacacg ttgcctgttg attgtgatga tgttcaatgt
72901 catcattatt cttttccatc ttatttagcc cagtccttct atttaaaaat acagattgct
72961 cacaactgta atcccagcat tttgggggtc caaggcagta ggatcgcttc agtccaggag
73021 ttcgagaaat agagactaca aaacataaaa taggctgggc gtggtggctc acgcctgtaa
73081 tcccaacact ttgggaggcc taggtgggca gatcacgagg tctggagatg gagaccatcc
73141 tggccacaat ggtgaaaccc catctctact aaaaaaaaaa cagctgggcg tggtggtgtg
73201 cgcttgtaat cctagctact cgggaggctg aggcagggga atcgctcgaa cccgagaggc
73261 agagcttgca gtgagccaaa atcgtgccac tgcactccag cctggcgaca gagtgagact
73321 ctgtctcaaa aaaaaaacca aaaaaaataa aatatataat ttgacaaaat ataattcttt
73381 tccccctttt tgtgctgtct atagctctaa ctgattattt caggataaat tggtatctta
73441 ataaagatgt tattagaact ccgtgaacca gggagacgtt gtgtatacaa agtgctgaac
73501 cgttacgagt ggtgagctga gaaaccttca tagtgctcct tgagaaggag gcttcctgct
73561 caagattagg cttcagtgcc tttcatagca aaggccacaa accgtactgg ccttgtttgc
73621 cctctggctc cttggcttgt gtcatccttt tatttggaat atttatagat gaatagcagt
73681 ctttttgact tagagcaagt gaagaaggat catccgtgct gattttcttt atccctctta
73741 agataatcat aattccctgg gcttagccag tgttgttgtt aaggggctta cggtgctttc
73801 taacaaagac tgtccagtga gggtcagtaa gtggctggtg tatttcgtcc tggccaggta
73861 gatgccacat ggggtaggtg gctcttcctc gccaccctgt tttatttatt tgtttttgtt
73921 ttttcaaact acagatatag tgaaagtgcc tgccttgctt gcttttgcag aggtatcaat
73981 tactgtctgg aaggaaaggc accttgttga tgtttctcag gcacttacat aaacatgact
74041 gtataaagag agatcacttc aaaaatggtt acgtactcta ttgtgccatt tactgtgact
74101 tgttaacaca cttaaaccat attttcatag acctgacttg ctcttttgct gcagtgtgcg
74161 tgatatggat ataccatgcc tctgatgatg aacctttggg ttgtcagttt ccacaattac
74221 aggcagtgaa tcagtgaata tccatatata tgttgtacac aggcatattt ttagggatca
74281 ctacctagaa gtagaattgt ggattgaacg atacttgctt tttaagtttt tttaaatcag
74341 cttactgagg tatagtttat ttttttaagt agagatgagc caccagctcg cgcctgttaa
74401 tcccagctct ttgggaagcc aaggcgggtg gatcacgagg tcaggagttc gagaccagcc
74461 tgaccaacat ggtgaaaccc cgtctctact aaaaatacaa aaaaatcagc cgggcgtggt
74521 ggcaggtacc tgtaatccca gctatttagg aggctgaggc aggagaatcg cttgaacctg
74581 ggaggtggag gttgcagtga gctgagattg cgccactgca ctccagcctg ggtgacaaag
74641 caagactctg tctcaaaaaa aacaaaaaac aaaaaacagg tgtgaaccac catgcccagc
74701 catgaggtgt agtttaatat acaataaaat gtaccagaat cagtgtacag tggatgagat
74761 tcaacaaatg tgtacagtgc cacaatcaag ataaaagaac gtttcaatca ctttgaatta
74821 taataaatag taatatattg tcccaccaat gggctgtatc aaattgtgtt actaccagcg
74881 gtgtttgaga gcaactgttg gtttttttgt ttctttgttt aagagatggg ggtcttgcct
74941 ttatggtcca ggctggagtg cagtggtgtg atcatagctc attgcagcct tgacctcctg

75001 ggctcaagca atccccccac cttagcctcc tgagtagctc ggactacagg tgcacccagc
75061 tgatttttaa tttgttgcag agatggggtc ttgaaatcct gggataaagc aattctccct
75121 gctcagcctc caaaagtgct gggattacag gcgtgagcca ccatgcctag ccaagaccaa
75181 ctgttttctg tactggatat tatcagtctg tctaattttt gtcagttcaa tgggaaagaa
75241 attctggccg ggcgcagtgg ctcacgcctg taatcttagc actctgggag gccaaggcgg
75301 gcagatcact tgaggccagg agttcgagac cagcctggcc aacatggtga aaccccgtct
75361 ctactaaaaa tacaaaaatt acccagactt ggtggtgcat gtctgtaatc ccagctactc
75421 agcagctgag ggatgagaat cacttgaacc gagaggcaga ggttgcagtg agctgagatc
75481 acaccaccgc actccaccct gggcagtaga gtgagattcc gtctcaaaaa aaaaaaaaaa
75541 aaaatcagcg cggtggttca cacctgtaat cccagcactt tgggaggccg aggcaggcgg
75601 atcatgaggt caagagattg agaccatcct ggctaacacg gtgaaacccc gtctctacta
75661 aaaatacaaa aaaatttgcc aggcatggtg gtgggcgcct gtagtcccag ctactcagga
75721 ggctgaggca ggagaatggc gtgaacccag gaggcggagc ttgcagagag ccgagattgc
75781 accactgcac tccagcctcg gagacagagc gagactccat ctcaaaaaaa aaaaaaaaag
75841 aaaaagaaaa aaaaatcata cttttctgat ttcccatatt aattgtcaat tttgttttct
75901 ttgaagtgat catcactatt ctttgcctgt tttctatttg attgcttttt taattgcatt
75961 acgagaattc tttgtaggat tttttttttt tttttttttt tgagacagag tctcgctctt
76021 tcgcccaggc tggagtgcag tggcatgatc tctgctcact gcagtctcca cctcctgtgt
76081 tcaagcgatt ttcctgcctc agcttcccaa gtatctggat tacaggtgtg caccaccacg
76141 ccctgctaat tttttttttt tttttgcatt tttagtagag acggggtttc accatgttgg
76201 ccaggctggt tttgaactcc tgacctcaag ggatccaccc atctcggcct cccaaagtgc
76261 ttggataaca ggtgtaagcg ccaccgcacc cagcctgaga attctttata tattctggat
76321 attctttttt tttcccccct aaggcggagt cttgctctgt cacctaggct ggagtgcagt
76381 ggcgtgatct cagctcactg caacctctgc ctcccaggtt caagggattc tcctgcctca
76441 gcctcccgag tagctgggat tacaggcatg tgccaccaca cctggctatt tttgttttgg
76501 tgcttttttt tttttttttt tttttttgga gacggagttt tgctcttgtt gcccaggctg
76561 gagtgcaatg gtgcgatctt ggctcaccgc aacctctgcc tcccaggttc aagcgattca
76621 cctgcctcag cctcccgagt agctgggatt acaggcatgt atcactgtac ctggctagtt
76681 ttgcattttt tgtacagatg gggtttctcc atgttggtca ggctggtgtc aaactcccga
76741 cctcaggtaa tccgcctgca tcggcctccc aaagtgctgg gattacaggc atgagccacc
76801 gcacccggcc tgtttttgta tttttagtag agatggggtt tcaccatgtt ggccaagctg
76861 ttctcgaact cttgatctca tgatccatcc accttggctt cccaaagtgc tgggattata
76921 ggcgtgagcc accgtgcctg gcatattctt tgtttttata ttacaaaatt agcattttgt
76981 cccacagtct agaagaagat aattttttgt gttttgagac agagttttac tctgtcactc
77041 acactggaat gcagtggcac aacctcacct cactgtagcc tccgcctcct gggctcaggc
77101 aaagcccgta cctcagcctc cttagtagcg gagactgcag gtgcctgcca ccattcctgg
77161 ctaattcgtt tgtgtttttt atagcgatag ggttttgcca tgtttcccag gctggtcttg
77221 agctcctgga cccaagtgat ctgcccacct cagtttccca aagtgctggg tttacagggg
77281 tgagcctctg atctctgctg gaagagttga agaacagtct tagatcttct aagacctagg
77341 ttttaggtct gtgatttcca aacctgatgc acaccagatt caaccagaga actttaaaaa
77401 attgagtagg taggtctgtt gtgttccatc tgtgtgactg ctggagccag gccaggagtg
77461 cttccaggtt ctacttctgt cccaaacaga ttcctgtggc atatcttctc tgtgttcgtt
77521 gtgcttggcc agacgactct ggttgagagt ggtagagagg tgacatagtc tgggaactga
77581 tggtcagctg tggtagggaa ggctcacacg tgcagatacc tattaccaca tcactgccca
77641 taacccaggg cgtaccacag tctagctgca tctgaataga tggtacaaga gcccctgtgg
77701 tgtttctggt ggcccggtca actgcattgt gaggcccaga gacaggaagt gactttgggt
77761 tgtgtttgac tccagattgg atgtttttac cacagactac ctctaatcct agagattttt
77821 acttcactgt gtagtaagta acattcagta atgaacagaa ttcacagttt agtcaagatg
77881 gctttgaaaa acagtgtacg gaatggttta gaatgagaag tggtcatctc accctcacct
77941 tgccccatcc agagccagag cattgcctcg ttttcagcat tgtggacatg gatgatcact
78001 ttgggcttat accactactg catgtttctt ttcttcctca acagtttcag tcattcagtc
78061 agtaccgctg taagactgcc aagaagtcag aggaggagat tgactttctt cgttccaatc
78121 ccaaaatctg gaatgttcat agtgtcctca atgtccttca ttccctggta gacaaatcca
78181 acatcaaccg acagttggag gtatacacaa gcggaggtga gtgcagcagg ccgacagccg
78241 tggcctgcga atttccagct gcttgccatc tgttccaaat gaatagatca ctgcaaggag
78301 agcagatgag tcaccatgtc ttaggtggtg gtctgttgac tctttgtagg tgcttgagta
78361 caaacagtag cagagtattt gtgctggggg ctctggggct tgggagtcca ttggccaaca
78421 gactgaacca gcacattgag atttgcgttt gtaaacactt ctgtgtctcc agtgcttttg
78481 tttagataag cacttctcac acgtgttggt gtcagagcca ctttaccctg ttaaaatatt
78541 gagaaccaca aaatgtatct attttaaaaa atttttttct ttttttttca gtgaagagtg
78601 gcatttttgc agatctctct gtctggctga gatagctggc ttctcatttc tgcttctgca
78661 ttcaatctgt tgcaatgtgt tcttttgttt gaagcacata aagaaaatct ggcccaggca
78721 ctgtggctca cacctgtaat cccagcactt tgggaggctt aggcagaagg atcgcttgag

78781 tccaggagtt tgagaccagc ctgggcaata tagtgagact ttgtctcaaa aaaaatgttt
78841 aaattgactg agcatggtgg gtggggtgcc cctgtagtcc cagctactcg ggaggctgag
78901 gtgggaggat tgcttgagcc caggaagcaa agattgcagt gaactgagat cacaccactg
78961 tactctagcc tgggccacag agcgagaact tgtctgaaaa aaaaaaagaa ggaaaatctg
79021 gcttcacacg gttataggaa gtttttttgt tgtttgtttt tgagacaggg tgtcgctgtg
79081 tcacccagtc tggtcttgga ctcctgggct caagcagtcc tgccttggcc tcccaaaagt
79141 gccaggatta ttggagagga ttttctttga tactacacca tgtagtagta tgtatgggtt
79201 gaatattcct gacttgaaaa tttgaaatct gaaatgctcc aaaatctgac tttttgagca
79261 ccaacatgac gccgtaagta gaaaattcta cacccaacct catatgagca gttgcagtca
79321 gaacttcgtc tcatgcacaa aagtatttaa aagattataa taaagttact ttcaggctgt
79381 gtgtataaga catatatgaa aaataaatga attttatgtt tagatttgga tcctatcctt
79441 aagatactca ttagggccgg gtgccatggc tcacgcctat tatcctggca ttttgggagg
79501 ctgaggcggg cagatcacct gaggtcag//

- LOCUS: AB018272 4143 bp mRNA linear PRI 16-JUN-1999
- DEFINITION: Homo sapiens mRNA for KIAA0729 protein, partial cds. *ubiquitin specific protease 34 (USP34)*
- ACCESSION: AB018272
- VERSION: AB018272.1 GI:3882178

1 ggctgatgac gactggtggc caatgcagat actaattaag tgccctaatc aaattgtgag
61 acagatgttt cagcgtttgt gtatccatgt gattcagagg ctgagacctg tgcatgctca
121 tctctatttg cagccaggaa tggaagatgg gtcagatgat atggatacct cagtagaaga
181 tattggtggt cgttcatgtg tcactcgctt tgtgagaacc ctgttattaa ttatggaaca
241 tggtgtaaaa cctcacagta aacatcttac agagtatttt gccttccttt acgaatttgc
301 aaaaatgggt gaagaagaga gccaattttt gctttcattg caagctatat ctacaatggt
361 acatttttac atgggaacaa aaggacctga aaatcctcaa gttgaagtgt tatcagagga
421 agaaggggaa gaagaagagg aggaagaaga tatcctctct ctggcagaag aaaaatacag
481 gccagctgcc cttgaaaaga tgatagcttt agttgctctt ttggttgaac agtctcgatc
541 agaaaggcat ttgacattat cacagactga catggcagca ttaacaggag gaaagggatt
601 tcccttcttg tttcaacata ttcgtgatgg catcaatata agacaaactt gtaatctgat
661 tttcagcctg tgtcgataca ataatcgact tgcagaacat attgtatcta tgcttttcac
721 atcaatagca aagttgactc ctgaggcagc caatcctttc tttaagttgt tgactatgct
781 aatggagttt gctggtggac ctccaggaat gcctcccttt gcatcttata ttctgcagag
841 gatatgggag gtgattgaat acaatccttc tcagtgtcta gattggttgg cagtgcagac
901 accccgaaat aaactggcac acagctgggt cttacagaat atggaaaact gggtcgagcg
961 gtttcttttg gctcacaatt atcctagagt gaggacttct gcagcttatc ttctggtgtc
1021 ccttatacca agcaattcat tccgtcagat gttccggtca acaaggtctt tgcacatccc
1081 aacccgtgac cttccactca gtccagacac aacagtagtc ctacatcagg tctacaacgt
1141 gctccttggt ttgctctcaa gagccaaact ttatgttgat gctgctgttc atggcactac
1201 aaagctagtg ccctatttta gctttatgac ttactgttta atttccaaaa ctgagaagct
1261 gatgttttcc acatatttca tggatttgtg gaaccttttc cagcctaaac tttctgagcc
1321 agcaatagct acaaatcaca ataaacaggc tttgctttca ttttggtaca atgtctgtgc
1381 tgactgtcca gagaatatcc gccttattgt tcagaaccca gtggtaacca agaacattgc
1441 cttcaattac atccttgctg accatgatga tcaggatgtg gtgcttttta accgtgggat
1501 gctgccagcg tactatggca ttctgaggct ctgctgtgag cagtctcctg cattcacacg
1561 acaactggct tctcaccaga acatccagtg ggcctttaag aatcttacac cacatgccag
1621 ccaataccct ggagcagtag aagaactgtt taacctgatg cagctgttta tagctcagag
1681 gccagatatg agagaagaag aattagaaga tattaaacag ttcaagaaaa caaccataag
1741 ttgttactta cgttgcttag atggccgctc ctgctggact actttaataa gtgccttcag
1801 aatactatta gaatctgatg aagacagact tcttgttgta tttaatcgag gattgattct
1861 aatgacagag tctttcaaca ctttgcacat gatgtatcac gaagctacag cttgccatgt
1921 gactggagat ttagtagaac ttctgtcaat atttctttcg gttttgaagt ctacacgccc
1981 ttatcttcag agaaaagatg tgaaacaagc attaatccag tggcaggagc gaattgaatt
2041 tgcccataaa ctgttaactc ttcttaattc ctatagtcct ccagaactta gaaatgcctg
2101 tatagatgtc ctcaaggaac ttgtactttt gagtccccat gattttcttc atactctggt
2161 tccctttcta caacacaacc attgtactta ccatcacagt aatataccaa tgtctcttgg
2221 accttatttc ccttgtcgag aaaatatcaa gctaatagga gggaaaagca atattcggcc
2281 tccgcgccct gaactcaata tgtgcctctt gcccacaatg gtggaaacca gtaagggcaa
2341 agatgacgtt tatgatcgta tgctgctaga ctacttcttt tcttatcatc agttcatcca
2401 tctattatgc cgagttgcaa tcaactgtga aaaatttact gaaacattag ttaagctgag
2461 tgtcctagtt gcctatgaag gtttgccact tcatcttgca ctgttcccca aactttggac
2521 tgagctatgc cagactcagt ctgctatgtc aaaaaactgc atcaagcttt tgtgtgaaga

2581 tcctgttttc gcagaatata ttaaatgtat cctaatggat gaaagaactt ttttaaacaa
2641 caacattgtc tacacgttca tgacacattt ccttctaaag gttcaaagtc aagtgttttc
2701 tgaagcaaac tgtgccaatt tgatcagcac tcttattaca aacttgataa gccagtatca
2761 gaacctacag tctgatttct ccaaccgagt tgaaatttcc aaagcaagtg cttctttaaa
2821 tggggacctg agggcactcg ctttgctcct gtcagtacac actcccaaac agttaaaccc
2881 agctctaatt ccaactctgc aagagctttt aagcaaatgc aggacttgtc tgcaacagag
2941 aaactcactc caagagcaag aagccaaaga aagaaaaact aaagatgatg aaggagcaac
3001 tcccattaaa aggcggcgtg ttagcagtga tgaggagcac actgtagaca gctgcatcag
3061 tgacatgaaa acagaaacca gggaggtcct gaccccaacg agcacttctg acaatgagac
3121 cagagactcc tcaattattg atccaggaac tgagcaagat cttccttccc ctgaaaatag
3181 ttctgttaaa gaataccgaa tggaagttcc atcttcgttt tcagaagaca tgtcaaatat
3241 caggtcacag catgcagaag aacagtccaa caatggtaga tatgacgatt gtaaagaatt
3301 taaagacctc cactgttcca aggattctac cctagctgag gaagaatctg agttcccttc
3361 tacttctatc tctgcagttc tgtctgactt agctgacttg agaagctgtg atggccaagc
3421 tttgccctcc caggaccctg aggttgcttt atctctcagt tgtggccatt ccagaggact
3481 ctttagtcat atgcagcaac atgacatttt agataccctg tgtaggacca ttgaatctac
3541 aatccatgtc gtcacaagga tatctggcaa aggaaaccaa gctgcttctt gacattaggt
3601 gtagcatgtc tacttttaag tccctcaccc ccaaccccca tgctgtttgt ataagttttg
3661 cttatttgtt tttgtgcttc agtttgtcca gtgctctctg cttgaatggc aagatagatt
3721 tataggctta attcttggtc aggcagaact ccagatgaaa aaaacttgca tcttcagtat
3781 acttcctaaa gggcaatcag ataatggata tgttttatgt aattaagagt tcactttagt
3841 ggctttcatt taatatggct gtctgggaag aacaggttgg cctagccctg tacaatgtaa
3901 tttaaactta cagcattttt actgtgtatg atatggtgtc ctctgtgcca gttttgtacc
3961 ttatagaggc agattgcctc cgatcgctgt ggttcttatt atcaaaatta agtttacttg
4021 tatacggaac aaccacaaga aatttgattc tgtaaagaat cctctttagc tgtggcctgg
4081 cagtatataa atggtgcttt atttaacaga atacctgtgg aggaaataaa gcacacttga
4141 tgt//

- LOCUS: N71116 472 bp mRNA linear EST 14-MAR-1996
- DEFINITION: za87h11.s1 Soares_fetal_lung_NbHL19W Homo sapiens cDNA clone IMAGE:299589 3', mRNA sequence. *phospholipase A2, group IVB (cytosolic) (PLA2G4B)*
- ACCESSION: N71116
- VERSION: N71116.1 GI:1227696
- KEYWORDS: EST.

1 gtatgagtct gtgatgtatc aagtgctcca actactcaag gtagcgcaga agggaaaaca
61 ggcacaggcc ggcggggttt tgggtgatta cacaaatggg cttggctcct taccccactg
121 caaactgctg aggcgcaagg gagctcccag ccctcagcct ggaccctggg acagtgccac
181 ctgagcccga ggtctggaag cactgcgtga tgacagttcc cacctgcaac tcagcagcca
241 gggaatgaat gagagttagg ggtggcaggg gcccggcatc aagtggggcc tgcgctgccg
301 cctccgctgc actgcctggc gcagagctcc agcagctgct cctggttgtt gcagacattg
361 taatgtgtca ggtgcagcag ttgtccacgt cctcctggct gtaggtcacc ttcgtgtagt
421 ggtagggaga gtccgatgaa gacaggttca nctccccagc tgccgcctcc ta//

- LOCUS: AK026273 1835 bp mRNA linear PRI 12-SEP-2006
- DEFINITION: Homo sapiens cDNA: FLJ22620 fis, clone HSI05629. *FK506 binding protein 1B (FKBP1B)*
- ACCESSION: AK026273
- VERSION: AK026273.1 GI:10439077
- KEYWORDS: FLI_CDNA; oligo capping; fis (full insert sequence).

1 agcatcatag atgctcctac actaagctgg ccctgcttcc tatgttaaat atgacacatc
61 tgaggccctg ggagaggaag tgatttgccc agtctcacac aatgagttag agccagagtg
121 aagtcaaaac ccagtctctg gatgtacaag caaggtcttt ttctagtccc aaatggcctt
181 ttgtggtggt ccagggactg ccgggagcag tcgtggaact gcatcattta cagaaggtct
241 gatctttgag tcagagtcac agaagaattg agaatagctg ttgggccttg ggctgctgga
301 ctgagatgac atgtggacat caggatgaca aggcttctga agcagaggct gggggaacca
361 gaactgctgg accagcacct gggggggggt tctggtttca aacagccctc ttagtacaga
421 gtctggaggc cacctctgac cagctaggtc accttacact gttccctaca gtaggggata
481 atgtaacctt tgggttttcc cttttggggg aaacttagga aaccttagga accttatgcg
541 aggactcaac ctgaggctgg aatagctggg aaaacagaac tttaggtgga ggaagtggca
601 taaaaaagtt gaaaagtctg gctgggcatg atagctcaca cctgtaatcc cagcactttg
661 ggaggctgag gtgggcagat cacctgaggt caggagttcg agaccagcct ggccaacata

721 gcaaaacccc atctctacta aaaatataaa aaaattagca ggcatagtgg caggtacctg
781 tagtcccagc tactcaggaa gctgaggcag gagaatccct tgaacccggg aggcggaggt
841 tgcagtgaac cgagatcaca ccactgcact ccagcctggg caacagaggg agactccgac
901 tcaaaaaaaa aaaaaaaaaa aaaaaagttg aaaagtgcag aggaatttga gaaaggtatg
961 acctgaaaag gacacagagg gaagggaagg aaggggagaa cagagagggt tataagcagg
1021 ccagagccgg attgtgaagt gcctgtatgc aaggtaggca tttggattct gctctgggga
1081 gctattgaag tattttgagc agagattgtg ccactgtact ccagcccagg cgatggagtg
1141 agactgtctc aaaaaaacaa aacaaaaaaa taaactagct gaggtctacc agttgcctgg
1201 gactgaggta gtggaagagt tggggtggag taggccaaga aaatttttcg ttttttcgct
1261 tttttgaaaa gaatttcaga attacaaaaa tattaaagaa atagtacaga gttcttctat
1321 attctatcca gcttcccctt cttgcataac catagtacaa ttatcaaaac caggacattc
1381 acactgatac aaattactta cttattcaaa tttttgatta ttcaaaattc aaaagtagat
1441 ttaaatttca ccaattgtcc cactaatgct gagtgggttc tcaacaactg ctgtgatgag
1501 ccctggttca agctgttctt tcttggtctg tctgtgaagt cctatgatgc tcccttacga
1561 taggtcttgt ttttggtgtt tttttttaga aagagtatca ctctgtcacc aaggctggac
1621 tgcagtggcg caatcttggc tcactgcaag ctccgcgtcc tgggttcacg ccattcttct
1681 gcctcagcct cccagctact caggaagctg aggcaggaga atcccttgaa cccgggaggc
1741 ggaggttgca gtgaaccgag atcacaccac tgcactccag cctgggcaac agagggagac
1801 tccgactcaa aaaaaaaaaa aaaaaaaaaa aaaaa//

- LOCUS: AL581473 986 bp mRNA linear EST 07-APR-2004
- DEFINITION: AL581473 Homo sapiens B CELLS (RAMOS CELL LINE) Homo sapiens cDNA clone CS0DG004YK09 3-PRIME, mRNA sequence. *exosome component* 7 *(EXOSC7)*
- ACCESSION: AL581473
- VERSION: AL581473.3 GI:46260049
- KEYWORDS: EST.

1 tttawtttta catttacaaa tgctgctgta aattcgtaac agcnaagaaa aatatatacg
61 aaccggttta aaaggaaaag taaaaaacaa tccacagttg agcakttgwt ktgcaaatca
121 tcccaggaat ccaaytttct ktctcttggg ccccaggctt tcttccttgt gcagaacact
181 ctgcaagbmg gcatgcagta ccttgcccac acgcttgcca ktctccatca tctcgaagat
241 gctctctggg tccaggctgc ccttccccac tttcctcatg cacgtcacaa ctcccttgct
301 ggtcaccgac accagcaagc tggccagcga gcaggcctcc tcctgaagag tagcatccac
361 cacatgccga tagccaatct tgcacagagt gacaatgcag gggacattct ccacacttak
421 tcgtatgcag tcataagggt catctgacaa ttcaatgtcc ttcgacccct cttcatcctc
481 caaaactcga acccttggta tccttgtatt gaagagagca gcctttacag caatggaaat
541 ggcatcaaac aaatttccac cacattccag aagcagcaca tccacataga gaacccagca
601 gtgctcccga ggactaatgc agagggtctt taagtcgaca ctgcttttat tgttaaatat
661 ccgatagagg gtgttagcga tctcggtgcc aaggtctatc acctcctcta ccttcaaatt
721 caggggtagc actggctgaa cagtcaacaa agaactccaa gtagccttca tttggtttct
781 ccagcttcgg cgtccccatt tctgctttca ctcccaccaa gatgtctgtg tgacccagct
841 tgaccctggc ggacccacta gtgttggaca ccacatcagt ttccacttcg acacatcggt
901 agtcctcaca gccacggcca tccacacggg aggtcttcct ggacgccatg cacgatgtac
961 acttctccgc ctcgctcagc gtcacg//

- LOCUS: AF073483 1517 bp mRNA linear PRI 02-JAN-2001
- DEFINITION: Homo sapiens p5326 mRNA, complete cds. *basophilic leukaemia expressed protein BLES03 (Bles03)*
- ACCESSION: AF073483
- VERSION: AF073483.1 GI:12002057

1 acggcaggag cggagccggg cccggggctg ggccggcgtc gaacgcagcg aaggccggag
61 gatggaacca ggtgaggagc tggaagagga gggctctcca ggtggccgtg aggatggctt
121 caccgccgag cacctggctg cagaggccat ggcagctgac atggacccct ggctagtgtt
181 tgatgcccgc acaacgcctg ccactgagct ggatgcctgg ctggccaagt acccaccatc
241 ccaagttacc cgctatgggg accccggttc acccaactca gagcctgtgg gctggattgc
301 agtgtatggg cagggctaca gccccaactc cggggacgtg cagggcctgc aggcagcctg
361 ggaagctctg cagaccagtg ggcggcccat cacaccgggt accctgcgcc agctcgccat
421 cacccaccac gtgctctcgg gcaagtggct tatgcatctg gcaccgggct tcaagctgga
481 ccacgcctgg gctggcattg cccgggccgt ggttgaaggc cggcttcagg tggccaaggt
541 gagcccacgt gccaaggagg gtgggcgcca ggtcatctgt gtttacacgg acgacttcac
601 ggaccgcttg ggtgtactgg aggcggattc agccatccgt gcagcgggca ttaagtgcct
661 gctcacctac aagcctgatg tctacaccta cctgggcatc taccgggcca atcgctggca

721 cctctgcccc actctctatg agagccgttt ccagcttggg ggtagtgccc gtggctcccg
781 agtgcttgac cgtgccaaca acgtggaact gacctagagg ggccaaattg ggagactgc
841 ccactccccc ctccatgctg gggttggatc ctcctgtctt cctccttgtc ccatgaaggc
901 cacacccccc agctctgggg actcctaggt cacttgggaa ctacctgcat cttcagtccc
961 cttgaacttc tgccctctgt tcagggctga cacaagcccc acaggctggg gggctccggt
1021 tccctgaggg atgagccttc agcctccctt tgtaatgctg ctcctctcca ctgcccagca
1081 ccatgagttg ggtgcagaca cctagaagga gagacttctt ggaacgctca tcccccgcta
1141 tacctcccct tcctcctgca tctccccttc tttccttccc cctcaggaga gagaaaactt
1201 agtgcttcca gcccttcttg gagccttcat ggtccagggg taggggcccc actggcctga
1261 gcatgccatt ttgaggggag ggtagttgtg cctacttatc ccctggcaga ggggatgcca
1321 ggaccatgga catgaggctt gcccatccct gccaacttac acagcctgta ccactgtccc
1381 cccttccttg gctactttga catgtgcctg ctcctggcat ttcaataaaa cccggcttgg
1441 gtctgaaaaa aaaaaaaaaa aaaaaaaaaa aaaaaaaaaa aaaaaaaaaa aaaaaaaaaa
1501 aaaaaaaaaa aaaaaaa//

- LOCUS: AK024944 2357 bp mRNA linear PRI 12-SEP-2006
- DEFINITION: Homo sapiens cDNA: FLJ21291 fis, clone COL01963. *homolog (MED28) mediator of RNA polymerase II transcription, subunit 28*
- ACCESSION: AK024944
- VERSION: AK024944.1 GI:10437366
- KEYWORDS: FLI_CDNA; oligo capping; fis (full insert sequence).

1 ttttaccaac aaatttagaa ttatttcaag ttgctttatg ttaaaatata taacaagtta
61 cattttcttg ctgcattaat aagtttttga tttttaaaat ctgatatttc agcaactaaa
121 ggaatacttt tctttatagg tctgttctgt ttttaacctc attgactgta tatgttttat
181 ttacccgtgc caggagtcta gaatcgtgct taggtgccat tcttaggtat gaaaacaagt
241 ttggagacca atgcaggtct ctgtgcagaa atctggggct ctaaatgaat taccaaacca
301 ataggaaata aaatactact catgatgtag tgtgtcatct gaagctatga tagaatatat
361 tatttctaaa atagtattga gaagttaatt gttcagtcca tagtagtgta agtgaagtgt
421 ggaggtcagc catagttgtc tttcttcagc aggtactcta accagagact gggttctgtg
481 gaaaatagaa ttatattctt ggtttgggat caacaagtgt tttgttattg ttttaatgtt
541 gctggcattg cctgaaacaa agtgccctgt tattcctctt atgtcatcag aactctgctt
601 actgaagttg aagtttaatc ttaggatcta gaattcttgt ggttggtcgt gtattaagaa
661 aagaaggtgg tttgatttta tttcttgtac ttggtggaac ctgttactat acaagtcatg
721 ttttgacctg taagccgagc aaatctgaaa ggtgaatcgt taatggtagt ataattgttt
781 catttgaaac tgtcagtttc ttcaagtcac ttctctgaaa aaaattatga tagattgata
841 gcctgccaga aaatagcaat ttctaaggtg ctgccatcca cacttactta ttgtgaaata
901 tgaaaatcca gagtggtttg tatgagaggg gaaatactgg ctctaagctt tctacagaaa
961 acaggaaatt catgctgttg ttgacataat ttttaaggaa acttttaaca agaaaattca
1021 tgagggatac aatcaaacat agttaatctc ttgagtgcta atcatttgag tatagtccac
1081 atcaggaagt tttatgaggt tgaaaacttt actttgaaat atttaattgg gagactagac
1141 catttctttt tccaagtcat tctgatttat tgtcctagta aatgtatgtg tatgcatata
1201 tactgatgta ttgactgaat gtgtgtgtgt gtgtgtgcgc gtgtaaatct gttttcatag
1261 ggaacataca ttgatggctc ctagtctatt ttatactaaa acttaaaaaa atttgctgca
1321 ctttgcagtt gccacttttt aaaagattaa tgctttgcac atttcagtag gccttaaaga
1381 tgaattgctc accaacattt tagaaataca ctgcttacat gtgacatact ctaaacaaag
1441 tcatagtaaa ctgttctctt aggttcatgt attattgagc aaataaggat gtcctttctt
1501 aaaatttcta catttatgtt tgcttttgtg taaaatcttt attagtttgt aagtttctca
1561 aagaggaggt ttatactgga agcagtattt ctaaagtaca gagattcgac catggattga
1621 atacagtagt ggaggaatgc tggtattgtg gcctaattta tccttttaaa ctgtatgtca
1681 ttagtattag ccaaaacctc agagtggagt gtcttgggct gaatgatctt ctcaactttg
1741 ccttgtttcc catctcataa aggctccttc tggttctttt cccttccaag gattgtgagt
1801 catgctcaca taggcctggg atcttctgga tggggtcggg gaggaggtgg gggtttcctg
1861 tggctggccc agagttctag ccaatggaat cattcaagaa tgcctaaagt tcaggggtgg
1921 gaatgccata tattggtgtt aatcacttgg cactttcttg ggcaccaata ttagcactgt
1981 ttcattttta ataactggaa gcacttaatg attattttgt ttgtttaatt tcaacttaag
2041 gatattgagg gtcagagaag taaggtttat ttcttgttta tgttttctgg aatttatagg
2101 gtgtttgtag aagggataaa tgggttacct aatgagccat tcttctaggc tctgtaaggt
2161 atgccctttt ggctaggata ttttaaaata atttgcaagc ttttaatatt gtgctttgta
2221 gaaatttacg atttttaaaa ataatttgca attgatagat tacacttaaa tttataaagg
2281 atccaatttt aagtttcaaa aaaaaaaaaa aaaaaaaaaa aaaaaaaaaa aaaaaaaaaa
2341 aaaaaaaaaa aaaaaaa//

- LOCUS: NM_018234 3938 bp mRNA linear PRI 18-NOV-2006
- DEFINITION: Homo sapiens STEAP family member 3 (STEAP3), transcript variant 2,mRNA. *dudulin 2 (TSAP6)*
- ACCESSION: NM_018234
- VERSION: NM_018234.2 GI:59853424

1 gaggaggagc ctcgggccga gccaccgcct tcgccgcgga ccttcagctg ccgcggtcgc
61 tccgagcggc gggccgcaga ggttcaagcg attctcctgc ttcagcctcc ggagtagctg
121 ggattacagg cacgtgccaa cacacccagc caccaaaatg ccagaagaga tggacaagcc
181 actgatcagc ctccacctgg tggacagcga tagtagcctt gccaaggtcc ccgatgaggc
241 ccccaaagtg ggcatcctgg gtagcgggga ctttgcccgc tccctggcca cacgcctggt
301 gggctctggc ttcaaagtgg tggtggggag ccgcaacccc aaacgcacag ccaggctgtt
361 tccctcagcg gcccaagtga ctttccaaga ggaggcagtg agctccccgg aggtcatctt
421 tgtggctgtg ttccgggagc actactcttc actgtgcagt ctcagtgacc agctggcggg
481 caagatcctg gtggatgtga gcaaccctac agagcaagag caccttcagc atcgtgagtc
541 caatgctgag tacctggcct ccctcttccc cacttgcaca gtggtcaagg ccttcaatgt
601 catctctgcc tggaccctgc aggctggccc aagggatggt aacaggcagg tgcccatctg
661 cggtgaccag ccagaagcca agcgtgctgt ctcggagatg gcgctcgcca tgggcttcat
721 gcccgtggac atgggatccc tggcgtcagc ctgggaggtg gaggccatgc ccctgcgcct
781 cctcccggcc tggaaggtgc ccaccctgct ggccctgggg ctcttcgtct gcttctatgc
841 ctacaacttc gtccgggacg ttctgcagcc ctatgtgcag gaaagccaga acaagttctt
901 caagctgccc gtgtccgtgg tcaacaccac actgccgtgc gtggcctacg tgctgctgtc
961 actcgtgtac ttgcccggcg tgctggcggc tgccctgcag ctgcggcgcg gcaccaagta
1021 ccagcgcttc cccgactggc tggaccactg gctacagcac cgcaagcaga tcgggctgct
1081 cagcttcttc tgcgccgccc tgcacgccct ctacagcttc tgcttgccgc tgcgccgcgc
1141 ccaccgctac gacctggtca acctggcagt caagcaggtc ttggccaaca agagccacct
1201 ctgggtggag gaggaggtct ggcggatgga gatctacctc tccctgggag tgctggccct
1261 cggcacgttg tccctgctgg ccgtgacctc actgccgtcc attgcaaact cgctcaactg
1321 gagggagttc agcttcgttc agtcctcact gggctttgtg gccctcgtgc tgagcacact
1381 gcacacgctc acctacggct ggacccgcgc cttcgaggag agccgctaca agttctacct
1441 gcctcccacc ttcacgctca cgctgctggt gccctgcgtc gtcatcctgg ccaaagccct
1501 gtttctcctg ccctgcatca gccgcagact cgccaggatc cggagaggct gggagaggga
1561 gagcaccatc aagttcacgc tgcccacaga ccacgccctg gccgagaaga cgagccacgt
1621 atgaggtgcc tgccctgggc tctggacccc gggcacacga gggacggtgc cctgagcccg
1681 ttaggttttc ttttcttggt ggtgcaaagt ggtataactg tgtgcaaata ggaggtttga
1741 ggtccaaatt cctgggactc aaatgtatgc agtactattc agaatgatat acacacatat
1801 gtgtatatgt atttacatat attccacata tataacagga tttgcaatta tacatagcta
1861 gctaaaaagt tgggtctctg agatttcaac ttgtagattt aaaaacaagt gccgtacgtt
1921 aagagaagag cagatcatgc tattgtgaca tttgcagaga tatacacaca ctttttgtac
1981 agaagaggct tgtgctgtgg tgggttcgat ttatccctgc ccaccccacc cccacaactt
2041 cccttttgct acttccccaa ggctcttgca gagctagggc tctgaagggg agggaaggca
2101 acggctctgc ccagagccat ccctggagca tgtgagcagc ggctggtctc ttccctccac
2161 ctggggcagc agcaggaggc ctggggagga ggaaaatcag gcagtcggcc tggagtctgt
2221 gcctggtcct ttgcccggtg gtgggaggat ggagggattg ggctgaagct gctccacctc
2281 atccttgctg agtgggggag acattttccc tgaaagtcag aagtcaccat agagcctgca
2341 aatggatcct cctgtgagag tgacgtcacc tcctttccag agccattagt gagcctggct
2401 tgggaacaag tgtaatttcc ttccctcctt taacctggcg atgagcgtcc tttaaaccac
2461 tgtgccttct caccctttcc atcttcagtt tgaatgactc ccaggaaggc ctagagcaga
2521 ccctttagaa atcagcccaa gggggagagc aagagaaaac actctaggga gtaaagctcc
2581 ccgggcgtca gagttgagcc ctgcctgggc tgaaggactg tcttcacgaa gtcagtcctg
2641 aggaaaaata ttggggactc caaatgtcct ctggcagagg acccagaaaa ccacactggc
2701 tccaacttcc tcctcatggg gcattacact tcaaaacagt ggggagcaac ttttccacca
2761 aagctacaaa cctaaaatgc tgctgcccca aagcacaaga gggaagagca ccgccggggc
2821 cacaggacgt ctgtcctcca gtcacaggcc atccttgctg ctccctactg actctagctt
2881 acttcccctg tgaagaaaca ggtgttctcg gctgagcccc caaccctctg cagaaccagg
2941 ttgatctgcc acagaaaaag catctttgaa gacaaagagg gtgaggtctt catgagtctc
3001 ctgggcccaa agccatcttc tgatggaagg aagagagtag ggccagtgaa ggctgcccag
3061 agagaatgtc acagatgagg ctgcccctgc cccccccccg ccagggaggt ttcatgagct
3121 catgtctatg cagcacataa gggttcttca gtgaaaagca ggagaagagc ccactgcaag
3181 gatagctcat taggcacatg accgatgcag ggaaggccat gccggggaag ctcttcctgc
3241 aggtattttc catctgctgt gccaaggctg agcggcagaa acttgtctca taaattggca

3301 ctgatggagc atcagctgtg gcccacagag agccttgctg agaagggggc aggtaaagca
3361 gagattttag cattgccttg gcataacaag ggcccatcga ttccctacta atgagaggca
3421 gggagagcat gggcaatgga gacccaccaa tgatccccaa ccccggtggg tactggctgc
3481 ctgccctggg ccagggaatg gctccttata ccaaagatgc tggcacatag cagaacccag
3541 tgcacgtcct ccccttccca cccacctctg gctgaaggtg ctcaagaggg aagcaattat
3601 aaggtgggtg gcaggaggga acaggtgcca cctgctggac aatcacacga aaggcaggcg
3661 ggctgtgtac tgggccctga ctgtgcgtcc actgctgtct tccctacctc accaggctac
3721 tggcagcagc atcccgagag cacatcatct ccacagcctg gtaaattcca tgtgcctctg
3781 ggtacaaaag tgcctcaacg acatgctctg gaaatcccaa atgccacagt ctgaggttga
3841 tatctaaaat ctatgccttc aaaagagtct ctgttttttt tttttaacct ggtagacagt
3901 ataaaagcag tgcaaataaa cacctaacct tctgcaaa//

- LOCUS: H61826 477 bp mRNA linear EST 06-OCT-1995
- DEFINITION: yu41g09.r1 Soares ovary tumor NbHOT Homo sapiens cDNA clone IMAGE:236416 5', mRNA sequence. *Tularik gene 1 (T1)*
- ACCESSION: H61826
- VERSION: H61826.1 GI:1014658
- KEYWORDS: EST.

1 cctcnngcta ncgtggtgtg ccttggaggc ggagacgcca ggcccgccta cttccgtccc
61 tctttctgca gggccagagg tgaccctgcc tggcctccca caccccctgc aatgagcaag
121 gccttcactg cagccccatc tcctcctcct cccccagacc cctcccagcc ctctcctcct
181 gttcctcctg tagcatcttt gctgggctac gcagaagccc cgggacatgg cagccccacc
241 ccatgccacg ncccttccta cactgttccc tggaccatac acaggctgaa gcagaggaaa
301 tcccaaagcg ggtgcccatc cagcccaggt nccagggatc cctgncaccc atttctgtga
361 actgggggcc ccagccgtgg tgtggttgnt tcattccnag cagagggaac ttnccttgtt
421 ccagcgaatt tnccttttgg ccattagaaa gaatggtgag catgagattg ggaaca//

- LOCUS: N64803 490 bp mRNA linear EST 30-JAN-1997
- DEFINITION: yz31b07.s1 Soares_multiple_sclerosis_2NbHMSP Homo sapiens cDNA clone IMAGE:284629 3', mRNA sequence. *trinucleotide repeat containing 6B (TNRC6B)*
- ACCESSION: N64803
- VERSION: N64803.1 GI:1212632
- KEYWORDS: EST.

1 aattttttta atttttttgt gtttaaattt aaatggtatg tgtacttgct tcacattgtc
61 tttctaggtt ggcgttcatg attcaagtat ttcaagagtg atatgttctc tttttggatt
121 catattccaa acgaaaaaac tgaagttata agggaggcaa ctatgtgctc agacagtctg
181 tcaatgaccc accttttttt ctctccgttt tcttttttcc ttttaaaaat gtatttattg
241 caagttgaaa aaaaaaacga aaaggtcaag ttaagtgctg ctgctgttcc aaaaaaggtc
301 acgaggtcag agttgaaagt tctaagttca gattgaatcc gaccctcctc ccagaaggtc
361 accaggtagt aaaggagcan ggctggccca tcctatgggg atctttccac cggttgggga
421 ctcccccata aagccagaag aanagtttgg ggggncccca caaatgaagc cnccccaaga
481 ttcgggcccc//

- LOCUS: NM_016458 2441 bp mRNA linear PRI 17-NOV-2006
- DEFINITION: Homo sapiens chromosome 8 open reading frame 30A (C8orf30A), mRNA. *brain protein 16 ( LOC51236)*
- ACCESSION: NM_016458 XM_937693
- VERSION: NM_016458.2 GI:13124772

1 gggccacaca gcggacccct aagcggaccg ctggcaccgg tcgggtggca gcagagtgtc
61 gctcgacatg ggggaggccg gggctggcgc tggcgcctcg ggagggccgg aggcaagccc
121 ggaggcagag gtggtgaagc tgctgccctt cctggcgccg ggcgcgcggg cggacctgca
181 ggcggcggcg gtgcggcacg tgctggcgct gactggctgc ggacccggcc gcgcgctgtt
241 ggcggggcag gcggcgctgc tgcaggcgct gatggagctg gcgccggcct ctgccccggc
301 ccgggacgcc gcccgcgcgc tcgtgaactt ggccgccgac cccggcctgc acgagacatt
361 gctggcggcc gaccccgggc tgccagcgcg cctgatgggc cgcgcgttgg acccgcagtg
421 gccctgggcc gaggaggcgg ccgccgcgct agccaacctc agtcgcgagc cggcgccgtg
481 tgcagcgctc atggcggcgc tggcggccgc ggagccggca gactcgggcc tggagcggct
541 ggtgcgcgcg ctgtgcacgc ccggctacaa cgcccgcgcg cccctgcact acctagcgcc
601 gctgctctcc aacctcagcc aacgccctgc ggcgcgggcc ttcctactgg accccgacag

661 gtgcgtggtc cagcggctgc tgccccttac ccagtacccc gactcctctg tacgcagggg
721 cggggtggtg gggacgctgc ggaattgctg cttcgagcac cgacatcacg agtggttgct
781 tggacctgag gtggacattc tccccttttt gctcctgccc ttggctgggc ctgaggattt
841 ctccgaggaa gagatggaac ggctgcctgt cgacttgcag tacctgccac cagacaagca
901 gcgagaacct gatgcagaca tccgcaagat gcttgttgaa gccatcatgc tgctgacagc
961 cacagcacca ggtcggcagc aggtgcggga ccagggagcc tacctgatcc ttcgagagct
1021 gcacagctgg gagccggagc ccgacgtgcg gacggcttgt gagaagctca tccaggtgct
1081 tattggggac gagcctgaac gtggcatgga aaacctgctg gaggtgcagg tgcctgagga
1141 tgtggagcag cagctgcagc agctggattg cagggagcag gagcagttgg agcgggagct
1201 ggccccagag ccatgggtgg agagggccac acccacctga ggcccctgca gccggacacc
1261 agctccaggg tcccctttgc caggctttct cagaccaggc cttcctgcag ctgtctgcag
1321 gccccctggg tccccttgtt cagagactgt gctcttctga gaagcagagc tacgcttagg
1381 ctccagtaag gattggaggc actttccagc cctgtgcagt gttgctacca gcaagaatga
1441 aggttgtgca gagcagtact gtgaggtagg caccgtgact cggctgcctg tgcagaaggt
1501 ggaggtggca ggcggatggc cagggagcca tgagaagact cttcctgggc acggcgtggg
1561 gactggacag aaaaccactg caagccccaa ggagccagct gaagcctccc aatccccagg
1621 gcactgcaga gtctgggcag aggtgggcgc ataggtacaa gtgctgcttt gtcagcctca
1681 ctccctgcag ctctcagctc cctgttccct gagacctggc ctcaaagggc tgacccagcc
1741 atacgtagat cctgggtct ttctaggact gtgcctccct gcttgaccta tgctccagga
1801 ccctgccctc aacccttgtc agctccctaa gtcttcctgt ttcccagtct cagattgagt
1861 gggtgctctc attggcctct ctagctaggc cgtctccctg gtcagcttcc ttgagcctgg
1921 gccactcctc cctggccttc ttacctctgc caagagcctc cagatgtgcc tgcagcctag
1981 tccctgccca agcttctccc ccagccaccc tctgcccctg tggtagagcc cactataggc
2041 ccaggcgcct tacgtgcttc tcccctggac ctatgtgcat ccctcacctg ccagttatgt
2101 ggctgccaag ccccacaggc tgtcatggtg gctcttgcca cacttccctt cctgagctga
2161 cctgacctca ctgccacctc ttgcattggg atcctgaggg ctggcccctg ctcactgatg
2221 ggccctccca ggcccgtcaa gccctggtga aggagcttag cttgtcttct tggcaattca
2281 caatgcctca ctccacaccc taaaactgga gcgtccccta tggtctcctg ctgtggcttg
2341 ggtgccgggc gtctcccgcc tccaggatct cctctcacct gcccccatta aagctctgcc
2401 actcaaaaaa aaaaaaaaaa aaaaaaaaaa aaaaaaaaaa c//

- LOCUS: BF055496 694 bp mRNA linear EST 16-OCT-2000
- DEFINITION: 7j80h12.x1 Soares_NSF_F8_9W_OT_PA_P_S1 Homo sapiens cDNA clone IMAGE:3392807 3' similar to TR:Q9Y3W8 Q9Y3W8 HYPOTHETICAL 71.4 KD PROTEIN ;, mRNA sequence. *DKFZP586J0619 protein (DKFZP586J0619)*
- ACCESSION: BF055496
- VERSION: BF055496.1 GI:1.0809392
- KEYWORDS: EST.

1 tttttttttt tttgacagtt caggtcgttt cattcaaaac cagcccaggc catgcccggg
61 gggatccgcc gctccgcggc agggctgtgg cccaggtgaa tgagggcttg ctggacggcc
121 cctgatgcca gcggccggcc cggagccacc ccagggctcg gagtattgct cctgggcctg
181 cctggcctca gggctcggcg ccctcacctc ctcggacaga ccagcaacgc ccacgcttcc
241 tgggctttgc ctcgaggatc cccggggacg ggacgggccg gggcttggag gggggtcggc
301 tgccacaggc tcacatcacg gcctccatat gcaggatcct cagggcctcg gagatctgcg
361 cgctggggtc catctggccg tacatgccca ccaggaaggc ccggtggagc agcacagccg
421 cgtgctcttg gcacaggaga gcgtactcag gcaggttccg gagggccgtc tgcaccacct
481 taaagtcctt gctgcccaag agtacatgaa cgtgggcagg aaagcggctg caattgctgg
541 gctgttctgc atggagcgca gggccaggct tgaaggcggg ttgcggcaac ccttcttggg
601 ccaactcatc aaccccttgg aggtgggtcg gaaagaagct tagaatctcg gggctcgcgc
661 gggacaatct ngctttggcc atcagaacct ccac//

- LOCUS: AK001854 2077 bp mRNA linear PRI 12-SEP-2006
- DEFINITION: Homo sapiens cDNA FLJ10992 fis, clone PLACE1002073, weakly similar to ADENYLATE CYCLASE (EC 4.6.1.1). *KIAA0931 protein (PHLPPL)*
- ACCESSION: AK001854
- VERSION: AK001854.1 GI:7023379
- KEYWORDS: FLI_CDNA; oligo capping; fis (full insert sequence).

1 ctgtcctggt agggcacctg cacctgcgaa tcttgcacct tgcaaacaat cagttacaga
61 cctttcctgc aagcaaacta aataaattgg agcaattgga ggaactgaac ctaagtggca
121 acaagcttaa aaccattccc acaaccatag caaactgtaa aaggctgcac acccttgttg

181 cacactccaa caacatcagc attttcccag aaatactgca gttgcctcag atccagtttg
241 tagacctaag ttgcaacgac ttgacagaaa tcctgattcc agaggctttg cctgctacat
301 tacaagacct tgacctgact ggaaatacaa atctggttct ggaacacaag acactggaca
361 tatttagcca tatcacaacc ctgaaaattg atcagaaacc tttgccaacc acagattcta
421 cagttacgtc aaccttctgg agccatggac tggctgagat ggcagggcag agaaataagc
481 tgtgtgtctc agcacttgct atggatagct ttgcagaggg ggtgggagct gtgtatggca
541 tgtttgatgg agaccgaaat gaggagctcc cgcgcctgct gcagtgtacg atggcagatg
601 tgcttttaga agaggtacag cagtcaacta atgacacagt tttcatggct aacaccttct
661 tggtatctca caggaaatta ggaatggctg gccagaagtt gggctcctcc gctctcctgt
721 gctacatccg ccctgacact gccgatccag caagtagctt tagcttgact gtaggcaatg
781 ttggcacgtg ccaagcagtc ctgtgccgag gtgggaagcc agtgcccctc tctaaagtct
841 tcagcctgga gcaggaccca gaggaggctc aaagggtgaa ggaccaaaaa gccatcatca
901 cagagaattg ggttcttaac caaaagcacc tgcaggattt tcctggggaa gataagtgag
961 aacaaacaca tgaaaccaca ttccgtgaca ctgcaaagct gtgagttaag gttccggaag
1021 atctcaaaga gttccagacc acagatattc cggtgcatca ctgtaacttc ccggctgttc
1081 tcggctgcct ttgtagcgct tctgttctcg gtagctacgc agggccagca ccacgctggc
1141 gccgtacatg atcacgagaa gacaagcaaa ggtggctgct gctccgtcgg tgcctgccag
1201 ctggcagtcc atccaggtga gacccttgcg ggcatagagc ctctctcttg ttttgcaagt
1261 gtcggtgcta ttgatctgca gagccacgtg gaggtaaaca ccaatgcctg tgcagtagcc
1321 cactgccgct aggaggctga aggcggcctc cgtgaggagc cacttccctg acaacattgt
1381 tcgactcttg gctccttgga gtaaaacacc catggtgagg acccccagac ccagagaaac
1441 agccacgcca ccgtatatca ggggcgagcg gaggatcgtg tactgctggt ccagctgccg
1501 aacctgctct agctcagtgc cctcgaacga tgttttcaga tcagttccta caagaggagc
1561 cacattttgt taagtggcct tcgacttcat tgtatggatg gatttgtcta ttcaaccagg
1621 accttactga tagatatttg gactgaaagg aacccacaaa tggcacgagg cagaggaagc
1681 caacaggcca acagactgaa gatcttcttg tccgaggtca gcttttattt gttactgaca
1741 gaggccccaa atccttctta ggtgtccttc tgggagaggg tctcctgcca ttcccgtggt
1801 gttcagggat gagcatgaag agaaatacaa atgaaccgag agcaggacca ccaggttcaa
1861 gtccccgctt tttactgcct tgtcatcttg gccaagccat tcagtccttc taagcctcag
1921 acaatatgac aaatgaagct aagtctgcca atcttcaccc acagggatgt ggggaggctc
1981 aaatgaaagc aaccgtgtga tactgtggga agttaacaac aacaacaaaa aagcaagtat
2041 gtgaaaatac tctaaaaata aaataatgta caaatgg//

- LOCUS: AL137303 4434 bp mRNA linear HTC 22-SEP-2004
- DEFINITION: Homo sapiens mRNA; cDNA DKFZp4340086 (from clone DKFZp4340086). *KIAA1055 protein (KIAA1055)*
- ACCESSION: AL137303
- VERSION: AL137303.1 GI:6807767
- KEYWORDS: HTC.

1 ggtgttttga aacataaaag gttgcaccct cgtgtgtgcc ttgtgttaaa aggagcccac
61 gcagccgacg gcagcaggag caaatggcct gctctgatgg ggacaggcag gaagtccttc
121 ggggcatttc tgtttcatca cagcttttcc tggggctggt gagtctgttg ggcctttggt
181 tgacagggaa gcgcatgagc ccatgttggt ggatacaaag catttggggc ctggcagaat
241 ccagagaggg gaagaaggtg gccctaggaa ctggcctatt ttgtgatccc cagagaccag
301 tgtggctgac ccattagtgc tacaaatcca tagccaggcc tcaggacagc ttttggtctt
361 gtttttatta agccactgac cagtggttag gagcaagtgg atcataacct taacattttt
421 attgaaaatt ggagccttga cctggggatg tgaaacccac aagctgcaca tttctctgta
481 gactgcatct ggacacttgt cacattgccg actgggtctg acagtcatcg ccaggactgc
541 ctgggcctgt gttcattccc agtcaatgag tcctgggaca cagacgtccc ggactcgtgc
601 ccggtgccag gcctcatgcc cgcccaagga tgaagacata cctgccatca ccatgcaagt
661 gccgggagct cttctcagcg cttatgtgtg ggaacacgag taatcccaca ccctaggagg
721 tggcactact cttaccccag tgtgcagatg aagatcagag gccagaaaag gggggtgact
781 tcagtcacac agcaagcatg tggcagagct ggagtccagg tcgtcattct gagcctgccc
841 ctggctgggc cattgtgctt tgtcacctct ctacagaagg tgggcctcat cccctacatc
901 aggccccaag cctggccagc gctcttccca ccctgcccca ggccacctgc tatgcctgtg
961 agggctgtgt gtgagcactc tctggagcgc cgctgattct cttgtctctc ccctgcgtcc
1021 tgccaggaag ctgatcagta tctcctttgg ggacctgaac cctttccccc tacgccagat
1081 ccggaaccga cgcgcctacc acttggagaa agtccggctg gagctgaccg agctggaggc
1141 catccgtgag gacttcctgc gtgagcggga caccagccct gacaagggtg agctggtcag
1201 tgacgaggag gaggatacct gagtgggctc gggatgctgc tcttccttcc ttcacaacca
1261 aagtgtgccc aaagggtgaa agtgcactta aaagccagaa tgtaacgtgt gtgtcctctg

1321 gggatctgct gggcagatgt cccaagccag gctgcccttc ctcatttccc actgtgcggg
1381 gggcaggggt gggaggtcta atctgtttac aaccatttcc gtgccatgct tttggttaca
1441 cgtatctcag tcacgctttc tgcctggttg gtggatcctc aggcttacca gtggctattt
1501 gcaagagcct gtttccagcc tccgtcagcc tgtcggtgct ctgaccctct gaaaccttgc
1561 tttgctgggc atctccccat attctctctc ctgtacatag tgggcagtgg tgtgtggttt
1621 tgaatggcag tgctataaag gatgccagct gtgctttagg cacctgtagg ccagagggtt
1681 gctgcctcag gcataaggtt ggtatgaaaa ggggctgcct tagaaaagca ggcacatccc
1741 ttgttcagaa atggccactg taggaaagcc tgtgaacctc ccaccagtgg ctttaagctg
1801 ccagccatgc cccatttcta gaatagcctt tgctcttcca cacgcaaccc ttccatcagt
1861 gtcttccagc ctgcatgggg agggcctggc tgcccagaca cccaccctca cttctcatgg
1921 ccagcctgtg tttgcagatc actgggctgc tctgtcccca gcttgccccg tgccaggcat
1981 ggctctgtga gattctgtgg aatgcccttc ttgacttgtg gaaatcattc accaagtacg
2041 atgggatggg attccaagta aacagtggac cgtcatattg caaagcaggc ccacgcctgg
2101 gctggccttg gttgagggga tggaggaatg ttttcggggg tgctttgcag ggcagtggtg
2161 cagccgcagc gtgaacgctc ttggacagct gaaaaccatg gcctccttgg ggatggccag
2221 tgctcctggt gtctccacct gcctgggttg tgcaggaggc cggctgccag tgcctggggc
2281 tgggaaactt ttttgcttgg ggcaggcttg ggtgagctct cccagatgtg tgcccacccc
2341 accactgcct ggaacatcct tgttctctgc caggatgcag gggcactgtt gcctctgaca
2401 cagcagtttc tgggaactca gaggactgcc tttctgaagg ctggggagag gctttgagca
2461 aggagcttgt ttttgcttaa tcgctctaga ccatccctga tgcgtagatg tgagaggatg
2521 ttttctggca cagtgttatg aaaatacaag ataaatgatc tcagtggaat gtttcatatt
2581 acccgaaaaa aactggaaat catcctttgt tgtgaacaca ctgagactaa gaattgaagc
2641 aatgcagttt taagaagtat ttttcctatt ttttgctgtc aactttttgg caatgccacg
2701 gcaaagttgg tgtttcagca catgctagag tgagctccgg cttcgtgtgt ttaaccaact
2761 ccactaatgt ttcactgggt agacggagct ggcctctata tttagccaga agcctttgcc
2821 tcttttcagg catcaaaaat gggttttgtc attgtctttg tctttgagag cagcagtttg
2881 aggtctgact ccttcagttg tcctgtgagc tgggcgttgc agtctgctcg aatcaagtgt
2941 tggaaccccc ccacccctca gcccatcgtg aaacgacagc tgtcagcctg gggggcggct
3001 gctgaggagg tgcagcagga atggggctgg gccctgaggt tttactttcc gagccaatgg
3061 cccctggaac atgccagcac acactgccat gtcctggggc taagagctgc ctttagggac
3121 aggctcgtgt tgtccagttt gccctgagag atgcacctga ccaggggccc gtcgtggctg
3181 caacccacgg tggtatgtta gtgtttccag gcagttgtca gcagctgccg ggtagtgtgt
3241 gtgtgcgaca gcaggagagt tcctggagga aagggactgg ggacactttc tcagaggttc
3301 tcctgcctgg aatcaagtga tgcgtgtcca gaaggcaaga gagggcggct ccgcctggtt
3361 aaagtttggg aacgggacgg agccaactcc tccttccagg actgtgggcc ccactctttt
3421 gcgctaacac ttcccctccc tcgggtcact gaggaaagag ctgctctgtc ccctccagtg
3481 ccctgaaggt ccctggcctc cactggctgc catggcaggg ggcccttctg acaggggctg
3541 ccccaagctg ctgttcctcc tctgaacctt caccccaaca cccagcccgt ggcccctgca
3601 agggggaccc tgccagctgg gaaacccaaa ggcctgtcca aacgtgcgca ccaggaccga
3661 ggggagctcc ctcccaacac ctgctaggaa ttgccaactt ttaaatggat ggggtttttt
3721 atgggttgaa cctctgttaa tacttttgta cactctcact acagtttata tttttatagg
3781 ctattttctc aaggtgtttc tagattccac atatctattt tatataacaa gttcttatgt
3841 tatgtgtgtg actcccttgt gtgtatctgt gccagcctca gcctccgagt tgcttttccc
3901 tctggccctg actctcactg actcaccgat gtgatgtgca ggcccacttc ttaccccaga
3961 tagcctcggg cgctgcctgt agtcatgctg acagctgtac agtagccgcc aagactgctg
4021 acagctggag acggttctgg tttcaactac ggtatattga tatcggaagt attctagaca
4081 gatcctcggt tgggttttct agctacatgt ttgtattgca cagatcccca cctgccatcc
4141 tatagtgttg tcttcctgtg tgttccgggg cttctgggca gctgggcctg cccggggaag
4201 tccttgcagg tgggaggcca tacagagacc cactgtgtgc cactgagcgt cccactgctg
4261 ctgggcaact ggaggactgc agggggcgcc aggtgactct ctccttttat atcacagcag
4321 ctcctgtgct gaccttcaag ttacgttttg gaactgtaat actaaaggaa gaaataaact
4381 actaatttgt ataaaaaaaa aaaaaaaaaa aaaaaaaaaa aaaaaaaaaa aaaa//

- LOCUS: AK025343 2012 bp mRNA linear PRI 12-SEP-2006
- DEFINITION: *Homo* sapiens cDNA: FLJ21690 fis, clone COL09538. *hepatocellular carcinoma-related HCRP1 (HCRP1)*
- ACCESSION: AK025343
- VERSION: AK025343.1 GI:10437841
- KEYWORDS: FLI_CDNA; oligo capping; fis (full insert sequence).

1 tttttttttt ttctcctagt gctttctggg tccaggcaat cttcatttga gaaccactag
61 tttacagcac tctctgagcc aaggaatttg gagtcaacca gtgctcatct aggtatcaaa
121 atggtggcaa gcccccaagtg caactaacaa cctacagaag acggaagtct aactttagtc

181 aacactcagg gagtggggat gaagaatgta gtcttcaaag ctcctggcca ctatggaaca
241 ctgttctcct gctgcaccct acatgctcac ccctccatcg tcaaagcctc ctcctcctgg
301 ttctggaaca ggtggtaaag cttttggagg ccctgtgcca accagtgctc tccgcaccgt
361 taatctggga accccaaatc aagatgctcg accctcctgg gattctgaga ggtcagccct
421 gtggcaccag aggctttctg gaatgattca tcatattgtc agctgaaatt ctactgaagt
481 ggatgcccaa gactagtttc tcagtactgc agtatctcac tcaatcccag tgatccgaaa
541 atccatccat cctcccagga cagtctgact gtgtagctgg ttcaggaaac caaaggcaag
601 cgggggtcac tcatccctcc agagcctcca gactgagaat aaggaaaaaa atcgctaggt
661 gcagtggctc acgcctataa tcccagcact ttgggaggct gaggtgggtg aattatgagg
721 tcaggagatt gagaccatcc tggccaacat ggtgaaaccc catctctact aaaaatacaa
781 aaattagctg ggcgcggtgg cgtgcgcctg cagtccctgc tactcgggag gctcaggcag
841 gagaatcact tgaacctggg aggcggaggt tgcagtgagc cgagatcgca ccacaacact
901 ctagcctggt gacagagcga gactccgtct caaaaaaaca cccaaaaaaa ccaacaaaaa
961 aaaccccaaa aatcagctgg gataggtcat gaggtcatga caggccatca actatttaag
1021 agatttttca actaagaata taagaacata ttgccataaa attttaaaat ccctatattc
1081 tagaggttct atctattttc agttgtatgc tccttaaact tatctgtatt taggcttccc
1141 ccaaaatgaa ttctaacaga aaaaaaaaac ttcggtaaaa aaagaatcca taaacactaa
1201 atattttaat acctttattt acctcctctc taacagaact aatcagcagc agataatata
1261 tttgttacga agagaattaa ttttctagtt aaacatgcac ataaaaacat aaaggtgaca
1321 atatttggca atatatgaca ctaataaagg taacatttat tgggctatcc actgggctaa
1381 gcgcttgact cagatcacct cagttaatcc ttacagcaaa cctataaagt aggtgctatt
1441 cttatttcca ttttatagat gagcttataa atgaggaacc tcattactgt aatttaatct
1501 tcaccaaccc agaatttgag ttcagtgttt actctggcta ttaaaaggtt gaataagcaa
1561 attagaaatt aaacggattt tcagaagata tatttaacct actagaaggt cgtacgtttt
1621 aagacatact gaaaaacaga tgctaactgc ttccaacagg atgtgtttag gaagatcctg
1681 cctgacacta taccacatta aaacacttga agattcaaac ttttactagt taaggttgag
1741 tgtcctctca ctattatccc caatgactgg tccaagttag taaggtatta ttctacttct
1801 atgggtggta cagtatggtg gatgtcagct gcggctccca agttctattt ccctcctacc
1861 ctgacaaagt gtccagaact ctacacaccc ccagcctccc cacagcctgg tgcaggagcc
1921 agtctggtgc agaagccagc ctgaggatga actgacccag ggaggaagga gtggagagat
1981 taaaaaaaaa aaaaaaaaaa aaaaaaaaaa aa//

- LOCUS: AK025833 1695 bp mRNA linear PRI 12-SEP-2006
- DEFINITION: Homo sapiens cDNA: FLJ22180 fis, clone HRC00936. *CD33 antigen-like 3 (CD33L3)*
- ACCESSION: AK025833
- VERSION: AK025833.1 GI:10438467
- KEYWORDS: FLI_CDNA; oligo capping; fis (full insert sequence).

1 tcgtaaatgt gcatacgtct gtgtgtgtgt gtgtgtgtga gagagagaga gagagagtac
61 acgcattagc ttgagcgtga aacttccaga aatgttccct tgccctttct tacctagaac
121 acctgctata gtaaagcaga caggaaactg tttacagggc ctggaggccc agtcttgtcc
181 tcctctgtca ccgacttgct gtgtggacct gggacactct cttcacttct ctgggtctca
241 attcatttac tgttgaacac ggacaatcat attcttccct cctggctggg aggactacaa
301 agatctgagg aaataatgga tatgaagggg ctttggaaag tacaaagcca tcttctcatg
361 aggggcatga cagaaccctc tttgcagagt gacctttgga cgtgcagtca gttcttggag
421 aagtgtggcc cctgcagcag tgtcacggag aacacagagt tccagagagc aagcctgggg
481 caggggctgt gcaaacgtgt ggtcagagga acatcaaatc ctggaaactt ggtggcaggg
541 agaggcctgg ggcctttacc ccatgccccc tgggatctcc actgtgactg tcccacaccc
601 tctgcccaac tcttgcccca tcagctgccc tgcattgcct tgagctggga cccctaggaa
661 atggaaacca ggctcccctc tctcaaggta tctgggaacc actgtctcag gagctgaagc
721 caggagacct gaggggaggt ctggccggag agtccgtgcg taaagcagct acagctctga
781 cctctctgac ctgcagcctc tcagggcgtg cgaagtccaa acgcactggc aacttcaccc
841 cctttgccct aacgcgggca ggctgacttg gaggggtgct tccctattta cacctcaggg
901 tgaatttgtt gatcacctta gctcctgtgg tccttggggc ttagggagag gagggaggca
961 tgcctgggtc ccaggtaagc tggcaagagg aaagggccac aggctagggg aataacgagg
1021 ccatgaacaa gacatctcac cccagttaca atggctttta tccaaaagac aggcaatgac
1081 aaatgctgat gagaaagtgg agaaagagga atccccatat actattggtg ggaatgtaaa
1141 ttagtatagc cactatggag aacagtacag tttcctcaaa aaactaaaaa taggctgggc
1201 gtggtggctc atgcctgtaa tcccagcacc ctgggaggcc gaggcaggtg gatcacttga
1261 ggtcaggagt tcgagaccag cctagctaac atggtaaaac tctgtctcta ctaaaaatac
1321 aaaaattagc tgggcgtgct ggcacgtgcc tatagtccca gcaacttggg aggctgaggc
1381 agaagtattg cttgaaccca ggaggcagag gttgcagtga gctgagatca ccccactgtc

1441 ctccagcctg ggcaacagag agactgtctc caaagaaaaa aaaagaaata gaactaccat
1501 atgatccagt aatcccactg ctgggtatct gtcagcaagg aaatcagtat attgaaaaga
1561 taccagtact cccatgttta ctgcagcact atttgcaata gccaagatat ggaatcagcc
1621 taagtgtcca tccacgaatg aatgaagaaa atgaacacac aatgaaatat tcagccataa
1681 aaaaaaaaaa aaaaa//

- LOCUS: AK022688 2376 bp mRNA linear PRI 16-SEP-2006
- DEFINITION: Homo sapiens cDNA FLJ12626 fis, clone NT2RM4001810, weakly similar to AGGRECAN CORE PROTEIN PRECURSOR. *KIAA0863 protein (KIAA0863)*
- ACCESSION: AK022688
- VERSION: AK022688.1 GI:10434228
- KEYWORDS: FLI_CDNA; oligo capping; fis (full insert sequence).

1 aacagcctca ccctgcagcc cccagcacct cagcccgtct ttctttctca cggggttcca
61 cttcatcagt ctgtgaatcc tcctgtgttg cccttgagtc agccagtcgg acctgtcaat
121 aagtctgttg gaactagtgt cctccccata aatcagactg ttcgccctgg ggttttaccc
181 ctcacccagc ctgtgggacc cataaacaga cctgttgggc ctggtgttct tcctgtgagc
241 ccctctgtca cccctggggt cctgcaggct gtctcgccag gggtgctttc tgtgagtcgg
301 gcggtcccgt ctggagtcct tcctgcaggc cagatgactc ctgcaggcca gatgactcct
361 gcaggggtta tcctgggcaa acagcaactt ctggggttct tcctactggc cagatggtcc
421 agtcaggagt tctccctgtg ggccagacag ctccgtcacg ggttcttccc ccaggccaga
481 cagccccatt gagggttatc tctgcaggcc aggtggtccc atctgggctt ctttctccca
541 accagacagt ctcctcctca gctgttgtgc ctgtaaacca gggtgtgaat tctggtgttc
601 tgcagcttag tcagcctgtt gtgtcgggag ttcttcctgt gggccagcca gtgaggcctg
661 gggtcttgca actcaaccag actgtgggca ccaacattct gcctgtgaat cagccagtga
721 gacctggtgc ttcgcagaac accaccttcc tgacatcagg ctctattctc agacagctca
781 tccctacagg gaaacaagtg aatggattcc caacctacac gctggccccc gtgtctgtca
841 ctctgccggt tccccctgga ggccttgcga ctgtcgctcc gccccagatg cccatccagc
901 tcctgccgtc aggtgcagct gcaccaatgg ccggttccat gcccggcatg ccctctcctc
961 cagtgctggt gaatgctgct cagagcgtgt ttgttcaggc ctcctcctct gcagcagaca
1021 caaaccaggt gctcaaacag gccaagcagt ggaagacctg ccctgtctgc aacgagctct
1081 ttccgtccaa cgtctaccag gtccacatgg aggtagcgca taagcacagc gagtccaagt
1141 ctggtgagaa acttgagcct gaaaaactgg cagcgtgtgc accatttcta aagtggatga
1201 gagagaaaac ggtgcgatgt ctgtcttgta agtgcttggt ccctgaggaa gagcttatac
1261 accacttgct gatgcatggc ttggggtgct tgttctgtcc atgcaccttc catgatatca
1321 aaggtctttc agagcacagc aggaataggc acctggggaa gaagaagttg cctatggatt
1381 atagcaacag aggttttcaa ttagatgtcg atgccaatgg caacctgctc tttccccacc
1441 ttgatttcat caccatattg ccaaaggaga agcttgggga gcgggaagtc tacttggcaa
1501 tcctggctgg gatacactcc aagtcactgg tgcctgtgta tgtgaaggtg aggcctcagg
1561 ctgagggcac ccccgggagc accggcaagc gagtgtccac ctgccccttt tgctttggcc
1621 cctttgtgac aactgaggcc tatgagctgc atttgaagga gaggcaccac atcatgccca
1681 cagtccacac ggtcctgaag tctcccgcct tcaagtgcat ccactgctgt ggggtctaca
1741 cgggaaatat gaccctggct gccatcgccg tccatttggt gcgctgcaga agtgctccca
1801 aggacagcag ctcagacctg caggcccagc cgggttttat tcacaacagt gaactgcttt
1861 tagtcagtgg tgaagtgatg catgattcca gtttttctgt taagagaaag ctgcctgatg
1921 gccacttagg ggccgaagac cagcggcatg gggaggagca gcctcccatc ctaaatgccg
1981 atgcagcccc gggtccagaa aaggtgacga gtgttgtgcc ttttaaaaga caaaggaatg
2041 aaagcagaac agagggacct attgtcaagg acgaggctct tcagatttta gcattagatc
2101 ctaaaaaata tgaaggccgt tcttatgaag aaaagaagca atttcttaaa gattatttcc
2161 ataagaaacc atatcctagt aaaaaggaaa tagaactgtt gtcctcactc ttttgggtgt
2221 ggaaaattga tgtggcttca ttttttggaa aaagaaggta tatttgcatg aaagcaataa
2281 aaaatcacaa gccttctgta cttttaggct ttgatatgtc tgaacttaaa aatgtgaaac
2341 atagattgaa ctttgaatat gaaccataaa acttgc//

- LOCUS: NM_024519 4088 bp mRNA linear PRI 17-NOV-2006
- DEFINITION: Homo sapiens family with sequence similarity 65, member A (FAM65A),mRNA. *hypothetical protein FLJ13725*
- ACCESSION: NM_024519
- VERSION: NM_024519.2 GI:39995079

1 cgaccaggcc gggcacctcc gagcgcaagg acagcgcgag gtccgcgcag cccagcgcag
61 ccatggagcc ccgcgcggac tcactctatg atgtccctgt cggtgcggcc gcagcgccgt

121 ctgctcagcg cccgggtcaa taggagccag tccttcgcag gcgtcctcgg cagccacgag
181 cgggggccca ggagtttccc ggtcttcagc ccgccggggc ccccacggaa gcccccgcg
241 ctctcccgag tgtccaggat gttttccgtg gctcacccag ccgccaaggt gccgcagccc
301 gagcggctgg acctggtgta cacggcgctg aagcggggcc tgacggccta cttggaagtg
361 caccagcagg agcaagagaa actccagggg cagataaggg agtccaagag gaattcccgc
421 ttgggcttcc tgtatgatct ggacaagcaa gtcaagtcca ttgaacgctt cctgcgacga
481 ctggagttcc atgccagcaa gatcgatgag ctgtatgagg catactgtgt ccagcggcgt
541 ctccgggatg gtgcctacaa catggtccgt gcctacacca ctgggtcccc gggaagccga
601 gaggcccggg acagcctggc agaggccact cgggggcatc gcgagtacac ggagagcatg
661 tgtctgctgg agagcgagct ggaggcacag ctgggcgagt ttcatctccg aatgaaaggg
721 ctggctggct tcgccaggct gtgtgtaggc gatcagtatg agatctgcat gaaatatggg
781 cgtcagcgct ggaaactacg gggccgaatt gagggtagtg gaaagcaggt gtgggacagt
841 gaagaaacca tctttctccc tctactcacg gaatttctgt ctattaaggt gacagaactg
901 aagggcctgg ccaaccatgt ggttgtgggc agtgtctcct gtgagaccaa ggacctgttt
961 gccgccctgc cccaggttgt ggctgtggat atcaatgacc ttggtaccat caagctcagc
1021 ctggaagtca catggagccc cttcgacaag gatgaccagc cctcagctgc ttcttctgtc
1081 aacaaggcct ccacagtcac caagcgcttc tccacctata gccagagccc accggacaca
1141 ccctcacttc gggaacaggc tttctataac atgctgcgac ggcaggagga gctggagaat
1201 gggacagcat ggtccctgtc atctgaatct tcagacgact catccagccc acagctctca
1261 ggcactgccc gccactcacc agcccctagg cccctggtgc agcagcccga gccccttccc
1321 atccaagttg ccttccgcag gcctgagacc cccagctctg ggcccttgga tgaggagggg
1381 gccgtggccc cagtcctggc aaatggcatt gcaccctaca gtcggactct gagccacatc
1441 agtgaggcta gtgtagatgc tgccttggct gaggcttcag tggaggccgt tggcccagaa
1501 agcctagcct ggggacctag cccacctaca cacccagctc ccacccatgg agagcacccc
1561 agtcctgttc ctcctgccct ggaccctggc cactctgcca caagctctac cctcggtaca
1621 acaggctctg tccccacatc tacagaccct gccccatctg cacacctaga ctcagttcat
1681 aagtccacag actctggccc ttcagaactg ccaggcccca ctcacaccac tacaggctct
1741 acctatagtg ccattaccac tacccacagt gctccaagcc ccctcactca cactactaca
1801 ggctccaccc acaagcccat aatctctacc cttactacta caggccctac cctcaatatc
1861 ataggcccag tccagactac cacaagcccc acccacacta tgccaagccc tacccatacc
1921 acagcaagcc ccactcatac ttccacaagc cccacccata cccccacaag tcccacccac
1981 aaaaccagta tgtcacctcc caccactaca agtcctaccc ccagtggtat gggcctagtc
2041 cagactgcca caagtcccac ccatcctacc acaagcccca cccatcccac cacaagcccc
2101 atccttataa atgtaagccc ttccacttct ctagaacttg ctaccctctc cagcccctcc
2161 aaacactcag accccaccct cccaggcact gactcccttc cctgtagtcc cccagtctcc
2221 aattcctaca ctcaggcaga ccctatggcc cccagaactc cccacccaag tcctgcccat
2281 tccagtagga aacccctcac aagccctgcc ccagatccct cagagtctac ggttcagagt
2341 ctaagcccca ctccctcacc cccaacccct gcaccccagc attcagacct ttgcctggcc
2401 atggctgtcc agaccccagt cccaacggca gccggagggt ctggggacag gagcctggag
2461 gaggcactgg gggccctaat ggctgccctg gatgactacc gtggccagtt tcctgagctg
2521 cagggcctgg agcaggaggt gacccgccta gaaagtctgc tcatgcagag acaaggtctg
2581 actcgcagcc gggcctccag tctcagcatc actgtggagc atgccttgga gagcttcagc
2641 ttcctcaatg aagacgaaga tgaagacaat gatgttcctg gggacaggcc tccaagcagc
2701 ccggaggctg gggctgagga cagcatagac tcacccagtg cccgccccct cagcacgggg
2761 tgtccagctc tggatgctgc cttggtccgg cacctgtacc actgcagtcg cctcctgctg
2821 aaactgggca catttggccc cctgcgctgc caggaggcat gggccctgga gcggctgctg
2881 cgggaagccc gagtactgga ggcagtatgc gagttcagca ggcggtggga gatcccggcc
2941 agctctgccc aggaagtggt gcagttctcg gcctctcggc ctggcttcct gaccttctgg
3001 gaccagtgca cagagagact cagctgcttc ctctgcccgg tggagcgggt gcttctcacc
3061 ttctgcaacc agtatggtgc ccgcctctcc ctgcgccagc caggcttggc tgaggctgtg
3121 tgtgtgaagt tcctggagga tgccctgggg cagaagctgc ccagaaggcc ccagccaggg
3181 cctggagagc agctcacagt cttccagttc tggagttttg tggaaacctt ggacagcccc
3241 accatggagg cctacgtgac tgagaccgct gaggaggtgc tactggtgcg gaatctgaac
3301 tcggatgatc aggctgttgt gctgaaggcc ctgagattgg cgcccgaggg gcgtctgcga
3361 agggacgggc tgcgggccct cagctccctg ctcgtccatg gcaacaacaa ggtcatggct
3421 gctgtcagca cccagctccg gagcctgtca ctgggcccta ccttccggga gagggccctc
3481 ctgtgcttcc tggaccagct ggaggatgag gacgtgcaga ctcgagtggc tggctgcctg
3541 gccctaggct gcatcaaggc tcccgagggc attgagcccc tggtgtacct ctgccaaact
3601 gacacagaag ctgtgaggga agctgcccgg caaagcctac agcagtgtgg agaagaggga
3661 cagtctgccc atcgacggct ggaggagtcc ctggacgccc tgccccgcat ctttggccct
3721 ggcagcatgg ccagcacagc attctaaact attcacccat gggttcctgg tgcccctttc
3781 cccccacttt cagggctcac caggcactgg cagggagggt aagggctggc tccagatacc
3841 cctcccccac agattcctag caatgaaaat ctaatatatt cttctgttgc cctggggtt

3901 ggagagtcag tgcctgcagt caagtgcctc ccagcctcgg ctcagcacat cccttgccac
3961 aaatcagtgt ctggggcttg gccaccctgc cgctgcccag ccacatccct tggttttgta
4021 ttttatttac agagttttac agaaaataaa aaagcaaaat gtctttccta aaaaaaaaaa
4081 aaaaaaa
//

- LOCUS: BF436315 565 bp mRNA linear EST 29-NOV-2000
- DEFINITION: 7p06b05.x1 NCI_CGAP_Ov18 Homo sapiens cDNA clone IMAGE:3644888 3',mRNA sequence. *Nedd4 binding protein 1 (N4BP1)*
- ACCESSION: BF436315
- VERSION: BF436315.1 GI:11448630
- KEYWORDS: EST.

1 tttcagttga gcagacattt attaagcacc tatcaagtgc aaggcactgt gctaggcgcc
61 gtgggaaata cagagaacac aggcggtccc tgcccacgag gagctcacag tctagaaagg
121 gcagcaagac agtacacaat cagtggcagc agcaccagcc agagtggcaa gtgctcaaag
181 cgagacacaa agtgctgtgc ggtcacaaca tcatggggat gcttctggca gaagcactgg
241 aaaggagacg aggactcagg ctgggccttc caggagggaa gccatttggg agaagggcat
301 ctctagcgga gagaggtcca tctgcagagc ccacaggtca tgggaaacat gtggctgcag
361 ggagagtttg gggacagtca agtatggctg ggaggtggac agccacggac actaagctca
421 ggagatgtga actttgtggt ctggtcaaac agccactgca gatcacttcg caggaggagg
481 ggtgactaga agtatgttac tggaaggtaa atctggccac actgcagcat ctggacggca
541 gagggcagcg ctgggagggg ccagc//

- LOCUS: NM_018053 2179 bp mRNA linear PRI 17-NOV-2006
- DEFINITION: Homo sapiens XK, Kell blood group complex subunit-related family,member 8 (XKR8), mRNA. *hypothetical protein FLJ10307 (FLJ10307)*
- ACCESSION: NM_018053
- VERSION: NM_018053.2 GI:24431976

1 tcctgcctcg gcaaccccgg gccctgaggg caggccccaa ccgcggagga gcaggagagg
61 gcggaggccg gcgggccatg ccctggtcgt cccgcggcgc cctccttcgg gacctggtcc
121 tgggcgtgct gggcaccgcc gccttcctgc tcgacctggg caccgacctg tgggccgccg
181 tccagtatgc gctcggcggc cgctacctgt gggcggcgct ggtgctggcg ctgctgggcc
241 tggcctccgt ggcgctgcag ctcttcagct ggctctggct gcgcgctgac cctgccggcc
301 tgcacgggtc gcagcccccg cgccgctgcc tggcgctgct gcatctcctg cagctgggtt
361 acctgtacag gtgcgtgcag gagctgcggc aggggctgct ggtgtggcag caggaggagc
421 cctctgagtt tgacttggcc tacgccgact tcctcgccct ggacatcagc atgctgcggc
481 tcttcgagac cttcttggag acggcaccac agctcacgct ggtgctggcc atcatgctgc
541 agagtggccg ggctgagtac taccagtggg ttggcatctg cacatccttc ctgggcatct
601 cgtgggcact gctcgactac caccgggcct tgcgcacctg cctcccctcc aagccgctcc
661 tgggcctggg ctcctccgtg atctacttcc tgtggaacct gctgctgctg tggccccgag
721 tcctggctgt ggccctgttc tcagccctct tccccagcta tgtggccctg cacttcctgg
781 gcctgtggct ggtactgctg ctctgggtct ggcttcaggg cacagacttc atgccggacc
841 ccagctccga gtggctgtac cgggtgacgg tggccaccat cctctatttc tcctggttca
901 acgtggctga gggccgcacc cgaggccggg ccatcatcca cttcgccttc ctcctgagtg
961 acagcattct cctggtggcc acctgggtga ctcatagctc ctggctgccc agcgggattc
1021 cactgcagct gtggctgcct gtgggatgcg gctgcttctt tctgggcctg gctctgcggc
1081 ttgtgtacta ccactggctg caccctagct gctgctggaa gcccgaccct gaccaggtag
1141 acgggcccgg gagtctgctt tctccagagg ggtatcagct gcctcagaac aggcgcatga
1201 cccatttagc acagaagttt ttccccaagg ctaaggatga ggctgcttcg ccagtgaagg
1261 gataggtgaa cggcgtcctt tgaagcagga tcagacccag ccagcagaga tggagagtga
1321 ctctgttggc agaaggcagg cgaggataag ctaacgatgc tgctgtggcc tctatgcact
1381 cagcaagagc gggacgcctg tgctgggccg ggcaccaggg atggtgctga gtcgggcaga
1441 ggcctccttt caaggagttc acagtgaaca agatgagaag ggctgggccc tggagggtca
1501 agagccccaa ttatgtacaa gacactttgg gaggaaagaa gactaccttt tccccctgcc
1561 attggtatag ctggtgcccc aaaacttcca cctccctccc tggccacctc taaaatgact
1621 ggtataggtg ctgccccacc ccttagctcc cctatcctgg gctaggaggc cacaggggct
1681 gtcctctaga attcttcctt ccctccccca caccattcat tcaattcatg aaacaaatct
1741 ttgccaagag cagtttatgt gccaggaaca tcattctgtc cttgcaacct ggaacaagac
1801 cagctaccag cctagcttca tccgctactt gcaccaacca gtcccgggtt agatcccaaa
1861 tgctagaagc caggatgccc caactctggg tggccccagt cagaacctct gggatctcag
1921 tgaagctggc ctggcctctg ctcctgctct caaggggctg cttttcaacc aagagccttg

1981 tgagcctggt ctgagccttg cacagccact gagtattttt tttgccttag ccagtgtacc
2041 tcctacctca gtctatgtga gaggaagaga atgtgtgtgc ctgtgggtct ctacaagtga
2101 cagatgtgtt gttttcaaca gtattattag gttatgaata aagcctcatg aaatcctcaa
2161 aaaaaaaaaa aaaaaaaag//

- LOCUS: AF052091 1353 bp mRNA linear PRI 05-AUG-1998
- DEFINITION: Homo sapiens clone 23578 mRNA sequence. *BTB* (POZ) *domain containing 9 (BTBD9)*
- ACCESSION: AF052091
- VERSION: AF052091.1 GI:3360397
- KEYWORDS: FLI_CDNA.

1 caatatctgt gtcgttcttg gcagaaatta tattttccag cccgtgtctg caggtatatt
61 cgaattgttg ggactcacaa cacagtgaac aagatttttc acattgtggc ttttgaatgt
121 atgtttacaa acaaaacctt cactcttgag aaggggctga taggttacta ctttgggatt
181 gtgacactca aagtggacac agtggaacac ctgggagaga aaaggaaaag accccatgag
241 tgaagggtca gcatgcggcc ctggagttgc cgcaagttgg gatactgccc tgcccagctt
301 tgggggccac cacgagggag ttagaggagg tccttcccac tgcccacgcc tgccccggcg
361 cagacacctg atggcaacta gcatgctgta aacacatttt gatcagtcaa gaggaaagga
421 aatgagccag tggcccaaaa gttagagaca ccatgacacg gattcccaca aaatcttcac
481 actggattac tccgtttttg ccaacctttg tctaatgggc tcttttttct tttttaatta
541 atttttgtat ttggcagttg ggttaaaagt caacaggagt tgactggtgg tggtggtggt
601 ggtggttcga ttctgtgagg ttgtggaaag caggtccaca gataacgtcc accatctgtg
661 tggcatctgc gtgggttgtc tctgggtatt ctggttttct cccgcgtccc aaagctgtgc
721 acttgaggtt cattggtgtg tctgcagggt cccagtctga gtgcatgtga atgtgtgtga
781 gcgtgccctg caatggcatg gtgtcctgtc ctgagctggt tcctgatgca tagttctcct
841 ggccctgcaa tggttgagca cagaccctgc taagcagccg tgctggggag tggacttcaa
901 cctcatgtgg ctccagccag cctggaacaa acgcccccaa tgtgtagaag ccagccacct
961 ggccgacagt caccctctag gagctaagaa gagtttgcat acctgacagc aggtcagcct
1021 ggaagcacca tgaactctgt gccaacaccg tgccagcact gtgcagacag ggcacagcac
1081 cgagctcagc gccccgcaag caagggtaag ccatggccct gacctggaga gtagacaacg
1141 tgcttcgggg ggtcctgaca tgcatgtgtg agctgtgggg tgcaggagag aagggcgggg
1201 tgggggctgt cgttgccact gcacgagcat gtgtgcacat acatacacac ggccttctac
1261 cgtagaatca agttcacaga gacaaatgta gtatcaactg cagaattata ttgaaaatat
1321 aaattccacg cttaaaaaaa aaaaaaaaaa aaa//

- LOCUS: NM_024037 2061 bp mRNA linear PRI 17-NOV-2006
- DEFINITION: Homo sapiens chromosome 1 open reading frame 135 (Clorf135), mRNA. *hypothetical protein MGC2603 (MGC2603)*
- ACCESSION: NM_024037
- VERSION: NM_024037.1 GI:13128989

1 atgaggcgga caggccccga ggaggaggcc tgcggcgtgt ggctggacgc ggcggcgctg
61 aagaggcgga aagtgcagac acatttaatc aaaccaggca ccaaaatgct aacactcctt
121 cctggagaaa gaaaggctaa tatttatttt actcaaagaa gagctccatc tacaggcatt
181 caccagagaa gcattgcttc cttcttcacc ttgcagccag gaaagacaaa tggcagtgac
241 cagaagagtg tttcatctca tacagaaagt cagatcaaca aagagtccaa gaaaaatgcg
301 acccagctag accatttgat cccaggctta gcacacgatt gcatggcatc ccctttagcc
361 acttcaacca ctgcggacat ccaggaagct ggactctctc ctcagtccct ccagacttct
421 ggccaccaca gaatgaaaac cccattttca actgagctat ctttgctcca gcctgatact
481 ccagactgtg ctggagatag tcatacccca ctggcttttt ccttcaccga ggacttggaa
541 agttcttgtt tgctagaccg aaaggaagaa aaagggattt ctgccaggaa atgggaatgg
601 cttcatgagt ctaagaagaa ctatcagagt atggagaaac acaccaaact acctggggac
661 aaatgctgtc agcccttagg caagactaaa ttggaaagaa aggtgtctgc caaagaaaac
721 aggcaggccc ctgtcctcct tcaaacatac agggaatcct ggaatggaga aaacatagaa
781 tcggtgaaac aaagccgtag tccagtttct gtgttttcct gggacaatga aaagaatgac
841 aaggactcct ggagtcaact tttcactgaa gattctcaag gccagcgggt cattgcccac
901 aacactagag ctccttttca agatgtaacc aataactgga attgggactt agggccgttt
961 cctaacagtc cttgggctca gtgccaggag gatgggccaa ctcaaaatct gaagcctgat
1021 ttgctcttta cccaggactc tgaaggtaat caagttatca gacaccaatt ctaaatgttt
1081 gaagctttgt ttctaaaagt accttgaaat gatagagatg taggaaaata tagttgtggg
1141 tggagagagg agtgagtttg tttaggtggg aaggtggcat gggatgaagt tgtcattact
1201 gagcatcttc tctgtgtaaa taaagggcag taccattgtt aagacagtgg gattggcatc

1261 atggctttcc ctcaggaagg tggtggctgg taaattccct gaatgagtct atgatgaaca
1321 ctgaggcagc acagtgggta tttatctcta tgaaagtgcc ttttactcag cctgcacaga
1381 gccatctctt tgcccttcca gatgtctgac tgggaccttg cttatggatg tgtttttttt
1441 tttttttttt tgagatggag tctcgctctg tcgccaggct ggagtgcagt ggtgcgacct
1501 cagctcactg caccctctgt gtcccggatt caagcgattc tcctgcctca gcctcccgaa
1561 tagcagggac tacaggcatg cgccaccacg cccagctaat tttttttgga tttttagtag
1621 agacgaggtt tcaccatatt agccaggatg gtctccatct cctgacctcc tgatccgccc
1681 acctcagcct cccaaagtgc tgagattaca ggcataagcc accgcgccca gccagatgtg
1741 tgagctttta atctctggct gatcttaacc cacatcagcc taagcttggg atgattactc
1801 ttgacccttt tttttcagtg attagcaaat ctccccacaa cccaggtgtg gagagaagag
1861 aggtagaatg gtgctagttt cctattttat ttttgtggta actgtacagc actttaaagt
1921 tatatactct atgtttaaat atctccctta aaaagcctga gctgtacaac aatctggatg
1981 tgactctgtt acccttttcc cacaagatag gagggaatcc cctttgtaaa actatgaatc
2041 caaataaatg tttacaaagt g//

- LOCUS: X72882 214 bp DNA linear PRI 23-JUN-1999
- DEFINITION: H.sapiens 14A6CK DNA sequence. *KIAA0409 protein (KIAA0409)*
- ACCESSION: X72882
- VERSION: X72882.1 GI:667005
- KEYWORDS: Alu repeat; L1 repeat.

1 ccaaaccatc tagttttcat aacacctgaa gtgtcatgag acagatatcc catgttctca
61 caataactct ttattgttta gctagcccca gtgacaagtc tgagccagct agccccagtg
121 actttatgca tcttataacc aagaagcctt cagtagagca agtctgagcc agaggtttta
181 tcacactttg cctcagggtc caccaggaac cagg//

- LOCUS: AK025127 2306 bp mRNA linear PRI 12-SEP-2006
- DEFINITION: Homo sapiens cDNA: FLJ21474 fis, clone COL04941. *SATB family member 2 (SATB2*
- ACCESSION: AK025127
- VERSION: AK025127.1 GI:10437581
- KEYWORDS: FLI_CDNA; oligo capping; fis (full insert sequence).

1 cagtggaaag agtggaacga gaaaaccttt cagactattg tgttctgggc cagcgtccaa
61 tgcatttacc aaatatgaac cagctggcat ccctggggaa aaccaacgaa cagtctcctc
121 acagccaaat tcaccacagt actccaatcc gaaaccaagt gcccgcatta cagcccatca
181 tgagccctgg tcttctttct ccccagctta gtccacaact tgtaaggcaa caaatagcca
241 tggcccatct gataaaccaa cagattgccg ttagccggct cctggctcac cagcatcctc
301 aagccatcaa ccagcagttc ctgaaccatc cacccatccc cagagcagtt aagccagagc
361 caaccaactc ttccgtggaa gtctctccag atatctacca gcaagtcaga gatgagctga
421 agagggccag tgtgtcccaa gctgtctttg caagagtggc attcaaccgc acacaggtac
481 aattagcatt aaacactgta attaacagta atactgggga cagaattgag ataggttata
541 attattttta tctctttaaa aagctttatg gatctcagga catagaatta gattaattaa
601 taatccttag ttaatgaaac gctattacca ttgatccatg catgaccatg attaattaac
661 taattgatta attttgatga ggtaatggac atctttgagc ccaacatgca actcaggacc
721 ccaaatgtta ctaatgactt gggtatgtac tctctcatcc tctccattta ctgtttccag
781 atgtaaccct gatcttgaat tttgtgatta tcagtctctt gccatttttt aaaacaaatc
841 ataaaactat gcatgcccag acaatctatt tttttccctt ggttttgaac gttataaaag
901 aaataaaatt atgttttata attttataag taaagtttta tttaaaaatt tataaaaatc
961 cttcaaaaca taaaagttag atttaagata ttgataaaat gataatggat agtttacttt
1021 tatttttaat gtttgagaga attatatgcc aattataaca ctaaatgttg aaaatgtaaa
1081 cagttaattc acacgtctta tttttattga gggtgcttat atgtaagaca ttcttttctg
1141 ctagtagttc ttaaactgga gtgtgcatca gaatcacctg gagggctgtg aaacacagac
1201 tgctgggcct catcccatag tttctgattg agtaagtctg aggttggact cagaaatgga
1261 catttctaac aagatcccag gtgattatga tggtactgat ccggggccgc atgttgagaa
1321 aaacttacct agcccttata tgagtaagaa caaagcagat gggcataatc tcggggagct
1381 aacgatctag tggtgaaaac caggtaacca gataattaga gtaaaatgtg aagtgttatg
1441 agtaatgttg gagagtgtaa gcaatagttg tggtggaggt tcatggtgag ctggaggtga
1501 ggaatacttc caggagaatg aatatggtaa gccttcaaga aaacagtaga ttaaaatttc
1561 agcaccctcg taaatgcaag gcaggaagct aagctctatg tgggtgcaaa gaggaaaaag
1621 ataatgggca agcacatatg taaacaacag agtattaggt tgtcaatggg ttagaccttg
1681 aaggattaat aaagtacagc aggcaaagat gatgaagaag aagattatta ttttaaggta
1741 aagacattca taggaaagat gttgagttaa aaaatgcagg aataatttga agacataaaa

1801 caactgattt ctgagaaaaa ccctgtgaac gtacagaatg taggaatgct ttcatttatc
1861 tgatacctgt tcagagacac atgataacac acgctgtaga tggaccttac aaatgaaaga
1921 aagcatgctg gattgaaacc tcaataattt ggaaaataca ggaatgtttc caaatttaat
1981 gattacttca gaagttaagg ggctgggcac agtggctcat gtctgtagtc ctagcacttt
2041 gggaggccaa ggtgggcgga tcacttgagg tcaggagttc aagaccagcc tgggcaagat
2101 ggtaaaacac catctctact aaaacagaac aaaacaaaaa aattagctgg gcgtggtgat
2161 gcgtgcctgt agtctcagct actcaggagg ctgaggcaga agaatccctt gaacccagga
2221 ggcggaggct gcagtgggcc gagatcatgc cactgcactc cagcctgagt gagagtgaaa
2281 ctccatctca aaaaaaaaaa aaaaaa//

- LOCUS: HUMORFHA 4203 bp mRNA linear PRI 10-JAN-2004
- DEFINITION: Homo sapiens KIAA0028 mRNA, partial cds. *leucyl-tRNA synthetase 2, mitochondrial (LARS2)*
- ACCESSION: D21851
- VERSION: D21851.1 GI:434766

1 tgacaacatg gcggcgccca tggtccgtgg cccggcagtg ctcgcctaaa ggtggagaac
61 gaggagtaga ggaggccgca gccagagcct gtgagcagat ccagacctac agataaaaaa
121 cattatttaa tctatctggg atttactccg gcttatgatt tgaggccttt ctcaccttct
181 gaagaatggc ttctgtttgg cagagattgg gtttttatgc ctctcttctg aaaagacagc
241 taaatggtgg gccagatgtc atcaagtggg aaaggagagt aattcccgga tgtaccagaa
301 gcatctacag tgccacggga aagtggacaa aagagtatac attgcagaca agaaaggatg
361 ttgagaaatg gtggcatcaa cgaataaaag aacaggcctc caaaatttca gaagctgata
421 aatcgaagcc aaaattttac gtgctttcca tgttccctta tccttctggt aagctgcaca
481 tgggccatgt gcgtgtctac accatcagcg acaccatagc acggttccag aagatgagag
541 ggatgcaggt catcaacccc atgggatggg atgcttttgg attgcctgct gaaaatgccg
601 cagtcgagag gaatctacat ccacaaagtt ggacacaaag taatattaaa cacatgagga
661 aacagcttga tcgtctgggc ctgtgtttca gctgggatag ggaaataact acgtgtttgc
721 cagattacta caagtggact cagtatctct ttattaaact gtatgaggct gggctggcct
781 atcaaaagga ggccctggtt aactgggacc cagtggatca aacagtgctt gccaatgagc
841 aggtggatga acatggctgt tcatggcgtt ctggagcaaa ggtggaacag aagtacctca
901 gacaatggtt tattaagaca accgcttatg caaaggccat gcaggacgcg ttggcagacc
961 ttccagaatg gtatggaata aaaggcatgc aagcccactg gattggggac tgtgtgggct
1021 gccacctgga cttcacatta aaggttcatg ggcaagccac gggcgaaaag ctgactgcct
1081 atacggccac ccctgaagcc atttatggca cctcccacgt ggccatctcg cccagccaca
1141 gactcctaca tgggcacagc tctctgaagg aagccttgag gatggccctt gtccctggca
1201 aagattgcct cacgcctgta atggctgtga acatgcttac ccagcaggag gtccctgtcg
1261 ttattttggc caaagctgac ttggaaggct ctctggattc aaaaatagga attcccagta
1321 ctagctcaga ggacaccatc ttagcccaaa ccctgggcct ggcctactct gaagtcattg
1381 aaactttgcc agatggcaca gagagactga gcagctctgc tgagttcaca ggtatgaccc
1441 ggcaggatgc ttttctagcc ctgactcaga aagcccgggg gaagagagtg ggtggagacg
1501 tgacaagtga taaactgaaa gactggctga tttcacggca gcggtactgg ggcacaccaa
1561 tccccattgt ccactgccca gtctgtggcc ccacacctgt gcccctggag gacttgcctg
1621 tgaccctgcc caacatcgcg tctttcactg gcaagggagg ccccccactg gccatggctt
1681 cagagtgggt gaactgctcc tgcccaaggt gcaagggagc agccaagaga gagacagaca
1741 cgatggatac ctttgttgat tctgcttggt actacttcag atacactgac cctcataatc
1801 cacacagccc ttttaacaca gcagtggccg attactggat gcctgtggat ttgtacattg
1861 gagggaaaga acatgccgtc atgcacttgt tctatgcaag attctttagt catttttgcc
1921 atgatcaaaa aatggttaaa catagggagc cttttcataa gctgctggcc caaggcctta
1981 tcaaggggca gacattccgc ctaccatctg gacagtatct acagagagag gaagtggatc
2041 tcacaggttc cgttcctgtt catgcaaaaa cgaaagagaa gttagaggtg acgtgggaga
2101 agatgagtaa gtccaaacac aacggggtgg acccagagga agttgtggag cagtatggga
2161 tcgacacgat tcggctctac atcctttttg ctgcccctcc tgagaaggat atcttgtggg
2221 atgtgaaaac tgatgctctc cctggggtgc tgagatggca acaacgactg tggaccttga
2281 caactcggtt tattgaggcc agggcttctg ggaagtctcc ccagcctcag ctgctgagta
2341 acaaggagaa agctgaggcc aggaagctct gggagtacaa gaactccgtc atctctcagg
2401 tgaccaccca tttcacagag gacttctcac tgaattctgc aatttctcag ctgatgggac
2461 tcagcaatgc cctctcgcaa gcctctcaga gcgtcattct ccacagcccc gagtttgagg
2521 atgctttgtg tgccctgatg gtaatggctg ctccactggc ccctcatgta acctcagaga
2581 tctgggcagg cctggcgctg gtgccgagga agctctgtgc ccactacact tgggatgcca
2641 gtgtgctgct ccaggcatgg cctgctgtgg acccggagtt cctgcagcag cctgaggttg
2701 tccagatggc agttctgatc aacaataaag cttgtggcaa aattcctgtg ccccaacaag
2761 ttgcccggga ccaggacaaa gtccacgaat ttgttcttca aagcgagctg ggtgtcaggc

2821 ttttgcaagg acgaagcatc aagaagtcct tcctttcccc gagaactgcc ctcatcaact
2881 tcctggtgca agattgacag ccaggaggct gcagctacca cgagggcctc tgaggaacct
2941 ccttccaggc ctgggatgag ggggcgatgt ctgctggccc aggggaaggg aaaagacaaa
3001 tgtcttgact gttgacctcg gtcctgtggc agactgcagt caacagtgtg cctctgtagt
3061 gtggcctggt gctggggtga aggtgagctg ggcaaaggag aaatatgagc tactgaggag
3121 ggggttggac atcctgcccc tcacccccca cccacactgc aggtagagga ggccatctga
3181 tcccatggga agccatcaga gacactgctg gtgggagcag gaaggagcag tgcccctcga
3241 gcagccagga agcctgcgga tctgggaaat ggctctgcct taggcacttc tcgggaattt
3301 gaggccagcc tgaggaactg caggactcag gtgcaatgtg ccagccactt ggaactgcta
3361 actgagcctc cagatggtag tgaatggtct ctttgccttc aggctggatg aggaagtcat
3421 ttaggaaatg ttcaaataac caatatgtgg aaatggacac agggatcttc tgaagttgct
3481 ttgaatcaaa aggcaggcag tgctggttcc tctgcctgtg tccccaccac tccccagctc
3541 tgtcatgcag gcctgtcctc cccaacccca gctggatgtg cctcccaggc ctgctgtggt
3601 tctgacacac aggatcccag gcaaggcacc acttcctcac atgaatgagg agcagcaagt
3661 cataaccact cccttgggta tacaatttgc tgtgtagtga agtggaacca ggctcaggct
3721 gctggtccca acctcagagc cccaccgcag cccagtaggg atgcagcacg ccccagaggg
3781 ctcatgtggg ccccagatgg caatgccacc attgttgatg tgactccaga gccagttatt
3841 aggaagagca agctcaccac agaggagtgg aactgaggcc ccccagatgt tgcctccggt
3901 gtccaagcca cagcggtctg gctgttggga agatggccag gaatggactc ataccattgg
3961 cacattaggc taatcctggt tttatgtgaa gtcagcaatt aagtgttccc actagaactg
4021 acctaagcca ctgattaata tttaatgagg gaaggtaggg gagaatctag ccattttata
4081 atgccagaaa tctatatatg ttatctgatg ccatttttct gaagtagcct cacatgtggt
4141 ccccctgcag ttcagcagtt aacagatgac ttttttagtg taataaaatg tttatcatct
4201 atg//

- LOCUS: AW118072 718 bp mRNA linear EST 20-OCT-1999
- DEFINITION: xe03a06.x1 Soares_NFL_T_GBC_S1 Homo sapiens cDNA clone IMAGE:2606002 3', mRNA sequence. *signal recognition particle receptor, B* subunit *(SRPRB)*
- ACCESSION: AW118072
- VERSION: AW118072.1 GI:6086656
- KEYWORDS: EST.

1 tttttttttt tttttttgag aattaggaca gtttattgtt tgaccaacat gctgagtctt
61 ttccacattt tacacagttt aatgtgaaat caacatggcg gctatgtctt ctgagcccat
121 aacaaatgga attgccaccc tttgtgctcc tcacagccaa tcactttaaa gggatgggtg
181 aggggaaagt gaggggagaa gtggacacac accccnagaa tgcggctggc cttcaatgct
241 atggaggctt cccacctcct gaaggaacca tctaaacccc tgctgcaagg atttcctgat
301 gaaaccacac actgctggga gtgccaacca aacaggggtc tggagtccaa ggagtttgca
361 cattgagatc ccaaggtttt ggaacaccta aatagttcat gtcaaacaaa aattcaaagg
421 gtgtcctgat ctgtgtgggt gcccatgaca atcaatcana gtaaacttgg ggactggccc
481 ttgtgcagta gaggagccca aaataccacc aatattctca ctcatatgct gggaagaacc
541 tagtgtccta accaaaaaga gtagagatgg tctgaggaac acacccaccc atagcagtcc
601 ctgctgtgta ataaatatga gtcacatttg ttccacacca ggcagcaatg ggataaaaat
661 gggaacctta actgttctga tcacttgtaa ataagctggt tgcttnttcc ctcgtgcc//

- LOCUS: BF195526 550 bp mRNA linear EST 03-NOV-2000
- DEFINITION: 7n89c08.x1 NCI_CGAP_Ov18 Homo sapiens cDNA clone IMAGE:3571599 3', mRNA sequence. *heterogeneous nuclear ribonucleoprotein A3 (HNRPA3P1)*
- ACCESSION: BF195526
- VERSION: BF195526.1 GI:11082509
- KEYWORDS: EST.

1 gacaatttag ctcaatttta atgtttccca agcattattt tgaccaggta cccaggttta
61 agttatgaac attgacagtg tccattatat aaccacactt gaagttattt aggacttaac
121 cattttctaa tattagccta ttttctacac tgcttttcac atatatgccc attaacaaat
181 ggaatgtctg ttacatttat tggtttgtga atgtttctgg aaaactgcag tatatgtgaa
241 gaccaatttc catgctggca ttgcatgcat ccaaatatta atgcacagag gcacagaatt
301 agagcaacaa gagagcatat tcaaacacta gcacacccca ttcccctttt tattgcttgt
361 ttgcttagta cttcttaaaa caaaagactt gaattcaacg ttcaactacc aaagagagta
421 acagcagggc acatactcag ggctcgaatg aaactgtaag cactagctgg agcaaaacag
481 ttagacattg gtttatatta atatatgtat atatgacctt attaactaaa actcttctca
541 accagggcct//

- LOCUS: BF033354 657 bp mRNA linear EST 20-OCT-2000
- DEFINITION: 601458055F1 NIH_MGC_66 Homo sapiens cDNA clone IMAGE:3861709 5',mRNA sequence. *splicing factor, arginine*/*serine-rich 7, 35kDa (SFRS7)*
- ACCESSION: BF033354
- VERSION: BF033354.1 GI:10741066
- KEYWORDS: EST.

1 cggagatcac gtttgataga ccacctgccc gacgtccctt tgatccaaat gatagatgct
61 atgagtgtgg cgaaaaggga cattatgctt atgattgtca tcgttacagc cggcgaagaa
121 gaagcaggtc acggtctaga tcacattctc gatccagagg aaggcgatac tctcgctcac
181 gcagcaggag caggggacga aggtcaaggt cagcatctcc tcgacgatca agatctatct
241 ctcttcgtag atcaagatca gcttcactca gaagatctag gtctggttct ataaaaggat
301 cgaggtattt ccaatccccg tcgaggtcaa gatcaagatc caggtctatt tcacgaccaa
361 gaagcagtcg ttccccatca ggaagtcctc gcagaagtgc aagtcctgaa agaatggact
421 gaagctctca agttcaccct ttagggaaaa gttatttgtt tacattatta taaaggatt
481 gtgatgtctg taaaggtgta acctaggaaa gataattcaa catctaatca aaatggatct
541 ggattactat gtaaattcac agcagtaaga taatataaat tttgttgaat gtattaacat
601 catatggtct gaaaaatgtg ggtctttatt tggcacattt acaatcacat gtttcta//

- LOCUS: NM_006365 879 bp mRNA linear PRI 10-JAN-2007
- DEFINITION: Homo sapiens chromosome 1 open reading frame 61 (Clorf61), mRNA. *transcriptional activator of the c-fos promoter (CROC4)*
- ACCESSION: NM_006365
- VERSION: NM_006365.1 GI:5453624

1 ctcctcacag aagcctggag ctgggcatcc aagaagaagc agcctcattt gttttctggt
61 gtcatcgtag gtggccacct atggcttttg ggaatgtaaa aagggcagct ctctggcatg
121 ttcctgactg aggatctcat aacatttaac ttgaggaact tcctcctttt ccagctttgg
181 gagtcaagct tctcacctgg ggcgggtggg ttctgcacca ccctcccacc ctccttcctc
241 cgtgtggacg atagagccac atccagcacc acggacagct cccgggcgcc ttcatctcct
301 cgtcctccag gcagcacaag ccattgtgga atctccacca ggtgtacaga acggtgcctc
361 tgcgtcctgc cactcaggac ctctcaagtc cccgatgtga tggctcctca gcatgatcag
421 gagaaattcc atgatcttgc ttattcctgt cttgggaagt ccttctccat gtctaaccaa
481 gatctatatg gctatagcac cagctctttg gctcttggct tggcatggct aagttgggag
541 accaaaaaga agaatgtact tcatctggtt gggctggatt ccctctgata agccttccca
601 gttgactgaa agatgaggct aggctctagc aagttgaagt caaaccagct ccttcaagaa
661 gctttgagca gaatgaagtg gggaggaccc agcttccagc ccaggaagcc cactgtacct
721 ggagccatct gggataagac tttgacccat gactcccata tccacagcct gtccatccta
781 gcccatccca gtttatcctg tatcatttga gctgggattc ccacatcctc tgagttggaa
841 gtcccatctc aagtcttcaa taaagactct tgaatattg//

- LOCUS: AL080144 2524 bp mRNA linear PRI 18-FEB-2000
- DEFINITION: Homo sapiens mRNA; cDNA DKFZp434N093 (from clone DKFZp434N093); partial cds. *ELYS transcription factor-like protein TMBS62 (ELYS)*
- ACCESSION: AL080144
- VERSION: AL080144.1 GI:5262592

1 ggtgattgaa caagaaaagc ttgaagctca agattcagga gaagaggcta ggaatctttc
61 atttaatgag ttatatccct ctggaacact taagcttcag tacaattttg atactattga
121 ccaacagttt tgtgacttag ctgataacaa agacactgct gaatgtgaca ttgctgaagt
181 agatggggaa ctttttgtgg ctcaaagcaa ctttaccttg atattggaag gtgaagaagg
241 agaagttgag ccaggtgatt ttgcatcatc tgatgtgtta cctaaagcag ctaacacagc
301 aactgaagaa aaacttgtat gcagtgggga aaatgataat catggacaaa ttgcaaattt
361 gccatctgcc gtaactagtg accaaaagtc ccaaaaagta gacactttac catatgtgcc
421 tgaacctatt aaagtagcaa ttgcagaaaa tttactagat gtaattaaag acacaagaag
481 taaagaaatt acttcagata caatggaaca gtccattcat gaaacaatac ctttagtgag
541 ccaaaacata atgtgtccca ctaaattggt caaatctgca tttaagactg ctcaggaaac
601 aagcacaatg actatgaatg tcagccaggt tgatgacgtg gtttccttca aaactcgtac
661 gagaggtcaa cgtatccaaa acgtgaatgt caaatcagca caacaggaag catcagcaga
721 tgttgctact cctaagatgc cagggcagtc agtcaggaag aaaactagga aggcaaaaga
781 aatttctgaa gcttctgaaa acatctattc tgatgtcaga ggactatctc agaaccagca

841 aatacctcaa aattctgtta cgcctaggag aggaaggaga aagaaagaag ttaatcagga
901 catactagaa aacaccagtt ctgtggaaca agaattacag atcactacag gtagggaatc
961 aaaaagatta aaatcatctc agctgttgga accagcagtt gaagaaacta ctaaaaaaga
1021 agttaaggtt tcatctgtta caaaaaggac tcctagaaga attaaaagat ctgtagaaaa
1081 tcaggaaagt gttgaaatta taaatgatct aaaagttagt acggtaacaa gtcctagcag
1141 aatgatcaga aaattgagaa gtactaattt agatgcttct gaaaatacag gaaataagca
1201 agatgataaa tccagtgaca agcagctgcg tattaaacat gttagaaggg tcagagggag
1261 agaagttagt ccatcagatg tgagagaaga ctccaacctt gagtcatctc agttgactgt
1321 tcaagcagaa tttgatatgt ctgccatacc tagaaaacgt ggtagaccaa gaaaaatcaa
1381 tccatctgaa gatgtaggat ctaaggctgt taaggaagag agaagcccca agaagaaaga
1441 agctcccagc attagaagga gatctacaag aaatacccca gctaaaagtg aaaatgttga
1501 tgttggaaaa ccagctttag gaaaatccat tttagtgcca aacgaggaac tttcgatggt
1561 gatgagctct aagaaaaaac ttacaaaaaa gactgaaagt caaagccaaa aacgttcatt
1621 gcactcagta tcagaagaac gcacagatga aatgacacat aaagaaacaa atgagcagga
1681 agaaagattg ctcgccacag cttccttcac taaatcatcc cgcagcagca ggactcggtc
1741 tagcaaggcc atcttgttgc cggacctttc tgaaccaaac aatgagcctt tattttctcc
1801 agcgtcagaa gttccaagga aagcaaaagc taaaaaaata gaggttcctg cacagctgaa
1861 agaattagtt tcggatttat cttctcagtt tgtcatctca cctcctgctt taaggagcag
1921 acaaaaaaac acatccaata agaacaagct tgaagatgaa ctgaaagatg atgcacaatc
1981 agtagaaact ctgggaaagc caaaagcgaa acgaatcagg acgtcaaaaa caaaacaagc
2041 aagcaaaaac acagaaaaag aaagtgcttg gtcacctcct cccatagaaa ttcggctgat
2101 ttcccccttg gctagcccag ctgacggagt caagagcaaa ccaagaaaaa ctacagaagt
2161 gacaggaaca ggtcttggaa ggaacagaaa gaaactgtct tcctatccaa agcaaatttt
2221 acgcagaaaa atgctgtaat ttcttgggaa gattttaatg tacacctatt tgtaaagtca
2281 tcagaatagt gtggattatt aaatatctag tttggaagaa aataatttat ataaattatt
2341 gtaaattttt atgtaaacag aaggtcttca ataagtaaag taactccata tggagtgatt
2401 gtttcagtcc aggcaatttt tctattttat attaagactt catacattta tatatgtaaa
2461 tatggcttat taatggaatg ttaaataaaa tgtatacttc acaaaaaaaa aaaaaaaaaa
2521 aaaa//

- LOCUS: NM_024844 2311 bp mRNA linear PRI 01-MAR-2007
- DEFINITION: Homo sapiens nucleoporin 85kDa (NUP85), mRNA.
- ACCESSION: NM_024844
- VERSION ORIGIN: NM_024844.3 GI:82880675

1 cggtactctg agtgcagtca gtcctcggcg ctgttttccc tgatttgctg tcgacgccag
61 gaaggaagga cgcgtgcaga ggaccgcaga ggggtggccg tggctgagag gagacagcgc
121 cgcagcactg agggtttggg cttgcaggcg ctgcaggaga cgcccaggcg gagtcttgtc
181 tcgcagccag ctctgagcgg gaggcctgag cgggaagcat tggcgtccga gcgacttcta
241 ggagcctggg gttcggcgct atggaggagc tcgatggcga gccaacagtc actttgattc
301 caggcgtgaa ttccaagaag aaccaaatgt attttgactg gggtccaggg gagatgctgg
361 tatgtgaaac ctccttcaac aaaaaagaaa aatcagagat ggtgccaagt tgccccttta
421 tctatatcat ccgtaaggat gtagatgttt actctcaaat cttgagaaaa ctcttcaatg
481 aatcccatgg aatctttctg ggcctccaga gaattgacga agagttgact ggaaaatcca
541 gaaaatctca attggttcga gtgagtaaaa actaccgatc agtcatcaga gcatgtatgg
601 aggaaatgca ccaggttgca attgctgcta aagatccagc caatggccgc cagttcagca
661 gccaggtctc cattttgtca gcaatggagc tcatctggaa cctgtgtgag attcttttta
721 ttgaagtggc cccagctggc cctctcctcc tccatctcct tgactgggtc cggctccatg
781 tgtgcgaggt ggacagtttg tcggcagatg ttctgggcag tgagaatcca agcaaacatg
841 acagcttctg gaacttggtg accatcttgg tgctgcaggg ccggctggat gaggcccgac
901 agatgctctc caaggaagcc gatgccagcc ccgcctctgc aggcatatgc cgaatcatgg
961 gggacctgat gaggacaatg cccattctta gtcctgggaa cacccagaca ctgacagagc
1021 tggagctgaa gtggcagcac tggcacgagg aatgtgagcg gtacctccag gacagcacat
1081 tcgccaccag ccctcacctg gagtctctct tgaagattat gctgggagac gaagctgcct
1141 tgttagagca gaaggaactt ctgagtaatt ggtatcattt cctagtgact cggctcttgt
1201 actccaatcc cacagtaaaa cccattgatc tgcactacta tgcccagtcc agcctggacc
1261 tgtttctggg aggtgagagc agcccagaac ccctggacaa catcttgttg gcagcctttg
1321 agtttgacat ccatcaagta atcaaagagt gcagcatcgc cctgagcaac tggtggtttg
1381 tggcccacct gacagacctg ctggaccact gcaagctcct ccagtcacac aacctctatt
1441 tcggttccaa catgagagag ttcctcctgc tggagtacgc ctcgggactg tttgctcatc
1501 ccagcctgtg gcagctgggg gtcgattact ttgattactg ccccgagctg ggccgagtct

1561 ccctggagct gcacattgag cggatacctc tgaacaccga gcagaaagcc ctgaaggtgc
1621 tgcggatctg tgagcagcgg cagatgactg aacaagttcg cagcatttgt aagatcttag
1681 ccatgaaagc cgtccgcaac aatcgcctgg gttctgccct ctcttggagc atccgtgcta
1741 aggatgccgc ctttgccacg ctcgtgtcag acaggttcct cagggattac tgtgagcgag
1801 gctgcttttc tgatttggat ctcattgaca acctggggcc agccatgatg ctcagtgacc
1861 gactgacatt cctgggaaag tatcgcgagt tccaccgtat gtacggggag aagcgttttg
1921 ccgacgcagc ttctctcctt ctgtccttga tgacgtctcg gattgcccct cggtctttct
1981 ggatgactct gctgacagac gccttgcccc ttttggaaca gaaacaggtg attttctcag
2041 cagaacagac ttatgagttg atgcggtgtc tggaggactt gacgtcaaga agacctgtgc
2101 atggagaatc tgataccgag cagctccagg atgatgacat agagaccacc aaggtggaaa
2161 tgctgagact ttctctggca cgaaatcttg ctcgggcaat tataagagaa ggctcactgg
2221 aaggttcctg agaactgctt caatgtggta tctttgtatg gcaatgtata tagatttttt
2281 taaaagaata aatgttgttt tgcaaatgta g//

- LOCUS: AF047338 3613 bp mRNA linear PRI 26-AUG-1998
- DEFINITION: Homo sapiens erythroid K:C1 cotransporter (KCC1) mRNA, complete cds., (SLC12A4)
- ACCESSION: AF047338
- VERSION ORIGIN: AF047338.1 GI:2921846

1 atgcctcact tcaccgtggt gccagtggac gggccgaggc gcggcgacta tgacaacctc
61 gaggggctca gttgggtgga ctacggggag cgcgccgagc tggatgactc ggacggacat
121 ggcaaccaca gagagagcag cccttttctt tcccccttgg aggcttccag aggaattgac
181 tactatgaca ggaacctggc actgtttgag gaagagctgg acatccgccc aaaggtatcg
241 tctcttctgg gaaagctcgt cagctacacc aacctcaccc agggcgccaa agagcatgag
301 gaggccgaga gtggggaggg cacccgccgg agggcagccg aggcacccag catgggcacc
361 ctcatggggg tgtacctgcc ctgcctgcag aatatctttg gggttatcct cttcctgcgg
421 ctgacctgga tggtgggcac agcaggtgtg ctacaggccc tcctcatcgt gcttatctgc
481 tgctgttgta ccctgctgac ggccatctcc atgagtgcca tcgccaccaa cggtgtggtt
541 ccagctgggg gctcctattt catgatctct cgttcactgg ggccagaatt tggaggtgct
601 gtgggcctgt gcttctacct gggaacaaca ttcgcagcag ccatgtacat cctgggggcc
661 atcgagatct tgctgaccta cattgcccca ccagctgcca ttttttaccc atcgggtgct
721 catgacacgt cgaatgccac tttgaacaat atgcgtgtgt atgggaccat tttcctgacc
781 ttcatgaccc tggtggtgtt tgtgggggtc aagtatgtga acaaatttgc ctcgctcttc
841 ctggcctgtg tgatcatctc catcctctcc atctatgctg ggggcataaa gtctatattt
901 gaccctcccg tgtttccggt atgcatgctg ggcaacagga ccctgtcccg ggaccagttt
961 gacatctgtg ccaagacagc tgtagtggac aatgagacag tggccaccca gctatggagt
1021 ttcttctgcc acagccccaa ccttacgacc gactcctgtg acccctactt catgctcaac
1081 aatgtgaccg agatccctgg catccccggg gcagctgctg gtgtgctcca ggaaaacctg
1141 tggagcgcct acctggagaa gggtgacatc gtggagaagc atggctgccc ctccgcagat
1201 gccccgagcc tgaaggagag cctgcctctg tacgtggtcg ctgacatcgc cacatccttc
1261 accgtgctgg tcggcatctt cttcccttct gtaacaggca tcatggctgg ctcaaaccgc
1321 tctggggacc ttcgtgacgc ccagaagtct atccctgtgg ggaccattct ggccatcatt
1381 acaacttccc tcgtgtactt cagcagtgtg gttctctttg gtgcctgcat tgagggtgtg
1441 gttctccggg acaagtatgg cgatggtgtc agcaggaact tggtggtggg cacactggcc
1501 tggccttcac cctgggtcat cgtcatcggc tccttctttt caacgtgtgg cgctggcctc
1561 cagagcctca caggggcacc acgcctattg caggccattg ccaaggacaa catcatcccc
1621 ttcctccggg tgtttggcca cgggaaggtg aatggtgaac ccacatgggc actcctcctg
1681 acggcactca tcgccgagct gggcatcctc atcgcctccc tcgacatggt ggcccccatc
1741 ttatccatgt tctttctgat gtgctacctg ttcgtgaacc tcgcctgtgc ggtgcagaca
1801 ctcctgagga cccccaactg gcggccccgg ttcaagtact atcactgggc gctgtccttc
1861 ctgggcatga gtctctgcct ggcccttatg tttgtctcct cctggtacta tgccctggtg
1921 gccatgctca tcgccggcat gatctacaaa tacatcgagt accaaggggc tgagaaggag
1981 tggggtgacg ggatccgagg cctgtccctg agcgctgccc gctacgcgct gttgcggctg
2041 gaggaggggc ctcctcacac caagaactgg cggccgcagc tgctggtgct gctgaagctg
2101 gacgaggacc tccacgtgaa gtacccgcgg ctcctcacct tcgcctccca gctcaaggct
2161 ggcaagggcc tgaccattgt tggttctgtc atccagggga gcttcttgga gagctatggc
2221 gaggctcagg ccgccgagca gaccatcaag aacatgatgg aaattgagaa ggtgaagggc
2281 ttctgccagg tggtggtggc cagcaaggtg cgggaggggc tggcccacct catccagtcc
2341 tgtggcctgg gaggcatgcg gcataactcc gtggtgctgg gctggcccta cggctggcga
2401 cagagcgagg acccccgtgc ctggaagacc ttcattgaca ccgtgcgctg cactacggct
2461 gcccacctgg ccctgctcgt gcccaagaac atcgccttct accccagcaa ccacgagcgc
2521 tacctggagg gccacataga cgtgtggtgg atcgtgcacg atggtggcat gctcatgctt

2581 ctgcccttcc tgctgcgcca gcataaggtc tggaggaagt gccggatgcg catcttcaca
2641 gtggcccaga tggatgacaa cagcatccag atgaagaagg acctggctgt ctttctgtac
2701 catctgcgcc ttgaggccga ggtggaggtg gtggagatgc ataacagtga catctctgca
2761 tacacctacg agcggacgct gatgatggag cagcggtcgc agatgctgcg gcagatgaga
2821 ctgaccaaga ctgagcggga gcgagaagcc cagctggtca aggatcggca ctcggccctg
2881 cggctggaga gcctgtactc ggacgaggaa gatgagtctg cagtgggggc tgacaagatc
2941 cagatgacgt ggaccaggga caagtacatg actgagacct gggaccccag ccatgcccct
3001 gacaatttcc gggagctggt gcacattaag ccggaccaat ccaatgtgcg gcgcatgcac
3061 actgctgtga agctcaatga agtcattgtc acgcgctccc acgacgcccg cctggttctc
3121 ctaaacatgc ctggcccacc caggaacagt gagggcgacg agaactacat ggagttcctc
3181 gaggtgctga ccgagggcct tgagcgggtg ctgttggtgc gcggtggtgg ccgtgaagtc
3241 atcaccatct actcctgagc ccagtgtcat cttgtggcct ggagtcgagg tcttggccag
3301 gacataacaa gctgtggtct ggggtaacag cctcttccca gcacccacct gccagccctg
3361 cttgcctggc cctgtcctgg acccagcttt gctaggtctc cttggaaacc aggcctgggc
3421 ctcaaaatgg agatggatcc caggtcttgt gggaccctgg gatgtttggg gactttacta
3481 tctagcaccc cagtaggcct gtcctggcca gagaagactg gtaggggccg agtggggttt
3541 gaaggcagcc ggcccggccc agcccaggag cgctatttat tgcatattta ttgtttggat
3601 gtcaccatca gag//

- LOCUS: AB046836 3957 bp mRNA linear PRI 22-FEB-2001
- DEFINITION: Homo sapiens mRNA for KIAA1616 protein, partial cds., transient receptor potential cation channel(TRPM3)
- ACCESSION: AB046836
- VERSION ORIGIN: AB046836.1 GI:10047308

1 cccaggagct gaatcacaat tccagagact ttggccagct ggctgtggag ctcctggacc
61 agtcctacaa gcaggacgaa cagctggcca tgaaactgct gacgtatgag ctgaagaact
121 ggagcaacgc cacgtgcctg cagcttgccg tggctgccaa acaccgcgac ttcatcgcgc
181 acacgtgcag ccagatgctg ctcaccgaca tgtggatggg ccggctccgc atgcgcaaga
241 actcaggcct caaggtaatt ctgggaattc tacttcctcc ttcaattctc agcttggagt
301 tcaagaacaa agacgacatg ccctatatgt ctcaggccca ggaaatccac ctccaagaga
361 aggaggcaga agaaccagag aagcccacaa aggaaaaaga ggaagaggac atggagctca
421 cagcaatgtt gggacgaaac aacggggagt cctccaggaa gaaggatgaa gaggaagttc
481 agagcaagca ccggttaatc cccctcggca gaaaaatcta tgaattctac aatgcaccca
541 tcgtgaagtt ctggttctac acactggcgt atatcggata cctgatgctc ttcaactata
601 tcgtgttagt gaagatggaa cgctggccgt ccacccagga atggatcgta atctcctata
661 ttttcaccct gggaatagaa aagatgagag agattctgat gtcagagcca gggaagttgc
721 tacagaaagt gaaggtatgg ctgcaggagt actggaatgt cacggacctc atcgccatcc
781 ttctgttttc tgtcggaatg atccttcgtc tccaagacca gcccttcagg agtgacggga
841 gggtcatcta ctgcgtgaac atcatttact ggtatatccg tctcctagac atcttcggcg
901 tgaacaagta tttgggcccg tatgtaatga tgattggaaa aatgatgata gacatgatgt
961 actttgtcat cattatgctg gtggttctga tgagctttgg ggtcgccagg caagccatcc
1021 tttttcccaa tgaggagcca tcatggaaac tggccaagaa catcttctac atgccctatt
1081 ggatgattta tggggaagtg tttgcggacc agatagaccc tccctgtgga cagaatgaga
1141 cccgagagga tggtaaaata atccagctgc ctccctgcaa gacaggagct tggatcgtgc
1201 cggccatcat ggcctgctac ctcttagtgg caaacatctt gctggtcaac ctcctcattg
1261 ctgtctttaa caatacattt tttgaagtaa aatcgatatc caaccaagtc tggaagtttc
1321 agaggtatca gctcatcatg actttccatg aaaggccagt tctgccccca ccactgatca
1381 tcttcagcca catgaccatg atattccagc acctgtgctg ccgatggagg aaacacgaga
1441 gcgacccgga tgaaagggac tacggcctga aactcttcat aaccgatgat gagctcaaga
1501 aagtacatga ctttgaagag caatgcatag aagaatactt cagagaaaag gatgatcggt
1561 tcaactcatc taatgatgag aggatacggg tgacttcaga aagggtggag aacatgtcta
1621 tgcggctgga ggaagtcaac gagagagagc actccatgaa ggcttcactc cagaccgtgg
1681 acatccggct ggcgcagctg gaagacctta tcgggcgcat ggccacggcc ctggagcgcc
1741 tgacaggtct ggagcgggcc gagtccaaca aaatccgctc gaggacctcg tcagactgca
1801 cggacgccgc ctacattgtc cgtcagagca gcttcaacag ccaggaaggg aacaccttca
1861 agctccaaga gagtatagac cctgcaggtg aggagaccat gtccccaact tctccaacct
1921 taatgccccg tatgcgaagc cattctttct attcggtcaa tatgaaagac aaaggtggta
1981 tagaaaagtt ggaaagtatt tttaaagaaa ggtccctgag cctacaccgg gctactagtt
2041 cccactctgt agcaaaagaa cccaaagctc ctgcagcccc tgccaacacc ttggccattg
2101 ttcctgattc cagaagacca tcatcgtgta tagacatcta tgtctctgct atggatgagc
2161 tccactgtga tatagaccct ctggacaatt ccgtgaacat ccttgggcta ggcgagccaa
2221 gcttttcaac tccagtacct tccacagccc cttcaagtag tgcctatgca acacttgcac

2281 ccacagacag acctccaagc cggagcattg attttgagga catcacctcc atggacacta
2341 gatctttttc ttcagactac acccacctcc cagaatgcca aaacccctgg gactcagagc
2401 ctccgatgta ccacaccatt gagcgttcca aaagtagccg ctacctagcc accacaccct
2461 ttcttctaga agaggctccc attgtgaaat ctcatagctt tatgttttcc ccctcaagga
2521 gctattatgc caactttggg gtgcctgtaa aaacagcaga atacacaagt attacagact
2581 gtattgacac aaggtgtgtc aatgcccctc aagcaattgc ggacagagct gccttccctg
2641 gaggtcttgg agacaaagtg gaggacttaa cttgctgcca tccagagcga gaagcagaac
2701 tgagtcaccc cagctctgac agtgaggaga atgaggccaa aggccgcaga gccaccattg
2761 caatatcctc ccaggagggt gataactcag agagaaccct gtccaacaac atcactgttc
2821 ccaagataga gcgcgccaac agctactcgg cagaggagcc aagtgcgcca tatgcacaca
2881 ccaggaagag cttctccatc agtgacaaac tcgacaggca gcggaacaca gcaagcctgc
2941 gaaatccctt ccagagaagc aagtcctcca agccggaggg ccgaggggac agcctgtcca
3001 tgaggaaact gtccagaaca tcggctttcc aaagctttga aagcaagcac acctaaacct
3061 tcttaatatc cgccacagaa ggctcaagaa tccagcccta aaattctctc caactccagt
3121 ttttcccctt tccttgaatc atacctgctt tattcttagc tgagcaaaac aagcaatgct
3181 ttgggaggtg ttaactcaaa ggtgacttct gggccacaga tcaagaaagc atttgatctg
3241 acccagtgcc agacacaggg gatttaaggc atgttcacac ttgctgggca gggaggggga
3301 agagagggag aaggaagggt tagagatgaa tgtgtatccg cagtcacagc agaaagccat
3361 gagagcaggg gaaacaaggg gcttcgagca cgctccatgc caggaggcat ctgttgattt
3421 ctgaccatta tcaagagttg taggatgcag ggctaaattg caaaataaaa taaaatagcc
3481 agcgtacaca atgagatatt ctaaacttcc attctgtttt cttttcacat tggctccatc
3541 actggtgact gatgaagagc atcctcttta ttcagtataa gccggcagca agcagttcta
3601 cctaacgtcc cacatccttc tcatgccaac acttctgtaa ttgatcatta taaagaaaaa
3661 acaaggtaac agtcatagtt cacctgtctc ttatctattc acttctggtg ccacaactgt
3721 ttatcctttt ttgaagaaaa taagggaaca gaaatgcctt tttgtattgc aatcgaaatg
3781 aaagaagagt tgatgttaaa aaaacaaaag tcaagtgatt tattatatac agtgggcgtt
3841 caagtctagt cgagcaagct caggagaatg taattaaata attttatatt ttttaattta
3901 ttttgtatct cacctgtcat ggatgaattc attcactgaa tatgtaatat tgaactt//

- LOCUS: NM_007357 2904 bp mRNA linear PRI 17-NOV-2006
- DEFINITION: Homo sapiens component of oligomeric golgi complex 2 (COG2),mRNA.
- ACCESSION: NM_007357
- VERSION ORIGIN: NM_007357.1 GI:6678675

1 ggaaactggc ggtggccgcg gccgccgagt cggtctgcgc atcctcctgc gttttctcgc
61 ttggatcttg gcactgagag gcggtggccg gcgggatgga gaaaagtagg atgaacctgc
121 ccaaggggcc ggacacgctc tgcttcgaca aggacgagtt catgaaggaa gatttcgatg
181 tcgatcattt tgtgtctgac tgtaggaagc gggtccagct ggaagaactg agagatgacc
241 tggagctcta ctataaactt cttaaaacag ccatggtcga actcatcaac aaggattatg
301 cagattttgt caatctttca acaaacttgg ttggcatgga caaagccctc aaccagcttt
361 ctgtgccttt gggacaatta cgagaagagg ttctgagcct tagatcgtct gtcagtgaag
421 gaattcgggc agttgatgaa cgaatgtcta aacaagagga cattaggaaa aaaaagatgt
481 gtgtattgag gcttatacaa gttattcggt cagttgagaa aattgaaaaa atcttaaact
541 ctcaaagttc taaagaaacc tctgcactag aagcaagcag cccccttttg actggacaaa
601 ttttggagag aattgccaca gaatttaatc agttacagtt tcatgctgtt caaagcaaag
661 gcatgcctct tttggacaaa gtaagaccgc gtatagctgg cattacagcc atgttacagc
721 agtcactgga aggtctccta ttagaaggcc ttcagacgtc tgacgtcgat ataatacggc
781 actgcttgcg gacttacgcc acgattgaca agacacggga cgcggaggcc ttagttggcc
841 aagtactagt gaaaccatac atagacgagg tgattataga gcagtttgtt gaatctcatc
901 ccaatggcct tcaggtcatg tataataaac tcctggagtt tgttcctcac cattgccgcc
961 ttcttcgaga agtcacagga ggtgccatct ccagtgaaaa aggcaatact gttcctggat
1021 atgacttttt ggtgaattct gtttggccac aaatagtaca aggattagaa gaaaagttac
1081 cctcgctttt taatcctggg aatcccgatg catttcatga gaaatatacc ataagtatgg
1141 attttgtcag aagattggaa cggcagtgtg gatcacaggc tagtgtaaag agattaagag
1201 cccatcctgc ctatcacagc ttcaataaga agtggaactt gcctgtttat tttcaaataa
1261 gatttagaga aatagcggga tccttagaag cagcacttac agatgtcctg gaagatgccc
1321 cagctgaaag tccgtattgc cttttggctt ctcatagaac ttggagcagc cttaggaggt
1381 gttggtcaga tgagatgttc ttgccattac tggtgcatcg cctgtggaga ctcactctgc
1441 agattttggc acgatactct gtgtttgtca atgagctttc actcaggccc atttctaatg
1501 aaagtcccaa ggagatcaag aaacctttgg taactggtag caaagaacct tccatcaccc
1561 aaggaaacac tgaagaccaa ggaagtggtc cttcggaaac aaagcctgtg gtttccattt
1621 cccgcactca gctcgtgtat gtggttgcag acctggacaa gcttcaggag cagcttccag

1681 aactcttgga aataatcaag ccaaaacttg aaatgattgg ctttaagaat ttttcttcta
1741 tctcagcagc cctggaggac tcccagagct ctttttcagc ctgtgtgccc tccttgagta
1801 gcaagatcat ccaggattta agtgactctt gcttcggttt cctaaaaagc gccctggagg
1861 ttcccaggct ttaccgaaga accaataagg aggtcccaac cacagcttcc tcctatgtgg
1921 acagtgctct gaagccctta ttccagcttc agagcggaca caaggataag ctcaaacaag
1981 caataattca gcagtggcta gaaggcactc tcagtgaaag cactcataag tactatgaaa
2041 ccgtgtcaga tgtattaaac tctgtgaaga agatggaaga gagcctgaaa aggctgaaac
2101 aagccagaaa aaccactccc gccaaccccg tcggtcccag tggtggcatg agcgacgacg
2161 acaaaatcag gctgcagttg gccctagatg ttgagtactt gggagagcag atacaaaagt
2221 tgggactaca agcaagtgac ataaaaagct tctcagctct cgcagagctt gttgctgctg
2281 ccaaggacca ggcaacagca gagcagcctt aagcatcttg gaagatcccg aggttagatt
2341 cttaagcaag agaagagttg gacttccagg ctgaagggga gaaagtgact ctgttctctt
2401 agcaaccgtc tgtagcaaag aagtgcttcc agcatcactc cagcaacacg cccatgcgtc
2461 ttctctcagc gtatttgggt cttctttgcc caaaagaaca caaaagcctt tttccattgt
2521 atggaagata gtttttaaga catttgaaac tttctactat agtttacaga acaaattatt
2581 ttatttttat tgtaaatctt agtgtggaag agctgatttc taaaatatga ttaaagtaaa
2641 tatataccta tgaatatcaa gagtcgtctc cctgagcctg tagttggaag tgacgactgt
2701 aatggaatga tgtcttgtat agaaatgccc ttctctgaaa taaagagaac tcctgggctt
2761 tctaaagagg ctgcgggaag ccatcctcca ctcccactgt gtgtgagagc agtgcttctg
2821 atcctgctgt caccccgacc tctggcagga gccggcgcca gtaggaaaga cctccttcct
2881 aaataaaaga agtgtctccc aaaa//

- LOCUS: NM_005374 4412 bp mRNA linear PRI 18-NOV-2006
- DEFINITION: Homo sapiens membrane protein, palmitoylated 2 (MAGUK p55 subfamily member 2) (MPP2), mRNA.
- ACCESSION: NM_005374
- VERSION ORIGIN: NM_005374.3 GI:52630446

1 cttcccagtg ggactccagc agggcgtcgt ggtctgtgcg gtgtctccca aaccactacc
61 tggctgcccc cgtaccagct gctgggaaag actacttcct ccgagttgtg ccggcctccg
121 cgttctcctc acctcccctc tcctccggac ctccgccccc tcgcggagag gccttgccgc
181 tttaagagcc gggctagcga ttgacaagca ataaacgctg agcgcccggc tgcgctggag
241 ccgcccggag ctaggggctt cccggggcgc aggagagacg tttcagagcc cttgcctcct
301 tcaccatgcc ggttgccgcc accaactctg aaactgccat gcagcaagtc ctggacaact
361 tgggatccct ccccagtgcc acgggggctg cagagctgga cctgatcttc cttcgaggca
421 ttatggaaag tcccatagta agatccctgg ccaaggccca tgagaggctg gaggagacga
481 agctggaggc cgtgagagac aacaacctgg agctggtgca ggagatcctg cgggacctgg
541 cgcacgtggc tgagcagagc agcacagccg ccgagctggc ccacatcctc caggagcccc
601 acttccagtc cctcctggag acgcacgact ctgtggcctc aaagacctat gagacaccac
661 cccccagccc tggcctggac cctacattca gcaaccagcc tgtacctccc gatgctgtgc
721 gcatggtggg catccgcaag acagccggag aacatctggg tgtaacgttc cgcgtggagg
781 gcggcgagct ggtgatcgcg cgcattctgc atgggggcat ggtggctcaa caaggcctgc
841 tgcatgtggg tgacatcatc aaggaggtga acgggcagcc agtgggcagt gacccccgcg
901 cactgcagga gctcctgcgc aatgccagtg gcagtgtcat cctcaagatc ctgcccagct
961 accaggagcc ccatctgccc cgccaggtat ttgtgaaatg tcactttgac tatgacccgg
1021 cccgagacag cctcatcccc tgcaaggaag caggcctgcg cttcaacgcc ggggacttgc
1081 tccagatcgt aaaccaggat gatgccaact ggtggcaggc atgccatgtc gaagggggca
1141 gtgctgggct cattcccagc cagctgctgg aggagaagcg gaaagcattt gtcaagaggg
1201 acctggagct gacaccaaac tcagggaccc tatgcggcag cctttcagga aagaaaaaga
1261 agcgaatgat gtatttgacc accaagaatg cagagtttga ccgtcatgag ctgctcattt
1321 atgaggaggt ggcccgcatg cccccgttcc gccggaaaac cctggtactg attggggctc
1381 agggcgtggg acggcgcagc ctgaagaaca agctcatcat gtgggatcca gatcgctatg
1441 gcaccacggt gccctacacc tcccggcggc cgaaagactc agagcgggaa ggtcagggtt
1501 acagctttgt gtcccgtggg gagatggagg ctgacgtccg tgctgggcgc tacctggagc
1561 atggcgaata cgagggcaac ctgtatggca cacgtattga ctccatccgg ggcgtggtcg
1621 ctgctgggaa ggtgtgcgtg ctggatgtca acccccaggc ggtgaaggtg ctacgaacgg
1681 ccgagtttgt cccttacgtg gtgttcatcg aggccccaga cttcgagacc ctgcgggcca
1741 tgaacagggc tgcgctggag agtggaatat ccaccaagca gctcacggag gcggacctga
1801 gacggacagt ggaggagagc agccgcatcc agcggggcta cgggcactac tttgacctct
1861 gcctggtcaa tagcaacctg gagaggacct tccgcgagct ccagacagcc atggagaagc
1921 tacggacaga gccccagtgg gtgcctgtca gctgggtgta ctgagcctgt tcacctggtc
1981 tttggctcac tctgtgttga aacccagaac ctgaatccat ccccctcctg acctgtgacc
2041 ccctgccaca atccttagcc cccatatctg gctgtccttg ggtaacagct cccagcaggc

2101 cctaagtctg gcttcagcac agaggcgtgc actgccaggg aggtgggcat tcatggggta
2161 ccttgtgccc aggtgctgcc cactcctgat gcccattggt caccagatat ctctgagggc
2221 caagctatgc ccaggaatgt gtcagagtca cctccataat ggtcagtaca gagaagagaa
2281 aagctgcttt gggaccacat ggtcagtagg cacactgccc cctgccaccc ctccccagtc
2341 accagttctc ctctggactg gccacaccca ccccattcct ggactcctcc cacctctcac
2401 ccctgtgtcg gaggaacagg ccttgggctg tttccgtgtg accaggggaa tgtgtggccc
2461 gctggcagcc aggcaggccc gggtggtggt gccagcctgg tgccatcttg aaggctggag
2521 gagtcagagt gagagctagt ggccacagct gcagagcact gcagctccca gctcctttgg
2581 aaagggacag ggtcgcaggg cagatgctgc tcggtccttc cctcatccac agcttctcac
2641 tgccgaagtt tctccagatt tctccaatgt gtcctgacag gtcagccctg ctccccacag
2701 ggccaggctg gcaggggcca gtgggctcag cccaggtagg ggcaggatgg agggctgagc
2761 cctgtgacaa cctgctgtta ccaactgaag agccccaagc tctccatggc ccacagcagg
2821 cacaggtctg agctctatgt ccttgacctt ggtccatttg gttttctgtc tagccaggtc
2881 caggtagccc acttgcatca gggctgctgg gttggagggg ctaaggagga gtgcagaggg
2941 gaccttggga gcctgggctt gaaggacagt tgccctccag gaggttcctc acacacaact
3001 ccagaggcgc catttacact gtagtctgta caacctgtgg ttccacgtgc atgttcggca
3061 cctgtctgtg cctctggcac caggttgtgt gtatgtgtgt gcgtgtgcac gtgcgtgtgt
3121 gtgtgtgtgt gtcaggttta atttggggag gaagcaaagg gttttgtttt ggaggtcact
3181 ctttggggcc cctttctggg ggttccccat cagccctcat ttcttataat accctgatcc
3241 cagactccaa agccctggtc ctttcctgat gtctcctccc ttgtcttatt gtccccctac
3301 cctaaatgcc cccctgccat aacttgggga gggcagtttt gtaaaatagg agactccctt
3361 taagaaagaa tgctgtccta gatgtacttg ggcatctcat ccttcattat tctctgcatt
3421 ccttccgggg ggagcctgtc ctcagagggg acaacctgtg acaccctgag tccaaaccct
3481 tgtgcctccc agttcttcca agtgtctaac tagtcttcgc tgcagcgtca gccaaagctg
3541 gcccctgaac cactgtgtgc ccatttccta gggaagggga aggagaataa acagaatatt
3601 tattacaaat gttagaatat atttcttata ctaggaatct catttgcatt tgcatagact
3661 atacacatgg ggtggaaagg ccaggcctgc ccccatctcg ttggtgtggc tctgcgtata
3721 ctacacactc attctcctgc tcctcttttc ccttagtcag tgtcctttca tcctgattca
3781 gctctgcctt gcatcaccct cagcctaagg gagtgggaag gaaatggggt gttttcttgc
3841 tgacctgagg ctatagggtc acttgccatt tcctaccttc tctgggggat ttgagggtag
3901 aggcagggga agatctgttg ttgcagttgc ttctgccccc ttgatccaaa tgaccatcat
3961 ctctgatgga gatgggttgg gtacctggcc ttcatggcac cttcactgct agggatgctc
4021 aaggggcagg cctgggcccc ttccctcctg tctcttctcg gtctttcctc tctgagcagc
4081 ctcctacctc ccctgcctga gccctcactc cacagccctc ccaggtacct agcagaggct
4141 gtcagtcctt ggctcacctg gaacagggct ggggctgggt tggaacaggt gtgtgccccc
4201 accacagctc tatgactctg ttctccctcc ctgccattgt ggactcttgt atttgaggga
4261 cctcaagaga gtgaggaccc taccatccac tgtccatatt cagtcccagc cccagtgcgc
4321 ttcctctgtt ccctccctca gccatccaat tcttgagttt tctcactgat tggttttctt
4381 tctttttcct tggattaaat gtgaaagcaa ag//

- LOCUS: NM_001883 2107 bp mRNA linear PRI 11-FEB-2007
- DEFINITION: Homo sapiens corticotropin releasing hormone receptor 2
- (CRHR2),: mRNA.
- ACCESSION: NM_001883
- VERSION ORIGIN: NM_001883.2 GI:32307158

1 atggacgcgg cactgctcca cagcctgctg gaggccaact gcagcctggc gctggctgaa
61 gagctgctct tggacggctg ggggccaccc ctggaccccg agggtcccta ctcctactgc
121 aacacgacct tggaccagat cggaacgtgc tggccccgca gcgctgccgg agccctcgtg
181 gagaggccgt gccccgagta cttcaacggc gtcaagtaca acacgacccg gaatgcctat
241 cgagaatgct tggagaatgg gacgtgggcc tcaaagatca actactcaca gtgtgagccc
301 attttggatg acaagcagag gaagtatgac ctgcactacc gcatcgccct tgtcgtcaac
361 tacctgggcc actgcgtatc tgtggcagcc ctggtggccg ccttcctgct tttcctggcc
421 ctgcggagca ttcgctgtct gcggaatgtg attcactgga acctcatcac cacctttatc
481 ctgcgaaatg tcatgtggtt cctgctgcag ctcgttgacc atgaagtgca cgagagcaat
541 gaggtctggt gccgctgcat caccaccatc ttcaactact tcgtggtgac caacttcttc
601 tggatgtttg tggaaggctg ctacctgcac acggccattg tcatgaccta ctccactgag
661 cgcctgcgca agtgcctctt cctcttcatc ggatggtgca tccccttccc catcatcgtc
721 gcctgggcca tcggcaagct ctactatgag aatgaacagt gctggtttgg caaggagcct
781 ggcgacctgg tggactacat ctaccaaggc cccatcattc tcgtgctcct gatcaatttc
841 gtatttctgt tcaacatcgt caggatccta atgacaaagt tacgcgcgtc caccacatcc
901 gagacaatcc agtacaggaa ggcagtgaag gccaccctgg tgctcctgcc cctcctgggc

961 atcacctaca tgctcttctt cgtcaatccc ggggaggacg acctgtcaca gatcatgttc
1021 atctatttca actccttcct gcagtcgttc cagggtttct tcgtgtctgt cttctactgc
1081 ttcttcaatg gagaggtgcg ctcagccgtg aggaagaggt ggcaccgctg gcaggaccat
1141 cactcccttc gagtccccat ggcccgggcc atgtccatcc ctacatcacc cacacggatc
1201 agcttccaca gcatcaagca gacggccgct gtgtgacccc tcggtcgccc acctgcacag
1261 ctcccctgtc ctcctccacc ttcttcctct gggttctctg tgctgggcag gctctcgtgg
1321 ggcaggagat gggaggggag agaccagctc tccagcctgg caggaaagag ggggtgcggc
1381 agccaagggg gactgcaagg gacagggatg agtgggggcc accaggctca gcgcaagagg
1441 aagcagaggg aattcacagg accccctgag aagagccagt cagatgtctg caggcatttg
1501 cccatcccag cctctctggc cagggcctta ctgggcccag agcagagaag gacctgtcca
1561 acacacacag ctatttatag tagcagacac agggctcccc tgccctactc atggagccag
1621 cagccaggca atggtgtggc cctgcactgg cccttggact ccacactcag tggtgccctg
1681 cagttgggtg ggttacgcca gcaaaggatc agtttggctg ccttatccca gggctgtcac
1741 ctagagaggc tcacttgtac cccaccctgt tcctgtgtcc cctccccagc catcctcccg
1801 ccttgggggc tccatgaagg atgcaggctt ccaggcctgg cttcctctct tgggagaccc
1861 cttctctgcc tagtccacag attaggcaat caaggaagac gccatcaggg aagccacatc
1921 cttagtcaac cagttgcatc gtgcggggca aaatgaggag cagaggcatg gaggagggag
1981 gcgtgggatg ggaatagcag aaccaccatg tcttcagtga ttgaaactca taccccattg
2041 ccctttgccc tccagtctcc ccttcagaaa catctctgct ctctgtgaaa taaaccatgc
2101 ctcttgg//

- LOCUS: NM_022559 777 bp mRNA linear PRI 15-APR-2007
- DEFINITION: Homo sapiens growth hormone 1 (GH1), transcript variant 2,
- mRNA.:
- ACCESSION: NM_022559
- VERSION ORIGIN: NM_022559.2 GI:20809249

1 aggatcccaa ggcccaactc cccgaaccac tcagggtcct gtggacagct cacctagctg
61 caatggctac aggctcccgg acgtccctgc tcctggcttt tggcctgctc tgcctgccct
121 ggcttcaaga gggcagtgcc ttcccaacca ttcccttatc caggcttttt gacaacgcta
181 tgctccgcgc ccatcgtctg caccagctgg cctttgacac ctaccaggag tttaaccccc
241 agacctccct ctgtttctca gagtctattc cgacaccctc caacagggag gaaacacaac
301 agaaatccaa cctagagctg ctccgcatct ccctgctgct catccagtcg tggctggagc
361 ccgtgcagtt cctcaggagt gtcttcgcca acagcctggt gtacggcgcc tctgacagca
421 acgtctatga cctcctaaag gacctagagg aaggcatcca aacgctgatg gggaggctgg
481 aagatggcag cccccggact gggcagatct tcaagcagac ctacagcaag ttcgacacaa
541 actcacacaa cgatgacgca ctactcaaga actacgggct gctctactgc ttcaggaagg
601 acatggacaa ggtcgagaca ttcctgcgca tcgtgcagtg ccgctctgtg gagggcagct
661 gtggcttcta gctgcccggg tggcatccct gtgacccctc cccagtgcct ctcctggccc
721 tggaagttgc cactccagtg cccaccagcc ttgtcctaat aaaattaagt tgcatca//

- LOCUS: HUMFSHREC 2186 bp mRNA linear PRI 08-NOV-1994
- DEFINITION: H.sapiens follicle stimulating hormone receptor mRNA, complete cds.
- ACCESSION: M95489
- VERSION ORIGIN: M95489.1 GI:182772

1 gtgtggagct tctgagatct gtggaggttt ttctctgcaa atgcaggaag aaatcaggtg
61 gatggatgca taattatggc cctgctcctg gtctctttgc tggcattcct gagcttgggc
121 tcaggatgtc atcatcggat ctgtcactgc tctaacaggg tttttctctg ccaagagagc
181 aaggtgacag agattccttc tgacctcccg aggaatgcca ttgaactgag gtttgtcctc
241 accaagcttc gagtcatcca aaaaggtgca ttttcaggat ttggggacct ggagaaaata
301 gagatctctc agaatgatgt cttggaggtg atagaggcag atgtgttctc caaccttccc
361 aaattacatg aaattagaat tgaaaaggcc aacaacctgc tctacatcaa ccctgaggcc
421 ttccagaacc ttcccaacct tcaatatctg ttaatatcca acacaggtat taagcacctt
481 ccagatgttc acaagattca ttctctccaa aaagttttac ttgacattca agataacata
541 aacatccaca caattgaaag aaattctttc gtggggctga gctttgaaag tgtgattcta
601 tggctgaata agaatgggat tcaagaaata cacaactgtg cattcaatgg aacccaacta
661 gatgagctga atctaagcga taataataat ttagaagaat tgcctaatga tgttttccac
721 ggagcctctg gaccagtcat tctagatatt tcaagaacaa ggatccattc cctgcctagc
781 tatggcttag aaaatcttaa gaagctgagg gccaggtcga cttacaactt aaaaaagctg
841 cctactctgg aaaagcttgt cgccctcatg gaagccagcc tcacctatcc cagccattgc
901 tgtgcctttg caaactggag acggcaaatc tctgagcttc atccaatttg caacaaatct

961 attttaaggc aagaagttga ttatatgact cagactaggg gtcagagatc ctctctggca
1021 gaagacaatg agtccagcta cagcagagga tttgacatga cgtacactga gtttgactat
1081 gacttatgca atgaagtggt tgacgtgacc tgctccccta agccagatgc attcaaccca
1141 tgtgaagata tcatggggta caacatcctc agagtcctga tatggtttat cagcatcctg
1201 gccatcactg ggaacatcat agtgctagtg atcctaacta ccagccaata taaactcaca
1261 gtccccaggt tccttatgtg caacctggcc tttgctgatc tctgcattgg aatctacctg
1321 ctgctcattg catcagttga tatccatacc aagagccaat atcacaacta tgccattgac
1381 tggcaaactg gggcaggctg tgatgctgct ggctttttca ctgtctttgc cagtgagctg
1441 tcagtctaca ctctgacagc tatcaccttg gaaagatggc ataccatcac gcatgccatg
1501 cagctggact gcaaggtgca gctccgccat gctgccagtg tcatggtgat gggctggatt
1561 tttgcttttg cagctgccct ctttcccatc tttggcatca gcagctacat gaaggtgagc
1621 atctgcctgc ccatggatat tgacagccct ttgtcacagc tgtatgtcat gtccctcctt
1681 gtgctcaatg tcctggcctt tgtggtcatc tgtggctgct atatccacat ctacctcaca
1741 gtgcggaacc ccaacatcgt gtcctcctct agtgacacca ggatcgccaa gcgcatggcc
1801 atgctcatct tcactgactt cctctgcatg gcacccattt ctttctttgc catttctgcc
1861 tccctcaagg tgcccctcat cactgtgtcc aaagcaaaga ttctgctggt tctgtttcac
1921 cccatcaact cctgtgccaa cccctttctc tatgccatct ttaccaaaaa ctttcgcaga
1981 gatttcttca ttctgctgag caagtgtggc tgctatgaaa tgcaagccca aatttatagg
2041 acagaaactt catccactgt ccacaacacc catccaagga atggccactg ctcttcagct
2101 cccagagtca ccaatggttc cacttacata cttgtccctc taagtcattt agcccaaaac
2161 taaaacacaa tgtgaaaatg tatctg//

- LOCUS: NM_000055 2444 bp mRNA linear PRI 22-APR-2007
- DEFINITION: Homo sapiens butyrylcholinesterase (BCHE), mRNA.
- ACCESSION: NM_000055
- VERSION ORIGIN: NM_000055.1 GI:4557350

1 agtaacagtt gattgttaca ttcagtaaca ctgaatgtca gtgcagtcca atttacaggc
61 tggagcagca gctgcatcct gcatttcccc gaagtattac atgattttca ctccttgcaa
121 actttaccat ctttgttgca gagaatcgga aatcaatatg catagcaaag tcacaatcat
181 atgcatcaga tttctctttt ggtttctttt gctctgcatg cttattggga agtcacatac
241 tgaagatgac atcataattg caacaaagaa tggaaaagtc agaggatgaa acttgacagt
301 ttttggtggc acggtaacag cctttcttgg aattccctat gcacagccac ctcttggtag
361 acttcgattc aaaaagccac agtctctgac caagtggtct gatatttgga atgccacaaa
421 atatgcaaat tcttgctgtc agaacataga tcaaagtttt ccaggcttcc atggatcaga
481 gatgtggaac ccaaacactg acctcagtga agactgttta tatctaaatg tatggattcc
541 agcacctaaa ccaaaaaatg ccactgtatt gatatggatt tatggtggtg gttttcaaac
601 tggaacatca tctttacatg tttatgatgg caagtttctg gctcgggttg aaagagttat
661 tgtagtgtca atgaactata gggtgggtgc cctaggattc ttagctttgc caggaaatcc
721 tgaggctcca gggaacatgg gtttatttga tcaacagttg gctcttcagt gggttcaaaa
781 aaatatagca gcctttggtg gaaatcctaa aagtgtaact ctctttggag aaagtgcagg
841 agcagcttca gttagcctgc atttgctttc tcctggaagc cattcattgt tcaccagagc
901 cattctgcaa agtggatcct ttaatgctcc ttgggcggta acatctcttt atgaagctag
961 gaacagaacg ttgaacttag ctaaattgac tggttgctct agagagaatg agactgaaat
1021 aatcaagtgt cttagaaata aagatcccca agaaattctt ctgaatgaag catttgttgt
1081 cccctatggg actcctttgt cagtaaactt tggtccgacc gtggatggtg attttctcac
1141 tgacatgcca gacatattac ttgaacttgg acaatttaaa aaaacccaga ttttggtggg
1201 tgttaataaa gatgaaggga cagctttttt agtctatggt gctcctggct tcagcaaaga
1261 taacaatagt atcataacta gaaaagaatt tcaggaaggt ttaaaaatat tttttccagg
1321 agtgagtgag tttggaaagg aatccatcct ttttcattac acagactggg tagatgatca
1381 gagacctgaa aactaccgtg aggccttggg tgatgttgtt ggggattata atttcatatg
1441 ccctgccttg gagttcacca agaagttctc agaatgggga aataatgcct ttttctacta
1501 ttttgaacac cgatcctcca aacttccgtg gccagaatgg atgggagtga tgcatggcta
1561 tgaaattgaa tttgtctttg gtttacctct ggaaagaaga gataattaca caaaagccga
1621 ggaaattttg agtagatcca tagtgaaacg gtgggcaaat tttgcaaaat atgggaatcc
1681 aaatgagact cagaacaata gcacaagctg gcctgtcttc aaaagcactg aacaaaaata
1741 tctaaccttg aatacagagt caacaagaat aatgacgaaa ctacgtgctc aacaatgtcg
1801 attctggaca tcattttttc caaaagtctt ggaaatgaca ggaaatattg atgaagcaga
1861 atgggagtgg aaagcaggat tccatcgctg gaacaattac atgatggact ggaaaaatca
1921 atttaacgat tacactagca agaaagaaag ttgtgtgggt ctctaattaa tagatttacc
1981 ctttatagaa catattttcc tttagatcaa ggcaaaaata tcaggagctt ttttacacac
2041 ctactaaaaa agttattatg tagctgaaac aaaaatgcca gaaggataat attgattcct
2101 cacatcttta acttagtatt ttacctagca tttcaaaacc caaatggcta gaacatgttt

2161 aattaaattt cacaatataa agttctacag ttaattatgt gcatattaaa acaatggcct
2221 ggttcaattt ctttctttcc ttaataaatt taagtttttt ccccccaaaa ttatcagtgc
2281 tctgctttta gtcacgtgta ttttcattac cactcgtaaa aaggtatctt ttttaaatga
2341 attaaatatt gaaacactgt acaccatagt ttacaatatt atgtttccta attaaaataa
2401 gaattgaatg tcaatatgag atattaaaat aagcacagaa aatc//

- LOCUS: AF168617 650 bp mRNA linear PRI 19-JUL-2000
- DEFINITION: Homo sapiens HE2 beta1 mRNA, complete cds.sperm associated antigen 11 (SPAG11)
- ACCESSION: AF168617
- VERSION ORIGIN: AF168617.1 GI:7140925.

1 tccacattgt cctttgagaa tgagaagtca tcctggagca cagctcttcc cacgctccgg
61 gcccacacac ccagcctcac tccatcaaag gagccccgct gcctgcccac ccaccctggg
121 tgctttctgg cttgcagtgc tcttggcaga catgaggcaa cgattgctcc cgtccgtcac
181 cagccttctc cttgtggccc tgctgtttcc aggatcgtct caagccagac atgtgaacca
241 ctcagccact gaggctctcg gagaactcag ggaaagagcc cctgggcaag gcacaaacgg
301 gtttcagctg ctacgccacg cagtgaaacg ggacctctta ccaccgcgca ccccacctta
361 ccaaggggat gttccaccgg gaattagaaa taccatctgc cgtatgcagc aaggatctg
421 cagacttttt ttctgccatt ctggtgagaa aaagcgtgac atttgctctg atccctggaa
481 taggtgttgc gtatcaaata cagatgaaga aggaaaagag aaaccagaga tggatggcag
541 atctgggatc taaaatataa gctcccggaa ggcagggatg ttgaagtatc ccaaggctt
601 aaaggaatgt gtggcttata gtaggtgttc aataaatatt tgttgaatga//

- LOCUS: BC006333 1789 bp mRNA linear PRI 15-JUL-2006
- DEFINITION: Homo sapiens tripartite motif-containing 14, mRNA (cDNA clone MGC:12564 IMAGE:4299815), complete cds.(TRIM14)
- ACCESSION: BC006333
- VERSION ORIGIN: BC006333.2 GI:39645945

1 cggaggaggt ggagatgaat ggcgggcgcg gcgaccggga gccggacccc tgggaggtcg
61 gagcttgtcg agggatgcgg ctggcgctgc ccggagcatg gcgaccgcgt ggctgagctc
121 ttctgtcgcc gctgccgccg ctgcgtgtgc gcgctttgcc cggtgctggg cgcgcaccgt
181 ggccaccctg tgggcctggc gctggaggca gcggtgcacg tgcagaaact cagccaagaa
241 tgtttaaagc agctggcaat caagaagcag cagcacattg acaacataac ccagatagaa
301 gatgccaccg agaagctcaa ggctaatgca gagtcaagta aaacctggct gaaggggaaa
361 ttcactgaac tcagattact acttgacgaa gaggaagcgc tggccaagaa attcattgat
421 aaaaacacgc agcttaccct ccaggtgtac agggaacaag ctgactcttg cagagagcaa
481 cttgacatca tgaatgatct ctccaacagg gtctggagta tcagccagga gcccgatcct
541 gtccagaggc ttcaggcata cacggccacc gagcaggaga tgcagcagca gatgagcctc
601 ggggagctgt gccatcccgt gcccctctcc tttgagcccg tcaagagctt ctttaagggc
661 ctcgtggaag ccgtggagag tacattacag acgccattgg acattcgcct taaggaaagc
721 ataaactgcc agctctcaga cccttccagc accaagccag gtaccttgtt gaaaaccagc
781 ccctcaccag agcgatcgct attgctgaaa tacgcgcgca cgcccacgct ggatcctgac
841 acgatgcacg cgcgcctgcg cctgtccgcc gatcgcctga cggtgcgctg cggcctgctg
901 ggcagcctgg ggcccgtgcc cgtgctgcgg ttcgacgcgc tctggcaagt gctggctcgt
961 gactgcttcg ccaccggccg ccactactgg gaggttgacg tgcaggaggc gggcgccggc
1021 tggtgggtgg gcgcggccta cgcctccctt cggcgccgcg gggcctcggc cgccgcccgc
1081 ctgggctgca accgccagtc ctggtgcctc aagcgctacg accttgagta ctgggccttc
1141 cacgacggcc agcgcagccg cctgcggccc cgcgacgacc tcgaccggct cggcgtcttc
1201 ctggactacg aggccggcgt cctcgccttc tacgacgtga cgggcggcat gagccacctg
1261 catacettcc gcgccacgtt ccaggagccg ctctacccgg ccctgcggct ctgggagggg
1321 gccatcagca tcccccggct gccctagggg ccaggaccgg cgtgacagcc tccagaatgt
1381 catggaagca gaatcgtaca gtatgttccc ttctgcatat gctgctttct cactcagcat
1441 catttccttg agatgcatcc aggctgctgc acgcatcaat agttcattcc tggctgggcg
1501 cggtggctca cgcctgtaat cccagcgctt tgggaggccg atgtgggtgg atcacaaggt
1561 caggagtttg agactagcct ggccaagatg gtgaaacccc atctctgcaa aaaatacaaa
1621 aattagccgg gcaccatggc atgtgcctgt aatccctgct actcaggagg ctgaggaagg
1681 agaatcactt gaacccgggc agcggaggtt gcagtgagcc gagatcacac cactgcactc
1741 cagcctgggc aatagagtga gactccgttt caaaaaaaaa aaaaaaaaa//

- LOCUS: NM_007009 1177 bp mRNA linear PRI 17-NOV-2006
- DEFINITION: Homo sapiens zona pellucida binding protein (ZPBP), mRNA.
- ACCESSION: NM_007009
- VERSION ORIGIN: NM_007009.1 GI:5902115

1 ccgcgcggac ggtgggcagg cgacggcggc gtgtggatgg aggccttcgc ccttggccca
61 gcgcggcggg gcaggcggcg gacccgggcc gccggctccc tgctctctcg ggccgccatc
121 ctcctcttta tctccgcctt cctggtgcgg gtgccctcat cagttggaca cttggttcga
181 ttaccaagag cttttcgctt gaccaaagat tcagtgaaaa tagtgggatc aacaagtttt
241 ccagtgaaag cgtatgtcat gctccatcaa aagagtccac acgtgttatg tgtaacgcaa
301 caactgcgaa atgctgaact gatagaccca tcattccaat ggtatgggcc taaaggaaaa
361 gttgtttcag tagaaaaccg cactgcacaa ataacatcca caggaagcct tgtattccaa
421 aattttgagg agagtatgag tggaatttat acatgtttcc tcgaatataa acctactgtg
481 gaagaaattg ttaaacgtct tcaactaaaa tatgctatat atgcttatcg tgagcctcat
541 tattattatc agttcacagc tcgatatcat gcagtcccct gcaatagcat ttataatatt
601 tcttttgaga agaaacttct tcagatttta agcaaactgc ttcttgacct ttcatgtgaa
661 atttccttac ttaagtctga atgccatcgc gttaaaatgc aaagagctgg tttgcaaaat
721 gaattgttct ttgcattttc agtttcatct ctagacactg aaaaaggacc caagcgatgt
781 acagaccata actgtgaacc ttacaaaaga ctttttaagg ctaaaaatct catagagaga
841 ttttttaatc aacaagtaga aattcttggc agacgtgcag aacaattacc tcaaatatac
901 tatattgaag gtactctcca aatggtttgg attaatcgct gctttccagg atatggaatg
961 aatgtccagc aacatccaaa atgtcctgag tgctgtgtga tctgcagccc tggatcatat
1021 aacccccgtg atggaattca ttgccttcaa tgcaatagca gcctggtgta tggagcaaaa
1081 acgtgcttat aagccattat cttcagttat tcagtggttt attaaatgca aagtatatat
1141 ttgaaatatt aacataataa attattatgc caaaatt//

- LOCUS: NM_014420 835 bp mRNA linear PRI 18-NOV-2006
- DEFINITION: Homo sapiens dickkopf homolog 4 (Xenopus laevis) (DKK4), mRNA.
- ACCESSION: NM_014420
- VERSION ORIGIN: NM_014420.2 GI:66346690

  1 cagacgacgt gctgagctgc cagcttagtg gaagctctgc tctgggtgga gagcagcctc
 61 gctttggtga cgcacagtgc tgggaccctc caggagcccc gggattgaag gatggtggcg
121 gccgtcctgc tggggctgag ctggctctgc tctcccctgg gagctctggt cctggacttc
181 aacaacatca ggagctctgc tgacctgcat ggggcccgga agggctcaca gtgcctgtct
241 gacacggact gcaataccag aaagttctgc ctccagcccc gcgatgagaa gccgttctgt
301 gctacatgtc gtgggttgcg gaggaggtgc cagcgagatg ccatgtgctg ccctgggaca
361 ctctgtgtga acgatgtttg tactacgatg gaagatgcaa ccccaatatt agaaaggcag
421 cttgatgagc aagatggcac acatgcagaa ggaacaactg ggcacccagt ccaggaaaac
481 caacccaaaa ggaagccaag tattaagaaa tcacaaggca ggaagggaca agagggagaa
541 agttgtctga gaacttttga ctgtggccct ggactttgct gtgctcgtca tttttggacg
601 aaaatttgta agccagtcct tttggaggga caggtctgct ccagaagagg gcataaagac
661 actgctcaag ctccagaaat cttccagcgt tgcgactgtg gccctggact actgtgtcga
721 agccaattga ccagcaatcg gcagcatgct cgattaagag tatgccaaaa aatagaaaag
781 ctataaatat ttcaaaataa agaagaatcc acattgcaaa aaaaaaaaaa aaaaa//

- LOCUS: X98568 10058 bp DNA linear PRI 14-NOV-2006
- DEFINITION: H.sapiens type X collagen gene.
- ACCESSION: X98568
- VERSION ORIGIN: X98568.1 GI:1405722

1 ctcgaggatc agtttgaatg acaggcatat aggcatttat tgtaactatt ctcactacta
61 taaaagacaa gttttctgta aaaaagttta aaaagcatat gaacacaaat aacaaaaacc
121 aatgtaacta atattaacaa ggtggtaata gaacactgat atcagactat acattctaac
181 ttaggaggcc atcatgatca cctgaggagc ttcatgaaaa tatagtcttt gaaccaccac
241 tattttaatg aacctcaaag gctgaaggaa ttgtgcattt ccctcaggac agcaggagtg
301 gggaaaagag gctgagaacc actgtgtaaa cactttaagg gtatagaata tatattattc
361 acccagtatt tatcttaatg tctaacactt aagaagcact taagaaacat aaggtgaatt
421 aattaacgat ccacagatgt acaaatgaat aggcaagctt aatagtagct aactgccaga
481 tgtgaaaaaa atcattattc atatttgata tttaaaaaaa ttactcaggt atagagttta
541 aagagtatgg ctctctgttt gagtggaatt gggtttcctt cataaagcca cagacttagt
601 tcactttcaa acacttacaa gtaaacaccc agagtaaaaa tagttttaaa tacacaattt
661 gggtggggtt gttgcttaga aatattcttg atttcttaca tctgtttgac aagacagtgc
721 tcttatgtga ctaacaaagc aaacagcagt cagattcctt gtgctgaaat aagtacacta
781 tttgcaaaaa gggttgaatg tccaaggctt attcaataag gacaccacat tttccaaaac

841 taaaggggag tatagaatga caaataccca catatattta catatcccta aaagaagaca
901 tacaacctcc tggtatggat ttttttgtgt ctctaataag tactaactgg agccaagtct
961 accatctgac catggagaac agcagaatac ctgaagcaca ggaataatct aattttaaaa
1021 ctgggaggag cttagaacca cctagtccaa acttttcatt gtgttgataa aaaacagaca
1081 cccagagagg ttaaagtgaa aggctctgaa aattgttgca atagccacag atgaacaaat
1141 ggtatacaca ggggcatagc actatgtatt aagagctgag aattagtctt ctgaacgttc
1201 acgagtgata actaacaggt atcagctcta ttatagcaat agcagtatgt gtgtgcatgt
1261 gtatcaccat atatacacac aaaatattaa aatatatatt ttaataattc ctttcaaaac
1321 aaattcaacc tagcattcaa aagcatccat aaagttacct cacctacttt ttcaaaattg
1381 tgtccaaatg ggccataagc caaaaggcag gtgtttctct cacttatctt tgtagtctcc
1441 tacagatgta gcagagtaca tattataatt acaaaagcta ttccatatat aatttccaaa
1501 tgatgaatta taaactttcc tattttgtta ttctaaactg ctataagaaa catattttat
1561 atgtatctat gagtattata cagagatata tacaacaatg gtaaattttt ttaatatggc
1621 aatcaacaaa atgcttacaa gcaagagaga gaggcaatga gatgccttat ccatttacat
1681 ttccataaag ggctatacaa ggctttgttt aaaatacaga aaattgcttg aaggggagag
1741 aaagggaaaa gaaaaaggac aatctttagt tcattcactg gtcaactttc aattattcat
1801 tattgttagt actgaatcca tactacttta attctgtgga ggtagaaaaa tgatgtgtct
1861 atgcctgtgt atggaattgt cctcctcaac cagttcacct gcatagtcaa tttgattttg
1921 attcaaaacg atgaaattaa ataagatgaa aaatttggac tctcaacttc ccacctttat
1981 tagatttttt aggatcctaa aaactgattt taaaggcttc ctaatattgt taaacagagt
2041 ctaactacca aaattgacaa caatagtgtt tacttccacc tttgagggaa agtgtttcta
2101 attgggatga ttcctaactg caagttaagc caataatatt ttttttaagc tgaaagcaaa
2161 gtttgaaaat ttttttctcc tgaaataaac acagcaatct tatgtaaatt aacataatat
2221 gagattatca aattaagggg tttttgcaaa ctctactgca actctaaagt aaacccaaat
2281 ttaagcaaga aaatctgaag tcatttactg aatatagcgt agtgttgtga ataagggatc
2341 taaatcctaa aagcctaaaa gtaattaaga attggggtca gctattttca caaaaattta
2401 tgaatattaa tagacacatt gataacttta tgtaataact caataattta gatgatggtc
2461 taaatccagt acccccttca aggaatttta tccgctttta tgtactacca catattatat
2521 aatatcaacc aattaacata agaatgtgct tttcaaaaat aaaaaggggg tatcatcatt
2581 ccaccgcgaa ccagcctgaa cactagagta ttaattaata aaatagaatc acagacaaag
2641 acggtccttg gttaaaacta ttaatatata tttaagacaa tgggtttatt tttaaataag
2701 aaaacaagga aaaggagagc ccagcccgat cttgtgtgcc tacccaatag aactttccac
2761 cataattagt gcttaccaac tttgagggga ggagcttaag ctcagggtaa cctcatgtag
2821 taagggaaaa acagtataaa taccactgaa cagctcttga gaggcacctt ctgcactgct
2881 catctgggca gaggaagctt cagaaagctg ccaaggcacc atctccagga actcccagca
2941 cgcaggtaag cgtacgctta ctggtggact tcccatgtta acttgcttgg aaagaaactt
3001 taaagattta aatacttgag ttttctcaag tattagaagt attaagtgat ttaaaatggt
3061 tctactaatg cactccaaag tctttgcatc agtctgtgga tgatagtcca ataaaaccat
3121 attgtaaaat ttgccctaat ccgaatttca gtgtattttg aaactattag atatttcagg
3181 ctagttcaaa gatcctagat tatttaatac agacatccaa tctcaatttt cgtttctttc
3241 cttaggtaat catttatttc actgatcctg agtatagctt ccaaaaataa ctaagacata
3301 tattcacata actaaaaaat gatctctcat ttatttatgg cacatattaa aaatatatag
3361 gataggttaa aaaagtagat aactgtataa agaactcatc tgtgaaacat gagaataaaa
3421 aaataggtta ataacaatgc tgtataaagt tattcttttc tgtttttcag aatccatctg
3481 agaatatgct gccacaaata ccctttttgc tgctagtatc cttgaacttg gttcatggag
3541 tgttttacgc tgaacgatac caaatgccca caggcataaa aggcccacta cccaacacca
3601 agacacagtt cttcattccc tacaccataa agagtaaagg taaattgaat tgtatatttt
3661 gttctattaa caagttgctt gttgctttct ataatcttaa taatctcttt aacaaaatgc
3721 atttaacttt ggtgttactt tccaacttct gaattagccc aaattattac aacaaagtga
3781 tctgtgaaaa aagtagaaca agaatatcca ataagtgtag tagaaaggat atacttgaaa
3841 atatagttga aagatatacc tgcaaaaaat cagaactttt gtgtgtacaa atttttttcc
3901 tcagatgctg cttttgcaca aataactgca tgtaatcaga atcttgatct aatcaaaact
3961 aatcagttta aattttagcc taaaaataac tgccaattgt ataaagaagg tagctggttt
4021 atttagctaa atattaaaca tttactttta aaactaattc aactagccca caaacagata
4081 taaaccgagt cttatcaaaa aaatctgatg gcctcccccc caacaatgaa caaatgtaat
4141 tatgagaatt gcacagacgg acaatgaaac tgcaattctc ataattactt atacattttg
4201 aatatagttt agcggtaaaa tccagagact taactgtaat gcataagctg tgtgacctga
4261 ggtaagttac ttgtcctccc atgccttagt ttcctcattt gtaaaataag gagcagctat
4321 ctcaagggct gctgcgaagt ttaaatgaat taatacctgt aaagtgcttt aagtgttgct
4381 attacttcta ctactacttc tgttactcta gttttatttc tactactttt agctactaat
4441 atagccttca ataagttaac tttcttcttg aatctgttgc ttacctgtcc agtttctggt
4501 actcttgaag attactgaga taatatatgt aaagcactaa ggttctcaaa ggagggcaac
4561 tctaggcctc atattcacta acttttattt aacagcaact gacacgaccg cctataaaat

4621 atctacagaa taatataaac tgactgtagg cttcaaactc cctttccata atttttaatt
4681 atatatttta tactgtaaat aaagctaagg atattgttat aaagtttaaa tattatgtct
4741 tttgggaaca accattttga gcaatgaaac ttatagaatt gtctttttaa gaagaaagat
4801 ggaatttaaa atatttcaca aaaggaaact taatatttga aaataaatgc acaggtatac
4861 aacagatact tttgaatgtc tcaaaatata aataaaaatt ttaaaattta aaggcatgta
4921 aagaaaaaaa taaaagacaa agcaaccttc cgctttactt gaagcaacaa acacaatttg
4981 aaaacttttt aacaccttaa taagagcata acaagtcttg aattaaaaag aaaatgcact
5041 attttagaag gttaaaaata ctttgatctt ctcttaatat acaccattat cttggctact
5101 tttgcagaac aaatgaaaat atcttaacaa atgaaaagtt cagaagctgt atttaaacta
5161 ctaaatccac cttagtctct ttttaagata gacactcaac tacactatca tccattggca
5221 gacattaatg caccataaca aacacaaaag cccacagtct atacaagtaa gacattaagt
5281 tgaaacagga attcaaagag ttattacata attcctaggc atatccatcc aacacaggtt
5341 aaaaaagagt tctgtcaaaa catactaaat tatgctgcag aggattccac agctggtggg
5401 cagatgcacg tgccacattc tgacacaacc aatcacgatt tcagtttgtg gcatttaaaa
5461 aacttaatag aaggaagtag tcgttttacc ttgaggattg tgccacccta atcttgaatg
5521 aagaaaattt tttcagatga caacctacaa acaggggctg tcccccaagc attcaataga
5581 agccaaattc tttctcgcac tgagaagttg agacccatta ggacaacatt ccaagtgaca
5641 aagttcgccc tgtcactgga gttccagtct cggatgacgc ccaatgcact catttccatt
5701 gacttgtttc tcctttttaa tagtaaaaca gatttccaat cagactaaga caattatttt
5761 tagtgtgaac atgaccctta taatcagatt ctaaagcaaa gctcaatcta acatagttgg
5821 cattttaaaa aatcactatt ttattctcca acccattaaa aaattcctta tttattcctc
5881 tatttgcaat atatcaatat ccacttatac ataatataat catgtatgta cactacatca
5941 atgaaatatt taatatacgg aaatctttta cataaatttt tctcacagtc tttttcctga
6001 tgtcattttc tgaaaatcca tggggaaagc tctatgtatt tatattaatc aatgttttca
6061 aattgaaata taactctaat tagtaaaaaa aaatctaaat ctgaaacaat tatttctat
6121 ttcggcatat ctattttaat gattcgctct gcatttatat acaccaaatg tttcaaatct
6181 cagaacatct gtaccctgtg cttcccatac tgtgcccagc cgcattttca catcacaggc
6241 actatgattt ttagtttaag tttagtttat caggtcaagt gaactcacag ctctatgaaa
6301 tcaaacacta tatatttgta tttaggtaag aactttaata ctattttatg tacctcacct
6361 attatgtcta attctctagt tctttgtttc aattttaagt caataatgga gagtataaga
6421 gctcagagta tattctgagg taaaagtaaa tcaaccaaaa tctaacctgt gttcagcaga
6481 ggcatggcag atgtaaatca gaattaattc tgtttctctt ttatgtctga cagggtagct
6541 ttgggaggga aaatttttat agttcttaaa cattttctca cttattctgc actgtctttt
6601 tctgcctttt ctgtacatga ttttcccaaa ataaatgtac taaatttacc tccactgcac
6661 ttattctaga ggaattcttc aatcctagct cattttcatg aatcactgtc atgggataaa
6721 agtattatgg tagccatggt tttgtgaaac catcttctcc agcagaattt tttaaaacag
6781 tataaaacat ataatctaaa accatttcag actgttctgt ctacatggaa taaatgatgc
6841 tgaaatatcg tgtcccaatt catagtttga aaggcaatga ttctctttaa tgtcactaac
6901 catccccttc tatgggtggg tgtgttgggt gtgtctttta ggtatagcag taagaggaga
6961 gcaaggtact cctggtccac caggccctgc tggacctcga gggcacccag gtccttctgg
7021 accaccagga aaaccaggct acggaagtcc tggactccaa ggagagccag ggttgccagg
7081 accaccggga ccatcagctg tagggaaacc aggtgtgcca ggactcccag gaaaaccagg
7141 agagagagga ccatatggac caaaaggaga tgttggacca gctggcctac caggaccccg
7201 gggcccacca ggaccacctg gaatccctgg accggctgga atttctgtgc caggaaaacc
7261 tggacaacag ggacccacag gagccccagg acccaggggc tttcctggag aaaagggtgc
7321 accaggagtc cctggtatga atggacagaa aggggaaatg ggatatggtg ctcctggtcg
7381 tccaggtgag aggggtcttc caggccctca gggtcccaca ggaccatctg gccctcctgg
7441 agtgggaaaa agaggtgaaa atggggttcc aggacagcca ggcatcaaag gtgatagagg
7501 ttttccggga gaaatgggac caattggccc accaggtccc caaggccctc ctggggaacg
7561 agggccagaa ggcattggaa agccaggagc tgctggagcc ccaggccagc cagggattcc
7621 aggaacaaaa ggtctccctg gggctccagg aatagctggg cccccagggc ctcctggctt
7681 tgggaaacca ggcttgccag gcctgaaggg agaaagagga cctgctggcc ttcctggggg
7741 tccaggtgcc aaaggggaac aagggccagc aggtcttcct gggaagccag gtctgactgg
7801 accccctggg aatatgggac cccaaggacc aaaaggcatc ccgggtagcc atggtctccc
7861 aggccctaaa ggtgagacag ggccagctgg gcctgcagga taccctgggg ctaagggtga
7921 aaggggttcc cctgggtcag atggaaaacc agggtaccca ggaaaaccag gtctcgatgg
7981 tcctaagggt aacccagggt taccaggtcc aaaaggtgat cctggagttg gaggacctcc
8041 tggtctccca ggccctgtgg gcccagcagg agcaaaggga atgcccggac acaatggaga
8101 ggctggccca agaggtgccc ctggaatacc aggtactaga ggccctattg ggccaccagg
8161 cattccagga ttccctgggt ctaaagggga tccaggaagt cccggtcctc ctggcccagc
8221 tggcatagca actaagggcc tcaatggacc caccgggcca ccagggcctc caggtccaag
8281 aggccactct ggagagcctg gtcttccagg gccccctggg cctccaggcc caccaggtca
8341 agcagtcatg cctgagggtt ttataaaggc aggccaaagg cccagtcttt ctgggacccc

8401 tcttgttagt gccaaccagg gggtaacagg aatgcctgtg tctgctttta ctgttattct
8461 ctccaaagct tacccagcaa taggaactcc cataccattt gataaaattt tgtataacag
8521 gcaacagcat tatgacccaa ggactggaat ctttacttgt cagataccag gaatatacta
8581 tttttcatac cacgtgcatg tgaaagggac tcatgtttgg gtaggcctgt ataagaatgg
8641 cacccctgta atgtacacct atgatgaata caccaaaggc tacctggatc aggcttcagg
8701 gagtgccatc atcgatctca cagaaaatga ccaggtgtgg ctccagcttc ccaatgccga
8761 gtcaaatggc ctatactcct ctgagtatgt ccactcctct ttctcaggat tcctagtggc
8821 tccaatgtga gtacacccca cagagctaat ctaaatcttg tgctagaaaa agcattctct
8881 aactctaccc caccctacaa aatgcatatg gaggtaggct gaaaagaatg taatttttat
8941 tttctgaaat acagatttga gctatcagac caacaaacct tccccctgaa aagtgagcag
9001 caacgtaaaa acgtatgtga agcctctctt gaatttctag ttagcaatct taaggctctt
9061 taaggttttc tccaatatta aaaaatatca ccaaagaagt cctgctatgt taaaaacaaa
9121 caacaaaaaa caaacaacaa aaaaaaaaat taaaaaaaaa aacagaaata gagctctaag
9181 ttatgtgaaa tttgatttga gaaactcggc atttcctttt taaaaaagcc tgtttctaac
9241 tatgaatatg agaacttcta ggaaacatcc aggaggtatc atataacttt gtagaactta
9301 aatacttgaa tattcaaatt taaaagacac tgtatcccct aaaatatttc tgatggtgca
9361 ctactctgag gcctgtatgg cccctttcat caatatctat tcaaatatac aggtgcatat
9421 atacttgtta aagctcttat ataaaaaagc cccaaaatat tgaagttcat ctgaaatgca
9481 aggtgctttc atcaatgaac cttttcaaac ttttctatga ttgcagagaa gctttttata
9541 tacccagcat aacttggaaa caggtatctg acctattctt atttagttaa cacaagtgtg
9601 attaatttga tttctttaat tccttattga atcttatgtg atatgatttt ctggatttac
9661 agaacattag cacatgtacc ttgtgcctcc cattcaagtg aagttataat ttacactgag
9721 ggtttcaaaa ttcgactaga agtggagata tattatttat ttatgcactg tactgtattt
9781 ttatattgct gtttaaaact tttaagctgt gcctcactta ttaaagcaca aaatgtttta
9841 cctactcctt atttacgaca caataaaata acatcaatag atttttaggc tgaattaatt
9901 tgaaagcagc aatttgctgt tctcaaccat tctttcaagg cttttcattg ttcaaagtta
9961 ataaaaaagt aggacaataa agtgatgggt ggcttttaca gttgtcatgg atatgtccta
10021 tattggtgat gcatctttat tggtgatgca tctttatt//

- LOCUS: BC000340 1700 bp mRNA linear PRI 15-JUL-2006
- DEFINITION: Homo sapiens melanoma antigen family A, 3, mRNA (cDNA clone MGC:8564 IMAGE:2822978), complete cds.
- ACCESSION: BC000340
- VERSION ORIGIN: BC000340.2 GI:33875329

1 cgtcggcgga gggaagccgg cccaggctcg gtgaggaggc aaggttctga ggggacaggc
61 tgacctggag gaccagaggc ccccggagga gcactgaagg agaagatctg ccagtgggtc
121 tccattgccc agctcctgcc cacactcccg cctgttgccc tgaccagagt catcatgcct
181 cttgagcaga ggagtcagca ctgcaagcct gaagaaggcc ttgaggcccg aggagaggcc
241 ctgggcctgg tgggtgcgca ggctcctgct actgaggagc aggaggctgc ctcctcctct
301 tctactctag ttgaagtcac cctgggggag gtgcctgctg ccgagtcacc agatcctccc
361 cagagtcctc agggagcctc cagcctcccc actaccatga actaccctct ctggagccaa
421 tcctatgagg actccagcaa ccaagaagag gaggggccaa gcaccttccc tgacctggag
481 tccgagttcc aagcagcact cagtaggaag gtggccgagt tggttcattt tctgctcctc
541 aagtatcgag ccagggagcc ggtcacaaag gcagaaatgc tggggagtgt cgtcggaaat
601 tggcagtatt tctttcctgt gatcttcagc aaagcttcca gttccttgca gctggtcttt
661 ggcatcgagc tgatggaagt ggaccccatc ggccacttgt acatctttgc cacctgcctg
721 ggcctctcct acgatggcct gctgggtgac aatcagatca tgcccaaggc aggcctcctg
781 ataatcgtcc tggccataat cgcaagagag ggcgactgtg cccctgagga gaaaatctgg
841 gaggagctga gtgtgttaga ggtgtttgag gggagggaag acagtatctt gggggatccc
901 aagaagctgc tcacccaaca tttcgtgcag gaaaactacc tggagtaccg gcaggtcccc
961 ggcagtgatc ctgcatgtta tgaattcctg tggggtccaa gggccctcgt tgaaaccagc
1021 tatgtgaaag tcctgcacca tatggtaaag atcagtggag gacctcacat ttcctaccca
1081 cccctgcatg agtgggtttt gagagagggg gaagagtgag tctgagcacg agttgcagcc
1141 agggccagtg ggagggggtc tgggccagtg caccttccgg ggccgcatcc cttagtttcc
1201 actgcctcct gtgacgtgag gcccattctt cactctttga agcgagcagt cagcattctt
1261 agtagtgggt ttctgttctg ttggatgact ttgagattat tctttgtttc ctgttggagt
1321 tgttcaaatg ttccttttaa cggatggttg aatgagcgtc agcatccagg tttatgaatg
1381 acagtagtca cacatagtgc tgtttatata gtttaggagt aagagtcttg ttttttactc
1441 aaattgggaa atccattcca ttttgtgaat tgtgacataa taatagcagt ggtaaaagta
1501 tttgcttaaa attgtgagcg aattagcaat aacatacatg agataactca agaaatcaaa
1561 agatagttga ttcttgcctt gtacctcaat ctattctgta aaattaaaca aatatgcaaa
1621 ccaggatttc cttgacttct ttgagaatgc aagcgaaatt aaatctgaat aaataattaa

1681 aaaaaaaaaa aaaaaaaaaa//

- LOCUS: NM_005940 2276 bp mRNA linear PRI 11-MAR-2007
- DEFINITION: Homo sapiens matrix metallopeptidase 11 (stromelysin 3) (MMP11), mRNA.
- ACCESSION: NM_005940
- VERSION ORIGIN: NM_005940.3 GI:58331147

1 aagcccagca gccccggggc ggatggctcc ggccgcctgg ctccgcagcg cggccgcgcg
61 cgccctcctg cccccgatgc tgctgctgct gctccagccg ccgccgctgc tggcccgggc
121 tctgccgccg gacgcccacc acctccatgc cgagaggagg gggccacagc cctggcatgc
181 agccctgccc agtagcccgg cacctgcccc tgccacgcag gaagcccccc ggcctgccag
241 cagcctcagg cctccccgct gtggcgtgcc cgacccatct gatgggctga gtgcccgcaa
301 ccgacagaag aggttcgtgc tttctggcgg gcgctgggag aagacggacc tcacctacag
361 gatccttcgg ttcccatggc agttggtgca ggagcaggtg cggcagacga tggcagaggc
421 cctaaaggta tggagcgatg tgacgccact cacctttact gaggtgcacg agggccgtgc
481 tgacatcatg atcgacttcg ccaggtactg gcatggggac gacctgccgt ttgatggccc
541 tgggggcatc ctggcccatg ccttcttccc caagactcac cgagaagggg atgtccactt
601 cgactatgat gagacctgga ctatcgggga tgaccagggc acagacctgc tgcaggtggc
661 agcccatgaa tttggccacg tgctggggct gcagcacaca acagcagcca aggccctgat
721 gtccgccttc tacacctttc gctacccact gagtctcagc ccagatgact gcaggggcgt
781 tcaacaccta tatggccagc cctggcccac tgtcacctcc aggaccccag ccctgggccc
841 ccaggctggg atagacacca atgagattgc accgctggag ccagacgccc cgccagatgc
901 ctgtgaggcc tcctttgacg cggtctccac catccgaggc gagctctttt tcttcaaagc
961 gggctttgtg tggcgcctcc gtgggggcca gctgcagccc ggctacccag cattggcctc
1021 tcgccactgg cagggactgc ccagccctgt ggacgctgcc ttcgaggatg cccagggcca
1081 catttggttc ttccaaggtg ctcagtactg ggtgtacgac ggtgaaaagc cagtcctggg
1141 ccccgcaccc ctcaccgagc tgggcctggt gaggttcccg gtccatgctg ccttggtctg
1201 gggtcccgag aagaacaaga tctacttctt ccgaggcagg gactactggc gtttccaccc
1261 cagcacccgg cgtgtagaca gtcccgtgcc ccgcagggcc actgactgga gaggggtgcc
1321 ctctgagatc gacgctgcct tccaggatgc tgatggctat gcctacttcc tgcgcggccg
1381 cctctactgg aagtttgacc ctgtgaaggt gaaggctctg gaaggcttcc cccgtctcgt
1441 gggtcctgac ttctttggct gtgccgagcc tgccaacact ttcctctgac catggcttgg
1501 atgccctcag gggtgctgac ccctgccagg ccacgaatat caggctagag acccatggcc
1561 atctttgtgg ctgtgggcac caggcatggg actgagccca tgtctcctca gggggatggg
1621 gtggggtaca accaccatga caactgccgg gagggccacg caggtcgtgg tcacctgcca
1681 gcgactgtct cagactgggc agggaggctt tggcatgact taagaggaag ggcagtcttg
1741 ggcccgctat gcaggtcctg gcaaacctgg ctgccctgtc tccatccctg tccctcaggg
1801 tagcaccatg gcaggactgg gggaactgga gtgtccttgc tgtatccctg ttgtgaggtt
1861 ccttccaggg gctggcactg aagcaagggt gctggggccc catggccttc agccctggct
1921 gagcaactgg gctgtagggc agggccactt cctgaggtca ggtcttggta ggtgcctgca
1981 tctgtctgcc ttctggctga caatcctgga aatctgttct ccagaatcca ggccaaaaag
2041 ttcacagtca aatggggagg ggtattcttc atgcaggaga ccccaggccc tggaggctgc
2101 aacatacctc aatcctgtcc caggccggat cctcctgaag cccttttcgc agcactgcta
2161 tcctccaaag ccattgtaaa tgtgtgtaca gtgtgtataa accttcttct tctttttttt
2221 tttttaaact gaggattgtc attaaacaca gttgttttct aaaaaaaaaa aaaaaa//

- LOCUS: NM_013307 765 bp mRNA linear PRI 02-NOV-2000
- DEFINITION: Homo sapiens non-functional folate binding protein (HSAF000381), mRNA.
- ACCESSION: NM_013307
- VERSION ORIGIN: NM_013307.1 GI:7019412

1 atggcctcag ttccgaaaac caacaaaata gaaccgcggt cctattccat tattcctagc
61 tgcagtatca ggcggctcgg gcctgctttg aacactccaa tttttcaaag taaacgcaac
121 gggccccgcg gacactcagc ttacagcatc gaggggcgcc agaggcaagg ggcgggacgg
181 gcggtggtcc ctcgcgcgga ccgcccgccc gctcccaaga tccaactacg agctttttac
241 ctgcagcaac tttactatac gctattggag ctggaattac cgcggctgct ggcaccagac
301 ttgccctcca atggctcctc gttaaaggat ttaaagtgga ctcattccaa ttacaggcc
361 tcgaaagagt cctgtattgt tattttcgtc actacctccc cgggtcggga gtgggtaatt
421 tgcgcgcctg ctgccttcct tggatgtggt agcctccagg ctccctctcc ggaatctgaa

481 ccctcattcc ccgtcacccg tggtcaccat ggtcggcacg gcgactacca tcgaaagttg
541 atagggcaga cgttcgaatg ggtcgtcgtc cgccgccacg gggggcgtgc gatcggcccg
601 aggttatcta gagtcaccaa agccgccggc gcccgccccc cggccggggc cggagagggg
661 ctgagggttg gttttgatct gataaatgca ccgatccccc ccgcgaaggg ggtcagcgcc
721 cgtcggcatg tattagctct agaattacca cagttatcca agtag//

- LOCUS: NM_018678 842 bp mRNA linear PRI 06-SEP-2003
- DEFINITION: Homo sapiens chromosome 14 open reading frame 117 (C14orf117), mRNA., lipopolysaccharide specific response-68 protein (LSR68)
- ACCESSION: NM_018678
- VERSION ORIGIN: NM_018678.1 GI:8923914

1 gtggtatgta ccttagctgt gaggggggct gctgaaggca tggaggacag taatgacggc
61 tgtgcctcct tggatggtgg agacctcaga aatggtctgc tacacaggca ctaggtcagt
121 ttcccgccct tagctgaatg cagatggatg ctgggagatg gatatggtga gatggtgtgg
181 ggaggatgtt agaaagctgg aggtgtttat cacttctcaa ggagctactg aatatagggg
241 gaaaaaaaca acaaaaagac aagcccaggg agaaagcacc attaaggata tacctatgcc
301 agcttctata gctgcacctg ctttattggc aggtcacttg ccacaattgc atctgccctc
361 taaacttttt aattttcaca ctgtatccta gacatgaatg cataaaatac agtgtctggc
421 gggataatgc agagaggtac catcttgatt ttccccagtt acattcacct ggtctggtct
481 ctgtaagctt agaagagtag aatcacttta ttttttaaag acttccggcc aggcacggtg
541 gttcacgcct gtaatgccag cactttggga ggccgaggcg ggtggaccac ctgaggtcag
601 gagttcaaga ccagcctggc cgacatggcg aaaccctgtc tctactaaaa aaaaatacaa
661 aaagattagc tgggtgtggt ggtgtgtgcc tataatccca gctactcagg aggctgaggc
721 aggagaattc ttttaaccca aaaggcggag gttgcagtga gccaagatca caccactgca
781 ctccagcctg ggcgacagaa tgagactctg tctcaaaaaa aaaaaaaaaa aaaaaaaaaa
841 aa//

- LOCUS: AF036973 1353 bp mRNA linear EST 06-JAN-1999
- DEFINITION: AF036973 Homo sapiens library (Carn G) Homo sapiens cDNA clone HCG IV.5, mRNA sequence.(HCG4P6)
- ACCESSION: AF036973
- VERSION ORIGIN: AF036973.1 GI:4104610

1 ctcgtcatgg gccccgtccc tcttcatttt tcatcccgga atccccgtcc cagaactgga
61 ctccctgcct cccactcctt acctatttcc ctgaactctt ctagaaaaaa actcacccca
121 gggagcttgg tgacagagag ggagctcgcc gccagagagg gagctcgccc tgggaatgga
181 ggcgtagaga caggtttttg tttttttttc tttaaatccg gaaaagttgt gcctgagtac
241 atgagacagc atagagacaa gttttctctt tattaactac agtgggtagc agaatcttgg
301 caacccctaa atgatcagga atctaatcgg taaaaaatgt aactttggcc ccttgatcta
361 taaatgtgtc tgaaagcatt acaacaggac tcacaaagct actaagtttg actttcccag
421 acaatgtatc tgtgactccc gcttgttttt tacatttacc ttcattccac agccctgagt
481 tactgggtga gtccaagaca tctcctcaat ataaagtagc acactgcgtt actatatgtt
541 gcaaccggga gccagtacag actttattca cctcacagtt gcaagtgttc aatgcagtca
601 caatgcccct cagcagtgct catgtgctgc ctgtttttag gaagtattca cgtctaagtg
661 gtgtgtatat cttataggaa tacttagtat ttttaaaacc tgattaatta aaaaaaatta
721 gtttctaggc agtcccaaat atagtattaa aggccaactg caaagaagga acactaagta
781 ttttttaaac cttattaaca taaaaaaaca ttagttttta ggcagtccta cattaggtat
841 taaaggccaa ctgcaaagaa cactgagcga ggctctgtag atggatgtaa taaaaatcta
901 taaaacaatg tgtttaaacc taagaattct actgctttcc aattccttcc ctctgctcct
961 tttcctaacc tcctgcttct ccagcccttc cctctgtccc tttcacccct caggccctcc
1021 tctcccctta gtccccacca ccctgtcact tctaaattgt ggctctagca ttgtcccatt
1081 acctgctatg tgactgttct ctccacagtg gtcctgctcc tgtgagtcag agtgtgtcat
1141 ttcctcacct aaaacactcc agtggctcca cctcggtctt gtgaagcttc tagaatgtca
1201 ggcacgtgag catatgaggg catacctggt tcatcttagg cactaaatta attttgttg
1261 actgaatgaa tgaaatatga atgtattaaa ttgcatcaca gaaagttata aaatgtaaaa
1321 cactgaaaaa ttaagaaata ttttatctta tgt//

- LOCUS: AK022688 2376 bp mRNA linear PRI 16-SEP-2006
- DEFINITION: Homo sapiens cDNA FLJ12626 fis, clone NT2RM4001810, weakly similar to AGGRECAN CORE PROTEIN PRECURSOR,KIAA0863 protein
- ACCESSION: AK022688
- VERSION ORIGIN: AK022688.1 GI:10434228

1 aacagcctca ccctgcagcc cccagcacct cagcccgtct ttctttctca cggggttcca
61 cttcatcagt ctgtgaatcc tcctgtgttg cccttgagtc agccagtcgg acctgtcaat
121 aagtctgttg gaactagtgt cctccccata aatcagactg ttcgccctgg ggttttaccc
181 ctcacccagc ctgtgggacc cataaacaga cctgttgggc ctggtgttct tcctgtgagc
241 ccctctgtca cccctggggt cctgcaggct gtctcgccag gggtgctttc tgtgagtcgg
301 gcggtcccgt ctggagtcct tcctgcaggc cagatgactc ctgcaggcca gatgactcct
361 gcaggggtta tcctgggcaa acagcaactt ctggggttct tcctactggc cagatggtcc
421 agtcaggagt tctccctgtg ggccagacag ctccgtcacg ggttcttccc ccaggccaga
481 cagccccatt gagggttatc tctgcaggcc aggtggtccc atctgggctt ctttctccca
541 accagacagt ctcctcctca gctgttgtgc ctgtaaacca gggtgtgaat tctggtgttc
601 tgcagcttag tcagcctgtt gtgtcgggag ttcttcctgt gggccagcca gtgaggcctg
661 gggtcttgca actcaaccag actgtgggca ccaacattct gcctgtgaat cagccagtga
721 gacctggtgc ttcgcagaac accaccttcc tgacatcagg ctctattctc agacagctca
781 tccctacagg gaaacaagtg aatgggattc caacctacac gctggccccc gtgtctgtca
841 ctctgccggt tccccctgga ggccttgcga ctgtcgctcc gccccagatg cccatccagc
901 tcctgccgtc aggtgcagct gcaccaatgg ccggttccat gcccggcatg ccctctcctc
961 cagtgctggt gaatgctgct cagagcgtgt ttgttcaggc ctcctcctct gcagcagaca
1021 caaaccaggt gctcaaacag gccaagcagt ggaagacctg ccctgtctgc aacgagctct
1081 ttccgtccaa cgtctaccag gtccacatgg aggtagcgca taagcacagc gagtccaagt
1141 ctggtgagaa acttgagcct gaaaaactgg cagcgtgtgc accatttcta aagtggatga
1201 gagagaaaac ggtgcgatgt ctgtcttgta agtgcttggt ccctgaggaa gagcttatac
1261 accacttgct gatgcatggc ttggggtgct tgttctgtcc atgcaccttc catgatatca
1321 aaggtctttc agagcacagc aggaataggc acctggggaa gaagaagttg cctatggatt
1381 atagcaacag aggttttcaa ttagatgtcg atgccaatgg caacctgctc tttccccacc
1441 ttgatttcat caccatattg ccaaaggaga agcttgggga gcgggaagtc tacttggcaa
1501 tcctggctgg gatacactcc aagtcactgg tgcctgtgta tgtgaaggtg aggcctcagg
1561 ctgagggcac ccccgggagc accggcaagc gagtgtccac ctgccccttt tgctttggcc
1621 cctttgtgac aactgaggcc tatgagctgc atttgaagga gaggcaccac atcatgccca
1681 cagtccacac ggtcctgaag tctcccgcct tcaagtgcat ccactgctgt ggggtctaca
1741 cgggaaatat gaccctggct gccatcgccg tccatttggt gcgctgcaga agtgctccca
1801 aggacagcag ctcagacctg caggcccagc cgggttttat tcacaacagt gaactgcttt
1861 tagtcagtgg tgaagtgatg catgattcca gtttttctgt taagagaaag ctgcctgatg
1921 gccacttagg ggccgaagac cagcggcatg gggaggagca gcctcccatc ctaaatgccg
1981 atgcagcccc gggtccagaa aaggtgacga gtgttgtgcc ttttaaaaga caaaggaatg
2041 aaagcagaac agagggacct attgtcaagg acgaggctct tcagatttta gcattagatc
2101 ctaaaaaata tgaaggccgt tcttatgaag aaaagaagca atttcttaaa gattatttcc
2161 ataagaaacc atatcctagt aaaaaggaaa tagaactgtt gtcctcactc ttttgggtgt
2221 ggaaaattga tgtggcttca ttttttggaa aaagaaggta tatttgcatg aaagcaataa
2281 aaaatcacaa gccttctgta cttttaggct ttgatatgtc tgaacttaaa aatgtgaaac
2341 atagattgaa ctttgaatat gaaccataaa acttgc//

- LOCUS: AF052091 1353 bp mRNA linear PRI 05-AUG-1998
- DEFINITION: Homo sapiens clone 23578 mRNA sequence, BTBD9
- ACCESSION: AF052091
- VERSION ORIGIN: AF052091.1 GI:3360397

1 caatatctgt gtcgttcttg gcagaaatta tattttccag cccgtgtctg caggtatatt
61 cgaattgttg ggactcacaa cacagtgaac aagatttttc acattgtggc ttttgaatgt
121 atgtttacaa acaaaacctt cactcttgag aaggggctga taggttacta ctttgggatt
181 gtgacactca aagtggacac agtggaacac ctgggagaga aaaggaaaag accccatgag
241 tgaagggtca gcatgcggcc ctggagttgc cgcaagttgg gatactgccc tgcccagctt
301 tgggggccac cacgagggag ttagaggagg tccttcccac tgcccacgcc tgccccggcg
361 cagacacctg atggcaacta gcatgctgta aacacatttt gatcagtcaa gaggaaagga
421 aatgagccag tggcccaaaa gttagagaca ccatgacacg gattcccaca aaatcttcac
481 actggattac tccgtttttg ccaacctttg tctaatgggc tcttttttct tttttaatta
541 atttttgtat ttggcagttg ggttaaaagt caacaggagt tgactggtgg tggtggtggt
601 ggtggttcga ttctgtgagg ttgtggaaag caggtccaca gataacgtcc accatctgtg
661 tggcatctgc gtgggttgtc tctgggtatt ctggttttct cccgcgtccc aaagctgtgc
721 acttgaggtt cattggtgtg tctgcagggt cccagtctga gtgcatgtga atgtgtgtga
781 gcgtgccctg caatggcatg gtgtcctgtc ctgagctggt tcctgatgca tagttctcct
841 ggccctgcaa tggttgagca cagaccctgc taagcagccg tgctggggag tggacttcaa

901 cctcatgtgg ctccagccag cctggaacaa acgcccccaa tgtgtagaag ccagccacct
961 ggccgacagt caccctctag gagctaagaa gagtttgcat acctgacagc aggtcagcct
1021 ggaagcacca tgaactctgt gccaacaccg tgccagcact gtgcagacag ggcacagcac
1081 cgagctcagc gccccgcaag caagggtaag ccatggccct gacctggaga gtagacaacg
1141 tgcttcgggg ggtcctgaca tgcatgtgtg agctgtgggg tgcaggagag aagggcgggg
1201 tgggggctgt cgttgccact gcacgagcat gtgtgcacat acatacacac ggccttctac
1261 cgtagaatca agttcacaga gacaaatgta gtatcaactg cagaattata ttgaaatat
1321 aaattccacg cttaaaaaaa aaaaaaaaaa aaa//

- LOCUS: BC000978 2955 bp mRNA linear PRI 12-JAN-2006
- DEFINITION: Homo sapiens glutamine-rich 1, mRNA (cDNA clone IMAGE:3447148), partial cds, FJ20259
- ACCESSION: BC000978
- VERSION ORIGIN: BC000978.2 GI:12803027

1 ggaatttctg gactcactgg cctctaaggg gccagaagcc cttcaggagt tccagcagac
61 agccaccact accatggtgt accaacaggg tgggaactgc atatacacag acagcactga
121 agtggctggg tctttgcttg aacttgcctg tccagtcacc accagtgttc agccacaaac
181 ccagcaagaa cagcagatcc aggttcagca gccgcagcag gttcaggtcc aggtgcaggt
241 acagcagtct ccgcaacagg tctcggctca gctctcccca caactcaccg ttcaccagcc
301 tactgagcaa cccatccagg tccaggtgca gatccaaggc caggcaccac agtcagcagc
361 cccctccatt cagaccccgt ctctgcagag tcccagtccc tcgcagctgc aagcagctca
421 gatccaggtg cagcacgtgc aagcagccca gcagatccag gctgcagaaa tcccggagga
481 gcacatccca catcagcaaa tccaggctca gctggtggct ggccagtctc ttgctggtgg
541 tcagcagatc caaatccaga ccgtgggtgc cctttcccca ccaccatccc agcagggctc
601 accccgggaa ggggagcggc gggttggcac ggccagtgtc ctccaaccag tgaagaagcg
661 caaagtggac atgcccatca ctgtgtccta cgccatctca gggcagccgg tggccaccgt
721 gctggccatt ccacagggcc agcagcagag ttatgtgtct ttgaggccag acttactgac
781 agtagacagt gcccacctgt acagtgccac tgggaccatt actagcccta caggagaaac
841 ctggaccatc cctgtttatt ctgcccagcc ccggggggac cctcagcagc agagcattac
901 ccacattgcc attccccagg aagcctacaa cgcagttcac gtcagtggct cacccacagc
961 cctggcagct gttaagctgg aggatgacaa ggagaagatg gtgggcacca catctgtagt
1021 gaaaaactcc catgaagagg tagtgcagac ccttgcaaac tctctctttc cagcacagtt
1081 catgaatggc aacatccaca ttccagtggc tgtgcaggct gtggcaggca cgtaccagaa
1141 tacggctcaa actgtccata tatgggaccc ccaacagcag ccgcagcagc aaactcccca
1201 ggaacagaca ccaccaccac agcagcagca gcagcaactc caagttactt gttcagctca
1261 aactgtccag gttgctgaag ttgaaccaca gtcacagcca cagccttccc cagaacttct
1321 gcttccaaat tctttgaagc cagaagaagg gcttgaagta tggaaaaact gggcccagac
1381 caagaatgct gaactagaga aggatgctca gaacagattg gcacccattg ggaggcgcca
1441 actgctgcga ttccaggaag atctcatctc ctctgctgtg gcagagttga attatgggct
1501 ctgtctaatg acacgggaag ctcgaaatgg agaaggtgaa ccctatgacc cagatgtgct
1561 ctactatatt ttcctgtgta ttcaaaagta tctttttgaa aatggaaggg tagatgacat
1621 tttctccgat ctttattatg ttcggttcac ggagtggcta catgaagttc tgaaggatgt
1681 tcagccccgg gtcactccac ttggctatgt cttgcccagc cacgtgactg aggagatgct
1741 atgggagtgc aagcagcttg gggctcactc cccctccacc ttgctgacca ccctcatgtt
1801 ctttaatacc aagtacttcc tattgaagac agtggaccag cacatgaagc tggccttctc
1861 caaggtcttg cgacagacaa agaagaaccc ctctaatccc aaggataaaa gcacgagtat
1921 ccggtacttg aaggcccttg gaatacacca gactggccag aaagttacag atgacatgta
1981 tgcagaacag acggaaaatc cagagaatcc attgagatgt cccatcaagc tctatgattt
2041 ctacctcttc aaatgccccc agagtgtgaa aggccggaat gacacctttt acctgacacc
2101 tgagccagtg gtggccccca acagcccaat ctggtactca gtccagccta tcagcagaga
2161 gcagatggga caaatgctga cgcggatcct ggtgataaga gaaattcagg aggccatcgc
2221 agtggccaat gcaagcacta tgcactgaga tgccttggcc atggcacaag agaaaccagc
2281 caggaaaaac cagacagact ttcacactaa agaagaggcc tccatttttt tttttctttt
2341 ttttattggt gtagttacga agcctttcag gctgcttctg tttaaaatat aaaagaaaac
2401 tttgccccct ttgcatcttc ataaacctgc tgcggcagac tcctcagccg atggtggctc
2461 tgggtttcct tgagtgtcat atgtcctaga aagttgctgg ctgactcttt tttgtctggg
2521 gcctggggaa agggcttgga ctgtgaaaag aaatgtggcc cctttccatc ttcaagagag
2581 atggaattaa tgatggatgg accctggagg gaatctcccc agccgacttc cactgggctg
2641 acagactttg ctgaccacag gggaacgatg ttcttttctt tcttcatgat cagacataaa
2701 cttagcatct taatggaaga aaaatgaggg gaacttcaat tatgatttat taaagacaat
2761 ttctattaca ccctccttta tgacaagtga cattttagat gtaaaagtaa aaactttacc
2821 atgccttttt tttttttgtt ggcctaacat tgaggcctta aaacctgagg ctcctgtgcc

2881 tgatggaatt cttgtaacat acacttgtgt atcatataaa gataccactc tgtttctctt
2941 aaaaaaaaa aaaaa//

- LOCUS: N55205 458 bp mRNA linear EST 20-FEB-1996
- DEFINITION: yv44g05.s1 Soares fetal liver spleen 1NFLS Homo sapiens cDNA clone IMAGE:245624 3' similar to gb:M91036_rna2 HEMOGLOBIN
- GAMMA-: A AND GAMMA-G CHAINS (HUMAN);, mRNA sequence.(HBBP1)
- ACCESSION: N55205
- VERSION ORIGIN: N55205.1 GI:1198084

1 ttttcttaga gagcatattt ctttattcta tttttatcat gcaggaactg aatcagcagg
61 caggatactg ctcttctccc caacctcccc catctaaagg agatgagatt ttgggacata
121 ggtacaaaca tagtggacca taggtacaaa catagtggac agggaactca gtggtcttgt
181 gggctacagc attaaccaca gcatttgtta gttactgcca agaagcctgt atctgtaggg
241 taaaatcctc gctgaagtgg gttgccaaaa caatcaatat cacgttgcct aagagcagga
301 agttctcagg gtccacgtgc agcttgttac agtgcaggtc actcagtgta gcaaaggtgc
361 ctttgaggtc atccgtgagc ataacagctt ttccgaagng gattcagcac cttcttgcca
421 tgtgccttga ctttggggtt gnccattatt ggcacagt//

- LOCUS: NM_024748 2158 bp mRNA linear PRI 26-JAN-2004
- DEFINITION: Homo sapiens hypothetical protein FLJ11539 (FLJ11539), mRNA.
- ACCESSION: NM_024748
- VERSION ORIGIN: NM_024748.1 GI:13376075

1 ggaggcaaac aacccatctc tcttggagaa tccacacttc gtttcttcta ggtccagcct
61 aaatgttacc tcctccaggg agcctttcca gatactgctt ctcctctctc ccccaactcc
121 ctcccacaaa tgaagttttc tcttctgaat tccttgctgg tactgtcact tatattcttt
181 tgggtagatc tctgggcatg catatgtcac ctcctgtgac tgtgggtttc ttgacaacct
241 aatcaatgca ttgaacaaag gagatgttta gacaacccat ataaaatgaa taagagaaat
301 agggtgaata ccgtctaccc caattgcaat cagattagtt tcctttattt tgaaaacgat
361 tagaactagt agaggagtta gatgtggccc actagggaac aaattttcat ctccaagccg
421 tcctctggct cgatcgggtc tgcactgggt ctgcatcaac ttgcgctggc tcagagggag
481 gctcagcccc agcctggcct tcctggtgcc cactcccaac atcctgagca gggccgactc
541 taggcacccc acgattagct gagtgatcta gggcaaggac ctaggcgtct ctaagtctta
601 gtttccccat ctgaacaatg tggataatga cacgtacctg gggtgggcag gctagagtaa
661 atgggaaaat caaagcgcca gccagagcgg gtcgcactgt tagttcctgt attttctcct
721 cctgcctctg gtttcttccg ttccgttcta gctgcctgaa gacccagccc gacagcgatg
781 cctgtcagcc cgcgtcgccc accagggcag ctgctctccc aactcgcatg gggggcacca
841 ccccgcctcg gtgccctaga gctgagcgca gccgcggctc cacggggatc gccagggcct
901 cagccctcgc ggccggcggc gcgggagtgt tgcggggcag ggaccagagc gcgatcagag
961 cggccacccc cgatttaggt cgccagcttt ccagtcactg cgatggccac tggggcgccc
1021 cttccatcct ggttaaattc tcattgtgat ccccagccct cttctcggtt ccccacaccc
1081 aggaggccgg ttctccgccg ctcaggctgg gagaggtcac cgctccgggc agggtggacc
1141 cccagctccg tctcggagga cctttcgccg gaggcgagca ggaaggggtt aaccatgagc
1201 ctcccaccct cacctcccag accctggtgc cgggctaagg cctccgcttc cctcctgagg
1261 tctcattagg aaaccctcac cgctattttc aacatttcct caatcaagtg cctttaaata
1321 gagctgcaca aacagattgg cggcaaaagc gtagatcttg tcacgattga gaccaatcca
1381 gtatttcagc cttgtttgct ttgcaattac gggttggctt gtggctacgg tgccaccggg
1441 gcaaatctgc agccgctcgc tttctcctct gcgctccggg cgggtggagg aagcgacccg
1501 cggagtcact gcagggagga gaggggacg cgcgcgggg gaggcggcgg agggcggagg
1561 agactcgctt gcaccccagg gccgcgggca cgcagagctg tgttgaagga catacttttc
1621 aggttcctga cagtcactga gctatacgca ataataaagt gcgtgattcg cgtgtggaaa
1681 ccagtgtcgt gtccttaccg gcttcctttt ctgtccacct tccctctctc ttctacctcc
1741 gtctcgcttt tcttcagtgc atgttagcgg tttgctggat ttcccttctc cctcgctgtt
1801 caactgctag catgatttgt tacattttcc acagctgttt attcttgttt gactttttgt
1861 acaaagtcac tttaaatctg ctgcagaaag aataaagcac ctagcccttt ggatggactc
1921 ctgcaggcgt gtggctgtcc ctggcccact tttaattctt tggagatggc tcttctgtgt
1981 aaatcagctg ctgggtgcgt tcggtcacct cgtaggccag cctagtttgt atcagcagaa
2041 cagtagtgga ctcgactcag gtcgtaagcc ttatatttca cagacaatta aatcttggct
2101 gctgttgaac aaaactgtaa tatttacttt gttctatacc gagaagcact tttaaagt//

- LOCUS: AF288389 5174 bp mRNA linear PRI 08-MAY-2001
- DEFINITION: Homo sapiens Clorf17 mRNA, complete cds.(GLT25D2)
- ACCESSION: AF288389
- VERSION ORIGIN: AF288389.1 GI:12620187

1 cacaactcgc ggctctaggg aaggccacag acctcagctg tacgagcgga acctggacta
61 gaggaacttt cctcaggact cagggcacac agcccctcgg ccactactgt ctgcgagccc
121 gaggcgcagg ggagcgccag cggctgcagg tacgctgcgc acccgcactg gggcctgggc
181 ggtgcagcgg cggcgggagg gtcctgggaa ccgcagccgc cgaggccagt gtgtgagccg
241 gccggccgcg gcaagccgca tcccccggcc gcccctcgca gccccttgct ccccggcagc
301 cgccgccgcc ctcacgcagc ccagccgggc gctcgcgcca ggacttcccc gccgcccgcc
361 tcggcccgga acatggctgc gcgccctgct gccaccctcg cctggtcgct actgctcctc
421 tcctcagccc tgctccgcga aggctgccga gcgcgcttcg tcgccgagcg ggactcggag
481 gacgacggag aggagccggt ggttttcccg gagtcgcccc tgcagagccc cacggtgctc
541 gtggcggtcc tcgcccgcaa cgcggcgcac acgctgccgc acttcctcgg ctgcctggag
601 cggctggact accccaagag caggatggcc atctgggcag ccactgatca caatgtggat
661 aatacaacag aaatattcag ggagtggttg aaaaatgtac agagactcta tcactatgtg
721 gagtggaggc ctatggatga accagagtct taccctgatg aaattggacc aaagcactgg
781 ccaacctccc ggtttgccca tgtgatgaaa ctacgacagg cagcccttcg aactgcgagg
841 gaaaaatggt cagactacat tctgttcata gatgttgaca atttcctgac taatccacag
901 accctcaatc tactgattgc agaaaacaaa actattgtgg cccccatgct ggagtctcgg
961 ggcctgtatt ctaatttctg gtgcggaatc acccctaagg gcttctataa gaggacccca
1021 gactacgttc agattcgaga atggaagagg acaggctgct tccccgtccc catggtccac
1081 tccaccttcc taattgacct caggaaggag gcctcggaca agctgacttt ctacccccca
1141 caccaggact acacctggac ctttgatgac atcattgtct ttgccttctc cagcaggcaa
1201 gcaggcatcc agatgtacct ctgcaacaga gagcactatg gctacctgcc catccccctg
1261 aagccccatc agacactgca ggaagacatc gagaacctca tccatgtgca gattgaagca
1321 atgattgacc gtcctccaat ggaaccctcc cagtatgtct cagttgtccc taaatatcca
1381 gacaagatgg gatttgatga gattttcatg ataaacctca aacgcagaaa ggacaggcgg
1441 gaccggatgc tgcgcacact gtatgaacag gagattgagg tcaagattgt cgaggctgtg
1501 gatggaaagg cactcaacac aagccagctg aaggcactga atattgaaat gctgcctggc
1561 tatcgagatc cctattcctc caggcctcta acaaggggtg aaatcggctg ctttctcagc
1621 cactactcag tctggaaaga ggtaattgat cgagagctag agaagactct tgtaattgaa
1681 gacgatgtgc gttttgagca tcagtttaag aagaagctga tgaagctgat ggataacatt
1741 gaccaggctc agctggactg ggaactgatt tatattggta ggaagaggat gcaagtaaag
1801 gagccagaga aagcagtgcc caatgtggca aacctggtcg aagccgacta ttcctactgg
1861 accctgggct acgtcatctc tctggaagga gcacagaagc tggttggagc caatcctttt
1921 gggaagatgc tgccagtgga tgagtttctg ccagtcatgt acaacaagca tcccgtagcc
1981 gagtacaagg agtattatga atccagggac ctgaaagcct tctctgcaga acccttgctc
2041 atctacccta cgcactacac aggccagccg gggtacctga gtgacacgga gacctccacc
2101 atctgggaca atgagacagt ggccaccgac tgggatagga cacatgcctg gaagtcccgg
2161 aagcaaagcc gcatctacag caatgccaag aacacagagg ccctgccacc gccaacctcc
2221 ctggacactg tgccttcaag ggatgagcta tgaaggctcc ctgggagtgt ggcccacatc
2281 agttcaacat cctctggttt ttctaaaggg ctattcatct gtttgctcca gttttctgtt
2341 ttgttttgtt cttagtggtc acagtcatct aaccaaagtg atctagtgtg atagatcgaa
2401 attaacatat ttttgaccat ggaaggaaat aaggaaattc aacccaaatt tcccaagacg
2461 gctgaaagac aggttttttg gaaactgtta agataaactg taatccagac acctaattct
2521 tcagttcact actcatgtga tactgattcc cacattaagg ttgaacaaca tggctcagag
2581 tcttgttcaa gagaaagtga tcaccgagct gtcacatcag caaatatgta gtcaaggcag
2641 ccaggccaac tagaccacac ttattggtct agtttgtccg ttttatatga cattgaaaac
2701 ttgtgtgtgc aacttttggg ggacaggaat cacttaaaat catatttatt tggcttttta
2761 tttaaaggat tctgtcacaa gtcttattga aaagtagatt ttttaaaaaa aaaaatctta
2821 gtccctgtta tccagtaggg gtgggtattt gggtccgact gagacttggc ctgtgaccat
2881 catggcagtt ggagttctca tatagaggtg accagtttgc catgtggata taatttagta
2941 gatatttgac agtttgtgta ggtatttgag ggaaaaaact caatgtttgg cttttttatt
3001 atggccactc gagtcaggat gctctattta taaagataaa tgtaatatat aaagggtgag
3061 gactggctgt gcatcctgcc ctgtcccggg tttgcgcgct gctacagagc ttcacgctct
3121 ccgctccacc ccttagcctg ggaacccacc gcagtgwagt tctgtgagtc actgctaaga
3181 gacagagcac attttcaggc cagcaactat ccttgccagg gttttttcat tatattttga
3241 attatttatt ttacaaaatg ggcgaagata ttgtctttta ggataaggca gagaaacaga
3301 tgttgcagac ttccacagca cccggggagt ggtgggtgtg gacacattgg ttcggcaatc
3361 tgattctcct gaatttccca gccaggctct tgtggggagg cctgtggatg gggggatttg
3421 aactatttgg aaacaaatga ttctctatct caggtgagaa acctggtcag aaacaaaggg
3481 ctggtcacct gatttaggcc agcaaccagg gaagctctta gaatcccagg cggacaccct

3541 ttctcaaaag atatccccta agagtccttt ctgctttctt cacagattga ttttatgtaa
3601 aatgcagagt tggactacac gatttcttcc cactccacaa tctgtcatcc tagtatagat
3661 catggtggtt tccctcaagt ttatgttctc atgccctcaa tctgtaaatt tttgtctcca
3721 gaaaaaccct cccaggcatc ccataccagc accgttcctc atcactgtcc atgcaccatg
3781 cagccatatg gggggccgtg cacaccccaa accctgagct tcacacttaa actcatgggg
3841 aggggccctt cagagcagag tccacaggcg ggtggtgcta catacacaag cttagtgtac
3901 gagtgtaaga tacactttaa gccagacacc taattcttca gttcactgcc catgtgatac
3961 tgattgccac attaaggttg aacagcatgg ctcagagtct agagaaagtg atcaccaagc
4021 tgtctcatca ccaaataggt agtcaaggca gcctcatctc cccaggtgag gggcgggtcc
4081 ccactttagg acaagaggca gcttgccttc caccagacgc cagcctcggc cttccttccc
4141 gactcactgt gggtaccctt ctacactgac cagcaagcta ggccgctgga ggaaagggaa
4201 ctcacccaac tctaaattgt gccgcttaga cttagctgtc agtgtgactt cctttcccac
4261 ccacccccag aaaaacagaa agagcatctg gggagcgagt gaaaattcct taggtgattc
4321 ctaagatttc cttgggtatc tggtttttgt tttcatattt gagtgtgtgc atgtgtgcat
4381 gactttaatg acttttttaa tggggtggta gttgtctggg ttgctgggtt tgaaggaagt
4441 ttgggttgat ttttgtggtg ttttgtttaa tagagaattt ttttttccct gtycccctgt
4501 cagctggtct gacagattta agaactctca ttcttaaaag actttggact taaatcctag
4561 cattttagac taggactgtt ctactgtgaa gaaagttctg tctcctttag cccggtttgt
4621 ttctccctgc tcaggtctag aatcccaagc agtgtccttt cctggtgaac actgtgagcc
4681 gcagatgtga ctttttttta aagtcatctc ttcagcaatc cagaggttcc ttgacctcat
4741 tatttgtcct atctctccct tatagtccta agccaagaca tttgaccttt gacatttgac
4801 ctttgcagtg tcatgtgagg gcgtcagtat agaggccttt gcatctgggc ctggcacccg
4861 ctctctgcct ctggaggcta aaccctgtct ggatttctct tgggatctaa cgtgggatct
4921 tctggacaga caaccgtgac atcagcagtg ctggtgctgc tgtgtgtgga ctgaacacct
4981 gcactttgca gaggacacgc tgcatggccc ccgcttgcgg ttcattcagg cctgctgcag
5041 gagctctgag aacaagaaag agtggacacc cgttcccctg catcatctgt cttgcgtgct
5101 atttcagagt ggggaagtga taaactattt gccttctgga gctctttgtg aaaaattaaa
5161 aaaaaactta gctc//

- LOCUS: AL121891 125856 bp DNA linear PRI 18-JAN-2007
- DEFINITION: Human DNA sequence from clone RP5-1187M17 on chromosome 20, complete sequence.(UBOX5)
- ACCESSION: AL121891
- VERSION ORIGIN: AL121891.22 GI:9367201

1 gatcataggg gaggggtacg ggccgccagg ctgtcatggg ccgcggaatg acagcaattg
61 cctctactgt gactgcagcg ctctggattc agggagaggg acagactggg gggtcacctg
121 gagaccatct ctgagctttc tgattaccca gaaatggcgt agggtccaag aagagggtaa
181 gaggaaccga ggggctgcgt gttaggggca gggacaggcc ctggcctagg agctgtgggg
241 caggggacag ccaggtgtgt ggggagggtt agccctgtga caacaagata agggaccaag
301 aacagcacaa gcccagagtg gtgaggacag gggctgcacc agagatgaga atccctgcct
361 cgtggacaag gcagccgccg tcgtggagtc aagggacaca gaatgagagg ggcttgttct
421 cacagggtag gggggtggca ggttgctggg gaaagtgcaa gacatgacac ccccgcaccc
481 ccatagagca gggcatgggg actcaggccc tgaaactggg ggccttgggg acacattggc
541 acatgacccg tcaccccctc ccaggtagaa acatgagtgt ggagagctac tatggggaga
601 aagaactcca tgccaagtaa ggttcaaagg cggggggagc cccaggaaac ctgacaacag
661 tttgtcccaa ttcctacccc caccaggacc ttagtatgtg gcaaccaggg caggtacatc
721 tcaggcctgg ggtgggagag ccgggggtgg ggggtgtctg ggcctggggg aaggtgcagg
781 aggagccata cctaaatgga cccagcctga gaaagccact tttggtccct aatcccaagg
841 tggcaggtct aaagaggaca tggcctctct ccctgggcca ccagaatgcc ccgctggtct
901 ggggttcagg cccggtaccc cctgcctctc aagctgtgcg cagtccccag ctctcgcagt
961 tcccacaggc tcttggcctc ctctccaggg tgcatagagg tgctgcgagg aaattccagg
1021 ggacacagca gccccatctc cagcaagggc agtgaggggg ccagcaaccc aggaggggcc
1081 tcctatactc aggccatggc gtcggtcttt tcaggaatga cccctcaggg actgcaagga
1141 ccctttgaga ggcaacactg ggcagttgtt attgcaggaa cactggctcc caatagcttg
1201 gctcagggtc tggcctctgt tgctcgacac ttcatggctt gggcagctca ctggacttct
1261 ctgtgcctta ctttctgcca gtgtgaagta aggaaaatca tggtacccac ctcctagggt
1321 caatgtgagg acagaaataa ataatttatt atacaaaagc tttagagcca ggcatggcag
1381 cttatgcctg taatcccagc tacttgggag acccagacag gaggttgaga ccagtctcgg
1441 caacatagca agaccccatc tctatttttt tttttttttt ttgagacaga gtctcactct
1501 gtcaacccag gctggagtgc agtggcacaa tctcggctca gtacaacctc cacctcccgg
1561 gttcaagcga tcctcctgcc ttagcctccc aagtagctgg gattacaggc acgcgtcacc
1621 acgccaggtt aatttttgta tttttagtag aaatggggtt tcaccatatt ggccagtctg

1681 gtcttgaact cctggcttca agtgatccac ccgcctcggc ctcccaaagt gctgggatta
1741 caggcatgag ccaccgtgct cggccccatc tctaatttta aataaataaa taaatacttt
1801 ttttttgaga cagagtcccg ttctgtcacc cagactggag tgcagtggcg tgatctcagc
1861 tcactgcagc ttccaactcc ggggttcaag tgattctctt gcctcggcct cccaagtagc
1921 tgggattaca ggcgcctacc accacgcctg gctaattttt ttgtattttt actaaagatg
1981 gggttttgcc atgttggcca ggctggtctt gaacctgacc ttgtgatcct cctgccttgg
2041 cctcccaaag tgctgggatt acaagtgtga gccgccgcgc ccggcctata aatacatttt
2101 aaattgaaaa aaaaaaacaa cagctttaag gctgggcacg gtggctcaca cctgtaatcc
2161 cagcactttg agaggcaaag gcagaaggat cacttgagcc caggagttcg agaccagcct
2221 ggacaacata gaccttatca ctacagataa tttaaaaatt gggcaggtgt ggtggtgcat
2281 gcctgtggtc ccagctactc gggaggctga ggtggaagaa ttgcttgagc ccaggctgca
2341 gtgagccata cctgagccac tgcactccag cttgggtgag agtgagacac tgtttcaaaa
2401 gaaaaagtag ctttagaaaa gtgcctgcat gtggccaggt gcggtggctc atgcctgtaa
2461 tcccagcact ttgggaggcc gaggcaggcg gatcatgagg tcaggagatc tagaccatcc
2521 tggctaacat ggtgaaaccc cgtctctact aaaaatccaa aaaaaaaaaa aaattagccg
2581 ggtgtgatgg cgggcgcctg tagtcccagc tactcaggag gctgaggcag gagaatggca
2641 tggacctggg aggcagagct tgcagtgagc caagatcgcg ccactgcact ccagcctggg
2701 cgacagagcg agactccgtc tcaaaaaaaa gaaaaagggg gctgcagcat tgtaaagtta
2761 atgaacagta gtattgcatt agtaatgcca ttattctttg ttttgttttg tttgagatgg
2821 agttttgctc ttgttgccca ggctggagtg cagtgacgcg atcttggctc actgcaacct
2881 ctgcctccca ggttcaagcg attcttctgc ctcagcctcc tgagaagctg ggattacagg
2941 cgcccatctc cacgtccagc taatttttgt atttttagta gagacagggt ttcaccatgt
3001 tggccaggct ggtctcaaac tcctggcctc aggtgatcca ccccactcag cctcccaaag
3061 tgctgggatt acaggcatga accaccatgc ctggccaatg ccattattct tgttaagagc
3121 taggagattt ttccagagag agcagcttcc tggattagtc taggaggaac attttcattc
3181 tactcctggc ctggaaacaa gcttggtctg aggccgttgc cccgctgaga ctctgtgggt
3241 tgtgaggcta gctgaggaga gaggctggag gcctggctaa ggcgccttgg cccagcacct
3301 taccccttct tcactgcagg gggttgagtc aacaactgaa aacggatgag gccagactga
3361 ctgaagggcc caagccatgg gacagccagg acctctgtgc tgaccagatc tgtctaccag
3421 gatcttcatt ctaagaccgg cctcccacac cgaggcccct ttctcccagg acctaccttc
3481 tcggaacctt ggctgactgg gaaaagtctc tatcctgtgc tgactggctg catttccaac
3541 acatcacccc atatgtcaca tggacccccc agacactgca caccctteta cacgtccctt
3601 acaggtttcc tttcccaatc tactttttac atcttctcgg tccttttatc tcctacatct
3661 cagctcatga tcctgattca ttcatccttc caaaagaaag gagtcagctc cttcaccagt
3721 tccttccccc tcccctgatg tataccettc atccaccatt ttctaaatac aatgacgtta
3781 tgatagatca gtattacaag ttagattgca attctgccct ctgagctaag ccacgtagta
3841 tattatgatg atctttctat ctgtgaacaa ctttttgtat tccttggagt tagtggtcat
3901 cttggttttt catttgtggt ccatctgccc tttgtttttg tttttgtttt tgtttttgtt
3961 tttagagatg gggtctcact ccacctgcca tttatccaga agtcaacatt tattcttcaa
4021 ctccctccac ctgggcttct gctcaaacca gcccattgaa actcctgttg tcaaggtgtt
4081 aaactacttc catattgaaa aatgtggtcg gctgggcgca atggcttaca cctgtaatcc
4141 cagcactttg ggaggccgag gccggtggat cacgaggtca ggtgttcgag accagcctgg
4201 ccagcatagt aaaacctcgt ctctactaaa aatagaaaaa ttagccgggc gtggtggcac
4261 ctgcctgtaa tcccagctac tcgggaggct gaggcaggaa aatcgcctga acccaggagg
4321 cggaggttgc agtgagccaa gatcgtgcca ctgcactcca gcctgggcaa cagagcaaga
4381 ctccgtctca aaaaaaaaaa aaaaaaaaaa aaaacgaaaa atgtggtggt gatgtctatg
4441 tccttatttc acatgatcta gcagcatttg acatgggtga acacttatcc ttttcttttt
4501 tttttttttt ctgagacgga gtcttgctct gtcgcccagg ctggagtgca gtggtgtgat
4561 ctccgctcac tgcaagctcc gcctcctggg ttcatgccat tctcctgcct cagcctcccg
4621 agtagctggg actacaggtg cccgccaccg cgcccggcta attttttgta tttttagtag
4681 agacggggtt tcaccgtgtt agccaggatg gtctcgatct cctgacctca tgatctgcct
4741 gcctcggact cccaaagggc tgggattata ggcgtgagcc accgcgcccg gccacactta
4801 tccttttctg aaacacactt tgtttggttc caccgatgct acactctctt ggttttcctt
4861 ctgttacacc gggtgttctc tctgtctccg tttctggctc cttctctttg accttgactt
4921 ctaaacgttg gagcatccca ggaatcagtc ctgtgtccct tctcttcctg ggtgatccca
4981 caattttata tacttactac aagctaataa ttcccaaact ggtatttcca tcctgggcct
5041 ctccccaaag agccagactt atagtttcaa ctgcccagtg acacctcaca ttggatatcc
5101 aataggcttc tccaacttaa caaagtcaac acgcaactcc agattctcag ctgtttccct
5161 gctcttccag ggttcccacc tgtgtgatct taccaccatc caaccagctg ctcctcccag
5221 aacctgagac tcaccctggg tcttctcttt ccctcactgt tgcttatgtt acctctaaaa
5281 tcagtctcaa ctccaactgc tgctttggga tctgagatcc tgctgtttcc gtgtcctgga
5341 agattcctct acccgcaccc tccccaggat tcatttgatt ggtttaaatg gcatctctta
5401 agaggaggct cccctgactg ccctatctag agcatccccc atctctgtta tcttctgtct

5461 gggactatgt tgatgaattt catagcacct actgcaattc acaattcatt ttttgtgtgt
5521 ctatcttttt gtgtgtgtat gcctataacc cctttaaaat gaaacaccag ggcagaaagg
5581 aaggactcta cctttcatgc tcagtacggt aaccccactc caggtagagg gtgttcaata
5641 aattatggaa agaaggaagg gaagctctgg gtaccagctt cagctctcca gggagttctg
5701 tgtgacccag ggcaagtcac acctgttccc aaggttattt acttgaagta ctaaccccac
5761 ttcctgtccc actgaggtct taagggaatc agatgagggt gccataaagg gttaagattt
5821 ctgcaatcct tacataataa aacgtggcaa gcctccagat gaaaccccaa atgtcagaat
5881 gttctataaa gtacttcctt acccttagac caccttttct gagaagaagt aacagttaac
5941 taatcttagc agagcatctg ccctgcccac accttctgtt tcttattccc ctgctgtata
6001 taccatagaa catctccact actgccagag agggcatggc ctcattttct ttctatcttt
6061 tttccttttt aatttttttt tttttttttt ttttttttgt agagatgagg gtctcactgt
6121 gttgcccaag ctagtctcaa acccctggtc ccaagtaatc ctcccacctt ggcttcccaa
6181 agtgctgcaa ttacaggcgt gagccactgt gcctggccat tggcttcatg ttctttttct
6241 tttcttttct tttttctttt ttcttttttt tttttttttt ttttgagaca gtgtctcgct
6301 ctgtcgccca ggctggagtg cagtggtgcg atcatggctc actgcaaact ctacctcctg
6361 ggcttaagag tgatcctccc acctcagcct cccgagtggc tggaaccaca gcaccatgcc
6421 cagctaatct ttgtttttgt gcagacagga tttcaccatt ttccccaggc tggtctcaaa
6481 ctcccgagcc caaatgatgt gcccaccttg acctcccaaa gtgccgggat tacaggcatg
6541 agccaccgtg cctagctggc ttcattttca aaagaacact gctggtagca gtaagtccag
6601 ctttctctct tttcacctgt tggctcactg caacctccgc ctcccgggtt caagcaattc
6661 tcgtgcctca gcctccctag tagctgggat tacagatgct caccaccatg cctggctaat
6721 ttttgtattt tttgtagaaa tgggctttca ccatgttggc caggctggtc tcgaactcct
6781 gacctcaagt gatccgccta cctctgcctc ccaaagtgct gggattaccg gtgtgagcca
6841 cggcgccagc ctccagtagt atttttgagg acccacacaa atgctctgta aactatgaag
6901 aggggaacaa gagcaaacaa aagatgttgt gggagcccaa gacctgctta ctgaattgaa
6961 tcaaagagga tacattgagc agggatgaga aacctctgag aaaaatgctg agcctgaggg
7021 gacaggtatg gaactgaaat ggatccactg caggaccaag taaaaaaggg cgggggaggc
7081 cttctgctct ttccagacca aggcacatct cagctggatt gaagagctaa atagaataaa
7141 atcagccgac aagctgggcg cggtggctca tgcctgtaat cccaacactt tgggaggccg
7201 aggcgggcag atcacgaggt caggagttca agaccatcct ggccaacatg gtgaaacccc
7261 gtctctacca aaaatacaaa aaaaaatgag ctgggcatgg tggtgcgcgc ctgcagtctt
7321 agctgcttgg gaggctgagg caggagaatc acttgaaccc aggaggcgga ggttgcagta
7381 agccaagatc gcgccactgc actccagcct gggcgacaga gtgagactcc gtctcaaaaa
7441 aaaatcagct gacgagggcc ggggagcggc ggggagggga tgcgggaagc caggtctgaa
7501 tatttatcgt atctctagag gggggttaca ttaaactttt aaaagaaatc agaaatgaaa
7561 agacaataaa aattctttaa cttgcaaaaa taacctaagt aaaaggcaaa ataaacgaag
7621 aaaaatattt ttgacaaact cgtttctata tggaaaacct gtacggcagc aagacctgta
7681 gactcgcatc tagagacgct caaccctagc tccgtgttcc aagtgacgtg gcctgtcgca
7741 cccgccgggg acaatgactg cggccctggg gtcccacaga cgttcccttt gctagactta
7801 aggaagagct aggcctgtcc gcgctcccag cccccacgca gccagacacc cgcgggtgct
7861 gagatgaaga gtgaagtcac caggagagac aggaaacagc gcgggcctcg ggacccgag
7921 ggcgcggaag ctcgggggcc ggggtcgagc acagattggg aggcgcagcg ctgggcgcac
7981 gtccccgtcg gcccggcggc ccctaatgag gcgcgctgtg cgcagactgc taagagcagc
8041 atgagcacgg ctccggcggc cccagggccc ccgcgggtcg gcgccacccc ggagcgcggt
8101 gcccggaatc acttagcgcc atcagccggg cggctgggag ggggaagcgg ccgatccggg
8161 cggctgggag ggggaagcgg ccgatccagg cggcagcgga gggggctgca gcggaggcgg
8221 ccccggcttg cacgctcgcc cttccgcctg cccggcaggg ccccgtctga gccccaggcc
8281 ccaggcccag cggcgccgct cgccggtgaa ggaaaccagc gcagagaggc cgcgctgcca
8341 ggcgcgtggt ggtccggggg tccacgtgtg agcctcgccc taggctgcca attgagagtt
8401 ccacctccgc ccagagagac cccacctcac ccctccaacc tggggcgcgc gggccggcgg
8461 aggggtcctg ggcgagtggg caggataggc cgccgcgcgg ggtgggcttc tacggggtg
8521 tactcgttgc ctgtggctgg ggactgaaat tccccggaaa aggagacggg gaggacacac
8581 ctggagggat aaacagcttg tgcagggatg tgaaagagag aacccagctg tgtgtgcgtg
8641 tgcgcgcgtg tgtgtggtag ggacgtcata caggacgcgt ggcctacgaa cgtgctaaac
8701 cactggcaat aaaaacacag agtgtggggc cgggagcggt ggctcacgcc tgtgatccca
8761 gcactttggg aggccaaggc gggtggatca cctgagatca ggagttcaag accagcctgg
8821 ccaacatggt gaaaccccat ctctactaaa aatacaaaaa ttagccggcc gttgtggcag
8881 gcgcctgtaa tcccagctag tcgggaggtt gaggcaggag aatcgcttga gccagggagg
8941 cggctgttgc agtgagccga gattgcgcca ctgcactcca gcctgggcaa caagagtgaa
9001 actctgtctc aaaaaaaaaa aaaaaaaaaa aaggagtgtg tgccagcatc actgttttaa
9061 gccttttacg tatattttcc tccttgaatc acataaaccc tgtgaaggag gaagtatttt
9121 tatctctatt tgcagacagg aaactgaggg gcagagaggt tgaataactt gcccaagtta
9181 ttcaacgaac aagtgataaa cagcagacct gggattcaaa ctcaggctcc agggtctgtg

9241 cccttcacca cgctactgcc acaaaaccct agcagagccg ggtgctgggg cacgcgcctg
9301 gagtcccagc tactcctgag gctgaggcag gaggatgctt gagcccagga ggtacaggct
9361 gcagtgagcc atgatcacgc cactgcactc ctgcctgggc tgccgtgggc tatgcctagc
9421 tggcgtctgc actaagttca gcatcagcat ggtgacctcc cgggtgcaag ggaccacca
9481 ggttgcctaa ggaggggtga accgactcag atcagaaatg gagcaggtga aaaccctgtg
9541 ctaatcagta gtgggatcct gcctgtgaat agccactgca ctccaggctg ggtgacatgg
9601 tcagaaccca tctttgaagg ggaaaaaaaa aagattgggc ttgggggtgc atgccggtgg
9661 tcccaactac tagcaaggca gaggcaggat gatggcttga gcccaggaac cggaggctga
9721 aatgagctat gatcgtgcca ctgcacttca gcctgggtga cacagtcaga ccctgtctta
9781 taaaaaataa taataataaa atataaaata aacaaaataa aatagcagtg actaaacccc
9841 aaagacattt gtaatttact ataattcaag ttgggaagtg ggtggtccca ggcctggctg
9901 aaattcaggg atggcatcta gggcttcccc actcctgtct ggtaccctcc acatgtctga
9961 gtgtccctcc ttgtggcaag gggacagcca caaaatgggt tccctcttct gagcctctcc
10021 gctctcaggg aggagaaacc tgcccagagt ccccacccct aaatccctcc agactggaca
10081 agcaccacta gccggctgcc ttctttgggt taggccagcc aaagctcacc cctaagatta
10141 ggtgtgctca caggcccctg acaaaatggg gtcctgttgg tgaggaagag gagggacggg
10201 actggctgct gggtcagtaa ctggggtatt tgtccccggc cccaggctgg aaggcattgg
10261 tagacttgta cagatcactt cacttgtggg gaccctgtgg cacagagaga ccccgtggct
10321 tgtccgggac cacacagcta agccgggcag aactactgag cgaggagcct atcagtcctg
10381 gttcccaagc tccctgcccc agcctgcccc tggcctccca gtgccttggc ctgcctcaca
10441 tgcctgggct tggccccagg ttatcttcag agccatccca tcctgcctcc ctgactgacc
10501 atgccacttc ccgctcaaaa cctgtccatg gctccccatt gccctctgga caatgcccag
10561 acatgtccaa caggcctgca ggtctggccc tgctaactct cacctttgtc cactcccccg
10621 agtgacacac cgttgtcccc agcccccaag ggtacgccaa ttccaggcag tgctgccggg
10681 tcttctctgt tggtgccttt tatgtgattt tttttttttt tcttttgaga cagggtctca
10741 ctctgttgct cagactggag tacagtggcg tgatctcagc tcatgcaacc tctgcctctt
10801 gggttcaagc aattctcctg cctcagcctc cggaatagct ggcattgcag gcatgtgcca
10861 ctactgcccg gttaattttt ttattttcag tagaaatggg gtttcaccag gtcggctagg
10921 ctggtatcga gctcctgacc tcaaatgatc cacctgtctc agcctcccaa agtgctggga
10981 ttacaggcgt gagccactgc acccgactcc ttttatgtga ttgttttaaa gaaccaacct
11041 ctggttgtgt tgaatctcca tagtgtttgt ttcactgatt tataccttta ttacatttca
11101 tcttctttct ttctttcttt tctttttttt tgcgacagag tcttgctgta ttgccaagga
11161 tggagggcag tggtgtgttt ttcagctcac tgcagccgct gcctcccagg ttcaagcaat
11221 tctcctgtct cagcctctca agtagctggg attacaggca cctgccaacg cacccagcta
11281 atttttgtat ttttagtaga gatggccatg ttcaccatgt tggccaggct ggtcttgaac
11341 tcctgacctc aagtgatcca cccacctcag ctcctaaagt gctgggatta caggcgtaag
11401 tgtgtgtttt tttagagata ggctctgttg cccaggctgg agtgcagtgg tgcaatcata
11461 gctcactgca gcctcaaact tctgggttca actgatcctc ttgcctcggc ctcccaagta
11521 gctgggacta taggtaccga ccgccacgac cagctgattt ttatttttta tgtttttgta
11581 gagatgggat cttattatgt tgctcaggct agtctcgaat tcctagcctc aaggaattct
11641 ccggtgttgg cctctggaag tgctggaatt acacgtgtaa gccaccacgc ccggcaaaga
11701 ttttattttt tattcaacct gagagctccg gttttaacaa ggcaaattga atctgttaac
11761 attgatggtg gctgcccgtc tacttggatt catttgcaaa catttagctt ctgttttctt
11821 tttgttattg tggagacacg gtctagctct gtcactcagg ctggagtgca gtggcgcggt
11881 catcaaacta tcctcctacc tcagattccc aagtagctgg gactacaagc ccaacccacc
11941 atgccagcta attttttttt ttttttttga gacagacttt tctcttgttg cccaggcttg
12001 agtgcaatgg cacgatctcg gctcactgca acctctgcct cccgggttca agcgattctc
12061 ctgcctcagc ctcccgagta gctgggatta caggcatgtg ccaccacacc tggctaattt
12121 tgtattttta gtagagacag ggtttctcca tattggtcag gctgaactcc tgacgtcagg
12181 tgatctaccc gcctcagcct ccctaagtgc tgggattaca ggcatgagct accgtgcctg
12241 gcccatgcca gttttttttt tttttttttt ttttttttga gacagagtct cgctctgttg
12301 cccaggctgg agtgcagtgg cgcaatcttg gctcactgca acctccgcct cccaggttaa
12361 agcaattctc ctgcctcagc ctcccaagta gctgggacta caggcgccta ccaccacgcc
12421 tggctaattt ttgtagtttc agtaaagacg gggtttctcc atattggcca agctggtctt
12481 gaactcctga ccttgtgatc cgcccacctc agcttcccaa agtacatgag ccaccgcccc
12541 cggccaccag ctaatttttt tttttttttt tgagatggag tctcgctctc tatcacccag
12601 gctggagtgc agtggtgcca tcttggctca ctgcaagctc tgcctcccgg gttcatgcca
12661 ttctcctgcc tcagcctcct gagtagctgg gactacaggc gccggccacc atgcctggct
12721 aattttttgt atttttagta gagacagggt ttcattgtgt tagccaggat ggtctcaatc
12781 ttctgacctc gtgatccgcc tgtctcggcc tcccaaagtg ctgggattat aggcgtgagc
12841 taccgcgccc agcctgccac cagctaattt ttaaaaaaat gttttataga gacatggtct
12901 catatgttgc ccaggctggt ctcgaacttc tggtttcatt gcttctgttt tccatttact
12961 atgacaccct ttcacttctt tttccttctt tcttgtctta aaatggatta tctttaaatc

13021 ttatttctat tatttcagta gtttaagcaa tatttaagat taatccatat ctttattctc
13081 ctccaaacaa gctgggttcc ttagaatgtt ttaaaaagga tttcttgccc agtgcggtgg
13141 ctcacgcctg taatcccaat actttggcag gccgaggcag gtggatcact tgaggccagg
13201 agttcgagac cagcctggcc aacatgacga aaccctgtct ctactaaaaa tacaaaaact
13261 agccgggtgt agtcgtgcgt gcctatagtc ccagctactg aggaggctga ggcaggagaa
13321 tcgcttgaac ccaggaggtg gaggttgcag tgaatcaaga ttgccctact gtactccaac
13381 ctgggtgaca gagcaggatt ctgactccaa aaataataaa ataaaatata aaatgaaaag
13441 gattccttta ctgtaatcat atattttatt gtagttaatg ttttagtttc accttgtttt
13501 taagacttct cagtactcat tgctgttatt attaatattg attaaaactc cactcaccct
13561 tcctttgaca tccagatagt ctgtacctcc tgatggaaaa ataaaaaaaa aattccccag
13621 atcaataact gcatacaaaa cccaggggct gtgcagattt actgcagggg gaagactata
13681 tttggaaccg gcctccatta ggtgccccca ggctaaattt agcaaaatcc ttctcatttt
13741 ccaagaccta ttcatttccc tgcactaaag aggaaaactg tataaatagg gataatgtag
13801 aatagctaat gtttactgta tgctgataat gtgttcttgg catgtacatt ataagccaca
13861 taacaatatc atgaaggcat gggctcacac ttgtaatccc agcacttagg gaggctgaca
13921 cgggcagatc acctgggttc aggagttcaa gaccagcctg gccaacatgg cgaaacccca
13981 tctctactaa aaatataaaa attaggccgg gcaccaggac tcacgtctgt aatcccagca
14041 ctttgggagg ccgaggcagg cggatcatga ggtcaggaga tccagaccat cctggctaac
14101 atgctgaaac cccgtctcta ctgaaagtac aaaaaattag ccaggcatgg tggcacacac
14161 ctgtagtctc agctacttgg gaggctgaag caggagaagg aggtgaaggt tccagtgagc
14221 tgagatcgtg ccactgtact ccagcctagg tgacagagcg agactccatc tcaaaaaaaa
14281 tgaataaata aaaaaattag ccagatccct gtagtcccag gtatttggga ggctgagaga
14341 ggagaattgc ttgagcccag gaaacagagg ttgcagtgag ccaagatcac accactgcac
14401 tccagtctgg gcaacgggag tgaaaccctg tctcaaaaac aaacaaacaa atatatcaca
14461 aaatatgttt agtgtttttg atgggttttt ttcttttctt ttcttttttg agacagagtt
14521 tcgatctgtt gcccaggctg gagtgcaatg gcatggtctt ggctcactgc aacctctgcc
14581 tctgtgttca agcgattctc ctgcctcagc ctcccaagta gctgggattg caggcacgca
14641 tcaccacacc aggctaattt ttgtatttc aatagagatg gggttttacc aagttggcca
14701 ggctggtctt gaactcctga cctcaggtga tctgcccacc tcaggctccc aaagtgctgg
14761 gattacaggt gtgagccact gcgccaggtc ttttcttttt tcttttttga ggcaggatct
14821 ggctttgtca cctaggctgg agcacagtgg ggcaatcaca gctcactgca gccttgacct
14881 ccttcctggg ctcaagtgat ccacctgcct cagcctccca agtagctggg aacataggtg
14941 tgtgccacaa cacaagctaa ttttttttct ttttttgaga cggagtttcg ctcttgttgc
15001 ccaggctgga gtgcaatggc gcgatattgg ctcactgcaa cctccgcctc ccaggttcaa
15061 gtgattctcc tgccttagct tcccgagtag ctgggattac agggatgcac caccatgccc
15121 ggctagtttt tttgtacttt attagtagag atggtttttc tccatgttgg tcaagctggt
15181 ctcaaactcc caacctcagg tgatctgccc acctcggcct cccaaagcgc tgggattaca
15241 gccatggtgc ctgacctgag ggtcaagttt ttaatatttg tgtattagtc attgactaag
15301 ggggctgcag ggagggagtc tctcttggtg aagtggctac cactggctga aggcacgcgt
15361 ccagaggaag gccagctgca aaccactgac cccaatgtgc ggcatctgag ggacggacac
15421 gcccaggggt caagagaacg gagcctggga gtggcatcca cagggtctgc tgtaggcgac
15481 tccagtgcct tcctcttgat ccctctgctc aggtgcctac tccagggagg ggctggcttt
15541 ttggattagg ttggatgatg accatcctca agtgtctgaa taaagctcct tcagagtgaa
15601 tgcaatggag aaagggtagt gccttgagag gatctcagga tgatagtagg aagggaaata
15661 aatgctgtaa agctaagcag ccctcacccc cacaaatcca ctgagatctt ttcttttctt
15721 ttagagaggg tctcactcta ttgcccaggc tggggggcag tggcacaaac acagctcgct
15781 gcagccttca cctcctgggc tcaagcgatc ctcccatctc agcctcctga gtagctggga
15841 ctacagacgt gtgccaccat gcccagctaa ttacgttacc caggctggtc ttgaactcct
15901 gggctcgaac gatcctctca ctttggcctc ccaaagtgct gggattacag gcatgagcta
15961 ccacacccgg cccactgaga tattttctga tatcagaacc tgagtttcat ctttcctgcc
16021 cctaccccta accttagcta aaccaacctc ctcctcccgc ttcaggtcaa tgtggagccc
16081 tagacatgct ttattcacgt gtttccctgt ctttctgccc tactagagag tggtcctttc
16141 ggtcactaca tcccccggac ccgccatgga cccaaccctg ggtcttgggt aaatgaccag
16201 tcgagttggt gtgtcctctg cctgcccagg cccagcagca gtgaagggca cgaattatca
16261 gcacaggggc atttgcagtc cttcttggct aaggaggatc ccggctttgc ctcctggttt
16321 ctccgtcctt ccctagcccc gctgtagcaa tcatctagga ttttgtggtt gtttccgtta
16381 ctgttgctgt gtaacaaacc gctcaacatg cagtggtatc aacccaccgc cattttatga
16441 tgctcacagg ttctgacggt cacagtgaac accttttatc tgttccacaa tgtctggggc
16501 ctccacagga aggaggggcg ggctacaagc tgaaatcacc tgggccttct tcattcacat
16561 gcctgggcac ttgggcggga gttgcccaaa ggctcagctc agttgcagtg ctgttgcctg
16621 gactgcctgc tttcctgtga gttggattca accttttttt tttttttttt ttttttttg
16681 agacagggtc ttgctggagt gcagtggcaa gatctcagct cactgcaacc accacctccc
16741 aggttcaagc aattctcctg cctcggccta ctgagtagct ggaattaggc acccgccacc

16801 tcacccagct aatttttgta tttttagtag acttggggtt tcaccatgtt ggataggctg
16861 gtctcgaact cctgacctca ggtgatccac ccatcttggc ctcccaaagc gttgggatta
16921 caggcgtgag ccaccgcgcc cagcctggat tcactcttga agccattgaa tggcagctca
16981 gggcttcgat gaagagtgaa tctccagaaa acaagggcag agtgacatgg cattttatgt
17041 tagcctcaga aatcacagaa ggtcagtgtt gccacgcttt ataggctgaa gcagccacaa
17101 gcctatccaa ggtcaagaga aggaacagag cccacttttg gctgggaaag tgggccaaat
17161 aattggcagc caataattta aaataatcag tggcatgaag ccaggaggaa aggagactaa
17221 gctttggaac agattttttc aaggtggaaa aatccaaggt tgaagagaaa ggtggacaag
17281 cagctgcagg gaagccgtga gggaggcagc taaaagcagc cgtctaagca gcactgccga
17341 gtcactccac agtgccaacc acacacccag tgccaaccac attctggcca cggaagcggt
17401 ttcactctca gactgagttg caggggctcg cagctttgca ggtgttgtag acccacctgg
17461 gccacgccca agcctgtccc tgggtttctt cagtagagga aacatttagg gtttaggcca
17521 cttcgcatca tttccatcat ctcatgattg aaggtcaatt cgccgaggct gtcgcatgca
17581 gacacgcgtg cctggtatct cattccccct attgctcaca cttcactttc ccctcctcag
17641 tcccagaggc cggacctccc cagcttgatg tcatcctttg ggtggaaggg aggaaggcag
17701 gtgagagggg ctctctctgg aggctcctgg tttctgagac aggctaatta caggcttcct
17761 aattaatgag ccatggggtt gcatttcatc cgctgagtca gagctggtgt ctgggccctg
17821 ggcccaaggg aagggtatca agaatcatgg gagggggagg ccagcctgtg cctctgtctc
17881 cccatgctga ttccctgtcc cctccctttt caagcccagt tctctccagc agtttagagg
17941 acattgtagg cctcagccca gctttgctcc caactcccgc ctctgcttac tgaccttcct
18001 gggcctgttc cactgtctgc ccttcacacc ttcccactcc accaggacca cgcaggcctc
18061 ctttggttac cgactctcac aaggttttcc aacacccagt ttagtttctc tttcaaattg
18121 gatcttcttt ggccacgtgt atcccacact gggggctctg gagagaggca gtgcacttcg
18181 gtaggatcct aatacccaga gccctgggtc gtagataggg ccgttcttga gcccgtctgt
18241 gcgtcctcct ccccacaccc ttctctccca ggaactgcac acgacgagta cttttccatt
18301 cacatttcaa ccaggagatt catctattta catgagacta actccgctga gcctttatgg
18361 gggaagcatc ttttattttt gtgcctagag gtgtaatact gaatttcaaa ggctgtgctg
18421 gagcctggtc atgaggttgt tattccataa ttagactctg ttgtgcctct ggttctggat
18481 tgcacagtgg atttgaaatc aagtgaagac atttctgtag gtcgtgtagg caatcagagg
18541 ggctctttgt ggtctgagcc tctgtttcat cctaactcca agtggggatt aaataagata
18601 atacataggc atttagtgca atgcctagga cattgtttat tcaatatata aacttttatt
18661 aaagcttttg aatgcaaagg tttttaacat gcatggatgg ccttcaaggt ctccataatc
18721 tccttgataa aatcatgcat gtgtaaagca cttgcttctg ggcccatagg ctggacacag
18781 ctggcccctc cacagagggt gacatggctc ctcagccctg caaagattgg ctctgggtgc
18841 ccaggctgcc tgctggtggg gctggaattc ttttcatatt ttttcagggg atctttggac
18901 tagaatccta gatgtaggat ggctgggtag aagggtacat gcatacgtga ctcagccaga
18961 tattgccaca ttccccgcca aagattgtac cctttgtatt ctgagcatta gtatatgaca
19021 gctcctgttt acctgaagtc tcacctacag attgtgacca aacatggaga tttttgccaa
19081 tctgctaagc aagaagtagt ttttcatggt cctctttatt tcatcatatt atgagtgagg
19141 ctgaacattg tttacatgtt taaaagccat ttgtatttct ttgtgaatct gttattttta
19201 gaagctatat gttatgggta ttaaccattt aatacaggtt gcaaatatat tttccaagct
19261 tgtaattttc gtttttactt gctaatgaat tttgccatgt aagagggttt ttggtttgtt
19321 ttgagacagg gtctccctct gtcgcacagg tagagacagg gtttcatcat gttgccaggc
19381 tggtctcaaa ctcctgacct caggtgatcc gcccaccttg gcctcccaaa gtgctgggat
19441 tacagtggct cgatctcggc tcactgcaac ctctgcttcc caagctcaag tgattctctt
19501 gccttggcct tctgagtagc tgggactaca ggcatgcacc accatgcctg gctaattttt
19561 gtattttttt tagagacagg gtctccctgt gttgcccagg ctggtctcaa actcctgggc
19621 tcaaatgatt ctccctcctt ggcctcccaa agagttggaa gtacaggcgt gagccaccac
19681 gcccggctgt tttttttgtt tgttctattt tgtttcttaa ggtagtcaaa tctatcagtc
19741 tttttcctta ctgaatctga atttttttgt ttgtttgttt tagatggagt tttgctctgt
19801 cgcccaggct ggagtacagt ggtgccatct cggctccctg caacctctgc ctcctgggtt
19861 caagtgattc tcccacgtca gcctcctgag tagctgggat tacaggcacc cgccacgacg
19921 ccggctaatt tttgtatttt tagtagagac ggggtttcac catgttggcc aaggtggtct
19981 ctaactcctg acctcaggtg atccgcccac cttggccacc caaagtgctg ggattacagg
20041 cgtgagccac cgcatctggc ctggattttg aattaggaca gttttcctca ctcccaggtt
20101 gcgaaggagt tctctcgttt tccatttatc tctagttctt ccatggtttc attcgcccag
20161 tgactgcagc gccacctgtg gaaaggtcag ttttccacca ttcctgcctt gcagtacaca
20221 aaatttccat gtgtagttgg gcctgcctat gggtttttca ttctgttctg ttcatctgcc
20281 tgtgtattcg tgggttagtt ctccccaccc acaaaccaaa ctgttctttt tcagagtttt
20341 cctggctacc ttgacttatt cttacaaatg agctttattt ttattttttt ttgagatgga
20401 gttttgctct tgttgcccag gctggagtgc agtggtgcga tcctggctag tgagatcact
20461 tgaacctctg cctcccgggt tcaagcgatt ctcctgcctc agcctcctga gtagctggga
20521 ttacagacgt gcgccaccac acccggctaa ttttttgtct ttttagtaga gacagggttt

20581 caccatgttg gccaggtctc aaactctcga cctcaggtga tccacctacc tcggcctccc
20641 aaagtgctgg gattacaggc ataaaccacc gtgcccggcc tacaaatgag ctttacaaga
20701 agagtatcta acgacaaagg caaatgtcca tgagatagtg ttagaaatag caggcaacag
20761 acccacacac tatataccta gaaatatttt tttctttaca gaaaaacatt gaaagggaaa
20821 acatcaagat gtttgtgtgg tgctcacttg atagccgggt taagggtagc gttatcctat
20881 tattggtgaa aacccggagg gaataaactt gaccctccca agtcttcctc ctttgtcctc
20941 accttagtcc tgtgccactc tggagatctt gtcacccctc ttggcacaga cagagccatt
21001 tcaggcacct ctctgcaagg gagggagtgc attacagcca ggtggagcct gggtgcaccg
21061 cttctgccca gcgggggagg aaggcaggtg gggttcaagc ctgtctgatt ttgtacacca
21121 ttgttttgct ctcctgcccc catggggggg ggggctttcc tcctctccca ggtgacagtt
21181 cataagaggg aagggcgaca agctcttatt ttggcccagc actgtccagg agaactttcg
21241 gtgatggtgg aaatggtctc tatctgtgct gtccaatata ggacccacct gtgctgatca
21301 taactcccaa aacaggatcc ccatcaccat aatcctaaat gttgaaatcc tgaaagatca
21361 aaatccctga agtctgaaat cctaaaaatc acaatctcaa aagtccaaat ttaagaagac
21421 ttaattgtac ctaatttcaa atctgtcact tatggtttgg ggattcaatt agacatcagg
21481 gattttagac ttcagggatt ttgatcttta gggatttcaa cattcggtgt tatggccttt
21541 gggactgtgt cttttgggat tatggccgaa acccagtagc cactaacccc atgtgactat
21601 caagcacttt aaatgtaggt ggtgtgagta atgaaccgag tttaaaaatt catttacttt
21661 taattaaata gccactgtgg ctagtggcta ctctactgga cagtgcggtt ttcactaatt
21721 cacaacccag ccctgcccct tccaatccag aactggaagg aaagagcctg cactggcaaa
21781 cacgctgagc agccaggtga ggtgacagca ggcctagccg gggggggcca gctctgaagc
21841 caggacctgc tcaacgaagg cccacaggag tcctggggga gaaatcagcc tgaagcagct
21901 tcccacccca gtggccatca caatggcagc cagcccagcc tcacaatgtc acaataccac
21961 aggcaggagg ccgggcaggc aaagtggttt cctccagggc aggactcttt gtctgcagac
22021 ctacttacag ccaccgagcg tcagaaggcc catctcctgg ggaatggaaa agtgcccagc
22081 cgctgggagg cctcaagctg gagagctacg gggaagcagg ggcaggtgtg gggctgggag
22141 aagctactaa gccccacacc ccaggtgctc tggggcctcg cagcccagct gcagggagat
22201 ggaacactgt gagcagctgt tcttcttcct ggccatttgg ttcatctact tcttttctga
22261 tgccaacctc tcttccacct actcaggggc tggaagggcg actggagagc caagcccctg
22321 cgagtgagga cagagaatgg ccaaaggcag agccacaggg tagacctttg caccttggga
22381 ggagagattt ggagaatggt gctgcctggg cccctttgcc cccaccaaac cgcaggggta
22441 ccatggccct gacctgcgtt cagagggccg agtcaatgtc acaggaaggg gaagagacat
22501 gtgcccacag accctgtggt catggagggg gtggaagatc tctggagggc agcagggcca
22561 cagcaaggct ctgaggtcct gagtcttctc cgtccgtgga cttaaactct gcgtgtttac
22621 agctgttctg tgcctgggtc caggctctag tgagaaataa actgccttca gagccaggtg
22681 acagcaaaag aaattttatt cccattttct aggggacaga aacagagccc agtggtgcct
22741 agcatgtttc tggcaagggt agtgaaaaga ccacttcctt ttgggggaag tctttcaatt
22801 aagaaaaaca cacgcaactc ctgggggtgg ctccacctgt acccaggaca gggcctgcca
22861 ctccaggggc tcccaggctg gattttccac cagagcagtc ttgggggcag gagcctgtta
22921 gaaatgcaga tccccaggga cctgcagcgg tctgggcagt acctccaaca cagctgctta
22981 aggggaggca gtccggccct catctgggag cgccgtgctg acgctgactg cacgctaggg
23041 ctctcggggt tgggtggaca gggtttctcc gagagatggg ctcccagcag ccagagcaca
23101 ctgtgggtgt caagctgctg gtgggaagca ggctgttccc tgagaccatg acagggtgaa
23161 ggtgggaatc accagccccc acactcacca agaaatgctt ttgctacatg ctggggcttt
23221 tttctttttt tgagagggag tctcactccg tcgcccaggc tggagtacag tggtgagatc
23281 tcggctcact gcaacctctg cctccccggt tcaagcgatt ctcatgcctc agcctcctga
23341 gtagctggca ttacaggcac acaccaccat gcccagctaa tttttgtatt tttagtagag
23401 atggggtttt gccatgttgg ccaggctggt ctcgaactct tgacctcaag tgatccaccc
23461 accttggcct cccaaagtgc tgggataata ggcatgagcc accatgcccg gccatgctgg
23521 ggcttttcta agaacacaag cacaccggag ggtaagacaa gattctggag aggcacagac
23581 ccagaaagcc aggggcacac aggagcctcg ccagcgaggg cggcacaggc catccatgcg
23641 gaccctcaaa tctcatgtga aggctgcatc aaggtcagtg ccaagaggaa gcagagcaaa
23701 gcatgtgacc acctctctga agccatcttt gtggacaaat gatgtaggaa ctcaaaaaaa
23761 tgcaggcttg gctaggcgaa gtggctcatg cctgcaatcc aagcactttg ggaggatcgc
23821 ttgagcccag gagtttgaga ccagtctgga caacatagca agaccttgtc tctacaaaaa
23881 ataaaaaaat tagctgggca tggtggcaca tgcctatagt tcctgctacc tgggaggctg
23941 aggtagaagg atcccttaag cccaggagtt cgaggctgca atgagttatg attgcaccac
24001 tgccctcaag cttgggcaac agagaccaac cctgtctcaa ttaaaaaaaa aaaaaaaaaa
24061 gcaactaacc ccaaacccag atctctaaag tattgggtgt ggactagagc tctggacggc
24121 ctaaaggaaa ggaatgtgcc ggttcacagg gacccgcggc taagctcaag ggtaaaatac
24181 agctttacaa agcatcttta ggctgttcct tcccaaacgt gcttagaagg gaacaggaa
24241 aggcgggtgt gttttctcac tgaggttctt ctagtggctg gaatctgata gagtaccaag
24301 ttgtagggat atggatatat tttccctttg gcactccata aagctaaatg ttgggctgaa

24361 aaaaggatgc agcctataaa caagtatttt tcctgaaacc aactgcatga ggaaacgctg
24421 cgctccccct cagggagcag tttctgaagc cagctgagca cagctggcac tggccagagg
24481 gagccctcca ccctcccacc acgtatgccc acctgcaaac ctgggttctg agtccccatg
24541 caggggacag acctgaaaat tccagtttgt gtcctttcag gtcatcgaca ggaatgacag
24601 cctggcaagc tgcagtgact gcacacagct accctgtgag ctccacttgt gtgggtgcag
24661 gtgggcgaca ggagtgtgtg acacagacag gcactccacc aggaggaaac ccacagcaga
24721 cgtcaaccat cgctttatta aggctgcgag tcggggggct gagtcatgca ctccacagac
24781 acccccactg ctcccaaggt ccacttttgg atgaccctga aggcagagac tcctgagatc
24841 tgggccacaa tctagggtga gccacccaca gtgccctgct ggacaggggg gtatgcggac
24901 tgcacggggg ggccctcagc aggggtcttc ctgcctaggg tggggctggc tccagtgggt
24961 cctgggctca ggcagggggg gtggcaggga ggcagggaca tccccccgcc ctctggccta
25021 tggctttgtt gccctattgc caccagcgca gaagcaatgt gctataccgt gaggtgatga
25081 agaagagccc cgggagggag caggcagctc tgtgcctggg gcctggccag acctcagggg
25141 tgctgtggcc ctgctcctgt tccccctcag ctcctcccag caatgggtct cctccagtgg
25201 aggtcagtca ctcagaagtg gacccgcagc acgtcttggc tagcaaccgg ccgctggcag
25261 gctgtgcacg tcatgggcag ggagcgttgc ttctcaccca ggcagggtcg gcacaggagg
25321 tggccgcagg gcagctggta caccggctcc tttttgaagt agggagaaaa tactcttttg
25381 caggaggcac attcggggcc caggatgctc ccaggctgct ctggtaaatc aggaaggaaa
25441 acaggccagg gttaggaaag ctgctccatg gtccaggctg ctctgagggg cagagccttc
25501 ccaccgtgct gctgcagcat ctggcttcat ccctcccgag tccatcccag tctgatcagg
25561 taggggagtg gaagcgggag agggagcctg ggaacccggg aggcctcttc tctatcatct
25621 ttgaccaaat ctcagtgcct ctacgaatgc ttgagaagag ctggcttctg agggcagcag
25681 gcaggactgg gcccttcctc ctggtctccc agcaaggttt actttcccct gcgataggtg
25741 gccaaggctg gagcaaggca cagctcactc tgacaagata aactgcaaga aatgtgagag
25801 gcgacaagag aaggaaaagg caggtcagtg ggtggctcaa agcctgtaat cccagcattt
25861 tgggaggttg agacaggagg atcacttgaa gccaggagtt cgagaccagc ctgggcaaca
25921 tagtgagata ctgtctctac aaaaaagaaa aaaaaaaaaa aaggaaaagc agcacatggt
25981 ccagtgcacc aaggttgggg aagtgtgaga agggaggtca gcagagacaa ggctgaggcc
26041 tgcagtgcac ctgccactgg ggcctagctc ctgctccctt cagccctgca gcctgcagcc
26101 ccctcactgc tactggcagc cctgctcctg actcctcagt ccccctcctg tgctgccctg
26161 gccctctcgg caggctctcc cttcttccgc aactccttca gggcatcctt gaatgagggt
26221 cacagtgagc agctctggcc atgccccacc aggctgcacc cacggctctt cctttcctca
26281 tggcccctct ggagagtgac aggggatggg cttgcctgtc ctccttgtgg tccatgtctc
26341 cattcctagc cactactctc tggaggaccc caaaggaacc tcaaaccaat ttgcttcaga
26401 attgtttctt aacccccctt ctctctgtcc atttcatcac cataatccag gccgctcgac
26461 acatctcttc ctaactattc acctgctcca ggcttgtctt ctcaatctgt cctcaaagct
26521 gcctccaatg acccacctat gtaacttgac caccccataa ctctgctttc tacctcttag
26581 gagcttccca actttcggga cagtcaagac tccctgcacg gcccacccag cctcctcacc
26641 tctcttcaca ccagtctcaa cttttagagt ccagcagctt ccaatcactt ctttgcacac
26701 tctgcgctgc tggcctcctc actttgtgct tgctgttccc tctgcctaga atgcccttat
26761 ctggagacct ggcttagacc acacccattc catgacgcca ccccggattg cccctttcct
26821 gtgctttcag agcccaccag cccttctcag cctccaaggt aggatgccta cttcctcccc
26881 aggctttgca ggtcctgagg gtcaggacta tgtccatgtt tctcatccgg cctcaggacc
26941 tggcgcagca cctcacaggg caagtgctcc atccacatgt gatgaatgga cctgccatct
27001 acactcgggc accaggcacc aaagaaggga gggcaggggt gccctacacg ggagcaggga
27061 actgaagtgt tcctgcctcc gcatccctga gaactgtagc caggaaagag tggcctggct
27121 gcgggtgttt tctttggccc aaacattaag caacataaaa gtaccgggtg cctctcctgt
27181 gcacacaagc caaaaattcc ccagtgagtg gaaagttgaa cctctacagg gaagggaagg
27241 agggaggaat gaaggtgggg agggaggggg gaaagaaggg aggggaggaa gcaggaggaa
27301 agagccactg actgcttttc agtggtgccc ctttcatgca ggctctgcca gaggcttcag
27361 agatctgcct gacccacagt gcatgatgag aatttatttt ccatcagttt taaaaagcac
27421 acctggcaca acaccacaga agcatgcaag ggccccaatt gcccccagca ctgaccagaa
27481 gtcccattaa aagatctcag aggcccagag ccccaggggc agtttcagtg acagaaaaaa
27541 aaaaaaaaaa atcatcaagg aaaagacaat tattgtgccc tgggcaggat ggaaacccaa
27601 gtatataact gcaaactgag gtgacagaga ggataaaaaa gagggcttgc tttgaaaagg
27661 gtgggctaac atggcctcct gtaggaagag ctgtgagaat aaaaaggggc cagccacaca
27721 gaatgaaagg agagcaagca caaagaacag cctatccgtt aaagaaaaag acacagtggt
27781 cgggcacggt ggctcacacc tgtaatccca gtgtaatccc agcactctgg gaggccgagg
27841 tgggtggatc acctgaggtc aggagttcga gaccagcctg accaacatag cgaaaccccg
27901 tttctaacta aaaatacaaa aattagccgg gcgtggtggc gggcgcctgt agccccagct
27961 actcagaagg ctgagacagg aggattgctt gaaccaggga gaaagaggtt gcagtgagcc
28021 aggattgtgc cactgcactc cagcctgggc gacagagcga gactccatct caaaaagaaa
28081 agaaaaagac acagtgaagc tgggtgcaga ggctcacacc tgtaatccca gcactttggg

28141 aagctgaggc aggtagatca cctgaggtca ggagttcgag accagcctgg ccaacatggc
28201 gaaacgctgt ctctactaaa tatacaaaaa aaaaaaaaaa aaattagctg ggcttggtgg
28261 cgtttgcctg taatcccagc tactagtggg ggctgagaca ggaagattgc ttgaacccag
28321 gaagtggagg ttgcagtgag ccgagattgc gccattgtac tctagcccgg gcaacagagc
28381 gaaacagagc aagactctgt ctcaaaaaaa aaaaaaaaaa aaagacacag tgagagtggg
28441 cttcagataa gactgcagag gaggagacat gggggctcca gcaggcagga cctcagaggg
28501 agtaggagtt gaggttttat cctaagttca ggggatgcca tggtaggatt tgaagaagtg
28561 gagcatggtg atgggctgac atgataaaaa gatccaactg ctatgagtat gatgagccac
28621 agtaaggcga ggatgggggg ataccagcag gggctactgc aggagtctgg gagtgaggca
28681 gtgggagctg cagccagggg aggggccaca gaaggagagc aggggactaa cctgaggtca
28741 ccttggggga ggatggacag gacctgataa tgaacagaat gtcaggatgg ggacactgaa
28801 ggaactcgag aatgacttct gtgttagaag cctcaccaca agacagaaga tgctgccctt
28861 acccaggtag gatgatgggg gcagtgaggg ggaaaagctc tggagggcag atgtgagcag
28921 tacagagtct gaatgtttcc tttagagagg atgctgagcc tggccatgat ggctcatgcc
28981 tgtattccca gcactttggg aggccgaggc agatggatca cctgaggtca ggagttcaaa
29041 accagcctgg ccaacatggt gaaaccccat ctctactaaa aatacaaaaa ttagctgggc
29101 gtggtggcgg gtgcctgtaa tcccagctac ttgggaggct gaggcaggag aatcacttga
29161 acctaggagg cggaagttgc agtgagctga aatcgcgcca tcgcactcca gctagggcaa
29221 caggggcaag actccatctt aaaaaaagaa aaaagagaga ggatgttgag tgctgtcagg
29281 gcggcaggat gagttccaag gagaagcagg agctacaggg gaacatggac agttgccgga
29341 gatgtgagtt ttcacttggg taacagagaa tattgagatt agagagtcca gggccagagc
29401 tggtagagaa ggcaattggc aatggaaact gaagagggac caccgaagat aggagaaaag
29461 aagcaaaaca gcctaaaagc caagagagaa ggcgtttcgc acagcaggag ctccatctcg
29521 taaaaaatgc ctcccgttgc aatcagtttc tgttgctttg tatcctaata gattctggca
29581 tacaatgggg gtcaaggttc attttaggca aaattggccc ataagctgcc actgctcttt
29641 ctaagacact gacggaagtg ttaatctgaa gggatgagga cgacaaaccc aaccaaacct
29701 ctcttcacat ttacataaag tgccccggat gtcagactgc agactggcta gatgagaaaa
29761 aaataacaaa aaaggccagg cgcggtggct cactcctgta atctcagcgc tttgcgaggc
29821 cgaggcgggc ggatcacgag gtcgggagtt cgagaccagc ctggcaacat ggtgaaaccc
29881 cgtctctact aaaaatacaa aaaattagcc gggcatggtg gcgggcgcct gtaatcccag
29941 ctactcagga ggctgaggca ggagaatagc ttgaatccag gaggcggagg ttgcagtgag
30001 ctgagactgt gccactgcac tccagcctgg gcaacagggc gagatgcagt ctcaaataaa
30061 taaataaata acaaacaaaa aaaagagaaa caaaaatcct caggagaaaa taattaacat
30121 tagatctcac aaggaaacac atcagagggt ggccaagccc tacatccaca gaattactaa
30181 ggattgacta aagaaagcct ttcttgtggg gagtttaagt tcataggcag cccctcaact
30241 acccctggaa aaggcaagag aacaggttct ctcccagggt gggtgttgga actgacgggg
30301 aggctggcca ggcaggtcca cagagccccc agggactcgg cccagcatcc agagacagaa
30361 aacagaccac ccattccaag gctccaagga gatggcgggg cccatgttac ccgagccggt
30421 gccaggcctc caggaagtgt tgctccctct tgtgccaagg tgctggagct gtcccctggt
30481 cagccgtgcc gtgaaggagg gcatagagcc aagggtggat gccaaggcaa tttccaagct
30541 ttgtgacagc ttctgctcgt gggacagtgg acctgtcgag agatgcgcac agcacaacat
30601 ccagagacag gctccgcaaa agagaacagc ctcaagtcct tcgagggctg taaaaacggc
30661 acctccaatc tcacacatca atgtaatgaa actccatcct aatgtctact ggcggaccat
30721 gacagaacac cgggaggtaa agccctagga acaccggcag ttcctgccca tcccaccggc
30781 ttcaggcttt tctcctcctg gagaaactgc tgggcagaca tgagccacct cgtggctcaa
30841 ctgcagaact gcacgctcct tttttttttt tttttttttt ttgagatgga gccttggtct
30901 gtcgcccagg ctgagaagga gtgcaggggt gccatctcag ctcactgcaa ccttcgcctc
30961 ctggattcaa gcgattctcc tgtctcagcc tcccaagtag ctgggactac aggtgtgcgc
31021 caccacgcct ggctaatttt ttgtatttta gtagagacgg ggtttcacca tgttggctag
31081 gagatctcct gacctcgtga tccacctgcc tcagcctccc aaagtgccgg gattacaggc
31141 gtgagccaca gcgcctggcc tcctttctaa aggcctcctt gctggccaag aaaataactg
31201 gggcaagcaa acgccagctt tggactgctg ctccctgccc cccattccct ccccagcggc
31261 cttagtcttc tctttcccta actccaccct tctggggacc ccaccacccc ccagaggggt
31321 tcatctgcat tttctaggtg ttcatgtcct ctctctcctg ttaagtaaaa caaagcagtt
31381 tttaccgaat agaactaaga ccccacgttg accacgccac aatttagggg acctttagtg
31441 taggatcaac agaaagccaa gccatggaat gagggtgaag gccaaagggg tagggggaca
31501 gctgcccaag aaagacccca gggacctgtg tggcagaaaa atttgcaatg ctgtctcaaa
31561 gaggagaatg gaaacttttg tccactgttt gcaggacaca ggacatcagc tcagggcctt
31621 gaaggagccc tagtcagcgt ggtcctgagt caccaagtct gtcaaggcca acaggactga
31681 gacctccaca aactgacgct gaagtctaag gacctttttc cttgttccac cccactgcgc
31741 ctcagatgtt tccaggagtt tctgaaggtc atgttaacta gaatttactc ttaaaaccaa
31801 agacacagaa ataaacgtta agcatagtat ttgcatagtc ttttagaaga acataggaaa
31861 ctaaaagaat gatcaaccta aacagaataa tttccataag gaaaaagaaa aggttgccag

31921 gccaggcaca gtggctcaca cctgtaatcc cagcactttg ggaggctgag acaggcagat
31981 cgcctgaagt caggagttca agaccagcct ggtcaacatg gcgaaatcct gtctctactg
32041 aaaatacaaa aattagccag gcgtagtagt gcgtgcctgt aatcccagct actcgggaag
32101 ctgaggcagg agattcgctt gaactcagga ggcagaggtt gcagtgagcc ataatcacac
32161 tactgcactc cagcctgggt gacagagcga gactccatct caaaaaaaaa aaggaaacgt
32221 gtcaaagagc taccctccac aagcaccaag cactgacaat cacaaggact tctactagac
32281 aggcagagtg ggtattccaa tactattcaa ttgttccaca gcacagaaaa gaaaaaattc
32341 taaattcttt ctatggaaca aaaaaatcat caatgacacc tgaccaaaga tggcacacac
32401 acacacacac acacacacac acacacacac acactacagt ccaaccccac taatgaatac
32461 aaaaatccta acactagcaa acagaaccat taaaatctat taacaggggc caggcgctgt
32521 agctcatgcc tgtaatccca gagttttggg aggccaaggc gggtggattg cctgagctca
32581 ggagtttgtg accagcctgg gcaacatggt gaaacaccgt ctctaataaa atacgaaaaa
32641 ttagccgggt gtggcggcat gtgcctgtag tcccagctac tcaggaggca gaagcaggag
32701 aattgctaga atccaggagg cagaggttgc agtgagccaa gatcgcacaa gtgcactcca
32761 gcccgggcga cagagcgaga ctccgtcact atatagataa ataaataaat aacagggctg
32821 ggcgtggtgg ctcacacttg taatcccagc actttgggag gctgaggcag gcagatcacc
32881 tgaggtcagg agttcaagac cagcctggcc aacatggcga aaccccatct ctactacaaa
32941 tacaaaaatt agccaggcat ggtggcgagc gcctgtaatc tcagctaatc aggaggctga
33001 ggcaggaaaa tcacttgaat ccgggaggcg gaggttgcag tgagcagaga ttgcgacact
33061 gcactccagc cagggcgaca gaatgagact ctgtctcaaa aaataaaaat aaaaataaaa
33121 aaaattagcc agtatggtgc tgtgcgcctg tagtcttggc taccgaggca ggagaatcac
33181 ttaaatccca gcagcagagg tcgcagtgag cagagattgc gtcactgctg cactccagcc
33241 tgggcgacag agggagactg tttccaaaaa aacaaaacaa aaagaaacca ttaacaggcc
33301 gggcactatc gctcatgcct gtaatcccag cactttggga ggctgaggca ggtagataac
33361 ctgaggtcag gagttcaaga tcagcttggc caacatgaca aaacccagtc tatactaaaa
33421 atacaaaaat tagctgggca cggttgtggg cacctgtaat cccaactact ctggaggctg
33481 aggcaggaga attgcttaga ccaggaggca gaggttgcag tgagccaaga ttgtgctatt
33541 gcactccagc ctgggtgaca gagtgagact ctgcctcata aaaatacaaa aatcagccag
33601 gtgtagtggc tcacacctgt aatcccagca ctttgggagg ccgaggtggg tggatcatga
33661 ggtcaggaga tcaagaccat cctggctaac acagtgaaac cccgtctcta ctaaaagtat
33721 taaaaaaatt agccaggtgt gctggcgggc gcctgtagtc ccagctactc aggaggctga
33781 ggcaggagaa tggcgtgaac cagggaggcg gagctggcag tgagccaaga tcgcgccact
33841 gcactccagc ctgggcgaca gagcgagact ccgtctcaaa aaaaaagaaa aaaaatcagc
33901 caggcgtgtt ggcacgcacc tatagtccca gctattccag aggctgaggc atgagaattg
33961 cttgaacccg ggaggcagag attgaagtga gccgagatca caccactgca ctctaccctg
34021 agtgacacag tgagactcca tctcaaaaaa aaaggaagga aggaaggaat tatttaggtt
34081 gaattacaca gaagtttgtc actgatctgt gttctcgaac taagaaatat gaagatggag
34141 gctaagaaat catttctctt aatattataa ataagcaatt aacaaatacc ttgggaaaaa
34201 taagaataca atagaagtga ggagtgtgac tttcaagatt acattctaaa agatggattt
34261 ccctcgtctg tgcactctct ctggaacgca cagctcccac acagtacact gccctatgga
34321 gaggcccaca aggcaaggaa ccgagggcag ccttgggcca acagccaacc agcaagtgag
34381 gcctcagtcc aacaatgcat caacgagctg aatcttgcaa caatgatgtg ggtgagacta
34441 gaagtaaatg cttccccagt tgatccttga gatgaccaca gcctcagcca ccagtttgat
34501 ggcagacttg gaagaccctg aagcagagaa tccagccaag ttacgctggg atttgtgatc
34561 cacagaagtt atgagacaat gaatgtttgt tgctgtaagc cagtaagttt tggggtaatt
34621 tgttacctag caatagataa ccaatacttc tttaacatga taaaaacatc tatctcaggc
34681 caggcgcggt ggcttatacc tgtaatccta gcactctggg aggctgaggt gggcgaatca
34741 cctgaggtca ggagttcgag accaacctgg ccaacatggt gaaaacctgt ctttactaaa
34801 aatacaaaaa attagccggt gtggtggtac atgcctgtaa tcccagctac ttgggaggct
34861 gaggcaggag aattgcttga agctgcgagg cggaggctgc agtgagacga gattgcgcca
34921 ttgcactcca gcctgggtaa cagagtgagg ctccatctca acaacaacaa aaaaacatct
34981 atctcagcct aagatccaac atccagcttg ccctgaggta ttcacactga acacattcag
35041 ctgcagaggt cacccagtga accagggctg ctcacctcag gagagagcag catcaccggc
35101 cctagaaatc actggcaatg ccccagggaa gaggcaggaa gccaggatcc ttaaatctca
35161 tactcaagaa tgttgcaggc caggcacagt ggcttgcaga tcccagcact ttaggaagcc
35221 gaggcaggtg gatcacgagg ttaacaggtt gagaccatcc cgggcaccat ggtgaaaccc
35281 cttctctact aaaaatacaa aaattagctg ggcgtggtgg cgcgcgtctg taatcccagc
35341 tactccagag gctgaggcag gagaatcact tgaacctggg aggcggaggt tgcagtgagc
35401 caatatcacg ccactgcact ccagcctggt gacagagcga gactccatct caaaaaaaaa
35461 aaaaaaaaca gaagtcgagt gcagtggctt acatctgtaa tcccggcact ttgggaggcc
35521 aaggtgggtg gatcacaagg tcaggagatt gagaccatcc tagctaacaa ggtgaaaccc
35581 cgtctttact aaaaatacaa aaacttagcc aggcgtggtg gcgggcgcct aatcccagct
35641 actcgggagg ctgaggcagg agaatggtgt gaacccggaa ggcagagctt gcagtgagcc

35701 gagatcatgc caccgcactc cagcctgggc gacacagcga gactccatct caaaaaaaaa
35761 aaaaaaagat tgttgcaatt ttacctggtg gatcctgcca aatgcaataa agaagaaaaa
35821 cagaaaaaaa cacaaagaga atattgcaat ttgtccacac atttactgag tgcaccctct
35881 tcagtgtggc ttttctcttc agtaaccctc tcctgaaagg aaggcccaca gacctagtcc
35941 tttttccttt tctgtgtcct catgccacat ggtgaggggg gcgcacagag aacctccatg
36001 caatatatcc ttgtacctct gtgacggtta gaaataacac ccagaggagc ccactgtcac
36061 atgagtaaca caaagaaggc accagaagag attctctgct ttgagggact ggtatctttc
36121 ctcccagcag ctctgaccag aggcttcaag cccaggaaaa agtgttgacc aggccagggc
36181 acataaaggg accccaagga aggcaacagt gcccatggga ccatcaccaa cagaggagcg
36241 aaacacgcag actgaagcct cggaacagat gagaatagac gagaacacat ggaacccctc
36301 caaggctcca ggttgtccct ggtctgaaga caaagctgag gggccccagg atgggaaacg
36361 ggaaggaggc agcacaggca cagcctcggg aatgtaagct ggccaagcac aaagcaaagg
36421 gcagcagagc tgagagctcc tgggaaggag atggatgagc ctggctgcgg acacaggatg
36481 ctgaattacc tgtcgagcag tccatatgtg tcaggctggg ctcattggtg gctttcattt
36541 tcttagcagt gtgctctgag gtagtgggta agaccaaagg gcttgtggca gaaaaacagg
36601 aagcatttac accaaagtta ctgtctggga catgttcagc ctgctctatc ttccttttct
36661 gagagggcag aacaatggaa gaagggatca ctgccaatgc cgtctgtgct ctcccaagca
36721 ggtggcagcc agggatggag tgctggagca ggaaatggtc aatccgggcc ttgagggagg
36781 ggtgaggcag gggctgagag tgcggagtaa aagctacccc cgtgaaaggg tcactggcca
36841 ctcggcccca tgtggcttca ctgcggttac acttctccag tgtgctctgg tcgatgacct
36901 tgcctgaggg cagcagcatg ggacaaggca tgatctccag ggtgatggga tccaggaact
36961 cctcaggcac atcctgaatg atctcggcca gcttctgcag gctggaggga gcctgctggc
37021 tctcaggctg gtccccaggg tcacagtcac tttccatggg caaggctgga gcctgcagag
37081 ccacatcctg aggcaggttc tctgaggtga ccagcaggat gctgtctatc acttcctggg
37141 agcaggtctt ggccggctga ccccacactt ccaaccgctt gatacaaggg ataccgccgc
37201 ctgtcacatg ggtgatacag atccttaagt gggccacgtg gctaagggaa agagcccctt
37261 tattccagag ctcctgggcc acaacagcag gggaggggag tgtggcttcc atcgcgccaa
37321 aagggggcct ggccttgaag cccctgtggc taaacaccac ttggctctgg tttttcagta
37381 agactttgcc taccaaggtg aacgcctcct tgtctgggac agatggctca gctgggccca
37441 gggtccggca ctggggcgta ttccaagaca ctctgctaga tgaggcagat gtgtacattt
37501 ccaggccagt gacgttctga cctcccccag ctgtgaggtc tatgttgatc ctacagattt
37561 ccacattaaa gggaaatgaa actgtcacat agactggtgg cttaatgaaa tactctgtcc
37621 tgaaaccatg acttctcttt gtgagatctt cagagatgag attttctact tcgtaaccat
37681 cagctgatat ctagatttaa taaaaaacac aagatacaat gatccaaatg aagacaactt
37741 gtcacttgta agaaaactct taagcctgag gttcctcgtt ttaaaatttc ctctattaaa
37801 ctgaattgtc ccagatcagg gcacccaaga tcagatcaaa ggccaagggc aattgtccca
37861 gatcggggcc cccaagtatt gcccaacctt aaaaatatga gcaactgagc tgggcatgtt
37921 ggtacacacc tgtagtccca gctactcagg aggctgaggc aggaggatcc cattagccca
37981 ggagttagat aggcctgggc aacatagcaa gaccccatat cccttttccc tgccaaaaaa
38041 aaaagcaagc aacaaaatcc tgcccaatcc aaccaaaact cccttcaaaa cagtatctgg
38101 ggagagaagt gcaggggaga gaaagtattt ctacctttgc tatgactttg acgagactgg
38161 agattgatca tcaaagaaca aagagcacag tgaagaggac cctgcacaaa atgagtcaca
38221 gctcacctca ccatggcgct gtgtgattct agaggtgagc ctgccccatg cttacctaaa
38281 taccatctga tggaatctaa agtaacaaga gcaaaagtgc agtaagcctg aatcaaatcc
38341 acacacacaa ggcaggaaat gatgaaacac atgacaaaca tagcatatat ttatgtgtat
38401 atatttgttt tgtttacctt gttgcagtga attcttggtc tgaactgtgg gaggcaaaga
38461 tttattacca tctttgtggc tgagaggata tcttccaagc atcagaagca gccttaaata
38521 agaaataaaa ctatgtatta gaaaatgtaa aattcaattc taaactttca gaagttgatt
38581 cttaagagaa tcaaacagga tggcaacaaa aacttgaaca cacacactct cacggtccac
38641 actgactttc cttatatcct atacatgatg ttggggaaaa attagtcact gaaaaattat
38701 agagaagact tctatggcag gctcaacagg ttggcaaaga ctgccagttc tctggccaac
38761 agctgttcca aagacccaat ggaggcaagg cccatcagct gtgctggggg ttgccacagg
38821 aagggagtgg caaaagtatc tgagctcaga gaaaggacag agtgttcgga atggaaaggg
38881 tcataaaaaa taattatagt ggtggtaagc atccttgtca tttaatactt gtcaatgcaa
38941 ttatgttttc ttattaaaat gaaaacaatg agtcaaaaac atcattattg agacatagaa
39001 aatttattag aggccaggtg tggtggctca tgcctgtaat cccaacaatt tgggaggctg
39061 aggtggtagg actgcttgag cccagaagtt caagaccagc ctgggaaaca tagctggtct
39121 tgttgtagag accccatctc tacaacaaaa cgttgtaggg atcttgctat gtctgatatg
39181 cacctgtagt cacaactatt tgggaggctg aggtggatca cttgagctca ggagttcaag
39241 gctgcagtga gctatgatca caccactgca ttccagccta ggtgacagag tgagaccctg
39301 tctcaaagga aaaaagaaaa gcctctcccc tctcccctct cccctttctt cggtctccct
39361 ctgttgccga ggctggactg tactgccgtg atctcagctc gctgcaacct ccctgcctcg
39421 ggctcccgtg attctcctgc ctcggcctgc cgagtgcctg ccttgagtga tctgcccgcc

39481 ttggcctccc gaggtgctgg gattgcagac ggagtctcgt tcactcaatg ctcaatgttg
39541 cccaggctgg agtgcagtgg cgtgatctcg gctcgctaca acctccacct cccagccgcc
39601 tgccttgacc tcccaaagtg ctaagattac agcctctgcc cggccgccac cccatctagg
39661 aagtgaggag cctctctgcc tggccaccca ttgtctggga tgtgaggaga gcctctgccc
39721 agctgcccca tctgggatgt gaggagcacc tctgcccggc cgccctgtct gggaggtgag
39781 gagtgcctct gcccggccgc caccctgtct gggatgtgag gagcgtctct gcccggccgc
39841 cctgtctggg aggtgaggag cgcctctgcc cagccgccac cccgtctggg atgtgaggag
39901 cgtctctgcc tggctgcccc atctaggaag tgaggagcgc ctctgcctgg ctgccccttc
39961 tgagaagtga ggagcgcctc tgcccagccg ccccgtctgg gaggtgagga gtgcctctgc
40021 ctggccaccc atcatctggg aagcgaggag cgctgctgcc cggccacccc gtctgggaac
40081 tgaggagcgc ctctgcccgg ccgccccgtc tgagaagtga ggagcacctc tgcctggcag
40141 ccaccccatc tggtaagtga ggagcatctc tgcctggccg ccccatctgg gaagtgggga
40201 gtgcctctgc ccggccgccc cgtctggagg tgaggagcgc ctctgcctgg cagccacccc
40261 atctgggaag tgaggagcgc cactgcccgg ctgcaccatc tgagaagtga ggagtgcctc
40321 tgcctggcag ccaccccatc tgggaagtga ggagtgcctc tgcccggctg ccccgtctgg
40381 gaggtgagga gcgcctctgc ccagccgccc atcatctggg aagtgaggag cgcctctgcc
40441 cggctgcccc gtctgggagg tgaggagcgc ctctgcccag ctgccccgtc tgggaggtga
40501 ggagcgcctc tgcccggccg cccatcatct gggaagtgag gagcgcctct gcccggccgc
40561 cccgtctgag aagtgaggag tacctctgcc cggccgccca tcatctgaga agtgaggagt
40621 gcctctgccc ggccacccat cgtctgggag gtgaggagca cctctgcccg gccgcccatc
40681 gtctgggagg tgaggagcgc ctctgcccgg ccgcccatcg tctgggaggt gaggagtgcc
40741 tctgcccggc caccccgtct gagaagtgag gagcgcctct gcccggcagc cgccccgtct
40801 gggaagtgag gagtgcctct gcctggccgc ccatcatctg ggaggtgagg agcgcctctg
40861 cccggccacc ccgtctggga ggtgaggagc gcctctgccc agccgccacc ccgtctggga
40921 agtgaggagc gcctctgccc ggctgccccg tttgggatgt gaggagcgcc tctgcctggc
40981 cgccaccccg tctgggaagt gaggagcgcc tctgcccagc cgccccgtct gggaagtgag
41041 gagtgcctct gcctggccgc tgtgcaatct tccaagtgtg aagtgacagc ctttctgcag
41101 gtgtacccaa tagctccgaa gagacagcga acatcaagaa cgggccatga tgacaatggc
41161 ggttttgtcg aaaagaaaag ggaaatgtgg ggaaaagaaa gagagatcag attgttactg
41221 tgtctgtgta gaaagtagac ataggagact ccattttgtt ctgtactaag aaaaattctt
41281 ctgccttggg atgctgttaa tctataacct tacccccaac cccgtgctct ctgaatcatg
41341 tgctgtgtca actcagggtt aaatggatta agggcggtgc aagatgtgct ttgtcaaaca
41401 gatgcttgaa ggcagcatgc tcgttaagag tcatcaccac tccctaatct caagtaccca
41461 gggacacaaa cactgcggaa ggccgcaggg acctctgcct aggaaaacca aagacctttg
41521 ttcacgtgtt tatctgctga ccttctctcc actattatcc tatgaccctg ccacatcccc
41581 ctctccgaga aacacccaag aatgatcaat aaatactaaa aaaaaaaaaa gaaaagaaaa
41641 agaaaaaaaa aagaaaggaa ggacagaagg aagtaagttg ggcagggaca gaaagaaaga
41701 aaaagacaga gaaagagagg gagggaggga gtgaaggagg aagggaggga gggagagaag
41761 acagagaagg agggaagaga gagagggagg gagggaagaa aggaaggaaa agaaagaaag
41821 aaaatattag ggttggaaag caccttaaaa tgacatctag gcctggcacg gtggctcata
41881 cctgtaatcc cagcactttg ggagacagag gcaggcagat cacctgaggt caggagttca
41941 agagcagcct ggccaacatg gtgaaactcc atctctacta aaaatacaaa aagcagttgg
42001 gcatggtggc aggcacctgt aatcccagct actcgggagg ccaaggcagg agaatcactt
42061 gaacccggga ggtggagttt gcagtgagcc gagattgcgc tactgcactc cagcctgggc
42121 gacaagagca aaactccgtc tcaaaaaaaa aaaaaaaaaa aaaaaaaagg acatctaatg
42181 caagtagtca ttttaatggc caattccaat ggcttctggt aactctgtgg agaaggaggg
42241 aagccactga aaacacccct cgcagtggca gacctctcca gtggaactca actccttggt
42301 gaggccagcc agccatttat gcttttctcc ctgacagtcg tggccatgta aaattgttag
42361 cccgacaggg atagggtcac cctctgggcc ccttttgctg tcaaaatcag taggagccac
42421 ttgcaggagc caggcatact aagcgtcctg caacatctga gtggatagtt gctgcacagt
42481 gaagatctgt catgggcctt gtaaatgtct attatctcag cttagcccct aactcagttt
42541 tacatattaa cacaaagtat ttttaaacat ggttttcata tatgctaaat tttctaggag
42601 gggaacttcc atgtaagcca gaggaaggtc atatattgtt ttgttcagac ctttaccaag
42661 agatgcccac catttctgaa aatcacatca ccgagagcaa agctctccac ctcggtcagt
42721 ctgcatttgt agcagtctgc attccacatg cgctcctgcg tgatggtaaa agcttctgaa
42781 tacttcttta tgccttctag tatggtcaca cccaaacatt tatacacgag aataaatttt
42841 attatcatac ctaaaatttt ttcctttaaa gtactgttag ggcattctgt gtgttggtgg
42901 ggggagggtg tgtatgtgtg tgtggaccta cacacattag cgtgtgtaca taagtaggtt
42961 atactatttg gctttaagtt catcatgata gaagaaacat gaatctcatg ggtagaaag
43021 cccctggcct aaagcctcct gttctttgtg agcctgtgtt gaaactccta gtgcttccca
43081 ctttgaggca catacacctt tgctttgcca gagaatttta gaatctggcc tggcatccac
43141 atggactaaa gtttctacaa ttccttttct gaaagattat attaacaatg tctccaggct
43201 tctgatacct ttcgtgttct ccacgataag taaaaacaaa tgaagtatct gatgcattgt

43261 gtctacaaat atcctcagaa gccctatagt tacctctttt tggccaggac accagaactc
43321 atttagagca gcatttcccc cagcttccct gcccaactag catagtagct tcctgtcggt
43381 ttgttttaaa tgtaaaatta agccattcac tttaattgaa tatagaacca ttaaaataaa
43441 tatatagcca agccaatgaa ataaaacatt cctccttgga aaagcaagcc taggatgcca
43501 aaggaaagca gacaatatgc tactgacata gatgatgaca ctagagacag acatatgcag
43561 aacggtgctg gccccaggca gatggctgag gttgggcccc tgggacttga gagggttttc
43621 ttttcactgc tccaggcagt tccctcttcc cctcttggcc ctgacagaaa gccaagaaaa
43681 aacaaagccg ggcggagtgg ctcacaccta taatccgaac actttaggag gttaaggtga
43741 gaggatcact tgagcccagg agttcgagat cagcccgggc aacacagcaa gaccccgtct
43801 ctacaaaaaa ataaaaagat tagttaggtg tggtagcaca cacctctagt cccagctact
43861 tgggaggcgg aggcaagagg atcacttgaa gccaggagtg caaggttaca gtgagctatg
43921 actgtgctat tgtactccag cctgagtgac agtgagaccc tgtctcaaaa aataaagtaa
43981 aataaaataa gagttcttct agacttaaat gtggttatta tgctctatga tatagaaatg
44041 tggaaacctt gactgccctt tccaatatgt gttagatcag acctgaaatt aaggaaaatc
44101 ttggtatacc cttagctact tcttcccacc tctgtgtgga agcgagtcag ccttctgttc
44161 aagcatctca gctcatgagg cttctccacc cgcgtcaggg gctgtcaact accccagtca
44221 actacaaaag catatggacc taaccctgac cagacacaga ctacctgctc tccacagcac
44281 tcatcaaaat ataagttgag ggaacattta cagaagagtg ttcagtccag ccccgttaca
44341 tgctccttgg tgctggtcca agtagcttct cttgcctctt gtctggcact cctcgtggct
44401 gctgcagatc ttatcccaca ccactatctg acagatttct tagcagtttt gatcctcacc
44461 agttgcaaaa ataaatgtaa cacattgata aaaattcagt ggccgacttg tgacgattca
44521 taggacacca gagtagcagg acatatcagg tgataaccat aattttaatc tctaaaatat
44581 accactccat tgagctgacc ctcagaccag tgcaccggcc tatcctcatg ttcatcccag
44641 ccacaaccat caccaccctc accaccccct gcaaatactc ttcaccaagc tactcattgg
44701 cactgtgtga caaggaactt caataagaaa cagctcttta aagtcagtta ctgttttgtt
44761 tagtggacaa aatgatcatt gtctgttcta tatggtatcc tctcttggag acccataaga
44821 aaattttctc tttggtctca gtactagaag agttgtttat cgcaagcaat tcaatatttt
44881 atggtatgaa tatggatcac attccttctt gactctggct gttagaaagc tccgaataaa
44941 atctgtggcc catccctcaa atttctccct acctgaatta tttattttta tataatagag
45001 cataatatgt aaggtagctt aaaataattt tggaacaaag tggggtataa atatgctggg
45061 cataggggct cacgcctgtg atcccagtac tctgggagac caaggcagga ggattgcttg
45121 aggccagaaa tttgagacca gcctgggcaa caaagtgaga tgccatctct acaaaaaatt
45181 ttaaaaatat tagctgggca tggtggcatg tgtccgtagt cccagctact caggagtatg
45241 gcttgacccc aggagtttga ggctacagtg agctatgatc acaccactgt actccatcca
45301 gactgggtga cagagtgaga ccctgtctcc aaaaataaat aaataaataa ataaataaat
45361 aaataaataa ataaacaaac aaagagggtg gggggcataa ataattctcc ttttgggaga
45421 catttaggtt gtttatgcct tttttttttt tttttttaaa gacagggtct cactatgttg
45481 cccaggctgg agtgtagtgg ctattcacag gtgccattac agtgcaccac agcctcaaat
45541 tcgtgagctc aagcaatcct cctgcctgag tgccccaagt cctggaacta caggtatggg
45601 tcaccaaacc caacttgttt gcaacttttt actactacct taagatcaaa atcttaagta
45661 gatttttgtc attatttttt cctttagtac aattttcact tttttactca aggggtccta
45721 catgtttcta gttaaggttg ctatttatga aaggttcttc ttaacccatt aatttttcta
45781 actggtcatt cctatatagg aaagcttttc actttggtat ctttactata tgttcagctg
45841 tcttattgaa ctcttattga ttttaacaca ttttcagttg tttttgcttt tttttttttt
45901 acaatcctat tatctcaata atgagaattc ctttctcaaa atggtaactc ttatttctgt
45961 ctctcattta ctgcattagc cagaacttcc ggaattacac aaaacagtaa gacagttgcc
46021 atcctcgttt gtgcccatta gaaaatgcct ctttgggcca ggcgcagtgg ctcacacctg
46081 taatcccagc actttgggag gccgaggcgg gcagatcata aggtcaggag ttcaagacca
46141 gcctgggcaa tatggtgaaa ccctatctct atgaaaatac aaaaattatc caggcatggt
46201 ggcgtgagcc tgtagcccca gttactcggg aggctgaagc agaagaatcg cttgaaccta
46261 ggaagcagag gttgcagtga gccgagatca tgccactgca ttccagcctg ggcaacagag
46321 tgagactcca tctcaaaaaa aaaaaaagaa aatgcctctt tgaacataat gttgattgtt
46381 agattgaaat atcatcttta tgttaataaa gcattctaat tttcatattt tttgaagtca
46441 ggaatatgct ctgaattctg atttttctct atatattgtc ttttattatt tatcttatct
46501 gacagactaa tgggatacat taacactttt ttctaatttt aaaccatcct ttttattccc
46561 aggagagatt ctatttggtt gtagcatatt gttctttaaa tgtactgtac tactgaattt
46621 attttgctca tatttaattt tagatgggat attattgcac attcatggaa catttgcaag
46681 ccctgtgcta ggcactggag tatagagata aaaatgaaat ggcagtgaaa ctattctgta
46741 ttacactgta atggtagata catgacatta tacatttcaa aacctatata ttcaaaacct
46801 gtaattcaaa acctatagaa ttatacaaca caggctgggc gtggtggctc acgcctataa
46861 tcccagcact ttgggaggca aaggcgggca aagcacttga ggtcaggagt tcgagatcag
46921 cctggtcaac atgatgaaac cccatctcta ttaaagataa aaaattagcc gggcaaggtg
46981 gcgggcacct gtaatcccag ctactgggga ggctgaggca ggagaatcgc ttgaacctgg

47041 gaagcagagg ttgcactgag ccgagatcga gccactgcac tccagcctgg gcaatagagt
47101 gagacactgt ctcaaaaaaa aaaaaaaaaa aaaaactggg cgtggtggtt cacatctgta
47161 atcccagcac tttgggaggc caaggcgtga ggatcagttg aggccaggag ttcgagacca
47221 gccttgccaa catggtgaaa acccgatgct actaaaaata caaaaattag ctgggcatgg
47281 ttgcgcatgc ctgtaatccc agctactctg aaggctgagg caggagaatc acttgagccc
47341 aggaggcaga ggctgcagta agccgagatc acgccactgc actccagcct ggacaacaga
47401 gtgagaccct gtctcaaaac aaaacagaac aaaaacaaaa cacaaagaat tatacagcac
47461 aaagaatgaa ccttaaatta taaacttaat aataattaat caatattggc tcattagtta
47521 taagaaatgt accgcactaa gatgttaata gcattaataa tactcaattt ttaggtaaat
47581 ctaaactgct ctaaattata aggtgtatta ataataataa taacaaagaa atggctgggt
47641 gaaacgaggc gtgattggtc atgcctgtaa tcccagcact ttgggtggct gaggcggca
47701 gatcacttga ggtcaggagt tcgagaccag tctggctaac atggcgaaat cccgtctcta
47761 ctaaaaatac aaaaaattac ccgggtgtgg tggcaggtgc ctgtaatccc agctacttgg
47821 gagagtgaag caggagaact gctggaaccc tggagacgga ggttgcagtg agccaagata
47881 gcgccactgc actccagtct gggtgacaga gtgggactgt ctcaaaaaaa aaaaaaaaag
47941 aaatggctgg gtggggttgt gtacacctat aaattccagc actttgggag gccaaggcag
48001 ggggattgct tgaggtcaga agttcaaggc tagcctgggc aacatagcga gacccagtct
48061 ctacaaaaaa aaaaattaaa taattaataa taataaagaa tgattggggg aaaaagtgat
48121 tgaaaacaac tcaggactga agatcaaatt gaaatatgca aattgaaacg aatgagtgtg
48181 ttgtcacagg agaatggcag atctttttta aaaatatctt acaaatgata tgtttctgtg
48241 ggaaacagtg atgtccaaac agtaggattc cgtaagaacc tggttgctat caaagtcttt
48301 aataggtata atctcatcat ctatcttgtt ccaataccct ggacttttaa ggtcagatgc
48361 cttctgcctt aacagaaaaa aaataatggc tcaacatatg aaaatgcaaa cagcctagcc
48421 cagaaagacg gcctccctag tcctgctgag gaaatggcca tgtaacttga actcactgca
48481 aacactgtcc agtacttaca aagcatagcc actttctcca gatgtggcat ctaagtacaa
48541 gaaagacaag gcttgagtat aacttaatct aaaacctcag attgtggcta atgtgccctg
48601 ctttcagaga agtagcttgg agacagataa taatggcatt gtaagatggc aatacagttt
48661 ataagaaaaa aaaaagaata taaaatagta gacactcttg tacctaccac ctagatttag
48721 caaatgttaa cattttgcta tgtttgcttc tgatttcagt gttaaaagca atatttaaaa
48781 gataaaaaca aaacccagca cctatcctat ttcctctctc tttttgccaa ggggtaagct
48841 cttttttttt cttatttttt ttgggggggg gaaacaggct ctcactccca tgcccaggtt
48901 ggaaagcagt ggtgcaatct cggctcactg caacctcaac ttccagggct ctggtgatgg
48961 gtattctcct acctctgcct cccgagtatc tgggactaca ggcgcgcacc accacatccg
49021 gctaattttt gggggttttt tgggtagaga tggggtttca ccatgttgcc caggctcttc
49081 tcaaactcct gggctcaggc aatccaccca ccttggcctc ccaaagtgct gagattacag
49141 gtgtgagcca ccttgcccgg ctaaggtaag ctttatctaa tgttgtctgt cattcaaatc
49201 agtataggta tgcatatata tatataaact acatatagta gtcatgtatt ttaatatacc
49261 ttaatgataa tatattatgt gtatcatctt gcaacttgct tttccattaa gcattatatt
49321 aaggttttcc atattgaaat gtttacattt ggtgtattca ttttaacttc ctcatattta
49381 attgtatgac taaaacagtt tctccactgc cctattactt atgttgttta aaatttttta
49441 ctatgaaaaa aatgctgcaa taaacattct ctacaaatct tcttgtggga aaaataagaa
49501 atgaaatgaa cggccttctc tgtgagtcat gagacaataa agacttcttt ctaacagtga
49561 ttatataata gaatatatgc ttgaacccgg gaggtggagg ttgcagtgag ctaagttcgc
49621 accattgcac tccagcctgg gtaacagagt gagaccccat ctgaaaaaaa aaaaaaaaaa
49681 aaaggccagg cgtggtggct catgcctgta attccagcac tctgggaggc cagacaggcg
49741 gatcatctga ggtcaagagt tcaagaccag cctggccaat atggtgaaac cccatctcca
49801 ctaaaaatac aaaaattagc caggcatggt ggcgggcgcc tgtaatccca gctactcggg
49861 aggctgaggc aggagaatca caatcacttg aacccgggag gtggaggttg cacttggccg
49921 acaccgtgcc actgcactcc agcctgggca acaagagcga aactccgtct caaaaaaaaa
49981 aaaaatttat atacataata taaaggtaac agagcattct gcggatgaca cagtaaggaa
50041 agataaccta gatcaatgtt tttcaatttg tggtttttgg aatcaagtaa tgggttaaag
50101 aaaagaaata attagaagag aatacaaaat atgaaggtgc aaatagtatg gatatgtcat
50161 gaaattgttt cagttttgta caaatatatt taacaaatgc atgtatatca tctaatataa
50221 aatgcatttc ttattgtgac ttgataaaaa aaattttttt aaaaaaacgt tggtcaaaag
50281 ttttgaccaa aacattggtc aactcagggg aaaattcaca ggagtaaaca tgtgaactta
50341 gggtgactag gacacatgcc tgatctcatg gacaactctg aagagggaca gacactccat
50401 attaatgcta ccacagactg tagactacag gagccaagag gtgagaacat gaacaaaaga
50461 gaaggagccc tggggaaagg gatcaggaag tgtcaaggca cagaggaggg aagtgtgagg
50521 ctggttcagg agatgctgaa catgccactt agctatggag gtggagtagg agccacagct
50581 gggaaggcaa acggagccgg atgaggaagg ccttgaacgt catgccaaaa agtttagact
50641 acttcactta gtaggctgag gtagtgtttc ccaaactgca ggttgagaat taagaactta
50701 aattaccgag ccataactag tatttttaaa aattaagtag aatagaaaat gtcagcatgc
50761 accacataca agggtgagca ctgttttgtg aaagttttgt ttcagttaca tatatatgtg

50821 aacacacaaa cacacgcaat tattcatttg gggttttgat gtaaaagtta tttcttacta
50881 aaggttgtag ttaaaaaaaa aaaagttgaa agctactggt cagaagtaag tcatcggtga
50941 atcattatca gtatcagtag gctggttatg aagttctagt atagctatgc aagatgttac
51001 agccaggcac ggtggctcac acctttaatc ccaacacttt gggaggctga ggtgggtgga
51061 tcagagagag aaatgacact aatgaaaagt ctgctattaa cctcctagat gacaacagca
51121 attctgaacc acttagaggc aattcaccct tcaagaggtc aatttccctt ttctctggct
51181 gatgtgttcc ccactatctt ttctgtacta catgaggggc tcctggcaaa tggcttcctg
51241 aagacatcaa agatgagtac caaggtacta actacttagg aataaactgg taatcccatt
51301 tttccttcgg cattttcttc ctttagtttt tctcttttta ttcttttttt ttttttgagg
51361 gaggggcatt cttcaaaagg aggaaaatga actatgtata atgtattact gagcatctca
51421 caaggatttc aaagctactt tctaagagat tgtttatttt tattttttat tttttttgag
51481 acagagtctc tctctgtcac ctaggctgga gtgcagtggc acaatctcgg cttaatgaaa
51541 cctccacctc ccaggttcaa gcaattctcc cacctcagcc tcccgagtag ctgggattac
51601 aggcatgcgc catcacactc ggctaatttt ttgcattttt agtagaaatg ggatttcccc
51661 acgttggcca gagtggtctc gaactcctga cctcaagcga tccacccacc tcagcctccc
51721 aaagtgctgg gattacaggc gtgagccact gtgcccggcc ttaatattac tattattttg
51781 agatggagtc ttgttctgtt gcccaggctg gagtgcagtg gcatgatctc agctcactgc
51841 aagctccacc tcccaggttc acgccattct cctgcctcag cctcccgagt agctgggact
51901 ataggcgcct ggcaccatgc ccagctaatt ttttgtattt ttagtagaga cggggtttca
51961 ctgtgttagc caggattatc tcaatctcct tacctcgtga tccactcacc ttggcctccc
52021 aaagtgctgg gtttacaggc gtgagctact gcacccagtc cctgtttttc tgagacagtg
52081 tctcactctg atgcccgggc tggagtgcag tggggcaatc tcgactcact gcagccttca
52141 cctcccgggt tcaagcaatt tttgtgcctc agcctcccaa gtaggtagga ctacaggcat
52201 gacccatgcc tggctaattt tcgcattttc agtagagaca gggtttcacc atggtagcca
52261 ggatggtctc gaacttctga cctcaagtga tccacctgcc tcggcctccc aaagtgctgg
52321 gattacaggc gtgagccacc acacccaacc agaaattgtt tctttttaaa agatctaatt
52381 aaacttttaa agataattgt agactcatgt gtagttgtta aaaaacaata cagagagatc
52441 ccacatactc tttcaggctc actgcaacct ccgcctcctg ggttcaagtg attctccttg
52501 agtagctgag attacaggtg cccgccacca tgcctggcta ttttttgtat ttttagtaga
52561 gacggggttt caacatgttg gccaggctgg tcttgaactc ctgacctcag gtgatccacc
52621 cacctcagcc tcccaaagtg ttgggattac aagtgtgagc caccgtgcct ggctgtaaca
52681 tcttgcatag ctatactaga acttcataac cagcctactg atactggtaa tgattcaccg
52741 attttcagat ttcaccaggt ctgcatgcat tcatttgtgt gtgcctatct aattagttca
52801 gtgcaatttt actctatgtg caaagttggg taatcacaac cacagttgat acagaacact
52861 tttatcacaa gaatttcatg ttatccttta gaaagtcttt tatcttataa atccaaatac
52921 tctacttcca cagaagtctt gaaatttttc acccagatct aagccttggc tgaagactcc
52981 agttaagctc actgcccaca ctgtaagaaa caaacttgag tgacacattt agtcttggca
53041 ttcatgtcac ttaaatgata aatataacta tgcaggaaaa tgcaaaagta tatataggct
53101 gggcatggtg gctcacgcct gtaatcccgg cactttggga ggccaaagtg ggcagatcac
53161 ttgaggtcaa gagtttgaga taagcctggc caacatggtg aaatgctgtt tcaactaaaa
53221 acacaaaaat tagccagcca tggtggcgca tgcctgtagt cccagctact caggaggctg
53281 aggtgagaga atggcttgaa cctgggaggc ggaggttgca gtgagctggg atcacaccac
53341 tgcactccat ccagcctggg cgacagaggg agactgtctc aaaaaaaaaa aaaaaagtat
53401 tatgaaagta ttatgatcat acatttgtta aaaatctatg tctaggaata tgtatacata
53461 aatagttgga gaggtaatgg tgggcatgca gaaatgtaaa catccgacat actaggctgg
53521 tgggataagt tgttcttctt ttccttttat gtattgaaag ttgatgtatg cctgaggtct
53581 caggataaca gcggagaaga ctgagattta tgttcagagt actttttgca gctgccagta
53641 ctagggctca gaaagggaag tgcaagggaa gggccaagaa gggatccagg agttgagtgg
53701 ccaactgtct tggcataccc acagctaagg agttttccag aacatagtac ttttggtgct
53761 aaaacctggg caaacttgga agagttggtt gccctgtctc gaaggttgtt gtctgtgctt
53821 ccttggagct tgaagcaacc aacaaacctt ttgtttttgg tgttgaggtt cagcacctgg
53881 gggtagggaa gtgtggaagg ataatttaga agagaataag gtgaccatga cactttctga
53941 acacctcagc atccagcttc cttcagccta caaagttacc atagctgcgt tatacaatct
54001 gattttgagt ctcatagaga tgtaatgaaa agtaattctg gcagacacac atattttaaa
54061 gactagactc aaacaggtta cagaagggaa aaaaaagaag aagaaaagat tacctgtcaa
54121 gtaagagaaa agcatttcac ctcaaagagt gggtggaata tcaatgcact gagcaatttc
54181 ataaattggg cacttcagct tttaaagaag tgcactactt cttataattt ctaaattgag
54241 ttacctgtcc atccatgaaa aaagtttgac tcatgtgcct ttttcttaca actggttaaa
54301 atgaaagaca gttgtcccag cccatcccac acagttaccc accagtgaga ggtatgcaaa
54361 aaaaccagaa cacaggttag agccccaaag aagcctgcag ttttatacca cgtggagcca
54421 agtcaccctc taagggctta acatgtgcta ggcacaagta gactcagttt ttcctcttta
54481 tttttaattt ttatttattt atttatttat tgagacaaga gttttgctct tgttgcccag
54541 gctggagtgc aatggcgcga tctcggctca ctgcaacctc cccgtcgtgg gttcaagcga

54601 ttctcctgcc tcagcctcct gagtagctgg gattacaggc acccaccacc acacccaact
54661 aatttttttg tatttttagt agagacgagg tttcaccatg tttgccagcc tggtcttaaa
54721 ctcctgacag cctggtctta aactcctgac ctcaggtgat tcacccacct tggcctccca
54781 aagtgctagg attacaggcg tgagccactg cacccaacct atttttattt tttgagacgg
54841 agtcttgctg tgtcacctcg gctggagagc aatggtacga tcttggctca ctgcaacctc
54901 cgtctcctgg gttcaagcga ttctcctgcc tcagcctcct gagtaactgg tattacaggc
54961 atgcaccacc acacccagct aattttttta tttctagtag agatggggtt tcaccatgtt
55021 ggccaggctg gtctcgaact cctgaccttg tcatctgccc acttggcctc cccaaaatgc
55081 tggcatcata ggcatgaacc accacactcg gcctttttac tttttttttt tttttttttt
55141 tttttgagat ggagtctcac tctgtcaccc aggctggagt gcaatggtgc aatctcagct
55201 cactgcaacc tccacctcct gggctcaagc gattgtcctg tctcagcctc ctgagtagct
55261 gggattatag gcacacacca tcatgcccag ctgatttttg tattttcagt agagatgggg
55321 tttcactatg ttggctaggc tggtctcgaa ctcctgacct caggtgatcc gcccaccttg
55381 gcctcccaca atgctgggat tacaggcgtt gagtgagcca ccgcacccag cccagttttt
55441 acgtttaaga aatgtatagg ctcaaaacaa ggcacagata agccgtcttt ctcctgagag
55501 actcagtctc aaaacaagat ctacaaaggt ttttactacc atttactcag caccttagta
55561 acagtgtcag caaaattcta cgggaaccca ggtgaggtgg ccagtctctc caacataaaa
55621 cctgacccta accctaaccc tttccctctc ctccatggcc tactccatct gctcttgctt
55681 ctctcctgaa ttttcaactt acctctgact actgaaacct atcgtcaaca tgtaaacaca
55741 ttcaaggctc ttctgccaac tcctcccaca aaaaaccacg ctggtttaat ggtgcatctt
55801 tcttagggtt accagatttt tttttttcaa cttttatttt agattcagga gacacatgtg
55861 caggtttgtt acctgggtat attgcatgat gcttaaggtt tggggtacaa ataatcatgt
55921 cacccaggta ctgagcatag tactaacagt ttgtttttca acctttgctg ccccctctcc
55981 cctccctctc tcccatctat tttttttttt agggttgcca gatttttttt tttttttttt
56041 tttagactga gtctcactct gtcacccagg cttgagtgca gtggcacaat ctcagctcac
56101 tgcaacctct gcctcccagg ttcaagtgat tctcctgcct cagcctccca agtagctggg
56161 attacagaca cgtgccatca tgcccggcta atttctgtat tttcagtaga gacggggttt
56221 caacatgttg gacaggctag tcttgaactc ctgacctcag gtgatccgcc cgcctcagcc
56281 tcccaaagtg ctgggattat agatgtaagc caccacaccc ggccagttgc caggtttaac
56341 aaataaaaat aaagaatgtt cacttaatct gaaaagtggt gtgtgctgaa aaaattttaa
56401 aaataaagaa tatttgctgc atattatatt tattgaacaa tcagcaaact aggaataaaa
56461 aataacttcc tcatcaggtg tggtggctca cacctgtaat cccaacactt tgggaggccg
56521 aggcaggtgg attacctgag gtcaggagtt tttgatacca gcctgaccaa catggagaaa
56581 ccccgtctct actaaaaata caaagttagc tgggggtggt gcatgcctgt aatcctagct
56641 ccttgggagg cttaggcagg agaatcactt gaacccggga ggcggaggtt gtggttagcc
56701 gagattatgc cattgcactc cggcctgggc aacaagagcg aaactccgtc tcaaaaaaat
56761 aaataaataa aaataaaaaa taacttcctc aacatgataa aaggtttata aaaaccccat
56821 agttaacatc atactcagcc gggagtggtg gctcatgcct gtaatcccaa cactttgaga
56881 gactgaagca ggagtattgc ttgagcccaa gagttcaaga ccagccttgg caacatagaa
56941 agactccact ctacaaaata taaaaattta gccagatgtc gtagtgtgcc cacaggtggt
57001 cccagctact tggaaggctg aggcaggagg atcacttgaa cctgggattt tgaggctgca
57061 gtgagccata atcatcccac tgcactccag cctgggcaac agagtgagac cctttttctt
57121 ttcttttctt ttcttttttt tttgcctcag ctacttggaa ggctgaggca aaagggatca
57181 cctgagtcca ggaggtcgag gctgcggtga gccatgatag tgccactgca ctcccagtat
57241 gggcaacagg gtgagacagt ctcaaaaaga aaaaaaaaaa aaaaaggtca gtattggtgg
57301 ctcacaccta taatcccagc actctgggag gccaaggtgg gcagatcact tgaggccagg
57361 agttcgagac cagcctgggc aacatggtga aaccccatct ctactaaaaa tacaaaaaaa
57421 aattggccag gcatggtggc ttatgcctgt aatctcagca ctttgggatg ccaaggtggg
57481 cagataacct gaggtcagga gtttgagacc agcctggcca atttggcgaa acactgtctc
57541 tactaaaaat acaaaaaaaa tcacccaggc gtagtggtgg gtgcctataa tcctagctac
57601 tatggaggct gaggcaggag aatctcttga acccgggagg tggaggttgc agtgagctga
57661 gatcgtgcta ctacactcca gcctgggtga cagagtgaga ctccgtctca aaaaaaaaaa
57721 aaaaaaaaag gtgccgggct tggtggcaca ctcctgtaat cccagctact tgggtggctg
57781 aggcacgaga atcgcttgta cctgggtggc agaagctgca gtgagcagag atcacgctac
57841 tgcactccag cctgggcgac agagagaaac tctgtctcaa aaaaaaaaaa aaaaaaaaga
57901 aaagtatgtg tatataccca atggtaggtg aaagactgga aacattcccc taaaatccag
57961 aacaagacaa agatgcctac tttcattgtt tgtattcgat atcttattag aagttccaac
58021 cagagcaata aggcaagaaa aaagaaaaaa aaggaatcca aagtggaaaa gaagatataa
58081 aactgtattc acagatgaaa agagcctata tataggaaat tctgaaaaat tcacacagca
58141 acaaaaaaaa ttattagggc taacaaatga atattaagca caatagcagg ttaatcatct
58201 gtcttttttt tttttttttt tttttttttg agacagagtc tctggctctg ttacccaggc
58261 tggagtacag tggcatgatc tcaactcaat gcaacctctc tgcctcctag gctcaagtga
58321 ttctcatgct tcagcctcct gagttgctaa ggtgtgtgcc accacatctg gctaattttt

58381 gcatttttag tagagacagg tttcaccatg ttggcaaggc tggtctcaaa ctcctggcct
58441 caagtggtct gcccgcctca gcttcccaga gtgctgggat tacaggtgtg agccgccatg
58501 cctgattaat catctgtatt tctaaacact cacaattaac aactcataaa agaagtttta
58561 aaaacaattg catctacaat agcataaaaa agaataaaat aattaggaat aaagttaaca
58621 tcggggtaca gtggctcaca cctgtaatcc cagcactttg ggaggccgag gtgggtggat
58681 cacgaggtca ggagttcaag accagcctgg ccaagatggc gaaaccctgt ctctattaaa
58741 aatacaaaaa attagctggg cgtggtggca ggtgcctgta atcccagcta ctcgggaggc
58801 tgggcagaga actgcctgaa cctgggaggc ggagcttgca gtgagtcgag atcgcaccac
58861 tgcactccag ctgggtgaca gagtgagact ccgtctcaaa aaaacaaaaa aaaaattagc
58921 cagttatggt ggcatgcacc tgtagtacca gctacttggg aggctgaggt tagaggatcg
58981 cttgagccca ggatttgagg ctgcagtgag ctacgatcac atcattgcac tccagcctgg
59041 gtgacagagc gagaccctat atcaaacaaa acaaaacaaa aaactaagaa aatgtaagat
59101 ttatacactg aaaactataa aacattcctg aattaaaaat ctaaataaat taaaagatat
59161 ataccatgat tatggattag aaggcttaat actgtcaaga tggcaaaatt ccccaaagca
59221 aacgacagat tcaatactgt ccctatcaaa agcccaaagg cttttcttgc agaaatgggc
59281 aaactgatcc taaaattcat atggaagtgc aaggaccatg agtagccaaa acaatattga
59341 aaaataacaa agttggagga ctcatacttc ccaattatgt ggtattggca taagaataga
59401 tgaatggaat agaaatgaaa atccataaat aacccccccac atgtatggtc aattgatttg
59461 acacagatgc caaaaccatt caatgcagga agaatatcct cttcaacaaa tgatgctggg
59521 acaactagat atccacatgc aaaagaatga agttggactg ttccctcatg ctacatataa
59581 aaattaactc aatgtgatca aagacctaaa tataaagagc tcaaactatg agaaaagaca
59641 taggggtaaa tctccctgac cttggacttg gcaatggttt cttaaatatg ataccaaaag
59701 cacaagcaac taaagaaaaa ataataaatt gggcttcaac aaaattaaaa acagaaagtg
59761 aagacaactc acagaatggg agaaactatt tgcaaatcac atatattgta tctgttacct
59821 gtattatgtt acttatatat atacaatctc cttatgtttg tgctctaaca taaccaaacc
59881 tgaatcccac cagacttcct aatataacac atagcatgtt tccttctcat ctgagctact
59941 tgctatgaaa accattctgc ctaagcgtca gacttgggaa ccattggccc aacaggtgct
60001 tgtgtaattc tgttcttcta ggtccaattt taagaagaaa aatatgaggg tagggggagt
60061 ttatgcaaga caagtagaaa tgtgaaagag ggagctaaaa gaaaaatatt aagttcacca
60121 attctgaaat ggctcttaaa gcaaaggaaa tcagcaacta aggacaaaca accacatcta
60181 aacaagaata agaattttaa ttttagagca cgtgaaaaga tgtgttggag gaaaaatcct
60241 gaaatataag tgaagctttg agagcctttt tttttttgga cagggtctca ctctgtcagc
60301 caggtgggag tgcactgaca caatcacagc tctctgcagt cttgaccttc taaactacgg
60361 tgatcctccc acctcagcct cctaagtagc tggactacag gtgtgcatca ccatgcccag
60421 ctaatttttt gtatttttag tagagatggg gttttgccat gttgctggtc tggaactcct
60481 gggttcaaga gatcacccaa agtgctggga ctacagacct gagtcaccac acttggcctt
60541 tttttccttt tttttttttt tttgagttag ggtcttgctc tgtcatccag gctaaagcac
60601 agtggtgtca tctcagctca ctgcagcctc aacctcccag gctcaacaga tcctcccacc
60661 cagcctcctg agtagctagg actgcggggg cacaccacca cgcctggcta attttatttt
60721 tttgcagagg caaggtctca ctatgttggc caggctggtc tcaaactcct ggactcaagc
60781 aatcttccca ctttggcctc ctaaaggctg agataacagg catgagccac cacatccagc
60841 atgagattct tattttaaaa caaggtatag gccagccaca gtggctcatg cctgtaatcc
60901 cagcactttg ggaggccaag gtgagtggat catctgaggt caggagtgtg agaccagcct
60961 gaacaacatg gtgaaacccc atctcaaata aaaatgcaaa aattagctgg gcgtggtggt
61021 gggcgcctgt aaccccagct actcaggtgg ctgaggcagg agaactgcct gaacctggga
61081 ggcagagctt gcagtgagcc aagatcgcgc cattgctctc caacctgggc aaccgaggga
61141 gactccgtct caaaaaaaaa aaaaaggcca gacgcggtgg ctcacacctg taatcccagc
61201 atttgggagg ccaaggcagg cggatcaccg gaagtcagga gttcaagacc agcctggtca
61261 acatggagaa accccgtttc tgctaaaaag acaaaaatta tccaggtgta gtgatacgcg
61321 cctgtagtct cagctactcg ggaggatgag gcaggagaat tgtttgaatc tgggaggcgg
61381 aggttgcagt gagctgagat tgtgccactg cattccagcc tgggtgacag agcgagactc
61441 catctcaaaa aacaaacaaa aaaactggct ttaaaaaaaa aaaaaatctg taagggtggg
61501 gggagggtct ccagaaaaga ataaaatttt taaaaattgg ctttatataa tgtctgacta
61561 tacttttatt ctaaaaactc tgtgacataa ggctggtaaa cagaaagaaa aatccaacaa
61621 ggacttactt gaccgtagga catattaaga tgtttattat ttactaagag taacatgtat
61681 acatttgcag taatttgtac aatccaacta cattacaatt cacagtaaca tacactagct
61741 ctaacctgcc ttggatacaa ttaagtctcc tcaacacact attttatcgc caaacttaca
61801 ttctggcttt tataatcatt ttgcaacacc tggtacagta tacacctata gctttgccat
61861 agaaatgccc ctaaatgccc ttcagagagc agaggtgaat actttctcat gaagaaacgc
61921 caacttttct aagcgagttc gtttcagtag tgggagccat tcccagtagg ataactctac
61981 cacacggtag ccaagccgag ccagctgccg cctcttcata ttgtgcagtc caaggagatc
62041 cctggagcca tagcaatact ggttcctgtt tgtgaactga acagccagct tcattcttgg
62101 ggtctgcatg caggctgcgg ggcacaggcc agccatctcc atggcccctg atttatctgc

62161 tacattgcaa aggaagcccc ccagaggtac agctgcctta ttctccaact ccatgggctg
62221 ctgcccaggc tctgactcag ttttgccctg gaaatgtcct cttgctttcc cttttagtaa
62281 cttattcatc aaatcatctg taaggctgac tcccacatgc tcaagcctta atttggctac
62341 attttcagcc ggcgtggctt ctctattaaa tggtaatggc ttcaggttaa catcaagctg
62401 gacctctaag tcagaagatc gggtatgagg caaaatcata tggtgcttga cgtactgggg
62461 cccccccagc atggtctcca gtaaaaagac agtttctaag aattcaggct ttgaattcat
62521 atccttctct gctaaatacc acagcaattc agacccctct tgctgaaggt gagtactaag
62581 acgattgcct ctgtaatctg gacactcaat gccaactgta ccatcgaggg tatatagttc
62641 cttaaggagg tcaaacttag ttctctcctg agctaacctg acaaaccctg gactgagagc
62701 gaaatcaatt aactctactg gaaagtactc caaaaatgcc aggcccagca ggcaggtggg
62761 caggtgttct gggtactggt tgaattcagg catctttctg tgaatctcac ttatcaggct
62821 ggagtaaaat tcttctgcat tgggtggctt ataattcaga gttccaaatg accacagaat
62881 cttggcaaca tctttacttc gacagtgtgc cactctagga ggcaaagacg cagccactgc
62941 attcattact ccttcattca ggaagcgtaa ggccgagcag taaagagtca ggtgcatgac
63001 accttgaact cccagggaag gaattcgctg aggagctatc tctccaatct gcttcatgaa
63061 attgatgtga tccacgtgag tgaaacggaa cattttaaca atattcacta aggagcgact
63121 actcagatgc tgaatgttag cacaagccaa gtctccaatt ttccgcatga caaattcaga
63181 gagattagta cttgatttaa agaaccccaa acagatggta ccaacctcct ccaaattgat
63241 caaatctata tatttaagga tcaatgattc caatttttgc attaggtcct gggatacctg
63301 acgattttca cctataacat aaattaagtg aactagctga gacaaggata gatccttcca
63361 gtgcaaatta agataactag aaaaaatgtt taaaaaccta ggtactttgc ggcctaagta
63421 cctccagaga tcagccacca aaaggagctg atccatattc atctcccata cctgatggca
63481 acacttggtc tcatacacat ctagcattga atgggagtga gggattccta aaatgacaaa
63541 agctttcaaa acattgatca gatcttgggt atcaaagagc tgtatcttct tcacactcag
63601 ctggcagagc agagcaaagc tggtactgcc cagcaagaca ggatgctgct ctgcaggcaa
63661 agagctcagc ttacacaaat aatcaacaat gacttgagcc tggagattat tttgattaac
63721 tctgactttg tgcaaaatta gttcaccttc tgaaacagac aggagctgag aagtctcaga
63781 tgcattatag ctgtgaacac ggtattctgg tcttagctgt aggaaaactc gcatgtttc
63841 aaaggaatca aacacttcta cgtcctcttc atcaactcct gtggccctgg gtgagcccag
63901 ctgcaatgta ctggccttag aggaagaagt cttgctgaat tccaaacctg ggtgggcact
63961 gctggttgtc aggattctcc gagaagagaa ggtgctacat atgttcttaa ctttttggc
64021 agaatggcag aggctaatgt gttctggagg gtcctgtccc ccatgctgtg tgctgctcac
64081 attccagtat gacacacttc ggactgcacc aaaggcagaa ggactgcaaa atgctcggta
64141 tcttacaagt tttaatgact tgagagtagc tgccattctg gtgtcagtat tgatctcttt
64201 ggcagtcaga atacagtcct cacagatttg accaggcaat ttcttgttta tatggtgctt
64261 gattagagct ggacggggag gtgttccaca aaaactgcct ggaaatcttc agcatatcac
64321 aagagccagg gatcacaaat gactgggtca gaattggtgc cagatactga aaaaagggta
64381 gatgaagaat gagtggcttc tacctataaa agcatccaaa tttacacatt ataaaaaaca
64441 ggttgaaact acactgctgt ctactcaaat aagttcaatg ctagtaaatg cccaaaccac
64501 atgacagcat atatcaagga taaattctgc ggtctgtgta aaggcctagg aaaacccca
64561 acccagggta cccgtgggca gttcctggac tgctgctcac cagacttcct tcactgtaca
64621 aggcaagcag tgacggctaa agaagagacg gtgctccgct aacatttgat aggaagaagg
64681 aaggcagatt tctagaaaga aggctgggaa caaaagtgag caactccttc catctctttg
64741 atatcaaggc aaacaggtcc tacctgaaga atgtttataa aaactgctat gctaacagca
64801 ctaagtaaaa agtgggatct ggtataaaag ataattagtt ggctgggtgt gggtggctca
64861 tgcctgtaat gccagcactt tgggaggccg aggcaggcag atcacttgag gtcaggagtt
64921 caagaccagc ctggccaaca aggcgaaatc ccgtctctat taaaaataca aaaaattacc
64981 caggtgtggc agcacacacc tgtaatccca gctactcagg aggctgaggc aggagaatcc
65041 cttgaaccca ggaggcagag gttgcggtga gccaagaccg tgccactgca ctccagccta
65101 ggcgaaaaag tgagactcca tctcaaaaaa aaaaaaaaaa gatttaatta gttataccta
65161 cgataaccct ccctgttctt atagcgcaaa actgttaatt ccaaaattct gtcattctaa
65221 tgctgttctt ttacttttta gaagatgcct actctcctct tcaggaagct tataacagaa
65281 tttgagtcac tattagagag tagtcacaga gtaatggtgt tattagaaag tatactaaaa
65341 aactcactca ttttggtggg agaaagcaga aggttgaggg tctgctcact gtcacttcac
65401 gggtgggaag caaagagtac cccaaagctg ggctcagggt tcagggaacg ccaattgacg
65461 caactgcccc gctgacatct tctggagaag gcagcttagc aatgtttcca gtgagtcttt
65521 attctgcctg tgagcagtat aatgcccatt tttctcccat ggcagaaacc atgagagact
65581 atgaaattga ttctgctgtg ctctcgcttt ccttaatgct tattcttttt ctttccactt
65641 aagtgccaaa gcctgacaag caagacccaa ggagacctga gttgtcaact gatgtaccag
65701 acagggctgt tgctgcagaa aactccataa aagttccaag ggtgccagaa tgactttctg
65761 aaatgcctgc ctaactctgc tgtcagccac tttcttccag agcccatagc acatcatcat
65821 ttagttcatc aaagaacagc agggcaggtc agtggctctg ttccagtagc ccagctacac
65881 aggcttctct agatggcagg aacatatgta tgaatttctg tgtgaatcag agcagaattc

65941 tctcacatta aatttttata cacttggttc tttactgtgg cttgtggacc attctttttt
66001 tctttttgga gacagtctca ctctgtcacc caggttggag tatagtggca tgataactgc
66061 agctttgacc tcccacttca agcaatcctc ccacttcagc ctcagcctct tgaagtagca
66121 aaaactacag gtgcacacca ccaaacccag ttagtgtgtg tttgtgtgtg tgtttgtgtg
66181 tgtgtgtgtg tggagatgga gtctcacaat gttgccaagg ctggtctcaa actcctgggc
66241 tcaagtgatc ctcctaccct ggcctcctga agtgctagta ttacaggcat cagccaccat
66301 gcctgaccac ctgtggacca ttctgcccac caaatttcaa tctgtggttc agatcaaggg
66361 tgagcagact atgtcctggg gcccaaatct agctcttcac atgtttttgt aaacaaggtt
66421 ttttcagaac atagctaggc cattcattat atattgtcta tgtctgcttt cctgctacaa
66481 gagcagaact gtgtaactgt ggcagattgt atggcctgca aagcatattt agtgtttggt
66541 ctattacata aaaagtctgc tgatctctgc tacacactat ttctataaat gcagatagcc
66601 agctttttaa aaattagaaa aacttggcca cgcctataat cccaacactt tgggaagctg
66661 aggcagaagg attgcttgag gccaggagtt tgagaccagc ctgaacaaca cagcaagact
66721 gtctaaaaaa taaaataaaa taaaaaataa attaaaaaaa taaaaaattt taattaaaat
66781 tggaaaaact atagaaaaat agtactaaaa taatgttttt ctttaatatt tagatgataa
66841 ttgaagatta cagtcctcaa aatagtcatt aggaaatcca attaatacta cagcacagga
66901 aaggtgggaa caaaagagac agcaggatgg ccaggcgtgg tggctcatgc ctgtaattcc
66961 agcactttga gaggccaagg caggcagatc acttgaggtc aggagttcga gaccagcctg
67021 gccatcatgg tgaaacccta tctctactac aaatacaaaa attagccagg tgtggtggca
67081 tgagcgtgta atcccagcca ctcggggggc tggggcacaa gagtcgcttg aacccaggag
67141 gcggaggttg cagtgagcca agatggtgcc actgcactgc agcctgggtg acagagactc
67201 tgtctcaaaa aaaaaaaaaa aaaaaaaaga accaaaaaaa cagagatagc aggtaacaga
67261 actctccagc tgacagaaca gggcttttaa gaagacctat ttttggctgg gcacagtgac
67321 tcatgtctgt aatcccagca ctttgggagg ctgaggcggg tggatctatg aggtcaggca
67381 ttcgagacca gcctggccaa catagtgaaa ctctgtctct actaaaaata caaaaaatta
67441 gccaggcgtg gtggtgggcg cctgcaattg cagctggggc acctgcaatc ccagctactc
67501 gggaggctga ggcgggagaa tcacttgaac ctgggaggca gaggttgcag tgagctgaga
67561 tagcgccact gcactgcagc ccgggcgata gtgcaagact ctgtctcaaa aaaaaagaag
67621 aggaagacct actttcgaat ttggaattta atttaatcca ccacaatgtg caactaaagc
67681 catatactgt tcaaatctgt agtcttcatc tcatttgagg aaggagatcc tggccaggca
67741 cagtggctca cacctataat cccagcactt caggaggcca aggtgggcgg accacttgag
67801 gacaggagtt caagaccagc ctagccaata tggggaaacg ccatctctac taaaaataca
67861 aaaattagct gggcgtggta gtgcgcctgt aatcccagct acttgagagg ctgaggcagg
67921 agaattgctt gaatccagga ggcagaggtt gcagtgagct gagatcatac tactgcattg
67981 cagcctgggc gaaagagcaa gactgtctca aaaaaaaaaa aaagtagatc ctaaaaaaat
68041 agatttaaag gtcccatttt ttaaataaaa tagctgtgcc atagtaaagc gaaaaacaga
68101 aaatgtctgt ctagaaggag gctggtttac aagataaatg tgtggcaggc atcaacacat
68161 gaaaaagtac acactaatat tgagtggaac aaatagcaca taaacaagaa ttaacaaata
68221 tctctataaa tgatattaat tttaaaacat taaataatta aaattacttc aattaattct
68281 acctaagagt acttaatgaa attactatac gtggtctgga aataaaagaa ccatctccta
68341 gaacatcaat gtttggtgca gctccagtgt tttgaattca tgtgacactt tttgatttgt
68401 aaaaacacag gctaagatcc aagatggaaa taaccagagc tatgccttag ggcctcctct
68461 gtgccaaggc ttagagcctt gtaaagctgc tttatctgca ttagttccca tccttataac
68521 atctctgtaa tgtaagttta taatccccat attactgatg agaaagagac cacaagatta
68581 agaaacctgc caaaagttac acagccaagg gattgagtgg agcctaatat taaagactgt
68641 ctgactcttc agcccacatt attggtgtac tagagagaca agaatggatg tcagaaaaca
68701 ggttcggggg gcagtacggg gtgggatggc tgaacaatct atggtcttct aggatttcca
68761 ggtgccaggg agctggaggg ttagaactag gtaagcttga aagtcccttc cacctctaac
68821 agtctacgat tctatcacta cggtttatac tgcaagttga gtatcccttg tccaaaatgc
68881 ttgagaccag aagtgtttca gattttaaac tttttcagtt tttggactat gtgctttata
68941 ctgaccagtg agcattccaa attcaaaaat ccaaaatctg aaatactcca atcatttttt
69001 tttcctttga gtatcatgtt ggtgctcaaa aagtttcaga ttttggagca ttttgcattt
69061 tggagtttca ggtttggaat gctcaacctg tactttaaaa aagaacaaag agaagacctc
69121 aaaggctgaa atctacatga ggcaaagagt tcgcaggatg ctccaggatc aaagtaagga
69181 gagctaccac aattgaagac ctgaaagttg gcagtagaga tatgggatcc agaaactagg
69241 gttcaataca gagaagcaaa gggaaatccc aggaaaaagg tgaaggaaga tcccaggaca
69301 agagctgtga acagacataa agagcaacca gctcatgctg gacgacatag acagtaccca
69361 ggagaggaga atcatacttc taccggataa tccgggcatg aacaagagat aaggatacaa
69421 aaagccatgc taataaaaac agtaacaaaa attattaatg ccagggaaga caaaaaagtt
69481 atacaagata gaatatgtta ccacaggcag gatgcaatgg cccattcctg taatcatagt
69541 actctgggag gccaaagtga gaagatcact tgaggccggg agtttgagac cagcctgggc
69601 aatgtagtga gacccccacc tctacaaaat atttagaaat tagccaagcc tggtggcaca
69661 cacctgtggt cctagctact caggaggtag aagtgggaag gttgcttgag cccaaaaagt

69721 taaggctgca gtgagctgtg atcctgctat ggcagggtga cagagagaga ccctatctca
69781 aaaaataaat aaataaataa aaagagtaaa gaaaatatta gtatatcata ctccctattc
69841 agttgagaat aatactaaca taattttatt aatataaatc ttgatattag cctaaccaaa
69901 aattatgagc aaactatatt gggaggataa agtgagtaag aggaggagta gggggaggtt
69961 gatgatacaa gagacctaga gcctcaaagt ccataataaa gaagtacaat tggacaggtg
70021 tggtggctca caactgtaat cccaatattt tgggaggctg aggcaggagg atcacttgaa
70081 cccaggaggc agaggttgca gtgaaccaag atcgtgccac tgcactccag cctgggcgac
70141 agagcaagac acagtctcaa aaaaaaaaaa aaaaaaaaga aaagaaaaga aaagaaaaaa
70201 taaatatagt gatggccagg atcagtgagt ggctcacacc tgtaatccca gcactttggg
70261 aagctgaggt gggcggattg ctttaggcca ggagttctag accagcctgg gtaacatggt
70321 gaaaccctgt ctctactaaa aatacaaaaa gtaggctggg cacagcggct caagtctgta
70381 atcccagcac tttggaaggc tgaggcagga ggatcacctg aggtcaggag tttgagacca
70441 gcctggccaa cgtggcgaaa ccacgtctct cctaaaaata caaaaaatta gccaggcatg
70501 gtggcaggca cctgtaatct cagctactca gaaggccgag gcaggagaat cacttgaacc
70561 cgcgaggcag aggttgtggt gaggcgaggt cgcaccactg cactccagtc tgggcaacag
70621 agcgaaactc catctcaaaa aaaaaaagtg ggtcgggaga ggaaactatt atttagtgtt
70681 acaagctcta ctgaactatt tgactgttta aaccatgtac atgtacaatt ttgacaaaaa
70741 ttaaattaaa aaatccatgt attatgaaga tattatggta ttgttcaaga taatgaatgg
70801 agaaagtagc ttaatagata aggaaaatgt tgaaaagtta tttttagtgc aagcactttc
70861 tactacctaa gaacttcttt aaagatgttc aataacagaa atgataacaa aataaggaat
70921 cttagaggca tgaagagcca acctacaaat ggtaaacagt ggctacttac tcttttcact
70981 atgcaacatt tacagtaact ctatagaaac tgtatctaca tacacaggtc tgaaacagag
71041 gacttcagaa gttggtcttg agtactgtgg agaaggaaag aaaaacaagg aagatacaga
71101 agcagcagag aagaacagta gggagaaaag acactgcaaa gaagcagcag tcaggatgac
71161 cagatagcga aaacatacct gagaaggtta gttcctcctt actcaaagac tctcagaaaa
71221 tccataggaa agctggaaag agaacactat gagatgctca gtcaattaac ttctgactct
71281 tttttttttt tttttttttg agacagagtc ttgctctgtt gcccaggctg gagtgcagtg
71341 gcgtgatctc agctcactgc aacctctgca tcccgggttt aagtaattct cctgcctcag
71401 cctcccaagt agctgagact aaaggctcac gccaacatgc ccacctaatt tttttgtatt
71461 ttttggtaga gacggggttt tgccatgttg gtcaggctgg tctcaaactc ctggcctcaa
71521 gtgatccacc tgccttggcc tcccaaagta ctgagtacag gcaggagcca ccacgcccgg
71581 cctgactctt tcaataaagg tcaaagatac ctgatatgat ttcacaaaac tcctttccta
71641 gtttaagtgc cccaagtata cctttctccc ctttaggggt cctaacgaaa cagagtagag
71701 cctcagacac tcctggaccc tgtcagttcc aaaaggcaat gcccaaacct ccctgaatga
71761 tgtgatttca caggactgaa gaatcttacc tttctccctt ccacataccg gtctgagcag
71821 agccgaatag cttcatttta aactagagtc tagaagttag aggagggggg ccaggcagag
71881 gaaggagcga tgctaactgt agactgagta taagacacct taaacacata caatttacca
71941 cttaccagat ctgatccctc aattctaaca cttttccttt ctatgtccca ataaaattgc
72001 cacaaataaa tgaacactcc aaccctattc ttgtctcagc aaataatttg ttgagcaacc
72061 ttaagaatta aaaaggcaaa aaaactggct agacaggtgg ctcatgacca taatcccagc
72121 actttggaag gccgagcagg cagatcacgt gaggccagga gttcaagacc agtctgggct
72181 acatggcgag accctgtctc taccaaaaaa aaagggcaaa aaaaccgaac tttctctagg
72241 tgctgaggta ttctgctgag aatctacaaa acttcaaagc ccctagtgag ggcttagaga
72301 ctggcagtaa ggaccttagc tgttaatgtg gatgttaact gggctagact cttctgtgtt
72361 tctgtttcca cacgggagca gggcctcaga gaggagtcta ccagtactgc gaaggtgaag
72421 cactagtgca aaggtgttta ggtcaagttc agtgtcactc gcaggtctaa gccaaactaa
72481 aattattcag tctagcaatc catcccttcc tttttgttct agactccatg ctgataggag
72541 aaaaacagca catcacaaac atttgtgaaa ctttttcagc tacacagccc ttccgcttct
72601 cttttaagac tctgctattc ctctatcaga atggcaagaa ggatgtgggt agatatagta
72661 tgactaagcc ctgtagatga ttctgttaga gctactgctt ttaaaggttt tcataaaacc
72721 ctgtctttag aaaacctctc catataacaa caggcagtag gcactcatgt cctctcagcc
72781 catctaaatg gggcttctag aggaattaca aaatccaggc aggtaaagag aagtagctgc
72841 atgcacacag tcgtcataag agaaaattcc caaataaatg gtccaagtac ctataactgt
72901 tagctcaata aacacttggt tttatctgct tttacaattt gcaaggctgt atagaggctg
72961 tatagtacat acttcagaca aataaagaag gagtttcaga attcttttgt tttttttttc
73021 agacagggtc tcgctctgtt gttcagtctg gagggcagtg gtgcaatctc cgctcactgc
73081 atcctcgacc actgcccccc acctctcacc ccggcaacct cccgggcgag gatcgctcaa
73141 ggatctcaaa cgatcctccc acctcagcct cctaagtagc ttggactaca agcccaccac
73201 cacgcctggc tacattttgt ttttttgtgt gttttttttg ttttttgttt ttttcgtaga
73261 gacagagttt ccctatgttg cccccaggtt ggtctcgaac tcctgggctc aagtaatcct
73321 tccacttcgg cctcccaaag tgctgggatt acaggcatga gccaccgcac ctgaccttag
73381 aattatttta tatcaaagtt gacttattca catgatgaat ggcaaggcct tattagggac
73441 taaaaaaata aaccaatgga tggggaagaa aatcatactg actaataaac attagacctg

73501 cataactgcc aaaggcctca ctttttaagt tatgttatgc agcacagagt cttcagattt
73561 tatttactta tttatttatt tatttattca tttatttgag acggactctc gctctctcgc
73621 ccaggctgga gtgcagtggc gcgatctcgg ctcactgcac actccgcctc ccggggttca
73681 cgccacgctc ctgcctcagc ctccagagta gctgggacta caggcgcccg ccaccacgcc
73741 cagctatttt tttgtatttt tagtagagac ggggtttcac catattagcc aagatggtct
73801 cgatctcctg acctcgtgat ccgcccgcct tgacctccca aagtgctggg attacaggcg
73861 tgagccactg tgtccggccc agattttatt ttagtctacc aggtagtagc aaactatcca
73921 aggacttgcc caggaccatt caggtcagtg agctacagaa tctagttctg gaaaggcagc
73981 cattcttact attcacaaag tgtagaaaac gtgaattcag attccagcag caacaaatga
74041 ctggagcaca aaacgccagc cacagttggt gctaacgcag ctggctatca gctccacagg
74101 atgaagcact gtccaagggt ctgatcagtg aagcatcacc caagactgat gaacacagct
74161 gatatgaggg gacccaaagt tccctgagtc aagacagaag agctgtgtca cggtttgcac
74221 agaaagagaa agagcaacta ttcttaccca cggaaagaaa aagggagaag gccatcaata
74281 ctacccttag gcttgtgcaa cgagaaactg ctaaaaatgg ggggaaggta tatatcctat
74341 catactgatg tttaccgaag actccgcatc ccaaatgcta agcaacatac tgtagggcct
74401 tgctactcag tgtgtggtct gcgtgccaga ggcttcagcc tcgtctggaa tctagttagg
74461 aacacagtta tctcagaacc caccccagtc ctacagaatc agaatctcta agcgattcgc
74521 aggcacatca aagtgtgatg agaggcgcgg cagaatatct gactacattc tcttaacagc
74581 accgtgagac aagtactgtt acgatctcca tttcacagag gaaaaaaaat caagggtcag
74641 ataagccccc tgccaaagat cacaaactca ggagcggcga gagtcactga agttctcacc
74701 acagccaggc ctgcgagagg cactggcaac ccaggccaag agctgaacaa ggcctcgatg
74761 cgctccagct ctgggccggc cgggaggaca ggaggcacag gccaaggccc aggacggtcg
74821 tgaacgcgga aagaggccgc cgcgacctcc tccccggccg ccgccactca cctgatcctc
74881 ccgaaggtgg gcgccgcaac accaaaccgg ggacgccgcg ggcggcggcg acacagatac
74941 tggctcctcc ggcgactccg agcctcacag ccccacttcc ggccaactgc ccgctacttc
75001 cgcctgctgc gcgagtgacg gatgcggccg aggctcaaaa gactctgtca gcgtttccca
75061 aggacatttt ccactggagg acgttgttga ggaggcaggg ggagtctagg acctgcagag
75121 ggaaaacacc ttcctggatg gatcgctctg gaagtcatta agcagcttag tcccggcgcg
75181 gtggctcacg cctgtaatcc cagtactttg ggaggccaag gcgggcggat cacctgagtt
75241 caggagttcg agaccagcct ggctaacgtg gtgaaacccc cgtctctact aaaaatacaa
75301 aaattagccg ggcgtggtgg cgcacgccta taatccagct attcaggaag cggaagcagg
75361 agagtcgctt gaacccggga ggcggaggtt gcagtgagcc gatatctcgc cactgtactc
75421 cagtctgggc acccctgtac tccagctctg ggacacagag cgagactcca tctaaaaaaa
75481 aaaaaaaaaa aaaaaaagta agtagcttag acacatgttc aaatctgagg accaccactc
75541 agggttttat ggccttggag aagtcacata aattcattct tgttatatta ttattattat
75601 tttcatagag acggggatgg cagtgtcttc ctatgttgcc caggctagtc tcgaactcct
75661 gggctcatgc aatactgccg cctcagcctc cgaaagtact gggattacag gtatgagcca
75721 tcgcacccgg cccacataaa ttctctaagc ctgtacagtg agaaaaacaa tacatttttt
75781 ttttttttga gacaaagtct tgccccattg cccaggctgg ggtgcaatgg tgcaatcatg
75841 gctcactgca gcctcaacct cctgggttca agtgatcctc ctgcctcagc ctcctgagta
75901 gctaagacca caggcatgca ccaccacacc tgtctaattt taacaatgca ttggtcgggt
75961 gtggtggctc acacctgtaa tccccggtgt tggagaccgg cctggccaac atggtgaaac
76021 ctcgtctcta ctaaaaatac aaaaaaaaat tagcggggca tggtggcgtg cacctgtaac
76081 cccagctact tgggaggcta aggcaggaga atcacttgaa cgcaggaggt agaggttgca
76141 gtgagccgag attgcaccac tgcactccag ccggggcaac agtgagactc catctttttt
76201 tttttttaat gcatttataa tagagatgta tctgtaaagt gctctcagga aaagagctag
76261 tgacatgatg gatagatgag cctgctgaga gaaatggatg tggagctggg gcttagcgag
76321 gccagagcta tggagcatga gccagggtga ttctccacca ccctaagaag gagggctggg
76381 tcctactggg tacatatatt gacacagctg tcagtatagg tacctagaca agaaagagac
76441 tctactaaac ctctacctat cagaaagtcc tggccccagg ctgccattcc tgagggcact
76501 tgagatctgc tgatcagggt cttcatatct tgtcaatgcc ttggctctaa ggggtgtgaa
76561 ggagagctga gaaatcatgc tccctccagg gcctatcttg tagggaagac aaaatcaatg
76621 taggaagaac acgtccctgc caggaggggc aagcctgaca tcccaactcc tgagctctgg
76681 agacagcgct gtttgaatat ctggagcccc agaccacaga caccactgct tagggagggt
76741 tctctccctc aggccagacc agggcaaaca ctattcattg ttatgactct gtccttgcca
76801 gtagaaccca gagaaagcag agagcctgcc cccaaggaat gaaccatgtt tgttcctagg
76861 cagccatgac aatcctgttt ctctttgtaa tactcaaatt cccattctcc cttgaagtgg
76921 gaggagcatg tggctcagtt ctggccaaca ggacttaaag ggaagggtcc tgggaggct
76981 ctgggaaagc ttttactttt catgataaag tagggccgcc tcaatgtctt catggagctg
77041 tggcagccac tttgtggtaa ggagggaaca acatgccaag gatggaacag tgggaggatg
77101 aaaatctagg tccccgagga tccaacaacc atgacaccac ctccggattt cttgtcaggt
77161 tggggaaaaa taaaccactc tttgaggtcc tttgattact tacagcagag tgcattcttg
77221 attgtgtgct ttgagaggat gacagaggaa ctgttgtaaa gggaagctct ctgctgtgca

77281 gcacgcttgg gtcccttttg cctttggggc ttcgccatta attgcaaacc tgttcccagt
77341 cttcacttct atccattcag cccgcaaaca tctaccagtt gctgtaaggt ggcttaggcg
77401 actgcaggct gcatctagct gtcctgcgtc acctgttttc tggctagcag cttagtgtcc
77461 agattctgag ctggagccag cttttaaccc ctttggcaac agtctgagct gaagagaaag
77521 ccccactggg catatacatg gcaccacgac tcttcccggg caacgtggca gaacttgggc
77581 tttgggccaa cctcctagca cggcccagtg agcacctcca gtctcatgcc tcctctggga
77641 aatctgttta ctccagcttt gcccccatcc cccacccggg cctcacctcc cgcacctgca
77701 aagacacaag caggccaaag aataccactc aagaggcttt ttccgtttta tttctttggg
77761 aaaggaaaac aaaaagtctt tccatcacat atggtagaga tatatattta tatatattta
77821 tatataattt cttttgtggt tggacgtccc aaagctgagt ctgttcatat ttttttctat
77881 ttttttctta ctaatgcctt ctcttctccc tgcccctgtg gcctaggccc aggttctagg
77941 gcaggtacag tgaggggtga atggaatggg cagtggtcgc caggatggcc tcccacccag
78001 ctcagagacc tggtgaggac cacccagaag ggctgtgcag agggttagag gccacacaga
78061 ggggcactca tgtttttgtg ggggatgcac agctcattaa acagtcacca gtcatgggag
78121 gaggctggtc aggggctagg ggacttgctc acagtagttg ccagctctct ccaccttgcc
78181 ccccaaaact cagtcttggg ggtgggagga agagagtgaa aaagagagag atataagggc
78241 tgagggccaa gcctcctcca atttttattg ctggtgaatt agaaatttgc ttttcccttc
78301 ctgtcttggg gagatagact ctgagagggc cccaagagaa gcagctgaca ttggctgacc
78361 taacccctca tgccaggcct accctgggca ggtgctgagc cacctcaggg cgccaggaac
78421 ctctcaggtc agccttgttc ttcaagggca atcacatcca actctccccc cgcccccaaa
78481 acaagggcct ccggctcaga cttgggccag tggagctggg aagtgggcag tgcaccaaag
78541 gtcaagcgga attttctatt ggaagaaaag gtgagacttt ggggaagaga ctgcctagga
78601 agatgatgtg ctgagcatga aagcagaaag aggctggggg aatagagtgc gggaagacat
78661 cgcagacact cgtctgacct cagaaactgg gcgaggagag tgcctgagag gcaggtagca
78721 cttaatagct gtgttagagc cggacagtgg tcctgggtgt ggggtcagcc aagcagatgg
78781 ggctgttgcc ctgcgggatc ctggccagaa gggtgagggt agagccgaga agccgtcagg
78841 caacgcttcc caagaacagg aggacagatc ttccctgggt gctggacttc cagccccagt
78901 cctcccctcc cgcaacacac acacagggac acacactcac aaccaggcca ggctctgagg
78961 cctgagtcca agagatcttg ttgggggagg ggctgggcag gtagccctct cagccaaagg
79021 caggccatct gctcccactt gtcctgccaa tctgcccagg gagggagagc actgtaggag
79081 ggagagaggt ctggagccca ggctctgtca gtttcagcaa acctggtgca ggccccctca
79141 ggctccatgg cctaggaggt gagcacagag agcatcacta gacacagggt ggccaacagt
79201 agcagcccag gcctccagaa ggactggggc accttatggc actgcacgtc tgctgcctcc
79261 atctccaaca agggggtgcc gagaaaggga gcatgactcc cccaccacct aggattgccc
79321 ccactcaccc tgccctcctc ctattccctc ctttaggggg ggtccaagtg ggctgccacg
79381 ggggcagtgc tggagctagg agggcaggtg ggagggaggg aacctggtca cagctgctta
79441 gagaacctct ttggtctggg gttatggggt ctatggggaa gagagatgga ggggagggga
79501 cactggggac tctggccttt tgacaggaca tgtgtcaaaa tgccgagtgc ccaggtcgat
79561 cagatttctg tggactcaat gcgctcgagg cgggaggggg tccaggcctt cttctcctcg
79621 ccatgctggg gagtgggcgt gcttgcaccc cctgcggccc cgcgctcccg cagcctgcgc
79681 tccagctgct ggttgcgctg gtacatctcc acgtagctca gctgcagctg cttctggtac
79741 tcgatcacct tctccttctc ctccagccac acgcggcgct cctcggcgaa gctggcaccc
79801 tggcgctccc gggcccgccg ctcagccgcc agctcggcct gcagccgccc cacctcccgc
79861 cgcagggccc gcgtcccgct ctccccgcca gcctccgcct ccccgtccac ggaaaccaag
79921 gaggcggcag cggccacccc ggcctgacgg cgcatcttag cctcgtcgct ctcgcaggtg
79981 gccagagcat cctgtggctc ggccgggtcc acgggggtca gcgccggctt gaggcaggcg
80041 gcaggcagct cgccttcgcc cagctccagg ctggcctgct tgctgctgaa ggagtcccgc
80101 aggctgagca gctgctcctc cttctcccgc agcgaagccc ggccctcccg cagctgcgag
80161 cgcagtccca cgatctcact caacttctgc gacacatccg cctgcgagtc cttcagctgc
80221 tgcttcagga gggagatctc gccagccttc tggcacacct gggcaggaac aggtgagagg
80281 agaagccagg taaaggcaga gctctgctca ccccaaccca gctaccagat ctggagccag
80341 gggcaccaag cttcccgggg ctgcaggctc agcccctcat cagcagcgac gcacccgatt
80401 ccctgcctgg actcaagatg gacatgtctc acccagcgga tggcctcggg tcctcacaga
80461 cactcccaat tgatttttgt cccccctgct cctttcatcc ccccccccat cccaccgtta
80521 tgatagtgag gggcaactgc tctggggttt cccagaggga cagaagggag gcagagggga
80581 ggcccagatc cccagcccgc acctcccact tagtttcctc tatccggggc aggaagtcgg
80641 cctgctcctt ctggcaggcg gccaccttgt cctccagctc ttcccgctgc cgcatcagcc
80701 gggccgcctc ctcctgcagc tgcttcttgt cctgctgcag ccgcaacacc tgcagctgta
80761 ggccctgctg ggcgcgctgg gcacgtcggg ccacctgctg tagcttcccg ctgcagccct
80821 gccgcagctc ggccagctcc cgctcccacg ccttctgccg ctcctccagt acctggccca
80881 cagccgcctc gctctgctcc aggctgcgcc gcagagctgc cacctcctgc tccttctccc
80941 acagccgctc ctccagctcc tggatgagtg cactggggga gggcggtgag caggccgcga
81001 aaggcaggcc tccacctccg ccctccccag agcccaggtg gcctggccgc cccatagatg

81061 acgacgaccc actcttgctg gaggcccgtc cactatcgga ggtccccagg tcctggtagc
81121 ccgacccccc accgctgctg ccgccactac cactaccata gctggcagtg ccaatgcggt
81181 taatgtggct ggtggaggca ctgaggggtg ccaggtgctg gctgtagctg gagctgtagg
81241 tgggcaggct tgtgagggag ttccggcctg agtctgagag gccactccca ctcccacccg
81301 ctggagtcat ggtccgagac ttgtccagtc cgcctttgag gccaccaggg ccctgccgtc
81361 cctcaggagt cccattggtc tgcggggggc acaaattctg catggagtgg aaattcttgg
81421 gtacgacagg cttgaaggcc gacggccgaa ctagtggttc tgagcactgc aggaaacgca
81481 ggagggggct gggggtcagg atgaggcctt gggcttggcc caggctcttc cctctggcca
81541 agacttgtcc agccccacct cccaccccta ctggatttcc accctgaggt tccccatccg
81601 gggttctacc tcatggctcc ctgataccag atctctgtcc ttgatctacc cgctagactt
81661 ggccccaggt ccccagtcca tgactcccca gcccagcctc catcctgccc ccacaaccca
81721 acctccaaga aggcccactt tgccttcctc tctgggttgc ctcccacccc cagaaaaagg
81781 ctgtgagggt ctcaggccct gcggactgac ctggtctagc ttgccagagg tggccaggag
81841 cttgggtggg tggctgggct cacgatactg cggtggggct ttgtcactgc caccactgct
81901 gctgctgcct ccgctgctgc caaccacgtt gccacagaca tcggtgtggt cactgccccg
81961 cagctcacca ttgaggtaga gggagttggc gagacccttg tcctctgagg ggtagcggcc
82021 cggcctctcc ctgctggccc cactgccact gcctcggggg ccagggaaac tgccctggct
82081 gcccccaccc cctgtgcggg tacccacgct cttcatggca aactcctggg catgggccac
82141 cccactgccc acgctgccca tggccaggcg gggtccgggg ggtccaagct cggagggccg
82201 tggggcaaag gccaggagag gatcccgccc tgggtcagcg cgcacaggca gcgtctccag
82261 cttcgccatg actaagccag gggggcactg tggcacaggg tgggaaggca gagataggta
82321 agaccctact cctattccca ccaccccagc caagtcaacc cggcaccgct ctgcccctca
82381 gtgtcccctt ccctacaatg ggtacaaaaa gttcccaggc atccaaggtg aaggaataag
82441 gctgggataa aagagagaac ttgggaatgg gggctgcctg ttgcccacct gataaagccc
82501 aacccccagg gctccatgca catagccccc agcgttgctc agctcagctc agctcagctc
82561 agctcagcgt tccattccta aacttctgcc ttagccccac ctccgccatg agcttcccag
82621 gctcagtgac cctctcaact ctttgctgca accccagaat tttggggttc tttatcttgg
82681 gtctgggacc ctaatatccc aacctcatgg gctggggaaa tggggttgga aggggagagc
82741 agtacctgaa tccaacaggg aaccaacctc agaaattagg ggagggagag aaatacaccc
82801 ctatccaaaa attgagggtg ggggtccttc ccagccccct aacctaagtg ttgcagtcag
82861 ggcagaagtg gaagctcact accagcgcag gcttctctcc ttgcctttcc aaggggttga
82921 ggggccgacg ggtcctcaag cctgggaccc tccgaggccc ggtctgcagg ggccatgtgc
82981 ctcactctcg cagtcggggc tcggcccgaa ccagctgcgc gactttggag gggccgactg
83041 agccatcccg gactgcagtt ttctcctctg cgaaatggga ggaatgaagg acctaccttg
83101 cggggctgcc gcgtgccagg cgccccgagg cggcgagcgc ctgaggaaac gggggtctgg
83161 gggagggtgg gtaggcgacg cacatctggg tcgcgggtgg gcgccgccaa gggcacagcc
83221 tgtccctccc ggatggacgg gggccgcggc tgcagtaggt ggcaggcctg ccctccccaa
83281 ggtcttccca gctcggccgc accggggccc tgggaagaca gggccccctt cccgggatca
83341 gtcctccgca gaggtcccag ggctggctga ggccggggca ggggacgcag tggcgccagc
83401 tgctggcact gacggagcct ctctcagggc ctgcgtttcc cacaggtggg aggggggagg
83461 ggaggggagg ggccgctgca gggtgcgcgg ccccaaggat cctgcgcctg ccgaaccccg
83521 caaagaattc actgccacca ggaagacttc ccggattact accctctccc gctccctgtg
83581 gaccttgacc ctgctcagct taccgtgaag ccctctgagt ccttctgtgg gtgcgcgtcc
83641 ccctccccca aggccggccc acaagcgcag ggggagccag gcctcgggtt acctgtgctc
83701 agggaggccg tgcttcctcg ttcctttgtc tcaagttcgc acctgggaac acctgcccca
83761 gagatctgca taatgcagcc gggcaggagc accaggcagt gtctgcctcg ggttagaccc
83821 tcaaataagt gtgtgggaaa tgggagaggg cgcccttcag gccttttccc actacaccct
83881 gcataaacca ggcagggagc cctccttccc tatcaagtta aggggcccac gtggcaccct
83941 atggccccac agaccgtctt ctacccagat gcccagttct aatgctgagt ccctggccca
84001 gaccaagggc acctgacaaa actgttgtcc ttcgggagtc ttctggcccc cacaccagga
84061 atgagagctc taggttgttg gaaagttcac taccaagcct gtccactgtc tctgtagaat
84121 tccattctca gcatctgtcc ccacaggtgt ggagaagagc acttcgccac cctcctccct
84181 tggacccgtc tgtccccata agcaagggcc aggactagga tgaggacaga gagccacgta
84241 gggcacaaat gaagggcaca aacagcaagt acacagccct gagggtgaca gcctccttcc
84301 ctttgtgcct tgggtccctc tctggcctca ctctagtccc tgctctgcct ctactcccca
84361 gcatactcca gccccctatg cctcagccct gcaaccccct cctaccttaa gtatggaccc
84421 ttctcttgcc acctctgggt gcaatgtggg gagcagctgg ggactgggag gatgaagcct
84481 gaaaggcctg aggcttggaa ccagtaggaa ggcctggcca gggtttggga aagaactggc
84541 aaaatttcat acagatgagg tcaggtggtg gggacaaggg ataatgctgg actgggcacc
84601 agcgtgcgtg tgacactgtt aacatgatag ggggagactg cttggggaaa atagttgctt
84661 tgggaaacac cagaggaaga tacctctcaa gcactaaatg gaggccaagc aaggtggctc
84721 atgcctgtaa tcccagcact ttgggaggtc aaggtgggca gattacttga ggccaagagt
84781 tcaagaccat cctggccaaa ttggtggaac cccatctcta ctaaaaatac aaaaattagc

84841 tgggtgcggt atgcattcct gtaatcccag ctacttggga ggctgaggca cgagaacccc
84901 cttgaaccca ggaggcagag gttgcagtga gctgtaattg caccactgca ctccagcctg
84961 ggtgacagag cgagactctt tccttaaaaa aaaaaaaaaa aaaaaaaaaa gcactagatg
85021 gagagagatt gagatcatca gttttcaagg gtgctggggg tgtaggccag cactgagcca
85081 gggagcaaag ccccagaagt ctggaaggca ctctgggagc aggaagaagg ccaggagaag
85141 gtgggccctg aaggccaaga ttggggggat tcaagaatgt ggggacagtt tccagtgtta
85201 aaaataacag ggccaggtaa cactggggtt ggagagtgtg ctgcggattt aggagctggt
85261 ctcgagacat caccaatgtc tatgtcagaa tggtttcaga aaagtggtac aagtgccgga
85321 tgcggtggct cacacctgta atgccagcac tttgggaggc caaggcaggc aaatcacttg
85381 aggtcaggag ttcgagacca gcctggccaa cgtggtgaaa ccccgtctct actaaaaata
85441 caaaaaatta gctgggcgtg gtggtgcgcg cctgtaatcc cctactcagg aggctgaggt
85501 gggagaatca ctggaacccg ggaggcagaa gttgcagtaa gccgagatca tgccactgca
85561 ctccagcctg gaagacagag cgagactaca tcaaaaaaaa aaaaaaaaaa aacaggttgg
85621 cgcggtggct tacgcttgta atcctagcac ttagggaggt ccaagacagg tggatcccct
85681 aagatcagga gttcgagacc agcctgacca acagggcgaa accctgcctc tactaaaaat
85741 acaaaaaaat tagctgggtg tggtggtgca tgcctgtaat cccagctact caggaggctg
85801 aggcgggaga atggcttgaa cccaggaggt ggaggttgca gtgagccaag atcgcaccac
85861 tgcactccag cataggtgac agagcaagac tccctctcaa acaaataaac aaaacagaac
85921 aaaaaaagtg gtacaaaaca aaggcagtcc acaaggctag aggagtgcag agaaagacat
85981 ggcagagttc ctgctaaaag agaagacagt tatttaagga ggtttgagat tcaagggctt
86041 ttttaatttt aaaattaacc atggtagaaa cctgtgtgtt aatatgctga gggaagttgc
86101 caatagtgag atgaaagatc taggcaaggg gatagcaagg tgactgcacc agatacctgg
86161 atctgggcag gaataaacct caacagtgat catgttgacc ttgagtgaca gggtataatg
86221 tttgggaagg tgctttgtaa actataaggg gcaatacaaa catgtagtac accttctctg
86281 agatccggga aaggacaacc tgagcatagg ttggggctgg tcataggcca ctctccccta
86341 ctgtttctct agagtgtgtg tagtccctag caagtctcag atgcctcgag tcgtggatgg
86401 aaacaaggtc ccaggaccat tgtcctgagc ctcctttctc cgtgctctac ctggggaatg
86461 cttgactatc aacccacaga agctctctct ggagcctcac tgcctaaaga gcttcttgtc
86521 gtaattgtaa cctgtcatgg ttaacccctt gcaagcatgc cctgcccctt gtctctccca
86581 agctttggct gctgcagctt ctttccacaa aatcccagca gatagccggg cgtcgtggtg
86641 cgtgtctgta atcccagcta ctcgggaggc tgaggcatga gaatcgcttg aacccaggag
86701 gtggaggttg cagtgatatt gcgccactgc actccagcag cctgggcaac ggagtgagat
86761 accccctcaa aaaaaaaaaa aaaaaaatcc cagcaggaca ccggcaggac aaggaagggg
86821 atgtttccct ggtaagcagg aactgaactg agaggaaagg tcacggtcct gaactagtcc
86881 aggtaactga gcatggtctg caaaggcagc ctcctggcat cagtgcacca ccctgagatg
86941 acagcctgtt gcctgactgc ctcgatctct gtcacctcag ccctcagtgc ctggggcatt
87001 atacacagta ttagcgtcat gagggggcca aacggactct acctgacagt tacatgtaca
87061 ggtacctcca acccacccct caacctgtgt ctctcccaaa gctaggaaca cttggggtct
87121 cagaggcccc aaccccagcc cattctatct cctaaaaatc tctagccagc ccctccatcc
87181 agcacccctg ctgggggctc ctcaatggac tttcttctcc tgcactccct cccccaggca
87241 gctgccaggg gattccttct aaagggcaaa gctgatcttg cccctgctct gtccaaatcc
87301 ttccatgagc aagaccaagt gcagtagaca gagttctggc agcatcagct ccaagtgtaa
87361 atcccagctt gactgattag gggtgcaacc tcatgcaggt tatttcacct cgggttctca
87421 cacctacatt gcaggcattt taagaagtaa atgtatgatg ggtgaaatgc ctggtattcc
87481 tcttggtccc cagaaccaga gcagagcttg cagcagggtc cccaaggact ctcctgatgc
87541 ccctcctcta tctaccctga cctgagcccc tctctacttc acacacaagc tcttgccagc
87601 tctctgtgcc catcctacat gcttctagac cagttcaaag gctggcctca ggacagcatc
87661 cctgatctcc accccccaga gggggcatct tggagcactt ggctgaggac accccaggga
87721 gatttcactc tgaggcctcc ccagggctgg cctataggtt tcccaggagc acagaggca
87781 gagtcctggg ggtctctggg ccctgcacca aggaaatgca gggcaaatgc ctgtgggaat
87841 gctagttatg ggcaatgcaa agaggcagtg aatccgggca gggagagcag gaggggtttc
87901 caggaagcag gactgaggga ggaaagggat ctgggggcac tgcagggctt ttggggtgca
87961 gagatatcca gaacttcttg gttgggatga gtgaggggag atccgtcttc cctcggtggc
88021 ctaagcactg gcagcggaat gggcccatca cctagctact ggctctgatg aagccagtgt
88081 ttcaggactg agggggccga gagcgccccc tccaccccga gcacacacac gcgcgcacac
88141 acgagcttct gctgggggtg ggaggggggg tcgccattca ccagcagccc acggcccgca
88201 gagccaactg atagagaccc cagccacagg ccacaggacc tgggagtccc tgcgatgggg
88261 gaaaccccgc ccgcacctcc ccaggctccc caccaacgcg tgtggccccg gggggcgggg
88321 cgcagaggga agaggtgaca taatccgagc cgcaccccct ctggccgcca cctcccccca
88381 ggccgggcga agcatccatg acatcatccg cctcctctcc gctcccctcc cccttgggac
88441 cctagcagcg ggacgcgccc cctacccagt ccgggtcagg tcagacatgc cgcgcagtgc
88501 agcaggcacg gcggcaggtc ggagccggga gggatgccac gatgggcctg cctcacttac
88561 tcgctgcctc agggtccggc gcggggtctc cgacacgggc gccaccggcc ccgcttgctg

88621 gcgcagcccg aagcacgccc ggcggggcgc gctgcgcggg cccggctccg ctccgcgccc
88681 ctcctgctcc ccgcccgagc cccgcccacc agcggctgcg gcaaacgcga gcccgccctc
88741 cggccacgcc ccccagggca gccaatgccc gggctggggc ggggtgaggc gccttaaccc
88801 cttcgtcgct gcgccccgcc cgcgcgtggg acccctcagc ctcaccctct ctagggcgca
88861 gcaagtccgc cgcgagcccc tctgctcatc gctcgttacc ccccaggccc ttgggcttgg
88921 gctcaggccc agtcgttcct ctgcaacaaa tccaatggaa ccgcgccctc cctgaatccc
88981 acgtctagga agaggaggag gagcccctct tgcttggggg aaggggctcc cgactcggag
89041 ccccgagccc cattcctctc ccgcgccagc tcttcgggac gcggcctccc agcggacgcc
89101 ctgagacctt gcaagtccgc tagggagggg tcggacgagg ccgtcgcggg tggatccgcc
89161 cggccgagca acgacccgcc cgggcgcgag gaccacgggc tgtcgtgcgg gaggcgtcga
89221 ggggcagtaa actccgttcg tgaccaaacg gagacgattt gaaacttgct gctcacactg
89281 gatcaccgtc tggaaggtgc gacggggaaa aatacacatt tacactccag caggattgaa
89341 cgctttcacc ctgtgccccg cgcctggcgt aacgcgtggc cccagaccac acgctgtaac
89401 ccggggtgag agggagtgag gtgggacttc gtaccggacc cggagcgccg gcctcgcccg
89461 cgcgggcagc gttcactgtg gggagtgctc actcagccta gggcggcccg ggaggactgc
89521 cgggaggagg gagtcggcct tgaattgagg cctcagcctt gaatagggta gggaggcaag
89581 cctagccgag agtttagcac tagcaaaagc ctggaggcac cagggtctgc cctaagaact
89641 gcagcgcctc tgctctggct gggatttaac gcattacgtc tctgctgttt ataggtgttg
89701 tgtttggtct tacgacttcg tattctatag tttttattta ttcctgtttt ctgaattttc
89761 ccacatttgc ttttatcttc tatcgtgatt ttcaaggcag aaagtatttt taattcttct
89821 cgcggctgcc tttagattta tcaaatgaaa atagtttaca tttcacacat tagtaaagtc
89881 agtgacaaat aattcgtttt tatattcccc catgagaaat tttgtgtgct tttctgcctc
89941 ctacctgccg ccctcactac ttatatttgt ccacttaatc aggacgacag acctaacttt
90001 tgtttacact ttttttttaa gccacaacat tctttttcaa tccttgtttt tggcttttgc
90061 caactctgtt taattggttt caatgttact gccggttttt tgtgctctaa tttttctatt
90121 cctcatttgg acccatctct cacatgactg gtgatttcag ggaggactct ggaatgttgt
90181 atttcctaag cctttgcctt aggtgagaat gcctttataa tgatttcctg tttataggat
90241 tcttgagtct caagctttcc ccctcaaaac tcttgtcttt atttcattgt cttctggcac
90301 tgaaaatgca aagctgggtt tgtctgttta aggtcagcag tttgcttggt gcttgcaagg
90361 gtttttcttt accctcaaaa gtcaaacatt ttgccagtat aaatcaatat ggccttatc
90421 tattaatttt ccagaaacaa aagaggtctt ctttgtagct tagaaaagtt ttcttcctgt
90481 ccattctgtg aaaagatttg ggtttttttc tttcctctcc tctaaaacta tcacattgga
90541 tcaccaatgt caaataaatc tgtttgtttt ctctgcaccc tgggagacta tatcaagttt
90601 ttcttcacca ttacggattt aattttcttc agagccaaga gtactgttta ttttctccaa
90661 taagacattt gattctgctt gttttatttt tctttcagcc aatttccatt ccacctctgt
90721 cttgatctca acttaatctc tctttgtagt ctccattcct atttcataga ctaatgtggt
90781 tttgcatctt taaaagaaca ccaggaaaat aagagaatac tatctatatc tgactctggt
90841 ccaatgcata tgaaaacttt tttgtttttt tgagacagag tctcacttaa aatttacaca
90901 aactctagca tagtgtgtaa ctattgaatg aataactaat gcaatcccct tcctttcttt
90961 cagagcaact atgaactaga cttttttttt tttttttttt tttttttttt ctgaaatgga
91021 gtctccctct ggaatgcaat ggtgcaatct cagcccactg caacgttcgc ctcctgggtt
91081 caagcaattc tcctgcctca gcctcctgag tagctgggat tacaggcatg tgccaccacc
91141 acgccgggct aatttttgta tttttagtag agacggggtt tcattatgtt ggtcaggctg
91201 gtctcgaact cctgaccttg agatccgccc gcctcggcct cccaaagtgc tgagattaga
91261 ctatttgtat ctaggttcat cagttcagtg aactaatcaa tactttgttt tgttttgttt
91321 tgttgttgct taagatgatt tgtcacctgc tgattaatga tcacacaagt tgtttttttt
91381 tgagagagtc ttgctctgtc acccaggctg gagtgcagtg gcatgatctt ggctccctgc
91441 aacctccgcc tcccgggttt aagtgcttct tttgcctcag tctcagtagc tggcattaca
91501 ggcgtgcacc attacaccca gctaattttt tgtattttta gtagagacag tgtttcgcca
91561 tgttggccag gctggtctcg aattcctggc ctcaagtgat ccaccctcct ccaccttcca
91621 aagtgctggg attacagaac tgagccaccg tgcccggccc tgaaaacctt gataaagggg
91681 ctcattttct gggaaataaa attacaagga taacaaagat attaaaaaca tcatccaaga
91741 aatatgtcca aaaaaggttc tggtggccgg catcgttgct cacgcctata atctcaacac
91801 tttaggaggc cgaggcagga ggatggcttg agcccaggag ttcaagacca gcctggacaa
91861 catagggaga ccctgtctct acaaaaaaat ttaaaaattg ggctgggcat ggtggcttac
91921 acctgtaatc ccagcacttt gggagaccaa ggcaggtgga tcacctgaag tcaggagttt
91981 gagaccagcc tggccaacat agtgaaaccc ccatctttac caaaaaatac aaaaattagc
92041 cagttgtggt ggtgaatgcc tgtagtccca gctactcggg aagctgaggc aagagaatca
92101 cttgaaccca ggaggtggaa gttgcagtga gctgagattg cgctgttgca ctccagcctg
92161 ggcgacagag tgagaccctg tctcaaaaaa aaaaaaaaaa aaaaattaaa aaattagcta
92221 ggcacagtgg tgcacacctg tgatctcagc tactcaggag gctgaggttg gatgatggct
92281 tgggcctggg aggttcaggc tgcaagtggg ccgtgatcgt gctactgcac tctagtctgg
92341 gcagcagagc aagaccttgt ctcaaaaata aataaatgaa attttaaaaa ggctctggcc

92401 tcagacagtt taatggaaga aatctctcaa actcactgaa tagataattt tcaccctact
92461 aatctgcatc agaacccaga aaagatagaa tgctgtctat ttcattttat gaagccacaa
92521 ttccccttat accaattcct aataatagca caggaagaaa ggaaaactga aaccatact
92581 catcagtcat cagttccagg ggtggaaaat cctacataca attcaagaaa ataaaactgt
92641 agattattct gtatgaaatt aataagccat gagtttgtca actgagaaaa aaaaacaatt
92701 atcataatag atgccaaaaa aatcagtcaa aaatgaatac ccaaaaagaa ccctcctaat
92761 taaccaggaa cagttgaaat taagaattat gtattaccgg ccaggcacgg tggctcatgc
92821 cttaatccca gcactttggg aggctgaggt gggtagatca cctgaagtca ggagtttgag
92881 accagcctgg ccaatatggt gaagcctcat ctctactaaa aatacaaaaa ttagctgggt
92941 ggggtggcaa gtgcctgtaa tcccagcgac tcaggaggct gaagcgggag gatcacctga
93001 acttgggagg tcgtgactgc agtgaacagt catcatgcca ctgcacaatg cagcctgggc
93061 tacagagtga gttgccatct caaaaaaaaa aaaaaaaaaa aaaaagccac gtgagatgtg
93121 agttcctgtt tccctacagc ctcaccagca taggatatca ttagtctttt aaatatttgt
93181 tttaatcacg tcttctttgc cccatagctg acggaggggt tcccactctc atgtgatggc
93241 agtggccaaa cacaacacct gacactgggc aggtgagatt aaccgcagtt tattagttac
93301 aacatactca atgggaaagg acaccacaag ccacgcaggg ccacacaggg ggtgtactca
93361 ggagtgaaga gaacaaccag gggttgtgga gggcaggctt tgtagtgata agagagtgag
93421 gtgctccctg gttcctgcat gggaggtaac tggcttgttt gaataattct acaggctggc
93481 agggaactga agcctactac taagagataa gcaggaactg ctcctggttc atttgatgaa
93541 aggattggct aggggacctt acccacagaa cacagcagga gggcagctta cagctagacc
93601 atttgaggcc ctcttgattt taccagaagt caagtcaata gttcttttta tttttacat
93661 ttttatttat ttatttattt ttgagacaga gtctcactct tcttgcccag gctggagtgc
93721 agtggcgcga tcttgtctca ctgcaacctc cgcctcccgg gttcaagtga ttctactgcc
93781 tcagcctccc gagtagctgg gactacggca tttactagca tgtccagcta atttttgtag
93841 tttttgtaga gacaagcttt tgccatgttt tccaggctgg tctcaaactc ctgagctcaa
93901 gcgatccacc tgagagctga gcactcccac agtgctgaga ttacaggtgt gagccactgc
93961 acctagcctc acatagcatt ttgagggtat aaattggtac aaccttttgg gaggacaatt
94021 tggtagtata taaaaatatg aaatgtgtat atccttggac ccagcaatct catttcttgg
94081 gaagagatac catcacaaat aatatgatgt atgtctgaag acagtcacta atgtactgaa
94141 tgtaaaagta aaaaaaaaaa aaaaacctcc taaatacatc aatctattaa tagaagattg
94201 attaaatgaa ttacaaggca tccaatgcaa tgtaacaata gacagctatt aatatttaaa
94261 aggacatgga tgcctatgtg ttgacacaga aagatgacta cagtaagtga aaaaagctgt
94321 ggcatatcat atgtatacct atcaacctac tgtactctaa tttttctatt attatttttt
94381 atttattgag cagcccccaa agcagagagt cctcctactc tttttttttt tttctttttt
94441 tgagatggag tcttactctg tcaccaagac tggaatgcag tggcatgatc ttggctcact
94501 gcaacctctg cctcccgggt tcaagtgatt ttcctgcctc aacttcccga gtagctggga
94561 ttataggtgc ctgccaccat gcccagctaa tttttattag aaatttttag tagaaacagg
94621 gtttcaccat gttggccagg atggtctcga actcctgacc tcaggtgatc tgcccgcctt
94681 ggcctcccaa agtgcctggg attaaagatg tgagccacca tgcccagccc ctctattctt
94741 tttcaaagaa tatatatgta cgtgcataga aaattcagga aagatacata caagattaaa
94801 catgggccac cttgagatgg tgggatcata ggtcatatgg cagaagaaag gagtctaggc
94861 actaatgtaa tttttgcact ttggcacaat cagctcactg cagcctcgaa ttcctagact
94921 caagtgatcc tcctgcctgc ctcagtctcc tgaggaggca ggactacagg catgcccagc
94981 ttccttttaa aaattttttt gtagagaagg ggtcttgcta agttgcccag gccggtcatg
95041 aattcctgac ctcaagcaat cctcctgcct ctgcctccca aagtgctgag ataacagatg
95101 tgagccacca cagctgggca aatatttaat atcttaatac tttgctattt ctacattttc
95161 agagcttacc tgtttattcg tccacataaa cttattttcc tttttgactg tctttaaaaa
95221 tatatttaaa atataaattt aaaaaatcgt atctcttaat actctagagt aagcagaata
95281 atgcccccca cccaaaatgt ccaaatccta attcccagaa cctgtgaata tgttgttaca
95341 tggccaagcg aaattaaggt tgtagataga gttaaggctt tgatcagcag accttaaaat
95401 aggaagatta gcctggatta tccaggcagg cccagtgtca tcacaagcgt tcttaaaagt
95461 gaaagaggac ggcagaaaga gtgagatgga gatgtgacta ggaaagaatg atcagagaga
95521 tgcaacataa ctggctttgc agatggaaga agggggccat gagccaagaa atgtgggtgg
95581 cctctagctg gaaaagaacc tccagaaagg gagcacagtc cttccttgaa agggagcaca
95641 gtccttcctt gattttagca cagaagaccc gcactggact tctatgctac agaagcagaa
95701 gataataagt ttgtgtagtt ttttgccact aagattcggt catttgttac agcagcacta
95761 gaaaatgaat acaaatacat aatagacaca acattcctta gttgaatgat gaatgaaatc
95821 acaaaaaccc taggtctaca gataaatagg taaaagaaat atatcactta gggccgggta
95881 cggtggctca cgcttacaat cccagcactt tgggaggccg aggcaggtgg attacctgaa
95941 gtcagaagtt cgagaccagg ctggccaaca tggcaaaacc ccgtctctac taaaaataca
96001 aaaattagcc aggcgtggtg gcacgtgcct ataatcccag ctacttggga ggctgaggca
96061 ggagaaccac ttgaacccgg gaggtggagg ttgtagtgag ccaagatgcc actccactcc
96121 agcctgggtg acagagcgag actctgtcgc aaaaaaacag atagatagac agatagatag

96181 acagatagat agatagatag atagatagat agatagatag atagatagat tttttatat
96241 atctatatat atatatatca ctatgaatcc agatagccaa taagtagttg aaaacatcta
96301 tatcctcgct gatactgaag agatgcagat caaaacctat caaattagga aagactattg
96361 caatcctttg gggtcctacc caccaagttg tccagaaggg gatctgggtt tacctctctg
96421 agatgactgc ttgcagttac ctctgaacag caaattaggg ggcagacaag gcactttaat
96481 tcatgctaaa gttcgaatgt ttgtactaaa gccttttata gctatgctta ctttatgagg
96541 aaatgaaaac ttctggaatt gggccaaaaa ttcctgggaa ttaccagtaa aggtcttggg
96601 ggttatctaa accaatataa aagtaactga aatgattata atccaagaat gctggggtct
96661 tttattaccg tttgataagc tgatgtgttt tttgtttgtt tgtttttttg agacagagtg
96721 tcaccctgtc acccaggctg aagtgcagcg gcacgatctc ggctcactgc aacctctgcc
96781 tcttgggttc aagtgattct cccgcctcag cctcccgagt agctgggatt acaggcaccc
96841 cccaccatgc ctggctaact tttgtatttt tgtagagatg gggtttcacc atgttggcca
96901 ggctggtctt aaactcctga cctcaggtga tccacccacc ttggcctccc aaagtgctgg
96961 gattacaggc atgagccacc gcacccagcc gagctgatgt ttatacacac acacacacac
97021 atggtccatg aaaaataaaa tttaaggctg agtgtggtgg ctcatgccta taatcccagc
97081 actttgggag gctgaggcgg gtggatcacc tgaggccagg agtttgagac caggctggcc
97141 aacatggtga aaccccgtct ctactaaaaa aaaaaataca aaaattagcc aggcgtggtg
97201 gcaggcgcct gtaatcccag ctactcggga ggctgaggcg ggagaatcac ttgaacccag
97261 gaggcagagg ttgcagtaag ctgagatcgt gccattgcac tccagcctga gagacagagt
97321 gagactccat ctcaaaaaat gaaaaataaa taaataaata aatataaaag ttaaaggaaa
97381 aatatacaaa aaattaaaaa ctgtattctc tttcttaaaa ctattcttta ctaattgcac
97441 attttaccta agatagaaaa tggtttatgt ttttcctgtg ttccacaggg tccccctgta
97501 acatctgatt ctgtttttat ataattgatg cattgtttat atgatactaa gggactcatc
97561 tgtacctata ccctgtaagc ctcatttcaa gatatttaga tacaaagcga aaaggaacac
97621 aagtcaccac ttcaagtgag tgcctctgca tcatacatgt acatgtgaaa catgtaggtc
97681 cttttttttt ttttggttag atggggtctc actctgttgc ccaggctgga gtacagtggc
97741 gcaatcacgg ctcactgcaa cctccacctc ccaggctcaa gtgattctcc cacctcagct
97801 tcctgtagct gggactacag gtgcacacca ccacgcctgg ctaatttctt ttttttggta
97861 tagatggggt ttcaccatgt tgcccaggct gatcttaaac tctggggctc aagtgatcca
97921 cctgcctcgg cctcccaaag tgctgggatt ataggcatga gccactgtgc ctgaggcggg
97981 aggatcatgc tgaaactcca tctctacaaa aatacaaaaa ttggtcgggc gtggtggctc
98041 acacctgtaa tcccagcact ttcggaggct gagacgggag gatcacttga ggtcgtgagt
98101 tcgagaccag catgaccaac atggtgaaac catgtctcta ttgaaaatac aaaaaaatta
98161 gccaggcata gtggcacacg cctgtaatcc cagctacttg ggaggctgag gcaggagaat
98221 cacttgaatc caggaggtga acgttgcagt gagctgagat cacgccattg cactccagcc
98281 tgggcaacaa aagcgagact ctgtctcaaa aaaaaaaaaa accccacaaa aattagccag
98341 gcatgatggc aggtgcctgt aatcccagct actcgggaca ctgaggcaga agaattgctt
98401 gaacccagga ggtggaggtt gcagcgagcc gagatggcac cactgtccag cctaggtgac
98461 agagcgaaag tccatctcaa aaaaaaaaaa aaaaaaaaag acaactgccc aactagacgg
98521 caagcacatg tccgcacaac tacactatta tgtgaacaac acagggaacc gtggagttta
98581 gctcaacaag gtgacttgac ttaccttgct gagaaacagc taaagggaaa tctcttacag
98641 caattgagga attaagtctc agttactcag ttctgaacag ttagaacatt aagagtttta
98701 gttttattat attatagatg tgtttttgtg tcatatggta gaaatgtaaa tagtaaatgt
98761 aaaatagaaa gttaaaatga ctaaaaaacc agtcagcgta aatctaccaa gctatacgca
98821 aaaggttata tttaaataag tttaatgtta cagtattggt atttggtagg ttttcttata
98881 aggttccctt atccattttg ctgtaattaa caatcggata atgtattagc cagccaaaaa
98941 ttatgtatgc tgtcaggata aatcaagtca aaatgttttt caatgtcact acagaaattg
99001 tttttggttc aactatatga ctatcaaagg ttatgtacaa cgcacaactc tacccagata
99061 ctcatccttg cactgttcta aataaaggaa acaagactct tcagtactct gtttttcaaa
99121 ctgtaggtca agatctgtta gtgagttgta aaatcaattt agtcaccatt ggcaataaaa
99181 tagaatagag aggcaagtat caacatgcat caaaccaaaa ggattgtact gtgaaacgcc
99241 tgcttcatgg atctatatgc atacatgcac acataagaaa atggatttct tactaagaga
99301 tactgttaaa agagtctgaa aacacagcct tagtggaata gctgtctcta atcttggggc
99361 tgaggcatgc acgcatgaag acttgaggga tattctcaaa tagtccagtg gcttgtggag
99421 ggagaagttg atggtataat gacaatttgt gggataaagt gatgtaaagg tttaagaaat
99481 gagttaacag attcaaaaca ctaagtaaat aaacattccc acttcttccc cacccagact
99541 taacaaacat agctcagatc tctgggctca gaccttccca gccaggtccc agcctccttt
99601 tccagctttg tctctcattt gccctggcct ttcctccacc caacactcca tgctcccctc
99661 agggaaccac aggccttatt ctccactcca gatctctgca cctgtcctcc ttctagcctg
99721 gaaggcctct cctattatcc actggtccct ttcatacact ccagcatctg gctgtgcact
99781 tgctagcttg tgattgattg gtctctctgt cagattaata ccactgcact ggccgggcgc
99841 ggtggctcaa gcctgtaatc ccagcactta gggaggccaa ggcgggtgga tcatgaggtc
99901 aggagatcaa gaccatcctg gctaacacgg tgaaaccccg tctctactaa aaatacaaaa

99961 atattagccg ggcgtggtgg cggacgcctg tggtcccagc tactcgggag gctgaggcag
100021 gagaatggcg tgaacccagg aggcggagct ttcagtgagc tgagattgca ccactgcact
100081 ccagcctggg cgacagagcg agactctgtc tcaaagaaaa aaaaaaatac cactgcacta
100141 acccctactt gtggctgggg ccatgttttg ctcactgtac ttccctaggg tgttcaaaat
100201 catgccttgt actcagtaag ctccccataa attacgttat tttttaacaa catatttcag
100261 atgtgtttaa atacttttct gttacaaaga ttcaatacag gctgggtgca gtggtacacg
100321 gctataatcc cagtgctttg cgaggccaag gtaggaggat cacttgagcc caggagttcg
100381 agactaccat gagcaacata gtgagatccc gtctctacca aaaagagaaa aaatatatat
100441 atatattagc tgggcatggt ggagtgctcc tgtagtccta gctactagaa aggctgaggt
100501 gggaggatca cttgagccca gaagtttgag gcagcagtga gccatgatca caccactgta
100561 ctccagcctg ggcaagagtg agactctgtc tcaaaaaaca aaaatccaac atgcatccag
100621 tatcagtatc actgtggtag cctgggggaa tcactgaact gccaccatat taccggtggc
100681 ttcacataac ccagctactt tagctccaca taacccagct attttagctc cttttcttga
100741 ggtttagcat gagccaagga tacacaaggc catctaggac tctttatgtt ttgttataga
100801 aaagtttaaa catgtaggaa aatagatggc tgggtgcagt ggctcatgcc tgtaatccca
100861 acactttggg aggccaagat aggtggattg cttgagctca tgagttcgag accagcctag
100921 gcaacatggc aaaaccccat ctctacaaaa agttaaaaaa ttagccgggc atggtggtgc
100981 acacctgtaa gtcccagcta ctcaggaggc tgaggtgaga ggatggcttg agcctgggag
101041 gcagaggtgc agtgacctga catcgtgcca ctgtactcca gcctgggcaa cagagctaga
101101 tcttatccta aaaacaaaaa caaggccagg cgcggtggct catgcctgta atcccagcac
101161 tttgggaggc cgaggcgggt ggatcatgag gtcaggagat tgagaccatc ctggctaaca
101221 cagtgacacc gcgtctctac taaaaatata aaaaattagc cgagcgtggt ggtgggcgcc
101281 tgtagtccca gctacgcaga agactgaggc aggagaatgg catgaacttg ggagacggag
101341 cttgcagtga gccgagatca cgccactgca ctccagcctg ggcaacagag caagactctg
101401 tctcaaaaaa acaaacaaaa aaaccagaaa gaagaaagaa aatgagcaca acctccatgt
101461 acccattccc agtttttcag taattttcag ctcatggtta atcttgtaaa taactatttt
101521 ttgaaaaatt gtgtaatata actccaatct agaacttgtc aataataagc cttgaagatc
101581 cacaagaaag agaagttact ggactctata gggcgcaagt agtatcacaa tagacctgtg
101641 tagggctggg ctgtgaagat aaggcaccca ggacaagcac tgcttgagag ttacaaaaat
101701 caaaatgtat ctgattggtt ggagccaaga gtcaagggtt ccaaaatcta actatgaaaa
101761 agggaccacc aggctgggca cggtggctcg tgcctgtagt cccagcactt tgggaggcca
101821 acatggtgaa accccgtctc tactaaaaat acaaaaatta gccgggcatg gtggcatgtg
101881 cctgtagtcc cagctacttg gaaggctgag gcagaatcgc ttgaaccctg gaggcggagg
101941 ttgcagtgag ctgagatcgc gccactgcac tccagcttga gtgacagagt gagactgtct
102001 caaaaaaata acaatactaa taaaaaaaga aaagaaaaag ggactaccca acaagtaaaa
102061 gactcaggta gaattccaat gaagttggct gggcgtggtg gctcatggct gtaatcccag
102121 cactttagga ggccaaggcg ggtggatcat gaggtcagga gttcaagacc agcctggcca
102181 acatagtgaa accccatctc tactaaaaat acaaaaaatt agcagggcgt ggtggtgggc
102241 acatgtaatc ccagctactt gggaggctga ggcaggagaa tcgcttgaac ccgggaggtg
102301 gaggttgcag tgagccgaga tcacgccatt gcattccagc ctgggcgaca gtgcgaggct
102361 gtctcaaaaa aataaaaata aaataaataa aaattggccg ggcgcggtgg ctcacacctg
102421 taatcccagc actttgggag gcagaggcgg gcagatcacg aggtcaggag atcgagacca
102481 tcctggctaa cacagtgaaa ccccgtctct actaaaaata caaaaaatta gccaggtgtg
102541 gtggcgggcg cctgtagtcc cagctacttg ggaggctgag gcaggagaat ggcgtgaacc
102601 tgggaggcgg agcttgcagt gagccaagat tgtgccactg cactccagcc tgggtgacag
102661 agcaagactc catctcaaaa aaaataaata aaaaataaat aaataaataa gtaaaaatga
102721 aaaagaatta caatgaagtc ttgagtcctt ggttctgaat catcatccat cagtcatagc
102781 tatgtcctca gctatcacgg taaccctggc gctccagagt aagtgaaaac taccctcaga
102841 caaaaataaa aattaaaggc caggtgtagg ggttcacgct tataatccca gcactttggg
102901 aggccaaggt gggcggatca cctgaggtca ggagtttgag accaacctgg caaccatggc
102961 aaaatcctgt ctctactaaa aatacaaaaa attagccagg catggtggag ggcgccagta
103021 attccagcta ttcaggaggc tgaggcagga gaatcacttg aactcgggaa gtggaggttt
103081 cagtgagctg agattgcgcc actgcacccc tgcctgggcg acagagcgag attccctctc
103141 taaataagtg aatcaataaa atttaattag agctagcaag gctgggcaca gtggctcatg
103201 cctgtaatgc agcactttgg gaggctgagg cggggggatc acttgaagtc aggagtttga
103261 gaccagcctg cccaacatgg cgaaaccccg tctctagtaa aaatacaaaa attagccagg
103321 tgtggtggcg catgcctcta atcccagcta tttgggagga tgaggcagga gaatcactga
103381 acgtgggagg tggaggttgc agtgagccaa gattgcgcca gtgcacttct gcctaggtga
103441 cagtgagact ccaactcaag aaaaaaaaaa aagtagagct tgtgaagtgt ttatgatcag
103501 ttttagtgtc acatgtacag atcagtaatg taatcattat aatactatgt ctggtctacg
103561 gctgggcgcg gtggctcacg cttgtaatcc cagcactttg ggaggctgag gcaggcagat
103621 tgcctgaggt caggagttcg agatcagcct ggccaacaaa gtgaaacccc gtctctacta
103681 aaaatacaaa aattagctgg gagtggtggc acgctcctgt aatctcagct actcgggagg

103741 ctgaggcagg agaatcgctt gaacctgggt ggtggaggtt gtagtaagcc gagatcacta
103801 cagcctgggc aacagagcga gactgcctca aaaaaaaaaa aaagcattaa atttcatttt
103861 attttcattt ttccctttat tttttcattt tttagttgtg aaagtagttc gtatatattt
103921 agaaaaatgt ttacttttaa taaactattc atttaataaa tgtattaact gagcactaaa
103981 taatgctcaa aggatcatag atttttttcc tccacactaa gcccatggga catgaagaat
104041 atctgggtgc ttccaggata taaggaagga atgtgccttt tcccactcct ggcctttggg
104101 tactgtcctg agcatgggat gctggggcag gggctgcctt ggccatcttg ccatctgcat
104161 ggcagtggca gaggatagct gagggaaagg actagagagc cctggagctg ccgccttgct
104221 ctgtgagaag ccccttccaa tggagatttg aattacttgc aacttgttgg cctgcctctc
104281 caccccaccc ccatctgttc cctagctctc aactttggct gcacactgga atctcctact
104341 catgctccct gactatgccc tcacagattc tgatttgact ggtcctgggt gcaacggggc
104401 tggcatgaga cgactaaaaa ctccccaggt gaatccaatg agcaaccagg ctaaggacca
104461 gtgatctaac tgtaagtcat tctcttcttt tccctttgta attttatgtt tccacctcat
104521 actgtggttg gctacatctg tttcttccct ctcctactag aatgtaggct ccagatggcc
104581 ttccaaacaa tacatgtggc ccttcacgct ccaggtgatg acaagtaccc gttgctgatg
104641 gaagtagcag agtcatacct ttcttggaat ttaggttcta cagtcaatgc ttatggcacc
104701 caatgagtca aaaacaccct tggcaactcc taaggaaggc tctgccctgg aacctgatgt
104761 actgtttccc catcaccatc cagggagagt ttcctcctgg gccggaacag aaagctgcct
104821 cctccatcag gcaccagagg aagtaggggg cttctgttta gggaccactt tgcataaata
104881 cacatttaaa catgtgcctc accctcagca cagcaggatg ctcacgccat gaacagccaa
104941 caggaaccag agtgaccgac cctccaggag gtacacacct ccctcccatc ccacccactt
105001 ctgagcatat ggttctcaca tccaccagtg ggtggaatca cccacaccat ttaaactttt
105061 tgtcaaggat gtagagctga attcatgtga tttcccacat gagcaatgtc cctccctcca
105121 ctgcacaatt atagccagct caagcaggtc ctctccttcc ttctacctgc cctgcaaatg
105181 gctctctcct tcactgaccc cccgccctcc ttcttaaagg ctgatgctcc ctaggactct
105241 atccttgatg cttctcatta attcttctcc ccgctggctc tcagccctac ttagggctcc
105301 tgatcagtta aaaaagaccc atgagacctc ccctccccag ttaacagaga tgtggcccaa
105361 gaggctcccc acttctaatt tgagtggggg caaaagaaga gaaagatctt atctaggtct
105421 tgggggcagg gcttgcaggg cacaaagaaa cagagtaaat gtgcaacagg ctattttctt
105481 gaatgaatgg attcataata agaacaggac ttcaccagct tccaaggacc cctccccacc
105541 tctcggatat attctgaagc ctaaatttca cctgcttatc taggatccta ggccaggcct
105601 tctgccacac caagccacac caagctcagc agtactcaca ggcctttaat ctataactgc
105661 ctggccacac catcacttcc ccaggatggt ggggagggat gaagatgtat agccaggtag
105721 gccacaccaa ctctgagtcc aagagggaag gactggggtc aggctggggc ttgggcaggg
105781 gactcccggc cccaggcgat gagggagttg ctggcttggg caagctcggc gatggacacc
105841 cggccccgct gtcggatgaa gttggccacg gcggccagtt cctctggggt tatgtagatg
105901 aacttgcccc ggtcgtcaat cacacctgtg gggacatgca ggttgtgggg ctgaggcctc
105961 ctgggcgttc cccatctggc ctgagaatgc ccacctactc cttcacagct ggctcagggc
106021 ccttccggcc tcctgcctgc ctggactttc ctggctgcat ccagtcctgc taggagagtc
106081 gaatcaggct gccctgcctg tcctctccca ctgctgccac ctccaggccc tggcatgtcc
106141 tccttgtaac tgcatccagc tactcagtgc ccctccttgc acacccctcc ccccatcttg
106201 ggtggtcatc ctgcctctgc agcacatccc tgcagcccct gtggctagac actctccaag
106261 gccaggactg cagtgattgg ctctcatcat ctcacctgtt atagtcccct cagccagcag
106321 gtcctggatg cgatttatgg tgtcctatga ggagaacaac tctcagaata gagataggca
106381 ccaatgtccc cagaaaaagc actgaaacct ccctcccact acagccaaat ctctttattt
106441 ccctctcatg ccacgcccaa atgtgactct gtgctttgcc tttggaaggg ccctgggtag
106501 aaggggctct ggtcctgggc cctgcaagac cccaaaaaga gtatctcttc ctgttagaag
106561 cggggcagtg atgtttgcaa cacaggctgg tgcagtctgt gcatgagagt tgtcaaggta
106621 actgatttgg gctgctttgg tcacaggggc tggcagagtg atggggtttg ggtttttttt
106681 ttctttcctg cctgttcgtc cattaaaaat gcaggattcc cagggtgcca ggagacttct
106741 gtgcactact cactcttggt tggggacaag gtagacggtg catcaagact ctatctttag
106801 ggcaggtcct ctctgctagc cacagacccc tggccacact gcacacagca ggccacgttc
106861 cagggcttac ctgagtgcgt aggcccacct gggaagccag gtcttccaag agcacaacct
106921 tggactgcta caaaaagaag gaggaaaaga aagagaggct gagcttgggc aaatccctct
106981 aagcatgggc accctccagg tttgcattct gatctgatct gttctcggtg gctggaaaaa
107041 cactgtacac actcctgtag gacagccttg ctcccctgac acacacacac acacacacac
107101 acacactcac tatcacccat tgacagagac ccgcacaatc ataaaccctc tcattcccct
107161 tcttgcgagg aaggaatcta cccaaaacaa ccacctgcct catgggccct gtcctggact
107221 ccgagacaca cgagtggtaa tcagagcaca gcagccacca tcctctgcat ccagccatct
107281 ctttttaaca tcatggacaa gttcaagggg acgcaccata gggaggaagg aggcggtgag
107341 agtcaccaaa agctatttta aataactgtc accaggactt atgcttgctg aggacctact
107401 agaacaaggc cctgagaagg taggagggac acatgctgaa caagccatag tccccgccct
107461 ggagctcgca gtccggctgg tgagacaggt gagtacatgg acaagaggga gagcagataa

107521 aagcccgagc aagccaaggt ctctcagtag aggctgtggg ggcccactcc tgccttgcct
107581 tcgctggccg ccccactggc ctggtccccc acaacctgac acacccacac tttttatcca
107641 tcctctttat accatctgag gcactgagcc cacccttgaa atcactgtca ctaataccgc
107701 tgagtgcttt ccagcacagc cacgttctgt gctccaagtg cttcttcata ccctcagtag
107761 aaacctgagt tcccaagggc gcagcgaagg gtccaccgct gtgtccacac aggcacctgc
107821 ccccactacc ccaacacaca gcacaggact tgagaaaagg cagttctttt gcatttctgt
107881 gaatcctcca ccgcaaatga gctgggggag gactgacatg gtgacacctc cctgactgca
107941 gatagcatgt gacttgtttg aaatgccaaa gccgtatgta caagcaagaa ggcaagcgag
108001 aggcagctgg gtcaggatgg gcaaggacaa agtgacgtgg tcagctggtc tctacctaca
108061 aaacagggat caggcagcgg gctctcagtg acagtgtggt gcgagaacat gaagtcaccc
108121 caggtgctcc ctgatgacct ggcctgaggt gctgctgggg ccaaaacacg tttgataaaa
108181 caagctaggt gtggtggcac ccacctgcaa tctcagctac tcaggaggca gaggcaggaa
108241 gatcgcctga gcccagccat ggtcagacag gagaatggaa caagatcgag tgaaccctac
108301 acctctcata aacgatgctg ggtgcaggca tgttcctcca gttctgaaat cttaaggcaa
108361 aaaaggtcta tccccttgga gcaaggagga aagcagacag actgaatttc agaagtcctc
108421 cttttccttg caagacagca gctctagaag aattagccag cacaggctgg aaaaactttt
108481 tttctttttt ttttgagaca gagtctcact ctgtcgccca ggctggagtg cagtggcacc
108541 atcttgcctc actgcaacct ccacctcctg ggttcaagtg attctcctgc ctcagcctcc
108601 tgagtagctg ggactacagg tgtgcaccac catgcctggc taatttttgt atttttagca
108661 gagatggggt ttcagcatgt tgcccaggct agtctcaaac tcctgacctc aagtgatctg
108721 cccgcctcgg cctctcaaag tgctgggatt gcaggcgtga gccaccatgc ccggacaagc
108781 tggaaaaact tctaagctcc aaaagagttt ggagaactcc cagagaatag ttacctccag
108841 ggagccacca aaagtgaggg ctagaactgg ggacctttct tctggtttct gcagagtaaa
108901 gggggatgtt gaagctgacc ccctcctcta attccacctg ggaaggcttc ctggggaggg
108961 gcttcaggac catcttactg cctactgggt aggtggggac aagttccaaa cagcagcagg
109021 caagtgtgag aaataacctg cccggccaca gcccctaatc aagtgtgcca gggtagagag
109081 agggctccat gagaggggag tgcacacagc ctgaacagac aggaccctct caaagctgcc
109141 agcatttccc ccacttaaga accccagact caaggctgtg agcccagccc ctcgctgctg
109201 gcaggcgtct aaaccctgct ctctttcagg agaggtggcg ggaggattat gcaagccctg
109261 ggtccttggc agcccctggt agcaccccca ccaccgcccc cgctccctct gcactccttc
109321 cctcagccgc ctctgcacct ctgagttgca cttgaaaacc tctcccctca tggcatcctt
109381 cgtgggagag aagttgctta gcagcaaagc aaagtaggat gtgcttatgc aacctggagc
109441 agcatcgagg cggcctgcgc agtggctgcg gacagaaggg gacacactgc cgccccaccc
109501 taaactccaa aggaaacacc atcctgttcc ccctgaaaga cccaagttcc cagtcaggag
109561 cacatggcag aggatgcttg tttcaactca gtggagcctc agtgtgacag aactgacaga
109621 aaacacatgt gaagagactt gaaaacacac tgactcactg agcgtggggt ctgaagagca
109681 gggaaactgt caaactgagt gtgaggaagt gatcaaaggc ttgcgcctcc cacacagacc
109741 caggtccttc tccaccttcg ggttggaggg gagagaaact ctcctggcca ggaccctgcg
109801 gctctgcccc tctgggcccc cctgtccact cctgcatgag cctgaatgcc ctgcctgcag
109861 cccccgctgg aggcatccaa cctgcacatg ccaggcaggg agctgacact caggctctgt
109921 tcagtggctt cttaccttga tgtagttgat gaactctgtc aggaagctct gggactagag
109981 aagcagagaa atcagggtga gcacccccta gcaagcccac agggttctgt acccattcac
110041 ccaagtacca cctgctcact tgagggccac ctgcctgcag cctgcctttg gcccgcccaa
110101 ccacctgcac ataccgcttg cctacacaag cagcagagcc ttccagggtg agagtttcgg
110161 aggcaccagc ctctgctgag gacaaggcct ctggccacta aagcctgctg gtactgatgc
110221 ccacccacct ctcactgcct ggccaggggc gtctgggatg gggtggctag ccttgcgtct
110281 ggcacaggct gcactgatgg gtgcagagag gggcttccca ggggcagcag gcccacctgt
110341 tcctcagtca tggtctctcc tacgccttcc tcctccacca caaaggcctc cttcagtttc
110401 aggtactcct catgctcccg ctgggcctgc tcctcgcggg ccttcctctc ctcctcctcc
110461 tgtggacata ggaggcaaaa gtcaggtatc gggggcagaa cagggcaggc acctgttctg
110521 gttgctgcta cccctgcagg acaccagagc atatagccca gccttttctc agagacagag
110581 ctccttcagg acccccattt ctccattccc ccaagctgaa ccatgggccg aggcccctct
110641 tgtcatcggt ttcatcatca cctggacaaa tgctgctcag ccccttctcc cgccctccgt
110701 gctcagggga tcttagcaaa aatctgttga aaaccctgcc atgaccccac tctacaatac
110761 cctccaatca cctgccccat cctctccttg tgtaactcac caagcatacc ccgtcatgtc
110821 cacacctcat aagcaaatgt gaaccctgca tttcctcacc acccactcac ccagtcagcc
110881 cactccagtc tggcttctgt gttcccagtt ctatgaaaca actcctgcca cagtctcacc
110941 catgccctcc actcaccaaa ctcaaggtac aaccttcagg tctcatatgt ctgtcctctc
111001 agtgatcctt ctttcctccc ttggcttcca gatcccaccc tctcctggat ttcctcctgc
111061 tccttgggcc attcttcccg cctccttcat cattcagccc tgcctcttct ctctcctcca
111121 cattctctgg gccacctcgc ccatgcccaa agcatcaacc ccttccttgc cagtgactac
111181 cacacttgta gccccacccg aatctcttct cccaaacaca caacggtcca tgtgacacag
111241 ctactgggcc atctcacagg cagcagacag accctgggac ctactcttct acgtttagac

111301 ctcctcctag gtccctggct ctgtgaatta acacagccaa ctggtcagaa atgaggtcct
111361 attgacccct tctttctcta acttccagaa aactccctgg tctgtctatg ttgctccatc
111421 accactactg cctggtaaaa cagcctccct caacccaaca cattttccag agcactcaga
111481 aaaattttta aatggaaagc tagccgggcg tggtggctca cgcctgtaat cccagcactt
111541 tgggaggcca aggcgggcgg atcacgaggt caggagatcg agaccatcct ggctaacacg
111601 gtgaaaccct gtctccacta aaaatacaaa aaaaaaatta gccgggcgtg gtggtgggcg
111661 cctgtagtcc cagctactcg ggaggctgag gcaggagaat ggcgtgaacc tgggaggcag
111721 agcttgcagt gagccgagac tgtgccactg cactccagcc tgggggacag agcgagactc
111781 catctcaaaa acaaaaacaa gaatcagaaa gctaaagctg aggcaagtaa tatacaagat
111841 gagtctgggg catcttgcag tgccagaaac taagtgctca aaaaacaaaa cgagtgtgtg
111901 ttaaaggtac acaggagcca actgaaaggg ctcccaatgg tcaaagctgg aacaatctca
111961 acaacgtaat aaataacgtt actggattat aacacaaagt acaaaataaa tactcatgag
112021 tctatacaaa tataaaaaaa atggctggtc gggcacggtg gctcacgctg gtaatcccac
112081 cactttggga ggctgaggca ggcggatcac ctgaggtcag gagttcaaga ccatcctggc
112141 caacatggtg aaacctcgtc tctactaaaa atacaaaaat tagctgggtg tggtggcggg
112201 tgcctgtaat cccagctact cgagaggctg aggcagcaga atcgcttgaa cctgggaagc
112261 ggaggttgca gtgagcccag attgcgccac tggactccag cctggatgac agagtgagac
112321 tccatctcag aaaaaaaaaa aaaaaaaaaa aagactgcat gaatatttaa tgaataggga
112381 aggagagaca tatctcctgt aagagatttc catataattt atgtaagaac tctaactccc
112441 caacccttaa gtgtgggctg tgcacagtga cttctttcca aagaaatacg ggaagagggg
112501 aaaaaaacag tcacattaca gtggagaaag ctggcaaaca aaaccgcagc caggcgacca
112561 aggttaacaa cattagggac aagtgaagcg gataacttgc atatttggta tgttgcattg
112621 aagatggaac ttcacttatt gtcttcttca gtcagatcca taaccccagt ctaaccatga
112681 gaaaaacatc aaccaaactc aaattaaggg accttctaca aactacttgc ccaggactcc
112741 tcaaaattgt taaggctgga cacagtggct cacacatgta atcccagcac tttgggaggc
112801 tgaggtgggt ggatcacctg aagtcaagag ttcaagacca gcctggccaa tacggtgaaa
112861 ccccgcctct accaaaaata caaaaattag ccaggtgtgg tggtgcacgt ctgtcatcct
112921 agctgcttgg gaggctgaag caggagaatc atttgaaatc tgaaggtgga ggttgcagtg
112981 agctatcgcg ccactgcact ccagccttga gagcaagact gtatctcaaa aaaaaaaaaa
113041 aaaaaggtta agatcatcaa aagcaaggag tcttagctgg gcacagtggt gcacacctgt
113101 agccccagct actagggagg ctgaggcagg aggattgctt gagcccaaga gtttgaaggt
113161 gcagtgagca ctccagcctg ggtgagagtg agactccatc tctcttaaaa aaaaaaaaaa
113221 aaaaaaaaaa aggccgggca cagtggctca tgcctgtaat cccagcactt tgggaagcca
113281 aggcaggagg atcacaaggt caagagattg agaccatact ggccaacatg gtgaaaccct
113341 ctctctacta aaaatataaa aattagctga gcgtggtggt gcacctgtag tcccacctac
113401 tcgggagaat gaggcaggag agtcacttga acccaagagg cagaggttgc agtgagccga
113461 gatcgtgcca ctgcactcca gcctggagac agagagggac tctgtctcaa aaaaaaaaaa
113521 aaaaagcctg gtgcagtggc tcacgcctgt aatcccagcg cttcgggagg ctgaggtggg
113581 tggatcacct gaggtcggga gttcgagacc agcctgacca acatagagaa accccgtctc
113641 tactaaaaat acaaaattag ccgggcgtgg tggcacatgc ctgtaatccc agctacttgg
113701 gaggctgagg cagcagaatt gcttgaaccc aggaggtgga ggttgtggtg agccaagatg
113761 atgccactgc actccagcct aggcaacaag agtggaactc cgtttcaaaa aaaaaaaaaa
113821 aaaaacaaaa cagaaattct gataaactgt catgacacag aagagcctaa ggagacatga
113881 ccactaaatg taacatggca tcctagatgg gaccctggaa aagaaaaagg atactgggaa
113941 aaaacaaata aaatatgaat aatggggtca ggtgtggtgg ctcacacctg taatcccagc
114001 actttgggag gccaagggaa gagaattgct tgagtctagg aattcaaaag cagcctggga
114061 aacacggtga aacccaatct ctaccaaaaa aaaaaaaaaa aaattagcca ggcatggtgg
114121 tgcacaccta tagtcccagc tactcgcagg gacggggaac tgaagtggga ggattgcttg
114181 aggctgggag gcagaggttg cagtgagcca agatcgtgcc attgcactcc agcctgggca
114241 acggagcaag accctgtctt aatcaatcaa taaagaaaaa tctgaataat gttagagttt
114301 agttaacaat aatgtgtcaa tattagttgt gaggaaggta ttaaagtgag gtaggacgtt
114361 aaggataagg gaaactggtt acaggggaga agagccagga tatttaaggt acattaagcg
114421 ggaccaatat tcagaaagga aaaaaccctc tctgctaatg cctcccccat gctgtattct
114481 ggcttatcag ccacagcacc cacatccagg cttcctgcca tccaggatcc tgggacaaca
114541 ggtcagtcag aggtcacagg tcttcccagc tagggcagcc ctggctctaa ccctatgtac
114601 cccacatctt tatggtgttc tctttttggg aaacctggtt ctcagaacaa agaaagcatc
114661 tggcaaacaa atgttcatta gtcactactg cagtggaccc cattccctga gcaccttctg
114721 tatgccaggc cctcgatgta agaaccatct cttacaatga tcttcctcag cctcaaggcc
114781 ttcaggatcc agacccccat cacctctctg ccatcttctc ctcctctcct ggctttctct
114841 gcccagctat cctcctcgcc tcgcgttcct cacatgctcc ccgaaagttc aatagtcacg
114901 tgcacaaaaa tccccactcc aggctctgct tttcagcagt ggcttttcta ggctgtggtg
114961 gagcctaggg accaaataaa ccaggacctg cccttgtgcc aggtctatgg cccaccttct
115021 aggttggagg tgccagggag ctgcataaga agcaaggctg ccttgcctgg tcaaggtca

115081 gaggtcaggg taggggctgg cctcaccttc tgctcctcct ccaggcgaag ccgctcctcc
115141 tccttcttcc actcagcttc gcgctgggac tcgagtcgtt tccgctcctc acgttcagcc
115201 tcctctgcct ggagagaggt cttcataggg gcacatccct cacccccggc tagagtgtgc
115261 ccaccgccca gacagactag ggaaggcaat gcctgggcca gggcacagac ccaggcggca
115321 ggagacagag ccagggaagc agcaaacacc agcccagcag gcaacaagcc ctcagtctgc
115381 cttagggaaa aaggaaggca gtgccctttc gcacttccca gagctgcacc ccatccctcc
115441 ggcttgcctc acgctgggcc tttcgcgctt gtttctcctc cagcttccgc agtttcttag
115501 ctccaatttt ccccgacagg tgagtttccg ctggcttctc gacaccttcc tcctcctggg
115561 ctgggtatgg tcaaagaaag acagttcagg ttctgaccag agaggactga tgacgatgga
115621 tccccaaccc ctcgtccctc ccctaacagg gggatcccct tccacccacc tgcaaatgtc
115681 actagcccag ctctgccctc cagaggcatc tgccatgagt gcgtgtgtgt gtgtgcatga
115741 atatgtaggg atgggagaac ggtgaggggc ccatagggca agggaaaaga tgagaacttt
115801 aggccgggca cggtggctga agcctgtaat cccagcactt tgggaggccg aggtgggtgg
115861 atcacaaggt caggagattg agaccatcct ggcaatcatg gtgaaacccc atctctacta
115921 aaagtacaaa aaagggctgg gcacggtggc tcatgcctgt aatcccagaa ctttgggagg
115981 caaaggtggg cggatcacga ggttaggaga tcgagatcat cctggctaac atggtgaaac
116041 ctcatctcta ctaaaaatac aaaaaattag ctgggtgtgg tggcgggcac ctgtagtccc
116101 agctactcag gaggctgagg caggagaata atgtgaaccc aggagccgga acttgcagtg
116161 agctgagatc acgccactgc actccaacct gggcgacaga gtgagacact gtctcagggg
116221 aaaaaaaaat acaaaaataa ttagccgggc atggtggcgg gtgcctgtag tcccagctac
116281 tcgggaggct gaggcagggg aatggtgtga acccaggagg cggaacttgc agtgagcgga
116341 gatcgcgcca ctgcactcca tccagcctgg gcgacagagc gagactctgt ctcaaaaaaa
116401 aaaaaaaaaa aaaaattagc caggtgtggt ggtggccacc cgtagcccca gctactaggg
116461 acgctgaagc aggagaatgg catgaaccca gcaggcagag cttgccgaga tctgctgcca
116521 agtggtgtga gccgagatca cgccactgca ctccagcctg agtgacagag caagactctg
116581 tctcaaaaaa aaaaaaaaaa agatgagaac tttagatatt gcatagccac aaagaaggag
116641 gtatcagatt aaaatatgca agtgtctcgg ggtcacagat agcgctgcct ggatatttaa
116701 ggtactgtgg ctgataagcc agaatacagc ataggggagg cattaggaga gagggttgtt
116761 ttttttggtt tttttttttt tgagacggag tctcgctctg ttgcccaggg tggagtgcag
116821 tggcgcgatc tcggctcgct gcaagcacca cctcctgggt tcacgccatt ctcctgcctc
116881 agccttccga gtagctggaa ctacaggcgc cagccaccat gcccccctga tttttttatt
116941 tttattttta gtagagaccg ggtttcacca tgttagccag gatggtctca atctcctgac
117001 ctcgtgatcc acccgcctcg gcctcccaaa gtgctgggat tacaggcgtg agccaccatg
117061 cccagcccag agagggtttt ttttgtttgt ttatttgttt ttggagacgg agtctcgctc
117121 tgtcgcccag gctggagtgc agtggcgcga tctcggctca ctgcaagctc cacctcccgg
117181 gttcatggca ttctcctgcc tcagcctccc aagtagctgg aactacaggc acataccatc
117241 acgcccggct aattttttgt attgttagcc aggatggtct cgatctcctg acctcgtgat
117301 ccaccctcct tggcctccca aagtgctgga attacaggtg tgagccacca cgcccggccc
117361 agagagggtt ttttaccttt ctgaatgctg gtctcaccta atgtacctta aatatcctgg
117421 ctaatgaact ccagagttct ctggcttctg tctctggtgc caaaccccca ggcaagggaa
117481 gtcccatgag gaaatttatc ccgcctcaaa tctgaaatcc tcctcacaat cgtcaggagt
117541 tgctgaggat tctgggctta gtccctggcc aggattatcc ctttgcccat caaaccctgg
117601 ctgaaatgac agtcccaaca ctatgactac ctcagggcca gctgcaagtt aaagacccac
117661 tgatgcctgt gcagcaaggg tctctgaacc caagtctgag ccagaaggta cggcaaagaa
117721 agggcctctt cttgagctaa agggataatc cccaaaggta caaggcaaaa gcctcaaatg
117781 cccccagttt ctgagaaacg cccagacgcc ccctcctcag gctcaagcca gctgccaagt
117841 actggagaag acaatcctga gggctctcct ggtctcctgg aagcagggat atggaggcct
117901 ctgtgtcaac agaatgtctc tatctttaaa ctgttttcct ggggttgtgg gtgaccccac
117961 tccaggaact gcagaaactc ttggccaggc actctgcctc tagggtgaaa tcctcaggga
118021 ataagtgtgc ccataacagg aagggtgcga ggatagaagg ggtgaattga ggaacattct
118081 gaatcaaact gaagaccctt gccctctgct tggacactga gagccaagat gaaggtaggg
118141 ggggttgacc cagtggaaca aatgccaggt cccaagtccc cccctactct accgtgtggc
118201 tcccagacgt ccctcctacc ctgaggcttc ataccctact cccccactta gctttggggg
118261 cccttttatc cccccctcca acccaaaccc tctccccacc aggctgggcc cctctcacct
118321 aggatgacag cttcctcctc gttctcatct gcttctgccc aggccacccg ctgggctcga
118381 cgctgggcct gtaggcggct gcccaggtcc ctccggcgcc gaggcctgcc tccagctctc
118441 ggctcctcag gctccagggg cccaggctgg gccacccggc ctgctcctgc cagctcctca
118501 ttgtgcagtg gctcttggcc ggctgtaggg aagacagaaa tgacaatgct gcctataagc
118561 ccagcccggc ccatggcagg aggagcggca gcccggaagc ctcacctcct ctgcttagcg
118621 gcctgctgcc cacagcacaa ggccccgact tctcagtctg actggcaagg cctttccac
118681 caggcccaca gcttggtccc aggcgggggc tcagcccttc cctcgggcct gtgtgaatac
118741 tcctctcccc tcccggctcc acctttccag ctggcctgtc aggaggcccc tatcctgatg
118801 tccccagctc tcatcgagta ccccctgcag gaatccactg atttctcgcc taacaccacc

118861 tgtggtttta tctggctccc cagccagaga gtacacttcc cggcggtggc cgagcctccc
118921 ccgggaagac ctgctggatc tccgccttcc ctcagcgtgt cccttccgct cctgggagcg
118981 cgcaggtgct cagaggtagc ttgtgggccc agggagcagg aaaacacata tacaaacccg
119041 cccgctgacc agaaatgggg ggaaagaggg aggatgggtg tatctgttct gtgccctggc
119101 caatgacatg gccttctgat cggggagcgg attggggagg gagagtgggc cctctccttg
119161 gcgggaaggg agagagcttc aggaatttcc tggagccgcc tcgcgcttct ccagctcgtg
119221 ggggacaggc agaaaatatt cagatgcccc cttcctggca cctgctctgg agccaggctg
119281 ggaggaatgg aaaacccctg cggcctccct aacaggtgca gagggaaagg gccccgacgg
119341 tgacggatgg gagtgtgcgc cggccagggc ctcaggcctt tggaggtatg gagttcccgg
119401 gggtgaagag agggatgagg ccagactctg gcttggagtc gagagaaagg ggtagcggcg
119461 cggccaccga ttccaggtag ggcactgcgg aagccccacc cggcacacga ctagcgcacg
119521 gacgcgcatg cgtcccgccc gcccaggtgc gcacgcgcag gagccgcggc gcgacggtcc
119581 acaaaggctc agaaggccag aaaacccggt accaccaacc ctggtgcagc ggcatctcct
119641 acctgatgcc gcccggcccc ggctgcgagt caggaagagg ataaagccga ctagcagagc
119701 cgccgctacc aagtaccaca caggcgccac catgacgagg gcctcagtgc agaaccactg
119761 cgtccaccct gaggccggga tctcataggc cccgccccta tttcccaatc tagactccgc
119821 ccactactcg gagccccgcc cagcggcaca tcacagagag gctttaatgt cagacattaa
119881 aatgagtttg gggggtgtaa agaaacccac aatgttaaga ggaaattata gcagacggta
119941 tgtccagtag aaaacatcta tgtatgtttt ttttttttga gacggagtct ctgtcgcaca
120001 ggctggagtg cggtggtgcg atctcggctc actgcaacct ccacctccca ggttcaagcg
120061 attctcctgc ctcagactcc cgagtagctg ggattacagg cgcccgccac cacgcccggc
120121 taatttttgt atttttagta gagatggggg tttcaccgtg ttggccaggc tggtctcgaa
120181 ctcctgacct tgtgatccac ccaccttggc ctcccaaagt gctgggatta caggcgtgag
120241 ccaccgcgcc cggactatat atgttttaaa aatcactaaa caatcccaca tagtgtttat
120301 ggaaacaacc gtggaaaaag tacgaaaaga ggcttgggac tgatgcacac caacttcagg
120361 atagcgatta cctctaggag gagggaagcg ggttggggtt ttgctgtatc tgtgacactg
120421 aatttctttc tttctttctt tcttttgagg ggaaggggag gggaggggag ggcagtactg
120481 gaatttatta agctttttgt atacatgata acagaagtaa agatttattt caggagccag
120541 gcacagaggc tcgtctgtta acccaatact ttgggaagtc taggtagagg tgagcctagg
120601 agttcaagac cagcctgggc aacatgatga aaccctgtct ctacaaaata caaaaattag
120661 ccaggtgtgg tggcatgcac ctgtgattcc aggtacttgg gaagatgagg tgggaagatc
120721 acctgagccc agggaggtcg aggctgcagt gagccatgat catgccagtg cactccagcc
120781 tgggtgatag agtgagaccc tgtctcagaa aataaaaata aataaaaata aattcaggac
120841 caatgaacaa tggtgagaaa gggatttgaa aaaatacggg ccagatgagg tggctctccc
120901 ctgtaatcct agcactttgg gaggccgagg tgggcggatc acttcaggtc aagtgttcaa
120961 aaccagccta gctgacgtga tgaaatcctg tctctactaa aaatacaaac aatgagtggg
121021 gcgtggtggc acatgcctgt aatcccagct actcgggagg ctgaggcagg agaatcactt
121081 gaacccaggg ggcggacgtt gcagtgagcc aagattgcac cactgcacac cagcctgagc
121141 gacagagcaa gactctgtct caaaaaaaaa aaaaaaagaa agaaagaaag aaaaagaaaa
121201 agaaaaaata tgggctgggc gaggtggctc acacctgtaa tcccagcact ttgggaggct
121261 gaggtgggtg gatcacctga actcaggagt ttgagaccag cctgacaaac acagtgaaac
121321 cctgtctcta ctaaaaatac aaaattagct tggcgtggtg gtgggtgcct gtaatcccag
121381 ctacttggga ggctgaggca ggagaatctc tcgaagccgg gaggcagagg ttgcagtgag
121441 ccgagatcac actattgcac tccagcctgg gcaacagagt gagactcagt ctcaaaaaaa
121501 aaaaaaaaaa aaaaaaagct gggcaccgtg gctcacgcct acaatcccaa ccaacacttt
121561 gggaggtcga ggtgggtgga tcacctgagg tcgggaattt gagaccagcc tgaccaacat
121621 ggagaaaccc catctctact aaaaatacaa aattagccgg gtgtggtggc tcatgcccat
121681 aatcccagca cttcgggagg ctgaggcagg cggatcacga ggtcaggaga tcaaaaccat
121741 cctggctaac acggtgaaac cccgtctcta ctaaaaatac aaaaaaatta gccaggcgtg
121801 gtggtgggcg cctgtagtcc cagctacttg ggaggctgag gcaggagaat ggtggggacc
121861 cgggaggcgg agcttgcagt gagctgagat cgcgccactg cactccagcc tgggcgacag
121921 agtgagactg tctcaaaaaa aaaaaaaaaa gaagaagaaa gatacaaaaa ttaaccaggc
121981 atggtggtgc atgcctggag tcccagctac tcaggaggct gaggtgggag aactgctcga
122041 acccaatatg cagaggttgc agtgagccgg gaccacacca ctgtacttca gcctgggcaa
122101 cagagcaaga ctccatctca aaaaaaataa ataaataaaa gaatgaaaaa gaaagttatc
122161 tatggacttg taattataga tgtcagttgt gatctttttg tttgttggtt ttttgagaca
122221 gggtctcact cacatcaccc aggctagagt acagtggcac tcttggctca ctgcagcctc
122281 gacttcccag gctcaggtga tcctcccacc tcagcctcct aaacagctgt gactacaggt
122341 gtgtgccccc aagcccaact aattttttgt attttttgta gagactgggt ttcaccatgt
122401 tgtctaggct tgtcttgaac tcctgggatc aagtgatcca ctgtgcccaa gctagttatc
122461 ttacattaca gaacacctac tatgtaccag gagctgggat ttaagaatga gaggtgaggc
122521 tgggcaccgt ggctcaagcc tgtaatctca gcactttggg aggctgaggt ggagggatct
122581 cctgaggtca ggagtttgag accagcctgg tcaatatggt gaaaccccac ctctactaaa

122641 agtacaaaaa ttcactgggt gtggtggtgc atgtctgtaa tcccagctac tcaggaggct
122701 gaggcaggag aatcccttga acccgggagt cggaggttga agtgagccga gatcgtgcca
122761 ctgcactcaa gcctgggcga caagagcaag actccatctc aaaacaacaa caacaacaac
122821 aacaaaacac agtggctgat gcctgtaatc ccagcactta gggaggccga ggtgggcata
122881 tcacgacgtc aggagttcga gaccagcctg gccaacatgg tgaaaccccg tctctactaa
122941 aaatgcagaa attagctggg cgtcgtgggg ggtgcttgta gtcccagcta ctcaggaggc
123001 tgaggcagga gaatcgcttc aacccaggag acagaggttg cagtgagcca agatcatgcc
123061 attgcactcc aggccaggtg actagagcaa gactccgtct caaaaaaaaa aaaaaaaaag
123121 aaaaagagag gtgagcaaaa acagatatgg tttttaccat taggattttt agaagagaaa
123181 acagaatcac acaaatgaat atctatttgc agtgcagagg tcagtgctat gcaagcagat
123241 gaaagggagc tcagggaagg ttttcctgag ctggggcttg agctggctaa gatccaagaa
123301 aggcatccat gtaaggtggg ctgtttttct tcttttcagg ctcctcgggg taactgatgt
123361 cccccaagcc ctcctctgcc cccaaatccc catggccact cctctaggga ctctgcataa
123421 tggttagtgt ttctacttca ataaaagaaa acaggtcacc acccaaagca gtggttccca
123481 cacatttttt ttctttttca gacgcagttt tgctcttgtt gcccaggctg gagtccgatg
123541 gcacgatctc cgctcactgc aacccccacc tcccgaattc aggcgattct cctgaatcct
123601 gaattctctg aactgggatt acaggcatgc accaccatgc ccagctaatt ttgtactttt
123661 tggtagagat ggggtttctc catgttggtc aggctggttt caaactcctg acctcgggtg
123721 atccactcgc ctcagcctcc caaagtgctg ggattacagg cgtgagccac cgggctttcc
123781 cacactttga tgagcatcag aatggcctga ggagcttggt aagaactcag actccctccc
123841 tttcttgtcc atgggcagaa gtggccattc aagaggtact gacacagggg tccaggcggg
123901 gggcagaggg ctgagtctct ggaggaggga ggtcagatga cttgctgcaa agagagtatc
123961 agcagaggtt cagcagaccc acctttgaga gggcacttcc tactttctgt tgtgtttgga
124021 atgcaaagat ctggctgaaa cggtggccac ggagaagggg tgatagattt gagaggctgg
124081 agggatgagg aagtgaggaa ctctacatac tgagctctca agcctaggag tgaaaacggg
124141 tgagaagttt gggcttgttt taggctatgc cgagtttaag ttcacagtcg agtggatcat
124201 agctggcggg taaggctgaa ggtgagatca ggatgagggt ttggaagccc ttgcctagaa
124261 ggaggcagtt ttttgagaca gcataggaga gcgcaaacct ccagggtcac agggctgcag
124321 tcttctcctt tcggagaccc cattgcagag aagagcgaaa gcaggggcgg ggtgggggtg
124381 ggagtggtct cttccggttc ttgaagatag cgtcccttag gcaggagggg tgggtcactc
124441 agtctccagg ctcgggaggc ccccagcaaa tcggacgctg tgggctaggc cgccaccgcg
124501 agcccactcg cccgatacgc gccttggggt ccgggtcggc tttccccggg acccctggcc
124561 ggaaactgag ccgttcactt ccgccaccag ccggaagttt tctgtcactg gacgccaagg
124621 agttttcggt ggctcagctg ggtaaccggg gatcaccatg gcggcctcat tggtggggaa
124681 gaagatcgtg tttgtaacgg ggaacgccaa gaagctggag gaggtgccgg gagggtgttg
124741 ggggctaact gggaggcggc tgggaatagg gcggagaagg ggcttccggg aggagggaag
124801 cacgtgggag gaggcatgga gagagaacag aattcagccc ggaagaatgg gtcctgaccc
124861 ggctgtgtgg aaaagctggg gcgcaagaag ggagtggctg cagaatcggg gcgccccggg
124921 gttggcgagg gaacgagggt ctcgacagat cccgacacag ggcttgtgtg gtcagcgact
124981 gcgggacccg gaggagtagc ggggctctta acaaccgccc cgaaggtcac ctattgaagt
125041 agaccccaac ccagtgtccc gacactcttc tgggacttat ccacatcccg ctgctgggcc
125101 agggctagca ggacccagag agggcgtggg acggagttcc acactgagca gtccgtgcct
125161 caccaccgtc ctgaaatgtt ctaacctctt ttcgcgtgtg tgaaatgggg atgatgcagg
125221 acatttgctt cacagcgcta tagagaggag gagtaaagga ggcagtggat ctaaagtttc
125281 tgtttgcagg gactttgtgg ctagcaagta acagaaacac acagggagct catgtaagcc
125341 accagatgac tcctgtggga atatagtgtt atcgtgtccc acaggacctg aagttctctc
125401 aagaagctga cttgtccttg ttctctctgg atgctgatcc ctattcctgt tcatatcttt
125461 cccctttctt ccctgctggg ggatggaaca atgaggcttc taccagatat cagctccgac
125521 tggctttgct tgaatcaaga gtttgcccct gttcaatcag ccatagccat ggagtggggg
125581 tcatgtgtgg gggatcagga tgacacccac tggatatgtc tgaggcagac cagtggggtg
125641 taatcactag ggacacctac atttgcctgt agtgtaggga gggactgatg tcactttggt
125701 gccaggactg agtggccttc tcaggaacca gagccttttg ccgaaaaaag gtttgggatc
125761 ctgaggccag accagtcagg cagtccaccc tgaacagagc ccatgcagga cagtgggcat
125821 gagaccccaa acctctggct gagaatattg ccctca//

- LOCUS: Auf070610 1621 bp mRNA linear PRI 05-AUG-1998
- DEFINITION: Homo sapiens clone 24505 mRNA sequence.
- ACCESSION: AF070610
- VERSION ORIGIN: AF070610.1 GI:3387992

1 tgcctctgta gagagcgctt cggagagaga ggcgaagtag gaagtgggat tttctcttcc
61 ctctcctggg cccgtttgcc cctaccctcg cccagcaagc tgcgcccaaa ttctattctg
121 cctctggaaa ctgctggacc atccaaggtc agctgcctgc cctgacccct accccagggc

181 cagcttgtcc tcctgggagg cgggacaggc cccagtgagg ttccgttgtg cgctgtgcct
241 atctctcgat tccagggcag atgagccaca acatcaccac cctgccactt acaaggtggg
301 ggacctgggt ctggggtctc aggcgcaaac tggaggccct cacagcccac taggccccct
361 cccaacccca gtaccctcag tccctcagtc aggtggtgct agtagagcta tctctgacgc
421 tgcaggcccc aggtagatgg gcagggccct ccgtgggtgt gcagcggcgg ttccctcatc
481 agtccagcca ctgcccagga ctaagggtct tcaaggaaga tgaggccatt gcccttctcc
541 aggtggcttc accacagtgc tgcctgggca cctctctgag gcagagaagt ggcccctccc
601 tcagtccctg cctggccgac cactgtggcc tgcacgcgaa tgggtcagag ccagaccccg
661 gagctggtgc tgtccttcta ggtggctgct catagctggg gatggtgagt ggctgtgttc
721 tggccttgcc cacgtgggtg tttgcagaga caaaatgaag gggatatgct ccccataaat
781 accaccttgc caccacaaga ggaccagaag gctactcagc cttgctgcca cagaagggga
841 tttgtagggt cagcctggct gcagaaaggc tggctcagat cttgaccaag aaaccccagc
901 gatttccatc aaagaccacg caggtaacag aggtgattcc tgaagaccca gcccacagag
961 ggtggcagcc aagcatctct gagcagtgtc cctgctagca tcgccagcgg ccatcactct
1021 gcctccctcc cacctcttgt ccctcgtcgt taaaaaacag caccctatcc tgcttcccca
1081 catttctgtt cctccaatga agggctaaga ctatttagta atctctttct taagcagagg
1141 agtggcaagg atggcaatct tgaattttat tttctgtaga gatagcattt cttctggtgc
1201 ggagctgaaa ggaatccacc cagaagttct gtagcatcct gcgtgcagcc tcctggagcc
1261 ccagactcca tctgggggag ggacttgttt acaagcagtt ctgaccacct tagtggtgta
1321 ctgttttcta ggcaaaaaat atctgtctgt tgtactgtat agcctttaaa atgcagtcca
1381 ggaatgagac tcttttaaga aacacatcct gcttctgcaa ttccagagag tgctggggga
1441 aaaaaaggga taaaaattcc tacctactca tcagtgtttg aaagatggag ctgaatagct
1501 tttcttgttc ctggactagg caatgaataa gcaacaatgt atcttttctg tgttcaaaat
1561 aaatacataa tatcaataaa atgtagcaag aagtaacact ttcaaaaaaa aaaaaaaaaa
1621 a//

- LOCUS: D85939 1167 bp mRNA linear PRI 17-APR-2007
- DEFINITION: Homo sapiens bcnt mRNA for bucentaur, complete cds, clone:hRACE#1.(CFDP1)
- ACCESSION: D85939
- VERSION ORIGIN: D85939.1 GI:2114175

1 cctagctgcc gtcgccgccg ccggggctct atggtctctc cctagagctt tgccgttgga
61 ggcggctgct gcggtcttgt gagtttgacc agcgtcgagc ggcagcaaca tggaggaatt
121 cgactccgaa gacttctcta cgtcggagga ggacgaggac tacgtgccgt cgggtggaga
181 gtatagtgaa gatgatgtaa atgaattagt gaaggaagat gaagtggatg gtgaagagca
241 gacacagaaa acccaaggga aaaaaagaaa ggcccagagc attccagcca ggaagagaag
301 acaaggtggc ctctcattag aagaagagga agaggaggat gccaattcag aatctgaggg
361 aagcagtagt gaggaggaag atgacgctgc agagcaggaa aaaggcattg gatcagagga
421 tgccaggaaa aagaaggagg acgaactctg ggccagcttc ctcaatgatg tgggaccaaa
481 atcaaaagtg cccccaagta cacaagttaa gaaaggagag gagactgaag agacaagttc
541 aagtaaattg ttggtaaaag cagaagagct agagaaacct aaagaaacag aaaaagttaa
601 aatcaccaag gtgtttgatt ttgctggtga agaagtaagg gtaactaagg aagtggatgc
661 tacatctaaa gaggccaaat ccttcttcaa gcagaatgag aaagaaaaac cacaggctaa
721 tgttccttca gctctgccat cactccctgc cgggtcaggg tgagtatttg agtctcactc
781 cctgatgtac ttggcacgtt ggagaagctt tcagtgcaaa tacctacttt cgctgcagat
841 cagactcctt gcctgagaga gagaattatg atgctgcttt gcctttgctg caatatttac
901 cagaatggaa gatgcaaagg aaaattagcc ataactttct caggaggatt gcttgaaccc
961 aggagtttgt gaccagcctg ggcaatacag taagaccctg tctcttaaaa aaaaaaaaat
1021 tagctgggca tggtagcttg cacctctgtc ctagctactg tggaggctga ggcaggagga
1081 tcccttgagc ccagaaggtt gaggctgcag tgagccgtga ttgtgccact gcactccagc
1141 ctgggtgaca gagagatcct gtctctt//

- LOCUS: HUMHTAR 2932 bp mRNA linear PRI 07-FEB-2003
- DEFINITION: Homo sapiens mRNA for thromboxane A2 receptor, complete cds.
- ACCESSION: D38081
- VERSION ORIGIN: D38081.1 GI:533325

1 gtaatgcaga gataataaaa cttcttaggt ccataggtct tataataatt taataaccta
61 aacatggtat acaaattcct ccaaacccaa taacataatt atagtttcaa aaagttcccc
121 aaactttcaa gttagatttt attgctttga tgagtggctt taaatatgaa aagtcttgcc

181 tgtgaagggc aatccttttc ccgtggactg ggatctatag aaatacagaa atgtgcccag
241 gggttcatct ccctaataac catcattcac atttctcaac ctccctaata accagccacc
301 atgtgagaag gatccacagt tactgtttat gactataatt aactagtacc tgggactggt
361 cagtggagtt ggttgcaacc tgatgctaag gatgtcaaag ttgtctcggc ctctgttccc
421 agccagtaag taattccctg gcctcgggcc atacccccta atcttggtca gctgattatg
481 acaggcagac agcacagtaa ataacactat atattaagaa aacccaaagc atatgtatca
541 atggtatata cccaacagca tcctaggaat ggagagtctg tagcaagggc ctccaatgtg
601 aaggtcaaca cagtcactgt gatgcgtgta tttccatttt gtaaagcatg atctctggtg
661 gtcattttta tcttcctaac ttattggaaa agtctcctgt tttgggggcc cgcccctggt
721 cacagccaga ctgactcagt ttccctggga ggtcccgctc gagcccgtcc ttcccctccc
781 tctgcccgcc cccagccctc gccccaccct cggcgcccgc acatctgcct gctcagctcc
841 agacggcgcc cggacccccg ggcgcgggat ccagccaggt gggagccccg cagatgaggt
901 ctctgaaggt gtgcctgaac cagtgccagc ctgccctgtc tgcagcatcg gcctgatggg
961 gtggtgactg atccctcagg gctccggagc catgtggccc aacggcagtt ccctggggcc
1021 ctgtttccgg cccacaaaca ttaccctgga ggagagacgg ctgatcgcct cgccctggtt
1081 cgccgcctcc ttctgcgtgg tgggcctggc ctccaacctg ctggccctga gcgtgctggc
1141 gggcgcgcgg caggggggtt cgcacacgcg ctcctccttc ctcaccttcc tctgcggcct
1201 cgtcctcacc gacttcctgg ggctgctggt gaccggtacc atcgtggtgt cccagcacgc
1261 cgcgctcttc gagtggcacg ccgtggaccc tggctgccgt ctctgtcgct tcatgggcgt
1321 cgtcatgatc ttcttcggcc tgtccccgct gctgctgggg gccgccatgg cctcagagcg
1381 ctacctgggt atcacccggc ccttctcgcg cccggcggtc gcctcgcagc gccgcgcctg
1441 ggccaccgtg gggctggtgt gggcggccgc gctggcgctg ggcctgctgc ccctgctggg
1501 cgtgggtcgc tacaccgtgc aatacccggg gtcctggtgc ttcctgacgc tgggcgccga
1561 gtccggggac gtggccttcg ggctgctctt ctccatgctg ggcggcctct cggtcgggct
1621 gtccttcctg ctgaacacgg tcagcgtggc caccctgtgc cacgtctacc acgggcagga
1681 ggcggcccag cagcgtcccc gggactccga ggtggagatg atggctcagc tcctggggat
1741 catggtggtg gccagcgtgt gttggctgcc ccttctggtc ttcattgccc agacagtgct
1801 gcgaaacccg cctgccatga gccccgccgg gcagctgtcc cgcaccacgg agaaggagct
1861 gctcatctac ttgcgcgtgg ccacctggaa ccagatcctg gacccctggg tgtatatcct
1921 gttccgccgc gccgtgctcc ggcgtctcca gcctcgcctc agcacccggc ccaggtcgct
1981 gtccctccag ccccagctca cgcagcgctc cgggctgcag taggaagtgg acagagcgcc
2041 cctcccgcgc ctttccgcgg agcccttggc ccctcggaca gcccatctgc ctgttctgag
2101 gattcagggg ctgggggtgc tggatggaca gtgggcatca gcagcagggt tttgggttga
2161 ccccaatcca acccggggac ccccaactcc tccctgatcc ttttaccaag cactctccct
2221 tcctcggccc ctttttccca tccagagctc ccaccccttc tctgcgtccc tcccaacccc
2281 aggaagggca tgcagacatt ggaagagggt cttgcattgc tatttttttt tttagacgga
2341 gtcttgctct gtcccccagg ctggagtgca gtggcgcaat ctcagctcac tgcaacctcc
2401 acctcccggg ttcaagcgat tctcctgcct cagcctcctg agtagctggg actataggcg
2461 cgcgccacca cgcccggcta atttttgtat ttttagtaga gacggggttt caccgtgttg
2521 gccaggctgg tcttgaactc ctgacctcag gtgattcacc agcctcagcc tcccaaagtg
2581 ctgggatcac aggcatgaac caccacacct ggccattttt tttttttttt tagacggagt
2641 ctcactctgt ggcccagcct ggagtacagt ggcacgatct cggctcactg caacctccgc
2701 ctcccgggtt caagcgattc tcgtgcctca gcctcccgag cagctgggat tacaggcgta
2761 agccactgcg cccggccttg catgctcttt gaccctgaat ttgacctact tgctggggta
2821 cagttgcttc cttttgaacc tccaacaggg aaggctctgt ccagaaagga ttgaatgtga
2881 aacgggggca cccccttttc ttgccaaaat atatctctgc ctttggtttt at//

- LOCUS: AI263044 464 bp mRNA linear EST 03-FEB-1999
- DEFINITION: qz29e03.x1 NCI_CGAP_Kid11 Homo sapiens cDNA clone IMAGE:2028316 3',mRNA sequence.
- ACCESSION: AI263044
- VERSION ORIGIN: AI263044.1 GI:3871247

1 aggtcattcc ttatttctta tttattccct ggcacaatca gaccacacta caagtttctt
61 ttaccagttg ccaaaatgaa atcatgacac ccttcagagc tactgtcgtt acatgcaact
121 caagacaacg agcagcatca taaccattgt caacaggcaa actccaacaa tgtctgggtt
181 tggatttggt tttatgttga gagaagcaga tttcctctga ttttaataat ccataagaaa
241 cagaaatcat aatatattga ctccatatta cctaaaaaga gtcaaaagaa gcagaagttc
301 tcccccgatt catcctcttg ttccacagac attttgaaaa taaaagaaga caaaatagaa
361 aggaaaggtg tgagaaagac acagaaatga cttgacgatt taaatcctat taacatcact
421 gggttgattt taccattaga ctgactgaat tagtctcact gtca//

- LOCUS: NM_014772 5737 bp mRNA linear PRI 17-NOV-2006
- DEFINITION: Homo sapiens KIAA0427 (KIAA0427), mRNA.
- ACCESSION: NM_014772 XM_375538
- VERSION ORIGIN: NM_014772.1 GI:7662111

1 gtcagatcag ggatcatttt ttttccttcc tctactccct cccccctacc cgcccctccc
61 tccctgtttc ccttccctcc ctccctcccc tctctgctgg gtctgtgcgc tggggcgccc
121 gatcccctcc gcagctggga cgctccgaac tcgaggcagg agtcggctct ccggagcctc
181 gtccctccct tccccttccc tgcccccttc ccccaccccc gactcgggct tggcgcggcg
241 gccagaggaa ccccgagtcc cggcccaggc ccctgagctg gagggatgga aaactcctct
301 gcagcatcag cctcctcgga ggcagggagc agccgctccc aggagatcga ggagctggag
361 cgcttcatcg acagctacgt gctggagtac caggtgcagg ggctgctggc tgacaagacg
421 gagggtgatg gcgagagcga gaggacccag tcccacatct cccagtggac agcggactgc
481 agcgaaccgc tggacagcag ctgttccttc tcccgagggc gagccccccc acagcagaat
541 ggcagcaaag acaactctct ggacatgctg ggcacggaca tctgggcggc caacaccttc
601 gattccttca gtggtgccac ctgggacctg cagccggaaa agctggactt cacccagttc
661 caccgcaaag tccgacacac gcccaagcag cccctgccac acatcgaccg cgaagggtgt
721 ggcaaaggga agctggaaga tggggatggc atcaacctga atgacatcga gaaggtcctt
781 ccagcctggc agggctacca cccgatgccc catgaagtgg agatcgcaca caccaagaag
841 ctgttccgca ggaggagaaa tgatcgaagg cggcagcaga gacctccggg gggcaacaag
901 ccccaacagc atggtgacca ccagccaggc agtgccaaac acaacaggga ccaccagaaa
961 tcctaccagg ggggctcagc accccacccc tcagggaggc ccactcacca tggctacagc
1021 cagaaccggc gctggcacca tggcaacatg aagcacccac caggcgacaa gggggaggca
1081 ggcgcacacc gcaatgccaa agagaccatg accatcgaga acccaaaact ggaggacact
1141 gcaggggaca ccgggcacag cagcctcgag gccccccgca gccctgacac cctggccccg
1201 gtggcttctg agcggctgcc cccacagcag tcaggggggc cagaggttga gacaaaacgt
1261 aaagacagta ttcttcccga gcgcatcggg gagcggccca aaattaccct gctccagtct
1321 tccaaagaca gactgcggcg aaggctaaag gaaaaggatg aagtggccgt ggagacgacc
1381 actccccagc agaacaagat ggacaagctg atcgagatcc tgaacagcat gcggaacaac
1441 agcagcgacg tggacaccaa gctcaccacc ttcatggagg aggcccagaa ctccaccaac
1501 tccgaggaga tgctgggcga gatcgtgcgc acaatctacc agaaggctgt gtccgaccgc
1561 agcttcgcct tcaccgctgc caagctctgc gacaagatgg cgctctttat ggtggagggg
1621 accaagttcc ggagcctgct cctcaacatg ctgcagaagg acttcacggt gcgcgaggag
1681 ctgcagcagc aggacgtgga gcgctggctg ggcttcatca ccttcctgtg tgaggtcttc
1741 ggcaccatgc gcagcagcac aggcgagccc ttccgtgtgc tcgtgtgccc catctacacc
1801 tgcctcaggg agctcttgca atctcaggat gtgaaggaag atgctgtcct ttgctgctct
1861 atggagctgc agagtacagg ccggctgctg gaggaacagc tgcctgagat gatgacagag
1921 ctcctggcca gcgcacggga caagatgctg tgcccctcgg agtccatgct gacccggtcg
1981 ctgctcctag aggtcatcga gctccacgct aacagctgga accctctgac gccccccatc
2041 acgcagtact acaacagaac catccagaaa ctgacagcct gacagccagg ggcctggca
2101 ggcggcccac gggcagctgg ggccctggtg cacagggcca gatggacagg cgggaggaca
2161 ggggtggccc tggcgggaga aagaaatggg gaggagggca ggcagagtcg gtggccagtc
2221 tggagccaga cggggaaggg agcaaatccc tgagaggagt gcccccgcac aagcccccca
2281 gcccgagcat gcaagctcac accaataagg gaagcatgtt tctttttcct ggtggccctg
2341 gccctcccct tcctcactcc cgcctctccc ctccccatca gacccatccc ccacggagct
2401 ttgtgtgagg gatctcatcg ctgtgactcc tcggagacct tggcagcctc gcacgccggg
2461 gcaccgcttg ggtcagaaag gacctcggaa ggctgaaaaa gtgggtcgga gacgggctcg
2521 cattgttccc gcatgctgtc agccgcagtc gccaactggc agcaggcgac gtgtagcaga
2581 tgtccgggag gacaaaggca ggcacggtcc ccaccagccg cccgtaattg acggcctttg
2641 tcagccatgg cagagctgac gctccacctc ccacctccaa gtcctcctca ctgcagcccc
2701 cacagcctca ggcctagggg gtcaggcgca gcgggggaga tggagtttgc agttccactt
2761 gcactctttt gtttattgtg ttttattttt caaaagtcgg ttgctttgaa gtctctttgg
2821 ccaatgaaaa tgcccgtgag gtgatcacac agtcagcact gttgaggacc cccggattag
2881 tgggagatca aacccagctc ccctctagaa gaaggattcg agccacagac agcttgccag
2941 tagccaatta gggtaattgg aaacttctgc cccggcgggg ggtccccgct ggaatcctgt
3001 gttcctcgcc actggcttcc agcgcctctg ttttctcaaa gggctgatac tgtcaccact
3061 gggaccaagt taaacctggt cctggcccca ggggccttgt ggcaaacagg gcacagaacg
3121 agactggcaa attaaaacca aaattctaga tggtgtcttg cgctccacac gcaggtctta
3181 ctggggaaaa ggatgggagt gggggctccc caggactcga ttttagctaa tgcgctgtgt
3241 cactgcccca gctcggacgt agaagcccag ccctccgtga gctcttggga aaggggtgaa
3301 ttcactgggt catggaaggg acagtcaggt gaccagcggg gtcgccagat gaagcttccc
3361 agccgggaaa caagacgggg tttcttggca ggccctggtc ctggggagca ggccctgttg
3421 ttggctggag aggaaggtgt ggggtggaac aggtgtccac atagctccat ctctggggc
3481 tggagcacac actttgatga gcccccccgg aaatgatgtc agagcctagc cgcttcctta

3541 tttgctcttt tattgaggcc gggcaggccc tgggtcactt tggaggcccc tcttggtcca
3601 cactggactg gccgggaggt gatgggcggg gaaggttctc gtgattgatt gattctgagt
3661 ctgagagtgg cgagtgggga gaggcttccc cagttctctc cagctttccc tgcagctgca
3721 acctgccctc tggtcccagg tgtggagcct ttgcctgtct ctaaaaagag cctgttggcg
3781 acaaggtgta gggggcacaa gtttacctga aacaggtcag tggtctctcc caagaagcgc
3841 acgccacctc tggtccctgg ccctgaaccc tgccttcttc ctccctccac ggtttcttcc
3901 cagactttct caagctcctc ctcactgccc ttcctcccca gcccagcctg ggaacacaga
3961 tgccccgcgg gtaggaggcc tcgagggagg agccgggctg atgcggggct gctcagggca
4021 ggccccaggg cgagcttgcc atcgtggcca ggcagcctcc acctgtgctt cagtggcccc
4081 tgcccccctg aagcatgtgg ggtttgtccg ctaggaggag gcaaggcccc cgaagagagg
4141 agagacctgg gagtgggagc tcaggtcagg gaggaggcag gggagtgggg tctcccagac
4201 ccaacggtga gctcagagca agcttcacgc aggacgctcc gaaacactgt gtggaggggg
4261 ctgtgttgtg ggcaccttgg ggcctgattc tccttcctcc gaacgggctc cttgatggcc
4321 tggccacagg ggcagctccc cattggctgt taggaccaga gtgtgaagaa gaagtgaaat
4381 ataaatatgt atacatatat aaatatattt ttaattacat gtcgtgtcac ggtggctcca
4441 gacatactgt ttgcctagtt tattccactg cttgaaagcg cttcctagcc aatctgaaca
4501 acaacacttt aagctgtttt tctaaatgca ggttgctgct cctttttcag atatggaagg
4561 aaaacgttaa gactattttt tttttaaaga aacaacagtc aagcctaaaa tttgagaccc
4621 cgaggcagct tcccgaggga gactgctcag acaggaactg caggacagaa gtggatgccc
4681 cacagaccct ggccccctcc ccaagtccat cccctctctg tggcatgagg aaggccgcgt
4741 ccgagttgac ctctgaatgt atgtgatgag aggcagagct ggatattgca tttctaaggc
4801 ttgcattgct ttcccctcgc ccgcggttct tggcgcatgg aagaggcggt ccagccatct
4861 gatgttgatc ctgtctcagt ctccccactg cctgtcagga tgagttagtc attgtttttc
4921 tccgaggcgg cctgcttgcc acagccctgc tccccaaggc ctggtggctt tgccgaagct
4981 ctgggaccgc agccccagcg aggcccccaa cctcacccag acgaggccag gagccccgcc
5041 accctccacg ggatgtgcac cctcagaccc cattctctct gttcgtcctt ccttgaccag
5101 tctgtaaacc ttcactgttt ggggatcgtc ctgtccatcc atgtaaatgt aaatgttggc
5161 cgagtcggta tttattctga ttgattttta ttttattcta ttattttctc cgaggatga
5221 gggtgggggg tgtgggaagg gtaccacaga tcaggccggg gcagctgtag ggcgggggc
5281 ccagacagcc aggccgccac cagagcagcc ccatggggtg ccccagacgc gggcctccaa
5341 gaagccaagt cccagtctgt tttctggcat cagacaccgg cccgtgttcc ttgtcagaca
5401 gacagactct caggcctgcc tggggagtcg tgtccctcag ctgcagggca ctgtgttggg
5461 aaaccattgg ctgggccttt gaggacacag atcagaagaa agaaagacaa ctttcctctg
5521 cgcggaacac tcacacggaa gggctggccg cctccctgag ccggctggga gtggacgaca
5581 ggacctacct ccccagagca agggcctggg gcttcccgcc aaagctgccg cggaaccccg
5641 ctagtgcgac caccctccct ccgtcggtat gtcctgcttt ccagctgaac ccaaactaca
5701 agtgggttta aaaaaaataa acaccaccac caaaaac//

- LOCUS: HSU85995 1696 bp mRNA linear PRI 01-JUL-1997
- DEFINITION: Human clone IMAGE:22181 unknown protein mRNA, partial cds. (PTHB1)
- ACCESSION: U85995
- VERSION ORIGIN: U85995.1 GI:1835749

1 cagtttctca agcgaccgat gttgaggtgg gaactgacct tgtcccttct gtcacggtga
61 aggtcacact gcagaacaga gtgatattgc aaaaagccaa attatcagtc tacgtgcaac
121 caccattaga attgacttgt gatcagttca cctttgaatt tatgaatcga aatcctgatg
181 gcattccgcg agttatccaa tgtaaattta gacttcccct aaagttaatt tgcctaccag
241 gtcagccttc aaaaactgca agccacaaaa ttactattga taccaacaaa tctccagtca
301 gtcttcttag tctcttccca ggttttgcca gtcagtcaga tgatgatcag gtgaatgtaa
361 tgggttttca cttcttagga ggtgctcgaa ttactgttct tgcttccaaa acttctcaac
421 gatatcgcat tcagagtgaa caatttgaag atctttggct cataaccaat gagcttattc
481 ttcgccttca agaatatttt gaaaaacagg gagtcaaaga ttttgcatgt tctttttcgg
541 gatctatacc ccttcaagaa tattttgagt tgattgatca tcattttgag ctacggataa
601 atggtgaaaa attaraagaa ctcttatctg agagagctgt acaatttcgg gccattcaac
661 gccggctact agcaagattc aaagataaaa ctcctgcccc tcttcaacac ctggacacct
721 tgttagatgg aacctacaag caggtaattg ctctagcaga tgcagtggag gaaaaccaag
781 gcaatctgtt ccagtcattc accaggctga agagtgccac ccatttggtg attctgctga
841 tcgcgctgtg gcagaagctt agtgctgacc aggttgctat tctggaagcg gcatttctgc
901 cgctacaaga agacactcaa gaattgggct gggaagaaac ggtggatgcc gccatttccc
961 acctgttgaa gacttgcctg tcgaagagtt ctaaggagca ggctttgaac ctcaacagcc
1021 agctgaacat acccaaagac acaagccaac tgaagaaaca tatcaccttg ctctgcgata
1081 gattatccaa aggtggccgt ctctgcctaa gtaccgatgc agcagcccca cagaccatgg

1141 tcatgccagg tggttgtact acaatcccag agtcagacct agaagaaaga tcagtagaac
1201 aagactctac agaactgttt accaaccaca gacatctcac tgcagagaca cccaggcctg
1261 aagtttcacc cctccaagga gtctcggaat aattcaagta gagttgtttg gttgagagga
1321 acatccccat ctcaaggccg aacctgtgtg aacctcatgc caagcacaga tatagggctg
1381 gcgcaggtgc ttcctaaagc tcaccttcct ggagatgaca tgcatagaaa gaggggttgg
1441 gactttttac ttcactagga gaacttgtaa caccatgggg aagtcagctg aaacttgtct
1501 tgttttgcca ggaaaggaag tagttgcctt tggtcatcca tctgctaata gtcacagaat
1561 acagtgaaat gacatagttt tgggttagat tttataatgc aaagattcag atccaaaata
1621 atttcatacc ccattttttc acagaattct tatatagtaa atgtatcaag tttaataaag
1681 catctcattg tcaaat//

- LOCUS: AW975818 719 bp mRNA linear EST 02-JUN-2000
- DEFINITION: EST387927 MAGE resequences, MAGN Homo sapiens cDNA, mRNA sequence. (YTHDC2)
- ACCESSION: AW975818
- VERSION ORIGIN: AW975818.1 GI:8167038

1 ccaaataatg aaactactat tttattcatt acaaacatca tataatcatt ctctataatc
61 actataaaaa tttaaatgct tcctttctcc tgtatatgaa gatggtagtt ctaaagcaac
121 actctaaaag tgttaaaaag cccattcgta acacacaccc cacctcagtt tttgaaagtc
181 agtgcattca gtggttataa ataggcatga tgttcttcca cagctaagag ttctgagtgt
241 caatccgttg tgtttttttc tcccaaggga agacgttccc ataactggag caactgttca
301 ccaacctgag gttctagttc ctgcccatcc ctgcttatct gcactttctt gttgtcattc
361 catggattga gtaaatggtg tgcaaattga aagggaaggc tttcttttcg tatccactcc
421 accttaaata ctcctcctag tccagcagag ccccagtcct gactcttttc ccttccaatc
481 tcagaagaca tcctagaaaa tccctggaaa tgtccagatc cttgaacaga aaataccaag
541 taaactatgc tgctttccca aaaggctcga tttagcttcc gttcattact aggagttgta
601 gaccagatac ccttctgttg agaaattcaa ggtttctcaa attgctactc ttcattatga
661 agtttcgaac aggcattttg ggtcttgggg aaagaaattt tgagcccctt
ccttaggat//

- LOCUS: NM_024593 2129 bp mRNA linear PRI 17-NOV-2006
- DEFINITION: Homo sapiens EF-hand calcium binding domain 1 (EFCAB1), mRNA.
- ACCESSION: NM_024593
- VERSION ORIGIN: NM_024593.2 GI:21314718

1 ggctcgcggg ggtcccgcga gggtctcggt agccgcgcgc ccagagaccc ccgccgcggc
61 ccgattctct gagcgccaga catgaaccgc aagaaactgc agaagctgac ggacacctta
121 accaaaaatt gcaagcattt taataaattt gaagtgaact gtcttataaa gcttttttat
181 gacttggtgg gaggagtaga gaggcaaggt ctggttgttg gactggatcg taatgcattt
241 cgaaacatcc tgcatgtgac atttggaatg acagatgaca tgattatgga cagagtattc
301 cgaggttttg ataaagataa tgatggctgt gtaaatgtat tggagtggat tcatggatta
361 tcactgtttc ttcgaggatc tttggaagaa aaaatgaaat attgctttga agtgtttgat
421 ttgaatggtg acggattcat ttcaaaggag gaaatgtttc acatgttgaa gaacagcctt
481 ctcaaacagc catctgagga agaccctgat gaaggaatta aagatttggt tgaaataaca
541 ctgaagaaaa tggatcatga ccatgatggg aagctgtctt ttgcagacta tgaactggct
601 gtgagagaag agactcttct actggaggcc tttgggccat gtcttcctga tccaaagagc
661 cagatggaat ttgaagctca agtattcaaa gatccaaatg aattcaatga tatgtgaata
721 actaaatgtc tatattgtct caagtgagta aaaaaggagg cacacacata ttgcttattt
781 tttctactta gttcagaaaa atgatgttga attgtgtgtt tcagggtgaa gatgaggttc
841 acttacatat atcatttaac atcagtgtaa gatgcaatta aaatagatgg aagacaagaa
901 gtcccttatt agatgatggg tttggcaaga cagttaactt ttctacagat gttttatcac
961 atagactatc tgtcgaagca cataactggc ataaaacctt tgggttcatt aaatgaatag
1021 agagattaca taattatgaa ttgttaatac aaatattctg catgtcaaaa agaagctggg
1081 tgtacactga aaaatcattg aaactactgg atttcctttg aaacaaataa tgaagatggc
1141 atcaaaagaa taagagtatt ctattgagca ttgaaacgcc tttctggtag gcattgaggg
1201 agatctagag agatgaaaga tacatggaaa tcatattata ttgaaatata atatgatttg
1261 cattattgtt tgaggctttg gctaaataac gttactgatt gtaagcacct gaaggcaagg
1321 gttttatcca ccacatcttt gtctgccata gcatttagtc ctctgcatac aatgggctgt
1381 ccataattac tgtagattgg actgaaaatc aaaaagtacc acaagataaa tgcaaactct
1441 tggtgagcag agggagaaca tgtactgagt ttcttaggtt tcatggctaa tgcattggca
1501 cagctttcag tgtatccctg cagtccttcc agatagtccc tgccccgtgc attgcagatg
1561 gttgccaagg cccccctctg tcctggcacc cagtgttcct ccatcctgac tccacgtatt

1621 gcccccttgc cactgcctga gtgcaccata gtgtccccat ccccccactg cctggagttc
1681 cccttgtgta cccccttatc agggcacgac ctaactgtgc cctgctggct gtgacctcct
1741 gaagacagac ctggcttctc cctgcatcca gttccccagt atgtgctgtg ggcatcctga
1801 gaagcctgaa aaagacaggg ccaggttagg cttgggtgag ctgctttctg ccttcttggt
1861 acacacagta agcagtggag aggcgtgtta caccgtatct gttctatcct catctaaata
1921 ctgagaggct agttgggata gtaaatcttt cttactacat atgcattaag tttaagtaaa
1981 atagaatatt gtgtgaaaaa atgcactgtt actaataaat ggaataaaca gatccatgaa
2041 ataaaagaaa aaaaaaaaaa aaaaaaaaaa aaaaaaaaaa aaaaaaaaaa aaaaaaaaaa
2101 aaaaaaaaaa aaaaaaaaaa aaaaaaaaa//

- LOCUS: BF968960 961 bp mRNA linear EST 22-JAN-2001
- DEFINITION: 602270156F1 NIH_MGC_84 Homo sapiens cDNA clone IMAGE:4358628 5', mRNA sequence.(TM2D1)
- ACCESSION: BF968960
- VERSION ORIGIN: BF968960.1 GI:12336175

1 actcgttggt gtcctgtggt tcgtctcagt cactacagga ccctgggggg ctgttgccac
61 ctccgccggg ggcgaggagt cgcttaagtg cgaggacctc aaagtgggac aatatatttg
121 taaagatcca aaaataaatg acgctacgca agaaccagtt aactgtacaa actacacagc
181 tcatgtttcc tgttttccag cacccaacat aacttgtaag gattccagtg gcaatgaaac
241 acattttact gggaacgaag ttggtttttt caagcccata tcttgccgaa atgtaaatgg
301 ctattcctac aaagtggcag tcgcattgtc tctttttctt ggatggttgg gagcagatcg
361 attttacctt ggataccctg ctttgggttt gttaaagttt tgcactgtag ggttttgtgg
421 aattgggagc ctaattgatt tcattcttat ttcaatgcag attgttggac cttcagatgg
481 aagtagttac attatagatt actatggaac cagacttaca agactgagta ttactaatga
541 aacatttaga aaaacgcaat tatatccata aatatttttt aaaagaaaca gatttgagcc
601 tccttgattt taatagagaa cttctagtgt atggatttaa agatttctct tttccaattc
661 atataccatt ttatgagttc tgtataatcg ccgcggctct tgtacgtgag ttaaggtata
721 ttattgggag aatcattcaa attggactcc ttggaagccg ataagagtgt tccggcctcc
781 gccctatgaa aaaagtcttc ccggcaacgc tgctaccttc cacagggagg gtgccctgtt
841 gcctttgggg ccgaacacca gggctccttc tcccacgaaa gcacaacacc aacccacccc
901 actcacaccc cgaccacgca caaaacggcc gcatcagcgc gccccccctt accacttaca
961 g//

- LOCUS: AF151022 1802 bp mRNA linear HTC 22-MAY-2001
- DEFINITION: Homo sapiens HSPC188 mRNA, complete cds.
- ACCESSION: AF151022
- VERSION ORIGIN: AF151022.1 GI:7106765

1 atttttgctc cctcccttgg ctgggcctga ggatttctcc gaggaagaga tgcaacgtga
61 gtggctagtg tggagcctca gagttggccg gaagaggagg gaggccagtg gagggagaga
121 gaggtgtcca ggccacaggc tcctgaaact ttgcagcagt gagttgctga gcacaggtgg
181 gacttacaca ggcagagcct ggctcagctg cccagcccag ggacacagtg gctcccacac
241 actcacccct agggctgcct gtcgacttgc agtacctgcc accagacaag cagcgagaac
301 ctgatgcaga catccgcaag atgcttgttg aagccatcat gctggtgaca gccacagcac
361 caggtcggca gcaggtgcgg gaccagggag cctacctgat ccttcgagag ctgcacagct
421 gggagccgga gcccgacgtg cggacggctt gtgagaagct catccaggtg cttattgggg
481 acgagcctga acgtggcatg gaaaacctgc tggaggtgca ggtgcctgag gatgtggagc
541 agcagctgca gcagctggat tgcagggagc aggagcagtt ggagcgggag ctggccccag
601 agccatgggt ggagagggcc acacccacct gaggcccctg cagccggaca ccagctccag
661 ggtccccttt gccaggcttt ctcagaccag gccttcctgc agctgtctgc aggccccctg
721 ggtccccttg ttcagagact gtgctcttct gagaagcaga gctacgctta ggctccagta
781 aggattggag gcactttcca gccctgtgca gtgttgctac cagcaagaat gaaggttgtg
841 cagagcagta ctgtgaggta ggcaccgtga ctcggctgcc tgtgcagaag gtggaggtgg
901 caggcgatgg ccagggagcc atgagaagac tcttctgggc acggcgtggg gactggacag
961 aaaaccactg caagccccaa ggagccagct gaagcctccc aatccccagg gcactgcaga
1021 gtctgggcag aggtgggcgc ataggtacaa gtgctgcttt gtcagcctac ttccctgcag
1081 ctctcagctc cctgttccct gagacctggc cttaaagggc tgacccagcc atacgtagat
1141 cctggggtct ttctaggact gtgcctccct gcttgaccta tgctccagga ccctgccctc
1201 aacccttgtc agctccctaa gtcttcctgt ttcccagtct cagattgagt gggtgctctc
1261 attggcctct ctagctaggc cgtctccctg gtcagcttcc ttgagcctgg gccactcctc
1321 cctggccttc ttacctctgc caagagcctc agatgtgcct gcagctagtc cctgcccaag

1381 cttctccccc agccaccctc tgcccctgtg gtagagccca ctataggccc aggcgcctta
1441 cgtgcttctc ccctggacct atgtgcatcc ctcacctgcc agttatgtgg ctgccaagcc
1501 ctacaggctg tcatggtggc tcttgccaca cttcccttcc tgagctgacc tgacctcact
1561 gccacctctt gcattgggat cctgagggct ggcccctgct cactgatggg ccctcccagg
1621 cccgtcaagc cctggtgaag gagcttaagc ttggcttctt ggcaattcac aatgcctcac
1681 tccacaccct aaaactggaa gcgtccccta tgggtcttcc tgcttgtggc ttgggtgccg
1741 gcgttccggc ttcaggatct ctcttaactg gccccaatta aagcttctgc cataaaaaaa
1801 aa//

- LOCUS: NM_006365879 bp mRNA linear PRI 10-JAN-2007
- DEFINITION: Homo sapiens chromosome 1 open reading frame 61 (Clorf61), mRNA.(CRCO4)
- ACCESSION: NM_006365
- VERSION ORIGIN: NM_006365.1 GI:5453624

1 ctcctcacag aagcctggag ctgggcatcc aagaagaagc agcctcattt gttttctggt
61 gtcatcgtag gtggccacct atggcttttg ggaatgtaaa aagggcagct ctctggcatg
121 ttcctgactg aggatctcat aacatttaac ttgaggaact tcctcctttt ccagctttgg
181 gagtcaagct tctcacctgg ggcgggtggg ttctgcacca ccctcccacc ctccttcctc
241 cgtgtggacg atagagccac atccagcacc acggacagct cccgggcgcc ttcatctcct
301 cgtcctccag gcagcacaag ccattgtgga atctccacca ggtgtacaga acggtgcctc
361 tgcgtcctgc cactcaggac ctctcaagtc cccgatgtga tggctcctca gcatgatcag
421 gagaaattcc atgatcttgc ttattcctgt cttgggaagt ccttctccat gtctaaccaa
481 gatctatatg gctatagcac cagctctttg gctcttggct tggcatggct aagttgggag
541 accaaaaaga agaatgtact tcatctggtt gggctggatt ccctctgata agccttccca
601 gttgactgaa agatgaggct aggctctagc aagttgaagt caaaccagct ccttcaagaa
661 gctttgagca gaatgaagtg gggaggaccc agcttccagc ccaggaagcc cactgtacct
721 ggagccatct gggataagac tttgacccat gactcccata tccacagcct gtccatccta
781 gcccatccca gtttatcctg tatcatttga gctgggattc ccacatcctc tgagttggaa
841 gtcccatctc aagtcttcaa taaagactct tgaatattg//

- LOCUS: NM_001189 2165 bp mRNA linear PRI 21-JAN-2007
- DEFINITION: Homo sapiens bagpipe homeobox homolog 1 (Drosophila) (BAPX1), mRNA.
- ACCESSION: NM_001189
- VERSION ORIGIN: NM_001189.2 GI:25121985

1 atggctgtgc gcggcgccaa caccttgacg tccttctcca tccaggcgat cctcaacaag
61 aaagaggagc gcggcgggct ggccgcgcca gagggcgccc cggcgcccgg gggcacagcg
121 gcatcggtgg ccgcggctcc cgctgtctgc tgttggcggc tctttgggga gagggacgcg
181 ggcgcgttgg ggggcgccga ggactctctg ctggcgtctc ctgccggtac cagaacagct
241 gcggggcgga ctgcggagag cccggaaggc tgggactcgg actccgcgct cagcgaggag
301 aacgagagca ggcggcgctg cgcggacgcg cggggggcca gcggggccgg ccttgcgggg
361 ggatccttga gcctcggcca gccggtctgt gagctggccg cttccaaaga cctagaggag
421 gaagccgcgg gccggagcga cagcgagatg tccgccagcg tctcaggcga ccgcagccca
481 aggaccgagg acgacggtgt tggccccaga ggtgcacacg tgtccgcgct gtgcagcggg
541 gccggcggcg ggggcggcag cgggccggca ggcgtcgcgg aggaggagga ggagccggcg
601 gcgcccaagc cacgcaagaa gcgctcgcgg gccgctttct cccacgcgca ggtcttcgag
661 ctggagcgcc gctttaacca ccagcgctac ctgtccgggc ccgagcgcgc agacctggcc
721 gcgtcgctga agctcaccga gacgcaggtg aaaatctggt tccagaaccg tcgctacaag
781 acaaagcgcc ggcagatggc agccgacctg ctggcctcgg cgcccgccgc caagaaggtg
841 gccgtaaagg tgctggtgcg cgacgaccag agacaatacc tgcccggcga agtgctgcgg
901 ccaccctcgc ttctgccact gcagccctcc tactattacc cgtactactg cctcccaggc
961 tgggcgctct ccacctgcgc agctgccgca ggcacccagt gaacccgctt gggctgaggc
1021 agcgagtgat tcccgcgctc cggctccgga ccggcgctga cagctgtagg ctgtagcctg
1081 cacggggcgc cccgccaagg aggcacctgg aggtgaaacc cagctccagc tcccgttagc
1141 caggacttgt cccctggcag ctgggctgag tctgccctga gggggcgcct tttctaatt
1201 tgaacagagg caccctatgg cctaggggcc ctgatcgccc acctgcctgg aagcccctgg
1261 gctctattta ttatcatgac aatgttggaa ttaaattttg attcgaatat gtctgcctgg
1321 gggtggggtt ttccctgagc ggcaactcct ggagaccaca tagcctgaat cctcagaatt
1381 tcaggcctgc tgggagcttt ctgcactagg ccacactagt tcatggtatc catgctacca
1441 atctatgtgt atctacatat cttttatttt tggaaattgc atttgtaacc aaggggtgcg
1501 aaaccctggc agtcccaggc agcaccaggc caggggttga tttgaaacgt gaaggattgg

1561 gttttcaggc cctctgctcc acccctcctg tgtgtcagag ctagggtggg ggtgcccgat
1621 tcgggtgctg aatgtaagga ggggagcctc caagtgtggt gcaagccggg ggtctccaca
1681 tcttccttct ctgaagtcca ggtacctgca caagcaggaa gcgcctggga gtcccggaag
1741 gaggagagcg cacacccagg cagccctctg cggaaacttt ccttggtttc tttttatttg
1801 tgtaaaggag gttaagacgt gtcgcacttt tcagttgttt gtattcaaat gacgattatt
1861 tttctactca atgtgaatat ccctggccag cctttccacg gcgcccaccg cagtgccgct
1921 gcctggccct cagtgtctac cttctgccct ctgcgactcc agtgctctgg cccgggactc
1981 ccctatccgc ccctcactta cccttaaaca ggtgatccca cctgtcttgt caacctcgcc
2041 gcttttcgcc tccttaatgg cactgtgcac tcaactagag tattaactgt aaaaagattt
2101 gtgaagtttg gaagctctat tcgctgtatt ttttctttaa tttataaact tttagtttaa
2161 catgc//

- LOCUS: BC001205 4985 bp mRNA linear HTC 15-AUG-2006
- DEFINITION: Homo sapiens mRNA similar to KIAA0700 protein (cDNA clone IMAGE:3501686).(SIN3B)
- ACCESSION: BC001205
- VERSION ORIGIN: BC001205.1 GI:12654726

1 gccgcttcaa gctcagcact cacaagatgg tgttcatcgt gaactccgag gactacatgt
61 accgtcgcgg gaccctctgc cgggccaagc aggtgcagcc cctggtcctg ctccgccacc
121 accagcactt tgaggagtgg cacagccgct gctggaggac aatgtgacgg tggaggcggc
181 tagcctggtg caggactggc tgatgggtga ggaggacgag gacatggtac cctgcaagac
241 gctgtgtgag acagtgcacg tgcacggcct gcccgtgacc cgctaccgcg tgcagtacag
301 ccgccgcccg gcctcgccct gacccgccct catgggcacc gggcaggcgc ctcacagagc
361 acagacgtgc cctcggcctt ggtcgtgtcg gggccgtttt cttgaacgac gtgagaggca
421 tctccoagcc cctctgctgc cggacagcgc actccagggc aggacgccgc ccccgtggct
481 cccggtctcc tgtgggcctg ctgtgtgcca aacctgagct acctgcaccc gagccctggg
541 gctcagcccc accacagggg cgggtggacg tgctggccga ggaacaagca atcatgtttg
601 cgtccccgtc cgtcacatgt gccaaatccc tggcaggcag atggctcctg ccccatattc
661 ttgccgtgtc ttggaaaagt cacaggtgac agccctcctg aggcccacac tttggagggg
721 gtccatgtcc aagagaccct gacaaggaag gagttgagcc ctgggtaggc ttgcttgaaa
781 agtgagtcct tccagaatat tctcagagct gccagaaaca tcatctgagg gtttgggcat
841 cagctctgcc tggctggtgt tcaggccctc agtcactttg gaagtgagaa gaacccttca
901 gccaaacctt tgggatccca ttttgaagtc cagagctgga gcaggcgggc agtgccctgg
961 gagaccccgc tctggataag acacccaacc ccaactccag cagctggact ctgggcccac
1021 aaggggaggg accccagcca gtattccctc agagccggca cgaccctgct gtggggcggc
1081 gggcccagtc gtgttcctgc agtgggcacc ctaggcgtat gtgtatacac gcacagatat
1141 ttattccttt cgcttttagt ggttgttatt tctgtagcag ccaagtcctg cggctccctg
1201 gggctgagat ctccctttgt ggaactccga gagccgccct gacagtccca gccgccaccc
1261 aaggagcccc atccgcccac ccaccctggg acccacgcgg ccactccagg gatcattgtt
1321 gtccttttgt gaggggctga ggcgactgtc ccatggaggc atccaaggct ttctgtgcca
1381 agaatcccaa gtgactgggg ctggggacca cttctccagg cctctctccc cgaccatgag
1441 agcccctggc cctggcccct gcacaccagt gggcccagaa taaccgccac ccttgccatc
1501 ctgcaacagc tggactcaag tttccaggaa gtggagatgt gtttctagaa gtttcctgtg
1561 gaacctggag gctcctgacg cctttgactc caggctgggc gattccgatg ctgacccgtc
1621 ctcctgggct ccccaccccc gctccccaag tgtcacacag gcactttgta cacatcctgg
1681 ttttctaaga agggaacttg ccatcaagat tcctgtacca ggcccacggc cgtctcgggc
1741 agagccacct gggccccggg tgcagccgga gaccccgccg agcccctcct cccagtattc
1801 ccatccccgc atgcttctag ggagcgcctc ttttccgttc ttgaggaatt tctgttctgg
1861 ggcttgtcct tcctttgcag ctgttttgaa tgtagttttc cttttctatt tatttgcaca
1921 ttaaagttaa taattgaata ttgaaaaaaa aaaaaaaaaa actcgagact agttctcccc
1981 ccatccctca aattggcagc accccagagt tacccggtga cctggccagg ctctgggcgt
2041 gaggccttca ccaatccaag ggctattagt acagacgtgt aaatccttcc accccgaccc
2101 gcacacacac ccacactgct gccctggcgg gggccatggg ggtgatgaat gaccctccaa
2161 cagccccaca tgggttgttt ctgtttcttt ggcttttctc gctccgcagt ggagggttta
2221 ctaggattta agcttttgag tgcattgaga accaagacag ggcctggctc caactctgtg
2281 ggccagaggt gggggactgc taggtcgagt ctgcagcttc gccagtttct tggttgggac
2341 actcctctgg cagccccagc accaccacaa ccccttgcag tgtgccccag tcccctggat
2401 tcgctggact gcaaaaagga gcaccaggga aagctcagca aaggctcagg aggcctcccg
2461 ggtcctcggg agatgaagca tccgtgccta ggaaaagggg acagagggca aggagagaag
2521 tgagagctac aattccagca ttggagaagc gaggggcggg gcgtcaacgg cactgcttca
2581 ggacgcgctt gctgaaacga ctccaacagc tagttcacag cccagctttg tacgttggtt

2641 accatagcta ctgctgtcac tgtagctgct cccgtagggt cgttgattcc tgaacgtatc
2701 acatctcacc tgcccccttc ctcgtgggac gtgtcaagtt gactttaaag cctaaggtgg
2761 cttgtgggga ctgcaccaga aagtgtctaa ccttgtgttc cccgaaatcc tttctctgac
2821 ttaggaacca gcgccccctg ctggagaagt tttttcattt tgttagccga cttctggtag
2881 cttagcaaag agaccagctg actgtctctc ggcccagccc accacgccga gtagcttgtg
2941 tggatgcagt cctgtgaggg tgtgcataac cgttcccagg tgtacgcacg cgcgcgcgcg
3001 cgcacagaca cacacacaca cacacacaca cacacacaca cagtaaccac gggtaggcac
3061 caggggcttt gtacattcag gagctgcaga gaggaaaggt ctcccatgta ccagagaagg
3121 aaaagtctga tcccgaaaca gcttgaacga aaggtggtta acacgtgacc aatccccatg
3181 gcagacagcg ggctggtgaa caggaagagc acagaatctc tgtggggcga tgctgcagcg
3241 gctgaaacca cgtcaagtcc cccagagccc ggcattctat gtaagcatcc acctgtgacg
3301 ccgtggagca ggatcttcat tcatctcacc agagcaacag cccagtcctg aggatgggag
3361 ggacgtgact tagccttggg attccacgct cgccgccctc ccttggccgc cctactcccc
3421 agtttcctgg cgatgagcca tttattgtgt ctcacaccag cctcatttcc aactccgact
3481 tcctacttag aatgtggcag tggctttgtg agtgagaatc ctgatgagtt ttccagtggc
3541 ctctgcagaa gctggccccc ttctaagggc atagctaacc cccttcccac tcctgagtca
3601 ctgaattcga ggagggtggg gcagggaaag ggctggcccc cctgctgaaa tcttgattct
3661 gcatttgagg aaggtcaggg tgccgctggg ggagcaggga gcggggaggg aggaagaggg
3721 gaatgcacat cttttgcgcc actattaagg cacctgtcac cttacatctc aatctggaat
3781 caaagtgccc tgggttgaga agcagacctg ggctctggtc acactttggt cactcattag
3841 ctctaggacc agtcactaat ctctgagact ccattttctc cagggaaacg aggcttgccc
3901 agatagacta acaacccttt agtgctccaa gaagtgagaa tttccgaaaa agatctgccg
3961 gcccagagca ctccctcttg ccctaatcct ggcagggtct ggatgtattt acccaactct
4021 gatcttgcgt gcagggatgc ttgctgatgg atgaaagacc acaataaaac aagattagca
4081 gcaaaacaat tttaattgtc agtgaaaagc aaacatgttt gtcagactct cctgtggagt
4141 catttcaaat ggtaactctt ctcttgtacc aggaagcagc tgtaggtggg gtaatgggat
4201 ctgtctcctg gtgcagaggg gcagcttatg gcggcagcag cttcaacaag cccgtcccac
4261 ggccagccct aactgggcca agtgcaagct cctgatgagc tgctgggcct ggcagcgttc
4321 cccacttcct agtgccttgc tcccctggaa ttccacagcc aatcaaccac agcagccgtg
4381 tcctccttgc cagaaataac atcattcgta cgtcctgcct ttgtaaaaat caagacccag
4441 ttaaccatca cccacttctt gcccctgatt gatggggtcc ctgagtacaa gcgtcctgtg
4501 caatcccctc cctcctaccg ctttcctagc taagccctgc tagccccaca aatcagaatc
4561 tgggtttggg gtgctggaga gatggtgggc cccagctctt ccccacatag cccacgctgc
4621 tcagggcacg gtctccaacc aggctcgaga ttgcctgcct gcccgctgca gccgcagggc
4681 cccaccctgt ggaaaaggaa gcttccccca ctcaactgca ctgtcctttt ctgctgtcta
4741 gtctgtgcta agggacacta cttgtacact tttcaaacgg tgccctaaat tggaagagaa
4801 ggaggggcca ggaacacagc cctctccgcc gtctttctcc acctccactt caccactcct
4861 gctctagact tcttgtcttg catctttgat aacttggagt cataaccgag tggatgtcgg
4921 aataaatgag agatttgaag tagaaaaaaa aaaaaaaaaa aaaaaaaaaa aaaaaaaaaa
4981 aaaaa//

### Literatur:

1. Hohenberger W: The effect of specialization or organisation of rectal cancer surgery ; in: Rectal Cancer Surgery, Optimisation, Standardisation, Documentation. Springer Verlag Berlin Heidelberg, Soreide O,Norstein J (eds) pp353-363 (1997)
2. Hohenberger W, Schick Ch, Göhl J: Mesorectal lymph node dissection: is it beneficial? Langenbeck's Arch Surg 383: 402-408 (1998)
3. Hermanek P, Wichelt H, Staimmer D, Riedl St. and the German Study Group Colo-Rectal Carcinoma (SGCRC): Prognostic factors of rectum carcinoma - experience of the German multicentre study SGCRC Tumori 81 Supplement: 60-64; (1995)
4. Birkenkamp-Demtroder, K., L. L. Christensen, et al. (2002). "Gene expression in colorectal cancer." Cancer Res 62: 4352-63.
5. Notterman, D. A., U. Alon, et al. (2001). "Transcriptional gene expression profiles of colorectal adenoma, adenocarcinoma, and normal tissue examined by oligonucleotide arrays." Cancer Res 61: 3124-30.
6. Croner RS, Foertsch T, Brueckl WM, Guenther K, Siebenhaar R, Matzel KE, Kirchner T, Behrens J, Klein-Hitpass L, Stuerzl M, Hohenberger W and Reingruber B. Common denominator genes that distinguish colorectal mucosa from carcinoma. Int J Colorectal Disease, Epub ahead of print 22.12.2004
7. S. Merkel, C.Rödel, W.Hohenberger. Colorectal Carcinoma-Surgical treatment. Endo heute 2004; 17:97-103.
8. Tanaka S, Haruma K, Tatsuta S, Hiraga Y, Teixeira CR, Shimamoto F, Yoshihara M, Sumii K, Kajiyama G. Proliferating cell nuclear antigen expression correlates with the metastatic potential of submucosal invasive colorectal carcinoma. Oncology. 1995 Mar-Apr;52(2):134-9.
9. Aoki R, Tanaka S, Haruma K, Yoshihara M, Sumii K, Kajiyama G, Shimamoto F, Kohno N. MUC-1 expression as a predictor of the curative endoscopic treatment of submucosally invasive colorectal carcinoma. Dis Colon Rectum. 1998 Oct;41(10):1262-72.
10. Yamamoto H, Iku S, Adachi Y, Imsumran A, Taniguchi H, Nosho K, Horiuchi S, Yoshida M, Itoh F, Imai K. Association of trypsin expression with tumour progression and matrilysin expression in human colorectal cancer. J Pathol. 2003 Feb; 199(2):176-84.
11. Frederiksen CM, Knudsen S, Laurberg S, Orntoft TF. Classification of Dukes B and C colorectal Cancers using expression arrays. J Cancer Res Clin Oncol (2003)129:263-271.
12. Kwon HC, Kim SH, Roh MS, Kim JS, Lee HS, Choi HJ, Jeong JS, Kim HJ, Hwang TH. Gene expression profiling in lymph node positive and lymph node negative colorectal cancer. Dis Colon Rectum (2004)47:141-152.
13. Friederichs J, Rosenberg R, Mages J, Janssen KP, Maeckl C, Nekarda H, Holzmann B, Siewert JR: Gene expression profiles of different clinical stages of colorectal carcinoma: toward a molecular genetic understanding of tumor progression. Int J Colorectal Dis (2005)20:391-402.
14. Koehler A, Bataille F, Schmid C, Ruemmele P, Waldeck A, Blaszyk H, Hartmann A, Hofstaedter F, Dietmaier W. Gene expression profiling of colorectal cancer and metastases divides tumours according to their clinopathological stage. J Pathol (2004) 204:65-74.
15. Croner RS, Foertsch T, Brückl WM, Siebenhaar R, Günther K, Hlubek F, Stremmel C, Hohenberger W, Reingruber B. Tissue preperation for gene expression profiling of colorectal carcinoma - three alternatives to laser microdissection with preamplification. J Lab Clin Med 2004 Jun; 143 (6):344-351.
16. Croner RS, Peters A, Brueckl WM, Matzel KE, Klein-Hitpass L, Brabletz T, Papadopoulos T, Hohenberger W, Reingruber B, Lausen B. Microarray versus conventional prediction of lymph node metastasis in colorectal carcinoma. Cancer 2005;104(2); 395-404.

## Patentansprüche

1. Ex-vivo Verfahren zur Prädiktion einer Lymphknotenmetastasierung bei kolorektalem Karzinom, das die nachfolgenden Schritte umfasst:
- Gewinnen einer Probe des Tumorgewebes des kolorektalen Karzinoms;
- Extrahieren der RNA aus der Probe des Tumorgewebes des kolorektalen Karzinoms;
- Gegebenenfalls Umschreiben der RNA in cDNA oder cRNA;
- Nachweis, ob die Probe cDNA oder cRNA enthält, die zumindest den Nukleinsäuresequenzen nach Tabelle 1 oder 2 entspricht, wobei die differentielle Expression dieser cDNA oder cRNA für die Prädiktion einer Lymphknotenmetastasierung bei kolorektalem Karzinom verwendet wird.

2. Verfahren nach Anspruch 1, bei dem der Nachweis durch RT-PCR erfolgt.

3. Verfahren nach einem der Ansprüche 1 oder 2, wobei der Nachweis mittels komplementärer Nukleinsäuresonden, vorzugsweise cDNA- oder Oligonukleotid-Sonden, durchgeführt wird.

4. Verfahren nach Anspruch 3, bei dem die Nukleinsäuren durch Nukleinsäuresonden nachgewiesen werden, die zur Hybridisierung an zumindest einen Abschnitt jeder der Nukleinsäuren oder ihre komplementären oder reversen oder revers-komplementären Sequenzen sowie der hiervon abgeleiteten RNA-Sequenzen oder Derivate dieser Sequenzen in der Lage sind.

## Claims

1. *Ex vivo* method for predicting a lymph node metastasis in connection with colorectal carcinoma, comprising the following steps:
- taking a sample of the tumor tissue of the colorectal carcinoma;
- extracting the RNA from the sample of the tumor tissue of the colorectal carcinoma;
- optionally transcribing the RNA into cDNA or cRNA;
- detecting if the sample contains cDNA or cRNA which at least corresponds to the nucleotide sequences according to Tables 1 or 2, wherein the differential expression of this cDNA or cRNA is used for predicting a lymph node metastasis in connection with colorectal carcinoma.

2. The method according to claim 1, wherein the detection is carried out by RT-PCR.

3. The method according to claim 1 or 2, wherein the detection is carried out by complementary nucleic acid probes, preferably cDNA or oligonucleotide probes.

4. The method according to claim 3, wherein the nucleic acids are detected by nucleic acid probes being able to hybridize to at least a segment of each of the nucleic acids or to their complementary or reverse or reverse-complementary sequences as well as to RNA sequences derived therefrom or to derivatives of these sequences.

## Revendications

1. Procédé ex vivo permettant de prédire une métastase aux ganglions lymphatiques en cas de cancer colorectal, qui comprend les étapes suivantes :
- obtention d'un échantillon du tissu tumoral du cancer colorectal ;
- extraction de l'ARN depuis l'échantillon du tissu tumoral du cancer colorectal ;
- le cas échéant, conversion de l'ARN en ADNc ou ARNc ;
- détection de l'éventuelle présence d'ADNc ou ARNc correspondant au moins aux séquences d'acide nucléique selon le Tableau 1 ou 2, l'expression différentielle de ces ADNc ou ARNc étant utilisée pour la prédiction d'une métastase aux ganglions lymphatiques en cas de cancer colorectal.

2. Procédé selon la revendication 1, dans lequel la mise en évidence est effectuée par RT-PCR.

3. Procédé selon l'une des revendications 1 ou 2, la détection étant réalisée à l'aide de sondes d'acides nucléiques complémentaires, préférentiellement des sondes ADNc ou oligonucléotides.

4. Procédé selon la revendication 3, dans lequel les acides nucléiques sont détectés par sondes d'acides nucléiques, aptes à s'hybrider avec au moins une section de chacun des acides nucléiques ou des séquences complémentaires ou inverses ou complémentaires inverses ainsi que des séquences ARN en étant déduites ou des dérivés de ces séquences.
